# EUROPÄISCHE PATENTSCHRIFT

(11) **EP 2 900 645 B1**
(45) Veröffentlichungstag und Bekanntmachung des Hinweises auf die Patenterteilung: **21.08.2019**
(21) Anmeldenummer: 13766930.5
(22) Anmeldetag: 19.09.2013
(51) Int. Cl.: C07D 261/04, A01N 43/80

(54) **HERBIZID WIRKSAME 3-PHENYLISOXAZOLINDERIVATE**
HERBICIDAL AND FUNGICIDAL 3-PHENYLISOXAZOLIN DERIVATIVES
DÉRIVÉS DE 3-PHÉNYLISOXAZOLINE À ACTION HERBICIDE ET FONGICIDE

(30) Priorität: 25.09.2012 EP 12185768
(43) Veröffentlichungstag der Anmeldung: 05.08.2015
(73) Patentinhaber: Bayer CropScience AG, 40789 Monheim am Rhein (DE)
(72) Erfinder: KUHN, Birgit, 65779 Kelkheim (DE); WILLMS, Lothar, 65719 Hofheim (DE); FRENZEL, Thomas, 51061 Köln (DE); HAAF, Klaus, Bernhard, 65779 Kelkheim (DE); LINDELL, Stephen, David, 65779 Kelkheim (DE); DIETRICH, Hansjörg, 65835 Liederbach am Taunus (DE); SCHMUTZLER, Dirk, 65795 Hattersheim (DE); GATZWEILER, Elmar, 61231 Bad Nauheim (DE); ROSINGER, Christopher, Hugh, 65719 Hofheim (DE)
(74) Vertreter: BIP Patents
(86) Internationale Anmeldenummer: PCT/EP2013/069453
(87) Internationale Veröffentlichungsnummer: WO 2014/048827

(56) Entgegenhaltungen:
- EP-A1- 1 203 768
- EP-A2- 0 174 685
- WO-A1-92/03053
- WO-A1-95/14680
- WO-A1-99/05130
- WO-A1-2005/021516
- WO-A2-2005/021515
- WO-A2-2005/051931
- WO-A2-2008/035315
- US-A- 5 939 418
- GUCMA MIROSLAW ET AL: "Synthesis and fungicidal activity of substituted isoxazolecarboxamides", PESTYCYDY = PESTICIDES : QUARTERLY OF THE INSTITUTE OF INDUSTRIAL ORGANIC CHEMISTRY, INSTYTUT PRZEMYS U ORGANICZNEGO <WARSCHAU> , Bd. 2010, Nr. 1-4 1. Januar 2011 (2011-01-01), Seiten 21-31, XP008159982, ISSN: 0208-8703 Gefunden im Internet: URL:http://www.wydawnictwa.ipo.waw.pl/pest ycydy/Pestycydy1-4-2010/Gucma.pdf
- PRIYA B S ET AL: "DELTA.2-Isoxazoline derivatives as antimicrobials", HETEROCYCLIC COMMUNICATIONS, FREUND PUBLISHING HOUSE, TEL AVIV, Bd. 12, Nr. 1, 1. Januar 2006 (2006-01-01) , Seiten 35-42, XP009140872, ISSN: 0793-0283
- QUAN M L ET AL: "Design and Synthesis of Isoxazoline Derivatives as Factor Xa Inhibitors", JOURNAL OF MEDICINAL CHEMISTRY, AMERICAN CHEMICAL SOCIETY, US, Bd. 42, Nr. 15, 1. Januar 1999 (1999-01-01), Seiten 2760-2773, XP002213660, ISSN: 0022-2623, DOI: 10.1021/JM980406A in der Anmeldung erwähnt
- NAGARAJAN ET AL: "Synthesis of some isoxazolines and evaluation of carbon-13 NMR substituent parameters for 5-methyl substitution", INDIAN JOURNAL OF CHEMISTRY : IJC, COUNCIL OF SCIENTIFIC AND INDUSTRIAL RESEARCH, IN, Bd. 32B, Nr. 9, 1. Januar 1993 (1993-01-01), Seiten 938-941, XP009167082, ISSN: 0019-5103 in der Anmeldung erwähnt
- QUAN M L ET AL: "Bisbenzamidine isoxazoline derivatives as factor Xa inhibitors", BIOORGANIC & MEDICINAL CHEMISTRY LETTERS, PERGAMON, AMSTERDAM, NL, vol. 7, no. 21, 4 November 1997 (1997-11-04), pages 2813-2818, XP004136536, ISSN: 0960-894X, DOI: 10.1016/S0960-894X(97)10081-6
- NAGARAJAN A ET AL: "An improved synthesis of isoxazolines based on Torssell's procedure", INDIAN JOURNAL OF CHEMISTRY. SECTION B, COUNCIL OF SCIENTIFIC AND INDUSTRIAL RESEARCH (C S I R), IN, vol. 32, no. 4, 1 April 1993 (1993-04-01), pages 471-474, XP009101573, ISSN: 0019-5103

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO1999/05130 A1 sind herbizid wirksame 3-Phenylisoxazoline bekannt, die in 5-Position u.a. durch eine Hydroxy- und Trifluormethylgruppe substituiert sind. WO2005/021516 A1, WO1995/014680 A1, WO 2008/035315 A1, WO2005/051931 A1 und WO2005/021515 A1 beschreiben unter anderem jeweils 3-Phenylisoxazolin-5-carbonsäuren, -ester und -aldehyde, die am Phenylring in 3- und 4-Position durch Alkoxy-Reste substiuiert sind. Diese Verbindungen werden als Vorstufen zur Herstellung von pharmakologisch wirksamen 3-Aryl-isoxazolin-5-carbonsäureamiden beschrieben. In J. Med. Chem. 42 (1999) 2760 werden die Verbindungen Methyl-3-(3-cyan-phenyl)-5-methyl-4,5-dihydro-1,2-oxazol-5-carboxylat, Ethyl-3-(3-cyanphenyl)-5-methyl-4,5-dihydro-1,2-oxazol-5-carboxylat, 3-(3-Cyanophenyl)-5-methyl-4,5-dihydro-1,2-oxazol-5-carbonsäure und Methyl-3-(3-cyanphenyl)-5-(methoxymethyl)-4,5-dihydro-1,2-oxazol-5-carboxylat als pharmakologisch wirksam beschrieben. Aus Indian Journal of Chemistry, Section B: Organic Chemistry Including Medicinal Chemistry (1993), 32B(9), 938-41 ist die Verbindung Methyl-5-methyl-3-(3-nitro-phenyl)-4,5-dihydro-1,2-oxazol-5-carboxylat bekannt. Izvestiya Akademii Nauk, Seriya Khimicheskaya (1996), (2), 426- 429 nennt die Verbindung Methyl-3-(3,5-di-tert-butyl-4-hydroxyphenyl)-5-methyl-4,5-dihydro-1,2-oxazol-5-carboxylat. Unter den nachfolgenden CAS-Nummern sind jeweils die darauf folgenden Verbindungen bekannt:
1326815-55-7: 3-(3-Bromphenyl)-5-methyl-4,5-dihydro-1,2-oxazol-5-carbonsäure,
1326814-80-5: 3-(5-Brom-2-fluorphenyl)-5-methyl-4,5-dihydro-1,2-oxazol-5-carbonsäure,
1326811-51-1: 3-(5-Brom-2-methoxyphenyl)-5-methyl-4,5-dihydro-1,2-oxazol-5-carbonsäure,
1326813-57-3: 3-(3-Brom-4-methoxyphenyl)-5-methyl-4,5-dihydro-1,2-oxazol-5-carbonsäure,
1326814-71-4: Ethyl-3-(3-chlorphenyl)-5-methyl-4,5-dihydro-1,2-oxazol-5-carboxylat,
1326810-45-0: 3-(3,4-Dichlorphenyl)-5-methyl-4,5-dihydro-1,2-oxazol-5-carbonsäure,
1326815-81-9: 3-(2,5-Dichlorphenyl)-5-methyl-4,5-dihydro-1,2-oxazol-5-carbonsäure,
1326813-44-8: 3-(4-Chlor-3-nitrophenyl)-5-methyl-4,5-dihydro-1,2-oxazol-5-carbonsäure,
1326810-59-6: 3-(2-Chlor-5-nitrophenyl)-5-methyl-4,5-dihydro-1,2-oxazol-5-carbonsäure,
1326814-16-7: 3-(3-Chlor-4-fluorphenyl)-5-methyl-4,5-dihydro-1,2-oxazol-5-carbonsäure,
1326815-03-5: 3-(3-Fluorphenyl)-5-methyl-4,5-dihydro-1,2-oxazol-5-carbonsäure,
1326810-73-4: 3-(3,4-Difluorphenyl)-5-methyl-4,5-dihydro-1,2-oxazol-5-carbonsäure,
1326811-86-2: 3-(2,5-Difluorphenyl)-5-methyl-4,5-dihydro-1,2-oxazol-5-carbonsäure,
1326811-50-0: 3-(2,3-Difluorphenyl)-5-methyl-4,5-dihydro-1,2-oxazol-5-carbonsäure,
1326813-45-9: 3-(4-Fluor-3-methoxyphenyl)-5-methyl-4,5-dihydro-1,2-oxazol-5-carbonsäure,
1326813-37-9: 3-[2-Fluor-5-(trifluormethyl)phenyl]-5-methyl-4,5-dihydro-1,2-oxazol-5-carbonsäure,
1326811-79-3: 3-(3-Methoxyphenyl)-5-methyl-4,5-dihydro-1,2-oxazol-5-carbonsäure
1326811-74-8: 3-(2,5-Dimethoxyphenyl)-5-methyl-4,5-dihydro-1,2-oxazol-5-carbonsäure,
1326814-78-1: 3-(3-Methylphenyl)-5-methyl-4,5-dihydro-1,2-oxazol-5-carbonsäure
1326813-55-1: 3-(2,5-Dimethylphenyl)-5-methyl-4,5-dihydro-1,2-oxazol-5-carbonsäure,
1326815-05-7: 5-Methyl-3-(3-nitrophenyl)-4,5-dihydro-1,2-oxazol-5-carbonsäure,
1326812-83-2: 5-Methyl-3-[3-(trifluormethyl)phenyl]-4,5-dihydro-1,2-oxazol-5-carbonsäure,
1027426-25-0: Methyl-3-(3-cyanphenyl)-5-(ethoxymethyl)-4,5-dihydro-1,2-oxazol-5-carboxylat,
1026410-22-9: Methyl-3-(3-cyanphenyl)-5-(isopropoxymethyl)-4,5-dihydro-1,2-oxazol-5-carboxylat,
1027205-06-6: Methyl-5-(butoxymethyl)-3-(3-cyanphenyl)-4,5-dihydro-1,2-oxazol-5-carboxylat,
231300-28-0: Methyl-3-(3-cyanphenyl)-5-(2-methoxyethyl)-4,5-dihydro-1,2-oxazol-5-carboxylat, und
1026948-50-4: 3-(3-Cyanphenyl)-5-(trifluormethyl)-4,5-dihydro-1,2-oxazol-5-carbonsäuremethylester.

Eine herbizide Wirkung der über ihre CAS-Nummern bekannten Verbindungen ist nicht offenbart.

Aufgabe der vorliegenden Erfindung ist die Bereitstellung herbizid wirksamer Verbindungen, die darüberhinaus auch als Vorstufen zur Herstellung weiterer herbizid wirksamer Verbindungen geeignet sind.

Es wurde gefunden, daß bestimmte 3-Phenylisoxazolinderivate dafür besonders gut geeignet sind. Ein Gegenstand der vorliegenden Erfindung sind 3-Phenylisoxazolinderivate oder deren Salze der Formel (I) 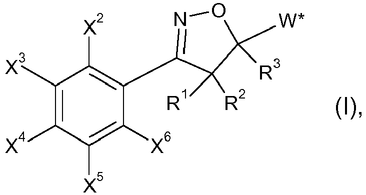 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
   oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet durch jeweils m Reste aus der Gruppe bestehend aus Fluor, Chlor, Brom, lod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
W* bedeutet COOH, COOY, CN oder CHO;
Y bedeutet durch m Reste aus der Gruppe bestehend aus Fluor, Chlor, Brom, Cyano, Hydroxy und COO-(C₁-C₈)-Alkyl substituiertes (C₁-C₈)-Alkyl, das durch n Heteroatome aus der Gruppe bestehend aus Sauerstoff, Schwefel und Stickstoff unterbrochen ist, oder
   einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom, Iod, Cyano, Nitro,
   oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₂-C₆)-Alkenyloxy oder (C₂-C₆)-Alkinyloxy;
X⁵ bedeutet Wasserstoff, Fluor, Chlor, Brom, lod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₂-C₆)-Alkenyloxy oder (C₂-C₆)-Alkinyloxy;
m bedeutet 0, 1, 2, 3, 4 oder 5;
n bedeutet 0, 1 oder 2;
p bedeutet 0 oder 1;
q bedeutet 3, 4 oder 5;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
   Methyl-3-(3-cyanphenyl)-5-(isopropoxymethyl)-4,5-dihydro-1,2-oxazol-5-carboxylat,
   Methyl-5-(butoxymethyl)-3-(3-cyanphenyl)-4,5- dihydro-1,2-oxazol-5-carboxylat,
   Methyl-3-(3-cyanphenyl)-5-(methoxy-methyl)-4,5-dihydro-1,2-oxazol-5-carboxylat,
   Methyl-3-(3-cyanphenyl)-5-(2-methoxyethyl)-4,5-dihydro-1,2-oxazol-5-carboxylat,
   3-(3-Cyanphenyl)-5-(trifluormethyl)-4,5-dihydro-1,2-oxazol-5-carbonsäuremethylester,
   Methyl-3-(3-cyan-phenyl)-5-methyl-4,5-dihydro-1,2-oxazol-5-carboxylat,
   Ethyl-3-(3-cyanphenyl)-5-methyl-4,5-dihydro-1,2-oxazol-5-carboxylat,
   Methyl-5-methyl-3-(3-nitro-phenyl)-4,5-dihydro-1,2-oxazol-5-carboxylat,
   Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

Alkyl bedeutet gesättigte, geradkettige oder verzweigte Kohlenwasserstoffreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit der jeweils angegebenen Anzahl von Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit der jeweils angegebenen Anzahl von Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Dimethylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlor-fluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome und/oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der Formel (I) umfaßt, jedoch nicht spezifisch definiert sind. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Je nach Art der oben definierten Substituenten weisen die Verbindungen der Formel (I) saure Eigenschaften auf und können mit anorganischen oder organischen Basen oder mit Metallionen Salze, gegebenenfalls auch innere Salze oder Addukte bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Ist eine Gruppe mehrfach durch Reste substituiert, so bedeutet dies, daß diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Bevorzugt sind 3-Phenylisoxazolinderivate der Formel (I), worin
R¹ und R² bedeuten jeweils Wasserstoff;
R³ bedeutet (C₁-C₄)-Alkoxy-(C₁-C₄)-alkyl, Vinyl, (C₂-C₄)-Alkinyl, (C₂-C₄)-Alkinyl, Halogen-(C₁-C₄)-Alkyl oder Halogen-(C₂-C₄)-Alkenyl;
R⁵ bedeutet durch jeweils m Reste aus der Gruppe bestehend aus Fluor, Chlor, Brom, lod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₂-C₆)-Alkenyloxy oder (C₂-C₆)-Alkinyloxy;
X⁵ bedeutet Wasserstoff, Fluor, Chlor, Brom, lod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸, oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₂-C₆)-Alkenyloxy oder (C₂-C₆)-Alkinyloxy;
m bedeutet 0, 1, 2, 3, 4 oder 5;
n bedeutet 0, 1 oder 2;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
   Methyl-3-(3-cyanphenyl)-5-(isopropoxymethyl)-4,5-dihydro-1,2-oxazol-5-carboxylat,
   Methyl-5-(butoxymethyl)-3-(3-cyanphenyl)-4,5- dihydro-1,2-oxazol-5-carboxylat,
   Methyl-3-(3-cyanphenyl)-5-(methoxy-methyl)-4,5-dihydro-1,2-oxazol-5-carboxylat,
   Methyl-3-(3-cyanphenyl)-5-(2-methoxyethyl)-4,5-dihydro-1,2-oxazol-5-carboxylat,
   3-(3-Cyanphenyl)-5-(trifluormethyl)-4,5-dihydro-1,2-oxazol-5-carbonsäuremethylester,
   Methyl-3-(3-cyan-phenyl)-5-methyl-4,5-dihydro-1,2-oxazol-5-carboxylat,
   Ethyl-3-(3-cyanphenyl)-5-methyl-4,5-dihydro-1,2-oxazol-5-carboxylat,
   Methyl-5-methyl-3-(3-nitro-phenyl)-4,5-dihydro-1,2-oxazol-5-carboxylat,
   Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

Besonders bevorzugt sind 3-Phenylisoxazolinderivate der Formel (I), worin
R¹ und R² bedeuten jeweils Wasserstoff;
R³ bedeutet (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet Methyl oder Ethyl;
R⁶ bedeutet Wasserstoff oder R⁵;
R⁷ bedeutet Wasserstoff oder durch jeweils m Reste aus der Gruppe bestehend aus Fluor und Chlor substituiertes (C₁-C₆)-Alkyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und (C₁-C₄)-Alkoxy substituiertes (C₁-C₄)-Alkyl oder (C₁-C₄)-Alkoxy;
X³ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
X⁵ bedeutet Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
m bedeutet 0, 1, 2 oder 3;
n bedeutet 0, 1 oder 2;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
   Methyl-3-(3-cyanphenyl)-5-(isopropoxymethyl)-4,5-dihydro-1,2-oxazol-5-carboxylat,
   Methyl-5-(butoxymethyl)-3-(3-cyanphenyl)-4,5- dihydro-1,2-oxazol-5-carboxylat,
   Methyl-3-(3-cyanphenyl)-5-(methoxy-methyl)-4,5-dihydro-1,2-oxazol-5-carboxylat,
   Methyl-3-(3-cyanphenyl)-5-(2-methoxyethyl)-4,5-dihydro-1,2-oxazol-5-carboxylat,
   3-(3-Cyanphenyl)-5-(trifluormethyl)-4,5-dihydro-1,2-oxazol-5-carbonsäuremethylester,
   Methyl-3-(3-cyan-phenyl)-5-methyl-4,5-dihydro-1,2-oxazol-5-carboxylat,
   Ethyl-3-(3-cyanphenyl)-5-methyl-4,5-dihydro-1,2-oxazol-5-carboxylat,
   Methyl-5-methyl-3-(3-nitro-phenyl)-4,5-dihydro-1,2-oxazol-5-carboxylat,
   Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

Ganz besonders bevorzugt sind 3-Phenylisoxazolinderivate der Formel (I), worin
R¹ und R² bedeuten jeweils Wasserstoff;
R³ bedeutet (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet Methyl oder Ethyl;
R⁶ bedeutet Wasserstoff oder R⁵;
R⁷ bedeutet Wasserstoff oder durch jeweils m Reste aus der Gruppe bestehend aus Fluor und Chlor substituiertes (C₁-C₆)-Alkyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und (C₁-C₄)-Alkoxy substituiertes (C₁-C₄)-Alkyl oder (C₁-C₄)-Alkoxy;
X³ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
X⁵ bedeutet Fluor, Chlor, Brom, Cyano, oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
m bedeutet 0, 1, 2 oder 3;
n bedeutet 0, 1 oder 2;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
   Methyl-3-(3-cyanphenyl)-5-(isopropoxymethyl)-4,5-dihydro-1,2-oxazol-5-carboxylat,
   Methyl-5-(butoxymethyl)-3-(3-cyanphenyl)-4,5- dihydro-1,2-oxazol-5-carboxylat,
   Methyl-3-(3-cyanphenyl)-5-(methoxy-methyl)-4,5-dihydro-1,2-oxazol-5-carboxylat,
   Methyl-3-(3-cyanphenyl)-5-(2-methoxyethyl)-4,5-dihydro-1,2-oxazol-5-carboxylat,
   3-(3-Cyanphenyl)-5-(trifluormethyl)-4,5-dihydro-1,2-oxazol-5-carbonsäuremethylester,
   Methyl-3-(3-cyan-phenyl)-5-methyl-4,5-dihydro-1,2-oxazol-5-carboxylat,
   Ethyl-3-(3-cyanphenyl)-5-methyl-4,5-dihydro-1,2-oxazol-5-carboxylat,
   Methyl-5-methyl-3-(3-nitro-phenyl)-4,5-dihydro-1,2-oxazol-5-carboxylat,
   Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

Die in oben genannten Dokumenten beschriebenen Verbindungen weisen nicht nur eine pharmakologische Wirkung auf, sondern überraschenderweise auch eine herbizide Wirkung. Gegenstand der vorliegenden Erfindung sind daher ferner herbizide Mittel, gekennzeichnet durch einen herbizid wirksamen Gehalt an mindestens einer Verbindung der Formel (I) 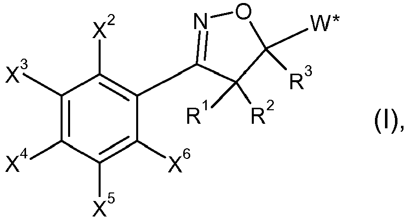 worin
R¹ und R² unabhängig voneinander jeweils Wasserstoff, Fluor, Chlor, Brom, lod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy bedeuten,
   oder
R¹ und R² gemeinsam mit dem Kohlenstoffatom, an das sie gebunden sind, einen gesättigten, teilweise oder vollständig ungesättigten drei-, vier- oder fünfgliedrigen Ring bilden, der aus q Kohlenstoffatomen und p Sauerstoffatomen aufgebaut ist;
R³ Fluor, Chlor, Cyano, (C₁-C₃)-Alkylcarbonyloxy oder S(O)ₙR⁵,
   oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
   oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl bedeutet;
R⁵ durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl bedeutet;
R⁶ Wasserstoff oder R⁵ bedeutet;
R⁷ Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl bedeutet;
R⁸ R⁷ bedeutet;
W* COOH, COOY, CN oder CHO bedeutet;
Y durch m Reste aus der Gruppe bestehend aus Fluor, Chlor, Brom, Cyano, Hydroxy und COO-(C₁-C₈)-Alkyl substituiertes (C₁-C₈)-Alkyl, das durch n Heteroatome aus der Gruppe bestehend aus Sauerstoff, Schwefel und Stickstoff unterbrochen ist, oder
   einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl bedeutet;
X², X⁴ und X⁶ unabhängig voneinander jeweils Wasserstoff, Fluor, Chlor, Brom, lod, Cyano, Nitro,
   oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl bedeuten;
X³ Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy bedeutet;
X⁵ Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
   oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
   oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy bedeutet;
m 0, 1, 2, 3, 4 oder bedeutet 5;
n 0, 1 oder 2 bedeutet;
p 0 oder 1 bedeutet;
q 3, 4 oder 5 bedeutet;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
   Methyl-3-(3-cyanphenyl)-5-(isopropoxymethyl)-4,5-dihydro-1,2-oxazol-5-carboxylat,
   Methyl-5-(butoxymethyl)-3-(3-cyanphenyl)-4,5- dihydro-1,2-oxazol-5-carboxylat,
   Methyl-3-(3-cyanphenyl)-5-(methoxy-methyl)-4,5-dihydro-1,2-oxazol-5-carboxylat,
   Methyl-3-(3-cyanphenyl)-5-(2-methoxyethyl)-4,5-dihydro-1,2-oxazol-5-carboxylat,
   3-(3-Cyanphenyl)-5-(trifluormethyl)-4,5-dihydro-1,2-oxazol-5-carbonsäure,
   Methyl-3-(3-cyan-phenyl)-5-methyl-4,5-dihydro-1,2-oxazol-5-carboxylat,

   Ethyl-3-(3-cyanphenyl)-5-methyl-4,5-dihydro-1,2-oxazol-5-carboxylat, Methyl-5-methyl-3-(3-nitro-phenyl)-4,5-dihydro-1,2-oxazol-5-carboxylat,
   Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

Darüberhinaus eignen sie sich hervorragend als Zwischenstufen zur Herstellung von herbizid wirksamen 3-Phenylisoxazolin-5-carboxamiden und 3-Phenylisoxazolin-5-thioamiden.

Erfindungsgemäße Verbindungen, in denen W* für COOH steht, können gemäß dem Fachmann an sich bekannten Reaktionen beispielsweise nach der in Schema 1 genannten Reaktionsfolge hergestellt werden.

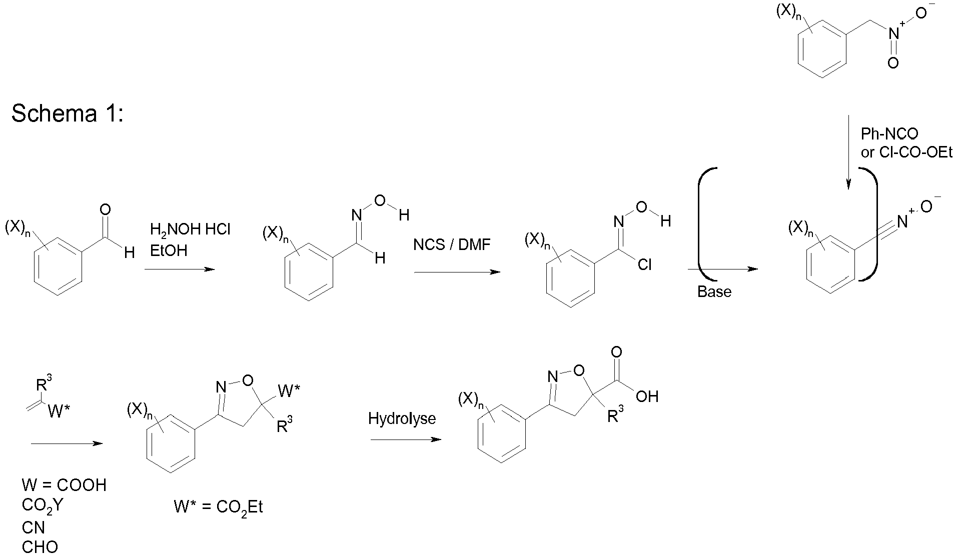

In Schema 1 und den nachfolgenden Schemata steht (X)ₙ für die Substituenten X², X³, X⁴, X⁵ und X⁶. Solche 1,3-dipolaren Cycloadditionen von Nitriloxiden mit geeigneten Dipolarophilen sind beispielsweise beschrieben in Reviews: 1,3 dipolar Cycloaddition Chemistry, Padwa (Editor), Wiley, New York, 1984; Kanemasa and Tsuge, Heterocycles 1990, 30, 719 und H. Feurer, Nitrones and Nitronates in Organic Synthesis, Second Edition, 1-129, Hoboken, New Jersey 2008.

Die Cycloadditionen können in einer Eintopfreaktion ohne Isolierung des Dipolvorläufers bzw. mit Isolierung des Dipolvorläufers durchgeführt werden. Erfindungsgemäße Verbindungen, die in der 4- und 5- Position des Isoxazolin-Ringsystems substituiert sind, lassen sich ebenfalls durch 1,3 dipolare Cycloaddition herstellen, indem geeignet 1,2-disubstituierte Olefine als Dipolarophile eingesetzt werden. Zumeist entstehen bei dieser Reaktion Diastereomerengemische, die durch Säulenchromatographie getrennt werden können. Optisch aktive Isoxazoline können durch chirale HPLC geeigneter Vorstufen oder Endstufen erhalten werden, ebenso auch durch enantioselektive Reakionen wie z.B. enzymatische Ester- oder Amidspaltungen oder durch den Einsatz von chiralen Hilfsreagenzien am Dipolarophil wie von Olssen beschrieben, (J. Org.Chem. 1988, 53, 2468). Neben den erfindungsgemäßen Säuren (W* = COOH) werden auch deren Salze beansprucht, die auf den üblichen Weg durch Umsetzung mit einer anorganischen bzw. organischen Base hergestellt werden können (Schema 2). Darin steht Me⁺ für ein Metallkation.

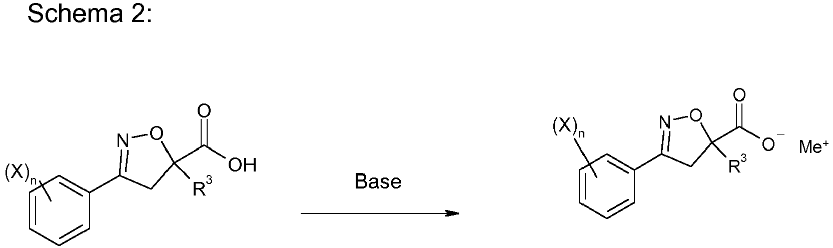

Erfindungsgemäße Verbindungen, in denen W* für COOH steht, können auch durch die in Schema 3a angegebenen Methoden durch Verseifung von Anhydriden oder Hydrolyse von Nitrilen, oder durch die in Schema 3ba angegebenen Methoden durch Oxidation von Alkoholen oder Aldehyde hergestellt werden. 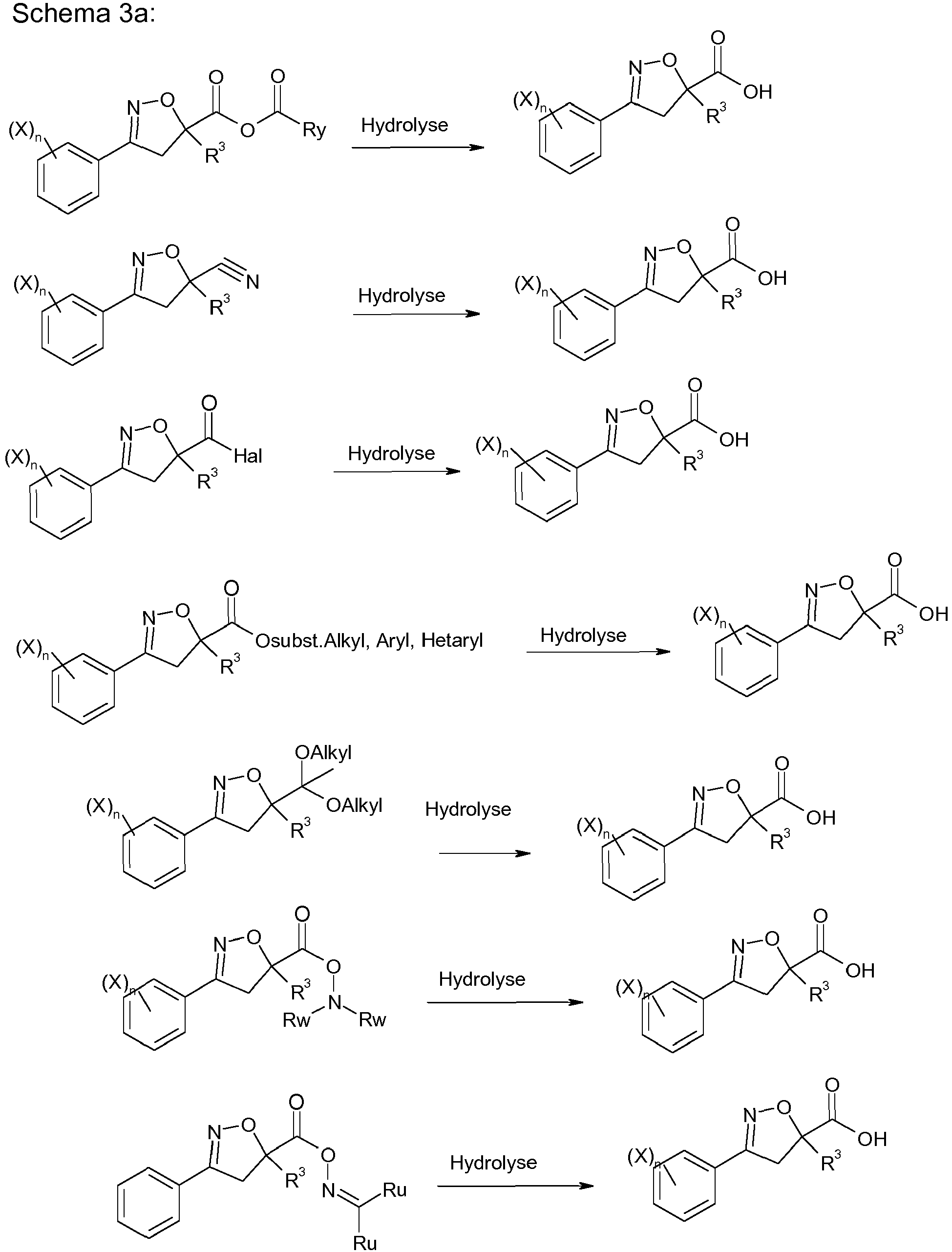 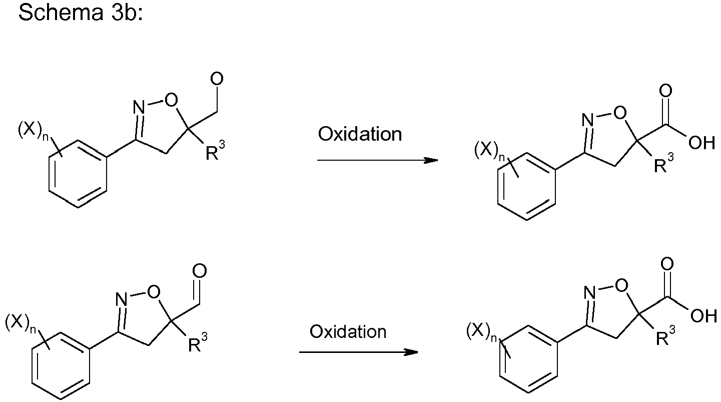 Erfindungsgemäße Verbindungen, in denen W* für COOY steht, können beispielsweise nach Schema 4 durch Umsetzung von erfindungsgemäßen Verbindungen, in denen W* für COOH steht, unter Zusatz von Mineralsäuren und einem entsprechenden Alkohol bzw. durch Umsetzung des jeweiligen Säurehalogenids mit dem jeweiligen Alkohol oder durch Kupplung der jeweiligen Säure mit Kupplungsreagentien hergestellt werden.

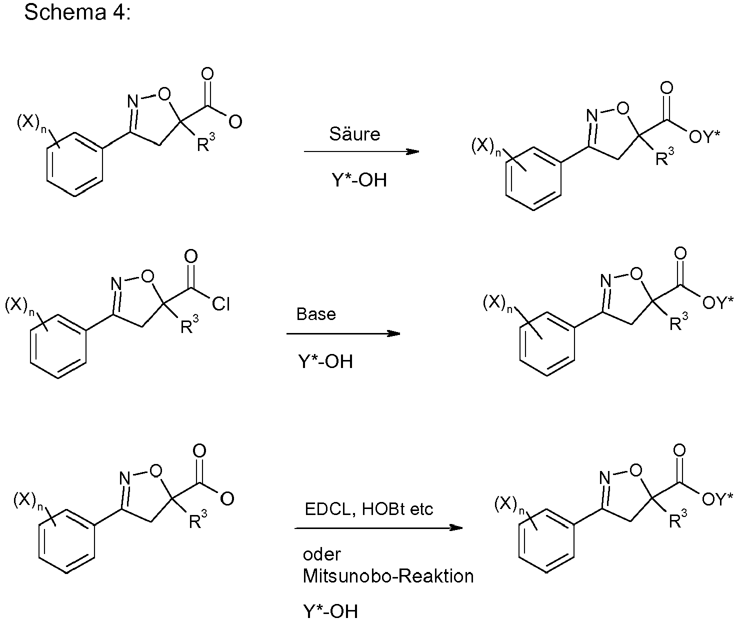

Erfindungsgemäße Verbindungen, in denen W* für CN steht, können beispielsweise nach Schema 5 durch Cycloaddition oder mit wasserentziehenden Mitteln aus einem Carbonsäureamid oder einem Aldoxim (The Chemistry of the Cyano Group", pp92-96, pp. 1345-1390, Interscience, New York, 1970) hergestellt werden.

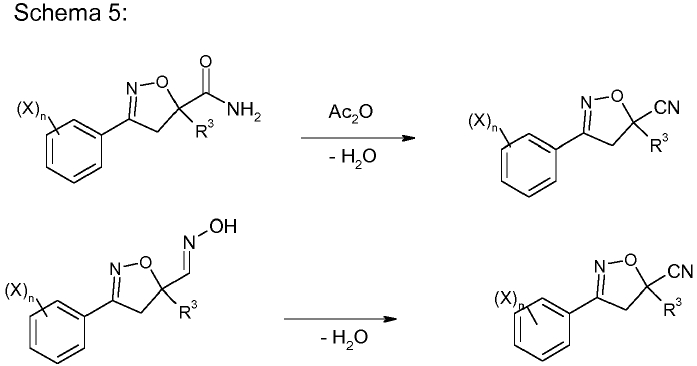

Erfindungsgemäße Verbindungen, in denen W* für CHO steht, können beispielsweise nach Schema 6 durch selektive Reduktion von Carbonsäuren, Carbonsäureestern, Carbonsäurehalogenide bzw. von Weinreb-Amiden bzw. durch Oxidation von Alkoholen oder Verseifung von Bisacetaten hergestellt werden.

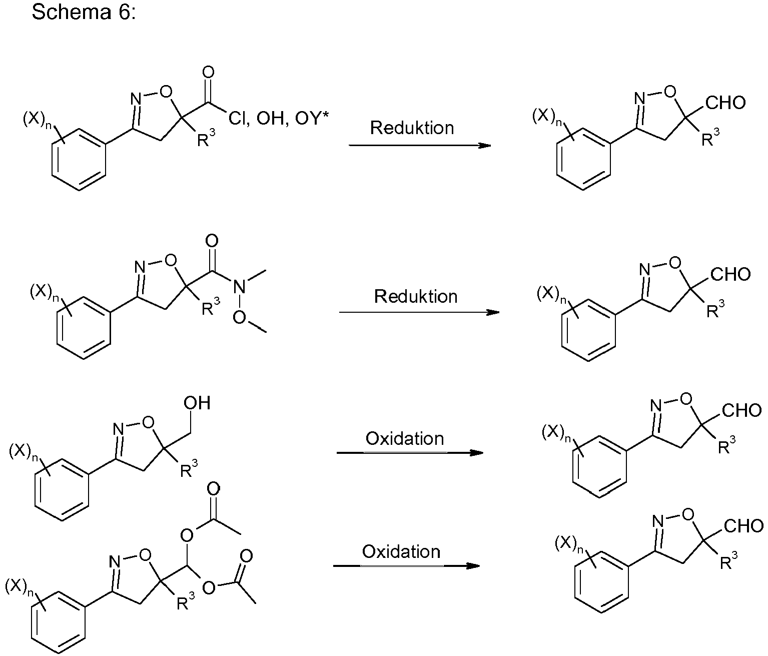

Eine allgemeine Synthesemöglichkeit zur Herstellung der erfindungsgemäßen Verbindungen 1 ist die in Schema 7 beschriebene Cycloaddition mit Dihalogenformoximen (Dichlorformoxim: Wade, Peter A.; Singh, Shankar M.; Pillay, M. Krishna, Tetrahedron (1984), 40(3), 601-11 und Dibromformoxim: DE 3612278) und nachträglicher Einführung des Phenylkerns mittels Suzuki-Kupplung (EP 0 792 870 A1) oder Stille-Reaktion (WO 2004/029066 A1).

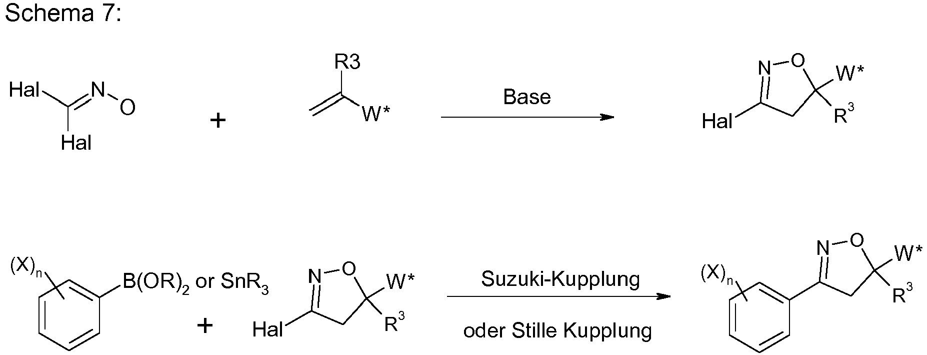

Umwandlungen der funktionellen Gruppe R³ sind sowohl auf der Stufe der Alkene als auch auf der Stufe der Isoxazoline möglich. In Schema 8 sind Herstellmethoden zu unterschiedlichen erfindungsgemäßen 3-Phenylisoxazolinderivaten beschrieben. 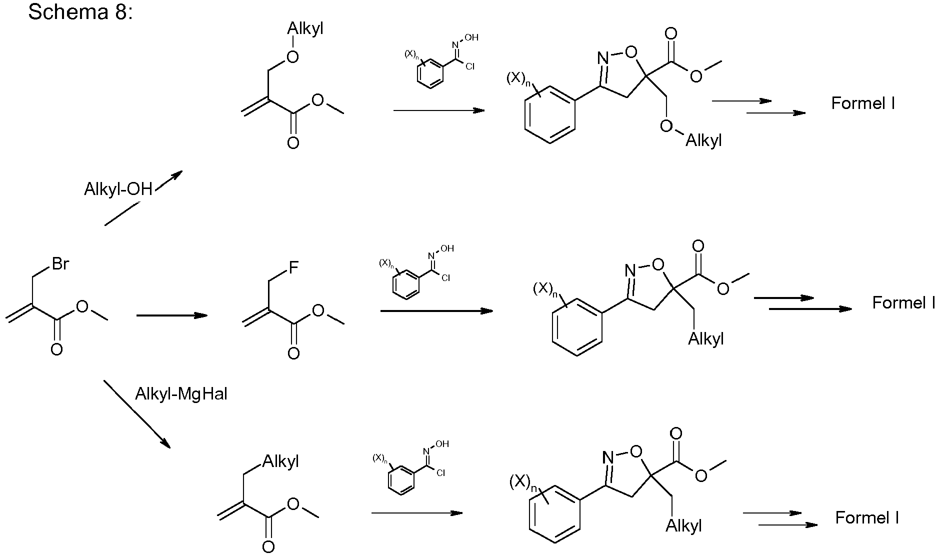 Käuflicher 2-Brommethylacrylsäuremethylester kann unter basischen Bedingungen mit Alkoholen zu 2-Alkoyxmethylacrylsäuremethylestern umgesetzt werden, die dann mit Chloroximen über die Nitriloxide zu 3-Phenyl-5-methoxymethyl-isoxazolinen reagieren Verschiedene 2-Alkylacrylsäureester können ausgehend von 2-Brom-methylacrylsäuremethylester beispielsweise durch Umsetzung mit metall-organischen Reagentien hergestellt werden. Solche Methoden sind beispielsweise beschrieben in Metzger, Albrecht; Piller, Fabian M.; Knochel, Paul; Chemical Communications, 2008, 44, p. 5824 - 5826 und auch aus WO2006/33551 bekannt.

Chem. Ber., 1963, 96, 1373; Tanoury, G.J.; Chen, M.; Dong, Y.; Forslund, R. E.; Magdziak, D.; Organic Letters, 2008, 10, 185.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) und deren Salzen beziehungsweise von bei der Herstellung anfallenden Zwischenprodukten stehen unter anderem Chromatographie-apparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automations-module beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen. Darüber hinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A),
transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und der Verbindungen der Formel (la) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl-oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispiels-weise Mefenpyr-diethyl, Cyprosulfamid, Isoxadifen-ethyl, Cloquintocet-mexyl, Benoxacor und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B.

Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-ÖI-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die nachstehenden Beispiele erläutern die Erfindung näher.

### A. Chemische Beispiele

### 1. Herstellung von Ethyl-3-(2,3-difluorphenyl)-5-methyl-4,5-dihydro-1,2-oxazol-5-carboxylat (Beispiel-Nr. 2.1.056)

Hierzu werden 3.320 g (21.13 mmol) 2,3-Difluorbenzaldehydoxim in 25 ml Dimethylformamid vorgelegt, 2.822 g (21.13 mmol) N-Chlorsuccinimid dazugegeben, wobei die Temperatur auf 79 °C steigt. Anschließend wird 1 h auf 40 °C erhitzt, eine Spatelspitze N-Chlorsuccinimid nachdosiert, nach 15 Minuten auf Raumtemperatur (RT) abgekühlt, dann 3.618 g (31.70 mmol) Methacrylsäure-ethylester und 4.097 g (31.70 mmol) N,N-Diisopropylethylamin tropfenweise zugegeben. Nach Abkühlen auf RT wird noch 15 Minuten nachgerührt, der Kolbeninhalt wird auf 50 ml Wasser gegeben, mit 1 M Schwefelsäure angesäuert und mehrmals mit Methyl-t-butylether extrahiert. Die vereinten organischen Phasen werden einmal mit Wasser und mit Kochsalzlösung gewaschen, über Natriumsulfat getrocknet, abfiltriert und das Filtrat im Vakuum eingeengt. Man erhält 6.06 g gelbes Öles, welches auf Kieselgel mit Hilfe eines Essigester/Heptan-Gradienten chromatographiert wurde. Man erhält 5.140 g (90 %) farbloses Öles.
¹H-NMR [CDCl₃]: δ = 1.31 (t, 3H); 1.72 (s, 3H); 3.33 (d, 1H); 3.95 (d, 1H); 4.36 (q, 2H); 7.12 (m, 1H); 7.23 (m, 1H); 7.53 (m, 1H).

### 2. Herstellung von Isopropyl-5-cyan-3-(3,5-difluorphenyl)-4,5-dihydro-1,2-oxazol-5-carboxylat (Beispiel-Nr. 2.2.475)

1.00 g (6.37 mmol) 3,5-Difluorbenzaldehydoxim werden in 25 ml Dichlormethan gelöst, auf 0 °C abgekühlt und mit 1.195 g (9.55 mmol) Ethyl-2-cyanacrylat versetzt. Dann wird mit 2.050 g (6.37 mmol) lodosobenzol versetzt und auf RT erwärmt. Nach Ende der Reaktion wird die Lösung mit Thiosulat-Lösung versetzt, mit Ethylacetat extrahiert und über Natriumsulfat getrocknet. Man erhält nach chromatographischer Aufreinigung über Kieselgel 1.010 g (54 %) des gewünschten Produktes.
¹H-NMR [DMSO]: δ = 1.30 (t, 3H); 4.11 (d, 1H); 4.32 (q, 2H); 4.48 (d, 1H); 7.48 (m, 3H).

### 3. Herstellung von Ethyl-3-(3,5-difluorphenyl)-5-ethyl-4,5-dihydro-1,2-oxazol-5-carboxylat (Beispiel-Nr. 2.1.471)

Intermediat: 3,5-Difluor-N-hydroxybenzolcarboximidoylchlorid
19.000 g (259.94 mmol) 3,5-Difluorbenzaldehydoxim werden in 20 ml trockenen DMF unter Stickstoff vorgelegt und zunächst mit 510 mg (3.81 mmol) N-Chlorsuccinimid versetzt. Anschließend wird in die Reaktionslösung solange gasförmige Salzsäure eingegeben, bis die Reaktionstemperatur auf 35 °C gestiegen ist. Dann werden portionsweise die restlichen 1.990 g (15.28 mmol) N-Chlorsuccinimid zudosiert, so dass die Innentemperatur von 40 °C nicht überschritten wird. Nach Abkühlen auf RT wird auf Eiswasser gegeben, mit Diethylether verdünnt und die organische Phase wird zweimal mit Eiswasser extrahiert. Nach Trocknen über Natriumsulfat wird das Lösungsmittel im Vakuum entfernt. Man erhält 3.177 g (87 %) farblosen Feststoff.

2.000 g (10.44 mmol) 3,5-Difluor-N-hydroxybenzolcarboximidoylchlorid werden in 117 g Isopropanol gelöst. Dann werden 1.606 g (9.39 mmol) 75 %iges Ethyl-2-methylenbutanoat dazugegeben, mit 4.385 g (52.20 mmol) Natriumhydrogencarbonat versetzt und bei RT gerührt. Nach 12 h wird vom Ungelösten abfiltriert, die Mutterlauge eingedampft und auf Kieselgel mit Ethylacetat/Heptan getrennt. Man erhält 1.900 g (61%) des gewünschten Produktes.
¹H-NMR [CDCl₃]: δ = 1.00 (t, 3H); 1.32 (t, 3H); 2.06 (q, 2H); 3.18 (d, 1H); 3.88 (d, 1H); 4.27 (M, 2H); 6.85 (m, 1H), 7.18 (m, 2H).

### 4. Herstellung von 3-[3-Chlor-5-(trifluormethoxy)phenyl]-5-methyl-4,5-dihydro-1,2-oxazol-5-carbonsäure (Beispiel-Nr. 1.1.554)

Es werden 3.878 g (11.03 mmol) Ethyl-3-[3-chlor-5-(trifluormethoxy)phenyl]-5-methyl-4,5-dihydro-1,2-oxazol-5-carboxylat in 50 ml THF vorgelegt und mit 33.1 ml (33.079 mmol) 1 m Natronlauge 16 h bei RT lang gerührt. Anschließend wird mit verdünnter Schwefelsäure angesäuert, mit Kochsalzlösung versetzt und mehrmals mit Ethylacetat extrahiert. Man erhält 2.323 g (65 %) obiger Verbindung nach chromatographischer Reinigung an Kieselgel mit Ethylacetat/Heptan-Gemischen.
¹H-NMR [DMSO]: δ = 1.58 (s, 3H): 3.3 (OH); 3.32 (d, 1H); 3.84 (d; 1H); 7.61 (m, 1H); 7.70 (s, 1H); 7.78 (m, 1H).

### 5. Herstellung von 3-Methoxy-3-oxopropyl-3-(3,5-difluorphenyl)-5-ethyl-4,5-dihydro-1,2-oxazol-5-carboxylat (Beispiel Beispiel-Nr. 2.6.486)

Intermediat: 3-(3,5-Difluorphenyl)-5-ethyl-4,5-dihydro-1,2-oxazol-5-carbonylchlorid Man löst 2.000 g (7.84 mmol) 3-(3,5-Difluorphenyl)-5-ethyl-4,5-dihydro-1,2-oxazol-5-carbonsäure in 50 ml Dichlormethan und gibt 29,0 mg (73.10 mmol) Dimethylformamid dazu. Nun wird die Reaktionslösung mit 1.492 g (11.76 mmol) Oxalylchlorid versetzt und bei RT gerührt. Nach 1 h wird die Reaktionsmischung einrotiert, noch zweimal mit Toluol versetzt und einrotiert. Man erhält 2.30 g (97 %) obiges Carbonsäurechlorid, welches ohne weitere Reinigung umgesetzt wird.

Es werden 200,0 mg (0.73 mmol) 3-(3,5-Difluorphenyl)-5-ethyl-4,5-dihydro-1,2-oxazol-5-carbonylchlorid in 5 ml Dichlormethan vorgelegt, dann 221,86 mg (2.19 mmol) Triethylamin dazugegeben und schließlich 91,3 mg (0.88 mmol) Methyl-3-hydroxypropanoat in 5 ml Dichlormethan dazugegeben. Nach 1 h wird die Reaktionslösung einrotiert und an Kieselgel mit einem Heptan/Essigester-Gemisch getrennt. Man erhält 134 mg (51 %) obiger Verbindung als farbloses Öl.
¹H-NMR [CDCl₃]: δ = 0.99 (t, 3H); 2.01-2.07 (m, 2H); 2.72 (t,2H), 3.17 (d, 1H); 3.68 (s, 3H); 3.75 (d,1H); 4.41-4.53 (m, 2H); 6.86 (t, 1H); 7.18 (d, 2H).

### 6. Herstellung von 3-(3,5-Dichlorphenyl)-5-methyl-4,5-dihydro-1,2-oxazol-5-carbonitril (Beispiel Beispiel-Nr. 3.417)

Es werden 0.700g (3.68 mmol) 3,5-Dichlorbenzaldehydoxim in 50 ml DMF vorgelegt, dann gibt man 0.516 g (3.87 mmol) N-Chlorsuccinimid dazu und läßt 4 h bei RT rühren. Anschließend gibt man 0.371 g (5.53 mmol) Methacrylsäurenitril sowie 0.559 g (5.53 mmol) Triethylamin dazu und läßt noch 18 h bei RT rühren. Der Ansatz wird im Vakuum einrotiert und auf der präparativen HPLC (RP-Phase) getrennt. Man erhält 110 mg (12 %) des gewünschten Produktes als farbloses Öl.
¹H-NMR [CDCl₃]: δ = [CDCl₃] 1.91 (s, 3H); 3.38 (d, 1H); 3.80 (d, 1H); 7.45 (s, 1H); 7.52 (s, 1H).

### 7. Herstellung von Methyl-4-brom-3-(3,5-difluorphenyl)-5-methyl-4,5-dihydro-1,2-oxazol-5-carboxylat (Beispiel-Nr. 2.1.470)

1,000 g (5.22 mmol) 3,5-Difluor-N-hydroxybenzolcarboximidoylchlorid werden in 20 ml Isopropanol gelöst, anschließend gibt man 1121 mg (6.26 mmol) Methyl-3-brom-2-methylacrylat dazu und 1754,1 mg (20.88 mmol) festes Natriumhydrogencarbonat. Man läßt 2 d bei RT rühren und reinigt das erhaltene Rohgemisch über Kieselgel mit Ethylacetat/Heptan. Man erhält 540 mg (31 %) eines gelbfarbenen Öls.
¹H-NMR [CDCl₃]: δ = D1= 1.58 (s, 3H); 3.82 (s, 3H); 5.50 (s, 1H); D2 = 1.85 (s, 3H); 3.72 (s, 3H); 5.78 (s, 1H); 6.8-7.5 (m, 3H).

### 8. Herstellung von Methyl-3-(3,5-difluorphenyl)-5-fluor-4,5-dihydro-1,2-oxazol-5-carboxylat (Beispiel-Nr. 2.2.468)

Es werden 1.000 g (5.22 mmol) 3,5-Difluor-N-hydroxybenzolcarboximidoylchlorid in 23 ml Isopropanol gelöst, dann 1.086,6 mg (10.44 mmol) Methyl-2-fluoracrylat dazugegeben und danach mit 2.192 g (26.10 mmol) Natriumhydrogencarbonat versetzt. Man läßt 1 h bei 40 °C rühren, nimmt in Wasser auf, extrahiert mit Ethylacetat und erhält 1.230 g (91 %) obiger Verbindung als Feststoff.
¹H-NMR [CDCl₃]: δ = 3.65 (dd,1H); 3.96 (s,3H); 4.12 (dd,1H); 6.96 (t,1H); 7.22 (d,2H).

### 9. Herstellung von 5-(Fluormethyl)-3-(3-fluorphenyl)-4,5-dihydro-1,2-oxazol-5-carbonsäure (Beispiel-Nr. 1.2.001)

Es werden 3.00 g (11.14 mmol) Ethyl-5-(fluormethyl)-3-(3-fluorphenyl)-4,5-dihydro-1,2-oxazol-5-carboxylat in 150 ml THF gelöst und mit 0,543 g (22.28 mmol) Lithiumhyroxid bei RT gelöst.nach 20 h wird im Vakuum einrotiert, der Rückstand mit Dichlormethan aufgenommen und mit gesättigter Natriumhydrogencarbonatlösung extrahiert. Die wäßrige Phase wird anschließend auf pH1-2 gestellt und mit Essigester extrahiert. Man erhält 2.530 g (89 %) der Carbonsäure.
¹H-NMR [CDCl₃]: δ = 3.56 (d,1H); 3.80 (d,1H); 4.71 (d,1H); 4.83 (d,1H); 7.18 (m,1H); 7.41 (m,3H).

### 10. Herstellung von Methyl-3-(3-chlor-4-fluorphenyl)-5-(trifluormethyl)-4,5-dihydro-1,2-oxazol-5-carboxylat (Beispiel-Nr. 2.2.1208)

1.500 g (8.64 mmol) 3-Chlor-4-fluorbenzaldehydoxim werden in 47.5 g DMF vorgelegt und mit 1.212 g (9.07 mmol) N-Chlorsuccinimid, 1.312 g (12.96 mmol) und 2.179 g (12.96 mmol) Methyl-2-(trifluormethyl)acrylat cyclisiert. Nach Aufreinigung auf Kieselgel mit Ethylacetat/Heptan als Solvensgemisch erhält man 1.300 g (43 %) des obigen Produktes.
¹H-NMR [CDCl₃]: δ = 1.52 8s, 3H); 3.74 (d, 1H); 3.94 (s, 3H); 4.03 (d, 1H); 7.22 (m, 1H); 7.58 (m, 1H); 7.72 (m, 1H).

### 11. Herstellung von 3-(3-Chlor-4-fluorphenyl)-5-(trifluormethyl)-4,5-dihydro-1,2-oxazol-5-carbonsäure (Beispiel-Nr. 1.2.1205)

2.000 g (6.14 mmol) des Esters Methyl-3-(3-chlor-4-fluorphenyl)-5-(trifluormethyl)-4,5-dihydro-1,2-oxazol-5-carboxylat werden in 20 ml THF gelöst, mit 0.295 g (7.37 mmol) Natriumhydroxid in Wasser versetzt und bei RT gerührt. Nach 18 h wird das Solvens abrotiert, mit Wasser versetzt, einmal mit Dichlormethan gewaschen und die Wasserphase mit verdünnter Salzsäure angesäuert. Diese wird noch zweimal mit Dichlormethan extrahiert. Man trocknet über Magnesiumsulfat und erhält 1.740 g (85 %) der Carbonsäure.
¹H-NMR [CDCl₃]: δ = 3.81 (d, 1H); 4.00 (d, 1H); 7.22 (m, 1H); 7.58 (m, 1H); 7.74 (m, 1H).

### 12. Herstellung von Methyl-3-(3,5-dichlorphenyl)-5-(methoxymethyl)-4,5-dihydro-1,2-oxazol-5-carboxylat (Beispiel-Nr. 2.5.544)

1.156 g (6.08 mmol) 3,5-Dichlorbenzaldehydoxim werden mit 0.950 g (7.30 mmol) Methyl-2-(methoxymethyl)acrylat, 0.853 g (6.39 mmol) N-Chlorsuccinimid und 0.923 g (9.13 mmol) Triethylamin in 47.5 g Dimethylformamid cyclisiert. Nach chromatographischer Reinigung auf Kieselgel erhält man 0.150 g (7.34 %) obiger Verbindung als gelbliches Öl.
¹H-NMR [CDCl₃]: δ = 3.43 (s, 3H); 3.50 (d, 1H); 3.72 (d, 1H); 3.80 (mc, 2H); 3.82 (s, 3H);7.41 (m, 1H); 7.55 (s, 2H).

### 13. Herstellung von 3-(3,5-Dichlorphenyl)-5-(methoxymethyl)-4,5-dihydro-1,2-oxazol-5-carbonsäure (Beispiel-Nr. 1.5.544)

100,0 mg (0.31 mmol) Methyl-3-(3,5-dichlorphenyl)-5-(methoxymethyl)-4,5-dihydro-1,2-oxazol-5-carboxylat werden in 5 ml THF gelöst, mit 40,0 mg (0.47 mmol) Natriumhydroxid in 2 ml Wasser 18 h bei RT gerührt. Nach Entfernen des Lösungsmittels wird mit Wasser verdünnt, die Wasserphase mit Dichlormethan gewaschen, anschließend die Wasserphase mit verdünnter Salzsäure angesäuert und nochmals mit Dichlormethan gewaschen. Die letzte Dichlormeethanphase wird getrocknet. Dabei erhält man 100 mg (100 %) obiger Säure als farbloses Öl.
¹H-NMR [CDCl₃]: δ = 3.48 (s, 3H); 3.50 (d, 1H); 3.72 8d, 1H); 3.85 (q, 2H); 7.42 (m, 1H); 7.55 (m, 2H).

### 14. Herstellung von Methyl-3-(3-fluor-5-methylphenyl)-5-(1-hydroxyethyl)-4,5-dihydro-1,2-oxazol-5-carboxylat (Beispiel-Nr. 2.5.498)

10.0 g (53.31 mmol) 3-Fluor-N-hydroxy-5-methylbenzolcarboximidoylchlorid werden mit 7.631 g (58.64 mmol) Methyl-3-hydroxy-2-methylenbutanoat und 22.390 g (22.54 mmol) Natriumhydrogencarbonat gemäß Methode C über Nacht zur Reaktion gebracht. Nach Filtration der Reaktionslösung wurde an Kieselgel chromatographiert. Man erhält 15.00 g (95 %) obiger Verbindung als Öl.
¹H-NMR [CDCl₃]: δ = D1 1.20 (d,3H); 2.37 (d,1H); 2.38 (s,3H); 3.54 (d,1H), 3.69 (d,1H); 3.83 (s,3H); 4.33 (m,1H); 6.96 (s,1H); 7.23 (s,1H); 7.25 (s,1H). D2 1.26 (d,3H); 2.18 (d,1H); 2.38 (s,3H); 3.60 (d,1H); 3.72 (d,1H); 3.83 (s,3H); 4.22 (m,1H); 6.93 (s,1H); 7.21 (s,1H); 7.25 (s,1H)

### 15. Herstellung von 3-(3,5-Difluorphenyl)-5-(1-hydroxyethyl)-4,5-dihydro-1,2-oxazol-5-carbonsäure (Beispiel-Nr. 1.5.479)

Es werden 1.5 g (5.26 mmol) Methyl-3-(3,5-difluorphenyl)-5-(1-hydroxyethyl)-4,5-dihydro-1,2-oxazol-5-carboxylat in 75 ml THF mit 0.151 g (6.31 mmol) LiOH bei RT verseift. Nach 1 h wird das Solvens im Vakuum entfernt, mit Salzsäure auf pH1-2 gebracht und mit Ethylacetat extrahiert. Man erhält 1.50 g (99 %) obiger Säure.
¹H-NMR [CDCl₃]: δ = D1 1.25 (d,3H); 1.36 (d,1H); 3.60 (d,1H); 3.70 (d,1H); 4.29 (m,1H); 6.91 (t,1H); 7.20 (d,2H). D2 1.29 (d,3H); 1.42 (d,1H); 3.60 (d,1H); 3.69 (d,1H); 4.22 (m,1H); 6.91 (t,1H); 7.20 (d,2H).

### 16. Herstellung von Methyl-5-acetyl-3-(3,5-difluorphenyl)-4,5-dihydro-1,2-oxazol-5-carboxylat (Beispiel-Nr. 2.4.462)

Hierzu werden 2.000 g (7.01 mmol) Methyl-3-(3,5-difluorphenyl)-5-(1-hydroxyethyl)-4,5-dihydro-1,2-oxazol-5-carboxylat mit 6.048 g (28.05 mmol) Pyridiniumchlorochromat sowie Molekularsieb in 75 ml Dichlormethan 5 h bei RT gerührt. Die Reaktionslösung wurde mit Diethylether über eine SPE-Kartusche filtriert und einrotiert. Man erhält 2,000 g (96 %) obiger Verbindung als Feststoff.
¹H-NMR [CDCl₃]: δ = 2.44 (s,3H); 3.80 (d,1H); 3.86 (s,3H); 3.89 (d,1H); 6.90 (m,1H); 7.19 (d,2H).

### 17. Herstellung von Methyl-3-(3-chlor-5-fluorphenyl)-5-vinyl-4,5-dihydro-1,2-oxazol-5-carboxylat (Beispiel-Nr. 2.3.572)

4.400 g (15.76 mmol) Methyl-3-(3-brom-5-methylphenyl)-5-(1-{[(trifluormethyl) sulfonyl]oxy}ethyl)-4,5-dihydro-1,2-oxazol-5-carboxylat , hergestellt aus dem entsprechenden Alkohol , Trifluormethansulfonsäureanhydrid und Pyridin (Ausbeute 62%), zusammen mit 600.29 mg (3.94 mmol) DBU umgesetzt. Nach 2 Tagen wird die Reaktionsmischung mit Wasser verdünnt, mit verdünnter Salzsäure angesäuert bis zum pH 4-5 und schließlich mit Dichlormethan extrahiert. Anschließend wird auf Kieselgel mit Ethylacetat/Heptan chromatographiert. Man erhält 450 mg (57 %) obiger Verbindung als farbloses Öl.
¹H-NMR [CDCl₃]: δ = 2.35 (s,3H), 3.35 (d,1H); 3.83 (s,3H); 3.93 (d,1H); 5.35 (d,1H), 5.55 (d,1H); 6.13 (dd,1H); 7.38 (s,1H), 7.42 (s,1H); 7.58 (s,1H).

### 18. Herstellung von Methyl-5-(1-chlorvinyl)-3-(3,5-difluorphenyl)-4,5-dihydro-1,2-oxazol-5-carboxylat (Beispiel-Nr. 2.3.473)

730 mg (2.58 mmol) Methyl-5-acetyl-3-(3,5-difluorphenyl)-4,5-dihydro-1,2-oxazol-5-carboxylat werden in 20 ml Dichlormethan gelöst, mit einem Tropfen DMF versetzt und mit 590 mg (2.84 mmol) Phosphorpentachlorid 6 h bei 100 °C in der Mikrowelle erhitzt. Anschließend wird die Reaktionsmischung auf ein Gemisch aus warmen Wasser (ca. 35°C) und Dichlormethan gegeben 1 h bei der Temperatur nachgerührt. Die Wasserphase (pH 2) wurde abgetrennt. Die Dichlormethanphase wurde mit gesättigter Natriumcarbonatlösung nachgewaschen und über Natriumsulfat getrocknet. Anschließend wurde über Kieselgel chromatographiert (Ethylacetat/Heptan). Man erhält 230 mg (25 %) obiger Verbindung als Feststoff
¹H-NMR [CDCl₃]: δ = 3.53 (d,1H); 3.88 (s,3H); 4.22 (d,1H); 5.55 (s,1H); 5.93 (s,1H); 6.90 (m,1H); 7.20 (d,2H).

In Analogie zu der Herstellung der oben genannten Verbindungen und gemäß den allgemeinen Angaben zur Herstellung sind die in folgenden Tabellen genannten Verbindungen erhältlich. Die NMR-Daten der in diesen Tabellen offenbarten Beispiele werden entweder in klassischer Form (δ-Werte, Anzahl der H-Atome, Multiplettaufspaltung) oder als sogenannte NMR-Peak-Listen aufgeführt. Beim NMR-Peak-Listenverfahren werden die NMR-Daten ausgewählter Beispiele in Form von NMR-Peaklisten notiert, wobei zu jedem Signalpeak erst der δ-Wert in ppm und dann die Signalintensität durch ein Leerzeichen getrennt aufgeführt wird. Die δ-Wert - Signalintensitäts - Zahlenpaare von verschiedenen Signalpeaks werden durch Semikolons voneinander getrennt aufgelistet.

**Die verwendeten Abkürzungen bedeuten:**

| | | | | | | | |
|---|---|---|---|---|---|---|---|
| Ac | Acetoxy | Bu | Butyl | Et | Ethyl | Me | Methyl |
| Pr | Propyl | Pen | Pentyl | Hex | Hexyl | Ph | Phenyl |
| c | cyclo | s | sekundär | i | Iso | t | tertiär |
| THF | Tetrahydrofuran | | | | | | |

D1, D2 bezeichnen Diastereomere eines Diastereomerenpaares, die als Racemate zweier Enantiomere vorliegen.

**Tabelle 1.1: Erfindungsgemäße Verbindungen der allgemeinen Formel (I), worin W* für COOH, R¹ für Wasserstoff steht, und Aryl den Rest**

| | | | | |
|---|---|---|---|---|
| | 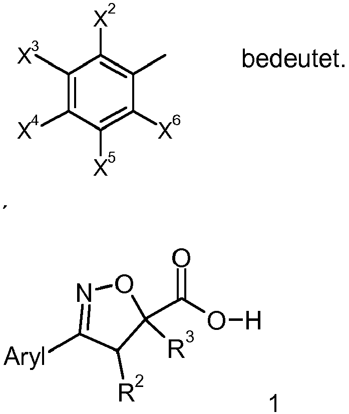 | | | |

| Nr. | Aryl | R² | R³ | Physikalische Daten |
|---|---|---|---|---|
| 1.1.001 | 3-Fluor-Phenyl | H | Methyl | [CDCl₃] 1.78 (s, 3H); 3.29 (d, 1H); 3.85 (d, 1H); 7.12 (m, 1H); 7.40 (m, 2H). |
| 1.1.002 | 3-Fluor-Phenyl | H | Ethyl | [CDCl₃] 1.08 (t, 3H); 2.14 (mc; 2H); 3.32 (d, 1H); 3.30 (d, 1H); 7.15 (m, 1H); 7.40 (m, 2H). |
| 1.1.003 | 3-Fluor-Phenyl | H | Propyl | |
| 1.1.004 | 3-Fluor-Phenyl | H | Cyclopropyl | |
| 1.1.005 | 3-Chlor-Phenyl | H | Methyl | [CDCl₃] 1.78 (s, 3H); 3.28 (d, 1H); 3.86 (d, 1H); 7.35 (m, 1H); 7.42 (m, 1H); 7.51 (m, 1H); 7.65 (m, 1H). |
| 1.1.006 | 3-Chlor-Phenyl | H | Ethyl | |
| 1.1.007 | 3-Chlor-Phenyl | H | Propyl | |
| 1.1.008 | 3-Chlor-Phenyl | H | Cyclopropyl | |
| 1.1.009 | 3-Brom-Phenyl | H | Methyl | [CDCl₃] 1.72 (s, 3H); 3.19 (d, 1H); 3.82 (s, 3H); 3.86 (d, 1H); 7,.28 (t, 1H); 7.55 (m, 1H); 7.59 (m, 1H); 7.80 (t, 1H). |
| 1.1.010 | 3-Brom-Phenyl | H | Ethyl | |
| 1.1.011 | 3-Iod-Phenyl | H | Methyl | |
| 1.1.012 | 3-Iod-Phenyl | H | Ethyl | |
| 1.1.013 | 3-Methyl-Phenyl | H | Methyl | [DMSO] 1.55 (s, 3H); 2.32 (s, 3H); 3.35 (d, 1H); 3.76 (d, 1H); 7.20-7.50 (m, 4H). |
| 1.1.014 | 3-Methyl-Phenyl | H | Ethyl | |
| 1.1.015 | 3-Ethyl-Phenyl | H | Methyl | [DMSO] 1.20 (t, 3H); 1.55 (s, 3H); 2.65 (q, 2H); 3.38 (d, 1H); 3.80 (d, 1H); 7.28-7.50 (m, 4H). |
| 1.1.016 | 3-Propyl-Phenyl | H | Methyl | |
| 1.1.017 | 3-isoPropyl-Phenyl | H | Methyl | [DMSO] 1.20 (d, 6H); 1.55 (s, 3H); 2.94 (m, 1H); 3.40 (d, 1H); 3.80 (d, 1H); 7.35 (m, 2H); 7.48 (m, 1H); 7.55 (s, 1H). |
| 1.1.018 | 3-nButyl-Phenyl | H | Methyl | |
| 1.1.019 | 3-iButyl-Phenyl | H | Methyl | |
| 1.1.020 | 3-tert.Butyl-Phenyl | H | Methyl | |
| 1.1.021 | 3-Cyclopropyl-Phenyl | H | Methyl | |
| 1.1.022 | 3-Cyclobutyl-Phenyl | H | Methyl | |
| 1.1.023 | 3-Cyclopentyl-Phenyl | H | Methyl | |
| 1.1.024 | 3-Vinyl-Phenyl | H | Methyl | |
| 1.1.025 | 3-Ethinyl-Phenyl | H | Methyl | [CDCl₃] 1.78 (s, 1H); 3.10 (s, 1H); 3.30 (d, 1H); 3.87 (d, 1H); 7.35 (m, 1H); 7.52 (m, 1H); 7.68 (m 1H); 7.75 (s, 1H). |
| 1.1.026 | 3-Cyano-Phenyl | H | Methyl | [CDCl₃] 1.80 (s, 3H); 3.29 (d, 1H); 3.89 (d, 1H); 7.55 (m, 1H); 7.72 (m, 1H); 7.90 (m, 2H). |
| 1.1.027 | 3-TrifluormethylPhenyl | H | Methyl | [CDCl₃] 1.80 (s, 3H); 3.32 (d, 1H); 3.91 (d, 1H); 7.56 (m, 1H); 7.69 (m, 1H); 7.88 (m, 2H). |
| 1.1.028 | 3-Difluormethyl-Phenyl | H | Methyl | |
| 1.1.029 | 3-(Hydroxycarbonyl)-Phenyl | H | Methyl | [DMSO] 1.58 (s, 3H); 3.42 (d, 1H);3.82 (d, 1H); 7.60 (t, 1H); 7.90 (d, 1H); 8.03 (d, 1H); 8.20 (s, 1H).13.2 (bs, 1H) |
| 1.1.030 | 3-(Methoxycarbony)l-Phenyl | H | Methyl | |
| 1.1.031 | 3-(Ethoxycarbonyl)-Phenyl | H | Methyl | |
| 1.1.032 | 3-Hydroxymethyl-Phenyl | H | Methyl | |
| 1.1.033 | 3-Carbamoyl-Phenyl | H | Methyl | [DMSO] 1.58 (s, 3H); 3.40 (d, 1H); 3.85 (d, 1H); 7.43 (bs, 1H); 7.53t, 1H); 7.85 (d, 1H); 7.95 (d, 1H); 8.08 (bs, 1H); 8.11 (s, 1H). |
| 1.1.034 | 3-Hydroxy-Phenyl- | H | Methyl | [DMSO] 1.52 (s, 3H); 3.32 (d, 1H); 3.71 (d, 1H); 6.85 (d, 1H); 7.08 (m, 2H); 7.24 /t, 1H); 9.65 (s, 1H); 13.2 (bs, 1H). |
| 1.1.035 | 3-Methoxy-Phenyl | H | Methyl | [CDCl₃] 1.75 (s, 3H); 3.30 (d, 1H); 3.85 (s, 3H); 3.89 (d, 1H); 7.00 (m, 1H); 7.16 (m, 1H); 7.25 (m, 1H); 7.35 (m, 1H). |
| 1.1.036 | 3-Ethoxy-Phenyl | H | Methyl | [CDCl₃] 1.41 (t, 1H), 1.75 (s, 1H); 3.25 (d, 1H); 3.83 (d, 1H); 4.05 (q, 2H); 6.95 (m, 1H); 7.15 (m, 1H); 7.20-7.32 (m, 2H). |
| 1.1.037 | 3-Propyloxy-Phenyl | H | Methyl | |
| 1.1.038 | 3-isoPropyloxy-Phenyl | H | Methyl | |
| 1.1.039 | 3-nButyloxy-Phenyl | H | Methyl | |
| 1.1.040 | 3-iButyloxy-Phenyl | H | Methyl | |
| 1.1.041 | 3-tButyloxy-Phenyl | H | Methyl | |
| 1.1.042 | 3-Difluormethoxy-Phenyl | H | Methyl | [CDCl₃] 1.78 (s, 3H); 3.38 (d, 1H); 3.86 (d, 1H); 6.52 (t, 1H); 7.21 (m, 1H); 7.43 (m, 2H). |
| 1.1.043 | 3-Trifluormethoxy-Phenyl | H | Methyl | [CDCl₃] 1.80 (s, 3H); 3.30 (d, 1H); 3.88 (d, 1H); 7.25 (m, 1H); 7.45 (m, 1H); 7.55 (m, 2H). |
| 1.1.044 | 3-(2,2,2-Trifluorethoxy)-Phenyl | H | Methyl | |
| 1.1.045 | 3-(2-Chlorethoxy)-Phenyl | H | Methyl | |
| 1.1.046 | 3-(2-Hydroxyethoxy)-Phenyl- | H | Methyl | |
| 1.1.047 | 3-(2-Methoxyethoxy)-Phenyl- | H | Methyl | [CDCl₃] 1.75 (s, 3H); 3.35 (d, 1H); 3.43 (s, 1H); 3.75 (dd, 2H); 3.85 (d, 1H); 4.14 (dd, 2H); 7.04 (d, 1H); 7.15 (d, 1H); 7.25-7.40 (m, 2H). |
| 1.1.048 | 3-[(tert-Butoxycarbonyl)oxy]-Phenyl | H | Methyl | |
| 1.1.049 | 3-Nitro-Phenyl | H | Methyl | [DMSO] 1.52 (s, 3H); 3.40 (d, 1H); 3.85 (d, 1H); 7.75 (t, 1H); 8.08 (d, 1H); 8.29 (d, 1H); 8.4 (s, 1H). |
| 1.1.050 | 3-Acetoxy-Phenyl | H | Methyl | |
| 1.1.051 | {3-[(tert-Butoxycarbonyl)amino]-Phenyl}- | H | Methyl | [CDCl₃] 1.50 (s, 9H); 1.75 (s, 3H); 3.30 (d, 1H); 3.90 (d, 1H); 6.70 (bs, 1H); 7.30 (s, 2H), 7.40 (s, 1H), 7.70 (s, 1H). |
| 1.1.052 | 3-Methylsulfanyl-Phenyl | H | Methyl | |
| 1.1.053 | 3-Ethylsulfanyl-Phenyl | H | Methyl | |
| 1.1.054 | 3-(Pentafluor-lambda⁶-sulfanyl)-Phenyl | H | Methyl | [CDCl₃] 1.80 (s, 3H); 3.30 (d, 1H); 3.90 (d, 1H); 7.51 (t, 1H); 7.80 (2d, 2H); 8.00 (s, 1H). |
| 1.1.055 | 2,3-Difluor-Phenyl | H | Methyl | [CDCl₃] 1.78 (s, 3H); 3.40 (dd, 1H); 3.95 (dd, 1H); 7.13 (m, 1H); 7.22 (m, 2H); 7.61 (t, 1H). |
| 1.1.056 | 2,3-Difluor-Phenyl | H | Ethyl | |
| 1.1.057 | 2,3-Difluor-Phenyl | H | Propyl | |
| 1.1.058 | 2,3-Difluor-Phenyl | H | Cyclopropyl | |
| 1.1.059 | 2-Chlor-3-fluorPhenyl | H | Methyl | |
| 1.1.060 | 2-Brom-3-fluorPhenyl | H | Methyl | |
| 1.1.061 | 2-Methyl-3-fluorPhenyl | H | Methyl | |
| 1.1.062 | 2-Ethyl-3-fluorPhenyl | H | Methyl | |
| 1.1.063 | 2-Cyclopropyl-3-fluor-Phenyl | H | Methyl | |
| 1.1.064 | 2-Vinyl-3-fluorPhenyl | H | Methyl | |
| 1.1.065 | 2-Ethinyl-3-fluorPhenyl | H | Methyl | |
| 1.1.066 | 2-Cyano-3-fluorPhenyl | H | Methyl | |
| 1.1.067 | 2-Methoxy-3-fluorPhenyl | H | Methyl | |
| 1.1.068 | 2-Ethoxy-3-fluorPhenyl | H | Methyl | |
| 1.1.069 | 2-Trifluormethoxy-3-fluor-Phenyl | H | Methyl | |
| 1.1.070 | 2-Nitro-3-fluorPhenyl | H | Methyl | |
| 1.1.071 | 2-Fluor-3-chlorPhenyl | H | Methyl | |
| 1.1.072 | 2,3-Dichlor-Phenyl | H | Methyl | [CDCl₃] 1.80 (s, 3H); 3.50 (d, 1H); 3.95 (d, 1H); 7.25 (m, 1H); 7.26 (m, 1H); 7.50 (m, 1H); 7.58 (m, 1H). |
| 1.1.073 | 2,3-Dichlor-Phenyl | H | Ethyl | |
| 1.1.074 | 2,3-Dichlor-Phenyl | H | Propyl | |
| 1.1.075 | 2,3-Dichlor-Phenyl | H | Cyclopropyl | |
| 1.1.076 | 2-Brom-3-chlorPhenyl | H | Methyl | |
| 1.1.077 | 2-Methyl-3-chlorPhenyl | H | Methyl | |
| 1.1.078 | 2-Ethyl-3-chlorPhenyl | H | Methyl | |
| 1.1.079 | 2-Cyclopropyl-3-chlor-Phenyl | H | Methyl | |
| 1.1.080 | 2-Vinyl-3-chlorPhenyl | H | Methyl | |
| 1.1.081 | 2-Ethinyl-3-chlorPhenyl | H | Methyl | |
| 1.1.082 | 2-Cyano-3-chlorPhenyl | H | Methyl | |
| 1.1.083 | 2-Trifluormethyl-2-chlor-Phenyl | H | Methyl | |
| 1.1.084 | 2-Methoxy-3-chlorPhenyl | H | Methyl | |
| 1.1.085 | 2-Ethoxy-3-chlorPhenyl | H | Methyl | |
| 1.1.086 | 2-Trifluormethoxy-3-chlor-Phenyl | H | Methyl | |
| 1.1.087 | 2-Nitro-3-chlorPhenyl | H | Methyl | |
| 1.1.088 | 2-Fluor-3-bromPhenyl | H | Methyl | |
| 1.1.089 | 2-Chlor-3-bromPhenyl | H | Methyl | |
| 1.1.090 | 2,3-Dibrom-Phenyl | H | Methyl | |
| 1.1.091 | 2-Methyl-3-bromPhenyl | H | Methyl | |
| 1.1.092 | 2-Ethyl-3-bromPhenyl | H | Methyl | |
| 1.1.093 | 2-Cyclopropyl-3-brom-Phenyl | H | Methyl | |
| 1.1.094 | 2-Vinyl-3-bromPhenyl | H | Methyl | |
| 1.1.095 | 2-Ethinyl-3-bromPhenyl | H | Methyl | |
| 1.1.096 | 2-Cyano-3-bromPhenyl | H | Methyl | |
| 1.1.097 | 2-Trifluormethyl-3-brom-Phenyl | H | Methyl | |
| 1.1.098 | 2-Methoxy-3-Phenyl | H | Methyl | |
| 1.1.099 | 2-Ethoxy-3-bromPhenyl | H | Methyl | |
| 1.1.100 | 2-Trifluormethoxy-3-brom-Phenyl | H | Methyl | |
| 1.1.101 | 2-Nitro-3-bromPhenyl | H | Methyl | |
| 1.1.102 | 2-Fluor-3-iod-Phenyl | H | Methyl | |
| 1.1.103 | 2-Chlor-3-iod-Phenyl | H | Methyl | |
| 1.1.104 | 2-Brom-3-iod-Phenyl | H | Methyl | |
| 1.1.105 | 2-Methyl-3-iod-Phenyl | H | Methyl | |
| 1.1.106 | 2-Ethyl-3-iod-Phenyl | H | Methyl | |
| 1.1.107 | 2-Cyclopropyl-3-iod-Phenyl | H | Methyl | |
| 1.1.108 | 2-Vinyl-3-iod-Phenyl | H | Methyl | |
| 1.1.109 | 2-Ethinyl-3-iod-Phenyl | H | Methyl | |
| 1.1.110 | 2-Cyano-3-iod-Phenyl | H | Methyl | |
| 1.1.111 | 2-Trifluormethyl-3-iod-Phenyl | H | Methyl | |
| 1.1.112 | 2-Methoxy-3-iod-Phenyl | H | Methyl | |
| 1.1.113 | 2-Ethoxy-3-iod-Phenyl | H | Methyl | |
| 1.1.114 | 2-Trifluormethoxy-3-iod-Phenyl | H | Methyl | |
| 1.1.115 | 2-Nitro-3-iod-Phenyl | H | Methyl | |
| 1.1.116 | 2-Fluor-3-methylPhenyl | H | Methyl | [CDCl₃] 1.56 (s, 3H); 2.25 (s, 3H); 3.35 (d, 1H); 3.80 (d, 1H); 7.15 (t, 1H); 7.40 (t, 1H); 7.51 (t, 1H). |
| 1.1.117 | 2-Fluor-3-methylPhenyl | H | Ethyl | |
| 1.1.118 | 2-Fluor-3-methylPhenyl | H | Propyl | |
| 1.1.119 | 2-Fluor-3-methylPhenyl | H | Cyclopropyl | |
| 1.1.120 | 2-Chlor-3-methylPhenyl | H | Methyl | |
| 1.1.121 | 2-Chlor-3-methylPhenyl | H | Ethyl | |
| 1.1.122 | 2-Chlor-3-methylPhenyl | H | Propyl | |
| 1.1.123 | 2-Chlor-3-methylPhenyl | H | Cyclopropyl | |
| 1.1.124 | 2-Brom-3-methylPhenyl | H | Methyl | |
| 1.1.125 | 2,3-Dimethyl-Phenyl | H | Methyl | |
| 1.1.126 | 2,3-Dimethyl-Phenyl | H | Ethyl | |
| 1.1.127 | 2,3-Dimethyl-Phenyl | H | Propyl | |
| 1.1.128 | 2,3-Dimethyl-Phenyl | H | Cyclopropyl | |
| 1.1.129 | 2-Ethyl-3-methylPhenyl | H | Methyl | |
| 1.1.130 | 2-Cyclopropyl-3-methyl-Phenyl | H | Methyl | |
| 1.1.131 | 2-Vinyl-3-methylPhenyl | H | Methyl | |
| 1.1.132 | 2-Ethinyl-3-methylPhenyl | H | Methyl | |
| 1.1.133 | 2-Cyano-3-methylPhenyl | H | Methyl | |
| 1.1.134 | 2-Trifluormethyl-3-methyl-Phenyl | H | Methyl | |
| 1.1.135 | 2-Methoxy-3-methylPhenyl | H | Methyl | |
| 1.1.136 | 2-Ethoxy-3-methylPhenyl | H | Methyl | |
| 1.1.137 | 2-Trifluormethoxy-3-methyl-Phenyl | H | Methyl | |
| 1.1.138 | 2-Nitro-3-methylPhenyl | H | Methyl | |
| 1.1.139 | 2-Fluor-3-ethylPhenyl | H | Methyl | |
| 1.1.140 | 2-Chlor-3-ethylPhenyl | H | Methyl | |
| 1.1.141 | 2-Brom-3-ethylPhenyl | H | Methyl | |
| 1.1.142 | 2-Methyl-3-ethylPhenyl | H | Methyl | |
| 1.1.143 | 2,3-Diethyl-Phenyl | H | Methyl | |
| 1.1.144 | 2-Cyclopropyl-3-ethyl-Phenyl | H | Methyl | |
| 1.1.145 | 2-Vinyl-3-ethylPhenyl | H | Methyl | |
| 1.1.146 | 2-Ethinyl-3-ethylPhenyl | H | Methyl | |
| 1.1.147 | 2-Cyano-3-ethylPhenyl | H | Methyl | |
| 1.1.148 | 2-Trifluormethyl-3-ethyl-Phenyl | H | Methyl | |
| 1.1.149 | 2-Methoxy-3-ethylPhenyl | H | Methyl | |
| 1.1.150 | 2-Ethoxy-3-ethylPhenyl | H | Methyl | |
| 1.1.151 | 2-Trifluormethoxy-3-ethyl-Phenyl | H | Methyl | |
| 1.1.152 | 2-Nitro-3-ethylPhenyl | H | Methyl | |
| 1.1.153 | 2-Fluor-3-propylPhenyl | H | Methyl | |
| 1.1.154 | 2-Chlor-3-propylPhenyl | H | Methyl | |
| 1.1.155 | 2-Brom-3-propylPhenyl | H | Methyl | |
| 1.1.156 | 2-Methyl-3-propylPhenyl | H | Methyl | |
| 1.1.157 | 2-Methyl-3-propylPhenyl | H | Methyl | |
| 1.1.158 | 2-Cyclopropyl-3-propyl-Phenyl | H | Methyl | |
| 1.1.159 | 2-Vinyl-3-propylPhenyl | H | Methyl | |
| 1.1.160 | 2-Ethinyl-3propylPhenyl | H | Methyl | |
| 1.1.161 | 2-Cyano-3-propylPhenyl | H | Methyl | |
| 1.1.162 | 2-Trifluormethyl-3-propyl-Phenyl | H | Methyl | |
| 1.1.163 | 2-Methoxy-3-propylPhenyl | H | Methyl | |
| 1.1.164 | 2-Ethoxy-3-propylPhenyl | H | Methyl | |
| 1.1.165 | 2-Trifluormethoxy-3-propyl-Phenyl | H | Methyl | |
| 1.1.166 | 2-Nitro-3-propylPhenyl | H | Methyl | |
| 1.1.167 | 2-Fluor-3-isopropylPhenyl | H | Methyl | |
| 1.1.168 | 2-Chlor-3-isopropylPhenyl | H | Methyl | |
| 1.1.169 | 2-Brom-3-isopropylPhenyl | H | Methyl | |
| 1.1.170 | 2-Methyl-3-isopropylPhenyl | H | Methyl | |
| 1.1.171 | 2-Ethyl-3-isopropylPhenyl | H | Methyl | |
| 1.1.172 | 2-Cyclopropyl-3-isopropyl-Phenyl | H | Methyl | |
| 1.1.173 | 2-Vinyl-3-isopropylPhenyl | H | Methyl | |
| 1.1.174 | 2-Ethinyl-3-isopropylPhenyl | H | Methyl | |
| 1.1.175 | 2-Cyano-3-isopropylPhenyl | H | Methyl | |
| 1.1.176 | 2-Trifluormethyl-3-isopropyl-Phenyl | H | Methyl | |
| 1.1.177 | 2-Methoxy-3-isopropyl-Phenyl | H | Methyl | |
| 1.1.178 | 2-Ethoxy-3-isopropylPhenyl | H | Methyl | |
| 1.1.179 | 2-Trifluormethoxy-3-isopropyl-Phenyl | H | Methyl | |
| 1.1.180 | 2-Nitro-3-isopropylPhenyl | H | Methyl | |
| 1.1.181 | 2-Fluor-3-tert.butyl-Phenyl | H | Methyl | |
| 1.1.182 | 2-Chlor-3-tert.buty-Phenyl | H | Methyl | |
| 1.1.183 | 2-Brom-3-tert.butyl-Phenyl | H | Methyl | |
| 1.1.184 | 2-Methyl-3-tert.butyl-Phenyl | H | Methyl | |
| 1.1.185 | 2-Ethyl-3-tert.butyl-Phenyl | H | Methyl | |
| 1.1.186 | 2-Cyclopropyl-3-tert. butyl-Phenyl | H | Methyl | |
| 1.1.187 | 2-Vinyl-3-tert.butyl-Phenyl | H | Methyl | |
| 1.1.188 | 2-Ethinyl-3-tert.butyl-Phenyl | H | Methyl | |
| 1.1.189 | 2-Cyano-3-tert.butyl-Phenyl | H | Methyl | |
| 1.1.190 | 2-Trifluormethyl-3-tert.butyl-Phenyl | H | Methyl | |
| 1.1.191 | 2-Methoxy-3-tert.butyl-Phenyl | H | Methyl | |
| 1.1.192 | 2-Ethoxy-3-tert.butyl -Phenyl | H | Methyl | |
| 1.1.193 | 2-Trifluormethoxy-3-tert.butyl-Phenyl | H | Methyl | |
| 1.1.194 | 2-Nitro-3-tert.butyl-Phenyl | H | Methyl | |
| 1.1.195 | 2-Fluor-3-hydroxymethyl-Phenyl | H | Methyl | |
| 1.1.196 | 2-Chlor-3-hydroxymethyl-Phenyl | H | Methyl | |
| 1.1.197 | 2-Brom-3-hydroxymethyl -Phenyl | H | Methyl | |
| 1.1.198 | 2-Methyl-3-hydroxymethyl - Phenyl | H | Methyl | |
| 1.1.199 | 2-Ethyl-3-hydroxymethyl - Phenyl | H | Methyl | |
| 1.1.200 | 2-Cyclopropyl-3-hydroxymethyl-Phenyl | H | Methyl | |
| 1.1.201 | 2-Vinyl-3-hydroxymethyl - Phenyl | H | Methyl | |
| 1.1.202 | 2-Ethinyl-3-hydroxymethyl-Phenyl | H | Methyl | |
| 1.1.203 | 2-Cyano-3-hydroxymethyl - Phenyl | H | Methyl | |
| 1.1.204 | 2-Trifluormethyl-3-hydroxymethyl-Phenyl | H | Methyl | |
| 1.1.205 | 2-Methoxy-3-hydroxymethyl - Phenyl | H | Methyl | |
| 1.1.206 | 2-Ethoxy-3-hydroxymethyl - Phenyl | H | Methyl | |
| 1.1.207 | 2-Trifluormethoxy-3-hydroxymethyl-Phenyl | H | Methyl | |
| 1.1.208 | 2-Nitro-3-hydroxymethyl-Phenyl | H | Methyl | |
| 1.1.209 | 2-Fluor-3-cyclopropyl-Phenyl | H | Methyl | |
| 1.1.210 | 2-Chlor-3-cyclopropyl-Phenyl | H | Methyl | |
| 1.1.211 | 2-Brom-3-cyclopropyl-Phenyl | H | Methyl | |
| 1.1.212 | 2-Methyl-3-cyclopropyl-Phenyl | H | Methyl | |
| 1.1.213 | 2-Ethyl-3-cyclopropyl -Phenyl | H | Methyl | |
| 1.1.214 | 2-Cyclopropyl-3-cyclopropyl-Phenyl | H | Methyl | |
| 1.1.215 | 2-Vinyl-3-cyclopropyl-Phenyl | H | Methyl | |
| 1.1.216 | 2-Ethinyl-3-cyclopropyl-Phenyl | H | Methyl | |
| 1.1.217 | 2-Cyano-3-cyclopropyl-Phenyl | H | Methyl | |
| 1.1.218 | 2-Trifluormethyl-3-cyclopropyl-Phenyl | H | Methyl | |
| 1.1.219 | 2-Methoxy-3-cyclopropyl-Phenyl | H | Methyl | |
| 1.1.220 | 2-Ethoxy-3-cyclopropyl-Phenyl | H | Methyl | |
| 1.1.221 | 2-Trifluormethoxy-3-cyclopropyl-Phenyl | H | Methyl | |
| 1.1.222 | 2-Fluor-3-methoxycarbonyl-Phenyl | H | Methyl | |
| 1.1.223 | 2-Chlor-3-methoxycarbonyl-Phenyl | H | Methyl | |
| 1.1.224 | 2-Brom-3-methoxycarbonyl-Phenyl | H | Methyl | |
| 1.1.225 | 2-Methyl-3-methoxycarbonyl-Phenyl | H | Methyl | |
| 1.1.226 | 2-Ethyl-3-methoxycarbonyl-Phenyl | H | Methyl | |
| 1.1.227 | 2-Cyclopropyl-3-methoxycarbonyl-Phenyl | H | Methyl | |
| 1.1.228 | 2-Vinyl-3-methoxycarbonyl-Phenyl | H | Methyl | |
| 1.1.229 | 2-Ethinyl-3-methoxycarbonyl-Phenyl | H | Methyl | |
| 1.1.230 | 2-Cyano-3-methoxycarbonyl-Phenyl | H | Methyl | |
| 1.1.231 | 2-Trifluormethyl-3-methoxycarbonyl-Phenyl | H | Methyl | |
| 1.1.232 | 2-Methoxy-3-methoxycarbonyl - Phenyl | H | Methyl | |
| 1.1.233 | 2-Ethoxy-3-methoxycarbonyl-Phenyl | H | Methyl | |
| 1.1.234 | 2-Trifluormethoxy-3-methoxycarbonyl-Phenyl | H | Methyl | |
| 1.1.235 | 2-Nitro-3-methoxycarbonyl-Phenyl | H | Methyl | |
| 1.1.236 | 2-Fluor-3-vinyl-Phenyl | H | Methyl | |
| 1.1.237 | 2-Chlor-3-vinyl-Phenyl | H | Methyl | |
| 1.1.238 | 2-Brom-3-vinyl-Phenyl | H | Methyl | |
| 1.1.239 | 2-Methyl-3-vinyl-Phenyl | H | Methyl | |
| 1.1.240 | 2-Ethyl-3-vinyl-Phenyl | H | Methyl | |
| 1.1.241 | 2-Cyclopropyl-3-vinyl-Phenyl | H | Methyl | |
| 1.1.242 | 2-Vinyl-3-vinyl-Phenyl | H | Methyl | |
| 1.1.243 | 2-Ethinyl-3-vinyl-Phenyl | H | Methyl | |
| 1.1.244 | 2-Cyano-3-vinyl-Phenyl | H | Methyl | |
| 1.1.245 | 2-Trifluormethyl-3-vinyl-Phenyl | H | Methyl | |
| 1.1.246 | 2-Methoxy-3-vinyl-Phenyl | H | Methyl | |
| 1.1.247 | 2-Ethoxy-3-vinyl-Phenyl | H | Methyl | |
| 1.1.248 | 2-Trifluormethoxy-3-vinyl-Phenyl | H | Methyl | |
| 1.1.249 | 2-Nitro-3-vinyl-Phenyl | H | Methyl | |
| 1.1.250 | 2-Fluor-3-ethinyl-Phenyl | H | Methyl | |
| 1.1.251 | 2-Chlor-3-ethinyl-Phenyl | H | Methyl | |
| 1.1.252 | 2-Brom-3-ethinyl-Phenyl | H | Methyl | |
| 1.1.253 | 2-Methyl-3-ethinyl-Phenyl | H | Methyl | |
| 1.1.254 | 2-Ethyl-3-ethinyl-Phenyl | H | Methyl | |
| 1.1.255 | 2-Cyclopropyl-3-ethinyl-Phenyl | H | Methyl | |
| 1.1.256 | 2-Vinyl-3-ethinyl-Phenyl | H | Methyl | |
| 1.1.257 | 2-Cyano-3-ethinyl-Phenyl | H | Methyl | |
| 1.1.258 | 2-Trifluormethyl-3-ethinyl-Phenyl | H | Methyl | |
| 1.1.259 | 2-Methoxy-3-ethinyl-Phenyl | H | Methyl | |
| 1.1.260 | 2-Ethoxy-3-ethinyl-Phenyl | H | Methyl | |
| 1.1.261 | 2-Trifluormethoxy-3-ethinyl--Phenyl | H | Methyl | |
| 1.1.262 | 2-Nitro-3-ethinyl-Phenyl | H | Methyl | |
| 1.1.263 | 2-Fluor-3-ethinyl-Phenyl | H | Methyl | |
| 1.1.264 | 2-Fluor-3-cyano-Phenyl | H | Methyl | |
| 1.1.265 | 2-Chlor-3-cyano-Phenyl | H | Methyl | |
| 1.1.266 | 2-Brom-3-cyano-Phenyl | H | Methyl | |
| 1.1.267 | 2-Methyl-3-cyano-Phenyl | H | Methyl | |
| 1.1.268 | 2-Ethyl-3-cyano-Phenyl | H | Methyl | |
| 1.1.269 | 2-Ethyl-3-cyano-Phenyl | H | Ethyl | |
| 1.1.270 | 2-Ethyl-3-cyano-Phenyl | H | Propyl | |
| 1.1.271 | 2-Ethyl-3-cyano-Phenyl | H | Cyclopropyl | |
| 1.1.272 | 2-Cyclopropyl-3-cyano-Phenyl | H | Methyl | |
| 1.1.273 | 2-Vinyl-3-cyano-Phenyl | H | Methyl | |
| 1.1.274 | 2-Ethinyl-3-cyano-Phenyl | H | Methyl | |
| 1.1.275 | 2-Cyano-3-cyanoPhenyl | H | Methyl | |
| 1.1.276 | 2-Trifluormethyl-3-cyano-Phenyl | H | Methyl | |
| 1.1.277 | 2-Methoxy-3-cyano- Phenyl | H | Methyl | |
| 1.1.278 | 2-Ethoxy-3-cyano-Phenyl | H | Methyl | |
| 1.1.279 | 2-Trifluormethoxy-3-cyano-Phenyl | H | Methyl | |
| 1.1.280 | 2-Nitro-3-cyano-Phenyl | H | Methyl | |
| 1.1.281 | 2-Fluor-3-hydroxyPhenyl | H | Methyl | |
| 1.1.282 | 2-Chlor-3-hydroxyPhenyl | H | Methyl | |
| 1.1.283 | 2-Brom-3-hydroxyPhenyl | H | Methyl | |
| 1.1.284 | 2-Methyl-3-hydroxyPhenyl | H | Methyl | |
| 1.1.285 | 2-Ethyl-3-hydroxyPhenyl | H | Methyl | |
| 1.1.286 | 2-Cyclopropyl-3-hydroxy-Phenyl | H | Methyl | |
| 1.1.287 | 2-Vinyl-3-hydroxyPhenyl | H | Methyl | |
| 1.1.288 | 2-Ethinyl-3-hydroxyPhenyl | H | Methyl | |
| 1.1.289 | 2-Cyano-3-hydroxyPhenyl | H | Methyl | |
| 1.1.290 | 2-Trifluormethyl-3-hydroxy-Phenyl | H | Methyl | |
| 1.1.291 | 2-Methoxy-3-hydroxy -Phenyl | H | Methyl | |
| 1.1.292 | 2-Ethoxy-3-hydroxyPhenyl | H | Methyl | |
| 1.1.293 | 2-Trifluormethoxy-3-hydroxy-Phenyl | H | Methyl | |
| 1.1.294 | 2-Nitro-3-hydroxyPhenyl | H | Methyl | |
| 1.1.295 | 2-Fluor-3-methoxyPhenyl | H | Methyl | |
| 1.1.296 | 2-Chlor-3-methoxyPhenyl | H | Methyl | |
| 1.1.297 | 2-Brom-3-methoxyPhenyl | H | Methyl | |
| 1.1.298 | 2-Methyl-3-methoxyPhenyl | H | Methyl | |
| 1.1.299 | 2-Ethyl-3-methoxyPhenyl | H | Methyl | |
| 1.1.300 | 2-Cyclopropyl-3-methoxy-Phenyl | H | Methyl | |
| 1.1.301 | 2-Vinyl-3-methoxyPhenyl | H | Methyl | |
| 1.1.302 | 2-Ethinyl-3-methoxyPhenyl | H | Methyl | |
| 1.1.303 | 2-Cyano-3-methoxyPhenyl | H | Methyl | |
| 1.1.304 | 2-Trifluormethyl-3-methoxy-Phenyl | H | Methyl | |
| 1.1.305 | 2,3-Dimethoxy-Phenyl | H | Methyl | |
| 1.1.306 | 2-Ethoxy-3-methoxyPhenyl | H | Methyl | |
| 1.1.307 | 2-Trifluormethoxy-3-methoxy-Phenyl | H | Methyl | |
| 1.1.308 | 2-Nitro-3-methoxyPhenyl | H | Methyl | |
| 1.1.309 | 2-Fluor-3-ethoxyPhenyl | H | Methyl | |
| 1.1.310 | 2-Chlor-3-ethoxyPhenyl | H | Methyl | |
| 1.1.311 | 2-Brom-3-ethoxyPhenyl | H | Methyl | |
| 1.1.312 | 2-Methyl-3-ethoxyPhenyl | H | Methyl | |
| 1.1.313 | 2-Ethyl-3-ethoxyPhenyl | H | Methyl | |
| 1.1.314 | 2-Cyclopropyl-3-ethoxy-Phenyl | H | Methyl | |
| 1.1.315 | 2-Vinyl-3-ethoxyPhenyl | H | Methyl | |
| 1.1.316 | 2-Ethinyl-3-ethoxyPhenyl | H | Methyl | |
| 1.1.317 | 2-Cyano-3-ethoxyPhenyl | H | Methyl | |
| 1.1.318 | 2-Trifluormethyl-3-ethoxy-Phenyl | H | Methyl | |
| 1.1.319 | 2-Methoxy-3-ethoxyPhenyl | H | Methyl | |
| 1.1.320 | 2,3-Diethoxy--Phenyl | H | Methyl | |
| 1.1.321 | 2-Trifluormethoxy-3-ethoxy-Phenyl | H | Methyl | |
| 1.1.322 | 2-Nitro-3-ethoxyPhenyl | H | Methyl | |
| 1.1.323 | 2-Fluor-3-propoxy-Phenyl | H | Methyl | |
| 1.1.324 | 2-Chlor-3-propoxy-Phenyl | H | Methyl | |
| 1.1.325 | 2-Brom-3-propoxy-Phenyl | H | Methyl | |
| 1.1.326 | 2-Methyl-3-propoxy-Phenyl | H | Methyl | |
| 1.1.327 | 2-Ethyl-3-propoxy-Phenyl | H | Methyl | |
| 1.1.328 | 2-Cyclopropyl-3-propoxy-Phenyl | H | Methyl | |
| 1.1.329 | 2-Vinyl-3-propoxy-Phenyl | H | Methyl | |
| 1.1.330 | 2-Ethinyl-3-propoxy-Phenyl | H | Methyl | |
| 1.1.331 | 2-Cyano-3-propoxy-Phenyl | H | Methyl | |
| 1.1.332 | 2-Trifluormethyl-3-propoxy-Phenyl | H | Methyl | |
| 1.1.333 | 2-Methoxy-3-propoxy-Phenyl | H | Methyl | |
| 1.1.334 | 2-Ethoxy-3-propoxy-Phenyl | H | Methyl | |
| 1.1.335 | 2-Trifluormethoxy-3-propoxy-Phenyl | H | Methyl | |
| 1.1.336 | 2-Nitro-3-propoxy-Phenyl | H | Methyl | |
| 1.1.337 | 2-Fluor-3-isopropoxy-Phenyl | H | Methyl | |
| 1.1.338 | 2-Chlor-3-isopropoxy-Phenyl | H | Methyl | |
| 1.1.339 | 2-Brom-3-isopropoxy-Phenyl | H | Methyl | |
| 1.1.340 | 2-Methyl-3-isopropoxy-Phenyl | H | Methyl | |
| 1.1.341 | 2-Ethyl-3-isopropoxy-Phenyl | H | Methyl | |
| 1.1.342 | 2-Cyclopropyl-3-isopropoxy-Phenyl | H | Methyl | |
| 1.1.343 | 2-Vinyl-3-isopropoxy-Phenyl | H | Methyl | |
| 1.1.344 | 2-Ethinyl-3-isopropoxy-Phenyl | H | Methyl | |
| 1.1.345 | 2-Cyano-3-isopropoxy-Phenyl | H | Methyl | |
| 1.1.346 | 2-Trifluormethyl-3-isopropoxy-Phenyl | H | Methyl | |
| 1.1.347 | 2-Methoxy-3-isopropoxy-Phenyl | H | Methyl | |
| 1.1.348 | 2-Ethoxy-3-isopropoxy-Phenyl | H | Methyl | |
| 1.1.349 | 2-Trifluormethoxy-3-isopropoxy-Phenyl | H | Methyl | |
| 1.1.350 | 2-Nitro-3-isopropoxy-Phenyl | H | Methyl | |
| 1.1.351 | 2-Fluor-3-tert.butoxyPhenyl | H | Methyl | |
| 1.1.352 | 2-Chlor-3-tert.butoxyPhenyl | H | Methyl | |
| 1.1.353 | 2-Brom-3-tert.butoxyPhenyl | H | Methyl | |
| 1.1.354 | 2-Methyl-3-tert.butoxy-Phenyl | H | Methyl | |
| 1.1.355 | 2-Ethyl-3-tert.butoxyPhenyl | H | Methyl | |
| 1.1.356 | 2-Cyclopropyl-3-tert.butoxy-Phenyl | H | Methyl | |
| 1.1.357 | 2-Vinyl-3-tert.butoxyPhenyl | H | Methyl | |
| 1.1.358 | 2-Ethinyl-3-tert.butoxy-Phenyl | H | Methyl | |
| 1.1.359 | 2-Cyano-3-tert.butoxy-Phenyl | H | Methyl | |
| 1.1.360 | 2-Trifluormethyl-3-tert.butoxy-Phenyl | H | Methyl | |
| 1.1.361 | 2-Methoxy-3-tert.butoxy-Phenyl | H | Methyl | |
| 1.1.362 | 2-Ethoxy-3-tert.butoxy-Phenyl | H | Methyl | |
| 1.1.363 | 2-Trifluormethoxy-3-tert.butoxy-Phenyl | H | Methyl | |
| 1.1.364 | 2-Nitro-3-tert.butoxyPhenyl | H | Methyl | |
| 1.1.365 | 2-Fluor-3-trifluormethoxy-Phenyl | H | Methyl | |
| 1.1.366 | 2-Chlor-3-trifluormethoxy-Phenyl | H | Methyl | |
| 1.1.367 | 2-Brom-3-trifluormethoxy-Phenyl | H | Methyl | |
| 1.1.368 | 2-Methyl-3-trifluormethoxy-Phenyl | H | Methyl | |
| 1.1.369 | 2-Ethyl-3-trifluormethoxy-Phenyl | H | Methyl | |
| 1.1.370 | 2-Cyclopropyl-3-trifluormethoxy-Phenyl | H | Methyl | |
| 1.1.371 | 2-Vinyl-3-trifluormethoxy-Phenyl | H | Methyl | |
| 1.1.372 | 2-Ethinyl-3-trifluormethoxy-Phenyl | H | Methyl | |
| 1.1.373 | 2-Cyano-3-trifluormethoxy-Phenyl | H | Methyl | |
| 1.1.374 | 2-Trifluormethyl-3-trifluormethoxy-Phenyl | H | Methyl | |
| 1.1.375 | 2-Methoxy-3-trifluormethoxy-Phenyl | H | Methyl | |
| 1.1.376 | 2-Ethoxy-3-trifluormethoxy-Phenyl | H | Methyl | |
| 1.1.377 | 2,3-Bis(trifluormethoxy)-Phenyl | H | Methyl | |
| 1.1.378 | 2-Nitro-3-trifluormethoxy-Phenyl | H | Methyl | |
| 1.1.379 | 2-Fluor-3-(2,2,2-trifluorethoxy)-Phenyl | H | Methyl | |
| 1.1.380 | 2-Chlor-3-(2,2,2-trifluorethoxy)-Phenyl | H | Methyl | |
| 1.1.381 | 2-Brom-3-(2,2,2-trifluorethoxy)-Phenyl | H | Methyl | |
| 1.1.382 | 2-Methyl-3-(2,2,2-trifluorethoxy)-Phenyl | H | Methyl | |
| 1.1.383 | 2-Ethyl-3-(2,2,2-trifluorethoxy)-Phenyl | H | Methyl | |
| 1.1.384 | 2-Cyclopropyl-3-(2,2,2-trifluorethoxy)-Phenyl | H | Methyl | |
| 1.1.385 | 2-Vinyl-3-(2,2,2-trifluorethoxy)-Phenyl | H | Methyl | |
| 1.1.386 | 2-Ethinyl-3-(2,2,2-trifluorethoxy-Phenyl | H | Methyl | |
| 1.1.387 | 2-Cyano-3-(2,2,2-trifluorethoxy)-Phenyl | H | Methyl | |
| 1.1.388 | 2-Trifluormethyl-3-(2,2,2-trifluorethoxy)-Phenyl | H | Methyl | |
| 1.1.389 | 2-Methoxy-3-(2,2,2-trifluorethoxy)-Phenyl | H | Methyl | |
| 1.1.390 | 2-Ethoxy-3-(2,2,2-trifluorethoxy)-Phenyl | H | Methyl | |
| 1.1.391 | 2-Trifluormethoxy-3-(2,2,2-trifluorethoxy)-Phenyl | H | Methyl | |
| 1.1.392 | 2-Nitro-3-(2,2,2-trifluorethoxy)-Phenyl | H | Methyl | |
| 1.1.393 | 2-Fluor-3-difluormethoxy-Phenyl | H | Methyl | |
| 1.1.394 | 2-Chlor-3-difluormethoxy-Phenyl | H | Methyl | |
| 1.1.395 | 2-Brom-3-difluormethoxy-Phenyl | H | Methyl | |
| 1.1.396 | 2-Methyl-3-difluormethoxy-Phenyl | H | Methyl | |
| 1.1.397 | 2-Ethyl-3-difluormethoxy-Phenyl | H | Methyl | |
| 1.1.398 | 2-Cyclopropyl-3-difluormethoxy-Phenyl | H | Methyl | |
| 1.1.399 | 2-Vinyl-3-difluormethoxy-Phenyl | H | Methyl | |
| 1.1.400 | 2-Ethinyl-3-difluormethoxy-Phenyl | H | Methyl | |
| 1.1.401 | 2-Cyano-3-difluormethoxy - Phenyl | H | Methyl | |
| 1.1.402 | 2-Trifluormethyl-3-difluormethoxy - Phenyl | H | Methyl | |
| 1.1.403 | 2-Methoxy-3-difluormethoxy - Phenyl | H | Methyl | |
| 1.1.404 | 2-Ethoxy-3-difluormethoxy-Phenyl | H | Methyl | |
| 1.1.405 | 2-Trifluormethoxy-3-difluormethoxy-Phenyl | H | Methyl | |
| 1.1.406 | 2-Nitro-3-difluormethoxy-Phenyl | H | Methyl | |
| 1.1.407 | 2-Fluor-3-(2-methoxyethoxy)-Phenyl | H | Methyl | |
| 1.1.408 | 2-Chlor-3-(2-methoxyethoxy)-Phenyl | H | Methyl | |
| 1.1.409 | 2-Brom-3-(2-methoxyethoxy)-Phenyl | H | Methyl | |
| 1.1.410 | 2-Methyl-3-(2-methoxyethoxy)-Phenyl | H | Methyl | |
| 1.1.411 | 2-Ethyl-3-(2-methoxyethoxy)-Phenyl | H | Methyl | |
| 1.1.412 | 2-Cyclopropyl-3-(2-methoxyethoxy)-Phenyl | H | Methyl | |
| 1.1.413 | 2-Vinyl-3-(2-methoxyethoxy)-Phenyl | H | Methyl | |
| 1.1.414 | 2-Ethinyl-3-(2-methoxyethoxy)-Phenyl | H | Methyl | |
| 1.1.415 | 2-Cyano-3-(2-methoxyethoxy) - Phenyl | H | Methyl | |
| 1.1.416 | 2-Trifluormethyl-3-(2-methoxyethoxy) - Phenyl | H | Methyl | |
| 1.1.417 | 2-Methoxy-3-(2-methoxyethoxy) - Phenyl | H | Methyl | |
| 1.1.418 | 2-Ethoxy-3-(2-methoxyethoxy)-Phenyl | H | Methyl | |
| 1.1.419 | 2-Trifluormethoxy-(2-methoxyethoxy)-Phenyl | H | Methyl | |
| 1.1.420 | 2-Nitro-3-(2-methoxyethoxy)-Phenyl | H | Methyl | |
| 1.1.421 | 2-Fluor-3-(tert.butoxycarbonyloxy)-Phenyl | H | Methyl | |
| 1.1.422 | 2-Chlor-3-(tert.butoxycarbonyloxy)-Phenyl | H | Methyl | |
| 1.1.423 | 2-Brom-3-(tert.butoxycarbonyloxy)-Phenyl | H | Methyl | |
| 1.1.424 | 2-Methyl-3-(tert.butoxycarbonyloxy)-Phenyl | H | Methyl | |
| 1.1.425 | 2-Ethyl-3-(tert.butoxycarbonyloxy)-Phenyl | H | Methyl | |
| 1.1.426 | 2-Cyclopropyl-3-(tert.butoxycarbonyloxy)-Phenyl | H | Methyl | |
| 1.1.427 | 2-Vinyl-3-(tert.butoxycarbonyloxy)-Phenyl | H | Methyl | |
| 1.1.428 | 2-Ethinyl-3-(tert.butoxycarbonyloxy)-Phenyl | H | Methyl | |
| 1.1.429 | 2-Cyano-3-(tert.butoxycarbonyloxy)-Phenyl | H | Methyl | |
| 1.1.430 | 2-Trifluormethyl-3-(tert.butoxycarbonyloxy)-Phenyl | H | Methyl | |
| 1.1.431 | 2-Methoxy-3-(tert.butoxycarbonyloxy)-Phenyl | H | Methyl | |
| 1.1.432 | 2-Ethoxy-3-(tert.butoxycarbonyloxy)-Phenyl | H | Methyl | |
| 1.1.433 | 2-Trifluormethoxy-3-(tert.butoxycarbonyloxy)-Phenyl | H | Methyl | |
| 1.1.434 | 2-Nitro-3-(tert.butoxycarbonyloxy)-Phenyl | H | Methyl | |
| 1.1.435 | 2-Fluor-3-nitroPhenyl | H | Methyl | |
| 1.1.436 | 2-Chlor-3-nitroPhenyl | H | Methyl | |
| 1.1.437 | 2-Brom-3-nitroPhenyl | H | Methyl | |
| 1.1.438 | 2-Methyl-3-nitroPhenyl | H | Methyl | |
| 1.1.439 | 2-Ethyl-3-nitroPhenyl | H | Methyl | |
| 1.1.440 | 2-Cyclopropyl-3-nitro-Phenyl | H | Methyl | |
| 1.1.441 | 2-Vinyl-3-nitroPhenyl | H | Methyl | |
| 1.1.442 | 2-Ethinyl-3-nitroPhenyl | H | Methyl | |
| 1.1.443 | 2-Cyano-3-nitroPhenyl | H | Methyl | |
| 1.1.444 | 2-Trifluormethyl-3-nitro-Phenyl | H | Methyl | |
| 1.1.445 | 2-Methoxy-3-nitroPhenyl | H | Methyl | |
| 1.1.446 | 2-Ethoxy-3-nitroPhenyl | H | Methyl | |
| 1.1.447 | 2-Trifluormethoxy-3-nitro-Phenyl | H | Methyl | |
| 1.1.448 | 2-Fluor-3-methylsulfanylPhenyl | H | Methyl | |
| 1.1.449 | 2-Chlor-3-methylsulfanyl-Phenyl | H | Methyl | |
| 1.1.450 | 2-Brom-3-methylsulfanyl-Phenyl | H | Methyl | |
| 1.1.451 | 2-Methyl-3-methylsulfanyl-Phenyl | H | Methyl | |
| 1.1.452 | 2-Ethyl-3-methylsulfanyl-Phenyl | H | Methyl | |
| 1.1.453 | 2-Cyclopropyl-3-methylsulfanyl-Phenyl | H | Methyl | |
| 1.1.454 | 2-Vinyl-3-methylsulfanyl-Phenyl | H | Methyl | |
| 1.1.455 | 2-Ethinyl-3-methylsulfanyl-Phenyl | H | Methyl | |
| 1.1.456 | 2-Cyano-3-methylsulfanyl - Phenyl | H | Methyl | |
| 1.1.457 | 2-Trifluormethyl-3-methylsulfanyl - Phenyl | H | Methyl | |
| 1.1.458 | 2-Methoxy-3-methylsulfanyl - Phenyl | H | Methyl | |
| 1.1.459 | 2-Ethoxy-3-methylsulfanyl-Phenyl | H | Methyl | |
| 1.1.460 | 2-Trifluormethoxy-3-methylsulfanyl-Phenyl | H | Methyl | |
| 1.1.461 | 2-Nitro-3-methylsulfanyl-Phenyl | H | Methyl | |
| 1.1.462 | 3,5-Difluor-Phenyl | H | Methyl | [CDCl₃] 1.80 (s, 3H); 3.25 (d, 1H); 3.83 (d, 1H); 6.90 (m, 1H); 7.15 (m, 2H). |
| 1.1.463 | (R)-3,5-Difluor-Phenyl | H | Methyl | [CDCl₃] 1.79 (s,3H); 1.90 (s br,1H); 3.26 (d,1H); 3.84 (d,1H), 6.90 (t,1H); 7.18 (d,2H). |
| 1.1.464 | (S)-3,5-Difluor-Phenyl | H | Methyl | [CDCl3] 1.79 (s,3H); 3.25 (d,1H); 3.83 (d,1H), 6.89 (t,1H); 7.18 (d,2H). |
| 1.1.465 | rel-(4R,5R)-3,5-Difluor-Phenyl | Methyl | Methyl | |
| 1.1.466 | rel-(4R,5R)-3,5-Difluor-Phenyl | Ethyl | Methyl | [CDCl₃] 0.93 (t, 3H); 1.61 (s, 3H); 1.75 (m, 2H); 3.84 (dd, 1H); 6.90 (m, 1H); 7.21 (m, 2H). |
| 1.1.467 | 3,5-Difluor-Phenyl | Br | Methyl | |
| 1.1.468 | 3,5-Difluor-Phenyl | H | Ethyl | [CDCl₃] 1.09 (t, 3H); 2.12 (mc, 2H); 3.30 (d, 1H); 3.75 (d, 1H); 6.90 (m, 1H); 7.18 (m, 2H). |
| 1.1.469 | 3,5-Difluor-Phenyl | H | isoPropyl | [CDCl₃] 1.06 (m,6H); 2.40 (m,1H); 3.30 (d,1H); 3.70 (d,1H); 6.90 (m,1H); 7.19 (m,2H). |
| 1.1.470 | 3,5-Difluor-Phenyl | H | Cyclopropyl | |
| 1.1.471 | 3-Chlor-5-fluor-Phenyl | H | Methyl | [CDCl₃] 1.76 (s, 3H); 3.25 (d, 1H); 3.82 (d, 1H); 7.16 (m, 1H); 7.30 (m, 1H); 7.4 (bs, 1H). |
| 1.1.472 | 3-Chlor-5-fluor-Phenyl | H | Ethyl | [CDCl₃] 1.05 (t, 3H); 2.11 (mc, 2H); 3.28 (d, 1H); 3.75 (d, 1H); 7.18 (m, 1H); 7.28 (m, 1H); 7.40 (1H). |
| 1.1.473 | 3-Chlor-5-fluor-Phenyl | H | isoPropyl | |
| 1.1.474 | 3-Chlor-5-fluor-Phenyl | H | Cyclopropyl | |
| 1.1.475 | 3-Brom-5-fluor-Phenyl | H | Methyl | [CDCl₃] 1.70 (s, 3H); 3.25 (d, 1H); 3.85 (d, 1H); 7.35 (m, 2H); 7.56 (m, 1H). |
| 1.1.476 | 3-Brom-5-fluor-Phenyl | H | Ethyl | |
| 1.1.477 | 3-Brom-5-fluor-Phenyl | H | isoPropyl | |
| 1.1.478 | 3-Brom-5-fluor-Phenyl | H | Cyclopropyl | |
| 1.1.479 | 3-Iod-5-fluor-Phenyl | H | Methyl | |
| 1.1.480 | 3-Methyl-5-fluor-Phenyl | H | Methyl | [DMSO] 1.51 (s, 3H); 2.35 (s, 3H); 3.31 (d, 1H); 3.76 (d, 1H); 7.14 (d, 1H); 7.25 (d, 1H); 7.35 (s, 1H). |
| 1.1.481 | 3-Methyl-5-fluor-Phenyl | H | Ethyl | |
| 1.1.482 | 3-Methyl-5-fluor-Phenyl | H | Propyl | |
| 1.1.483 | 3-Methyl-5-fluor-Phenyl | H | Cyclopropyl | |
| 1.1.484 | 3-Ethyl-5-fluor-Phenyl | H | Methyl | [DMSO] 1.20 (t, 3H); 1.55 (s, 3H); 2.65 (q, 2H); 3.38 (d, 1H); 3.80 (d, 1H); 7.15 (d, 1H);7.38 (d, 1H); 7.39 (s, 1H). |
| 1.1.485 | 3-Propyl-5-fluor-Phenyl | H | Methyl | |
| 1.1.486 | 3-iPropyl-5-fluor-Phenyl | H | Methyl | |
| 1.1.487 | 3-nButyl-5-fluor-Phenyl | H | Methyl | |
| 1.1.488 | 3-isoButyl-5-fluor-Phenyl | H | Methyl | |
| 1.1.489 | 3-tert.Butyl-5-fluor-Phenyl | H | Methyl | |
| 1.1.490 | 3-Cyclopropyl-5-fluor-Phenyl | H | Methyl | [CDCl₃] 0.71 (m, 2H); 1.03 (m, 2H); 1.75 (s, 3H); 1.91 (m, 1H); 3.26 (d, 1H); 3.85 (d, 1H); 6.81 (dd, H); 7.13 (dd, 2H). |
| 1.1.491 | 3-Vinyl-5-fluor- | H | Methyl | [CDCl₃] 1.80 (s, 3H); 3.27 (d, 1H); 3.86 (d, 1H); 5.39 (d, 1H); 5.8 (d, 1H); 6.69 (dd 1H); 7.19 (m, 1H); 7.25 (m, 1H); 7.40 (bs, 1H). |
| 1.1.492 | 3-Ethinyl-5-fluor-Phenyl | H | Methyl | |
| 1.1.493 | 3-Cyano-5-fluor-Phenyl | H | Methyl | |
| 1.1.494 | 3-Trifluormethyl-5-fluor-Phenyl | H | Methyl | [CDCl₃] 1.80 (s, 3H); 3.29 (d, 1H); 3.90 (d, 1H); 7.40 (m, 1H); 7.59 (m, 1H); 7.66 (s, 1H). |
| 1.1.495 | 3-Trifluormethyl-5-fluor-Phenyl | H | Ethyl | |
| 1.1.496 | 3-Trifluormethyl-5-fluor-Phenyl | H | Propyl | |
| 1.1.497 | 3-Trifluormethyl-5-fluor-Phenyl | H | Cyclopropyl | |
| 1.1.498 | 3-(Methoxycarbony)l-5-fluor-Phenyl | H | Methyl | |
| 1.1.499 | 3-Hydroxymethyl-5-fluor-Phenyl | H | Methyl | |
| 1.1.500 | 3-Carbamoyl-5-fluor-Phenyl | H | Methyl | |
| 1.1.501 | 3-Hydoxy-5-fluor-Phenyl- | H | Methyl | |
| 1.1.502 | 3-Methoxy-5-fluor-Phenyl | H | Methyl | [CDCl₃] 1.78 (s, 3H); 3.25 (d, 1H); 3.82 (s, 3H); 3.88 (d, 1H); 6.7 (dd, 1H); 6.92 (dd, 1H); 7.05 (bs, 1H). |
| 1.1.503 | 3-Ethoxy-5-fluor-Phenyl | H | Methyl | |
| 1.1.504 | 3-nPropyoxy-5-fluor-Phenyl | H | Methyl | |
| 1.1.505 | 3-isoPropoxy-5-fluor-Phenyl | H | Methyl | |
| 1.1.506 | 3-nButoxy-5-fluor-Phenyl | H | Methyl | |
| 1.1.507 | 3-isoButoxy-5-fluor-Phenyl | H | Methyl | |
| 1.1.508 | 3-tert.Butoxy-5-fluor-Phenyl | H | Methyl | |
| 1.1.509 | 3-Difluormethoxy-5-fluor-Phenyl | H | Methyl | |
| 1.1.510 | 3-Trifluormethoxy-5-fluor-Phenyl | H | Methyl | |
| 1.1.511 | 3-(2,2,2-Trifluorethoxy)-5-fluor-Phenyl | H | Methyl | |
| 1.1.512 | 3-(2-Chlorethoxy)-5-fluor-Phenyl | H | Methyl | |
| 1.1.513 | 3-(2-Hydroxyethoxy)-5-fluor-Phenyl- | H | Methyl | |
| 1.1.514 | 3-[(tert-Butoxycarbonyl)oxy]-5-fluor-Phenyl | H | Methyl | |
| 1.1.515 | 3-Nitro-5-fluor-Phenyl | H | Methyl | |
| 1.1.516 | 3-Acetoxy-5-fluor-Phenyl | H | Methyl | |
| 1.1.517 | {3-[(tert-Butoxycarbonyl)amino]-5-fluor-Phenyl} | H | Methyl | |
| 1.1.518 | 3-Methylsulfanyl-5-fluor-Phenyl | H | Methyl | |
| 1.1.519 | 3,5-Dichlor-Phenyl | H | Methyl | [CDCl3] 1.79 (s,3H); 3.25 (d,1H), 3.83 (d,1H); 7.41 (s,1H); 7.53 (s,2H). |
| 1.1.520 | (R)-3,5-Dichlor-Phenyl | H | Methyl | [CDCl3] 1.78 (s,3H); 3.25 (d,1H); 3.83 (d,1H); 5.3 (s br,1H); 7.42 (s,1H); 7.52 (s,2H). |
| 1.1.521 | (S)-3,5-Dichlor-Phenyl | H | Methyl | [CDCl3] 1.78 (s,3H); 3.25 (d,1H), 3.83 (d,1H); 5.3 (s br, 1H); 7.42 (s,1H); 7.52 (s,2H). |
| 1.1.522 | rel-(4R,5S)-3,5-Dichlor-Phenyl | Methyl | Methyl | [CDCl₃] 1.30 (d, 3H); 1.70 (s, 3H); 3.55 (q, 1H); 7.44 (m, 1H); 7.55 (m, 2H). |
| 1.1.523 | rel-(4R,5R)-3,5-Dichlor-Phenyl | Methyl | Methyl | [CDCl₃] 1.25 (d, 3H); 1.70 (s, 3H); 3.95 (q, 2H); 7.42 (m, 1H); 7.58 (m, 2H). |
| 1.1.524 | 3,5-Dichlor-Phenyl | H | Ethyl | [CDCl₃]1.18 (dq, 3H); 2.11 (m, 2H); 3.30 (d, 1H); 3.75 (d, 1H); 7.42 (s, 1H), 7.72 (s, 1H). |
| 1.1.525 | 3,5-Dichlor-Phenyl | H | isoPropyl | [CDCl₃] 1.18 (dd, 6H); 2.40 (dq, 1H); 3.32 (d, 1H); 3.70 (d, 1H); 7.41 (bs, 1H); 7.55 (bs, 2H). |
| 1.1.526 | 3,5-Dichlor-Phenyl | H | Cyclopropyl | [CDCl₃] 0.45-0.80 (m, 4H); 1.51 (m, 1H); 3.39(d, 1H); 3.82 (d, 1H); 7.42 (m, 1H); 7.50 (m, m, 2H). |
| 1.1.527 | 3-Brom-5-chlor-Phenyl | H | Methyl | [CDCl₃] 1.79 (s, 3H); 3.25 (d, 1H); 3.85 (d, 1H); 7.60 (s, 2H); 7.70 (s, 1H). |
| 1.1.528 | 3-Iod-5-chlor-Phenyl | H | Methyl | |
| 1.1.529 | 3-Methyl-5-chlor-Phenyl | H | Methyl | [DMSO] 1.55 (s, 3H); 2.35 (s, 3H); 3.38 (d, 1H); 3.77 (d, 1H); 7.38 (s, 1H); 7.50 (d, 1H). |
| 1.1.530 | 3-Ethyl-5-chlor-Phenyl | H | Methyl | [CDCl₃] 1.25 (7. 3H); 1.80 (s, 3H); 2.68 (q, 2H); 3.27 (d, 1H); 3.85 (d, 1H); 7.28 (m, 1H); 3.38 (s, 1H); 7.43 (s, 1H). |
| 1.1.531 | 3-Propyl-5-chlor-Phenyl | H | Methyl | |
| 1.1.532 | 3-isoPropyl-5-chlor-Phenyl | H | Methyl | |
| 1.1.533 | 3-nButyl-5-chlor-Phenyl | H | Methyl | |
| 1.1.534 | 3-isoButyl-5-chlor-Phenyl | H | Methyl | |
| 1.1.535 | 3-tert.Butyl-5-chlor-Phenyl | H | Methyl | |
| 1.1.536 | 3-Cyclopropyl-5-chlor-Phenyl | H | Methyl | |
| 1.1.537 | 3-Vinyl-5-chlor-Phenyl | H | Methyl | |
| 1.1.538 | 3-Ethinyl-5-chlor-Phenyl | H | Methyl | |
| 1.1.539 | 3-Cyano-5-chlor-Phenyl | H | Methyl | |
| 1.1.540 | 3-Trifluormethyl-5-chlor-Phenyl | H | Methyl | [CDCl₃] 1.80 (s, 3H); 3.30 (d, 1H); 3.89 (d, 1H); 7.69 (s, 1H); 7.78 (s, 1H); 7.82 (s, 1H). |
| 1.1.541 | 3-(Hydroxycarbonyl)-5-chlor-Phenyl | H | Methyl | |
| 1.1.542 | 3-(Methoxycarbony)l-5-chlor-Phenyl | H | Methyl | |
| 1.1.543 | 3-Hydroxymethyl-5-chlor-Phenyl | H | Methyl | |
| 1.1.544 | 3-Carbamoyl-5-chlor-Phenyl | H | Methyl | |
| 1.1.545 | 3-Hydroxy-5-chlor-Phenyl- | H | Methyl | |
| 1.1.546 | 3-Methoxy-5-chlor-Phenyl | H | Methyl | [CDCl3] 1.77 (s, 3H); 3.25 (d, 1H); 3.71 (s, 3H); 3.82 (d, 1H); 6.95-7.18 (m, Ar, 3H) |
| 1.1.547 | 3-Ethoxy-5-chlor-Phenyl | H | Methyl | |
| 1.1.548 | 3-nPropyoxy-5-chlor-Phenyl | H | Methyl | |
| 1.1.549 | 3-isoPropoxy-5-chlor-Phenyl | H | Methyl | |
| 1.1.550 | 3-nButoxy-5-chlor-Phenyl | H | Methyl | |
| 1.1.551 | 3-isoButoxy-5-chlor-Phenyl | H | Methyl | |
| 1.1.552 | 3-tert.Butoxy-5-chlor-Phenyl | H | Methyl | |
| 1.1.553 | 3-Difluormethoxy-5-chlor-Phenyl | H | Methyl | |
| 1.1.554 | 3-Trifluormethoxy-5-chlor-Phenyl | H | Methyl | [DMSO] 1.59 (s, 3H); 3.43 (d, 1H); 3.85 (d, 1H); 7.63 (s, 1H); 7.69 (s, 1H); 6.78 (s, 1H). |
| 1.1.555 | 3-(2,2,2-Trifluorethoxy)-5-chlor-Phenyl | H | Methyl | |
| 1.1.556 | 3-(2-Chlorethoxy)-5-chlor-Phenyl | H | Methyl | |
| 1.1.557 | 3-(2-Hydroxyethoxy)-5-chlor-Phenyl- | H | Methyl | |
| 1.1.558 | 3-[(tert-Butoxycarbonyl)oxy]-5-chlor-Phenyl | H | Methyl | |
| 1.1.559 | 3-Nitro-5-chlor-Phenyl | H | Methyl | |
| 1.1.560 | 3-Acetoxy-5-chlor-Phenyl | H | Methyl | |
| 1.1.561 | {3-[(tert-Butoxycarbonyl)amino]-5-chlor-Phenyl} | H | Methyl | |
| 1.1.562 | 3-Methylsulfanyl-5-chlor-Phenyl | H | Methyl | |
| 1.1.563 | 3,5-Dibrom-Phenyl | H | Methyl | [CDCl₃] 1.80 (s, 3H); 3.25 (d, 1H); 3.85 (d, 1H); 7.73 (s, 3H). |
| 1.1.564 | 3,5-Dibrom-Phenyl | H | Ethyl | [CDCl₃] 1.08 (t, 3H); 2.10 (m, 2H); 3.25 (d, 1H); 3.75 (d, 1H); 7.72 (s, 3H). |
| 1.1.565 | 3-Iod-5-brom-Phenyl | H | Methyl | |
| 1.1.566 | 3-Methyl-5-brom-Phenyl | H | Methyl | [CDCl₃] 1.78 (s,3H); 2.36 (s,3H); 3.27 (d,1H); 3.84 (d,1H); 7.40 (s,2H); 7.57 (s,1H). |
| 1.1.567 | 3-Methyl-5-brom-Phenyl | H | Ethyl | |
| 1.1.568 | 3-Methyl-5-brom-Phenyl | H | isoPropyl | |
| 1.1.569 | 3-Methyl-5-brom-Phenyl | H | Cyclopropyl | |
| 1.1.570 | 3-Ethyl-5-brom-Phenyl | H | Methyl | |
| 1.1.571 | 3-Propyl-5-brom-Phenyl | H | Methyl | |
| 1.1.572 | 3-isoPropyl-5-brom-Phenyl | H | Methyl | |
| 1.1.573 | 3-nButyl-5-brom-Phenyl | H | Methyl | |
| 1.1.574 | 3-isoButyl-5-brom-Phenyl | H | Methyl | |
| 1.1.575 | 3-tert.Butyl-5-brom-Phenyl | H | Methyl | |
| 1.1.576 | 3-Cyclopropyl-5-brom-Phenyl | H | Methyl | |
| 1.1.577 | 3-Vinyl-5-brom-Phenyl | H | Methyl | |
| 1.1.578 | 3-Ethinyl-5-brom-Phenyl | H | Methyl | |
| 1.1.579 | 3-Cyano-5-brom-Phenyl | H | Methyl | |
| 1.1.580 | 3-Trifluormethyl-5-brom-Phenyl | H | Methyl | [CDCl₃] 1.80 (s, 3H); 3.30 (d, 1H); 3.90 (d, 1H); 7.81 (s, 2H); 8.0 (s, 1H). |
| 1.1.581 | 3-(Hydroxycarbonyl)-5-brom-Phenyl | H | Methyl | |
| 1.1.582 | 3-(Methoxycarbony)l-5-brom-Phenyl | H | Methyl | |
| 1.1.583 | 3-Hydroxymethyl-5-brom-Phenyl | H | Methyl | |
| 1.1.584 | 3-Carbamoyl-5-brom-Phenyl | H | Methyl | |
| 1.1.585 | 3-Hydroxy-5-brom-Phenyl- | H | Methyl | |
| 1.1.586 | 3-Methoxy-5-brom-Phenyl | H | Methyl | |
| 1.1.587 | 3-Ethoxy-5-brom-Phenyl | H | Methyl | |
| 1.1.588 | 3-nPropyoxy-5-brom-Phenyl | H | Methyl | |
| 1.1.589 | 3-isoPropoxy-5-brom-Phenyl | H | Methyl | |
| 1.1.590 | 3-nButoxy-5-brom-Phenyl | H | Methyl | |
| 1.1.591 | 3-isoButoxy-5-brom-Phenyl | H | Methyl | |
| 1.1.592 | 3-tert.Butoxy-5-brom-Phenyl | H | Methyl | |
| 1.1.593 | 3-Difluormethoxy-5-brom-Phenyl | H | Methyl | |
| 1.1.594 | 3-Trifluormethoxy-5-brom-Phenyl | H | Methyl | |
| 1.1.595 | 3-(2,2,2-Trifluorethoxy)-5-brom-Phenyl | H | Methyl | |
| 1.1.596 | 3-(2-Chlorethoxy)-5-brom-Phenyl | H | Methyl | |
| 1.1.597 | 3-(2-Hydroxyethoxy)-5-brom-Phenyl- | H | Methyl | |
| 1.1.598 | 3-[(tert-Butoxycarbonyl)oxy]-5-brom-Phenyl | H | Methyl | |
| 1.1.599 | 3-Nitro-5-brom-Phenyl | H | Methyl | |
| 1.1.600 | 3-Acetoxy-5-brom-Phenyl | H | Methyl | |
| 1.1.601 | {3-[(tert-Butoxycarbonyl)amino]-5-brom-Phenyl} | H | Methyl | |
| 1.1.602 | 3-Methylsulfanyl-5-brom-Phenyl | H | Methyl | |
| 1.1.603 | 3,5-Diiod-Phenyl | H | Methyl | [CDCl₃] 1.75 (s, 3H); 3.23 (d; 1H); 3.80 (d, 1H); 7.91 (s, 2H); 8.10 (s, 1H): |
| 1.1.604 | 3-Methyl-5-iod-Phenyl | H | Methyl | |
| 1.1.605 | 3-Ethyl-5-iod-Phenyl | H | Methyl | |
| 1.1.606 | 3-Propyl-5-iod-Phenyl | H | Methyl | |
| 1.1.607 | 3-isoPropyl-5-iod-Phenyl | H | Methyl | |
| 1.1.608 | 3-nButyl-5-iod-Phenyl | H | Methyl | |
| 1.1.609 | 3-isoButyl-5-iod-Phenyl | H | Methyl | |
| 1.1.610 | 3-tert.Butyl-5-iod-Phenyl | H | Methyl | |
| 1.1.611 | 3-Cyclopropyl-5-iod-Phenyl | H | Methyl | |
| 1.1.612 | 3-Vinyl-5-iod-Phenyl | H | Methyl | |
| 1.1.613 | 3-Ethinyl-5-iod-Phenyl | H | Methyl | |
| 1.1.614 | 3-Cyano-5-iod-Phenyl | H | Methyl | |
| 1.1.615 | 3-Trifluormethyl-5-iod-Phenyl | H | Methyl | |
| 1.1.616 | 3-(Hydroxycarbonyl)-5-iod-Phenyl | H | Methyl | |
| 1.1.617 | 3-(Methoxycarbonyl)-5-iod-Phenyl | H | Methyl | |
| 1.1.618 | 3-Hydroxymethyl-5-iod-Phenyl | H | Methyl | |
| 1.1.619 | 3-Carbamoyl-5-iod-Phenyl | H | Methyl | |
| 1.1.620 | 3-Hydroxy-5-iod-Phenyl- | H | Methyl | |
| 1.1.621 | 3-Methoxy-5-iod-Phenyl | H | Methyl | |
| 1.1.622 | 3-Ethoxy-5-iod-Phenyl | H | Methyl | |
| 1.1.623 | 3-nPropyoxy-5-iod-Phenyl | H | Methyl | |
| 1.1.624 | 3-isoPropoxy-5-iod-Phenyl | H | Methyl | |
| 1.1.625 | 3-nButoxy-5-iod-Phenyl | H | Methyl | |
| 1.1.626 | 3-isoButoxy-5-iod-Phenyl | H | Methyl | |
| 1.1.627 | 3-tert. Butoxy-5-iod-Phenyl | H | Methyl | |
| 1.1.628 | 3-Difluormethoxy-5-iod-Phenyl | H | Methyl | |
| 1.1.629 | 3-Trifluormethoxy-5-iod-Phenyl | H | Methyl | |
| 1.1.630 | 3-(2,2,2-Trifluorethoxy)-5-iod-Phenyl | H | Methyl | |
| 1.1.631 | 3-(2-Chlorethoxy)-5-iod-Phenyl | H | Methyl | |
| 1.1.632 | 3-(2-Hydroxyethoxy)-5-iod-Phenyl- | H | Methyl | |
| 1.1.633 | 3-[(tert-Butoxycarbonyl)oxy]-5-iod-Phenyl | H | Methyl | |
| 1.1.634 | 3-Nitro-5-iod-Phenyl | H | Methyl | |
| 1.1.635 | 3-Acetoxy-5-iod-Phenyl | H | Methyl | |
| 1.1.636 | {3-[(tert-Butoxycarbonyl)amino]-5-iod-Phenyl} | H | Methyl | |
| 1.1.637 | 3-Methylsulfanyl-5-iod-Phenyl | H | Methyl | |
| 1.1.638 | 3,5-Dimethyl-Phenyl | H | Methyl | [CDCl3] 1.75 (s, 3H);, 2.32 (s, 6H); 3.3 (d, 1H); 3.85 (d. 1H); 7.09 (s, 1H); 7.35 (s, 2H). |
| 1.1.639 | 3-Ethyl-5-methylPhenyl | H | Methyl | |
| 1.1.640 | 3-Propyl-5-methylPhenyl | H | Methyl | |
| 1.1.641 | 3-isoPropyl-5-methylPhenyl | H | Methyl | |
| 1.1.642 | 3-nButyl-5-methylPhenyl | H | Methyl | |
| 1.1.643 | 3-isoButyl-5-methylPhenyl | H | Methyl | |
| 1.1.644 | 3-tert. Butyl-5-methylPhenyl | H | Methyl | |
| 1.1.645 | 3-Cyclopropyl-5-methyl-Phenyl | H | Methyl | |
| 1.1.646 | 3-Vinyl-5-methylPhenyl | H | Methyl | |
| 1.1.647 | 3-Ethinyl-5-methylPhenyl | H | Methyl | |
| 1.1.648 | 3-Cyano-5-methyl-Phenyl | H | Methyl | |
| 1.1.649 | 3-Trifluormethyl-5-methyl-Phenyl | H | Methyl | |
| 1.1.650 | 3-(Hydroxycarbonyl)-5-methyl-Phenyl | H | Methyl | |
| 1.1.651 | 3-(Methoxycarbony)l-5-methyl-Phenyl | H | Methyl | |
| 1.1.652 | 3-Hydroxymethyl-5-methyl-Phenyl | H | Methyl | |
| 1.1.653 | 3-Carbamoyl-5-methyl-Phenyl | H | Methyl | |
| 1.1.654 | 3-Hydroxy-5-methyl-Phenyl- | H | Methyl | |
| 1.1.655 | 3-Methoxy-5-methyl-Phenyl | H | Methyl | |
| 1.1.656 | 3-Ethoxy-5-methyl-Phenyl | H | Methyl | |
| 1.1.657 | 3-nPropyoxy-5-methyl-Phenyl | H | Methyl | |
| 1.1.658 | 3-nButoxy-5-methyl-Phenyl | H | Methyl | |
| 1.1.659 | 3-isoButoxy-5-methyl-Phenyl | H | Methyl | |
| 1.1.660 | 3-tert.Butoxy-5-methyl-Phenyl | H | Methyl | |
| 1.1.661 | 3-Difluormethoxy-5-methyl-Phenyl | H | Methyl | |
| 1.1.662 | 3-Trifluormethoxy-5-methyl-Phenyl | H | Methyl | |
| 1.1.663 | 3-(2,2,2-Trifluorethoxy)-5-methyl-Phenyl | H | Methyl | |
| 1.1.664 | 3-(2-Chlorethoxy)-5-methyl-Phenyl | H | Methyl | |
| 1.1.665 | 3-(2-Hydroxyethoxy)-5-methyl-Phenyl- | H | Methyl | |
| 1.1.666 | 3-[(tert-Butoxycarbonyl)oxy]-5-methyl-Phenyl | H | Methyl | |
| 1.1.667 | 3-Nitro-5-methyl-Phenyl | H | Methyl | |
| 1.1.668 | 3-Acetoxy-5-methyl-Phenyl | H | Methyl | |
| 1.1.669 | {3-[(tert-Butoxycarbonyl)amino]-5-methyl-Phenyl} | H | Methyl | |
| 1.1.670 | 3-Methylsulfanyl-5-methyl-Phenyl | H | Methyl | |
| 1.1.671 | 3,5-Diethyl-Phenyl | H | Methyl | |
| 1.1.672 | 3-Propyl-5-ethyl-Phenyl | H | Methyl | |
| 1.1.673 | 3-isoPropyl-5-ethyl-Phenyl | H | Methyl | |
| 1.1.674 | 3-nButyl-5-ethyl-Phenyl | H | Methyl | |
| 1.1.675 | 3-isoButyl-5-ethyl-Phenyl | H | Methyl | |
| 1.1.676 | 3-tert.Butyl-5-ethyl-Phenyl | H | Methyl | |
| 1.1.677 | 3-Cyclopropyl-5-ethyl-Phenyl | H | Methyl | |
| 1.1.678 | 3-Vinyl-5-ethyl-Phenyl | H | Methyl | |
| 1.1.679 | 3-Ethinyl-5-ethyl-Phenyl | H | Methyl | |
| 1.1.680 | 3-Cyano-5-ethyl-Phenyl | H | Methyl | |
| 1.1.681 | 3-Trifluormethyl-5-ethyl-Phenyl | H | Methyl | |
| 1.1.682 | 3-(Hydroxycarbonyl)-5-ethyl-Phenyl | H | Methyl | |
| 1.1.683 | 3-(Methoxycarbony)l-5-ethyl-Phenyl | H | Methyl | |
| 1.1.684 | 3-Hydroxymethyl-5-ethyl-Phenyl | H | Methyl | |
| 1.1.685 | 3-Carbamoyl-5-ethyl-Phenyl | H | Methyl | |
| 1.1.686 | 3-Hydroxy-5-ethyl-Phenyl- | H | Methyl | |
| 1.1.687 | 3-Methoxy-5-ethyl-Phenyl | H | Methyl | |
| 1.1.688 | 3-Ethoxy-5-ethyl-Phenyl | H | Methyl | |
| 1.1.689 | 3-nPropyoxy-5-ethyl-Phenyl | H | Methyl | |
| 1.1.690 | 3-nButoxy-5-ethyl-Phenyl | H | Methyl | |
| 1.1.691 | 3-isoButoxy-5-ethyl-Phenyl | H | Methyl | |
| 1.1.692 | 3-tert.Butoxy-5-ethyl-Phenyl | H | Methyl | |
| 1.1.693 | 3-Difluormethoxy-5-ethyl-Phenyl | H | Methyl | |
| 1.1.694 | 3-Trifluormethoxy-5-ethyl-Phenyl | H | Methyl | |
| 1.1.695 | 3-(2,2,2-Trifluorethoxy)-5-ethyl-Phenyl | H | Methyl | |
| 1.1.696 | 3-(2-Chlorethoxy)-5-ethyl-Phenyl | H | Methyl | |
| 1.1.697 | 3-(2-Hydroxyethoxy)-5-ethyl-Phenyl- | H | Methyl | |
| 1.1.698 | 3-[(tert-Butoxycarbonyl)oxy]-5-ethyl-Phenyl | H | Methyl | |
| 1.1.699 | 3-Nitro-5-ethyl-Phenyl | H | Methyl | |
| 1.1.700 | 3-Acetoxy-5-ethyl-Phenyl | H | Methyl | |
| 1.1.701 | {3-[(tert-Butoxycarbonyl)amino]-5-ethyl-Phenyl} | H | Methyl | |
| 1.1.702 | 3-Methylsulfanyl-5-ethyl-Phenyl | H | Methyl | |
| 1.1.703 | 3,5-Dipropyl-Phenyl | H | Methyl | |
| 1.1.704 | 3-isoPropyl-5-propyl-Phenyl | H | Methyl | |
| 1.1.705 | 3-nButyl-5-propyl-Phenyl | H | Methyl | |
| 1.1.706 | 3-isoButyl-5-propyl-Phenyl | H | Methyl | |
| 1.1.707 | 3-tert.Butyl-5-propyl-Phenyl | H | Methyl | |
| 1.1.708 | 3-Cyclopropyl-5-propyl-Phenyl | H | Methyl | |
| 1.1.709 | 3-Vinyl-5-propyl-Phenyl | H | Methyl | |
| 1.1.710 | 3-Ethinyl-5-propyl-Phenyl | H | Methyl | |
| 1.1.711 | 3-Cyano-5-propyl-Phenyl | H | Methyl | |
| 1.1.712 | 3-Trifluormethyl-5-propyl-Phenyl | H | Methyl | |
| 1.1.713 | 3-(Hydroxycarbonyl)-5-propyl-Phenyl | H | Methyl | |
| 1.1.714 | 3-(Methoxycarbonyl)-5-propyl-Phenyl | H | Methyl | |
| 1.1.715 | 3-Hydroxymethyl-5-propyl-Phenyl | H | Methyl | |
| 1.1.716 | 3-Carbamoyl-5-propyl-Phenyl | H | Methyl | |
| 1.1.717 | 3-Hydroxy-5-propyl-Phenyl- | H | Methyl | |
| 1.1.718 | 3-Methoxy-5-propyl-Phenyl | H | Methyl | |
| 1.1.719 | 3-Ethoxy-5-propyl-Phenyl | H | Methyl | |
| 1.1.720 | 3-nPropyoxy-5-propyl-Phenyl | H | Methyl | |
| 1.1.721 | 3-nButoxy-5-propyl-Phenyl | H | Methyl | |
| 1.1.722 | 3-isoButoxy-5-propyl-Phenyl | H | Methyl | |
| 1.1.723 | 3-tert.Butoxy-5-propyl-Phenyl | H | Methyl | |
| 1.1.724 | 3-Difluormethoxy-5-propyl-Phenyl | H | Methyl | |
| 1.1.725 | 3-Trifluormethoxy-5-ethyl-Phenyl | H | Methyl | |
| 1.1.726 | 3-(2,2,2-Trifluorethoxy)-5-propyl-Phenyl | H | Methyl | |
| 1.1.727 | 3-(2-Chlorethoxy)-5-propyl-Phenyl | H | Methyl | |
| 1.1.728 | 3-(2-Hydroxyethoxy)-5-propyl-Phenyl- | H | Methyl | |
| 1.1.729 | 3-[(tert-Butoxycarbonyl)oxy]-5-propyl-Phenyl | H | Methyl | |
| 1.1.730 | 3-Nitro-5-propyl-Phenyl | H | Methyl | |
| 1.1.731 | 3-Acetoxy-5-propyl-Phenyl | H | Methyl | |
| 1.1.732 | {3-[(tert-Butoxycarbonyl)amino]-5-propyl-Phenyl} | H | Methyl | |
| 1.1.733 | 3-Methylsulfanyl-5-propyl-Phenyl | H | Methyl | |
| 1.1.734 | 3,5-DiisopropylPhenyl | H | Methyl | |
| 1.1.735 | 3-nButyl-5-isopropyl-Phenyl | H | Methyl | |
| 1.1.736 | 3-isoButyl-5-isopropyl-Phenyl | H | Methyl | |
| 1.1.737 | 3-tert.Butyl-5-isopropyl-Phenyl | H | Methyl | |
| 1.1.738 | 3-Cyclopropyl-5-isopropyl-Phenyl | H | Methyl | |
| 1.1.739 | 3-Vinyl-5-isopropyl-Phenyl | H | Methyl | |
| 1.1.740 | 3-Ethinyl-5-isopropyl-Phenyl | H | Methyl | |
| 1.1.741 | 3-Cyano-5-isopropyl-Phenyl | H | Methyl | |
| 1.1.742 | 3-Trifluormethyl-5-isopropyl-Phenyl | H | Methyl | |
| 1.1.743 | 3-(Hydroxycarbonyl)-5-isopropyl-Phenyl | H | Methyl | |
| 1.1.744 | 3-(Methoxycarbony)l-5-isopropyl-Phenyl | H | Methyl | |
| 1.1.745 | 3-Hydroxymethyl-5-isopropyl-Phenyl | H | Methyl | |
| 1.1.746 | 3-Carbamoyl-5-isopropyl-Phenyl | H | Methyl | |
| 1.1.747 | 3-Hydroxy-5-isopropyl-Phenyl- | H | Methyl | |
| 1.1.748 | 3-Methoxy-5-isopropyl-Phenyl | H | Methyl | |
| 1.1.749 | 3-Ethoxy-5-isopropyl-Phenyl | H | Methyl | |
| 1.1.750 | 3-nPropyoxy-5-isopropyl-Phenyl | H | Methyl | |
| 1.1.751 | 3-nButoxy-5-isopropyl-Phenyl | H | Methyl | |
| 1.1.752 | 3-isoButoxy-5-isopropyl-Phenyl | H | Methyl | |
| 1.1.753 | 3-tert.Butoxy-5-isopropyl-Phenyl | H | Methyl | |
| 1.1.754 | 3-Difluormethoxy-5-isopropyl-Phenyl | H | Methyl | |
| 1.1.755 | 3-Trifluormethoxy-5-isopropyl-Phenyl | H | Methyl | |
| 1.1.756 | 3-(2,2,2-Trifluorethoxy)-5-isopropyl-Phenyl | H | Methyl | |
| 1.1.757 | 3-(2-Chlorethoxy)-5-isopropyl-Phenyl | H | Methyl | |
| 1.1.758 | 3-(2-Hydroxyethoxy)-5-propyl-Phenyl- | H | Methyl | |
| 1.1.759 | 3-[(tert-Butoxycarbonyl)oxy]-5-isopropyl-Phenyl | H | Methyl | |
| 1.1.760 | 3-Nitro-5-isopropyl-Phenyl | H | Methyl | |
| 1.1.761 | 3-Acetoxy-5-isopropyl-Phenyl | H | Methyl | |
| 1.1.762 | {3-[(tert-Butoxycarbonyl)amino]-5-isopropyl-Phenyl} | H | Methyl | |
| 1.1.763 | 3-Methylsulfanyl-5-isopropyl-Phenyl | H | Methyl | |
| 1.1.764 | 3,5-Dibutyl-Phenyl | H | Methyl | |
| 1.1.765 | 3-isoButyl-5-butyl-Phenyl | H | Methyl | |
| 1.1.766 | 3-tert.Butyl-5-butyl-Phenyl | H | Methyl | |
| 1.1.767 | 3-Cyclopropyl-5-butyl-Phenyl | H | Methyl | |
| 1.1.768 | 3-Vinyl-5-butyl-Phenyl | H | Methyl | |
| 1.1.769 | 3-Ethinyl-5-butyl-Phenyl | H | Methyl | |
| 1.1.770 | 3-Cyano-5-butyl-Phenyl | H | Methyl | |
| 1.1.771 | 3-Trifluormethyl-5-butyl-Phenyl | H | Methyl | |
| 1.1.772 | 3-(Hydroxycarbonyl)-5-butyl-Phenyl | H | Methyl | |
| 1.1.773 | 3-(Methoxycarbony)l-5-butyl-Phenyl | H | Methyl | |
| 1.1.774 | 3-Hydroxymethyl-5-butyl-Phenyl | H | Methyl | |
| 1.1.775 | 3-Carbamoyl-5-butyl-Phenyl | H | Methyl | |
| 1.1.776 | 3-Hydroxy-5-butyl-Phenyl- | H | Methyl | |
| 1.1.777 | 3-Methoxy-5-butyl-Phenyl | H | Methyl | |
| 1.1.778 | 3-Ethoxy-5-butyl-Phenyl | H | Methyl | |
| 1.1.779 | 3-nPropyoxy-5-butyl-Phenyl | H | Methyl | |
| 1.1.780 | 3-nButoxy-5-butyl-Phenyl | H | Methyl | |
| 1.1.781 | 3-isoButoxy-5-butyl-Phenyl | H | Methyl | |
| 1.1.782 | 3-tert. Butoxy-5-butyl-Phenyl | H | Methyl | |
| 1.1.783 | 3-Difluormethoxy-5-butyl-Phenyl | H | Methyl | |
| 1.1.784 | 3-Trifluormethoxy-5-butyl-Phenyl | H | Methyl | |
| 1.1.785 | 3-(2,2,2-Trifluorethoxy)-5-butyl-Phenyl | H | Methyl | |
| 1.1.786 | 3-(2-Chlorethoxy)-5-butyl-Phenyl | H | Methyl | |
| 1.1.787 | 3-(2-Hydroxyethoxy)-5-butyl-Phenyl- | H | Methyl | |
| 1.1.788 | 3-[(tert-Butoxycarbonyl)oxy]-5-butyl-Phenyl | H | Methyl | |
| 1.1.789 | 3-Nitro-5-butyl-Phenyl | H | Methyl | |
| 1.1.790 | 3-Acetoxy-5-butyl-Phenyl | H | Methyl | |
| 1.1.791 | {3-[(tert-Butoxycarbonyl)amino]-5-butyl-Phenyl} | H | Methyl | |
| 1.1.792 | 3-Methylsulfanyl-5-butyl-Phenyl | H | Methyl | |
| 1.1.793 | 3,5-Diisobutyl-Phenyl | H | Methyl | |
| 1.1.794 | 3-tert.Butyl-5-isobutyl-Phenyl | H | Methyl | |
| 1.1.795 | 3-Cyclopropyl-5-isobutyl-Phenyl | H | Methyl | |
| 1.1.796 | 3-Vinyl-5-isobutyl-Phenyl | H | Methyl | |
| 1.1.797 | 3-Ethinyl-5-isobutyl-Phenyl | H | Methyl | |
| 1.1.798 | 3-Cyano-5-isobutyl-Phenyl | H | Methyl | |
| 1.1.799 | 3-Trifluormethyl-5-isobutyl-Phenyl | H | Methyl | |
| 1.1.800 | 3-(Hydroxycarbonyl)-5-isobutyl-Phenyl | H | Methyl | |
| 1.1.801 | 3-(Methoxycarbonyl)-5-isobutyl-Phenyl | H | Methyl | |
| 1.1.802 | 3-Hydroxymethyl-5-isobutyl-Phenyl | H | Methyl | |
| 1.1.803 | 3-Carbamoyl-5-isobutyl-Phenyl | H | Methyl | |
| 1.1.804 | 3-Hydroxy-5-isobutyl-Phenyl- | H | Methyl | |
| 1.1.805 | 3-Methoxy-5-isobutyl-Phenyl | H | Methyl | |
| 1.1.806 | 3-Ethoxy-5-isobutyl-Phenyl | H | Methyl | |
| 1.1.807 | 3-nPropyoxy-5-isobutyl-Phenyl | H | Methyl | |
| 1.1.808 | 3-nButoxy-5-isobutyl-Phenyl | H | Methyl | |
| 1.1.809 | 3-isoButoxy-5-isobutyl-Phenyl | H | Methyl | |
| 1.1.810 | 3-tert.Butoxy-5-isobutyl-Phenyl | H | Methyl | |
| 1.1.811 | 3-Difluormethoxy-5-isobutyl-Phenyl | H | Methyl | |
| 1.1.812 | 3-Trifluormethoxy-5-isobutyl-Phenyl | H | Methyl | |
| 1.1.813 | 3-(2,2,2-Trifluorethoxy)-5-isobutyl-Phenyl | H | Methyl | |
| 1.1.814 | 3-(2-Chlorethoxy)-5-isobutyl-Phenyl | H | Methyl | |
| 1.1.815 | 3-(2-Hydroxyethoxy)-5-isobutyl-Phenyl- | H | Methyl | |
| 1.1.816 | 3-[(tert-Butoxycarbonyl)oxy]-5-isobutyl-Phenyl | H | Methyl | |
| 1.1.817 | 3-Nitro-5-isobutyl-Phenyl | H | Methyl | |
| 1.1.818 | 3-Acetoxy-5-isobutyl-Phenyl | H | Methyl | |
| 1.1.819 | {3-[(tert-Butoxycarbonyl)amino]-5-isobutyl-Phenyl} | H | Methyl | |
| 1.1.820 | 3-Methylsulfanyl-5-isobutyl-Phenyl | H | Methyl | |
| 1.1.821 | 3,5-D(itert.butyl)-Phenyl | H | Methyl | [DMSO] 1.30 (s, 9H); 1.56 (s, 3H); 3.41 (d, 1H); 3.82 (d, 1H); 7.48 (s, 2H); 7.5 (s, 1H). |
| 1.1.822 | 3-Cyclopropyl-5-tert.butyl-Phenyl | H | Methyl | |
| 1.1.823 | 3-Vinyl-5-tert.butyl-Phenyl | H | Methyl | |
| 1.1.824 | 3-Ethinyl-5-tert.butyl-Phenyl | H | Methyl | |
| 1.1.825 | 3-Cyano-5-tert.butyl-Phenyl | H | Methyl | |
| 1.1.826 | 3-Trifluormethyl-5-tert.butyl-Phenyl | H | Methyl | |
| 1.1.827 | 3-(Hydroxycarbonyl)-5-tert.butyl-Phenyl | H | Methyl | |
| 1.1.828 | 3-(Methoxycarbonyl)-5-tert.butyl-Phenyl | H | Methyl | |
| 1.1.829 | 3-Hydroxymethyl-5-tert.butyl-Phenyl | H | Methyl | |
| 1.1.830 | 3-Carbamoyl-5-tert.butyl-Phenyl | H | Methyl | |
| 1.1.831 | 3-Hydroxy-5-tert.butyl-Phenyl- | H | Methyl | |
| 1.1.832 | 3-Methoxy-5-tert.butyl-Phenyl | H | Methyl | |
| 1.1.833 | 3-Ethoxy-5-tert.butyl-Phenyl | H | Methyl | |
| 1.1.834 | 3-nPropyoxy-5-tert.butyl-Phenyl | H | Methyl | |
| 1.1.835 | 3-nButoxy-5-tert.butyl-Phenyl | H | Methyl | |
| 1.1.836 | 3-isoButoxy-5-tert.butyl-Phenyl | H | Methyl | |
| 1.1.837 | 3-tert.Butoxy-5-tert.butyl-Phenyl | H | Methyl | |
| 1.1.838 | 3-Difluormethoxy-5-tert.butyl-Phenyl | H | Methyl | |
| 1.1.839 | 3-Trifluormethoxy-5-tert.butyl-Phenyl | H | Methyl | |
| 1.1.840 | 3-(2,2,2-Trifluorethoxy)-5-tert.butyl-Phenyl | H | Methyl | |
| 1.1.841 | 3-(2-Chlorethoxy)-5-tert.butyl-Phenyl | H | Methyl | |
| 1.1.842 | 3-(2-Hydroxyethoxy)-5-tert.butyl-Phenyl- | H | Methyl | |
| 1.1.843 | 3-[(tert-Butoxycarbonyl)oxy]-5-tert.butyl-Phenyl | H | Methyl | |
| 1.1.844 | 3-Nitro-5-tert.butyl-Phenyl | H | Methyl | |
| 1.1.845 | 3-Acetoxy-5-tert.butyl-Phenyl | H | Methyl | |
| 1.1.846 | {3-[(tert-Butoxycarbonyl)amino]-5-tert.butyl-Phenyl} | H | Methyl | |
| 1.1.847 | 3-Methylsulfanyl-5-tert.butyl-Phenyl | H | Methyl | |
| 1.1.848 | 3-tert.Butyl-5-cyclopropyl-Phenyl | H | Methyl | |
| 1.1.849 | 3,5-Dicyclopropyl-Phenyl | H | Methyl | |
| 1.1.850 | 3-Vinyl-5-cyclopropyl-Phenyl | H | Methyl | |
| 1.1.851 | 3-Ethinyl-5-cyclopropyl-Phenyl | H | Methyl | |
| 1.1.852 | 3-Cyano-5-cyclopropyl-Phenyl | H | Methyl | |
| 1.1.853 | 3-Trifluormethyl-5-cyclopropyl-Phenyl | H | Methyl | |
| 1.1.854 | 3-(Hydroxycarbonyl)-5-cyclopropyl-Phenyl | H | Methyl | |
| 1.1.855 | 3-(Methoxycarbonyl)-5-cyclopropyl-Phenyl | H | Methyl | |
| 1.1.856 | 3-Hydroxymethyl-5-cyclopropyl-Phenyl | H | Methyl | |
| 1.1.857 | 3-Carbamoyl-5-cyclopropyl-Phenyl | H | Methyl | |
| 1.1.858 | 3-Hydroxy-5-cyclopropyl-Phenyl- | H | Methyl | |
| 1.1.859 | 3-Methoxy-5-cyclopropyl-Phenyl | H | Methyl | |
| 1.1.860 | 3-Ethoxy-5-cyclopropyl-Phenyl | H | Methyl | |
| 1.1.861 | 3-nPropyoxy-5-cyclopropyl-Phenyl | H | Methyl | |
| 1.1.862 | 3-nButoxy-5-cyclopropyl-Phenyl | H | Methyl | |
| 1.1.863 | 3-isoButoxy-5-cyclopropyl-Phenyl | H | Methyl | |
| 1.1.864 | 3-tert.Butoxy-5-cyclopropyl-Phenyl | H | Methyl | |
| 1.1.865 | 3-Difluormethoxy-5-cyclopropyl-Phenyl | H | Methyl | |
| 1.1.866 | 3-Trifluormethoxy-5-cyclopropyl-Phenyl | H | Methyl | |
| 1.1.867 | 3-(2,2,2-Trifluorethoxy)-5-cyclopropyl-Phenyl | H | Methyl | |
| 1.1.868 | 3-(2-Chlorethoxy)-5-cyclopropyl-Phenyl | H | Methyl | |
| 1.1.869 | 3-(2-Hydroxyethoxy)-5-cyclopropyl-Phenyl- | H | Methyl | |
| 1.1.870 | 3-[(tert-Butoxycarbonyl)oxy]-5-cyclopropyl-Phenyl | H | Methyl | |
| 1.1.871 | 3-Nitro-5-cyclopropyl-Phenyl | H | Methyl | |
| 1.1.872 | 3-Acetoxy-5-cyclopropyl-Phenyl | H | Methyl | |
| 1.1.873 | {3-[(tert-Butoxycarbonyl)amino]-5-cyclopropyl-Phenyl} | H | Methyl | |
| 1.1.874 | 3-Methylsulfanyl-5-cyclopropyl-Phenyl | H | Methyl | |
| 1.1.875 | 3,5-Divinyl-Phenyl | H | Methyl | |
| 1.1.876 | 3-Ethinyl-5-vinyl-Phenyl | H | Methyl | |
| 1.1.877 | 3-Cyano-5-vinyl-Phenyl | H | Methyl | |
| 1.1.878 | 3-Trifluormethyl-5-vinyl-Phenyl | H | Methyl | |
| 1.1.879 | 3-(Hydroxycarbonyl)-5-vinyl-Phenyl | H | Methyl | |
| 1.1.880 | 3-(Methoxycarbonyl)-5-vinyl-Phenyl | H | Methyl | |
| 1.1.881 | 3-Hydroxymethyl-5-vinyl-Phenyl | H | Methyl | |
| 1.1.882 | 3-Carbamoyl-5-vinyl-Phenyl | H | Methyl | |
| 1.1.883 | 3-Hydroxy-5-vinyl-Phenyl- | H | Methyl | |
| 1.1.884 | 3-Methoxy-5-vinyl-Phenyl | H | Methyl | |
| 1.1.885 | 3-Ethoxy-5-vinyl-Phenyl | H | Methyl | |
| 1.1.886 | 3-nPropyoxy-5-vinyl-Phenyl | H | Methyl | |
| 1.1.887 | 3-nButoxy-5-vinyl-Phenyl | H | Methyl | |
| 1.1.888 | 3-isoButoxy-5-vinyl-Phenyl | H | Methyl | |
| 1.1.889 | 3-tert.Butoxy-5-vinyl-Phenyl | H | Methyl | |
| 1.1.890 | 3-Difluormethoxy-5-vinyl-Phenyl | H | Methyl | |
| 1.1.891 | 3-Trifluormethoxy-5-vinyl-Phenyl | H | Methyl | |
| 1.1.892 | 3-(2,2,2-Trifluorethoxy)-5-vinyl-Phenyl | H | Methyl | |
| 1.1.893 | 3-(2-Chlorethoxy)-5-vinyl-Phenyl | H | Methyl | |
| 1.1.894 | 3-(2-Hydroxyethoxy)-5-vinyl-Phenyl- | H | Methyl | |
| 1.1.895 | 3-[(tert-Butoxycarbonyl)oxy]-5-vinyl-Phenyl | H | Methyl | |
| 1.1.896 | 3-Nitro-5-vinyl-Phenyl | H | Methyl | |
| 1.1.897 | 3-Acetoxy-5-vinyl-Phenyl | H | Methyl | |
| 1.1.898 | {3-[(tert-Butoxycarbonyl)amino]-5-vinyl-Phenyl} | H | Methyl | |
| 1.1.899 | 3-Methylsulfanyl-5-vinyl-Phenyl | H | Methyl | |
| 1.1.900 | 3,5-Diethinyl-Phenyl | H | Methyl | |
| 1.1.901 | 3-Cyano-5-ethinyl-Phenyl | H | Methyl | |
| 1.1.902 | 3-Trifluormethyl-5-ethinyl-Phenyl | H | Methyl | |
| 1.1.903 | 3-(Hydroxycarbonyl)-5-ethinyl-Phenyl | H | Methyl | |
| 1.1.904 | 3-(Methoxycarbonyl)-5-ethinyl-Phenyl | H | Methyl | |
| 1.1.905 | 3-Hydroxymethyl-5-ethinyl-Phenyl | H | Methyl | |
| 1.1.906 | 3-Carbamoyl-5-ethinyl-Phenyl | H | Methyl | |
| 1.1.907 | 3-Hydroxy-5-ethinyl-Phenyl- | H | Methyl | |
| 1.1.908 | 3-Methoxy-5-ethinyl-Phenyl | H | Methyl | |
| 1.1.909 | 3-Ethoxy-5-ethinyl-Phenyl | H | Methyl | |
| 1.1.910 | 3-nPropyoxy-5-ethinyl-Phenyl | H | Methyl | |
| 1.1.911 | 3-nButoxy-5-ethinyl-Phenyl | H | Methyl | |
| 1.1.912 | 3-isoButoxy-5-ethinyl-Phenyl | H | Methyl | |
| 1.1.913 | 3-tert.Butoxy-5-ethinyl-Phenyl | H | Methyl | |
| 1.1.914 | 3-Difluormethoxy-5-ethinyl-Phenyl | H | Methyl | |
| 1.1.915 | 3-Trifluormethoxy-5-ethinyl-Phenyl | H | Methyl | |
| 1.1.916 | 3-(2,2,2-Trifluorethoxy)-5-ethinyl-Phenyl | H | Methyl | |
| 1.1.917 | 3-(2-Chlorethoxy)-5-ethinyl-Phenyl | H | Methyl | |
| 1.1.918 | 3-(2-Hydroxyethoxy)-5-ethinyl-Phenyl- | H | Methyl | |
| 1.1.919 | 3-[(tert-Butoxycarbonyl)oxy]-5-ethinyl-Phenyl | H | Methyl | |
| 1.1.920 | 3-Nitro-5-ethinyl-Phenyl | H | Methyl | |
| 1.1.921 | 3-Acetoxy-5-ethinyl-Phenyl | H | Methyl | |
| 1.1.922 | {3-[(tert-Butoxycarbonyl)amino]-5-ethinyl-Phenyl} | H | Methyl | |
| 1.1.923 | 3-Methylsulfanyl-5-ethinyl-Phenyl | H | Methyl | |
| 1.1.924 | 3,5-Dicyano-Phenyl | H | Methyl | [CDCl₃] 1.80 (s, 3H); 3.28 (d, 1H); 3.90 (d, 1H); 7.98 (s, 1H); 8.15 (s, 2H). |
| 1.1.925 | 3-Trifluormethyl-5-cyano-Phenyl | H | Methyl | [CDCl₃] 1.81 (s, 3H); 3.30 (d, 1H); 3.91 (d, 1H); 7.92 (s, 1H); 8.11 (d, 2H). |
| 1.1.926 | 3-(Hydroxycarbonyl)-5-cyano-Phenyl | H | Methyl | |
| 1.1.927 | 3-(Methoxycarbonyl)-5-cyano-Phenyl | H | Methyl | |
| 1.1.928 | 3-Hydroxymethyl-5-cyano-Phenyl | H | Methyl | |
| 1.1.929 | 3-Carbamoyl-5-cyano-Phenyl | H | Methyl | |
| 1.1.930 | 3-Hydroxy-5-cyano-Phenyl- | H | Methyl | |
| 1.1.931 | 3-Methoxy-5-cyano-Phenyl | H | Methyl | |
| 1.1.932 | 3-Ethoxy-5-cyano-Phenyl | H | Methyl | |
| 1.1.933 | 3-nPropyoxy-5-cyano-Phenyl | H | Methyl | |
| 1.1.934 | 3-nButoxy-5-cyano-Phenyl | H | Methyl | |
| 1.1.935 | 3-isoButoxy-5-cyano-Phenyl | H | Methyl | |
| 1.1.936 | 3-tert. Butoxy-5-cyano-Phenyl | H | Methyl | |
| 1.1.937 | 3-Difluormethoxy-5-cyano-Phenyl | H | Methyl | |
| 1.1.938 | 3-Trifluormethoxy-5-cyano-Phenyl | H | Methyl | |
| 1.1.939 | 3-(2,2,2-Trifluorethoxy)-5-cyano-Phenyl | H | Methyl | |
| 1.1.940 | 3-(2-Chlorethoxy)-5-cyano-Phenyl | H | Methyl | |
| 1.1.941 | 3-(2-Hydroxyethoxy)-5-cyano-Phenyl- | H | Methyl | |
| 1.1.942 | 3-[(tert-Butoxycarbonyl)oxy]-5-cyano-Phenyl | H | Methyl | |
| 1.1.943 | 3-Nitro-5-cyano-Phenyl | H | Methyl | |
| 1.1.944 | 3-Acetoxy-5-cyano-Phenyl | H | Methyl | |
| 1.1.945 | {3-[(tert-Butoxycarbonyl)amino]-5-cyano-Phenyl} | H | Methyl | |
| 1.1.946 | 3-Methylsulfanyl-5-cyano-Phenyl | H | Methyl | |
| 1.1.947 | 3,5-Di(trifluormethyl)-Phenyl | H | Methyl | [CDCl₃] 1.81 (s, 3H); 3.35 (d, 1H); 3.95 (d, 1H); 7.92 (s, 1H); 8.08 (s, 2H). |
| 1.1.948 | 3-(Hydroxycarbonyl)-5-trifluormethyl-Phenyl | H | Methyl | |
| 1.1.949 | 3-(Methoxycarbonyl)-5-trifluormethyl-Phenyl | H | Methyl | |
| 1.1.950 | 3-Hydroxymethyl-5-trifluormethyl-Phenyl | H | Methyl | |
| 1.1.951 | 3-Carbamoyl-5-trifluormethyl-Phenyl | H | Methyl | |
| 1.1.952 | 3-Hydroxy-5-trifluormethyl-Phenyl- | H | Methyl | |
| 1.1.953 | 3-Methoxy-5-trifluormethyl-Phenyl | H | Methyl | |
| 1.1.954 | 3-Ethoxy-5trifluormethyl-Phenyl | H | Methyl | |
| 1.1.955 | 3-nPropyoxy-5-trifluormethyl-Phenyl | H | Methyl | |
| 1.1.956 | 3-nButoxy-5-trifluormethyl-Phenyl | H | Methyl | |
| 1.1.957 | 3-isoButoxy-5-trifluormethyl-Phenyl | H | Methyl | |
| 1.1.958 | 3-tert.Butoxy-5-trifluormethyl-Phenyl | H | Methyl | |
| 1.1.959 | 3-Difluormethoxy-5-trifluormethyl-Phenyl | H | Methyl | |
| 1.1.960 | 3-Trifluormethoxy-5-trifluormethyl-Phenyl | H | Methyl | |
| 1.1.961 | 3-(2,2,2-Trifluorethoxy)-5-trifluormethyl-Phenyl | H | Methyl | |
| 1.1.962 | 3-(2-Chlorethoxy)-5-trifluormethyl-Phenyl | H | Methyl | |
| 1.1.963 | 3-(2-Hydroxyethoxy)-5-trifluormethyl-Phenyl- | H | Methyl | |
| 1.1.964 | 3-[(tert-Butoxycarbonyl)oxy]-5-trifluormethyl-Phenyl | H | Methyl | |
| 1.1.965 | 3-Nitro-5-trifluormethyl-Phenyl | H | Methyl | |
| 1.1.966 | 3-Acetoxy-5-trifluormethyl-Phenyl | H | Methyl | |
| 1.1.967 | {3-[(tert-Butoxycarbonyl)amino]-5-trifluormethyl-Phenyl} | H | Methyl | |
| 1.1.968 | 3-Methylsulfanyl-5-trifluormethyl-Phenyl | H | Methyl | |
| 1.1.969 | 3,5-Bis(hydroxycarbonyl)-Phenyl | H | Methyl | |
| 1.1.970 | 3-(Methoxycarbonyl)-5-(hydroxycarbonyl)-Phenyl | H | Methyl | |
| 1.1.971 | 3-Hydroxymethyl-5-(hydroxycarbonyl)-Phenyl | H | Methyl | |
| 1.1.972 | 3-Carbamoyl-5-(hydroxycarbonyl)-Phenyl | H | Methyl | |
| 1.1.973 | 3-Hydroxy-5-(hydroxycarbonyl)-Phenyl- | H | Methyl | |
| 1.1.974 | 3-Methoxy-5-(hydroxycarbonyl)-Phenyl | H | Methyl | |
| 1.1.975 | 3-Ethoxy-5-(hydroxycarbonyl)-Phenyl | H | Methyl | |
| 1.1.976 | 3-nPropyoxy-5-(hydroxycarbonyl)-Phenyl | H | Methyl | |
| 1.1.977 | 3-nButoxy-5-(hydroxycarbonyl)-Phenyl | H | Methyl | |
| 1.1.978 | 3-isoButoxy-5-(hydroxycarbonyl)-Phenyl | H | Methyl | |
| 1.1.979 | 3-tert.Butoxy-5-(hydroxycarbonyl)-Phenyl | H | Methyl | |
| 1.1.980 | 3-Difluormethoxy-5-(hydroxycarbonyl)-Phenyl | H | Methyl | |
| 1.1.981 | 3-Trifluormethoxy-5-(hydroxycarbonyl)-Phenyl | H | Methyl | |
| 1.1.982 | 3-(2,2,2-Trifluorethoxy)-5-(hydroxycarbonyl)-Phenyl | H | Methyl | |
| 1.1.983 | 3-(2-Chlorethoxy)-5-(hydroxycarbonyl)-Phenyl | H | Methyl | |
| 1.1.984 | 3-(2-Hydroxyethoxy)-5-(hydroxycarbonyl)-Phenyl- | H | Methyl | |
| 1.1.985 | 3-[(tert-Butoxycarbonyl)oxy]-5-(hydroxycarbonyl)-Phenyl | H | Methyl | |
| 1.1.986 | 3-Nitro-5-(hydroxycarbonyl)-Phenyl | H | Methyl | |
| 1.1.987 | 3-Acetoxy-5-(hydroxycarbonyl)-Phenyl | H | Methyl | |
| 1.1.988 | {3-[(tert-Butoxycarbonyl)amino]-5-(hydroxycarbonyl)-Phenyl} | H | Methyl | |
| 1.1.989 | 3-Methylsulfanyl-5-(hydroxycarbonyl)-Phenyl | H | Methyl | |
| 1.1.990 | 3,5-Di(methoxycarbonyl)-Phenyl | H | Methyl | |
| 1.1.991 | 3-Hydroxymethyl-5-(methoxycarbonyl)-Phenyl | H | Methyl | |
| 1.1.992 | 3-Carbamoyl-5-(methoxycarbonyl)-Phenyl | H | Methyl | |
| 1.1.993 | 3-Hydroxy-5-(methoxycarbonyl)-Phenyl- | H | Methyl | |
| 1.1.994 | 3-Methoxy-5-(methoxycarbonyl)-Phenyl | H | Methyl | |
| 1.1.995 | 3-Ethoxy-5-(methoxycarbonyl)-Phenyl | H | Methyl | |
| 1.1.996 | 3-nPropyoxy-5-(methoxycarbonyl)-Phenyl | H | Methyl | |
| 1.1.997 | 3-nButoxy-5-(methoxycarbonyl)-Phenyl | H | Methyl | |
| 1.1.998 | 3-isoButoxy-5-(methoxycarbonyl)-Phenyl | H | Methyl | |
| 1.1.999 | 3-tert.Butoxy-5-(methoxycarbonyl)-Phenyl | H | Methyl | |
| 1.1.1000 | 3-Difluormethoxy-5-(methoxycarbonyl)-Phenyl | H | Methyl | |
| 1.1.1001 | 3-Trifluormethoxy-5-(methoxycarbonyl)-Phenyl | H | Methyl | |
| 1.1.1002 | 3-(2,2,2-Trifluorethoxy)-5-(methoxycarbonyl)-Phenyl | H | Methyl | |
| 1.1.1003 | 3-(2-Chlorethoxy)-5-(methoxycarbonyl)-Phenyl | H | Methyl | |
| 1.1.1004 | 3-(2-Hydroxyethoxy)-5-(methoxycarbonyl)-Phenyl- | H | Methyl | |
| 1.1.1005 | 3-[(tert-Butoxycarbonyl)oxy]-5-(methoxycarbonyl)-Phenyl | H | Methyl | |
| 1.1.1006 | 3-Nitro-5-(methoxycarbonyl)-Phenyl | H | Methyl | |
| 1.1.1007 | 3-Acetoxy-5-(methoxycarbonyl)-Phenyl | H | Methyl | |
| 1.1.1008 | {3-[(tert-Butoxycarbonyl)amino]-5-(methoxycarbonyl)-Phenyl} | H | Methyl | |
| 1.1.1009 | 3-Methylsulfanyl-5-(methoxycarbonyl)-Phenyl | H | Methyl | |
| 1.1.1010 | 3,5-Di(hydroxymethy)l-Phenyl | H | Methyl | |
| 1.1.1011 | 3-Carbamoyl-5-hydroxymethyl-Phenyl | H | Methyl | |
| 1.1.1012 | 3-Hydroxy-5-hydroxymethyl-Phenyl- | H | Methyl | |
| 1.1.1013 | 3-Methoxy-5-hydroxymethyl-Phenyl | H | Methyl | |
| 1.1.1014 | 3-Ethoxy-5-hydroxymethyl-Phenyl | H | Methyl | |
| 1.1.1015 | 3-nPropyoxy-5-hydroxymethyl-Phenyl | H | Methyl | |
| 1.1.1016 | 3-nButoxy-5-hydroxymethyl-Phenyl | H | Methyl | |
| 1.1.1017 | 3-isoButoxy-5-hydroxymethyl-Phenyl | H | Methyl | |
| 1.1.1018 | 3-tert.Butoxy-5-hydroxymethyl-Phenyl | H | Methyl | |
| 1.1.1019 | 3-Difluormethoxy-5-hydroxymethyl-Phenyl | H | Methyl | |
| 1.1.1020 | 3-Trifluormethoxy-5-hydroxymethyl-Phenyl | H | Methyl | |
| 1.1.1021 | 3-(2,2,2-Trifluorethoxy)-5-hydroxymethyl-Phenyl | H | Methyl | |
| 1.1.1022 | 3-(2-Chlorethoxy)-5-hydroxymethyl-Phenyl | H | Methyl | |
| 1.1.1023 | 3-(2-Hydroxyethoxy)-5-hydroxymethyl-Phenyl | H | Methyl | |
| 1.1.1024 | 3-[(tert-Butoxycarbonyl)oxy]-5-hydroxymethyl-Phenyl | H | Methyl | |
| 1.1.1025 | 3-Nitro-5-hydroxymethyl-Phenyl | H | Methyl | |
| 1.1.1026 | 3-Acetoxy-5-hydroxymethyl-Phenyl | H | Methyl | |
| 1.1.1027 | {3-[(tert-Butoxycarbonyl)amino]-5-hydroxymethyl-Phenyl} | H | Methyl | |
| 1.1.1028 | 3-Methylsulfanyl-5-hydroxymethyl-Phenyl | H | Methyl | |
| 1.1.1029 | 3,5-Dicarbamoyl-5-carbamoyl-Phenyl | H | Methyl | |
| 1.1.1030 | 3-Hydroxy-5-carbamoyl-Phenyl- | H | Methyl | |
| 1.1.1031 | 3-Methoxy-5-carbamoyl-Phenyl | H | Methyl | |
| 1.1.1032 | 3-Ethoxy-5-carbamoyl-Phenyl | H | Methyl | |
| 1.1.1033 | 3-nPropyoxy-5-carbamoyl-Phenyl | H | Methyl | |
| 1.1.1034 | 3-nButoxy-5-carbamoyl-Phenyl | H | Methyl | |
| 1.1.1035 | 3-isoButoxy-5-carbamoyl-Phenyl | H | Methyl | |
| 1.1.1036 | 3-tert.Butoxy-5-carbamoyl-Phenyl | H | Methyl | |
| 1.1.1037 | 3-Difluormethoxy-5-carbamoyl-Phenyl | H | Methyl | |
| 1.1.1038 | 3-Trifluormethoxy-5-carbamoyl-Phenyl | H | Methyl | |
| 1.1.1039 | 3-(2,2,2-Trifluorethoxy)-5-carbamoyl-Phenyl | H | Methyl | |
| 1.1.1040 | 3-(2-Chlorethoxy)-5-carbamoyl-Phenyl | H | Methyl | |
| 1.1.1041 | 3-(2-Hydroxyethoxy)-5-carbamoyl-Phenyl- | H | Methyl | |
| 1.1.1042 | 3-[(tert-Butoxycarbonyl)oxy]-5-carbamoyl-Phenyl | H | Methyl | |
| 1.1.1043 | 3-Nitro-5-carbamoyl-Phenyl | H | Methyl | |
| 1.1.1044 | 3-Acetoxy-5-carbamoyl-Phenyl | H | Methyl | |
| 1.1.1045 | {3-[(tert-Butoxycarbonyl)amino]-5-carbamoyl-Phenyl} | H | Methyl | |
| 1.1.1046 | 3-Methylsulfanyl-5-carbamoyl-Phenyl | H | Methyl | |
| 1.1.1047 | 3,5-DihydroxyPhenyl | H | Methyl | |
| 1.1.1048 | 3-Methoxy-5-hydroxy-Phenyl | H | Methyl | |
| 1.1.1049 | 3-Ethoxy-5-hydroxyPhenyl | H | Methyl | |
| 1.1.1050 | 3-nPropyoxy-5-hydroxy-Phenyl | H | Methyl | |
| 1.1.1051 | 3-nButoxy-5-hydroxy-Phenyl | H | Methyl | |
| 1.1.1052 | 3-isoButoxy-5-hydroxy-Phenyl | H | Methyl | |
| 1.1.1053 | 3-tert.Butoxy-5-hydroxy-Phenyl | H | Methyl | |
| 1.1.1054 | 3-Difluormethoxy-5-hydroxy-Phenyl | H | Methyl | |
| 1.1.1055 | 3-Trifluormethoxy-5-hydroxy-Phenyl | H | Methyl | |
| 1.1.1056 | 3-(2,2,2-Trifluorethoxy)-5-hydroxy-Phenyl | H | Methyl | |
| 1.1.1057 | 3-(2-Chlorethoxy)-5-hydroxy-Phenyl | H | Methyl | |
| 1.1.1058 | 3-(2-Hydroxyethoxy)-5-hydroxy-Phenyl- | H | Methyl | |
| 1.1.1059 | 3-[(tert-Butoxycarbonyl)oxy]-5-hydroxy-Phenyl | H | Methyl | |
| 1.1.1060 | 3-Nitro-5-hydroxyPhenyl | H | Methyl | |
| 1.1.1061 | 3-Acetoxy-5-hydroxyPhenyl | H | Methyl | |
| 1.1.1062 | {3-[(tert-Butoxycarbonyl)amino]-5-hydroxy-Phenyl} | H | Methyl | |
| 1.1.1063 | 3-Methylsulfanyl-5-hydroxy-Phenyl | H | Methyl | |
| 1.1.1064 | 3,5-DimethoxyPhenyl | H | Methyl | |
| 1.1.1065 | 3-Ethoxy-5-methoxy-Phenyl | H | Methyl | |
| 1.1.1066 | 3-nPropyoxy-5-methoxy-Phenyl | H | Methyl | |
| 1.1.1067 | 3-nButoxy-5-methoxy-Phenyl | H | Methyl | |
| 1.1.1068 | 3-isoButoxy-5-methoxy-Phenyl | H | Methyl | |
| 1.1.1069 | 3-tert.Butoxy-5-methoxy-Phenyl | H | Methyl | |
| 1.1.1070 | 3-Difluormethoxy-5-methoxy-Phenyl | H | Methyl | |
| 1.1.1071 | 3-Trifluormethoxy-5-methoxy-Phenyl | H | Methyl | |
| 1.1.1072 | 3-(2,2,2-Trifluorethoxy)-5-methoxy-Phenyl | H | Methyl | |
| 1.1.1073 | 3-(2-Chlorethoxy)-5-methoxy-Phenyl | H | Methyl | |
| 1.1.1074 | 3-(2-Hydroxyethoxy)-5-methoxy-Phenyl- | H | Methyl | |
| 1.1.1075 | 3-[(tert-Butoxycarbonyl)oxy]-5-methoxy-Phenyl | H | Methyl | |
| 1.1.1076 | 3-Nitro-5-methoxy-Phenyl | H | Methyl | |
| 1.1.1077 | 3-Acetoxy-5-methoxy-Phenyl | H | Methyl | |
| 1.1.1078 | {3-[(tert-Butoxycarbonyl)amino]-5-methoxy-Phenyl} | H | Methyl | |
| 1.1.1079 | 3-Methylsulfanyl-5-methoxy-Phenyl | H | Methyl | |
| 1.1.1080 | 3,5-Diethoxy-Phenyl | H | Methyl | |
| 1.1.1081 | 3-nPropyoxy-5-ethoxy-Phenyl | H | Methyl | |
| 1.1.1082 | 3-nButoxy-5-ethoxy-Phenyl | H | Methyl | |
| 1.1.1083 | 3-isoButoxy-5-ethoxy-Phenyl | H | Methyl | |
| 1.1.1084 | 3-tert.Butoxy-5-ethoxy-Phenyl | H | Methyl | |
| 1.1.1085 | 3-Difluormethoxy-5-ethoxy-Phenyl | H | Methyl | |
| 1.1.1086 | 3-Trifluormethoxy-5-ethoxy-Phenyl | H | Methyl | |
| 1.1.1087 | 3-(2,2,2-Trifluorethoxy)-5-ethoxy-Phenyl | H | Methyl | |
| 1.1.1088 | 3-(2-Chlorethoxy)-5-ethoxy-Phenyl | H | Methyl | |
| 1.1.1089 | 3-(2-Hydroxyethoxy)-5-ethoxy-Phenyl- | H | Methyl | |
| 1.1.1090 | 3-[(tert-Butoxycarbonyl)oxy]-5-ethoxy-Phenyl | H | Methyl | |
| 1.1.1091 | 3-Nitro-5-ethoxy-Phenyl | H | Methyl | |
| 1.1.1092 | 3-Acetoxy-5-ethoxy-Phenyl | H | Methyl | |
| 1.1.1093 | {3-[(tert-Butoxycarbonyl)amino]-5-ethoxy-Phenyl} | H | Methyl | |
| 1.1.1094 | 3-Methylsulfanyl-5-ethoxy-Phenyl | H | Methyl | |
| 1.1.1095 | 3,5-Dipropyoxy-Phenyl | H | Methyl | |
| 1.1.1096 | 3-nButoxy-5-propoxy-Phenyl | H | Methyl | |
| 1.1.1097 | 3-isoButoxy-5-propoxy-Phenyl | H | Methyl | |
| 1.1.1098 | 3-tert.Butoxy-5-propoxy-Phenyl | H | Methyl | |
| 1.1.1099 | 3-Difluormethoxy-5-propoxy-Phenyl | H | Methyl | |
| 1.1.1100 | 3-Trifluormethoxy-5-propoxy-Phenyl | H | Methyl | |
| 1.1.1101 | 3-(2,2,2-Trifluorethoxy)-5-propoxy-Phenyl | H | Methyl | |
| 1.1.1102 | 3-(2-Chlorethoxy)-5-propoxy-Phenyl | H | Methyl | |
| 1.1.1103 | 3-(2-Hydroxyethoxy)-5-propoxy-Phenyl- | H | Methyl | |
| 1.1.1104 | 3-[(tert-Butoxycarbonyl)oxy]-5-propoxy-Phenyl | H | Methyl | |
| 1.1.1105 | 3-N itro-5-propoxy-Phenyl | H | Methyl | |
| 1.1.1106 | 3-Acetoxy-5-propoxy-Phenyl | H | Methyl | |
| 1.1.1107 | {3-[(tert-Butoxycarbonyl)amino]-5-propoxy-Phenyl} | H | Methyl | |
| 1.1.1108 | 3-Methylsulfanyl-5-propoxy-Phenyl | H | Methyl | |
| 1.1.1109 | 3,5-Di(isopropyoxy)-Phenyl | H | Methyl | |
| 1.1.1110 | 3-nButoxy-5-isopropoxy-Phenyl | H | Methyl | |
| 1.1.1111 | 3-isoButoxy-5-isopropoxy-Phenyl | H | Methyl | |
| 1.1.1112 | 3-tert.Butoxy-5-isopropoxy-Phenyl | H | Methyl | |
| 1.1.1113 | 3-Difluormethoxy-5-isopropoxy-Phenyl | H | Methyl | |
| 1.1.1114 | 3-Trifluormethoxy-5-isopropoxy-Phenyl | H | Methyl | |
| 1.1.1115 | 3-(2,2,2-Trifluorethoxy)-5-isopropoxy-Phenyl | H | Methyl | |
| 1.1.1116 | 3-(2-Chlorethoxy)-5-isopropoxy-Phenyl | H | Methyl | |
| 1.1.1117 | 3-(2-Hydroxyethoxy)-5-isopropoxy-Phenyl- | H | Methyl | |
| 1.1.1118 | 3-[(tert-Butoxycarbonyl)oxy]-5-isopropoxy-Phenyl | H | Methyl | |
| 1.1.1119 | 3-Nitro-5-isopropoxy-Phenyl | H | Methyl | |
| 1.1.1120 | 3-Acetoxy-5-isopropoxy-Phenyl | H | Methyl | |
| 1.1.1121 | {3-[(tert-Butoxycarbonyl)amino]-5-isopropoxy-Phenyl} | H | Methyl | |
| 1.1.1122 | 3-Methylsulfanyl-5-isopropoxy-Phenyl | H | Methyl | |
| 1.1.1123 | 3,5-Di(tert.butoxy)-Phenyl | H | Methyl | |
| 1.1.1124 | 3-Difluormethoxy-5-tert.butoxy-Phenyl | H | Methyl | |
| 1.1.1125 | 3-Trifluormethoxy-5-tert.butoxy-Phenyl | H | Methyl | |
| 1.1.1126 | 3-(2,2,2-Trifluorethoxy)-5-tert.butoxy-Phenyl | H | Methyl | |
| 1.1.1127 | 3-(2-Chlorethoxy)-5-tert.butoxyl-Phenyl | H | Methyl | |
| 1.1.1128 | 3-(2-Hydroxyethoxy)-5-tert.butoxy-Phenyl- | H | Methyl | |
| 1.1.1129 | 3-[(tert-Butoxycarbonyl)oxy]-5-tert.butoxy-Phenyl | H | Methyl | |
| 1.1.1130 | 3-Nitro-5-tert.butoxyPhenyl | H | Methyl | |
| 1.1.1131 | 3-Acetoxy-5-tert.butoxy-Phenyl | H | Methyl | |
| 1.1.1132 | {3-[(tert-Butoxycarbonyl)amino]-5-tert.butoxy-Phenyl} | H | Methyl | |
| 1.1.1133 | 3-Methylsulfanyl-5-tert.butoxy-Phenyl | H | Methyl | |
| 1.1.1134 | 3,5-Di(trifluormethoxy)-Phenyl | H | Methyl | |
| 1.1.1135 | 3-(2,2,2-Trifluorethoxy)-5-trifluormethoxy-Phenyl | H | Methyl | |
| 1.1.1136 | 3-(2-Chlorethoxy)-5-trifluormethoxy-Phenyl | H | Methyl | |
| 1.1.1137 | 3-(2-Hydroxyethoxy)-5-trifluormethoxy-Phenyl- | H | Methyl | |
| 1.1.1138 | 3-[(tert-Butoxycarbonyl)oxy]-5-trifluormethoxy-Phenyl | H | Methyl | |
| 1.1.1139 | 3-Nitro-5-trifluormethoxy-Phenyl | H | Methyl | |
| 1.1.1140 | 3-Acetoxy-5-tert.butoxy-Phenyl | H | Methyl | |
| 1.1.1141 | {3-[(tert-Butoxycarbonyl)amino]-5-trifluormethoxy-Phenyl} | H | Methyl | |
| 1.1.1142 | 3-Methylsulfanyl-5-trifluormethoxy-Phenyl | H | Methyl | |
| 1.1.1143 | 3,5-Bis(difluormethoxy)-Phenyl | H | Methyl | |
| 1.1.1144 | 3,5-Bis(difluormethoxy)-Phenyl | H | Ethyl | |
| 1.1.1145 | 3,5-Bis(difluormethoxy)-Phenyl | H | isoPropyl | |
| 1.1.1146 | 3,5-Bis(difluormethoxy)-Phenyl | H | Cyclopropyl | |
| 1.1.1147 | 3-Trifluormethoxy-5-difluormethoxy-Phenyl | H | Methyl | |
| 1.1.1148 | 3-(2,2,2-Trifluorethoxy)-5-difluormethoxy-Phenyl | H | Methyl | |
| 1.1.1149 | 3-(2-Chlorethoxy)-5-difluormethoxy-Phenyl | H | Methyl | |
| 1.1.1150 | 3-(2-Hydroxyethoxy)-5-difluormethoxy-Phenyl- | H | Methyl | |
| 1.1.1151 | 3-[(tert-Butoxycarbonyl)oxy]-5-difluormethoxy-Phenyl | H | Methyl | |
| 1.1.1152 | 3-Nitro-5-difluormethoxy-Phenyl | H | Methyl | |
| 1.1.1153 | 3-Acetoxy-5-difluormethoxy-Phenyl | H | Methyl | |
| 1.1.1154 | {3-[(tert-Butoxycarbonyl)amino]-5-difluormethoxy -Phenyl} | H | Methyl | |
| 1.1.1155 | 3-Methylsulfanyl-5-difluormethoxy-Phenyl | H | Methyl | |
| 1.1.1156 | 3,5-Bis(2,2,2-Trifluorethoxy)-Phenyl | H | Methyl | |
| 1.1.1157 | 3-(2-Chlorethoxy)-5-(2,2,2-trifluorethoxy)-Phenyl | H | Methyl | |
| 1.1.1158 | 3-(2-Hydroxyethoxy)-5-(2,2,2-trifluorethoxy)-Phenyl- | H | Methyl | |
| 1.1.1159 | 3-[(tert-Butoxycarbonyl)oxy]-5-(2,2,2-trifluorethoxy)-Phenyl | H | Methyl | |
| 1.1.1160 | 3-Nitro-5--(2,2,2-trifluorethoxy)-Phenyl | H | Methyl | |
| 1.1.1161 | 3-Acetoxy-5-(2,2,2-trifluorethoxy)-Phenyl | H | Methyl | |
| 1.1.1162 | {3-[(tert-Butoxycarbonyl)amino]-5-(2,2,2-trifluorethoxy)-Phenyl} | H | Methyl | |
| 1.1.1163 | 3-Methylsulfanyl-5-(2,2,2-trifluorethoxy)-Phenyl | H | Methyl | |
| 1.1.1164 | 3,5-Bis(2-Chlorethoxy)-Phenyl | H | Methyl | |
| 1.1.1165 | 3-(2-Hydroxyethoxy)-5-(2-chlorethoxy)-Phenyl- | H | Methyl | |
| 1.1.1166 | 3-[(tert-Butoxycarbonyl)oxy]-5-(2-chlorethoxy)-Phenyl | H | Methyl | |
| 1.1.1167 | 3-Nitro-5-(2-chlorethoxy)-Phenyl | H | Methyl | |
| 1.1.1168 | 3-Acetoxy-5-(2-chlorethoxy)-Phenyl | H | Methyl | |
| 1.1.1169 | {3-[(tert-Butoxycarbonyl)amino]-5-(2-chlorethoxy)-Phenyl} | H | Methyl | |
| 1.1.1170 | 3-Methylsulfanyl-5-(2-chlorethoxy)-Phenyl | H | Methyl | |
| 1.1.1171 | 3,5-Bis(2-hydroxyethoxy)-Phenyl | H | Methyl | |
| 1.1.1172 | 3-[(tert-Butoxycarbonyl)oxy]-5-(2-hydroxyethoxy)-Phenyl | H | Methyl | |
| 1.1.1173 | 3-Nitro-5-(2-hydroxyethoxy)-Phenyl | H | Methyl | |
| 1.1.1174 | 3-Acetoxy-5-(2-hydroxyethoxy)-Phenyl | H | Methyl | |
| 1.1.1175 | 3-[(tert-Butoxycarbonyl)amino]-5-(2-hydroxyethoxy)-Phenyl | H | Methyl | |
| 1.1.1176 | 3-Methylsulfanyl-5-(2-hydroxyethoxy)-Phenyl | H | Methyl | |
| 1.1.1177 | 3,5-Bis[(tert-Butoxycarbonyl)oxy]-Phenyl | H | Methyl | |
| 1.1.1178 | 3-Nitro-5-[(tert-Butoxycarbonyl)oxy]--Phenyl | H | Methyl | |
| 1.1.1179 | 3-Acetoxy-5-[(tert-Butoxycarbonyl)oxy]-Phenyl | H | Methyl | |
| 1.1.1180 | {3-[(tert-Butoxycarbonyl)amino]-5[(tert-Butoxy-carbonyl)oxy]-Phenyl} | H | Methyl | |
| 1.1.1181 | 3,5-Bis(acetoxy)-Phenyl | H | Methyl | |
| 1.1.1182 | {3-[(tert-Butoxycarbonyl)amino]-5-acetoxy-Phenyl} | H | Methyl | |
| 1.1.1183 | 3-Methylsulfanyl-5-acetoxy-Phenyl | H | Methyl | |
| 1.1.1184 | 3,5-Dinitro -Phenyl | H | Methyl | |
| 1.1.1185 | 3-Acetoxy-5-nitroPhenyl | H | Methyl | |
| 1.1.1186 | {3-[(tert-Butoxycarbonyl)amino]-5-nitro-Phenyl} | H | Methyl | |
| 1.1.1187 | 3-Methylsulfanyl-5-nitro-Phenyl | H | Methyl | |
| 1.1.1188 | 3,5-Bis[(tert-Butoxycarbonyl)amino]-Phenyl | H | Methyl | |
| 1.1.1189 | 3-Methylsulfanyl-5-[(tert. butoxycarbonyl) amino]-Phenyl | H | Methyl | |
| 1.1.1190 | 3,5-Di(methylsulfanyl)-Phenyl | H | Methyl | |
| 1.1.1191 | 3,4-Difluor-Phenyl | H | Methyl | [CDCl₃] 1.80 (s, 3H); 3.25 (d, 1H); 3.85 (d, 1H); 7.22 (m, 1H); 7.30 (m, 1H); 7.55 (m, 1H). |
| 1.1.1192 | 3,4-Difluor-Phenyl | H | Ethyl | [CDCl₃] 1.09 (t, 3H); 2.12 (mc, 2H); 3.29 (d, 1H); 3.25 (d, 1H); 7.22 (m, 1H); 7.35 (m, 1H); 7.55 (m, 1H). |
| 1.1.1193 | 3,4-Difluor-Phenyl | H | isoPropyl | |
| 1.1.1194 | 3,4-Difluor-Phenyl | H | Cyclopropyl | |
| 1.1.1195 | 3-Chlor-4-fluorPhenyl | H | Methyl | [CDCl₃] 1.81 (s, 3H); 3.28 (d, 1H); 3.85 (d, 1H), 7.21 (m, 1H); 7.52 (m, 1H); 7.72 (m, 1H). |
| 1.1.1196 | 3-Chlor-4-fluorPhenyl | H | Ethyl | |
| 1.1.1197 | 3-Chlor-4-fluorPhenyl | H | isoPropyl | |
| 1.1.1198 | 3-Chlor-4-fluorPhenyl | H | Cyclopropyl | |
| 1.1.1199 | 3-Brom-4-fluorPhenyl | H | Methyl | |
| 1.1.1200 | 3-Methyl-4-fluorPhenyl | H | Methyl | |
| 1.1.1201 | 3-Methyl-4-fluorPhenyl | H | Ethyl | |
| 1.1.1202 | 3-Ethyl-4-fluorPhenyl | H | Methyl | |
| 1.1.1203 | 3-Cyclopropyl-4-fluor-Phenyl | H | Methyl | |
| 1.1.1204 | 3-Vinyl-4-fluorPhenyl | H | Methyl | |
| 1.1.1205 | 3-Ethinyl-4-fluorPhenyl | H | Methyl | |
| 1.1.1206 | 3-Cyano-4-fluorPhenyl | H | Methyl | |
| 1.1.1207 | 3-Methoxy-4-fluorPhenyl | H | Methyl | |
| 1.1.1208 | 3-Ethoxy-4-fluorPhenyl | H | Methyl | |
| 1.1.1209 | 3-Trifluormethoxy-4-fluor-Phenyl | H | Methyl | |
| 1.1.1210 | 3-Nitro-4-fluorPhenyl | H | Methyl | |
| 1.1.1211 | 3-Fluor-4-chlorPhenyl | H | Methyl | [CDCl₃] 1.71 (s, 3H); 3.25 (d, 1H); 3.82 (d, 1H); 7.35 (m, 1H); 7.48 (m, 1H). |
| 1.1.1212 | 3,4-Dichlor-Phenyl | H | Methyl | [CDCl₃] 1.8 (s, 3H); 3.25 (d, 1H); 3.83 (d, 1H); 7.5 (s, 2H); 7.72 (s, 1H). |
| 1.1.1213 | 3-Brom-4-chlorPhenyl | H | Methyl | |
| 1.1.1214 | 3-Methyl-4-chlorPhenyl | H | Methyl | |
| 1.1.1215 | 3-Ethyl-4-chlorPhenyl | H | Methyl | |
| 1.1.1216 | 3-Cyclopropyl-4-chlor-Phenyl | H | Methyl | |
| 1.1.1217 | 3-Vinyl-4-chlorPhenyl | H | Methyl | |
| 1.1.1218 | 3-Ethinyl-4-chlorPhenyl | H | Methyl | |
| 1.1.1219 | 3-Cyano-4-chlorPhenyl | H | Methyl | |
| 1.1.1220 | 3-Trifluormethyl-4-chlor-Phenyl | H | Methyl | |
| 1.1.1221 | 3-Methoxy-4-chlorPhenyl | H | Methyl | |
| 1.1.1222 | 3-Ethoxy-4-chlorPhenyl | H | Methyl | |
| 1.1.1223 | 3-Trifluormethoxy-4-chlor-Phenyl | H | Methyl | |
| 1.1.1224 | 3-Nitro-4-chlorPhenyl | H | Methyl | |
| 1.1.1225 | 3-Fluor-4-bromPhenyl | H | Methyl | |
| 1.1.1226 | 3-Chlor-4-bromPhenyl | H | Methyl | |
| 1.1.1227 | 3,4-Dibrom-Phenyl | H | Methyl | |
| 1.1.1228 | 3-Methyl-4-bromPhenyl | H | Methyl | |
| 1.1.1229 | 3-Ethyl-4-bromPhenyl | H | Methyl | |
| 1.1.1230 | 3-Cyclopropyl-4-brom-Phenyl | H | Methyl | |
| 1.1.1231 | 3-Vinyl-4-bromPhenyl | H | Methyl | |
| 1.1.1232 | 3-Ethinyl-4-bromPhenyl | H | Methyl | |
| 1.1.1233 | 3-Cyano-4-bromPhenyl | H | Methyl | |
| 1.1.1234 | 3-Trifluormethyl-4-brom-Phenyl | H | Methyl | |
| 1.1.1235 | 3-Methoxy-4-Phenyl | H | Methyl | |
| 1.1.1236 | 3-Ethoxy-4-bromPhenyl | H | Methyl | |
| 1.1.1237 | 3-Trifluormethoxy-4-brom-Phenyl | H | Methyl | |
| 1.1.1238 | 3-Nitro-4-bromPhenyl | H | Methyl | |
| 1.1.1239 | 3-Fluor-4-iod-Phenyl | H | Methyl | |
| 1.1.1240 | 3-Chlor-4-iod-Phenyl | H | Methyl | |
| 1.1.1241 | 3-Brom-4-iod-Phenyl | H | Methyl | |
| 1.1.1242 | 3-Methyl-4-iod-Phenyl | H | Methyl | |
| 1.1.1243 | 3-Ethyl-4-iod-Phenyl | H | Methyl | |
| 1.1.1244 | 3-Cyclopropyl-4-iod-Phenyl | H | Methyl | |
| 1.1.1245 | 3-Vinyl-4-iod-Phenyl | H | Methyl | |
| 1.1.1246 | 3-Ethinyl-4-iod-Phenyl | H | Methyl | |
| 1.1.1247 | 3-Cyano-4-iod-Phenyl | H | Methyl | |
| 1.1.1248 | 3-Trifluormethyl-4-iod-Phenyl | H | Methyl | |
| 1.1.1249 | 3-Methoxy-4-iod-Phenyl | H | Methyl | |
| 1.1.1250 | 3-Ethoxy-4-iod-Phenyl | H | Methyl | |
| 1.1.1251 | 3-Trifluormethoxy-4-iod-Phenyl | H | Methyl | |
| 1.1.1252 | 3-Nitro-4-iod-Phenyl | H | Methyl | |
| 1.1.1253 | 3-Fluor-4-methylPhenyl | H | Methyl | |
| 1.1.1254 | 3-Chlor-4-methylPhenyl | H | Methyl | [CDCl₃] 1.75 (s, 3H); 2.42 (s, 3H); 3.29 (d, 1H); 3.85 (d, 1H); 7.25 (m, 1H); 7.41 (m, 1H); 7.51 (m, 1H). |
| 1.1.1255 | 3-Brom-4-methylPhenyl | H | Methyl | |
| 1.1.1256 | 3.4-Dimethyl-Phenyl | H | Methyl | |
| 1.1.1257 | 3.4-Dimethyl-Phenyl | H | Ethyl | |
| 1.1.1258 | 3.4-Dimethyl-Phenyl | H | isoPropyl | |
| 1.1.1259 | 3.4-Dimethyl-Phenyl | H | Cyclopropyl | |
| 1.1.1260 | 3-Ethyl-4-methylPhenyl | H | Methyl | |
| 1.1.1261 | 3-Cyclopropyl-4-methyl-Phenyl | H | Methyl | |
| 1.1.1262 | 3-Vinyl-4-methylPhenyl | H | Methyl | |
| 1.1.1263 | 3-Ethinyl-4-methylPhenyl | H | Methyl | |
| 1.1.1264 | 3-Cyano-4-methylPhenyl | H | Methyl | |
| 1.1.1265 | 3-Trifluormethyl-4-methyl-Phenyl | H | Methyl | |
| 1.1.1266 | 3-Methoxy-4-methylPhenyl | H | Methyl | |
| 1.1.1267 | 3-Ethoxy-4-methylPhenyl | H | Methyl | |
| 1.1.1268 | 3-Trifluormethoxy-4-methyl-Phenyl | H | Methyl | |
| 1.1.1269 | 3-Nitro-4-methylPhenyl | H | Methyl | |
| 1.1.1270 | 3-Fluor-4-ethylPhenyl | H | Methyl | |
| 1.1.1271 | 3-Chlor-4-ethylPhenyl | H | Methyl | |
| 1.1.1272 | 3-Brom-4-ethylPhenyl | H | Methyl | |
| 1.1.1273 | 3-Methyl-4-ethylPhenyl | H | Methyl | |
| 1.1.1274 | 3,4-Diethyl-Phenyl | H | Methyl | |
| 1.1.1275 | 3-Cyclopropyl-4-ethyl-Phenyl | H | Methyl | |
| 1.1.1276 | 3-Vinyl-4-ethylPhenyl | H | Methyl | |
| 1.1.1277 | 3-Ethinyl-4-ethylPhenyl | H | Methyl | |
| 1.1.1278 | 3-Cyano-4-ethylPhenyl | H | Methyl | |
| 1.1.1279 | 3-Trifluormethyl-4-ethyl-Phenyl | H | Methyl | |
| 1.1.1280 | 3-Methoxy-4-ethylPhenyl | H | Methyl | |
| 1.1.1281 | 3-Ethoxy-4-ethylPhenyl | H | Methyl | |
| 1.1.1282 | 3-Trifluormethoxy-4-ethyl-Phenyl | H | Methyl | |
| 1.1.1283 | 3-Nitro-4-ethylPhenyl | H | Methyl | |
| 1.1.1284 | 3-Fluor-4-propylPhenyl | H | Methyl | |
| 1.1.1285 | 3-Chlor-4-propylPhenyl | H | Methyl | |
| 1.1.1286 | 3-Brom-4-propylPhenyl | H | Methyl | |
| 1.1.1287 | 3-Methyl-4-propylPhenyl | H | Methyl | |
| 1.1.1288 | 3-Methyl-4-propylPhenyl | H | Methyl | |
| 1.1.1289 | 3-Cyclopropyl-4-propyl-Phenyl | H | Methyl | |
| 1.1.1290 | 3-Vinyl-4-propylPhenyl | H | Methyl | |
| 1.1.1291 | 3-Ethinyl-4-propylPhenyl | H | Methyl | |
| 1.1.1292 | 3-Cyano-4-propylPhenyl | H | Methyl | |
| 1.1.1293 | 3-Trifluormethyl-4-propyl-Phenyl | H | Methyl | |
| 1.1.1294 | 3-Methoxy-4-propylPhenyl | H | Methyl | |
| 1.1.1295 | 3-Ethoxy-4-propylPhenyl | H | Methyl | |
| 1.1.1296 | 3-Trifluormethoxy-4-propyl-Phenyl | H | Methyl | |
| 1.1.1297 | 3-Nitro-4-propylPhenyl | H | Methyl | |
| 1.1.1298 | 3-Fluor-4-isopropylPhenyl | H | Methyl | |
| 1.1.1299 | 3-Chlor-4-isopropylPhenyl | H | Methyl | |
| 1.1.1300 | 3-Brom-4-isopropylPhenyl | H | Methyl | |
| 1.1.1301 | 3-Methyl-4-isopropylPhenyl | H | Methyl | |
| 1.1.1302 | 3-Ethyl-4-isopropylPhenyl | H | Methyl | |
| 1.1.1303 | 3-Cyclopropyl-4-isopropyl-Phenyl | H | Methyl | |
| 1.1.1304 | 3-Vinyl-4-isopropylPhenyl | H | Methyl | |
| 1.1.1305 | 3-Ethinyl-4-isopropylPhenyl | H | Methyl | |
| 1.1.1306 | 3-Cyano-4-isopropylPhenyl | H | Methyl | |
| 1.1.1307 | 3-Trifluormethyl-4-isopropyl-Phenyl | H | Methyl | |
| 1.1.1308 | 3-Methoxy-4-isopropyl-Phenyl | H | Methyl | |
| 1.1.1309 | 3-Ethoxy-4-isopropylPhenyl | H | Methyl | |
| 1.1.1310 | 3-Trifluormethoxy-4-isopropyl-Phenyl | H | Methyl | |
| 1.1.1311 | 3-Nitro-4-isopropylPhenyl | H | Methyl | |
| 1.1.1312 | 3-Fluor-4-tert.butyl-Phenyl | H | Methyl | |
| 1.1.1313 | 3-Chlor-4-tert.buty-Phenyl | H | Methyl | |
| 1.1.1314 | 3-Brom-4-tert.butyl-Phenyl | H | Methyl | |
| 1.1.1315 | 3-Methyl-4-tert.butyl-Phenyl | H | Methyl | |
| 1.1.1316 | 3-Ethyl-4-tert.butyl-Phenyl | H | Methyl | |
| 1.1.1317 | 3-Cyclopropyl-4-tert.butyl-Phenyl | H | Methyl | |
| 1.1.1318 | 3-Vinyl-4-tert.butyl-Phenyl | H | Methyl | |
| 1.1.1319 | 3-Ethinyl-4-tert.butyl-Phenyl | H | Methyl | |
| 1.1.1320 | 3-Cyano-4-tert.butyl-Phenyl | H | Methyl | |
| 1.1.1321 | 3-Trifluormethyl-4-tert.butyl-Phenyl | H | Methyl | |
| 1.1.1322 | 3-Trifluormethyl-4-tert.butyl-Phenyl | H | Ethyl | |
| 1.1.1323 | 3-Trifluormethyl-4-tert.butyl-Phenyl | H | isoPropyl | |
| 1.1.1324 | 3-Trifluormethyl-4-tert.butyl-Phenyl | H | Cyclopropyl | |
| 1.1.1325 | 3-Methoxy-4-tert.butyl-Phenyl | H | Methyl | |
| 1.1.1326 | 3-Ethoxy-4-tert.butyl -Phenyl | H | Methyl | |
| 1.1.1327 | 3-Trifluormethoxy-4-tert.butyl-Phenyl | H | Methyl | |
| 1.1.1328 | 3-Nitro-4-tert.butyl-Phenyl | H | Methyl | |
| 1.1.1329 | 3-Fluor-4-hydroxymethyl-Phenyl | H | Methyl | |
| 1.1.1330 | 3-Chlor-4-hydroxymethyl-Phenyl | H | Methyl | |
| 1.1.1331 | 3-Brom-4-hydroxymethyl - Phenyl | H | Methyl | |
| 1.1.1332 | 3-Methyl-4-hydroxymethyl - Phenyl | H | Methyl | |
| 1.1.1333 | 3-Ethyl-4-hydroxymethyl - Phenyl | H | Methyl | |
| 1.1.1334 | 3-Cyclopropyl-4-hydroxymethyl-Phenyl | H | Methyl | |
| 1.1.1335 | 3-Vinyl-4-hydroxymethyl - Phenyl | H | Methyl | |
| 1.1.1336 | 3-Ethinyl-4-hydroxymethyl-Phenyl | H | Methyl | |
| 1.1.1337 | 3-Cyano-4-hydroxymethyl - Phenyl | H | Methyl | |
| 1.1.1338 | 3-Trifluormethyl-4-hydroxymethyl-Phenyl | H | Methyl | |
| 1.1.1339 | 3-Methoxy-4-hydroxymethyl - Phenyl | H | Methyl | |
| 1.1.1340 | 3-Ethoxy-4-hydroxymethyl - Phenyl | H | Methyl | |
| 1.1.1341 | 3-Trifluormethoxy-4-hydroxymethyl-Phenyl | H | Methyl | |
| 1.1.1342 | 3-Nitro-4-hydroxymethyl-Phenyl | H | Methyl | |
| 1.1.1343 | 3-Fluor-4-cyclopropyl-Phenyl | H | Methyl | |
| 1.1.1344 | 3-Chlor-4-cyclopropyl-Phenyl | H | Methyl | |
| 1.1.1345 | 3-Brom-4-cyclopropyl-Phenyl | H | Methyl | |
| 1.1.1346 | 3-Methyl-4-cyclopropyl-Phenyl | H | Methyl | |
| 1.1.1347 | 3-Ethyl-4-cyclopropyl -Phenyl | H | Methyl | |
| 1.1.1348 | 3-Cyclopropyl-4-cyclopropyl-Phenyl | H | Methyl | |
| 1.1.1349 | 3-Vinyl-4-cyclopropyl-Phenyl | H | Methyl | |
| 1.1.1350 | 3-Ethinyl-4-cyclopropyl-Phenyl | H | Methyl | |
| 1.1.1351 | 3-Cyano-4-cyclopropyl-Phenyl | H | Methyl | |
| 1.1.1352 | 3-Trifluormethyl-4-cyclopropyl-Phenyl | H | Methyl | |
| 1.1.1353 | 3-Methoxy-4-cyclopropyl-Phenyl | H | Methyl | |
| 1.1.1354 | 3-Ethoxy-4-cyclopropyl-Phenyl | H | Methyl | |
| 1.1.1355 | 3-Trifluormethoxy-4-cyclopropyl-Phenyl | H | Methyl | |
| 1.1.1356 | 3-Fluor-4-methoxycarbonyl-Phenyl | H | Methyl | |
| 1.1.1357 | 3-Chlor-4-methoxycarbonyl-Phenyl | H | Methyl | |
| 1.1.1358 | 3-Brom-4-methoxycarbonyl-Phenyl | H | Methyl | |
| 1.1.1359 | 3-Methyl-4-methoxycarbonyl-Phenyl | H | Methyl | |
| 1.1.1360 | 3-Ethyl-4-methoxycarbonyl-Phenyl | H | Methyl | |
| 1.1.1361 | 3-Cyclopropyl-4-methoxycarbonyl-Phenyl | H | Methyl | |
| 1.1.1362 | 3-Vinyl-4-methoxycarbonyl-Phenyl | H | Methyl | |
| 1.1.1363 | 3-Ethinyl-4-methoxycarbonyl-Phenyl | H | Methyl | |
| 1.1.1364 | 3-Cyano-4-methoxycarbonyl-Phenyl | H | Methyl | |
| 1.1.1365 | 3-Trifluormethyl-4-methoxycarbonyl-Phenyl | H | Methyl | |
| 1.1.1366 | 3-Methoxy-4-methoxycarbonyl - Phenyl | H | Methyl | |
| 1.1.1367 | 3-Ethoxy-4-methoxycarbonyl-Phenyl | H | Methyl | |
| 1.1.1368 | 3-Trifluormethoxy-4-methoxycarbonyl-Phenyl | H | Methyl | |
| 1.1.1369 | 3-Nitro-4-methoxycarbonyl-Phenyl | H | Methyl | |
| 1.1.1370 | 3-Fluor-4-vinyl-Phenyl | H | Methyl | |
| 1.1.1371 | 3-Chlor-4-vinyl-Phenyl | H | Methyl | |
| 1.1.1372 | 3-Brom-4-vinyl-Phenyl | H | Methyl | |
| 1.1.1373 | 3-Methyl-4-vinyl-Phenyl | H | Methyl | |
| 1.1.1374 | 3-Ethyl-4-vinyl-Phenyl | H | Methyl | |
| 1.1.1375 | 3-Cyclopropyl-4-vinyl-Phenyl | H | Methyl | |
| 1.1.1376 | 3,4-Divinyl-Phenyl | H | Methyl | |
| 1.1.1377 | 3-Ethinyl-4-vinyl-Phenyl | H | Methyl | |
| 1.1.1378 | 3-Cyano-4-vinyl-Phenyl | H | Methyl | |
| 1.1.1379 | 3-Trifluormethyl-4-vinyl-Phenyl | H | Methyl | |
| 1.1.1380 | 3-Methoxy-4-vinyl-Phenyl | H | Methyl | |
| 1.1.1381 | 3-Ethoxy-4-vinyl-Phenyl | H | Methyl | |
| 1.1.1382 | 3-Trifluormethoxy-4-vinyl-Phenyl | H | Methyl | |
| 1.1.1383 | 3-Nitro-4-vinyl-Phenyl | H | Methyl | |
| 1.1.1384 | 3-Fluor-4-ethinyl-Phenyl | H | Methyl | |
| 1.1.1385 | 3-Chlor-4-ethinyl-Phenyl | H | Methyl | |
| 1.1.1386 | 3-Brom-4-ethinyl-Phenyl | H | Methyl | |
| 1.1.1387 | 3-Methyl-4-ethinyl - Phenyl | H | Methyl | |
| 1.1.1388 | 3-Ethyl-4-ethinyl-Phenyl | H | Methyl | |
| 1.1.1389 | 3-Cyclopropyl-4-ethinyl-Phenyl | H | Methyl | |
| 1.1.1390 | 3-Vinyl-4-ethinyl-Phenyl | H | Methyl | |
| 1.1.1391 | 3-Cyano-4-ethinyl-Phenyl | H | Methyl | |
| 1.1.1392 | 3-Trifluormethyl-4-ethinyl-Phenyl | H | Methyl | |
| 1.1.1393 | 3-Methoxy-4-ethinyl-Phenyl | H | Methyl | |
| 1.1.1394 | 3-Ethoxy-4-ethinyl-Phenyl | H | Methyl | |
| 1.1.1395 | 3-Trifluormethoxy-4-ethinyl--Phenyl | H | Methyl | |
| 1.1.1396 | 3-Nitro-4-ethinyl-Phenyl | H | Methyl | |
| 1.1.1397 | 3-Fluor-4-ethinyl-Phenyl | H | Methyl | |
| 1.1.1398 | 3-Fluor-4-cyano-Phenyl | H | Methyl | |
| 1.1.1399 | 3-Chlor-4-cyano-Phenyl | H | Methyl | |
| 1.1.1400 | 3-Brom-4-cyano-Phenyl | H | Methyl | |
| 1.1.1401 | 3-Methyl-4-cyano-Phenyl | H | Methyl | |
| 1.1.1402 | 3-Ethyl-4-cyano-Phenyl | H | Methyl | |
| 1.1.1403 | 3-Cyclopropyl-4-cyano-Phenyl | H | Methyl | |
| 1.1.1404 | 3-Vinyl-4-cyano-Phenyl | H | Methyl | |
| 1.1.1405 | 3-Ethinyl-4-cyano-Phenyl | H | Methyl | |
| 1.1.1406 | 3,4-Dicyano-Phenyl | H | Methyl | |
| 1.1.1407 | 3-Trifluormethyl-4-cyano-Phenyl | H | Methyl | |
| 1.1.1408 | 3-Trifluormethyl-4-cyano-Phenyl | H | Ethyl | |
| 1.1.1409 | 3-Trifluormethyl-4-cyano-Phenyl | H | isoPropyl | |
| 1.1.1410 | 3-Trifluormethyl-4-cyano-Phenyl | H | Cyclopropyl | |
| 1.1.1411 | 3-Methoxy-4-cyano - Phenyl | H | Methyl | |
| 1.1.1412 | 3-Ethoxy-4-cyano-Phenyl | H | Methyl | |
| 1.1.1413 | 3-Trifluormethoxy-4-cyano-Phenyl | H | Methyl | |
| 1.1.1414 | 3-Nitro-4-cyano-Phenyl | H | Methyl | |
| 1.1.1415 | 3-Fluor-4-hydroxy-Phenyl | H | Methyl | |
| 1.1.1416 | 3-Chlor-4-hydroxy-Phenyl | H | Methyl | |
| 1.1.1417 | 3-Brom-4-hydroxy-Phenyl | H | Methyl | |
| 1.1.1418 | 3-Methyl-4-hydroxy-Phenyl | H | Methyl | |
| 1.1.1419 | 3-Ethyl-4-hydroxy-Phenyl | H | Methyl | |
| 1.1.1420 | 3-Cyclopropyl-4-hydroxy-Phenyl | H | Methyl | |
| 1.1.1421 | 3-Vinyl-4-hydroxy-Phenyl | H | Methyl | |
| 1.1.1422 | 3-Ethinyl-4-hydroxy-Phenyl | H | Methyl | |
| 1.1.1423 | 3-Cyano-4-hydroxy-Phenyl | H | Methyl | |
| 1.1.1424 | 3-Trifluormethyl-4-hydroxy-Phenyl | H | Methyl | |
| 1.1.1425 | 3-Methoxy-4-hydroxy -Phenyl | H | Methyl | |
| 1.1.1426 | 3-Ethoxy-4-hydroxy-Phenyl | H | Methyl | |
| 1.1.1427 | 3-Trifluormethoxy-4-hydroxy-Phenyl | H | Methyl | |
| 1.1.1428 | 3-Nitro-4-hydroxy-Phenyl | H | Methyl | |
| 1.1.1429 | 3-Fluor-4-methoxy-Phenyl | H | Methyl | |
| 1.1.1430 | 3-Chlor-4-methoxy-Phenyl | H | Methyl | |
| 1.1.1431 | 3-Brom-4-methoxy-Phenyl | H | Methyl | |
| 1.1.1432 | 3-Methyl-4-methoxy-Phenyl | H | Methyl | |
| 1.1.1433 | 3-Ethyl-4-methoxy-Phenyl | H | Methyl | |
| 1.1.1434 | 3-Cyclopropyl-4-methoxy-Phenyl | H | Methyl | |
| 1.1.1435 | 3-Vinyl-4-methoxy-Phenyl | H | Methyl | |
| 1.1.1436 | 3-Ethinyl-4-methoxy-Phenyl | H | Methyl | |
| 1.1.1437 | 3-Cyano-4-methoxy-Phenyl | H | Methyl | |
| 1.1.1438 | 3-Trifluormethyl-4-methoxy-Phenyl | H | Methyl | |
| 1.1.1439 | 3,4-Dimethoxy-Phenyl | H | Methyl | |
| 1.1.1440 | 3-Ethoxy-4-methoxy-Phenyl | H | Methyl | |
| 1.1.1441 | 3-Trifluormethoxy-4-methoxy-Phenyl | H | Methyl | |
| 1.1.1442 | 3-Nitro-4-methoxy-Phenyl | H | Methyl | |
| 1.1.1443 | 3-Fluor-4-ethoxy-Phenyl | H | Methyl | |
| 1.1.1444 | 3-Chlor-4-ethoxy-Phenyl | H | Methyl | |
| 1.1.1445 | 3-Chlor-4-ethoxy-Phenyl | H | Ethyl | |
| 1.1.1446 | 3-Chlor-4-ethoxy-Phenyl | H | isoPropyl | |
| 1.1.1447 | 3-Chlor-4-ethoxy-Phenyl | H | Cyclopropyl | |
| 1.1.1448 | 3-Brom-4-ethoxy-Phenyl | H | Methyl | |
| 1.1.1449 | 3-Methyl-4-ethoxy-Phenyl | H | Methyl | |
| 1.1.1450 | 3-Ethyl-4-ethoxy-Phenyl | H | Methyl | |
| 1.1.1451 | 3-Cyclopropyl-4-ethoxy-Phenyl | H | Methyl | |
| 1.1.1452 | 3-Vinyl-4-ethoxy-Phenyl | H | Methyl | |
| 1.1.1453 | 3-Ethinyl-4-ethoxy-Phenyl | H | Methyl | |
| 1.1.1454 | 3-Cyano-4-ethoxy-Phenyl | H | Methyl | |
| 1.1.1455 | 3-Trifluormethyl-4-ethoxy-Phenyl | H | Methyl | |
| 1.1.1456 | 3-Methoxy-4-ethoxy - Phenyl | H | Methyl | |
| 1.1.1457 | 2,4-Diethoxy-Phenyl | H | Methyl | |
| 1.1.1458 | 3-Trifluormethoxy-4-ethoxy-Phenyl | H | Methyl | |
| 1.1.1459 | 3-Nitro-4-ethoxy-Phenyl | H | Methyl | |
| 1.1.1460 | 3-Fluor-4-propoxy-Phenyl | H | Methyl | |
| 1.1.1461 | 3-Chlor-4-propoxy-Phenyl | H | Methyl | |
| 1.1.1462 | 3-Brom-4-propoxy-Phenyl | H | Methyl | |
| 1.1.1463 | 3-Methyl-4-propoxy-Phenyl | H | Methyl | |
| 1.1.1464 | 3-Ethyl-4-propoxy-Phenyl | H | Methyl | |
| 1.1.1465 | 3-Cyclopropyl-4-propoxy-Phenyl | H | Methyl | |
| 1.1.1466 | 3-Vinyl-4-propoxy-Phenyl | H | Methyl | |
| 1.1.1467 | 3-Ethinyl-4-propoxy-Phenyl | H | Methyl | |
| 1.1.1468 | 3-Cyano-4-propoxy-Phenyl | H | Methyl | |
| 1.1.1469 | 3-Trifluormethyl-4-propoxy-Phenyl | H | Methyl | |
| 1.1.1470 | 3-Methoxy-4-propoxy -Phenyl | H | Methyl | |
| 1.1.1471 | 3-Ethoxy-4-propoxy-Phenyl | H | Methyl | |
| 1.1.1472 | 3-Trifluormethoxy-4-propoxy-Phenyl | H | Methyl | |
| 1.1.1473 | 3-Nitro-4-propoxy-Phenyl | H | Methyl | |
| 1.1.1474 | 3-Fluor-4-isopropoxy-Phenyl | H | Methyl | |
| 1.1.1475 | 3-Chlor-4-isopropoxy-Phenyl | H | Methyl | |
| 1.1.1476 | 3-Brom-4-isopropoxy-Phenyl | H | Methyl | |
| 1.1.1477 | 3-Methyl-4-isopropoxy-Phenyl | H | Methyl | |
| 1.1.1478 | 3-Ethyl-4-isopropoxy-Phenyl | H | Methyl | |
| 1.1.1479 | 3-Cyclopropyl-4-isopropoxy-Phenyl | H | Methyl | |
| 1.1.1480 | 3-Vinyl-4-isopropoxy-Phenyl | H | Methyl | |
| 1.1.1481 | 3-Ethinyl-4-isopropoxy-Phenyl | H | Methyl | |
| 1.1.1482 | 3-Cyano-4-isopropoxy-Phenyl | H | Methyl | |
| 1.1.1483 | 3-Trifluormethyl-4-isopropoxy-Phenyl | H | Methyl | |
| 1.1.1484 | 3-Methoxy-4-isopropoxy-Phenyl | H | Methyl | |
| 1.1.1485 | 3-Ethoxy-4-isopropoxy-Phenyl | H | Methyl | |
| 1.1.1486 | 3-Trifluormethoxy-4-isopropoxy-Phenyl | H | Methyl | |
| 1.1.1487 | 3-Nitro-4-isopropoxy-Phenyl | H | Methyl | |
| 1.1.1488 | 3-Fluor-4-tert.butoxy-Phenyl | H | Methyl | |
| 1.1.1489 | 3-Chlor-4-tert.butoxy-Phenyl | H | Methyl | |
| 1.1.1490 | 3-Brom-4-tert.butoxy-Phenyl | H | Methyl | |
| 1.1.1491 | 3-Methyl-4-tert.butoxy-Phenyl | H | Methyl | |
| 1.1.1492 | 3-Ethyl-4-tert.butoxy-Phenyl | H | Methyl | |
| 1.1.1493 | 3-Cyclopropyl-4-tert.butoxy-Phenyl | H | Methyl | |
| 1.1.1494 | 3-Vinyl-4-tert.butoxy-Phenyl | H | Methyl | |
| 1.1.1495 | 3-Ethinyl-4-tert.butoxy-Phenyl | H | Methyl | |
| 1.1.1496 | 3-Cyano-4-tert.butoxy-Phenyl | H | Methyl | |
| 1.1.1497 | 3-Trifluormethyl-4-tert.butoxy-Phenyl | H | Methyl | |
| 1.1.1498 | 3-Methoxy-4-tert.butoxy-Phenyl | H | Methyl | |
| 1.1.1499 | 3-Ethoxy-4-tert.butoxy-Phenyl | H | Methyl | |
| 1.1.1500 | 3-Trifluormethoxy-4-tert.butoxy-Phenyl | H | Methyl | |
| 1.1.1501 | 3-Nitro-4-tert.butoxy-Phenyl | H | Methyl | |
| 1.1.1502 | 3-Fluor-4-trifluormethoxy-Phenyl | H | Methyl | |
| 1.1.1503 | 3-Chlor-4-trifluormethoxy-Phenyl | H | Methyl | |
| 1.1.1504 | 3-Brom-4-trifluormethoxy-Phenyl | H | Methyl | |
| 1.1.1505 | 3-Methyl-4-trifluormethoxy-Phenyl | H | Methyl | |
| 1.1.1506 | 3-Ethyl-4-trifluormethoxy-Phenyl | H | Methyl | |
| 1.1.1507 | 3-Cyclopropyl-4-trifluormethoxy-Phenyl | H | Methyl | |
| 1.1.1508 | 3-Vinyl-4-trifluormethoxy-Phenyl | H | Methyl | |
| 1.1.1509 | 3-Ethinyl-4-trifluormethoxy-Phenyl | H | Methyl | |
| 1.1.1510 | 3-Cyano-4-trifluormethoxy-Phenyl | H | Methyl | |
| 1.1.1511 | 3-Trifluormethyl-4-trifluormethoxy-Phenyl | H | Methyl | |
| 1.1.1512 | 3-Methoxy-4-trifluormethoxy-Phenyl | H | Methyl | |
| 1.1.1513 | 3-Ethoxy-4-trifluormethoxy-Phenyl | H | Methyl | |
| 1.1.1514 | 3,4-Bis(trifluormethoxy)-Phenyl | H | Methyl | |
| 1.1.1515 | 3-Nitro-4-trifluormethoxy-Phenyl | H | Methyl | |
| 1.1.1516 | 3-Fluor-4-(2,2,2-trifluorethoxy)-Phenyl | H | Methyl | |
| 1.1.1517 | 3-Chlor-4-(2,2,2-trifluorethoxy)-Phenyl | H | Methyl | |
| 1.1.1518 | 3-Brom-4-(2,2,2-trifluorethoxy)-Phenyl | H | Methyl | |
| 1.1.1519 | 3-Methyl-4-(2,2,2-trifluorethoxy)-Phenyl | H | Methyl | |
| 1.1.1520 | 3-Ethyl-4-(2,2,2-trifluorethoxy)-Phenyl | H | Methyl | |
| 1.1.1521 | 3-Cyclopropyl-4-(2,2,2-trifluorethoxy)-Phenyl | H | Methyl | |
| 1.1.1522 | 3-Vinyl-4-(2,2,2-trifluorethoxy)-Phenyl | H | Methyl | |
| 1.1.1523 | 3-Ethinyl-4-(2,2,2-trifluorethoxy-Phenyl | H | Methyl | |
| 1.1.1524 | 3-Cyano-4-(2,2,2-trifluorethoxy)-Phenyl | H | Methyl | |
| 1.1.1525 | 3-Trifluormethyl-4-(2,2,2-trifluorethoxy)-Phenyl | H | Methyl | |
| 1.1.1526 | 3-Methoxy-4-(2,2,2-trifluorethoxy)-Phenyl | H | Methyl | |
| 1.1.1527 | 3-Ethoxy-4-(2,2,2-trifluorethoxy)-Phenyl | H | Methyl | |
| 1.1.1528 | 3-Trifiuormethoxy-4-(2,2,2-trifluorethoxy)-Phenyl | H | Methyl | |
| 1.1.1529 | 3-Nitro-4-(2,2,2-trifluorethoxy)-Phenyl | H | Methyl | |
| 1.1.1530 | 3-Fluor-4-difluormethoxy-Phenyl | H | Methyl | |
| 1.1.1531 | 3-Chlor-4-difluormethoxy-Phenyl | H | Methyl | |
| 1.1.1532 | 3-Brom-4-difluormethoxy-Phenyl | H | Methyl | |
| 1.1.1533 | 3-Methyl-4-difluormethoxy-Phenyl | H | Methyl | |
| 1.1.1534 | 3-Ethyl-4-difluormethoxy-Phenyl | H | Methyl | |
| 1.1.1535 | 3-Cyclopropyl-4-difluormethoxy-Phenyl | H | Methyl | |
| 1.1.1536 | 3-Vinyl-4-difluormethoxy-Phenyl | H | Methyl | |
| 1.1.1537 | 3-Ethinyl-4-difluormethoxy-Phenyl | H | Methyl | |
| 1.1.1538 | 3-Cyano-4-difluormethoxy - Phenyl | H | Methyl | |
| 1.1.1539 | 3-Trifluormethyl-4-difluormethoxy - Phenyl | H | Methyl | |
| 1.1.1540 | 3-Methoxy-4-difluormethoxy - Phenyl | H | Methyl | |
| 1.1.1541 | 3-Ethoxy-4-difluormethoxy-Phenyl | H | Methyl | |
| 1.1.1542 | 3-Trifluormethoxy-4-difluormethoxy-Phenyl | H | Methyl | |
| 1.1.1543 | 3-Nitro-4-difluormethoxy-Phenyl | H | Methyl | |
| 1.1.1544 | 3-Fluor-4-(2-methoxyethoxy)-Phenyl | H | Methyl | |
| 1.1.1545 | 3-Chlor-4-(2-methoxyethoxy)-Phenyl | H | Methyl | |
| 1.1.1546 | 3-Brom-4-(2-methoxyethoxy)-Phenyl | H | Methyl | |
| 1.1.1547 | 3-Methyl-4-(2-methoxyethoxy)-Phenyl | H | Methyl | |
| 1.1.1548 | 3-Ethyl-4-(2-methoxyethoxy)-Phenyl | H | Methyl | |
| 1.1.1549 | 3-Cyclopropyl-4-(2-methoxyethoxy)-Phenyl | H | Methyl | |
| 1.1.1550 | 3-Vinyl-4-(2-methoxyethoxy)-Phenyl | H | Methyl | |
| 1.1.1551 | 3-Ethinyl-4-(2-methoxyethoxy)-Phenyl | H | Methyl | |
| 1.1.1552 | 3-Cyano-4-(2-methoxyethoxy) - Phenyl | H | Methyl | |
| 1.1.1553 | 3-Trifluormethyl-4-(2-methoxyethoxy) - Phenyl | H | Methyl | |
| 1.1.1554 | 3-Methoxy-4-(2-methoxyethoxy) - Phenyl | H | Methyl | |
| 1.1.1555 | 3-Ethoxy-4-(2-methoxyethoxy)-Phenyl | H | Methyl | |
| 1.1.1556 | 3-Trifluormethoxy-(2-methoxyethoxy)-Phenyl | H | Methyl | |
| 1.1.1557 | 3-Nitro-4-(2-methoxyethoxy)-Phenyl | H | Methyl | |
| 1.1.1558 | 3-Fluor-4-(tert.butoxycarbonyloxy)-Phenyl | H | Methyl | |
| 1.1.1559 | 3-Chlor-4-(tert.butoxycarbonyloxy)-Phenyl | H | Methyl | |
| 1.1.1560 | 3-Brom-4-(tert.butoxycarbonyloxy)-Phenyl | H | Methyl | |
| 1.1.1561 | 3-Methyl-4-(tert.butoxycarbonyloxy)-Phenyl | H | Methyl | |
| 1.1.1562 | 3-Ethyl-4-(tert.butoxycarbonyloxy)-Phenyl | H | Methyl | |
| 1.1.1563 | 3-Cyclopropyl-4-(tert.butoxycarbonyloxy)-Phenyl | H | Methyl | |
| 1.1.1564 | 3-Vinyl-4-(tert.butoxycarbonyloxy)-Phenyl | H | Methyl | |
| 1.1.1565 | 3-Ethinyl-4-(tert.butoxycarbonyloxy)-Phenyl | H | Methyl | |
| 1.1.1566 | 3-Cyano-4-(tert.butoxycarbonyloxy)-Phenyl | H | Methyl | |
| 1.1.1567 | 3-Trifluormethyl-4-(tert.butoxycarbonyloxy)-Phenyl | H | Methyl | |
| 1.1.1568 | 3-Methoxy-4-(tert.butoxycarbonyloxy)-Phenyl | H | Methyl | |
| 1.1.1569 | 3-Ethoxy-4-(tert.butoxycarbonyloxy)-Phenyl | H | Methyl | |
| 1.1.1570 | 3-Trifluormethoxy-4-(tert.butoxycarbonyloxy)-Phenyl | H | Methyl | |
| 1.1.1571 | 2-Nitro-4-(tert.butoxycarbonyloxy)-Phenyl | H | Methyl | |
| 1.1.1572 | 3-Fluor-4-nitro-Phenyl | H | Methyl | |
| 1.1.1573 | 3-Chlor-4-nitro-Phenyl | H | Methyl | |
| 1.1.1574 | 3-Brom-4-nitro-Phenyl | H | Methyl | |
| 1.1.1575 | 3-Methyl-4-nitro-Phenyl | H | Methyl | |
| 1.1.1576 | 3-Ethyl-4-nitro-Phenyl | H | Methyl | |
| 1.1.1577 | 3-Cyclopropyl-4-nitro-Phenyl | H | Methyl | |
| 1.1.1578 | 3-Vinyl-4-nitro-Phenyl | H | Methyl | |
| 1.1.1579 | 3-Ethinyl-4-nitro-Phenyl | H | Methyl | |
| 1.1.1580 | 3-Cyano-4-nitro-Phenyl | H | Methyl | |
| 1.1.1581 | 3-Trifluormethyl-4-nitro-Phenyl | H | Methyl | |
| 1.1.1582 | 3-Methoxy-4-nitro-Phenyl | H | Methyl | |
| 1.1.1583 | 3-Ethoxy-4-nitro-Phenyl | H | Methyl | |
| 1.1.1584 | 3-Trifluormethoxy-4-nitro-Phenyl | H | Methyl | |
| 1.1.1585 | 3-Fluor-4-methylsulfanylPhenyl | H | Methyl | |
| 1.1.1586 | 3-Chlor-4-methylsulfanyl-Phenyl | H | Methyl | |
| 1.1.1587 | 3-Brom-4-methylsulfanyl-Phenyl | H | Methyl | |
| 1.1.1588 | 3-Methyl-4-methylsulfanyl-Phenyl | H | Methyl | |
| 1.1.1589 | 3-Ethyl-4-methylsulfanyl-Phenyl | H | Methyl | |
| 1.1.1590 | 3-Cyclopropyl-4-methylsulfanyl-Phenyl | H | Methyl | |
| 1.1.1591 | 3-Vinyl-4-methylsulfanyl-Phenyl | H | Methyl | |
| 1.1.1592 | 3-Ethinyl-4-methylsulfanyl-Phenyl | H | Methyl | |
| 1.1.1593 | 3-Cyano-4-methylsulfanyl -Phenyl | H | Methyl | |
| 1.1.1594 | 3-Trifluormethyl-4-methylsulfanyl -Phenyl | H | Methyl | |
| 1.1.1595 | 3-Methoxy-4-methylsulfanyl - Phenyl | H | Methyl | |
| 1.1.1596 | 3-Ethoxy-4-methylsulfanyl-Phenyl | H | Methyl | |
| 1.1.1597 | 3-Trifluormethoxy-4-methylsulfanyl-Phenyl | H | Methyl | |
| 1.1.1598 | 3-Nitro-4-methylsulfanyl-Phenyl | H | Methyl | |
| 1.1.1599 | 3,6-Difluor-Phenyl | H | Methyl | [CDCl₃] 1.78 (s, 3H); 3.39 (d, 1H); 3.91 (dd, 1H); 7.09 (m, 2H); 7.59 (m, 1H). |
| 1.1.1600 | 3,6-Difluor-Phenyl | H | Ethyl | |
| 1.1.1601 | 3,6-Difluor-Phenyl | H | isoPropyl | |
| 1.1.1602 | 3,6-Difluor-Phenyl | H | Cyclopropyl | |
| 1.1.1603 | 3-Chlor-6-fluor-Phenyl | H | Methyl | |
| 1.1.1604 | 3-Brom-6-fluor-Phenyl | H | Methyl | [CDCl₃] 1.75 (s, 3H); 3.35 (d, 1H); 3.90 (d, 1H); 7.00 (t, 3H); 7.51 (m, 1H); 8.0 (m, 1H). |
| 1.1.1605 | 3-Methyl-6-fluor-Phenyl | H | Methyl | |
| 1.1.1606 | 3-Ethyl-6-fluor-Phenyl | H | Methyl | |
| 1.1.1607 | 3-Cyclopropyl-6-fluor-Phenyl | H | Methyl | |
| 1.1.1608 | 3-Vinyl-6-fluor-Phenyl | H | Methyl | |
| 1.1.1609 | 3-Ethinyl-6-fluor-Phenyl | H | Methyl | |
| 1.1.1610 | 3-Cyano-6-fluor-Phenyl | H | Methyl | |
| 1.1.1611 | 3-Methoxy-6-fluor-Phenyl | H | Methyl | [DMSO] 1.53 (s, 3H); 3.39 (d, 1H); 3.75 (s, 3H); 3.81 (d, 1H); 7.06 (m, 1H); 7.18 (m, 1H); 7.38 (t, 1H). |
| 1.1.1612 | 3-Ethoxy-6-fluor-Phenyl | H | Methyl | |
| 1.1.1613 | 3-Trifluormethoxy-6-fluor-Phenyl | H | Methyl | |
| 1.1.1614 | 3-Nitro-6-fluor-Phenyl | H | Methyl | |
| 1.1.1615 | 3-Fluor-6-chlor-Phenyl | H | Methyl | [CDCl₃] 1.80 (s, 3H); 3.50 (d, 1H); 4.02 (d, 1H); 7.10 (m, 1H); 7.40 (m, 2H). |
| 1.1.1616 | 3-Fluor-6-chlor-Phenyl | H | Ethyl | |
| 1.1.1617 | 3-Fluor-6-chlor-Phenyl | H | isoPropyl | |
| 1.1.1618 | 3-Fluor-6-chlor-Phenyl | H | Cyclopropyl | |
| 1.1.1619 | 3,6-Dichlor-Phenyl | H | Methyl | [CDCl₃] 1.78 (s, 3 H); 3.48 (d, 1H); 3.97 (d, 1H); 7.35 (m, 2H); 7.67 (m, 1H). |
| 1.1.1620 | 3,6-Dichlor-Phenyl | H | Ethyl | |
| 1.1.1621 | 3,6-Dichlor-Phenyl | H | isoPropyl | |
| 1.1.1622 | 3,6-Dichlor-Phenyl | H | Cyclopropyl | |
| 1.1.1623 | 3-Brom-6-chlor-Phenyl | H | Methyl | |
| 1.1.1624 | 3-Methyl-6-chlor-Phenyl | H | Methyl | |
| 1.1.1625 | 3-Ethyl-6-chlor-Phenyl | H | Methyl | |
| 1.1.1626 | 3-Cyclopropyl-6-chlor-Phenyl | H | Methyl | |
| 1.1.1627 | 3-Vinyl-6-chlor-Phenyl | H | Methyl | |
| 1.1.1628 | 3-Ethinyl-6-chlor-Phenyl | H | Methyl | |
| 1.1.1629 | 3-Cyano-6-chlor-Phenyl | H | Methyl | |
| 1.1.1630 | 3-Trifluormethyl-6-chlor-Phenyl | H | Methyl | |
| 1.1.1631 | 3-Methoxy-6-chlor-Phenyl | H | Methyl | |
| 1.1.1632 | 3-Ethoxy-6-chlor-Phenyl | H | Methyl | |
| 1.1.1633 | 3-Trifluormethoxy-6-chlor-Phenyl | H | Methyl | |
| 1.1.1634 | 3-Nitro-6-chlor-Phenyl | H | Methyl | |
| 1.1.1635 | 3-Fluor-6-brom-Phenyl | H | Methyl | |
| 1.1.1636 | 3-Chlor-6-brom-Phenyl | H | Methyl | |
| 1.1.1637 | 3,6-Dibrom-Phenyl | H | Methyl | |
| 1.1.1638 | 3-Methyl-6-brom-Phenyl | H | Methyl | |
| 1.1.1639 | 3-Ethyl-6-brom-Phenyl | H | Methyl | |
| 1.1.1640 | 3-Cyclopropyl-6-brom-Phenyl | H | Methyl | |
| 1.1.1641 | 3-Vinyl-6-brom-Phenyl | H | Methyl | |
| 1.1.1642 | 3-Ethinyl-6-brom-Phenyl | H | Methyl | |
| 1.1.1643 | 3-Cyano-6-brom-Phenyl | H | Methyl | |
| 1.1.1644 | 3-Trifluormethyl-6-brom-Phenyl | H | Methyl | |
| 1.1.1645 | 3-Methoxy-6-Phenyl | H | Methyl | |
| 1.1.1646 | 3-Ethoxy-6-brom-Phenyl | H | Methyl | |
| 1.1.1647 | 3-Trifluormethoxy-6-brom-Phenyl | H | Methyl | |
| 1.1.1648 | 3-Nitro-6-brom-Phenyl | H | Methyl | |
| 1.1.1649 | 3-Fluor-6-iod-Phenyl | H | Methyl | |
| 1.1.1650 | 3-Chlor-6-iod-Phenyl | H | Methyl | |
| 1.1.1651 | 3-Brom-6-iod-Phenyl | H | Methyl | |
| 1.1.1652 | 3-Methyl-6-iod-Phenyl | H | Methyl | |
| 1.1.1653 | 3-Ethyl-6-iod-Phenyl | H | Methyl | |
| 1.1.1654 | 3-Cyclopropyl-6-iod-Phenyl | H | Methyl | |
| 1.1.1655 | 3-Vinyl-6-iod-Phenyl | H | Methyl | |
| 1.1.1656 | 3-Ethinyl-6-iod-Phenyl | H | Methyl | |
| 1.1.1657 | 3-Cyano-6-iod-Phenyl | H | Methyl | |
| 1.1.1658 | 3-Trifluormethyl-6-iod-Phenyl | H | Methyl | |
| 1.1.1659 | 3-Methoxy-6-iod-Phenyl | H | Methyl | |
| 1.1.1660 | 3-Ethoxy-6-iod-Phenyl | H | Methyl | |
| 1.1.1661 | 3-Trifluormethoxy-6-iod-Phenyl | H | Methyl | |
| 1.1.1662 | 3-Nitro-6-iod-Phenyl | H | Methyl | |
| 1.1.1663 | 3-Fluor-6-methyl-Phenyl | H | Methyl | |
| 1.1.1664 | 3-Chlor-6-methyl-Phenyl | H | Methyl | |
| 1.1.1665 | 3-Brom-6-methyl-Phenyl | H | Methyl | |
| 1.1.1666 | 3.6-Dimethyl-Phenyl | H | Methyl | [DMSO] 1.56 (s, 3H); 2.28 (s, 3H); 2.40 (s, 3H); 3.38 (d, 1H); 3.80 (d, 1H); 7.15 (m, 2H); 7.25 (d, 1H). |
| 1.1.1667 | 3-Ethyl-6-methyl-Phenyl | H | Methyl | |
| 1.1.1668 | 3-Cyclopropyl-6-methyl-Phenyl | H | Methyl | |
| 1.1.1669 | 3-Vinyl-6-methyl-Phenyl | H | Methyl | |
| 1.1.1670 | 3-Ethinyl-6-methyl-Phenyl | H | Methyl | |
| 1.1.1671 | 3-Cyano-6-methyl-Phenyl | H | Methyl | |
| 1.1.1672 | 3-Trifluormethyl-6-methyl-Phenyl | H | Methyl | |
| 1.1.1673 | 3-Methoxy-6-methyl-Phenyl | H | Methyl | |
| 1.1.1674 | 3-Ethoxy-6-methyl-Phenyl | H | Methyl | |
| 1.1.1675 | 3-Trifluormethoxy-6-methyl-Phenyl | H | Methyl | |
| 1.1.1676 | 3-Nitro-6-methyl-Phenyl | H | Methyl | |
| 1.1.1677 | 3-Fluor-6-ethyl-Phenyl | H | Methyl | |
| 1.1.1678 | 3-Chlor-6-ethyl-Phenyl | H | Methyl | |
| 1.1.1679 | 3-Brom-6-ethyl-Phenyl | H | Methyl | |
| 1.1.1680 | 3-Methyl-6-ethyl-Phenyl | H | Methyl | |
| 1.1.1681 | 3,6-Diethyl-Phenyl | H | Methyl | |
| 1.1.1682 | 3-Cyclopropyl-6-ethyl-Phenyl | H | Methyl | |
| 1.1.1683 | 3-Vinyl-6-ethyl-Phenyl | H | Methyl | |
| 1.1.1684 | 3-Ethinyl-6-ethyl-Phenyl | H | Methyl | |
| 1.1.1685 | 3-Cyano-6-ethyl-Phenyl | H | Methyl | |
| 1.1.1686 | 3-Trifluormethyl-6-ethyl-Phenyl | H | Methyl | |
| 1.1.1687 | 3-Methoxy-6-ethyl-Phenyl | H | Methyl | |
| 1.1.1688 | 3-Ethoxy-6-ethyl-Phenyl | H | Methyl | |
| 1.1.1689 | 3-Trifluormethoxy-6-ethyl-Phenyl | H | Methyl | |
| 1.1.1690 | 3-Nitro-6-ethyl-Phenyl | H | Methyl | |
| 1.1.1691 | 3-Fluor-6-propyl-Phenyl | H | Methyl | |
| 1.1.1692 | 3-Chlor-6-propyl-Phenyl | H | Methyl | |
| 1.1.1693 | 3-Brom-6-propyl-Phenyl | H | Methyl | |
| 1.1.1694 | 3-Methyl-6-propyl-Phenyl | H | Methyl | |
| 1.1.1695 | 3-Methyl-6-propyl-Phenyl | H | Methyl | |
| 1.1.1696 | 3-Cyclopropyl-6-propyl-Phenyl | H | Methyl | |
| 1.1.1697 | 3-Vinyl-6-propyl-Phenyl | H | Methyl | |
| 1.1.1698 | 3-Ethinyl-6-propyl-Phenyl | H | Methyl | |
| 1.1.1699 | 3-Cyano-6-propyl-Phenyl | H | Methyl | |
| 1.1.1700 | 3-Trifluormethyl-6-propyl-Phenyl | H | Methyl | |
| 1.1.1701 | 3-Methoxy-6-propyl-Phenyl | H | Methyl | |
| 1.1.1702 | 3-Ethoxy-6-propyl-Phenyl | H | Methyl | |
| 1.1.1703 | 3-Trifluormethoxy-6-propyl-Phenyl | H | Methyl | |
| 1.1.1704 | 3-Nitro-6-propyl-Phenyl | H | Methyl | |
| 1.1.1705 | 3-Fluor-6-isopropyl-Phenyl | H | Methyl | |
| 1.1.1706 | 3-Chlor-6-isopropyl-Phenyl | H | Methyl | |
| 1.1.1707 | 3-Brom-6-isopropyl-Phenyl | H | Methyl | |
| 1.1.1708 | 3-Methyl-6-isopropyl-Phenyl | H | Methyl | |
| 1.1.1709 | 3-Ethyl-6-isopropyl-Phenyl | H | Methyl | |
| 1.1.1710 | 3-Cyclopropyl-6-isopropyl-Phenyl | H | Methyl | |
| 1.1.1711 | 3-Vinyl-6-isopropyl-Phenyl | H | Methyl | |
| 1.1.1712 | 3-Ethinyl-6-isopropyl-Phenyl | H | Methyl | |
| 1.1.1713 | 3-Cyano-6-isopropyl-Phenyl | H | Methyl | |
| 1.1.1714 | 3-Trifluormethyl-6-isopropyl-Phenyl | H | Methyl | |
| 1.1.1715 | 3-Methoxy-6-isopropyl-Phenyl | H | Methyl | |
| 1.1.1716 | 3-Ethoxy-6-isopropyl-Phenyl | H | Methyl | |
| 1.1.1717 | 3-Trifluormethoxy-6-isopropyl-Phenyl | H | Methyl | |
| 1.1.1718 | 3-Nitro-6-isopropyl-Phenyl | H | Methyl | |
| 1.1.1719 | 3-Fluor-6-tert.butyl-Phenyl | H | Methyl | |
| 1.1.1720 | 3-Chlor-6-tert.buty - Phenyl | H | Methyl | |
| 1.1.1721 | 3-Brom-6-tert.butyl-Phenyl | H | Methyl | |
| 1.1.1722 | 3-Methyl-6-tert.butyl-Phenyl | H | Methyl | |
| 1.1.1723 | 3-Ethyl-6-tert.butyl-Phenyl | H | Methyl | |
| 1.1.1724 | 3-Cyclopropyl-6-tert.butyl-Phenyl | H | Methyl | |
| 1.1.1725 | 3-Vinyl-6-tert.butyl-Phenyl | H | Methyl | |
| 1.1.1726 | 3-Ethinyl-6-tert.butyl-Phenyl | H | Methyl | |
| 1.1.1727 | 3-Cyano-6-tert.butyl-Phenyl | H | Methyl | |
| 1.1.1728 | 3-Trifluormethyl-6-tert.butyl-Phenyl | H | Methyl | |
| 1.1.1729 | 3-Methoxy-6-tert.butyl -Phenyl | H | Methyl | |
| 1.1.1730 | 3-Ethoxy-6-tert.butyl -Phenyl | H | Methyl | |
| 1.1.1731 | 3-Trifluormethoxy-6-tert.butyl -Phenyl | H | Methyl | |
| 1.1.1732 | 3-Nitro-6-tert.butyl-Phenyl | H | Methyl | |
| 1.1.1733 | 3-Fluor-6-hydroxymethyl-Phenyl | H | Methyl | |
| 1.1.1734 | 3-Chlor-6-hydroxymethyl-Phenyl | H | Methyl | |
| 1.1.1735 | 3-Brom-6-hydroxymethyl - Phenyl | H | Methyl | |
| 1.1.1736 | 3-Methyl-6-hydroxymethyl - Phenyl | H | Methyl | |
| 1.1.1737 | 3-Ethyl-6-hydroxymethyl - Phenyl | H | Methyl | |
| 1.1.1738 | 3-Cyclopropyl-6-hydroxymethyl-Phenyl | H | Methyl | |
| 1.1.1739 | 3-Vinyl-6-hydroxymethyl - Phenyl | H | Methyl | |
| 1.1.1740 | 3-Ethinyl-6-hydroxymethyl-Phenyl | H | Methyl | |
| 1.1.1741 | 3-Cyano-6-hydroxymethyl - Phenyl | H | Methyl | |
| 1.1.1742 | 3-Trifluormethyl-6-hydroxymethyl-Phenyl | H | Methyl | |
| 1.1.1743 | 3-Methoxy-6-hydroxymethyl - Phenyl | H | Methyl | |
| 1.1.1744 | 3-Ethoxy-6-hydroxymethyl - Phenyl | H | Methyl | |
| 1.1.1745 | 3-Trifluormethoxy-6-hydroxymethylPhenyl | H | Methyl | |
| 1.1.1746 | 3-Nitro-6-hydroxymethyl-Phenyl | H | Methyl | |
| 1.1.1747 | 3-Fluor-6-cyclopropyl-Phenyl | H | Methyl | |
| 1.1.1748 | 3-Chlor-6-cyclopropyl-Phenyl | H | Methyl | |
| 1.1.1749 | 3-Brom-6-cyclopropyl-Phenyl | H | Methyl | |
| 1.1.1750 | 3-Methyl-6-cyclopropyl-Phenyl | H | Methyl | |
| 1.1.1751 | 3-Ethyl-6-cyclopro pyl -Phenyl | H | Methyl | |
| 1.1.1752 | 3-Cyclopropyl-6-cyclopropyl-Phenyl | H | Methyl | |
| 1.1.1753 | 3-Vinyl-6-cyclopropyl-Phenyl | H | Methyl | |
| 1.1.1754 | 3-Ethinyl-6-cyclopropyl-Phenyl | H | Methyl | |
| 1.1.1755 | 3-Cyano-6-cyclopropyl-Phenyl | H | Methyl | |
| 1.1.1756 | 3-Trifluormethyl-6-cyclopropyl-Phenyl | H | Methyl | |
| 1.1.1757 | 3-Methoxy-6-cyclopropyl-Phenyl | H | Methyl | |
| 1.1.1758 | 3-Ethoxy-6-cyclopropyl-Phenyl | H | Methyl | |
| 1.1.1759 | 3-Trifluormethoxy-6-cyclopropyl-Phenyl | H | Methyl | |
| 1.1.1760 | 3-Fluor-6-methoxycarbonyl-Phenyl | H | Methyl | |
| 1.1.1761 | 3-Chlor-6-methoxycarbonyl-Phenyl | H | Methyl | |
| 1.1.1762 | 3-Brom-6-methoxycarbonyl-Phenyl | H | Methyl | |
| 1.1.1763 | 3-Methyl-6-methoxycarbonyl-Phenyl | H | Methyl | |
| 1.1.1764 | 3-Ethyl-6-methoxycarbonyl-Phenyl | H | Methyl | |
| 1.1.1765 | 3-Cyclopropyl-6-methoxycarbonyl-Phenyl | H | Methyl | |
| 1.1.1766 | 3-Vinyl-6-methoxycarbonyl-Phenyl | H | Methyl | |
| 1.1.1767 | 3-Ethinyl-6-methoxycarbonyl-Phenyl | H | Methyl | |
| 1.1.1768 | 3-Cyano-6-methoxycarbonyl-Phenyl | H | Methyl | |
| 1.1.1769 | 3-Trifluormethyl-6-methoxycarbonyl-Phenyl | H | Methyl | |
| 1.1.1770 | 3-Methoxy-6-methoxycarbonyl - Phenyl | H | Methyl | |
| 1.1.1771 | 3-Ethoxy-6-methoxycarbonyl-Phenyl | H | Methyl | |
| 1.1.1772 | 3-Trifluormethoxy-6-methoxycarbonyl-Phenyl | H | Methyl | |
| 1.1.1773 | 3-Nitro-6-methoxycarbonyl-Phenyl | H | Methyl | |
| 1.1.1774 | 3-Fluor-6-vinyl-Phenyl | H | Methyl | |
| 1.1.1775 | 3-Chlor-6-vinyl-Phenyl | H | Methyl | |
| 1.1.1776 | 3-Brom-6-vinyl-Phenyl | H | Methyl | |
| 1.1.1777 | 3-Methyl-6-vinyl-Phenyl | H | Methyl | |
| 1.1.1778 | 3-Ethyl-6-vinyl-Phenyl | H | Methyl | |
| 1.1.1779 | 3-Cyclopropyl-6-vinyl-Phenyl | H | Methyl | |
| 1.1.1780 | 3,6-Divinyl-Phenyl | H | Methyl | |
| 1.1.1781 | 3-Ethinyl-6-vinyl-Phenyl | H | Methyl | |
| 1.1.1782 | 3-Cyano-6-vinyl-Phenyl | H | Methyl | |
| 1.1.1783 | 3-Trifluormethyl-6-vinyl-Phenyl | H | Methyl | |
| 1.1.1784 | 3-Methoxy-6-vinyl - Phenyl | H | Methyl | |
| 1.1.1785 | 3-Ethoxy-6-vinyl-Phenyl | H | Methyl | |
| 1.1.1786 | 3-Trifluormethoxy-6-vinyl-Phenyl | H | Methyl | |
| 1.1.1787 | 3-Nitro-6-vinyl-Phenyl | H | Methyl | |
| 1.1.1788 | 3-Fluor-6-ethinyl-Phenyl | H | Methyl | |
| 1.1.1789 | 3-Chlor-6-ethinyl-Phenyl | H | Methyl | |
| 1.1.1790 | 3-Brom-6-ethinyl - Phenyl | H | Methyl | |
| 1.1.1791 | 3-Methyl-6-ethinyl - Phenyl | H | Methyl | |
| 1.1.1792 | 3-Ethyl-6-ethinyl - Phenyl | H | Methyl | |
| 1.1.1793 | 3-Cyclopropyl-6-ethinyl-Phenyl | H | Methyl | |
| 1.1.1794 | 3-Vinyl-6-ethinyl-Phenyl | H | Methyl | |
| 1.1.1795 | 3-Cyano-6-ethinyl-Phenyl | H | Methyl | |
| 1.1.1796 | 3-Trifluormethyl-6-ethinyl-Phenyl | H | Methyl | |
| 1.1.1797 | 3-Methoxy-6-ethinyl-Phenyl | H | Methyl | |
| 1.1.1798 | 3-Ethoxy-6-ethinyl-Phenyl | H | Methyl | |
| 1.1.1799 | 3-Trifluormethoxy-6-ethinyl-Phenyl | H | Methyl | |
| 1.1.1800 | 3-Nitro-6-ethinyl-Phenyl | H | Methyl | |
| 1.1.1801 | 3-Fluor-6-ethinyl-Phenyl | H | Methyl | |
| 1.1.1802 | 3-Fluor-6-cyano-Phenyl | H | Methyl | |
| 1.1.1803 | 3-Chlor-6-cyano-Phenyl | H | Methyl | |
| 1.1.1804 | 3-Brom-6-cyano-Phenyl | H | Methyl | |
| 1.1.1805 | 3-Methyl-6-cyano-Phenyl | H | Methyl | |
| 1.1.1806 | 3-Ethyl-6-cyano-Phenyl | H | Methyl | |
| 1.1.1807 | 3-Cyclopropyl-6-cyano-Phenyl | H | Methyl | |
| 1.1.1808 | 3-Vinyl-6-cyano-Phenyl | H | Methyl | |
| 1.1.1809 | 3-Ethinyl-6-cyano-Phenyl | H | Methyl | |
| 1.1.1810 | 3-Cyano-6-cyanoPhenyl | H | Methyl | |
| 1.1.1811 | 3-Trifluormethyl-6-cyano-Phenyl | H | Methyl | |
| 1.1.1812 | 3-Methoxy-6-cyano - Phenyl | H | Methyl | |
| 1.1.1813 | 3-Ethoxy-6-cyano-Phenyl | H | Methyl | |
| 1.1.1814 | 3-Trifluormethoxy-6-cyano-Phenyl | H | Methyl | |
| 1.1.1815 | 3-Nitro-6-cyano-Phenyl | H | Methyl | |
| 1.1.1816 | 3-Fluor-6-hydroxyPhenyl | H | Methyl | |
| 1.1.1817 | 3-Chlor-6-hydroxyPhenyl | H | Methyl | |
| 1.1.1818 | 3-Brom-6-hydroxyPhenyl | H | Methyl | |
| 1.1.1819 | 3-Methyl-6-hydroxyPhenyl | H | Methyl | |
| 1.1.1820 | 3-Ethyl-6-hydroxyPhenyl | H | Methyl | |
| 1.1.1821 | 3-Cyclopropyl-6-hydroxy-Phenyl | H | Methyl | |
| 1.1.1822 | 3-Vinyl-6-hydroxyPhenyl | H | Methyl | |
| 1.1.1823 | 3-Ethinyl-6-hydroxyPhenyl | H | Methyl | |
| 1.1.1824 | 3-Cyano-6-hydroxy-Phenyl | H | Methyl | |
| 1.1.1825 | 3-Trifluormethyl-6-hydroxy-Phenyl | H | Methyl | |
| 1.1.1826 | 3-Methoxy-6-hydroxy-Phenyl | H | Methyl | |
| 1.1.1827 | 3-Ethoxy-6-hydroxyPhenyl | H | Methyl | |
| 1.1.1828 | 3-Trifluormethoxy-6-hydroxy-Phenyl | H | Methyl | |
| 1.1.1829 | 3-Nitro-6-hydroxyPhenyl | H | Methyl | |
| 1.1.1830 | 3-Fluor-6-methoxy-Phenyl | H | Methyl | |
| 1.1.1831 | 3-Chlor-6-methoxy-Phenyl | H | Methyl | |
| 1.1.1832 | 3-Brom-6-methoxy-Phenyl | H | Methyl | |
| 1.1.1833 | 3-Methyl-6-methoxy-Phenyl | H | Methyl | |
| 1.1.1834 | 3-Ethyl-6-methoxy-Phenyl | H | Methyl | |
| 1.1.1835 | 3-Cyclopropyl-6-methoxy-Phenyl | H | Methyl | |
| 1.1.1836 | 3-Vinyl-6-methoxy-Phenyl | H | Methyl | |
| 1.1.1837 | 3-Ethinyl-6-methoxy-Phenyl | H | Methyl | |
| 1.1.1838 | 3-Cyano-6-methoxy-Phenyl | H | Methyl | |
| 1.1.1839 | 3-Trifluormethyl-6-methoxy-Phenyl | H | Methyl | |
| 1.1.1840 | 3,6-Dimethoxy-Phenyl | H | Methyl | |
| 1.1.1841 | 3-Ethoxy-6-methoxy-Phenyl | H | Methyl | |
| 1.1.1842 | 3-Trifluormethoxy-6-methoxy-Phenyl | H | Methyl | |
| 1.1.1843 | 3-Nitro-6-methoxy-Phenyl | H | Methyl | |
| 1.1.1844 | 3-Fluor-6-ethoxy-Phenyl | H | Methyl | |
| 1.1.1845 | 3-Chlor-6-ethoxy-Phenyl | H | Methyl | |
| 1.1.1846 | 3-Brom-6-ethoxy-Phenyl | H | Methyl | |
| 1.1.1847 | 3-Methyl-6-ethoxy-Phenyl | H | Methyl | |
| 1.1.1848 | 3-Ethyl-6-ethoxy-Phenyl | H | Methyl | |
| 1.1.1849 | 3-Cyclopropyl-6-ethoxy-Phenyl | H | Methyl | |
| 1.1.1850 | 3-Vinyl-6-ethoxy-Phenyl | H | Methyl | |
| 1.1.1851 | 3-Ethinyl-6-ethoxy-Phenyl | H | Methyl | |
| 1.1.1852 | 3-Cyano-6-ethoxy-Phenyl | H | Methyl | |
| 1.1.1853 | 3-Trifluormethyl-6-ethoxy-Phenyl | H | Methyl | |
| 1.1.1854 | 3-Methoxy-6-ethoxy - Phenyl | H | Methyl | |
| 1.1.1855 | 2,6-Diethoxy-Phenyl | H | Methyl | |
| 1.1.1856 | 3-Trifluormethoxy-6-ethoxy-Phenyl | H | Methyl | |
| 1.1.1857 | 3-Nitro-6-ethoxy-Phenyl | H | Methyl | |
| 1.1.1858 | 3-Fluor-6-propoxy-Phenyl | H | Methyl | |
| 1.1.1859 | 3-Chlor-6-propoxy-Phenyl | H | Methyl | |
| 1.1.1860 | 3-Brom-6-propoxy-Phenyl | H | Methyl | |
| 1.1.1861 | 3-Methyl-6-propoxy-Phenyl | H | Methyl | |
| 1.1.1862 | 3-Ethyl-6-propoxy-Phenyl | H | Methyl | |
| 1.1.1863 | 3-Cyclopropyl-6-propoxy-Phenyl | H | Methyl | |
| 1.1.1864 | 3-Vinyl-6-propoxy-Phenyl | H | Methyl | |
| 1.1.1865 | 3-Ethinyl-6-propoxy-Phenyl | H | Methyl | |
| 1.1.1866 | 3-Cyano-6-propoxy-Phenyl | H | Methyl | |
| 1.1.1867 | 3-Trifluormethyl-6-propoxy-Phenyl | H | Methyl | |
| 1.1.1868 | 3-Methoxy-6-propoxy-Phenyl | H | Methyl | |
| 1.1.1869 | 3-Ethoxy-6-propoxy-Phenyl | H | Methyl | |
| 1.1.1870 | 3-Trifluormethoxy-6-propoxy-Phenyl | H | Methyl | |
| 1.1.1871 | 3-Nitro-6-propoxy-Phenyl | H | Methyl | |
| 1.1.1872 | 3-Fluor-6-isopropoxy-Phenyl | H | Methyl | |
| 1.1.1873 | 3-Chlor-6-isopropoxy-Phenyl | H | Methyl | |
| 1.1.1874 | 3-Brom-6-isopropoxy-Phenyl | H | Methyl | |
| 1.1.1875 | 3-Methyl-6-isopropoxy-Phenyl | H | Methyl | |
| 1.1.1876 | 3-Ethyl-6-isopropoxy-Phenyl | H | Methyl | |
| 1.1.1877 | 3-Cyclopropyl-6-isopropoxy-Phenyl | H | Methyl | |
| 1.1.1878 | 3-Vinyl-6-isopropoxy-Phenyl | H | Methyl | |
| 1.1.1879 | 3-Ethinyl-6-isopropoxy-Phenyl | H | Methyl | |
| 1.1.1880 | 3-Cyano-6-isopropoxy-Phenyl | H | Methyl | |
| 1.1.1881 | 3-Trifluormethyl-6-isopropoxy-Phenyl | H | Methyl | |
| 1.1.1882 | 3-Methoxy-6-isopropoxy-Phenyl | H | Methyl | |
| 1.1.1883 | 3-Ethoxy-6-isopropoxy-Phenyl | H | Methyl | |
| 1.1.1884 | 3-Trifluormethoxy-6-isopropoxy-Phenyl | H | Methyl | |
| 1.1.1885 | 3-Nitro-6-isopropoxy-Phenyl | H | Methyl | |
| 1.1.1886 | 3-Fluor-6-tert.butoxyPhenyl | H | Methyl | |
| 1.1.1887 | 3-Chlor-6-tert. butoxyPhenyl | H | Methyl | |
| 1.1.1888 | 3-Brom-6-tert.butoxyPhenyl | H | Methyl | |
| 1.1.1889 | 3-Methyl-6-tert.butoxy-Phenyl | H | Methyl | |
| 1.1.1890 | 3-Ethyl-6-tert.butoxyPhenyl | H | Methyl | |
| 1.1.1891 | 3-Cyclopropyl-6-tert.butoxy-Phenyl | H | Methyl | |
| 1.1.1892 | 3-Vinyl-6-tert.butoxyPhenyl | H | Methyl | |
| 1.1.1893 | 3-Ethinyl-6-tert.butoxy-Phenyl | H | Methyl | |
| 1.1.1894 | 3-Cyano-6-tert.butoxy-Phenyl | H | Methyl | |
| 1.1.1895 | 3-Trifluormethyl-6-tert.butoxy-Phenyl | H | Methyl | |
| 1.1.1896 | 3-Methoxy-6-tert.butoxy-Phenyl | H | Methyl | |
| 1.1.1897 | 3-Ethoxy-6-tert.butoxy-Phenyl | H | Methyl | |
| 1.1.1898 | 3-Trifluormethoxy-6-tert.butoxy-Phenyl | H | Methyl | |
| 1.1.1899 | 3-Nitro-6-tert.butoxyPhenyl | H | Methyl | |
| 1.1.1900 | 3-Fluor-6-trifluormethoxy-Phenyl | H | Methyl | |
| 1.1.1901 | 3-Chlor-6-trifluormethoxy-Phenyl | H | Methyl | |
| 1.1.1902 | 3-Brom-6-trifluormethoxy-Phenyl | H | Methyl | |
| 1.1.1903 | 3-Methyl-6-trifluormethoxy-Phenyl | H | Methyl | |
| 1.1.1904 | 3-Ethyl-6-trifluormethoxy-Phenyl | H | Methyl | |
| 1.1.1905 | 3-Cyclopropyl-6-trifluormethoxy-Phenyl | H | Methyl | |
| 1.1.1906 | 3-Vinyl-6-trifluormethoxy-Phenyl | H | Methyl | |
| 1.1.1907 | 3-Ethinyl-6-trifluormethoxy-Phenyl | H | Methyl | |
| 1.1.1908 | 3-Cyano-6-trifluormethoxy-Phenyl | H | Methyl | |
| 1.1.1909 | 3-Trifluormethyl-6-trifluormethoxy-Phenyl | H | Methyl | |
| 1.1.1910 | 3-Methoxy-6-trifluormethoxy-Phenyl | H | Methyl | |
| 1.1.1911 | 3-Ethoxy-6-trifluormethoxy-Phenyl | H | Methyl | |
| 1.1.1912 | 3,6-Bis(trifluormethoxy)-Phenyl | H | Methyl | |
| 1.1.1913 | 3-Nitro-6-trifluormethoxy-Phenyl | H | Methyl | |
| 1.1.1914 | 3-Fluor-6-(2,2,2-trifluorethoxy)-Phenyl | H | Methyl | |
| 1.1.1915 | 3-Chlor-6-(2,2,2-trifluorethoxy)-Phenyl | H | Methyl | |
| 1.1.1916 | 3-Brom-6-(2,2,2-trifluorethoxy)-Phenyl | H | Methyl | |
| 1.1.1917 | 3-Methyl-6-(2,2,2-trifluorethoxy)-Phenyl | H | Methyl | |
| 1.1.1918 | 3-Ethyl-6-(2,2,2-trifluorethoxy)-Phenyl | H | Methyl | |
| 1.1.1919 | 3-Cyclopropyl-6-(2,2,2-trifluorethoxy)-Phenyl | H | Methyl | |
| 1.1.1920 | 3-Vinyl-6-(2,2,2-trifluorethoxy)-Phenyl | H | Methyl | |
| 1.1.1921 | 3-Ethinyl-6-(2,2,2-trifluorethoxy-Phenyl | H | Methyl | |
| 1.1.1922 | 3-Cyano-6-(2,2,2-trifluorethoxy)-Phenyl | H | Methyl | |
| 1.1.1923 | 3-Trifluormethyl-6-(2,2,2-trifluorethoxy)-Phenyl | H | Methyl | |
| 1.1.1924 | 3-Methoxy-6-(2,2,2-trifluorethoxy)-Phenyl | H | Methyl | |
| 1.1.1925 | 3-Ethoxy-6-(2,2,2-trifluorethoxy)-Phenyl | H | Methyl | |
| 1.1.1926 | 3-Trifluormethoxy-6-(2,2,2-trifluorethoxy)-Phenyl | H | Methyl | |
| 1.1.1927 | 3-Nitro-6-(2,2,2-trifluorethoxy)-Phenyl | H | Methyl | |
| 1.1.1928 | 3-Fluor-6-difluormethoxy-Phenyl | H | Methyl | |
| 1.1.1929 | 3-Chlor-6-difluormethoxy-Phenyl | H | Methyl | |
| 1.1.1930 | 3-Brom-6-difluormethoxy-Phenyl | H | Methyl | |
| 1.1.1931 | 3-Methyl-6-difluormethoxy-Phenyl | H | Methyl | |
| 1.1.1932 | 3-Ethyl-6-difluormethoxy-Phenyl | H | Methyl | |
| 1.1.1933 | 3-Cyclopropyl-6-difluormethoxy-Phenyl | H | Methyl | |
| 1.1.1934 | 3-Vinyl-6-difluormethoxy-Phenyl | H | Methyl | |
| 1.1.1935 | 3-Ethinyl-6-difluormethoxy-Phenyl | H | Methyl | |
| 1.1.1936 | 3-Cyano-6-difluormethoxy - Phenyl | H | Methyl | |
| 1.1.1937 | 3-Trifluormethyl-6-difluormethoxy - Phenyl | H | Methyl | |
| 1.1.1938 | 3-Methoxy-6-difluormethoxy - Phenyl | H | Methyl | |
| 1.1.1939 | 3-Ethoxy-6-difluormethoxy-Phenyl | H | Methyl | |
| 1.1.1940 | 3-Trifluormethoxy-6-difluormethoxy-Phenyl | H | Methyl | |
| 1.1.1941 | 3-Nitro-6-difluormethoxy-Phenyl | H | Methyl | |
| 1.1.1942 | 3-Fluor-6-(2-methoxyethoxy)-Phenyl | H | Methyl | |
| 1.1.1943 | 3-Chlor-6-(2-methoxyethoxy)-Phenyl | H | Methyl | |
| 1.1.1944 | 3-Brom-6-(2-methoxyethoxy)-Phenyl | H | Methyl | |
| 1.1.1945 | 3-Methyl-6-(2-methoxyethoxy)-Phenyl | H | Methyl | |
| 1.1.1946 | 3-Ethyl-6-(2-methoxyethoxy)-Phenyl | H | Methyl | |
| 1.1.1947 | 3-Cyclopropyl-6-(2-methoxyethoxy)-Phenyl | H | Methyl | |
| 1.1.1948 | 3-Vinyl-6-(2-methoxyethoxy)-Phenyl | H | Methyl | |
| 1.1.1949 | 3-Ethinyl-6-(2-methoxyethoxy)-Phenyl | H | Methyl | |
| 1.1.1950 | 3-Cyano-6-(2-methoxyethoxy) - Phenyl | H | Methyl | |
| 1.1.1951 | 3-Trifluormethyl-6-(2-methoxyethoxy) - Phenyl | H | Methyl | |
| 1.1.1952 | 3-Methoxy-6-(2-methoxyethoxy) - Phenyl | H | Methyl | |
| 1.1.1953 | 3-Ethoxy-6-(2-methoxyethoxy)-Phenyl | H | Methyl | |
| 1.1.1954 | 3-Trifluormethoxy-(2-methoxyethoxy)-Phenyl | H | Methyl | |
| 1.1.1955 | 3-Nitro-6-(2-methoxyethoxy)-Phenyl | H | Methyl | |
| 1.1.1956 | 3-Fluor-6-(tert.butoxycarbonyloxy)-Phenyl | H | Methyl | |
| 1.1.1957 | 3-Chlor-6-(tert.butoxycarbonyloxy)-Phenyl | H | Methyl | |
| 1.1.1958 | 3-Brom-6-(tert.butoxycarbonyloxy)-Phenyl | H | Methyl | |
| 1.1.1959 | 3-Methyl-6-(tert.butoxycarbonyloxy)-Phenyl | H | Methyl | |
| 1.1.1960 | 3-Ethyl-6-(tert.butoxycarbonyloxy)-Phenyl | H | Methyl | |
| 1.1.1961 | 3-Cyclopropyl-6-(tert.butoxycarbonyloxy)-Phenyl | H | Methyl | |
| 1.1.1962 | 3-Vinyl-6-(tert.butoxycarbonyloxy)-Phenyl | H | Methyl | |
| 1.1.1963 | 3-Ethinyl-6-(tert.butoxycarbonyloxy)-Phenyl | H | Methyl | |
| 1.1.1964 | 3-Cyano-6-(tert.butoxycarbonyloxy)-Phenyl | H | Methyl | |
| 1.1.1965 | 3-Trifluormethyl-6-(tert.butoxycarbonyloxy)-Phenyl | H | Methyl | |
| 1.1.1966 | 3-Methoxy-6-(tert.butoxycarbonyloxy)-Phenyl | H | Methyl | |
| 1.1.1967 | 3-Ethoxy-6-(tert.butoxycarbonyloxy)-Phenyl | H | Methyl | |
| 1.1.1968 | 3-Trifluormethoxy-6-(tert.butoxycarbonyloxy)-Phenyl | H | Methyl | |
| 1.1.1969 | 2-Nitro-6-(tert.butoxycarbonyloxy)-Phenyl | H | Methyl | |
| 1.1.1970 | 3-Fluor-6-nitro-Phenyl | H | Methyl | |
| 1.1.1971 | 3-Chlor-6-nitro-Phenyl | H | Methyl | |
| 1.1.1972 | 3-Brom-6-nitro-Phenyl | H | Methyl | |
| 1.1.1973 | 3-Methyl-6-nitro-Phenyl | H | Methyl | |
| 1.1.1974 | 3-Ethyl-6-nitro-Phenyl | H | Methyl | |
| 1.1.1975 | 3-Cyclopropyl-6-nitro-Phenyl | H | Methyl | |
| 1.1.1976 | 3-Vinyl-6-nitro-Phenyl | H | Methyl | |
| 1.1.1977 | 3-Ethinyl-6-nitro-Phenyl | H | Methyl | |
| 1.1.1978 | 3-Cyano-6-nitro-Phenyl | H | Methyl | |
| 1.1.1979 | 3-Trifluormethyl-6-nitro-Phenyl | H | Methyl | |
| 1.1.1980 | 3-Methoxy-6-nitro-Phenyl | H | Methyl | |
| 1.1.1981 | 3-Ethoxy-6-nitro-Phenyl | H | Methyl | |
| 1.1.1982 | 3- Trifluormethoxy-6-nitro-Phenyl | H | Methyl | |
| 1.1.1983 | 3-Fluor-6-methylsulfanylPhenyl | H | Methyl | |
| 1.1.1984 | 3-Chlor-6-methylsulfanyl-Phenyl | H | Methyl | |
| 1.1.1985 | 3-Brom-6-methylsulfanyl-Phenyl | H | Methyl | |
| 1.1.1986 | 3-Methyl-6-methylsulfanyl-Phenyl | H | Methyl | |
| 1.1.1987 | 3-Ethyl-6-methylsulfanyl-Phenyl | H | Methyl | |
| 1.1.1988 | 3-Cyclopropyl-6-methylsulfanyl-Phenyl | H | Methyl | |
| 1.1.1989 | 3-Vinyl-6-methylsulfanyl-Phenyl | H | Methyl | |
| 1.1.1990 | 3-Ethinyl-6-methylsulfanyl-Phenyl | H | Methyl | |
| 1.1.1991 | 3-Cyano-6-methylsulfanyl - Phenyl | H | Methyl | |
| 1.1.1992 | 3-Trifluormethyl-6-methylsulfanyl - Phenyl | H | Methyl | |
| 1.1.1993 | 3-Methoxy-6-methylsulfanyl - Phenyl | H | Methyl | |
| 1.1.1994 | 3-Ethoxy-6-methylsulfanyl-Phenyl | H | Methyl | |
| 1.1.1995 | 3-Trifluormethoxy-6-methylsulfanyl-Phenyl | H | Methyl | |
| 1.1.1996 | 3-Nitro-6-methylsulfanyl-Phenyl | H | Methyl | |
| 1.1.1997 | 2,3,4-Trifluor-Phenyl | H | Methyl | [CDCl₃] 1.78 (s, 3H); 3.35 (dd, 1H); 3.90 (dd, 1H); 7.03 (m, 1H); 7.60 (m, 1H). |
| 1.1.1998 | 2,3,4-Trichlor-Phenyl | H | Methyl | |
| 1.1.1999 | 2,3.4-Trimethyl-Phenyl | H | Methyl | |
| 1.1.2000 | 2-Fluor-2-chlor-5-trifluormethyl-Phenyl | H | Methyl | |
| 1.1.2001 | 2,3,5-Trifluor-Phenyl | H | Methyl | [DMSO] 1.58 (s, 3H); 3.41 (d, 1H); 3.71 (d, 1H); 7.41 (m, 1H); 7.71 (m, 1H). |
| 1.1.2002 | 2,3,5-Trichlor-Phenyl | H | Methyl | |
| 1.1.2003 | 2,3,5-TrimethylPhenyl | H | Methyl | |
| 1.1.2004 | 2,3-Dichlor-5-methoxy-Phenyl | H | Methyl | [CDCl₃] 1.7.(s, 3H); 3,5 (d, 1 H); 3.7 (s, 3H); 3,95 (d, 1 H); 7.02-7.13 (m, 2 H). |
| 1.1.2005 | 2,3,6-Trifluor-Phenyl | H | Methyl | |
| 1.1.2006 | 2,3,6-Trichlor-Phenyl | H | Methyl | [CDCl₃] 1.85 (s, 3H); 3.26 (d, 1H); 3.78 (d, 1H); 7.33 (d, 1H); 7.49 (d, 1H). |
| 1.1.2007 | 2,3,6- TrimethylPhenyl | H | Methyl | |
| 1.1.2008 | 3,4,5-Trifluor-Phenyl | H | Methyl | [CDCl₃] 1.80 (s, 3H); 3.22 (d, 1H); 3.80 (d, 1H); 7.30 (m, 2H). |
| 1.1.2009 | 3,4,5-Trichlor-Phenyl | H | Methyl | [CDCl₃] 1.80 (s, 3H); 3.25 (d, 1H); 3.85 (d, 1H); 7.65 (, 2H). |
| 1.1.2010 | 3,4,5-TrimethylPhenyl | H | Methyl | |
| 1.1.2011 | 3, 5-Dimethyl-4-fluor-Phenyl | H | Methyl | |
| 1.1.2012 | 3,5-Dichlor-4-methoxy-Phenyl | H | Methyl | [CDCl₃] 1.81 (s, 3H); 3.25 (d, 1H); 3.82 (d, 1H); 3.95 (s, 3H); 7.60 (s, 2H). |
| 1.1.2013 | 3,5-Difluor-4-chlor-Phenyl | H | Methyl | [CDCl3] 1.81 (s, 3H); 3.24 (d, 1H); 3.83 (d, 1H); 7.27 (m, 3H). |
| 1.1.2014 | 3,5-Dichlor-4-hydroxy-Phenyl | H | Methyl | [CDCl₃] 1.79 (s, 3H); 3.25 (d, 1H); 3.80 (d, 1H); 7.59 (s, 2H). |
| 1.1.2015 | 3,5- Trifluormethyl-4-chlor-Phenyl | H | Methyl | |
| 1.1.2016 | 3,4,6-Trifluor-Phenyl | H | Methyl | |
| 1.1.2017 | 3,4,6-Trichlor-Phenyl | H | Methyl | |
| 1.1.2018 | 3,4,6-TrimethylPhenyl | H | Methyl | |
| 1.1.2019 | Pentafluorphenyl | H | Methyl | [CDCl₃] 1.80 (s, 3H); 3.34 (d, 1H); 3.9 (d, 1H). |

**Tabelle 1.2: Erfindungsgemäße Verbindungen der allgemeinen Formel (I), worin W* für COOH, R¹ und R² für Wasserstoff stehen, und Aryl den Rest**

| | | | |
|---|---|---|---|
| | 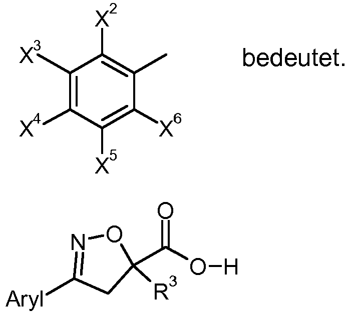 | | |

| Nr. | Aryl | R³ | Physikalische Daten |
|---|---|---|---|
| 1.2.001 | 3-Fluor-Phenyl | Fluormethyl | [CDCl₃] 3.56 (d,1H); 3.80 (d, 1H) 4.71 (d,1H) 4.83 (d,1H); 7.18 (m,1H); 7.41 (m,3H). |
| 1.2.002 | 3-Fluor-Phenyl | Chlormethyl | [CDCl₃] 3.62 (d,1H); 3.90 (m,2H); 4.03 (d,1H); 7.17 (m,1H); 7.41 (m,3H). |
| 1.2.003 | 3-Fluor-Phenyl | Brommethyl | |
| 1.2.004 | 3-Fluor-Phenyl | Difluormethyl | |
| 1.2.005 | 3-Fluor-Phenyl | Trifluormethyl | |
| 1.2.006 | 3-Fluor-Phenyl | Cyano | |
| 1.2.007 | 3-Chlor-Phenyl | Fluormethyl | |
| 1.2.008 | 3-Chlor-Phenyl | Chlormethyl | |
| 1.2.009 | 3-Chlor-Phenyl | Brommethyl | |
| 1.2.010 | 3-Chlor-Phenyl | Difluormethyl | |
| 1.2.011 | 3-Chlor-Phenyl | Trifluormethyl | |
| 1.2.012 | 3-Chlor-Phenyl | Cyano | |
| 1.2.013 | 3-Brom-Phenyl | Fluormethyl | |
| 1.2.014 | 3-Brom-Phenyl | Chlormethyl | |
| 1.2.015 | 3-Iod-Phenyl | Fluormethyl | |
| 1.2.016 | 3-Iod-Phenyl | Chlormethyl | |
| 1.2.017 | 3-Methyl-Phenyl | Fluormethyl | |
| 1.2.018 | 3-Methyl-Phenyl | Chlormethyl | |
| 1.2.019 | 3-Ethyl-Phenyl | Fluormethyl | |
| 1.2.020 | 3-Propyl-Phenyl | Fluormethyl | |
| 1.2.021 | 3-isoPropyl-Phenyl | Fluormethyl | |
| 1.2.022 | 3-nButyl-Phenyl | Fluormethyl | |
| 1.2.023 | 3-iButyl-Phenyl | Fluormethyl | |
| 1.2.024 | 3-tert.Butyl-Phenyl | Fluormethyl | |
| 1.2.025 | 3-Cyclopropyl-Phenyl | Fluormethyl | |
| 1.2.026 | 3-Cyclobutyl-Phenyl | Fluormethyl | |
| 1.2.027 | 3-Cyclopentyl-Phenyl | Fluormethyl | |
| 1.2.028 | 3-Vinyl-Phenyl | Fluormethyl | |
| 1.2.029 | 3-Ethinyl-Phenyl | Fluormethyl | |
| 1.2.030 | 3-Cyano-Phenyl | Fluormethyl | |
| 1.2.031 | 3-Trifluormethyl-Phenyl | Fluormethyl | |
| 1.2.032 | 3-Difluormethyl-Phenyl | Fluormethyl | |
| 1.2.033 | 3-(Hydroxycarbonyl)-Phenyl | Fluormethyl | |
| 1.2.034 | 3-(Methoxycarbony)l-Phenyl | Fluormethyl | |
| 1.2.035 | 3-(Ethoxycarbonyl)-Phenyl | Fluormethyl | |
| 1.2.036 | 3-Hydroxymethyl-Phenyl | Fluormethyl | |
| 1.2.037 | 3-Carbamoyl-Phenyl | Fluormethyl | |
| 1.2.038 | 3-Hydroxy-Phenyl- | Fluormethyl | |
| 1.2.039 | 3-Methoxy-Phenyl | Fluormethyl | |
| 1.2.040 | 3-Ethoxy-Phenyl | Fluormethyl | |
| 1.2.041 | 3-Propyloxy-Phenyl | Fluormethyl | |
| 1.2.042 | 3-isoPropyloxy-Phenyl | Fluormethyl | |
| 1.2.043 | 3-nButyloxy-Phenyl | Fluormethyl | |
| 1.2.044 | 3-iButyloxy-Phenyl | Fluormethyl | |
| 1.2.045 | 3-tButyloxy-Phenyl | Fluormethyl | |
| 1.2.046 | 3-Difluormethoxy-Phenyl | Fluormethyl | |
| 1.2.047 | 3-Trifluormethoxy-Phenyl | Fluormethyl | |
| 1.2.048 | 3-(2,2,2-Trifluorethoxy)-Phenyl | Fluormethyl | |
| 1.2.049 | 3-(2-Chlorethoxy)-Phenyl | Fluormethyl | |
| 1.2.050 | 3-(2-Hydroxyethoxy)-Phenyl- | Fluormethyl | |
| 1.2.051 | 3-(2-Methoxyethoxy)-Phenyl- | Fluormethyl | |
| 1.2.052 | 3-[(tert-Butoxycarbonyl)oxy]-Phenyl | Fluormethyl | |
| 1.2.053 | 3-Nitro-Phenyl | Fluormethyl | |
| 1.2.054 | 3-Acetoxy-Phenyl | Fluormethyl | |
| 1.2.055 | {3-[(tert-Butoxycarbonyl)amino]-Phenyl}- | Fluormethyl | |
| 1.2.056 | 3-Methylsulfanyl-Phenyl | Fluormethyl | |
| 1.2.057 | 3-Ethylsulfanyl-Phenyl | Fluormethyl | |
| 1.2.058 | 3-(Pentafluor-lambda⁶-sulfanyl)-Phenyl | Fluormethyl | |
| 1.2.059 | 2,3-Difluor-Phenyl | Fluormethyl | |
| 1.2.060 | 2,3-Difluor-Phenyl | Chlormethyl | |
| 1.2.061 | 2,3-Difluor-Phenyl | Brommethyl | |
| 1.2.062 | 2,3-Difluor-Phenyl | Difluormethyl | |
| 1.2.063 | 2,3-Difluor-Phenyl | Trifluormethyl | |
| 1.2.064 | 2,3-Difluor-Phenyl | Cyano | |
| 1.2.065 | 2-Chlor-3-fluor-Phenyl | Fluormethyl | |
| 1.2.066 | 2-Brom-3-fluor-Phenyl | Fluormethyl | |
| 1.2.067 | 2-Methyl-3-fluor-Phenyl | Fluormethyl | |
| 1.2.068 | 2-Ethyl-3-fluor-Phenyl | Fluormethyl | |
| 1.2.069 | 2-Cyclopropyl-3-fluor-Phenyl | Fluormethyl | |
| 1.2.070 | 2-Vinyl-3-fluor-Phenyl | Fluormethyl | |
| 1.2.071 | 2-Ethinyl-3-fluor-Phenyl | Fluormethyl | |
| 1.2.072 | 2-Cyano-3-fluor-Phenyl | Fluormethyl | |
| 1.2.073 | 2-Methoxy-3-fluor-Phenyl | Fluormethyl | |
| 1.2.074 | 2-Ethoxy-3-fluor-Phenyl | Fluormethyl | |
| 1.2.075 | 2-Trifluormethoxy-3-fluor-Phenyl | Fluormethyl | |
| 1.2.076 | 2-Nitro-3-fluor-Phenyl | Fluormethyl | |
| 1.2.077 | 2-Fluor-3-chlor-Phenyl | Fluormethyl | |
| 1.2.078 | 2,3-Dichlor-Phenyl | Fluormethyl | |
| 1.2.079 | 2,3-Dichlor-Phenyl | Chlormethyl | |
| 1.2.080 | 2,3-Dichlor-Phenyl | Brommethyl | |
| 1.2.081 | 2,3-Dichlor-Phenyl | Difluormethyl | |
| 1.2.082 | 2-Brom-3-chlor-Phenyl | Fluormethyl | |
| 1.2.083 | 2-Methyl-3-chlor-Phenyl | Fluormethyl | |
| 1.2.084 | 2-Ethyl-3-chlor-Phenyl | Fluormethyl | |
| 1.2.085 | 2-Cyclopropyl-3-chlor-Phenyl | Fluormethyl | |
| 1.2.086 | 2-Vinyl-3-chlor-Phenyl | Fluormethyl | |
| 1.2.087 | 2-Ethinyl-3-chlor-Phenyl | Fluormethyl | |
| 1.2.088 | 2-Cyano-3-chlor-Phenyl | Fluormethyl | |
| 1.2.089 | 2-Trifluormethyl-2-chlor-Phenyl | Fluormethyl | |
| 1.2.090 | 2-Methoxy-3-chlor-Phenyl | Fluormethyl | |
| 1.2.091 | 2-Ethoxy-3-chlor-Phenyl | Fluormethyl | |
| 1.2.092 | 2-Trifluormethoxy-3-chlor-Phenyl | Fluormethyl | |
| 1.2.093 | 2-Nitro-3-chlor-Phenyl | Fluormethyl | |
| 1.2.094 | 2-Fluor-3-brom-Phenyl | Fluormethyl | |
| 1.2.095 | 2-Chlor-3-brom-Phenyl | Fluormethyl | |
| 1.2.096 | 2,3-Dibrom-Phenyl | Fluormethyl | |
| 1.2.097 | 2-Methyl-3-brom-Phenyl | Fluormethyl | |
| 1.2.098 | 2-Ethyl-3-brom-Phenyl | Fluormethyl | |
| 1.2.099 | 2-Cyclopropyl-3-brom-Phenyl | Fluormethyl | |
| 1.2.100 | 2-Vinyl-3-brom-Phenyl | Fluormethyl | |
| 1.2.101 | 2-Ethinyl-3-brom-Phenyl | Fluormethyl | |
| 1.2.102 | 2-Cyano-3-brom-Phenyl | Fluormethyl | |
| 1.2.103 | 2-Trifluormethyl-3-brom-Phenyl | Fluormethyl | |
| 1.2.104 | 2-Methoxy-3-Phenyl | Fluormethyl | |
| 1.2.105 | 2-Ethoxy-3-brom-Phenyl | Fluormethyl | |
| 1.2.106 | 2-Trifluormethoxy-3-brom-Phenyl | Fluormethyl | |
| 1.2.107 | 2-Nitro-3-brom-Phenyl | Fluormethyl | |
| 1.2.108 | 2-Fluor-3-iod-Phenyl | Fluormethyl | |
| 1.2.109 | 2-Chlor-3-iod-Phenyl | Fluormethyl | |
| 1.2.110 | 2-Brom-3-iod-Phenyl | Fluormethyl | |
| 1.2.111 | 2-Methyl-3-iod-Phenyl | Fluormethyl | |
| 1.2.112 | 2-Ethyl-3-iod-Phenyl | Fluormethyl | |
| 1.2.113 | 2-Cyclopropyl-3-iod-Phenyl | Fluormethyl | |
| 1.2.114 | 2-Vinyl-3-iod-Phenyl | Fluormethyl | |
| 1.2.115 | 2-Ethinyl-3-iod-Phenyl | Fluormethyl | |
| 1.2.116 | 2-Cyano-3-iod-Phenyl | Fluormethyl | |
| 1.2.117 | 2-Trifluormethyl-3-iod-Phenyl | Fluormethyl | |
| 1.2.118 | 2-Methoxy-3-iod-Phenyl | Fluormethyl | |
| 1.2.119 | 2-Ethoxy-3-iod-Phenyl | Fluormethyl | |
| 1.2.120 | 2-Trifluormethoxy-3-iod-Phenyl | Fluormethyl | |
| 1.2.121 | 2-Nitro-3-iod-Phenyl | Fluormethyl | |
| 1.2.122 | 2-Fluor-3-methyl-Phenyl | Fluormethyl | |
| 1.2.123 | 2-Fluor-3-methyl-Phenyl | Chlormethyl | |
| 1.2.124 | 2-Fluor-3-methyl-Phenyl | Brommethyl | |
| 1.2.125 | 2-Fluor-3-methyl-Phenyl | Difluormethyl | |
| 1.2.126 | 2-Chlor-3-methyl-Phenyl | Fluormethyl | |
| 1.2.127 | 2-Chlor-3-methyl-Phenyl | Chlormethyl | |
| 1.2.128 | 2-Chlor-3-methyl-Phenyl | Brommethyl | |
| 1.2.129 | 2-Chlor-3-methyl-Phenyl | Difluormethyl | |
| 1.2.130 | 2-Brom-3-methyl-Phenyl | Fluormethyl | |
| 1.2.131 | 2,3-Dimethyl-Phenyl | Fluormethyl | |
| 1.2.132 | 2,3-Dimethyl-Phenyl | Chlormethyl | |
| 1.2.133 | 2,3-Dimethyl-Phenyl | Brommethyl | |
| 1.2.134 | 2,3-Dimethyl-Phenyl | Difluormethyl | |
| 1.2.135 | 2-Ethyl-3-methyl-Phenyl | Fluormethyl | |
| 1.2.136 | 2-Cyclopropyl-3-methyl-Phenyl | Fluormethyl | |
| 1.2.137 | 2-Vinyl-3-methyl-Phenyl | Fluormethyl | |
| 1.2.138 | 2-Ethinyl-3-methyl-Phenyl | Fluormethyl | |
| 1.2.139 | 2-Cyano-3-methyl-Phenyl | Fluormethyl | |
| 1.2.140 | 2-Trifluormethyl-3-methyl-Phenyl | Fluormethyl | |
| 1.2.141 | 2-Methoxy-3-methyl-Phenyl | Fluormethyl | |
| 1.2.142 | 2-Ethoxy-3-methyl-Phenyl | Fluormethyl | |
| 1.2.143 | 2-Trifluormethoxy-3-methyl-Phenyl | Fluormethyl | |
| 1.2.144 | 2-Nitro-3-methyl-Phenyl | Fluormethyl | |
| 1.2.145 | 2-Fluor-3-ethyl-Phenyl | Fluormethyl | |
| 1.2.146 | 2-Chlor-3-ethyl-Phenyl | Fluormethyl | |
| 1.2.147 | 2-Brom-3-ethyl-Phenyl | Fluormethyl | |
| 1.2.148 | 2-Methyl-3-ethyl-Phenyl | Fluormethyl | |
| 1.2.149 | 2,3-Diethyl-Phenyl | Fluormethyl | |
| 1.2.150 | 2-Cyclopropyl-3-ethyl-Phenyl | Fluormethyl | |
| 1.2.151 | 2-Vinyl-3-ethyl-Phenyl | Fluormethyl | |
| 1.2.152 | 2-Ethinyl-3-ethyl-Phenyl | Fluormethyl | |
| 1.2.153 | 2-Cyano-3-ethyl-Phenyl | Fluormethyl | |
| 1.2.154 | 2-Trifluormethyl-3-ethyl-Phenyl | Fluormethyl | |
| 1.2.155 | 2-Methoxy-3-ethyl-Phenyl | Fluormethyl | |
| 1.2.156 | 2-Ethoxy-3-ethyl-Phenyl | Fluormethyl | |
| 1.2.157 | 2-Trifluormethoxy-3-ethyl-Phenyl | Fluormethyl | |
| 1.2.158 | 2-Nitro-3-ethyl-Phenyl | Fluormethyl | |
| 1.2.159 | 2-Fluor-3-propyl-Phenyl | Fluormethyl | |
| 1.2.160 | 2-Chlor-3-propyl-Phenyl | Fluormethyl | |
| 1.2.161 | 2-Brom-3-propyl-Phenyl | Fluormethyl | |
| 1.2.162 | 2-Methyl-3-propyl-Phenyl | Fluormethyl | |
| 1.2.163 | 2-Methyl-3-propyl-Phenyl | Fluormethyl | |
| 1.2.164 | 2-Cyclopropyl-3-propyl-Phenyl | Fluormethyl | |
| 1.2.165 | 2-Vinyl-3-propyl-Phenyl | Fluormethyl | |
| 1.2.166 | 2-Ethinyl-3propyl-Phenyl | Fluormethyl | |
| 1.2.167 | 2-Cyano-3-propyl-Phenyl | Fluormethyl | |
| 1.2.168 | 2-Trifluormethyl-3-propyl-Phenyl | Fluormethyl | |
| 1.2.169 | 2-Methoxy-3-propyl-Phenyl | Fluormethyl | |
| 1.2.170 | 2-Ethoxy-3-propyl-Phenyl | Fluormethyl | |
| 1.2.171 | 2-Trifluormethoxy-3-propyl-Phenyl | Fluormethyl | |
| 1.2.172 | 2-Nitro-3-propyl-Phenyl | Fluormethyl | |
| 1.2.173 | 2-Fluor-3-isopropyl-Phenyl | Fluormethyl | |
| 1.2.174 | 2-Chlor-3-isopropyl-Phenyl | Fluormethyl | |
| 1.2.175 | 2-Brom-3-isopropyl-Phenyl | Fluormethyl | |
| 1.2.176 | 2-Methyl-3-isopropyl-Phenyl | Fluormethyl | |
| 1.2.177 | 2-Ethyl-3-isopropyl-Phenyl | Fluormethyl | |
| 1.2.178 | 2-Cyclopropyl-3-isopropyl-Phenyl | Fluormethyl | |
| 1.2.179 | 2-Vinyl-3-isopropyl-Phenyl | Fluormethyl | |
| 1.2.180 | 2-Ethinyl-3-isopropyl-Phenyl | Fluormethyl | |
| 1.2.181 | 2-Cyano-3-isopropyl-Phenyl | Fluormethyl | |
| 1.2.182 | 2-Trifluormethyl-3-isopropyl-Phenyl | Fluormethyl | |
| 1.2.183 | 2-Methoxy-3-isopropyl-Phenyl | Fluormethyl | |
| 1.2.184 | 2-Ethoxy-3-isopropyl-Phenyl | Fluormethyl | |
| 1.2.185 | 2-Trifluormethoxy-3-isopropylPhenyl | Fluormethyl | |
| 1.2.186 | 2-Nitro-3-isopropyl-Phenyl | Fluormethyl | |
| 1.2.187 | 2-Fluor-3-tert.butyl-Phenyl | Fluormethyl | |
| 1.2.188 | 2-Chlor-3-tert.buty-Phenyl | Fluormethyl | |
| 1.2.189 | 2-Brom-3-tert.butyl-Phenyl | Fluormethyl | |
| 1.2.190 | 2-Methyl-3-tert.butyl-Phenyl | Fluormethyl | |
| 1.2.191 | 2-Ethyl-3-tert.butyl-Phenyl | Fluormethyl | |
| 1.2.192 | 2-Cyclopropyl-3-tert.butyl-Phenyl | Fluormethyl | |
| 1.2.193 | 2-Vinyl-3-tert.butyl-Phenyl | Fluormethyl | |
| 1.2.194 | 2-Ethinyl-3-tert.butyl-Phenyl | Fluormethyl | |
| 1.2.195 | 2-Cyano-3-tert.butyl-Phenyl | Fluormethyl | |
| 1.2.196 | 2-Trifluormethyl-3-tert.butyl-Phenyl | Fluormethyl | |
| 1.2.197 | 2-Methoxy-3-tert.butyl-Phenyl | Fluormethyl | |
| 1.2.198 | 2-Ethoxy-3-tert.butyl-Phenyl | Fluormethyl | |
| 1.2.199 | 2-Trifluormethoxy-3-tert.butyl-Phenyl | Fluormethyl | |
| 1.2.200 | 2-Nitro-3-tert.butyl-Phenyl | Fluormethyl | |
| 1.2.201 | 2-Fluor-3-hydroxymethyl-Phenyl | Fluormethyl | |
| 1.2.202 | 2-Chlor-3-hydroxymethyl-Phenyl | Fluormethyl | |
| 1.2.203 | 2-Brom-3-hydroxymethyl-Phenyl | Fluormethyl | |
| 1.2.204 | 2-Methyl-3-hydroxymethyl-Phenyl | Fluormethyl | |
| 1.2.205 | 2-Ethyl-3-hydroxymethyl-Phenyl | Fluormethyl | |
| 1.2.206 | 2-Cyclopropyl-3-hydroxymethyl-Phenyl | Fluormethyl | |
| 1.2.207 | 2-Vinyl-3-hydroxymethyl-Phenyl | Fluormethyl | |
| 1.2.208 | 2-Ethinyl-3-hydroxymethyl-Phenyl | Fluormethyl | |
| 1.2.209 | 2-Cyano-3-hydroxymethyl-Phenyl | Fluormethyl | |
| 1.2.210 | 2-Trifluormethyl-3-hydroxymethyl-Phenyl | Fluormethyl | |
| 1.2.211 | 2-Methoxy-3-hydroxymethyl-Phenyl | Fluormethyl | |
| 1.2.212 | 2-Ethoxy-3-hydroxymethyl-Phenyl | Fluormethyl | |
| 1.2.213 | 2-Trifluormethoxy-3-hydroxymethyl--Phenyl | Fluormethyl | |
| 1.2.214 | 2-Nitro-3-hydroxymethyl-Phenyl | Fluormethyl | |
| 1.2.215 | 2-Fluor-3-cyclopropyl-Phenyl | Fluormethyl | |
| 1.2.216 | 2-Chlor-3-cyclopropyl-Phenyl | Fluormethyl | |
| 1.2.217 | 2-Brom-3-cyclopropyl-Phenyl | Fluormethyl | |
| 1.2.218 | 2-Methyl-3-cyclopropyl-Phenyl | Fluormethyl | |
| 1.2.219 | 2-Ethyl-3-cyclopropyl-Phenyl | Fluormethyl | |
| 1.2.220 | 2-Cyclopropyl-3-cyclopropyl-Phenyl | Fluormethyl | |
| 1.2.221 | 2-Vinyl-3-cyclopropyl-Phenyl | Fluormethyl | |
| 1.2.222 | 2-Ethinyl-3-cyclopropyl-Phenyl | Fluormethyl | |
| 1.2.223 | 2-Cyano-3-cyclopropyl-Phenyl | Fluormethyl | |
| 1.2.224 | 2-Trifluormethyl-3-cyclopropyl-Phenyl | Fluormethyl | |
| 1.2.225 | 2-Methoxy-3-cyclopropyl-Phenyl | Fluormethyl | |
| 1.2.226 | 2-Ethoxy-3-cyclopropyl-Phenyl | Fluormethyl | |
| 1.2.227 | 2-Trifluormethoxy-3-cyclopropyl-Phenyl | Fluormethyl | |
| 1.2.228 | 2-Fluor-3-methoxycarbonyl-Phenyl | Fluormethyl | |
| 1.2.229 | 2-Chlor-3-methoxycarbonyl-Phenyl | Fluormethyl | |
| 1.2.230 | 2-Brom-3-methoxycarbonyl-Phenyl | Fluormethyl | |
| 1.2.231 | 2-Methyl-3-methoxycarbonyl-Phenyl | Fluormethyl | |
| 1.2.232 | 2-Ethyl-3-methoxycarbonyl-Phenyl | Fluormethyl | |
| 1.2.233 | 2-Cyclopropyl-3-methoxycarbonyl-Phenyl | Fluormethyl | |
| 1.2.234 | 2-Vinyl-3-methoxycarbonyl-Phenyl | Fluormethyl | |
| 1.2.235 | 2-Ethinyl-3-methoxycarbonyl-Phenyl | Fluormethyl | |
| 1.2.236 | 2-Cyano-3-methoxycarbonyl-Phenyl | Fluormethyl | |
| 1.2.237 | 2-Trifluormethyl-3-methoxycarbonyl-Phenyl | Fluormethyl | |
| 1.2.238 | 2-Methoxy-3-methoxycarbonyl-Phenyl | Fluormethyl | |
| 1.2.239 | 2-Ethoxy-3-methoxycarbonyl-Phenyl | Fluormethyl | |
| 1.2.240 | 2-Trifluormethoxy-3-methoxycarbonyl-Phenyl | Fluormethyl | |
| 1.2.241 | 2-Nitro-3-methoxycarbonyl-Phenyl | Fluormethyl | |
| 1.2.242 | 2-Fluor-3-vinyl-Phenyl | Fluormethyl | |
| 1.2.243 | 2-Chlor-3-vinyl-Phenyl | Fluormethyl | |
| 1.2.244 | 2-Brom-3-vinyl-Phenyl | Fluormethyl | |
| 1.2.245 | 2-Methyl-3-vinyl-Phenyl | Fluormethyl | |
| 1.2.246 | 2-Ethyl-3-vinyl-Phenyl | Fluormethyl | |
| 1.2.247 | 2-Cyclopropyl-3-vinyl-Phenyl | Fluormethyl | |
| 1.2.248 | 2-Vinyl-3-vinyl-Phenyl | Fluormethyl | |
| 1.2.249 | 2-Ethinyl-3-vinyl-Phenyl | Fluormethyl | |
| 1.2.250 | 2-Cyano-3-vinyl-Phenyl | Fluormethyl | |
| 1.2.251 | 2-Trifluormethyl-3-vinyl-Phenyl | Fluormethyl | |
| 1.2.252 | 2-Methoxy-3-vinyl -Phenyl | Fluormethyl | |
| 1.2.253 | 2-Ethoxy-3-vinyl-Phenyl | Fluormethyl | |
| 1.2.254 | 2-Trifluormethoxy-3-vinyl-Phenyl | Fluormethyl | |
| 1.2.255 | 2-Nitro-3-vinyl-Phenyl | Fluormethyl | |
| 1.2.256 | 2-Fluor-3-ethinyl-Phenyl | Fluormethyl | |
| 1.2.257 | 2-Chlor-3-ethinyl-Phenyl | Fluormethyl | |
| 1.2.258 | 2-Brom-3-ethinyl-Phenyl | Fluormethyl | |
| 1.2.259 | 2-Methyl-3-ethinyl-Phenyl | Fluormethyl | |
| 1.2.260 | 2-Ethyl-3-ethinyl-Phenyl | Fluormethyl | |
| 1.2.261 | 2-Cyclopropyl-3-ethinyl-Phenyl | Fluormethyl | |
| 1.2.262 | 2-Vinyl-3-ethinyl-Phenyl | Fluormethyl | |
| 1.2.263 | 2-Cyano-3-ethinyl-Phenyl | Fluormethyl | |
| 1.2.264 | 2-Trifluormethyl-3-ethinyl-Phenyl | Fluormethyl | |
| 1.2.265 | 2-Methoxy-3-ethinyl-Phenyl | Fluormethyl | |
| 1.2.266 | 2-Ethoxy-3-ethinyl-Phenyl | Fluormethyl | |
| 1.2.267 | 2-Trifluormethoxy-3-ethinyl--Phenyl | Fluormethyl | |
| 1.2.268 | 2-Nitro-3-ethinyl-Phenyl | Fluormethyl | |
| 1.2.269 | 2-Fluor-3-ethinyl-Phenyl | Fluormethyl | |
| 1.2.270 | 2-Fluor-3-cyano-Phenyl | Fluormethyl | |
| 1.2.271 | 2-Chlor-3-cyano-Phenyl | Fluormethyl | |
| 1.2.272 | 2-Brom-3-cyano-Phenyl | Fluormethyl | |
| 1.2.273 | 2-Methyl-3-cyano-Phenyl | Fluormethyl | |
| 1.2.274 | 2-Ethyl-3-cyano-Phenyl | Fluormethyl | |
| 1.2.275 | 2-Ethyl-3-cyano-Phenyl | Chlormethyl | |
| 1.2.276 | 2-Ethyl-3-cyano-Phenyl | Brommethyl | |
| 1.2.277 | 2-Ethyl-3-cyano-Phenyl | Difluormethyl | |
| 1.2.278 | 2-Cyclopropyl-3-cyano-Phenyl | Fluormethyl | |
| 1.2.279 | 2-Vinyl-3-cyano-Phenyl | Fluormethyl | |
| 1.2.280 | 2-Ethinyl-3-cyano-Phenyl | Fluormethyl | |
| 1.2.281 | 2-Cyano-3-cyanoPhenyl | Fluormethyl | |
| 1.2.282 | 2-Trifluormethyl-3-cyano-Phenyl | Fluormethyl | |
| 1.2.283 | 2-Methoxy-3-cyano-Phenyl | Fluormethyl | |
| 1.2.284 | 2-Ethoxy-3-cyano-Phenyl | Fluormethyl | |
| 1.2.285 | 2-Trifluormethoxy-3-cyano-Phenyl | Fluormethyl | |
| 1.2.286 | 2-Nitro-3-cyano-Phenyl | Fluormethyl | |
| 1.2.287 | 2-Fluor-3-hydroxy-Phenyl | Fluormethyl | |
| 1.2.288 | 2-Chlor-3-hydroxy-Phenyl | Fluormethyl | |
| 1.2.289 | 2-Brom-3-hydroxy-Phenyl | Fluormethyl | |
| 1.2.290 | 2-Methyl-3-hyd roxy-Phenyl | Fluormethyl | |
| 1.2.291 | 2-Ethyl-3-hydroxy-Phenyl | Fluormethyl | |
| 1.2.292 | 2-Cyclopropyl-3-hydroxy-Phenyl | Fluormethyl | |
| 1.2.293 | 2-Vinyl-3-hydroxy-Phenyl | Fluormethyl | |
| 1.2.294 | 2-Ethinyl-3-hydroxy-Phenyl | Fluormethyl | |
| 1.2.295 | 2-Cyano-3-hydroxy-Phenyl | Fluormethyl | |
| 1.2.296 | 2-Trifluormethyl-3-hydroxy-Phenyl | Fluormethyl | |
| 1.2.297 | 2-Methoxy-3-hydroxy-Phenyl | Fluormethyl | |
| 1.2.298 | 2-Ethoxy-3-hydroxy-Phenyl | Fluormethyl | |
| 1.2.299 | 2-Trifluormethoxy-3-hydroxy-Phenyl | Fluormethyl | |
| 1.2.300 | 2-Nitro-3-hydroxy-Phenyl | Fluormethyl | |
| 1.2.301 | 2-Fluor-3-methoxy-Phenyl | Fluormethyl | |
| 1.2.302 | 2-Chlor-3-methoxy-Phenyl | Fluormethyl | |
| 1.2.303 | 2-Brom-3-methoxy-Phenyl | Fluormethyl | |
| 1.2.304 | 2-Methyl-3-methoxy-Phenyl | Fluormethyl | |
| 1.2.305 | 2-Ethyl-3-methoxy-Phenyl | Fluormethyl | |
| 1.2.306 | 2-Cyclopropyl-3-methoxy-Phenyl | Fluormethyl | |
| 1.2.307 | 2-Vinyl-3-methoxy-Phenyl | Fluormethyl | |
| 1.2.308 | 2-Ethinyl-3-methoxy-Phenyl | Fluormethyl | |
| 1.2.309 | 2-Cyano-3-methoxy-Phenyl | Fluormethyl | |
| 1.2.310 | 2-Trifluormethyl-3-methoxy-Phenyl | Fluormethyl | |
| 1.2.311 | 2,3-Dimethoxy--Phenyl | Fluormethyl | |
| 1.2.312 | 2-Ethoxy-3-methoxy-Phenyl | Fluormethyl | |
| 1.2.313 | 2-Trifluormethoxy-3-methoxy-Phenyl | Fluormethyl | |
| 1.2.314 | 2-Nitro-3-methoxy-Phenyl | Fluormethyl | |
| 1.2.315 | 2-Fluor-3-ethoxy-Phenyl | Fluormethyl | |
| 1.2.316 | 2-Chlor-3-ethoxy-Phenyl | Fluormethyl | |
| 1.2.317 | 2-Brom-3-ethoxy-Phenyl | Fluormethyl | |
| 1.2.318 | 2-Methyl-3-ethoxy-Phenyl | Fluormethyl | |
| 1.2.319 | 2-Ethyl-3-ethoxy-Phenyl | Fluormethyl | |
| 1.2.320 | 2-Cyclopropyl-3-ethoxy-Phenyl | Fluormethyl | |
| 1.2.321 | 2-Vinyl-3-ethoxy-Phenyl | Fluormethyl | |
| 1.2.322 | 2-Ethinyl-3-ethoxy-Phenyl | Fluormethyl | |
| 1.2.323 | 2-Cyano-3-ethoxy-Phenyl | Fluormethyl | |
| 1.2.324 | 2-Trifluormethyl-3-ethoxy-Phenyl | Fluormethyl | |
| 1.2.325 | 2-Methoxy-3-ethoxy -Phenyl | Fluormethyl | |
| 1.2.326 | 2,3-Diethoxy--Phenyl | Fluormethyl | |
| 1.2.327 | 2-Trifluormethoxy-3-ethoxy-Phenyl | Fluormethyl | |
| 1.2.328 | 2-Nitro-3-ethoxy-Phenyl | Fluormethyl | |
| 1.2.329 | 2-Fluor-3-propoxy-Phenyl | Fluormethyl | |
| 1.2.330 | 2-Chlor-3-propoxy-Phenyl | Fluormethyl | |
| 1.2.331 | 2-Brom-3-propoxy-Phenyl | Fluormethyl | |
| 1.2.332 | 2-Methyl-3-propoxy-Phenyl | Fluormethyl | |
| 1.2.333 | 2-Ethyl-3-propoxy-Phenyl | Fluormethyl | |
| 1.2.334 | 2-Cyclopropyl-3-propoxy-Phenyl | Fluormethyl | |
| 1.2.335 | 2-Vinyl-3-propoxy-Phenyl | Fluormethyl | |
| 1.2.336 | 2-Ethinyl-3-propoxy-Phenyl | Fluormethyl | |
| 1.2.337 | 2-Cyano-3-propoxy-Phenyl | Fluormethyl | |
| 1.2.338 | 2-Trifluormethyl-3-propoxy-Phenyl | Fluormethyl | |
| 1.2.339 | 2-Methoxy-3-propoxy-Phenyl | Fluormethyl | |
| 1.2.340 | 2-Ethoxy-3-propoxy-Phenyl | Fluormethyl | |
| 1.2.341 | 2-Trifluormethoxy-3-propoxy-Phenyl | Fluormethyl | |
| 1.2.342 | 2-Nitro-3-propoxy-Phenyl | Fluormethyl | |
| 1.2.343 | 2-Fluor-3-isopropoxy-Phenyl | Fluormethyl | |
| 1.2.344 | 2-Chlor-3-isopropoxy-Phenyl | Fluormethyl | |
| 1.2.345 | 2-Brom-3-isopropoxy-Phenyl | Fluormethyl | |
| 1.2.346 | 2-Methyl-3-isopropoxy-Phenyl | Fluormethyl | |
| 1.2.347 | 2-Ethyl-3-isopropoxy-Phenyl | Fluormethyl | |
| 1.2.348 | 2-Cyclopropyl-3-isopropoxy-Phenyl | Fluormethyl | |
| 1.2.349 | 2-Vinyl-3-isopropoxy-Phenyl | Fluormethyl | |
| 1.2.350 | 2-Ethinyl-3-isopropoxy-Phenyl | Fluormethyl | |
| 1.2.351 | 2-Cyano-3-isopropoxy-Phenyl | Fluormethyl | |
| 1.2.352 | 2-Trifluormethyl-3-isopropoxy-Phenyl | Fluormethyl | |
| 1.2.353 | 2-Methoxy-3-isopropoxy-Phenyl | Fluormethyl | |
| 1.2.354 | 2-Ethoxy-3-isopropoxy-Phenyl | Fluormethyl | |
| 1.2.355 | 2-Trifluormethoxy-3-isopropoxy-Phenyl | Fluormethyl | |
| 1.2.356 | 2-Nitro-3-isopropoxy-Phenyl | Fluormethyl | |
| 1.2.357 | 2-Fluor-3-tert.butoxy-Phenyl | Fluormethyl | |
| 1.2.358 | 2-Chlor-3-tert.butoxy-Phenyl | Fluormethyl | |
| 1.2.359 | 2-Brom-3-tert.butoxy-Phenyl | Fluormethyl | |
| 1.2.360 | 2-Methyl-3-tert.butoxy-Phenyl | Fluormethyl | |
| 1.2.361 | 2-Ethyl-3-tert.butoxy-Phenyl | Fluormethyl | |
| 1.2.362 | 2-Cyclopropyl-3-tert.butoxy-Phenyl | Fluormethyl | |
| 1.2.363 | 2-Vinyl-3-tert.butoxy-Phenyl | Fluormethyl | |
| 1.2.364 | 2-Ethinyl-3-tert.butoxy-Phenyl | Fluormethyl | |
| 1.2.365 | 2-Cyano-3-tert.butoxy-Phenyl | Fluormethyl | |
| 1.2.366 | 2-Trifluormethyl-3-tert.butoxy-Phenyl | Fluormethyl | |
| 1.2.367 | 2-Methoxy-3-tert.butoxy-Phenyl | Fluormethyl | |
| 1.2.368 | 2-Ethoxy-3-tert.butoxy-Phenyl | Fluormethyl | |
| 1.2.369 | 2-Trifluormethoxy-3-tert.butoxy-Phenyl | Fluormethyl | |
| 1.2.370 | 2-Nitro-3-tert.butoxy-Phenyl | Fluormethyl | |
| 1.2.371 | 2-Fluor-3-trifluormethoxy-Phenyl | Fluormethyl | |
| 1.2.372 | 2-Chlor-3-trifluormethoxy-Phenyl | Fluormethyl | |
| 1.2.373 | 2-Brom-3-trifluormethoxy-Phenyl | Fluormethyl | |
| 1.2.374 | 2-Methyl-3-trifluormethoxy-Phenyl | Fluormethyl | |
| 1.2.375 | 2-Ethyl-3-trifluormethoxy-Phenyl | Fluormethyl | |
| 1.2.376 | 2-Cyclopropyl-3-trifluormethoxy-Phenyl | Fluormethyl | |
| 1.2.377 | 2-Vinyl-3-trifluormethoxy-Phenyl | Fluormethyl | |
| 1.2.378 | 2-Ethinyl-3-trifluormethoxy-Phenyl | Fluormethyl | |
| 1.2.379 | 2-Cyano-3-trifluormethoxy-Phenyl | Fluormethyl | |
| 1.2.380 | 2-Trifluormethyl-3-trifluormethoxy-Phenyl | Fluormethyl | |
| 1.2.381 | 2-Methoxy-3-trifluormethoxy-Phenyl | Fluormethyl | |
| 1.2.382 | 2-Ethoxy-3-trifluormethoxy-Phenyl | Fluormethyl | |
| 1.2.383 | 2,3-Bis(trifluormethoxy)-Phenyl | Fluormethyl | |
| 1.2.384 | 2-Nitro-3-trifluormethoxy-Phenyl | Fluormethyl | |
| 1.2.385 | 2-Fluor-3-(2,2,2-trifluorethoxy)-Phenyl | Fluormethyl | |
| 1.2.386 | 2-Chlor-3-(2,2,2-trifluorethoxy)-Phenyl | Fluormethyl | |
| 1.2.387 | 2-Brom-3-(2,2,2-trifluorethoxy)-Phenyl | Fluormethyl | |
| 1.2.388 | 2-Methyl-3-(2,2,2-trifluorethoxy)-Phenyl | Fluormethyl | |
| 1.2.389 | 2-Ethyl-3-(2,2,2-trifluorethoxy)-Phenyl | Fluormethyl | |
| 1.2.390 | 2-Cyclopropyl-3-(2,2,2-trifluorethoxy)-Phenyl | Fluormethyl | |
| 1.2.391 | 2-Vinyl-3-(2,2,2-trifluorethoxy)-Phenyl | Fluormethyl | |
| 1.2.392 | 2-Ethinyl-3-(2,2,2-trifluorethoxy-Phenyl | Fluormethyl | |
| 1.2.393 | 2-Cyano-3-(2,2,2-trifluorethoxy)-Phenyl | Fluormethyl | |
| 1.2.394 | 2-Trifluormethyl-3-(2,2,2-trifluorethoxy)-Phenyl | Fluormethyl | |
| 1.2.395 | 2-Methoxy-3-(2,2,2-trifluorethoxy)-Phenyl | Fluormethyl | |
| 1.2.396 | 2-Ethoxy-3-(2,2,2-trifluorethoxy)-Phenyl | Fluormethyl | |
| 1.2.397 | 2-Trifluormethoxy-3-(2,2,2-trifluorethoxy)-Phenyl | Fluormethyl | |
| 1.2.398 | 2-Nitro-3-(2,2,2-trifluorethoxy)-Phenyl | Fluormethyl | |
| 1.2.399 | 2-Fluor-3-difluormethoxy-Phenyl | Fluormethyl | |
| 1.2.400 | 2-Chlor-3-difluormethoxy-Phenyl | Fluormethyl | |
| 1.2.401 | 2-Brom-3-difluormethoxy-Phenyl | Fluormethyl | |
| 1.2.402 | 2-Methyl-3-difluormethoxy-Phenyl | Fluormethyl | |
| 1.2.403 | 2-Ethyl-3-difluormethoxy-Phenyl | Fluormethyl | |
| 1.2.404 | 2-Cyclopropyl-3-difluormethoxy-Phenyl | Fluormethyl | |
| 1.2.405 | 2-Vinyl-3-difluormethoxy-Phenyl | Fluormethyl | |
| 1.2.406 | 2-Ethinyl-3-difluormethoxy-Phenyl | Fluormethyl | |
| 1.2.407 | 2-Cyano-3-difluormethoxy-Phenyl | Fluormethyl | |
| 1.2.408 | 2-Trifluormethyl-3-difluormethoxy-Phenyl | Fluormethyl | |
| 1.2.409 | 2-Methoxy-3-difluormethoxy-Phenyl | Fluormethyl | |
| 1.2.410 | 2-Ethoxy-3-difluormethoxy-Phenyl | Fluormethyl | |
| 1.2.411 | 2-Trifluormethoxy-3-difluormethoxy-Phenyl | Fluormethyl | |
| 1.2.412 | 2-Nitro-3-difluormethoxy-Phenyl | Fluormethyl | |
| 1.2.413 | 2-Fluor-3-(2-methoxyethoxy)-Phenyl | Fluormethyl | |
| 1.2.414 | 2-Chlor-3-(2-methoxyethoxy)-Phenyl | Fluormethyl | |
| 1.2.415 | 2-Brom-3-(2-methoxyethoxy)-Phenyl | Fluormethyl | |
| 1.2.416 | 2-Methyl-3-(2-methoxyethoxy)-Phenyl | Fluormethyl | |
| 1.2.417 | 2-Ethyl-3-(2-methoxyethoxy)-Phenyl | Fluormethyl | |
| 1.2.418 | 2-Cyclopropyl-3-(2-methoxyethoxy)-Phenyl | Fluormethyl | |
| 1.2.419 | 2-Vinyl-3-(2-methoxyethoxy)-Phenyl | Fluormethyl | |
| 1.2.420 | 2-Ethinyl-3-(2-methoxyethoxy)-Phenyl | Fluormethyl | |
| 1.2.421 | 2-Cyano-3-(2-methoxyethoxy)-Phenyl | Fluormethyl | |
| 1.2.422 | 2-Trifluormethyl-3-(2-methoxyethoxy)-Phenyl | Fluormethyl | |
| 1.2.423 | 2-Methoxy-3-(2-methoxyethoxy)-Phenyl | Fluormethyl | |
| 1.2.424 | 2-Ethoxy-3-(2-methoxyethoxy)-Phenyl | Fluormethyl | |
| 1.2.425 | 2-Trifluormethoxy-(2-methoxyethoxy)-Phenyl | Fluormethyl | |
| 1.2.426 | 2-Nitro-3-(2-methoxyethoxy)-Phenyl | Fluormethyl | |
| 1.2.427 | 2-Fluor-3-(tert.butoxycarbonyloxy)-Phenyl | Fluormethyl | |
| 1.2.428 | 2-Chlor-3-(tert.butoxycarbonyloxy)-Phenyl | Fluormethyl | |
| 1.2.429 | 2-Brom-3-(tert.butoxycarbonyloxy)-Phenyl | Fluormethyl | |
| 1.2.430 | 2-Methyl-3-(tert.butoxycarbonyloxy)-Phenyl | Fluormethyl | |
| 1.2.431 | 2-Ethyl-3-(tert.butoxycarbonyloxy)-Phenyl | Fluormethyl | |
| 1.2.432 | 2-Cyclopropyl-3-(tert.butoxycarbonyloxy)-Phenyl | Fluormethyl | |
| 1.2.433 | 2-Vinyl-3-(tert.butoxycarbonyloxy)-Phenyl | Fluormethyl | |
| 1.2.434 | 2-Ethinyl-3-(tert.butoxycarbonyloxy)-Phenyl | Fluormethyl | |
| 1.2.435 | 2-Cyano-3-(tert.butoxycarbonyloxy)-Phenyl | Fluormethyl | |
| 1.2.436 | 2-Trifluormethyl-3-(tert.butoxycarbonyloxy)-Phenyl | Fluormethyl | |
| 1.2.437 | 2-Methoxy-3-(tert.butoxycarbonyloxy)-Phenyl | Fluormethyl | |
| 1.2.438 | 2-Ethoxy-3-(tert.butoxycarbonyloxy)-Phenyl | Fluormethyl | |
| 1.2.439 | 2-Trifluormethoxy-3-(tert.butoxycarbonyloxy)-Phenyl | Fluormethyl | |
| 1.2.440 | 2-Nitro-3-(tert.butoxycarbonyloxy)-Phenyl | Fluormethyl | |
| 1.2.441 | 2-Fluor-3-nitro-Phenyl | Fluormethyl | |
| 1.2.442 | 2-Chlor-3-nitro-Phenyl | Fluormethyl | |
| 1.2.443 | 2-Brom-3-nitro-Phenyl | Fluormethyl | |
| 1.2.444 | 2-Methyl-3-nitro-Phenyl | Fluormethyl | |
| 1.2.445 | 2-Ethyl-3-nitro-Phenyl | Fluormethyl | |
| 1.2.446 | 2-Cyclopropyl-3-nitro-Phenyl | Fluormethyl | |
| 1.2.447 | 2-Vinyl-3-nitro-Phenyl | Fluormethyl | |
| 1.2.448 | 2-Ethinyl-3-nitro-Phenyl | Fluormethyl | |
| 1.2.449 | 2-Cyano-3-nitro-Phenyl | Fluormethyl | |
| 1.2.450 | 2-Trifluormethyl-3-nitro-Phenyl | Fluormethyl | |
| 1.2.451 | 2-Methoxy-3-nitro-Phenyl | Fluormethyl | |
| 1.2.452 | 2-Ethoxy-3-nitro-Phenyl | Fluormethyl | |
| 1.2.453 | 2-Trifluormethoxy-3-nitro-Phenyl | Fluormethyl | |
| 1.2.454 | 2-Fluor-3-methylsulfanylPhenyl | Fluormethyl | |
| 1.2.455 | 2-Chlor-3-methylsulfanyl-Phenyl | Fluormethyl | |
| 1.2.456 | 2-Brom-3-methylsulfanyl-Phenyl | Fluormethyl | |
| 1.2.457 | 2-Methyl-3-methylsulfanyl-Phenyl | Fluormethyl | |
| 1.2.458 | 2-Ethyl-3-methylsulfanyl-Phenyl | Fluormethyl | |
| 1.2.459 | 2-Cyclopropyl-3-methylsulfanyl-Phenyl | Fluormethyl | |
| 1.2.460 | 2-Vinyl-3-methylsulfanyl-Phenyl | Fluormethyl | |
| 1.2.461 | 2-Ethinyl-3-methylsulfanyl-Phenyl | Fluormethyl | |
| 1.2.462 | 2-Cyano-3-methylsulfanyl-Phenyl | Fluormethyl | |
| 1.2.463 | 2-Trifluormethyl-3-methylsulfanyl-Phenyl | Fluormethyl | |
| 1.2.464 | 2-Methoxy-3-methylsulfanyl-Phenyl | Fluormethyl | |
| 1.2.465 | 2-Ethoxy-3-methylsulfanyl-Phenyl | Fluormethyl | |
| 1.2.466 | 2-Trifluormethoxy-3-methylsulfanyl-Phenyl | Fluormethyl | |
| 1.2.467 | 2-Nitro-3-methylsulfanyl-Phenyl | Fluormethyl | |
| 1.2.468 | 3,5-Difluor-Phenyl | Fluormethyl | [CDCl3] 3.56 (d,1H); 3.80 (d,1H); 4.72 (s,1H); 4.84 (s,1H); 6.0 (s br,1H); 6.92 (t,1H); 7.19 (d,2H). |
| 1.2.469 | 3,5-Difluor-Phenyl | Chlormethyl | [CDCl₃] 3.57 (d,1H); 3.88 (s,1H), 3.92 (d,1H); 4.03 (d,1H), 6.93 (t,1H); 7.21 (d,2H). |
| 1.2.470 | 3,5-Difluor-Phenyl | Brommethyl | [CDCl₃] 3.56 (d,1H); 3.71 (d,1H); 3.91 (d,1H); 3.96 (d,1H); 6.91 (t,1H); 7.18 (d,2H). |
| 1.2.471 | 3,5-Difluor-Phenyl | Difluormethyl | [CDCl₃] 3.69 (d,1H); 3.91 (d,1H); 6.10-6.40 (s,1H); 6.92 (m,1H); 7.22 (m,2H). |
| 1.2.472 | 3,5-Difluor-Phenyl | Trifluormethyl | |
| 1.2.473 | 3,5-Difluor-Phenyl | 1,1-Dichlorethyl | [CDCl₃] 2.40 (s,3H); 3.93 (d,1H), 4.00 (d,1H); 6.94 (t,1H); 7.21 (d,2H). |
| 1.2.474 | 3,5-Difluor-Phenyl | CN | [CDCl₃] 3.97 (d,1H); 4.14 (d,1H); 6.97 (m,1H); 7.21 (m,2H). |
| 1.2.475 | 3-Chlor-5-fluor-Phenyl | Fluormethyl | |
| 1.2.476 | 3-Chlor-5-fluor-Phenyl | Chlormethyl | |
| 1.2.477 | 3-Chlor-5-fluor-Phenyl | Brommethyl | |
| 1.2.478 | 3-Chlor-5-fluor-Phenyl | Difluormethyl | |
| 1.2.479 | 3-Brom-5-fluor-Phenyl | Fluormethyl | |
| 1.2.480 | 3-Brom-5-fluor-Phenyl | Chlormethyl | |
| 1.2.481 | 3-Brom-5-fluor-Phenyl | Brommethyl | |
| 1.2.482 | 3-Brom-5-fluor-Phenyl | Difluormethyl | |
| 1.2.483 | 3-Iod-5-fluor-Phenyl | Fluormethyl | |
| 1.2.484 | 3-Methyl-5-fluor-Phenyl | Fluormethyl | |
| 1.2.485 | 3-Methyl-5-fluor-Phenyl | Chlormethyl | |
| 1.2.486 | 3-Methyl-5-fluor-Phenyl | Brommethyl | |
| 1.2.487 | 3-Methyl-5-fluor-Phenyl | Difluormethyl | |
| 1.2.488 | 3-Ethyl-5-fluor-Phenyl | Fluormethyl | |
| 1.2.489 | 3-Propyl-5-fluor-Phenyl | Fluormethyl | |
| 1.2.490 | 3-iPropyl-5-fluor-Phenyl | Fluormethyl | |
| 1.2.491 | 3-nButyl-5-fluor-Phenyl | Fluormethyl | |
| 1.2.492 | 3-isoButyl-5-fluor-Phenyl | Fluormethyl | |
| 1.2.493 | 3-tert.Butyl-5-fluor-Phenyl | Fluormethyl | |
| 1.2.494 | 3-Cyclopropyl-5-fluor-Phenyl | Fluormethyl | |
| 1.2.495 | 3-Vinyl-5-fluor-Phenyl | Fluormethyl | |
| 1.2.496 | 3-Ethinyl-5-fluor-Phenyl | Fluormethyl | |
| 1.2.497 | 3-Cyano-5-fluor-Phenyl | Fluormethyl | |
| 1.2.498 | 3-Trifluormethyl-5-fluor-Phenyl | Fluormethyl | |
| 1.2.499 | 3-Trifluormethyl-5-fluor-Phenyl | Chlormethyl | |
| 1.2.500 | 3-Trifluormethyl-5-fluor-Phenyl | Brommethyl | |
| 1.2.501 | 3-Trifluormethyl-5-fluor-Phenyl | Difluormethyl | |
| 1.2.502 | 3-(Methoxycarbony)l-5-fluor-Phenyl | Fluormethyl | |
| 1.2.503 | 3-Hydroxymethyl-5-fluor-Phenyl | Fluormethyl | |
| 1.2.504 | 3-Carbamoyl-5-fluor-Phenyl | Fluormethyl | |
| 1.2.505 | 3-Hydroxy-5-fluor-Phenyl | Fluormethyl | |
| 1.2.506 | 3-Methoxy-5-fluor-Phenyl | Fluormethyl | |
| 1.2.507 | 3-Ethoxy-5-fluor-Phenyl | Fluormethyl | |
| 1.2.508 | 3-nPropyoxy-5-fluor-Phenyl | Fluormethyl | |
| 1.2.509 | 3-isoPropoxy-5-fluor-Phenyl | Fluormethyl | |
| 1.2.510 | 3-nButoxy-5-fluor-Phenyl | Fluormethyl | |
| 1.2.511 | 3-isoButoxy-5-fluor-Phenyl | Fluormethyl | |
| 1.2.512 | 3-tert.Butoxy-5-fluor-Phenyl | Fluormethyl | |
| 1.2.513 | 3-Difluormethoxy-5-fluor-Phenyl | Fluormethyl | |
| 1.2.514 | 3-Trifluormethoxy-5-fluor-Phenyl | Fluormethyl | |
| 1.2.515 | 3-(2,2,2-Trifluorethoxy)-5-fluor-Phenyl | Fluormethyl | |
| 1.2.516 | 3-(2-Chlorethoxy)-5-fluor-Phenyl | Fluormethyl | |
| 1.2.517 | 3-(2-Hydroxyethoxy)-5-fluor-Phenyl- | Fluormethyl | |
| 1.2.518 | 3-[(tert-Butoxycarbonyl)oxy]-5-fluor-Phenyl | Fluormethyl | |
| 1.2.519 | 3-Nitro-5-fluor-Phenyl | Fluormethyl | |
| 1.2.520 | 3-Acetoxy-5-fluor-Phenyl | Fluormethyl | |
| 1.2.521 | {3-[(tert-Butoxycarbonyl)amino]-5-fluor-Phenyl} | Fluormethyl | |
| 1.2.522 | 3-Methylsulfanyl-5-fluor-Phenyl | Fluormethyl | |
| 1.2.523 | 3,5-Dichlor-Phenyl | Fluormethyl | |
| 1.2.524 | 3,5-Dichlor-Phenyl | Chlormethyl | [CDCl₃] 3.59 (d, 1H); 3.88 (d, 1H); 3.91 (d, 1H); 4.03 (d, 1H); 7.45 (m, 1H); 7.58 (m, 1H). |
| 1.2.525 | 3,5-Dichlor-Phenyl | Brommethyl | [CDCl₃] 3.55 (d, 1H); 3.71 (d, 1H); 3.90 (d, 1H); 3.96 (d, 1H); 7.45 (s, 1H); 7.55 (s, 2H). |
| 1.2.526 | 3,5-Dichlor-Phenyl | Difluormethyl | |
| 1.2.527 | 3,5-Dichlor-Phenyl | Trifluormethyl | [CDCl₃] 3.8 (d, 1H); 4.0 (d, 1H); 7.45 (m, 1H); 7.55 (m, 1H). |
| 1.2.528 | 3,5-Dichlor-Phenyl | Cyano | [DMSO] 3.87 (d, 1H); 4.02 (d, 1H); 7.74 (m, 3H). |
| 1.2.529 | 3-Brom-5-chlor-Phenyl | Fluormethyl | |
| 1.2.530 | 3-Iod-5-chlor-Phenyl | Fluormethyl | |
| 1.2.531 | 3-Methyl-5-chlor-Phenyl | Fluormethyl | |
| 1.2.532 | 3-Ethyl-5-chlor-Phenyl | Fluormethyl | |
| 1.2.533 | 3-Propyl-5-chlor-Phenyl | Fluormethyl | |
| 1.2.534 | 3-isoPropyl-5-chlor-Phenyl | Fluormethyl | |
| 1.2.535 | 3-nButyl-5-chlor-Phenyl | Fluormethyl | |
| 1.2.536 | 3-isoButyl-5-chlor-Phenyl | Fluormethyl | |
| 1.2.537 | 3-tert.Butyl-5-chlor-Phenyl | Fluormethyl | |
| 1.2.538 | 3-Cyclopropyl-5-chlor-Phenyl | Fluormethyl | |
| 1.2.539 | 3-Vinyl-5-chlor-Phenyl | Fluormethyl | |
| 1.2.540 | 3-Ethinyl-5-chlor-Phenyl | Fluormethyl | |
| 1.2.541 | 3-Cyano-5-chlor-Phenyl | Fluormethyl | |
| 1.2.542 | 3-Trifluormethyl-5-chlor-Phenyl | Fluormethyl | |
| 1.2.543 | 3-(Hydroxycarbonyl)-5-chlor-Phenyl | Fluormethyl | |
| 1.2.544 | 3-(Methoxycarbony)l-5-chlor-Phenyl | Fluormethyl | |
| 1.2.545 | 3-Hydroxymethyl-5-chlor-Phenyl | Fluormethyl | |
| 1.2.546 | 3-Carbamoyl-5-chlor-Phenyl | Fluormethyl | |
| 1.2.547 | 3-Hydroxy-5-chlor-Phenyl- | Fluormethyl | |
| 1.2.548 | 3-Methoxy-5-chlor-Phenyl | Fluormethyl | |
| 1.2.549 | 3-Ethoxy-5-chlor-Phenyl | Fluormethyl | |
| 1.2.550 | 3-nPropyoxy-5-chlor-Phenyl | Fluormethyl | |
| 1.2.551 | 3-isoPropoxy-5-chlor-Phenyl | Fluormethyl | |
| 1.2.552 | 3-nButoxy-5-chlor-Phenyl | Fluormethyl | |
| 1.2.553 | 3-isoButoxy-5-chlor-Phenyl | Fluormethyl | |
| 1.2.554 | 3-tert.Butoxy-5-chlor-Phenyl | Fluormethyl | |
| 1.2.555 | 3-Difluormethoxy-5-chlor-Phenyl | Fluormethyl | |
| 1.2.556 | 3-Trifluormethoxy-5-chlor-Phenyl | Fluormethyl | |
| 1.2.557 | 3-(2,2,2-Trifluorethoxy)-5-chlor-Phenyl | Fluormethyl | |
| 1.2.558 | 3-(2-Chlorethoxy)-5-chlor-Phenyl | Fluormethyl | |
| 1.2.559 | 3-(2-Hydroxyethoxy)-5-chlor-Phenyl- | Fluormethyl | |
| 1.2.560 | 3-[(tert-Butoxycarbonyl)oxy]-5-chlor-Phenyl | Fluormethyl | |
| 1.2.561 | 3-Nitro-5-chlor-Phenyl | Fluormethyl | |
| 1.2.562 | 3-Acetoxy-5-chlor-Phenyl | Fluormethyl | |
| 1.2.563 | {3-[(tert-Butoxycarbonyl)amino]-5-chlor-Phenyl} | Fluormethyl | |
| 1.2.564 | 3-Methylsulfanyl-5-chlor-Phenyl | Fluormethyl | |
| 1.2.565 | 3,5-Dibrom-Phenyl | Fluormethyl | |
| 1.2.566 | 3,5-Dibrom-Phenyl | Chlormethyl | |
| 1.2.567 | 3-Iod-5-brom-Phenyl | Fluormethyl | |
| 1.2.568 | 3-Methyl-5-brom-Phenyl | Fluormethyl | |
| 1.2.569 | 3-Methyl-5-brom-Phenyl | Chlormethyl | |
| 1.2.570 | 3-Methyl-5-brom-Phenyl | Brommethyl | |
| 1.2.571 | 3-Methyl-5-brom-Phenyl | Difluormethyl | |
| 1.2.572 | 3-Methyl-5-brom-Phenyl | Trifluormethyl | |
| 1.2.573 | 3-Methyl-5-brom-Phenyl | Cyano | |
| 1.2.574 | 3-Ethyl-5-brom-Phenyl | Fluormethyl | |
| 1.2.575 | 3-Propyl-5-brom-Phenyl | Fluormethyl | |
| 1.2.576 | 3-isoPropyl-5-brom-Phenyl | Fluormethyl | |
| 1.2.577 | 3-nButyl-5-brom-Phenyl | Fluormethyl | |
| 1.2.578 | 3-isoButyl-5-brom-Phenyl | Fluormethyl | |
| 1.2.579 | 3-tert.Butyl-5-brom-Phenyl | Fluormethyl | |
| 1.2.580 | 3-Cyclopropyl-5-brom-Phenyl | Fluormethyl | |
| 1.2.581 | 3-Vinyl-5-brom-Phenyl | Fluormethyl | |
| 1.2.582 | 3-Ethinyl-5-brom-Phenyl | Fluormethyl | |
| 1.2.583 | 3-Cyano-5-brom-Phenyl | Fluormethyl | |
| 1.2.584 | 3-Trifluormethyl-5-brom-Phenyl | Fluormethyl | |
| 1.2.585 | 3-(Hydroxycarbonyl)-5-brom-Phenyl | Fluormethyl | |
| 1.2.586 | 3-(Methoxycarbony)l-5-brom-Phenyl | Fluormethyl | |
| 1.2.587 | 3-Hydroxymethyl-5-brom-Phenyl | Fluormethyl | |
| 1.2.588 | 3-Carbamoyl-5-brom-Phenyl | Fluormethyl | |
| 1.2.589 | 3-Hydroxy-5-brom-Phenyl- | Fluormethyl | |
| 1.2.590 | 3-Methoxy-5-brom-Phenyl | Fluormethyl | |
| 1.2.591 | 3-Ethoxy-5-brom-Phenyl | Fluormethyl | |
| 1.2.592 | 3-nPropyoxy-5-brom-Phenyl | Fluormethyl | |
| 1.2.593 | 3-isoPropoxy-5-brom-Phenyl | Fluormethyl | |
| 1.2.594 | 3-nButoxy-5-brom-Phenyl | Fluormethyl | |
| 1.2.595 | 3-isoButoxy-5-brom-Phenyl | Fluormethyl | |
| 1.2.596 | 3-tert.Butoxy-5-brom-Phenyl | Fluormethyl | |
| 1.2.597 | 3-Difluormethoxy-5-brom-Phenyl | Fluormethyl | |
| 1.2.598 | 3-Trifluormethoxy-5-brom-Phenyl | Fluormethyl | |
| 1.2.599 | 3-(2,2,2-Trifluorethoxy)-5-brom-Phenyl | Fluormethyl | |
| 1.2.600 | 3-(2-Chlorethoxy)-5-brom-Phenyl | Fluormethyl | |
| 1.2.601 | 3-(2-Hydroxyethoxy)-5-brom-Phenyl- | Fluormethyl | |
| 1.2.602 | 3-[(tert-Butoxycarbonyl)oxy]-5-brom-Phenyl | Fluormethyl | |
| 1.2.603 | 3-Nitro-5-brom-Phenyl | Fluormethyl | |
| 1.2.604 | 3-Acetoxy-5-brom-Phenyl | Fluormethyl | |
| 1.2.605 | {3-[(tert-Butoxycarbonyl)amino]-5-brom-Phenyl} | Fluormethyl | |
| 1.2.606 | 3-Methylsulfanyl-5-brom-Phenyl | Fluormethyl | |
| 1.2.607 | 3,5-Diiod-Phenyl | Fluormethyl | |
| 1.2.608 | 3-Methyl-5-iod-Phenyl | Fluormethyl | |
| 1.2.609 | 3-Ethyl-5-iod-Phenyl | Fluormethyl | |
| 1.2.610 | 3-Propyl-5-iod-Phenyl | Fluormethyl | |
| 1.2.611 | 3-isoPropyl-5-iod-Phenyl | Fluormethyl | |
| 1.2.612 | 3-nButyl-5-iod-Phenyl | Fluormethyl | |
| 1.2.613 | 3-isoButyl-5-iod-Phenyl | Fluormethyl | |
| 1.2.614 | 3-tert.Butyl-5-iod-Phenyl | Fluormethyl | |
| 1.2.615 | 3-Cyclopropyl-5-iod-Phenyl | Fluormethyl | |
| 1.2.616 | 3-Vinyl-5-iod-Phenyl | Fluormethyl | |
| 1.2.617 | 3-Ethinyl-5-iod-Phenyl | Fluormethyl | |
| 1.2.618 | 3-Cyano-5-iod-Phenyl | Fluormethyl | |
| 1.2.619 | 3-Trifluormethyl-5-iod-Phenyl | Fluormethyl | |
| 1.2.620 | 3-(Hydroxycarbonyl)-5-iod-Phenyl | Fluormethyl | |
| 1.2.621 | 3-(Methoxycarbonyl)-5-iod-Phenyl | Fluormethyl | |
| 1.2.622 | 3-Hydroxymethyl-5-iod-Phenyl | Fluormethyl | |
| 1.2.623 | 3-Carbamoyl-5-iod-Phenyl | Fluormethyl | |
| 1.2.624 | 3-Hydroxy-5-iod-Phenyl- | Fluormethyl | |
| 1.2.625 | 3-Methoxy-5-iod-Phenyl | Fluormethyl | |
| 1.2.626 | 3-Ethoxy-5-iod-Phenyl | Fluormethyl | |
| 1.2.627 | 3-nPropyoxy-5-iod-Phenyl | Fluormethyl | |
| 1.2.628 | 3-isoPropoxy-5-iod-Phenyl | Fluormethyl | |
| 1.2.629 | 3-nButoxy-5-iod-Phenyl | Fluormethyl | |
| 1.2.630 | 3-isoButoxy-5-iod-Phenyl | Fluormethyl | |
| 1.2.631 | 3-tert.Butoxy-5-iod-Phenyl | Fluormethyl | |
| 1.2.632 | 3-Difluormethoxy-5-iod-Phenyl | Fluormethyl | |
| 1.2.633 | 3-Trifluormethoxy-5-iod-Phenyl | Fluormethyl | |
| 1.2.634 | 3-(2,2,2-Trifluorethoxy)-5-iod-Phenyl | Fluormethyl | |
| 1.2.635 | 3-(2-Chlorethoxy)-5-iod-Phenyl | Fluormethyl | |
| 1.2.636 | 3-(2-Hydroxyethoxy)-5-iod-Phenyl- | Fluormethyl | |
| 1.2.637 | 3-[(tert-Butoxycarbonyl)oxy]-5-iod-Phenyl | Fluormethyl | |
| 1.2.638 | 3-Nitro-5-iod-Phenyl | Fluormethyl | |
| 1.2.639 | 3-Acetoxy-5-iod-Phenyl | Fluormethyl | |
| 1.2.640 | {3-[(tert-Butoxycarbonyl)amino]-5-iod-Phenyl} | Fluormethyl | |
| 1.2.641 | 3-Methylsulfanyl-5-iod-Phenyl | Fluormethyl | |
| 1.2.642 | 3,5-Dimethyl-Phenyl | Fluormethyl | |
| 1.2.643 | 3-Ethyl-5-methyl-Phenyl | Fluormethyl | |
| 1.2.644 | 3-Propyl-5-methyl-Phenyl | Fluormethyl | |
| 1.2.645 | 3-isoPropyl-5-methyl-Phenyl | Fluormethyl | |
| 1.2.646 | 3-nButyl-5-methyl-Phenyl | Fluormethyl | |
| 1.2.647 | 3-isoButyl-5-methyl-Phenyl | Fluormethyl | |
| 1.2.648 | 3-tert.Butyl-5-methyl-Phenyl | Fluormethyl | |
| 1.2.649 | 3-Cyclopropyl-5-methyl-Phenyl | Fluormethyl | |
| 1.2.650 | 3-Vinyl-5-methyl-Phenyl | Fluormethyl | |
| 1.2.651 | 3-Ethinyl-5-methyl-Phenyl | Fluormethyl | |
| 1.2.652 | 3-Cyano-5-methyl-Phenyl | Fluormethyl | |
| 1.2.653 | 3-Trifluormethyl-5-methyl-Phenyl | Fluormethyl | |
| 1.2.654 | 3-(Hydroxycarbonyl)-5-methyl-Phenyl | Fluormethyl | |
| 1.2.655 | 3-(Methoxycarbony)l-5-methyl-Phenyl | Fluormethyl | |
| 1.2.656 | 3-Hydroxymethyl-5-methyl-Phenyl | Fluormethyl | |
| 1.2.657 | 3-Carbamoyl-5-methyl-Phenyl | Fluormethyl | |
| 1.2.658 | 3-Hydroxy-5-methyl-Phenyl- | Fluormethyl | |
| 1.2.659 | 3-Methoxy-5-methyl-Phenyl | Fluormethyl | |
| 1.2.660 | 3-Ethoxy-5-methyl-Phenyl | Fluormethyl | |
| 1.2.661 | 3-nPropyoxy-5-methyl-Phenyl | Fluormethyl | |
| 1.2.662 | 3-nButoxy-5-methyl-Phenyl | Fluormethyl | |
| 1.2.663 | 3-isoButoxy-5-methyl-Phenyl | Fluormethyl | |
| 1.2.664 | 3-tert.Butoxy-5-methyl-Phenyl | Fluormethyl | |
| 1.2.665 | 3-Difluormethoxy-5-methyl-Phenyl | Fluormethyl | |
| 1.2.666 | 3-Trifluormethoxy-5-methyl-Phenyl | Fluormethyl | |
| 1.2.667 | 3-(2,2,2-Trifluorethoxy)-5-methyl-Phenyl | Fluormethyl | |
| 1.2.668 | 3-(2-Chlorethoxy)-5-methyl-Phenyl | Fluormethyl | |
| 1.2.669 | 3-(2-Hydroxyethoxy)-5-methyl-Phenyl- | Fluormethyl | |
| 1.2.670 | 3-[(tert-Butoxycarbonyl)oxy]-5-methyl-Phenyl | Fluormethyl | |
| 1.2.671 | 3-Nitro-5-methyl-Phenyl | Fluormethyl | |
| 1.2.672 | 3-Acetoxy-5-methyl-Phenyl | Fluormethyl | |
| 1.2.673 | {3-[(tert-Butoxycarbonyl)amino]-5-methyl-Phenyl} | Fluormethyl | |
| 1.2.674 | 3-Methylsulfanyl-5-methyl-Phenyl | Fluormethyl | |
| 1.2.675 | 3,5-Diethyl-Phenyl | Fluormethyl | |
| 1.2.676 | 3-Propyl-5-ethyl-Phenyl | Fluormethyl | |
| 1.2.677 | 3-isoPropyl-5-ethyl-Phenyl | Fluormethyl | |
| 1.2.678 | 3-nButyl-5-ethyl-Phenyl | Fluormethyl | |
| 1.2.679 | 3-isoButyl-5-ethyl-Phenyl | Fluormethyl | |
| 1.2.680 | 3-tert.Butyl-5-ethyl-Phenyl | Fluormethyl | |
| 1.2.681 | 3-Cyclopropyl-5-ethyl-Phenyl | Fluormethyl | |
| 1.2.682 | 3-Vinyl-5-ethyl-Phenyl | Fluormethyl | |
| 1.2.683 | 3-Ethinyl-5-ethyl-Phenyl | Fluormethyl | |
| 1.2.684 | 3-Cyano-5-ethyl-Phenyl | Fluormethyl | |
| 1.2.685 | 3-Trifluormethyl-5-ethyl-Phenyl | Fluormethyl | |
| 1.2.686 | 3-(Hydroxycarbonyl)-5-ethyl-Phenyl | Fluormethyl | |
| 1.2.687 | 3-(Methoxycarbony)l-5-ethyl-Phenyl | Fluormethyl | |
| 1.2.688 | 3-Hydroxymethyl-5-ethyl-Phenyl | Fluormethyl | |
| 1.2.689 | 3-Carbamoyl-5-ethyl-Phenyl | Fluormethyl | |
| 1.2.690 | 3-Hydroxy-5-ethyl-Phenyl- | Fluormethyl | |
| 1.2.691 | 3-Methoxy-5-ethyl-Phenyl | Fluormethyl | |
| 1.2.692 | 3-Ethoxy-5-ethyl-Phenyl | Fluormethyl | |
| 1.2.693 | 3-nPropyoxy-5-ethyl-Phenyl | Fluormethyl | |
| 1.2.694 | 3-nButoxy-5-ethyl-Phenyl | Fluormethyl | |
| 1.2.695 | 3-isoButoxy-5-ethyl-Phenyl | Fluormethyl | |
| 1.2.696 | 3-tert.Butoxy-5-ethyl-Phenyl | Fluormethyl | |
| 1.2.697 | 3-Difluormethoxy-5-ethyl-Phenyl | Fluormethyl | |
| 1.2.698 | 3-Trifluormethoxy-5-ethyl-Phenyl | Fluormethyl | |
| 1.2.699 | 3-(2,2,2-Trifluorethoxy)-5-ethyl-Phenyl | Fluormethyl | |
| 1.2.700 | 3-(2-Chlorethoxy)-5-ethyl-Phenyl | Fluormethyl | |
| 1.2.701 | 3-(2-Hyd roxyethoxy)-5-ethyl-Phenyl- | Fluormethyl | |
| 1.2.702 | 3-[(tert-Butoxycarbonyl)oxy]-5-ethyl-Phenyl | Fluormethyl | |
| 1.2.703 | 3-Nitro-5-ethyl-Phenyl | Fluormethyl | |
| 1.2.704 | 3-Acetoxy-5-ethyl-Phenyl | Fluormethyl | |
| 1.2.705 | {3-[(tert-Butoxycarbonyl)amino]-5-ethyl-Phenyl} | Fluormethyl | |
| 1.2.706 | 3-Methylsulfanyl-5-ethyl-Phenyl | Fluormethyl | |
| 1.2.707 | 3,5-Dipropyl-Phenyl | Fluormethyl | |
| 1.2.708 | 3-isoPropyl-5-propyl-Phenyl | Fluormethyl | |
| 1.2.709 | 3-nButyl-5-propyl-Phenyl | Fluormethyl | |
| 1.2.710 | 3-isoButyl-5-propyl-Phenyl | Fluormethyl | |
| 1.2.711 | 3-tert.Butyl-5-propyl-Phenyl | Fluormethyl | |
| 1.2.712 | 3-Cyclopropyl-5-propyl-Phenyl | Fluormethyl | |
| 1.2.713 | 3-Vinyl-5-propyl-Phenyl | Fluormethyl | |
| 1.2.714 | 3-Ethinyl-5-propyl-Phenyl | Fluormethyl | |
| 1.2.715 | 3-Cyano-5-propyl-Phenyl | Fluormethyl | |
| 1.2.716 | 3-Trifluormethyl-5-propyl-Phenyl | Fluormethyl | |
| 1.2.717 | 3-(Hydroxycarbonyl)-5-propyl-Phenyl | Fluormethyl | |
| 1.2.718 | 3-(Methoxycarbonyl)-5-propyl-Phenyl | Fluormethyl | |
| 1.2.719 | 3-Hydroxymethyl-5-propyl-Phenyl | Fluormethyl | |
| 1.2.720 | 3-Carbamoyl-5-propyl-Phenyl | Fluormethyl | |
| 1.2.721 | 3-Hydroxy-5-propyl-Phenyl- | Fluormethyl | |
| 1.2.722 | 3-Methoxy-5-propyl-Phenyl | Fluormethyl | |
| 1.2.723 | 3-Ethoxy-5-propyl-Phenyl | Fluormethyl | |
| 1.2.724 | 3-nPropyoxy-5-propyl-Phenyl | Fluormethyl | |
| 1.2.725 | 3-nButoxy-5-propyl-Phenyl | Fluormethyl | |
| 1.2.726 | 3-isoButoxy-5-propyl-Phenyl | Fluormethyl | |
| 1.2.727 | 3-tert.Butoxy-5-propyl-Phenyl | Fluormethyl | |
| 1.2.728 | 3-Difluormethoxy-5-propyl-Phenyl | Fluormethyl | |
| 1.2.729 | 3-Trifluormethoxy-5-ethyl-Phenyl | Fluormethyl | |
| 1.2.730 | 3-(2,2,2-Trifluorethoxy)-5-propyl-Phenyl | Fluormethyl | |
| 1.2.731 | 3-(2-Chlorethoxy)-5-propyl-Phenyl | Fluormethyl | |
| 1.2.732 | 3-(2-Hydroxyethoxy)-5-propyl-Phenyl- | Fluormethyl | |
| 1.2.733 | 3-[(tert-Butoxycarbonyl)oxy]-5-propyl-Phenyl | Fluormethyl | |
| 1.2.734 | 3-Nitro-5-propyl-Phenyl | Fluormethyl | |
| 1.2.735 | 3-Acetoxy-5-propyl-Phenyl | Fluormethyl | |
| 1.2.736 | {3-[(tert-Butoxycarbonyl)amino]-5-propyl-Phenyl} | Fluormethyl | |
| 1.2.737 | 3-Methylsulfanyl-5-propyl-Phenyl | Fluormethyl | |
| 1.2.738 | 3,5-Diisopropyl-Phenyl | Fluormethyl | |
| 1.2.739 | 3-nButyl-5-isopropyl-Phenyl | Fluormethyl | |
| 1.2.740 | 3-isoButyl-5-isopropyl-Phenyl | Fluormethyl | |
| 1.2.741 | 3-tert.Butyl-5-isopropyl-Phenyl | Fluormethyl | |
| 1.2.742 | 3-Cyclopropyl-5-isopropyl-Phenyl | Fluormethyl | |
| 1.2.743 | 3-Vinyl-5-isopropyl-Phenyl | Fluormethyl | |
| 1.2.744 | 3-Ethinyl-5-isopropyl-Phenyl | Fluormethyl | |
| 1.2.745 | 3-Cyano-5-isopropyl-Phenyl | Fluormethyl | |
| 1.2.746 | 3-Trifluormethyl-5-isopropyl-Phenyl | Fluormethyl | |
| 1.2.747 | 3-(Hydroxycarbonyl)-5-isopropyl-Phenyl | Fluormethyl | |
| 1.2.748 | 3-(Methoxycarbony)l-5-isopropyl-Phenyl | Fluormethyl | |
| 1.2.749 | 3-Hydroxymethyl-5-isopropyl-Phenyl | Fluormethyl | |
| 1.2.750 | 3-Carbamoyl-5-isopropyl-Phenyl | Fluormethyl | |
| 1.2.751 | 3-Hydroxy-5-isopropyl-Phenyl- | Fluormethyl | |
| 1.2.752 | 3-Methoxy-5-isopropyl-Phenyl | Fluormethyl | |
| 1.2.753 | 3-Ethoxy-5-isopropyl-Phenyl | Fluormethyl | |
| 1.2.754 | 3-nPropyoxy-5-isopropyl-Phenyl | Fluormethyl | |
| 1.2.755 | 3-nButoxy-5-isopropyl-Phenyl | Fluormethyl | |
| 1.2.756 | 3-isoButoxy-5-isopropyl-Phenyl | Fluormethyl | |
| 1.2.757 | 3-tert.Butoxy-5-isopropyl-Phenyl | Fluormethyl | |
| 1.2.758 | 3-Difluormethoxy-5-isopropyl-Phenyl | Fluormethyl | |
| 1.2.759 | 3-Trifluormethoxy-5-isopropyl-Phenyl | Fluormethyl | |
| 1.2.760 | 3-(2,2,2-Trifluorethoxy)-5-isopropyl-Phenyl | Fluormethyl | |
| 1.2.761 | 3-(2-Chlorethoxy)-5-isopropyl-Phenyl | Fluormethyl | |
| 1.2.762 | 3-(2-Hydroxyethoxy)-5-propyl-Phenyl- | Fluormethyl | |
| 1.2.763 | 3-[(tert-Butoxycarbonyl)oxy]-5-isopropyl-Phenyl | Fluormethyl | |
| 1.2.764 | 3-Nitro-5-isopropyl-Phenyl | Fluormethyl | |
| 1.2.765 | 3-Acetoxy-5-isopropyl-Phenyl | Fluormethyl | |
| 1.2.766 | {3-[(tert-Butoxycarbonyl)amino]-5-isopropyl-Phenyl} | Fluormethyl | |
| 1.2.767 | 3-Methylsulfanyl-5-isopropyl-Phenyl | Fluormethyl | |
| 1.2.768 | 3,5-Dibutyl-Phenyl | Fluormethyl | |
| 1.2.769 | 3-isoButyl-5-butyl-Phenyl | Fluormethyl | |
| 1.2.770 | 3-tert.Butyl-5-butyl-Phenyl | Fluormethyl | |
| 1.2.771 | 3-Cyclopropyl-5-butyl-Phenyl | Fluormethyl | |
| 1.2.772 | 3-Vinyl-5-butyl-Phenyl | Fluormethyl | |
| 1.2.773 | 3-Ethinyl-5-butyl-Phenyl | Fluormethyl | |
| 1.2.774 | 3-Cyano-5-butyl-Phenyl | Fluormethyl | |
| 1.2.775 | 3-Trifluormethyl-5-butyl-Phenyl | Fluormethyl | |
| 1.2.776 | 3-(Hydroxycarbonyl)-5-butyl-Phenyl | Fluormethyl | |
| 1.2.777 | 3-(Methoxycarbony)l-5-butyl-Phenyl | Fluormethyl | |
| 1.2.778 | 3-Hydroxymethyl-5-butyl-Phenyl | Fluormethyl | |
| 1.2.779 | 3-Carbamoyl-5-butyl-Phenyl | Fluormethyl | |
| 1.2.780 | 3-Hydroxy-5-butyl-Phenyl- | Fluormethyl | |
| 1.2.781 | 3-Methoxy-5-butyl-Phenyl | Fluormethyl | |
| 1.2.782 | 3-Ethoxy-5-butyl-Phenyl | Fluormethyl | |
| 1.2.783 | 3-nPropyoxy-5-butyl-Phenyl | Fluormethyl | |
| 1.2.784 | 3-nButoxy-5-butyl-Phenyl | Fluormethyl | |
| 1.2.785 | 3-isoButoxy-5-butyl-Phenyl | Fluormethyl | |
| 1.2.786 | 3-tert.Butoxy-5-butyl-Phenyl | Fluormethyl | |
| 1.2.787 | 3-Difluormethoxy-5-butyl-Phenyl | Fluormethyl | |
| 1.2.788 | 3-Trifluormethoxy-5-butyl-Phenyl | Fluormethyl | |
| 1.2.789 | 3-(2,2,2-Trifluorethoxy)-5-butyl-Phenyl | Fluormethyl | |
| 1.2.790 | 3-(2-Chlorethoxy)-5-butyl-Phenyl | Fluormethyl | |
| 1.2.791 | 3-(2-Hydroxyethoxy)-5-butyl-Phenyl- | Fluormethyl | |
| 1.2.792 | 3-[(tert-Butoxycarbonyl)oxy]-5-butyl-Phenyl | Fluormethyl | |
| 1.2.793 | 3-Nitro-5-butyl-Phenyl | Fluormethyl | |
| 1.2.794 | 3-Acetoxy-5-butyl-Phenyl | Fluormethyl | |
| 1.2.795 | {3-[(tert-Butoxycarbonyl)amino]-5-butyl-Phenyl} | Fluormethyl | |
| 1.2.796 | 3-Methylsulfanyl-5-butyl-Phenyl | Fluormethyl | |
| 1.2.797 | 3,5-Diisobutyl-Phenyl | Fluormethyl | |
| 1.2.798 | 3-tert.Butyl-5-isobutyl-Phenyl | Fluormethyl | |
| 1.2.799 | 3-Cyclopropyl-5-isobutyl-Phenyl | Fluormethyl | |
| 1.2.800 | 3-Vinyl-5-isobutyl-Phenyl | Fluormethyl | |
| 1.2.801 | 3-Ethinyl-5-isobutyl-Phenyl | Fluormethyl | |
| 1.2.802 | 3-Cyano-5-isobutyl-Phenyl | Fluormethyl | |
| 1.2.803 | 3-Trifluormethyl-5-isobutyl-Phenyl | Fluormethyl | |
| 1.2.804 | 3-(Hydroxycarbonyl)-5-isobutyl-Phenyl | Fluormethyl | |
| 1.2.805 | 3-(Methoxycarbonyl)-5-isobutyl-Phenyl | Fluormethyl | |
| 1.2.806 | 3-Hydroxymethyl-5-isobutyl-Phenyl | Fluormethyl | |
| 1.2.807 | 3-Carbamoyl-5-isobutyl-Phenyl | Fluormethyl | |
| 1.2.808 | 3-Hydroxy-5-isobutyl-Phenyl- | Fluormethyl | |
| 1.2.809 | 3-Methoxy-5-isobutyl-Phenyl | Fluormethyl | |
| 1.2.810 | 3-Ethoxy-5-isobutyl-Phenyl | Fluormethyl | |
| 1.2.811 | 3-nPropyoxy-5-isobutyl-Phenyl | Fluormethyl | |
| 1.2.812 | 3-nButoxy-5-isobutyl-Phenyl | Fluormethyl | |
| 1.2.813 | 3-isoButoxy-5-isobutyl-Phenyl | Fluormethyl | |
| 1.2.814 | 3-tert.Butoxy-5-isobutyl-Phenyl | Fluormethyl | |
| 1.2.815 | 3-Difluormethoxy-5-isobutyl-Phenyl | Fluormethyl | |
| 1.2.816 | 3-Trifluormethoxy-5-isobutyl-Phenyl | Fluormethyl | |
| 1.2.817 | 3-(2,2,2-Trifluorethoxy)-5-isobutyl-Phenyl | Fluormethyl | |
| 1.2.818 | 3-(2-Chlorethoxy)-5-isobutyl-Phenyl | Fluormethyl | |
| 1.2.819 | 3-(2-Hydroxyethoxy)-5-isobutyl-Phenyl- | Fluormethyl | |
| 1.2.820 | 3-[(tert-Butoxycarbonyl)oxy]-5-isobutyl-Phenyl | Fluormethyl | |
| 1.2.821 | 3-Nitro-5-isobutyl-Phenyl | Fluormethyl | |
| 1.2.822 | 3-Acetoxy-5-isobutyl-Phenyl | Fluormethyl | |
| 1.2.823 | {3-[(tert-Butoxycarbonyl)amino]-5-isobutyl-Phenyl} | Fluormethyl | |
| 1.2.824 | 3-Methylsulfanyl-5-isobutyl-Phenyl | Fluormethyl | |
| 1.2.825 | 3,5-D(itert.butyl)-Phenyl | Fluormethyl | |
| 1.2.826 | 3-Cyclopropyl-5-tert.butyl-Phenyl | Fluormethyl | |
| 1.2.827 | 3-Vinyl-5-tert.butyl-Phenyl | Fluormethyl | |
| 1.2.828 | 3-Ethinyl-5-tert.butyl-Phenyl | Fluormethyl | |
| 1.2.829 | 3-Cyano-5-tert.butyl-Phenyl | Fluormethyl | |
| 1.2.830 | 3-Trifluormethyl-5-tert.butyl-Phenyl | Fluormethyl | |
| 1.2.831 | 3-(Hydroxycarbonyl)-5-tert.butyl-Phenyl | Fluormethyl | |
| 1.2.832 | 3-(Methoxycarbonyl)-5-tert.butyl-Phenyl | Fluormethyl | |
| 1.2.833 | 3-Hydroxymethyl-5-tert.butyl-Phenyl | Fluormethyl | |
| 1.2.834 | 3-Carbamoyl-5-tert.butyl-Phenyl | Fluormethyl | |
| 1.2.835 | 3-Hydroxy-5-tert.butyl-Phenyl- | Fluormethyl | |
| 1.2.836 | 3-Methoxy-5-tert.butyl-Phenyl | Fluormethyl | |
| 1.2.837 | 3-Ethoxy-5-tert.butyl-Phenyl | Fluormethyl | |
| 1.2.838 | 3-nPropyoxy-5-tert.butyl-Phenyl | Fluormethyl | |
| 1.2.839 | 3-nButoxy-5-tert.butyl-Phenyl | Fluormethyl | |
| 1.2.840 | 3-isoButoxy-5-tert.butyl-Phenyl | Fluormethyl | |
| 1.2.841 | 3-tert.Butoxy-5-tert.butyl-Phenyl | Fluormethyl | |
| 1.2.842 | 3-Difluormethoxy-5-tert.butyl-Phenyl | Fluormethyl | |
| 1.2.843 | 3-Trifluormethoxy-5-tert.butyl-Phenyl | Fluormethyl | |
| 1.2.844 | 3-(2,2,2-Trifluorethoxy)-5-tert.butyl-Phenyl | Fluormethyl | |
| 1.2.845 | 3-(2-Chlorethoxy)-5-tert.butyl-Phenyl | Fluormethyl | |
| 1.2.846 | 3-(2-Hydroxyethoxy)-5-tert.butyl-Phenyl- | Fluormethyl | |
| 1.2.847 | 3-[(tert-Butoxycarbonyl)oxy]-5-tert.butyl-Phenyl | Fluormethyl | |
| 1.2.848 | 3-Nitro-5-tert.butyl-Phenyl | Fluormethyl | |
| 1.2.849 | 3-Acetoxy-5-tert.butyl-Phenyl | Fluormethyl | |
| 1.2.850 | {3-[(tert-Butoxycarbonyl)amino]-5-tert.butyl-Phenyl} | Fluormethyl | |
| 1.2.851 | 3-Methylsulfanyl-5-tert.butyl-Phenyl | Fluormethyl | |
| 1.2.852 | 3-tert.Butyl-5-cyclopropyl-Phenyl | Fluormethyl | |
| 1.2.853 | 3,5-Dicyclopropyl-Phenyl | Fluormethyl | |
| 1.2.854 | 3-Vinyl-5-cyclopropyl-Phenyl | Fluormethyl | |
| 1.2.855 | 3-Ethinyl-5-cyclopropyl-Phenyl | Fluormethyl | |
| 1.2.856 | 3-Cyano-5-cyclopropyl-Phenyl | Fluormethyl | |
| 1.2.857 | 3-Trifluormethyl-5-cyclopropyl-Phenyl | Fluormethyl | |
| 1.2.858 | 3-(Hydroxycarbonyl)-5-cyclopropyl-Phenyl | Fluormethyl | |
| 1.2.859 | 3-(Methoxycarbonyl)-5-cyclopropyl-Phenyl | Fluormethyl | |
| 1.2.860 | 3-Hydroxymethyl-5-cyclopropyl-Phenyl | Fluormethyl | |
| 1.2.861 | 3-Carbamoyl-5-cyclopropyl-Phenyl | Fluormethyl | |
| 1.2.862 | 3-Hydroxy-5-cyclopropyl-Phenyl- | Fluormethyl | |
| 1.2.863 | 3-Methoxy-5-cyclopropyl-Phenyl | Fluormethyl | |
| 1.2.864 | 3-Ethoxy-5-cyclopropyl-Phenyl | Fluormethyl | |
| 1.2.865 | 3-nPropyoxy-5-cyclopropyl-Phenyl | Fluormethyl | |
| 1.2.866 | 3-nButoxy-5-cyclopropyl-Phenyl | Fluormethyl | |
| 1.2.867 | 3-isoButoxy-5-cyclopropyl-Phenyl | Fluormethyl | |
| 1.2.868 | 3-tert.Butoxy-5-cyclopropyl-Phenyl | Fluormethyl | |
| 1.2.869 | 3-Difluormethoxy-5-cyclopropyl-Phenyl | Fluormethyl | |
| 1.2.870 | 3-Trifluormethoxy-5-cyclopropyl-Phenyl | Fluormethyl | |
| 1.2.871 | 3-(2,2,2-Trifluorethoxy)-5-cyclopropyl-Phenyl | Fluormethyl | |
| 1.2.872 | 3-(2-Chlorethoxy)-5-cyclopropyl-Phenyl | Fluormethyl | |
| 1.2.873 | 3-(2-Hydroxyethoxy)-5-cyclopropyl-Phenyl- | Fluormethyl | |
| 1.2.874 | 3-[(tert-Butoxycarbonyl)oxy]-5-cyclopropyl-Phenyl | Fluormethyl | |
| 1.2.875 | 3-Nitro-5-cyclopropyl-Phenyl | Fluormethyl | |
| 1.2.876 | 3-Acetoxy-5-cyclopropyl-Phenyl | Fluormethyl | |
| 1.2.877 | {3-[(tert-Butoxycarbonyl)amino]-5-cyclopropyl-Phenyl} | Fluormethyl | |
| 1.2.878 | 3-Methylsulfanyl-5-cyclopropyl-Phenyl | Fluormethyl | |
| 1.2.879 | 3,5-Divinyl-Phenyl | Fluormethyl | |
| 1.2.880 | 3-Ethinyl-5-vinyl-Phenyl | Fluormethyl | |
| 1.2.881 | 3-Cyano-5-vinyl-Phenyl | Fluormethyl | |
| 1.2.882 | 3-Trifluormethyl-5-vinyl-Phenyl | Fluormethyl | |
| 1.2.883 | 3-(Hydroxycarbonyl)-5-vinyl-Phenyl | Fluormethyl | |
| 1.2.884 | 3-(Methoxycarbonyl)-5-vinyl-Phenyl | Fluormethyl | |
| 1.2.885 | 3-Hydroxymethyl-5-vinyl-Phenyl | Fluormethyl | |
| 1.2.886 | 3-Carbamoyl-5-vinyl-Phenyl | Fluormethyl | |
| 1.2.887 | 3-Hydroxy-5-vinyl-Phenyl- | Fluormethyl | |
| 1.2.888 | 3-Methoxy-5-vinyl-Phenyl | Fluormethyl | |
| 1.2.889 | 3-Ethoxy-5-vinyl-Phenyl | Fluormethyl | |
| 1.2.890 | 3-nPropyoxy-5-vinyl-Phenyl | Fluormethyl | |
| 1.2.891 | 3-nButoxy-5-vinyl-Phenyl | Fluormethyl | |
| 1.2.892 | 3-isoButoxy-5-vinyl-Phenyl | Fluormethyl | |
| 1.2.893 | 3-tert.Butoxy-5-vinyl-Phenyl | Fluormethyl | |
| 1.2.894 | 3-Difluormethoxy-5-vinyl-Phenyl | Fluormethyl | |
| 1.2.895 | 3-Trifluormethoxy-5-vinyl-Phenyl | Fluormethyl | |
| 1.2.896 | 3-(2,2,2-Trifluorethoxy)-5-vinyl-Phenyl | Fluormethyl | |
| 1.2.897 | 3-(2-Chlorethoxy)-5-vinyl-Phenyl | Fluormethyl | |
| 1.2.898 | 3-(2-Hydroxyethoxy)-5-vinyl-Phenyl- | Fluormethyl | |
| 1.2.899 | 3-[(tert-Butoxycarbonyl)oxy]-5-vinyl-Phenyl | Fluormethyl | |
| 1.2.900 | 3-Nitro-5-vinyl-Phenyl | Fluormethyl | |
| 1.2.901 | 3-Acetoxy-5-vinyl-Phenyl | Fluormethyl | |
| 1.2.902 | {3-[(tert-Butoxycarbonyl)amino]-5-vinyl-Phenyl} | Fluormethyl | |
| 1.2.903 | 3-Methylsulfanyl-5-vinyl-Phenyl | Fluormethyl | |
| 1.2.904 | 3,5-Diethinyl-Phenyl | Fluormethyl | |
| 1.2.905 | 3-Cyano-5-ethinyl-Phenyl | Fluormethyl | |
| 1.2.906 | 3-Trifluormethyl-5-ethinyl-Phenyl | Fluormethyl | |
| 1.2.907 | 3-(Hydroxycarbonyl)-5-ethinyl-Phenyl | Fluormethyl | |
| 1.2.908 | 3-(Methoxycarbonyl)-5-ethinyl-Phenyl | Fluormethyl | |
| 1.2.909 | 3-Hydroxymethyl-5-ethinyl-Phenyl | Fluormethyl | |
| 1.2.910 | 3-Carbamoyl-5-ethinyl-Phenyl | Fluormethyl | |
| 1.2.911 | 3-Hydroxy-5-ethinyl-Phenyl- | Fluormethyl | |
| 1.2.912 | 3-Methoxy-5-ethinyl-Phenyl | Fluormethyl | |
| 1.2.913 | 3-Ethoxy-5-ethinyl-Phenyl | Fluormethyl | |
| 1.2.914 | 3-nPropyoxy-5-ethinyl-Phenyl | Fluormethyl | |
| 1.2.915 | 3-nButoxy-5-ethinyl-Phenyl | Fluormethyl | |
| 1.2.916 | 3-isoButoxy-5-ethinyl-Phenyl | Fluormethyl | |
| 1.2.917 | 3-tert.Butoxy-5-ethinyl-Phenyl | Fluormethyl | |
| 1.2.918 | 3-Difluormethoxy-5-ethinyl-Phenyl | Fluormethyl | |
| 1.2.919 | 3-Trifluormethoxy-5-ethinyl-Phenyl | Fluormethyl | |
| 1.2.920 | 3-(2,2,2-Trifluorethoxy)-5-ethinyl-Phenyl | Fluormethyl | |
| 1.2.921 | 3-(2-Chlorethoxy)-5-ethinyl-Phenyl | Fluormethyl | |
| 1.2.922 | 3-(2-Hydroxyethoxy)-5-ethinyl-Phenyl- | Fluormethyl | |
| 1.2.923 | 3-[(tert-Butoxycarbonyl)oxy]-5-ethinyl-Phenyl | Fluormethyl | |
| 1.2.924 | 3-Nitro-5-ethinyl-Phenyl | Fluormethyl | |
| 1.2.925 | 3-Acetoxy-5-ethinyl-Phenyl | Fluormethyl | |
| 1.2.926 | {3-[(tert-Butoxycarbonyl)amino]-5-ethinyl-Phenyl} | Fluormethyl | |
| 1.2.927 | 3-Methylsulfanyl-5-ethinyl-Phenyl | Fluormethyl | |
| 1.2.928 | 3,5-Dicyano-Phenyl | Fluormethyl | |
| 1.2.929 | 3-Trifluormethyl-5-cyano-Phenyl | Fluormethyl | |
| 1.2.930 | 3-(Hydroxycarbonyl)-5-cyano-Phenyl | Fluormethyl | |
| 1.2.931 | 3-(Methoxycarbonyl)-5-cyano-Phenyl | Fluormethyl | |
| 1.2.932 | 3-Hydroxymethyl-5-cyano-Phenyl | Fluormethyl | |
| 1.2.933 | 3-Carbamoyl-5-cyano-Phenyl | Fluormethyl | |
| 1.2.934 | 3-Hydroxy-5-cyano-Phenyl- | Fluormethyl | |
| 1.2.935 | 3-Methoxy-5-cyano-Phenyl | Fluormethyl | |
| 1.2.936 | 3-Ethoxy-5-cyano-Phenyl | Fluormethyl | |
| 1.2.937 | 3-nPropyoxy-5-cyano-Phenyl | Fluormethyl | |
| 1.2.938 | 3-nButoxy-5-cyano-Phenyl | Fluormethyl | |
| 1.2.939 | 3-isoButoxy-5-cyano-Phenyl | Fluormethyl | |
| 1.2.940 | 3-tert.Butoxy-5-cyano-Phenyl | Fluormethyl | |
| 1.2.941 | 3-Difluormethoxy-5-cyano-Phenyl | Fluormethyl | |
| 1.2.942 | 3-Trifluormethoxy-5-cyano-Phenyl | Fluormethyl | |
| 1.2.943 | 3-(2,2,2-Trifluorethoxy)-5-cyano-Phenyl | Fluormethyl | |
| 1.2.944 | 3-(2-Chlorethoxy)-5-cyano-Phenyl | Fluormethyl | |
| 1.2.945 | 3-(2-Hydroxyethoxy)-5-cyano-Phenyl- | Fluormethyl | |
| 1.2.946 | 3-[(tert-Butoxycarbonyl)oxy]-5-cyano-Phenyl | Fluormethyl | |
| 1.2.947 | 3-Nitro-5-cyano-Phenyl | Fluormethyl | |
| 1.2.948 | 3-Acetoxy-5-cyano-Phenyl | Fluormethyl | |
| 1.2.949 | {3-[(tert-Butoxycarbonyl)amino]-5-cyano-Phenyl} | Fluormethyl | |
| 1.2.950 | 3-Methylsulfanyl-5-cyano-Phenyl | Fluormethyl | |
| 1.2.951 | 3,5-Di(trifluormethyl)-Phenyl | Fluormethyl | |
| 1.2.952 | 3-(Hydroxycarbonyl)-5-trifluormethyl-Phenyl | Fluormethyl | |
| 1.2.953 | 3-(Methoxycarbonyl)-5-trifluormethyl-Phenyl | Fluormethyl | |
| 1.2.954 | 3-Hydroxymethyl-5-trifluormethyl-Phenyl | Fluormethyl | |
| 1.2.955 | 3-Carbamoyl-5-trifluormethyl-Phenyl | Fluormethyl | |
| 1.2.956 | 3-Hydroxy-5-trifluormethyl-Phenyl- | Fluormethyl | |
| 1.2.957 | 3-Methoxy-5-trifluormethyl-Phenyl | Fluormethyl | |
| 1.2.958 | 3-Ethoxy-5trifluormethyl-Phenyl | Fluormethyl | |
| 1.2.959 | 3-nPropyoxy-5-trifluormethyl-Phenyl | Fluormethyl | |
| 1.2.960 | 3-nButoxy-5-trifluormethyl-Phenyl | Fluormethyl | |
| 1.2.961 | 3-isoButoxy-5-trifluormethyl-Phenyl | Fluormethyl | |
| 1.2.962 | 3-tert. Butoxy-5-trifluormethyl-Phenyl | Fluormethyl | |
| 1.2.963 | 3-Difluormethoxy-5-trifluormethyl-Phenyl | Fluormethyl | |
| 1.2.964 | 3-Trifluormethoxy-5-trifluormethyl-Phenyl | Fluormethyl | |
| 1.2.965 | 3-(2,2,2-Trifluorethoxy)-5-trifluormethyl-Phenyl | Fluormethyl | |
| 1.2.966 | 3-(2-Chlorethoxy)-5-trifluormethylPhenyl | Fluormethyl | |
| 1.2.967 | 3-(2-Hydroxyethoxy)-5-trifluormethyl-Phenyl- | Fluormethyl | |
| 1.2.968 | 3-[(tert-Butoxycarbonyl)oxy]-5-trifluormethyl-Phenyl | Fluormethyl | |
| 1.2.969 | 3-Nitro-5-trifluormethyl-Phenyl | Fluormethyl | |
| 1.2.970 | 3-Acetoxy-5-trifluormethyl-Phenyl | Fluormethyl | |
| 1.2.971 | {3-[(tert-Butoxycarbonyl)amino]-5-trifluormethyl-Phenyl} | Fluormethyl | |
| 1.2.972 | 3-Methylsulfanyl-5-trifluormethyl-Phenyl | Fluormethyl | |
| 1.2.973 | 3,5-Bis(hydroxycarbonyl)-Phenyl | Fluormethyl | |
| 1.2.974 | 3-(Methoxycarbonyl)-5-(hydroxycarbonyl)-Phenyl | Fluormethyl | |
| 1.2.975 | 3-Hydroxymethyl-5-(hydroxycarbonyl)-Phenyl | Fluormethyl | |
| 1.2.976 | 3-Carbamoyl-5-(hydroxycarbonyl)-Phenyl | Fluormethyl | |
| 1.2.977 | 3-Hydroxy-5-(hydroxycarbonyl)-Phenyl- | Fluormethyl | |
| 1.2.978 | 3-Methoxy-5-(hydroxycarbonyl)-Phenyl | Fluormethyl | |
| 1.2.979 | 3-Ethoxy-5-(hydroxycarbonyl)-Phenyl | Fluormethyl | |
| 1.2.980 | 3-nPropyoxy-5-(hydroxycarbonyl)-Phenyl | Fluormethyl | |
| 1.2.981 | 3-nButoxy-5-(hydroxycarbonyl)-Phenyl | Fluormethyl | |
| 1.2.982 | 3-isoButoxy-5-(hydroxycarbonyl)-Phenyl | Fluormethyl | |
| 1.2.983 | 3-tert.Butoxy-5-(hydroxycarbonyl)-Phenyl | Fluormethyl | |
| 1.2.984 | 3-Difluormethoxy-5-(hydroxycarbonyl)-Phenyl | Fluormethyl | |
| 1.2.985 | 3-Trifluormethoxy-5-(hydroxycarbonyl)-Phenyl | Fluormethyl | |
| 1.2.986 | 3-(2,2,2-Trifluorethoxy)-5-(hydroxycarbonyl)-Phenyl | Fluormethyl | |
| 1.2.987 | 3-(2-Chlorethoxy)-5-(hydroxycarbonyl)-Phenyl | Fluormethyl | |
| 1.2.988 | 3-(2-Hydroxyethoxy)-5-(hydroxycarbonyl)-Phenyl- | Fluormethyl | |
| 1.2.989 | 3-[(tert-Butoxycarbonyl)oxy]-5-(hydroxycarbonyl)-Phenyl | Fluormethyl | |
| 1.2.990 | 3-Nitro-5-(hydroxycarbonyl)-Phenyl | Fluormethyl | |
| 1.2.991 | 3-Acetoxy-5-(hydroxycarbonyl)-Phenyl | Fluormethyl | |
| 1.2.992 | {3-[(tert-Butoxycarbonyl)amino]-5-(hydroxycarbonyl)-Phenyl} | Fluormethyl | |
| 1.2.993 | 3-Methylsulfanyl-5-(hydroxycarbonyl)-Phenyl | Fluormethyl | |
| 1.2.994 | 3,5-Di(methoxycarbonyl)-Phenyl | Fluormethyl | |
| 1.2.995 | 3-Hydroxymethyl-5-(methoxycarbonyl)-Phenyl | Fluormethyl | |
| 1.2.996 | 3-Carbamoyl-5-(methoxycarbonyl)-Phenyl | Fluormethyl | |
| 1.2.997 | 3-Hydroxy-5-(methoxycarbonyl)-Phenyl- | Fluormethyl | |
| 1.2.998 | 3-Methoxy-5-(methoxycarbonyl)-Phenyl | Fluormethyl | |
| 1.2.999 | 3-Ethoxy-5-(methoxycarbonyl)-Phenyl | Fluormethyl | |
| 1.2.1000 | 3-nPropyoxy-5-(methoxycarbonyl)-Phenyl | Fluormethyl | |
| 1.2.1001 | 3-nButoxy-5-(methoxycarbonyl)-Phenyl | Fluormethyl | |
| 1.2.1002 | 3-isoButoxy-5-(methoxycarbonyl)-Phenyl | Fluormethyl | |
| 1.2.1003 | 3-tert. Butoxy-5-(methoxycarbonyl)-Phenyl | Fluormethyl | |
| 1.2.1004 | 3-Difluormethoxy-5-(methoxycarbonyl)-Phenyl | Fluormethyl | |
| 1.2.1005 | 3-Trifluormethoxy-5-(methoxycarbonyl)-Phenyl | Fluormethyl | |
| 1.2.1006 | 3-(2,2,2-Trifluorethoxy)-5-(methoxycarbonyl)-Phenyl | Fluormethyl | |
| 1.2.1007 | 3-(2-Chlorethoxy)-5-(methoxycarbonyl)-Phenyl | Fluormethyl | |
| 1.2.1008 | 3-(2-Hydroxyethoxy)-5-(methoxycarbonyl)-Phenyl- | Fluormethyl | |
| 1.2.1009 | 3-[(tert-Butoxycarbonyl)oxy]-5-(methoxycarbonyl)-Phenyl | Fluormethyl | |
| 1.2.1010 | 3-Nitro-5-(methoxycarbonyl)-Phenyl | Fluormethyl | |
| 1.2.1011 | 3-Acetoxy-5-(methoxycarbonyl)-Phenyl | Fluormethyl | |
| 1.2.1012 | {3-[(tert-Butoxycarbonyl)amino]-5-(methoxycarbonyl)-Phenyl} | Fluormethyl | |
| 1.2.1013 | 3-Methylsulfanyl-5-(methoxycarbonyl)-Phenyl | Fluormethyl | |
| 1.2.1014 | 3,5-Di(hydroxymethy)l-Phenyl | Fluormethyl | |
| 1.2.1015 | 3-Carbamoyl-5-hydroxymethyl-Phenyl | Fluormethyl | |
| 1.2.1016 | 3-Hydroxy-5-hydroxymethyl-Phenyl- | Fluormethyl | |
| 1.2.1017 | 3-Methoxy-5-hydroxymethyl-Phenyl | Fluormethyl | |
| 1.2.1018 | 3-Ethoxy-5-hydroxymethyl-Phenyl | Fluormethyl | |
| 1.2.1019 | 3-nPropyoxy-5-hydroxymethyl-Phenyl | Fluormethyl | |
| 1.2.1020 | 3-nButoxy-5-hydroxymethyl-Phenyl | Fluormethyl | |
| 1.2.1021 | 3-isoButoxy-5-hydroxymethyl-Phenyl | Fluormethyl | |
| 1.2.1022 | 3-tert. Butoxy-5-hydroxymethyl-Phenyl | Fluormethyl | |
| 1.2.1023 | 3-Difluormethoxy-5-hydroxymethyl-Phenyl | Fluormethyl | |
| 1.2.1024 | 3-Trifluormethoxy-5-hydroxymethyl-Phenyl | Fluormethyl | |
| 1.2.1025 | 3-(2,2,2-Trifluorethoxy)-5-hydroxymethyl-Phenyl | Fluormethyl | |
| 1.2.1026 | 3-(2-Chlorethoxy)-5-hydroxymethyl-Phenyl | Fluormethyl | |
| 1.2.1027 | 3-(2-Hydroxyethoxy)-5-hydroxymethyl-Phenyl | Fluormethyl | |
| 1.2.1028 | 3-[(tert-Butoxycarbonyl)oxy]-5-hydroxymethyl-Phenyl | Fluormethyl | |
| 1.2.1029 | 3-Nitro-5-hydroxymethyl-Phenyl | Fluormethyl | |
| 1.2.1030 | 3-Acetoxy-5-hydroxymethyl-Phenyl | Fluormethyl | |
| 1.2.1031 | {3-[(tert-Butoxycarbonyl)amino]-5-hydroxymethyl-Phenyl} | Fluormethyl | |
| 1.2.1032 | 3-Methylsulfanyl-5-hydroxymethyl-Phenyl | Fluormethyl | |
| 1.2.1033 | 3,5-Dicarbamoyl-5-carbamoyl-Phenyl | Fluormethyl | |
| 1.2.1034 | 3-Hydroxy-5-carbamoyl-Phenyl- | Fluormethyl | |
| 1.2.1035 | 3-Methoxy-5-carbamoyl-Phenyl | Fluormethyl | |
| 1.2.1036 | 3-Ethoxy-5-carbamoyl-Phenyl | Fluormethyl | |
| 1.2.1037 | 3-nPropyoxy-5-carbamoyl-Phenyl | Fluormethyl | |
| 1.2.1038 | 3-nButoxy-5-carbamoyl-Phenyl | Fluormethyl | |
| 1.2.1039 | 3-isoButoxy-5-carbamoyl-Phenyl | Fluormethyl | |
| 1.2.1040 | 3-tert.Butoxy-5-carbamoyl-Phenyl | Fluormethyl | |
| 1.2.1041 | 3-Difluormethoxy-5-carbamoyl-Phenyl | Fluormethyl | |
| 1.2.1042 | 3-Trifluormethoxy-5-carbamoyl-Phenyl | Fluormethyl | |
| 1.2.1043 | 3-(2,2,2-Trifluorethoxy)-5-carbamoyl-Phenyl | Fluormethyl | |
| 1.2.1044 | 3-(2-Chlorethoxy)-5-carbamoyl-Phenyl | Fluormethyl | |
| 1.2.1045 | 3-(2-Hydroxyethoxy)-5-carbamoyl-Phenyl- | Fluormethyl | |
| 1.2.1046 | 3-[(tert-Butoxycarbonyl)oxy]-5-carbamoyl-Phenyl | Fluormethyl | |
| 1.2.1047 | 3-Nitro-5-carbamoyl-Phenyl | Fluormethyl | |
| 1.2.1048 | 3-Acetoxy-5-carbamoyl-Phenyl | Fluormethyl | |
| 1.2.1049 | {3-[(tert-Butoxycarbonyl)amino]-5-carbamoyl-Phenyl} | Fluormethyl | |
| 1.2.1050 | 3-Methylsulfanyl-5-carbamoyl-Phenyl | Fluormethyl | |
| 1.2.1051 | 3,5-Dihydroxy-Phenyl | Fluormethyl | |
| 1.2.1052 | 3-Methoxy-5-hydroxy-Phenyl | Fluormethyl | |
| 1.2.1053 | 3-Ethoxy-5-hydroxy-Phenyl | Fluormethyl | |
| 1.2.1054 | 3-nPropyoxy-5-hydroxy-Phenyl | Fluormethyl | |
| 1.2.1055 | 3-nButoxy-5-hydroxy-Phenyl | Fluormethyl | |
| 1.2.1056 | 3-isoButoxy-5-hydroxy-Phenyl | Fluormethyl | |
| 1.2.1057 | 3-tert. Butoxy-5-hydroxy-Phenyl | Fluormethyl | |
| 1.2.1058 | 3-Difluormethoxy-5-hydroxy-Phenyl | Fluormethyl | |
| 1.2.1059 | 3-Trifluormethoxy-5-hydroxy-Phenyl | Fluormethyl | |
| 1.2.1060 | 3-(2,2,2-Trifluorethoxy)-5-hydroxy-Phenyl | Fluormethyl | |
| 1.2.1061 | 3-(2-Chlorethoxy)-5-hydroxy-Phenyl | Fluormethyl | |
| 1.2.1062 | 3-(2-Hydroxyethoxy)-5-hydroxy-Phenyl- | Fluormethyl | |
| 1.2.1063 | 3-[(tert-Butoxycarbonyl)oxy]-5-hydroxy-Phenyl | Fluormethyl | |
| 1.2.1064 | 3-Nitro-5-hydroxy-Phenyl | Fluormethyl | |
| 1.2.1065 | 3-Acetoxy-5-hydroxy-Phenyl | Fluormethyl | |
| 1.2.1066 | {3-[(tert-Butoxycarbonyl)amino]-5-hydroxy-Phenyl} | Fluormethyl | |
| 1.2.1067 | 3-Methylsulfanyl-5-hydroxy-Phenyl | Fluormethyl | |
| 1.2.1068 | 3,5-Dimethoxy-Phenyl | Fluormethyl | |
| 1.2.1069 | 3-Ethoxy-5-methoxy-Phenyl | Fluormethyl | |
| 1.2.1070 | 3-nPropyoxy-5-methoxy-Phenyl | Fluormethyl | |
| 1.2.1071 | 3-nButoxy-5-methoxy-Phenyl | Fluormethyl | |
| 1.2.1072 | 3-isoButoxy-5-methoxy-Phenyl | Fluormethyl | |
| 1.2.1073 | 3-tert.Butoxy-5-methoxy-Phenyl | Fluormethyl | |
| 1.2.1074 | 3-Difluormethoxy-5-methoxy-Phenyl | Fluormethyl | |
| 1.2.1075 | 3-Trifluormethoxy-5-methoxyPhenyl | Fluormethyl | |
| 1.2.1076 | 3-(2,2,2-Trifluorethoxy)-5-methoxy-Phenyl | Fluormethyl | |
| 1.2.1077 | 3-(2-Chlorethoxy)-5-methoxy-Phenyl | Fluormethyl | |
| 1.2.1078 | 3-(2-Hydroxyethoxy)-5-methoxy-Phenyl- | Fluormethyl | |
| 1.2.1079 | 3-[(tert-Butoxycarbonyl)oxy]-5-methoxy-Phenyl | Fluormethyl | |
| 1.2.1080 | 3-Nitro-5-methoxy-Phenyl | Fluormethyl | |
| 1.2.1081 | 3-Acetoxy-5-methoxy-Phenyl | Fluormethyl | |
| 1.2.1082 | {3-[(tert-Butoxycarbonyl)amino]-5-methoxy-Phenyl} | Fluormethyl | |
| 1.2.1083 | 3-Methylsulfanyl-5-methoxy-Phenyl | Fluormethyl | |
| 1.2.1084 | 3,5-Diethoxy-Phenyl | Fluormethyl | |
| 1.2.1085 | 3-nPropyoxy-5-ethoxy-Phenyl | Fluormethyl | |
| 1.2.1086 | 3-nButoxy-5-ethoxy-Phenyl | Fluormethyl | |
| 1.2.1087 | 3-isoButoxy-5-ethoxy-Phenyl | Fluormethyl | |
| 1.2.1088 | 3-tert.Butoxy-5-ethoxy-Phenyl | Fluormethyl | |
| 1.2.1089 | 3-Difluormethoxy-5-ethoxy-Phenyl | Fluormethyl | |
| 1.2.1090 | 3-Trifluormethoxy-5-ethoxy-Phenyl | Fluormethyl | |
| 1.2.1091 | 3-(2,2,2-Trifluorethoxy)-5-ethoxy-Phenyl | Fluormethyl | |
| 1.2.1092 | 3-(2-Chlorethoxy)-5-ethoxy-Phenyl | Fluormethyl | |
| 1.2.1093 | 3-(2-Hydroxyethoxy)-5-ethoxy-Phenyl- | Fluormethyl | |
| 1.2.1094 | 3-[(tert-Butoxycarbonyl)oxy]-5-ethoxy-Phenyl | Fluormethyl | |
| 1.2.1095 | 3-Nitro-5-ethoxy-Phenyl | Fluormethyl | |
| 1.2.1096 | 3-Acetoxy-5-ethoxy-Phenyl | Fluormethyl | |
| 1.2.1097 | {3-[(tert-Butoxycarbonyl)amino]-5-ethoxy-Phenyl} | Fluormethyl | |
| 1.2.1098 | 3-Methylsulfanyl-5-ethoxy-Phenyl | Fluormethyl | |
| 1.2.1099 | 3,5-Dipropyoxy-Phenyl | Fluormethyl | |
| 1.2.1100 | 3-nButoxy-5-propoxy-Phenyl | Fluormethyl | |
| 1.2.1101 | 3-isoButoxy-5-propoxy-Phenyl | Fluormethyl | |
| 1.2.1102 | 3-tert.Butoxy-5-propoxy-Phenyl | Fluormethyl | |
| 1.2.1103 | 3-Difluormethoxy-5-propoxy-Phenyl | Fluormethyl | |
| 1.2.1104 | 3-Trifluormethoxy-5-propoxy-Phenyl | Fluormethyl | |
| 1.2.1105 | 3-(2,2,2-Trifluorethoxy)-5-propoxy-Phenyl | Fluormethyl | |
| 1.2.1106 | 3-(2-Chlorethoxy)-5-propoxy-Phenyl | Fluormethyl | |
| 1.2.1107 | 3-(2-Hydroxyethoxy)-5-propoxy-Phenyl- | Fluormethyl | |
| 1.2.1108 | 3-[(tert-Butoxycarbonyl)oxy]-5-propoxy-Phenyl | Fluormethyl | |
| 1.2.1109 | 3-Nitro-5-propoxy-Phenyl | Fluormethyl | |
| 1.2.1110 | 3-Acetoxy-5-propoxy-Phenyl | Fluormethyl | |
| 1.2.1111 | {3-[(tert-Butoxycarbonyl)amino]-5-propoxy-Phenyl} | Fluormethyl | |
| 1.2.1112 | 3-Methylsulfanyl-5-propoxy-Phenyl | Fluormethyl | |
| 1.2.1113 | 3,5-Di(isopropyoxy)-Phenyl | Fluormethyl | |
| 1.2.1114 | 3-nButoxy-5-isopropoxy-Phenyl | Fluormethyl | |
| 1.2.1115 | 3-isoButoxy-5-isopropoxy-Phenyl | Fluormethyl | |
| 1.2.1116 | 3-tert.Butoxy-5-isopropoxy-Phenyl | Fluormethyl | |
| 1.2.1117 | 3-Difluormethoxy-5-isopropoxy-Phenyl | Fluormethyl | |
| 1.2.1118 | 3-Trifluormethoxy-5-isopropoxy-Phenyl | Fluormethyl | |
| 1.2.1119 | 3-(2,2,2-Trifluorethoxy)-5-isopropoxy-Phenyl | Fluormethyl | |
| 1.2.1120 | 3-(2-Chlorethoxy)-5-isopropoxy-Phenyl | Fluormethyl | |
| 1.2.1121 | 3-(2-Hydroxyethoxy)-5-isopropoxy-Phenyl- | Fluormethyl | |
| 1.2.1122 | 3-[(tert-Butoxycarbonyl)oxy]-5-isopropoxy-Phenyl | Fluormethyl | |
| 1.2.1123 | 3-Nitro-5-isopropoxy-Phenyl | Fluormethyl | |
| 1.2.1124 | 3-Acetoxy-5-isopropoxy-Phenyl | Fluormethyl | |
| 1.2.1125 | {3-[(tert-Butoxycarbonyl)amino]-5-isopropoxy-Phenyl} | Fluormethyl | |
| 1.2.1126 | 3-Methylsulfanyl-5-isopropoxy-Phenyl | Fluormethyl | |
| 1.2.1127 | 3,5-Di(tert.butoxy)-Phenyl | Fluormethyl | |
| 1.2.1128 | 3-Difluormethoxy-5-tert.butoxy-Phenyl | Fluormethyl | |
| 1.2.1129 | 3-Trifluormethoxy-5-tert.butoxy-Phenyl | Fluormethyl | |
| 1.2.1130 | 3-(2,2,2-Trifluorethoxy)-5-tert.butoxy-Phenyl | Fluormethyl | |
| 1.2.1131 | 3-(2-Chlorethoxy)-5-tert.butoxyl-Phenyl | Fluormethyl | |
| 1.2.1132 | 3-(2-Hydroxyethoxy)-5-tert.butoxy-Phenyl- | Fluormethyl | |
| 1.2.1133 | 3-[(tert-Butoxycarbonyl)oxy]-5-tert.butoxy-Phenyl | Fluormethyl | |
| 1.2.1134 | 3-Nitro-5-tert.butoxy-Phenyl | Fluormethyl | |
| 1.2.1135 | 3-Acetoxy-5-tert.butoxy-Phenyl | Fluormethyl | |
| 1.2.1136 | {3-[(tert-Butoxycarbonyl)amino]-5-tert.butoxy-Phenyl} | Fluormethyl | |
| 1.2.1137 | 3-Methylsulfanyl-5-tert.butoxy-Phenyl | Fluormethyl | |
| 1.2.1138 | 3,5-Di(trifluormethoxy)-Phenyl | Fluormethyl | |
| 1.2.1139 | 3-(2,2,2-Trifluorethoxy)-5-trifluormethoxy-Phenyl | Fluormethyl | |
| 1.2.1140 | 3-(2-Chlorethoxy)-5-trifluormethoxy-Phenyl | Fluormethyl | |
| 1.2.1141 | 3-(2-Hydroxyethoxy)-5-trifluormethoxy-Phenyl- | Fluormethyl | |
| 1.2.1142 | 3-[(tert-Butoxycarbonyl)oxy]-5-trifluormethoxy-Phenyl | Fluormethyl | |
| 1.2.1143 | 3-Nitro-5-trifluormethoxy-Phenyl | Fluormethyl | |
| 1.2.1144 | 3-Acetoxy-5-tert.butoxy-Phenyl | Fluormethyl | |
| 1.2.1145 | {3-[(tert-Butoxycarbonyl)amino]-5-trifluormethoxy-Phenyl} | Fluormethyl | |
| 1.2.1146 | 3-Methylsulfanyl-5-trifluormethoxy-Phenyl | Fluormethyl | |
| 1.2.1147 | 3,5-Bis(difluormethoxy)-Phenyl | Fluormethyl | |
| 1.2.1148 | 3,5-Bis(difluormethoxy)-Phenyl | Chlormethyl | |
| 1.2.1149 | 3,5-Bis(difluormethoxy)-Phenyl | Brommethyl | |
| 1.2.1150 | 3,5-Bis(difluormethoxy)-Phenyl | Fluormethyl | |
| 1.2.1151 | 3-Trifluormethoxy-5-difluormethoxy-Phenyl | Fluormethyl | |
| 1.2.1152 | 3-(2,2,2-Trifluorethoxy)-5-difluormethoxy-Phenyl | Fluormethyl | |
| 1.2.1153 | 3-(2-Chlorethoxy)-5-difluormethoxy-Phenyl | Fluormethyl | |
| 1.2.1154 | 3-(2-Hydroxyethoxy)-5-difluormethoxy-Phenyl- | Fluormethyl | |
| 1.2.1155 | 3-[(tert-Butoxycarbonyl)oxy]-5-difluormethoxy-Phenyl | Fluormethyl | |
| 1.2.1156 | 3-Nitro-5-difluormethoxy-Phenyl | Fluormethyl | |
| 1.2.1157 | 3-Acetoxy-5-difluormethoxy-Phenyl | Fluormethyl | |
| 1.2.1158 | {3-[(tert-Butoxycarbonyl)amino]-5-difluormethoxy -Phenyl} | Fluormethyl | |
| 1.2.1159 | 3-Methylsulfanyl-5-difluormethoxy-Phenyl | Fluormethyl | |
| 1.2.1160 | 3,5-Bis(2,2,2-Trifluorethoxy)-Phenyl | Fluormethyl | |
| 1.2.1161 | 3-(2-Chlorethoxy)-5--(2,2,2-trifluorethoxy)-Phenyl | Fluormethyl | |
| 1.2.1162 | 3-(2-Hydroxyethoxy)-5-(2,2,2-trifluorethoxy)-Phenyl- | Fluormethyl | |
| 1.2.1163 | 3-[(tert-Butoxycarbonyl)oxy]-5-(2,2,2-trifluorethoxy)-Phenyl | Fluormethyl | |
| 1.2.1164 | 3-Nitro-5--(2,2,2-trifluorethoxy)-Phenyl | Fluormethyl | |
| 1.2.1165 | 3-Acetoxy-5-(2,2,2-trifluorethoxy)-Phenyl | Fluormethyl | |
| 1.2.1166 | {3-[(tert-Butoxycarbonyl)amino]-5-(2,2,2-trifluorethoxy)-Phenyl} | Fluormethyl | |
| 1.2.1167 | 3-Methylsulfanyl-5-(2,2,2-trifluorethoxy)-Phenyl | Fluormethyl | |
| 1.2.1168 | 3,5-Bis(2-Chlorethoxy)-Phenyl | Fluormethyl | |
| 1.2.1169 | 3-(2-Hydroxyethoxy)-5-(2-chlorethoxy)-Phenyl- | Fluormethyl | |
| 1.2.1170 | 3-[(tert-Butoxycarbonyl)oxy]-5-(2-chlorethoxy)-Phenyl | Fluormethyl | |
| 1.2.1171 | 3-Nitro-5-(2-chlorethoxy)-Phenyl | Fluormethyl | |
| 1.2.1172 | 3-Acetoxy-5-(2-chlorethoxy)-Phenyl | Fluormethyl | |
| 1.2.1173 | {3-[(tert-Butoxycarbonyl)amino]-5-(2-chlorethoxy)-Phenyl} | Fluormethyl | |
| 1.2.1174 | 3-Methylsulfanyl-5-(2-chlorethoxy)-Phenyl | Fluormethyl | |
| 1.2.1175 | 3,5-Bis(2-hydroxyethoxy)-Phenyl | Fluormethyl | |
| 1.2.1176 | 3-[(tert-Butoxycarbonyl)oxy]-5-(2-hydroxyethoxy)-Phenyl | Fluormethyl | |
| 1.2.1177 | 3-Nitro-5-(2-hydroxyethoxy)-Phenyl | Fluormethyl | |
| 1.2.1178 | 3-Acetoxy-5-(2-hydroxyethoxy)-Phenyl | Fluormethyl | |
| 1.2.1179 | 3-[(tert-Butoxycarbonyl)amino]-5-(2-hydroxyethoxy)-Phenyl | Fluormethyl | |
| 1.2.1180 | 3-Methylsulfanyl-5-(2-hydroxyethoxy)-Phenyl | Fluormethyl | |
| 1.2.1181 | 3,5-Bis[(tert-Butoxycarbonyl)oxy]-Phenyl | Fluormethyl | |
| 1.2.1182 | 3-Nitro-5-[(tert-Butoxycarbonyl)oxy]--Phenyl | Fluormethyl | |
| 1.2.1183 | 3-Acetoxy-5-[(tert-Butoxy-carbonyl)oxy]-Phenyl | Fluormethyl | |
| 1.2.1184 | {3-[(tert-Butoxycarbonyl)amino]-5[(tert-Butoxycarbonyl)oxy]-Phenyl} | Fluormethyl | |
| 1.2.1185 | 3,5-Bis(acetoxy)-Phenyl | Fluormethyl | |
| 1.2.1186 | {3-[(tert-Butoxycarbonyl)amino]-5-acetoxy-Phenyl} | Fluormethyl | |
| 1.2.1187 | 3-Methylsulfanyl-5-acetoxy-Phenyl | Fluormethyl | |
| 1.2.1188 | 3,5-Dinitro-Phenyl | Fluormethyl | |
| 1.2.1189 | 3-Acetoxy-5-nitro-Phenyl | Fluormethyl | |
| 1.2.1190 | {3-[(tert-Butoxycarbonyl)amino]-5-nitro-Phenyl} | Fluormethyl | |
| 1.2.1191 | 3-Methylsulfanyl-5-nitro-Phenyl | Fluormethyl | |
| 1.2.1192 | 3,5-Bis[(tert-Butoxycarbonyl)amino]-Phenyl | Fluormethyl | |
| 1.2.1193 | 3-Methylsulfanyl-5-[(tert.butoxycarbonyl)amino]-Phenyl | Fluormethyl | |
| 1.2.1194 | 3,5-Di(methylsulfanyl)-Phenyl | Fluormethyl | |
| 1.2.1195 | 3,4-Difluor-Phenyl | Fluormethyl | |
| 1.2.1196 | 3,4-Difluor-Phenyl | Chlormethyl | |
| 1.2.1197 | 3,4-Difluor-Phenyl | Brommethyl | |
| 1.2.1198 | 3,4-Difluor-Phenyl | Difluormethyll | |
| 1.2.1199 | 3,4-Difluor-Phenyl | Trifluormethyl | |
| 1.2.1200 | 3,4-Difluor-Phenyl | Cyano | |
| 1.2.1201 | 3-Chlor-4-fluor-Phenyl | Fluormethyl | |
| 1.2.1202 | 3-Chlor-4-fluor-Phenyl | Chlormethyl | |
| 1.2.1203 | 3-Chlor-4-fluor-Phenyl | Brommethyl | |
| 1.2.1204 | 3-Chlor-4-fluor-Phenyl | Difluormethyl | |
| 1.2.1205 | 3-Chlor-4-fluor-Phenyl | Trifluormethyl | [CDCl₃] 3.81 (d, 1H); 4.00 (d, 1H); 7.22 (m, 1H); 7.58 (m, 1H); 7.74 (m, 1H). |
| 1.2.1206 | 3-Chlor-4-fluor-Phenyl | Cyano | |
| 1.2.1207 | 3-Brom-4-fluor-Phenyl | Fluormethyl | |
| 1.2.1208 | 3-Methyl-4-fluor-Phenyl | Fluormethyl | |
| 1.2.1209 | 3-Methyl-4-fluor-Phenyl | Chlormethyl | |
| 1.2.1210 | 3-Ethyl-4-fluor-Phenyl | Fluormethyl | |
| 1.2.1211 | 3-Cyclopropyl-4-fluor-Phenyl | Fluormethyl | |
| 1.2.1212 | 3-Cyano-4-fluor-Phenyl | Fluormethyl | |
| 1.2.1213 | 3-Methoxy-4-fluor-Phenyl | Fluormethyl | |
| 1.2.1214 | 3-Ethoxy-4-fluor-Phenyl | Fluormethyl | |
| 1.2.1215 | 3-Trifluormethoxy-4-fluor-Phenyl | Fluormethyl | |
| 1.2.1216 | 3-Nitro-4-fluor-Phenyl | Fluormethyl | |
| 1.2.1217 | 3-Fluor-4-chlor-Phenyl | Fluormethyl | |
| 1.2.1218 | 3,4-Dichlor-Phenyl | Fluormethyl | |
| 1.2.1219 | 3-Brom-4-chlor-Phenyl | Fluormethyl | |
| 1.2.1220 | 3-Methyl-4-chlor-Phenyl | Fluormethyl | |
| 1.2.1221 | 3-Cyclopropyl-4-chlor-Phenyl | Fluormethyl | |
| 1.2.1222 | 3-Cyano-4-chlor-Phenyl | Fluormethyl | |
| 1.2.1223 | 3-Trifluormethyl-4-chlor-Phenyl | Fluormethyl | |
| 1.2.1224 | 3-Methoxy-4-chlor-Phenyl | Fluormethyl | |
| 1.2.1225 | 3-Ethoxy-4-chlor-Phenyl | Fluormethyl | |
| 1.2.1226 | 3-Trifluormethoxy-4-chlor-Phenyl | Fluormethyl | |
| 1.2.1227 | 3-Nitro-4-chlor-Phenyl | Fluormethyl | |
| 1.2.1228 | 3-Fluor-4-brom-Phenyl | Fluormethyl | |
| 1.2.1229 | 3-Chlor-4-brom-Phenyl | Fluormethyl | |
| 1.2.1230 | 3,4-Dibrom-Phenyl | Fluormethyl | |
| 1.2.1231 | 3-Methyl-4-brom-Phenyl | Fluormethyl | |
| 1.2.1232 | 3-Ethyl-4-brom-Phenyl | Fluormethyl | |
| 1.2.1233 | 3-Cyclopropyl-4-brom-Phenyl | Fluormethyl | |
| 1.2.1234 | 3-Cyano-4-brom-Phenyl | Fluormethyl | |
| 1.2.1235 | 3-Trifluormethyl-4-brom-Phenyl | Fluormethyl | |
| 1.2.1236 | 3-Methoxy-4-Phenyl | Fluormethyl | |
| 1.2.1237 | 3-Ethoxy-4-brom-Phenyl | Fluormethyl | |
| 1.2.1238 | 3-Trifluormethoxy-4-brom-Phenyl | Fluormethyl | |
| 1.2.1239 | 3-Nitro-4-brom-Phenyl | Fluormethyl | |
| 1.2.1240 | 3-Fluor-4-iod-Phenyl | Fluormethyl | |
| 1.2.1241 | 3-Chlor-4-iod-Phenyl | Fluormethyl | |
| 1.2.1242 | 3-Brom-4-iod-Phenyl | Fluormethyl | |
| 1.2.1243 | 3-Methyl-4-iod-Phenyl | Fluormethyl | |
| 1.2.1244 | 3-Ethyl-4-iod-Phenyl | Fluormethyl | |
| 1.2.1245 | 3-Cyclopropyl-4-iod-Phenyl | Fluormethyl | |
| 1.2.1246 | 3-Cyano-4-iod-Phenyl | Fluormethyl | |
| 1.2.1247 | 3-Trifluormethyl-4-iod-Phenyl | Fluormethyl | |
| 1.2.1248 | 3-Methoxy-4-iod-Phenyl | Fluormethyl | |
| 1.2.1249 | 3-Ethoxy-4-iod-Phenyl | Fluormethyl | |
| 1.2.1250 | 3-Trifluormethoxy-4-iod-Phenyl | Fluormethyl | |
| 1.2.1251 | 3-Nitro-4-iod-Phenyl | Fluormethyl | |
| 1.2.1252 | 3-Fluor-4-methyl-Phenyl | Fluormethyl | |
| 1.2.1253 | 3-Chlor-4-methyl-Phenyl | Fluormethyl | |
| 1.2.1254 | 3-Brom-4-methyl-Phenyl | Fluormethyl | |
| 1.2.1255 | 3.4-Dimethyl-Phenyl | Fluormethyl | |
| 1.2.1256 | 3.4-Dimethyl-Phenyl | Chlormethyl | |
| 1.2.1257 | 3.4-Dimethyl-Phenyl | Brommethyl | |
| 1.2.1258 | 3.4-Dimethyl-Phenyl | Difluormethyl | |
| 1.2.1259 | 3.4-Dimethyl-Phenyl | Trifluormethyl | |
| 1.2.1260 | 3.4-Dimethyl-Phenyl | Cyano | |
| 1.2.1261 | 3-Ethyl-4-methyl-Phenyl | Fluormethyl | |
| 1.2.1262 | 3-Cyclopropyl-4-methyl-Phenyl | Fluormethyl | |
| 1.2.1263 | 3-Cyano-4-methyl-Phenyl | Fluormethyl | |
| 1.2.1264 | 3-Trifluormethyl-4-methyl-Phenyl | Fluormethyl | |
| 1.2.1265 | 3-Methoxy-4-methyl-Phenyl | Fluormethyl | |
| 1.2.1266 | 3-Ethoxy-4-methyl-Phenyl | Fluormethyl | |
| 1.2.1267 | 3-Trifluormethoxy-4-methyl-Phenyl | Fluormethyl | |
| 1.2.1268 | 3-Nitro-4-methyl-Phenyl | Fluormethyl | |
| 1.2.1269 | 3-Fluor-4-ethyl-Phenyl | Fluormethyl | |
| 1.2.1270 | 3-Chlor-4-ethyl-Phenyl | Fluormethyl | |
| 1.2.1271 | 3-Brom-4-ethyl-Phenyl | Fluormethyl | |
| 1.2.1272 | 3-Methyl-4-ethyl-Phenyl | Fluormethyl | |
| 1.2.1273 | 3,4-Diethyl-Phenyl | Fluormethyl | |
| 1.2.1274 | 3-Cyclopropyl-4-ethyl-Phenyl | Fluormethyl | |
| 1.2.1275 | 3-Cyano-4-ethyl-Phenyl | Fluormethyl | |
| 1.2.1276 | 3-Trifluormethyl-4-ethyl-Phenyl | Fluormethyl | |
| 1.2.1277 | 3-Methoxy-4-ethyl-Phenyl | Fluormethyl | |
| 1.2.1278 | 3-Ethoxy-4-ethyl-Phenyl | Fluormethyl | |
| 1.2.1279 | 3-Trifluormethoxy-4-ethyl-Phenyl | Fluormethyl | |
| 1.2.1280 | 3-Nitro-4-ethyl-Phenyl | Fluormethyl | |
| 1.2.1281 | 3-Fluor-4-propyl-Phenyl | Fluormethyl | |
| 1.2.1282 | 3-Chlor-4-propyl-Phenyl | Fluormethyl | |
| 1.2.1283 | 3-Brom-4-propyl-Phenyl | Fluormethyl | |
| 1.2.1284 | 3-Methyl-4-propyl-Phenyl | Fluormethyl | |
| 1.2.1285 | 3-Cyclopropyl-4-propyl-Phenyl | Fluormethyl | |
| 1.2.1286 | 3-Cyano-4-propyl-Phenyl | Fluormethyl | |
| 1.2.1287 | 3-Trifluormethyl-4-propyl-Phenyl | Fluormethyl | |
| 1.2.1288 | 3-Methoxy-4-propyl-Phenyl | Fluormethyl | |
| 1.2.1289 | 3-Ethoxy-4-propyl-Phenyl | Fluormethyl | |
| 1.2.1290 | 3-Trifluormethoxy-4-propyl-Phenyl | Fluormethyl | |
| 1.2.1291 | 3-Nitro-4-propyl-Phenyl | Fluormethyl | |
| 1.2.1292 | 3-Fluor-4-isopropyl-Phenyl | Fluormethyl | |
| 1.2.1293 | 3-Chlor-4-isopropyl-Phenyl | Fluormethyl | |
| 1.2.1294 | 3-Brom-4-isopropyl-Phenyl | Fluormethyl | |
| 1.2.1295 | 3-Methyl-4-isopropyl-Phenyl | Fluormethyl | |
| 1.2.1296 | 3-Ethyl-4-isopropyl-Phenyl | Fluormethyl | |
| 1.2.1297 | 3-Cyclopropyl-4-isopropyl-Phenyl | Fluormethyl | |
| 1.2.1298 | 3-Cyano-4-isopropyl-Phenyl | Fluormethyl | |
| 1.2.1299 | 3-Trifluormethyl-4-isopropyl-Phenyl | Fluormethyl | |
| 1.2.1300 | 3-Methoxy-4-isopropyl-Phenyl | Fluormethyl | |
| 1.2.1301 | 3-Ethoxy-4-isopropyl-Phenyl | Fluormethyl | |
| 1.2.1302 | 3-Trifluormethoxy-4-isopropyl-Phenyl | Fluormethyl | |
| 1.2.1303 | 3-Nitro-4-isopropyl-Phenyl | Fluormethyl | |
| 1.2.1304 | 3-Fluor-4-tert.butyl-Phenyl | Fluormethyl | |
| 1.2.1305 | 3-Chlor-4-tert.butyl-Phenyl | Fluormethyl | |
| 1.2.1306 | 3-Brom-4-tert.butyl-Phenyl | Fluormethyl | |
| 1.2.1307 | 3-Methyl-4-tert.butyl-Phenyl | Fluormethyl | |
| 1.2.1308 | 3-Cyclopropyl-4-tert.butyl-Phenyl | Fluormethyl | |
| 1.2.1309 | 3-Cyano-4-tert.butyl-Phenyl | Fluormethyl | |
| 1.2.1310 | 3-Trifluormethyl-4-tert.butyl-Phenyl | Fluormethyl | |
| 1.2.1311 | 3-Trifluormethyl-4-tert.butyl-Phenyl | Chlormethyl | |
| 1.2.1312 | 3-Trifluormethyl-4-tert.butyl-Phenyl | Brommethyl | |
| 1.2.1313 | 3-Trifluormethyl-4-tert.butyl-Phenyl | Difluormethyl | |
| 1.2.1314 | 3-Methoxy-4-tert.butyl-Phenyl | Fluormethyl | |
| 1.2.1315 | 3-Ethoxy-4-tert.butyl-Phenyl | Fluormethyl | |
| 1.2.1316 | 3-Trifluormethoxy-4-tert.butyl-Phenyl | Fluormethyl | |
| 1.2.1317 | 3-Nitro-4-tert.butyl-Phenyl | Fluormethyl | |
| 1.2.1318 | 3-Fluor-4-cyclopropyl-Phenyl | Fluormethyl | |
| 1.2.1319 | 3-Chlor-4-cyclopropyl-Phenyl | Fluormethyl | |
| 1.2.1320 | 3-Brom-4-cyclopropyl-Phenyl | Fluormethyl | |
| 1.2.1321 | 3-Methyl-4-cyclopropyl-Phenyl | Fluormethyl | |
| 1.2.1322 | 3-Ethyl-4-cyclopropyl-Phenyl | Fluormethyl | |
| 1.2.1323 | 3-Cyclopropyl-4-cyclopropyl-Phenyl | Fluormethyl | |
| 1.2.1324 | 3-Cyano-4-cyclopropyl-Phenyl | Fluormethyl | |
| 1.2.1325 | 3-Trifluormethyl-4-cyclopropyl-Phenyl | Fluormethyl | |
| 1.2.1326 | 3-Methoxy-4-cyclopropyl-Phenyl | Fluormethyl | |
| 1.2.1327 | 3-Ethoxy-4-cyclopropyl-Phenyl | Fluormethyl | |
| 1.2.1328 | 3-Trifluormethoxy-4-cyclopropyl-Phenyl | Fluormethyl | |
| 1.2.1329 | 3-Fluor-4-methoxycarbonyl-Phenyl | Fluormethyl | |
| 1.2.1330 | 3-Chlor-4-methoxycarbonyl-Phenyl | Fluormethyl | |
| 1.2.1331 | 3-Brom-4-methoxycarbonyl-Phenyl | Fluormethyl | |
| 1.2.1332 | 3-Methyl-4-methoxycarbonyl-Phenyl | Fluormethyl | |
| 1.2.1333 | 3-Cyclopropyl-4-methoxycarbonyl-Phenyl | Fluormethyl | |
| 1.2.1334 | 3-Cyano-4-methoxycarbonyl-Phenyl | Fluormethyl | |
| 1.2.1335 | 3-Trifluormethyl-4-methoxycarbonyl-Phenyl | Fluormethyl | |
| 1.2.1336 | 3-Methoxy-4-methoxycarbonyl-Phenyl | Fluormethyl | |
| 1.2.1337 | 3-Ethoxy-4-methoxycarbonyl-Phenyl | Fluormethyl | |
| 1.2.1338 | 3-Trifluormethoxy-4-methoxycarbonyl-Phenyl | Fluormethyl | |
| 1.2.1339 | 3-Nitro-4-methoxycarbonyl-Phenyl | Fluormethyl | |
| 1.2.1340 | 3-Fluor-4-cyano-Phenyl | Fluormethyl | |
| 1.2.1341 | 3-Chlor-4-cyano-Phenyl | Fluormethyl | |
| 1.2.1342 | 3-Brom-4-cyano-Phenyl | Fluormethyl | |
| 1.2.1343 | 3-Methyl-4-cyano-Phenyl | Fluormethyl | |
| 1.2.1344 | 3-Cyclopropyl-4-cyano-Phenyl | Fluormethyl | |
| 1.2.1345 | 3,4-Dicyano-Phenyl | Fluormethyl | |
| 1.2.1346 | 3-Trifluormethyl-4-cyano-Phenyl | Fluormethyl | |
| 1.2.1347 | 3-Trifluormethyl-4-cyano-Phenyl | Chlormethyl | |
| 1.2.1348 | 3-Trifluormethyl-4-cyano-Phenyl | Brommethyl | |
| 1.2.1349 | 3-Trifluormethyl-4-cyano-Phenyl | Difluormethyl | |
| 1.2.1350 | 3-Methoxy-4-cyano-Phenyl | Fluormethyl | |
| 1.2.1351 | 3-Ethoxy-4-cyano-Phenyl | Fluormethyl | |
| 1.2.1352 | 3-Trifluormethoxy-4-cyano-Phenyl | Fluormethyl | |
| 1.2.1353 | 3-Nitro-4-cyano-Phenyl | Fluormethyl | |
| 1.2.1354 | 3-Fluor-4-methoxy-Phenyl | Fluormethyl | |
| 1.2.1355 | 3-Chlor-4-methoxy-Phenyl | Fluormethyl | |
| 1.2.1356 | 3-Brom-4-methoxy-Phenyl | Fluormethyl | |
| 1.2.1357 | 3-Methyl-4-m ethoxy-Phenyl | Fluormethyl | |
| 1.2.1358 | 3-Cyclopropyl-4-methoxy-Phenyl | Fluormethyl | |
| 1.2.1359 | 3-Cyano-4-methoxy-Phenyl | Fluormethyl | |
| 1.2.1360 | 3-Trifluormethyl-4-methoxy-Phenyl | Fluormethyl | |
| 1.2.1361 | 3,4-Dimethoxy-Phenyl | Fluormethyl | |
| 1.2.1362 | 3-Ethoxy-4-methoxy-Phenyl | Fluormethyl | |
| 1.2.1363 | 3-Trifluormethoxy-4-methoxy-Phenyl | Fluormethyl | |
| 1.2.1364 | 3-N itro-4-m ethoxy-Phenyl | Fluormethyl | |
| 1.2.1365 | 3-Fluor-4-ethoxy-Phenyl | Fluormethyl | |
| 1.2.1366 | 3-Chlor-4-ethoxy-Phenyl | Fluormethyl | |
| 1.2.1367 | 3-Chlor-4-ethoxy-Phenyl | Chlormethyl | |
| 1.2.1368 | 3-Chlor-4-ethoxy-Phenyl | Brommethyl | |
| 1.2.1369 | 3-Chlor-4-ethoxy-Phenyl | Difluormethyl | |
| 1.2.1370 | 3-Brom-4-ethoxy-Phenyl | Fluormethyl | |
| 1.2.1371 | 3-Methyl-4-ethoxy-Phenyl | Fluormethyl | |
| 1.2.1372 | 3-Ethyl-4-ethoxy-Phenyl | Fluormethyl | |
| 1.2.1373 | 3-Cyclopropyl-4-ethoxy-Phenyl | Fluormethyl | |
| 1.2.1374 | 3-Cyano-4-ethoxy-Phenyl | Fluormethyl | |
| 1.2.1375 | 3-Trifluormethyl-4-ethoxy-Phenyl | Fluormethyl | |
| 1.2.1376 | 3-Methoxy-4-ethoxy -Phenyl | Fluormethyl | |
| 1.2.1377 | 2,4-Diethoxy-Phenyl | Fluormethyl | |
| 1.2.1378 | 3-Trifluormethoxy-4-ethoxy-Phenyl | Fluormethyl | |
| 1.2.1379 | 3-Nitro-4-ethoxy-Phenyl | Fluormethyl | |
| 1.2.1380 | 3-Fluor-4-isopropoxy-Phenyl | Fluormethyl | |
| 1.2.1381 | 3-Chlor-4-isopropoxy-Phenyl | Fluormethyl | |
| 1.2.1382 | 3-Brom-4-isopropoxy-Phenyl | Fluormethyl | |
| 1.2.1383 | 3-Methyl-4-isopropoxy-Phenyl | Fluormethyl | |
| 1.2.1384 | 3-Cyclopropyl-4-isopropoxy-Phenyl | Fluormethyl | |
| 1.2.1385 | 3-Cyano-4-isopropoxy-Phenyl | Fluormethyl | |
| 1.2.1386 | 3-Trifluormethyl-4-isopropoxy-Phenyl | Fluormethyl | |
| 1.2.1387 | 3-Methoxy-4-isopropoxy-Phenyl | Fluormethyl | |
| 1.2.1388 | 3-Ethoxy-4-isopropoxy-Phenyl | Fluormethyl | |
| 1.2.1389 | 3-Trifluormethoxy-4-isopropoxy-Phenyl | Fluormethyl | |
| 1.2.1390 | 3-Nitro-4-isopropoxy-Phenyl | Fluormethyl | |
| 1.2.1391 | 3-Fluor-4-trifluormethoxy-Phenyl | Fluormethyl | |
| 1.2.1392 | 3-Chlor-4-trifluormethoxy-Phenyl | Fluormethyl | |
| 1.2.1393 | 3-Brom-4-trifluormethoxy-Phenyl | Fluormethyl | |
| 1.2.1394 | 3-Methyl-4-trifluormethoxy-Phenyl | Fluormethyl | |
| 1.2.1395 | 3-Cyclopropyl-4-trifluormethoxy-Phenyl | Fluormethyl | |
| 1.2.1396 | 3-Cyano-4-trifluormethoxy-Phenyl | Fluormethyl | |
| 1.2.1397 | 3-Trifluormethyl-4-trifluormethoxy-Phenyl | Fluormethyl | |
| 1.2.1398 | 3-Methoxy-4-trifluormethoxy-Phenyl | Fluormethyl | |
| 1.2.1399 | 3-Ethoxy-4-trifluormethoxy-Phenyl | Fluormethyl | |
| 1.2.1400 | 3,4-Bis(trifluormethoxy)-Phenyl | Fluormethyl | |
| 1.2.1401 | 3-Nitro-4-trifluormethoxy-Phenyl | Fluormethyl | |
| 1.2.1402 | 3-Fluor-4-difluormethoxy-Phenyl | Fluormethyl | |
| 1.2.1403 | 3-Chlor-4-difluormethoxy-Phenyl | Fluormethyl | |
| 1.2.1404 | 3-Brom-4-difluormethoxy-Phenyl | Fluormethyl | |
| 1.2.1405 | 3-Methyl-4-difluormethoxy-Phenyl | Fluormethyl | |
| 1.2.1406 | 3-Cyclopropyl-4-difluormethoxy-Phenyl | Fluormethyl | |
| 1.2.1407 | 3-Cyano-4-difluormethoxy-Phenyl | Fluormethyl | |
| 1.2.1408 | 3-Trifluormethyl-4-difluormethoxy-Phenyl | Fluormethyl | |
| 1.2.1409 | 3-Methoxy-4-difluormethoxy-Phenyl | Fluormethyl | |
| 1.2.1410 | 3-Ethoxy-4-difluormethoxy-Phenyl | Fluormethyl | |
| 1.2.1411 | 3-Trifluormethoxy-4-difluormethoxy-Phenyl | Fluormethyl | |
| 1.2.1412 | 3-Nitro-4-difluormethoxy-Phenyl | Fluormethyl | |
| 1.2.1413 | 3-Fluor-4-nitro-Phenyl | Fluormethyl | |
| 1.2.1414 | 3-Chlor-4-nitro-Phenyl | Fluormethyl | |
| 1.2.1415 | 3-Brom-4-nitro-Phenyl | Fluormethyl | |
| 1.2.1416 | 3-Methyl-4-nitro-Phenyl | Fluormethyl | |
| 1.2.1417 | 3-Ethyl-4-nitro-Phenyl | Fluormethyl | |
| 1.2.1418 | 3-Cyclopropyl-4-nitro-Phenyl | Fluormethyl | |
| 1.2.1419 | 3-Cyano-4-nitro-Phenyl | Fluormethyl | |
| 1.2.1420 | 3-Trifluormethyl-4-nitro-Phenyl | Fluormethyl | |
| 1.2.1421 | 3-Methoxy-4-nitro-Phenyl | Fluormethyl | |
| 1.2.1422 | 3-Ethoxy-4-nitro-Phenyl | Fluormethyl | |
| 1.2.1423 | 3-Trifluormethoxy-4-nitro-Phenyl | Fluormethyl | |
| 1.2.1424 | 3-Fluor-4-methylsulfanylPhenyl | Fluormethyl | |
| 1.2.1425 | 3-Chlor-4-methylsulfanyl-Phenyl | Fluormethyl | |
| 1.2.1426 | 3-Brom-4-methylsulfanyl-Phenyl | Fluormethyl | |
| 1.2.1427 | 3-Methyl-4-methylsulfanyl-Phenyl | Fluormethyl | |
| 1.2.1428 | 3-Ethyl-4-methylsulfanyl-Phenyl | Fluormethyl | |
| 1.2.1429 | 3-Cyclopropyl-4-methylsulfanyl-Phenyl | Fluormethyl | |
| 1.2.1430 | 3-Cyano-4-methylsulfanyl-Phenyl | Fluormethyl | |
| 1.2.1431 | 3-Trifluormethyl-4-methylsulfanyl-Phenyl | Fluormethyl | |
| 1.2.1432 | 3-Methoxy-4-methylsulfanyl-Phenyl | Fluormethyl | |
| 1.2.1433 | 3-Ethoxy-4-methylsulfanyl-Phenyl | Fluormethyl | |
| 1.2.1434 | 3-Trifluormethoxy-4-methylsulfanyl-Phenyl | Fluormethyl | |
| 1.2.1435 | 3-Nitro-4-methylsulfanyl-Phenyl | Fluormethyl | |
| 1.2.1436 | 3,6-Difluor-Phenyl | Fluormethyl | |
| 1.2.1437 | 3,6-Difluor-Phenyl | Chlormethyl | |
| 1.2.1438 | 3,6-Difluor-Phenyl | Brommethyl | |
| 1.2.1439 | 3,6-Difluor-Phenyl | Difluormethyl | |
| 1.2.1440 | 3,6-Difluor-Phenyl | Trifluormethyl | |
| 1.2.1441 | 3,6-Difluor-Phenyl | Cyano | |
| 1.2.1442 | 3-Chlor-6-fluor-Phenyl | Fluormethyl | |
| 1.2.1443 | 3-Brom-6-fluor-Phenyl | Fluormethyl | |
| 1.2.1444 | 3-Methyl-6-fluor-Phenyl | Fluormethyl | |
| 1.2.1445 | 3-Cyclopropyl-6-fluor-Phenyl | Fluormethyl | |
| 1.2.1446 | 3-Cyano-6-fluor-Phenyl | Fluormethyl | |
| 1.2.1447 | 3-Methoxy-6-fluor-Phenyl | Fluormethyl | |
| 1.2.1448 | 3-Ethoxy-6-fluor-Phenyl | Fluormethyl | |
| 1.2.1449 | 3-Trifluormethoxy-6-fluor-Phenyl | Fluormethyl | |
| 1.2.1450 | 3-Nitro-6-fluor-Phenyl | Fluormethyl | |
| 1.2.1451 | 3-Fluor-6-chlor-Phenyl | Fluormethyl | |
| 1.2.1452 | 3-Fluor-6-chlor-Phenyl | Chlormethyl | |
| 1.2.1453 | 3-Fluor-6-chlor-Phenyl | Brommethyl | |
| 1.2.1454 | 3-Fluor-6-chlor-Phenyl | Difluormethyl | |
| 1.2.1455 | 3,6-Dichlor-Phenyl | Fluormethyl | |
| 1.2.1456 | 3,6-Dichlor-Phenyl | Chlormethyl | |
| 1.2.1457 | 3,6-Dichlor-Phenyl | Brommethyl | |
| 1.2.1458 | 3,6-Dichlor-Phenyl | Difluormethyl | |
| 1.2.1459 | 3,6-Dichlor-Phenyl | Trifluormethyl | |
| 1.2.1460 | 3,6-Dichlor-Phenyl | Cyano | |
| 1.2.1461 | 3-Brom-6-chlor-Phenyl | Fluormethyl | |
| 1.2.1462 | 3-Methyl-6-chlor-Phenyl | Fluormethyl | |
| 1.2.1463 | 3-Cyclopropyl-6-chlor-Phenyl | Fluormethyl | |
| 1.2.1464 | 3-Cyano-6-chlor-Phenyl | Fluormethyl | |
| 1.2.1465 | 3-Trifluormethyl-6-chlor-Phenyl | Fluormethyl | |
| 1.2.1466 | 3-Methoxy-6-chlor-Phenyl | Fluormethyl | |
| 1.2.1467 | 3-Ethoxy-6-chlor-Phenyl | Fluormethyl | |
| 1.2.1468 | 3-Trifluormethoxy-6-chlor-Phenyl | Fluormethyl | |
| 1.2.1469 | 3-Nitro-6-chlor-Phenyl | Fluormethyl | |
| 1.2.1470 | 3-Fluor-6-brom-Phenyl | Fluormethyl | |
| 1.2.1471 | 3-Chlor-6-brom-Phenyl | Fluormethyl | |
| 1.2.1472 | 3,6-Dibrom-Phenyl | Fluormethyl | |
| 1.2.1473 | 3-Methyl-6-brom-Phenyl | Fluormethyl | |
| 1.2.1474 | 3-Cyclopropyl-6-brom-Phenyl | Fluormethyl | |
| 1.2.1475 | 3-Cyano-6-brom-Phenyl | Fluormethyl | |
| 1.2.1476 | 3-Trifluormethyl-6-brom-Phenyl | Fluormethyl | |
| 1.2.1477 | 3-Methoxy-6-Phenyl | Fluormethyl | |
| 1.2.1478 | 3-Ethoxy-6-brom-Phenyl | Fluormethyl | |
| 1.2.1479 | 3-Trifluormethoxy-6-brom-Phenyl | Fluormethyl | |
| 1.2.1480 | 3-Nitro-6-brom-Phenyl | Fluormethyl | |
| 1.2.1481 | 3-Fluor-6-iod-Phenyl | Fluormethyl | |
| 1.2.1482 | 3-Chlor-6-iod-Phenyl | Fluormethyl | |
| 1.2.1483 | 3-Brom-6-iod-Phenyl | Fluormethyl | |
| 1.2.1484 | 3-Methyl-6-iod-Phenyl | Fluormethyl | |
| 1.2.1485 | 3-Cyclopropyl-6-iod-Phenyl | Fluormethyl | |
| 1.2.1486 | 3-Cyano-6-iod-Phenyl | Fluormethyl | |
| 1.2.1487 | 3-Trifluormethyl-6-iod-Phenyl | Fluormethyl | |
| 1.2.1488 | 3-Methoxy-6-iod-Phenyl | Fluormethyl | |
| 1.2.1489 | 3-Ethoxy-6-iod-Phenyl | Fluormethyl | |
| 1.2.1490 | 3-Trifluormethoxy-6-iod-Phenyl | Fluormethyl | |
| 1.2.1491 | 3-Nitro-6-iod-Phenyl | Fluormethyl | |
| 1.2.1492 | 3-Fluor-6-methyl-Phenyl | Fluormethyl | |
| 1.2.1493 | 3-Chlor-6-methyl-Phenyl | Fluormethyl | |
| 1.2.1494 | 3-Brom-6-methyl-Phenyl | Fluormethyl | |
| 1.2.1495 | 3.6-Dimethyl-Phenyl | Fluormethyl | |
| 1.2.1496 | 3-Ethyl-6-methyl-Phenyl | Fluormethyl | |
| 1.2.1497 | 3-Cyclopropyl-6-methyl-Phenyl | Fluormethyl | |
| 1.2.1498 | 3-Cyano-6-methyl-Phenyl | Fluormethyl | |
| 1.2.1499 | 3-Trifluormethyl-6-methyl-Phenyl | Fluormethyl | |
| 1.2.1500 | 3-Methoxy-6-methyl-Phenyl | Fluormethyl | |
| 1.2.1501 | 3-Ethoxy-6-methyl-Phenyl | Fluormethyl | |
| 1.2.1502 | 3-Trifluormethoxy-6-methyl-Phenyl | Fluormethyl | |
| 1.2.1503 | 3-Nitro-6-methyl-Phenyl | Fluormethyl | |
| 1.2.1504 | 3-Fluor-6-ethyl-Phenyl | Fluormethyl | |
| 1.2.1505 | 3-Chlor-6-ethyl-Phenyl | Fluormethyl | |
| 1.2.1506 | 3-Brom-6-ethyl-Phenyl | Fluormethyl | |
| 1.2.1507 | 3-Methyl-6-ethyl-Phenyl | Fluormethyl | |
| 1.2.1508 | 3,6-Diethyl-Phenyl | Fluormethyl | |
| 1.2.1509 | 3-Cyclopropyl-6-ethyl-Phenyl | Fluormethyl | |
| 1.2.1510 | 3-Cyano-6-ethyl-Phenyl | Fluormethyl | |
| 1.2.1511 | 3-Trifluormethyl-6-ethyl-Phenyl | Fluormethyl | |
| 1.2.1512 | 3-Methoxy-6-ethyl-Phenyl | Fluormethyl | |
| 1.2.1513 | 3-Ethoxy-6-ethyl-Phenyl | Fluormethyl | |
| 1.2.1514 | 3-Trifluormethoxy-6-ethyl-Phenyl | Fluormethyl | |
| 1.2.1515 | 3-Nitro-6-ethyl-Phenyl | Fluormethyl | |
| 1.2.1516 | 3-Fluor-6-propyl-Phenyl | Fluormethyl | |
| 1.2.1517 | 3-Chlor-6-propyl-Phenyl | Fluormethyl | |
| 1.2.1518 | 3-Brom-6-propyl-Phenyl | Fluormethyl | |
| 1.2.1519 | 3-Methyl-6-propyl-Phenyl | Fluormethyl | |
| 1.2.1520 | 3-Cyclopropyl-6-propyl-Phenyl | Fluormethyl | |
| 1.2.1521 | 3-Cyano-6-propyl-Phenyl | Fluormethyl | |
| 1.2.1522 | 3-Trifluormethyl-6-propyl-Phenyl | Fluormethyl | |
| 1.2.1523 | 3-Methoxy-6-propyl-Phenyl | Fluormethyl | |
| 1.2.1524 | 3-Ethoxy-6-propyl-Phenyl | Fluormethyl | |
| 1.2.1525 | 3-Trifluormethoxy-6-propyl-Phenyl | Fluormethyl | |
| 1.2.1526 | 3-Nitro-6-propyl-Phenyl | Fluormethyl | |
| 1.2.1527 | 3-Fluor-6-isopropyl-Phenyl | Fluormethyl | |
| 1.2.1528 | 3-Chlor-6-isopropyl-Phenyl | Fluormethyl | |
| 1.2.1529 | 3-Brom-6-isopropyl-Phenyl | Fluormethyl | |
| 1.2.1530 | 3-Methyl-6-isopropyl-Phenyl | Fluormethyl | |
| 1.2.1531 | 3-Cyclopropyl-6-isopropyl-Phenyl | Fluormethyl | |
| 1.2.1532 | 3-Cyano-6-isopropyl-Phenyl | Fluormethyl | |
| 1.2.1533 | 3-Trifluormethyl-6-isopropyl-Phenyl | Fluormethyl | |
| 1.2.1534 | 3-Methoxy-6-isopropyl-Phenyl | Fluormethyl | |
| 1.2.1535 | 3-Trifluormethoxy-6-isopropyl-Phenyl | Fluormethyl | |
| 1.2.1536 | 3-Nitro-6-isopropyl-Phenyl | Fluormethyl | |
| 1.2.1537 | 3-Fluor-6-tert.butyl-Phenyl | Fluormethyl | |
| 1.2.1538 | 3-Chlor-6-tert.buty-Phenyl | Fluormethyl | |
| 1.2.1539 | 3-Brom-6-tert.butyl-Phenyl | Fluormethyl | |
| 1.2.1540 | 3-Methyl-6-tert.butyl-Phenyl | Fluormethyl | |
| 1.2.1541 | 3-Cyclopropyl-6-tert.butyl-Phenyl | Fluormethyl | |
| 1.2.1542 | 3-Cyano-6-tert.butyl-Phenyl | Fluormethyl | |
| 1.2.1543 | 3-Trifluormethyl-6-tert.butyl-Phenyl | Fluormethyl | |
| 1.2.1544 | 3-Methoxy-6-tert.butyl-Phenyl | Fluormethyl | |
| 1.2.1545 | 3-Ethoxy-6-tert.butyl-Phenyl | Fluormethyl | |
| 1.2.1546 | 3-Trifluormethoxy-6-tert.butyl-Phenyl | Fluormethyl | |
| 1.2.1547 | 3-Nitro-6-tert.butyl-Phenyl | Fluormethyl | |
| 1.2.1548 | 3-Fluor-6-cyclopropyl-Phenyl | Fluormethyl | |
| 1.2.1549 | 3-Chlor-6-cyclopropyl-Phenyl | Fluormethyl | |
| 1.2.1550 | 3-Brom-6-cyclopropyl-Phenyl | Fluormethyl | |
| 1.2.1551 | 3-Methyl-6-cyclopropyl-Phenyl | Fluormethyl | |
| 1.2.1552 | 3-Cyclopropyl-6-cyclopropyl-Phenyl | Fluormethyl | |
| 1.2.1553 | 3-Cyano-6-cyclopropyl-Phenyl | Fluormethyl | |
| 1.2.1554 | 3-Trifluormethyl-6-cyclopropyl-Phenyl | Fluormethyl | |
| 1.2.1555 | 3-Methoxy-6-cyclopropyl-Phenyl | Fluormethyl | |
| 1.2.1556 | 3-Ethoxy-6-cyclopropyl-Phenyl | Fluormethyl | |
| 1.2.1557 | 3-Trifluormethoxy-6-cyclopropyl-Phenyl | Fluormethyl | |
| 1.2.1558 | 3-Fluor-6-methoxycarbonyl-Phenyl | Fluormethyl | |
| 1.2.1559 | 3-Chlor-6-methoxycarbonyl-Phenyl | Fluormethyl | |
| 1.2.1560 | 3-Brom-6-methoxycarbonyl-Phenyl | Fluormethyl | |
| 1.2.1561 | 3-Methyl-6-methoxycarbonyl-Phenyl | Fluormethyl | |
| 1.2.1562 | 3-Cyclopropyl-6-methoxycarbonyl-Phenyl | Fluormethyl | |
| 1.2.1563 | 3-Cyano-6-methoxycarbonyl-Phenyl | Fluormethyl | |
| 1.2.1564 | 3-Trifluormethyl-6-methoxycarbonyl-Phenyl | Fluormethyl | |
| 1.2.1565 | 3-Methoxy-6-methoxycarbonyl-Phenyl | Fluormethyl | |
| 1.2.1566 | 3-Ethoxy-6-methoxycarbonyl-Phenyl | Fluormethyl | |
| 1.2.1567 | 3-Trifluormethoxy-6-methoxycarbonyl-Phenyl | Fluormethyl | |
| 1.2.1568 | 3-Nitro-6-methoxycarbonyl-Phenyl | Fluormethyl | |
| 1.2.1569 | 3-Fluor-6-cyano-Phenyl | Fluormethyl | |
| 1.2.1570 | 3-Chlor-6-cyano-Phenyl | Fluormethyl | |
| 1.2.1571 | 3-Brom-6-cyano-Phenyl | Fluormethyl | |
| 1.2.1572 | 3-Methyl-6-cyano-Phenyl | Fluormethyl | |
| 1.2.1573 | 3-Cyclopropyl-6-cyano-Phenyl | Fluormethyl | |
| 1.2.1574 | 3-Cyano-6-cyanoPhenyl | Fluormethyl | |
| 1.2.1575 | 3-Trifluormethyl-6-cyano-Phenyl | Fluormethyl | |
| 1.2.1576 | 3-Methoxy-6-cyano-Phenyl | Fluormethyl | |
| 1.2.1577 | 3-Ethoxy-6-cyano-Phenyl | Fluormethyl | |
| 1.2.1578 | 3-Trifluormethoxy-6-cyano-Phenyl | Fluormethyl | |
| 1.2.1579 | 3-Nitro-6-cyano-Phenyl | Fluormethyl | |
| 1.2.1580 | 3-Fluor-6-methoxy-Phenyl | Fluormethyl | |
| 1.2.1581 | 3-Chlor-6-methoxy-Phenyl | Fluormethyl | |
| 1.2.1582 | 3-Brom-6-methoxy-Phenyl | Fluormethyl | |
| 1.2.1583 | 3-Methyl-6-methoxy-Phenyl | Fluormethyl | |
| 1.2.1584 | 3-Cyclopropyl-6-methoxy-Phenyl | Fluormethyl | |
| 1.2.1585 | 3-Cyano-6-methoxy-Phenyl | Fluormethyl | |
| 1.2.1586 | 3-Trifluormethyl-6-methoxy-Phenyl | Fluormethyl | |
| 1.2.1587 | 3,6-Dimethoxy-Phenyl | Fluormethyl | |
| 1.2.1588 | 3-Ethoxy-6-methoxy-Phenyl | Fluormethyl | |
| 1.2.1589 | 3-Trifluormethoxy-6-methoxy-Phenyl | Fluormethyl | |
| 1.2.1590 | 3-Nitro-6-methoxy-Phenyl | Fluormethyl | |
| 1.2.1591 | 3-Fluor-6-ethoxy-Phenyl | Fluormethyl | |
| 1.2.1592 | 3-Chlor-6-ethoxy-Phenyl | Fluormethyl | |
| 1.2.1593 | 3-Brom-6-ethoxy-Phenyl | Fluormethyl | |
| 1.2.1594 | 3-Methyl-6-ethoxy-Phenyl | Fluormethyl | |
| 1.2.1595 | 3-Ethyl-6-ethoxy-Phenyl | Fluormethyl | |
| 1.2.1596 | 3-Cyclopropyl-6-ethoxy-Phenyl | Fluormethyl | |
| 1.2.1597 | 3-Cyano-6-ethoxy-Phenyl | Fluormethyl | |
| 1.2.1598 | 3-Trifluormethyl-6-ethoxy-Phenyl | Fluormethyl | |
| 1.2.1599 | 3-Methoxy-6-ethoxy-Phenyl | Fluormethyl | |
| 1.2.1600 | 2,6-Diethoxy-Phenyl | Fluormethyl | |
| 1.2.1601 | 3-Trifluormethoxy-6-ethoxy-Phenyl | Fluormethyl | |
| 1.2.1602 | 3-Nitro-6-ethoxy-Phenyl | Fluormethyl | |
| 1.2.1603 | 3-Fluor-6-isopropoxy-Phenyl | Fluormethyl | |
| 1.2.1604 | 3-Chlor-6-isopropoxy-Phenyl | Fluormethyl | |
| 1.2.1605 | 3-Brom-6-isopropoxy-Phenyl | Fluormethyl | |
| 1.2.1606 | 3-Methyl-6-isopropoxy-Phenyl | Fluormethyl | |
| 1.2.1607 | 3-Cyclopropyl-6-isopropoxy-Phenyl | Fluormethyl | |
| 1.2.1608 | 3-Cyano-6-isopropoxy-Phenyl | Fluormethyl | |
| 1.2.1609 | 3-Trifluormethyl-6-isopropoxy-Phenyl | Fluormethyl | |
| 1.2.1610 | 3-Methoxy-6-isopropoxy-Phenyl | Fluormethyl | |
| 1.2.1611 | 3-Ethoxy-6-isopropoxy-Phenyl | Fluormethyl | |
| 1.2.1612 | 3-Trifluormethoxy-6-isopropoxy-Phenyl | Fluormethyl | |
| 1.2.1613 | 3-Nitro-6-isopropoxy-Phenyl | Fluormethyl | |
| 1.2.1614 | 3-Fluor-6-trifluormethoxy-Phenyl | Fluormethyl | |
| 1.2.1615 | 3-Chlor-6-trifluormethoxy-Phenyl | Fluormethyl | |
| 1.2.1616 | 3-Brom-6-trifluormethoxy-Phenyl | Fluormethyl | |
| 1.2.1617 | 3-Methyl-6-trifluormethoxy-Phenyl | Fluormethyl | |
| 1.2.1618 | 3-Cyclopropyl-6-trifluormethoxy-Phenyl | Fluormethyl | |
| 1.2.1619 | 3-Cyano-6-trifluormethoxy-Phenyl | Fluormethyl | |
| 1.2.1620 | 3-Trifluormethyl-6-trifluormethoxy-Phenyl | Fluormethyl | |
| 1.2.1621 | 3-Methoxy-6-trifluormethoxy-Phenyl | Fluormethyl | |
| 1.2.1622 | 3-Ethoxy-6-trifluormethoxy-Phenyl | Fluormethyl | |
| 1.2.1623 | 3,6-Bis(trifluormethoxy)-Phenyl | Fluormethyl | |
| 1.2.1624 | 3-Nitro-6-trifluormethoxy-Phenyl | Fluormethyl | |
| 1.2.1625 | 3-Fluor-6-difluormethoxy-Phenyl | Fluormethyl | |
| 1.2.1626 | 3-Chlor-6-difluormethoxy-Phenyl | Fluormethyl | |
| 1.2.1627 | 3-Brom-6-difluormethoxy-Phenyl | Fluormethyl | |
| 1.2.1628 | 3-Methyl-6-difluormethoxy-Phenyl | Fluormethyl | |
| 1.2.1629 | 3-Cyclopropyl-6-difluormethoxy-Phenyl | Fluormethyl | |
| 1.2.1630 | 3-Cyano-6-difluormethoxy-Phenyl | Fluormethyl | |
| 1.2.1631 | 3-Trifluormethyl-6-difluormethoxy-Phenyl | Fluormethyl | |
| 1.2.1632 | 3-Methoxy-6-difluormethoxy-Phenyl | Fluormethyl | |
| 1.2.1633 | 3-Ethoxy-6-difluormethoxy-Phenyl | Fluormethyl | |
| 1.2.1634 | 3-Trifluormethoxy-6-difluormethoxy-Phenyl | Fluormethyl | |
| 1.2.1635 | 3-Nitro-6-difluormethoxy-Phenyl | Fluormethyl | |
| 1.2.1636 | 3-Fluor-6-nitro-Phenyl | Fluormethyl | |
| 1.2.1637 | 3-Chlor-6-nitro-Phenyl | Fluormethyl | |
| 1.2.1638 | 3-Brom-6-nitro-Phenyl | Fluormethyl | |
| 1.2.1639 | 3-Methyl-6-nitro-Phenyl | Fluormethyl | |
| 1.2.1640 | 3-Cyclopropyl-6-nitro-Phenyl | Fluormethyl | |
| 1.2.1641 | 3-Cyano-6-nitro-Phenyl | Fluormethyl | |
| 1.2.1642 | 3-Trifluormethyl-6-nitro-Phenyl | Fluormethyl | |
| 1.2.1643 | 3-Methoxy-6-nitro-Phenyl | Fluormethyl | |
| 1.2.1644 | 3-Ethoxy-6-nitro-Phenyl | Fluormethyl | |
| 1.2.1645 | 3-Trifluormethoxy-6-nitro-Phenyl | Fluormethyl | |
| 1.2.1646 | 3-Fluor-6-methylsulfanylPhenyl | Fluormethyl | |
| 1.2.1647 | 3-Chlor-6-methylsulfanyl-Phenyl | Fluormethyl | |
| 1.2.1648 | 3-Brom-6-methylsulfanyl-Phenyl | Fluormethyl | |
| 1.2.1649 | 3-Methyl-6-methylsulfanyl-Phenyl | Fluormethyl | |
| 1.2.1650 | 3-Cyclopropyl-6-methylsulfanyl-Phenyl | Fluormethyl | |
| 1.2.1651 | 3-Cyano-6-methylsulfanyl-Phenyl | Fluormethyl | |
| 1.2.1652 | 3-Trifluormethyl-6-methylsulfanyl-Phenyl | Fluormethyl | |
| 1.2.1653 | 3-Methoxy-6-methylsulfanyl-Phenyl | Fluormethyl | |
| 1.2.1654 | 3-Ethoxy-6-methylsulfanyl-Phenyl | Fluormethyl | |
| 1.2.1655 | 3-Trifluormethoxy-6-methylsulfanyl-Phenyl | Fluormethyl | |
| 1.2.1656 | 3-Nitro-6-methylsulfanyl-Phenyl | Fluormethyl | |
| 1.2.1657 | 2,3,4-Trifluor-Phenyl | Fluormethyl | |
| 1.2.1658 | 2,3,4-Trichlor-Phenyl | Fluormethyl | |
| 1.2.1659 | 2,3.4-Trimethyl-Phenyl | Fluormethyl | |
| 1.2.1660 | 2-Fluor-2-chlor-5-trifluormethyl-Phenyl | Fluormethyl | |
| 1.2.1661 | 2,3,5-Trifluor-Phenyl | Fluormethyl | |
| 1.2.1662 | 2,3,5-Trichlor-Phenyl | Fluormethyl | |
| 1.2.1663 | 2,3,5-Trimethyl-Phenyl | Fluormethyl | |
| 1.2.1664 | 2,3-Dichlor-5-methoxy-Phenyl | Fluormethyl | |
| 1.2.1665 | 2,3,6-Trifluor-Phenyl | Fluormethyl | |
| 1.2.1666 | 2,3,6-Trichlor-Phenyl | Fluormethyl | |
| 1.2.1667 | 2,3,6-Trimethyl-Phenyl | Fluormethyl | |
| 1.2.1668 | 3,4,5-Trifluor-Phenyl | Fluormethyl | |
| 1.2.1669 | 3,4,5-Trichlor-Phenyl | Fluormethyl | |
| 1.2.1670 | 3,4,5-Trimethyl-Phenyl | Fluormethyl | |
| 1.2.1671 | 3,5-Dimethyl-4-fluor-Phenyl | Fluormethyl | |
| 1.2.1672 | 3,5-Dichlor-4-methoxy-Phenyl | Fluormethyl | |
| 1.2.1673 | 3,5-Difluor-4-chlor-Phenyl | Fluormethyl | |
| 1.2.1674 | 3,5-Dichlor-4-hydroxy-Phenyl | Fluormethyl | |
| 1.2.1675 | 3,5-Trifluormethyl-4-chlor-Phenyl | Fluormethyl | |
| 1.2.1676 | 3,4,6-Trifluor-Phenyl | Fluormethyl | |
| 1.2.1677 | 3,4,6-Trichlor-Phenyl | Fluormethyl | |
| 1.2.1678 | 3,4,6-Trimethyl-Phenyl | Fluormethyl | |
| 1.2.1679 | Pentafluorphenyl | Fluormethyl | |

**Tabelle 1.3: Erfindungsgemäße Verbindungen der allgemeinen Formel (I), worin W* für COOH, R¹ und R² für Wasserstoff stehen, und Aryl den Rest**

| | | | |
|---|---|---|---|
| | 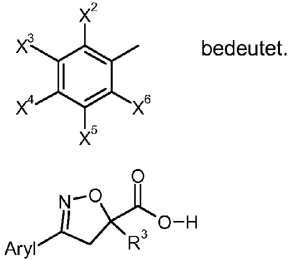 | | |

| Nr. | Aryl | R³ | Physikalische Daten |
|---|---|---|---|
| 1.3.001 | 3-Fluor-Phenyl | Vinyl | [CDCl₃] 3.44 (d,1H); 3.96 (d,1H); 5.44 (d,1H); 5.63 (d,1H); 6.16 (dd,1H); 7.10-7.20 (m,1H); 7.37-7.45 (m,2H). |
| 1.3.002 | 3-Fluor-Phenyl | 1-Methyl vinyl | |
| 1.3.003 | 3-Fluor-Phenyl | Allyl | |
| 1.3.004 | 3-Fluor-Phenyl | 1-Chlor vinyl | [CDCl₃] 3.66 (d,1H); 4.16 (d,1H); 5.61 (s,1H); 5.95 (s,1H); 7.13-7.20 (m,1H); 7.39-7.45 (m,2H). |
| 1.3.005 | 3-Fluor-Phenyl | Ethinyl | |
| 1.3.006 | 3-Chlor-Phenyl | Vinyl | |
| 1.3.007 | 3-Chlor-Phenyl | 1-Methyl vinyl | |
| 1.3.008 | 3-Chlor-Phenyl | Allyl | |
| 1.3.009 | 3-Chlor-Phenyl | 1-Chlor vinyl | |
| 1.3.010 | 3-Chlor-Phenyl | Ethinyl | |
| 1.3.011 | 3-Brom-Phenyl | Vinyl | |
| 1.3.012 | 3-Brom-Phenyl | 1-Methyl vinyl | |
| 1.3.013 | 3-Iod-Phenyl | Vinyl | |
| 1.3.014 | 3-Iod-Phenyl | 1-Methyl vinyl | |
| 1.3.015 | 3-Methyl-Phenyl | Vinyl | |
| 1.3.016 | 3-Methyl-Phenyl | 1-Methyl vinyl | |
| 1.3.017 | 3-Ethyl-Phenyl | Vinyl | |
| 1.3.018 | 3-Propyl-Phenyl | Vinyl | |
| 1.3.019 | 3-isoPropyl-Phenyl | Vinyl | |
| 1.3.020 | 3-nButyl-Phenyl | Vinyl | |
| 1.3.021 | 3-iButyl-Phenyl | Vinyl | |
| 1.3.022 | 3-tert.Butyl-Phenyl | Vinyl | |
| 1.3.023 | 3-Cyclopropyl-Phenyl | Vinyl | |
| 1.3.024 | 3-Cyclobutyl-Phenyl | Vinyl | |
| 1.3.025 | 3-Cyclopentyl-Phenyl | Vinyl | |
| 1.3.026 | 3-Vinyl-Phenyl | Vinyl | |
| 1.3.027 | 3-Ethinyl-Phenyl | Vinyl | |
| 1.3.028 | 3-Cyano-Phenyl | Vinyl | |
| 1.3.029 | 3-Trifluormethyl-Phenyl | Vinyl | |
| 1.3.030 | 3-Difluormethyl-Phenyl | Vinyl | |
| 1.3.031 | 3-(Hydroxycarbonyl)-Phenyl | Vinyl | |
| 1.3.032 | 3-(Methoxycarbony)l-Phenyl | Vinyl | |
| 1.3.033 | 3-(Ethoxycarbonyl)-Phenyl | Vinyl | |
| 1.3.034 | 3-Hydroxymethyl-Phenyl | Vinyl | |
| 1.3.035 | 3-Carbamoyl-Phenyl | Vinyl | |
| 1.3.036 | 3-Hydroxy-Phenyl- | Vinyl | |
| 1.3.037 | 3-Methoxy-Phenyl | Vinyl | |
| 1.3.038 | 3-Ethoxy-Phenyl | Vinyl | |
| 1.3.039 | 3-Propyloxy-Phenyl | Vinyl | |
| 1.3.040 | 3-isoPropyloxy-Phenyl | Vinyl | |
| 1.3.041 | 3-nButyloxy-Phenyl | Vinyl | |
| 1.3.042 | 3-iButyloxy-Phenyl | Vinyl | |
| 1.3.043 | 3-tButyloxy-Phenyl | Vinyl | |
| 1.3.044 | 3-Difluormethoxy-Phenyl | Vinyl | |
| 1.3.045 | 3-Trifluormethoxy-Phenyl | Vinyl | |
| 1.3.046 | 3-(2,2,2-Trifluorethoxy)-Phenyl | Vinyl | |
| 1.3.047 | 3-(2-Chlorethoxy)-Phenyl | Vinyl | |
| 1.3.048 | 3-(2-Hydroxyethoxy)-Phenyl- | Vinyl | |
| 1.3.049 | 3-(2-Methoxyethoxy)-Phenyl- | Vinyl | |
| 1.3.050 | 3-[(tert-Butoxycarbonyl)oxy]-Phenyl | Vinyl | |
| 1.3.051 | 3-Nitro-Phenyl | Vinyl | |
| 1.3.052 | 3-Acetoxy-Phenyl | Vinyl | |
| 1.3.053 | {3-[(tert-Butoxycarbonyl)amino]-Phenyl}- | Vinyl | |
| 1.3.054 | 3-Methylsulfanyl-Phenyl | Vinyl | |
| 1.3.055 | 3-Ethylsulfanyl-Phenyl | Vinyl | |
| 1.3.056 | 3-(Pentafluor-lambda⁶-sulfanyl)-Phenyl | Vinyl | |
| 1.3.057 | 2,3-Difluor-Phenyl | Vinyl | |
| 1.3.058 | 2,3-Difluor-Phenyl | 1-Methyl vinyl | |
| 1.3.059 | 2,3-Difluor-Phenyl | Allyl | |
| 1.3.060 | 2,3-Difluor-Phenyl | 1-Chlor vinyl | |
| 1.3.061 | 2,3-Difluor-Phenyl | Ethinyl | |
| 1.3.062 | 2-Chlor-3-fluor-Phenyl | Vinyl | |
| 1.3.063 | 2-Brom-3-fluor-Phenyl | Vinyl | |
| 1.3.064 | 2-Methyl-3-fluor-Phenyl | Vinyl | |
| 1.3.065 | 2-Ethyl-3-fluor-Phenyl | Vinyl | |
| 1.3.066 | 2-Cyclopropyl-3-fluor-Phenyl | Vinyl | |
| 1.3.067 | 2-Vinyl-3-fluor-Phenyl | Vinyl | |
| 1.3.068 | 2-Ethinyl-3-fluor-Phenyl | Vinyl | |
| 1.3.069 | 2-Cyano-3-fluor-Phenyl | Vinyl | |
| 1.3.070 | 2-Methoxy-3-fluor-Phenyl | Vinyl | |
| 1.3.071 | 2-Ethoxy-3-fluor-Phenyl | Vinyl | |
| 1.3.072 | 2-Trifluormethoxy-3-fluor-Phenyl | Vinyl | |
| 1.3.073 | 2-Nitro-3-fluor-Phenyl | Vinyl | |
| 1.3.074 | 2-Fluor-3-chlor-Phenyl | Vinyl | |
| 1.3.075 | 2,3-Dichlor-Phenyl | Vinyl | |
| 1.3.076 | 2,3-Dichlor-Phenyl | 1-Methyl vinyl | |
| 1.3.077 | 2,3-Dichlor-Phenyl | Allyl | |
| 1.3.078 | 2,3-Dichlor-Phenyl | 1-Chlor vinyl | |
| 1.3.079 | 2,3-Dichlor-Phenyl | Ethinyl | |
| 1.3.080 | 2-Brom-3-chlor-Phenyl | Vinyl | |
| 1.3.081 | 2-Methyl-3-chlor-Phenyl | Vinyl | |
| 1.3.082 | 2-Ethyl-3-chlor-Phenyl | Vinyl | |
| 1.3.083 | 2-Cyclopropyl-3-chlor-Phenyl | Vinyl | |
| 1.3.084 | 2-Vinyl-3-chlor-Phenyl | Vinyl | |
| 1.3.085 | 2-Ethinyl-3-chlor-Phenyl | Vinyl | |
| 1.3.086 | 2-Cyano-3-chlor-Phenyl | Vinyl | |
| 1.3.087 | 2-Trifluormethyl-2-chlor-Phenyl | Vinyl | |
| 1.3.088 | 2-Methoxy-3-chlor-Phenyl | Vinyl | |
| 1.3.089 | 2-Ethoxy-3-chlor-Phenyl | Vinyl | |
| 1.3.090 | 2-Trifluormethoxy-3-chlor-Phenyl | Vinyl | |
| 1.3.091 | 2-Nitro-3-chlor-Phenyl | Vinyl | |
| 1.3.092 | 2-Fluor-3-brom-Phenyl | Vinyl | |
| 1.3.093 | 2-Chlor-3-brom-Phenyl | Vinyl | |
| 1.3.094 | 2,3-Dibrom-Phenyl | Vinyl | |
| 1.3.095 | 2-Methyl-3-brom-Phenyl | Vinyl | |
| 1.3.096 | 2-Ethyl-3-brom-Phenyl | Vinyl | |
| 1.3.097 | 2-Cyclopropyl-3-brom-Phenyl | Vinyl | |
| 1.3.098 | 2-Vinyl-3-brom-Phenyl | Vinyl | |
| 1.3.099 | 2-Ethinyl-3-brom-Phenyl | Vinyl | |
| 1.3.100 | 2-Cyano-3-brom-Phenyl | Vinyl | |
| 1.3.101 | 2-Trifluormethyl-3-brom-Phenyl | Vinyl | |
| 1.3.102 | 2-Methoxy-3-Phenyl | Vinyl | |
| 1.3.103 | 2-Ethoxy-3-brom-Phenyl | Vinyl | |
| 1.3.104 | 2-Trifluormethoxy-3-brom-Phenyl | Vinyl | |
| 1.3.105 | 2-Nitro-3-brom-Phenyl | Vinyl | |
| 1.3.106 | 2-Fluor-3-iod-Phenyl | Vinyl | |
| 1.3.107 | 2-Chlor-3-iod-Phenyl | Vinyl | |
| 1.3.108 | 2-Brom-3-iod-Phenyl | Vinyl | |
| 1.3.109 | 2-Methyl-3-iod-Phenyl | Vinyl | |
| 1.3.110 | 2-Ethyl-3-iod-Phenyl | Vinyl | |
| 1.3.111 | 2-Cyclopropyl-3-iod-Phenyl | Vinyl | |
| 1.3.112 | 2-Vinyl-3-iod-Phenyl | Vinyl | |
| 1.3.113 | 2-Ethinyl-3-iod-Phenyl | Vinyl | |
| 1.3.114 | 2-Cyano-3-iod-Phenyl | Vinyl | |
| 1.3.115 | 2-Trifluormethyl-3-iod-Phenyl | Vinyl | |
| 1.3.116 | 2-Methoxy-3-iod-Phenyl | Vinyl | |
| 1.3.117 | 2-Ethoxy-3-iod-Phenyl | Vinyl | |
| 1.3.118 | 2-Trifluormethoxy-3-iod-Phenyl | Vinyl | |
| 1.3.119 | 2-Nitro-3-iod-Phenyl | Vinyl | |
| 1.3.120 | 2-Fluor-3-methyl-Phenyl | Vinyl | |
| 1.3.121 | 2-Fluor-3-methyl-Phenyl | 1-Methyl vinyl | |
| 1.3.122 | 2-Fluor-3-methyl-Phenyl | Allyl | |
| 1.3.123 | 2-Fluor-3-methyl-Phenyl | 1-Chlor vinyl | |
| 1.3.124 | 2-Fluor-3-methyl-Phenyl | Ethinyl | |
| 1.3.125 | 2-Chlor-3-methyl-Phenyl | Vinyl | |
| 1.3.126 | 2-Chlor-3-methyl-Phenyl | 1-Methyl vinyl | |
| 1.3.127 | 2-Chlor-3-methyl-Phenyl | Allyl | |
| 1.3.128 | 2-Chlor-3-methyl-Phenyl | 1-Chlor vinyl | |
| 1.3.129 | 2-Chlor-3-methyl-Phenyl | Ethinyl | |
| 1.3.130 | 2-Brom-3-methyl-Phenyl | Vinyl | |
| 1.3.131 | 2,3-Dimethyl-Phenyl | Vinyl | |
| 1.3.132 | 2,3-Dimethyl-Phenyl | 1-Methyl vinyl | |
| 1.3.133 | 2,3-Dimethyl-Phenyl | Allyl | |
| 1.3.134 | 2,3-Dimethyl-Phenyl | 1-Chlor vinyl | |
| 1.3.135 | 2,3-Dimethyl-Phenyl | Ethinyl | |
| 1.3.136 | 2-Ethyl-3-methyl-Phenyl | Vinyl | |
| 1.3.137 | 2-Cyclopropyl-3-methyl-Phenyl | Vinyl | |
| 1.3.138 | 2-Vinyl-3-methyl-Phenyl | Vinyl | |
| 1.3.139 | 2-Ethinyl-3-methyl-Phenyl | Vinyl | |
| 1.3.140 | 2-Cyano-3-methyl-Phenyl | Vinyl | |
| 1.3.141 | 2-Trifluormethyl-3-methyl-Phenyl | Vinyl | |
| 1.3.142 | 2-Methoxy-3-methyl-Phenyl | Vinyl | |
| 1.3.143 | 2-Ethoxy-3-methyl-Phenyl | Vinyl | |
| 1.3.144 | 2-Trifluormethoxy-3-methyl-Phenyl | Vinyl | |
| 1.3.145 | 2-Nitro-3-methyl-Phenyl | Vinyl | |
| 1.3.146 | 2-Fluor-3-ethyl-Phenyl | Vinyl | |
| 1.3.147 | 2-Chlor-3-ethyl-Phenyl | Vinyl | |
| 1.3.148 | 2-Brom-3-ethyl-Phenyl | Vinyl | |
| 1.3.149 | 2-Methyl-3-ethyl-Phenyl | Vinyl | |
| 1.3.150 | 2,3-Diethyl-Phenyl | Vinyl | |
| 1.3.151 | 2-Cyclopropyl-3-ethyl-Phenyl | Vinyl | |
| 1.3.152 | 2-Vinyl-3-ethyl-Phenyl | Vinyl | |
| 1.3.153 | 2-Ethinyl-3-ethyl-Phenyl | Vinyl | |
| 1.3.154 | 2-Cyano-3-ethyl-Phenyl | Vinyl | |
| 1.3.155 | 2-Trifluormethyl-3-ethyl-Phenyl | Vinyl | |
| 1.3.156 | 2-Methoxy-3-ethyl-Phenyl | Vinyl | |
| 1.3.157 | 2-Ethoxy-3-ethyl-Phenyl | Vinyl | |
| 1.3.158 | 2-Trifluormethoxy-3-ethyl-Phenyl | Vinyl | |
| 1.3.159 | 2-Nitro-3-ethyl-Phenyl | Vinyl | |
| 1.3.160 | 2-Fluor-3-propyl-Phenyl | Vinyl | |
| 1.3.161 | 2-Chlor-3-propyl-Phenyl | Vinyl | |
| 1.3.162 | 2-Brom-3-propyl-Phenyl | Vinyl | |
| 1.3.163 | 2-Methyl-3-propyl-Phenyl | Vinyl | |
| 1.3.164 | 2-Methyl-3-propyl-Phenyl | Vinyl | |
| 1.3.165 | 2-Cyclopropyl-3-propyl-Phenyl | Vinyl | |
| 1.3.166 | 2-Vinyl-3-propyl-Phenyl | Vinyl | |
| 1.3.167 | 2-Ethinyl-3propyl-Phenyl | Vinyl | |
| 1.3.168 | 2-Cyano-3-propyl-Phenyl | Vinyl | |
| 1.3.169 | 2-Trifluormethyl-3-propyl-Phenyl | Vinyl | |
| 1.3.170 | 2-Methoxy-3-propyl-Phenyl | Vinyl | |
| 1.3.171 | 2-Ethoxy-3-propyl-Phenyl | Vinyl | |
| 1.3.172 | 2-Trifluormethoxy-3-propyl-Phenyl | Vinyl | |
| 1.3.173 | 2-Nitro-3-propyl-Phenyl | Vinyl | |
| 1.3.174 | 2-Fluor-3-isopropyl-Phenyl | Vinyl | |
| 1.3.175 | 2-Chlor-3-isopropyl-Phenyl | Vinyl | |
| 1.3.176 | 2-Brom-3-isopropyl-Phenyl | Vinyl | |
| 1.3.177 | 2-Methyl-3-isopropyl-Phenyl | Vinyl | |
| 1.3.178 | 2-Ethyl-3-isopropyl-Phenyl | Vinyl | |
| 1.3.179 | 2-Cyclopropyl-3-isopropyl-Phenyl | Vinyl | |
| 1.3.180 | 2-Vinyl-3-isopropyl-Phenyl | Vinyl | |
| 1.3.181 | 2-Ethinyl-3-isopropyl-Phenyl | Vinyl | |
| 1.3.182 | 2-Cyano-3-isopropyl-Phenyl | Vinyl | |
| 1.3.183 | 2-Trifluormethyl-3-isopropyl-Phenyl | Vinyl | |
| 1.3.184 | 2-Methoxy-3-isopropyl-Phenyl | Vinyl | |
| 1.3.185 | 2-Ethoxy-3-isopropyl-Phenyl | Vinyl | |
| 1.3.186 | 2-Trifluormethoxy-3-isopropyl-Phenyl | Vinyl | |
| 1.3.187 | 2-Nitro-3-isopropyl-Phenyl | Vinyl | |
| 1.3.188 | 2-Fluor-3-tert.butyl-Phenyl | Vinyl | |
| 1.3.189 | 2-Chlor-3-tert.buty-Phenyl | Vinyl | |
| 1.3.190 | 2-Brom-3-tert.butyl-Phenyl | Vinyl | |
| 1.3.191 | 2-Methyl-3-tert.butyl-Phenyl | Vinyl | |
| 1.3.192 | 2-Ethyl-3-tert.butyl-Phenyl | Vinyl | |
| 1.3.193 | 2-Cyclopropyl-3-tert.butyl-Phenyl | Vinyl | |
| 1.3.194 | 2-Vinyl-3-tert.butyl-Phenyl | Vinyl | |
| 1.3.195 | 2-Ethinyl-3-tert.butylPhenyl | Vinyl | |
| 1.3.196 | 2-Cyano-3-tert.butyl-Phenyl | Vinyl | |
| 1.3.197 | 2-Trifluormethyl-3-tert.butyl-Phenyl | Vinyl | |
| 1.3.198 | 2-Methoxy-3-tert.butyl-Phenyl | Vinyl | |
| 1.3.199 | 2-Ethoxy-3-tert.butyl-Phenyl | Vinyl | |
| 1.3.200 | 2-Trifluormethoxy-3-tert.butyl-Phenyl | Vinyl | |
| 1.3.201 | 2-Nitro-3-tert.butyl-Phenyl | Vinyl | |
| 1.3.202 | 2-Fluor-3-hydroxymethyl-Phenyl | Vinyl | |
| 1.3.203 | 2-Chlor-3-hydroxymethyl-Phenyl | Vinyl | |
| 1.3.204 | 2-Brom-3-hydroxymethyl-Phenyl | Vinyl | |
| 1.3.205 | 2-Methyl-3-hydroxymethyl-Phenyl | Vinyl | |
| 1.3.206 | 2-Ethyl-3-hydroxymethyl-Phenyl | Vinyl | |
| 1.3.207 | 2-Cyclopropyl-3-hydroxymethyl-Phenyl | Vinyl | |
| 1.3.208 | 2-Vinyl-3-hydroxymethyl-Phenyl | Vinyl | |
| 1.3.209 | 2-Ethinyl-3-hydroxymethyl-Phenyl | Vinyl | |
| 1.3.210 | 2-Cyano-3-hydroxymethyl -Phenyl | Vinyl | |
| 1.3.211 | 2-Trifluormethyl-3-hydroxymethyl-Phenyl | Vinyl | |
| 1.3.212 | 2-Methoxy-3-hydroxymethyl -Phenyl | Vinyl | |
| 1.3.213 | 2-Ethoxy-3-hydroxymethyl-Phenyl | Vinyl | |
| 1.3.214 | 2-Trifluormethoxy-3-hydroxymethyl-Phenyl | Vinyl | |
| 1.3.215 | 2-Nitro-3-hydroxymethyl-Phenyl | Vinyl | |
| 1.3.216 | 2-Fluor-3-cyclopropyl-Phenyl | Vinyl | |
| 1.3.217 | 2-Chlor-3-cyclopropyl-Phenyl | Vinyl | |
| 1.3.218 | 2-Brom-3-cyclopropyl-Phenyl | Vinyl | |
| 1.3.219 | 2-Methyl-3-cyclopropyl-Phenyl | Vinyl | |
| 1.3.220 | 2-Ethyl-3-cyclopropyl -Phenyl | Vinyl | |
| 1.3.221 | 2-Cyclopropyl-3-cyclopropyl-Phenyl | Vinyl | |
| 1.3.222 | 2-Vinyl-3-cyclopropyl-Phenyl | Vinyl | |
| 1.3.223 | 2-Ethinyl-3-cyclopropyl-Phenyl | Vinyl | |
| 1.3.224 | 2-Cyano-3-cyclopropyl-Phenyl | Vinyl | |
| 1.3.225 | 2-Trifluormethyl-3-cyclopropyl-Phenyl | Vinyl | |
| 1.3.226 | 2-Methoxy-3-cyclopropyl-Phenyl | Vinyl | |
| 1.3.227 | 2-Ethoxy-3-cyclopropyl-Phenyl | Vinyl | |
| 1.3.228 | 2-Trifluormethoxy-3-cyclopropyl-Phenyl | Vinyl | |
| 1.3.229 | 2-Fluor-3-methoxycarbonyl-Phenyl | Vinyl | |
| 1.3.230 | 2-Chlor-3-methoxycarbonyl-Phenyl | Vinyl | |
| 1.3.231 | 2-Brom-3-methoxycarbonyl-Phenyl | Vinyl | |
| 1.3.232 | 2-Methyl-3-methoxycarbonyl-Phenyl | Vinyl | |
| 1.3.233 | 2-Ethyl-3-methoxycarbonyl-Phenyl | Vinyl | |
| 1.3.234 | 2-Cyclopropyl-3-methoxycarbonyl-Phenyl | Vinyl | |
| 1.3.235 | 2-Vinyl-3-methoxycarbonyl-Phenyl | Vinyl | |
| 1.3.236 | 2-Ethinyl-3-methoxycarbonyl-Phenyl | Vinyl | |
| 1.3.237 | 2-Cyano-3-methoxycarbonyl-Phenyl | Vinyl | |
| 1.3.238 | 2-Trifluormethyl-3-methoxycarbonyl-Phenyl | Vinyl | |
| 1.3.239 | 2-Methoxy-3-methoxycarbonyl-Phenyl | Vinyl | |
| 1.3.240 | 2-Ethoxy-3-methoxycarbonyl-Phenyl | Vinyl | |
| 1.3.241 | 2-Trifluormethoxy-3-methoxycarbonyl-Phenyl | Vinyl | |
| 1.3.242 | 2-Nitro-3-methoxycarbonyl-Phenyl | Vinyl | |
| 1.3.243 | 2-Fluor-3-vinyl-Phenyl | Vinyl | |
| 1.3.244 | 2-Chlor-3-vinyl-Phenyl | Vinyl | |
| 1.3.245 | 2-Brom-3-vinyl-Phenyl | Vinyl | |
| 1.3.246 | 2-Methyl-3-vinyl-Phenyl | Vinyl | |
| 1.3.247 | 2-Ethyl-3-vinyl-Phenyl | Vinyl | |
| 1.3.248 | 2-Cyclopropyl-3-vinyl-Phenyl | Vinyl | |
| 1.3.249 | 2-Vinyl-3-vinyl-Phenyl | Vinyl | |
| 1.3.250 | 2-Ethinyl-3-vinyl-Phenyl | Vinyl | |
| 1.3.251 | 2-Cyano-3-vinyl-Phenyl | Vinyl | |
| 1.3.252 | 2-Trifluormethyl-3-vinyl-Phenyl | Vinyl | |
| 1.3.253 | 2-Methoxy-3-vinyl-Phenyl | Vinyl | |
| 1.3.254 | 2-Ethoxy-3-vinyl-Phenyl | Vinyl | |
| 1.3.255 | 2-Trifluormethoxy-3-vinyl-Phenyl | Vinyl | |
| 1.3.256 | 2-Nitro-3-vinyl-Phenyl | Vinyl | |
| 1.3.257 | 2-Fluor-3-ethinyl-Phenyl | Vinyl | |
| 1.3.258 | 2-Chlor-3-ethinyl-Phenyl | Vinyl | |
| 1.3.259 | 2-Brom-3-ethinyl-Phenyl | Vinyl | |
| 1.3.260 | 2-Methyl-3-ethinyl-Phenyl | Vinyl | |
| 1.3.261 | 2-Ethyl-3-ethinyl-Phenyl | Vinyl | |
| 1.3.262 | 2-Cyclopropyl-3-ethinyl-Phenyl | Vinyl | |
| 1.3.263 | 2-Vinyl-3-ethinyl-Phenyl | Vinyl | |
| 1.3.264 | 2-Cyano-3-ethinyl-Phenyl | Vinyl | |
| 1.3.265 | 2-Trifluormethyl-3-ethinyl-Phenyl | Vinyl | |
| 1.3.266 | 2-Methoxy-3-ethinyl-Phenyl | Vinyl | |
| 1.3.267 | 2-Ethoxy-3-ethinyl-Phenyl | Vinyl | |
| 1.3.268 | 2-Trifluormethoxy-3-ethinyl--Phenyl | Vinyl | |
| 1.3.269 | 2-Nitro-3-ethinyl-Phenyl | Vinyl | |
| 1.3.270 | 2-Fluor-3-ethinyl-Phenyl | Vinyl | |
| 1.3.271 | 2-Fluor-3-cyano-Phenyl | Vinyl | |
| 1.3.272 | 2-Chlor-3-cyano-Phenyl | Vinyl | |
| 1.3.273 | 2-Brom-3-cyano-Phenyl | Vinyl | |
| 1.3.274 | 2-Methyl-3-cyano-Phenyl | Vinyl | |
| 1.3.275 | 2-Ethyl-3-cyano-Phenyl | Vinyl | |
| 1.3.276 | 2-Ethyl-3-cyano-Phenyl | 1-Methyl vinyl | |
| 1.3.277 | 2-Ethyl-3-cyano-Phenyl | Allyl | |
| 1.3.278 | 2-Ethyl-3-cyano-Phenyl | 1-Chlor vinyl | |
| 1.3.279 | 2-Ethyl-3-cyano-Phenyl | Ethinyl | |
| 1.3.280 | 2-Cyclopropyl-3-cyano-Phenyl | Vinyl | |
| 1.3.281 | 2-Vinyl-3-cyano-Phenyl | Vinyl | |
| 1.3.282 | 2-Ethinyl-3-cyano-Phenyl | Vinyl | |
| 1.3.283 | 2-Cyano-3-cyanoPhenyl | Vinyl | |
| 1.3.284 | 2-Trifluormethyl-3-cyano-Phenyl | Vinyl | |
| 1.3.285 | 2-Methoxy-3-cyano-Phenyl | Vinyl | |
| 1.3.286 | 2-Ethoxy-3-cyano-Phenyl | Vinyl | |
| 1.3.287 | 2-Trifluormethoxy-3-cyano-Phenyl | Vinyl | |
| 1.3.288 | 2-Nitro-3-cyano-Phenyl | Vinyl | |
| 1.3.289 | 2-Fluor-3-hydroxy-Phenyl | Vinyl | |
| 1.3.290 | 2-Chlor-3-hydroxy-Phenyl | Vinyl | |
| 1.3.291 | 2-Brom-3-hydroxy-Phenyl | Vinyl | |
| 1.3.292 | 2-Methyl-3-hydroxy-Phenyl | Vinyl | |
| 1.3.293 | 2-Ethyl-3-hydroxy-Phenyl | Vinyl | |
| 1.3.294 | 2-Cyclopropyl-3-hydroxy-Phenyl | Vinyl | |
| 1.3.295 | 2-Vinyl-3-hydroxy-Phenyl | Vinyl | |
| 1.3.296 | 2-Ethinyl-3-hydroxy-Phenyl | Vinyl | |
| 1.3.297 | 2-Cyano-3-hydroxy-Phenyl | Vinyl | |
| 1.3.298 | 2-Trifluormethyl-3-hydroxy-Phenyl | Vinyl | |
| 1.3.299 | 2-Methoxy-3-hydroxy-Phenyl | Vinyl | |
| 1.3.300 | 2-Ethoxy-3-hydroxy-Phenyl | Vinyl | |
| 1.3.301 | 2-Trifluormethoxy-3-hydroxy-Phenyl | Vinyl | |
| 1.3.302 | 2-Nitro-3-hydroxy-Phenyl | Vinyl | |
| 1.3.303 | 2-Fluor-3-methoxy-Phenyl | Vinyl | |
| 1.3.304 | 2-Chlor-3-methoxy-Phenyl | Vinyl | |
| 1.3.305 | 2-Brom-3-methoxy-Phenyl | Vinyl | |
| 1.3.306 | 2-Methyl-3-methoxy-Phenyl | Vinyl | |
| 1.3.307 | 2-Ethyl-3-methoxy-Phenyl | Vinyl | |
| 1.3.308 | 2-Cyclopropyl-3-methoxy-Phenyl | Vinyl | |
| 1.3.309 | 2-Vinyl-3-methoxy-Phenyl | Vinyl | |
| 1.3.310 | 2-Ethinyl-3-methoxy-Phenyl | Vinyl | |
| 1.3.311 | 2-Cyano-3-methoxy-Phenyl | Vinyl | |
| 1.3.312 | 2-Trifluormethyl-3-methoxy-Phenyl | Vinyl | |
| 1.3.313 | 2,3-Dimethoxy-Phenyl | Vinyl | |
| 1.3.314 | 2-Ethoxy-3-methoxy-Phenyl | Vinyl | |
| 1.3.315 | 2-Trifluormethoxy-3-methoxy-Phenyl | Vinyl | |
| 1.3.316 | 2-Nitro-3-methoxy-Phenyl | Vinyl | |
| 1.3.317 | 2-Fluor-3-ethoxy-Phenyl | Vinyl | |
| 1.3.318 | 2-Chlor-3-ethoxy-Phenyl | Vinyl | |
| 1.3.319 | 2-Brom-3-ethoxy-Phenyl | Vinyl | |
| 1.3.320 | 2-Methyl-3-ethoxy-Phenyl | Vinyl | |
| 1.3.321 | 2-Ethyl-3-ethoxy-Phenyl | Vinyl | |
| 1.3.322 | 2-Cyclopropyl-3-ethoxy-Phenyl | Vinyl | |
| 1.3.323 | 2-Vinyl-3-ethoxy-Phenyl | Vinyl | |
| 1.3.324 | 2-Ethinyl-3-ethoxy-Phenyl | Vinyl | |
| 1.3.325 | 2-Cyano-3-ethoxy-Phenyl | Vinyl | |
| 1.3.326 | 2-Trifluormethyl-3-ethoxy-Phenyl | Vinyl | |
| 1.3.327 | 2-Methoxy-3-ethoxy -Phenyl | Vinyl | |
| 1.3.328 | 2,3-Diethoxy--Phenyl | Vinyl | |
| 1.3.329 | 2-Trifluormethoxy-3-ethoxy-Phenyl | Vinyl | |
| 1.3.330 | 2-Nitro-3-ethoxy-Phenyl | Vinyl | |
| 1.3.331 | 2-Fluor-3-propoxy-Phenyl | Vinyl | |
| 1.3.332 | 2-Chlor-3-propoxy-Phenyl | Vinyl | |
| 1.3.333 | 2-Brom-3-propoxy-Phenyl | Vinyl | |
| 1.3.334 | 2-Methyl-3-propoxy-Phenyl | Vinyl | |
| 1.3.335 | 2-Ethyl-3-propoxy-Phenyl | Vinyl | |
| 1.3.336 | 2-Cyclopropyl-3-propoxy-Phenyl | Vinyl | |
| 1.3.337 | 2-Vinyl-3-propoxy-Phenyl | Vinyl | |
| 1.3.338 | 2-Ethinyl-3-propoxy-Phenyl | Vinyl | |
| 1.3.339 | 2-Cyano-3-propoxy-Phenyl | Vinyl | |
| 1.3.340 | 2-Trifluormethyl-3-propoxy-Phenyl | Vinyl | |
| 1.3.341 | 2-Methoxy-3-propoxy-Phenyl | Vinyl | |
| 1.3.342 | 2-Ethoxy-3-propoxy-Phenyl | Vinyl | |
| 1.3.343 | 2-Trifluormethoxy-3-propoxy-Phenyl | Vinyl | |
| 1.3.344 | 2-Nitro-3-propoxy-Phenyl | Vinyl | |
| 1.3.345 | 2-Fluor-3-isopropoxy-Phenyl | Vinyl | |
| 1.3.346 | 2-Chlor-3-isopropoxy-Phenyl | Vinyl | |
| 1.3.347 | 2-Brom-3-isopropoxy-Phenyl | Vinyl | |
| 1.3.348 | 2-Methyl-3-isopropoxy-Phenyl | Vinyl | |
| 1.3.349 | 2-Ethyl-3-isopropoxy-Phenyl | Vinyl | |
| 1.3.350 | 2-Cyclopropyl-3-isopropoxy-Phenyl | Vinyl | |
| 1.3.351 | 2-Vinyl-3-isopropoxy-Phenyl | Vinyl | |
| 1.3.352 | 2-Ethinyl-3-isopropoxy-Phenyl | Vinyl | |
| 1.3.353 | 2-Cyano-3-isopropoxy-Phenyl | Vinyl | |
| 1.3.354 | 2-Trifluormethyl-3-isopropoxy-Phenyl | Vinyl | |
| 1.3.355 | 2-Methoxy-3-isopropoxy-Phenyl | Vinyl | |
| 1.3.356 | 2-Ethoxy-3-isopropoxy-Phenyl | Vinyl | |
| 1.3.357 | 2-Trifluormethoxy-3-isopropoxy-Phenyl | Vinyl | |
| 1.3.358 | 2-Nitro-3-isopropoxy-Phenyl | Vinyl | |
| 1.3.359 | 2-Fluor-3-tert.butoxy-Phenyl | Vinyl | |
| 1.3.360 | 2-Chlor-3-tert.butoxy-Phenyl | Vinyl | |
| 1.3.361 | 2-Brom-3-tert.butoxy-Phenyl | Vinyl | |
| 1.3.362 | 2-Methyl-3-tert.butoxy-Phenyl | Vinyl | |
| 1.3.363 | 2-Ethyl-3-tert.butoxy-Phenyl | Vinyl | |
| 1.3.364 | 2-Cyclopropyl-3-tert.butoxy-Phenyl | Vinyl | |
| 1.3.365 | 2-Vinyl-3-tert.butoxy-Phenyl | Vinyl | |
| 1.3.366 | 2-Ethinyl-3-tert.butoxy-Phenyl | Vinyl | |
| 1.3.367 | 2-Cyano-3-tert.butoxy-Phenyl | Vinyl | |
| 1.3.368 | 2-Trifluormethyl-3-tert.butoxy-Phenyl | Vinyl | |
| 1.3.369 | 2-Methoxy-3-tert.butoxy-Phenyl | Vinyl | |
| 1.3.370 | 2-Ethoxy-3-tert.butoxy-Phenyl | Vinyl | |
| 1.3.371 | 2-Trifluormethoxy-3-tert.butoxy-Phenyl | Vinyl | |
| 1.3.372 | 2-Nitro-3-tert.butoxy-Phenyl | Vinyl | |
| 1.3.373 | 2-Fluor-3-trifluormethoxy-Phenyl | Vinyl | |
| 1.3.374 | 2-Chlor-3-trifluormethoxy-Phenyl | Vinyl | |
| 1.3.375 | 2-Brom-3-trifluormethoxy-Phenyl | Vinyl | |
| 1.3.376 | 2-Methyl-3-trifluormethoxy-Phenyl | Vinyl | |
| 1.3.377 | 2-Ethyl-3-trifluormethoxy-Phenyl | Vinyl | |
| 1.3.378 | 2-Cyclopropyl-3-trifluormethoxy-Phenyl | Vinyl | |
| 1.3.379 | 2-Vinyl-3-trifluormethoxy-Phenyl | Vinyl | |
| 1.3.380 | 2-Ethinyl-3-trifluormethoxy-Phenyl | Vinyl | |
| 1.3.381 | 2-Cyano-3-trifluormethoxy-Phenyl | Vinyl | |
| 1.3.382 | 2-Trifluormethyl-3-trifluormethoxy-Phenyl | Vinyl | |
| 1.3.383 | 2-Methoxy-3-trifluormethoxy-Phenyl | Vinyl | |
| 1.3.384 | 2-Ethoxy-3-trifluormethoxy-Phenyl | Vinyl | |
| 1.3.385 | 2,3-Bis(trifluormethoxy)-Phenyl | Vinyl | |
| 1.3.386 | 2-Nitro-3-trifluormethoxy-Phenyl | Vinyl | |
| 1.3.387 | 2-Fluor-3-(2,2,2-trifluorethoxy)-Phenyl | Vinyl | |
| 1.3.388 | 2-Chlor-3-(2,2,2-trifluorethoxy)-Phenyl | Vinyl | |
| 1.3.389 | 2-Brom-3-(2,2,2-trifluorethoxy)-Phenyl | Vinyl | |
| 1.3.390 | 2-Methyl-3-(2,2,2-trifluorethoxy)-Phenyl | Vinyl | |
| 1.3.391 | 2-Ethyl-3-(2,2,2-trifluorethoxy)-Phenyl | Vinyl | |
| 1.3.392 | 2-Cyclopropyl-3-(2,2,2-trifluorethoxy)-Phenyl | Vinyl | |
| 1.3.393 | 2-Vinyl-3-(2,2,2-trifluorethoxy)-Phenyl | Vinyl | |
| 1.3.394 | 2-Ethinyl-3-(2,2,2-trifluorethoxy-Phenyl | Vinyl | |
| 1.3.395 | 2-Cyano-3-(2,2,2-trifluorethoxy)-Phenyl | Vinyl | |
| 1.3.396 | 2-Trifluormethyl-3-(2,2,2-trifluorethoxy)-Phenyl | Vinyl | |
| 1.3.397 | 2-Methoxy-3-(2,2,2-trifluorethoxy)-Phenyl | Vinyl | |
| 1.3.398 | 2-Ethoxy-3-(2,2,2-trifluorethoxy)-Phenyl | Vinyl | |
| 1.3.399 | 2-Trifluormethoxy-3-(2,2,2-trifluorethoxy)-Phenyl | Vinyl | |
| 1.3.400 | 2-Nitro-3-(2,2,2-trifluorethoxy)-Phenyl | Vinyl | |
| 1.3.401 | 2-Fluor-3-difluormethoxy-Phenyl | Vinyl | |
| 1.3.402 | 2-Chlor-3-difluormethoxy-Phenyl | Vinyl | |
| 1.3.403 | 2-Brom-3-difluormethoxy-Phenyl | Vinyl | |
| 1.3.404 | 2-Methyl-3-difluormethoxy-Phenyl | Vinyl | |
| 1.3.405 | 2-Ethyl-3-difluormethoxy-Phenyl | Vinyl | |
| 1.3.406 | 2-Cyclopropyl-3-difluormethoxy-Phenyl | Vinyl | |
| 1.3.407 | 2-Vinyl-3-difluormethoxy-Phenyl | Vinyl | |
| 1.3.408 | 2-Ethinyl-3-difluormethoxy-Phenyl | Vinyl | |
| 1.3.409 | 2-Cyano-3-difluormethoxy-Phenyl | Vinyl | |
| 1.3.410 | 2-Trifluormethyl-3-difluormethoxy -Phenyl | Vinyl | |
| 1.3.411 | 2-Methoxy-3-difluormethoxy -Phenyl | Vinyl | |
| 1.3.412 | 2-Ethoxy-3-difluormethoxy-Phenyl | Vinyl | |
| 1.3.413 | 2-Trifluormethoxy-3-difluormethoxy-Phenyl | Vinyl | |
| 1.3.414 | 2-Nitro-3-difluormethoxy-Phenyl | Vinyl | |
| 1.3.415 | 2-Fluor-3-(2-methoxyethoxy)-Phenyl | Vinyl | |
| 1.3.416 | 2-Chlor-3-(2-methoxyethoxy)-Phenyl | Vinyl | |
| 1.3.417 | 2-Brom-3-(2-methoxyethoxy)-Phenyl | Vinyl | |
| 1.3.418 | 2-Methyl-3-(2-methoxyethoxy)-Phenyl | Vinyl | |
| 1.3.419 | 2-Ethyl-3-(2-methoxyethoxy)-Phenyl | Vinyl | |
| 1.3.420 | 2-Cyclopropyl-3-(2-methoxyethoxy)-Phenyl | Vinyl | |
| 1.3.421 | 2-Vinyl-3-(2-methoxyethoxy)-Phenyl | Vinyl | |
| 1.3.422 | 2-Ethinyl-3-(2-methoxyethoxy)-Phenyl | Vinyl | |
| 1.3.423 | 2-Cyano-3-(2-methoxyethoxy)-Phenyl | Vinyl | |
| 1.3.424 | 2-Trifluormethyl-3-(2-methoxyethoxy)-Phenyl | Vinyl | |
| 1.3.425 | 2-Methoxy-3-(2-methoxyethoxy) -Phenyl | Vinyl | |
| 1.3.426 | 2-Ethoxy-3-(2-methoxyethoxy)-Phenyl | Vinyl | |
| 1.3.427 | 2-Trifluormethoxy-(2-methoxyethoxy)-Phenyl | Vinyl | |
| 1.3.428 | 2-Nitro-3-(2-methoxyethoxy)-Phenyl | Vinyl | |
| 1.3.429 | 2-Fluor-3-(tert.butoxycarbonyloxy)-Phenyl | Vinyl | |
| 1.3.430 | 2-Chlor-3-(tert.butoxycarbonyloxy)-Phenyl | Vinyl | |
| 1.3.431 | 2-Brom-3-(tert.butoxycarbonyloxy)-Phenyl | Vinyl | |
| 1.3.432 | 2-Methyl-3-(tert.butoxycarbonyloxy)-Phenyl | Vinyl | |
| 1.3.433 | 2-Ethyl-3-(tert.butoxycarbonyloxy)-Phenyl | Vinyl | |
| 1.3.434 | 2-Cyclopropyl-3--(tert.butoxycarbonyloxy)-Phenyl | Vinyl | |
| 1.3.435 | 2-Vinyl-3-(tert.butoxycarbonyloxy)-Phenyl | Vinyl | |
| 1.3.436 | 2-Ethinyl-3-(tert.butoxycarbonyloxy)-Phenyl | Vinyl | |
| 1.3.437 | 2-Cyano-3-(tert.butoxycarbonyloxy)-Phenyl | Vinyl | |
| 1.3.438 | 2-Trifluormethyl-3-(tert.butoxycarbonyloxy)-Phenyl | Vinyl | |
| 1.3.439 | 2-Methoxy-3-(tert.butoxycarbonyloxy)-Phenyl | Vinyl | |
| 1.3.440 | 2-Ethoxy-3-(tert.butoxycarbonyloxy)-Phenyl | Vinyl | |
| 1.3.441 | 2-Trifluormethoxy-3-(tert.butoxycarbonyloxy)-Phenyl | Vinyl | |
| 1.3.442 | 2-Nitro-3-(tert.butoxycarbonyloxy)-Phenyl | Vinyl | |
| 1.3.443 | 2-Fluor-3-nitro-Phenyl | Vinyl | |
| 1.3.444 | 2-Chlor-3-nitro-Phenyl | Vinyl | |
| 1.3.445 | 2-Brom-3-nitro-Phenyl | Vinyl | |
| 1.3.446 | 2-Methyl-3-nitro-Phenyl | Vinyl | |
| 1.3.447 | 2-Ethyl-3-nitro-Phenyl | Vinyl | |
| 1.3.448 | 2-Cyclopropyl-3-nitro-Phenyl | Vinyl | |
| 1.3.449 | 2-Vinyl-3-nitro-Phenyl | Vinyl | |
| 1.3.450 | 2-Ethinyl-3-nitro-Phenyl | Vinyl | |
| 1.3.451 | 2-Cyano-3-nitro-Phenyl | Vinyl | |
| 1.3.452 | 2-Trifluormethyl-3-nitro-Phenyl | Vinyl | |
| 1.3.453 | 2-Methoxy-3-nitro-Phenyl | Vinyl | |
| 1.3.454 | 2-Ethoxy-3-nitro-Phenyl | Vinyl | |
| 1.3.455 | 2-Trifluormethoxy-3-nitro-Phenyl | Vinyl | |
| 1.3.456 | 2-Fluor-3-methylsulfanylPhenyl | Vinyl | |
| 1.3.457 | 2-Chlor-3-methylsulfanyl-Phenyl | Vinyl | |
| 1.3.458 | 2-Brom-3-methylsulfanyl-Phenyl | Vinyl | |
| 1.3.459 | 2-Methyl-3-methylsulfanyl-Phenyl | Vinyl | |
| 1.3.460 | 2-Ethyl-3-methylsulfanyl-Phenyl | Vinyl | |
| 1.3.461 | 2-Cyclopropyl-3-methylsulfanyl-Phenyl | Vinyl | |
| 1.3.462 | 2-Vinyl-3-methylsulfanyl-Phenyl | Vinyl | |
| 1.3.463 | 2-Ethinyl-3-methylsulfanyl-Phenyl | Vinyl | |
| 1.3.464 | 2-Cyano-3-methylsulfanyl-Phenyl | Vinyl | |
| 1.3.465 | 2-Trifluormethyl-3-methylsulfanyl-Phenyl | Vinyl | |
| 1.3.466 | 2-Methoxy-3-methylsulfanyl-Phenyl | Vinyl | |
| 1.3.467 | 2-Ethoxy-3-methylsulfanyl-Phenyl | Vinyl | |
| 1.3.468 | (R)-oder (S)-3,5-Difluor-Phenyl | Vinyl | Ein Enantiomeres ohne Zuordnung [CDCl₃] 3.41 (d,1H); 3.93 (d,1H); 5.45 (d,1H); 5.63 (d,1H); 6.16 (dd,1H); 6.91 (t,1H); 7.17 (d,2H). |
| 1.3.469 | (S)-oder (R)-3,5-Difluor-Phenyl | Vinyl | Ein Enantiomeres ohne Zuordnung [CDCl₃] 3.41 (d,1H); 3.93 (d,1H); 5.45 (d,1H); 5.63 (d,1H); 6.16 (dd,1H); 6.91 (t,1H); 7.17 (d,2H). |
| 1.3.470 | 3,5-Difluor-Phenyl | Vinyl | [CDCl3] 3.40 (d,1H); 3.93 (d,1H); 5.45 (d,1H); 5.63 (d,1H); 6.15 (dd,1H); 6.90 (t,1H); 7.18 (d,2H). |
| 1.3.471 | 3,5-Difluor-Phenyl | 1-Methyl vinyl | |
| 1.3.472 | 3,5-Difluor-Phenyl | 2,2-Difluorvinyl | [CDCl3] 3.54 (d,1H); 4.01 (d,1H); 4.90 (d,1H); 6.91 (t,1H); 7.18 (d,2H). |
| 1.3.473 | 3,5-Difluor-Phenyl | 1-Chlor vinyl | [CDCl₃] 3,62 (d,1H); 4,14 (d,1H); 5,61 (d,1H); 5,96 (d,1H); 6.91(t,1H); 7.20 (d,2H). |
| 1.3.474 | 3,5-Difluor-Phenyl | Ethinyl | [CDCl3] 2.1 (s br,1H); 2.86 (s,1H), 3.76 (d,1H); 4.08 (d,1H); 6.82 (t,1H); 7.19 (d,2H). |
| 1.3.475 | 3-Chlor-5-fluor-Phenyl | Vinyl | |
| 1.3.476 | 3-Chlor-5-fluor-Phenyl | 1-Methyl vinyl | [CDCl3] 1.91 (s,3H); 3.40 (d,1H); 4.00 (d,1H); 5.17 (s,1H); 5.35 (s,1H); 7.17 (d,1H); 7.32 (d,1H); 7.42 (s,1H), 8.9 (s br,1 H). |
| 1.3.477 | 3-Chlor-5-fluor-Phenyl | Allyl | |
| 1.3.478 | 3-Chlor-5-fluor-Phenyl | 1-Chlor vinyl | |
| 1.3.479 | 3-Chlor-5-fluor-Phenyl | Ethinyl | |
| 1.3.480 | 3-Brom-5-fluor-Phenyl | Vinyl | |
| 1.3.481 | 3-Brom-5-fluor-Phenyl | 1-Methyl vinyl | |
| 1.3.482 | 3-Brom-5-fluor-Phenyl | Allyl | |
| 1.3.483 | 3-Brom-5-fluor-Phenyl | 1-Chlor vinyl | |
| 1.3.484 | 3-Brom-5-fluor-Phenyl | Ethinyl | |
| 1.3.485 | 3-Iod-5-fluor-Phenyl | Vinyl | |
| 1.3.486 | 3-Methyl-5-fluor-Phenyl | Vinyl | [CDCl3] 2.38 (s,3H); 3.42 (d,1H); 3.94 (d,1H); 5.43 (d,1H); 5.65 (d,1H); 6.15 (dd,1H); 6.98 (d,1H); 7.18 (d,1H); 7.22 (s,1 H). |
| 1.3.487 | 3-Methyl-5-fluor-Phenyl | 1-Methyl vinyl | [CDCl3] 1.91 (s,3H), 2.38 (s,3H); 3.42 (d,1H), 4.00 (d,1H); 5.15 (s,1H); 5.35 (s,1H); 6.3 (s br,1H), 6.98 (d,1H); 7.17 (d,1H); 7.21 (s,1H). |
| 1.3.488 | 3-Methyl-5-fluor-Phenyl | Allyl | |
| 1.3.489 | 3-Methyl-5-fluor-Phenyl | 1-Chlor vinyl | |
| 1.3.490 | 3-Methyl-5-fluor-Phenyl | Ethinyl | |
| 1.3.491 | 3-Ethyl-5-fluor-Phenyl | Vinyl | |
| 1.3.492 | 3-Propyl-5-fluor-Phenyl | Vinyl | |
| 1.3.493 | 3-iPropyl-5-fluor-Phenyl | Vinyl | |
| 1.3.494 | 3-nButyl-5-fluor-Phenyl | Vinyl | |
| 1.3.495 | 3-isoButyl-5-fluor-Phenyl | Vinyl | |
| 1.3.496 | 3-tert.Butyl-5-fluor-Phenyl | Vinyl | |
| 1.3.497 | 3-Cyclopropyl-5-fluor-Phenyl | Vinyl | |
| 1.3.498 | 3-Vinyl-5-fluor-Phenyl | Vinyl | |
| 1.3.499 | 3-Ethinyl-5-fluor-Phenyl | Vinyl | |
| 1.3.500 | 3-Cyano-5-fluor-Phenyl | Vinyl | |
| 1.3.501 | 3-Trifluormethyl-5-fluor-Phenyl | Vinyl | |
| 1.3.502 | 3-Trifluormethyl-5-fluor-Phenyl | 1-Methyl vinyl | |
| 1.3.503 | 3-Trifluormethyl-5-fluor-Phenyl | Allyl | |
| 1.3.504 | 3-Trifluormethyl-5-fluor-Phenyl | 1-Chlor vinyl | |
| 1.3.505 | 3-Trifluormethyl-5-fluor-Phenyl | Ethinyl | |
| 1.3.506 | 3-(Methoxycarbony)l-5-fluor-Phenyl | Vinyl | |
| 1.3.507 | 3-Hydroxymethyl-5-fluor-Phenyl | Vinyl | |
| 1.3.508 | 3-Carbamoyl-5-fluor-Phenyl | Vinyl | |
| 1.3.509 | 3-Hydroxy-5-fluor-Phenyl- | Vinyl | |
| 1.3.510 | 3-Methoxy-5-fluor-Phenyl | Vinyl | |
| 1.3.511 | 3-Ethoxy-5-fluor-Phenyl | Vinyl | |
| 1.3.512 | 3-nPropyoxy-5-fluor-Phenyl | Vinyl | |
| 1.3.513 | 3-isoPropoxy-5-fluor-Phenyl | Vinyl | |
| 1.3.514 | 3-nButoxy-5-fluor-Phenyl | Vinyl | |
| 1.3.515 | 3-isoButoxy-5-fluor-Phenyl | Vinyl | |
| 1.3.516 | 3-tert.Butoxy-5-fluor-Phenyl | Vinyl | |
| 1.3.517 | 3-Difluormethoxy-5-fluor-Phenyl | Vinyl | |
| 1.3.518 | 3-Trifluormethoxy-5-fluor-Phenyl | Vinyl | |
| 1.3.519 | 3-(2,2,2-Trifluorethoxy)-5-fluor-Phenyl | Vinyl | |
| 1.3.520 | 3-(2-Chlorethoxy)-5-fluor-Phenyl | Vinyl | |
| 1.3.521 | 3-(2-Hydroxyethoxy)-5-fluor-Phenyl- | Vinyl | |
| 1.3.522 | 3-[(tert-Butoxycarbonyl)oxy]-5-fluor-Phenyl | Vinyl | |
| 1.3.523 | 3-Nitro-5-fluor-Phenyl | Vinyl | |
| 1.3.524 | 3-Acetoxy-5-fluor-Phenyl | Vinyl | |
| 1.3.525 | {3-[(tert-Butoxycarbonyl)amino]-5-fluor-Phenyl} | Vinyl | |
| 1.3.526 | 3-Methylsulfanyl-5-fluor-Phenyl | Vinyl | |
| 1.3.527 | 3,5-Dichlor-Phenyl | Vinyl | [CDCl₃] 3.41 (d,1H); 3.93 (d,1H); 5.45 (d,1H); 5.62 (d,1H); 6.15 (dd,1H); 7.44 (s,1H); 7.54 (s,2H). |
| 1.3.528 | 3,5-Dichlor-Phenyl | 1-Methyl vinyl | [CDCl3] 1.91 (s,3H); 3.40 (d,1H); 3.99 (d,1H); 5.16 (s,1H), 5.35 (s,1H); 5.9 (s br,1H), 7.42 (s,1H); 7.54 (s,2H). |
| 1.3.529 | 3,5-Dichlor-Phenyl | Allyl | |
| 1.3.530 | 3,5-Dichlor-Phenyl | 1-Chlor vinyl | |
| 1.3.531 | 3,5-Dichlor-Phenyl | Ethinyl | |
| 1.3.532 | 3-Brom-5-chlor-Phenyl | Vinyl | |
| 1.3.533 | 3-Iod-5-chlor-Phenyl | Vinyl | |
| 1.3.534 | 3-Methyl-5-chlor-Phenyl | Vinyl | |
| 1.3.535 | 3-Methyl-5-chlor-Phenyl | 1-Methyl vinyl | [CDCl₃] 1.91 (s,3H); 2.36 (s,3H); 3.42 (d,1H); 4.00 (d,1H); 5.15 (s,1H); 5.35 (s,1H); 6.6 (s br,1H); 7.24 (s,1H); 7.36 (s,1H); 7.45 (s,1H). |
| 1.3.536 | 3-Ethyl-5-chlor-Phenyl | Vinyl | [CDCₗ₃] 1.25 (t,3H); 2.65 (q,2H); 3.43 (d,1H); 3.95 (d,1H); 5.43 (d,1H); 5.63 (d,1H); 6.16 (dd,1H); 7.27 (s,1H); 7.38 (s,1H); 7.43 (s,1H). |
| 1.3.537 | 3-Propyl-5-chlor-Phenyl | Vinyl | |
| 1.3.538 | 3-nButyl-5-chlor-Phenyl | Vinyl | |
| 1.3.539 | 3-isoButyl-5-chlor-Phenyl | Vinyl | |
| 1.3.540 | 3-tert.Butyl-5-chlor-Phenyl | Vinyl | |
| 1.3.541 | 3-Cyclopropyl-5-chlor-Phenyl | Vinyl | |
| 1.3.542 | 3-Vinyl-5-chlor-Phenyl | Vinyl | |
| 1.3.543 | 3-Ethinyl-5-chlor-Phenyl | Vinyl | |
| 1.3.544 | 3-Cyano-5-chlor-Phenyl | Vinyl | |
| 1.3.545 | 3-Trifluormethyl-5-chlor-Phenyl | Vinyl | |
| 1.3.546 | 3-(Hydroxycarbonyl)-5-chlor-Phenyl | Vinyl | |
| 1.3.547 | 3-(Methoxycarbony)l-5-chlor-Phenyl | Vinyl | |
| 1.3.548 | 3-Hydroxymethyl-5-chlor-Phenyl | Vinyl | |
| 1.3.549 | 3-Carbamoyl-5-chlor-Phenyl | Vinyl | |
| 1.3.550 | 3-Hydroxy-5-chlor-Phenyl- | Vinyl | |
| 1.3.551 | 3-Methoxy-5-chlor-Phenyl | Vinyl | |
| 1.3.552 | 3-Ethoxy-5-chlor-Phenyl | Vinyl | |
| 1.3.553 | 3-nPropyoxy-5-chlor-Phenyl | Vinyl | |
| 1.3.554 | 3-isoPropoxy-5-chlor-Phenyl | Vinyl | |
| 1.3.555 | 3-nButoxy-5-chlor-Phenyl | Vinyl | |
| 1.3.556 | 3-isoButoxy-5-chlor-Phenyl | Vinyl | |
| 1.3.557 | 3-tert.Butoxy-5-chlor-Phenyl | Vinyl | |
| 1.3.558 | 3-Difluormethoxy-5-chlor-Phenyl | Vinyl | |
| 1.3.559 | 3-Trifluormethoxy-5-chlor-Phenyl | Vinyl | |
| 1.3.560 | 3-(2,2,2-Trifluorethoxy)-5-chlor-Phenyl | Vinyl | |
| 1.3.561 | 3-(2-Chlorethoxy)-5-chlor-Phenyl | Vinyl | |
| 1.3.562 | 3-(2-Hydroxyethoxy)-5-chlor-Phenyl- | Vinyl | |
| 1.3.563 | 3-[(tert-Butoxycarbonyl)oxy]-5-chlor-Phenyl | Vinyl | |
| 1.3.564 | 3-Nitro-5-chlor-Phenyl | Vinyl | |
| 1.3.565 | 3-Acetoxy-5-chlor-Phenyl | Vinyl | |
| 1.3.566 | {3-[(tert-Butoxycarbonyl)amino]-5-chlor-Phenyl} | Vinyl | |
| 1.3.567 | 3-Methylsulfanyl-5-chlor-Phenyl | Vinyl | |
| 1.3.568 | 3,5-Dibrom-Phenyl | Vinyl | |
| 1.3.569 | 3,5-Dibrom-Phenyl | 1-Methyl vinyl | |
| 1.3.570 | 3-Iod-5-brom-Phenyl | Vinyl | |
| 1.3.571 | 3-Methyl-5-brom-Phenyl | Vinyl | [CDCl3] 2.36 (s,3H); 3.42 (d,1H); 3.94 (d,1H); 5.43 (d,1H); 5.63 (d,1H); 6.15 (dd,1H); 7.40 (d,2H); 7.58 (s,1H). |
| 1.3.572 | 3-Methyl-5-brom-Phenyl | 1-Methyl vinyl | |
| 1.3.573 | 3-Methyl-5-brom-Phenyl | Allyl | |
| 1.3.574 | 3-Methyl-5-brom-Phenyl | 1-Chlor vinyl | |
| 1.3.575 | 3-Methyl-5-brom-Phenyl | Ethinyl | |
| 1.3.576 | 3-Ethyl-5-brom-Phenyl | Vinyl | |
| 1.3.577 | 3-Propyl-5-brom-Phenyl | Vinyl | |
| 1.3.578 | 3-isoPropyl-5-brom-Phenyl | Vinyl | |
| 1.3.579 | 3-nButyl-5-brom-Phenyl | Vinyl | |
| 1.3.580 | 3-isoButyl-5-brom-Phenyl | Vinyl | |
| 1.3.581 | 3-tert.Butyl-5-brom-Phenyl | Vinyl | |
| 1.3.582 | 3-Cyclopropyl-5-brom-Phenyl | Vinyl | |
| 1.3.583 | 3-Vinyl-5-brom-Phenyl | Vinyl | |
| 1.3.584 | 3-Ethinyl-5-brom-Phenyl | Vinyl | |
| 1.3.585 | 3-Cyano-5-brom-Phenyl | Vinyl | |
| 1.3.586 | 3-Trifluormethyl-5-brom-Phenyl | Vinyl | |
| 1.3.587 | 3-(Hydroxycarbonyl)-5-brom-Phenyl | Vinyl | |
| 1.3.588 | 3-(Methoxycarbony)l-5-brom-Phenyl | Vinyl | |
| 1.3.589 | 3-Hydroxymethyl-5-brom-Phenyl | Vinyl | |
| 1.3.590 | 3-Carbamoyl-5-brom-Phenyl | Vinyl | |
| 1.3.591 | 3-Hydroxy-5-brom-Phenyl- | Vinyl | |
| 1.3.592 | 3-Methoxy-5-brom-Phenyl | Vinyl | |
| 1.3.593 | 3-Ethoxy-5-brom-Phenyl | Vinyl | |
| 1.3.594 | 3-nPropyoxy-5-brom-Phenyl | Vinyl | |
| 1.3.595 | 3-isoPropoxy-5-brom-Phenyl | Vinyl | |
| 1.3.596 | 3-nButoxy-5-brom-Phenyl | Vinyl | |
| 1.3.597 | 3-isoButoxy-5-brom-Phenyl | Vinyl | |
| 1.3.598 | 3-tert.Butoxy-5-brom-Phenyl | Vinyl | |
| 1.3.599 | 3-Difluormethoxy-5-brom-Phenyl | Vinyl | |
| 1.3.600 | 3-Trifluormethoxy-5-brom-Phenyl | Vinyl | |
| 1.3.601 | 3-(2,2,2-Trifluorethoxy)-5-brom-Phenyl | Vinyl | |
| 1.3.602 | 3-(2-Chlorethoxy)-5-brom-Phenyl | Vinyl | |
| 1.3.603 | 3-(2-Hydroxyethoxy)-5-brom-Phenyl- | Vinyl | |
| 1.3.604 | 3-[(tert-Butoxycarbonyl)oxy]-5-brom-Phenyl | Vinyl | |
| 1.3.605 | 3-Nitro-5-brom-Phenyl | Vinyl | |
| 1.3.606 | 3-Acetoxy-5-brom-Phenyl | Vinyl | |
| 1.3.607 | {3-[(tert-Butoxycarbonyl)amino]-5-brom-Phenyl} | Vinyl | |
| 1.3.608 | 3-Methylsulfanyl-5-brom-Phenyl | Vinyl | |
| 1.3.609 | 3,5-Diiod-Phenyl | Vinyl | |
| 1.3.610 | 3-Methyl-5-iod-Phenyl | Vinyl | |
| 1.3.611 | 3-Ethyl-5-iod-Phenyl | Vinyl | |
| 1.3.612 | 3-Propyl-5-iod-Phenyl | Vinyl | |
| 1.3.613 | 3-isoPropyl-5-iod-Phenyl | Vinyl | |
| 1.3.614 | 3-nButyl-5-iod-Phenyl | Vinyl | |
| 1.3.615 | 3-isoButyl-5-iod-Phenyl | Vinyl | |
| 1.3.616 | 3-tert.Butyl-5-iod-Phenyl | Vinyl | |
| 1.3.617 | 3-Cyclopropyl-5-iod-Phenyl | Vinyl | |
| 1.3.618 | 3-Vinyl-5-iod-Phenyl | Vinyl | |
| 1.3.619 | 3-Ethinyl-5-iod-Phenyl | Vinyl | |
| 1.3.620 | 3-Cyano-5-iod-Phenyl | Vinyl | |
| 1.3.621 | 3-Trifluormethyl-5-iod-Phenyl | Vinyl | |
| 1.3.622 | 3-(Hydroxycarbonyl)-5-iod-Phenyl | Vinyl | |
| 1.3.623 | 3-(Methoxycarbonyl)-5-iod-Phenyl | Vinyl | |
| 1.3.624 | 3-Hydroxymethyl-5-iod-Phenyl | Vinyl | |
| 1.3.625 | 3-Carbamoyl-5-iod-Phenyl | Vinyl | |
| 1.3.626 | 3-Hydroxy-5-iod-Phenyl- | Vinyl | |
| 1.3.627 | 3-Methoxy-5-iod-Phenyl | Vinyl | |
| 1.3.628 | 3-Ethoxy-5-iod-Phenyl | Vinyl | |
| 1.3.629 | 3-nPropyoxy-5-iod-Phenyl | Vinyl | |
| 1.3.630 | 3-isoPropoxy-5-iod-Phenyl | Vinyl | |
| 1.3.631 | 3-nButoxy-5-iod-Phenyl | Vinyl | |
| 1.3.632 | 3-isoButoxy-5-iod-Phenyl | Vinyl | |
| 1.3.633 | 3-tert.Butoxy-5-iod-Phenyl | Vinyl | |
| 1.3.634 | 3-Difluormethoxy-5-iod-Phenyl | Vinyl | |
| 1.3.635 | 3-Trifluormethoxy-5-iod-Phenyl | Vinyl | |
| 1.3.636 | 3-(2,2,2-Trifluorethoxy)-5-iod-Phenyl | Vinyl | |
| 1.3.637 | 3-(2-Chlorethoxy)-5-iod-Phenyl | Vinyl | |
| 1.3.638 | 3-(2-Hydroxyethoxy)-5-iod-Phenyl- | Vinyl | |
| 1.3.639 | 3-[(tert-Butoxycarbonyl)oxy]-5-iod-Phenyl | Vinyl | |
| 1.3.640 | 3-Nitro-5-iod-Phenyl | Vinyl | |
| 1.3.641 | 3-Acetoxy-5-iod-Phenyl | Vinyl | |
| 1.3.642 | {3-[(tert-Butoxycarbonyl)amino]-5-iod-Phenyl} | Vinyl | |
| 1.3.643 | 3-Methylsulfanyl-5-iod-Phenyl | Vinyl | |
| 1.3.644 | 3,5-Dimethyl-Phenyl | Vinyl | [CDCl₃] 2.34 (s,6H); 3.45 (d,1H); 3.97 (d,1H); 5.42 (d,1H); 5.62 (d,1H); 6.15 (dd,1H); 7.09 (s,1H); 7.26 (s,2H). |
| 1.3.645 | 3-Ethyl-5-methyl-Phenyl | Vinyl | |
| 1.3.646 | 3-Propyl-5-methyl-Phenyl | Vinyl | |
| 1.3.647 | 3-isoPropyl-5-methyl-Phenyl | Vinyl | |
| 1.3.648 | 3-nButyl-5-methyl-Phenyl | Vinyl | |
| 1.3.649 | 3-isoButyl-5-methyl-Phenyl | Vinyl | |
| 1.3.650 | 3-tert.Butyl-5-methyl-Phenyl | Vinyl | |
| 1.3.651 | 3-Cyclopropyl-5-methyl-Phenyl | Vinyl | |
| 1.3.652 | 3-Cyano-5-methyl-Phenyl | Vinyl | |
| 1.3.653 | 3-Trifluormethyl-5-methyl-Phenyl | Vinyl | |
| 1.3.654 | 3-(Methoxycarbony)l-5-methyl-Phenyl | Vinyl | |
| 1.3.655 | 3-Methoxy-5-methyl-Phenyl | Vinyl | |
| 1.3.656 | 3-Ethoxy-5-methyl-Phenyl | Vinyl | |
| 1.3.657 | 3-nPropyoxy-5-methyl-Phenyl | Vinyl | |
| 1.3.658 | 3-nButoxy-5-methyl-Phenyl | Vinyl | |
| 1.3.659 | 3-isoButoxy-5-methyl-Phenyl | Vinyl | |
| 1.3.660 | 3-Difluormethoxy-5-methyl-Phenyl | Vinyl | |
| 1.3.661 | 3-Trifluormethoxy-5-methyl-Phenyl | Vinyl | |
| 1.3.662 | 3-Nitro-5-methyl-Phenyl | Vinyl | |
| 1.3.663 | 3-Acetoxy-5-methyl-Phenyl | Vinyl | |
| 1.3.664 | 3-Methylsulfanyl-5-methyl-Phenyl | Vinyl | |
| 1.3.665 | 3,5-Diethyl-Phenyl | Vinyl | |
| 1.3.666 | 3-Propyl-5-ethyl-Phenyl | Vinyl | |
| 1.3.667 | 3-isoPropyl-5-ethyl-Phenyl | Vinyl | |
| 1.3.668 | 3-nButyl-5-ethyl-Phenyl | Vinyl | |
| 1.3.669 | 3-isoButyl-5-ethyl-Phenyl | Vinyl | |
| 1.3.670 | 3-tert.Butyl-5-ethyl-Phenyl | Vinyl | |
| 1.3.671 | 3-Cyclopropyl-5-ethyl-Phenyl | Vinyl | |
| 1.3.672 | 3-Cyano-5-ethyl-Phenyl | Vinyl | |
| 1.3.673 | 3-Trifluormethyl-5-ethyl-Phenyl | Vinyl | |
| 1.3.674 | 3-(Methoxycarbonyl)-5-ethyl-Phenyl | Vinyl | |
| 1.3.675 | 3-Methoxy-5-ethyl-Phenyl | Vinyl | |
| 1.3.676 | 3-Ethoxy-5-ethyl-Phenyl | Vinyl | |
| 1.3.677 | 3-nPropyoxy-5-ethyl-Phenyl | Vinyl | |
| 1.3.678 | 3-nButoxy-5-ethyl-Phenyl | Vinyl | |
| 1.3.679 | 3-isoButoxy-5-ethyl-Phenyl | Vinyl | |
| 1.3.680 | 3-Difluormethoxy-5-ethyl-Phenyl | Vinyl | |
| 1.3.681 | 3-Trifluormethoxy-5-ethyl-Phenyl | Vinyl | |
| 1.3.682 | 3-Nitro-5-ethyl-Phenyl | Vinyl | |
| 1.3.683 | 3-Acetoxy-5-ethyl-Phenyl | Vinyl | |
| 1.3.684 | 3-Methylsulfanyl-5-ethyl-Phenyl | Vinyl | |
| 1.3.685 | 3,5-Dipropyl-Phenyl | Vinyl | |
| 1.3.686 | 3-isoPropyl-5-propyl-Phenyl | Vinyl | |
| 1.3.687 | 3-nButyl-5-propyl-Phenyl | Vinyl | |
| 1.3.688 | 3-isoButyl-5-propyl-Phenyl | Vinyl | |
| 1.3.689 | 3-tert.Butyl-5-propyl-Phenyl | Vinyl | |
| 1.3.690 | 3-Cyclopropyl-5-propyl-Phenyl | Vinyl | |
| 1.3.691 | 3-Cyano-5-propyl-Phenyl | Vinyl | |
| 1.3.692 | 3-Trifluormethyl-5-propyl-Phenyl | Vinyl | |
| 1.3.693 | 3-(Methoxycarbonyl)-5-propyl-Phenyl | Vinyl | |
| 1.3.694 | 3-Methoxy-5-propyl-Phenyl | Vinyl | |
| 1.3.695 | 3-Ethoxy-5-propyl-Phenyl | Vinyl | |
| 1.3.696 | 3-nPropyoxy-5-propyl-Phenyl | Vinyl | |
| 1.3.697 | 3-nButoxy-5-propyl-Phenyl | Vinyl | |
| 1.3.698 | 3-isoButoxy-5-propyl-Phenyl | Vinyl | |
| 1.3.699 | 3-tert.Butoxy-5-propyl-Phenyl | Vinyl | |
| 1.3.700 | 3-Difluormethoxy-5-propyl-Phenyl | Vinyl | |
| 1.3.701 | 3-Trifluormethoxy-5-ethyl-Phenyl | Vinyl | |
| 1.3.702 | 3-Nitro-5-propyl-Phenyl | Vinyl | |
| 1.3.703 | 3-Acetoxy-5-propyl-Phenyl | Vinyl | |
| 1.3.704 | 3-Methylsulfanyl-5-propyl-Phenyl | Vinyl | |
| 1.3.705 | 3,5-Diisopropyl-Phenyl | Vinyl | |
| 1.3.706 | 3-nButyl-5-isopropyl-Phenyl | Vinyl | |
| 1.3.707 | 3-isoButyl-5-isopropyl-Phenyl | Vinyl | |
| 1.3.708 | 3-tert.Butyl-5-isopropyl-Phenyl | Vinyl | |
| 1.3.709 | 3-Cyclopropyl-5-isopropyl-Phenyl | Vinyl | |
| 1.3.710 | 3-Cyano-5-isopropyl-Phenyl | Vinyl | |
| 1.3.711 | 3-Trifluormethyl-5-isopropyl-Phenyl | Vinyl | |
| 1.3.712 | 3-(Methoxycarbony)l-5-isopropyl-Phenyl | Vinyl | |
| 1.3.713 | 3-Methoxy-5-isopropyl-Phenyl | Vinyl | |
| 1.3.714 | 3-Ethoxy-5-isopropyl-Phenyl | Vinyl | |
| 1.3.715 | 3-nPropyoxy-5-isopropyl-Phenyl | Vinyl | |
| 1.3.716 | 3-nButoxy-5-isopropyl-Phenyl | Vinyl | |
| 1.3.717 | 3-isoButoxy-5-isopropyl-Phenyl | Vinyl | |
| 1.3.718 | 3-Difluormethoxy-5-isopropyl-Phenyl | Vinyl | |
| 1.3.719 | 3-Trifluormethoxy-5-isopropyl-Phenyl | Vinyl | |
| 1.3.720 | 3-Nitro-5-isopropyl-Phenyl | Vinyl | |
| 1.3.721 | 3-Acetoxy-5-isopropyl-Phenyl | Vinyl | |
| 1.3.722 | 3-Methylsulfanyl-5-isopropyl-Phenyl | Vinyl | |
| 1.3.723 | 3,5-Dibutyl-Phenyl | Vinyl | |
| 1.3.724 | 3-isoButyl-5-butyl-Phenyl | Vinyl | |
| 1.3.725 | 3-tert.Butyl-5-butyl-Phenyl | Vinyl | |
| 1.3.726 | 3-Cyclopropyl-5-butyl-Phenyl | Vinyl | |
| 1.3.727 | 3-Cyano-5-butyl-Phenyl | Vinyl | |
| 1.3.728 | 3-Trifluormethyl-5-butyl-Phenyl | Vinyl | |
| 1.3.729 | 3-(Methoxycarbonyl)-5-butyl-Phenyl | Vinyl | |
| 1.3.730 | 3-Methoxy-5-butyl-Phenyl | Vinyl | |
| 1.3.731 | 3-Ethoxy-5-butyl-Phenyl | Vinyl | |
| 1.3.732 | 3-nPropyoxy-5-butyl-Phenyl | Vinyl | |
| 1.3.733 | 3-nButoxy-5-butyl-Phenyl | Vinyl | |
| 1.3.734 | 3-isoButoxy-5-butyl-Phenyl | Vinyl | |
| 1.3.735 | 3-Difluormethoxy-5-butyl-Phenyl | Vinyl | |
| 1.3.736 | 3-Trifluormethoxy-5-butyl-Phenyl | Vinyl | |
| 1.3.737 | 3-Nitro-5-butyl-Phenyl | Vinyl | |
| 1.3.738 | 3-Acetoxy-5-butyl-Phenyl | Vinyl | |
| 1.3.739 | 3-Methylsulfanyl-5-butyl-Phenyl | Vinyl | |
| 1.3.740 | 3,5-Diisobutyl-Phenyl | Vinyl | |
| 1.3.741 | 3-tert.Butyl-5-isobutyl-Phenyl | Vinyl | |
| 1.3.742 | 3-Cyclopropyl-5-isobutyl-Phenyl | Vinyl | |
| 1.3.743 | 3-Cyano-5-isobutyl-Phenyl | Vinyl | |
| 1.3.744 | 3-Trifluormethyl-5-isobutyl-Phenyl | Vinyl | |
| 1.3.745 | 3-(Hydroxycarbonyl)-5-isobutyl-Phenyl | Vinyl | |
| 1.3.746 | 3-(Methoxycarbonyl)-5-isobutyl-Phenyl | Vinyl | |
| 1.3.747 | 3-Methoxy-5-isobutyl-Phenyl | Vinyl | |
| 1.3.748 | 3-Ethoxy-5-isobutyl-Phenyl | Vinyl | |
| 1.3.749 | 3-nPropyoxy-5-isobutyl-Phenyl | Vinyl | |
| 1.3.750 | 3-nButoxy-5-isobutyl-Phenyl | Vinyl | |
| 1.3.751 | 3-isoButoxy-5-isobutyl-Phenyl | Vinyl | |
| 1.3.752 | 3-Difluormethoxy-5-isobutyl-Phenyl | Vinyl | |
| 1.3.753 | 3-Trifluormethoxy-5-isobutyl-Phenyl | Vinyl | |
| 1.3.754 | 3-Nitro-5-isobutyl-Phenyl | Vinyl | |
| 1.3.755 | 3-Acetoxy-5-isobutyl-Phenyl | Vinyl | |
| 1.3.756 | 3-Methylsulfanyl-5-isobutyl-Phenyl | Vinyl | |
| 1.3.757 | 3,5-D(itert.butyl)-Phenyl | Vinyl | |
| 1.3.758 | 3-Cyclopropyl-5-tert.butyl-Phenyl | Vinyl | |
| 1.3.759 | 3-Cyano-5-tert.butyl-Phenyl | Vinyl | |
| 1.3.760 | 3-Trifluormethyl-5-tert.butyl-Phenyl | Vinyl | |
| 1.3.761 | 3-(Methoxycarbonyl)-5-tert.butyl-Phenyl | Vinyl | |
| 1.3.762 | 3-Methoxy-5-tert.butyl-Phenyl | Vinyl | |
| 1.3.763 | 3-Ethoxy-5-tert.butyl-Phenyl | Vinyl | |
| 1.3.764 | 3-nPropyoxy-5-tert.butyl-Phenyl | Vinyl | |
| 1.3.765 | 3-nButoxy-5-tert.butyl-Phenyl | Vinyl | |
| 1.3.766 | 3-isoButoxy-5-tert.butyl-Phenyl | Vinyl | |
| 1.3.767 | 3-tert.Butoxy-5-tert.butyl-Phenyl | Vinyl | |
| 1.3.768 | 3-Difluormethoxy-5-tert.butyl-Phenyl | Vinyl | |
| 1.3.769 | 3-Trifluormethoxy-5-tert.butyl-Phenyl | Vinyl | |
| 1.3.770 | 3-Nitro-5-tert.butyl-Phenyl | Vinyl | |
| 1.3.771 | 3-Acetoxy-5-tert.butyl-Phenyl | Vinyl | |
| 1.3.772 | 3-Methylsulfanyl-5-tert.butyl-Phenyl | Vinyl | |
| 1.3.773 | 3-tert.Butyl-5-cyclopropyl-Phenyl | Vinyl | |
| 1.3.774 | 3,5-Dicyclopropyl-Phenyl | Vinyl | |
| 1.3.775 | 3-Cyano-5-cyclopropyl-Phenyl | Vinyl | |
| 1.3.776 | 3-Trifluormethyl-5-cyclopropyl-Phenyl | Vinyl | |
| 1.3.777 | 3-(Methoxycarbonyl)-5-cyclopropyl-Phenyl | Vinyl | |
| 1.3.778 | 3-Methoxy-5-cyclopropyl-Phenyl | Vinyl | |
| 1.3.779 | 3-Ethoxy-5-cyclopropyl-Phenyl | Vinyl | |
| 1.3.780 | 3-nPropyoxy-5-cyclopropyl-Phenyl | Vinyl | |
| 1.3.781 | 3-nButoxy-5-cyclopropyl-Phenyl | Vinyl | |
| 1.3.782 | 3-isoButoxy-5-cyclopropyl-Phenyl | Vinyl | |
| 1.3.783 | 3-Difluormethoxy-5-cyclopropyl-Phenyl | Vinyl | |
| 1.3.784 | 3-Trifluormethoxy-5-cyclopropyl-Phenyl | Vinyl | |
| 1.3.785 | 3-Nitro-5-cyclopropyl-Phenyl | Vinyl | |
| 1.3.786 | 3-Acetoxy-5-cyclopropyl-Phenyl | Vinyl | |
| 1.3.787 | 3-Methylsulfanyl-5-cyclopropyl-Phenyl | Vinyl | |
| 1.3.788 | 3,5-Dicyano-Phenyl | Vinyl | |
| 1.3.789 | 3-Trifluormethyl-5-cyano-Phenyl | Vinyl | |
| 1.3.790 | 3-(Methoxycarbonyl)-5-cyano-Phenyl | Vinyl | |
| 1.3.791 | 3-Methoxy-5-cyano-Phenyl | Vinyl | |
| 1.3.792 | 3-Ethoxy-5-cyano-Phenyl | Vinyl | |
| 1.3.793 | 3-nPropyoxy-5-cyano-Phenyl | Vinyl | |
| 1.3.794 | 3-nButoxy-5-cyano-Phenyl | Vinyl | |
| 1.3.795 | 3-isoButoxy-5-cyano-Phenyl | Vinyl | |
| 1.3.796 | 3-Difluormethoxy-5-cyano-Phenyl | Vinyl | |
| 1.3.797 | 3-Trifluormethoxy-5-cyano-Phenyl | Vinyl | |
| 1.3.798 | 3-Nitro-5-cyano-Phenyl | Vinyl | |
| 1.3.799 | 3-Acetoxy-5-cyano-Phenyl | Vinyl | |
| 1.3.800 | 3-Methylsulfanyl-5-cyano-Phenyl | Vinyl | |
| 1.3.801 | 3,5-Di(trifluormethyl)-Phenyl | Vinyl | |
| 1.3.802 | 3-(Methoxycarbonyl)-5-trifluormethylPhenyl | Vinyl | |
| 1.3.803 | 3-Methoxy-5-trifluormethyl-Phenyl | Vinyl | |
| 1.3.804 | 3-Ethoxy-5-trifluormethyl-Phenyl | Vinyl | |
| 1.3.805 | 3-nPropyoxy-5-trifluormethyl-Phenyl | Vinyl | |
| 1.3.806 | 3-nButoxy-5-trifluormethyl-Phenyl | Vinyl | |
| 1.3.807 | 3-isoButoxy-5-trifluormethyl-Phenyl | Vinyl | |
| 1.3.808 | 3-Difluormethoxy-5-trifluormethyl-Phenyl | Vinyl | |
| 1.3.809 | 3-Trifluormethoxy-5-trifluormethyl-Phenyl | Vinyl | |
| 1.3.810 | 3-Nitro-5-trifluormethyl-Phenyl | Vinyl | |
| 1.3.811 | 3-Acetoxy-5-trifluormethyl-Phenyl | Vinyl | |
| 1.3.812 | 3-Methylsulfanyl-5-trifluormethyl-Phenyl | Vinyl | |
| 1.3.813 | 3,5-Di(methoxycarbonyl)-Phenyl | Vinyl | |
| 1.3.814 | 3-Methoxy-5-(methoxycarbonyl)-Phenyl | Vinyl | |
| 1.3.815 | 3-Ethoxy-5-(methoxycarbonyl)-Phenyl | Vinyl | |
| 1.3.816 | 3-nPropyoxy-5-(methoxycarbonyl)-Phenyl | Vinyl | |
| 1.3.817 | 3-nButoxy-5-(methoxycarbonyl)-Phenyl | Vinyl | |
| 1.3.818 | 3-isoButoxy-5-(methoxycarbonyl)-Phenyl | Vinyl | |
| 1.3.819 | 3-Difluormethoxy-5-(methoxycarbonyl)-Phenyl | Vinyl | |
| 1.3.820 | 3-Trifluormethoxy-5-(methoxycarbonyl)-Phenyl | Vinyl | |
| 1.3.821 | 3-Nitro-5-(methoxycarbonyl)-Phenyl | Vinyl | |
| 1.3.822 | 3-Acetoxy-5-(methoxycarbonyl)-Phenyl | Vinyl | |
| 1.3.823 | 3-Methylsulfanyl-5-(methoxycarbonyl)-Phenyl | Vinyl | |
| 1.3.824 | 3,5-Dimethoxy-Phenyl | Vinyl | |
| 1.3.825 | 3-Ethoxy-5-methoxy-Phenyl | Vinyl | |
| 1.3.826 | 3-nPropyoxy-5-methoxy-Phenyl | Vinyl | |
| 1.3.827 | 3-nButoxy-5-methoxy-Phenyl | Vinyl | |
| 1.3.828 | 3-isoButoxy-5-methoxy-Phenyl | Vinyl | |
| 1.3.829 | 3-Difluormethoxy-5-methoxy-Phenyl | Vinyl | |
| 1.3.830 | 3-Trifluormethoxy-5-methoxy-Phenyl | Vinyl | |
| 1.3.831 | 3-Nitro-5-methoxy-Phenyl | Vinyl | |
| 1.3.832 | 3-Acetoxy-5-methoxy-Phenyl | Vinyl | |
| 1.3.833 | 3-Methylsulfanyl-5-methoxy-Phenyl | Vinyl | |
| 1.3.834 | 3,5-Diethoxy-Phenyl | Vinyl | |
| 1.3.835 | 3-nPropyoxy-5-ethoxy-Phenyl | Vinyl | |
| 1.3.836 | 3-nButoxy-5-ethoxy-Phenyl | Vinyl | |
| 1.3.837 | 3-isoButoxy-5-ethoxy-Phenyl | Vinyl | |
| 1.3.838 | 3-Difluormethoxy-5-ethoxy-Phenyl | Vinyl | |
| 1.3.839 | 3-Trifluormethoxy-5-ethoxy-Phenyl | Vinyl | |
| 1.3.840 | 3-Nitro-5-ethoxy-Phenyl | Vinyl | |
| 1.3.841 | 3-Acetoxy-5-ethoxy-Phenyl | Vinyl | |
| 1.3.842 | 3-Methylsulfanyl-5-ethoxy-Phenyl | Vinyl | |
| 1.3.843 | 3,5-Di(isopropyoxy)-Phenyl | Vinyl | |
| 1.3.844 | 3-nButoxy-5-isopropoxy-Phenyl | Vinyl | |
| 1.3.845 | 3-isoButoxy-5-isopropoxy-Phenyl | Vinyl | |
| 1.3.846 | 3-Difluormethoxy-5-isopropoxy-Phenyl | Vinyl | |
| 1.3.847 | 3-Trifluormethoxy-5-isopropoxy-Phenyl | Vinyl | |
| 1.3.848 | 3-Nitro-5-isopropoxy-Phenyl | Vinyl | |
| 1.3.849 | 3-Acetoxy-5-isopropoxy-Phenyl | Vinyl | |
| 1.3.850 | 3-Methylsulfanyl-5-isopropoxy-Phenyl | Vinyl | |
| 1.3.851 | 3,5-Di(trifluormethoxy)-Phenyl | Vinyl | |
| 1.3.852 | 3-Nitro-5-trifluormethoxy-Phenyl | Vinyl | |
| 1.3.853 | 3-Acetoxy-5-tert.butoxy-Phenyl | Vinyl | |
| 1.3.854 | 3-Methylsulfanyl-5-trifluormethoxy-Phenyl | Vinyl | |
| 1.3.855 | 3,5-Bis(difluormethoxy)-Phenyl | Vinyl | |
| 1.3.856 | 3,5-Bis(difluormethoxy)-Phenyl | 1-Methyl vinyl | |
| 1.3.857 | 3,5-Bis(difluormethoxy)-Phenyl | Allyl | |
| 1.3.858 | 3,5-Bis(difluormethoxy)-Phenyl | 1-Chlor vinyl | |
| 1.3.859 | 3-Trifluormethoxy-5-difluormethoxy-Phenyl | Vinyl | |
| 1.3.860 | 3-Nitro-5-difluormethoxy-Phenyl | Vinyl | |
| 1.3.861 | 3-Acetoxy-5-difluormethoxy-Phenyl | Vinyl | |
| 1.3.862 | 3-Methylsulfanyl-5-difluormethoxy-Phenyl | Vinyl | |
| 1.3.863 | 3,5-Bis(acetoxy)-Phenyl | Vinyl | |
| 1.3.864 | 3-Methylsulfanyl-5-acetoxy-Phenyl | Vinyl | |
| 1.3.865 | 3-Acetoxy-5-nitro-Phenyl | Vinyl | |
| 1.3.866 | 3-Methylsulfanyl-5-nitro-Phenyl | Vinyl | |
| 1.3.867 | 3,5-Di(methylsulfanyl)-Phenyl | Vinyl | |
| 1.3.868 | 3,4-Difluor-Phenyl | Vinyl | |
| 1.3.869 | 3,4-Difluor-Phenyl | 1-Methyl vinyl | |
| 1.3.870 | 3,4-Difluor-Phenyl | Allyl | |
| 1.3.871 | 3,4-Difluor-Phenyl | 1-Chlor vinyl | |
| 1.3.872 | 3,4-Difluor-Phenyl | Ethinyl | |
| 1.3.873 | 3-Chlor-4-fluor-Phenyl | Vinyl | |
| 1.3.874 | 3-Chlor-4-fluor-Phenyl | 1-Methyl vinyl | |
| 1.3.875 | 3-Chlor-4-fluor-Phenyl | Allyl | |
| 1.3.876 | 3-Chlor-4-fluor-Phenyl | 1-Chlor vinyl | |
| 1.3.877 | 3-Chlor-4-fluor-Phenyl | Ethinyl | |
| 1.3.878 | 3-Brom-4-fluor-Phenyl | Vinyl | |
| 1.3.879 | 3-Methyl-4-fluor-Phenyl | Vinyl | |
| 1.3.880 | 3-Methyl-4-fluor-Phenyl | 1-Methyl vinyl | |
| 1.3.881 | 3-Cyclopropyl-4-fluor-Phenyl | Vinyl | |
| 1.3.882 | 3-Cyano-4-fluor-Phenyl | Vinyl | |
| 1.3.883 | 3-Methoxy-4-fluor-Phenyl | Vinyl | |
| 1.3.884 | 3-Ethoxy-4-fluor-Phenyl | Vinyl | |
| 1.3.885 | 3-Trifluormethoxy-4-fluor-Phenyl | Vinyl | |
| 1.3.886 | 3-Nitro-4-fluor-Phenyl | Vinyl | |
| 1.3.887 | 3-Fluor-4-chlor-Phenyl | Vinyl | |
| 1.3.888 | 3,4-Dichlor-Phenyl | Vinyl | |
| 1.3.889 | 3-Brom-4-chlor-Phenyl | Vinyl | |
| 1.3.890 | 3-Methyl-4-chlor-Phenyl | Vinyl | |
| 1.3.891 | 3-Ethyl-4-chlor-Phenyl | Vinyl | |
| 1.3.892 | 3-Cyclopropyl-4-chlor-Phenyl | Vinyl | |
| 1.3.893 | 3-Cyano-4-chlor-Phenyl | Vinyl | |
| 1.3.894 | 3-Trifluormethyl-4-chlor-Phenyl | Vinyl | |
| 1.3.895 | 3-Methoxy-4-chlor-Phenyl | Vinyl | |
| 1.3.896 | 3-Ethoxy-4-chlor-Phenyl | Vinyl | |
| 1.3.897 | 3-Trifluormethoxy-4-chlor-Phenyl | Vinyl | |
| 1.3.898 | 3-Nitro-4-chlor-Phenyl | Vinyl | |
| 1.3.899 | 3-Fluor-4-brom-Phenyl | Vinyl | |
| 1.3.900 | 3-Chlor-4-brom-Phenyl | Vinyl | |
| 1.3.901 | 3,4-Dibrom-Phenyl | Vinyl | |
| 1.3.902 | 3-Methyl-4-brom-Phenyl | Vinyl | |
| 1.3.903 | 3-Ethyl-4-brom-Phenyl | Vinyl | |
| 1.3.904 | 3-Cyclopropyl-4-brom-Phenyl | Vinyl | |
| 1.3.905 | 3-Cyano-4-brom-Phenyl | Vinyl | |
| 1.3.906 | 3-Trifluormethyl-4-brom-Phenyl | Vinyl | |
| 1.3.907 | 3-Methoxy-4-Phenyl | Vinyl | |
| 1.3.908 | 3-Ethoxy-4-brom-Phenyl | Vinyl | |
| 1.3.909 | 3-Trifluormethoxy-4-brom-Phenyl | Vinyl | |
| 1.3.910 | 3-Nitro-4-brom-Phenyl | Vinyl | |
| 1.3.911 | 3-Fluor-4-iod-Phenyl | Vinyl | |
| 1.3.912 | 3-Chlor-4-iod-Phenyl | Vinyl | |
| 1.3.913 | 3-Brom-4-iod-Phenyl | Vinyl | |
| 1.3.914 | 3-Methyl-4-iod-Phenyl | Vinyl | |
| 1.3.915 | 3-Cyclopropyl-4-iod-Phenyl | Vinyl | |
| 1.3.916 | 3-Cyano-4-iod-Phenyl | Vinyl | |
| 1.3.917 | 3-Trifluormethyl-4-iod-Phenyl | Vinyl | |
| 1.3.918 | 3-Methoxy-4-iod-Phenyl | Vinyl | |
| 1.3.919 | 3-Ethoxy-4-iod-Phenyl | Vinyl | |
| 1.3.920 | 3-Trifluormethoxy-4-iod-Phenyl | Vinyl | |
| 1.3.921 | 3-Nitro-4-iod-Phenyl | Vinyl | |
| 1.3.922 | 3-Fluor-4-methyl-Phenyl | Vinyl | |
| 1.3.923 | 3-Chlor-4-methyl-Phenyl | Vinyl | |
| 1.3.924 | 3-Brom-4-methyl-Phenyl | Vinyl | |
| 1.3.925 | 3.4-Dimethyl-Phenyl | Vinyl | |
| 1.3.926 | 3.4-Dimethyl-Phenyl | 1-Methyl vinyl | |
| 1.3.927 | 3.4-Dimethyl-Phenyl | Allyl | |
| 1.3.928 | 3.4-Dimethyl-Phenyl | 1-Chlor vinyl | |
| 1.3.929 | 3.4-Dimethyl-Phenyl | Ethinyl | |
| 1.3.930 | 3-Ethyl-4-methyl-Phenyl | Vinyl | |
| 1.3.931 | 3-Cyclopropyl-4-methyl-Phenyl | Vinyl | |
| 1.3.932 | 3-Cyano-4-methyl-Phenyl | Vinyl | |
| 1.3.933 | 3-Trifluormethyl-4-methyl-Phenyl | Vinyl | |
| 1.3.934 | 3-Methoxy-4-methyl-Phenyl | Vinyl | |
| 1.3.935 | 3-Ethoxy-4-methyl-Phenyl | Vinyl | |
| 1.3.936 | 3-Trifluormethoxy-4-methyl-Phenyl | Vinyl | |
| 1.3.937 | 3-Nitro-4-methyl-Phenyl | Vinyl | |
| 1.3.938 | 3-Fluor-4-ethyl-Phenyl | Vinyl | |
| 1.3.939 | 3-Chlor-4-ethyl-Phenyl | Vinyl | |
| 1.3.940 | 3-Brom-4-ethyl-Phenyl | Vinyl | |
| 1.3.941 | 3-Methyl-4-ethyl-Phenyl | Vinyl | |
| 1.3.942 | 3,4-Diethyl-Phenyl | Vinyl | |
| 1.3.943 | 3-Cyclopropyl-4-ethyl-Phenyl | Vinyl | |
| 1.3.944 | 3-Cyano-4-ethyl-Phenyl | Vinyl | |
| 1.3.945 | 3-Trifluormethyl-4-ethyl-Phenyl | Vinyl | |
| 1.3.946 | 3-Methoxy-4-ethyl-Phenyl | Vinyl | |
| 1.3.947 | 3-Ethoxy-4-ethyl-Phenyl | Vinyl | |
| 1.3.948 | 3-Trifluormethoxy-4-ethyl-Phenyl | Vinyl | |
| 1.3.949 | 3-Nitro-4-ethyl-Phenyl | Vinyl | |
| 1.3.950 | 3-Fluor-4-propyl-Phenyl | Vinyl | |
| 1.3.951 | 3-Chlor-4-propyl-Phenyl | Vinyl | |
| 1.3.952 | 3-Brom-4-propyl-Phenyl | Vinyl | |
| 1.3.953 | 3-Methyl-4-propyl-Phenyl | Vinyl | |
| 1.3.954 | 3-Methyl-4-propyl-Phenyl | Vinyl | |
| 1.3.955 | 3-Cyclopropyl-4-propyl-Phenyl | Vinyl | |
| 1.3.956 | 3-Cyano-4-propyl-Phenyl | Vinyl | |
| 1.3.957 | 3-Trifluormethyl-4-propyl-Phenyl | Vinyl | |
| 1.3.958 | 3-Methoxy-4-propyl-Phenyl | Vinyl | |
| 1.3.959 | 3-Ethoxy-4-propyl-Phenyl | Vinyl | |
| 1.3.960 | 3-Trifluormethoxy-4-propyl-Phenyl | Vinyl | |
| 1.3.961 | 3-Nitro-4-propyl-Phenyl | Vinyl | |
| 1.3.962 | 3-Fluor-4-isopropyl-Phenyl | Vinyl | |
| 1.3.963 | 3-Chlor-4-isopropyl-Phenyl | Vinyl | |
| 1.3.964 | 3-Brom-4-isopropyl-Phenyl | Vinyl | |
| 1.3.965 | 3-Methyl-4-isopropyl-Phenyl | Vinyl | |
| 1.3.966 | 3-Cyclopropyl-4-isopropyl-Phenyl | Vinyl | |
| 1.3.967 | 3-Cyano-4-isopropyl-Phenyl | Vinyl | |
| 1.3.968 | 3-Trifluormethyl-4-isopropyl-Phenyl | Vinyl | |
| 1.3.969 | 3-Methoxy-4-isopropyl-Phenyl | Vinyl | |
| 1.3.970 | 3-Ethoxy-4-isopropyl-Phenyl | Vinyl | |
| 1.3.971 | 3-Trifluormethoxy-4-isopropyl-Phenyl | Vinyl | |
| 1.3.972 | 3-Nitro-4-isopropyl-Phenyl | Vinyl | |
| 1.3.973 | 3-Fluor-4-tert.butyl-Phenyl | Vinyl | |
| 1.3.974 | 3-Chlor-4-tert.buty-Phenyl | Vinyl | |
| 1.3.975 | 3-Brom-4-tert.butyl-Phenyl | Vinyl | |
| 1.3.976 | 3-Methyl-4-tert.butyl-Phenyl | Vinyl | |
| 1.3.977 | 3-Cyclopropyl-4-tert.butyl-Phenyl | Vinyl | |
| 1.3.978 | 3-Cyano-4-tert.butyl-Phenyl | Vinyl | |
| 1.3.979 | 3-Trifluormethyl-4-tert.butyl-Phenyl | Vinyl | |
| 1.3.980 | 3-Trifluormethyl-4-tert.butyl-Phenyl | 1-Methyl vinyl | |
| 1.3.981 | 3-Trifluormethyl-4-tert.butyl-Phenyl | Allyl | |
| 1.3.982 | 3-Trifluormethyl-4-tert.butyl-Phenyl | 1-Chlor vinyl | |
| 1.3.983 | 3-Trifluormethyl-4-tert.butyl-Phenyl | Ethinyl | |
| 1.3.984 | 3-Methoxy-4-tert.butyl-Phenyl | Vinyl | |
| 1.3.985 | 3-Ethoxy-4-tert.butyl-Phenyl | Vinyl | |
| 1.3.986 | 3-Trifluormethoxy-4-tert.butyl-Phenyl | Vinyl | |
| 1.3.987 | 3-Nitro-4-tert.butyl-Phenyl | Vinyl | |
| 1.3.988 | 3-Fluor-4-cyclopropyl-Phenyl | Vinyl | |
| 1.3.989 | 3-Chlor-4-cyclopropyl-Phenyl | Vinyl | |
| 1.3.990 | 3-Brom-4-cyclopropyl-Phenyl | Vinyl | |
| 1.3.991 | 3-Methyl-4-cyclopropyl-Phenyl | Vinyl | |
| 1.3.992 | 3-Cyclopropyl-4-cyclopropyl-Phenyl | Vinyl | |
| 1.3.993 | 3-Cyano-4-cyclopropyl-Phenyl | Vinyl | |
| 1.3.994 | 3-Trifluormethyl-4-cyclopropyl-Phenyl | Vinyl | |
| 1.3.995 | 3-Methoxy-4-cyclopropyl-Phenyl | Vinyl | |
| 1.3.996 | 3-Ethoxy-4-cyclopropyl-Phenyl | Vinyl | |
| 1.3.997 | 3-Trifluormethoxy-4-cyclopropyl-Phenyl | Vinyl | |
| 1.3.998 | 3-Fluor-4-methoxycarbonyl-Phenyl | Vinyl | |
| 1.3.999 | 3-Chlor-4-methoxycarbonyl-Phenyl | Vinyl | |
| 1.3.1000 | 3-Brom-4-methoxycarbonyl-Phenyl | Vinyl | |
| 1.3.1001 | 3-Methyl-4-methoxycarbonyl-Phenyl | Vinyl | |
| 1.3.1002 | 3-Cyclopropyl-4-methoxycarbonyl-Phenyl | Vinyl | |
| 1.3.1003 | 3-Cyano-4-methoxycarbonyl-Phenyl | Vinyl | |
| 1.3.1004 | 3-Trifluormethyl-4-methoxycarbonyl-Phenyl | Vinyl | |
| 1.3.1005 | 3-Methoxy-4-methoxycarbonyl-Phenyl | Vinyl | |
| 1.3.1006 | 3-Ethoxy-4-methoxycarbonyl-Phenyl | Vinyl | |
| 1.3.1007 | 3-Trifluormethoxy-4-methoxycarbonyl-Phenyl | Vinyl | |
| 1.3.1008 | 3-Nitro-4-methoxycarbonyl-Phenyl | Vinyl | |
| 1.3.1009 | 3-Fluor-4-cyano-Phenyl | Vinyl | |
| 1.3.1010 | 3-Chlor-4-cyano-Phenyl | Vinyl | |
| 1.3.1011 | 3-Brom-4-cyano-Phenyl | Vinyl | |
| 1.3.1012 | 3-Methyl-4-cyano-Phenyl | Vinyl | |
| 1.3.1013 | 3-Cyclopropyl-4-cyano-Phenyl | Vinyl | |
| 1.3.1014 | 3,4-Dicyano-Phenyl | Vinyl | |
| 1.3.1015 | 3-Trifluormethyl-4-cyano-Phenyl | Vinyl | |
| 1.3.1016 | 3-Trifluormethyl-4-cyano-Phenyl | 1-Methyl vinyl | |
| 1.3.1017 | 3-Trifluormethyl-4-cyano-Phenyl | Allyl | |
| 1.3.1018 | 3-Trifluormethyl-4-cyano-Phenyl | 1-Chlor vinyl | |
| 1.3.1019 | 3-Trifluormethyl-4-cyano-Phenyl | Ethinyl | |
| 1.3.1020 | 3-Methoxy-4-cyano-Phenyl | Vinyl | |
| 1.3.1021 | 3-Ethoxy-4-cyano-Phenyl | Vinyl | |
| 1.3.1022 | 3-Trifluormethoxy-4-cyano-Phenyl | Vinyl | |
| 1.3.1023 | 3-Nitro-4-cyano-Phenyl | Vinyl | |
| 1.3.1024 | 3-Fluor-4-methoxy-Phenyl | Vinyl | |
| 1.3.1025 | 3-Chlor-4-methoxy-Phenyl | Vinyl | |
| 1.3.1026 | 3-Brom-4-methoxy-Phenyl | Vinyl | |
| 1.3.1027 | 3-Methyl-4-methoxy-Phenyl | Vinyl | |
| 1.3.1028 | 3-Cyclopropyl-4-methoxy-Phenyl | Vinyl | |
| 1.3.1029 | 3-Cyano-4-methoxy-Phenyl | Vinyl | |
| 1.3.1030 | 3-Trifluormethyl-4-methoxy-Phenyl | Vinyl | |
| 1.3.1031 | 3,4-Dimethoxy-Phenyl | Vinyl | |
| 1.3.1032 | 3-Ethoxy-4-methoxy-Phenyl | Vinyl | |
| 1.3.1033 | 3-Trifluormethoxy-4-methoxy-Phenyl | Vinyl | |
| 1.3.1034 | 3-Nitro-4-methoxy-Phenyl | Vinyl | |
| 1.3.1035 | 3-Fluor-4-ethoxy-Phenyl | Vinyl | |
| 1.3.1036 | 3-Chlor-4-ethoxy-Phenyl | Vinyl | |
| 1.3.1037 | 3-Chlor-4-ethoxy-Phenyl | 1-Methyl vinyl | |
| 1.3.1038 | 3-Chlor-4-ethoxy-Phenyl | Allyl | |
| 1.3.1039 | 3-Chlor-4-ethoxy-Phenyl | 1-Chlor vinyl | |
| 1.3.1040 | 3-Chlor-4-ethoxy-Phenyl | Ethinyl | |
| 1.3.1041 | 3-Brom-4-ethoxy-Phenyl | Vinyl | |
| 1.3.1042 | 3-Methyl-4-ethoxy-Phenyl | Vinyl | |
| 1.3.1043 | 3-Cyclopropyl-4-ethoxy-Phenyl | Vinyl | |
| 1.3.1044 | 3-Cyano-4-ethoxy-Phenyl | Vinyl | |
| 1.3.1045 | 3-Trifluormethyl-4-ethoxy-Phenyl | Vinyl | |
| 1.3.1046 | 3-Methoxy-4-ethoxy-Phenyl | Vinyl | |
| 1.3.1047 | 2,4-Diethoxy-Phenyl | Vinyl | |
| 1.3.1048 | 3-Trifluormethoxy-4-ethoxy-Phenyl | Vinyl | |
| 1.3.1049 | 3-Nitro-4-ethoxy-Phenyl | Vinyl | |
| 1.3.1050 | 3-Fluor-4-propoxy-Phenyl | Vinyl | |
| 1.3.1051 | 3-Chlor-4-propoxy-Phenyl | Vinyl | |
| 1.3.1052 | 3-Brom-4-propoxy-Phenyl | Vinyl | |
| 1.3.1053 | 3-Methyl-4-propoxy-Phenyl | Vinyl | |
| 1.3.1054 | 3-Cyclopropyl-4-propoxy-Phenyl | Vinyl | |
| 1.3.1055 | 3-Cyano-4-propoxy-Phenyl | Vinyl | |
| 1.3.1056 | 3-Trifluormethyl-4-propoxy-Phenyl | Vinyl | |
| 1.3.1057 | 3-Methoxy-4-propoxy-Phenyl | Vinyl | |
| 1.3.1058 | 3-Ethoxy-4-propoxy-Phenyl | Vinyl | |
| 1.3.1059 | 3-Trifluormethoxy-4-propoxy-Phenyl | Vinyl | |
| 1.3.1060 | 3-Nitro-4-propoxy-Phenyl | Vinyl | |
| 1.3.1061 | 3-Fluor-4-isopropoxy-Phenyl | Vinyl | |
| 1.3.1062 | 3-Chlor-4-isopropoxy-Phenyl | Vinyl | |
| 1.3.1063 | 3-Brom-4-isopropoxy-Phenyl | Vinyl | |
| 1.3.1064 | 3-Methyl-4-isopropoxy-Phenyl | Vinyl | |
| 1.3.1065 | 3-Cyclopropyl-4-isopropoxy-Phenyl | Vinyl | |
| 1.3.1066 | 3-Cyano-4-isopropoxy-Phenyl | Vinyl | |
| 1.3.1067 | 3-Trifluormethyl-4-isopropoxy-Phenyl | Vinyl | |
| 1.3.1068 | 3-Methoxy-4-isopropoxy-Phenyl | Vinyl | |
| 1.3.1069 | 3-Ethoxy-4-isopropoxy-Phenyl | Vinyl | |
| 1.3.1070 | 3-Trifluormethoxy-4-isopropoxy-Phenyl | Vinyl | |
| 1.3.1071 | 3-Nitro-4-isopropoxy-Phenyl | Vinyl | |
| 1.3.1072 | 3-Fluor-4-trifluormethoxy-Phenyl | Vinyl | |
| 1.3.1073 | 3-Chlor-4-trifluormethoxy-Phenyl | Vinyl | |
| 1.3.1074 | 3-Brom-4-trifluormethoxy-Phenyl | Vinyl | |
| 1.3.1075 | 3-Methyl-4-trifluormethoxy-Phenyl | Vinyl | |
| 1.3.1076 | 3-Cyclopropyl-4-trifluormethoxy-Phenyl | Vinyl | |
| 1.3.1077 | 3-Cyano-4-trifluormethoxy-Phenyl | Vinyl | |
| 1.3.1078 | 3-Trifluormethyl-4-trifluormethoxy-Phenyl | Vinyl | |
| 1.3.1079 | 3-Methoxy-4-trifluormethoxy-Phenyl | Vinyl | |
| 1.3.1080 | 3-Ethoxy-4-trifluormethoxy-Phenyl | Vinyl | |
| 1.3.1081 | 3,4-Bis(trifluormethoxy)-Phenyl | Vinyl | |
| 1.3.1082 | 3-Nitro-4-trifluormethoxy-Phenyl | Vinyl | |
| 1.3.1083 | 3-Fluor-4-difluormethoxy-Phenyl | Vinyl | |
| 1.3.1084 | 3-Chlor-4-difluormethoxy-Phenyl | Vinyl | |
| 1.3.1085 | 3-Brom-4-difluormethoxy-Phenyl | Vinyl | |
| 1.3.1086 | 3-Methyl-4-difluormethoxy-Phenyl | Vinyl | |
| 1.3.1087 | 3-Cyclopropyl-4-difluormethoxy-Phenyl | Vinyl | |
| 1.3.1088 | 3-Cyano-4-difluormethoxy-Phenyl | Vinyl | |
| 1.3.1089 | 3-Trifluormethyl-4-difluormethoxy-Phenyl | Vinyl | |
| 1.3.1090 | 3-Methoxy-4-difluormethoxy-Phenyl | Vinyl | |
| 1.3.1091 | 3-Ethoxy-4-difluormethoxy-Phenyl | Vinyl | |
| 1.3.1092 | 3-Trifluormethoxy-4-difluormethoxy-Phenyl | Vinyl | |
| 1.3.1093 | 3-Nitro-4-difluormethoxy-Phenyl | Vinyl | |
| 1.3.1094 | 3-Fluor-4-nitro-Phenyl | Vinyl | |
| 1.3.1095 | 3-Chlor-4-nitro-Phenyl | Vinyl | |
| 1.3.1096 | 3-Brom-4-nitro-Phenyl | Vinyl | |
| 1.3.1097 | 3-Methyl-4-nitro-Phenyl | Vinyl | |
| 1.3.1098 | 3-Cyclopropyl-4-nitro-Phenyl | Vinyl | |
| 1.3.1099 | 3-Cyano-4-nitro-Phenyl | Vinyl | |
| 1.3.1100 | 3-Trifluormethyl-4-nitro-Phenyl | Vinyl | |
| 1.3.1101 | 3-Methoxy-4-nitro-Phenyl | Vinyl | |
| 1.3.1102 | 3-Ethoxy-4-nitro-Phenyl | Vinyl | |
| 1.3.1103 | 3-Trifluormethoxy-4-nitro-Phenyl | Vinyl | |
| 1.3.1104 | 3-Fluor-4-methylsulfanyl-Phenyl | Vinyl | |
| 1.3.1105 | 3-Chlor-4-methylsulfanyl-Phenyl | Vinyl | |
| 1.3.1106 | 3-Brom-4-methylsulfanyl-Phenyl | Vinyl | |
| 1.3.1107 | 3-Methyl-4-methylsulfanyl-Phenyl | Vinyl | |
| 1.3.1108 | 3-Cyclopropyl-4-methylsulfanyl-Phenyl | Vinyl | |
| 1.3.1109 | 3-Cyano-4-methylsulfanyl-Phenyl | Vinyl | |
| 1.3.1110 | 3-Trifluormethyl-4-methylsulfanyl-Phenyl | Vinyl | |
| 1.3.1111 | 3-Methoxy-4-methylsulfanyl-Phenyl | Vinyl | |
| 1.3.1112 | 3-Ethoxy-4-methylsulfanyl-Phenyl | Vinyl | |
| 1.3.1113 | 3-Trifluormethoxy-4-methylsulfanyl-Phenyl | Vinyl | |
| 1.3.1114 | 3-Nitro-4-methylsulfanyl-Phenyl | Vinyl | |
| 1.3.1115 | 3,6-Difluor-Phenyl | Vinyl | |
| 1.3.1116 | 3,6-Difluor-Phenyl | 1-Methyl vinyl | |
| 1.3.1117 | 3,6-Difluor-Phenyl | Allyl | |
| 1.3.1118 | 3,6-Difluor-Phenyl | 1-Chlor vinyl | |
| 1.3.1119 | 3,6-Difluor-Phenyl | Ethinyl | |
| 1.3.1120 | 3-Chlor-6-fluor-Phenyl | Vinyl | |
| 1.3.1121 | 3-Brom-6-fluor-Phenyl | Vinyl | |
| 1.3.1122 | 3-Methyl-6-fluor-Phenyl | Vinyl | |
| 1.3.1123 | 3-Ethyl-6-fluor-Phenyl | Vinyl | |
| 1.3.1124 | 3-Cyclopropyl-6-fluor-Phenyl | Vinyl | |
| 1.3.1125 | 3-Cyano-6-fluor-Phenyl | Vinyl | |
| 1.3.1126 | 3-Methoxy-6-fluor-Phenyl | Vinyl | |
| 1.3.1127 | 3-Ethoxy-6-fluor-Phenyl | Vinyl | |
| 1.3.1128 | 3-Trifluormethoxy-6-fluor-Phenyl | Vinyl | |
| 1.3.1129 | 3-Nitro-6-fluor-Phenyl | Vinyl | |
| 1.3.1130 | 3-Fluor-6-chlor-Phenyl | Vinyl | |
| 1.3.1131 | 3-Fluor-6-chlor-Phenyl | 1-Methyl vinyl | |
| 1.3.1132 | 3-Fluor-6-chlor-Phenyl | Allyl | |
| 1.3.1133 | 3-Fluor-6-chlor-Phenyl | 1-Chlor vinyl | |
| 1.3.1134 | 3-Fluor-6-chlor-Phenyl | Ethinyl | |
| 1.3.1135 | 3,6-Dichlor-Phenyl | Vinyl | |
| 1.3.1136 | 3,6-Dichlor-Phenyl | 1-Methyl vinyl | |
| 1.3.1137 | 3,6-Dichlor-Phenyl | Allyl | |
| 1.3.1138 | 3,6-Dichlor-Phenyl | 1-Chlor vinyl | |
| 1.3.1139 | 3,6-Dichlor-Phenyl | Ethinyl | |
| 1.3.1140 | 3-Brom-6-chlor-Phenyl | Vinyl | |
| 1.3.1141 | 3-Methyl-6-chlor-Phenyl | Vinyl | |
| 1.3.1142 | 3-Cyclopropyl-6-chlor-Phenyl | Vinyl | |
| 1.3.1143 | 3-Cyano-6-chlor-Phenyl | Vinyl | |
| 1.3.1144 | 3-Trifluormethyl-6-chlor-Phenyl | Vinyl | |
| 1.3.1145 | 3-Methoxy-6-chlor-Phenyl | Vinyl | |
| 1.3.1146 | 3-Ethoxy-6-chlor-Phenyl | Vinyl | |
| 1.3.1147 | 3-Trifluormethoxy-6-chlor-Phenyl | Vinyl | |
| 1.3.1148 | 3-Nitro-6-chlor-Phenyl | Vinyl | |
| 1.3.1149 | 3-Fluor-6-brom-Phenyl | Vinyl | |
| 1.3.1150 | 3-Chlor-6-brom-Phenyl | Vinyl | |
| 1.3.1151 | 3,6-Dibrom-Phenyl | Vinyl | |
| 1.3.1152 | 3-Methyl-6-brom-Phenyl | Vinyl | |
| 1.3.1153 | 3-Cyclopropyl-6-brom-Phenyl | Vinyl | |
| 1.3.1154 | 3-Cyano-6-brom-Phenyl | Vinyl | |
| 1.3.1155 | 3-Trifluormethyl-6-brom-Phenyl | Vinyl | |
| 1.3.1156 | 3-Methoxy-6-Phenyl | Vinyl | |
| 1.3.1157 | 3-Ethoxy-6-brom-Phenyl | Vinyl | |
| 1.3.1158 | 3-Trifluormethoxy-6-brom-Phenyl | Vinyl | |
| 1.3.1159 | 3-Nitro-6-brom-Phenyl | Vinyl | |
| 1.3.1160 | 3-Fluor-6-iod-Phenyl | Vinyl | |
| 1.3.1161 | 3-Chlor-6-iod-Phenyl | Vinyl | |
| 1.3.1162 | 3-Brom-6-iod-Phenyl | Vinyl | |
| 1.3.1163 | 3-Methyl-6-iod-Phenyl | Vinyl | |
| 1.3.1164 | 3-Cyclopropyl-6-iod-Phenyl | Vinyl | |
| 1.3.1165 | 3-Cyano-6-iod-Phenyl | Vinyl | |
| 1.3.1166 | 3-Trifluormethyl-6-iod-Phenyl | Vinyl | |
| 1.3.1167 | 3-Methoxy-6-iod-Phenyl | Vinyl | |
| 1.3.1168 | 3-Ethoxy-6-iod-Phenyl | Vinyl | |
| 1.3.1169 | 3-Trifluormethoxy-6-iod-Phenyl | Vinyl | |
| 1.3.1170 | 3-Nitro-6-iod-Phenyl | Vinyl | |
| 1.3.1171 | 3-Fluor-6-methyl-Phenyl | Vinyl | |
| 1.3.1172 | 3-Chlor-6-methyl-Phenyl | Vinyl | |
| 1.3.1173 | 3-Brom-6-methyl-Phenyl | Vinyl | |
| 1.3.1174 | 3.6-Dimethyl-Phenyl | Vinyl | |
| 1.3.1175 | 3-Ethyl-6-methyl-Phenyl | Vinyl | |
| 1.3.1176 | 3-Cyclopropyl-6-methyl-Phenyl | Vinyl | |
| 1.3.1177 | 3-Cyano-6-methyl-Phenyl | Vinyl | |
| 1.3.1178 | 3-Trifluormethyl-6-methyl-Phenyl | Vinyl | |
| 1.3.1179 | 3-Methoxy-6-methyl-Phenyl | Vinyl | |
| 1.3.1180 | 3-Ethoxy-6-methyl-Phenyl | Vinyl | |
| 1.3.1181 | 3-Trifluormethoxy-6-methyl-Phenyl | Vinyl | |
| 1.3.1182 | 3-Nitro-6-methyl-Phenyl | Vinyl | |
| 1.3.1183 | 3-Fluor-6-ethyl-Phenyl | Vinyl | |
| 1.3.1184 | 3-Chlor-6-ethyl-Phenyl | Vinyl | |
| 1.3.1185 | 3-Brom-6-ethyl-Phenyl | Vinyl | |
| 1.3.1186 | 3-Methyl-6-ethyl-Phenyl | Vinyl | |
| 1.3.1187 | 3,6-Diethyl-Phenyl | Vinyl | |
| 1.3.1188 | 3-Cyclopropyl-6-ethyl-Phenyl | Vinyl | |
| 1.3.1189 | 3-Cyano-6-ethyl-Phenyl | Vinyl | |
| 1.3.1190 | 3-Trifluormethyl-6-ethyl-Phenyl | Vinyl | |
| 1.3.1191 | 3-Methoxy-6-ethyl-Phenyl | Vinyl | |
| 1.3.1192 | 3-Ethoxy-6-ethyl-Phenyl | Vinyl | |
| 1.3.1193 | 3-Trifluormethoxy-6-ethyl-Phenyl | Vinyl | |
| 1.3.1194 | 3-Nitro-6-ethyl-Phenyl | Vinyl | |
| 1.3.1195 | 3-Fluor-6-propyl-Phenyl | Vinyl | |
| 1.3.1196 | 3-Chlor-6-propyl-Phenyl | Vinyl | |
| 1.3.1197 | 3-Brom-6-propyl-Phenyl | Vinyl | |
| 1.3.1198 | 3-Methyl-6-propyl-Phenyl | Vinyl | |
| 1.3.1199 | 3-Methyl-6-propyl-Phenyl | Vinyl | |
| 1.3.1200 | 3-Cyclopropyl-6-propyl-Phenyl | Vinyl | |
| 1.3.1201 | 3-Cyano-6-propyl-Phenyl | Vinyl | |
| 1.3.1202 | 3-Trifluormethyl-6-propyl-Phenyl | Vinyl | |
| 1.3.1203 | 3-Methoxy-6-propyl-Phenyl | Vinyl | |
| 1.3.1204 | 3-Ethoxy-6-propyl-Phenyl | Vinyl | |
| 1.3.1205 | 3-Trifluormethoxy-6-propyl-Phenyl | Vinyl | |
| 1.3.1206 | 3-Nitro-6-propyl-Phenyl | Vinyl | |
| 1.3.1207 | 3-Fluor-6-isopropyl-Phenyl | Vinyl | |
| 1.3.1208 | 3-Chlor-6-isopropyl-Phenyl | Vinyl | |
| 1.3.1209 | 3-Brom-6-isopropyl-Phenyl | Vinyl | |
| 1.3.1210 | 3-Methyl-6-isopropyl-Phenyl | Vinyl | |
| 1.3.1211 | 3-Cyclopropyl-6-isopropyl-Phenyl | Vinyl | |
| 1.3.1212 | 3-Cyano-6-isopropyl-Phenyl | Vinyl | |
| 1.3.1213 | 3-Trifluormethyl-6-isopropyl-Phenyl | Vinyl | |
| 1.3.1214 | 3-Methoxy-6-isopropyl-Phenyl | Vinyl | |
| 1.3.1215 | 3-Ethoxy-6-isopropyl-Phenyl | Vinyl | |
| 1.3.1216 | 3-Trifluormethoxy-6-isopropyl-Phenyl | Vinyl | |
| 1.3.1217 | 3-Nitro-6-isopropyl-Phenyl | Vinyl | |
| 1.3.1218 | 3-Fluor-6-tert.butyl-Phenyl | Vinyl | |
| 1.3.1219 | 3-Chlor-6-tert.buty-Phenyl | Vinyl | |
| 1.3.1220 | 3-Brom-6-tert.butyl-Phenyl | Vinyl | |
| 1.3.1221 | 3-Methyl-6-tert.butyl-Phenyl | Vinyl | |
| 1.3.1222 | 3-Cyclopropyl-6-tert.butyl-Phenyl | Vinyl | |
| 1.3.1223 | 3-Cyano-6-tert.butyl-Phenyl | Vinyl | |
| 1.3.1224 | 3-Trifluormethyl-6-tert.butyl-Phenyl | Vinyl | |
| 1.3.1225 | 3-Methoxy-6-tert.butyl-Phenyl | Vinyl | |
| 1.3.1226 | 3-Ethoxy-6-tert.butyl-Phenyl | Vinyl | |
| 1.3.1227 | 3-Trifluormethoxy-6-tert.butyl-Phenyl | Vinyl | |
| 1.3.1228 | 3-Nitro-6-tert.butyl-Phenyl | Vinyl | |
| 1.3.1229 | 3-Fluor-6-cyclopropyl-Phenyl | Vinyl | |
| 1.3.1230 | 3-Chlor-6-cyclopropyl-Phenyl | Vinyl | |
| 1.3.1231 | 3-Brom-6-cyclopropyl-Phenyl | Vinyl | |
| 1.3.1232 | 3-Methyl-6-cyclopropyl-Phenyl | Vinyl | |
| 1.3.1233 | 3-Cyclopropyl-6-cyclopropyl-Phenyl | Vinyl | |
| 1.3.1234 | 3-Cyano-6-cyclopropyl-Phenyl | Vinyl | |
| 1.3.1235 | 3-Trifluormethyl-6-cyclopropyl-Phenyl | Vinyl | |
| 1.3.1236 | 3-Methoxy-6-cyclopropyl-Phenyl | Vinyl | |
| 1.3.1237 | 3-Ethoxy-6-cyclopropyl-Phenyl | Vinyl | |
| 1.3.1238 | 3-Trifluormethoxy-6-cyclopropyl-Phenyl | Vinyl | |
| 1.3.1239 | 3-Fluor-6-methoxycarbonyl-Phenyl | Vinyl | |
| 1.3.1240 | 3-Chlor-6-methoxycarbonyl-Phenyl | Vinyl | |
| 1.3.1241 | 3-Brom-6-methoxycarbonyl-Phenyl | Vinyl | |
| 1.3.1242 | 3-Methyl-6-methoxycarbonyl-Phenyl | Vinyl | |
| 1.3.1243 | 3-Cyclopropyl-6-methoxycarbonyl-Phenyl | Vinyl | |
| 1.3.1244 | 3-Cyano-6-methoxycarbonyl-Phenyl | Vinyl | |
| 1.3.1245 | 3-Trifluormethyl-6-methoxycarbonyl-Phenyl | Vinyl | |
| 1.3.1246 | 3-Methoxy-6-methoxycarbonyl -Phenyl | Vinyl | |
| 1.3.1247 | 3-Ethoxy-6-methoxycarbonyl-Phenyl | Vinyl | |
| 1.3.1248 | 3-Trifluormethoxy-6-methoxycarbonyl-Phenyl | Vinyl | |
| 1.3.1249 | 3-Nitro-6-methoxycarbonyl-Phenyl | Vinyl | |
| 1.3.1250 | 3-Fluor-6-cyano-Phenyl | Vinyl | |
| 1.3.1251 | 3-Chlor-6-cyano-Phenyl | Vinyl | |
| 1.3.1252 | 3-Brom-6-cyano-Phenyl | Vinyl | |
| 1.3.1253 | 3-Methyl-6-cyano-Phenyl | Vinyl | |
| 1.3.1254 | 3-Cyclopropyl-6-cyano-Phenyl | Vinyl | |
| 1.3.1255 | 3-Cyano-6-cyano-Phenyl | Vinyl | |
| 1.3.1256 | 3-Trifluormethyl-6-cyano-Phenyl | Vinyl | |
| 1.3.1257 | 3-Methoxy-6-cyano-Phenyl | Vinyl | |
| 1.3.1258 | 3-Ethoxy-6-cyano-Phenyl | Vinyl | |
| 1.3.1259 | 3-Trifluormethoxy-6-cyano-Phenyl | Vinyl | |
| 1.3.1260 | 3-Nitro-6-cyano-Phenyl | Vinyl | |
| 1.3.1261 | 3-Fluor-6-methoxy-Phenyl | Vinyl | |
| 1.3.1262 | 3-Chlor-6-methoxy-Phenyl | Vinyl | |
| 1.3.1263 | 3-Brom-6-methoxy-Phenyl | Vinyl | |
| 1.3.1264 | 3-Methyl-6-methoxy-Phenyl | Vinyl | |
| 1.3.1265 | 3-Cyclopropyl-6-methoxy-Phenyl | Vinyl | |
| 1.3.1266 | 3-Cyano-6-methoxy-Phenyl | Vinyl | |
| 1.3.1267 | 3-Trifluormethyl-6-methoxy-Phenyl | Vinyl | |
| 1.3.1268 | 3,6-Dimethoxy-Phenyl | Vinyl | |
| 1.3.1269 | 3-Ethoxy-6-methoxy-Phenyl | Vinyl | |
| 1.3.1270 | 3-Trifluormethoxy-6-methoxy-Phenyl | Vinyl | |
| 1.3.1271 | 3-Nitro-6-methoxy-Phenyl | Vinyl | |
| 1.3.1272 | 3-Fluor-6-ethoxy-Phenyl | Vinyl | |
| 1.3.1273 | 3-Chlor-6-ethoxy-Phenyl | Vinyl | |
| 1.3.1274 | 3-Brom-6-ethoxy-Phenyl | Vinyl | |
| 1.3.1275 | 3-Methyl-6-ethoxy-Phenyl | Vinyl | |
| 1.3.1276 | 3-Cyclopropyl-6-ethoxy-Phenyl | Vinyl | |
| 1.3.1277 | 3-Cyano-6-ethoxy-Phenyl | Vinyl | |
| 1.3.1278 | 3-Trifluormethyl-6-ethoxy-Phenyl | Vinyl | |
| 1.3.1279 | 3-Methoxy-6-ethoxy-Phenyl | Vinyl | |
| 1.3.1280 | 2,6-Diethoxy-Phenyl | Vinyl | |
| 1.3.1281 | 3-Trifluormethoxy-6-ethoxy-Phenyl | Vinyl | |
| 1.3.1282 | 3-Nitro-6-ethoxy-Phenyl | Vinyl | |
| 1.3.1283 | 3-Fluor-6-isopropoxy-Phenyl | Vinyl | |
| 1.3.1284 | 3-Chlor-6-isopropoxy-Phenyl | Vinyl | |
| 1.3.1285 | 3-Brom-6-isopropoxy-Phenyl | Vinyl | |
| 1.3.1286 | 3-Methyl-6-isopropoxy-Phenyl | Vinyl | |
| 1.3.1287 | 3-Cyclopropyl-6-isopropoxy-Phenyl | Vinyl | |
| 1.3.1288 | 3-Cyano-6-isopropoxy-Phenyl | Vinyl | |
| 1.3.1289 | 3-Trifluormethyl-6-isopropoxy-Phenyl | Vinyl | |
| 1.3.1290 | 3-Methoxy-6-isopropoxy-Phenyl | Vinyl | |
| 1.3.1291 | 3-Ethoxy-6-isopropoxy-Phenyl | Vinyl | |
| 1.3.1292 | 3-Trifluormethoxy-6-isopropoxy-Phenyl | Vinyl | |
| 1.3.1293 | 3-Nitro-6-isopropoxy-Phenyl | Vinyl | |
| 1.3.1294 | 3-Fluor-6-trifluormethoxy-Phenyl | Vinyl | |
| 1.3.1295 | 3-Chlor-6-trifluormethoxy-Phenyl | Vinyl | |
| 1.3.1296 | 3-Brom-6-trifluormethoxy-Phenyl | Vinyl | |
| 1.3.1297 | 3-Methyl-6-trifluormethoxy-Phenyl | Vinyl | |
| 1.3.1298 | 3-Cyclopropyl-6-trifluormethoxy-Phenyl | Vinyl | |
| 1.3.1299 | 3-Cyano-6-trifluormethoxy-Phenyl | Vinyl | |
| 1.3.1300 | 3-Trifluormethyl-6-trifluormethoxy-Phenyl | Vinyl | |
| 1.3.1301 | 3-Methoxy-6-trifluormethoxy-Phenyl | Vinyl | |
| 1.3.1302 | 3-Ethoxy-6-trifluormethoxy-Phenyl | Vinyl | |
| 1.3.1303 | 3,6-Bis(trifluormethoxy)-Phenyl | Vinyl | |
| 1.3.1304 | 3-Nitro-6-trifluormethoxy-Phenyl | Vinyl | |
| 1.3.1305 | 3-Fluor-6-difluormethoxy-Phenyl | Vinyl | |
| 1.3.1306 | 3-Chlor-6-difluormethoxy-Phenyl | Vinyl | |
| 1.3.1307 | 3-Brom-6-difluormethoxy-Phenyl | Vinyl | |
| 1.3.1308 | 3-Methyl-6-difluormethoxy-Phenyl | Vinyl | |
| 1.3.1309 | 3-Cyclopropyl-6-difluormethoxy-Phenyl | Vinyl | |
| 1.3.1310 | 3-Cyano-6-difluormethoxy-Phenyl | Vinyl | |
| 1.3.1311 | 3-Trifluormethyl-6-difluormethoxy-Phenyl | Vinyl | |
| 1.3.1312 | 3-Methoxy-6-difluormethoxy-Phenyl | Vinyl | |
| 1.3.1313 | 3-Ethoxy-6-difluormethoxy-Phenyl | Vinyl | |
| 1.3.1314 | 3-Trifluormethoxy-6-difluormethoxy-Phenyl | Vinyl | |
| 1.3.1315 | 3-Nitro-6-difluormethoxy-Phenyl | Vinyl | |
| 1.3.1316 | 3-Fluor-6-nitro-Phenyl | Vinyl | |
| 1.3.1317 | 3-Chlor-6-nitro-Phenyl | Vinyl | |
| 1.3.1318 | 3-Brom-6-nitro-Phenyl | Vinyl | |
| 1.3.1319 | 3-Methyl-6-nitro-Phenyl | Vinyl | |
| 1.3.1320 | 3-Cyclopropyl-6-nitro-Phenyl | Vinyl | |
| 1.3.1321 | 3-Cyano-6-nitro-Phenyl | Vinyl | |
| 1.3.1322 | 3-Trifluormethyl-6-nitro-Phenyl | Vinyl | |
| 1.3.1323 | 3-Methoxy-6-nitro-Phenyl | Vinyl | |
| 1.3.1324 | 3-Ethoxy-6-nitro-Phenyl | Vinyl | |
| 1.3.1325 | 3-Trifluormethoxy-6-nitro-Phenyl | Vinyl | |
| 1.3.1326 | 3-Fluor-6-methylsulfanylPhenyl | Vinyl | |
| 1.3.1327 | 3-Chlor-6-methylsulfanyl-Phenyl | Vinyl | |
| 1.3.1328 | 3-Brom-6-methylsulfanyl-Phenyl | Vinyl | |
| 1.3.1329 | 3-Methyl-6-methylsulfanyl-Phenyl | Vinyl | |
| 1.3.1330 | 3-Cyclopropyl-6-methylsulfanyl-Phenyl | Vinyl | |
| 1.3.1331 | 3-Cyano-6-methylsulfanyl-Phenyl | Vinyl | |
| 1.3.1332 | 3-Trifluormethyl-6-methylsulfanyl-Phenyl | Vinyl | |
| 1.3.1333 | 3-Methoxy-6-methylsulfanyl-Phenyl | Vinyl | |
| 1.3.1334 | 3-Ethoxy-6-methylsulfanyl-Phenyl | Vinyl | |
| 1.3.1335 | 3-Trifluormethoxy-6-nnethylsulfanyl-Phenyl | Vinyl | |
| 1.3.1336 | 3-Nitro-6-methylsulfanyl-Phenyl | Vinyl | |
| 1.3.1337 | 2,3,4-Trifluor-Phenyl | Vinyl | |
| 1.3.1338 | 2,3,4-Trichlor-Phenyl | Vinyl | |
| 1.3.1339 | 2,3.4-Trimethyl-Phenyl | Vinyl | |
| 1.3.1340 | 2-Fluor-2-chlor-5-trifluormethyl-Phenyl | Vinyl | |
| 1.3.1341 | 2,3,5-Trifluor-Phenyl | Vinyl | |
| 1.3.1342 | 2,3,5-Trichlor-Phenyl | Vinyl | |
| 1.3.1343 | 2,3,5-Trimethyl-Phenyl | Vinyl | |
| 1.3.1344 | 2,3-Dichlor-5-methoxy-Phenyl | Vinyl | |
| 1.3.1345 | 2,3,6-Trifluor-Phenyl | Vinyl | |
| 1.3.1346 | 2,3,6-Trichlor-Phenyl | Vinyl | |
| 1.3.1347 | 2,3,6-Trimethyl-Phenyl | Vinyl | |
| 1.3.1348 | 3,4,5-Trifluor-Phenyl | Vinyl | |
| 1.3.1349 | 3,4,5-Trichlor-Phenyl | Vinyl | |
| 1.3.1350 | 3,4,5-Trimethyl-Phenyl | Vinyl | |
| 1.3.1351 | 3,5-Dimethyl-4-fluor-Phenyl | Vinyl | |
| 1.3.1352 | 3,5-Dichlor-4-methoxy-Phenyl | Vinyl | |
| 1.3.1353 | 3,5-Difluor-4-chlor-Phenyl | Vinyl | |
| 1.3.1354 | 3,5-Dichlor-4-hydroxy-Phenyl | Vinyl | |
| 1.3.1355 | 3,5-Trifluormethyl-4-chlor-Phenyl | Vinyl | |
| 1.3.1356 | 3,4,6-Trifluor-Phenyl | Vinyl | |
| 1.3.1357 | 3,4,6-Trichlor-Phenyl | Vinyl | |
| 1.3.1358 | 3,4,6-Trimethyl-Phenyl | Vinyl | |
| 1.3.1359 | Pentafluorphenyl | Vinyl | |

**Tabelle 1.4 : Erfindungsgemäße Verbindungen der allgemeinen Formel (I), worin W* für COOH, R¹ und R² für Wasserstoff stehen, und Aryl den Rest**

| | | | |
|---|---|---|---|
| | 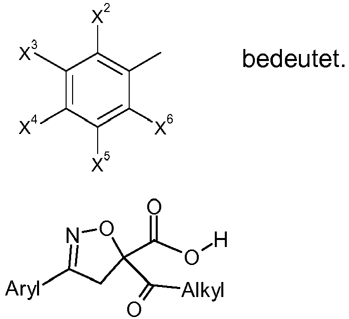 | | |

| Nr. | Aryl | Alkyl | Physikalische Daten |
|---|---|---|---|
| 1.4.001 | 3-Fluor-Phenyl | Methyl | |
| 1.4.002 | 3-Fluor-Phenyl | Ethyl | |
| 1.4.003 | 3-Fluor-Phenyl | Propyl | |
| 1.4.004 | 3-Fluor-Phenyl | Butyl | |
| 1.4.005 | 3-Chlor-Phenyl | Methyl | |
| 1.4.006 | 3-Chlor-Phenyl | Ethyl | |
| 1.4.007 | 3-Chlor-Phenyl | Propyl | |
| 1.4.008 | 3-Chlor-Phenyl | Butyl | |
| 1.4.009 | 3-Brom-Phenyl | Methyl | |
| 1.4.010 | 3-Brom-Phenyl | Ethyl | |
| 1.4.011 | 3-lod-Phenyl | Methyl | |
| 1.4.012 | 3-lod-Phenyl | Ethyl | |
| 1.4.013 | 3-Methyl-Phenyl | Methyl | |
| 1.4.014 | 3-Methyl-Phenyl | Ethyl | |
| 1.4.015 | 3-Ethyl-Phenyl | Methyl | |
| 1.4.016 | 3-Propyl-Phenyl | Methyl | |
| 1.4.017 | 3-isoPropyl-Phenyl | Methyl | |
| 1.4.018 | 3-nButyl-Phenyl | Methyl | |
| 1.4.019 | 3-iButyl-Phenyl | Methyl | |
| 1.4.020 | 3-tert.Butyl-Phenyl | Methyl | |
| 1.4.021 | 3-Cyclopropyl-Phenyl | Methyl | |
| 1.4.022 | 3-Cyclobutyl-Phenyl | Methyl | |
| 1.4.023 | 3-Cyclopentyl-Phenyl | Methyl | |
| 1.4.024 | 3-Vinyl-Phenyl | Methyl | |
| 1.4.025 | 3-Ethinyl-Phenyl | Methyl | |
| 1.4.026 | 3-Cyano-Phenyl | Methyl | |
| 1.4.027 | 3-Trifluormethyl-Phenyl | Methyl | |
| 1.4.028 | 3-Difluormethyl-Phenyl | Methyl | |
| 1.4.029 | 3-(Hydroxycarbonyl)-Phenyl | Methyl | |
| 1.4.030 | 3-(Methoxycarbony)l-Phenyl | Methyl | |
| 1.4.031 | 3-(Ethoxycarbonyl)-Phenyl | Methyl | |
| 1.4.032 | 3-Hydroxymethyl-Phenyl | Methyl | |
| 1.4.033 | 3-Carbamoyl-Phenyl | Methyl | |
| 1.4.034 | 3-Hydroxy-Phenyl- | Methyl | |
| 1.4.035 | 3-Methoxy-Phenyl | Methyl | |
| 1.4.036 | 3-Ethoxy-Phenyl | Methyl | |
| 1.4.037 | 3-Propyloxy-Phenyl | Methyl | |
| 1.4.038 | 3-isoPropyloxy-Phenyl | Methyl | |
| 1.4.039 | 3-nButyloxy-Phenyl | Methyl | |
| 1.4.040 | 3-iButyloxy-Phenyl | Methyl | |
| 1.4.041 | 3-tButyloxy-Phenyl | Methyl | |
| 1.4.042 | 3-Difluormethoxy-Phenyl | Methyl | |
| 1.4.043 | 3-Trifluormethoxy-Phenyl | Methyl | |
| 1.4.044 | 3-(2,2,2-Trifluorethoxy)-Phenyl | Methyl | |
| 1.4.045 | 3-(2-Chlorethoxy)-Phenyl | Methyl | |
| 1.4.046 | 3-(2-Hydroxyethoxy)-Phenyl | Methyl | |
| 1.4.047 | 3-(2-Methoxyethoxy)-Phenyl | Methyl | |
| 1.4.048 | 3-[(tert-Butoxycarbonyl)oxy]-Phenyl | Methyl | |
| 1.4.049 | 3-Nitro-Phenyl | Methyl | |
| 1.4.050 | 3-Acetoxy-Phenyl | Methyl | |
| 1.4.051 | {3-[(tert-Butoxy-carbonyl)amino]-Phenyl} | Methyl | |
| 1.4.052 | 3-Methylsulfanyl-Phenyl | Methyl | |
| 1.4.053 | 3-(Pentafluor-lambda⁶-sulfanyl)-Phenyl | Methyl | |
| 1.4.054 | 2,3-Difluor-Phenyl | Methyl | |
| 1.4.055 | 2,3-Difluor-Phenyl | Ethyl | |
| 1.4.056 | 2,3-Difluor-Phenyl | Propyl | |
| 1.4.057 | 2,3-Difluor-Phenyl | Butyl | |
| 1.4.058 | 2-Chlor-3-fluor-Phenyl | Methyl | |
| 1.4.059 | 2-Brom-3-fluor-Phenyl | Methyl | |
| 1.4.060 | 2-Methyl-3-fluor-Phenyl | Methyl | |
| 1.4.061 | 2-Cyclopropyl-3-fluor-Phenyl | Methyl | |
| 1.4.062 | 2-Cyano-3-fluor-Phenyl | Methyl | |
| 1.4.063 | 2-Methoxy-3-fluor-Phenyl | Methyl | |
| 1.4.064 | 2-Ethoxy-3-fluor-Phenyl | Methyl | |
| 1.4.065 | 2-Trifluormethoxy-3-fluor-Phenyl | Methyl | |
| 1.4.066 | 2-Nitro-3-fluor-Phenyl | Methyl | |
| 1.4.067 | 2-Fluor-3-chlor-Phenyl | Methyl | |
| 1.4.068 | 2,3-Dichlor-Phenyl | Methyl | |
| 1.4.069 | 2,3-Dichlor-Phenyl | Ethyl | |
| 1.4.070 | 2,3-Dichlor-Phenyl | Propyl | |
| 1.4.071 | 2,3-Dichlor-Phenyl | Butyl | |
| 1.4.072 | 2-Brom-3-chlor-Phenyl | Methyl | |
| 1.4.073 | 2-Methyl-3-chlor-Phenyl | Methyl | |
| 1.4.074 | 2-Cyclopropyl-3-chlor-Phenyl | Methyl | |
| 1.4.075 | 2-Cyano-3-chlor-Phenyl | Methyl | |
| 1.4.076 | 2-Trifluormethyl-2-chlor-Phenyl | Methyl | |
| 1.4.077 | 2-Methoxy-3-chlor-Phenyl | Methyl | |
| 1.4.078 | 2-Ethoxy-3-chlor-Phenyl | Methyl | |
| 1.4.079 | 2-Trifluormethoxy-3-chlor-Phenyl | Methyl | |
| 1.4.080 | 2-Nitro-3-chlor-Phenyl | Methyl | |
| 1.4.081 | 2-Fluor-3-brom-Phenyl | Methyl | |
| 1.4.082 | 2-Chlor-3-brom-Phenyl | Methyl | |
| 1.4.083 | 2,3-Dibrom-Phenyl | Methyl | |
| 1.4.084 | 2-Methyl-3-brom-Phenyl | Methyl | |
| 1.4.085 | 2-Ethyl-3-brom-Phenyl | Methyl | |
| 1.4.086 | 2-Cyclopropyl-3-brom-Phenyl | Methyl | |
| 1.4.087 | 2-Cyano-3-brom-Phenyl | Methyl | |
| 1.4.088 | 2-Trifluormethyl-3-brom-Phenyl | Methyl | |
| 1.4.089 | 2-Methoxy-3-Phenyl | Methyl | |
| 1.4.090 | 2-Ethoxy-3-brom-Phenyl | Methyl | |
| 1.4.091 | 2-Trifluormethoxy-3-brom-Phenyl | Methyl | |
| 1.4.092 | 2-Nitro-3-brom-Phenyl | Methyl | |
| 1.4.093 | 2-Fluor-3-iod-Phenyl | Methyl | |
| 1.4.094 | 2-Chlor-3-iod-Phenyl | Methyl | |
| 1.4.095 | 2-Brom-3-iod-Phenyl | Methyl | |
| 1.4.096 | 2-Methyl-3-iod-Phenyl | Methyl | |
| 1.4.097 | 2-Cyclopropyl-3-iod-Phenyl | Methyl | |
| 1.4.098 | 2-Cyano-3-iod-Phenyl | Methyl | |
| 1.4.099 | 2-Trifluormethyl-3-iod-Phenyl | Methyl | |
| 1.4.100 | 2-Methoxy-3-iod-Phenyl | Methyl | |
| 1.4.101 | 2-Ethoxy-3-iod-Phenyl | Methyl | |
| 1.4.102 | 2-Trifluormethoxy-3-iod-Phenyl | Methyl | |
| 1.4.103 | 2-Fluor-3-methyl-Phenyl | Methyl | |
| 1.4.104 | 2-Fluor-3-methyl-Phenyl | Ethyl | |
| 1.4.105 | 2-Fluor-3-methyl-Phenyl | Propyl | |
| 1.4.106 | 2-Fluor-3-methyl-Phenyl | Butyl | |
| 1.4.107 | 2-Chlor-3-methyl-Phenyl | Methyl | |
| 1.4.108 | 2-Chlor-3-methyl-Phenyl | Ethyl | |
| 1.4.109 | 2-Chlor-3-methyl-Phenyl | Propyl | |
| 1.4.110 | 2-Chlor-3-methyl-Phenyl | Butyl | |
| 1.4.111 | 2-Brom-3-methyl-Phenyl | Methyl | |
| 1.4.112 | 2,3-Dimethyl-Phenyl | Methyl | |
| 1.4.113 | 2,3-Dimethyl-Phenyl | Ethyl | |
| 1.4.114 | 2,3-Dimethyl-Phenyl | Propyl | |
| 1.4.115 | 2,3-Dimethyl-Phenyl | Butyl | |
| 1.4.116 | 2-Cyclopropyl-3-methyl-Phenyl | Methyl | |
| 1.4.117 | 2-Cyano-3-methyl-Phenyl | Methyl | |
| 1.4.118 | 2-Trifluormethyl-3-methyl-Phenyl | Methyl | |
| 1.4.119 | 2-Methoxy-3-methyl-Phenyl | Methyl | |
| 1.4.120 | 2-Ethoxy-3-methyl-Phenyl | Methyl | |
| 1.4.121 | 2-Trifluormethoxy-3-methyl-Phenyl | Methyl | |
| 1.4.122 | 2-Nitro-3-methyl-Phenyl | Methyl | |
| 1.4.123 | 2-Fluor-3-ethyl-Phenyl | Methyl | |
| 1.4.124 | 2-Chlor-3-ethyl-Phenyl | Methyl | |
| 1.4.125 | 2-Brom-3-ethyl-Phenyl | Methyl | |
| 1.4.126 | 2-Methyl-3-ethyl-Phenyl | Methyl | |
| 1.4.127 | 2,3-Diethyl-Phenyl | Methyl | |
| 1.4.128 | 2-Cyclopropyl-3-ethyl-Phenyl | Methyl | |
| 1.4.129 | 2-Cyano-3-ethyl-Phenyl | Methyl | |
| 1.4.130 | 2-Trifluormethyl-3-ethyl-Phenyl | Methyl | |
| 1.4.131 | 2-Methoxy-3-ethyl-Phenyl | Methyl | |
| 1.4.132 | 2-Ethoxy-3-ethyl-Phenyl | Methyl | |
| 1.4.133 | 2-Trifluormethoxy-3-ethyl-Phenyl | Methyl | |
| 1.4.134 | 2-Nitro-3-ethyl-Phenyl | Methyl | |
| 1.4.135 | 2-Fluor-3-propyl-Phenyl | Methyl | |
| 1.4.136 | 2-Chlor-3-propyl-Phenyl | Methyl | |
| 1.4.137 | 2-Brom-3-propyl-Phenyl | Methyl | |
| 1.4.138 | 2-Methyl-3-propyl-Phenyl | Methyl | |
| 1.4.139 | 2-Cyclopropyl-3-propyl-Phenyl | Methyl | |
| 1.4.140 | 2-Cyano-3-propyl-Phenyl | Methyl | |
| 1.4.141 | 2-Trifluormethyl-3-propyl-Phenyl | Methyl | |
| 1.4.142 | 2-Methoxy-3-propyl-Phenyl | Methyl | |
| 1.4.143 | 2-Ethoxy-3-propyl-Phenyl | Methyl | |
| 1.4.144 | 2-Trifluormethoxy-3-propyl-Phenyl | Methyl | |
| 1.4.145 | 2-Nitro-3-propyl-Phenyl | Methyl | |
| 1.4.146 | 2-Fluor-3-isopropyl-Phenyl | Methyl | |
| 1.4.147 | 2-Chlor-3-isopropyl-Phenyl | Methyl | |
| 1.4.148 | 2-Brom-3-isopropyl-Phenyl | Methyl | |
| 1.4.149 | 2-Methyl-3-isopropyl-Phenyl | Methyl | |
| 1.4.150 | 2-Cyclopropyl-3-isopropyl-Phenyl | Methyl | |
| 1.4.151 | 2-Cyano-3-isopropyl-Phenyl | Methyl | |
| 1.4.152 | 2-Trifluormethyl-3-isopropyl-Phenyl | Methyl | |
| 1.4.153 | 2-Methoxy-3-isopropyl-Phenyl | Methyl | |
| 1.4.154 | 2-Ethoxy-3-isopropyl-Phenyl | Methyl | |
| 1.4.155 | 2-Trifluormethoxy-3-isopropyl-Phenyl | Methyl | |
| 1.4.156 | 2-Nitro-3-isopropyl-Phenyl | Methyl | |
| 1.4.157 | 2-Fluor-3-tert.butyl-Phenyl | Methyl | |
| 1.4.158 | 2-Chlor-3-tert.butyl-Phenyl | Methyl | |
| 1.4.159 | 2-Brom-3-tert.butyl-Phenyl | Methyl | |
| 1.4.160 | 2-Methyl-3-tert.butyl-Phenyl | Methyl | |
| 1.4.161 | 2-Cyano-3-tert.butyl-Phenyl | Methyl | |
| 1.4.162 | 2-Trifluormethyl-3-tert.butyl-Phenyl | Methyl | |
| 1.4.163 | 2-Methoxy-3-tert.butyl-Phenyl | Methyl | |
| 1.4.164 | 2-Ethoxy-3-tert.butyl-Phenyl | Methyl | |
| 1.4.165 | 2-Nitro-3-tert.butyl-Phenyl | Methyl | |
| 1.4.166 | 2-Fluor-3-cyclopropyl-Phenyl | Methyl | |
| 1.4.167 | 2-Chlor-3-cyclopropyl-Phenyl | Methyl | |
| 1.4.168 | 2-Brom-3-cyclopropyl-Phenyl | Methyl | |
| 1.4.169 | 2-Methyl-3-cyclopropyl-Phenyl | Methyl | |
| 1.4.170 | 2-Cyano-3-cyclopropyl-Phenyl | Methyl | |
| 1.4.171 | 2-Trifluormethyl-3-cyclopropyl-Phenyl | Methyl | |
| 1.4.172 | 2-Methoxy-3-cyclopropyl-Phenyl | Methyl | |
| 1.4.173 | 2-Trifluormethoxy-3-cyclopropyl-Phenyl | Methyl | |
| 1.4.174 | 2-Fluor-3-methoxycarbonyl-Phenyl | Methyl | |
| 1.4.175 | 2-Chlor-3-methoxycarbonyl-Phenyl | Methyl | |
| 1.4.176 | 2-Brom-3-methoxycarbonyl-Phenyl | Methyl | |
| 1.4.177 | 2-Methyl-3-methoxycarbonyl-Phenyl | Methyl | |
| 1.4.178 | 2-Cyclopropyl-3-methoxycarbonyl-Phenyl | Methyl | |
| 1.4.179 | 2-Cyano-3-methoxycarbonyl-Phenyl | Methyl | |
| 1.4.180 | 2-Trifluormethyl-3-methoxycarbonyl-Phenyl | Methyl | |
| 1.4.181 | 2-Methoxy-3-methoxycarbonyl -Phenyl | Methyl | |
| 1.4.182 | 2-Ethoxy-3-methoxycarbonyl-Phenyl | Methyl | |
| 1.4.183 | 2-Trifluormethoxy-3-methoxycarbonyl-Phenyl | Methyl | |
| 1.4.184 | 2-Nitro-3-methoxycarbonyl-Phenyl | Methyl | |
| 1.4.185 | 2-Fluor-3-cyano-Phenyl | Methyl | |
| 1.4.186 | 2-Chlor-3-cyano-Phenyl | Methyl | |
| 1.4.187 | 2-Brom-3-cyano-Phenyl | Methyl | |
| 1.4.188 | 2-Methyl-3-cyano-Phenyl | Methyl | |
| 1.4.189 | 2-Ethyl-3-cyano-Phenyl | Methyl | |
| 1.4.190 | 2-Ethyl-3-cyano-Phenyl | Ethyl | |
| 1.4.191 | 2-Ethyl-3-cyano-Phenyl | Propyl | |
| 1.4.192 | 2-Ethyl-3-cyano-Phenyl | Butyl | |
| 1.4.193 | 2-Cyclopropyl-3-cyano-Phenyl | Methyl | |
| 1.4.194 | 2-Cyano-3-cyano-Phenyl | Methyl | |
| 1.4.195 | 2-Trifluormethyl-3-cyano-Phenyl | Methyl | |
| 1.4.196 | 2-Methoxy-3-cyano-Phenyl | Methyl | |
| 1.4.197 | 2-Ethoxy-3-cyano-Phenyl | Methyl | |
| 1.4.198 | 2-Trifluormethoxy-3-cyano-Phenyl | Methyl | |
| 1.4.199 | 2-Fluor-3-methoxy-Phenyl | Methyl | |
| 1.4.200 | 2-Chlor-3-methoxy-Phenyl | Methyl | |
| 1.4.201 | 2-Brom-3-methoxy-Phenyl | Methyl | |
| 1.4.202 | 2-Methyl-3-methoxy-Phenyl | Methyl | |
| 1.4.203 | 2-Cyclopropyl-3-methoxy-Phenyl | Methyl | |
| 1.4.204 | 2-Cyano-3-methoxy-Phenyl | Methyl | |
| 1.4.205 | 2-Trifluormethyl-3-methoxy-Phenyl | Methyl | |
| 1.4.206 | 2,3-Dimethoxy-Phenyl | Methyl | |
| 1.4.207 | 2-Ethoxy-3-methoxy-Phenyl | Methyl | |
| 1.4.208 | 2-Trifluormethoxy-3-methoxy-Phenyl | Methyl | |
| 1.4.209 | 2-Nitro-3-methoxy-Phenyl | Methyl | |
| 1.4.210 | 2-Fluor-3-ethoxy-Phenyl | Methyl | |
| 1.4.211 | 2-Chlor-3-ethoxy-Phenyl | Methyl | |
| 1.4.212 | 2-Brom-3-ethoxy-Phenyl | Methyl | |
| 1.4.213 | 2-Methyl-3-ethoxy-Phenyl | Methyl | |
| 1.4.214 | 2-Ethyl-3-ethoxy-Phenyl | Methyl | |
| 1.4.215 | 2-Cyclopropyl-3-ethoxy-Phenyl | Methyl | |
| 1.4.216 | 2-Cyano-3-ethoxy-Phenyl | Methyl | |
| 1.4.217 | 2-Trifluormethyl-3-ethoxy-Phenyl | Methyl | |
| 1.4.218 | 2-Methoxy-3-ethoxy -Phenyl | Methyl | |
| 1.4.219 | 2,3-Diethoxy-Phenyl | Methyl | |
| 1.4.220 | 2-Trifluormethoxy-3-ethoxy-Phenyl | Methyl | |
| 1.4.221 | 2-Nitro-3-ethoxy-Phenyl | Methyl | |
| 1.4.222 | 2-Fluor-3-isopropoxy-Phenyl | Methyl | |
| 1.4.223 | 2-Chlor-3-isopropoxy-Phenyl | Methyl | |
| 1.4.224 | 2-Brom-3-isopropoxy-Phenyl | Methyl | |
| 1.4.225 | 2-Methyl-3-isopropoxy-Phenyl | Methyl | |
| 1.4.226 | 2-Ethyl-3-isopropoxy-Phenyl | Methyl | |
| 1.4.227 | 2-Cyclopropyl-3-isopropoxy-Phenyl | Methyl | |
| 1.4.228 | 2-Cyano-3-isopropoxy-Phenyl | Methyl | |
| 1.4.229 | 2-Trifluormethyl-3-isopropoxy-Phenyl | Methyl | |
| 1.4.230 | 2-Methoxy-3-isopropoxy-Phenyl | Methyl | |
| 1.4.231 | 2-Ethoxy-3-isopropoxy-Phenyl | Methyl | |
| 1.4.232 | 2-Trifluormethoxy-3-isopropoxy-Phenyl | Methyl | |
| 1.4.233 | 2-Nitro-3-isopropoxy-Phenyl | Methyl | |
| 1.4.234 | 2-Fluor-3-trifluormethoxy-Phenyl | Methyl | |
| 1.4.235 | 2-Chlor-3-trifluormethoxy-Phenyl | Methyl | |
| 1.4.236 | 2-Brom-3-trifluormethoxy-Phenyl | Methyl | |
| 1.4.237 | 2-Methyl-3-trifluormethoxy-Phenyl | Methyl | |
| 1.4.238 | 2-Cyclopropyl-3-trifluormethoxy-Phenyl | Methyl | |
| 1.4.239 | 2-Cyano-3-trifluormethoxy-Phenyl | Methyl | |
| 1.4.240 | 2-Trifluormethyl-3-trifluormethoxy-Phenyl | Methyl | |
| 1.4.241 | 2-Methoxy-3-trifluormethoxy-Phenyl | Methyl | |
| 1.4.242 | 2-Ethoxy-3-trifluormethoxy-Phenyl | Methyl | |
| 1.4.243 | 2,3-Bis(trifluormethoxy)-Phenyl | Methyl | |
| 1.4.244 | 2-Nitro-3-trifluormethoxy-Phenyl | Methyl | |
| 1.4.245 | 2-Fluor-3-difluormethoxy-Phenyl | Methyl | |
| 1.4.246 | 2-Chlor-3-difluormethoxy-Phenyl | Methyl | |
| 1.4.247 | 2-Brom-3-difluormethoxy-Phenyl | Methyl | |
| 1.4.248 | 2-Methyl-3-difluormethoxy-Phenyl | Methyl | |
| 1.4.249 | 2-Cyclopropyl-3-difluormethoxy-Phenyl | Methyl | |
| 1.4.250 | 2-Cyano-3-difluormethoxy-Phenyl | Methyl | |
| 1.4.251 | 2-Trifluormethyl-3-difluormethoxy-Phenyl | Methyl | |
| 1.4.252 | 2-Methoxy-3-difluormethoxy-Phenyl | Methyl | |
| 1.4.253 | 2-Ethoxy-3-difluormethoxy-Phenyl | Methyl | |
| 1.4.254 | 2-Trifluormethoxy-3-difluormethoxy-Phenyl | Methyl | |
| 1.4.255 | 2-Nitro-3-difluormethoxy-Phenyl | Methyl | |
| 1.4.256 | 2-Fluor-3-nitro-Phenyl | Methyl | |
| 1.4.257 | 2-Chlor-3-nitro-Phenyl | Methyl | |
| 1.4.258 | 2-Brom-3-nitro-Phenyl | Methyl | |
| 1.4.259 | 2-Methyl-3-nitro-Phenyl | Methyl | |
| 1.4.260 | 2-Cyclopropyl-3-nitro-Phenyl | Methyl | |
| 1.4.261 | 2-Cyano-3-nitro-Phenyl | Methyl | |
| 1.4.262 | 2-Trifluormethyl-3-nitro-Phenyl | Methyl | |
| 1.4.263 | 2-Methoxy-3-nitro-Phenyl | Methyl | |
| 1.4.264 | 2-Ethoxy-3-nitro-Phenyl | Methyl | |
| 1.4.265 | 2-Trifluormethoxy-3-nitro-Phenyl | Methyl | |
| 1.4.266 | 2-Fluor-3-methylsulfanylPhenyl | Methyl | |
| 1.4.267 | 2-Chlor-3-methylsulfanyl-Phenyl | Methyl | |
| 1.4.268 | 2-Brom-3-methylsulfanyl-Phenyl | Methyl | |
| 1.4.269 | 2-Methyl-3-methylsulfanyl-Phenyl | Methyl | |
| 1.4.270 | 2-Ethyl-3-methylsulfanyl-Phenyl | Methyl | |
| 1.4.271 | 2-Cyclopropyl-3-methylsulfanyl-Phenyl | Methyl | |
| 1.4.272 | 2-Trifluormethyl-3-methylsulfanyl -Phenyl | Methyl | |
| 1.4.273 | 2-Methoxy-3-methylsulfanyl-Phenyl | Methyl | |
| 1.4.274 | 3,5-Difluor-Phenyl | Methyl | |
| 1.4.275 | 3,5-Difluor-Phenyl | Ethyl | |
| 1.4.276 | 3,5-Difluor-Phenyl | Propyl | |
| 1.4.277 | 3,5-Difluor-Phenyl | Butyl | |
| 1.4.278 | 3-Chlor-5-fluor-Phenyl | Methyl | |
| 1.4.279 | 3-Chlor-5-fluor-Phenyl | Ethyl | |
| 1.4.280 | 3-Chlor-5-fluor-Phenyl | Propyl | |
| 1.4.281 | 3-Chlor-5-fluor-Phenyl | Butyl | |
| 1.4.282 | 3-Brom-5-fluor-Phenyl | Methyl | |
| 1.4.283 | 3-Brom-5-fluor-Phenyl | Ethyl | |
| 1.4.284 | 3-Brom-5-fluor-Phenyl | Propyl | |
| 1.4.285 | 3-Brom-5-fluor-Phenyl | Butyl | |
| 1.4.286 | 3-Iod-5-fluor-Phenyl | Methyl | |
| 1.4.287 | 3-Methyl-5-fluor-Phenyl | Methyl | |
| 1.4.288 | 3-Methyl-5-fluor-Phenyl | Ethyl | |
| 1.4.289 | 3-Methyl-5-fluor-Phenyl | Propyl | |
| 1.4.290 | 3-Methyl-5-fluor-Phenyl | Butyl | |
| 1.4.291 | 3-Propyl-5-fluor-Phenyl | Methyl | |
| 1.4.292 | 3-iPropyl-5-fluor-Phenyl | Methyl | |
| 1.4.293 | 3-nButyl-5-fluor-Phenyl | Methyl | |
| 1.4.294 | 3-isoButyl-5-fluor-Phenyl | Methyl | |
| 1.4.295 | 3-tert.Butyl-5-fluor-Phenyl | Methyl | |
| 1.4.296 | 3-Cyclopropyl-5-fluor-Phenyl | Methyl | |
| 1.4.297 | 3-Cyano-5-fluor-Phenyl | Methyl | |
| 1.4.298 | 3-Trifluormethyl-5-fluor-Phenyl | Methyl | |
| 1.4.299 | 3-Trifluormethyl-5-fluor-Phenyl | Ethyl | |
| 1.4.300 | 3-Trifluormethyl-5-fluor-Phenyl | Propyl | |
| 1.4.301 | 3-Trifluormethyl-5-fluor-Phenyl | Butyl | |
| 1.4.302 | 3-(Methoxycarbony)l-5-fluor-Phenyl | Methyl | |
| 1.4.303 | 3-Methoxy-5-fluor-Phenyl | Methyl | |
| 1.4.304 | 3-Ethoxy-5-fluor-Phenyl | Methyl | |
| 1.4.305 | 3-nPropyoxy-5-fluor-Phenyl | Methyl | |
| 1.4.306 | 3-isoPropoxy-5-fluor-Phenyl | Methyl | |
| 1.4.307 | 3-Difluormethoxy-5-fluor-Phenyl | Methyl | |
| 1.4.308 | 3-Trifluormethoxy-5-fluor-Phenyl | Methyl | |
| 1.4.309 | 3-Nitro-5-fluor-Phenyl | Methyl | |
| 1.4.310 | 3-Acetoxy-5-fluor-Phenyl | Methyl | |
| 1.4.311 | 3-Methylsulfanyl-5-fluor-Phenyl | Methyl | |
| 1.4.312 | 3,5-Dichlor-Phenyl | Methyl | |
| 1.4.313 | 3,5-Dichlor-Phenyl | Ethyl | |
| 1.4.314 | 3,5-Dichlor-Phenyl | Propyl | |
| 1.4.315 | 3,5-Dichlor-Phenyl | Butyl | |
| 1.4.316 | 3-Brom-5-chlor-Phenyl | Methyl | |
| 1.4.317 | 3-lod-5-chlor-Phenyl | Methyl | |
| 1.4.318 | 3-Methyl-5-chlor-Phenyl | Methyl | |
| 1.4.319 | 3-Ethyl-5-chlor-Phenyl | Methyl | |
| 1.4.320 | 3-Propyl-5-chlor-Phenyl | Methyl | |
| 1.4.321 | 3-isoPropyl-5-chlor-Phenyl | Methyl | |
| 1.4.322 | 3-nButyl-5-chlor-Phenyl | Methyl | |
| 1.4.323 | 3-isoButyl-5-chlor-Phenyl | Methyl | |
| 1.4.324 | 3-tert.Butyl-5-chlor-Phenyl | Methyl | |
| 1.4.325 | 3-Cyclopropyl-5-chlor-Phenyl | Methyl | |
| 1.4.326 | 3-Cyano-5-chlor-Phenyl | Methyl | |
| 1.4.327 | 3-Trifluormethyl-5-chlor-Phenyl | Methyl | |
| 1.4.328 | 3-(Methoxycarbony)l-5-chlor-Phenyl | Methyl | |
| 1.4.329 | 3-Methoxy-5-chlor-Phenyl | Methyl | |
| 1.4.330 | 3-Ethoxy-5-chlor-Phenyl | Methyl | |
| 1.4.331 | 3-nPropyoxy-5-chlor-Phenyl | Methyl | |
| 1.4.332 | 3-isoPropoxy-5-chlor-Phenyl | Methyl | |
| 1.4.333 | 3-nButoxy-5-chlor-Phenyl | Methyl | |
| 1.4.334 | 3-isoButoxy-5-chlor-Phenyl | Methyl | |
| 1.4.335 | 3-tert.Butoxy-5-chlor-Phenyl | Methyl | |
| 1.4.336 | 3-Difluormethoxy-5-chlor-Phenyl | Methyl | |
| 1.4.337 | 3-Trifluormethoxy-5-chlor-Phenyl | Methyl | |
| 1.4.338 | 3-Nitro-5-chlor-Phenyl | Methyl | |
| 1.4.339 | 3-Acetoxy-5-chlor-Phenyl | Methyl | |
| 1.4.340 | 3-Methylsulfanyl-5-chlor-Phenyl | Methyl | |
| 1.4.341 | 3,5-Dibrom-Phenyl | Methyl | |
| 1.4.342 | 3,5-Dibrom-Phenyl | Ethyl | |
| 1.4.343 | 3-lod-5-brom-Phenyl | Methyl | |
| 1.4.344 | 3-Methyl-5-brom-Phenyl | Methyl | |
| 1.4.345 | 3-Methyl-5-brom-Phenyl | Ethyl | |
| 1.4.346 | 3-Methyl-5-brom-Phenyl | Propyl | |
| 1.4.347 | 3-Methyl-5-brom-Phenyl | Butyl | |
| 1.4.348 | 3-Ethyl-5-brom-Phenyl | Methyl | |
| 1.4.349 | 3-Propyl-5-brom-Phenyl | Methyl | |
| 1.4.350 | 3-isoPropyl-5-brom-Phenyl | Methyl | |
| 1.4.351 | 3-nButyl-5-brom-Phenyl | Methyl | |
| 1.4.352 | 3-isoButyl-5-brom-Phenyl | Methyl | |
| 1.4.353 | 3-tert.Butyl-5-brom-Phenyl | Methyl | |
| 1.4.354 | 3-Cyclopropyl-5-brom-Phenyl | Methyl | |
| 1.4.355 | 3-Cyano-5-brom-Phenyl | Methyl | |
| 1.4.356 | 3-Trifluormethyl-5-brom-Phenyl | Methyl | |
| 1.4.357 | 3-(Methoxycarbony)l-5-brom-Phenyl | Methyl | |
| 1.4.358 | 3-Methoxy-5-brom-Phenyl | Methyl | |
| 1.4.359 | 3-Ethoxy-5-brom-Phenyl | Methyl | |
| 1.4.360 | 3-nPropyoxy-5-brom-Phenyl | Methyl | |
| 1.4.361 | 3-isoPropoxy-5-brom-Phenyl | Methyl | |
| 1.4.362 | 3-nButoxy-5-brom-Phenyl | Methyl | |
| 1.4.363 | 3-isoButoxy-5-brom-Phenyl | Methyl | |
| 1.4.364 | 3-tert.Butoxy-5-brom-Phenyl | Methyl | |
| 1.4.365 | 3-Difluormethoxy-5-brom-Phenyl | Methyl | |
| 1.4.366 | 3-Trifluormethoxy-5-brom-Phenyl | Methyl | |
| 1.4.367 | 3-Nitro-5-brom-Phenyl | Methyl | |
| 1.4.368 | 3-Acetoxy-5-brom-Phenyl | Methyl | |
| 1.4.369 | 3-Methylsulfanyl-5-brom-Phenyl | Methyl | |
| 1.4.370 | 3,5-Diiod-Phenyl | Methyl | |
| 1.4.371 | 3-Methyl-5-iod-Phenyl | Methyl | |
| 1.4.372 | 3-Ethyl-5-iod-Phenyl | Methyl | |
| 1.4.373 | 3-Propyl-5-iod-Phenyl | Methyl | |
| 1.4.374 | 3-isoPropyl-5-iod-Phenyl | Methyl | |
| 1.4.375 | 3-nButyl-5-iod-Phenyl | Methyl | |
| 1.4.376 | 3-isoButyl-5-iod-Phenyl | Methyl | |
| 1.4.377 | 3-tert.Butyl-5-iod-Phenyl | Methyl | |
| 1.4.378 | 3-Cyclopropyl-5-iod-Phenyl | Methyl | |
| 1.4.379 | 3-Cyano-5-iod-Phenyl | Methyl | |
| 1.4.380 | 3-Trifluormethyl-5-iod-Phenyl | Methyl | |
| 1.4.381 | 3-(Methoxycarbonyl)-5-iod-Phenyl | Methyl | |
| 1.4.382 | 3-Methoxy-5-iod-Phenyl | Methyl | |
| 1.4.383 | 3-Ethoxy-5-iod-Phenyl | Methyl | |
| 1.4.384 | 3-nPropyoxy-5-iod-Phenyl | Methyl | |
| 1.4.385 | 3-isoPropoxy-5-iod-Phenyl | Methyl | |
| 1.4.386 | 3-isoButoxy-5-iod-Phenyl | Methyl | |
| 1.4.387 | 3-Difluormethoxy-5-iod-Phenyl | Methyl | |
| 1.4.388 | 3-Trifluormethoxy-5-iod-Phenyl | Methyl | |
| 1.4.389 | 3-Nitro-5-iod-Phenyl | Methyl | |
| 1.4.390 | 3-Acetoxy-5-iod-Phenyl | Methyl | |
| 1.4.391 | 3-Methylsulfanyl-5-iod-Phenyl | Methyl | |
| 1.4.392 | 3,5-Dimethyl-Phenyl | Methyl | |
| 1.4.393 | 3-Ethyl-5-methyl-Phenyl | Methyl | |
| 1.4.394 | 3-Propyl-5-methyl-Phenyl | Methyl | |
| 1.4.395 | 3-isoPropyl-5-methyl-Phenyl | Methyl | |
| 1.4.396 | 3-nButyl-5-methyl-Phenyl | Methyl | |
| 1.4.397 | 3-isoButyl-5-methyl-Phenyl | Methyl | |
| 1.4.398 | 3-tert.Butyl-5-methyl-Phenyl | Methyl | |
| 1.4.399 | 3-Cyclopropyl-5-methyl-Phenyl | Methyl | |
| 1.4.400 | 3-Cyano-5-methyl-Phenyl | Methyl | |
| 1.4.401 | 3-Trifluormethyl-5-methyl-Phenyl | Methyl | |
| 1.4.402 | 3-(Methoxycarbonyl)-5-methyl-Phenyl | Methyl | |
| 1.4.403 | 3-Methoxy-5-methyl-Phenyl | Methyl | |
| 1.4.404 | 3-Ethoxy-5-methyl-Phenyl | Methyl | |
| 1.4.405 | 3-nPropyoxy-5-methyl-Phenyl | Methyl | |
| 1.4.406 | 3-nButoxy-5-methyl-Phenyl | Methyl | |
| 1.4.407 | 3-isoButoxy-5-methyl-Phenyl | Methyl | |
| 1.4.408 | 3-Difluormethoxy-5-methyl-Phenyl | Methyl | |
| 1.4.409 | 3-Trifluormethoxy-5-methyl-Phenyl | Methyl | |
| 1.4.410 | 3-Nitro-5-methyl-Phenyl | Methyl | |
| 1.4.411 | 3-Acetoxy-5-methyl-Phenyl | Methyl | |
| 1.4.412 | 3-Methylsulfanyl-5-methyl-Phenyl | Methyl | |
| 1.4.413 | 3,5-Diethyl-Phenyl | Methyl | |
| 1.4.414 | 3-Propyl-5-ethyl-Phenyl | Methyl | |
| 1.4.415 | 3-isoPropyl-5-ethyl-Phenyl | Methyl | |
| 1.4.416 | 3-nButyl-5-ethyl-Phenyl | Methyl | |
| 1.4.417 | 3-isoButyl-5-ethyl-Phenyl | Methyl | |
| 1.4.418 | 3-tert.Butyl-5-ethyl-Phenyl | Methyl | |
| 1.4.419 | 3-Cyclopropyl-5-ethyl-Phenyl | Methyl | |
| 1.4.420 | 3-Cyano-5-ethyl-Phenyl | Methyl | |
| 1.4.421 | 3-Trifluormethyl-5-ethyl-Phenyl | Methyl | |
| 1.4.422 | 3-(Methoxycarbony)l-5-ethyl-Phenyl | Methyl | |
| 1.4.423 | 3-Methoxy-5-ethyl-Phenyl | Methyl | |
| 1.4.424 | 3-Ethoxy-5-ethyl-Phenyl | Methyl | |
| 1.4.425 | 3-nPropyoxy-5-ethyl-Phenyl | Methyl | |
| 1.4.426 | 3-nButoxy-5-ethyl-Phenyl | Methyl | |
| 1.4.427 | 3-isoButoxy-5-ethyl-Phenyl | Methyl | |
| 1.4.428 | 3-Difluormethoxy-5-ethyl-Phenyl | Methyl | |
| 1.4.429 | 3-Trifluormethoxy-5-ethyl-Phenyl | Methyl | |
| 1.4.430 | 3-Nitro-5-ethyl-Phenyl | Methyl | |
| 1.4.431 | 3-Acetoxy-5-ethyl-Phenyl | Methyl | |
| 1.4.432 | 3-Methylsulfanyl-5-ethyl-Phenyl | Methyl | |
| 1.4.433 | 3,5-Dipropyl-Phenyl | Methyl | |
| 1.4.434 | 3-isoPropyl-5-propyl-Phenyl | Methyl | |
| 1.4.435 | 3-nButyl-5-propyl-Phenyl | Methyl | |
| 1.4.436 | 3-isoButyl-5-propyl-Phenyl | Methyl | |
| 1.4.437 | 3-tert.Butyl-5-propyl-Phenyl | Methyl | |
| 1.4.438 | 3-Cyclopropyl-5-propyl-Phenyl | Methyl | |
| 1.4.439 | 3-Cyano-5-propyl-Phenyl | Methyl | |
| 1.4.440 | 3-Trifluormethyl-5-propyl-Phenyl | Methyl | |
| 1.4.441 | 3-(Methoxycarbonyl)-5-propyl-Phenyl | Methyl | |
| 1.4.442 | 3-Methoxy-5-propyl-Phenyl | Methyl | |
| 1.4.443 | 3-Ethoxy-5-propyl-Phenyl | Methyl | |
| 1.4.444 | 3-nPropyoxy-5-propyl-Phenyl | Methyl | |
| 1.4.445 | 3-nButoxy-5-propyl-Phenyl | Methyl | |
| 1.4.446 | 3-isoButoxy-5-propyl-Phenyl | Methyl | |
| 1.4.447 | 3-Difluormethoxy-5-propyl-Phenyl | Methyl | |
| 1.4.448 | 3-Trifluormethoxy-5-ethyl-Phenyl | Methyl | |
| 1.4.449 | 3-Nitro-5-propyl-Phenyl | Methyl | |
| 1.4.450 | 3-Acetoxy-5-propyl-Phenyl | Methyl | |
| 1.4.451 | 3-Methylsulfanyl-5-propyl-Phenyl | Methyl | |
| 1.4.452 | 3,5-Diisopropyl-Phenyl | Methyl | |
| 1.4.453 | 3-nButyl-5-isopropyl-Phenyl | Methyl | |
| 1.4.454 | 3-tert.Butyl-5-isopropyl-Phenyl | Methyl | |
| 1.4.455 | 3-Cyclopropyl-5-isopropyl-Phenyl | Methyl | |
| 1.4.456 | 3-Cyano-5-isopropyl-Phenyl | Methyl | |
| 1.4.457 | 3-Trifluormethyl-5-isopropyl-Phenyl | Methyl | |
| 1.4.458 | 3-(Methoxycarbony)l-5-isopropyl-Phenyl | Methyl | |
| 1.4.459 | 3-Methoxy-5-isopropyl-Phenyl | Methyl | |
| 1.4.460 | 3-Ethoxy-5-isopropyl-Phenyl | Methyl | |
| 1.4.461 | 3-nPropyoxy-5-isopropyl-Phenyl | Methyl | |
| 1.4.462 | 3-nButoxy-5-isopropyl-Phenyl | Methyl | |
| 1.4.463 | 3-isoButoxy-5-isopropyl-Phenyl | Methyl | |
| 1.4.464 | 3-Difluormethoxy-5-isopropyl-Phenyl | Methyl | |
| 1.4.465 | 3-Trifluormethoxy-5-isopropyl-Phenyl | Methyl | |
| 1.4.466 | 3-Nitro-5-isopropyl-Phenyl | Methyl | |
| 1.4.467 | 3-Acetoxy-5-isopropyl-Phenyl | Methyl | |
| 1.4.468 | 3-Methylsulfanyl-5-isopropyl-Phenyl | Methyl | |
| 1.4.469 | 3,5-Diisobutyl-Phenyl | Methyl | |
| 1.4.470 | 3-tert.Butyl-5-isobutyl-Phenyl | Methyl | |
| 1.4.471 | 3-Cyclopropyl-5-isobutyl-Phenyl | Methyl | |
| 1.4.472 | 3-Cyano-5-isobutyl-Phenyl | Methyl | |
| 1.4.473 | 3-Trifluormethyl-5-isobutyl-Phenyl | Methyl | |
| 1.4.474 | 3-(Methoxycarbonyl)-5-isobutyl-Phenyl | Methyl | |
| 1.4.475 | 3-Methoxy-5-isobutyl-Phenyl | Methyl | |
| 1.4.476 | 3-Ethoxy-5-isobutyl-Phenyl | Methyl | |
| 1.4.477 | 3-nPropyoxy-5-isobutyl-Phenyl | Methyl | |
| 1.4.478 | 3-nButoxy-5-isobutyl-Phenyl | Methyl | |
| 1.4.479 | 3-isoButoxy-5-isobutyl-Phenyl | Methyl | |
| 1.4.480 | 3-Difluormethoxy-5-isobutyl-Phenyl | Methyl | |
| 1.4.481 | 3-Trifluormethoxy-5-isobutyl-Phenyl | Methyl | |
| 1.4.482 | 3-Nitro-5-isobutyl-Phenyl | Methyl | |
| 1.4.483 | 3-Acetoxy-5-isobutyl-Phenyl | Methyl | |
| 1.4.484 | 3-Methylsulfanyl-5-isobutyl-Phenyl | Methyl | |
| 1.4.485 | 3,5-Di(tert.butyl)-Phenyl | Methyl | |
| 1.4.486 | 3-Cyclopropyl-5-tert.butyl-Phenyl | Methyl | |
| 1.4.487 | 3-Cyano-5-tert.butyl-Phenyl | Methyl | |
| 1.4.488 | 3-Trifluormethyl-5-tert.butyl-Phenyl | Methyl | |
| 1.4.489 | 3-Methoxy-5-tert.butyl-Phenyl | Methyl | |
| 1.4.490 | 3-Ethoxy-5-tert.butyl-Phenyl | Methyl | |
| 1.4.491 | 3-nPropyoxy-5-tert.butyl-Phenyl | Methyl | |
| 1.4.492 | 3-nButoxy-5-tert.butyl-Phenyl | Methyl | |
| 1.4.493 | 3-isoButoxy-5-tert.butyl-Phenyl | Methyl | |
| 1.4.494 | 3-Difluormethoxy-5-tert.butyl-Phenyl | Methyl | |
| 1.4.495 | 3-Trifluormethoxy-5-tert.butyl-Phenyl | Methyl | |
| 1.4.496 | 3-Nitro-5-tert.butyl-Phenyl | Methyl | |
| 1.4.497 | 3-Acetoxy-5-tert.butyl-Phenyl | Methyl | |
| 1.4.498 | 3-Methylsulfanyl-5-tert.butyl-Phenyl | Methyl | |
| 1.4.499 | 3-tert.Butyl-5-cyclopropyl-Phenyl | Methyl | |
| 1.4.500 | 3,5-Dicyclopropyl-Phenyl | Methyl | |
| 1.4.501 | 3-Cyano-5-cyclopropyl-Phenyl | Methyl | |
| 1.4.502 | 3-Trifluormethyl-5-cyclopropyl-Phenyl | Methyl | |
| 1.4.503 | 3-(Methoxycarbonyl)-5-cyclopropyl-Phenyl | Methyl | |
| 1.4.504 | 3-Methoxy-5-cyclopropyl-Phenyl | Methyl | |
| 1.4.505 | 3-Ethoxy-5-cyclopropyl-Phenyl | Methyl | |
| 1.4.506 | 3-nPropyoxy-5-cyclopropyl-Phenyl | Methyl | |
| 1.4.507 | 3-nButoxy-5-cyclopropyl-Phenyl | Methyl | |
| 1.4.508 | 3-isoButoxy-5-cyclopropyl-Phenyl | Methyl | |
| 1.4.509 | 3-Difluormethoxy-5-cyclopropyl-Phenyl | Methyl | |
| 1.4.510 | 3-Trifluormethoxy-5-cyclopropyl-Phenyl | Methyl | |
| 1.4.511 | 3-Nitro-5-cyclopropyl-Phenyl | Methyl | |
| 1.4.512 | 3-Acetoxy-5-cyclopropyl-Phenyl | Methyl | |
| 1.4.513 | 3-Methylsulfanyl-5-cyclopropyl-Phenyl | Methyl | |
| 1.4.514 | 3,5-Dicyano-Phenyl | Methyl | |
| 1.4.515 | 3-Trifluormethyl-5-cyano-Phenyl | Methyl | |
| 1.4.516 | 3-(Methoxycarbonyl)-5-cyano-Phenyl | Methyl | |
| 1.4.517 | 3-Methoxy-5-cyano-Phenyl | Methyl | |
| 1.4.518 | 3-Ethoxy-5-cyano-Phenyl | Methyl | |
| 1.4.519 | 3-nPropyoxy-5-cyano-Phenyl | Methyl | |
| 1.4.520 | 3-nButoxy-5-cyano-Phenyl | Methyl | |
| 1.4.521 | 3-isoButoxy-5-cyano-Phenyl | Methyl | |
| 1.4.522 | 3-Difluormethoxy-5-cyano-Phenyl | Methyl | |
| 1.4.523 | 3-Trifluormethoxy-5-cyano-Phenyl | Methyl | |
| 1.4.524 | 3-Nitro-5-cyano-Phenyl | Methyl | |
| 1.4.525 | 3-Acetoxy-5-cyano-Phenyl | Methyl | |
| 1.4.526 | 3-Methylsulfanyl-5-cyano-Phenyl | Methyl | |
| 1.4.527 | 3,5-Di(trifluormethyl)-Phenyl | Methyl | |
| 1.4.528 | 3-(Methoxycarbonyl)-5-trifluormethyl-Phenyl | Methyl | |
| 1.4.529 | 3-Methoxy-5-trifluormethyl-Phenyl | Methyl | |
| 1.4.530 | 3-Ethoxy-5-trifluormethyl-Phenyl | Methyl | |
| 1.4.531 | 3-nPropyoxy-5-trifluormethyl-Phenyl | Methyl | |
| 1.4.532 | 3-nButoxy-5-trifluormethyl-Phenyl | Methyl | |
| 1.4.533 | 3-isoButoxy-5-trifluormethyl-Phenyl | Methyl | |
| 1.4.534 | 3-Difluormethoxy-5-trifluormethyl-Phenyl | Methyl | |
| 1.4.535 | 3-Trifluormethoxy-5-trifluormethyl-Phenyl | Methyl | |
| 1.4.536 | 3-Nitro-5-trifluormethyl-Phenyl | Methyl | |
| 1.4.537 | 3-Acetoxy-5-trifluormethyl-Phenyl | Methyl | |
| 1.4.538 | 3-Methylsulfanyl-5-trifluormethyl-Phenyl | Methyl | |
| 1.4.539 | 3-Methoxy-5-(methoxycarbonyl)-Phenyl | Methyl | |
| 1.4.540 | 3-Ethoxy-5-(methoxycarbonyl)-Phenyl | Methyl | |
| 1.4.541 | 3-nPropyoxy-5-(methoxycarbonyl)-Phenyl | Methyl | |
| 1.4.542 | 3-nButoxy-5-(methoxycarbonyl)-Phenyl | Methyl | |
| 1.4.543 | 3-isoButoxy-5-(methoxycarbonyl)-Phenyl | Methyl | |
| 1.4.544 | 3-Difluormethoxy-5-(methoxycarbonyl)-Phenyl | Methyl | |
| 1.4.545 | 3-Trifluormethoxy-5-(methoxycarbonyl)-Phenyl | Methyl | |
| 1.4.546 | 3-Nitro-5-(methoxycarbonyl)-Phenyl | Methyl | |
| 1.4.547 | 3-Acetoxy-5-(methoxycarbonyl)-Phenyl | Methyl | |
| 1.4.548 | 3-Methylsulfanyl-5-(methoxycarbonyl)-Phenyl | Methyl | |
| 1.4.549 | 3,5-Dimethoxy-Phenyl | Methyl | |
| 1.4.550 | 3-Ethoxy-5-methoxy-Phenyl | Methyl | |
| 1.4.551 | 3-nPropyoxy-5-methoxy-Phenyl | Methyl | |
| 1.4.552 | 3-nButoxy-5-methoxy-Phenyl | Methyl | |
| 1.4.553 | 3-isoButoxy-5-methoxy-Phenyl | Methyl | |
| 1.4.554 | 3-tert.Butoxy-5-methoxy-Phenyl | Methyl | |
| 1.4.555 | 3-Difluormethoxy-5-methoxy-Phenyl | Methyl | |
| 1.4.556 | 3-Trifluormethoxy-5-methoxy-Phenyl | Methyl | |
| 1.4.557 | 3-Nitro-5-methoxy-Phenyl | Methyl | |
| 1.4.558 | 3-Acetoxy-5-methoxy-Phenyl | Methyl | |
| 1.4.559 | 3-Methylsulfanyl-5-methoxy-Phenyl | Methyl | |
| 1.4.560 | 3,5-Diethoxy-Phenyl | Methyl | |
| 1.4.561 | 3-nPropyoxy-5-ethoxy-Phenyl | Methyl | |
| 1.4.562 | 3-nButoxy-5-ethoxy-Phenyl | Methyl | |
| 1.4.563 | 3-isoButoxy-5-ethoxy-Phenyl | Methyl | |
| 1.4.564 | 3-Difluormethoxy-5-ethoxy-Phenyl | Methyl | |
| 1.4.565 | 3-Trifluormethoxy-5-ethoxy-Phenyl | Methyl | |
| 1.4.566 | 3-Nitro-5-ethoxy-Phenyl | Methyl | |
| 1.4.567 | 3-Acetoxy-5-ethoxy-Phenyl | Methyl | |
| 1.4.568 | 3-Methylsulfanyl-5-ethoxy-Phenyl | Methyl | |
| 1.4.569 | 3,5-Di(isopropyoxy)-Phenyl | Methyl | |
| 1.4.570 | 3-nButoxy-5-isopropoxy-Phenyl | Methyl | |
| 1.4.571 | 3-isoButoxy-5-isopropoxy-Phenyl | Methyl | |
| 1.4.572 | 3-Difluormethoxy-5-isopropoxy-Phenyl | Methyl | |
| 1.4.573 | 3-Trifluormethoxy-5-isopropoxy-Phenyl | Methyl | |
| 1.4.574 | 3-Nitro-5-isopropoxy-Phenyl | Methyl | |
| 1.4.575 | 3-Acetoxy-5-isopropoxy-Phenyl | Methyl | |
| 1.4.576 | 3-Methylsulfanyl-5-isopropoxy-Phenyl | Methyl | |
| 1.4.577 | 3,5-Di(trifluormethoxy)-Phenyl | Methyl | |
| 1.4.578 | 3-Nitro-5-trifluormethoxy-Phenyl | Methyl | |
| 1.4.579 | 3-Methylsulfanyl-5-trifluormethoxy-Phenyl | Methyl | |
| 1.4.580 | 3,5-Bis(difluormethoxy)-Phenyl | Methyl | |
| 1.4.581 | 3,5-Bis(difluormethoxy)-Phenyl | Ethyl | |
| 1.4.582 | 3,5-Bis(difluormethoxy)-Phenyl | Propyl | |
| 1.4.583 | 3,5-Bis(difluormethoxy)-Phenyl | Butyl | |
| 1.4.584 | 3-Trifluormethoxy-5-difluormethoxy-Phenyl | Methyl | |
| 1.4.585 | 3-Nitro-5-difluormethoxy-Phenyl | Methyl | |
| 1.4.586 | 3-Acetoxy-5-difluormethoxy-Phenyl | Methyl | |
| 1.4.587 | 3-Methylsulfanyl-5-difluormethoxy-Phenyl | Methyl | |
| 1.4.588 | 3-Acetoxy-5-nitro-Phenyl | Methyl | |
| 1.4.589 | 3-Methylsulfanyl-5-nitro-Phenyl | Methyl | |
| 1.4.590 | 3,4-Difluor-Phenyl | Methyl | |
| 1.4.591 | 3,4-Difluor-Phenyl | Ethyl | |
| 1.4.592 | 3,4-Difluor-Phenyl | Propyl | |
| 1.4.593 | 3,4-Difluor-Phenyl | Butyl | |
| 1.4.594 | 3-Chlor-4-fluor-Phenyl | Methyl | |
| 1.4.595 | 3-Chlor-4-fluor-Phenyl | Ethyl | |
| 1.4.596 | 3-Chlor-4-fluor-Phenyl | Propyl | |
| 1.4.597 | 3-Chlor-4-fluor-Phenyl | Butyl | |
| 1.4.598 | 3-Brom-4-fluor-Phenyl | Methyl | |
| 1.4.599 | 3-Methyl-4-fluor-Phenyl | Methyl | |
| 1.4.600 | 3-Methyl-4-fluor-Phenyl | Ethyl | |
| 1.4.601 | 3-Cyclopropyl-4-fluor-Phenyl | Methyl | |
| 1.4.602 | 3-Cyano-4-fluor-Phenyl | Methyl | |
| 1.4.603 | 3-Methoxy-4-fluor-Phenyl | Methyl | |
| 1.4.604 | 3-Ethoxy-4-fluor-Phenyl | Methyl | |
| 1.4.605 | 3-Trifluormethoxy-4-fluor-Phenyl | Methyl | |
| 1.4.606 | 3-Nitro-4-fluor-Phenyl | Methyl | |
| 1.4.607 | 3-Fluor-4-chlor-Phenyl | Methyl | |
| 1.4.608 | 3,4-Dichlor-Phenyl | Methyl | |
| 1.4.609 | 3-Brom-4-chlor-Phenyl | Methyl | |
| 1.4.610 | 3-Methyl-4-chlor-Phenyl | Methyl | |
| 1.4.611 | 3-Cyclopropyl-4-chlor-Phenyl | Methyl | |
| 1.4.612 | 3-Cyano-4-chlor-Phenyl | Methyl | |
| 1.4.613 | 3-Trifluormethyl-4-chlor-Phenyl | Methyl | |
| 1.4.614 | 3-Methoxy-4-chlor-Phenyl | Methyl | |
| 1.4.615 | 3-Ethoxy-4-chlor-Phenyl | Methyl | |
| 1.4.616 | 3-Trifluormethoxy-4-chlor-Phenyl | Methyl | |
| 1.4.617 | 3-Nitro-4-chlor-Phenyl | Methyl | |
| 1.4.618 | 3-Fluor-4-brom-Phenyl | Methyl | |
| 1.4.619 | 3-Chlor-4-brom-Phenyl | Methyl | |
| 1.4.620 | 3,4-Dibrom-Phenyl | Methyl | |
| 1.4.621 | 3-Methyl-4-brom-Phenyl | Methyl | |
| 1.4.622 | 3-Cyclopropyl-4-brom-Phenyl | Methyl | |
| 1.4.623 | 3-Cyano-4-brom-Phenyl | Methyl | |
| 1.4.624 | 3-Trifluormethyl-4-brom-Phenyl | Methyl | |
| 1.4.625 | 3-Methoxy-4-Phenyl | Methyl | |
| 1.4.626 | 3-Ethoxy-4-brom-Phenyl | Methyl | |
| 1.4.627 | 3-Trifluormethoxy-4-brom-Phenyl | Methyl | |
| 1.4.628 | 3-Nitro-4-brom-Phenyl | Methyl | |
| 1.4.629 | 3-Fluor-4-iod-Phenyl | Methyl | |
| 1.4.630 | 3-Chlor-4-iod-Phenyl | Methyl | |
| 1.4.631 | 3-Brom-4-iod-Phenyl | Methyl | |
| 1.4.632 | 3-Methyl-4-iod-Phenyl | Methyl | |
| 1.4.633 | 3-Cyclopropyl-4-iod-Phenyl | Methyl | |
| 1.4.634 | 3-Cyano-4-iod-Phenyl | Methyl | |
| 1.4.635 | 3-Trifluormethyl-4-iod-Phenyl | Methyl | |
| 1.4.636 | 3-Methoxy-4-iod-Phenyl | Methyl | |
| 1.4.637 | 3-Ethoxy-4-iod-Phenyl | Methyl | |
| 1.4.638 | 3-Trifluormethoxy-4-iod-Phenyl | Methyl | |
| 1.4.639 | 3-Nitro-4-iod-Phenyl | Methyl | |
| 1.4.640 | 3-Fluor-4-methyl-Phenyl | Methyl | |
| 1.4.641 | 3-Chlor-4-methyl-Phenyl | Methyl | |
| 1.4.642 | 3-Brom-4-methyl-Phenyl | Methyl | |
| 1.4.643 | 3.4-Dimethyl-Phenyl | Methyl | |
| 1.4.644 | 3.4-Dimethyl-Phenyl | Ethyl | |
| 1.4.645 | 3.4-Dimethyl-Phenyl | Propyl | |
| 1.4.646 | 3.4-Dimethyl-Phenyl | Butyl | |
| 1.4.647 | 3-Cyclopropyl-4-methyl-Phenyl | Methyl | |
| 1.4.648 | 3-Cyano-4-methyl-Phenyl | Methyl | |
| 1.4.649 | 3-Trifluormethyl-4-methyl-Phenyl | Methyl | |
| 1.4.650 | 3-Methoxy-4-methyl-Phenyl | Methyl | |
| 1.4.651 | 3-Ethoxy-4-methyl-Phenyl | Methyl | |
| 1.4.652 | 3-Trifluormethoxy-4-methyl-Phenyl | Methyl | |
| 1.4.653 | 3-Nitro-4-methyl-Phenyl | Methyl | |
| 1.4.654 | 3-Fluor-4-ethyl-Phenyl | Methyl | |
| 1.4.655 | 3-Chlor-4-ethyl-Phenyl | Methyl | |
| 1.4.656 | 3-Brom-4-ethyl-Phenyl | Methyl | |
| 1.4.657 | 3-Methyl-4-ethyl-Phenyl | Methyl | |
| 1.4.658 | 3,4-Diethyl-Phenyl | Methyl | |
| 1.4.659 | 3-Cyclopropyl-4-ethyl-Phenyl | Methyl | |
| 1.4.660 | 3-Cyano-4-ethyl-Phenyl | Methyl | |
| 1.4.661 | 3-Trifluormethyl-4-ethyl-Phenyl | Methyl | |
| 1.4.662 | 3-Methoxy-4-ethyl-Phenyl | Methyl | |
| 1.4.663 | 3-Ethoxy-4-ethyl-Phenyl | Methyl | |
| 1.4.664 | 3-Trifluormethoxy-4-ethyl-Phenyl | Methyl | |
| 1.4.665 | 3-Nitro-4-ethyl-Phenyl | Methyl | |
| 1.4.666 | 3-Fluor-4-propyl-Phenyl | Methyl | |
| 1.4.667 | 3-Chlor-4-propyl-Phenyl | Methyl | |
| 1.4.668 | 3-Brom-4-propyl-Phenyl | Methyl | |
| 1.4.669 | 3-Methyl-4-propyl-Phenyl | Methyl | |
| 1.4.670 | 3-Cyclopropyl-4-propyl-Phenyl | Methyl | |
| 1.4.671 | 3-Cyano-4-propyl-Phenyl | Methyl | |
| 1.4.672 | 3-Trifluormethyl-4-propyl-Phenyl | Methyl | |
| 1.4.673 | 3-Methoxy-4-propyl-Phenyl | Methyl | |
| 1.4.674 | 3-Ethoxy-4-propyl-Phenyl | Methyl | |
| 1.4.675 | 3-Trifluormethoxy-4-propyl-Phenyl | Methyl | |
| 1.4.676 | 3-Nitro-4-propyl-Phenyl | Methyl | |
| 1.4.677 | 3-Fluor-4-isopropyl-Phenyl | Methyl | |
| 1.4.678 | 3-Chlor-4-isopropyl-Phenyl | Methyl | |
| 1.4.679 | 3-Brom-4-isopropyl-Phenyl | Methyl | |
| 1.4.680 | 3-Methyl-4-isopropyl-Phenyl | Methyl | |
| 1.4.681 | 3-Cyclopropyl-4-isopropyl-Phenyl | Methyl | |
| 1.4.682 | 3-Cyano-4-isopropyl-Phenyl | Methyl | |
| 1.4.683 | 3-Trifluormethyl-4-isopropyl-Phenyl | Methyl | |
| 1.4.684 | 3-Methoxy-4-isopropyl-Phenyl | Methyl | |
| 1.4.685 | 3-Ethoxy-4-isopropyl-Phenyl | Methyl | |
| 1.4.686 | 3-Trifluormethoxy-4-isopropyl-Phenyl | Methyl | |
| 1.4.687 | 3-Nitro-4-isopropyl-Phenyl | Methyl | |
| 1.4.688 | 3-Fluor-4-tert.butyl-Phenyl | Methyl | |
| 1.4.689 | 3-Chlor-4-tert.butyl-Phenyl | Methyl | |
| 1.4.690 | 3-Brom-4-tert.butyl-Phenyl | Methyl | |
| 1.4.691 | 3-Methyl-4-tert.butyl-Phenyl | Methyl | |
| 1.4.692 | 3-Cyclopropyl-4-tert.butyl-Phenyl | Methyl | |
| 1.4.693 | 3-Cyano-4-tert.butyl-Phenyl | Methyl | |
| 1.4.694 | 3-Trifluormethyl-4-tert.butyl-Phenyl | Methyl | |
| 1.4.695 | 3-Trifluormethyl-4-tert.butyl-Phenyl | Ethyl | |
| 1.4.696 | 3-Trifluormethyl-4-tert.butyl-Phenyl | Propyl | |
| 1.4.697 | 3-Trifluormethyl-4-tert.butyl-Phenyl | Butyl | |
| 1.4.698 | 3-Methoxy-4-tert.butyl-Phenyl | Methyl | |
| 1.4.699 | 3-Ethoxy-4-tert.butyl-Phenyl | Methyl | |
| 1.4.700 | 3-Trifluormethoxy-4-tert.butyl-Phenyl | Methyl | |
| 1.4.701 | 3-Nitro-4-tert.butyl-Phenyl | Methyl | |
| 1.4.702 | 3-Fluor-4-cyclopropyl-Phenyl | Methyl | |
| 1.4.703 | 3-Chlor-4-cyclopropyl-Phenyl | Methyl | |
| 1.4.704 | 3-Brom-4-cyclopropyl-Phenyl | Methyl | |
| 1.4.705 | 3-Methyl-4-cyclopropyl-Phenyl | Methyl | |
| 1.4.706 | 3-Trifluormethyl-4-cyclopropyl-Phenyl | Methyl | |
| 1.4.707 | 3-Methoxy-4-cyclopropyl-Phenyl | Methyl | |
| 1.4.708 | 3-Ethoxy-4-cyclopropyl-Phenyl | Methyl | |
| 1.4.709 | 3-Trifluormethoxy-4-cyclopropyl-Phenyl | Methyl | |
| 1.4.710 | 3-Fluor-4-methoxycarbonyl-Phenyl | Methyl | |
| 1.4.711 | 3-Chlor-4-methoxycarbonyl-Phenyl | Methyl | |
| 1.4.712 | 3-Brom-4-methoxycarbonyl-Phenyl | Methyl | |
| 1.4.713 | 3-Methyl-4-methoxycarbonyl-Phenyl | Methyl | |
| 1.4.714 | 3-Cyclopropyl-4-methoxycarbonyl-Phenyl | Methyl | |
| 1.4.715 | 3-Cyano-4-methoxycarbonyl-Phenyl | Methyl | |
| 1.4.716 | 3-Trifluormethyl-4-methoxycarbonyl-Phenyl | Methyl | |
| 1.4.717 | 3-Methoxy-4-methoxycarbonyl-Phenyl | Methyl | |
| 1.4.718 | 3-Ethoxy-4-methoxycarbonyl-Phenyl | Methyl | |
| 1.4.719 | 3-Trifluormethoxy-4-methoxycarbonyl-Phenyl | Methyl | |
| 1.4.720 | 3-Nitro-4-m ethoxycarbonyl-Phenyl | Methyl | |
| 1.4.721 | 3-Fluor-4-cyano-Phenyl | Methyl | |
| 1.4.722 | 3-Chlor-4-cyano-Phenyl | Methyl | |
| 1.4.723 | 3-Brom-4-cyano-Phenyl | Methyl | |
| 1.4.724 | 3-Methyl-4-cyano-Phenyl | Methyl | |
| 1.4.725 | 3-Cyclopropyl-4-cyano-Phenyl | Methyl | |
| 1.4.726 | 3,4-Dicyano-Phenyl | Methyl | |
| 1.4.727 | 3-Trifluormethyl-4-cyano-Phenyl | Methyl | |
| 1.4.728 | 3-Trifluormethyl-4-cyano-Phenyl | Ethyl | |
| 1.4.729 | 3-Trifluormethyl-4-cyano-Phenyl | Propyl | |
| 1.4.730 | 3-Trifluormethyl-4-cyano-Phenyl | Butyl | |
| 1.4.731 | 3-Methoxy-4-cyano-Phenyl | Methyl | |
| 1.4.732 | 3-Ethoxy-4-cyano-Phenyl | Methyl | |
| 1.4.733 | 3-Trifluormethoxy-4-cyano-Phenyl | Methyl | |
| 1.4.734 | 3-Nitro-4-cyano-Phenyl | Methyl | |
| 1.4.735 | 3-Fluor-4-methoxy-Phenyl | Methyl | |
| 1.4.736 | 3-Chlor-4-methoxy-Phenyl | Methyl | |
| 1.4.737 | 3-Brom-4-methoxy-Phenyl | Methyl | |
| 1.4.738 | 3-Methyl-4-methoxy-Phenyl | Methyl | |
| 1.4.739 | 3-Cyclopropyl-4-methoxy-Phenyl | Methyl | |
| 1.4.740 | 3-Cyano-4-methoxy-Phenyl | Methyl | |
| 1.4.741 | 3-Trifluormethyl-4-methoxy-Phenyl | Methyl | |
| 1.4.742 | 3,4-Dimethoxy-Phenyl | Methyl | |
| 1.4.743 | 3-Ethoxy-4-methoxy-Phenyl | Methyl | |
| 1.4.744 | 3-Trifluormethoxy-4-methoxy-Phenyl | Methyl | |
| 1.4.745 | 3-Nitro-4-methoxy-Phenyl | Methyl | |
| 1.4.746 | 3-Fluor-4-ethoxy-Phenyl | Methyl | |
| 1.4.747 | 3-Chlor-4-ethoxy-Phenyl | Methyl | |
| 1.4.748 | 3-Chlor-4-ethoxy-Phenyl | Ethyl | |
| 1.4.749 | 3-Chlor-4-ethoxy-Phenyl | Propyl | |
| 1.4.750 | 3-Chlor-4-ethoxy-Phenyl | Butyl | |
| 1.4.751 | 3-Brom-4-ethoxy-Phenyl | Methyl | |
| 1.4.752 | 3-Methyl-4-ethoxy-Phenyl | Methyl | |
| 1.4.753 | 3-Cyclopropyl-4-ethoxy-Phenyl | Methyl | |
| 1.4.754 | 3-Cyano-4-ethoxy-Phenyl | Methyl | |
| 1.4.755 | 3-Trifluormethyl-4-ethoxy-Phenyl | Methyl | |
| 1.4.756 | 3-Methoxy-4-ethoxy-Phenyl | Methyl | |
| 1.4.757 | 2,4-Diethoxy-Phenyl | Methyl | |
| 1.4.758 | 3-Trifluormethoxy-4-ethoxy-Phenyl | Methyl | |
| 1.4.759 | 3-Nitro-4-ethoxy-Phenyl | Methyl | |
| 1.4.760 | 3-Fluor-4-isopropoxy-Phenyl | Methyl | |
| 1.4.761 | 3-Chlor-4-isopropoxy-Phenyl | Methyl | |
| 1.4.762 | 3-Brom-4-isopropoxy-Phenyl | Methyl | |
| 1.4.763 | 3-Methyl-4-isopropoxy-Phenyl | Methyl | |
| 1.4.764 | 3-Cyclopropyl-4-isopropoxy-Phenyl | Methyl | |
| 1.4.765 | 3-Cyano-4-isopropoxy-Phenyl | Methyl | |
| 1.4.766 | 3-Trifluormethyl-4-isopropoxy-Phenyl | Methyl | |
| 1.4.767 | 3-Methoxy-4-isopropoxy-Phenyl | Methyl | |
| 1.4.768 | 3-Ethoxy-4-isopropoxy-Phenyl | Methyl | |
| 1.4.769 | 3-Trifluormethoxy-4-isopropoxy-Phenyl | Methyl | |
| 1.4.770 | 3-Nitro-4-isopropoxy-Phenyl | Methyl | |
| 1.4.771 | 3-Fluor-4-trifluormethoxy-Phenyl | Methyl | |
| 1.4.772 | 3-Chlor-4-trifluormethoxy-Phenyl | Methyl | |
| 1.4.773 | 3-Brom-4-trifluormethoxy-Phenyl | Methyl | |
| 1.4.774 | 3-Methyl-4-trifluormethoxy-Phenyl | Methyl | |
| 1.4.775 | 3-Cyclopropyl-4-trifluormethoxy-Phenyl | Methyl | |
| 1.4.776 | 3-Cyano-4-trifluormethoxy-Phenyl | Methyl | |
| 1.4.777 | 3-Trifluormethyl-4-trifluormethoxy-Phenyl | Methyl | |
| 1.4.778 | 3-Methoxy-4-trifluormethoxy-Phenyl | Methyl | |
| 1.4.779 | 3-Ethoxy-4-trifluormethoxy-Phenyl | Methyl | |
| 1.4.780 | 3,4-Bis(trifluormethoxy)-Phenyl | Methyl | |
| 1.4.781 | 3-Nitro-4-trifluormethoxy-Phenyl | Methyl | |
| 1.4.782 | 3-Fluor-4-difluormethoxy-Phenyl | Methyl | |
| 1.4.783 | 3-Chlor-4-difluormethoxy-Phenyl | Methyl | |
| 1.4.784 | 3-Brom-4-difluormethoxy-Phenyl | Methyl | |
| 1.4.785 | 3-Methyl-4-difluormethoxy-Phenyl | Methyl | |
| 1.4.786 | 3-Cyclopropyl-4-difluormethoxy-Phenyl | Methyl | |
| 1.4.787 | 3-Cyano-4-difluormethoxy-Phenyl | Methyl | |
| 1.4.788 | 3-Trifluormethyl-4-difluornriethoxy-Phenyl | Methyl | |
| 1.4.789 | 3-Methoxy-4-difluormethoxy-Phenyl | Methyl | |
| 1.4.790 | 3-Ethoxy-4-difluormethoxy-Phenyl | Methyl | |
| 1.4.791 | 3-Nitro-4-difluormethoxy-Phenyl | Methyl | |
| 1.4.792 | 3-Fluor-4-nitro-Phenyl | Methyl | |
| 1.4.793 | 3-Chlor-4-nitro-Phenyl | Methyl | |
| 1.4.794 | 3-Brom-4-nitro-Phenyl | Methyl | |
| 1.4.795 | 3-Methyl-4-nitro-Phenyl | Methyl | |
| 1.4.796 | 3-Cyclopropyl-4-nitro-Phenyl | Methyl | |
| 1.4.797 | 3-Cyano-4-nitro-Phenyl | Methyl | |
| 1.4.798 | 3-Trifluormethyl-4-nitro-Phenyl | Methyl | |
| 1.4.799 | 3-Methoxy-4-nitro-Phenyl | Methyl | |
| 1.4.800 | 3-Ethoxy-4-nitro-Phenyl | Methyl | |
| 1.4.801 | 3-Trifluormethoxy-4-nitro-Phenyl | Methyl | |
| 1.4.802 | 3-Fluor-4-methylsulfanylPhenyl | Methyl | |
| 1.4.803 | 3-Chlor-4-methylsulfanyl-Phenyl | Methyl | |
| 1.4.804 | 3-Brom-4-methylsulfanyl-Phenyl | Methyl | |
| 1.4.805 | 3-Methyl-4-methylsulfanyl-Phenyl | Methyl | |
| 1.4.806 | 3-Cyclopropyl-4-methylsulfanyl-Phenyl | Methyl | |
| 1.4.807 | 3-Cyano-4-methylsulfanyl-Phenyl | Methyl | |
| 1.4.808 | 3-Nitro-4-methylsulfanyl-Phenyl | Methyl | |
| 1.4.809 | 3,6-Difluor-Phenyl | Methyl | |
| 1.4.810 | 3,6-Difluor-Phenyl | Ethyl | |
| 1.4.811 | 3,6-Difluor-Phenyl | Propyl | |
| 1.4.812 | 3,6-Difluor-Phenyl | Butyl | |
| 1.4.813 | 3-Chlor-6-fluor-Phenyl | Methyl | |
| 1.4.814 | 3-Brom-6-fluor-Phenyl | Methyl | |
| 1.4.815 | 3-Methyl-6-fluor-Phenyl | Methyl | |
| 1.4.816 | 3-Cyclopropyl-6-fluor-Phenyl | Methyl | |
| 1.4.817 | 3-Cyano-6-fluor-Phenyl | Methyl | |
| 1.4.818 | 3-Methoxy-6-fluor-Phenyl | Methyl | |
| 1.4.819 | 3-Ethoxy-6-fluor-Phenyl | Methyl | |
| 1.4.820 | 3-Trifluormethoxy-6-fluor-Phenyl | Methyl | |
| 1.4.821 | 3-Nitro-6-fluor-Phenyl | Methyl | |
| 1.4.822 | 3-Fluor-6-chlor-Phenyl | Methyl | |
| 1.4.823 | 3-Fluor-6-chlor-Phenyl | Ethyl | |
| 1.4.824 | 3-Fluor-6-chlor-Phenyl | Propyl | |
| 1.4.825 | 3-Fluor-6-chlor-Phenyl | Butyl | |
| 1.4.826 | 3,6-Dichlor-Phenyl | Methyl | |
| 1.4.827 | 3,6-Dichlor-Phenyl | Ethyl | |
| 1.4.828 | 3,6-Dichlor-Phenyl | Propyl | |
| 1.4.829 | 3,6-Dichlor-Phenyl | Butyl | |
| 1.4.830 | 3-Brom-6-chlor-Phenyl | Methyl | |
| 1.4.831 | 3-Methyl-6-chlor-Phenyl | Methyl | |
| 1.4.832 | 3-Ethyl-6-chlor-Phenyl | Methyl | |
| 1.4.833 | 3-Cyclopropyl-6-chlor-Phenyl | Methyl | |
| 1.4.834 | 3-Cyano-6-chlor-Phenyl | Methyl | |
| 1.4.835 | 3-Trifluormethyl-6-chlor-Phenyl | Methyl | |
| 1.4.836 | 3-Methoxy-6-chlor-Phenyl | Methyl | |
| 1.4.837 | 3-Ethoxy-6-chlor-Phenyl | Methyl | |
| 1.4.838 | 3-Trifluormethoxy-6-chlor-Phenyl | Methyl | |
| 1.4.839 | 3-Nitro-6-chlor-Phenyl | Methyl | |
| 1.4.840 | 3-Fluor-6-brom-Phenyl | Methyl | |
| 1.4.841 | 3-Chlor-6-brom-Phenyl | Methyl | |
| 1.4.842 | 3,6-Dibrom-Phenyl | Methyl | |
| 1.4.843 | 3-Methyl-6-brom-Phenyl | Methyl | |
| 1.4.844 | 3-Cyclopropyl-6-brom-Phenyl | Methyl | |
| 1.4.845 | 3-Cyano-6-brom-Phenyl | Methyl | |
| 1.4.846 | 3-Trifluormethyl-6-brom-Phenyl | Methyl | |
| 1.4.847 | 3-Methoxy-6-Phenyl | Methyl | |
| 1.4.848 | 3-Ethoxy-6-brom-Phenyl | Methyl | |
| 1.4.849 | 3-Trifluormethoxy-6-brom-Phenyl | Methyl | |
| 1.4.850 | 3-Nitro-6-brom-Phenyl | Methyl | |
| 1.4.851 | 3-Fluor-6-iod-Phenyl | Methyl | |
| 1.4.852 | 3-Chlor-6-iod-Phenyl | Methyl | |
| 1.4.853 | 3-Brom-6-iod-Phenyl | Methyl | |
| 1.4.854 | 3-Methyl-6-iod-Phenyl | Methyl | |
| 1.4.855 | 3-Cyclopropyl-6-iod-Phenyl | Methyl | |
| 1.4.856 | 3-Cyano-6-iod-Phenyl | Methyl | |
| 1.4.857 | 3-Trifluormethyl-6-iod-Phenyl | Methyl | |
| 1.4.858 | 3-Methoxy-6-iod-Phenyl | Methyl | |
| 1.4.859 | 3-Ethoxy-6-iod-Phenyl | Methyl | |
| 1.4.860 | 3-Trifluormethoxy-6-iod-Phenyl | Methyl | |
| 1.4.861 | 3-Nitro-6-iod-Phenyl | Methyl | |
| 1.4.862 | 3-Fluor-6-methyl-Phenyl | Methyl | |
| 1.4.863 | 3-Chlor-6-methyl-Phenyl | Methyl | |
| 1.4.864 | 3-Brom-6-methyl-Phenyl | Methyl | |
| 1.4.865 | 3.6-Dimethyl-Phenyl | Methyl | |
| 1.4.866 | 3-Cyclopropyl-6-methyl-Phenyl | Methyl | |
| 1.4.867 | 3-Cyano-6-methyl-Phenyl | Methyl | |
| 1.4.868 | 3-Trifluormethyl-6-methyl-Phenyl | Methyl | |
| 1.4.869 | 3-Methoxy-6-methyl-Phenyl | Methyl | |
| 1.4.870 | 3-Ethoxy-6-methyl-Phenyl | Methyl | |
| 1.4.871 | 3-Trifluormethoxy-6-methyl-Phenyl | Methyl | |
| 1.4.872 | 3-Nitro-6-methyl-Phenyl | Methyl | |
| 1.4.873 | 3-Fluor-6-ethyl-Phenyl | Methyl | |
| 1.4.874 | 3-Chlor-6-ethyl-Phenyl | Methyl | |
| 1.4.875 | 3-Brom-6-ethyl-Phenyl | Methyl | |
| 1.4.876 | 3-Methyl-6-ethyl-Phenyl | Methyl | |
| 1.4.877 | 3,6-Diethyl-Phenyl | Methyl | |
| 1.4.878 | 3-Cyclopropyl-6-ethyl-Phenyl | Methyl | |
| 1.4.879 | 3-Cyano-6-ethyl-Phenyl | Methyl | |
| 1.4.880 | 3-Trifluormethyl-6-ethyl-Phenyl | Methyl | |
| 1.4.881 | 3-Methoxy-6-ethyl-Phenyl | Methyl | |
| 1.4.882 | 3-Ethoxy-6-ethyl-Phenyl | Methyl | |
| 1.4.883 | 3-Trifluormethoxy-6-ethyl-Phenyl | Methyl | |
| 1.4.884 | 3-Nitro-6-ethyl-Phenyl | Methyl | |
| 1.4.885 | 3-Fluor-6-propyl-Phenyl | Methyl | |
| 1.4.886 | 3-Chlor-6-propyl-Phenyl | Methyl | |
| 1.4.887 | 3-Brom-6-propyl-Phenyl | Methyl | |
| 1.4.888 | 3-Methyl-6-propyl-Phenyl | Methyl | |
| 1.4.889 | 3-Cyclopropyl-6-propyl-Phenyl | Methyl | |
| 1.4.890 | 3-Cyano-6-propyl-Phenyl | Methyl | |
| 1.4.891 | 3-Trifluormethyl-6-propyl-Phenyl | Methyl | |
| 1.4.892 | 3-Methoxy-6-propyl-Phenyl | Methyl | |
| 1.4.893 | 3-Ethoxy-6-propyl-Phenyl | Methyl | |
| 1.4.894 | 3-Trifluormethoxy-6-propyl-Phenyl | Methyl | |
| 1.4.895 | 3-Nitro-6-propyl-Phenyl | Methyl | |
| 1.4.896 | 3-Fluor-6-isopropyl-Phenyl | Methyl | |
| 1.4.897 | 3-Chlor-6-isopropyl-Phenyl | Methyl | |
| 1.4.898 | 3-Brom-6-isopropyl-Phenyl | Methyl | |
| 1.4.899 | 3-Methyl-6-isopropyl-Phenyl | Methyl | |
| 1.4.900 | 3-Cyclopropyl-6-isopropyl-Phenyl | Methyl | |
| 1.4.901 | 3-Cyano-6-isopropyl-Phenyl | Methyl | |
| 1.4.902 | 3-Trifluormethyl-6-isopropyl-Phenyl | Methyl | |
| 1.4.903 | 3-Ethoxy-6-isopropyl-Phenyl | Methyl | |
| 1.4.904 | 3-Trifluormethoxy-6-isopropyl-Phenyl | Methyl | |
| 1.4.905 | 3-Nitro-6-isopropyl-Phenyl | Methyl | |
| 1.4.906 | 3-Fluor-6-tert.butyl-Phenyl | Methyl | |
| 1.4.907 | 3-Chlor-6-tert.buty-Phenyl | Methyl | |
| 1.4.908 | 3-Brom-6-tert.butyl-Phenyl | Methyl | |
| 1.4.909 | 3-Methyl-6-tert.butyl-Phenyl | Methyl | |
| 1.4.910 | 3-Cyclopropyl-6-tert.butyl-Phenyl | Methyl | |
| 1.4.911 | 3-Cyano-6-tert.butyl-Phenyl | Methyl | |
| 1.4.912 | 3-Trifluormethyl-6-tert.butyl-Phenyl | Methyl | |
| 1.4.913 | 3-Methoxy-6-tert.butyl-Phenyl | Methyl | |
| 1.4.914 | 3-Ethoxy-6-tert.butyl-Phenyl | Methyl | |
| 1.4.915 | 3-Trifluormethoxy-6-tert.butyl-Phenyl | Methyl | |
| 1.4.916 | 3-Nitro-6-tert.butyl-Phenyl | Methyl | |
| 1.4.917 | 3-Fluor-6-cyclopropyl-Phenyl | Methyl | |
| 1.4.918 | 3-Chlor-6-cyclopropyl-Phenyl | Methyl | |
| 1.4.919 | 3-Brom-6-cyclopropyl-Phenyl | Methyl | |
| 1.4.920 | 3-Methyl-6-cyclopropyl-Phenyl | Methyl | |
| 1.4.921 | 3-Cyano-6-cyclopropyl-Phenyl | Methyl | |
| 1.4.922 | 3-Trifluormethyl-6-cyclopropyl-Phenyl | Methyl | |
| 1.4.923 | 3-Methoxy-6-cyclopropyl-Phenyl | Methyl | |
| 1.4.924 | 3-Ethoxy-6-cyclopropyl-Phenyl | Methyl | |
| 1.4.925 | 3-Trifluormethoxy-6-cyclopropyl-Phenyl | Methyl | |
| 1.4.926 | 3-Fluor-6-methoxycarbonyl-Phenyl | Methyl | |
| 1.4.927 | 3-Chlor-6-methoxycarbonyl-Phenyl | Methyl | |
| 1.4.928 | 3-Brom-6-methoxycarbonyl-Phenyl | Methyl | |
| 1.4.929 | 3-Methyl-6-methoxycarbonyl-Phenyl | Methyl | |
| 1.4.930 | 3-Cyclopropyl-6-methoxycarbonyl-Phenyl | Methyl | |
| 1.4.931 | 3-Cyano-6-methoxycarbonyl-Phenyl | Methyl | |
| 1.4.932 | 3-Trifluormethyl-6-nriethoxycarbonyl-Phenyl | Methyl | |
| 1.4.933 | 3-Methoxy-6-methoxycarbonyl-Phenyl | Methyl | |
| 1.4.934 | 3-Ethoxy-6-methoxycarbonyl-Phenyl | Methyl | |
| 1.4.935 | 3-Trifluormethoxy-6-methoxycarbonyl-Phenyl | Methyl | |
| 1.4.936 | 3-Nitro-6-methoxycarbonyl-Phenyl | Methyl | |
| 1.4.937 | 3-Fluor-6-cyano-Phenyl | Methyl | |
| 1.4.938 | 3-Chlor-6-cyano-Phenyl | Methyl | |
| 1.4.939 | 3-Brom-6-cyano-Phenyl | Methyl | |
| 1.4.940 | 3-Methyl-6-cyano-Phenyl | Methyl | |
| 1.4.941 | 3-Cyclopropyl-6-cyano-Phenyl | Methyl | |
| 1.4.942 | 3-Cyano-6-cyano-Phenyl | Methyl | |
| 1.4.943 | 3-Trifluormethyl-6-cyano-Phenyl | Methyl | |
| 1.4.944 | 3-Methoxy-6-cyano-Phenyl | Methyl | |
| 1.4.945 | 3-Ethoxy-6-cyano-Phenyl | Methyl | |
| 1.4.946 | 3-Trifluormethoxy-6-cyano-Phenyl | Methyl | |
| 1.4.947 | 3-Nitro-6-cyano-Phenyl | Methyl | |
| 1.4.948 | 3-Fluor-6-methoxy-Phenyl | Methyl | |
| 1.4.949 | 3-Chlor-6-methoxy-Phenyl | Methyl | |
| 1.4.950 | 3-Brom-6-methoxy-Phenyl | Methyl | |
| 1.4.951 | 3-Methyl-6-methoxy-Phenyl | Methyl | |
| 1.4.952 | 3-Cyclopropyl-6-methoxy-Phenyl | Methyl | |
| 1.4.953 | 3-Cyano-6-methoxy-Phenyl | Methyl | |
| 1.4.954 | 3-Trifluormethyl-6-methoxy-Phenyl | Methyl | |
| 1.4.955 | 3,6-Dimethoxy-Phenyl | Methyl | |
| 1.4.956 | 3-Ethoxy-6-methoxy-Phenyl | Methyl | |
| 1.4.957 | 3-Trifluormethoxy-6-methoxy-Phenyl | Methyl | |
| 1.4.958 | 3-Nitro-6-methoxy-Phenyl | Methyl | |
| 1.4.959 | 3-Fluor-6-ethoxy-Phenyl | Methyl | |
| 1.4.960 | 3-Chlor-6-ethoxy-Phenyl | Methyl | |
| 1.4.961 | 3-Brom-6-ethoxy-Phenyl | Methyl | |
| 1.4.962 | 3-Methyl-6-ethoxy-Phenyl | Methyl | |
| 1.4.963 | 3-Cyclopropyl-6-ethoxy-Phenyl | Methyl | |
| 1.4.964 | 3-Cyano-6-ethoxy-Phenyl | Methyl | |
| 1.4.965 | 3-Trifluormethyl-6-ethoxy-Phenyl | Methyl | |
| 1.4.966 | 3-Methoxy-6-ethoxy-Phenyl | Methyl | |
| 1.4.967 | 2,6-Diethoxy-Phenyl | Methyl | |
| 1.4.968 | 3-Trifluormethoxy-6-ethoxy-Phenyl | Methyl | |
| 1.4.969 | 3-Nitro-6-ethoxy-Phenyl | Methyl | |
| 1.4.970 | 3-Fluor-6-isopropoxy-Phenyl | Methyl | |
| 1.4.971 | 3-Chlor-6-isopropoxy-Phenyl | Methyl | |
| 1.4.972 | 3-Brom-6-isopropoxy-Phenyl | Methyl | |
| 1.4.973 | 3-Methyl-6-isopropoxy-Phenyl | Methyl | |
| 1.4.974 | 3-Cyclopropyl-6-isopropoxy-Phenyl | Methyl | |
| 1.4.975 | 3-Cyano-6-isopropoxy-Phenyl | Methyl | |
| 1.4.976 | 3-Trifluormethyl-6-isopropoxy-Phenyl | Methyl | |
| 1.4.977 | 3-Methoxy-6-isopropoxy-Phenyl | Methyl | |
| 1.4.978 | 3-Ethoxy-6-isopropoxy-Phenyl | Methyl | |
| 1.4.979 | 3-Trifluormethoxy-6-isopropoxy-Phenyl | Methyl | |
| 1.4.980 | 3-Nitro-6-isopropoxy-Phenyl | Methyl | |
| 1.4.981 | 3-Fluor-6-trifluormethoxy-Phenyl | Methyl | |
| 1.4.982 | 3-Chlor-6-trifluormethoxy-Phenyl | Methyl | |
| 1.4.983 | 3-Brom-6-trifluormethoxy-Phenyl | Methyl | |
| 1.4.984 | 3-Methyl-6-trifluormethoxy-Phenyl | Methyl | |
| 1.4.985 | 3-Cyclopropyl-6-trifluormethoxy-Phenyl | Methyl | |
| 1.4.986 | 3-Cyano-6-trifluormethoxy-Phenyl | Methyl | |
| 1.4.987 | 3-Trifluormethyl-6-trifluormethoxy-Phenyl | Methyl | |
| 1.4.988 | 3-Methoxy-6-trifluormethoxy-Phenyl | Methyl | |
| 1.4.989 | 3-Ethoxy-6-trifluormethoxy-Phenyl | Methyl | |
| 1.4.990 | 3,6-Bis(trifluormethoxy)-Phenyl | Methyl | |
| 1.4.991 | 3-Nitro-6-trifluormethoxy-Phenyl | Methyl | |
| 1.4.992 | 3-Fluor-6-difluormethoxy-Phenyl | Methyl | |
| 1.4.993 | 3-Chlor-6-difluormethoxy-Phenyl | Methyl | |
| 1.4.994 | 3-Brom-6-difluormethoxy-Phenyl | Methyl | |
| 1.4.995 | 3-Methyl-6-difluormethoxy-Phenyl | Methyl | |
| 1.4.996 | 3-Cyclopropyl-6-difluormethoxy-Phenyl | Methyl | |
| 1.4.997 | 3-Cyano-6-difluormethoxy-Phenyl | Methyl | |
| 1.4.998 | 3-Trifluormethyl-6-difluormethoxy-Phenyl | Methyl | |
| 1.4.999 | 3-Methoxy-6-difluormethoxy-Phenyl | Methyl | |
| 1.4.1000 | 3-Ethoxy-6-difluormethoxy-Phenyl | Methyl | |
| 1.4.1001 | 3-Trifluormethoxy-6-difluormethoxy-Phenyl | Methyl | |
| 1.4.1002 | 3-Nitro-6-difluormethoxy-Phenyl | Methyl | |
| 1.4.1003 | 3-Fluor-6-nitro-Phenyl | Methyl | |
| 1.4.1004 | 3-Chlor-6-nitro-Phenyl | Methyl | |
| 1.4.1005 | 3-Brom-6-nitro-Phenyl | Methyl | |
| 1.4.1006 | 3-Methyl-6-nitro-Phenyl | Methyl | |
| 1.4.1007 | 3-Cyclopropyl-6-nitro-Phenyl | Methyl | |
| 1.4.1008 | 3-Trifluormethyl-6-nitro-Phenyl | Methyl | |
| 1.4.1009 | 3-Methoxy-6-nitro-Phenyl | Methyl | |
| 1.4.1010 | 3-Ethoxy-6-nitro-Phenyl | Methyl | |
| 1.4.1011 | 3-Trifluormethoxy-6-nitro-Phenyl | Methyl | |
| 1.4.1012 | 3-Fluor-6-methylsulfanylPhenyl | Methyl | |
| 1.4.1013 | 3-Chlor-6-methylsulfanyl-Phenyl | Methyl | |
| 1.4.1014 | 3-Brom-6-methylsulfanyl-Phenyl | Methyl | |
| 1.4.1015 | 3-Methyl-6-methylsulfanyl-Phenyl | Methyl | |
| 1.4.1016 | 3-Cyclopropyl-6-methylsulfanyl-Phenyl | Methyl | |
| 1.4.1017 | 3-Cyano-6-methylsulfanyl-Phenyl | Methyl | |
| 1.4.1018 | 3-Trifluormethyl-6-methylsulfanyl-Phenyl | Methyl | |
| 1.4.1019 | 2,3,4-Trifluor-Phenyl | Methyl | |
| 1.4.1020 | 2,3,4-Trichlor-Phenyl | Methyl | |
| 1.4.1021 | 2,3.4-Trimethyl-Phenyl | Methyl | |
| 1.4.1022 | 2-Fluor-2-chlor-5-trifluormethyl-Phenyl | Methyl | |
| 1.4.1023 | 2,3,5-Trifluor-Phenyl | Methyl | |
| 1.4.1024 | 2,3,5-Trichlor-Phenyl | Methyl | |
| 1.4.1025 | 2,3,5-Trimethyl-Phenyl | Methyl | |
| 1.4.1026 | 2,3-Dichlor-5-methoxy-Phenyl | Methyl | |
| 1.4.1027 | 2,3,6-Trifluor-Phenyl | Methyl | |
| 1.4.1028 | 2,3,6-Trichlor-Phenyl | Methyl | |
| 1.4.1029 | 2,3,6-Trimethyl-Phenyl | Methyl | |
| 1.4.1030 | 3,4,5-Trifluor-Phenyl | Methyl | |
| 1.4.1031 | 3,4,5-Trichlor-Phenyl | Methyl | |
| 1.4.1032 | 3,4,5-Trimethyl-Phenyl | Methyl | |
| 1.4.1033 | 3,5-Dimethyl-4-fluor-Phenyl | Methyl | |
| 1.4.1034 | 3,5-Dichlor-4-methoxy-Phenyl | Methyl | |
| 1.4.1035 | 3,5-Difluor-4-chlor-Phenyl | Methyl | |
| 1.4.1036 | 3,5-Dichlor-4-hydroxy-Phenyl | Methyl | |
| 1.4.1037 | 3,5-Trifluormethyl-4-chlor-Phenyl | Methyl | |
| 1.4.1038 | 3,4,6-Trifluor-Phenyl | Methyl | |
| 1.4.1039 | 3,4,6-Trichlor-Phenyl | Methyl | |
| 1.4.1040 | 3,4,6-Trimethyl-Phenyl | Methyl | |
| 1.4.1041 | Pentafluorphenyl | Methyl | |

**Tabelle 1.5: Erfindungsgemäße Verbindungen der allgemeinen Formel (I), worin W* für COOH, R¹ und R² für Wasserstoffr stehen, und Aryl den Rest**

| | | | |
|---|---|---|---|
| | 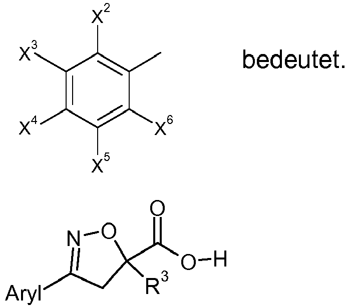 | | |

| Nr. | Aryl | R³ | Physikalische Daten |
|---|---|---|---|
| 1.5.001 | 3-Fluor-Phenyl | Hydroxymethyl | |
| 1.5.002 | 3-Fluor-Phenyl | 1-Hydroxyethyl | |
| 1.5.003 | 3-Fluor-Phenyl | 1-Hydroxypropyl | |
| 1.5.004 | 3-Fluor-Phenyl | (1-Hydroxy-2-methylpropyl) | |
| 1.5.005 | 3-Fluor-Phenyl | Methoxymethyl | |
| 1.5.006 | 3-Fluor-Phenyl | 2-Methoxyethyl | |
| 1.5.007 | 3-Chlor-Phenyl | Hydroxymethyl | |
| 1.5.008 | 3-Chlor-Phenyl | 1-Hydroxyethyl | |
| 1.5.009 | 3-Chlor-Phenyl | 1-Hydroxypropyl | |
| 1.5.010 | 3-Chlor-Phenyl | (1-Hydroxy-2-methylpropyl) | |
| 1.5.011 | 3-Chlor-Phenyl | Methoxymethyl | |
| 1.5.012 | 3-Chlor-Phenyl | 2-Methoxyethyl | |
| 1.5.013 | 3-Brom-Phenyl | Hydroxymethyl | |
| 1.5.014 | 3-Brom-Phenyl | 1-Hydroxyethyl | |
| 1.5.015 | 3-Iod-Phenyl | Hydroxymethyl | |
| 1.5.016 | 3-Iod-Phenyl | 1-Hydroxyethyl | |
| 1.5.017 | 3-Methyl-Phenyl | Hydroxymethyl | |
| 1.5.018 | 3-Methyl-Phenyl | 1-Hydroxyethyl | |
| 1.5.019 | 3-Ethyl-Phenyl | Hydroxymethyl | |
| 1.5.020 | 3-Propyl-Phenyl | Hydroxymethyl | |
| 1.5.021 | 3-isoPropyl-Phenyl | Hydroxymethyl | |
| 1.5.022 | 3-nButyl-Phenyl | Hydroxymethyl | |
| 1.5.023 | 3-iButyl-Phenyl | Hydroxymethyl | |
| 1.5.024 | 3-tert.Butyl-Phenyl | Hyd roxymethyl | |
| 1.5.025 | 3-Cyclopropyl-Phenyl | Hydroxymethyl | |
| 1.5.026 | 3-Cyclobutyl-Phenyl | Hydroxymethyl | |
| 1.5.027 | 3-Cyclopentyl-Phenyl | Hydroxymethyl | |
| 1.5.028 | 3-Vinyl-Phenyl | Hydroxymethyl | |
| 1.5.029 | 3-Ethinyl-Phenyl | Hydroxymethyl | |
| 1.5.030 | 3-Cyano-Phenyl | Hydroxymethyl | |
| 1.5.031 | 3-Trifluormethyl-Phenyl | Hydroxymethyl | |
| 1.5.032 | 3-Difluormethyl-Phenyl | Hydroxymethyl | |
| 1.5.033 | 3-(Hydroxycarbonyl)-Phenyl | Hydroxymethyl | |
| 1.5.034 | 3-(Methoxycarbony)l-Phenyl | Hydroxymethyl | |
| 1.5.035 | 3-(Ethoxycarbonyl)-Phenyl | Hydroxymethyl | |
| 1.5.036 | 3-Hydroxymethyl-Phenyl | Hydroxymethyl | |
| 1.5.037 | 3-Carbamoyl-Phenyl | Hydroxymethyl | |
| 1.5.038 | 3-Hydroxy-Phenyl- | Hydroxymethyl | |
| 1.5.039 | 3-Methoxy-Phenyl | Hydroxymethyl | |
| 1.5.040 | 3-Ethoxy-Phenyl | Hydroxymethyl | |
| 1.5.041 | 3-Propyloxy-Phenyl | Hydroxymethyl | |
| 1.5.042 | 3-isoPropyloxy-Phenyl | Hydroxymethyl | |
| 1.5.043 | 3-nButyloxy-Phenyl | Hydroxymethyl | |
| 1.5.044 | 3-iButyloxy-Phenyl | Hydroxymethyl | |
| 1.5.045 | 3-tButyloxy-Phenyl | Hydroxymethyl | |
| 1.5.046 | 3-Difluormethoxy-Phenyl | Hydroxymethyl | |
| 1.5.047 | 3-Trifluormethoxy-Phenyl | Hydroxymethyl | |
| 1.5.048 | 3-(2,2,2-Trifluorethoxy)-Phenyl | Hydroxymethyl | |
| 1.5.049 | 3-(2-Chlorethoxy)-Phenyl | Hydroxymethyl | |
| 1.5.050 | 3-(2-Hydroxyethoxy)-Phenyl- | Hydroxymethyl | |
| 1.5.051 | 3-(2-Methoxyethoxy)-Phenyl- | Hydroxymethyl | |
| 1.5.052 | 3-[(tert-Butoxycarbonyl)oxy]-Phenyl | Hydroxymethyl | |
| 1.5.053 | 3-Nitro-Phenyl | Hydroxymethyl | |
| 1.5.054 | 3-Acetoxy-Phenyl | Hydroxymethyl | |
| 1.5.055 | {3-[(tert-Butoxycarbonyl)amino]-Phenyl}- | Hydroxymethyl | |
| 1.5.056 | 3-Methylsulfanyl-Phenyl | Hydroxymethyl | |
| 1.5.057 | 3-Ethylsulfanyl-Phenyl | Hydroxymethyl | |
| 1.5.058 | 3-(Pentafluor-lambda⁶-sulfanyl)-Phenyl | Hydroxymethyl | |
| 1.5.059 | 2,3-Difluor-Phenyl | Hydroxymethyl | |
| 1.5.060 | 2,3-Difluor-Phenyl | 1-Hydroxyethyl | |
| 1.5.061 | 2,3-Difluor-Phenyl | 1-Hydroxypropyl | |
| 1.5.062 | 2,3-Difluor-Phenyl | (1-Hydroxy-2-methylpropyl) | |
| 1.5.063 | 2,3-Difluor-Phenyl | Methoxymethyl | |
| 1.5.064 | 2,3-Difluor-Phenyl | 2-Methoxyethyl | |
| 1.5.065 | 2-Chlor-3-fluor-Phenyl | Hydroxymethyl | |
| 1.5.066 | 2-Brom-3-fluor-Phenyl | Hydroxymethyl | |
| 1.5.067 | 2-Methyl-3-fluor-Phenyl | Hydroxymethyl | |
| 1.5.068 | 2-Ethyl-3-fluor-Phenyl | Hydroxymethyl | |
| 1.5.069 | 2-Cyclopropyl-3-fluor-Phenyl | Hydroxymethyl | |
| 1.5.070 | 2-Vinyl-3-fluor-Phenyl | Hydroxymethyl | |
| 1.5.071 | 2-Ethinyl-3-fluor-Phenyl | Hydroxymethyl | |
| 1.5.072 | 2-Cyano-3-fluor-Phenyl | Hydroxymethyl | |
| 1.5.073 | 2-Methoxy-3-fluor-Phenyl | Hydroxymethyl | |
| 1.5.074 | 2-Ethoxy-3-fluor-Phenyl | Hydroxymethyl | |
| 1.5.075 | 2-Trifluormethoxy-3-fluor-Phenyl | Hydroxymethyl | |
| 1.5.076 | 2-Nitro-3-fluor-Phenyl | Hydroxymethyl | |
| 1.5.077 | 2-Fluor-3-chlor-Phenyl | Hydroxymethyl | |
| 1.5.078 | 2,3-Dichlor-Phenyl | Hydroxymethyl | |
| 1.5.079 | 2,3-Dichlor-Phenyl | 1-Hydroxyethyl | |
| 1.5.080 | 2,3-Dichlor-Phenyl | 1-Hydroxypropyl | |
| 1.5.081 | 2,3-Dichlor-Phenyl | (1-Hydroxy-2-methylpropyl) | |
| 1.5.082 | 2,3-Dichlor-Phenyl | Methoxymethyl | |
| 1.5.083 | 2,3-Dichlor-Phenyl | 2-Methoxyethyl | |
| 1.5.084 | 2-Brom-3-chlor-Phenyl | Hydroxymethyl | |
| 1.5.085 | 2-Methyl-3-chlor-Phenyl | Hydroxymethyl | |
| 1.5.086 | 2-Ethyl-3-chlor-Phenyl | Hydroxymethyl | |
| 1.5.087 | 2-Cyclopropyl-3-chlor-Phenyl | Hydroxymethyl | |
| 1.5.088 | 2-Vinyl-3-chlor-Phenyl | Hydroxymethyl | |
| 1.5.089 | 2-Ethinyl-3-chlor-Phenyl | Hydroxymethyl | |
| 1.5.090 | 2-Cyano-3-chlor-Phenyl | Hydroxymethyl | |
| 1.5.091 | 2-Trifluormethyl-2-chlor-Phenyl | Hydroxymethyl | |
| 1.5.092 | 2-Methoxy-3-chlor-Phenyl | Hydroxymethyl | |
| 1.5.093 | 2-Ethoxy-3-chlor-Phenyl | Hydroxymethyl | |
| 1.5.094 | 2-Trifluormethoxy-3-chlor-Phenyl | Hydroxymethyl | |
| 1.5.095 | 2-Nitro-3-chlor-Phenyl | Hydroxymethyl | |
| 1.5.096 | 2-Fluor-3-brom-Phenyl | Hydroxymethyl | |
| 1.5.097 | 2-Chlor-3-brom-Phenyl | Hydroxymethyl | |
| 1.5.098 | 2,3-Dibrom-Phenyl | Hydroxymethyl | |
| 1.5.099 | 2-Methyl-3-brom-Phenyl | Hydroxymethyl | |
| 1.5.100 | 2-Ethyl-3-brom-Phenyl | Hydroxymethyl | |
| 1.5.101 | 2-Cyclopropyl-3-brom-Phenyl | Hydroxymethyl | |
| 1.5.102 | 2-Vinyl-3-brom-Phenyl | Hydroxymethyl | |
| 1.5.103 | 2-Ethinyl-3-brom-Phenyl | Hydroxymethyl | |
| 1.5.104 | 2-Cyano-3-brom-Phenyl | Hydroxymethyl | |
| 1.5.105 | 2-Trifluormethyl-3-brom-Phenyl | Hydroxymethyl | |
| 1.5.106 | 2-Methoxy-3-Phenyl | Hydroxymethyl | |
| 1.5.107 | 2-Ethoxy-3-brom-Phenyl | Hydroxymethyl | |
| 1.5.108 | 2-Trifluormethoxy-3-brom-Phenyl | Hydroxymethyl | |
| 1.5.109 | 2-Nitro-3-brom-Phenyl | Hydroxymethyl | |
| 1.5.110 | 2-Fluor-3-iod-Phenyl | Hydroxymethyl | |
| 1.5.111 | 2-Chlor-3-iod-Phenyl | Hydroxymethyl | |
| 1.5.112 | 2-Brom-3-iod-Phenyl | Hydroxymethyl | |
| 1.5.113 | 2-Methyl-3-iod-Phenyl | Hydroxymethyl | |
| 1.5.114 | 2-Ethyl-3-iod-Phenyl | Hydroxymethyl | |
| 1.5.115 | 2-Cyclopropyl-3-iod-Phenyl | Hydroxymethyl | |
| 1.5.116 | 2-Vinyl-3-iod-Phenyl | Hydroxymethyl | |
| 1.5.117 | 2-Ethinyl-3-iod-Phenyl | Hydroxymethyl | |
| 1.5.118 | 2-Cyano-3-iod-Phenyl | Hydroxymethyl | |
| 1.5.119 | 2-Trifluormethyl-3-iod-Phenyl | Hydroxymethyl | |
| 1.5.120 | 2-Methoxy-3-iod-Phenyl | Hydroxymethyl | |
| 1.5.121 | 2-Ethoxy-3-iod-Phenyl | Hydroxymethyl | |
| 1.5.122 | 2-Trifluormethoxy-3-iod-Phenyl | Hydroxymethyl | |
| 1.5.123 | 2-Nitro-3-iod-Phenyl | Hydroxymethyl | |
| 1.5.124 | 2-Fluor-3-methyl-Phenyl | Hydroxymethyl | |
| 1.5.125 | 2-Fluor-3-methyl-Phenyl | 1-Hydroxyethyl | |
| 1.5.126 | 2-Fluor-3-methyl-Phenyl | 1-Hydroxypropyl | |
| 1.5.127 | 2-Fluor-3-methyl-Phenyl | (1-Hydroxy-2-methylpropyl) | |
| 1.5.128 | 2-Fluor-3-methyl-Phenyl | Methoxymethyl | |
| 1.5.129 | 2-Fluor-3-methyl-Phenyl | 2-Methoxyethyl | |
| 1.5.130 | 2-Chlor-3-methyl-Phenyl | Hydroxymethyl | |
| 1.5.131 | 2-Chlor-3-methyl-Phenyl | 1-Hydroxyethyl | |
| 1.5.132 | 2-Chlor-3-methyl-Phenyl | 1-Hydroxypropyl | |
| 1.5.133 | 2-Chlor-3-methyl-Phenyl | (1-Hydroxy-2-methylpropyl) | |
| 1.5.134 | 2-Chlor-3-methyl-Phenyl | Methoxymethyl | |
| 1.5.135 | 2-Chlor-3-methyl-Phenyl | 2-Methoxyethyl | |
| 1.5.136 | 2-Brom-3-methyl-Phenyl | Hydroxymethyl | |
| 1.5.137 | 2,3-Dimethyl-Phenyl | Hydroxymethyl | |
| 1.5.138 | 2,3-Dimethyl-Phenyl | 1-Hydroxyethyl | |
| 1.5.139 | 2,3-Dimethyl-Phenyl | 1-Hydroxypropyl | |
| 1.5.140 | 2,3-Dimethyl-Phenyl | (1-Hydroxy-2-methylpropyl) | |
| 1.5.141 | 2,3-Dimethyl-Phenyl | Methoxymethyl | |
| 1.5.142 | 2,3-Dimethyl-Phenyl | 2-Methoxyethyl | |
| 1.5.143 | 2-Ethyl-3-methyl-Phenyl | Hydroxymethyl | |
| 1.5.144 | 2-Cyclopropyl-3-methyl-Phenyl | Hydroxymethyl | |
| 1.5.145 | 2-Vinyl-3-methyl-Phenyl | Hydroxymethyl | |
| 1.5.146 | 2-Ethinyl-3-methyl-Phenyl | Hydroxymethyl | |
| 1.5.147 | 2-Cyano-3-methyl-Phenyl | Hydroxymethyl | |
| 1.5.148 | 2-Trifluormethyl-3-methyl-Phenyl | Hydroxymethyl | |
| 1.5.149 | 2-Methoxy-3-methyl-Phenyl | Hydroxymethyl | |
| 1.5.150 | 2-Ethoxy-3-methyl-Phenyl | Hydroxymethyl | |
| 1.5.151 | 2-Trifluormethoxy-3-methyl-Phenyl | Hydroxymethyl | |
| 1.5.152 | 2-Nitro-3-methyl-Phenyl | Hydroxymethyl | |
| 1.5.153 | 2-Fluor-3-ethyl-Phenyl | Hydroxymethyl | |
| 1.5.154 | 2-Chlor-3-ethyl-Phenyl | Hydroxymethyl | |
| 1.5.155 | 2-Brom-3-ethyl-Phenyl | Hydroxymethyl | |
| 1.5.156 | 2-Methyl-3-ethyl-Phenyl | Hydroxymethyl | |
| 1.5.157 | 2,3-Diethyl-Phenyl | Hydroxymethyl | |
| 1.5.158 | 2-Cyclopropyl-3-ethyl-Phenyl | Hydroxymethyl | |
| 1.5.159 | 2-Vinyl-3-ethyl-Phenyl | Hydroxymethyl | |
| 1.5.160 | 2-Ethinyl-3-ethyl-Phenyl | Hydroxymethyl | |
| 1.5.161 | 2-Cyano-3-ethyl-Phenyl | Hydroxymethyl | |
| 1.5.162 | 2-Trifluormethyl-3-ethyl-Phenyl | Hydroxymethyl | |
| 1.5.163 | 2-Methoxy-3-ethyl-Phenyl | Hydroxymethyl | |
| 1.5.164 | 2-Ethoxy-3-ethyl-Phenyl | Hydroxymethyl | |
| 1.5.165 | 2-Trifluormethoxy-3-ethyl-Phenyl | Hydroxymethyl | |
| 1.5.166 | 2-Nitro-3-ethyl-Phenyl | Hydroxymethyl | |
| 1.5.167 | 2-Fluor-3-propyl-Phenyl | Hydroxymethyl | |
| 1.5.168 | 2-Chlor-3-propyl-Phenyl | Hydroxymethyl | |
| 1.5.169 | 2-Brom-3-propyl-Phenyl | Hydroxymethyl | |
| 1.5.170 | 2-Methyl-3-propyl-Phenyl | Hydroxymethyl | |
| 1.5.171 | 2-Methyl-3-propyl-Phenyl | Hydroxymethyl | |
| 1.5.172 | 2-Cyclopropyl-3-propyl-Phenyl | Hydroxymethyl | |
| 1.5.173 | 2-Vinyl-3-propyl-Phenyl | Hydroxymethyl | |
| 1.5.174 | 2-Ethinyl-3propyl-Phenyl | Hydroxymethyl | |
| 1.5.175 | 2-Cyano-3-propyl-Phenyl | Hydroxymethyl | |
| 1.5.176 | 2-Trifluormethyl-3-propyl-Phenyl | Hydroxymethyl | |
| 1.5.177 | 2-Methoxy-3-propyl-Phenyl | Hydroxymethyl | |
| 1.5.178 | 2-Ethoxy-3-propyl-Phenyl | Hydroxymethyl | |
| 1.5.179 | 2-Trifluormethoxy-3-propyl-Phenyl | Hydroxymethyl | |
| 1.5.180 | 2-Nitro-3-propyl-Phenyl | Hydroxymethyl | |
| 1.5.181 | 2-Fluor-3-isopropyl-Phenyl | Hydroxymethyl | |
| 1.5.182 | 2-Chlor-3-isopropyl-Phenyl | Hydroxymethyl | |
| 1.5.183 | 2-Brom-3-isopropyl-Phenyl | Hydroxymethyl | |
| 1.5.184 | 2-Methyl-3-isopropyl-Phenyl | Hydroxymethyl | |
| 1.5.185 | 2-Ethyl-3-isopropyl-Phenyl | Hydroxymethyl | |
| 1.5.186 | 2-Cyclopropyl-3-isopropyl-Phenyl | Hydroxymethyl | |
| 1.5.187 | 2-Vinyl-3-isopropyl-Phenyl | Hydroxymethyl | |
| 1.5.188 | 2-Ethinyl-3-isopropyl-Phenyl | Hydroxymethyl | |
| 1.5.189 | 2-Cyano-3-isopropyl-Phenyl | Hydroxymethyl | |
| 1.5.190 | 2-Trifluormethyl-3-isopropyl-Phenyl | Hydroxymethyl | |
| 1.5.191 | 2-Methoxy-3-isopropyl-Phenyl | Hydroxymethyl | |
| 1.5.192 | 2-Ethoxy-3-isopropyl-Phenyl | Hydroxymethyl | |
| 1.5.193 | 2-Trifluormethoxy-3-isopropyl-Phenyl | Hydroxymethyl | |
| 1.5.194 | 2-Nitro-3-isopropyl-Phenyl | Hydroxymethyl | |
| 1.5.195 | 2-Fluor-3-tert.butyl-Phenyl | Hydroxymethyl | |
| 1.5.196 | 2-Chlor-3-tert.buty-Phenyl | Hydroxymethyl | |
| 1.5.197 | 2-Brom-3-tert.butyl-Phenyl | Hydroxymethyl | |
| 1.5.198 | 2-Methyl-3-tert.butyl-Phenyl | Hydroxymethyl | |
| 1.5.199 | 2-Ethyl-3-tert.butyl-Phenyl | Hydroxymethyl | |
| 1.5.200 | 2-Cyclopropyl-3-tert.butyl-Phenyl | Hydroxymethyl | |
| 1.5.201 | 2-Vinyl-3-tert.butyl-Phenyl | Hydroxymethyl | |
| 1.5.202 | 2-Ethinyl-3-tert.butyl-Phenyl | Hydroxymethyl | |
| 1.5.203 | 2-Cyano-3-tert.butyl-Phenyl | Hydroxymethyl | |
| 1.5.204 | 2-Trifluormethyl-3-tert.butyl-Phenyl | Hydroxymethyl | |
| 1.5.205 | 2-Methoxy-3-tert.butyl-Phenyl | Hydroxymethyl | |
| 1.5.206 | 2-Ethoxy-3-tert.butyl-Phenyl | Hydroxymethyl | |
| 1.5.207 | 2-Trifluormethoxy-3-tert.butyl-Phenyl | Hydroxymethyl | |
| 1.5.208 | 2-Nitro-3-tert.butyl-Phenyl | Hydroxymethyl | |
| 1.5.209 | 2-Fluor-3-hydroxymethyl-Phenyl | Hydroxymethyl | |
| 1.5.210 | 2-Chlor-3-hydroxymethyl-Phenyl | Hydroxymethyl | |
| 1.5.211 | 2-Brom-3-hydroxymethyl-Phenyl | Hydroxymethyl | |
| 1.5.212 | 2-Methyl-3-hydroxymethyl-Phenyl | Hydroxymethyl | |
| 1.5.213 | 2-Ethyl-3-hydroxymethyl-Phenyl | Hydroxymethyl | |
| 1.5.214 | 2-Cyclopropyl-3-hydroxymethyl-Phenyl | Hydroxymethyl | |
| 1.5.215 | 2-Vinyl-3-hydroxymethyl-Phenyl | Hydroxymethyl | |
| 1.5.216 | 2-Ethinyl-3-hydroxymethyl-Phenyl | Hydroxymethyl | |
| 1.5.217 | 2-Cyano-3-hydroxymethyl-Phenyl | Hydroxymethyl | |
| 1.5.218 | 2-Trifluormethyl-3-hydroxymethyl-Phenyl | Hydroxymethyl | |
| 1.5.219 | 2-Methoxy-3-hydroxymethyl-Phenyl | Hydroxymethyl | |
| 1.5.220 | 2-Ethoxy-3-hydroxymethyl-Phenyl | Hydroxymethyl | |
| 1.5.221 | 2-Trifluormethoxy-3-hydroxymethyl-Phenyl | Hydroxymethyl | |
| 1.5.222 | 2-Nitro-3-hydroxymethyl-Phenyl | Hydroxymethyl | |
| 1.5.223 | 2-Fluor-3-cyclopropyl-Phenyl | Hydroxymethyl | |
| 1.5.224 | 2-Chlor-3-cyclopropyl-Phenyl | Hydroxymethyl | |
| 1.5.225 | 2-Brom-3-cyclopropyl-Phenyl | Hydroxymethyl | |
| 1.5.226 | 2-Methyl-3-cyclopropyl-Phenyl | Hydroxymethyl | |
| 1.5.227 | 2-Ethyl-3-cyclopropyl-Phenyl | Hydroxymethyl | |
| 1.5.228 | 2-Cyclopropyl-3-cyclopropyl-Phenyl | Hydroxymethyl | |
| 1.5.229 | 2-Vinyl-3-cyclopropyl-Phenyl | Hydroxymethyl | |
| 1.5.230 | 2-Ethinyl-3-cyclopropyl-Phenyl | Hydroxymethyl | |
| 1.5.231 | 2-Cyano-3-cyclopropyl-Phenyl | Hydroxymethyl | |
| 1.5.232 | 2-Trifluormethyl-3-cyclopropyl-Phenyl | Hydroxymethyl | |
| 1.5.233 | 2-Methoxy-3-cyclopropyl-Phenyl | Hydroxymethyl | |
| 1.5.234 | 2-Ethoxy-3-cyclopropyl-Phenyl | Hydroxymethyl | |
| 1.5.235 | 2-Trifluormethoxy-3-cyclopropyl-Phenyl | Hydroxymethyl | |
| 1.5.236 | 2-Fluor-3-methoxycarbonyl-Phenyl | Hydroxymethyl | |
| 1.5.237 | 2-Chlor-3-methoxycarbonyl-Phenyl | Hydroxymethyl | |
| 1.5.238 | 2-Brom-3-methoxycarbonyl-Phenyl | Hydroxymethyl | |
| 1.5.239 | 2-Methyl-3-methoxycarbonyl-Phenyl | Hydroxymethyl | |
| 1.5.240 | 2-Ethyl-3-methoxycarbonyl-Phenyl | Hydroxymethyl | |
| 1.5.241 | 2-Cyclopropyl-3-methoxycarbonyl-Phenyl | Hydroxymethyl | |
| 1.5.242 | 2-Vinyl-3-methoxycarbonyl-Phenyl | Hydroxymethyl | |
| 1.5.243 | 2-Ethinyl-3-methoxycarbonyl-Phenyl | Hydroxymethyl | |
| 1.5.244 | 2-Cyano-3-methoxycarbonyl-Phenyl | Hydroxymethyl | |
| 1.5.245 | 2-Trifluormethyl-3-methoxycarbonyl-Phenyl | Hydroxymethyl | |
| 1.5.246 | 2-Methoxy-3-methoxycarbonyl-Phenyl | Hydroxymethyl | |
| 1.5.247 | 2-Ethoxy-3-methoxycarbonyl-Phenyl | Hydroxymethyl | |
| 1.5.248 | 2-Trifluormethoxy-3-methoxycarbonyl-Phenyl | Hydroxymethyl | |
| 1.5.249 | 2-Nitro-3-methoxycarbonyl-Phenyl | Hydroxymethyl | |
| 1.5.250 | 2-Fluor-3-vinyl-Phenyl | Hydroxymethyl | |
| 1.5.251 | 2-Chlor-3-vinyl-Phenyl | Hydroxymethyl | |
| 1.5.252 | 2-Brom-3-vinyl-Phenyl | Hydroxymethyl | |
| 1.5.253 | 2-Methyl-3-vinyl-Phenyl | Hydroxymethyl | |
| 1.5.254 | 2-Ethyl-3-vinyl-Phenyl | Hydroxymethyl | |
| 1.5.255 | 2-Cyclopropyl-3-vinyl-Phenyl | Hydroxymethyl | |
| 1.5.256 | 2-Vinyl-3-vinyl-Phenyl | Hydroxymethyl | |
| 1.5.257 | 2-Ethinyl-3-vinyl-Phenyl | Hydroxymethyl | |
| 1.5.258 | 2-Cyano-3-vinyl-Phenyl | Hydroxymethyl | |
| 1.5.259 | 2-Trifluormethyl-3-vinyl-Phenyl | Hydroxymethyl | |
| 1.5.260 | 2-Methoxy-3-vinyl-Phenyl | Hydroxymethyl | |
| 1.5.261 | 2-Ethoxy-3-vinyl-Phenyl | Hydroxymethyl | |
| 1.5.262 | 2-Trifluormethoxy-3-vinyl-Phenyl | Hydroxymethyl | |
| 1.5.263 | 2-Nitro-3-vinyl-Phenyl | Hydroxymethyl | |
| 1.5.264 | 2-Fluor-3-ethinyl-Phenyl | Hydroxymethyl | |
| 1.5.265 | 2-Chlor-3-ethinyl-Phenyl | Hydroxymethyl | |
| 1.5.266 | 2-Brom-3-ethinyl-Phenyl | Hydroxymethyl | |
| 1.5.267 | 2-Methyl-3-ethinyl-Phenyl | Hydroxymethyl | |
| 1.5.268 | 2-Ethyl-3-ethinyl-Phenyl | Hydroxymethyl | |
| 1.5.269 | 2-Cyclopropyl-3-ethinyl-Phenyl | Hydroxymethyl | |
| 1.5.270 | 2-Vinyl-3-ethinyl-Phenyl | Hydroxymethyl | |
| 1.5.271 | 2-Cyano-3-ethinyl-Phenyl | Hydroxymethyl | |
| 1.5.272 | 2-Trifluormethyl-3-ethinyl-Phenyl | Hydroxymethyl | |
| 1.5.273 | 2-Methoxy-3-ethinyl-Phenyl | Hydroxymethyl | |
| 1.5.274 | 2-Ethoxy-3-ethinyl-Phenyl | Hydroxymethyl | |
| 1.5.275 | 2-Trifluormethoxy-3-ethinyl--Phenyl | Hydroxymethyl | |
| 1.5.276 | 2-Nitro-3-ethinyl-Phenyl | Hydroxymethyl | |
| 1.5.277 | 2-Fluor-3-ethinyl-Phenyl | Hydroxymethyl | |
| 1.5.278 | 2-Fluor-3-cyano-Phenyl | Hydroxymethyl | |
| 1.5.279 | 2-Chlor-3-cyano-Phenyl | Hydroxymethyl | |
| 1.5.280 | 2-Brom-3-cyano-Phenyl | Hydroxymethyl | |
| 1.5.281 | 2-Methyl-3-cyano-Phenyl | Hydroxymethyl | |
| 1.5.282 | 2-Ethyl-3-cyano-Phenyl | Hydroxymethyl | |
| 1.5.283 | 2-Ethyl-3-cyano-Phenyl | 1-Hydroxyethyl | |
| 1.5.284 | 2-Ethyl-3-cyano-Phenyl | 1-Hydroxypropyl | |
| 1.5.285 | 2-Ethyl-3-cyano-Phenyl | (1-Hydroxy-2-methylpropyl) | |
| 1.5.286 | 2-Ethyl-3-cyano-Phenyl | Methoxymethyl | |
| 1.5.287 | 2-Ethyl-3-cyano-Phenyl | 2-Methoxyethyl | |
| 1.5.288 | 2-Cyclopropyl-3-cyano-Phenyl | Hydroxymethyl | |
| 1.5.289 | 2-Vinyl-3-cyano-Phenyl | Hydroxymethyl | |
| 1.5.290 | 2-Ethinyl-3-cyano-Phenyl | Hydroxymethyl | |
| 1.5.291 | 2-Cyano-3-cyanoPhenyl | Hydroxymethyl | |
| 1.5.292 | 2-Trifluormethyl-3-cyano-Phenyl | Hydroxymethyl | |
| 1.5.293 | 2-Methoxy-3-cyano-Phenyl | Hydroxymethyl | |
| 1.5.294 | 2-Ethoxy-3-cyano-Phenyl | Hydroxymethyl | |
| 1.5.295 | 2-Trifluormethoxy-3-cyano-Phenyl | Hydroxymethyl | |
| 1.5.296 | 2-Nitro-3-cyano-Phenyl | Hydroxymethyl | |
| 1.5.297 | 2-Fluor-3-hydroxy-Phenyl | Hydroxymethyl | |
| 1.5.298 | 2-Chlor-3-hydroxy-Phenyl | Hydroxymethyl | |
| 1.5.299 | 2-Brom-3-hydroxy-Phenyl | Hydroxymethyl | |
| 1.5.300 | 2-Methyl-3-hydroxy-Phenyl | Hydroxymethyl | |
| 1.5.301 | 2-Ethyl-3-hydroxy-Phenyl | Hydroxymethyl | |
| 1.5.302 | 2-Cyclopropyl-3-hydroxy-Phenyl | Hydroxymethyl | |
| 1.5.303 | 2-Vinyl-3-hydroxy-Phenyl | Hydroxymethyl | |
| 1.5.304 | 2-Ethinyl-3-hydroxy-Phenyl | Hydroxymethyl | |
| 1.5.305 | 2-Cyano-3-hydroxy-Phenyl | Hydroxymethyl | |
| 1.5.306 | 2-Trifluormethyl-3-hydroxy-Phenyl | Hydroxymethyl | |
| 1.5.307 | 2-Methoxy-3-hydroxy-Phenyl | Hydroxymethyl | |
| 1.5.308 | 2-Ethoxy-3-hydroxy-Phenyl | Hydroxymethyl | |
| 1.5.309 | 2-Trifluormethoxy-3-hydroxy-Phenyl | Hydroxymethyl | |
| 1.5.310 | 2-Nitro-3-hydroxy-Phenyl | Hydroxymethyl | |
| 1.5.311 | 2-Fluor-3-methoxy-Phenyl | Hydroxymethyl | |
| 1.5.312 | 2-Chlor-3-methoxy-Phenyl | Hydroxymethyl | |
| 1.5.313 | 2-Brom-3-methoxy-Phenyl | Hydroxymethyl | |
| 1.5.314 | 2-Methyl-3-methoxy-Phenyl | Hydroxymethyl | |
| 1.5.315 | 2-Ethyl-3-methoxy-Phenyl | Hydroxymethyl | |
| 1.5.316 | 2-Cyclopropyl-3-methoxy-Phenyl | Hydroxymethyl | |
| 1.5.317 | 2-Vinyl-3-methoxy-Phenyl | Hydroxymethyl | |
| 1.5.318 | 2-Ethinyl-3-methoxy-Phenyl | Hydroxymethyl | |
| 1.5.319 | 2-Cyano-3-methoxy-Phenyl | Hydroxymethyl | |
| 1.5.320 | 2-Trifluormethyl-3-methoxy-Phenyl | Hydroxymethyl | |
| 1.5.321 | 2,3-Dimethoxy-Phenyl | Hydroxymethyl | |
| 1.5.322 | 2-Ethoxy-3-methoxy-Phenyl | Hydroxymethyl | |
| 1.5.323 | 2-Trifluormethoxy-3-methoxy-Phenyl | Hydroxymethyl | |
| 1.5.324 | 2-Nitro-3-methoxy-Phenyl | Hydroxymethyl | |
| 1.5.325 | 2-Fluor-3-ethoxy-Phenyl | Hydroxymethyl | |
| 1.5.326 | 2-Chlor-3-ethoxy-Phenyl | Hydroxymethyl | |
| 1.5.327 | 2-Brom-3-ethoxy-Phenyl | Hydroxymethyl | |
| 1.5.328 | 2-Methyl-3-ethoxy-Phenyl | Hydroxymethyl | |
| 1.5.329 | 2-Ethyl-3-ethoxy-Phenyl | Hydroxymethyl | |
| 1.5.330 | 2-Cyclopropyl-3-ethoxy-Phenyl | Hydroxymethyl | |
| 1.5.331 | 2-Vinyl-3-ethoxy-Phenyl | Hydroxymethyl | |
| 1.5.332 | 2-Ethinyl-3-ethoxy-Phenyl | Hydroxymethyl | |
| 1.5.333 | 2-Cyano-3-ethoxy-Phenyl | Hydroxymethyl | |
| 1.5.334 | 2-Trifluormethyl-3-ethoxy-Phenyl | Hydroxymethyl | |
| 1.5.335 | 2-Methoxy-3-ethoxy-Phenyl | Hydroxymethyl | |
| 1.5.336 | 2,3-Diethoxy--Phenyl | Hydroxymethyl | |
| 1.5.337 | 2-Trifluormethoxy-3-ethoxy-Phenyl | Hydroxymethyl | |
| 1.5.338 | 2-Nitro-3-ethoxy-Phenyl | Hydroxymethyl | |
| 1.5.339 | 2-Fluor-3-propoxy-Phenyl | Hydroxymethyl | |
| 1.5.340 | 2-Chlor-3-propoxy-Phenyl | Hydroxymethyl | |
| 1.5.341 | 2-Brom-3-propoxy-Phenyl | Hydroxymethyl | |
| 1.5.342 | 2-Methyl-3-propoxy-Phenyl | Hydroxymethyl | |
| 1.5.343 | 2-Ethyl-3-propoxy-Phenyl | Hydroxymethyl | |
| 1.5.344 | 2-Cyclopropyl-3-propoxy-Phenyl | Hydroxymethyl | |
| 1.5.345 | 2-Vinyl-3-propoxy-Phenyl | Hydroxymethyl | |
| 1.5.346 | 2-Ethinyl-3-propoxy-Phenyl | Hydroxymethyl | |
| 1.5.347 | 2-Cyano-3-propoxy-Phenyl | Hydroxymethyl | |
| 1.5.348 | 2-Trifluormethyl-3-propoxy-Phenyl | Hydroxymethyl | |
| 1.5.349 | 2-Methoxy-3-propoxy-Phenyl | Hydroxymethyl | |
| 1.5.350 | 2-Ethoxy-3-propoxy-Phenyl | Hydroxymethyl | |
| 1.5.351 | 2-Trifluormethoxy-3-propoxy-Phenyl | Hydroxymethyl | |
| 1.5.352 | 2-Nitro-3-propoxy-Phenyl | Hydroxymethyl | |
| 1.5.353 | 2-Fluor-3-isopropoxy-Phenyl | Hydroxymethyl | |
| 1.5.354 | 2-Chlor-3-isopropoxy-Phenyl | Hydroxymethyl | |
| 1.5.355 | 2-Brom-3-isopropoxy-Phenyl | Hydroxymethyl | |
| 1.5.356 | 2-Methyl-3-isopropoxy-Phenyl | Hydroxymethyl | |
| 1.5.357 | 2-Ethyl-3-isopropoxy-Phenyl | Hydroxymethyl | |
| 1.5.358 | 2-Cyclopropyl-3-isopropoxy-Phenyl | Hydroxymethyl | |
| 1.5.359 | 2-Vinyl-3-isopropoxy-Phenyl | Hydroxymethyl | |
| 1.5.360 | 2-Ethinyl-3-isopropoxy-Phenyl | Hydroxymethyl | |
| 1.5.361 | 2-Cyano-3-isopropoxy-Phenyl | Hydroxymethyl | |
| 1.5.362 | 2-Trifluormethyl-3-isopropoxy-Phenyl | Hydroxymethyl | |
| 1.5.363 | 2-Methoxy-3-isopropoxy-Phenyl | Hydroxymethyl | |
| 1.5.364 | 2-Ethoxy-3-isopropoxy-Phenyl | Hydroxymethyl | |
| 1.5.365 | 2-Trifluormethoxy-3-isopropoxy-Phenyl | Hydroxymethyl | |
| 1.5.366 | 2-Nitro-3-isopropoxy-Phenyl | Hydroxymethyl | |
| 1.5.367 | 2-Fluor-3-tert.butoxy-Phenyl | Hydroxymethyl | |
| 1.5.368 | 2-Chlor-3-tert.butoxy-Phenyl | Hydroxymethyl | |
| 1.5.369 | 2-Brom-3-tert.butoxy-Phenyl | Hydroxymethyl | |
| 1.5.370 | 2-Methyl-3-tert.butoxy-Phenyl | Hydroxymethyl | |
| 1.5.371 | 2-Ethyl-3-tert.butoxy-Phenyl | Hydroxymethyl | |
| 1.5.372 | 2-Cyclopropyl-3-tert.butoxy-Phenyl | Hydroxymethyl | |
| 1.5.373 | 2-Vinyl-3-tert.butoxy-Phenyl | Hydroxymethyl | |
| 1.5.374 | 2-Ethinyl-3-tert.butoxy-Phenyl | Hydroxymethyl | |
| 1.5.375 | 2-Cyano-3-tert.butoxy-Phenyl | Hydroxymethyl | |
| 1.5.376 | 2-Trifluormethyl-3-tert.butoxy-Phenyl | Hydroxymethyl | |
| 1.5.377 | 2-Methoxy-3-tert.butoxy-Phenyl | Hydroxymethyl | |
| 1.5.378 | 2-Ethoxy-3-tert.butoxy-Phenyl | Hydroxymethyl | |
| 1.5.379 | 2-Trifluormethoxy-3-tert.butoxy-Phenyl | Hydroxymethyl | |
| 1.5.380 | 2-Nitro-3-tert.butoxy-Phenyl | Hydroxymethyl | |
| 1.5.381 | 2-Fluor-3-trifluormethoxy-Phenyl | Hydroxymethyl | |
| 1.5.382 | 2-Chlor-3-trifluormethoxy-Phenyl | Hydroxymethyl | |
| 1.5.383 | 2-Brom-3-trifluormethoxy-Phenyl | Hydroxymethyl | |
| 1.5.384 | 2-Methyl-3-trifluormethoxy-Phenyl | Hydroxymethyl | |
| 1.5.385 | 2-Ethyl-3-trifluormethoxy-Phenyl | Hydroxymethyl | |
| 1.5.386 | 2-Cyclopropyl-3-trifluormethoxy-Phenyl | Hydroxymethyl | |
| 1.5.387 | 2-Vinyl-3-trifluormethoxy-Phenyl | Hydroxymethyl | |
| 1.5.388 | 2-Ethinyl-3-trifluormethoxy-Phenyl | Hydroxymethyl | |
| 1.5.389 | 2-Cyano-3-trifluormethoxy-Phenyl | Hydroxymethyl | |
| 1.5.390 | 2-Trifluormethyl-3-trifluormethoxy-Phenyl | Hydroxymethyl | |
| 1.5.391 | 2-Methoxy-3-trifluormethoxy-Phenyl | Hydroxymethyl | |
| 1.5.392 | 2-Ethoxy-3-trifluormethoxy-Phenyl | Hydroxymethyl | |
| 1.5.393 | 2,3-Bis(trifluormethoxy)-Phenyl | Hydroxymethyl | |
| 1.5.394 | 2-Nitro-3-trifluormethoxy-Phenyl | Hydroxymethyl | |
| 1.5.395 | 2-Fluor-3-(2,2,2-trifluorethoxy)-Phenyl | Hydroxymethyl | |
| 1.5.396 | 2-Chlor-3-(2,2,2-trifluorethoxy)-Phenyl | Hydroxymethyl | |
| 1.5.397 | 2-Brom-3-(2,2,2-trifluorethoxy)-Phenyl | Hydroxymethyl | |
| 1.5.398 | 2-Methyl-3-(2,2,2-trifluorethoxy)-Phenyl | Hydroxymethyl | |
| 1.5.399 | 2-Ethyl-3-(2,2,2-trifluorethoxy)-Phenyl | Hydroxymethyl | |
| 1.5.400 | 2-Cyclopropyl-3-(2,2,2-trifluorethoxy)-Phenyl | Hydroxymethyl | |
| 1.5.401 | 2-Vinyl-3-(2,2,2-trifluorethoxy)-Phenyl | Hydroxymethyl | |
| 1.5.402 | 2-Ethinyl-3-(2,2,2-trifluorethoxy-Phenyl | Hydroxymethyl | |
| 1.5.403 | 2-Cyano-3-(2,2,2-trifluorethoxy)-Phenyl | Hydroxymethyl | |
| 1.5.404 | 2-Trifluormethyl-3-(2,2,2-trifluorethoxy)-Phenyl | Hydroxymethyl | |
| 1.5.405 | 2-Methoxy-3-(2,2,2-trifluorethoxy)-Phenyl | Hydroxymethyl | |
| 1.5.406 | 2-Ethoxy-3-(2,2,2-trifluorethoxy)-Phenyl | Hydroxymethyl | |
| 1.5.407 | 2-Trifluormethoxy-3-(2,2,2-trifluorethoxy)-Phenyl | Hydroxymethyl | |
| 1.5.408 | 2-Nitro-3-(2,2,2-trifluorethoxy)-Phenyl | Hydroxymethyl | |
| 1.5.409 | 2-Fluor-3-difluormethoxy-Phenyl | Hydroxymethyl | |
| 1.5.410 | 2-Chlor-3-difluormethoxy-Phenyl | Hydroxymethyl | |
| 1.5.411 | 2-Brom-3-difluormethoxy-Phenyl | Hydroxymethyl | |
| 1.5.412 | 2-Methyl-3-difluormethoxy-Phenyl | Hydroxymethyl | |
| 1.5.413 | 2-Ethyl-3-difluormethoxy-Phenyl | Hydroxymethyl | |
| 1.5.414 | 2-Cyclopropyl-3-difluormethoxy-Phenyl | Hydroxymethyl | |
| 1.5.415 | 2-Vinyl-3-difluormethoxy-Phenyl | Hydroxymethyl | |
| 1.5.416 | 2-Ethinyl-3-difluormethoxy-Phenyl | Hydroxymethyl | |
| 1.5.417 | 2-Cyano-3-difluormethoxy-Phenyl | Hydroxymethyl | |
| 1.5.418 | 2-Trifluormethyl-3-difluormethoxy-Phenyl | Hydroxymethyl | |
| 1.5.419 | 2-Methoxy-3-difluormethoxy-Phenyl | Hydroxymethyl | |
| 1.5.420 | 2-Ethoxy-3-difluormethoxy-Phenyl | Hydroxymethyl | |
| 1.5.421 | 2-Trifluormethoxy-3-difluormethoxy-Phenyl | Hydroxymethyl | |
| 1.5.422 | 2-Nitro-3-difluormethoxy-Phenyl | Hydroxymethyl | |
| 1.5.423 | 2-Fluor-3-(2-methoxyethoxy)-Phenyl | Hydroxymethyl | |
| 1.5.424 | 2-Chlor-3-(2-methoxyethoxy)-Phenyl | Hydroxymethyl | |
| 1.5.425 | 2-Brom-3-(2-methoxyethoxy)-Phenyl | Hydroxymethyl | |
| 1.5.426 | 2-Methyl-3-(2-methoxyethoxy)-Phenyl | Hydroxymethyl | |
| 1.5.427 | 2-Ethyl-3-(2-methoxyethoxy)-Phenyl | Hydroxymethyl | |
| 1.5.428 | 2-Cyclopropyl-3-(2-methoxyethoxy)-Phenyl | Hydroxymethyl | |
| 1.5.429 | 2-Vinyl-3-(2-methoxyethoxy)-Phenyl | Hydroxymethyl | |
| 1.5.430 | 2-Ethinyl-3-(2-methoxyethoxy)-Phenyl | Hydroxymethyl | |
| 1.5.431 | 2-Cyano-3-(2-methoxyethoxy)-Phenyl | Hydroxymethyl | |
| 1.5.432 | 2-Trifluormethyl-3-(2-methoxyethoxy)-Phenyl | Hydroxymethyl | |
| 1.5.433 | 2-Methoxy-3-(2-methoxyethoxy)-Phenyl | Hydroxymethyl | |
| 1.5.434 | 2-Ethoxy-3-(2-methoxyethoxy)-Phenyl | Hydroxymethyl | |
| 1.5.435 | 2-Trifluormethoxy-(2-methoxyethoxy)-Phenyl | Hydroxymethyl | |
| 1.5.436 | 2-Nitro-3-(2-methoxyethoxy)-Phenyl | Hydroxymethyl | |
| 1.5.437 | 2-Fluor-3-(tert.butoxycarbonyloxy)-Phenyl | Hydroxymethyl | |
| 1.5.438 | 2-Chlor-3-(tert.butoxycarbonyloxy)-Phenyl | Hydroxymethyl | |
| 1.5.439 | 2-Brom-3-(tert.butoxycarbonyloxy)-Phenyl | Hydroxymethyl | |
| 1.5.440 | 2-Methyl-3-(tert.butoxycarbonyloxy)-Phenyl | Hydroxymethyl | |
| 1.5.441 | 2-Ethyl-3-(tert.butoxycarbonyloxy)-Phenyl | Hydroxymethyl | |
| 1.5.442 | 2-Cyclopropyl-3-(tert.butoxycarbonyloxy)-Phenyl | Hydroxymethyl | |
| 1.5.443 | 2-Vinyl-3-(tert.butoxycarbonyloxy)-Phenyl | Hydroxymethyl | |
| 1.5.444 | 2-Ethinyl-3- (tert.butoxycarbonyloxy)-Phenyl | Hydroxymethyl | |
| 1.5.445 | 2-Cyano-3-(tert.butoxycarbonyloxy)-Phenyl | Hydroxymethyl | |
| 1.5.446 | 2-Trifluormethyl-3-(tert.butoxycarbonyloxy)-Phenyl | Hydroxymethyl | |
| 1.5.447 | 2-Methoxy-3- (tert.butoxycarbonyloxy)-Phenyl | Hydroxymethyl | |
| 1.5.448 | 2-Ethoxy-3-(tert.butoxycarbonyloxy)-Phenyl | Hydroxymethyl | |
| 1.5.449 | 2-Trifluormethoxy-3-(tert.butoxycarbonyloxy)-Phenyl | Hydroxymethyl | |
| 1.5.450 | 2-Nitro-3-(tert.butoxycarbonyloxy)-Phenyl | Hydroxymethyl | |
| 1.5.451 | 2-Fluor-3-nitro-Phenyl | Hydroxymethyl | |
| 1.5.452 | 2-Chlor-3-nitro-Phenyl | Hydroxymethyl | |
| 1.5.453 | 2-Brom-3-nitro-Phenyl | Hydroxymethyl | |
| 1.5.454 | 2-Methyl-3-nitro-Phenyl | Hydroxymethyl | |
| 1.5.455 | 2-Ethyl-3-nitro-Phenyl | Hydroxymethyl | |
| 1.5.456 | 2-Cyclopropyl-3-nitro-Phenyl | Hydroxymethyl | |
| 1.5.457 | 2-Vinyl-3-nitro-Phenyl | Hydroxymethyl | |
| 1.5.458 | 2-Ethinyl-3-nitro-Phenyl | Hydroxymethyl | |
| 1.5.459 | 2-Cyano-3-nitro-Phenyl | Hydroxymethyl | |
| 1.5.460 | 2-Trifluormethyl-3-nitro-Phenyl | Hydroxymethyl | |
| 1.5.461 | 2-Methoxy-3-nitro-Phenyl | Hydroxymethyl | |
| 1.5.462 | 2-Ethoxy-3-nitro-Phenyl | Hydroxymethyl | |
| 1.5.463 | 2-Trifluormethoxy-3-nitro-Phenyl | Hydroxymethyl | |
| 1.5.464 | 2-Fluor-3-methylsulfanylPhenyl | Hydroxymethyl | |
| 1.5.465 | 2-Chlor-3-methylsulfanyl-Phenyl | Hydroxymethyl | |
| 1.5.466 | 2-Brom-3-methylsulfanyl-Phenyl | Hydroxymethyl | |
| 1.5.467 | 2-Methyl-3-methylsulfanyl-Phenyl | Hydroxymethyl | |
| 1.5.468 | 2-Ethyl-3-methylsulfanyl-Phenyl | Hydroxymethyl | |
| 1.5.469 | 2-Cyclopropyl-3-methylsulfanyl-Phenyl | Hydroxymethyl | |
| 1.5.470 | 2-Vinyl-3-methylsulfanyl-Phenyl | Hydroxymethyl | |
| 1.5.471 | 2-Ethinyl-3-methylsulfanyl-Phenyl | Hydroxymethyl | |
| 1.5.472 | 2-Cyano-3-methylsulfanyl-Phenyl | Hydroxymethyl | |
| 1.5.473 | 2-Trifluormethyl-3-methylsulfanyl-Phenyl | Hydroxymethyl | |
| 1.5.474 | 2-Methoxy-3-methylsulfanyl-Phenyl | Hydroxymethyl | |
| 1.5.475 | 2-Ethoxy-3-methylsulfanyl-Phenyl | Hydroxymethyl | |
| 1.5.476 | 2-Trifluormethoxy-3-methylsulfanyl-Phenyl | Hydroxymethyl | |
| 1.5.477 | 2-Nitro-3-methylsulfanyl-Phenyl | Hydroxymethyl | |
| 1.5.478 | 3,5-Difluor-Phenyl | Hydroxymethyl | [CDCl3] 2.0 (s br,1H); 3.61 (d,1H); 3.69 (d,1H), 3.95 (d,1H), 4.08 (d,1H), 6.91 (t,1H); 7.18 (d,2H). |
| 1.5.479 | 3,5-Difluor-Phenyl | 1-Hydroxyethyl | [CDCl3] D1 1.25 (d,3H); 1.36 (d,1H); 3.60 (d,1H); 3.70 (d,1H); 4.29 (m,1H); 6.91 (t,1H); 7.20 (d,2H). D2 1.29 (d,3H); 1.42 (d,1H); 3.60 (d,1H); 3.69 (d,1H); 4.22 (m,1H); 6.91 (t,1H); 7.20 (d,2H). |
| 1.5.480 | 3,5-Difluor-Phenyl | 1-Hydroxypropyl | |
| 1.5.481 | 3,5-Difluor-Phenyl | (1-Hydroxy-2-methylpropyl) | |
| 1.5.482 | 3,5-Difluor-Phenyl | Methoxymethyl | [CDCl3] 3.47 (s,3H); 3.50 (d,1H); 3.72 (d,1H); 3.84 (s,2H); 6.90 (m,1H); 7.19 (m,2H). |
| 1.5.483 | 2-Ethyl-3-cyano-Phenyl | 2-Methoxyethyl | |
| 1.5.484 | 3-Chlor-5-fluor-Phenyl | Hydroxymethyl | |
| 1.5.485 | 3-Chlor-5-fluor-Phenyl | 1-Hydroxyethyl | |
| 1.5.486 | 3-Chlor-5-fluor-Phenyl | 1-Hydroxypropyl | |
| 1.5.487 | 3-Chlor-5-fluor-Phenyl | (1-Hydroxy-2-methylpropyl) | |
| 1.5.488 | 3-Chlor-5-fluor-Phenyl | Methoxymethyl | |
| 1.5.489 | 3-Chlor-5-fluor-Phenyl | 2-Methoxyethyl | |
| 1.5.490 | 3-Brom-5-fluor-Phenyl | Hydroxymethyl | |
| 1.5.491 | 3-Brom-5-fluor-Phenyl | 1-Hydroxyethyl | |
| 1.5.492 | 3-Brom-5-fluor-Phenyl | 1-Hydroxypropyl | |
| 1.5.493 | 3-Brom-5-fluor-Phenyl | (1-Hydroxy-2-methylpropyl) | |
| 1.5.494 | 3-Brom-5-fluor-Phenyl | Methoxymethyl | |
| 1.5.495 | 3-Brom-5-fluor-Phenyl | 2-Methoxyethyl | |
| 1.5.496 | 3-Iod-5-fluor-Phenyl | Hydroxymethyl | |
| 1.5.497 | 3-Methyl-5-fluor-Phenyl | Hydroxymethyl | |
| 1.5.498 | 3-Methyl-5-fluor-Phenyl | 1-Hydroxyethyl | |
| 1.5.499 | 3-Methyl-5-fluor-Phenyl | 1-Hydroxypropyl | |
| 1.5.500 | 3-Methyl-5-fluor-Phenyl | (1-Hydroxy-2-methylpropyl) | |
| 1.5.501 | 3-Methyl-5-fluor-Phenyl | Methoxymethyl | |
| 1.5.502 | 3-Methyl-5-fluor-Phenyl | 2-Methoxyethyl | |
| 1.5.503 | 3-Ethyl-5-fluor-Phenyl | Hydroxymethyl | |
| 1.5.504 | 3-Propyl-5-fluor-Phenyl | Hydroxymethyl | |
| 1.5.505 | 3-iPropyl-5-fluor-Phenyl | Hydroxymethyl | |
| 1.5.506 | 3-nButyl-5-fluor-Phenyl | Hydroxymethyl | |
| 1.5.507 | 3-isoButyl-5-fluor-Phenyl | Hydroxymethyl | |
| 1.5.508 | 3-tert.Butyl-5-fluor-Phenyl | Hydroxymethyl | |
| 1.5.509 | 3-Cyclopropyl-5-fluor-Phenyl | Hydroxymethyl | |
| 1.5.510 | 3-Vinyl-5-fluor-Phenyl | Hydroxymethyl | |
| 1.5.511 | 3-Ethinyl-5-fluor-Phenyl | Hydroxymethyl | |
| 1.5.512 | 3-Cyano-5-fluor-Phenyl | Hydroxymethyl | |
| 1.5.513 | 3-Trifluormethyl-5-fluor-Phenyl | Hydroxymethyl | |
| 1.5.514 | 3-Trifluormethyl-5-fluor-Phenyl | 1-Hydroxyethyl | |
| 1.5.515 | 3-Trifluormethyl-5-fluor-Phenyl | 1-Hydroxypropyl | |
| 1.5.516 | 3-Trifluormethyl-5-fluor-Phenyl | (1-Hydroxy-2-methylpropyl) | |
| 1.5.517 | 3-Trifluormethyl-5-fluor-Phenyl | Methoxymethyl | |
| 1.5.518 | 3-Trifluormethyl-5-fluor-Phenyl | 2-Methoxyethyl | |
| 1.5.519 | 3-(Methoxycarbony)l-5-fluor-Phenyl | Hydroxymethyl | |
| 1.5.520 | 3-Hydroxymethyl-5-fluor-Phenyl | Hydroxymethyl | |
| 1.5.521 | 3-Carbamoyl-5-fluor-Phenyl | Hydroxymethyl | |
| 1.5.522 | 3-Hydroxy-5-fluor-Phenyl- | Hydroxymethyl | |
| 1.5.523 | 3-Methoxy-5-fluor-Phenyl | Hydroxymethyl | |
| 1.5.524 | 3-Ethoxy-5-fluor-Phenyl | Hydroxymethyl | |
| 1.5.525 | 3-nPropyoxy-5-fluor-Phenyl | Hydroxymethyl | |
| 1.5.526 | 3-isoPropoxy-5-fluor-Phenyl | Hydroxymethyl | |
| 1.5.527 | 3-nButoxy-5-fluor-Phenyl | Hydroxymethyl | |
| 1.5.528 | 3-isoButoxy-5-fluor-Phenyl | Hydroxymethyl | |
| 1.5.529 | 3-tert.Butoxy-5-fluor-Phenyl | Hydroxymethyl | |
| 1.5.530 | 3-Difluormethoxy-5-fluor-Phenyl | Hydroxymethyl | |
| 1.5.531 | 3-Trifluormethoxy-5-fluor-Phenyl | Hydroxymethyl | |
| 1.5.532 | 3-(2,2,2-Trifluorethoxy)-5-fluor-Phenyl | Hydroxymethyl | |
| 1.5.533 | 3-(2-Chlorethoxy)-5-fluor-Phenyl | Hydroxymethyl | |
| 1.5.534 | 3-(2-Hydroxyethoxy)-5-fluor-Phenyl- | Hydroxymethyl | |
| 1.5.535 | 3-[(tert-Butoxycarbonyl)oxy]-5-fluor-Phenyl | Hydroxymethyl | |
| 1.5.536 | 3-Nitro-5-fluor-Phenyl | Hydroxymethyl | |
| 1.5.537 | 3-Acetoxy-5-fluor-Phenyl | Hydroxymethyl | |
| 1.5.538 | {3-[(tert-Butoxycarbonyl)amino]-5-fluor-Phenyl} | Hydroxymethyl | |
| 1.5.539 | 3-Methylsulfanyl-5-fluor-Phenyl | Hydroxymethyl | |
| 1.5.540 | 3,5-Dichlor-Phenyl | Hydroxymethyl | [CDCl₃] 3.68 (q, 2H); 3.96 (d, 1H); 4.08 (d, 1H); 7.45 (s, 1H); 7.56 (s, 2H). |
| 1.5.541 | 3,5-Dichlor-Phenyl | 1-Hydroxyethyl | |
| 1.5.542 | 3,5-Dichlor-Phenyl | 1-Hydroxypropyl | |
| 1.5.543 | 3,5-Dichlor-Phenyl | (1-Hydroxy-2-methylpropyl) | |
| 1.5.544 | 3,5-Dichlor-Phenyl | Methoxymethyl | [CDCl₃] 3.48 (s, 3H); 3.50 (d, 1H); 3.72 (d, 1H); 3.85 (q, 2H); 7.42 (m, 1H); 7.55 (m, 2H). |
| 1.5.545 | 3,5-Dichlor-Phenyl | 2-Methoxyethyl | [CDCl₃] |
| 1.5.546 | 3-Brom-5-chlor-Phenyl | Hydroxymethyl | |
| 1.5.547 | 3-lod-5-chlor-Phenyl | Hydroxymethyl | |
| 1.5.548 | 3-Methyl-5-chlor-Phenyl | Hydroxymethyl | |
| 1.5.549 | 3-Propyl-5-chlor-Phenyl | Hydroxymethyl | |
| 1.5.550 | 3-isoPropyl-5-chlor-Phenyl | Hydroxymethyl | |
| 1.5.551 | 3-nButyl-5-chlor-Phenyl | Hydroxymethyl | |
| 1.5.552 | 3-isoButyl-5-chlor-Phenyl | Hydroxymethyl | |
| 1.5.553 | 3-tert.Butyl-5-chlor-Phenyl | Hydroxymethyl | |
| 1.5.554 | 3-Cyclopropyl-5-chlor-Phenyl | Hydroxymethyl | |
| 1.5.555 | 3-Cyano-5-chlor-Phenyl | Hydroxymethyl | |
| 1.5.556 | 3-Trifluormethyl-5-chlor-Phenyl | Hydroxymethyl | |
| 1.5.557 | 3-(Methoxycarbony)l-5-chlor-Phenyl | Hydroxymethyl | |
| 1.5.558 | 3-Methoxy-5-chlor-Phenyl | Hydroxymethyl | |
| 1.5.559 | 3-Ethoxy-5-chlor-Phenyl | Hydroxymethyl | |
| 1.5.560 | 3-nPropyoxy-5-chlor-Phenyl | Hydroxymethyl | |
| 1.5.561 | 3-isoPropoxy-5-chlor-Phenyl | Hydroxymethyl | |
| 1.5.562 | 3-nButoxy-5-chlor-Phenyl | Hydroxymethyl | |
| 1.5.563 | 3-isoButoxy-5-chlor-Phenyl | Hydroxymethyl | |
| 1.5.564 | 3-Difluormethoxy-5-chlor-Phenyl | Hydroxymethyl | |
| 1.5.565 | 3-Trifluormethoxy-5-chlor-Phenyl | Hydroxymethyl | |
| 1.5.566 | 3-Nitro-5-chlor-Phenyl | Hydroxymethyl | |
| 1.5.567 | 3-Acetoxy-5-chlor-Phenyl | Hydroxymethyl | |
| 1.5.568 | 3,5-Dibrom-Phenyl | Hydroxymethyl | |
| 1.5.569 | 3,5-Dibrom-Phenyl | 1-Hydroxyethyl | |
| 1.5.570 | 3-Iod-5-brom-Phenyl | Hydroxymethyl | |
| 1.5.571 | 3-Methyl-5-brom-Phenyl | Hydroxymethyl | |
| 1.5.572 | 3-Methyl-5-brom-Phenyl | 1-Hydroxyethyl | |
| 1.5.573 | 3-Methyl-5-brom-Phenyl | 1-Hydroxypropyl | |
| 1.5.574 | 3-Methyl-5-brom-Phenyl | (1-Hydroxy-2-methylpropyl) | |
| 1.5.575 | 3-Methyl-5-brom-Phenyl | Methoxymethyl | |
| 1.5.576 | 3-Methyl-5-brom-Phenyl | 2-Methoxyethyl | |
| 1.5.577 | 3-Ethyl-5-brom-Phenyl | Hydroxymethyl | |
| 1.5.578 | 3-Propyl-5-brom-Phenyl | Hydroxymethyl | |
| 1.5.579 | 3-isoPropyl-5-brom-Phenyl | Hydroxymethyl | |
| 1.5.580 | 3-nButyl-5-brom-Phenyl | Hydroxymethyl | |
| 1.5.581 | 3-isoButyl-5-brom-Phenyl | Hydroxymethyl | |
| 1.5.582 | 3-tert.Butyl-5-brom-Phenyl | Hydroxymethyl | |
| 1.5.583 | 3-Cyclopropyl-5-brom-Phenyl | Hydroxymethyl | |
| 1.5.584 | 3-Cyano-5-brom-Phenyl | Hydroxymethyl | |
| 1.5.585 | 3-Trifluormethyl-5-brom-Phenyl | Hydroxymethyl | |
| 1.5.586 | 3-(Methoxycarbony)l-5-brom-Phenyl | Hydroxymethyl | |
| 1.5.587 | 3-Methoxy-5-brom-Phenyl | Hydroxymethyl | |
| 1.5.588 | 3-Ethoxy-5-brom-Phenyl | Hydroxymethyl | |
| 1.5.589 | 3-nPropyoxy-5-brom-Phenyl | Hydroxymethyl | |
| 1.5.590 | 3-isoPropoxy-5-brom-Phenyl | Hydroxymethyl | |
| 1.5.591 | 3-nButoxy-5-brom-Phenyl | Hydroxymethyl | |
| 1.5.592 | 3-isoButoxy-5-brom-Phenyl | Hydroxymethyl | |
| 1.5.593 | 3-Difluormethoxy-5-brom-Phenyl | Hydroxymethyl | |
| 1.5.594 | 3-Trifluormethoxy-5-brom-Phenyl | Hydroxymethyl | |
| 1.5.595 | 3-Nitro-5-brom-Phenyl | Hydroxymethyl | |
| 1.5.596 | 3-Acetoxy-5-brom-Phenyl | Hydroxymethyl | |
| 1.5.597 | 3-Methylsulfanyl-5-brom-Phenyl | Hydroxymethyl | |
| 1.5.598 | 3,5-Diiod-Phenyl | Hydroxymethyl | |
| 1.5.599 | 3-Methyl-5-iod-Phenyl | Hydroxymethyl | |
| 1.5.600 | 3-Ethyl-5-iod-Phenyl | Hydroxymethyl | |
| 1.5.601 | 3-Propyl-5-iod-Phenyl | Hydroxymethyl | |
| 1.5.602 | 3-isoPropyl-5-iod-Phenyl | Hydroxymethyl | |
| 1.5.603 | 3-nButyl-5-iod-Phenyl | Hydroxymethyl | |
| 1.5.604 | 3-isoButyl-5-iod-Phenyl | Hydroxymethyl | |
| 1.5.605 | 3-tert.Butyl-5-iod-Phenyl | Hydroxymethyl | |
| 1.5.606 | 3-Cyclopropyl-5-iod-Phenyl | Hydroxymethyl | |
| 1.5.607 | 3-Cyano-5-iod-Phenyl | Hydroxymethyl | |
| 1.5.608 | 3-Trifluormethyl-5-iod-Phenyl | Hydroxymethyl | |
| 1.5.609 | 3-(Methoxycarbonyl)-5-iod-Phenyl | Hydroxymethyl | |
| 1.5.610 | 3-Methoxy-5-iod-Phenyl | Hydroxymethyl | |
| 1.5.611 | 3-Ethoxy-5-iod-Phenyl | Hydroxymethyl | |
| 1.5.612 | 3-nPropyoxy-5-iod-Phenyl | Hydroxymethyl | |
| 1.5.613 | 3-isoPropoxy-5-iod-Phenyl | Hydroxymethyl | |
| 1.5.614 | 3-nButoxy-5-iod-Phenyl | Hydroxymethyl | |
| 1.5.615 | 3-isoButoxy-5-iod-Phenyl | Hydroxymethyl | |
| 1.5.616 | 3-Difluormethoxy-5-iod-Phenyl | Hydroxymethyl | |
| 1.5.617 | 3-Trifluormethoxy-5-iod-Phenyl | Hydroxymethyl | |
| 1.5.618 | 3-Nitro-5-iod-Phenyl | Hydroxymethyl | |
| 1.5.619 | 3-Acetoxy-5-iod-Phenyl | Hydroxymethyl | |
| 1.5.620 | 3-Methylsulfanyl-5-iod-Phenyl | Hydroxymethyl | |
| 1.5.621 | 3,5-Dimethyl-Phenyl | Hydroxymethyl | |
| 1.5.622 | 3-Ethyl-5-methyl-Phenyl | Hydroxymethyl | |
| 1.5.623 | 3-Propyl-5-methyl-Phenyl | Hydroxymethyl | |
| 1.5.624 | 3-isoPropyl-5-methyl-Phenyl | Hydroxymethyl | |
| 1.5.625 | 3-nButyl-5-methyl-Phenyl | Hydroxymethyl | |
| 1.5.626 | 3-isoButyl-5-methyl-Phenyl | Hydroxymethyl | |
| 1.5.627 | 3-tert.Butyl-5-methyl-Phenyl | Hydroxymethyl | |
| 1.5.628 | 3-Cyclopropyl-5-methyl-Phenyl | Hydroxymethyl | |
| 1.5.629 | 3-Cyano-5-methyl-Phenyl | Hydroxymethyl | |
| 1.5.630 | 3-Trifluormethyl-5-methyl-Phenyl | Hydroxymethyl | |
| 1.5.631 | 3-(Methoxycarbonyl)-5-methyl-Phenyl | Hydroxymethyl | |
| 1.5.632 | 3-Methoxy-5-methyl-Phenyl | Hydroxymethyl | |
| 1.5.633 | 3-Ethoxy-5-methyl-Phenyl | Hydroxymethyl | |
| 1.5.634 | 3-nPropyoxy-5-methyl-Phenyl | Hydroxymethyl | |
| 1.5.635 | 3-nButoxy-5-methyl-Phenyl | Hydroxymethyl | |
| 1.5.636 | 3-isoButoxy-5-methyl-Phenyl | Hydroxymethyl | |
| 1.5.637 | 3-Difluormethoxy-5-methyl-Phenyl | Hydroxymethyl | |
| 1.5.638 | 3-Trifluormethoxy-5-methyl-Phenyl | Hydroxymethyl | |
| 1.5.639 | 3-Nitro-5-methyl-Phenyl | Hydroxymethyl | |
| 1.5.640 | 3-Acetoxy-5-methyl-Phenyl | Hydroxymethyl | |
| 1.5.641 | 3-Methylsulfanyl-5-methyl-Phenyl | Hydroxymethyl | |
| 1.5.642 | 3,5-Diethyl-Phenyl | Hydroxymethyl | |
| 1.5.643 | 3-Propyl-5-ethyl-Phenyl | Hydroxymethyl | |
| 1.5.644 | 3-isoPropyl-5-ethyl-Phenyl | Hydroxymethyl | |
| 1.5.645 | 3-nButyl-5-ethyl-Phenyl | Hydroxymethyl | |
| 1.5.646 | 3-isoButyl-5-ethyl-Phenyl | Hydroxymethyl | |
| 1.5.647 | 3-tert.Butyl-5-ethyl-Phenyl | Hydroxymethyl | |
| 1.5.648 | 3-Cyclopropyl-5-ethyl-Phenyl | Hydroxymethyl | |
| 1.5.649 | 3-Cyano-5-ethyl-Phenyl | Hydroxymethyl | |
| 1.5.650 | 3-Trifluormethyl-5-ethyl-Phenyl | Hydroxymethyl | |
| 1.5.651 | 3-(Methoxycarbony)l-5-ethyl-Phenyl | Hydroxymethyl | |
| 1.5.652 | 3-Methoxy-5-ethyl-Phenyl | Hydroxymethyl | |
| 1.5.653 | 3-Ethoxy-5-ethyl-Phenyl | Hydroxymethyl | |
| 1.5.654 | 3-nPropyoxy-5-ethyl-Phenyl | Hydroxymethyl | |
| 1.5.655 | 3-nButoxy-5-ethyl-Phenyl | Hydroxymethyl | |
| 1.5.656 | 3-isoButoxy-5-ethyl-Phenyl | Hydroxymethyl | |
| 1.5.657 | 3-Difluormethoxy-5-ethyl-Phenyl | Hydroxymethyl | |
| 1.5.658 | 3-Trifluormethoxy-5-ethyl-Phenyl | Hydroxymethyl | |
| 1.5.659 | 3-Nitro-5-ethyl-Phenyl | Hydroxymethyl | |
| 1.5.660 | 3-Acetoxy-5-ethyl-Phenyl | Hydroxymethyl | |
| 1.5.661 | 3-Methylsulfanyl-5-ethyl-Phenyl | Hydroxymethyl | |
| 1.5.662 | 3,5-Dipropyl-Phenyl | Hydroxymethyl | |
| 1.5.663 | 3-isoPropyl-5-propyl-Phenyl | Hydroxymethyl | |
| 1.5.664 | 3-nButyl-5-propyl-Phenyl | Hydroxymethyl | |
| 1.5.665 | 3-isoButyl-5-propyl-Phenyl | Hydroxymethyl | |
| 1.5.666 | 3-tert.Butyl-5-propyl-Phenyl | Hydroxymethyl | |
| 1.5.667 | 3-Cyclopropyl-5-propyl-Phenyl | Hydroxymethyl | |
| 1.5.668 | 3-Cyano-5-propyl-Phenyl | Hydroxymethyl | |
| 1.5.669 | 3-Trifluormethyl-5-propyl-Phenyl | Hydroxymethyl | |
| 1.5.670 | 3-(Methoxycarbonyl)-5-propyl-Phenyl | Hydroxymethyl | |
| 1.5.671 | 3-Methoxy-5-propyl-Phenyl | Hydroxymethyl | |
| 1.5.672 | 3-Ethoxy-5-propyl-Phenyl | Hydroxymethyl | |
| 1.5.673 | 3-nPropyoxy-5-propyl-Phenyl | Hydroxymethyl | |
| 1.5.674 | 3-nButoxy-5-propyl-Phenyl | Hydroxymethyl | |
| 1.5.675 | 3-isoButoxy-5-propyl-Phenyl | Hydroxymethyl | |
| 1.5.676 | 3-Difluormethoxy-5-propyl-Phenyl | Hydroxymethyl | |
| 1.5.677 | 3-Trifluormethoxy-5-ethyl-Phenyl | Hydroxymethyl | |
| 1.5.678 | 3-Nitro-5-propyl-Phenyl | Hydroxymethyl | |
| 1.5.679 | 3-Acetoxy-5-propyl-Phenyl | Hydroxymethyl | |
| 1.5.680 | 3-Methylsulfanyl-5-propyl-Phenyl | Hydroxymethyl | |
| 1.5.681 | 3,5-Diisopropyl-Phenyl | Hydroxymethyl | |
| 1.5.682 | 3-nButyl-5-isopropyl-Phenyl | Hydroxymethyl | |
| 1.5.683 | 3-isoButyl-5-isopropyl-Phenyl | Hydroxymethyl | |
| 1.5.684 | 3-tert.Butyl-5-isopropyl-Phenyl | Hydroxymethyl | |
| 1.5.685 | 3-Cyclopropyl-5-isopropyl-Phenyl | Hydroxymethyl | |
| 1.5.686 | 3-Cyano-5-isopropyl-Phenyl | Hydroxymethyl | |
| 1.5.687 | 3-Trifluormethyl-5-isopropyl-Phenyl | Hydroxymethyl | |
| 1.5.688 | 3-(Methoxycarbonyl)-5-isopropyl-Phenyl | Hydroxymethyl | |
| 1.5.689 | 3-Methoxy-5-isopropyl-Phenyl | Hydroxymethyl | |
| 1.5.690 | 3-Ethoxy-5-isopropyl-Phenyl | Hydroxymethyl | |
| 1.5.691 | 3-nPropyoxy-5-isopropyl-Phenyl | Hydroxymethyl | |
| 1.5.692 | 3-nButoxy-5-isopropyl-Phenyl | Hydroxymethyl | |
| 1.5.693 | 3-isoButoxy-5-isopropyl-Phenyl | Hydroxymethyl | |
| 1.5.694 | 3-Difluormethoxy-5-isopropyl-Phenyl | Hydroxymethyl | |
| 1.5.695 | 3-Trifluormethoxy-5-isopropyl-Phenyl | Hydroxymethyl | |
| 1.5.696 | 3-Nitro-5-isopropyl-Phenyl | Hydroxymethyl | |
| 1.5.697 | 3-Acetoxy-5-isopropyl-Phenyl | Hydroxymethyl | |
| 1.5.698 | 3-Methylsulfanyl-5-isopropyl-Phenyl | Hydroxymethyl | |
| 1.5.699 | 3,5-Dibutyl-Phenyl | Hydroxymethyl | |
| 1.5.700 | 3-isoButyl-5-butyl-Phenyl | Hydroxymethyl | |
| 1.5.701 | 3-tert.Butyl-5-butyl-Phenyl | Hydroxymethyl | |
| 1.5.702 | 3-Cyclopropyl-5-butyl-Phenyl | Hydroxymethyl | |
| 1.5.703 | 3-Cyano-5-butyl-Phenyl | Hydroxymethyl | |
| 1.5.704 | 3-Trifluormethyl-5-butyl-Phenyl | Hydroxymethyl | |
| 1.5.705 | 3-(Methoxycarbonyl)-5-butyl-Phenyl | Hydroxymethyl | |
| 1.5.706 | 3-Methoxy-5-butyl-Phenyl | Hydroxymethyl | |
| 1.5.707 | 3-Ethoxy-5-butyl-Phenyl | Hydroxymethyl | |
| 1.5.708 | 3-nPropyoxy-5-butyl-Phenyl | Hydroxymethyl | |
| 1.5.709 | 3-nButoxy-5-butyl-Phenyl | Hydroxymethyl | |
| 1.5.710 | 3-isoButoxy-5-butyl-Phenyl | Hydroxymethyl | |
| 1.5.711 | 3-Difluormethoxy-5-butyl-Phenyl | Hydroxymethyl | |
| 1.5.712 | 3-Trifluormethoxy-5-butyl-Phenyl | Hydroxymethyl | |
| 1.5.713 | 3-Nitro-5-butyl-Phenyl | Hydroxymethyl | |
| 1.5.714 | 3-Acetoxy-5-butyl-Phenyl | Hydroxymethyl | |
| 1.5.715 | 3-Methylsulfanyl-5-butyl-Phenyl | Hydroxymethyl | |
| 1.5.716 | 3,5-Diisobutyl-Phenyl | Hydroxymethyl | |
| 1.5.717 | 3-tert.Butyl-5-isobutyl-Phenyl | Hydroxymethyl | |
| 1.5.718 | 3-Cyclopropyl-5-isobutyl-Phenyl | Hydroxymethyl | |
| 1.5.719 | 3-Cyano-5-isobutyl-Phenyl | Hydroxymethyl | |
| 1.5.720 | 3-Trifluormethyl-5-isobutyl-Phenyl | Hydroxymethyl | |
| 1.5.721 | 3-(Methoxycarbonyl)-5-isobutyl-Phenyl | Hydroxymethyl | |
| 1.5.722 | 3-Methoxy-5-isobutyl-Phenyl | Hydroxymethyl | |
| 1.5.723 | 3-Ethoxy-5-isobutyl-Phenyl | Hydroxymethyl | |
| 1.5.724 | 3-nPropyoxy-5-isobutyl-Phenyl | Hydroxymethyl | |
| 1.5.725 | 3-nButoxy-5-isobutyl-Phenyl | Hydroxymethyl | |
| 1.5.726 | 3-isoButoxy-5-isobutyl-Phenyl | Hydroxymethyl | |
| 1.5.727 | 3-Difluormethoxy-5-isobutyl-Phenyl | Hydroxymethyl | |
| 1.5.728 | 3-Trifluormethoxy-5-isobutyl-Phenyl | Hydroxymethyl | |
| 1.5.729 | 3-Nitro-5-isobutyl-Phenyl | Hydroxymethyl | |
| 1.5.730 | 3-Acetoxy-5-isobutyl-Phenyl | Hydroxymethyl | |
| 1.5.731 | 3-Methylsulfanyl-5-isobutyl-Phenyl | Hydroxymethyl | |
| 1.5.732 | 3,5-D(itert.butyl)-Phenyl | Hydroxymethyl | |
| 1.5.733 | 3-Cyclopropyl-5-tert.butyl-Phenyl | Hydroxymethyl | |
| 1.5.734 | 3-Cyano-5-tert.butyl-Phenyl | Hydroxymethyl | |
| 1.5.735 | 3-Trifluormethyl-5-tert.butyl-Phenyl | Hydroxymethyl | |
| 1.5.736 | 3-(Methoxycarbonyl)-5-tert.butyl-Phenyl | Hydroxymethyl | |
| 1.5.737 | 3-Methoxy-5-tert.butyl-Phenyl | Hydroxymethyl | |
| 1.5.738 | 3-Ethoxy-5-tert.butyl-Phenyl | Hydroxymethyl | |
| 1.5.739 | 3-nPropyoxy-5-tert.butyl-Phenyl | Hydroxymethyl | |
| 1.5.740 | 3-nButoxy-5-tert.butyl-Phenyl | Hydroxymethyl | |
| 1.5.741 | 3-isoButoxy-5-tert.butyl-Phenyl | Hydroxymethyl | |
| 1.5.742 | 3-Difluormethoxy-5-tert.butyl-Phenyl | Hydroxymethyl | |
| 1.5.743 | 3-Trifluormethoxy-5-tert.butyl-Phenyl | Hydroxymethyl | |
| 1.5.744 | 3-Nitro-5-tert.butyl-Phenyl | Hydroxymethyl | |
| 1.5.745 | 3-Acetoxy-5-tert.butyl-Phenyl | Hydroxymethyl | |
| 1.5.746 | 3-Methylsulfanyl-5-tert.butyl-Phenyl | Hydroxymethyl | |
| 1.5.747 | 3-tert.Butyl-5-cyclopropyl-Phenyl | Hydroxymethyl | |
| 1.5.748 | 3,5-Dicyclopropyl-Phenyl | Hydroxymethyl | |
| 1.5.749 | 3-Cyano-5-cyclopropyl-Phenyl | Hydroxymethyl | |
| 1.5.750 | 3-Trifluormethyl-5-cyclopropyl-Phenyl | Hydroxymethyl | |
| 1.5.751 | 3-(Hydroxycarbonyl)-5-cyclopropyl-Phenyl | Hydroxymethyl | |
| 1.5.752 | 3-(Methoxycarbonyl)-5-cyclopropyl-Phenyl | Hydroxymethyl | |
| 1.5.753 | 3-Methoxy-5-cyclopropyl-Phenyl | Hydroxymethyl | |
| 1.5.754 | 3-Ethoxy-5-cyclopropyl-Phenyl | Hydroxymethyl | |
| 1.5.755 | 3-nPropyoxy-5-cyclopropyl-Phenyl | Hydroxymethyl | |
| 1.5.756 | 3-nButoxy-5-cyclopropyl-Phenyl | Hydroxymethyl | |
| 1.5.757 | 3-isoButoxy-5-cyclopropyl-Phenyl | Hydroxymethyl | |
| 1.5.758 | 3-Difluormethoxy-5-cyclopropyl-Phenyl | Hydroxymethyl | |
| 1.5.759 | 3-Trifluormethoxy-5-cyclopropyl-Phenyl | Hydroxymethyl | |
| 1.5.760 | 3-Nitro-5-cyclopropyl-Phenyl | Hydroxymethyl | |
| 1.5.761 | 3-Acetoxy-5-cyclopropyl-Phenyl | Hydroxymethyl | |
| 1.5.762 | 3-Methylsulfanyl-5-cyclopropyl-Phenyl | Hydroxymethyl | |
| 1.5.763 | 3,5-Dicyano-Phenyl | Hydroxymethyl | |
| 1.5.764 | 3-Trifluormethyl-5-cyano-Phenyl | Hydroxymethyl | |
| 1.5.765 | 3-(Methoxycarbonyl)-5-cyano-Phenyl | Hydroxymethyl | |
| 1.5.766 | 3-Methoxy-5-cyano-Phenyl | Hydroxymethyl | |
| 1.5.767 | 3-Ethoxy-5-cyano-Phenyl | Hydroxymethyl | |
| 1.5.768 | 3-nPropyoxy-5-cyano-Phenyl | Hydroxymethyl | |
| 1.5.769 | 3-nButoxy-5-cyano-Phenyl | Hydroxymethyl | |
| 1.5.770 | 3-isoButoxy-5-cyano-Phenyl | Hydroxymethyl | |
| 1.5.771 | 3-Difluormethoxy-5-cyano-Phenyl | Hydroxymethyl | |
| 1.5.772 | 3-Trifluormethoxy-5-cyano-Phenyl | Hydroxymethyl | |
| 1.5.773 | 3-Nitro-5-cyano-Phenyl | Hydroxymethyl | |
| 1.5.774 | 3-Acetoxy-5-cyano-Phenyl | Hydroxymethyl | |
| 1.5.775 | 3-Methylsulfanyl-5-cyano-Phenyl | Hydroxymethyl | |
| 1.5.776 | 3,5-Di(trifluormethyl)-Phenyl | Hydroxymethyl | |
| 1.5.777 | 3-(Methoxycarbonyl)-5-trifluormethyl-Phenyl | Hydroxymethyl | |
| 1.5.778 | 3-Methoxy-5-trifluormethyl-Phenyl | Hydroxymethyl | |
| 1.5.779 | 3-Ethoxy-5-trifluormethyl-Phenyl | Hydroxymethyl | |
| 1.5.780 | 3-nPropyoxy-5-trifluormethyl-Phenyl | Hydroxymethyl | |
| 1.5.781 | 3-isoButoxy-5-trifluormethyl-Phenyl | Hydroxymethyl | |
| 1.5.782 | 3-Difluormethoxy-5-trifluormethyl-Phenyl | Hydroxymethyl | |
| 1.5.783 | 3-Trifluormethoxy-5-trifluormethyl-Phenyl | Hydroxymethyl | |
| 1.5.784 | 3-Nitro-5-trifluormethyl-Phenyl | Hydroxymethyl | |
| 1.5.785 | 3-Acetoxy-5-trifluormethyl-Phenyl | Hydroxymethyl | |
| 1.5.786 | 3-Methylsulfanyl-5-trifluormethyl-Phenyl | Hydroxymethyl | |
| 1.5.787 | 3,5-Di(methoxycarbonyl)-Phenyl | Hydroxymethyl | |
| 1.5.788 | 3-Methoxy-5-(methoxycarbonyl)-Phenyl | Hydroxymethyl | |
| 1.5.789 | 3-Ethoxy-5-(methoxycarbonyl)-Phenyl | Hydroxymethyl | |
| 1.5.790 | 3-nPropyoxy-5-(methoxycarbonyl)-Phenyl | Hydroxymethyl | |
| 1.5.791 | 3-nButoxy-5-(methoxycarbonyl)-Phenyl | Hydroxymethyl | |
| 1.5.792 | 3-isoButoxy-5-(methoxycarbonyl)-Phenyl | Hydroxymethyl | |
| 1.5.793 | 3-Difluormethoxy-5-(methoxycarbonyl)-Phenyl | Hydroxymethyl | |
| 1.5.794 | 3-Trifluormethoxy-5-(methoxycarbonyl)-Phenyl | Hydroxymethyl | |
| 1.5.795 | 3-Nitro-5-(methoxycarbonyl)-Phenyl | Hydroxymethyl | |
| 1.5.796 | 3-Acetoxy-5-(methoxycarbonyl)-Phenyl | Hydroxymethyl | |
| 1.5.797 | 3-Methylsulfanyl-5-(methoxycarbonyl)-Phenyl | Hydroxymethyl | |
| 1.5.798 | 3,5-Dimethoxy-Phenyl | Hydroxymethyl | |
| 1.5.799 | 3-Ethoxy-5-methoxy-Phenyl | Hydroxymethyl | |
| 1.5.800 | 3-nPropyoxy-5-methoxy-Phenyl | Hydroxymethyl | |
| 1.5.801 | 3-isoButoxy-5-methoxy-Phenyl | Hydroxymethyl | |
| 1.5.802 | 3-Difluormethoxy-5-methoxy-Phenyl | Hydroxymethyl | |
| 1.5.803 | 3-Trifluormethoxy-5-methoxy-Phenyl | Hydroxymethyl | |
| 1.5.804 | 3-Nitro-5-methoxy-Phenyl | Hydroxymethyl | |
| 1.5.805 | 3-Acetoxy-5-methoxy-Phenyl | Hydroxymethyl | |
| 1.5.806 | 3-Methylsulfanyl-5-methoxy-Phenyl | Hydroxymethyl | |
| 1.5.807 | 3,5-Diethoxy-Phenyl | Hydroxymethyl | |
| 1.5.808 | 3-nPropyoxy-5-ethoxy-Phenyl | Hydroxymethyl | |
| 1.5.809 | 3-nButoxy-5-ethoxy-Phenyl | Hydroxymethyl | |
| 1.5.810 | 3-isoButoxy-5-ethoxy-Phenyl | Hydroxymethyl | |
| 1.5.811 | 3-Difluormethoxy-5-ethoxy-Phenyl | Hydroxymethyl | |
| 1.5.812 | 3-Trifluormethoxy-5-ethoxy-Phenyl | Hydroxymethyl | |
| 1.5.813 | 3-Nitro-5-ethoxy-Phenyl | Hydroxymethyl | |
| 1.5.814 | 3-Acetoxy-5-ethoxy-Phenyl | Hydroxymethyl | |
| 1.5.815 | 3-Methylsulfanyl-5-ethoxy-Phenyl | Hydroxymethyl | |
| 1.5.816 | 3,5-Dipropyoxy-Phenyl | Hydroxymethyl | |
| 1.5.817 | 3-nButoxy-5-propoxy-Phenyl | Hydroxymethyl | |
| 1.5.818 | 3-isoButoxy-5-propoxy-Phenyl | Hydroxymethyl | |
| 1.5.819 | 3-Difluormethoxy-5-propoxy-Phenyl | Hydroxymethyl | |
| 1.5.820 | 3-Trifluormethoxy-5-propoxy-Phenyl | Hydroxymethyl | |
| 1.5.821 | 3-Nitro-5-propoxy-Phenyl | Hydroxymethyl | |
| 1.5.822 | 3-Acetoxy-5-propoxy-Phenyl | Hydroxymethyl | |
| 1.5.823 | 3-Methylsulfanyl-5-propoxy-Phenyl | Hydroxymethyl | |
| 1.5.824 | 3,5-Di(isopropyoxy)-Phenyl | Hydroxymethyl | |
| 1.5.825 | 3-nButoxy-5-isopropoxy-Phenyl | Hydroxymethyl | |
| 1.5.826 | 3-isoButoxy-5-isopropoxy-Phenyl | Hydroxymethyl | |
| 1.5.827 | 3-Difluormethoxy-5-isopropoxy-Phenyl | Hydroxymethyl | |
| 1.5.828 | 3-Trifluormethoxy-5-isopropoxy-Phenyl | Hydroxymethyl | |
| 1.5.829 | 3-Nitro-5-isopropoxy-Phenyl | Hydroxymethyl | |
| 1.5.830 | 3-Acetoxy-5-isopropoxy-Phenyl | Hydroxymethyl | |
| 1.5.831 | 3-Methylsulfanyl-5-isopropoxy-Phenyl | Hydroxymethyl | |
| 1.5.832 | 3,5-Di(trifluormethoxy)-Phenyl | Hydroxymethyl | |
| 1.5.833 | 3-Nitro-5-trifluormethoxy-Phenyl | Hydroxymethyl | |
| 1.5.834 | 3-Methylsulfanyl-5-trifluormethoxy-Phenyl | Hydroxymethyl | |
| 1.5.835 | 3,5-Bis(difluormethoxy)-Phenyl | Hydroxymethyl | |
| 1.5.836 | 3,5-Bis(difluormethoxy)-Phenyl | 1-Hydroxyethyl | |
| 1.5.837 | 3,5-Bis(difluormethoxy)-Phenyl | 1-Hydroxypropyl | |
| 1.5.838 | 3,5-Bis(difluormethoxy)-Phenyl | (1-Hydroxy-2-methylpropyl) | |
| 1.5.839 | 3-Trifluormethoxy-5-difluormethoxy-Phenyl | Hydroxymethyl | |
| 1.5.840 | 3-Nitro-5-difluormethoxy-Phenyl | Hydroxymethyl | |
| 1.5.841 | 3-Acetoxy-5-difluormethoxy-Phenyl | Hydroxymethyl | |
| 1.5.842 | 3-Methylsulfanyl-5-difluormethoxy-Phenyl | Hydroxymethyl | |
| 1.5.843 | 3,5-Bis(acetoxy)-Phenyl | Hydroxymethyl | |
| 1.5.844 | 3-Methylsulfanyl-5-acetoxy-Phenyl | Hydroxymethyl | |
| 1.5.845 | 3,5-Dinitro-Phenyl | Hydroxymethyl | |
| 1.5.846 | 3-Acetoxy-5-nitro-Phenyl | Hydroxymethyl | |
| 1.5.847 | 3-Methylsulfanyl-5-nitro-Phenyl | Hydroxymethyl | |
| 1.5.848 | 3,5-Di(methylsulfanyl)-Phenyl | Hydroxymethyl | |
| 1.5.849 | 3,4-Difluor-Phenyl | Hydroxymethyl | |
| 1.5.850 | 3,4-Difluor-Phenyl | 1-Hydroxyethyl | |
| 1.5.851 | 3,4-Difluor-Phenyl | 1-Hydroxypropyl | |
| 1.5.852 | 3,4-Difluor-Phenyl | (1-Hydroxy-2-methylpropyl) | |
| 1.5.853 | 3,4-Difluor-Phenyl | Methoxymethyl | |
| 1.5.854 | 3,4-Difluor-Phenyl | 2-Methoxyethyl | |
| 1.5.855 | 3-Chlor-4-fluor-Phenyl | Hydroxymethyl | |
| 1.5.856 | 3-Chlor-4-fluor-Phenyl | 1-Hydroxyethyl | |
| 1.5.857 | 3-Chlor-4-fluor-Phenyl | 1-Hydroxypropyl | |
| 1.5.858 | 3-Chlor-4-fluor-Phenyl | (1-Hydroxy-2-methylpropyl) | |
| 1.5.859 | 3-Chlor-4-fluor-Phenyl | Methoxymethyl | |
| 1.5.860 | 3-Chlor-4-fluor-Phenyl | 2-Methoxyethyl | |
| 1.5.861 | 3-Brom-4-fluor-Phenyl | Hydroxymethyl | |
| 1.5.862 | 3-Methyl-4-fluor-Phenyl | Hydroxymethyl | |
| 1.5.863 | 3-Methyl-4-fluor-Phenyl | 1-Hydroxyethyl | |
| 1.5.864 | 3-Ethyl-4-fluor-Phenyl | Hydroxymethyl | |
| 1.5.865 | 3-Cyclopropyl-4-fluor-Phenyl | Hydroxymethyl | |
| 1.5.866 | 3-Cyano-4-fluor-Phenyl | Hydroxymethyl | |
| 1.5.867 | 3-Methoxy-4-fluor-Phenyl | Hydroxymethyl | |
| 1.5.868 | 3-Ethoxy-4-fluor-Phenyl | Hydroxymethyl | |
| 1.5.869 | 3-Trifluormethoxy-4-fluor-Phenyl | Hydroxymethyl | |
| 1.5.870 | 3-Nitro-4-fluor-Phenyl | Hydroxymethyl | |
| 1.5.871 | 3-Fluor-4-chlor-Phenyl | Hydroxymethyl | |
| 1.5.872 | 3,4-Dichlor-Phenyl | Hydroxymethyl | |
| 1.5.873 | 3-Brom-4-chlor-Phenyl | Hydroxymethyl | |
| 1.5.874 | 3-Methyl-4-chlor-Phenyl | Hydroxymethyl | |
| 1.5.875 | 3-Cyclopropyl-4-chlor-Phenyl | Hydroxymethyl | |
| 1.5.876 | 3-Cyano-4-chlor-Phenyl | Hydroxymethyl | |
| 1.5.877 | 3-Trifluormethyl-4-chlor-Phenyl | Hydroxymethyl | |
| 1.5.878 | 3-Methoxy-4-chlor-Phenyl | Hydroxymethyl | |
| 1.5.879 | 3-Ethoxy-4-chlor-Phenyl | Hydroxymethyl | |
| 1.5.880 | 3-Trifluormethoxy-4-chlor-Phenyl | Hydroxymethyl | |
| 1.5.881 | 3-Nitro-4-chlor-Phenyl | Hydroxymethyl | |
| 1.5.882 | 3-Fluor-4-brom-Phenyl | Hydroxymethyl | |
| 1.5.883 | 3-Chlor-4-brom-Phenyl | Hydroxymethyl | |
| 1.5.884 | 3,4-Dibrom-Phenyl | Hydroxymethyl | |
| 1.5.885 | 3-Methyl-4-brom-Phenyl | Hydroxymethyl | |
| 1.5.886 | 3-Cyclopropyl-4-brom-Phenyl | Hydroxymethyl | |
| 1.5.887 | 3-Cyano-4-brom-Phenyl | Hydroxymethyl | |
| 1.5.888 | 3-Trifluormethyl-4-brom-Phenyl | Hydroxymethyl | |
| 1.5.889 | 3-Methoxy-4-Phenyl | Hydroxymethyl | |
| 1.5.890 | 3-Ethoxy-4-brom-Phenyl | Hydroxymethyl | |
| 1.5.891 | 3-Trifluormethoxy-4-brom-Phenyl | Hydroxymethyl | |
| 1.5.892 | 3-Nitro-4-brom-Phenyl | Hydroxymethyl | |
| 1.5.893 | 3-Fluor-4-iod-Phenyl | Hydroxymethyl | |
| 1.5.894 | 3-Chlor-4-iod-Phenyl | Hydroxymethyl | |
| 1.5.895 | 3-Brom-4-iod-Phenyl | Hydroxymethyl | |
| 1.5.896 | 3-Methyl-4-iod-Phenyl | Hydroxymethyl | |
| 1.5.897 | 3-Cyclopropyl-4-iod-Phenyl | Hydroxymethyl | |
| 1.5.898 | 3-Cyano-4-iod-Phenyl | Hydroxymethyl | |
| 1.5.899 | 3-Trifluormethyl-4-iod-Phenyl | Hydroxymethyl | |
| 1.5.900 | 3-Methoxy-4-iod-Phenyl | Hydroxymethyl | |
| 1.5.901 | 3-Ethoxy-4-iod-Phenyl | Hydroxymethyl | |
| 1.5.902 | 3-Trifluormethoxy-4-iod-Phenyl | Hydroxymethyl | |
| 1.5.903 | 3-Nitro-4-iod-Phenyl | Hydroxymethyl | |
| 1.5.904 | 3-Fluor-4-methyl-Phenyl | Hydroxymethyl | |
| 1.5.905 | 3-Chlor-4-methyl-Phenyl | Hydroxymethyl | |
| 1.5.906 | 3-Brom-4-methyl-Phenyl | Hydroxymethyl | |
| 1.5.907 | 3.4-Dimethyl-Phenyl | Hydroxymethyl | |
| 1.5.908 | 3.4-Dimethyl-Phenyl | 1-Hydroxyethyl | |
| 1.5.909 | 3.4-Dimethyl-Phenyl | 1-Hydroxypropyl | |
| 1.5.910 | 3.4-Dimethyl-Phenyl | (1-Hydroxy-2-methylpropyl) | |
| 1.5.911 | 3.4-Dimethyl-Phenyl | Methoxymethyl | |
| 1.5.912 | 3.4-Dimethyl-Phenyl | 2-Methoxyethyl | |
| 1.5.913 | 3-Ethyl-4-methyl-Phenyl | Hydroxymethyl | |
| 1.5.914 | 3-Cyclopropyl-4-methyl-Phenyl | Hydroxymethyl | |
| 1.5.915 | 3-Cyano-4-methyl-Phenyl | Hydroxymethyl | |
| 1.5.916 | 3-Trifluormethyl-4-methyl-Phenyl | Hydroxymethyl | |
| 1.5.917 | 3-Methoxy-4-methyl-Phenyl | Hydroxymethyl | |
| 1.5.918 | 3-Ethoxy-4-methyl-Phenyl | Hydroxymethyl | |
| 1.5.919 | 3-Trifluormethoxy-4-methyl-Phenyl | Hydroxymethyl | |
| 1.5.920 | 3-Nitro-4-methyl-Phenyl | Hydroxymethyl | |
| 1.5.921 | 3-Fluor-4-ethyl-Phenyl | Hydroxymethyl | |
| 1.5.922 | 3-Chlor-4-ethyl-Phenyl | Hydroxymethyl | |
| 1.5.923 | 3-Brom-4-ethyl-Phenyl | Hydroxymethyl | |
| 1.5.924 | 3-Methyl-4-ethyl-Phenyl | Hydroxymethyl | |
| 1.5.925 | 3,4-Diethyl-Phenyl | Hydroxymethyl | |
| 1.5.926 | 3-Cyclopropyl-4-ethyl-Phenyl | Hydroxymethyl | |
| 1.5.927 | 3-Cyano-4-ethyl-Phenyl | Hydroxymethyl | |
| 1.5.928 | 3-Trifluormethyl-4-ethyl-Phenyl | Hydroxymethyl | |
| 1.5.929 | 3-Methoxy-4-ethyl-Phenyl | Hydroxymethyl | |
| 1.5.930 | 3-Ethoxy-4-ethyl-Phenyl | Hydroxymethyl | |
| 1.5.931 | 3-Trifluormethoxy-4-ethyl-Phenyl | Hydroxymethyl | |
| 1.5.932 | 3-Nitro-4-ethyl-Phenyl | Hydroxymethyl | |
| 1.5.933 | 3-Fluor-4-propyl-Phenyl | Hydroxymethyl | |
| 1.5.934 | 3-Chlor-4-propyl-Phenyl | Hydroxymethyl | |
| 1.5.935 | 3-Brom-4-propyl-Phenyl | Hydroxymethyl | |
| 1.5.936 | 3-Methyl-4-propyl-Phenyl | Hydroxymethyl | |
| 1.5.937 | 3-Cyclopropyl-4-propyl-Phenyl | Hydroxymethyl | |
| 1.5.938 | 3-Cyano-4-propyl-Phenyl | Hydroxymethyl | |
| 1.5.939 | 3-Trifluormethyl-4-propyl-Phenyl | Hydroxymethyl | |
| 1.5.940 | 3-Methoxy-4-propyl-Phenyl | Hydroxymethyl | |
| 1.5.941 | 3-Ethoxy-4-propyl-Phenyl | Hydroxymethyl | |
| 1.5.942 | 3-Trifluormethoxy-4-propyl-Phenyl | Hydroxymethyl | |
| 1.5.943 | 3-Nitro-4-propyl-Phenyl | Hydroxymethyl | |
| 1.5.944 | 3-Fluor-4-isopropyl-Phenyl | Hydroxymethyl | |
| 1.5.945 | 3-Chlor-4-isopropyl-Phenyl | Hydroxymethyl | |
| 1.5.946 | 3-Brom-4-isopropyl-Phenyl | Hydroxymethyl | |
| 1.5.947 | 3-Methyl-4-isopropyl-Phenyl | Hydroxymethyl | |
| 1.5.948 | 3-Cyclopropyl-4-isopropyl-Phenyl | Hydroxymethyl | |
| 1.5.949 | 3-Cyano-4-isopropyl-Phenyl | Hydroxymethyl | |
| 1.5.950 | 3-Trifluormethyl-4-isopropyl-Phenyl | Hydroxymethyl | |
| 1.5.951 | 3-Methoxy-4-isopropyl-Phenyl | Hydroxymethyl | |
| 1.5.952 | 3-Ethoxy-4-isopropyl-Phenyl | Hydroxymethyl | |
| 1.5.953 | 3-Trifluormethoxy-4-isopropyl-Phenyl | Hydroxymethyl | |
| 1.5.954 | 3-Nitro-4-isopropyl-Phenyl | Hydroxymethyl | |
| 1.5.955 | 3-Fluor-4-tert.butyl-Phenyl | Hydroxymethyl | |
| 1.5.956 | 3-Chlor-4-tert.butyl-Phenyl | Hydroxymethyl | |
| 1.5.957 | 3-Brom-4-tert.butyl-Phenyl | Hydroxymethyl | |
| 1.5.958 | 3-Methyl-4-tert.butyl-Phenyl | Hydroxymethyl | |
| 1.5.959 | 3-Cyclopropyl-4-tert.butyl-Phenyl | Hydroxymethyl | |
| 1.5.960 | 3-Cyano-4-tert.butyl-Phenyl | Hydroxymethyl | |
| 1.5.961 | 3-Trifluormethyl-4-tert.butyl-Phenyl | Hydroxymethyl | |
| 1.5.962 | 3-Trifluormethyl-4-tert.butyl-Phenyl | 1-Hydroxyethyl | |
| 1.5.963 | 3-Trifluormethyl-4-tert.butyl-Phenyl | 1-Hydroxypropyl | |
| 1.5.964 | 3-Trifluormethyl-4-tert.butyl-Phenyl | (1-Hydroxy-2-methylpropyl) | |
| 1.5.965 | 3-Trifluormethyl-4-tert.butyl-Phenyl | Methoxymethyl | |
| 1.5.966 | 3-Trifluormethyl-4-tert.butyl-Phenyl | 2-Methoxyethyl | |
| 1.5.967 | 3-Methoxy-4-tert.butyl-Phenyl | Hydroxymethyl | |
| 1.5.968 | 3-Ethoxy-4-tert.butyl-Phenyl | Hydroxymethyl | |
| 1.5.969 | 3-Trifluormethoxy-4-tert.butyl-Phenyl | Hydroxymethyl | |
| 1.5.970 | 3-Nitro-4-tert.butyl-Phenyl | Hydroxymethyl | |
| 1.5.971 | 3-Fluor-4-cyclopropyl-Phenyl | Hydroxymethyl | |
| 1.5.972 | 3-Chlor-4-cyclopropyl-Phenyl | Hydroxymethyl | |
| 1.5.973 | 3-Brom-4-cyclopropyl-Phenyl | Hydroxymethyl | |
| 1.5.974 | 3-Methyl-4-cyclopropyl-Phenyl | Hydroxymethyl | |
| 1.5.975 | 3-Cyclopropyl-4-cyclopropyl-Phenyl | Hydroxymethyl | |
| 1.5.976 | 3-Cyano-4-cyclopropyl-Phenyl | Hydroxymethyl | |
| 1.5.977 | 3-Trifluormethyl-4-cyclopropyl-Phenyl | Hydroxymethyl | |
| 1.5.978 | 3-Methoxy-4-cyclopropyl-Phenyl | Hydroxymethyl | |
| 1.5.979 | 3-Ethoxy-4-cyclopropyl-Phenyl | Hydroxymethyl | |
| 1.5.980 | 3-Trifluormethoxy-4-cyclopropyl-Phenyl | Hydroxymethyl | |
| 1.5.981 | 3-Fluor-4-methoxycarbonyl-Phenyl | Hydroxymethyl | |
| 1.5.982 | 3-Chlor-4-methoxycarbonyl-Phenyl | Hydroxymethyl | |
| 1.5.983 | 3-Brom-4-methoxycarbonyl-Phenyl | Hydroxymethyl | |
| 1.5.984 | 3-Methyl-4-methoxycarbonyl-Phenyl | Hydroxymethyl | |
| 1.5.985 | 3-Cyclopropyl-4-methoxycarbonyl-Phenyl | Hydroxymethyl | |
| 1.5.986 | 3-Cyano-4-methoxycarbonyl-Phenyl | Hydroxymethyl | |
| 1.5.987 | 3-Trifluormethyl-4-methoxycarbonyl-Phenyl | Hydroxymethyl | |
| 1.5.988 | 3-Methoxy-4-methoxycarbonyl-Phenyl | Hydroxymethyl | |
| 1.5.989 | 3-Ethoxy-4-methoxycarbonyl-Phenyl | Hydroxymethyl | |
| 1.5.990 | 3-Trifluormethoxy-4-methoxycarbonyl-Phenyl | Hydroxymethyl | |
| 1.5.991 | 3-Nitro-4-methoxycarbonyl-Phenyl | Hydroxymethyl | |
| 1.5.992 | 3-Fluor-4-cyano-Phenyl | Hydroxymethyl | |
| 1.5.993 | 3-Chlor-4-cyano-Phenyl | Hydroxymethyl | |
| 1.5.994 | 3-Brom-4-cyano-Phenyl | Hydroxymethyl | |
| 1.5.995 | 3-Methyl-4-cyano-Phenyl | Hydroxymethyl | |
| 1.5.996 | 3-Cyclopropyl-4-cyano-Phenyl | Hydroxymethyl | |
| 1.5.997 | 3,4-Dicyano-Phenyl | Hydroxymethyl | |
| 1.5.998 | 3-Trifluormethyl-4-cyano-Phenyl | Hydroxymethyl | |
| 1.5.999 | 3-Trifluormethyl-4-cyano-Phenyl | 1-Hydroxyethyl | |
| 1.5.1000 | 3-Trifluormethyl-4-cyano-Phenyl | 1-Hydroxypropyl | |
| 1.5.1001 | 3-Trifluormethyl-4-cyano-Phenyl | (1-Hydroxy-2-methylpropyl) | |
| 1.5.1002 | 3-Trifluormethyl-4-cyano-Phenyl | Methoxymethyl | |
| 1.5.1003 | 3-Trifluormethyl-4-cyano-Phenyl | 2-Methoxyethyl | |
| 1.5.1004 | 3-Methoxy-4-cyano-Phenyl | Hydroxymethyl | |
| 1.5.1005 | 3-Ethoxy-4-cyano-Phenyl | Hydroxymethyl | |
| 1.5.1006 | 3-Trifluormethoxy-4-cyano-Phenyl | Hydroxymethyl | |
| 1.5.1007 | 3-Nitro-4-cyano-Phenyl | Hydroxymethyl | |
| 1.5.1008 | 3-Fluor-4-methoxy-Phenyl | Hydroxymethyl | |
| 1.5.1009 | 3-Chlor-4-methoxy-Phenyl | Hydroxymethyl | |
| 1.5.1010 | 3-Brom-4-methoxy-Phenyl | Hydroxymethyl | |
| 1.5.1011 | 3-Methyl-4-methoxy-Phenyl | Hydroxymethyl | |
| 1.5.1012 | 3-Cyclopropyl-4-methoxy-Phenyl | Hydroxymethyl | |
| 1.5.1013 | 3-Cyano-4-methoxy-Phenyl | Hydroxymethyl | |
| 1.5.1014 | 3-Trifluormethyl-4-methoxy-Phenyl | Hydroxymethyl | |
| 1.5.1015 | 3,4-Dimethoxy-Phenyl | Hydroxymethyl | |
| 1.5.1016 | 3-Ethoxy-4-methoxy-Phenyl | Hydroxymethyl | |
| 1.5.1017 | 3-Trifluormethoxy-4-methoxy-Phenyl | Hydroxymethyl | |
| 1.5.1018 | 3-Nitro-4-methoxy-Phenyl | Hydroxymethyl | |
| 1.5.1019 | 3-Fluor-4-ethoxy-Phenyl | Hydroxymethyl | |
| 1.5.1020 | 3-Chlor-4-ethoxy-Phenyl | Hydroxymethyl | |
| 1.5.1021 | 3-Chlor-4-ethoxy-Phenyl | 1-Hydroxyethyl | |
| 1.5.1022 | 3-Chlor-4-ethoxy-Phenyl | 1-Hyd roxypropyl | |
| 1.5.1023 | 3-Chlor-4-ethoxy-Phenyl | (1-Hydroxy-2-methylpropyl) | |
| 1.5.1024 | 3-Chlor-4-ethoxy-Phenyl | Methoxymethyl | |
| 1.5.1025 | 3-Chlor-4-ethoxy-Phenyl | 2-Methoxyethyl | |
| 1.5.1026 | 3-Brom-4-ethoxy-Phenyl | Hydroxymethyl | |
| 1.5.1027 | 3-Methyl-4-ethoxy-Phenyl | Hydroxymethyl | |
| 1.5.1028 | 3-Ethyl-4-ethoxy-Phenyl | Hydroxymethyl | |
| 1.5.1029 | 3-Cyclopropyl-4-ethoxy-Phenyl | Hydroxymethyl | |
| 1.5.1030 | 3-Cyano-4-ethoxy-Phenyl | Hydroxymethyl | |
| 1.5.1031 | 3-Trifluormethyl-4-ethoxy-Phenyl | Hydroxymethyl | |
| 1.5.1032 | 3-Methoxy-4-ethoxy-Phenyl | Hydroxymethyl | |
| 1.5.1033 | 2,4-Diethoxy-Phenyl | Hydroxymethyl | |
| 1.5.1034 | 3-Trifluormethoxy-4-ethoxy-Phenyl | Hydroxymethyl | |
| 1.5.1035 | 3-Nitro-4-ethoxy-Phenyl | Hydroxymethyl | |
| 1.5.1036 | 3-Fluor-4-isopropoxy-Phenyl | Hydroxymethyl | |
| 1.5.1037 | 3-Chlor-4-isopropoxy-Phenyl | Hydroxymethyl | |
| 1.5.1038 | 3-Brom-4-isopropoxy-Phenyl | Hydroxymethyl | |
| 1.5.1039 | 3-Methyl-4-isopropoxy-Phenyl | Hydroxymethyl | |
| 1.5.1040 | 3-Cyclopropyl-4-isopropoxy-Phenyl | Hydroxymethyl | |
| 1.5.1041 | 3-Cyano-4-isopropoxy-Phenyl | Hydroxymethyl | |
| 1.5.1042 | 3-Trifluormethyl-4-isopropoxy-Phenyl | Hydroxymethyl | |
| 1.5.1043 | 3-Methoxy-4-isopropoxy-Phenyl | Hydroxymethyl | |
| 1.5.1044 | 3-Ethoxy-4-isopropoxy-Phenyl | Hydroxymethyl | |
| 1.5.1045 | 3-Trifluormethoxy-4-isopropoxy-Phenyl | Hydroxymethyl | |
| 1.5.1046 | 3-Nitro-4-isopropoxy-Phenyl | Hydroxymethyl | |
| 1.5.1047 | 3-Fluor-4-trifluormethoxy-Phenyl | Hydroxymethyl | |
| 1.5.1048 | 3-Chlor-4-trifluormethoxy-Phenyl | Hydroxymethyl | |
| 1.5.1049 | 3-Brom-4-trifluormethoxy-Phenyl | Hydroxymethyl | |
| 1.5.1050 | 3-Methyl-4-trifluormethoxy-Phenyl | Hydroxymethyl | |
| 1.5.1051 | 3-Cyclopropyl-4-trifluormethoxy-Phenyl | Hydroxymethyl | |
| 1.5.1052 | 3-Cyano-4-trifluormethoxy-Phenyl | Hydroxymethyl | |
| 1.5.1053 | 3-Trifluormethyl-4-trifluormethoxy-Phenyl | Hydroxymethyl | |
| 1.5.1054 | 3-Methoxy-4-trifluormethoxy-Phenyl | Hydroxymethyl | |
| 1.5.1055 | 3-Ethoxy-4-trifluormethoxy-Phenyl | Hydroxymethyl | |
| 1.5.1056 | 3,4-Bis(trifluormethoxy)-Phenyl | Hydroxymethyl | |
| 1.5.1057 | 3-Nitro-4-trifluormethoxy-Phenyl | Hydroxymethyl | |
| 1.5.1058 | 3-Fluor-4-difluormethoxy-Phenyl | Hydroxymethyl | |
| 1.5.1059 | 3-Chlor-4-difluormethoxy-Phenyl | Hydroxymethyl | |
| 1.5.1060 | 3-Brom-4-difluormethoxy-Phenyl | Hydroxymethyl | |
| 1.5.1061 | 3-Methyl-4-difluormethoxy-Phenyl | Hydroxymethyl | |
| 1.5.1062 | 3-Cyclopropyl-4-difluormethoxy-Phenyl | Hydroxymethyl | |
| 1.5.1063 | 3-Cyano-4-difluormethoxy-Phenyl | Hydroxymethyl | |
| 1.5.1064 | 3-Trifluormethyl-4-difluormethoxy-Phenyl | Hydroxymethyl | |
| 1.5.1065 | 3-Methoxy-4-difluormethoxy-Phenyl | Hydroxymethyl | |
| 1.5.1066 | 3-Ethoxy-4-difluormethoxy-Phenyl | Hydroxymethyl | |
| 1.5.1067 | 3-Trifluormethoxy-4-difluormethoxy-Phenyl | Hydroxymethyl | |
| 1.5.1068 | 3-Nitro-4-difluormethoxy-Phenyl | Hydroxymethyl | |
| 1.5.1069 | 3-Fluor-4-nitro-Phenyl | Hydroxymethyl | |
| 1.5.1070 | 3-Chlor-4-nitro-Phenyl | Hydroxymethyl | |
| 1.5.1071 | 3-Brom-4-nitro-Phenyl | Hydroxymethyl | |
| 1.5.1072 | 3-Methyl-4-nitro-Phenyl | Hydroxymethyl | |
| 1.5.1073 | 3-Cyclopropyl-4-nitro-Phenyl | Hydroxymethyl | |
| 1.5.1074 | 3-Cyano-4-nitro-Phenyl | Hydroxymethyl | |
| 1.5.1075 | 3-Trifluormethyl-4-nitro-Phenyl | Hydroxymethyl | |
| 1.5.1076 | 3-Methoxy-4-nitro-Phenyl | Hydroxymethyl | |
| 1.5.1077 | 3-Ethoxy-4-nitro-Phenyl | Hydroxymethyl | |
| 1.5.1078 | 3-Trifluormethoxy-4-nitro-Phenyl | Hydroxymethyl | |
| 1.5.1079 | 3-Fluor-4-methylsulfanylPhenyl | Hydroxymethyl | |
| 1.5.1080 | 3-Chlor-4-methylsulfanyl-Phenyl | Hydroxymethyl | |
| 1.5.1081 | 3-Brom-4-methylsulfanyl-Phenyl | Hydroxymethyl | |
| 1.5.1082 | 3-Methyl-4-methylsulfanyl-Phenyl | Hydroxymethyl | |
| 1.5.1083 | 3-Cyclopropyl-4-methylsulfanyl-Phenyl | Hydroxymethyl | |
| 1.5.1084 | 3-Cyano-4-methylsulfanyl-Phenyl | Hydroxymethyl | |
| 1.5.1085 | 3-Trifluormethyl-4-methylsulfanyl-Phenyl | Hydroxymethyl | |
| 1.5.1086 | 3-Methoxy-4-methylsulfanyl-Phenyl | Hydroxymethyl | |
| 1.5.1087 | 3-Ethoxy-4-methylsulfanyl-Phenyl | Hydroxymethyl | |
| 1.5.1088 | 3-Trifluormethoxy-4-methylsulfanyl-Phenyl | Hydroxymethyl | |
| 1.5.1089 | 3-Nitro-4-methylsulfanyl-Phenyl | Hydroxymethyl | |
| 1.5.1090 | 3,6-Difluor-Phenyl | Hydroxymethyl | |
| 1.5.1091 | 3,6-Difluor-Phenyl | 1-Hydroxyethyl | |
| 1.5.1092 | 3,6-Difluor-Phenyl | 1-Hydroxypropyl | |
| 1.5.1093 | 3,6-Difluor-Phenyl | (1-Hydroxy-2-methylpropyl) | |
| 1.5.1094 | 3,6-Difluor-Phenyl | Methoxymethyl | |
| 1.5.1095 | 3,6-Difluor-Phenyl | 2-Methoxyethyl | |
| 1.5.1096 | 3-Chlor-6-fluor-Phenyl | Hydroxymethyl | |
| 1.5.1097 | 3-Brom-6-fluor-Phenyl | Hydroxymethyl | |
| 1.5.1098 | 3-Methyl-6-fluor-Phenyl | Hydroxymethyl | |
| 1.5.1099 | 3-Ethyl-6-fluor-Phenyl | Hydroxymethyl | |
| 1.5.1100 | 3-Cyclopropyl-6-fluor-Phenyl | Hydroxymethyl | |
| 1.5.1101 | 3-Cyano-6-fluor-Phenyl | Hydroxymethyl | |
| 1.5.1102 | 3-Methoxy-6-fluor-Phenyl | Hydroxymethyl | |
| 1.5.1103 | 3-Ethoxy-6-fluor-Phenyl | Hydroxymethyl | |
| 1.5.1104 | 3-Trifluormethoxy-6-fluor-Phenyl | Hydroxymethyl | |
| 1.5.1105 | 3-Nitro-6-fluor-Phenyl | Hydroxymethyl | |
| 1.5.1106 | 3-Fluor-6-chlor-Phenyl | Hydroxymethyl | |
| 1.5.1107 | 3-Fluor-6-chlor-Phenyl | 1-Hydroxyethyl | |
| 1.5.1108 | 3-Fluor-6-chlor-Phenyl | 1-Hydroxypropyl | |
| 1.5.1109 | 3-Fluor-6-chlor-Phenyl | (1-Hydroxy-2-methylpropyl) | |
| 1.5.1110 | 3-Fluor-6-chlor-Phenyl | Methoxymethyl | |
| 1.5.1111 | 3-Fluor-6-chlor-Phenyl | 2-Methoxyethyl | |
| 1.5.1112 | 3,6-Dichlor-Phenyl | Hydroxymethyl | |
| 1.5.1113 | 3,6-Dichlor-Phenyl | 1-Hydroxyethyl | |
| 1.5.1114 | 3,6-Dichlor-Phenyl | 1-Hydroxypropyl | |
| 1.5.1115 | 3,6-Dichlor-Phenyl | (1-Hydroxy-2-methylpropyl) | |
| 1.5.1116 | 3,6-Dichlor-Phenyl | Methoxymethyl | |
| 1.5.1117 | 3,6-Dichlor-Phenyl | 2-Methoxyethyl | |
| 1.5.1118 | 3-Brom-6-chlor-Phenyl | Hydroxymethyl | |
| 1.5.1119 | 3-Methyl-6-chlor-Phenyl | Hydroxymethyl | |
| 1.5.1120 | 3-Cyclopropyl-6-chlor-Phenyl | Hydroxymethyl | |
| 1.5.1121 | 3-Cyano-6-chlor-Phenyl | Hydroxymethyl | |
| 1.5.1122 | 3-Trifluormethyl-6-chlor-Phenyl | Hydroxymethyl | |
| 1.5.1123 | 3-Methoxy-6-chlor-Phenyl | Hydroxymethyl | |
| 1.5.1124 | 3-Ethoxy-6-chlor-Phenyl | Hydroxymethyl | |
| 1.5.1125 | 3-Trifluormethoxy-6-chlor-Phenyl | Hydroxymethyl | |
| 1.5.1126 | 3-Nitro-6-chlor-Phenyl | Hydroxymethyl | |
| 1.5.1127 | 3-Fluor-6-brom-Phenyl | Hydroxymethyl | |
| 1.5.1128 | 3-Chlor-6-brom-Phenyl | Hydroxymethyl | |
| 1.5.1129 | 3,6-Dibrom-Phenyl | Hydroxymethyl | |
| 1.5.1130 | 3-Methyl-6-brom-Phenyl | Hydroxymethyl | |
| 1.5.1131 | 3-Cyclopropyl-6-brom-Phenyl | Hydroxymethyl | |
| 1.5.1132 | 3-Cyano-6-brom-Phenyl | Hydroxymethyl | |
| 1.5.1133 | 3-Trifluormethyl-6-brom-Phenyl | Hydroxymethyl | |
| 1.5.1134 | 3-Methoxy-6-Phenyl | Hydroxymethyl | |
| 1.5.1135 | 3-Ethoxy-6-brom-Phenyl | Hydroxymethyl | |
| 1.5.1136 | 3-Trifluormethoxy-6-brom-Phenyl | Hydroxymethyl | |
| 1.5.1137 | 3-Nitro-6-brom-Phenyl | Hydroxymethyl | |
| 1.5.1138 | 3-Fluor-6-iod-Phenyl | Hydroxymethyl | |
| 1.5.1139 | 3-Chlor-6-iod-Phenyl | Hydroxymethyl | |
| 1.5.1140 | 3-Brom-6-iod-Phenyl | Hydroxymethyl | |
| 1.5.1141 | 3-Methyl-6-iod-Phenyl | Hydroxymethyl | |
| 1.5.1142 | 3-Cyclopropyl-6-iod-Phenyl | Hydroxymethyl | |
| 1.5.1143 | 3-Cyano-6-iod-Phenyl | Hydroxymethyl | |
| 1.5.1144 | 3-Trifluormethyl-6-iod-Phenyl | Hydroxymethyl | |
| 1.5.1145 | 3-Methoxy-6-iod-Phenyl | Hydroxymethyl | |
| 1.5.1146 | 3-Ethoxy-6-iod-Phenyl | Hydroxymethyl | |
| 1.5.1147 | 3-Trifluormethoxy-6-iod-Phenyl | Hydroxymethyl | |
| 1.5.1148 | 3-Nitro-6-iod-Phenyl | Hydroxymethyl | |
| 1.5.1149 | 3-Fluor-6-methyl-Phenyl | Hydroxymethyl | |
| 1.5.1150 | 3-Chlor-6-methyl-Phenyl | Hydroxymethyl | |
| 1.5.1151 | 3-Brom-6-methyl-Phenyl | Hydroxymethyl | |
| 1.5.1152 | 3.6-Dimethyl-Phenyl | Hydroxymethyl | |
| 1.5.1153 | 3-Ethyl-6-methyl-Phenyl | Hydroxymethyl | |
| 1.5.1154 | 3-Cyclopropyl-6-methyl-Phenyl | Hydroxymethyl | |
| 1.5.1155 | 3-Cyano-6-methyl-Phenyl | Hydroxymethyl | |
| 1.5.1156 | 3-Trifluormethyl-6-methyl-Phenyl | Hydroxymethyl | |
| 1.5.1157 | 3-Methoxy-6-methyl-Phenyl | Hydroxymethyl | |
| 1.5.1158 | 3-Ethoxy-6-methyl-Phenyl | Hydroxymethyl | |
| 1.5.1159 | 3-Trifluormethoxy-6-methyl-Phenyl | Hydroxymethyl | |
| 1.5.1160 | 3-Nitro-6-methyl-Phenyl | Hydroxymethyl | |
| 1.5.1161 | 3-Fluor-6-ethyl-Phenyl | Hydroxymethyl | |
| 1.5.1162 | 3-Chlor-6-ethyl-Phenyl | Hydroxymethyl | |
| 1.5.1163 | 3-Brom-6-ethyl-Phenyl | Hydroxymethyl | |
| 1.5.1164 | 3-Methyl-6-ethyl-Phenyl | Hydroxymethyl | |
| 1.5.1165 | 3,6-Diethyl-Phenyl | Hydroxymethyl | |
| 1.5.1166 | 3-Cyclopropyl-6-ethyl-Phenyl | Hydroxymethyl | |
| 1.5.1167 | 3-Cyano-6-ethyl-Phenyl | Hydroxymethyl | |
| 1.5.1168 | 3-Trifluormethyl-6-ethyl-Phenyl | Hydroxymethyl | |
| 1.5.1169 | 3-Methoxy-6-ethyl-Phenyl | Hydroxymethyl | |
| 1.5.1170 | 3-Ethoxy-6-ethyl-Phenyl | Hydroxymethyl | |
| 1.5.1171 | 3-Trifluormethoxy-6-ethyl-Phenyl | Hydroxymethyl | |
| 1.5.1172 | 3-Nitro-6-ethyl-Phenyl | Hydroxymethyl | |
| 1.5.1173 | 3-Fluor-6-propyl-Phenyl | Hydroxymethyl | |
| 1.5.1174 | 3-Chlor-6-propyl-Phenyl | Hydroxymethyl | |
| 1.5.1175 | 3-Brom-6-propyl-Phenyl | Hydroxymethyl | |
| 1.5.1176 | 3-Methyl-6-propyl-Phenyl | Hydroxymethyl | |
| 1.5.1177 | 3-Methyl-6-propyl-Phenyl | Hydroxymethyl | |
| 1.5.1178 | 3-Cyclopropyl-6-propyl-Phenyl | Hydroxymethyl | |
| 1.5.1179 | 3-Cyano-6-propyl-Phenyl | Hydroxymethyl | |
| 1.5.1180 | 3-Trifluormethyl-6-propyl-Phenyl | Hydroxymethyl | |
| 1.5.1181 | 3-Methoxy-6-propyl-Phenyl | Hydroxymethyl | |
| 1.5.1182 | 3-Ethoxy-6-propyl-Phenyl | Hydroxymethyl | |
| 1.5.1183 | 3-Trifluormethoxy-6-propyl-Phenyl | Hydroxymethyl | |
| 1.5.1184 | 3-Nitro-6-propyl-Phenyl | Hydroxymethyl | |
| 1.5.1185 | 3-Fluor-6-isopropyl-Phenyl | Hydroxymethyl | |
| 1.5.1186 | 3-Chlor-6-isopropyl-Phenyl | Hydroxymethyl | |
| 1.5.1187 | 3-Brom-6-isopropyl-Phenyl | Hydroxymethyl | |
| 1.5.1188 | 3-Methyl-6-isopropyl-Phenyl | Hydroxymethyl | |
| 1.5.1189 | 3-Cyclopropyl-6-isopropyl-Phenyl | Hydroxymethyl | |
| 1.5.1190 | 3-Cyano-6-isopropyl-Phenyl | Hydroxymethyl | |
| 1.5.1191 | 3-Trifluormethyl-6-isopropyl-Phenyl | Hydroxymethyl | |
| 1.5.1192 | 3-Ethoxy-6-isopropyl-Phenyl | Hydroxymethyl | |
| 1.5.1193 | 3-Trifluormethoxy-6-isopropyl-Phenyl | Hydroxymethyl | |
| 1.5.1194 | 3-Nitro-6-isopropyl-Phenyl | Hydroxymethyl | |
| 1.5.1195 | 3-Fluor-6-tert.butyl-Phenyl | Hydroxymethyl | |
| 1.5.1196 | 3-Chlor-6-tert.butyl-Phenyl | Hydroxymethyl | |
| 1.5.1197 | 3-Brom-6-tert.butyl-Phenyl | Hydroxymethyl | |
| 1.5.1198 | 3-Methyl-6-tert.butyl-Phenyl | Hydroxymethyl | |
| 1.5.1199 | 3-Cyclopropyl-6-tert.butyl-Phenyl | Hydroxymethyl | |
| 1.5.1200 | 3-Cyano-6-tert.butyl-Phenyl | Hydroxymethyl | |
| 1.5.1201 | 3-Trifluormethyl-6-tert.butyl-Phenyl | Hydroxymethyl | |
| 1.5.1202 | 3-Methoxy-6-tert.butyl-Phenyl | Hydroxymethyl | |
| 1.5.1203 | 3-Ethoxy-6-tert.butyl-Phenyl | Hydroxymethyl | |
| 1.5.1204 | 3-Trifluormethoxy-6-tert.butyl -Phenyl | Hydroxymethyl | |
| 1.5.1205 | 3-Nitro-6-tert.butyl-Phenyl | Hydroxymethyl | |
| 1.5.1206 | 3-Fluor-6-cyclopropyl-Phenyl | Hydroxymethyl | |
| 1.5.1207 | 3-Chlor-6-cyclopropyl-Phenyl | Hydroxymethyl | |
| 1.5.1208 | 3-Brom-6-cyclopropyl-Phenyl | Hydroxymethyl | |
| 1.5.1209 | 3-Methyl-6-cyclopropyl-Phenyl | Hydroxymethyl | |
| 1.5.1210 | 3-Cyclopropyl-6-cyclopropyl-Phenyl | Hydroxymethyl | |
| 1.5.1211 | 3-Cyano-6-cyclopropyl-Phenyl | Hydroxymethyl | |
| 1.5.1212 | 3-Trifluormethyl-6-cyclopropyl-Phenyl | Hydroxymethyl | |
| 1.5.1213 | 3-Methoxy-6-cyclopropyl-Phenyl | Hydroxymethyl | |
| 1.5.1214 | 3-Ethoxy-6-cyclopropyl-Phenyl | Hydroxymethyl | |
| 1.5.1215 | 3-Trifluormethoxy-6-cyclopropyl-Phenyl | Hydroxymethyl | |
| 1.5.1216 | 3-Fluor-6-methoxycarbonyl-Phenyl | Hydroxymethyl | |
| 1.5.1217 | 3-Chlor-6-methoxycarbonyl-Phenyl | Hydroxymethyl | |
| 1.5.1218 | 3-Brom-6-methoxycarbonyl-Phenyl | Hydroxymethyl | |
| 1.5.1219 | 3-Methyl-6-methoxycarbonyl-Phenyl | Hydroxymethyl | |
| 1.5.1220 | 3-Cyclopropyl-6-methoxycarbonyl-Phenyl | Hydroxymethyl | |
| 1.5.1221 | 3-Cyano-6-methoxycarbonyl-Phenyl | Hydroxymethyl | |
| 1.5.1222 | 3-Trifluormethyl-6-methoxycarbonyl-Phenyl | Hydroxymethyl | |
| 1.5.1223 | 3-Methoxy-6-methoxycarbonyl-Phenyl | Hydroxymethyl | |
| 1.5.1224 | 3-Ethoxy-6-methoxycarbonyl-Phenyl | Hydroxymethyl | |
| 1.5.1225 | 3-Trifluormethoxy-6-methoxycarbonyl-Phenyl | Hydroxymethyl | |
| 1.5.1226 | 3-Nitro-6-methoxycarbonyl-Phenyl | Hydroxymethyl | |
| 1.5.1227 | 3-Fluor-6-cyano-Phenyl | Hydroxymethyl | |
| 1.5.1228 | 3-Chlor-6-cyano-Phenyl | Hydroxymethyl | |
| 1.5.1229 | 3-Brom-6-cyano-Phenyl | Hydroxymethyl | |
| 1.5.1230 | 3-Methyl-6-cyano-Phenyl | Hydroxymethyl | |
| 1.5.1231 | 3-Cyclopropyl-6-cyano-Phenyl | Hydroxymethyl | |
| 1.5.1232 | 3-Cyano-6-cyano-Phenyl | Hydroxymethyl | |
| 1.5.1233 | 3-Trifluormethyl-6-cyano-Phenyl | Hydroxymethyl | |
| 1.5.1234 | 3-Methoxy-6-cyano-Phenyl | Hydroxymethyl | |
| 1.5.1235 | 3-Ethoxy-6-cyano-Phenyl | Hydroxymethyl | |
| 1.5.1236 | 3-Trifluormethoxy-6-cyano-Phenyl | Hydroxymethyl | |
| 1.5.1237 | 3-Nitro-6-cyano-Phenyl | Hydroxymethyl | |
| 1.5.1238 | 3-Fluor-6-methoxy-Phenyl | Hydroxymethyl | |
| 1.5.1239 | 3-Chlor-6-methoxy-Phenyl | Hydroxymethyl | |
| 1.5.1240 | 3-Brom-6-methoxy-Phenyl | Hydroxymethyl | |
| 1.5.1241 | 3-Methyl-6-methoxy-Phenyl | Hydroxymethyl | |
| 1.5.1242 | 3-Cyclopropyl-6-methoxy-Phenyl | Hydroxymethyl | |
| 1.5.1243 | 3-Cyano-6-methoxy-Phenyl | Hydroxymethyl | |
| 1.5.1244 | 3-Trifluormethyl-6-methoxy-Phenyl | Hydroxymethyl | |
| 1.5.1245 | 3,6-Dimethoxy-Phenyl | Hydroxymethyl | |
| 1.5.1246 | 3-Ethoxy-6-methoxy-Phenyl | Hydroxymethyl | |
| 1.5.1247 | 3-Trifluormethoxy-6-methoxy-Phenyl | Hydroxymethyl | |
| 1.5.1248 | 3-Nitro-6-methoxy-Phenyl | Hydroxymethyl | |
| 1.5.1249 | 3-Fluor-6-ethoxy-Phenyl | Hydroxymethyl | |
| 1.5.1250 | 3-Chlor-6-ethoxy-Phenyl | Hydroxymethyl | |
| 1.5.1251 | 3-Brom-6-ethoxy-Phenyl | Hydroxymethyl | |
| 1.5.1252 | 3-Methyl-6-ethoxy-Phenyl | Hydroxymethyl | |
| 1.5.1253 | 3-Cyclopropyl-6-ethoxy-Phenyl | Hydroxymethyl | |
| 1.5.1254 | 3-Cyano-6-ethoxy-Phenyl | Hydroxymethyl | |
| 1.5.1255 | 3-Trifluormethyl-6-ethoxy-Phenyl | Hydroxymethyl | |
| 1.5.1256 | 3-Methoxy-6-ethoxy-Phenyl | Hydroxymethyl | |
| 1.5.1257 | 2,6-Diethoxy-Phenyl | Hydroxymethyl | |
| 1.5.1258 | 3-Trifluormethoxy-6-ethoxy-Phenyl | Hydroxymethyl | |
| 1.5.1259 | 3-Nitro-6-ethoxy-Phenyl | Hydroxymethyl | |
| 1.5.1260 | 3-Fluor-6-isopropoxy-Phenyl | Hydroxymethyl | |
| 1.5.1261 | 3-Chlor-6-isopropoxy-Phenyl | Hydroxymethyl | |
| 1.5.1262 | 3-Brom-6-isopropoxy-Phenyl | Hydroxymethyl | |
| 1.5.1263 | 3-Methyl-6-isopropoxy-Phenyl | Hydroxymethyl | |
| 1.5.1264 | 3-Cyclopropyl-6-isopropoxy-Phenyl | Hydroxymethyl | |
| 1.5.1265 | 3-Cyano-6-isopropoxy-Phenyl | Hydroxymethyl | |
| 1.5.1266 | 3-Trifluormethyl-6-isopropoxy-Phenyl | Hydroxymethyl | |
| 1.5.1267 | 3-Methoxy-6-isopropoxy-Phenyl | Hydroxymethyl | |
| 1.5.1268 | 3-Ethoxy-6-isopropoxy-Phenyl | Hydroxymethyl | |
| 1.5.1269 | 3-Trifluormethoxy-6-isopropoxy-Phenyl | Hydroxymethyl | |
| 1.5.1270 | 3-Nitro-6-isopropoxy-Phenyl | Hydroxymethyl | |
| 1.5.1271 | 3-Fluor-6-trifluormethoxy-Phenyl | Hydroxymethyl | |
| 1.5.1272 | 3-Chlor-6-trifluormethoxy-Phenyl | Hydroxymethyl | |
| 1.5.1273 | 3-Brom-6-trifluormethoxy-Phenyl | Hydroxymethyl | |
| 1.5.1274 | 3-Methyl-6-trifluormethoxy-Phenyl | Hydroxymethyl | |
| 1.5.1275 | 3-Cyclopropyl-6-trifluormethoxy-Phenyl | Hydroxymethyl | |
| 1.5.1276 | 3-Cyano-6-trifluormethoxy-Phenyl | Hydroxymethyl | |
| 1.5.1277 | 3-Trifluormethyl-6-trifluormethoxy-Phenyl | Hydroxymethyl | |
| 1.5.1278 | 3-Methoxy-6-trifluormethoxy-Phenyl | Hydroxymethyl | |
| 1.5.1279 | 3-Ethoxy-6-trifluormethoxy-Phenyl | Hydroxymethyl | |
| 1.5.1280 | 3,6-Bis(trifluormethoxy)-Phenyl | Hydroxymethyl | |
| 1.5.1281 | 3-Nitro-6-trifluormethoxy-Phenyl | Hydroxymethyl | |
| 1.5.1282 | 3-Fluor-6-difluormethoxy-Phenyl | Hydroxymethyl | |
| 1.5.1283 | 3-Chlor-6-difluormethoxy-Phenyl | Hydroxymethyl | |
| 1.5.1284 | 3-Brom-6-difluormethoxy-Phenyl | Hydroxymethyl | |
| 1.5.1285 | 3-Methyl-6-difluormethoxy-Phenyl | Hydroxymethyl | |
| 1.5.1286 | 3-Cyclopropyl-6-difluormethoxy-Phenyl | Hydroxymethyl | |
| 1.5.1287 | 3-Cyano-6-difluormethoxy-Phenyl | Hydroxymethyl | |
| 1.5.1288 | 3-Trifluormethyl-6-difluormethoxy-Phenyl | Hydroxymethyl | |
| 1.5.1289 | 3-Methoxy-6-difluormethoxy-Phenyl | Hydroxymethyl | |
| 1.5.1290 | 3-Ethoxy-6-difluormethoxy-Phenyl | Hydroxymethyl | |
| 1.5.1291 | 3-Trifluormethoxy-6-difluormethoxy-Phenyl | Hydroxymethyl | |
| 1.5.1292 | 3-Nitro-6-difluormethoxy-Phenyl | Hydroxymethyl | |
| 1.5.1293 | 3-Fluor-6-nitro-Phenyl | Hydroxymethyl | |
| 1.5.1294 | 3-Chlor-6-nitro-Phenyl | Hydroxymethyl | |
| 1.5.1295 | 3-Brom-6-nitro-Phenyl | Hydroxymethyl | |
| 1.5.1296 | 3-Methyl-6-nitro-Phenyl | Hydroxymethyl | |
| 1.5.1297 | 3-Cyclopropyl-6-nitro-Phenyl | Hydroxymethyl | |
| 1.5.1298 | 3-Cyano-6-nitro-Phenyl | Hydroxymethyl | |
| 1.5.1299 | 3-Trifluormethyl-6-nitro-Phenyl | Hydroxymethyl | |
| 1.5.1300 | 3-Methoxy-6-nitro-Phenyl | Hydroxymethyl | |
| 1.5.1301 | 3-Ethoxy-6-nitro-Phenyl | Hydroxymethyl | |
| 1.5.1302 | 3-Trifluormethoxy-6-nitro-Phenyl | Hydroxymethyl | |
| 1.5.1303 | 3-Fluor-6-methylsulfanylPhenyl | Hydroxymethyl | |
| 1.5.1304 | 3-Chlor-6-methylsulfanyl-Phenyl | Hydroxymethyl | |
| 1.5.1305 | 3-Brom-6-methylsulfanyl-Phenyl | Hydroxymethyl | |
| 1.5.1306 | 3-Methyl-6-methylsulfanyl-Phenyl | Hydroxymethyl | |
| 1.5.1307 | 3-Cyclopropyl-6-methylsulfanyl-Phenyl | Hydroxymethyl | |
| 1.5.1308 | 3-Cyano-6-methylsulfanyl-Phenyl | Hydroxymethyl | |
| 1.5.1309 | 3-Trifluormethyl-6-methylsulfanyl-Phenyl | Hydroxymethyl | |
| 1.5.1310 | 3-Methoxy-6-methylsulfanyl-Phenyl | Hydroxymethyl | |
| 1.5.1311 | 3-Ethoxy-6-methylsulfanyl-Phenyl | Hydroxymethyl | |
| 1.5.1312 | 3-Trifluormethoxy-6-methylsulfanyl-Phenyl | Hydroxymethyl | |
| 1.5.1313 | 3-Nitro-6-methylsulfanyl-Phenyl | Hydroxymethyl | |
| 1.5.1314 | 2,3,4-Trifluor-Phenyl | Hydroxymethyl | |
| 1.5.1315 | 2,3,4-Trichlor-Phenyl | Hydroxymethyl | |
| 1.5.1316 | 2,3.4-Trimethyl-Phenyl | Hydroxymethyl | |
| 1.5.1317 | 2-Fluor-2-chlor-5-trifluormethyl-Phenyl | Hydroxymethyl | |
| 1.5.1318 | 2,3,5-Trifluor-Phenyl | Hydroxymethyl | |
| 1.5.1319 | 2,3,5-Trichlor-Phenyl | Hydroxymethyl | |
| 1.5.1320 | 2,3,5-Trimethyl-Phenyl | Hydroxymethyl | |
| 1.5.1321 | 2,3-Dichlor-5-methoxy-Phenyl | Hydroxymethyl | |
| 1.5.1322 | 2,3,6-Trifluor-Phenyl | Hydroxymethyl | |
| 1.5.1323 | 2,3,6-Trichlor-Phenyl | Hydroxymethyl | |
| 1.5.1324 | 2,3,6-Trimethyl-Phenyl | Hydroxymethyl | |
| 1.5.1325 | 3,4,5-Trifluor-Phenyl | Hydroxymethyl | |
| 1.5.1326 | 3,4,5-Trichlor-Phenyl | Hydroxymethyl | |
| 1.5.1327 | 3,4,5-Trimethyl-Phenyl | Hydroxymethyl | |
| 1.5.1328 | 3,5-Dimethyl-4-fluor-Phenyl | Hydroxymethyl | |
| 1.5.1329 | 3,5-Dichlor-4-methoxy-Phenyl | Hydroxymethyl | |
| 1.5.1330 | 3,5-Difluor-4-chlor-Phenyl | Hydroxymethyl | |
| 1.5.1331 | 3,5-Dichlor-4-hydroxy-Phenyl | Hydroxymethyl | |
| 1.5.1332 | 3,5-Trifluormethyl-4-chlor-Phenyl | Hydroxymethyl | |
| 1.5.1333 | 3,4,6-Trifluor-Phenyl | Hydroxymethyl | |
| 1.5.1334 | 3,4,6-Trichlor-Phenyl | Hydroxymethyl | |
| 1.5.1335 | 3,4,6-Trimethyl-Phenyl | Hydroxymethyl | |
| 1.5.1336 | Pentafluorphenyl | Hydroxymethyl | |

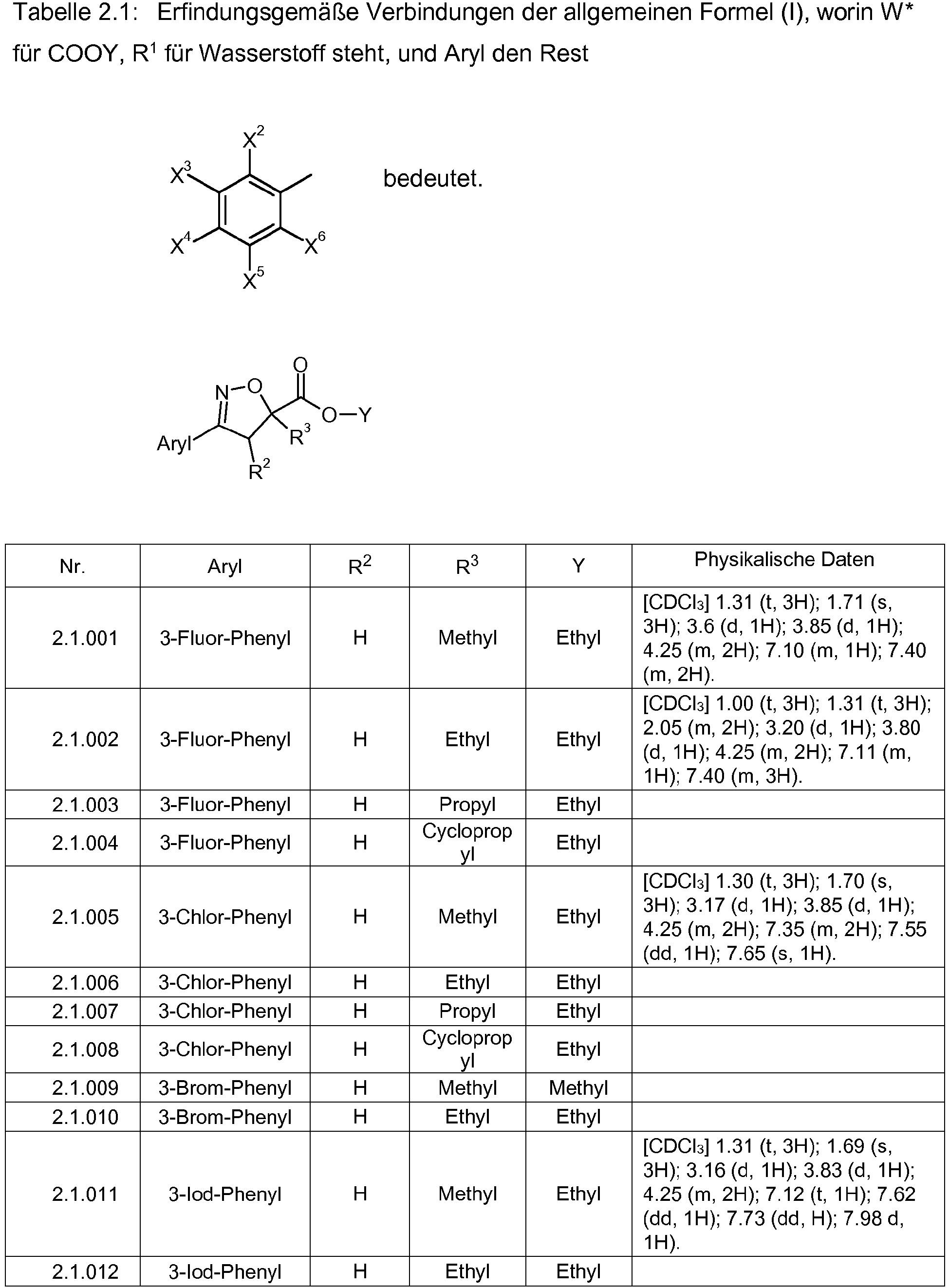

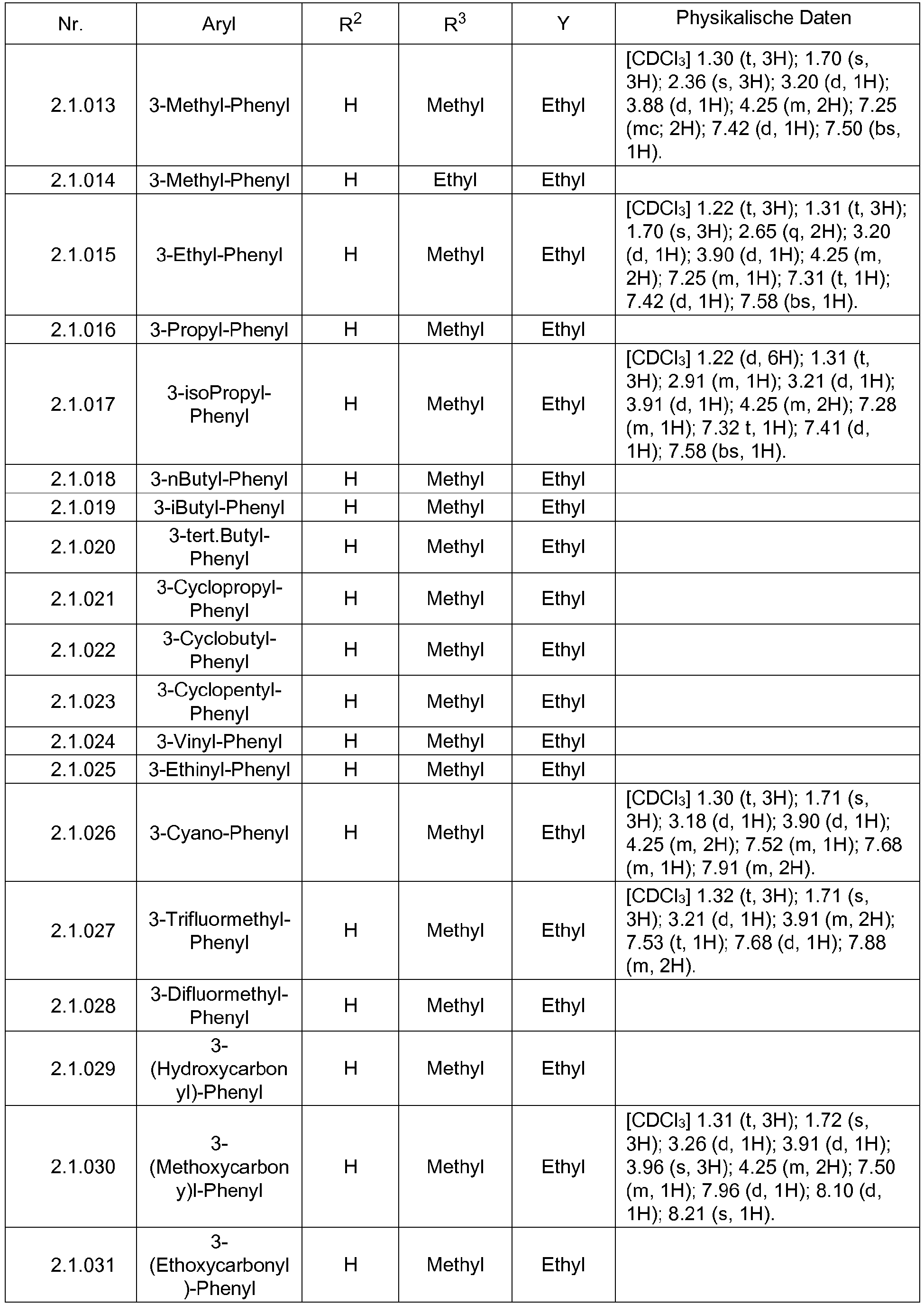

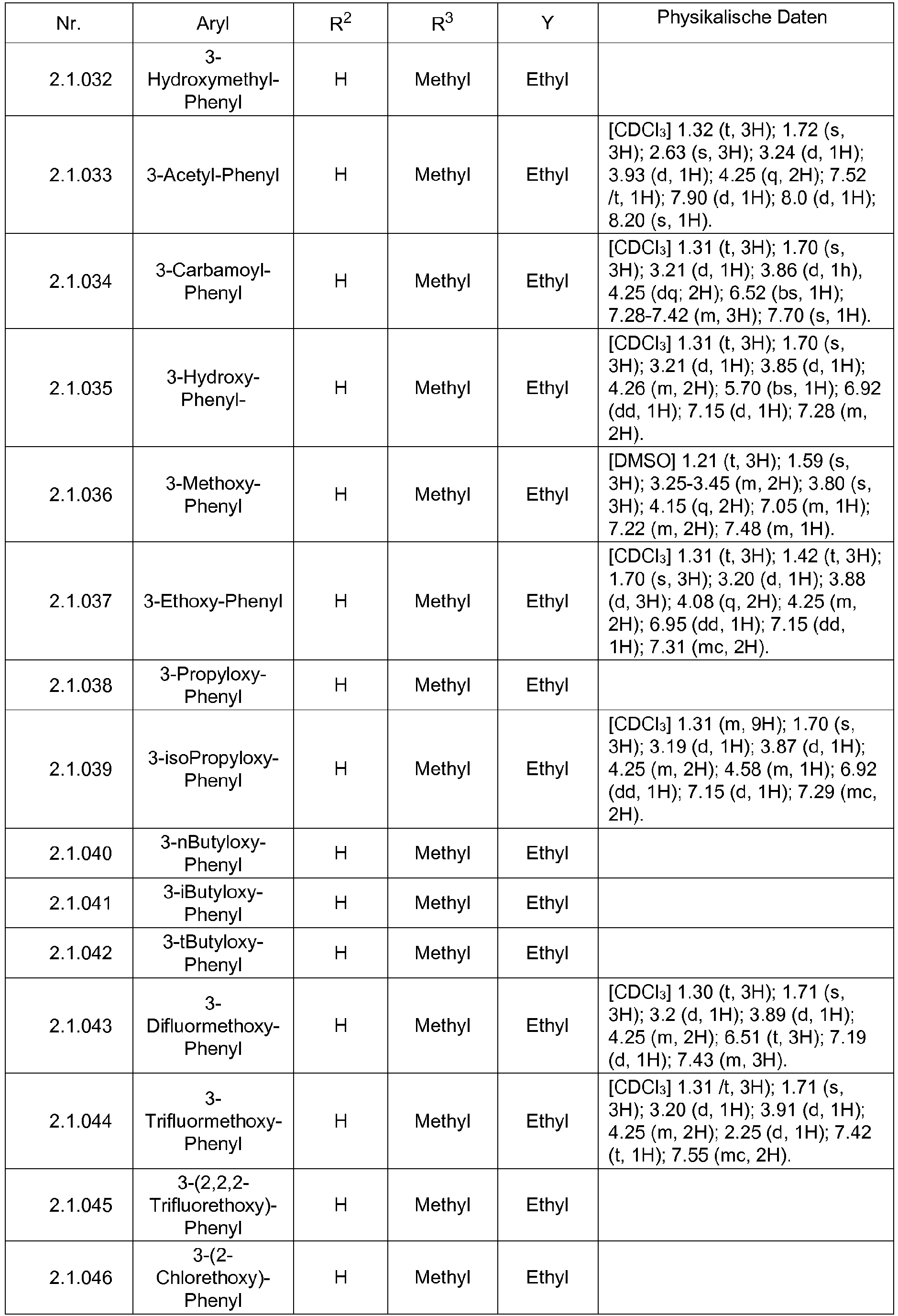

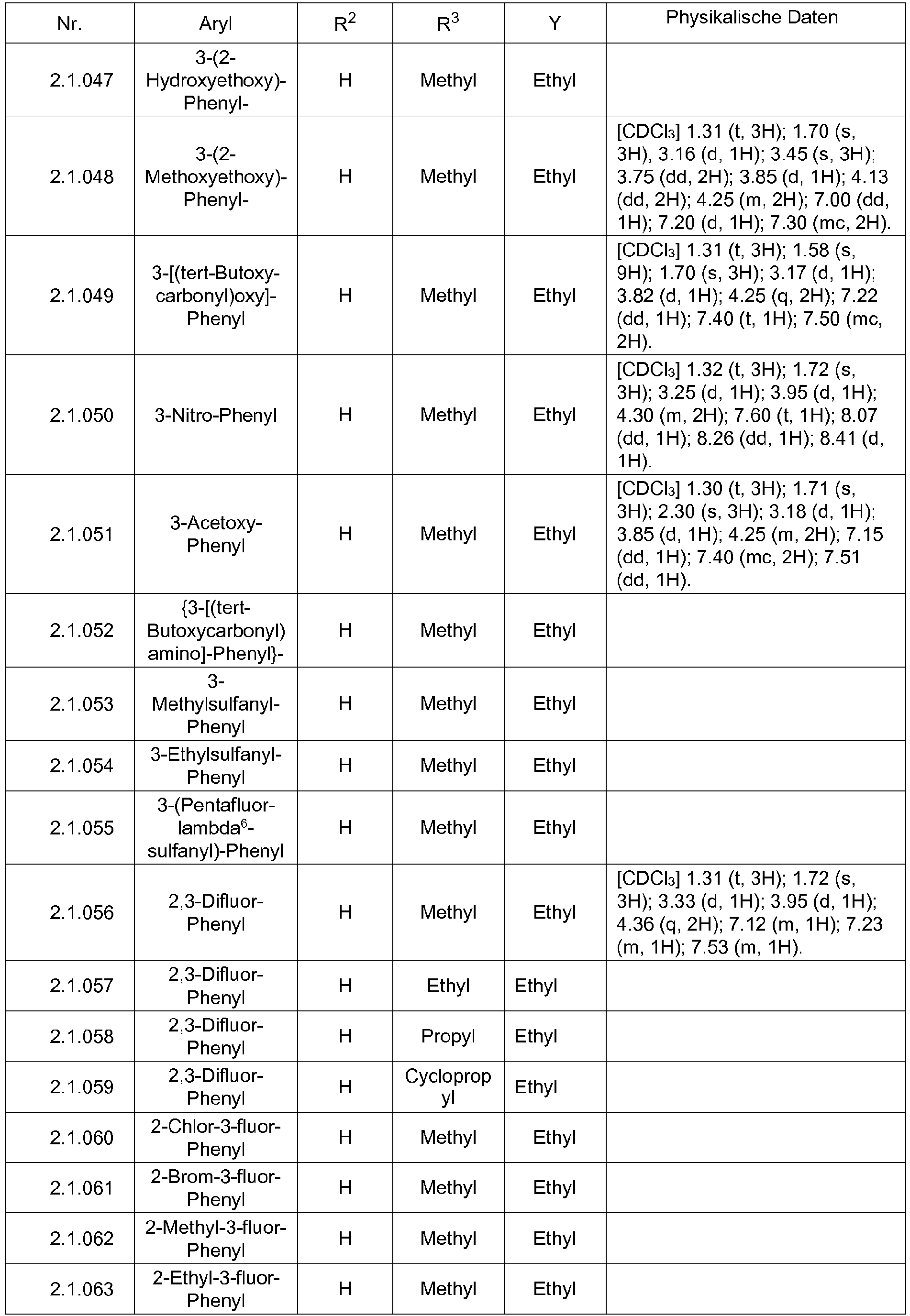

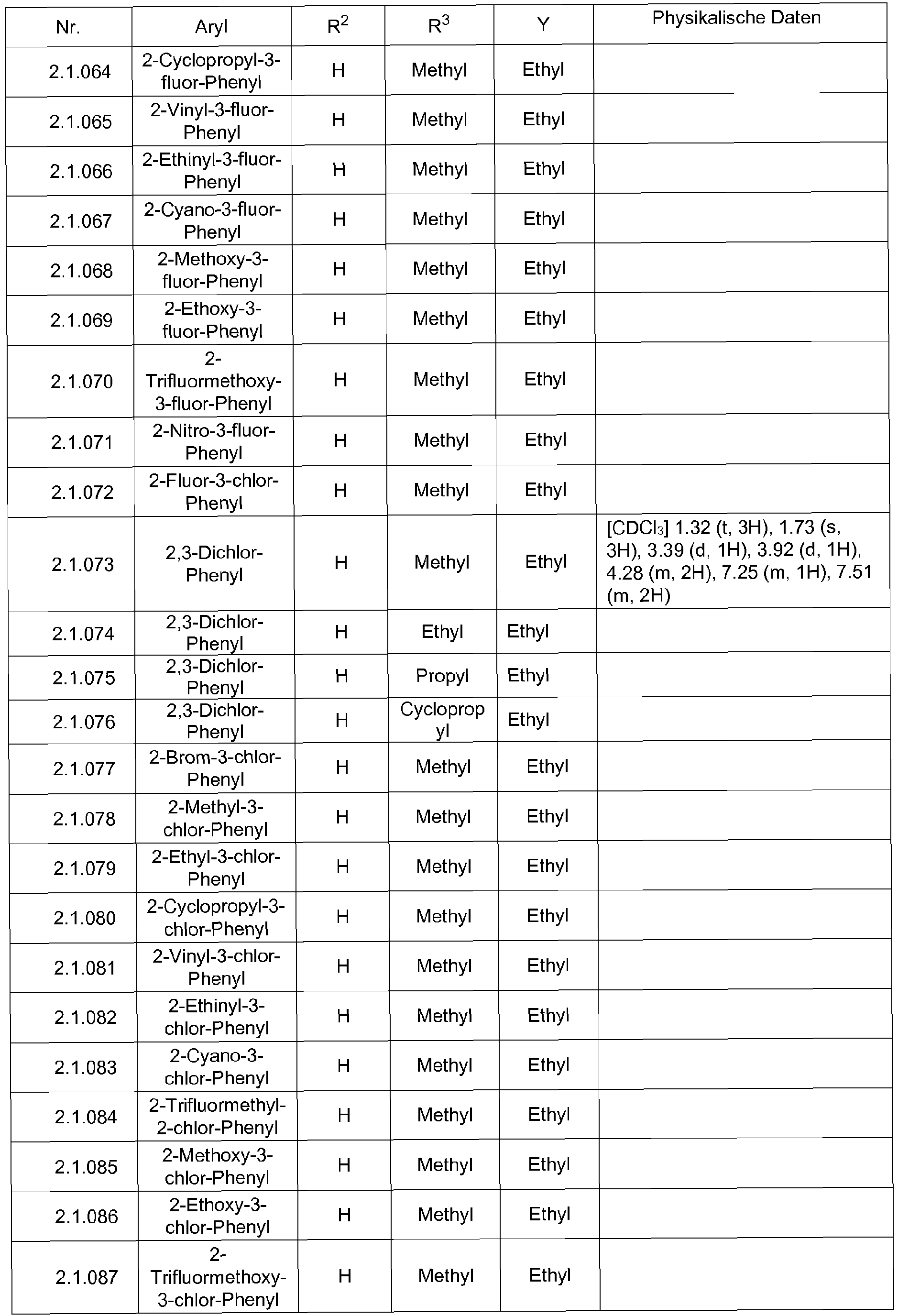

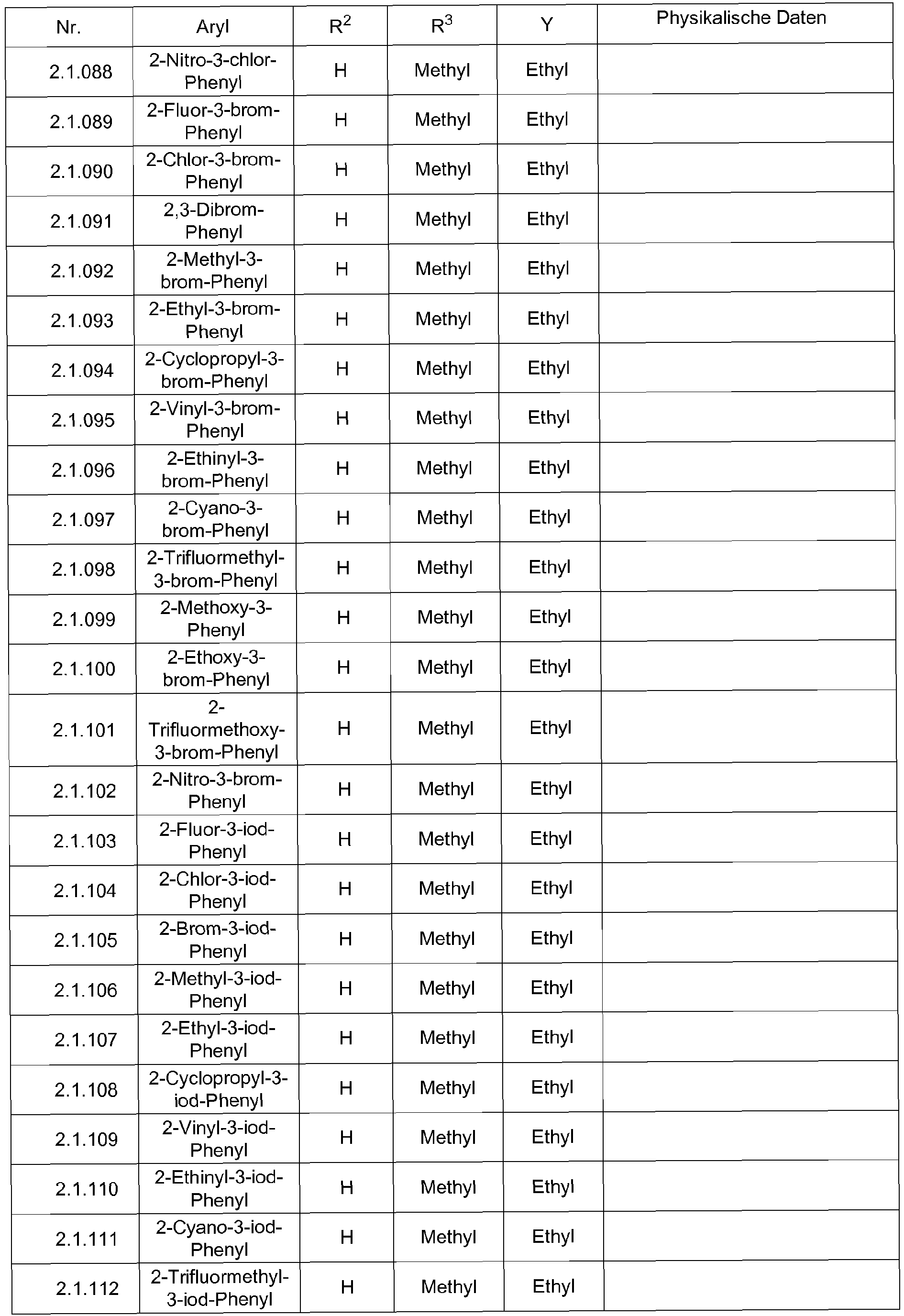

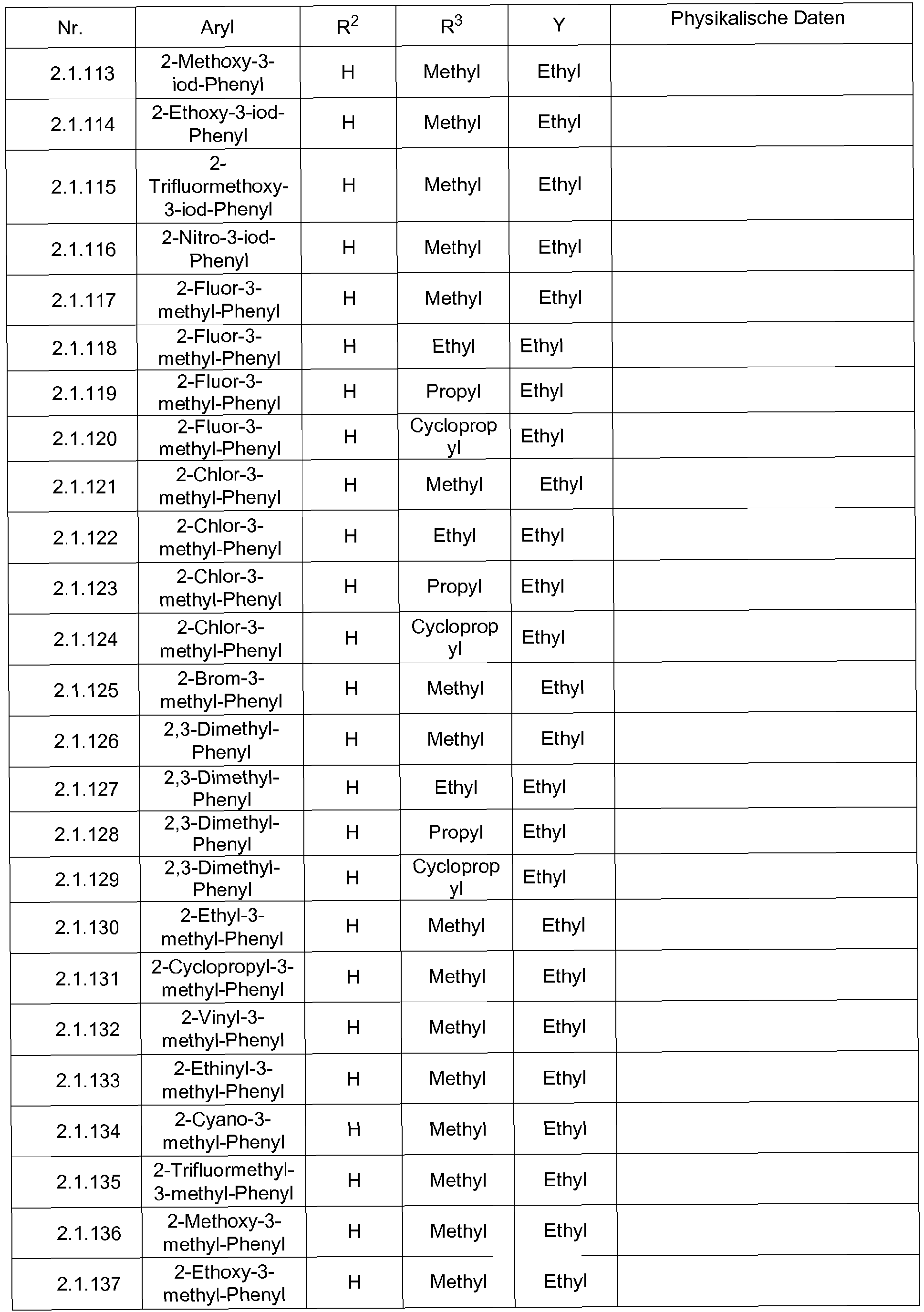

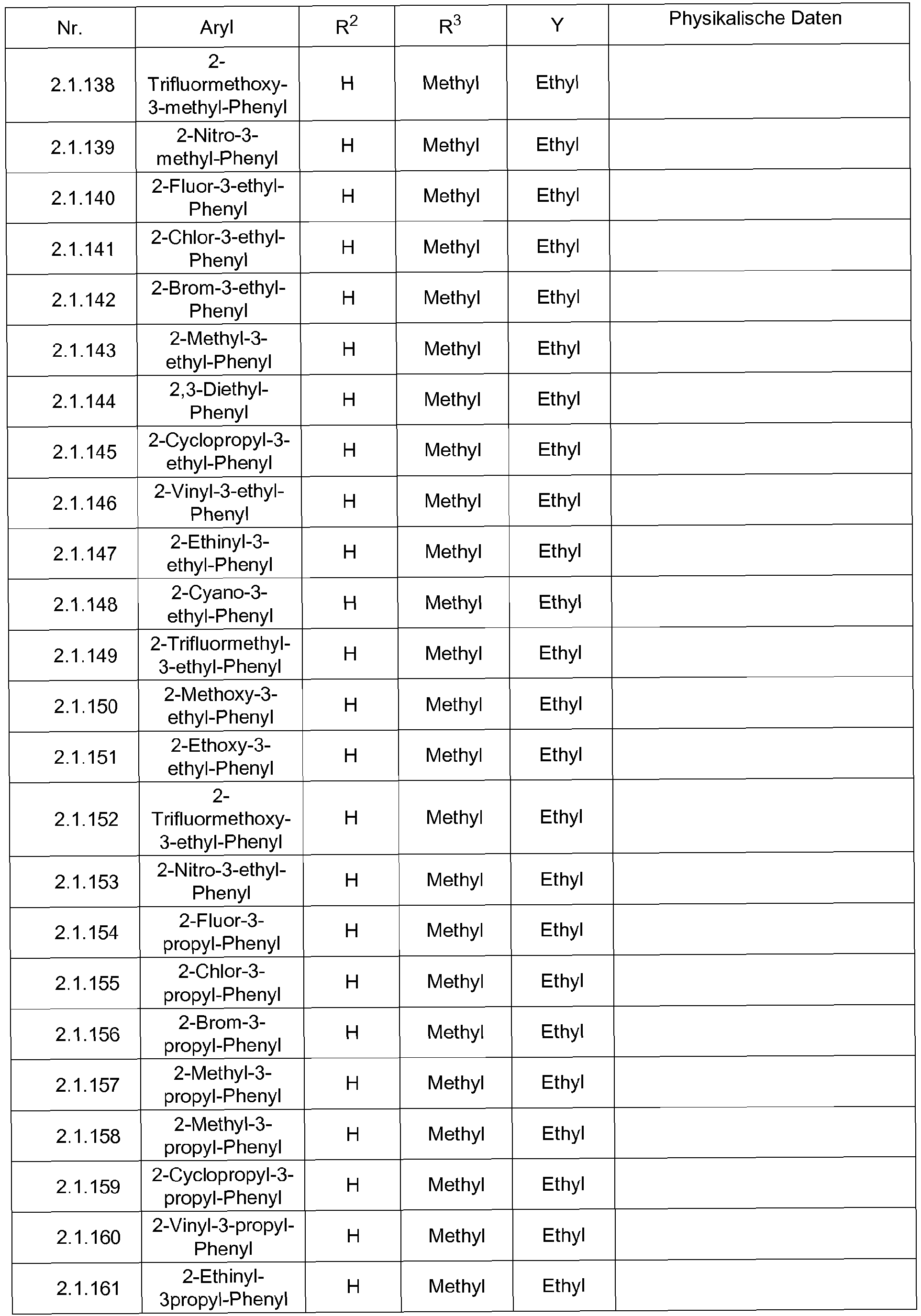

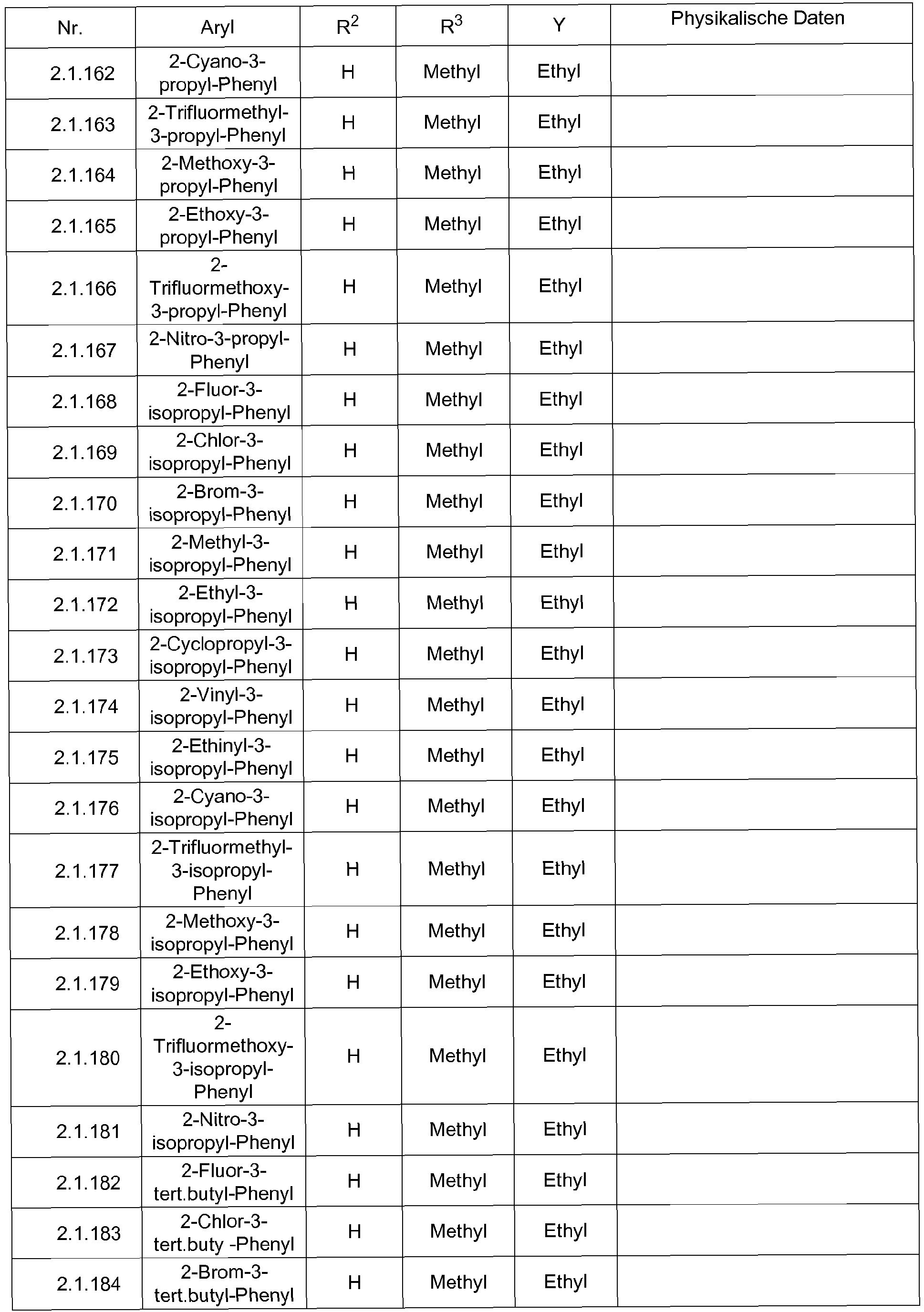

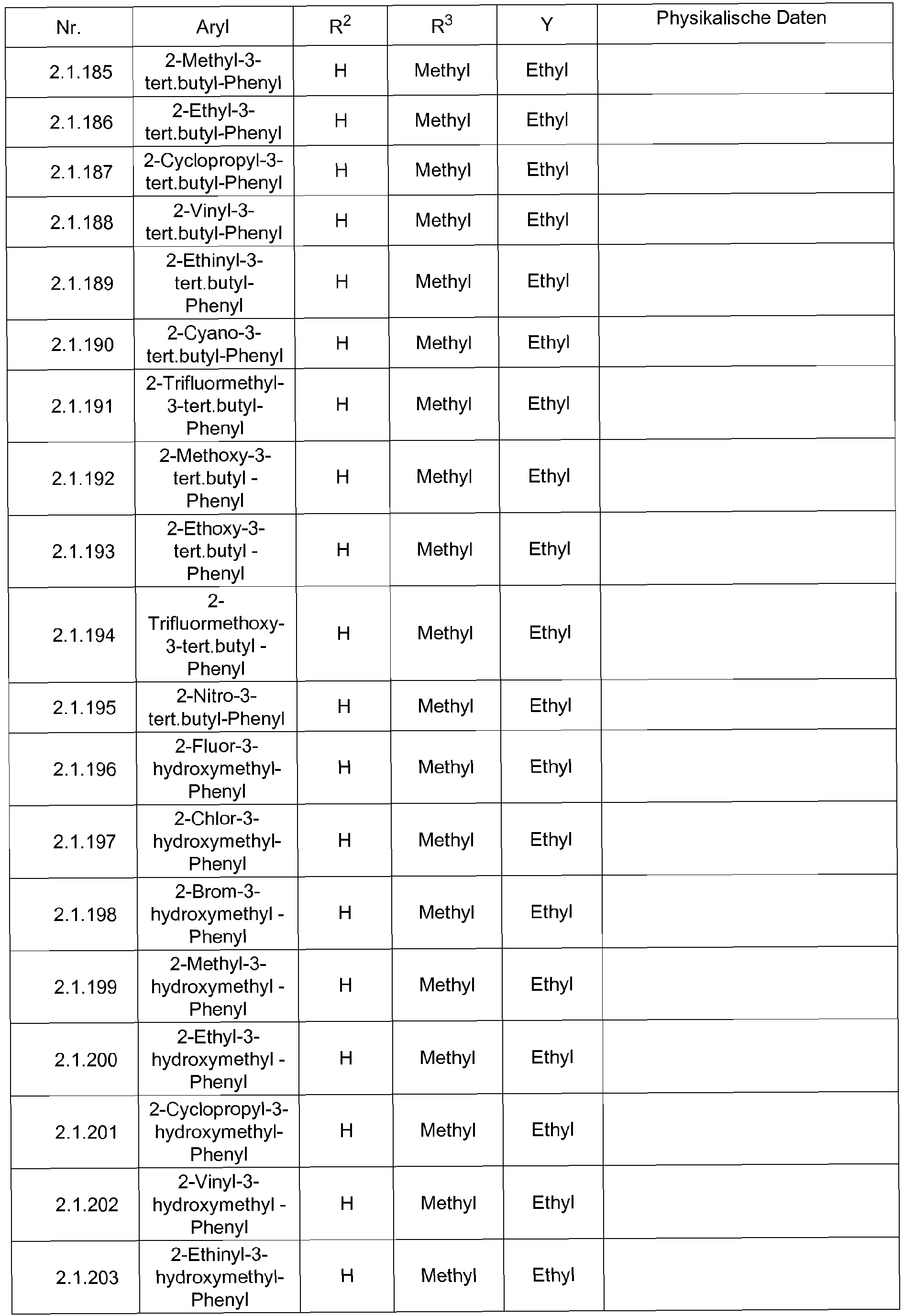

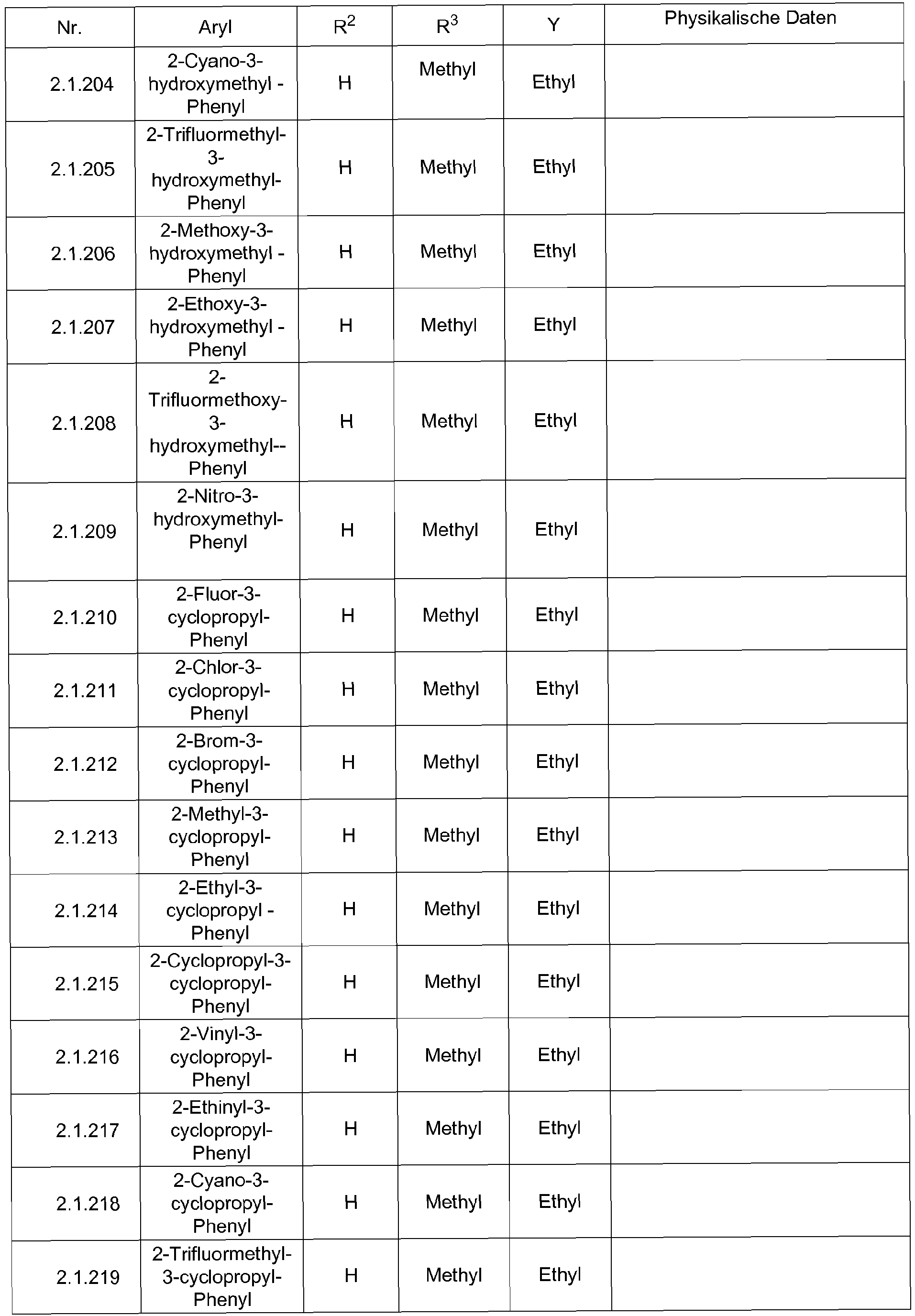

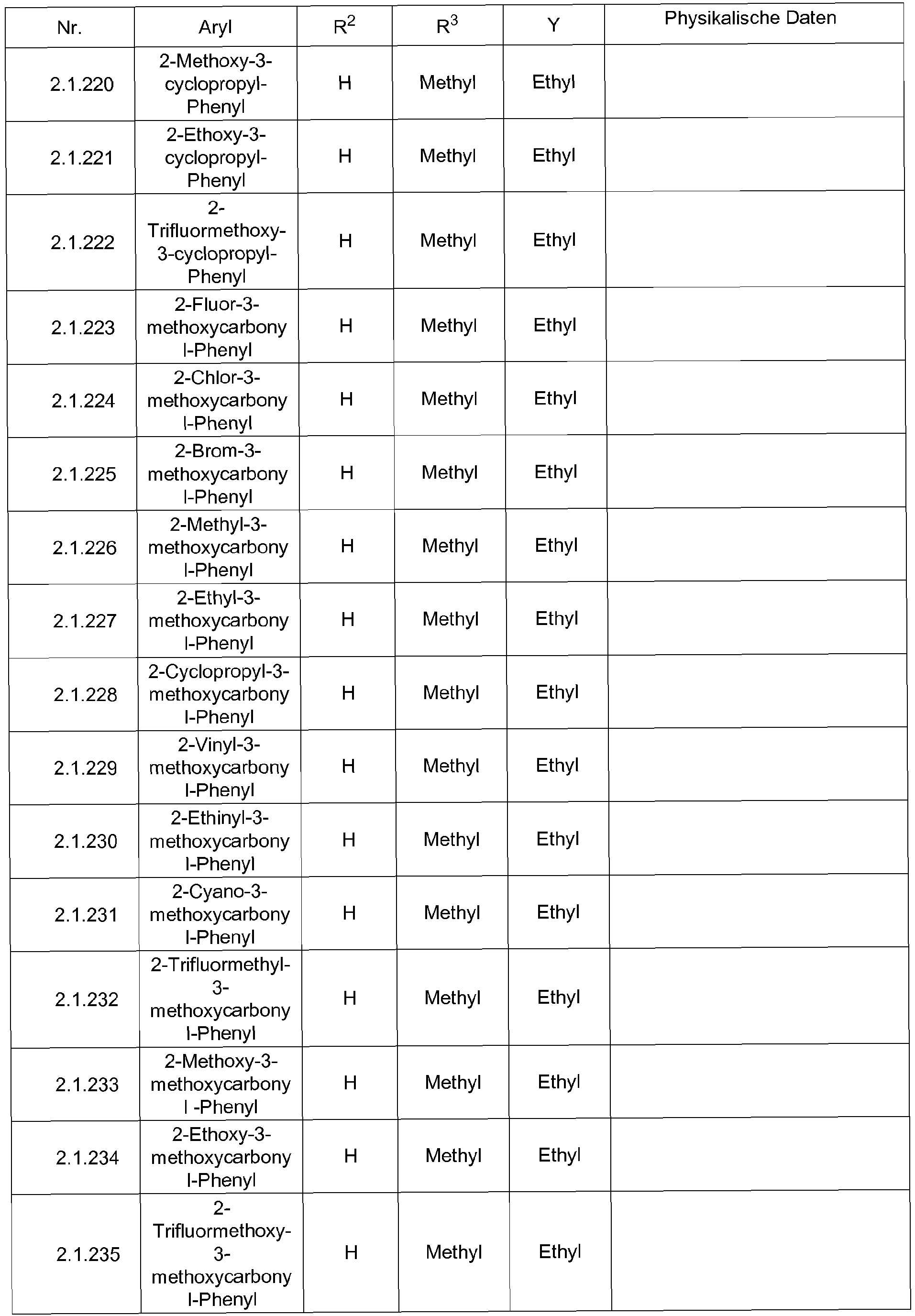

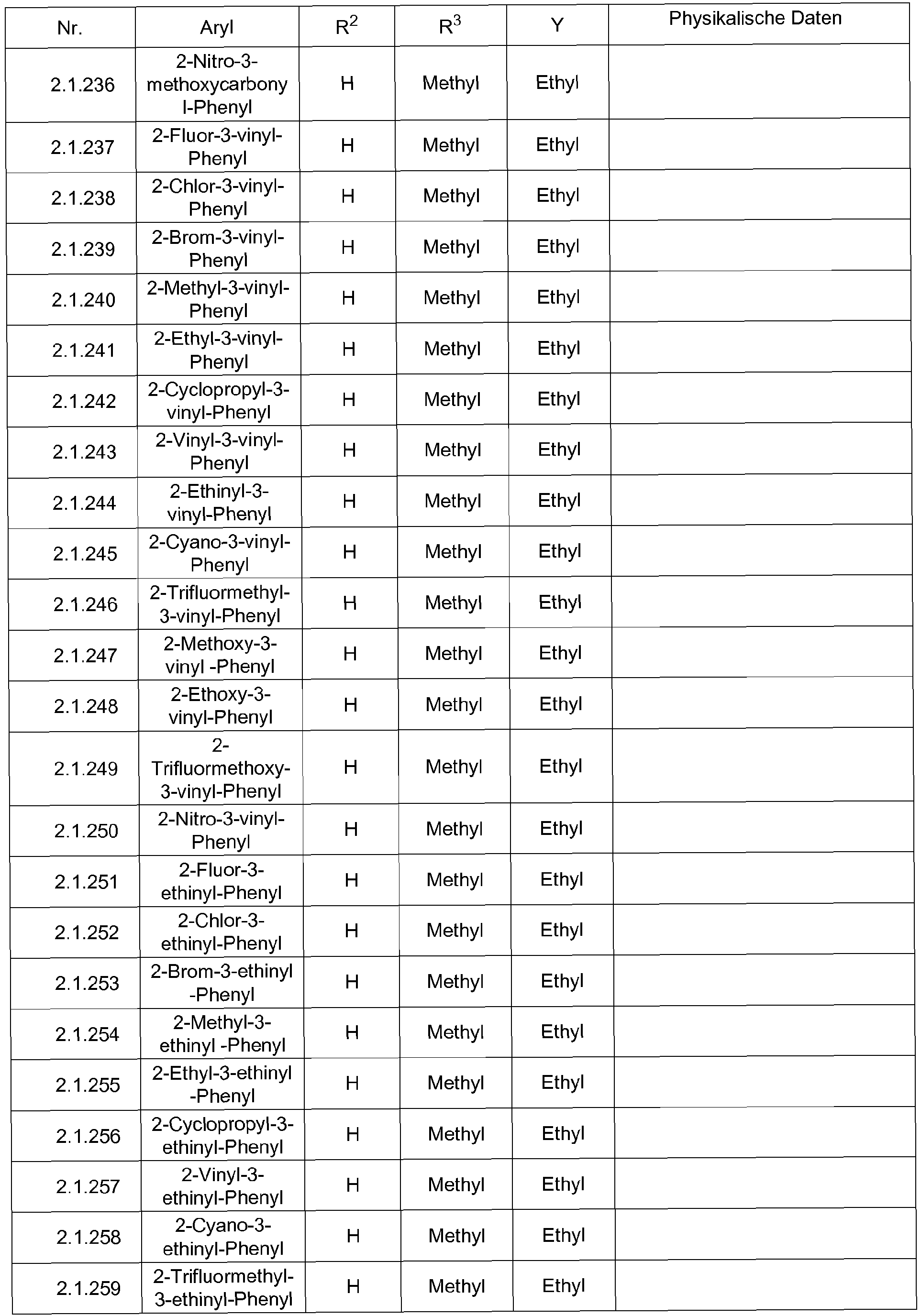

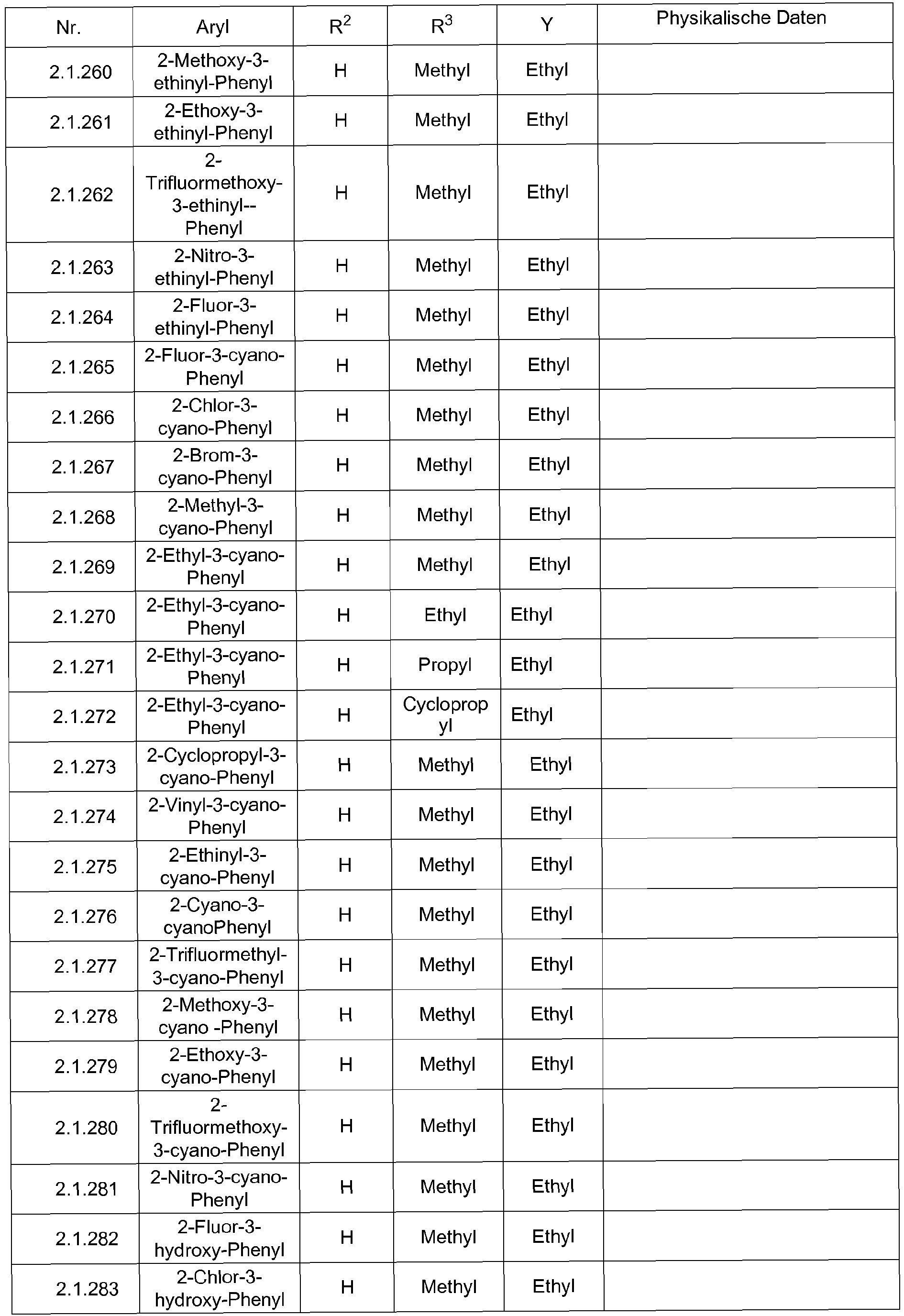

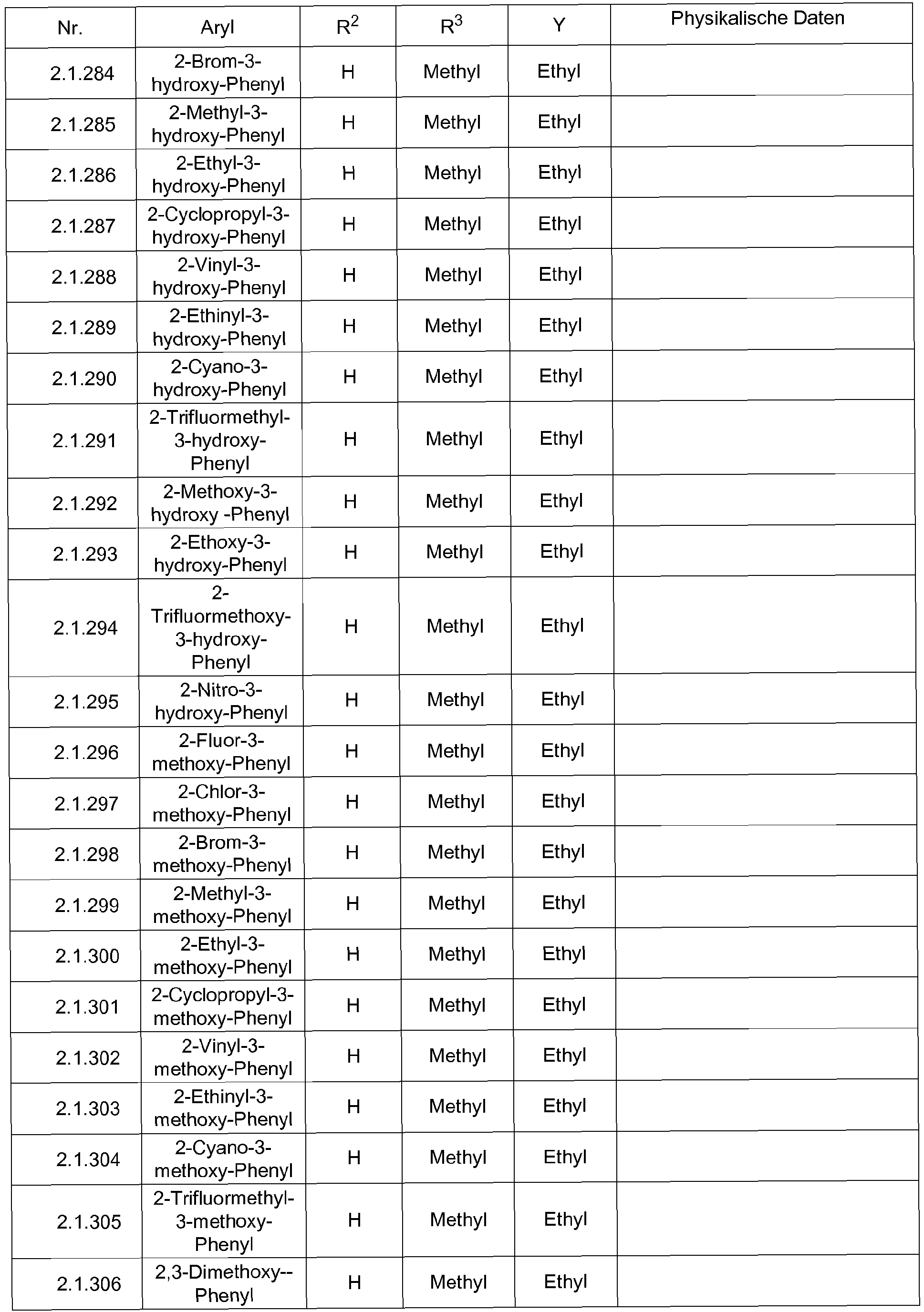

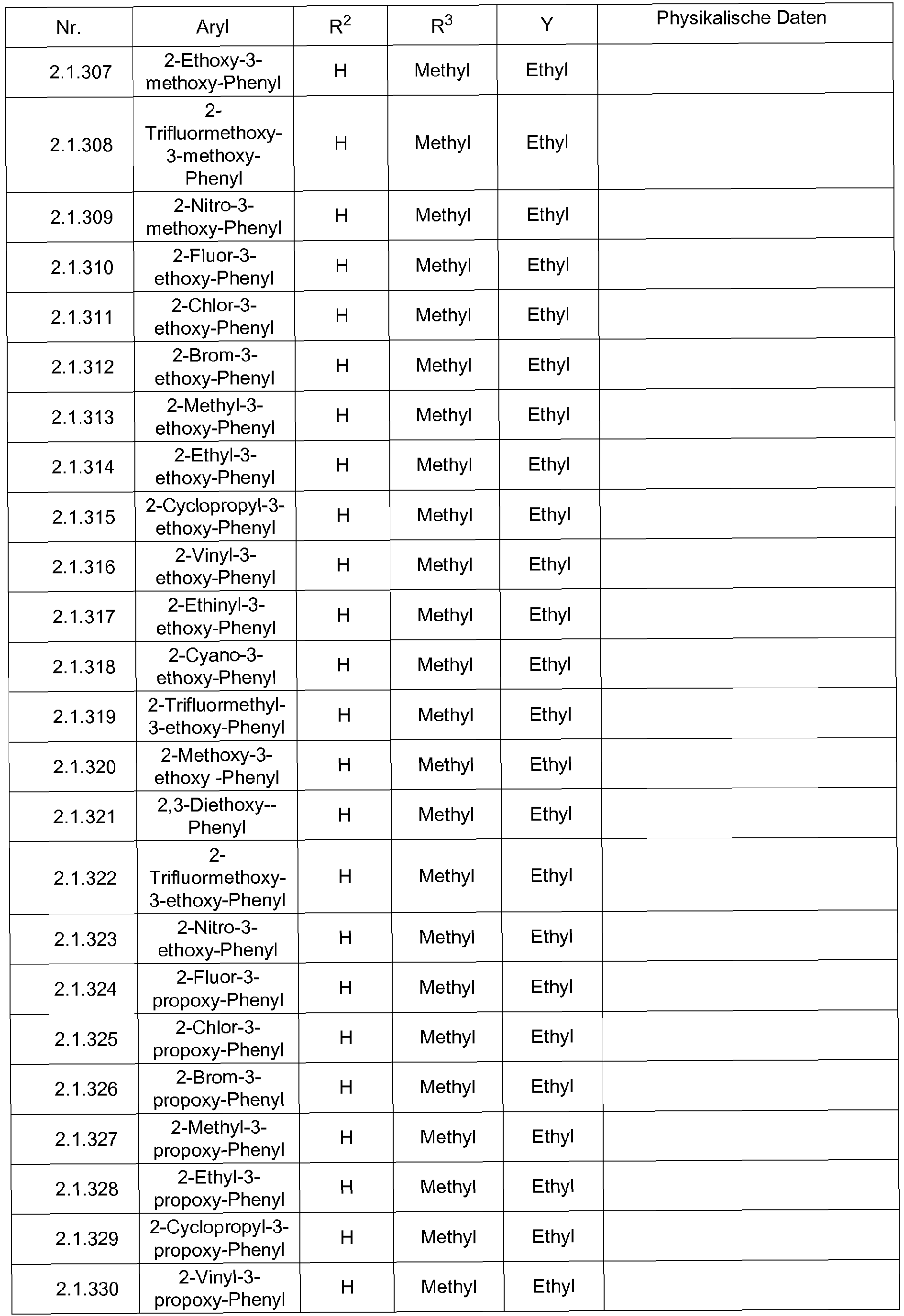

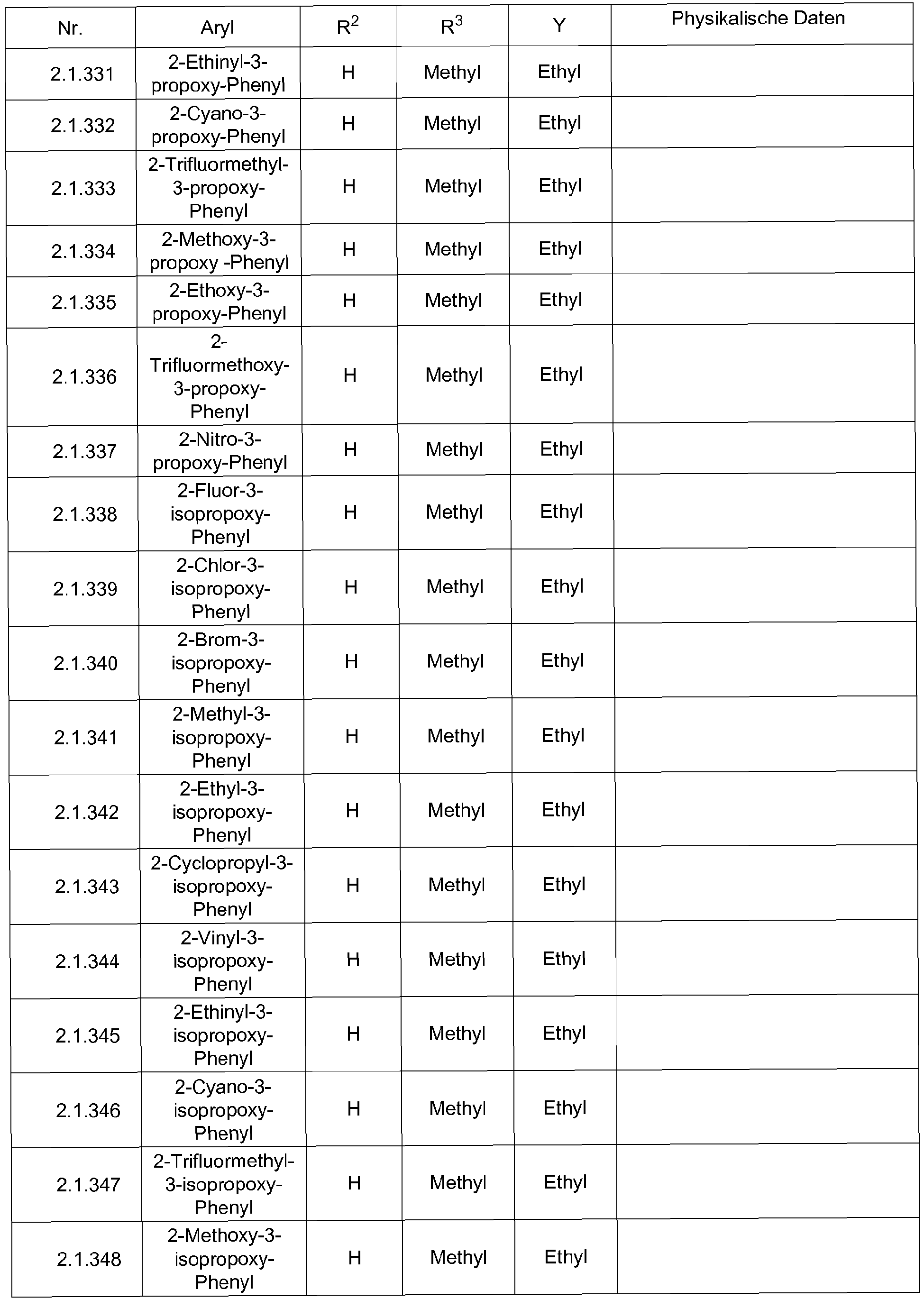

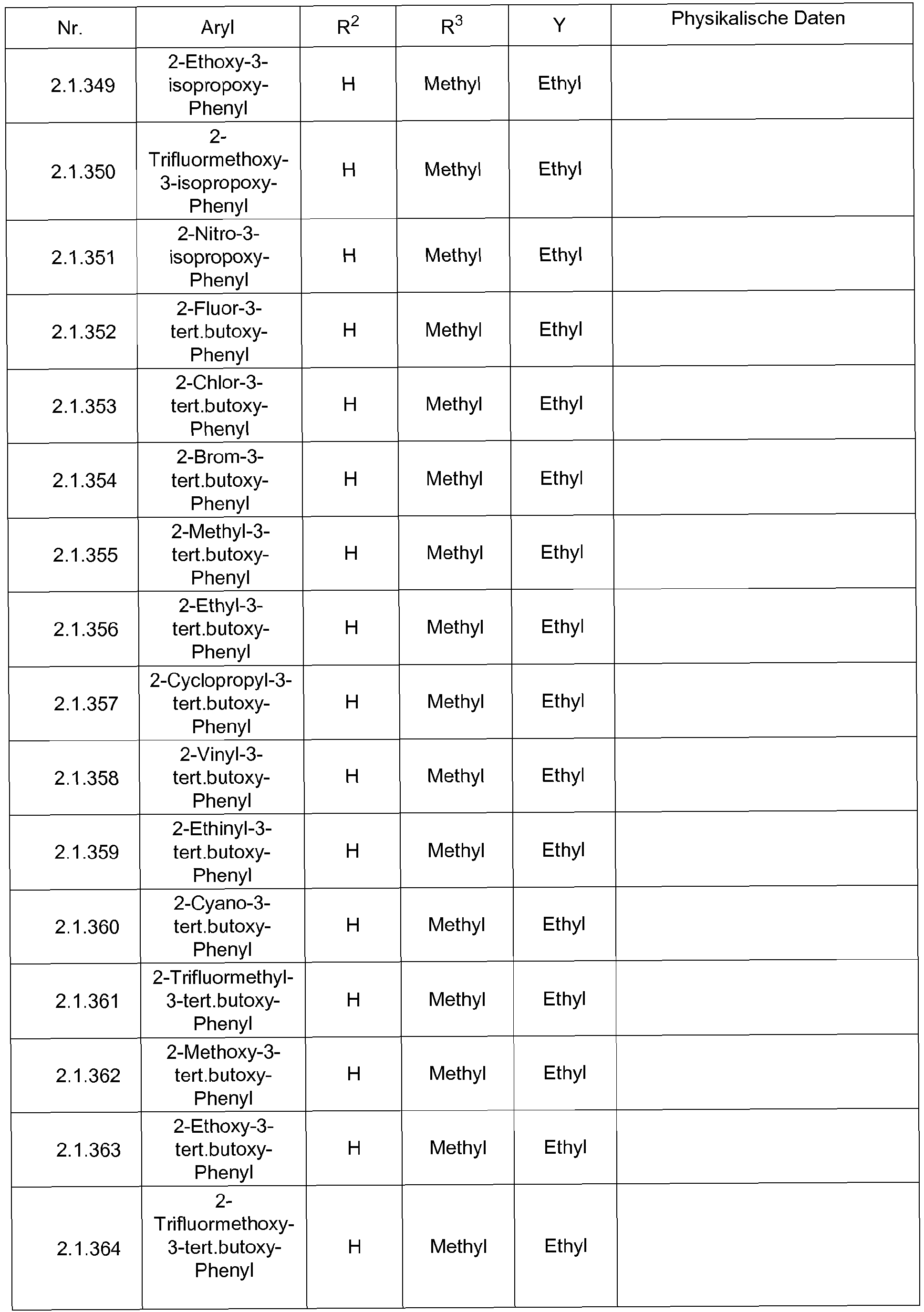

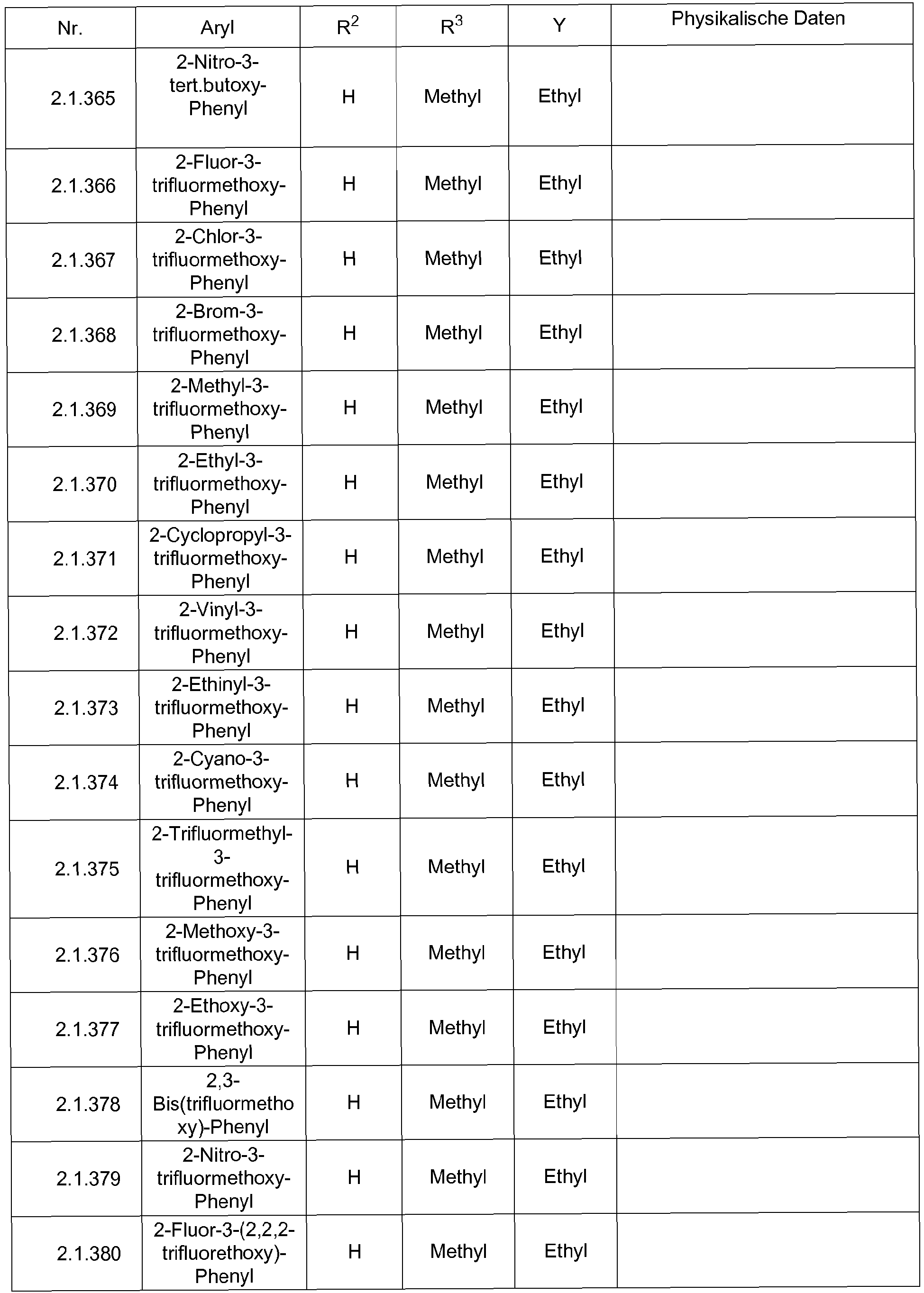

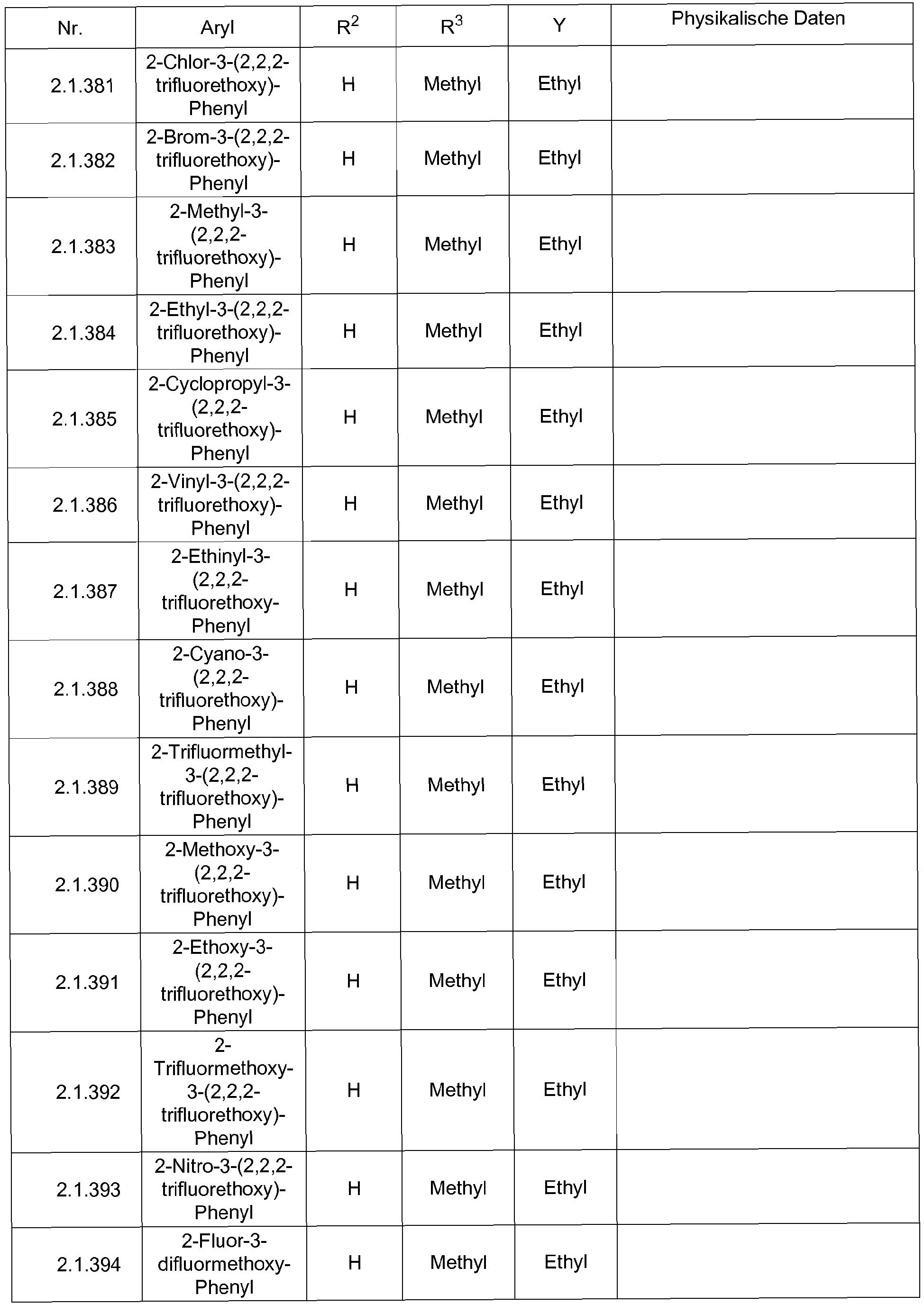

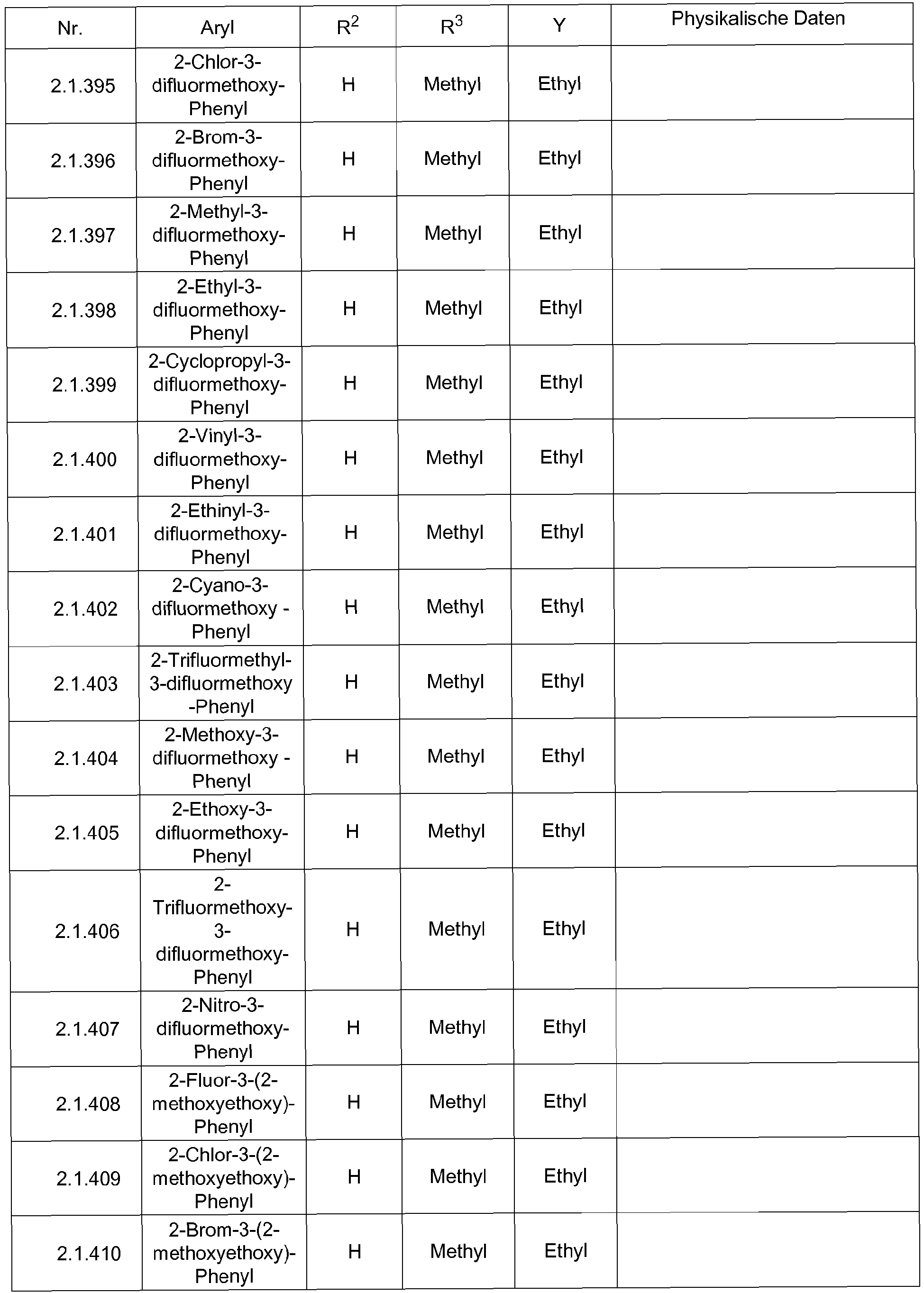

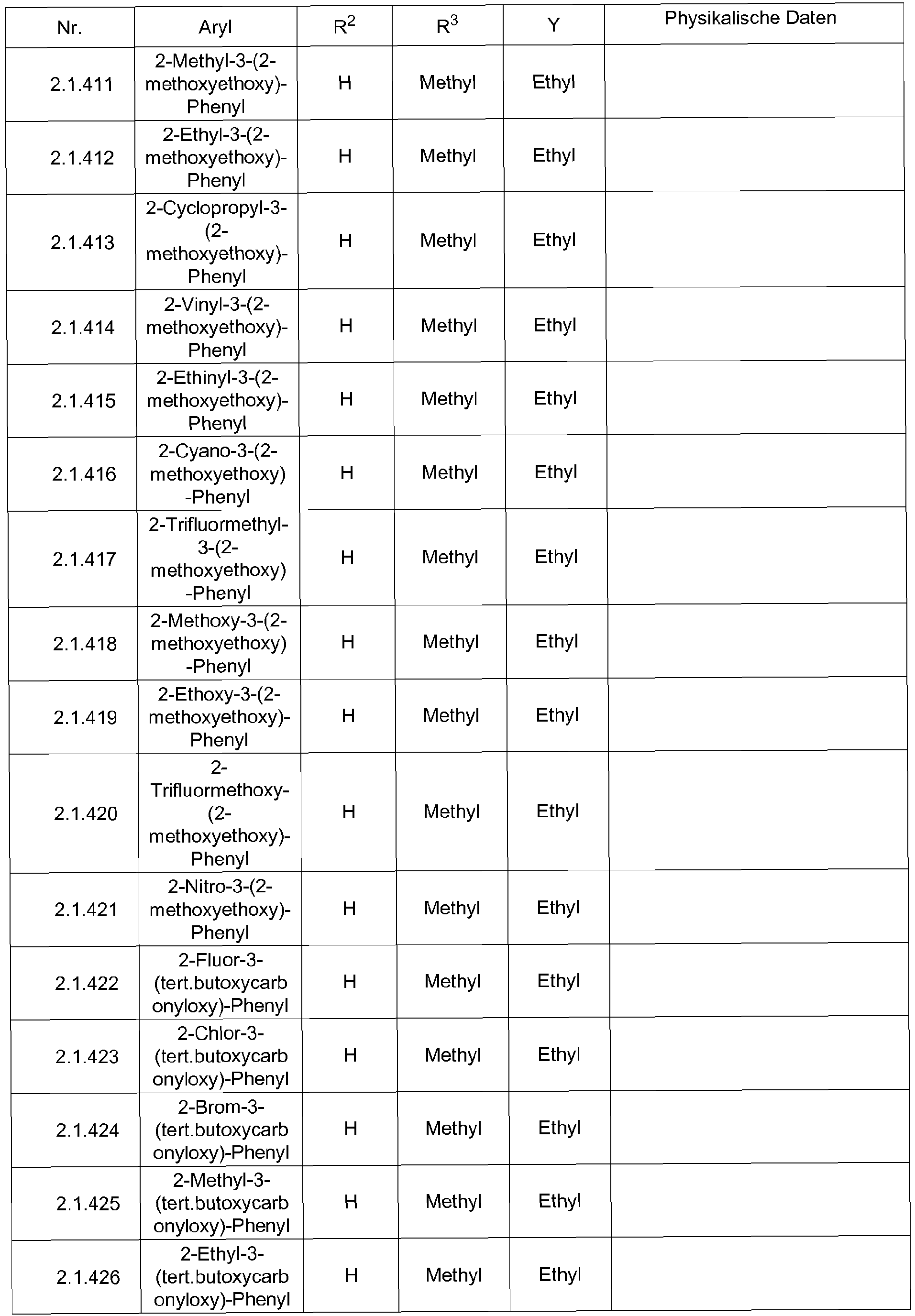

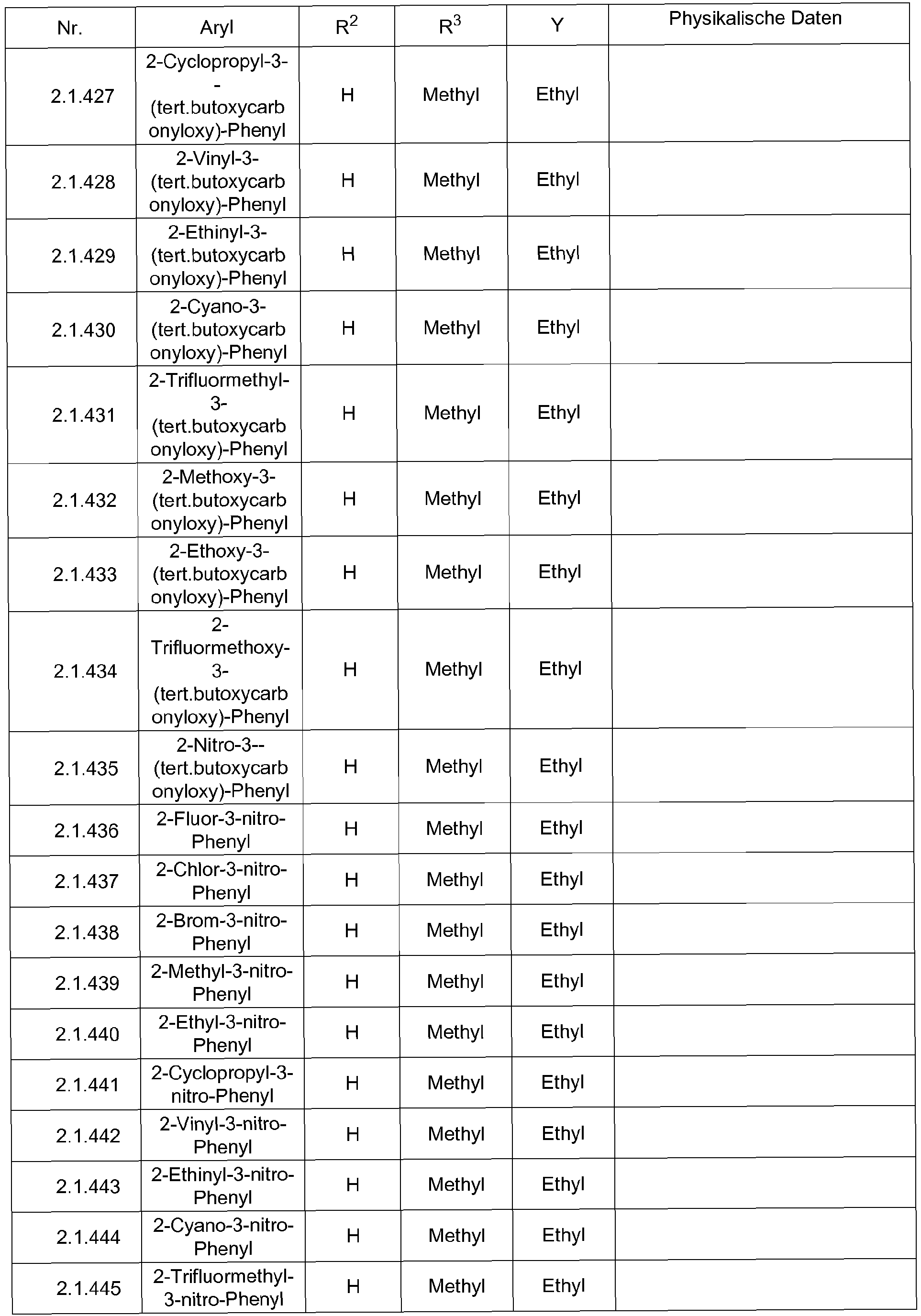

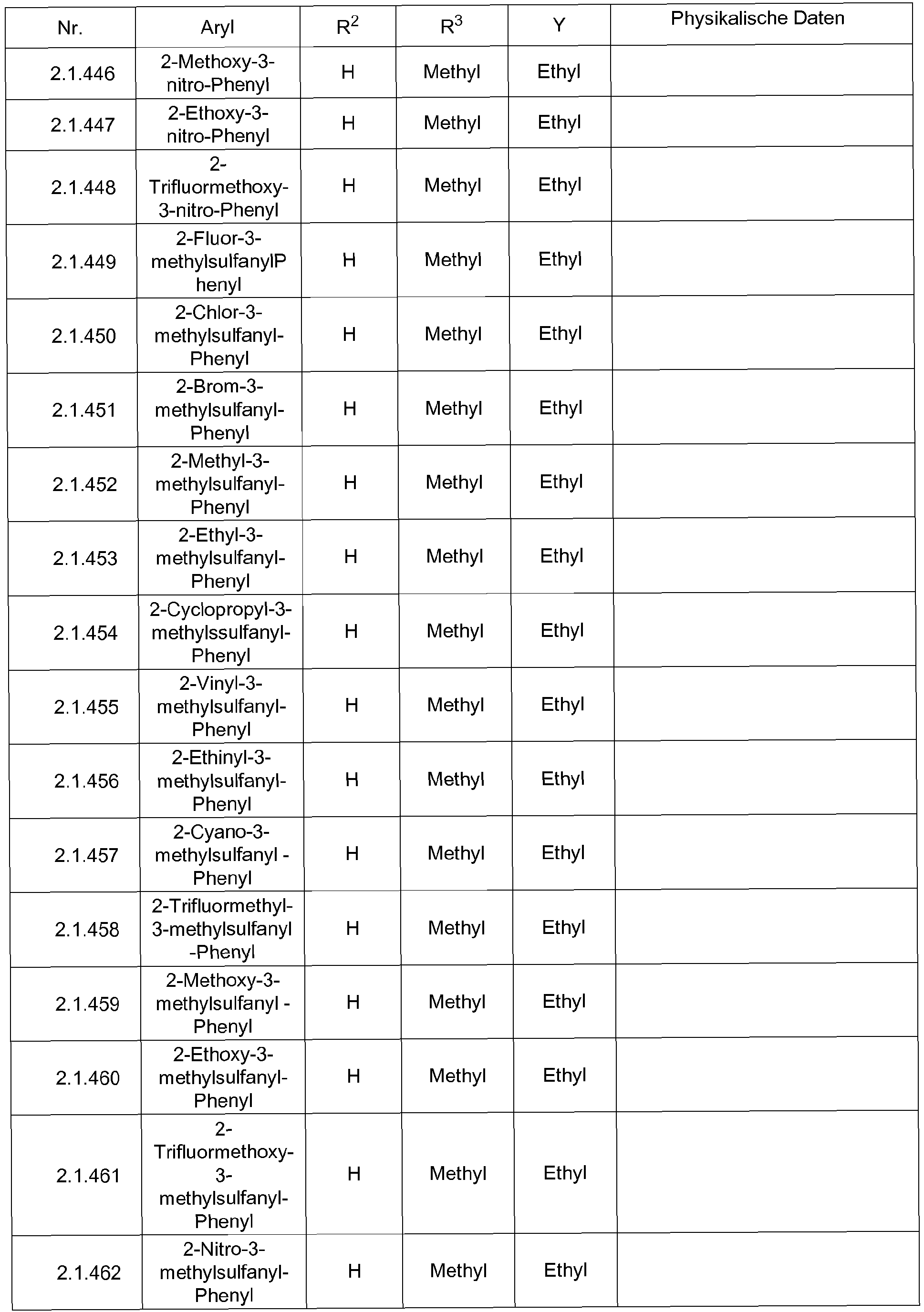

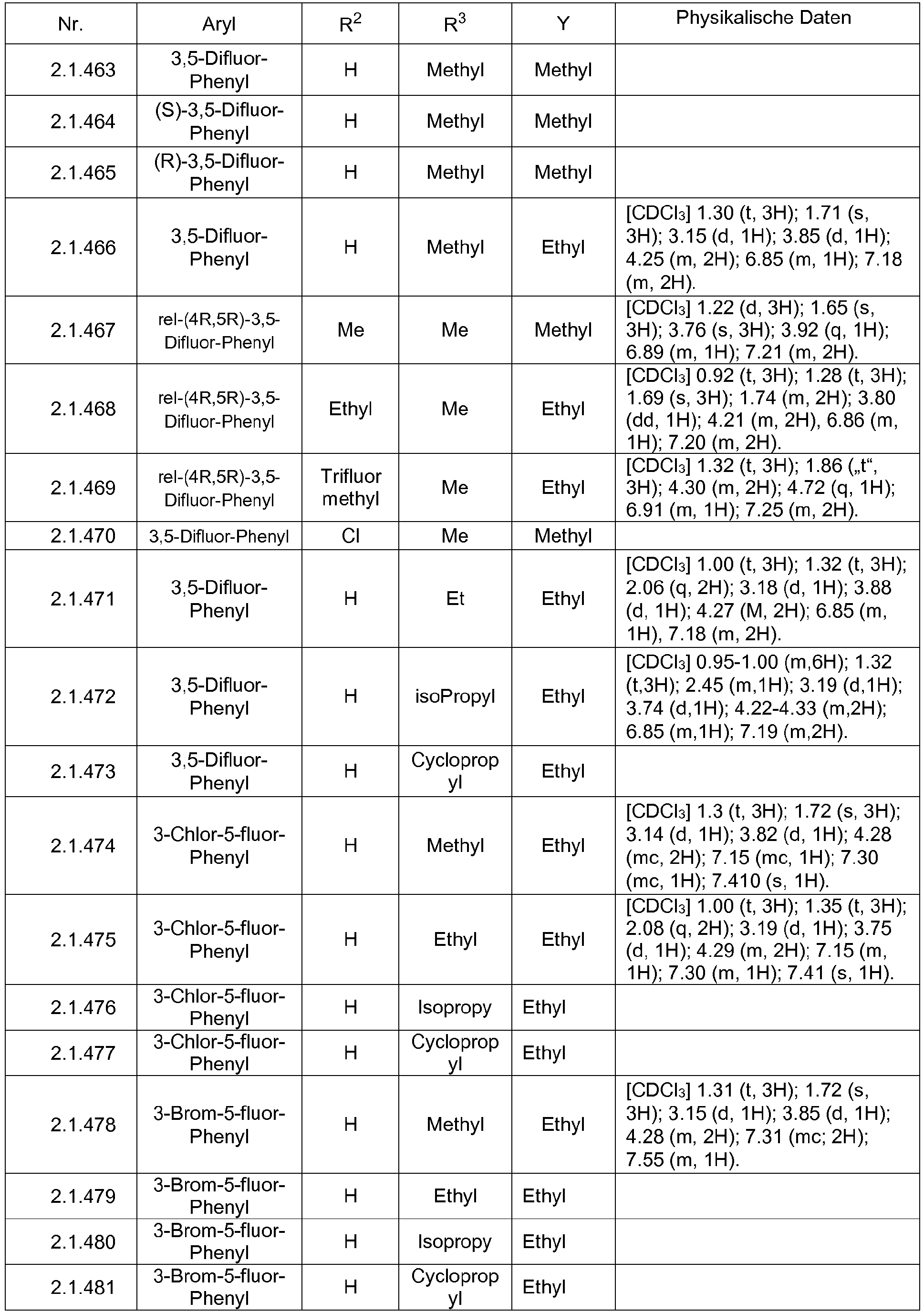

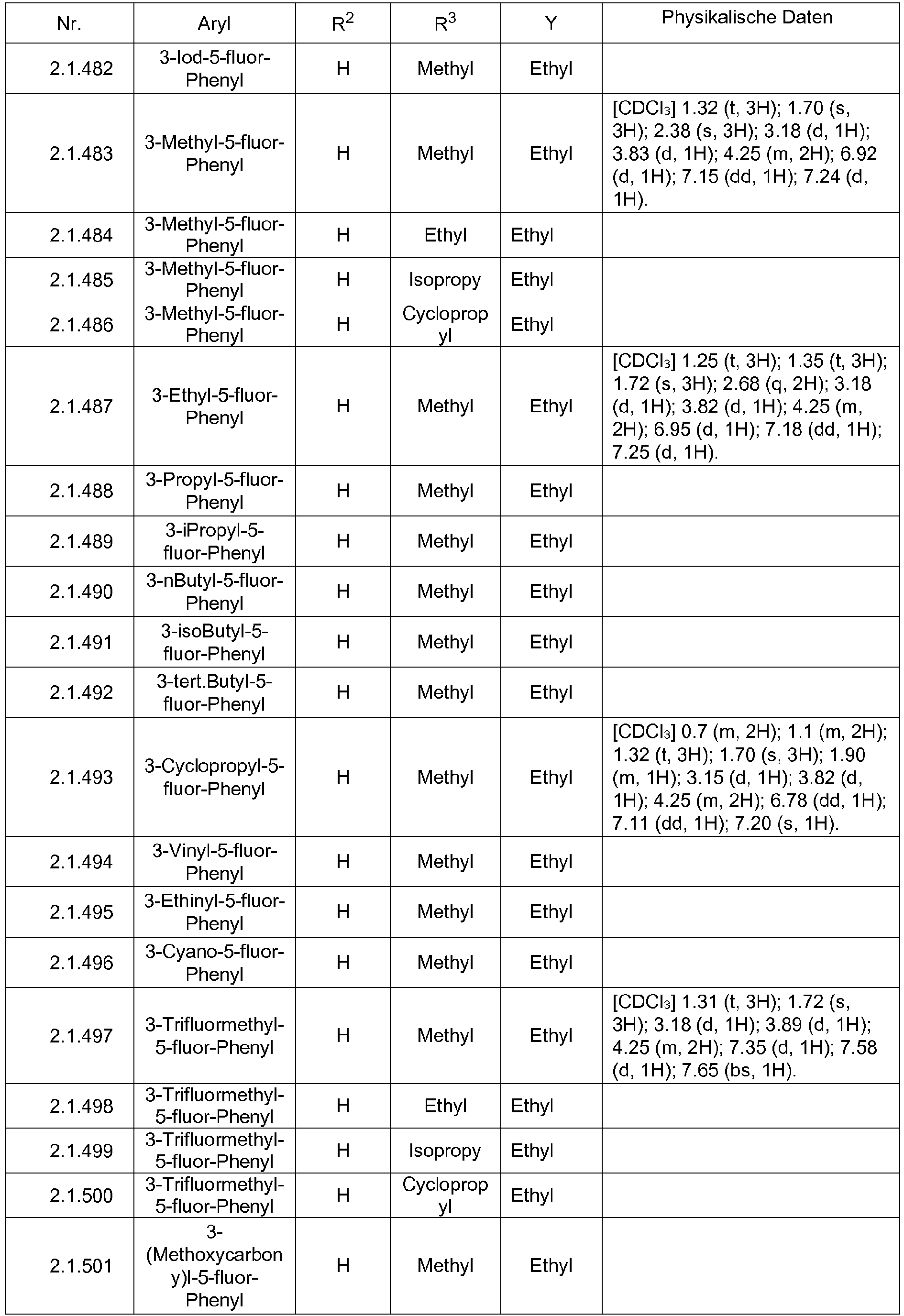

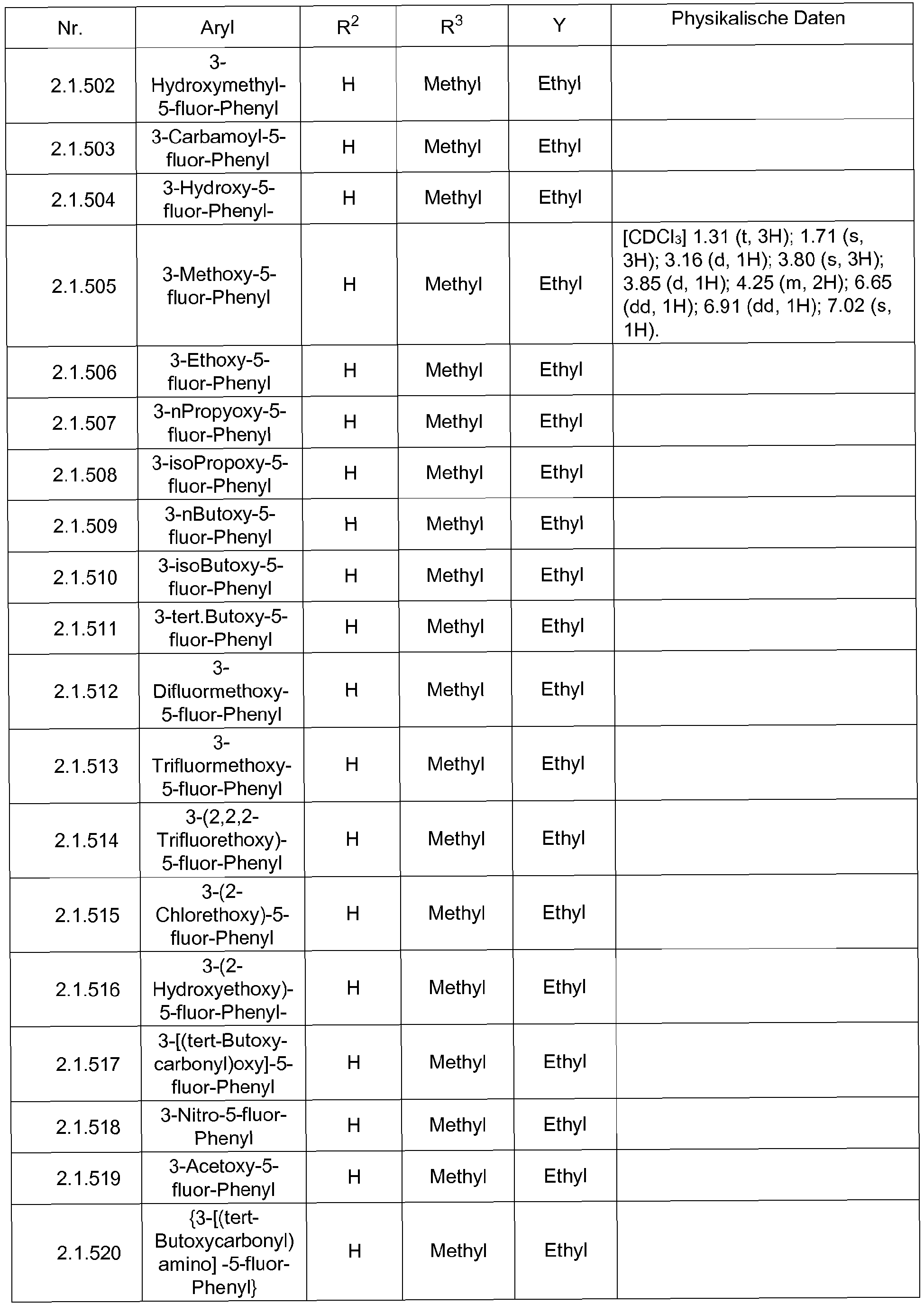

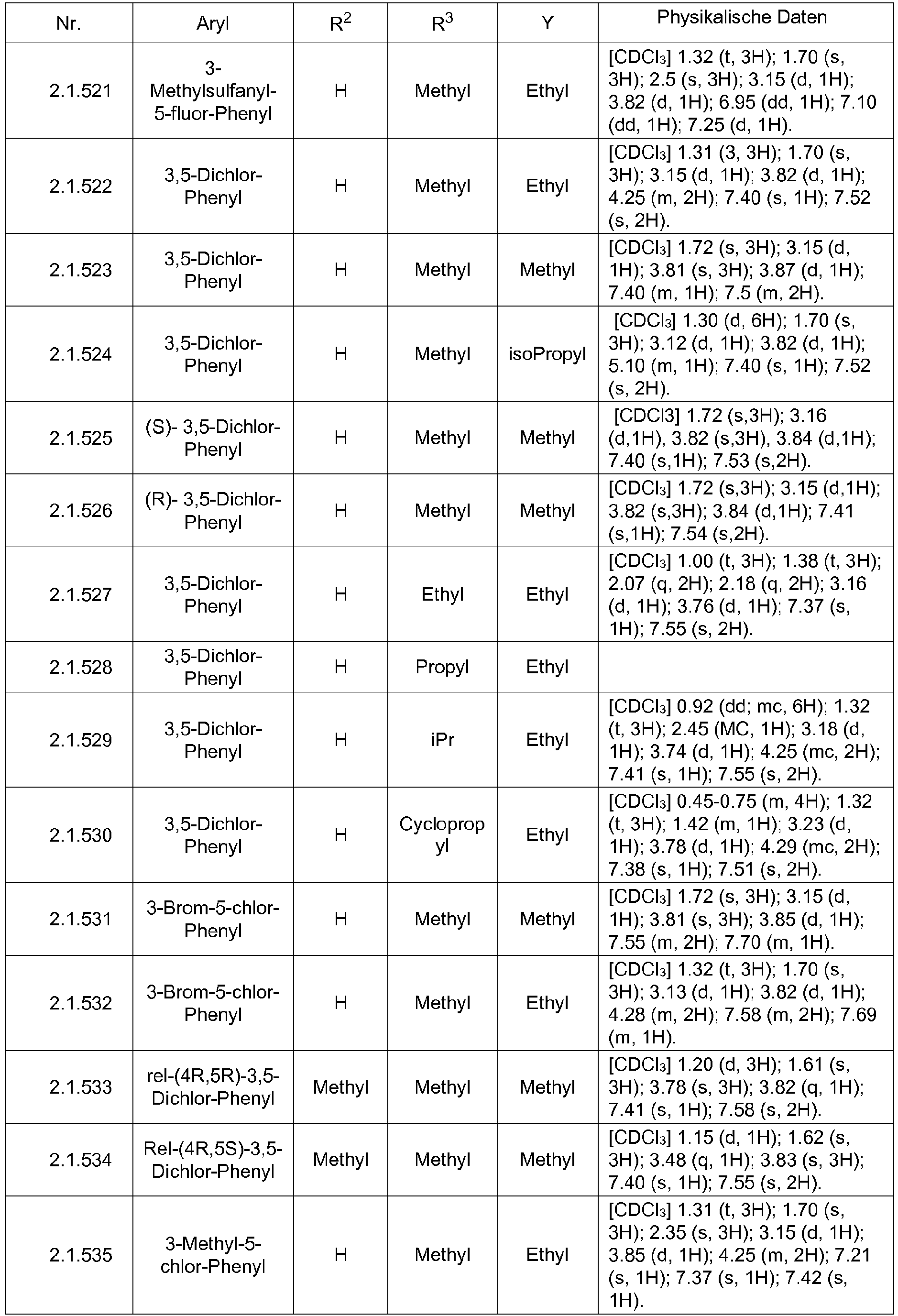

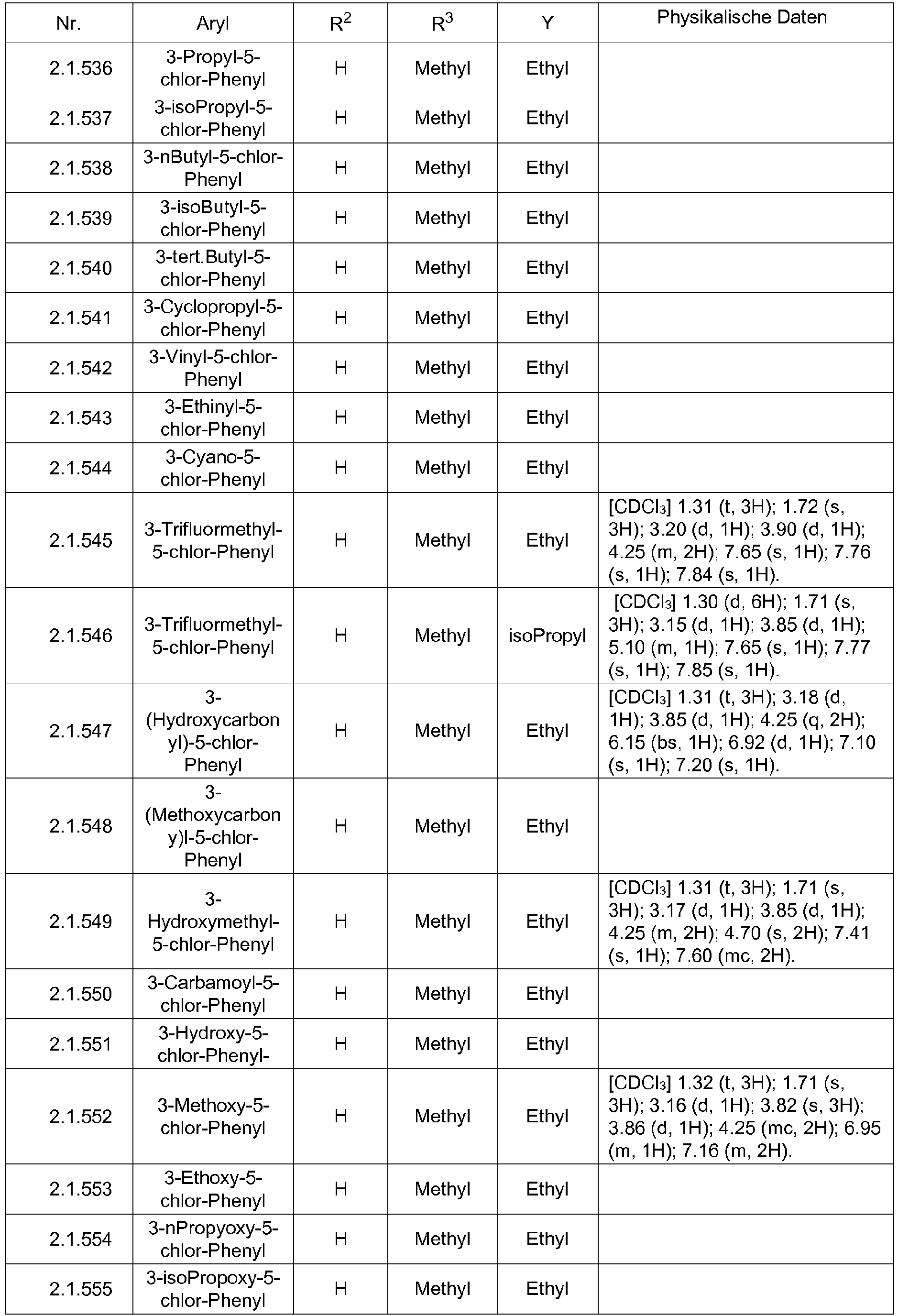

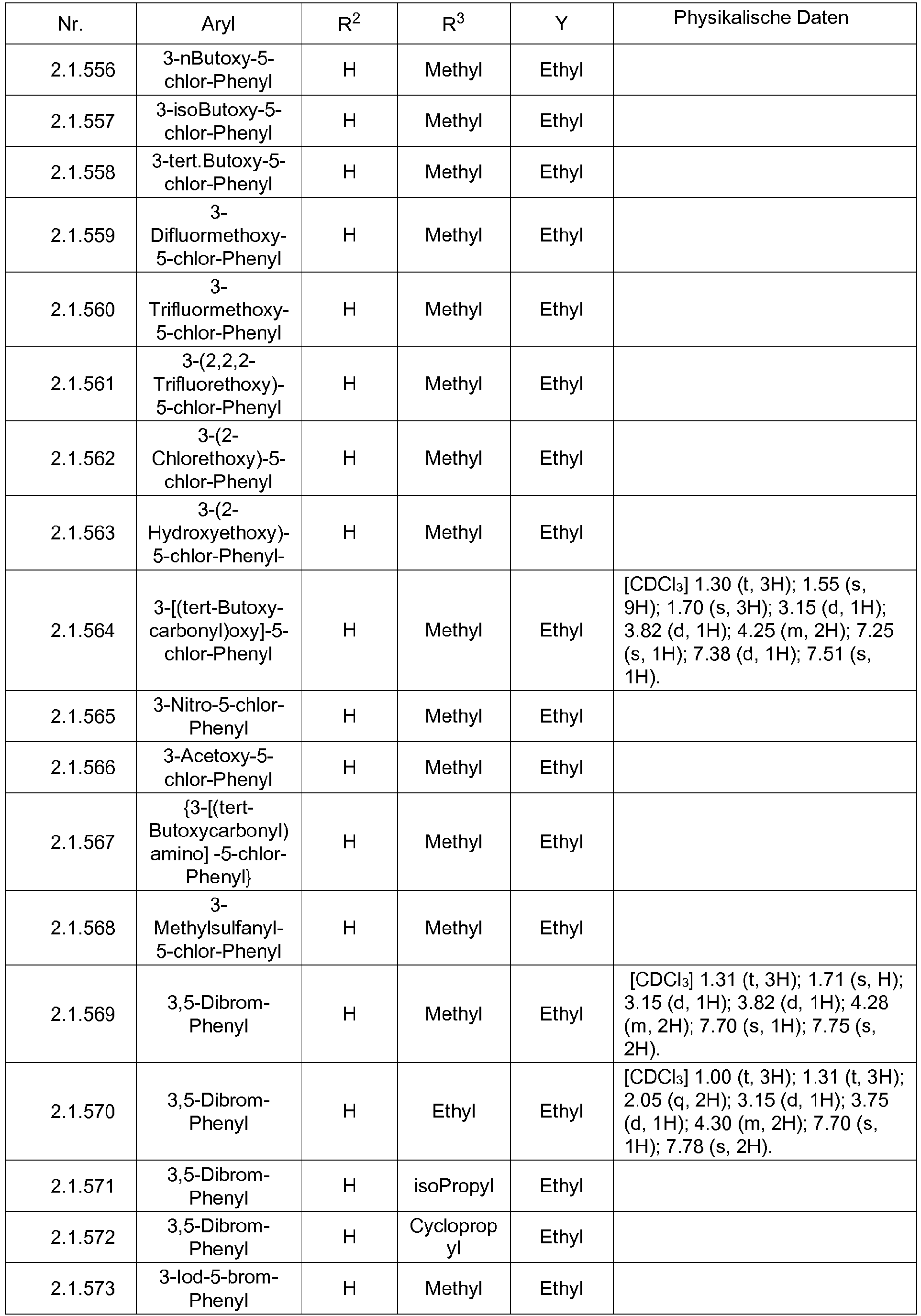

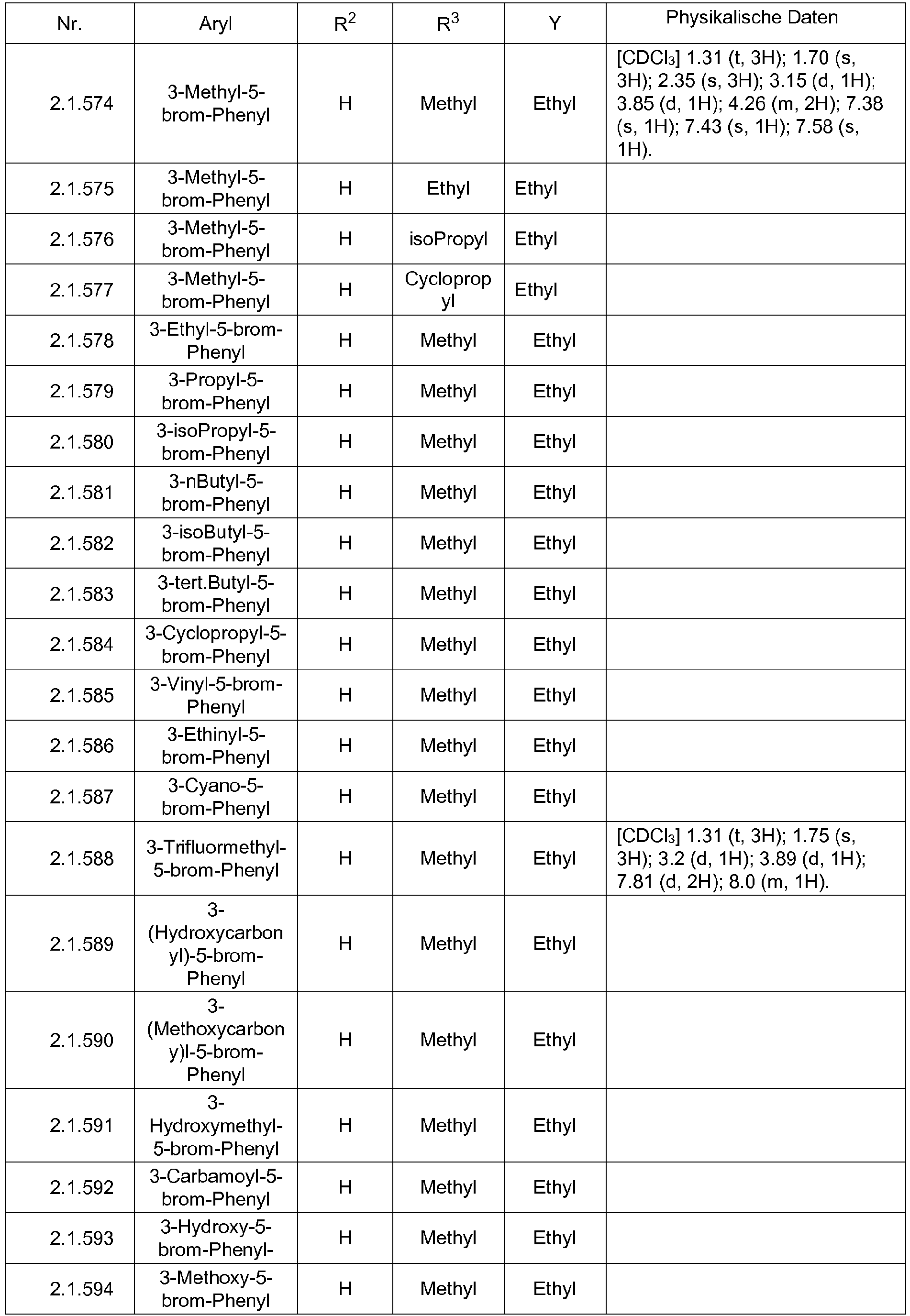

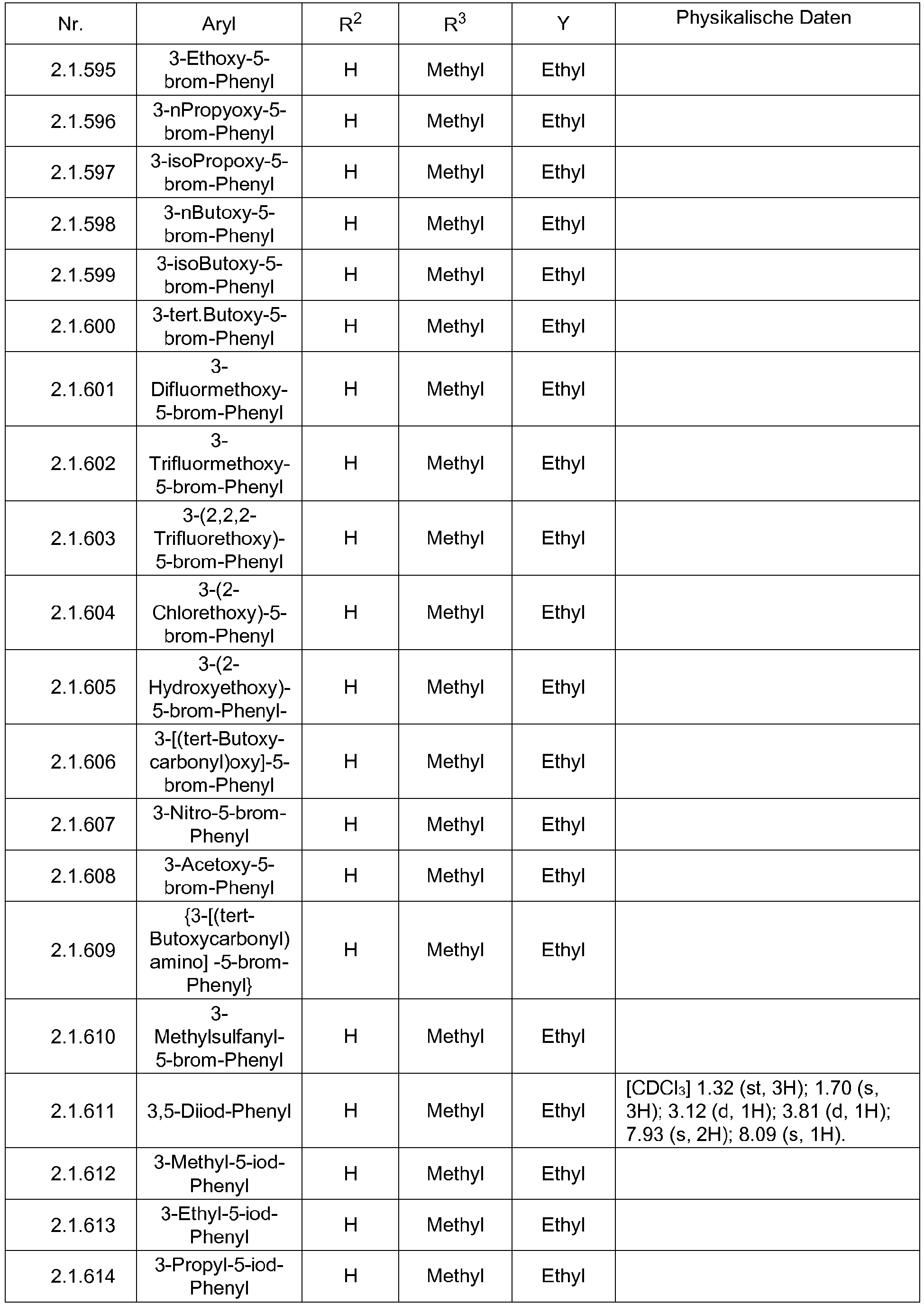

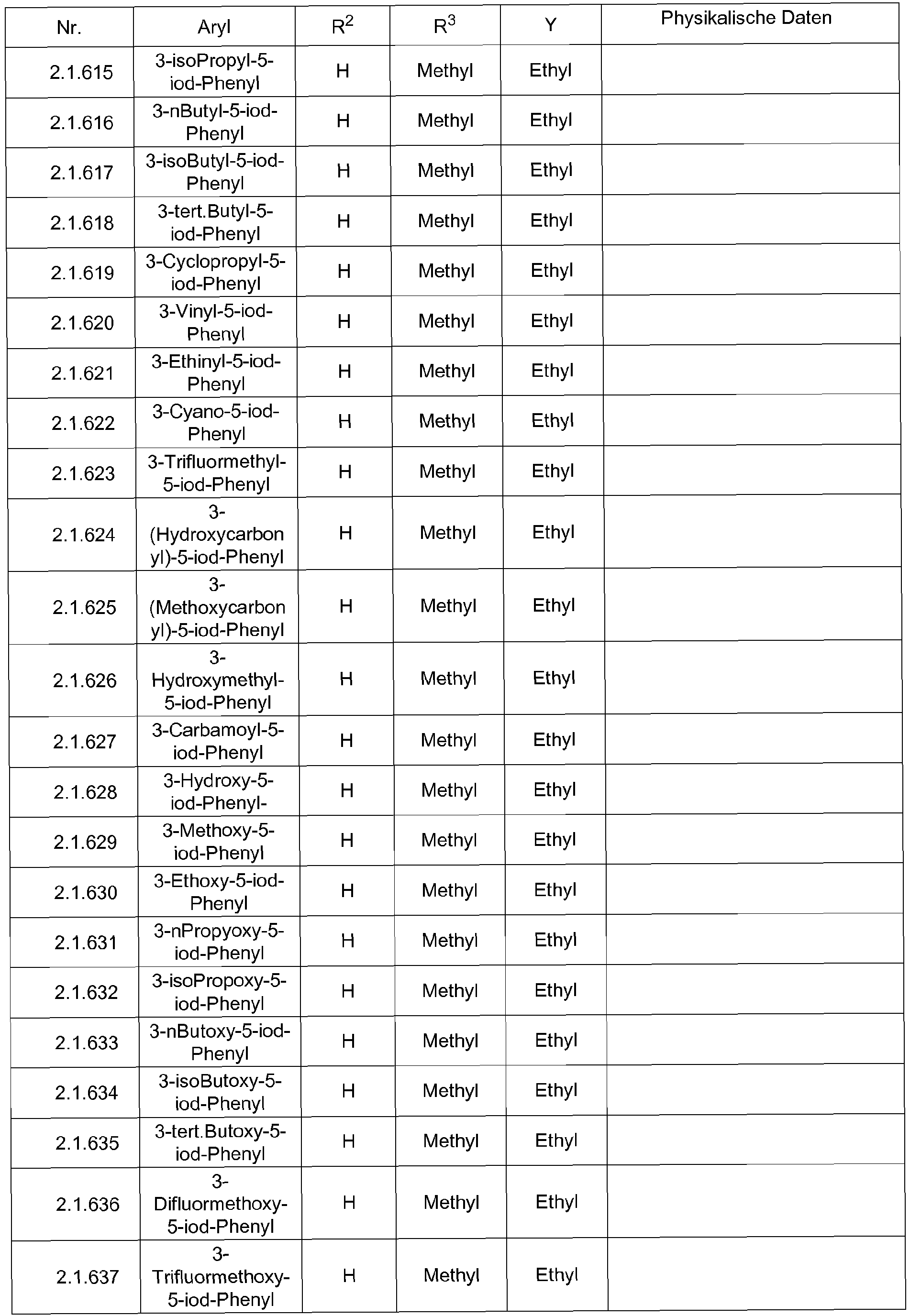

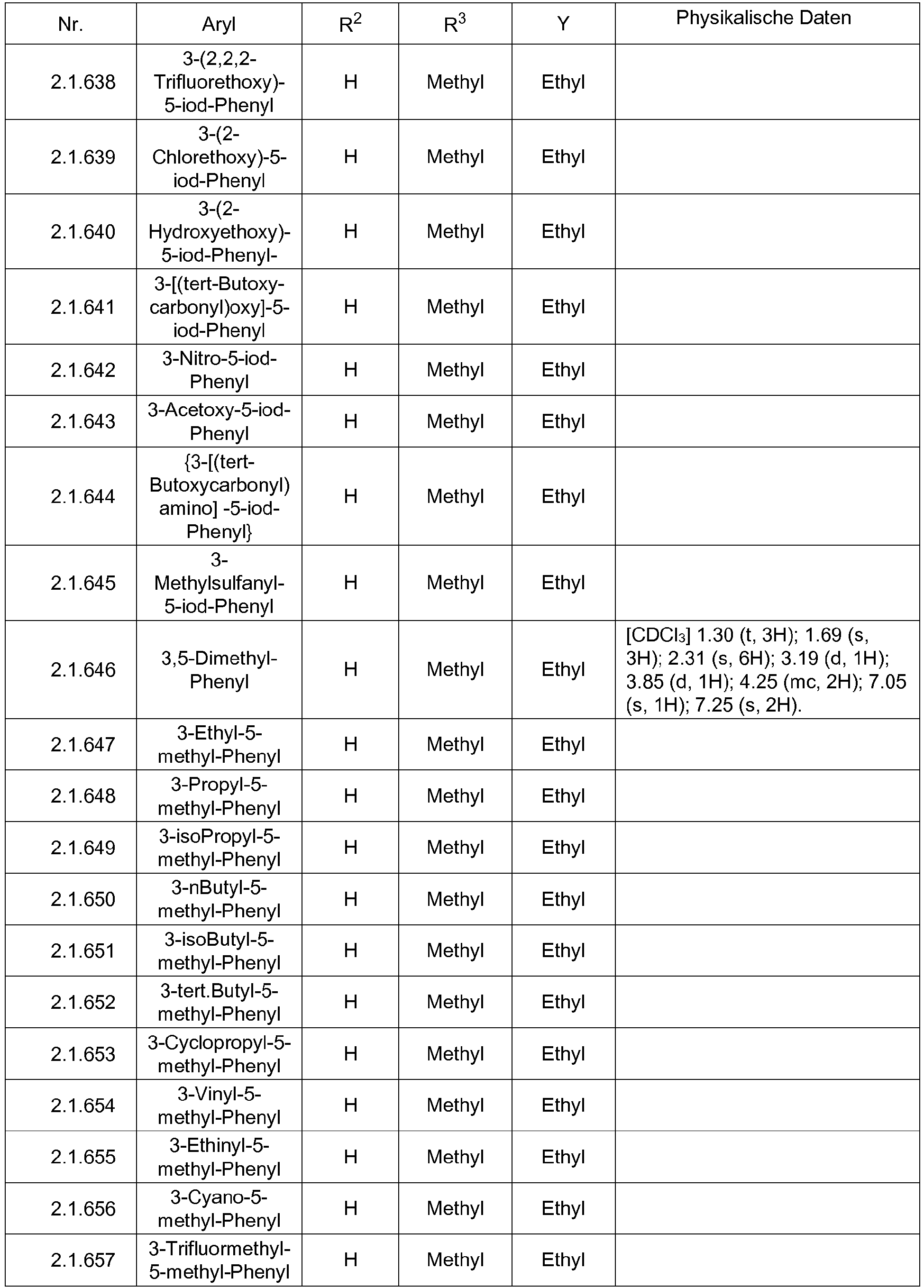

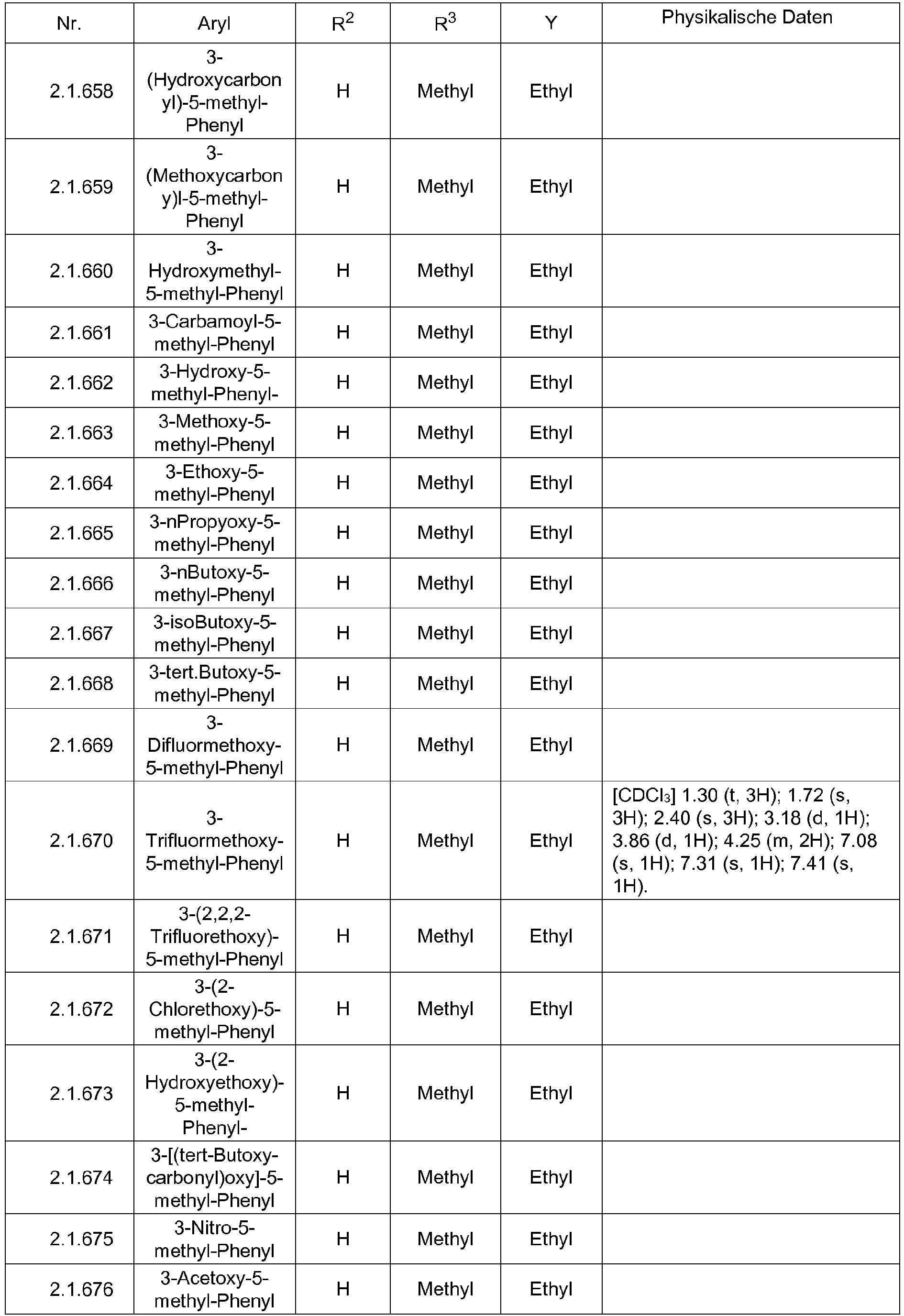

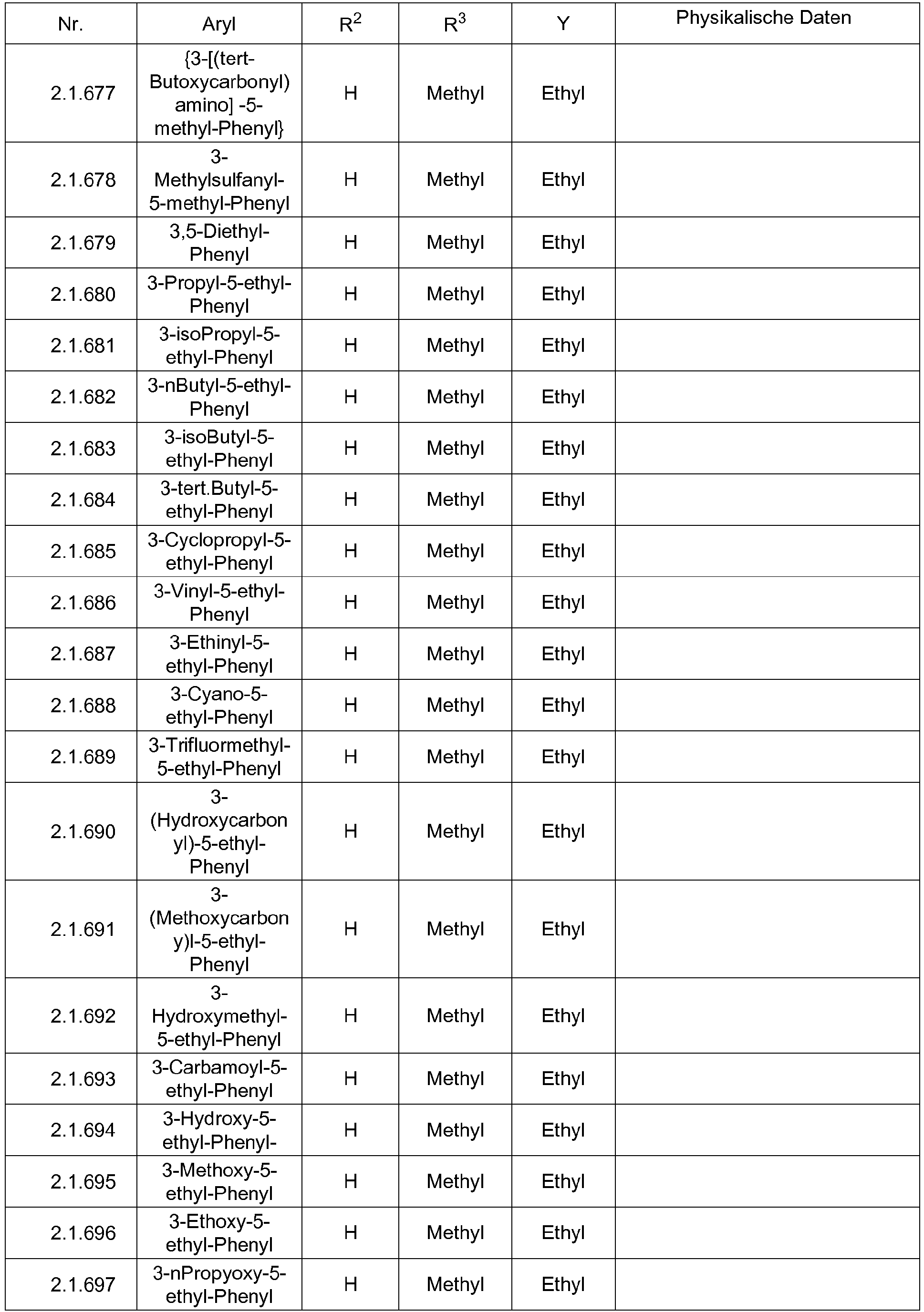

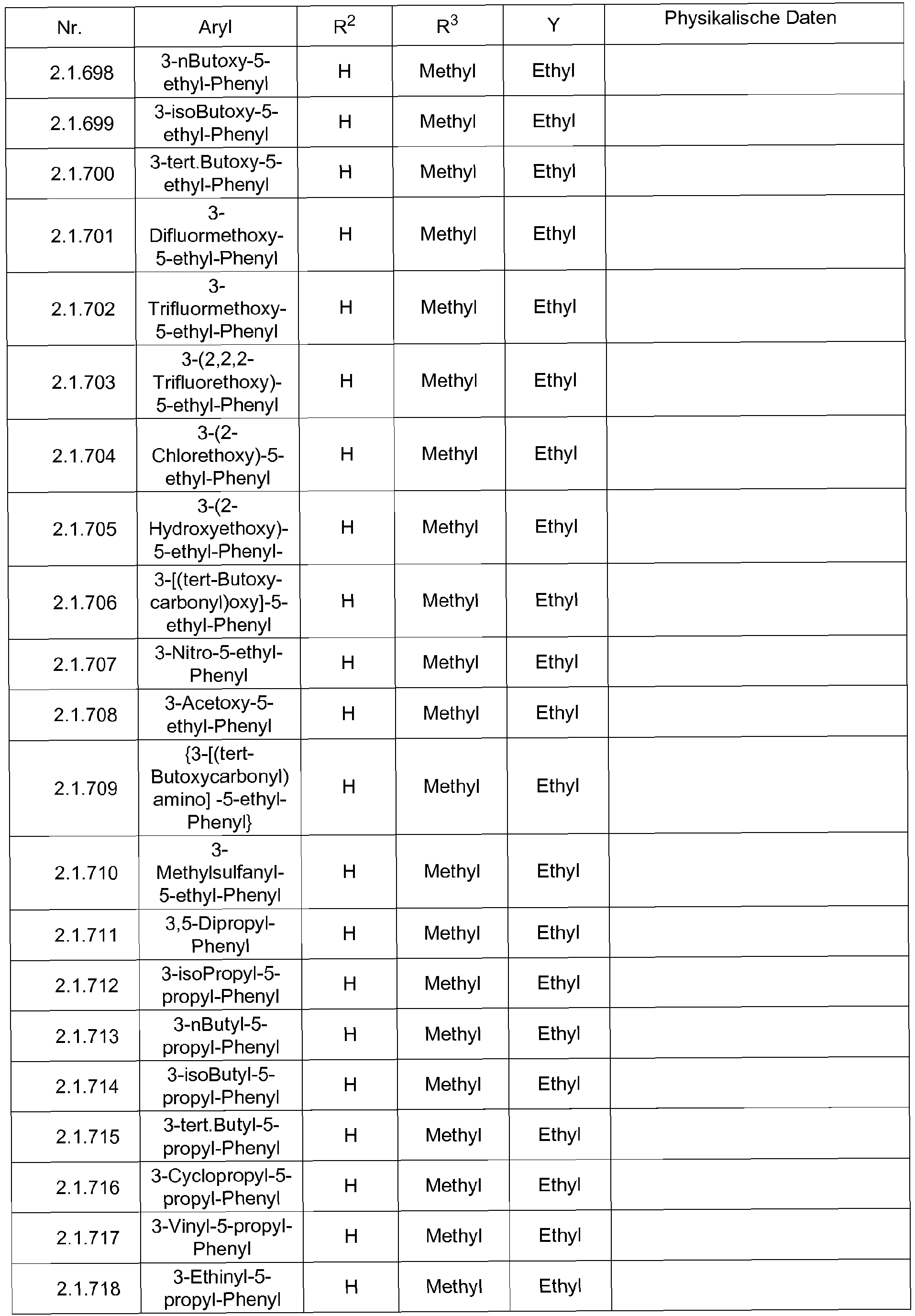

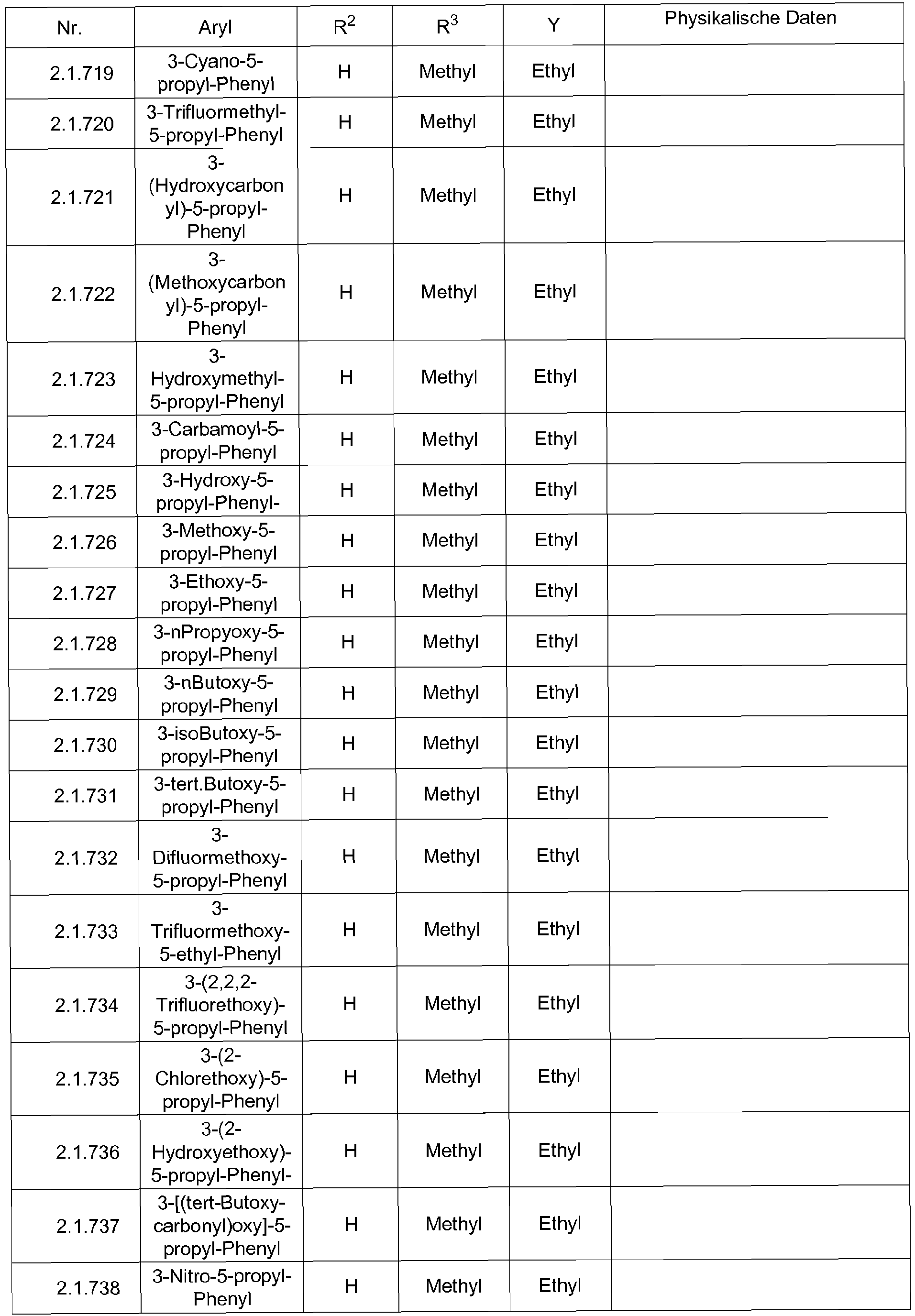

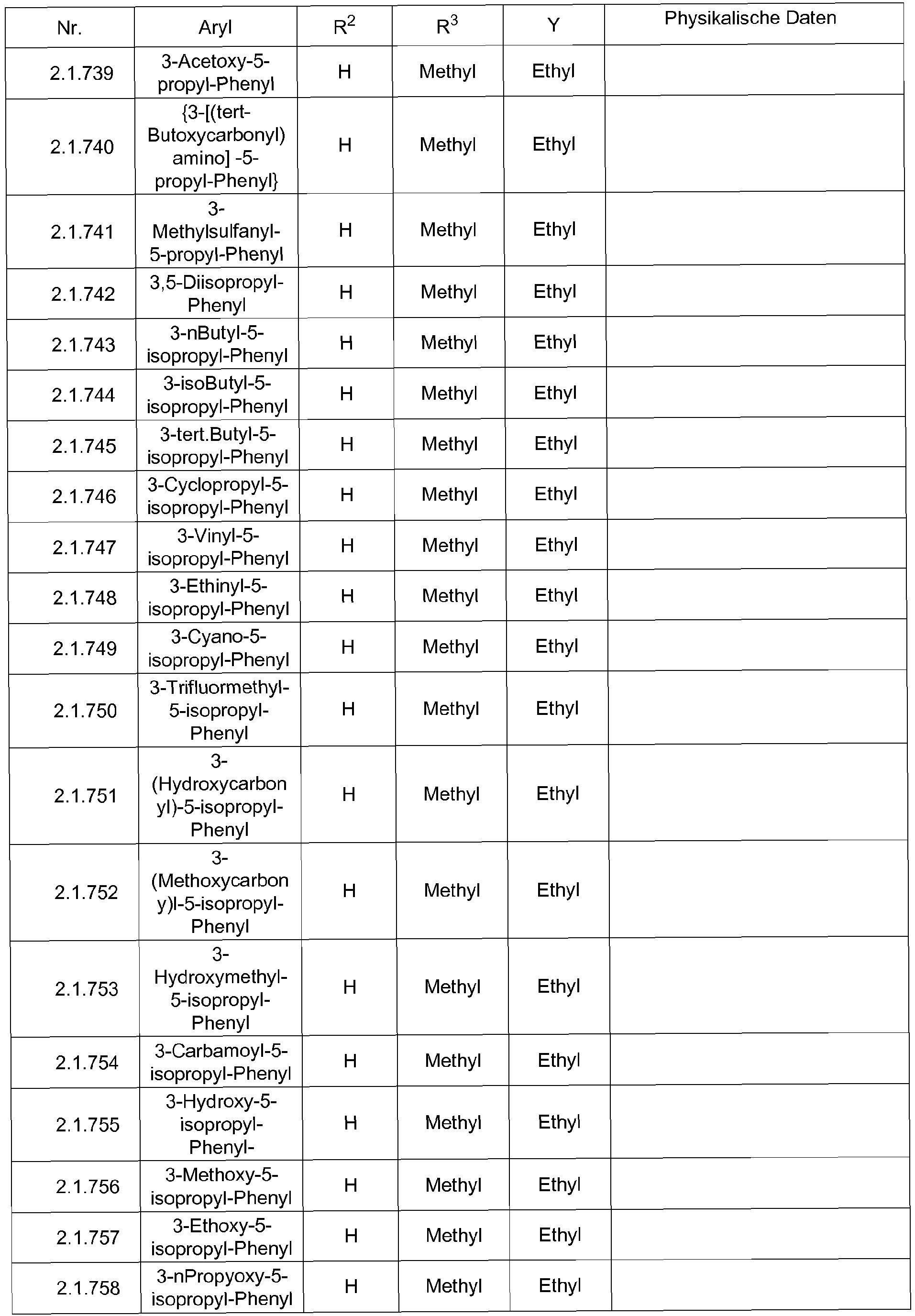

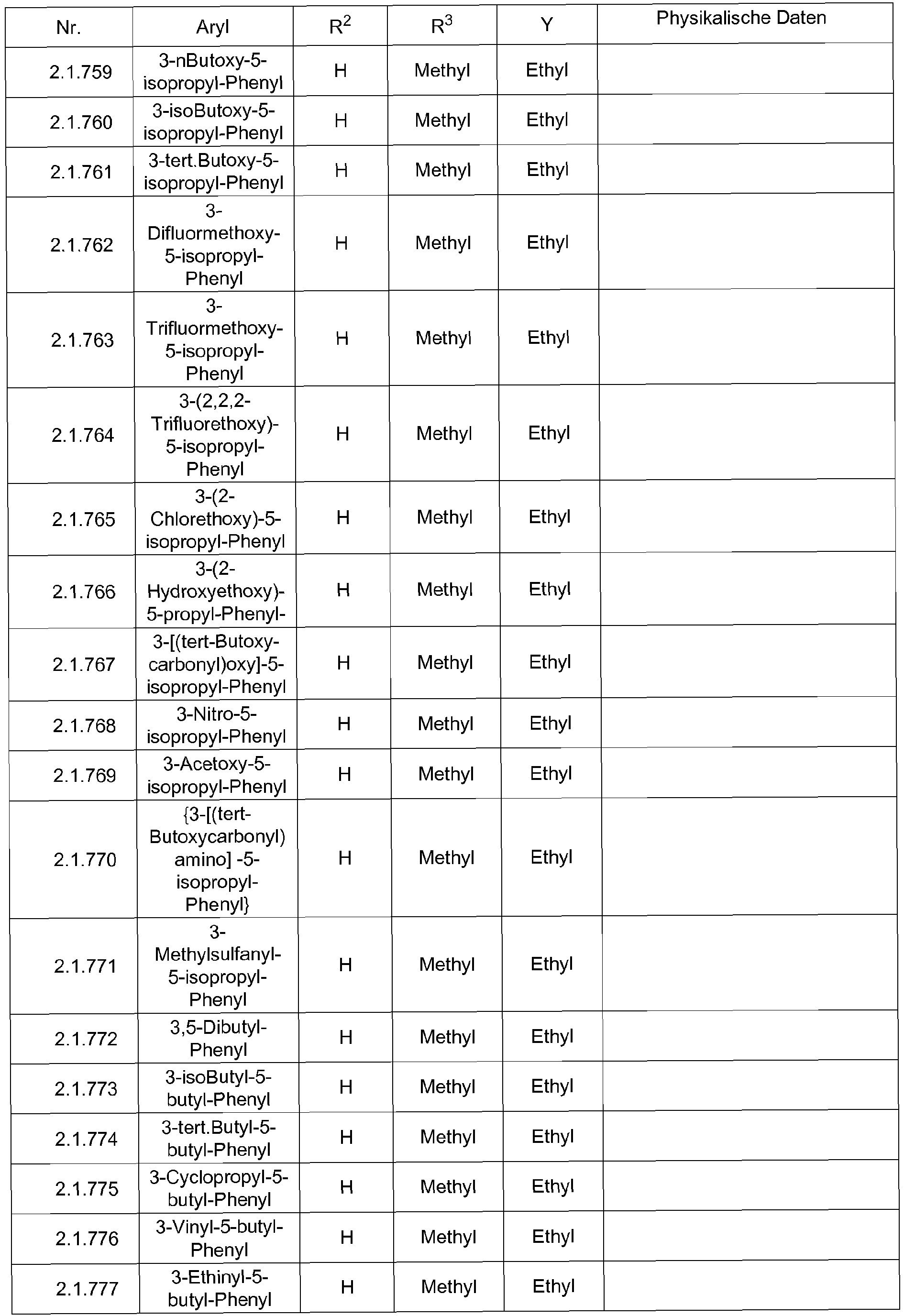

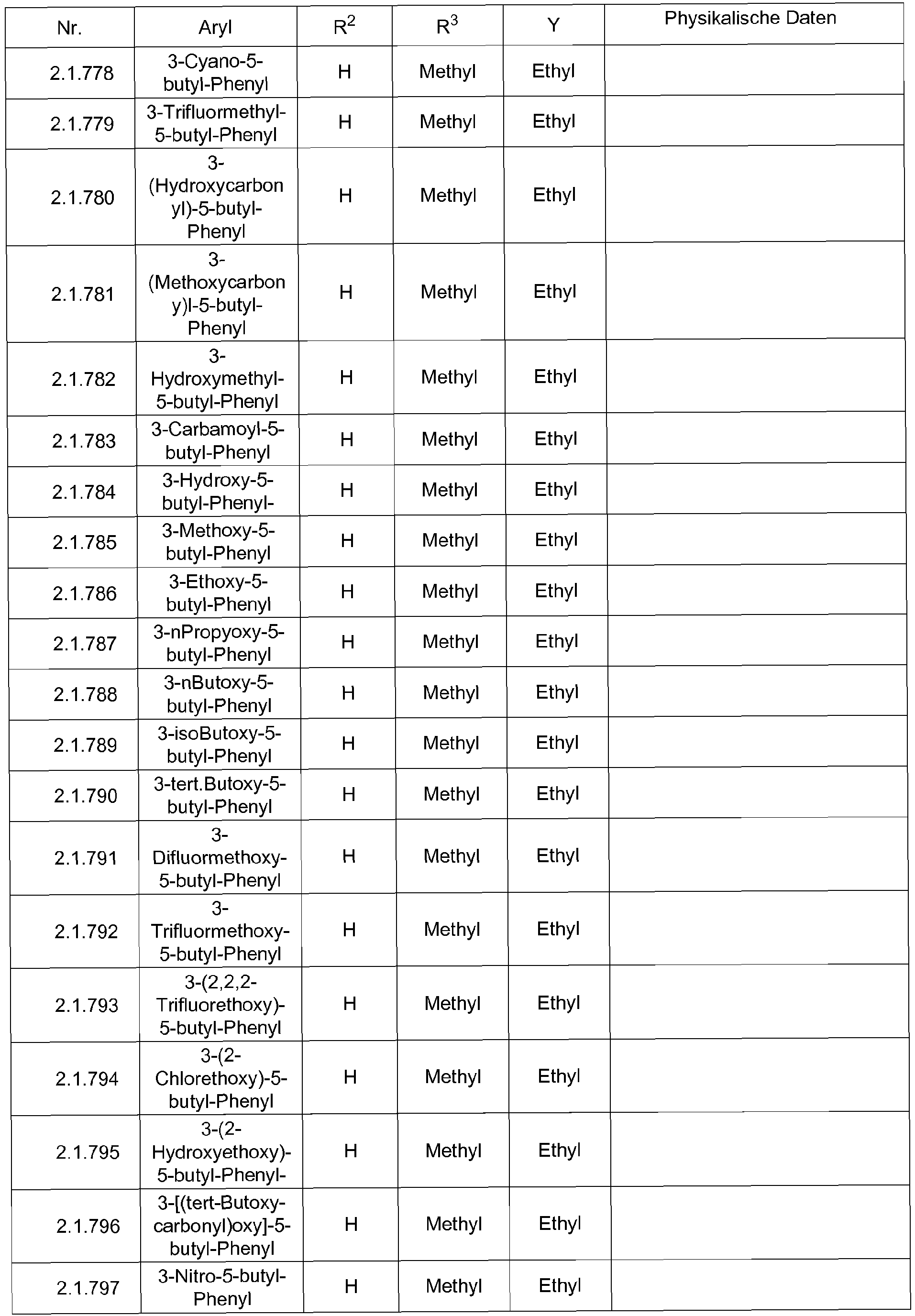

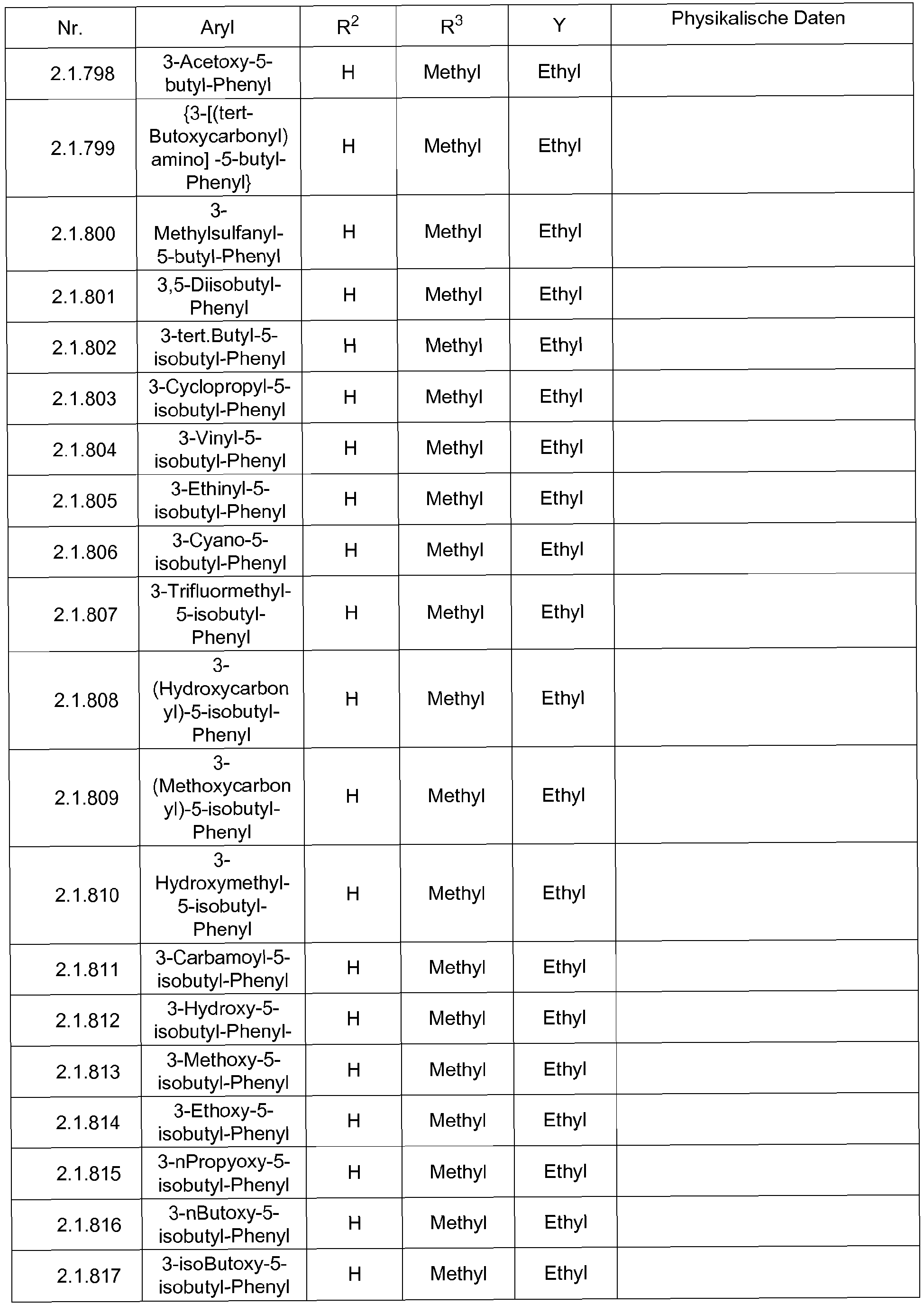

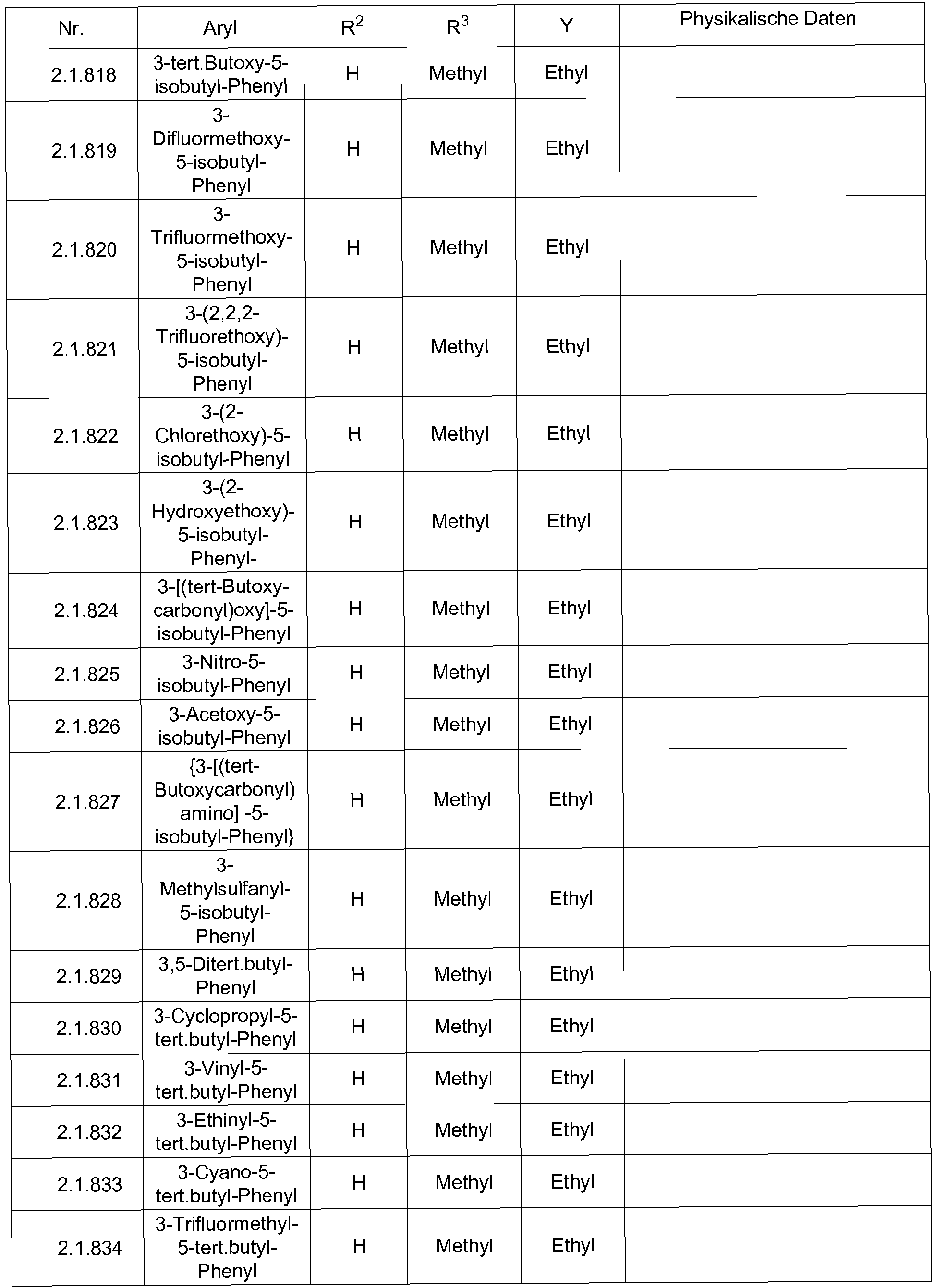

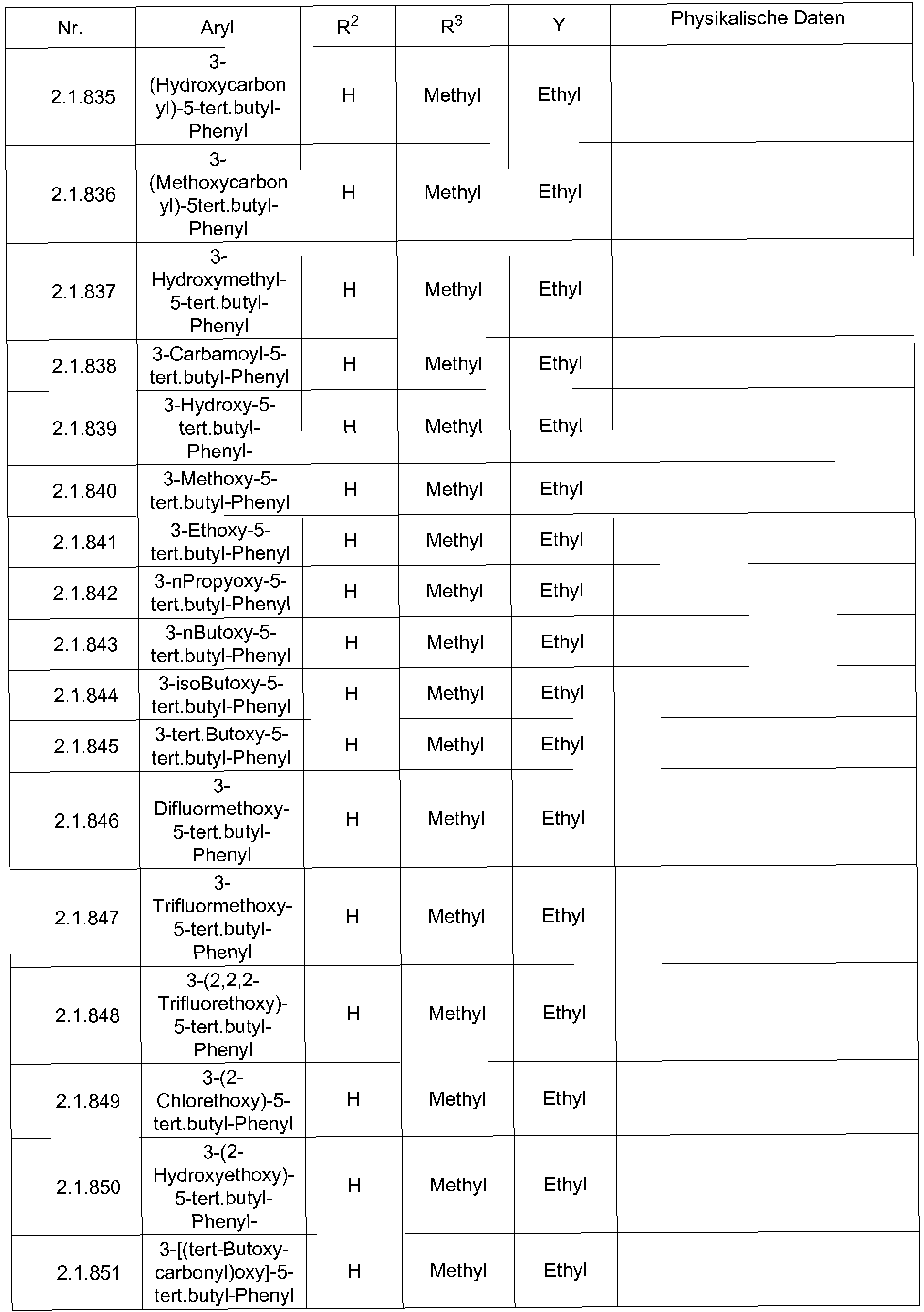

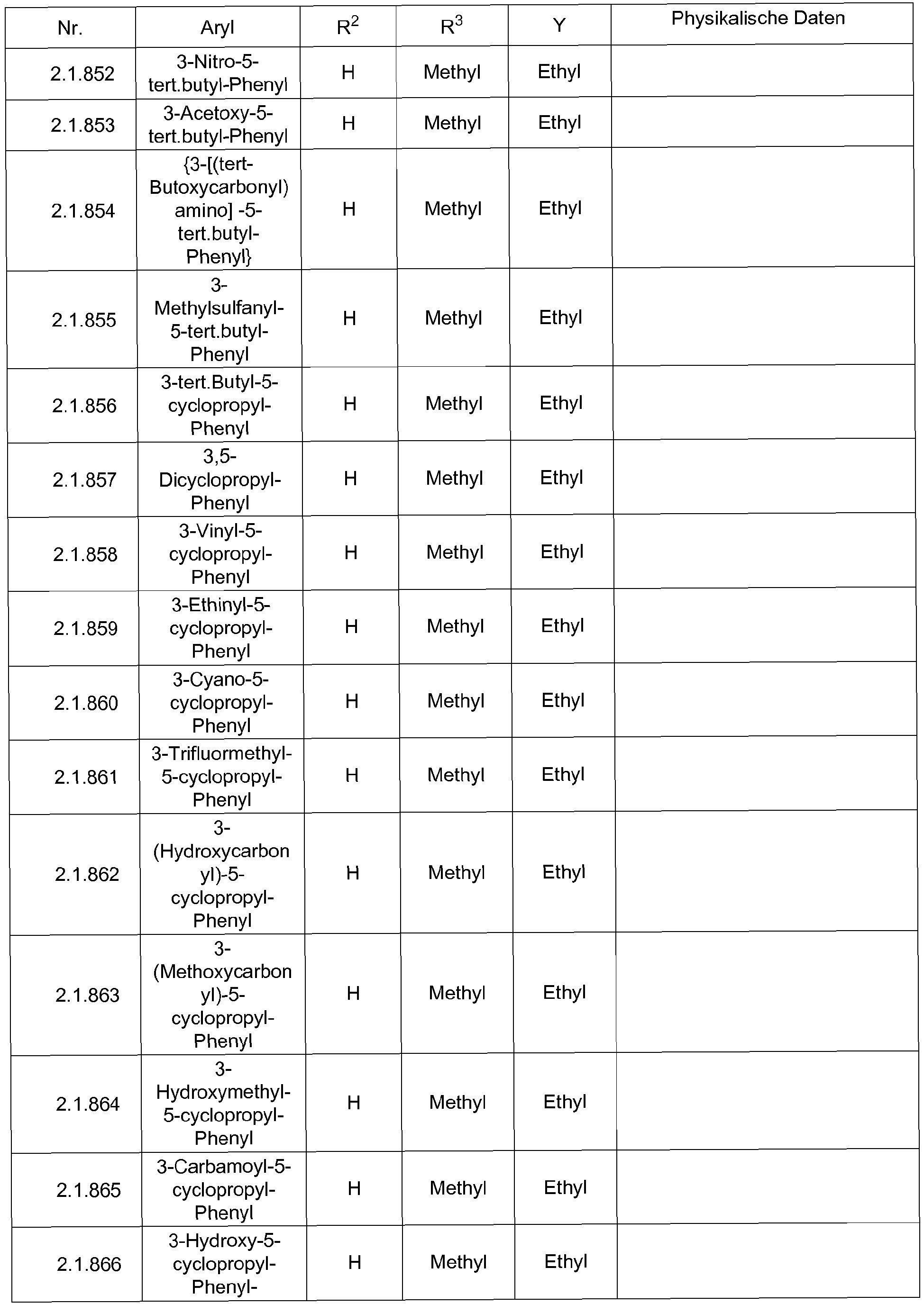

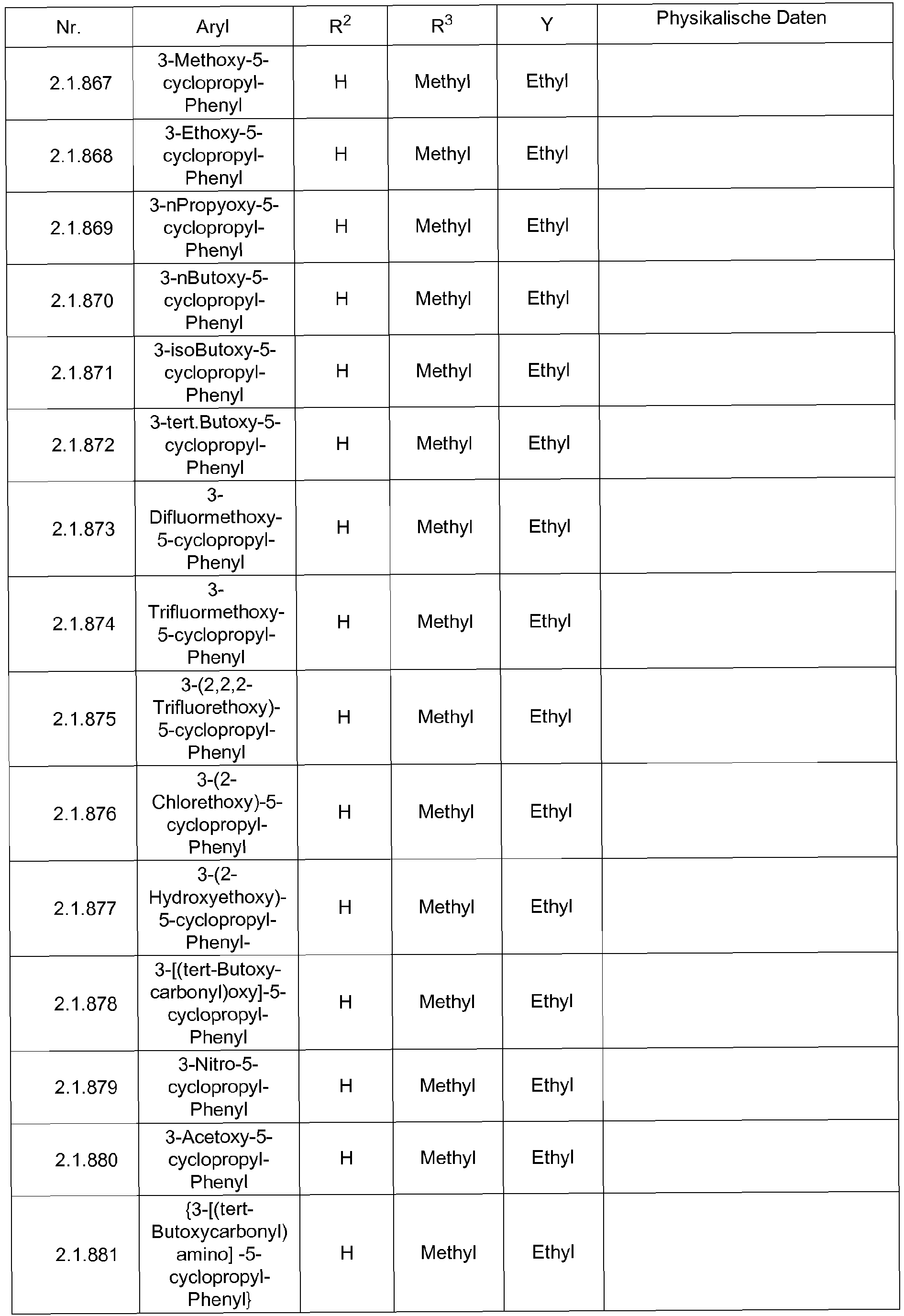

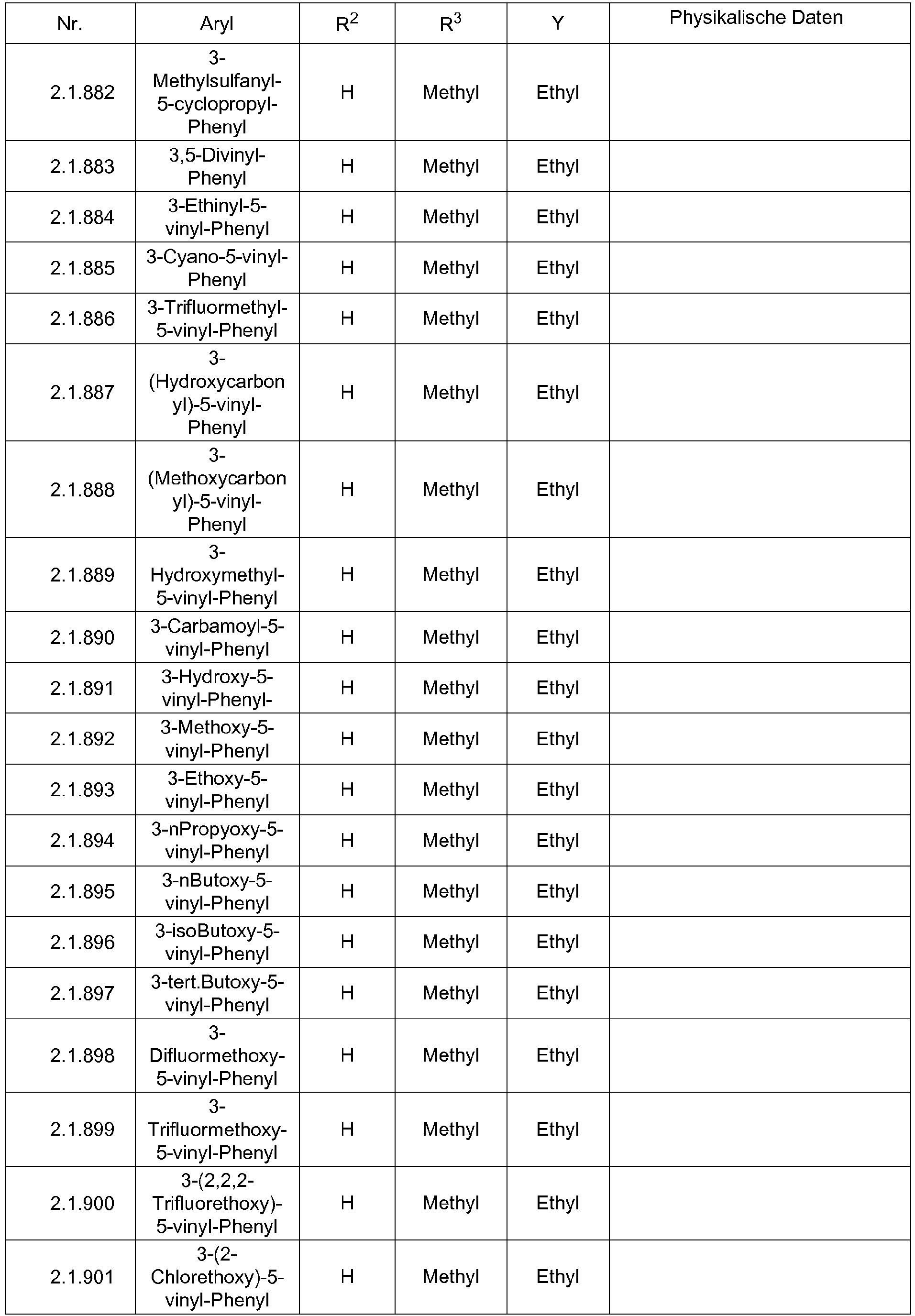

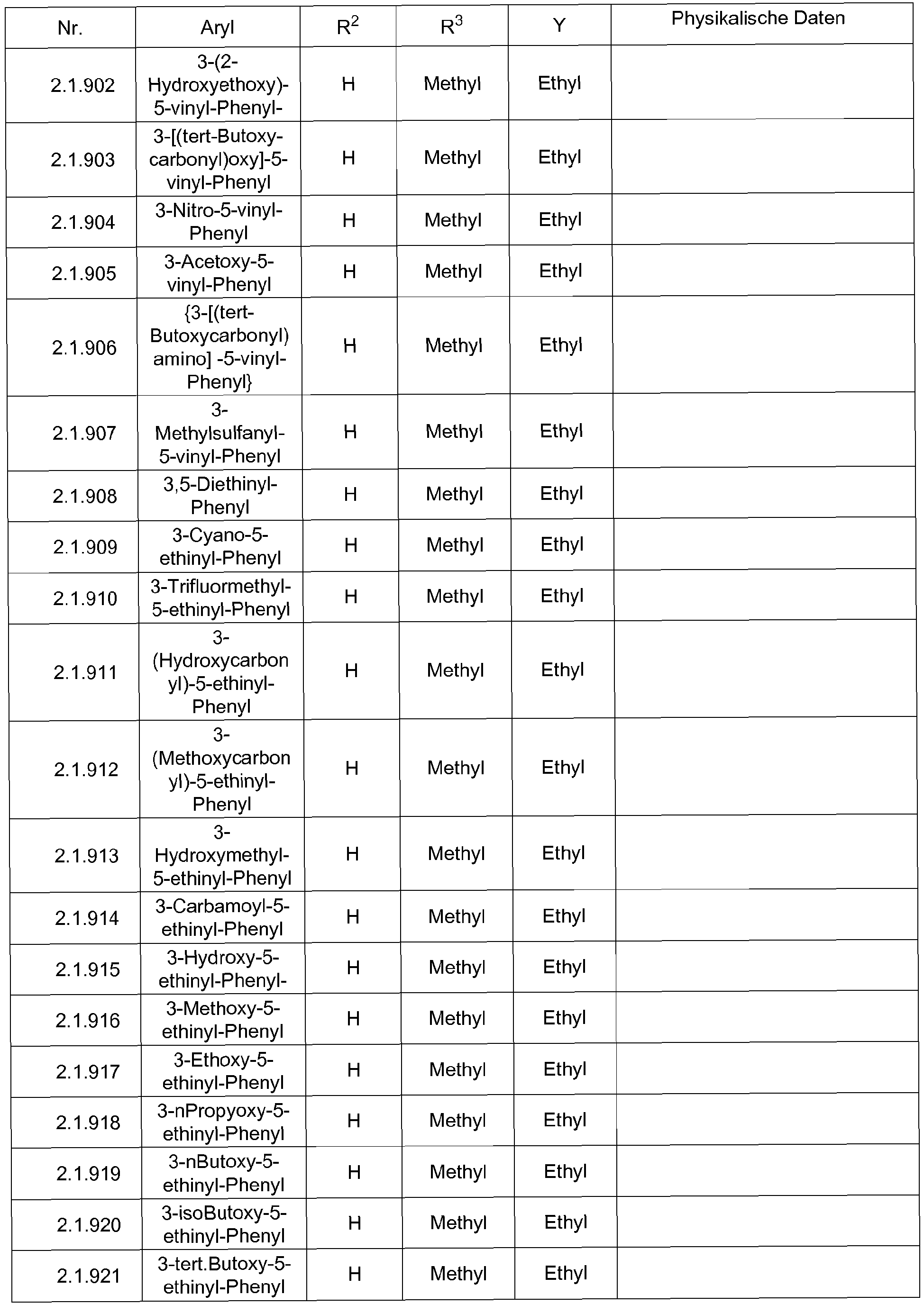

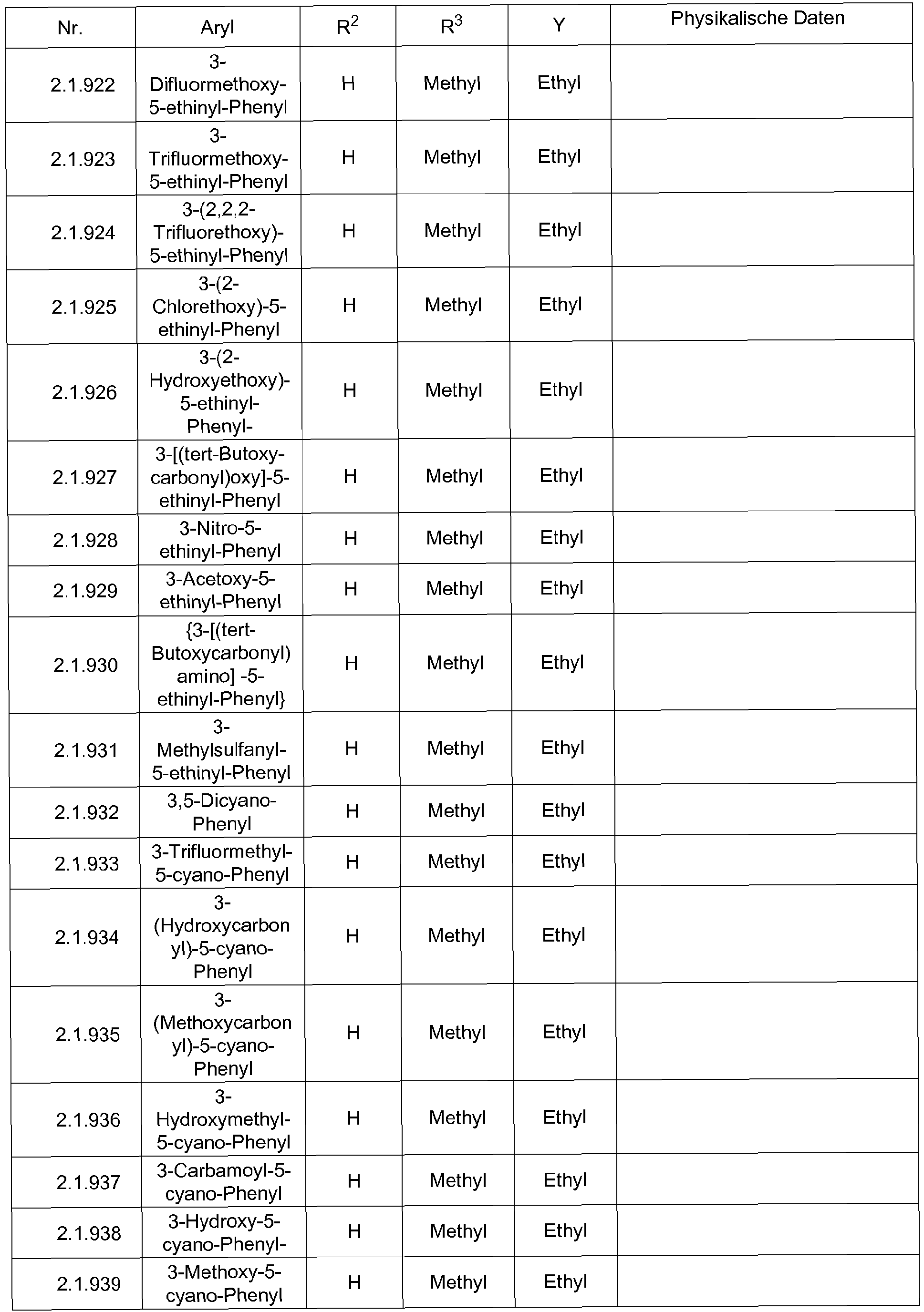

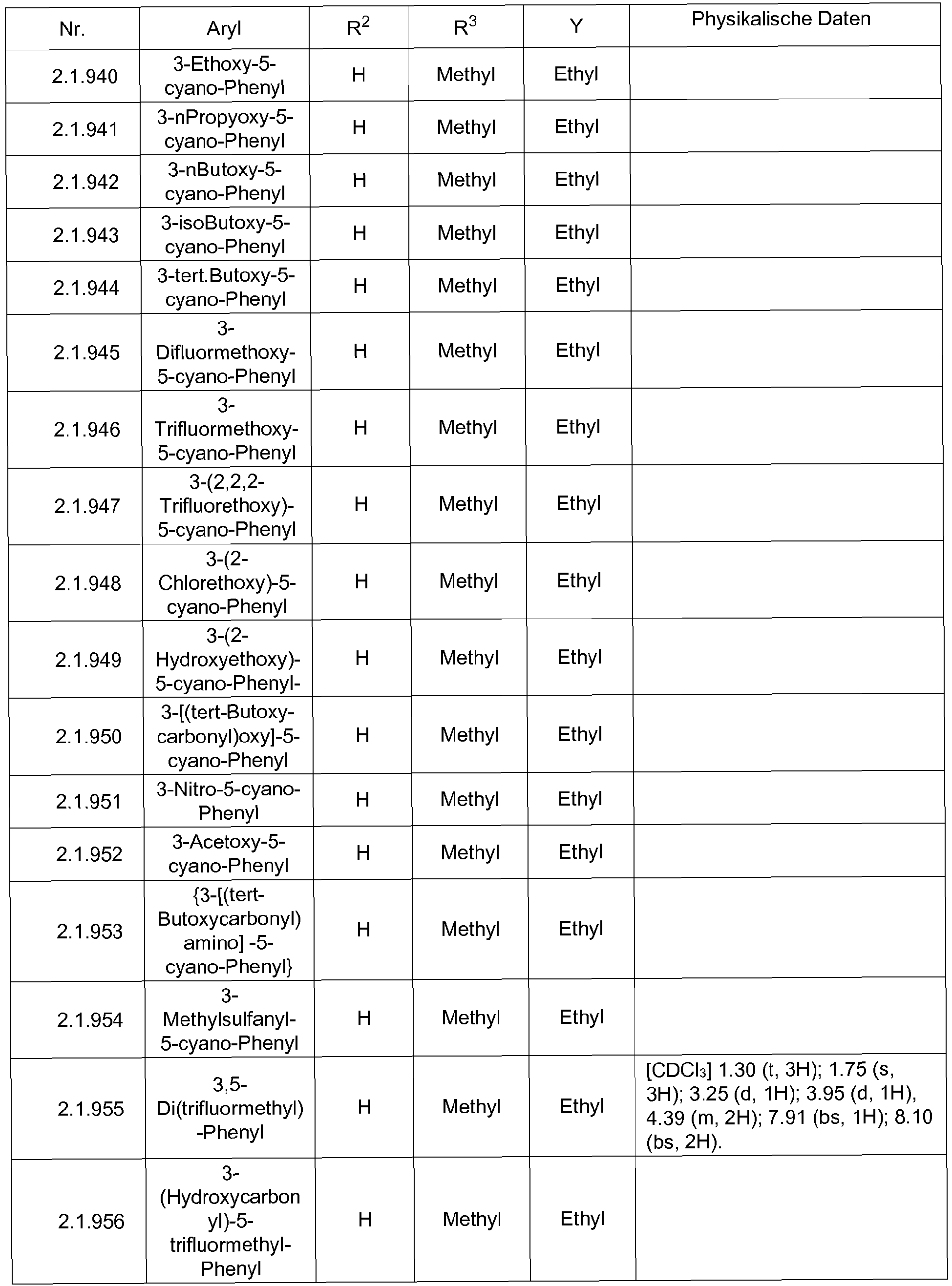

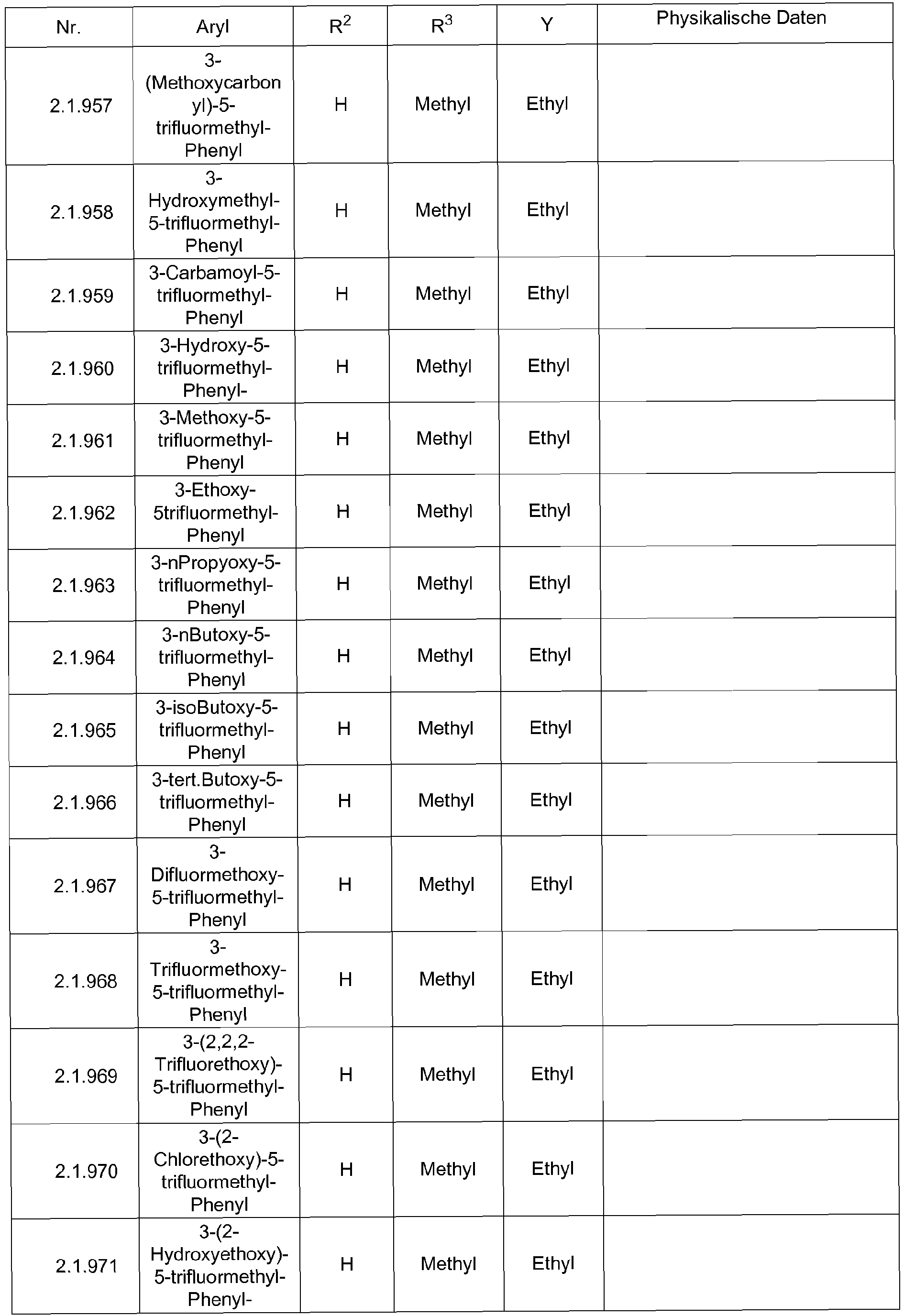

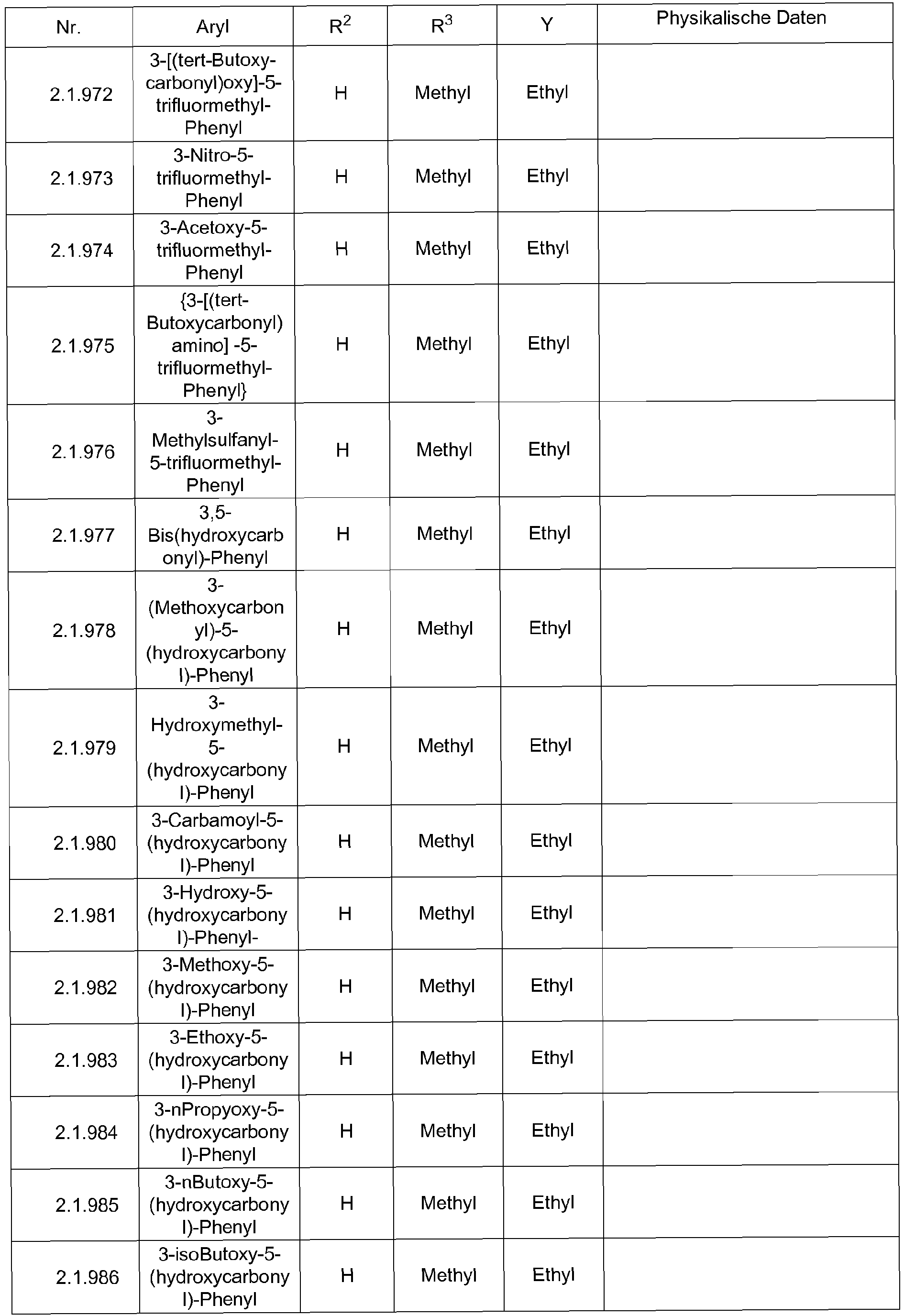

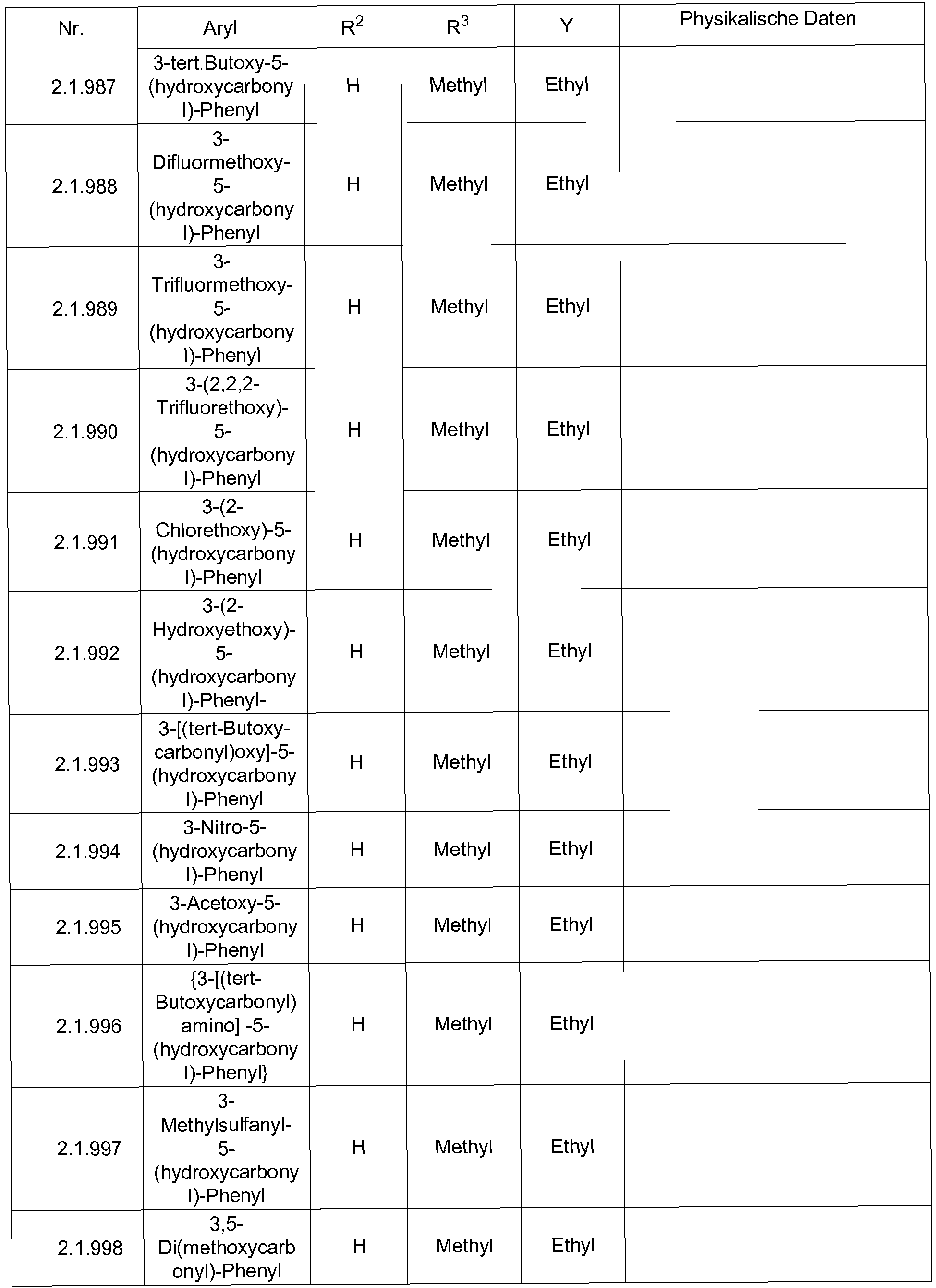

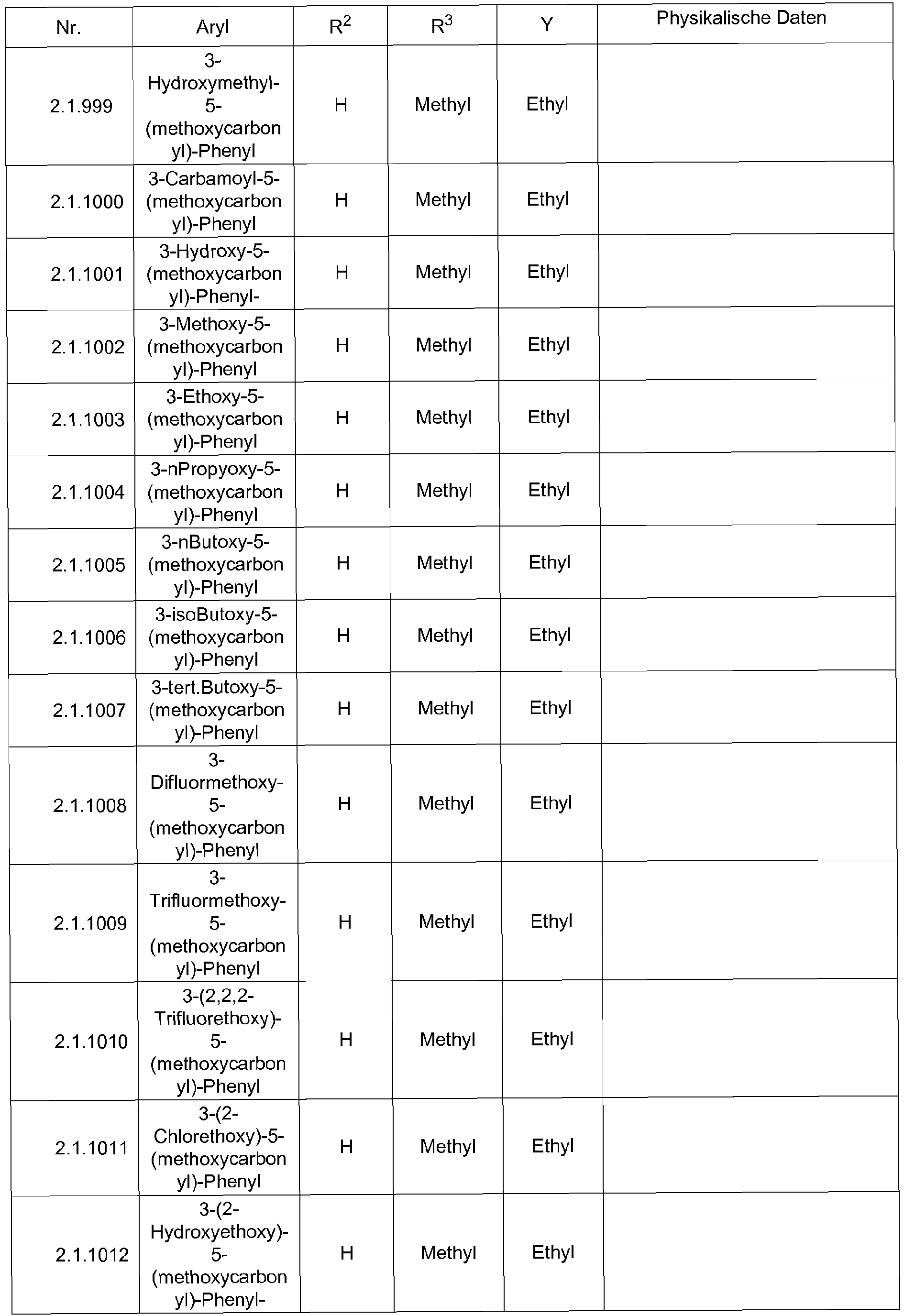

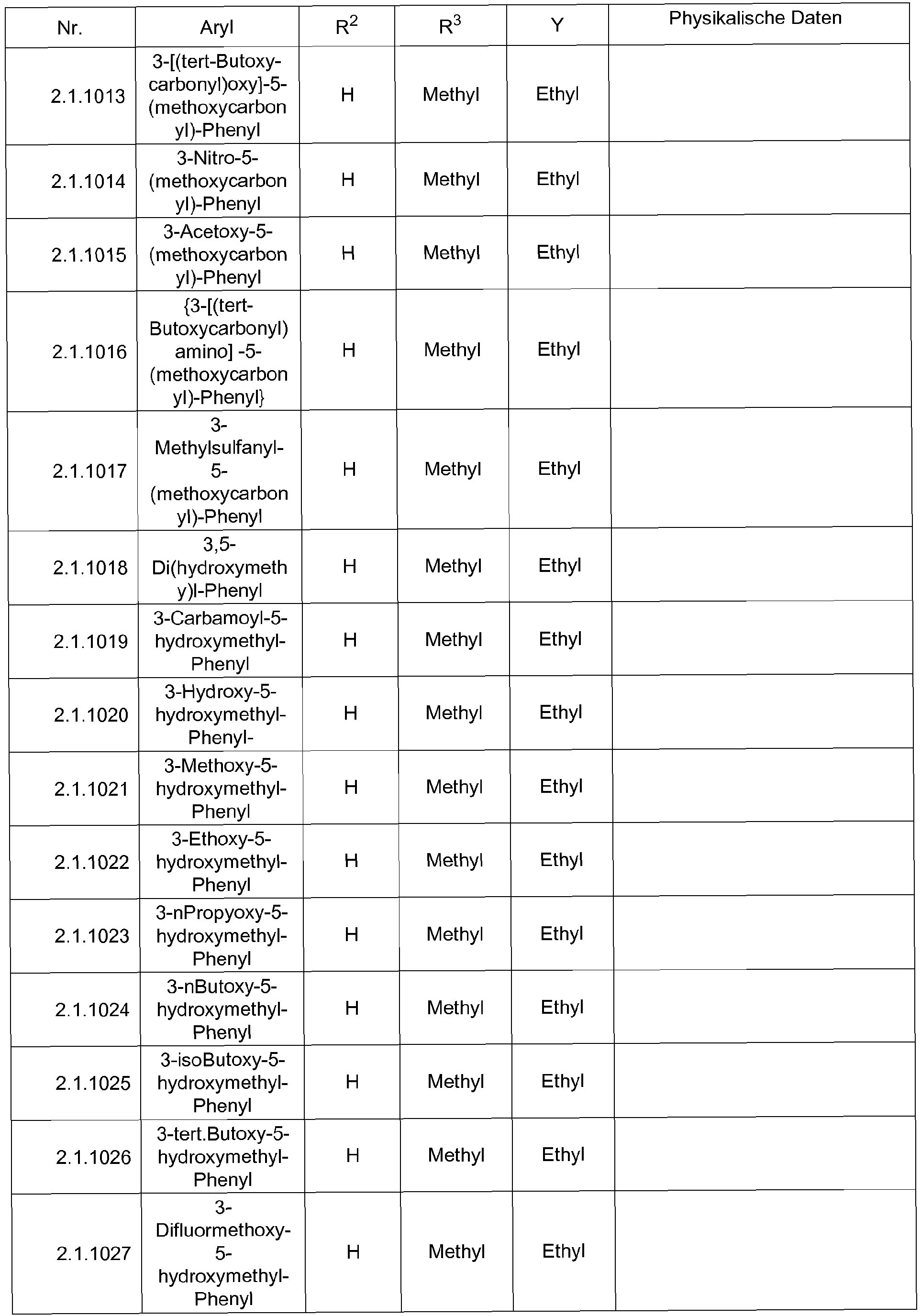

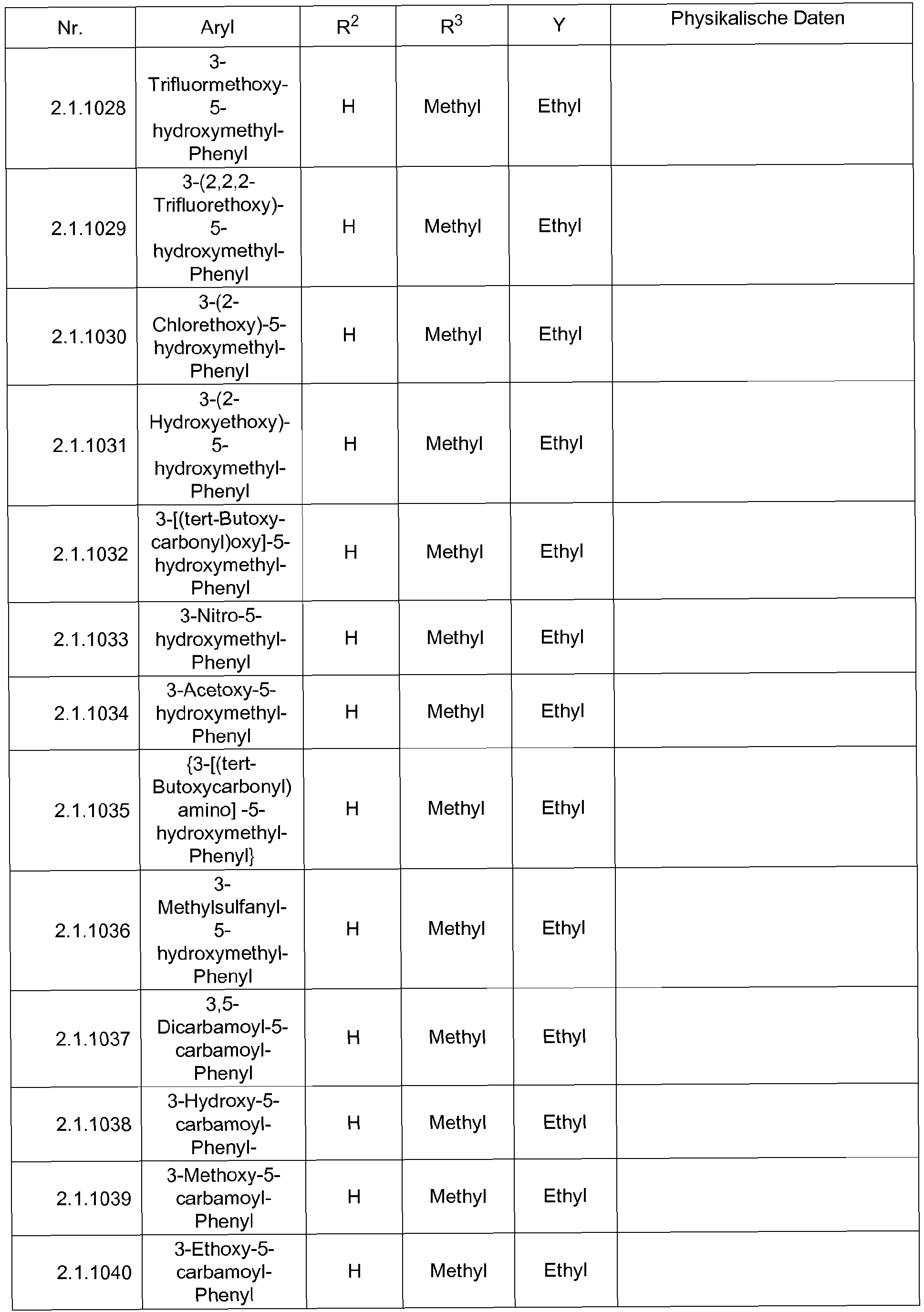

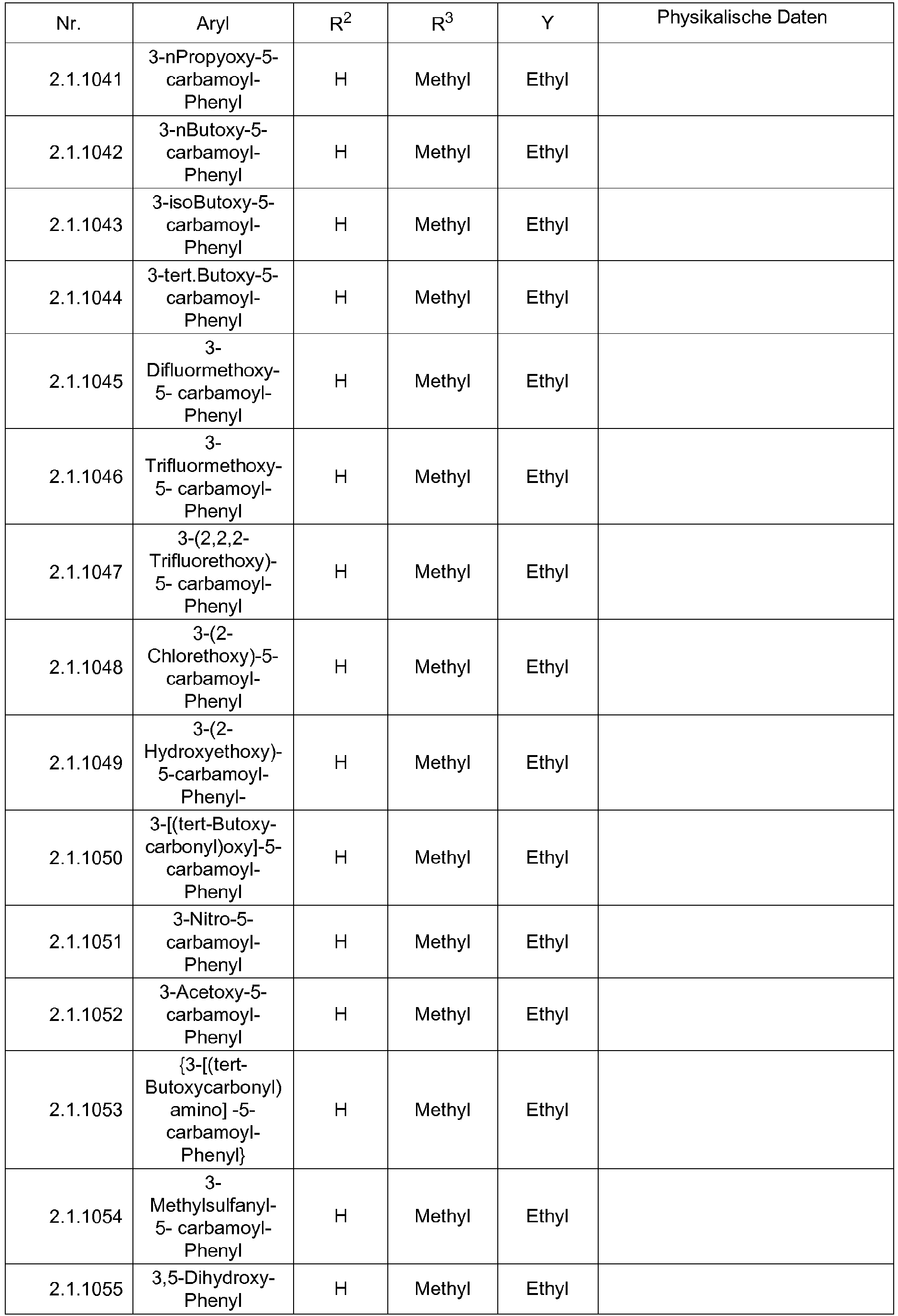

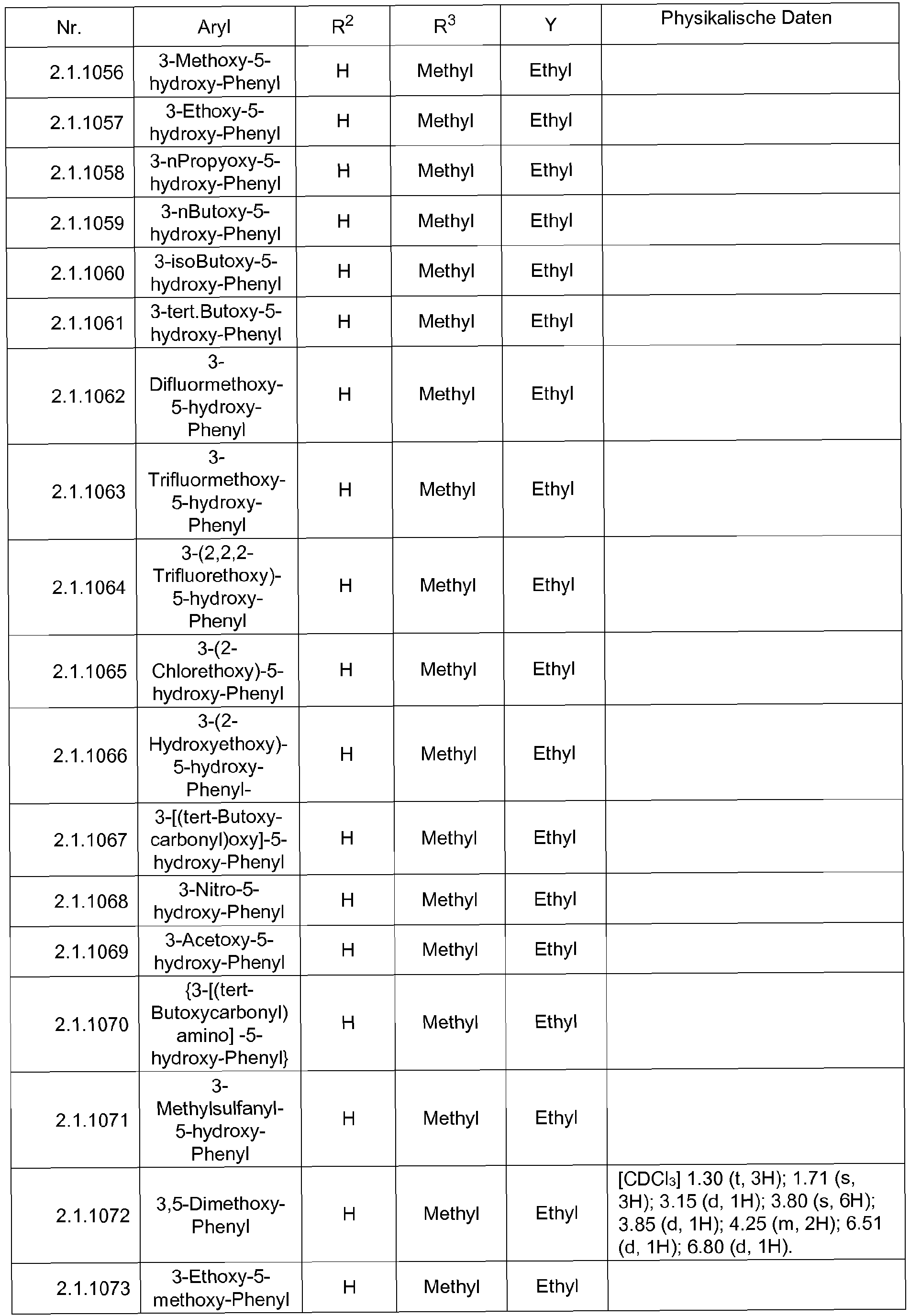

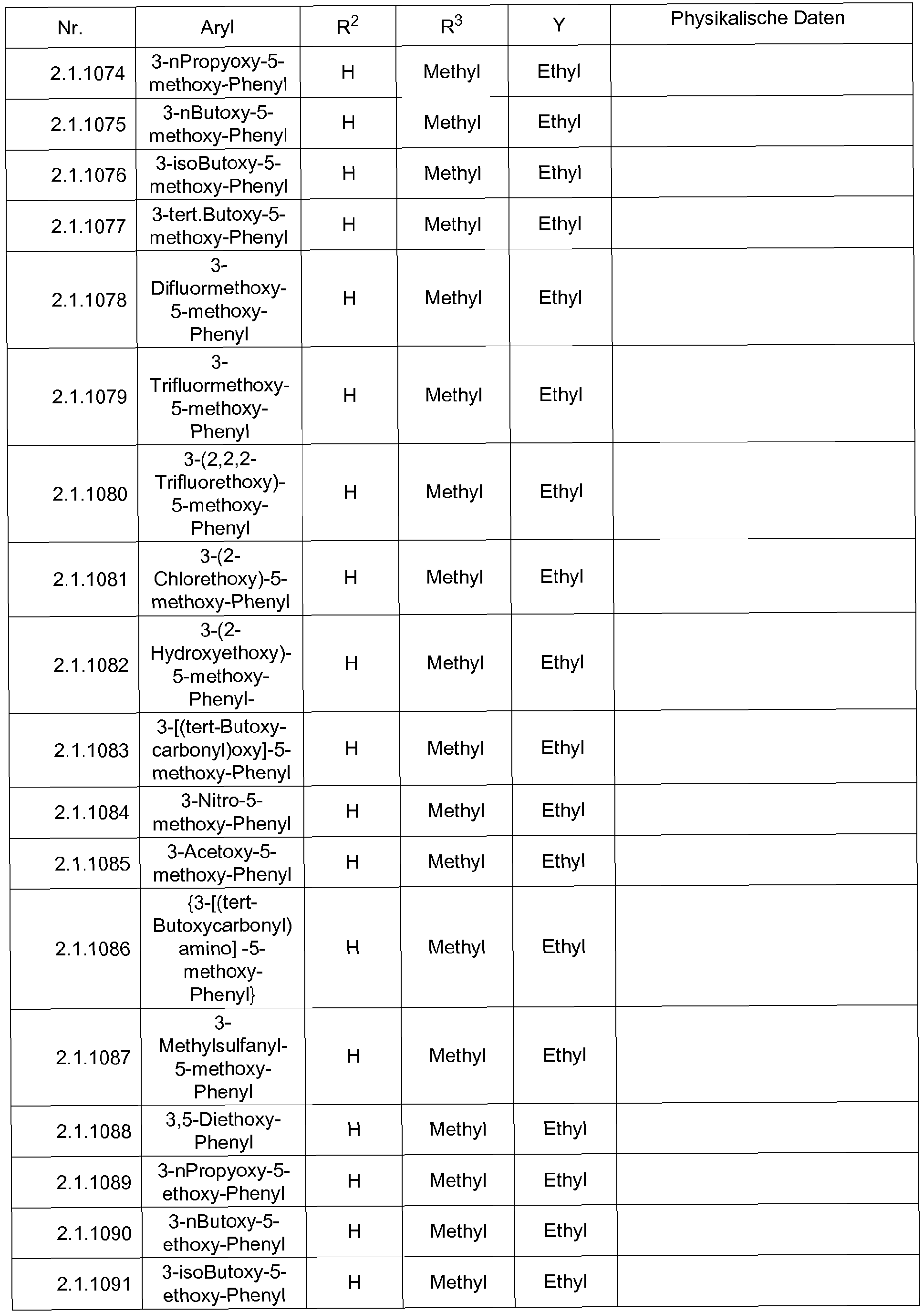

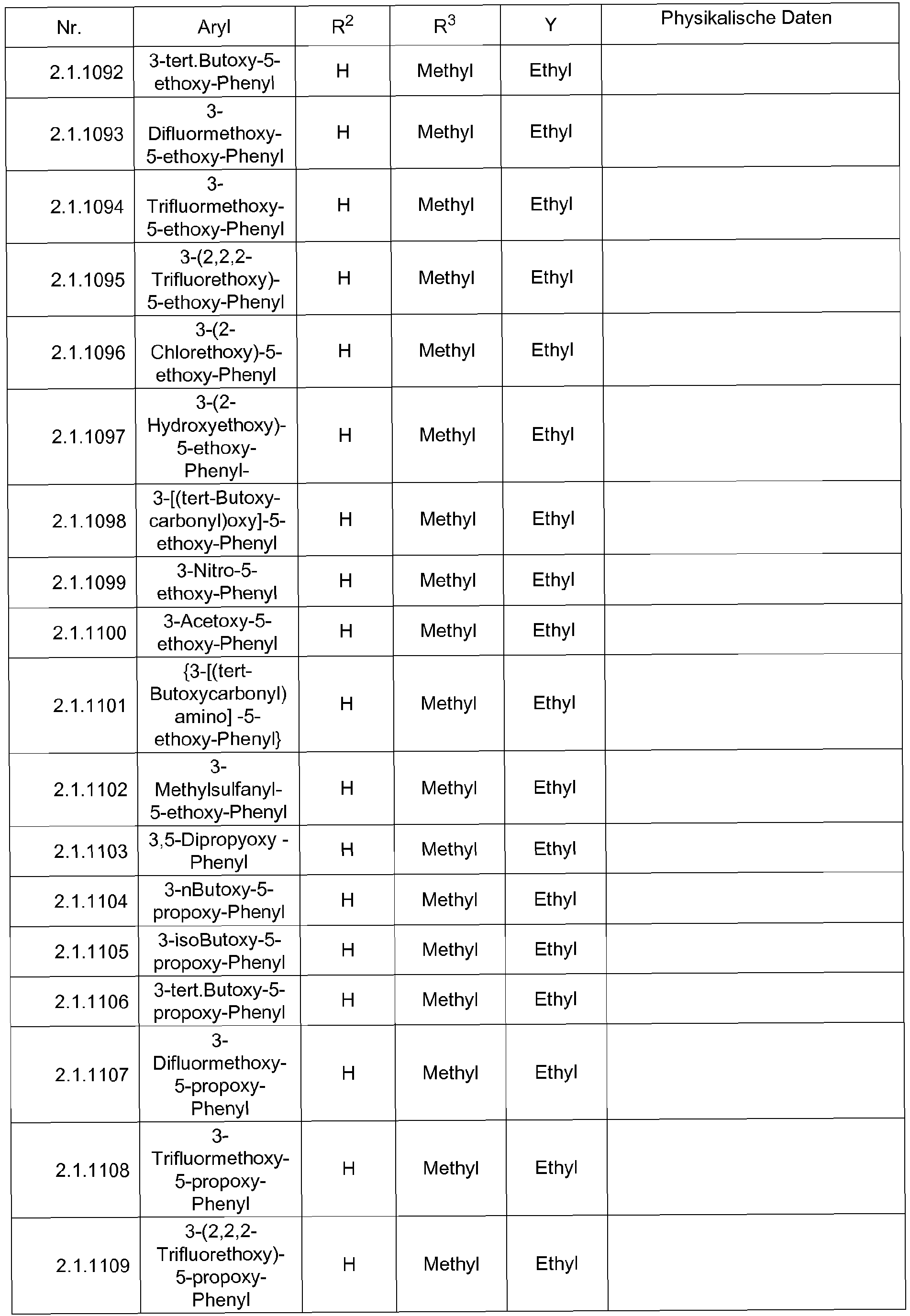

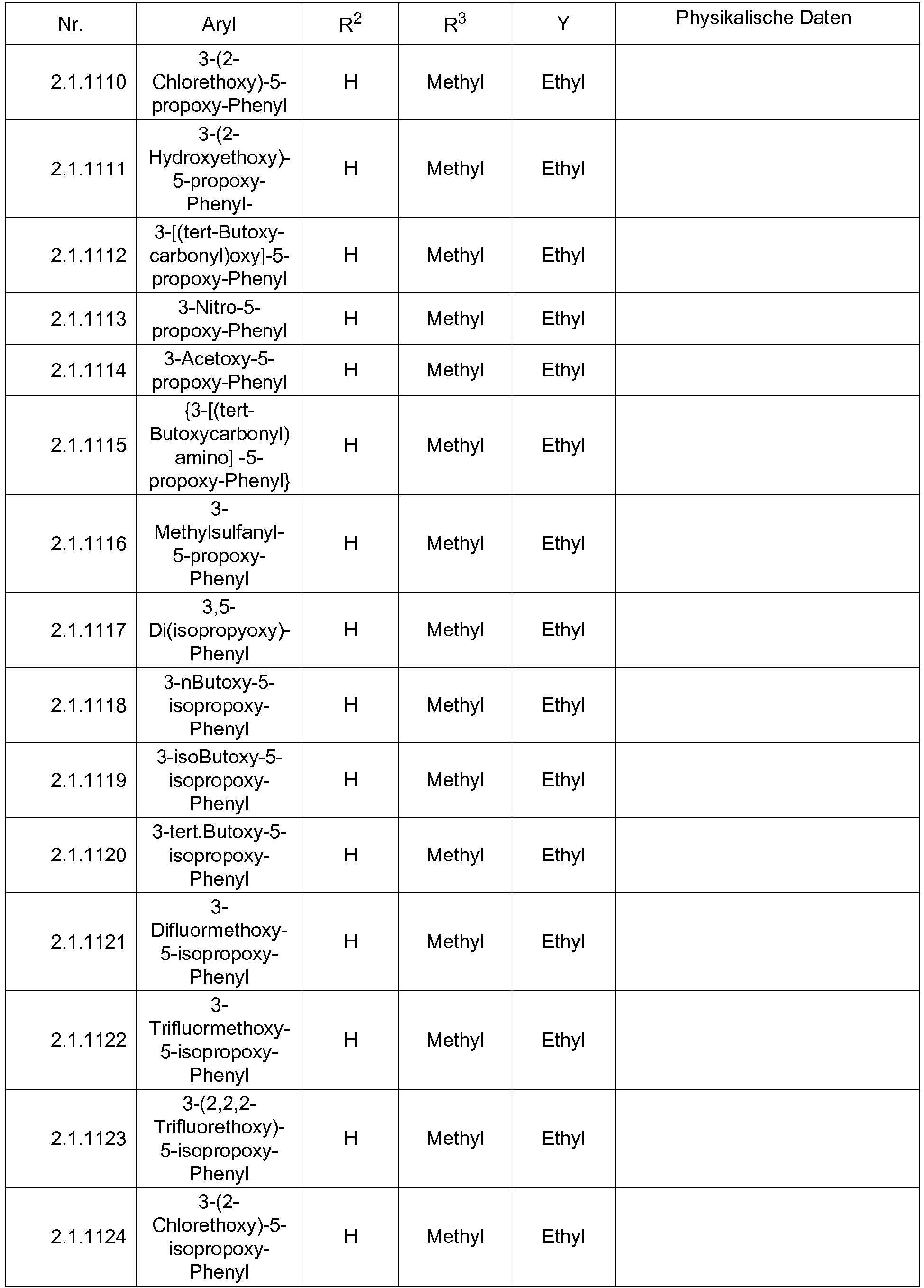

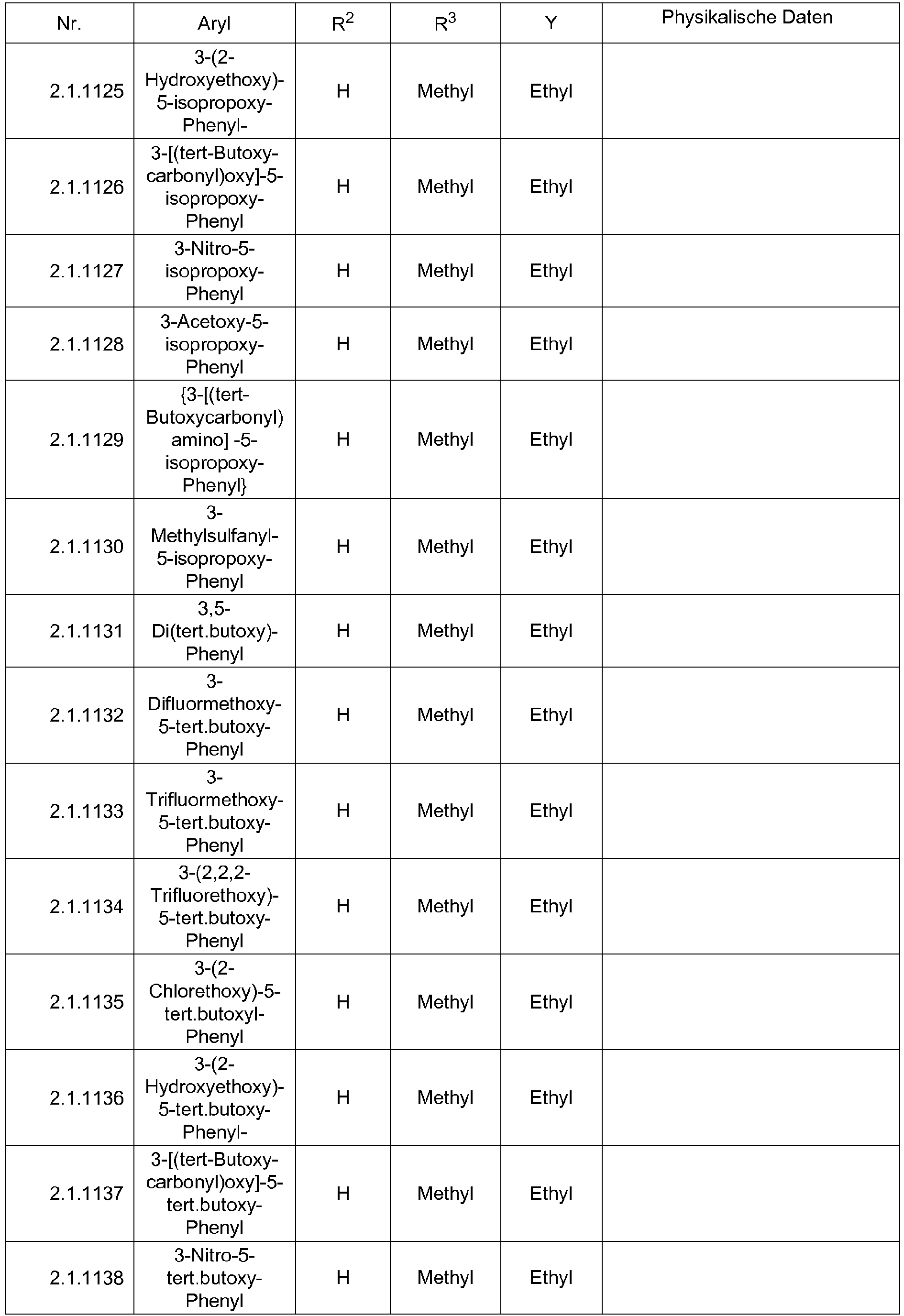

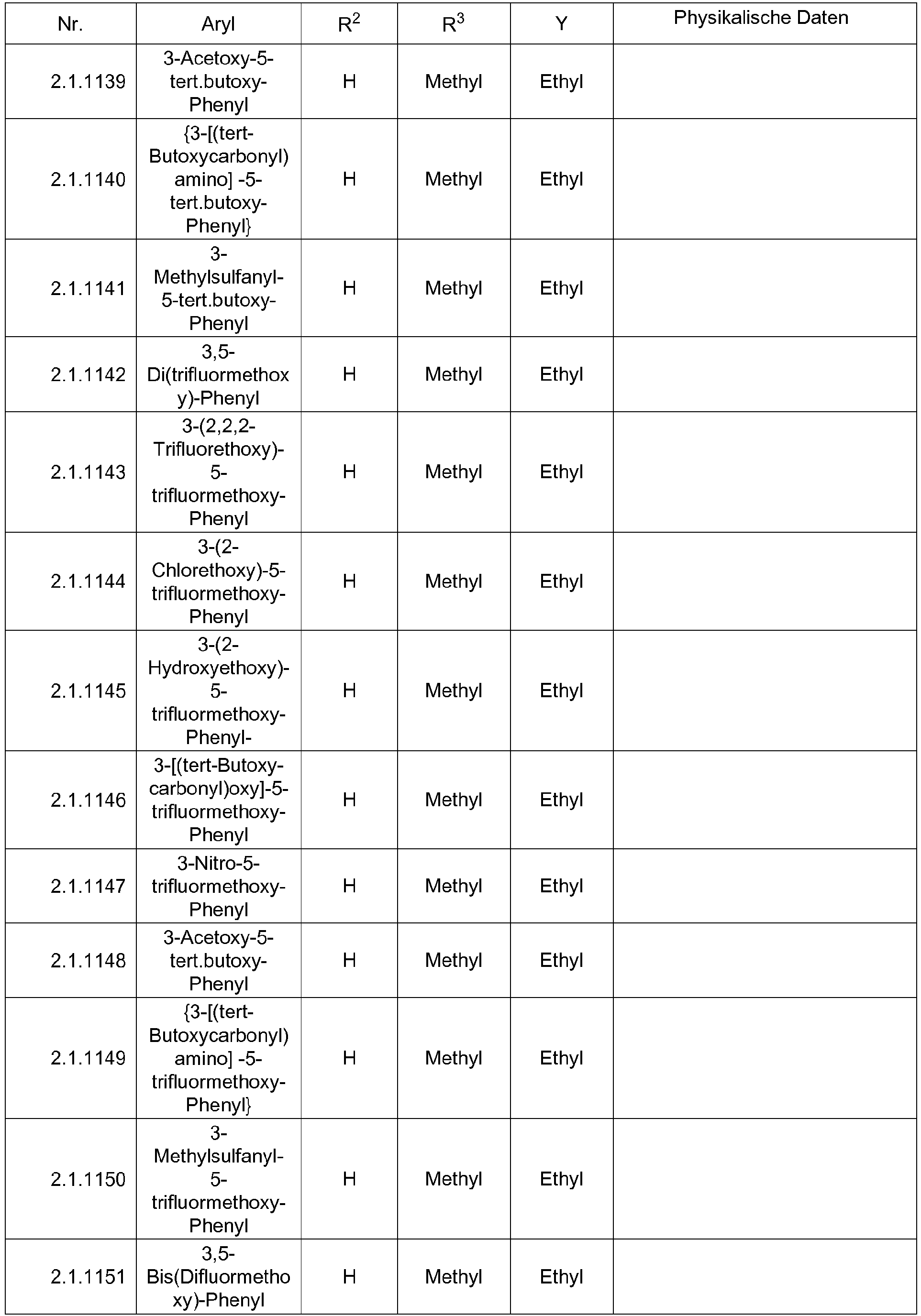

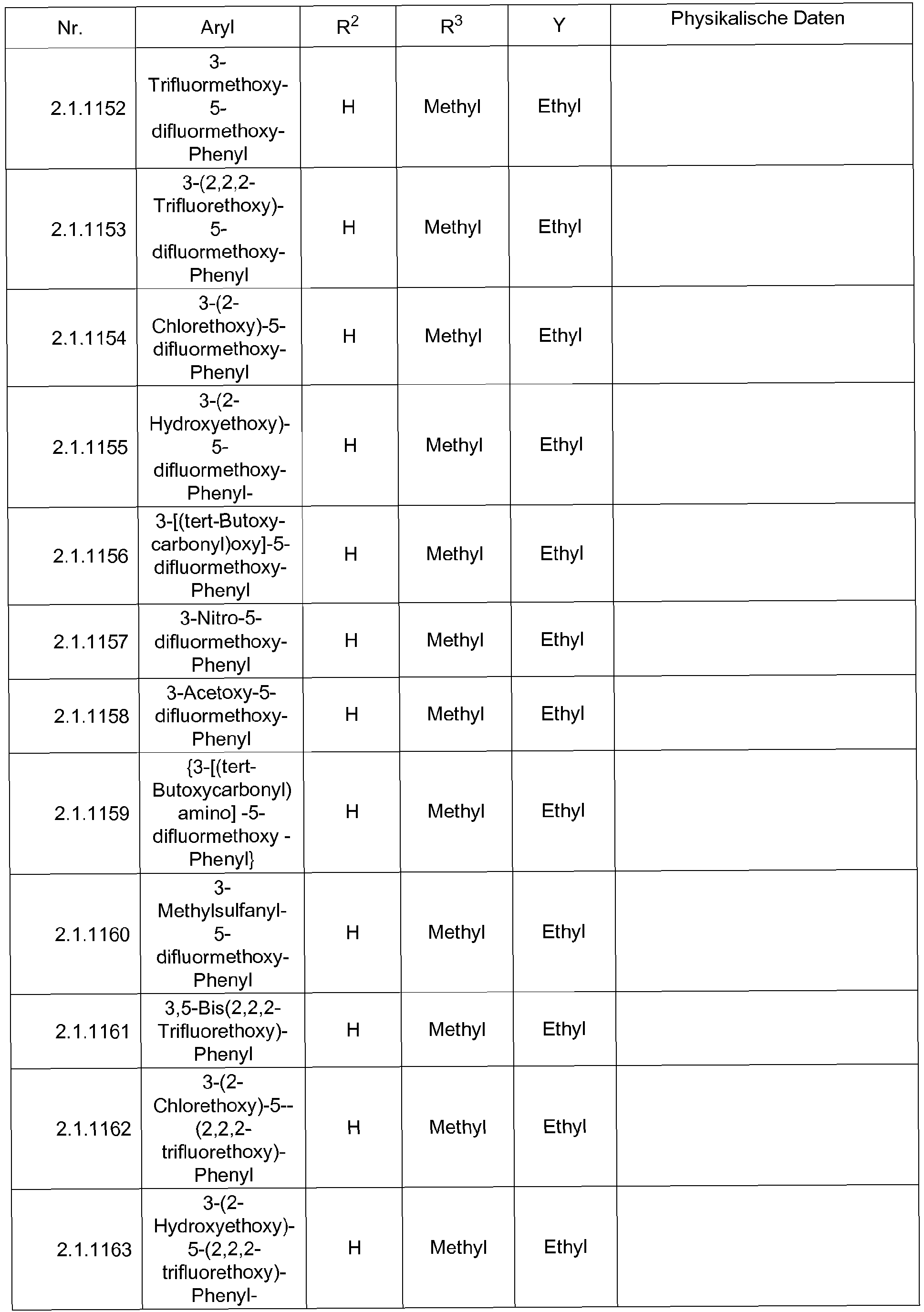

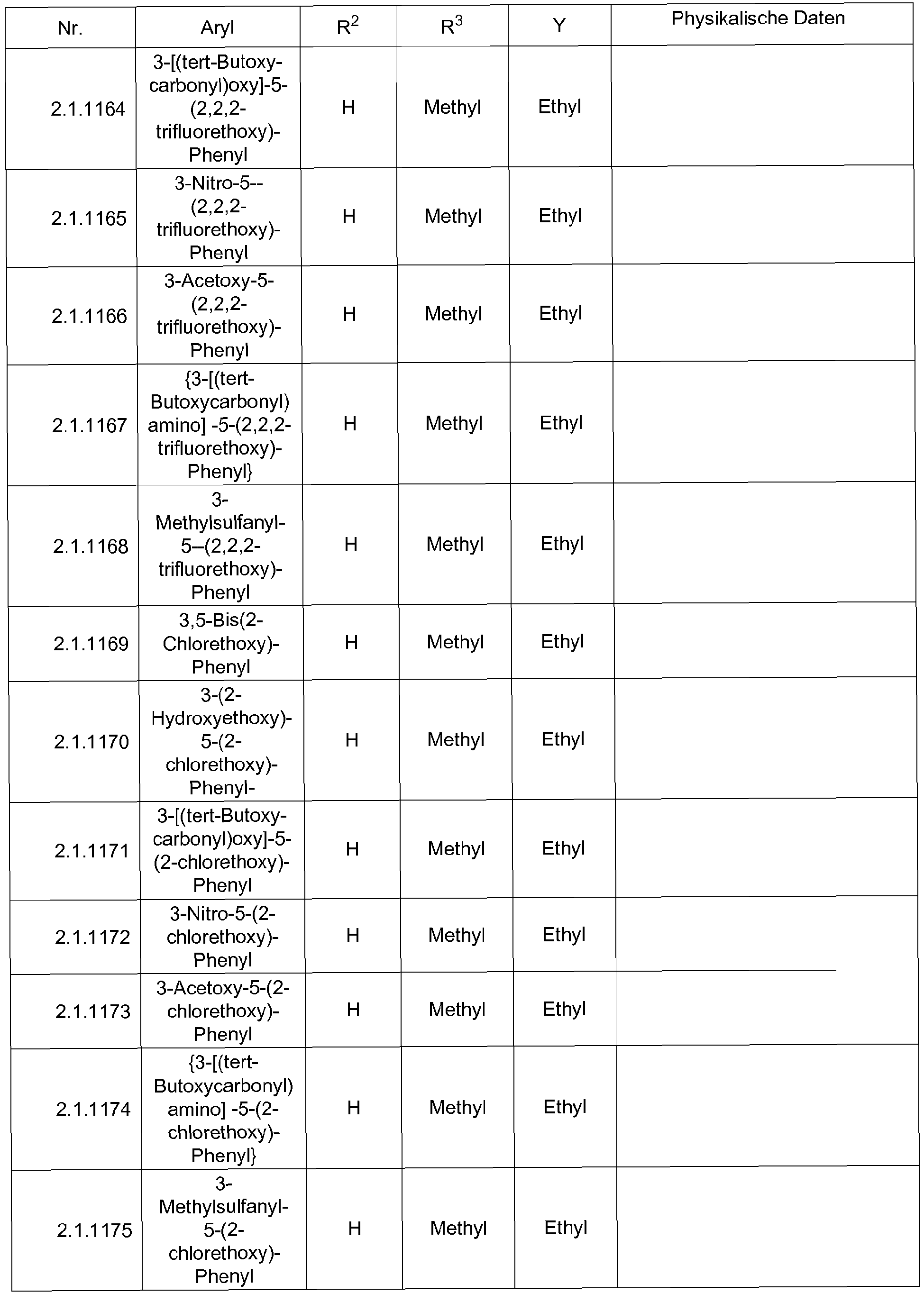

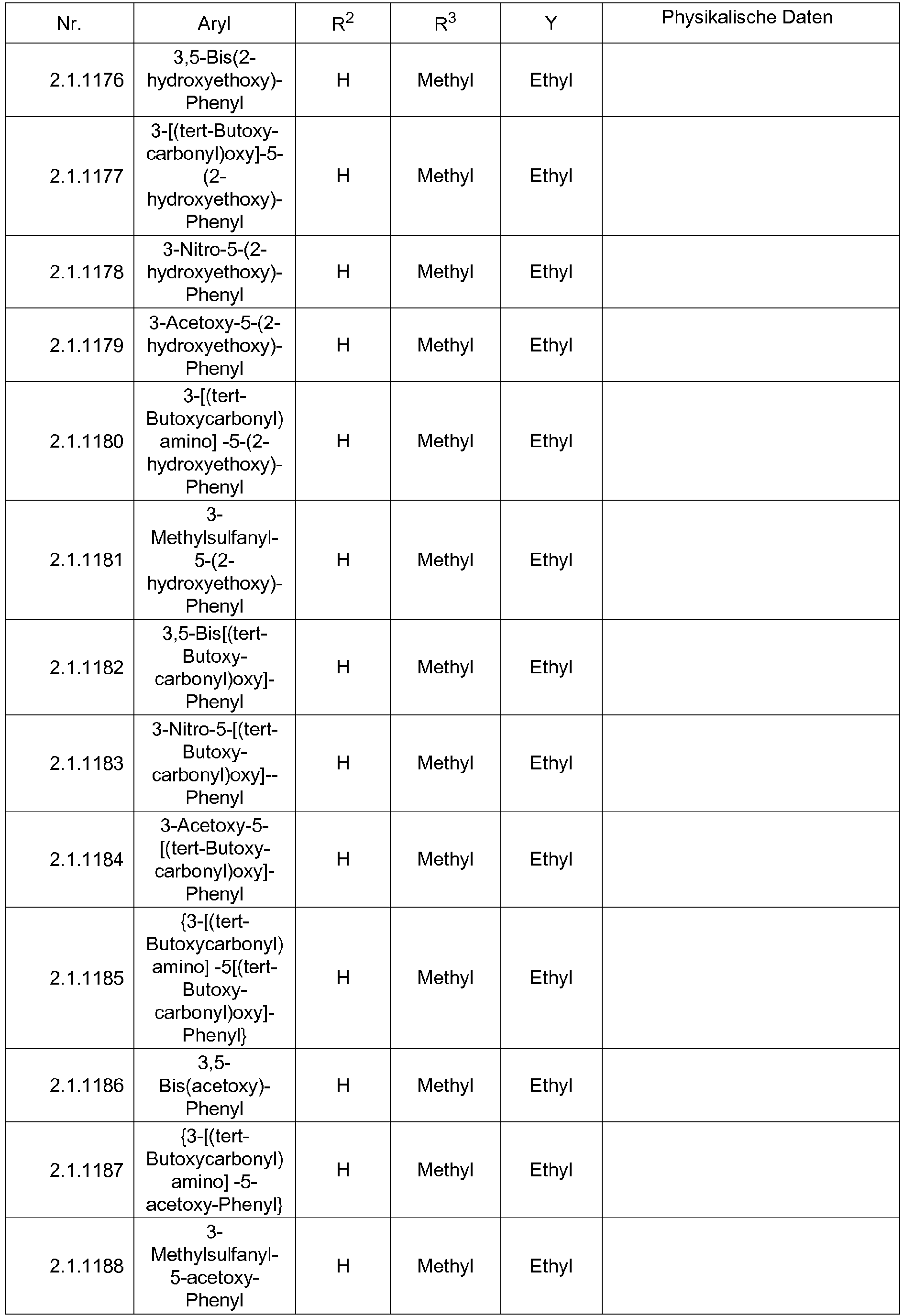

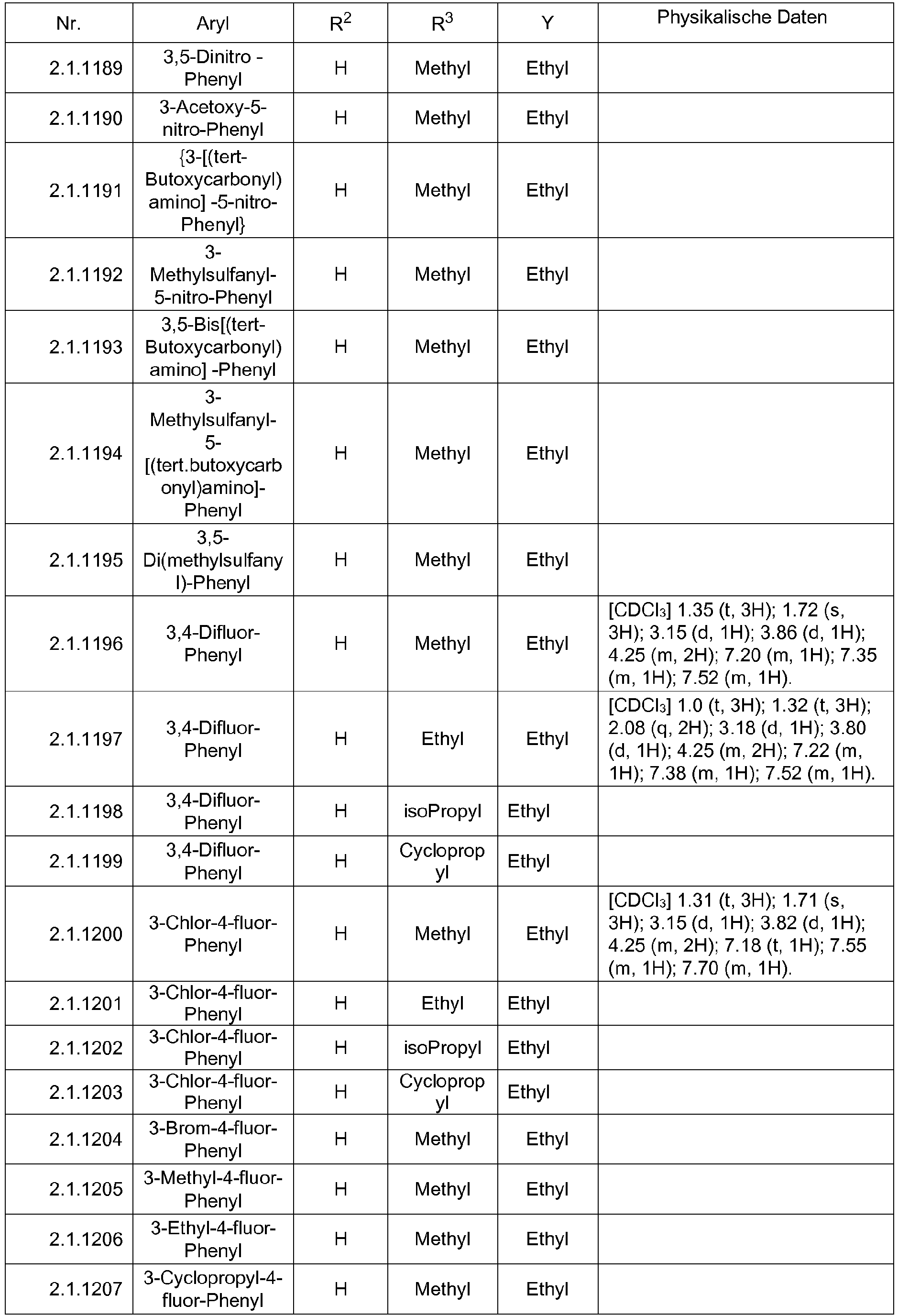

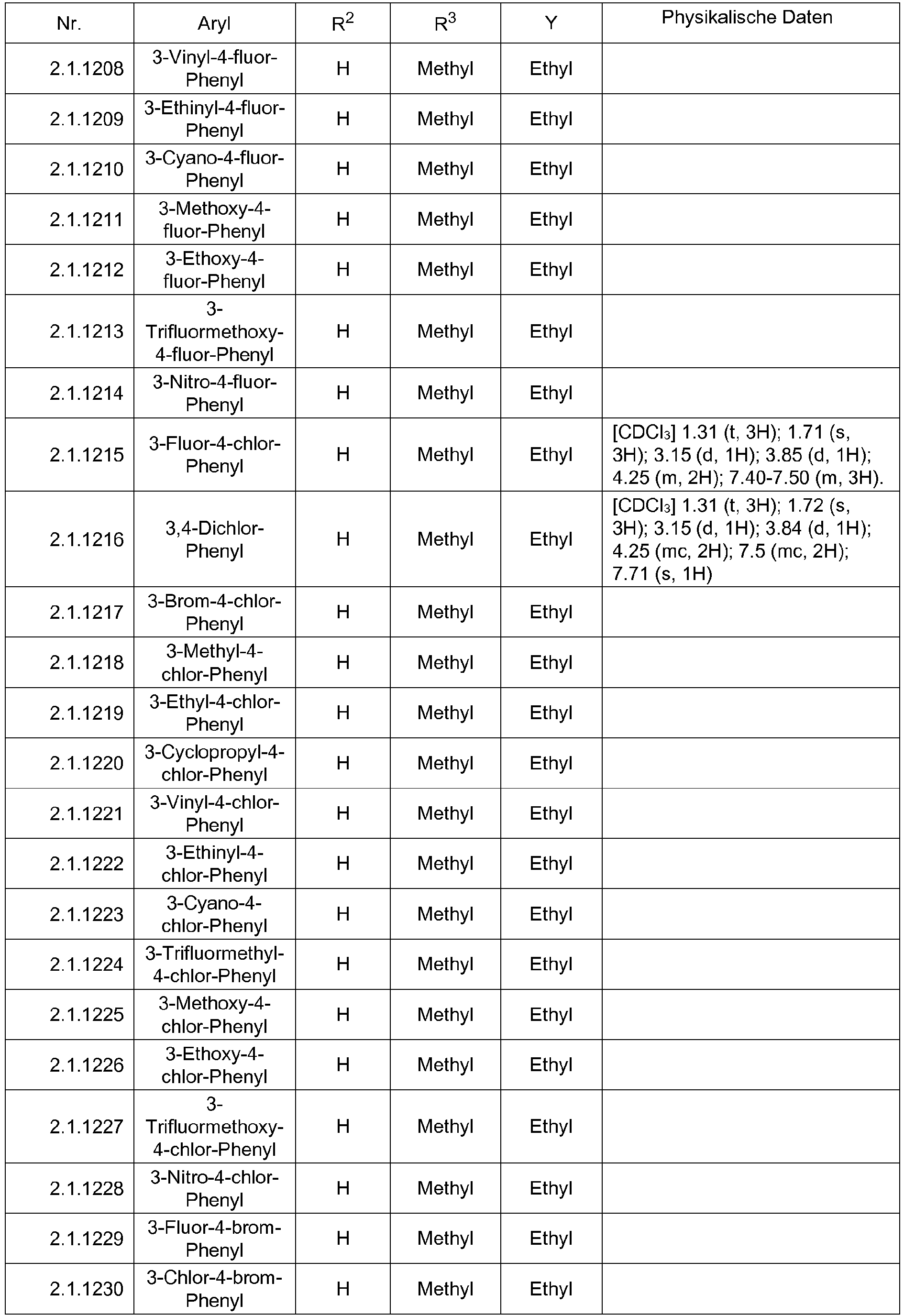

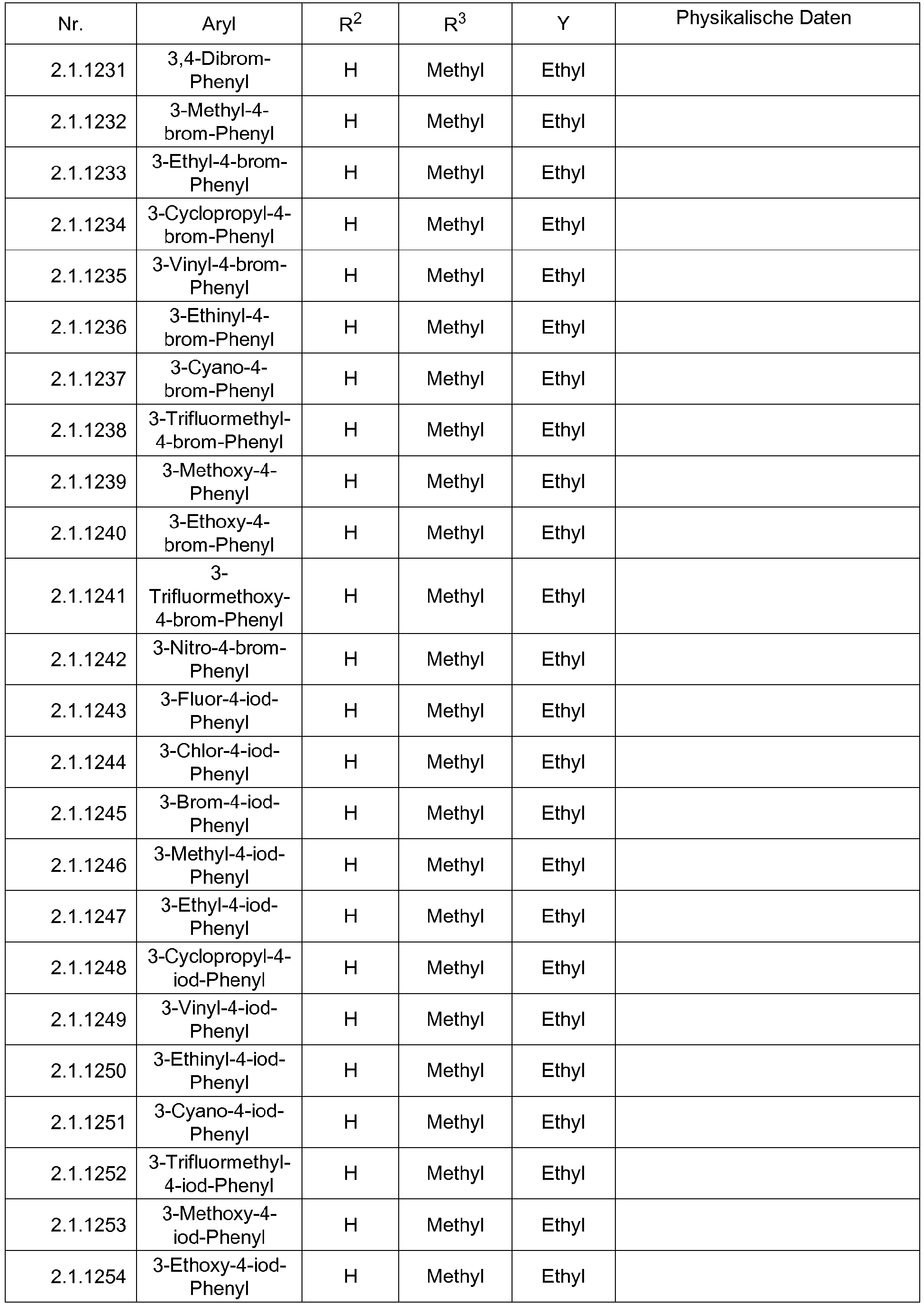

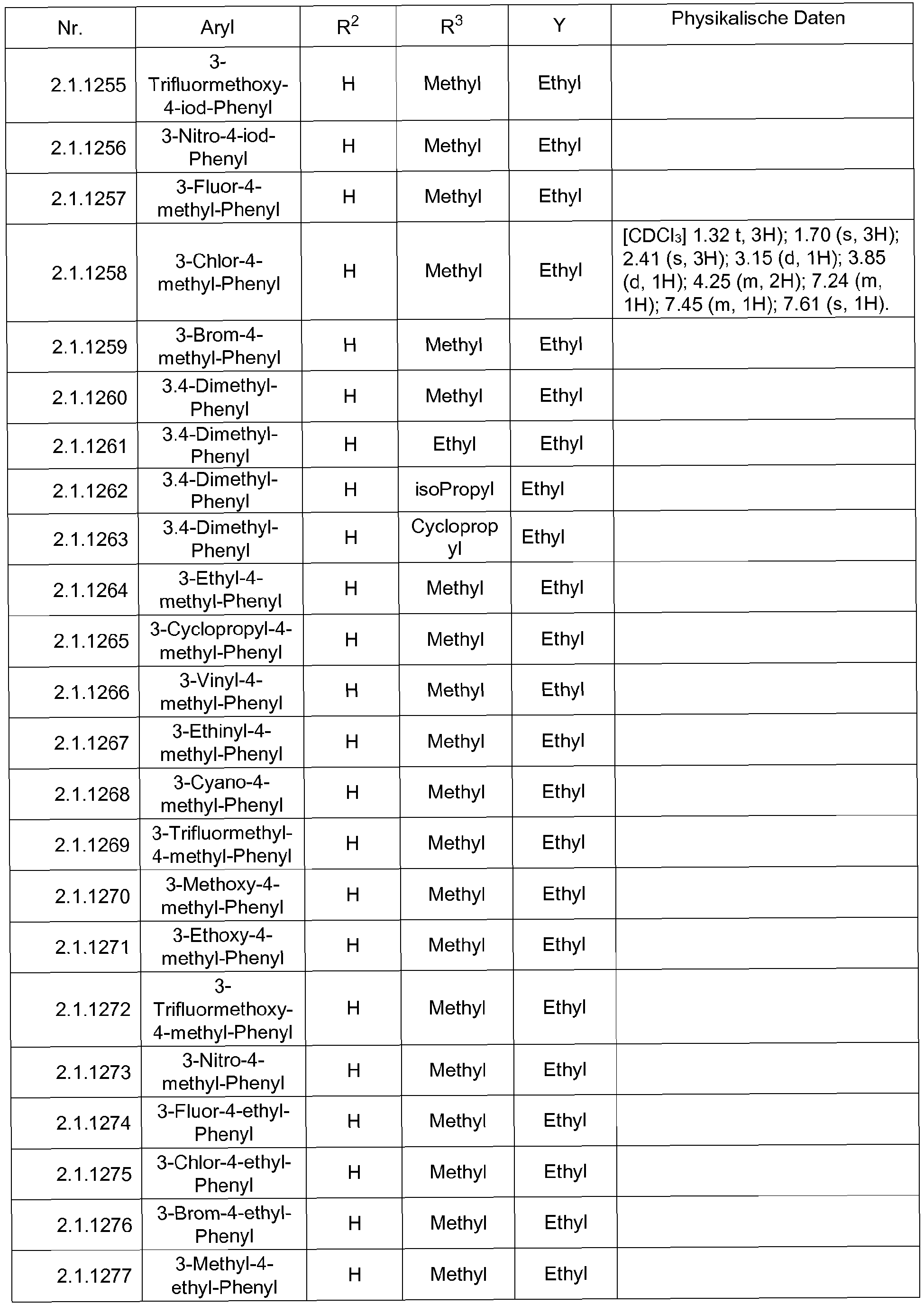

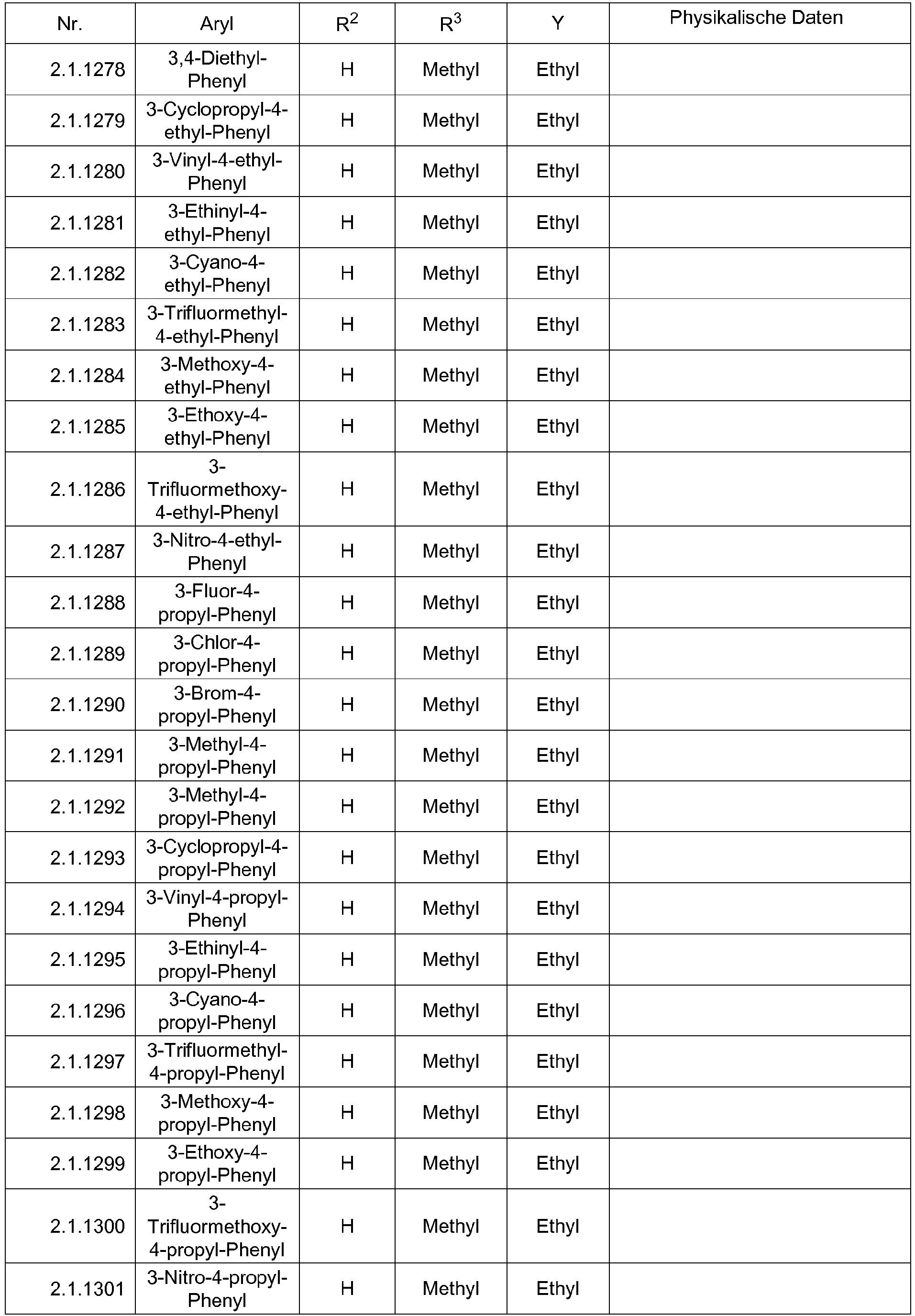

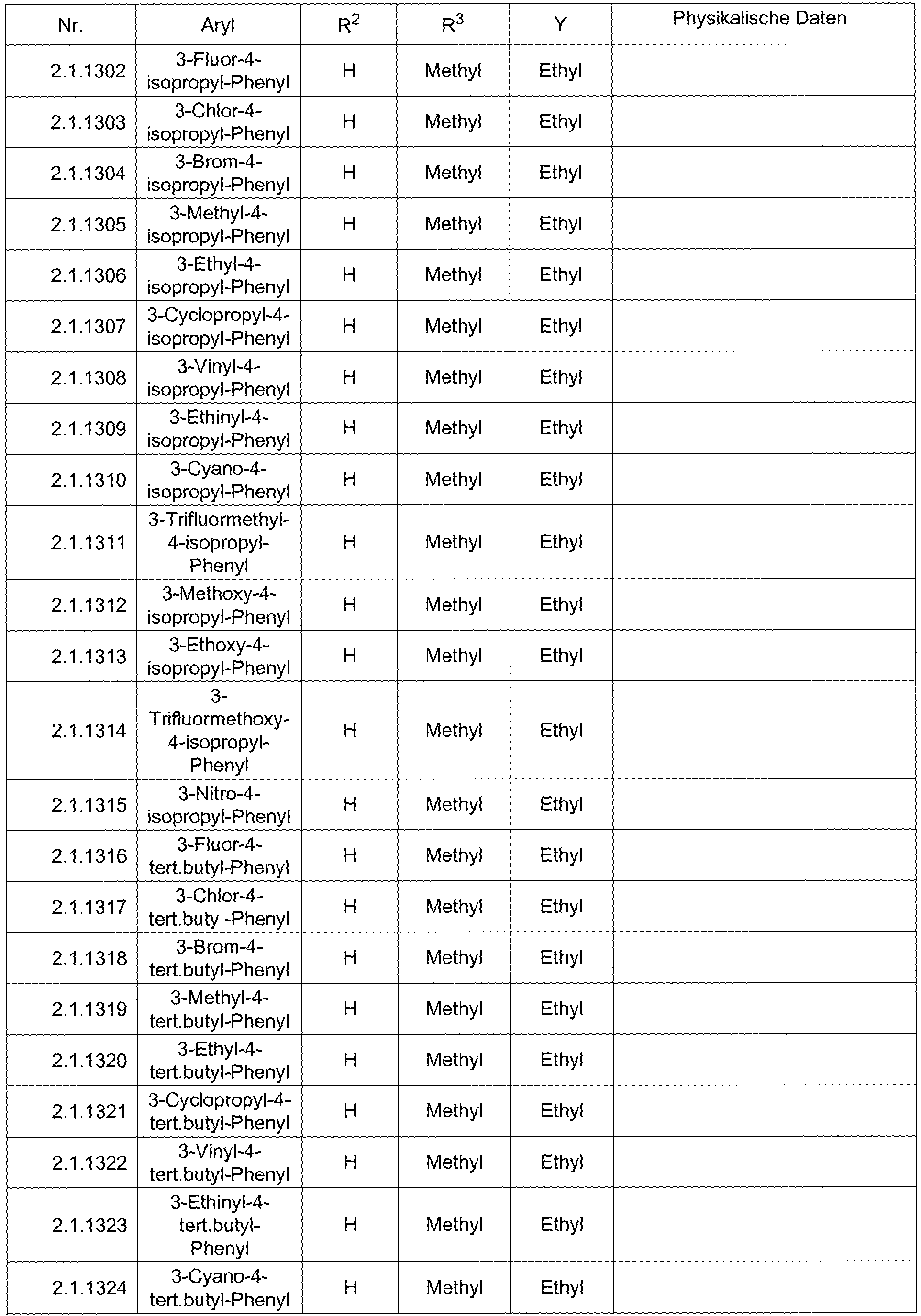

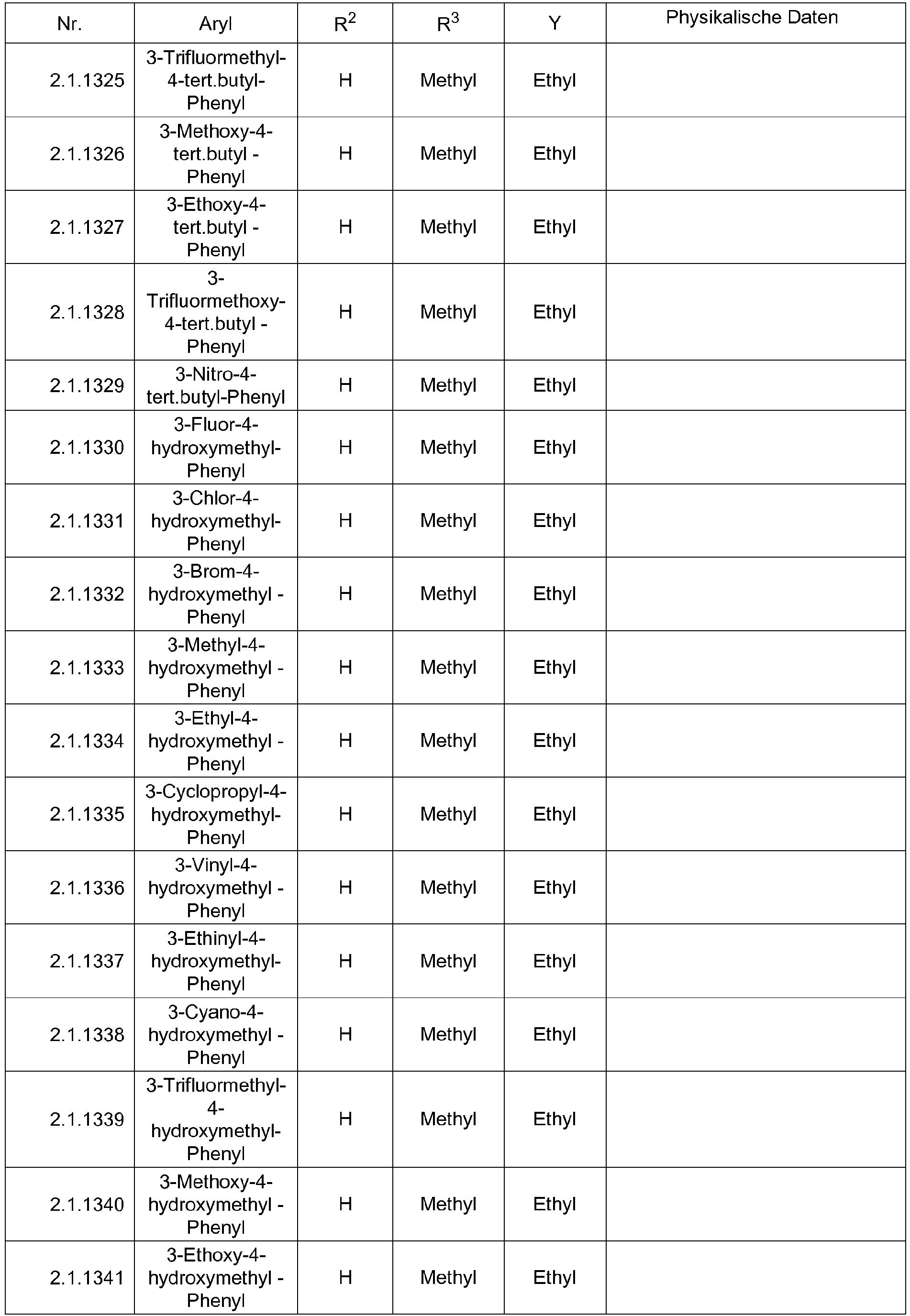

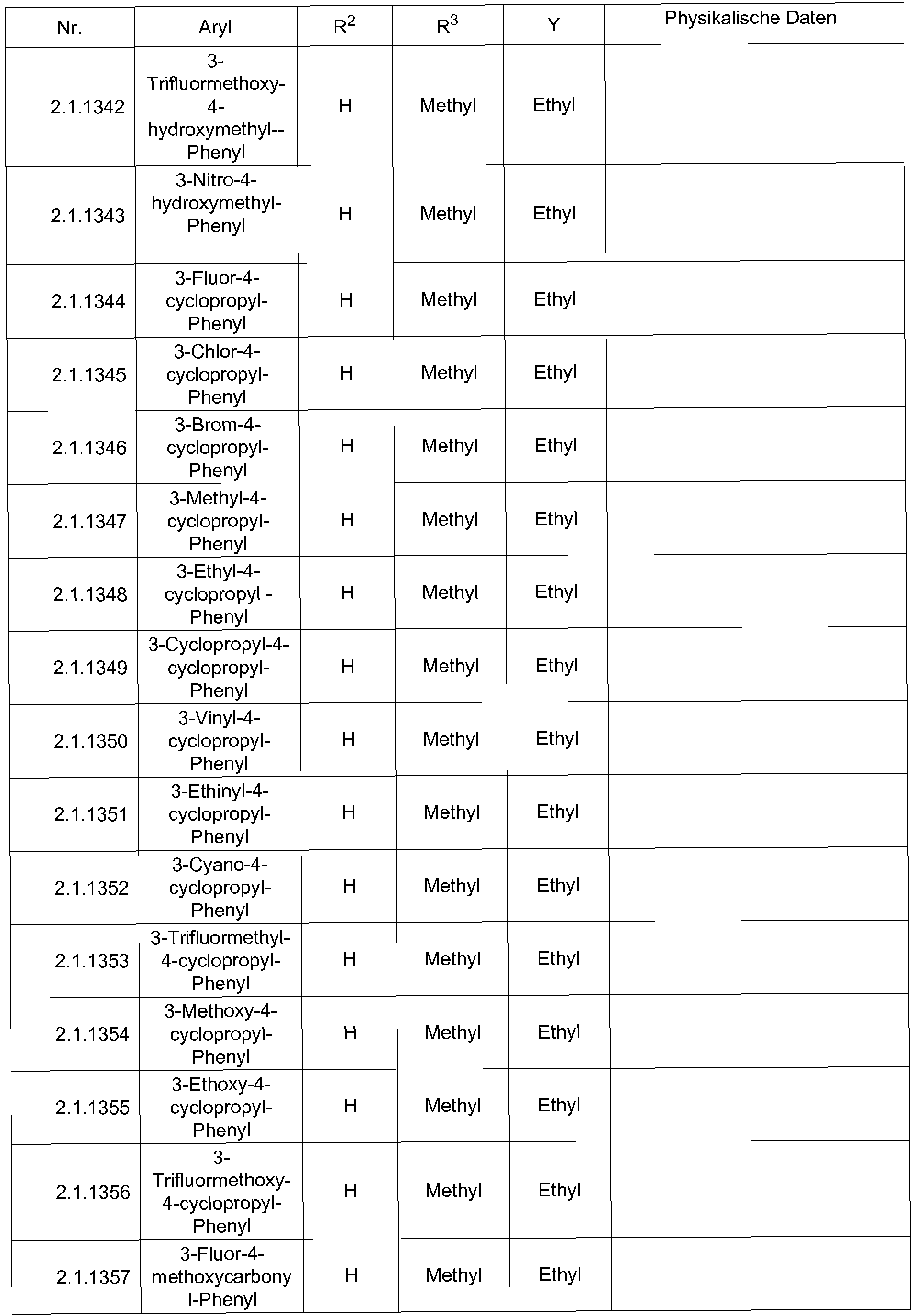

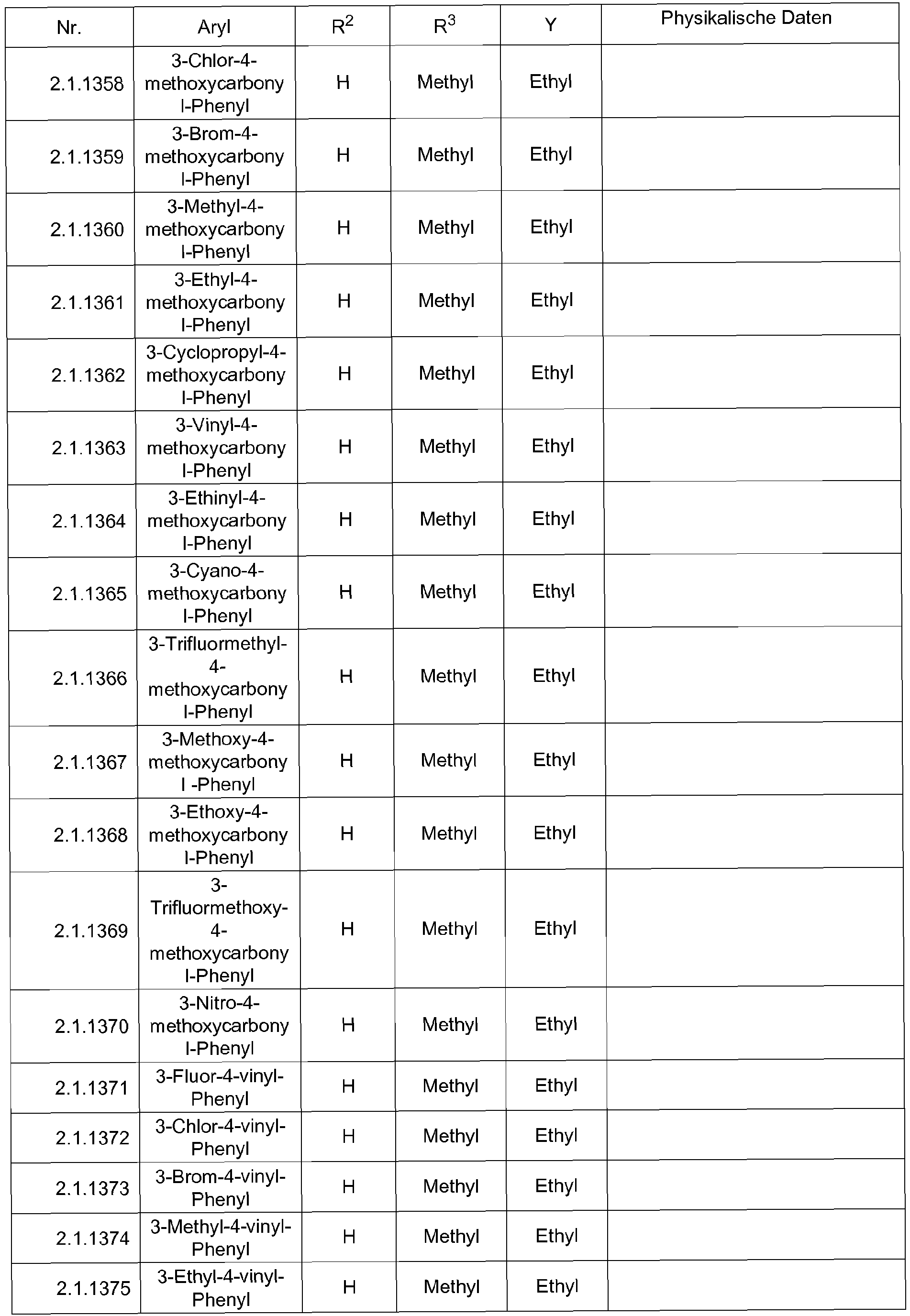

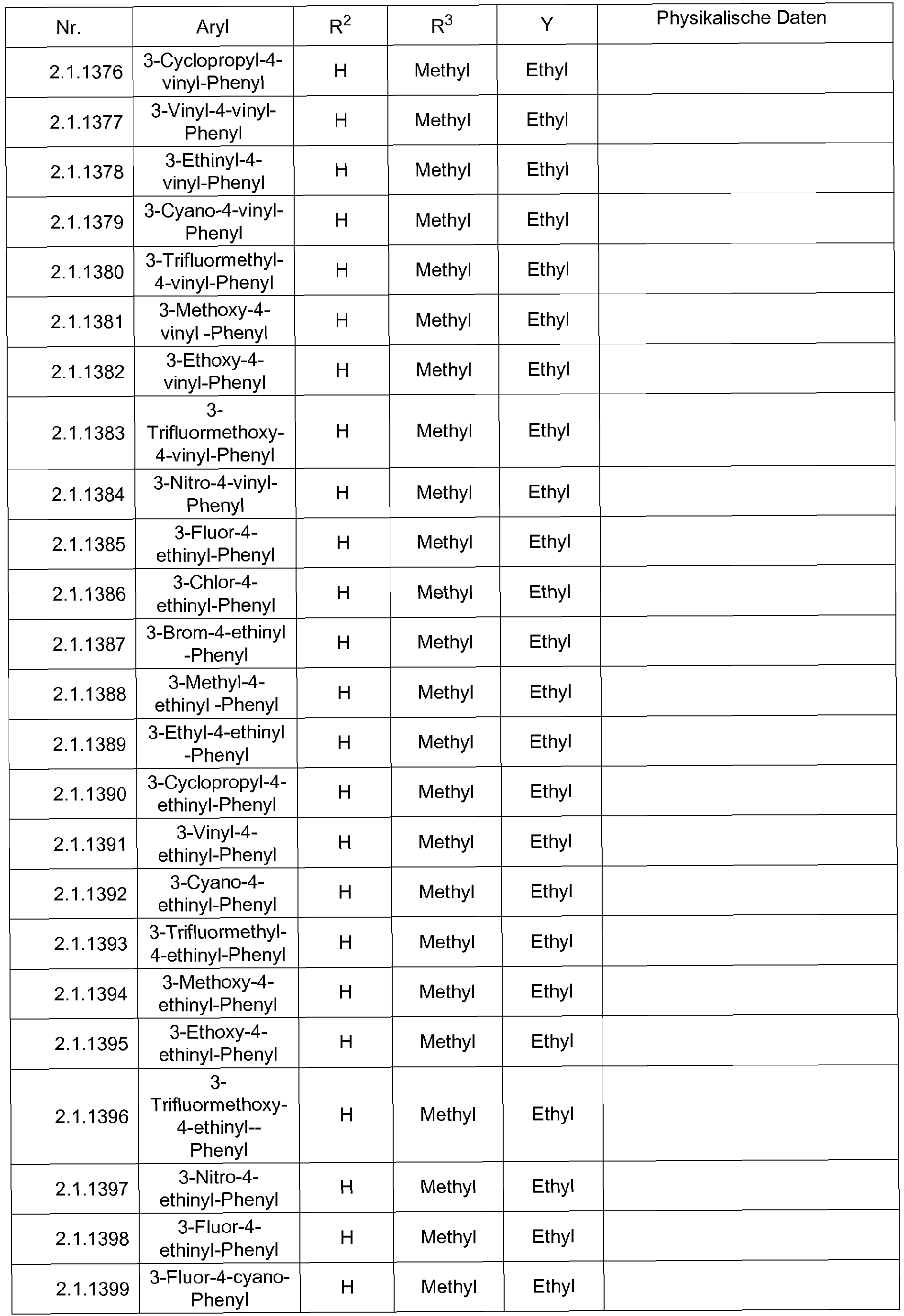

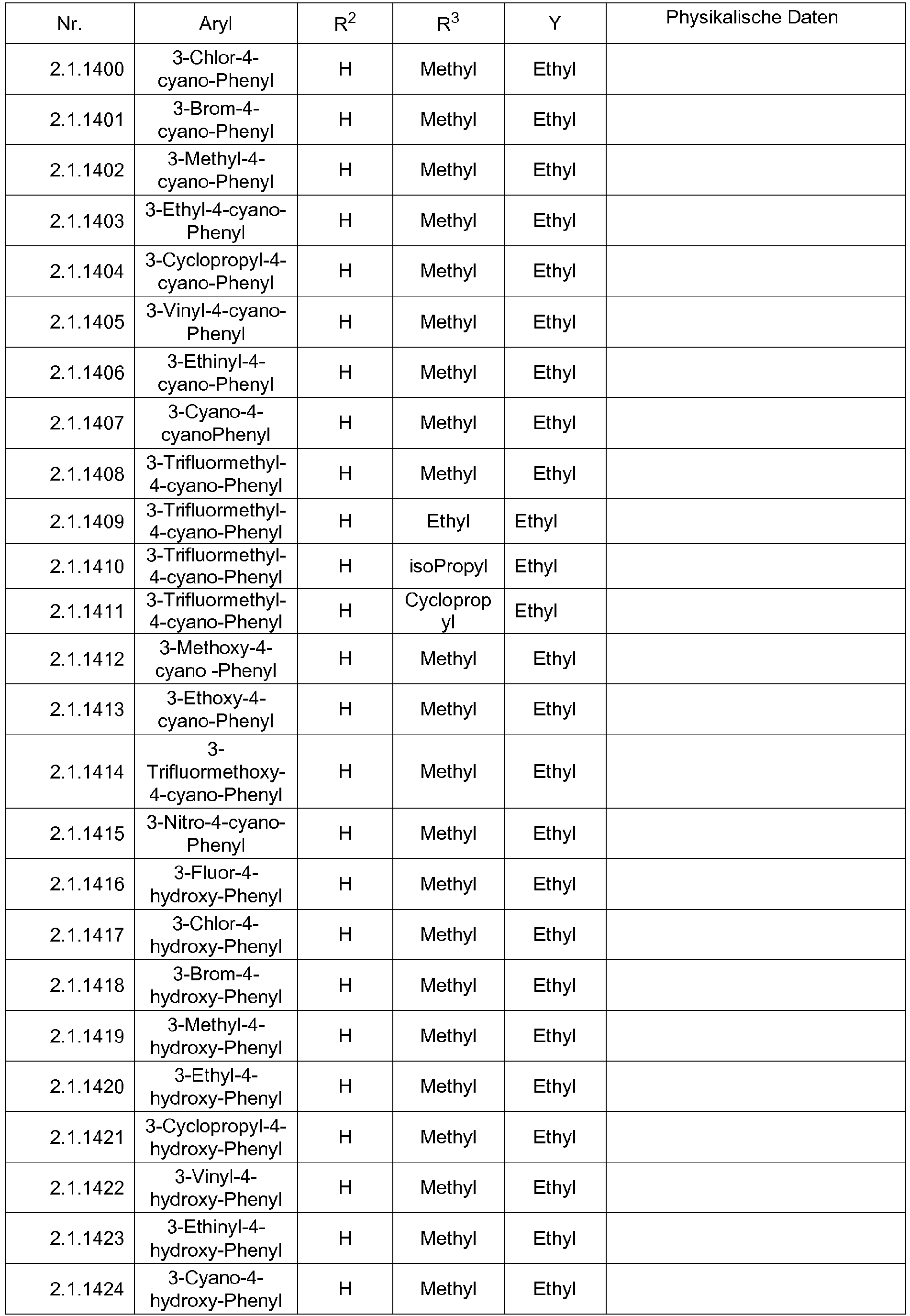

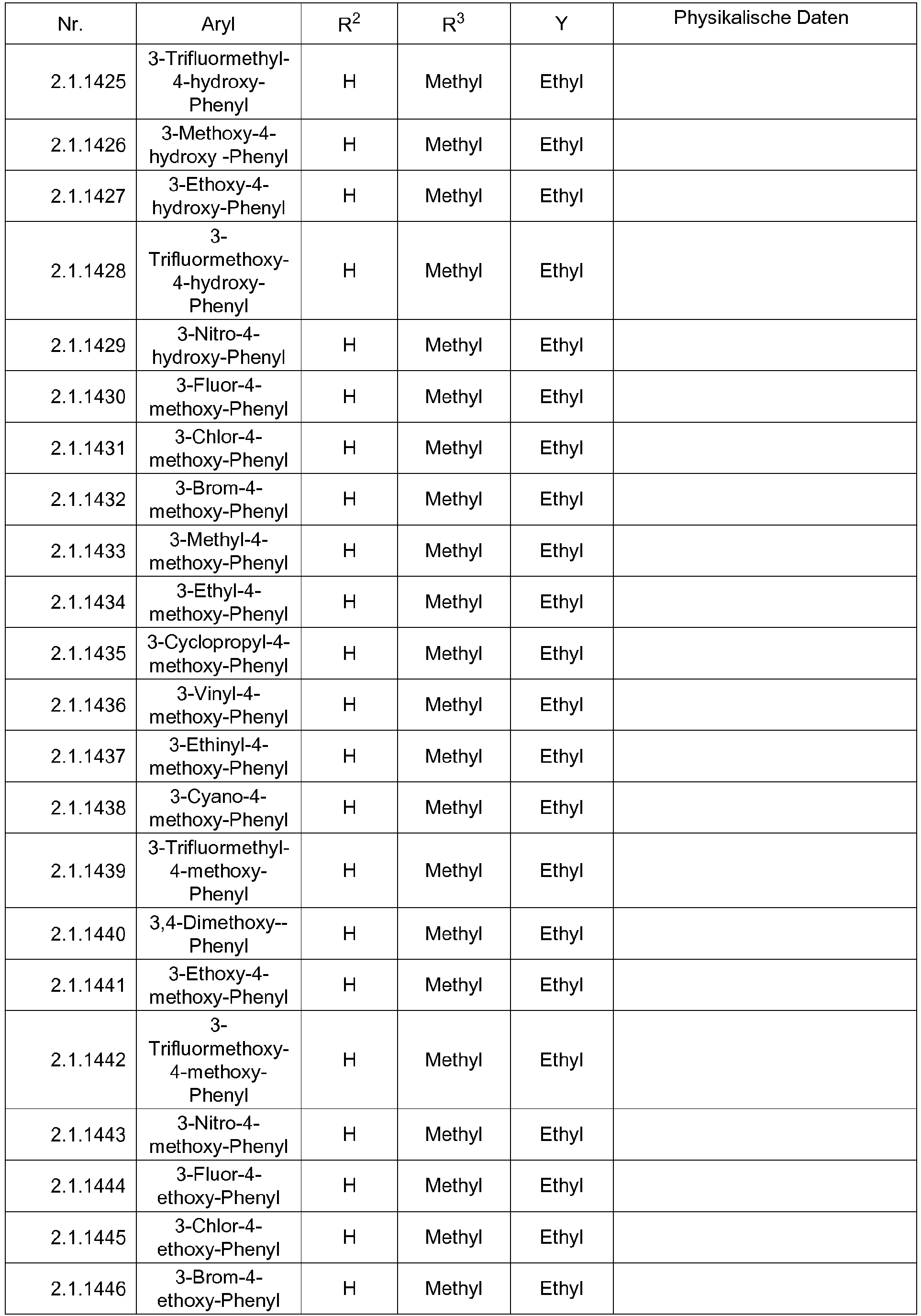

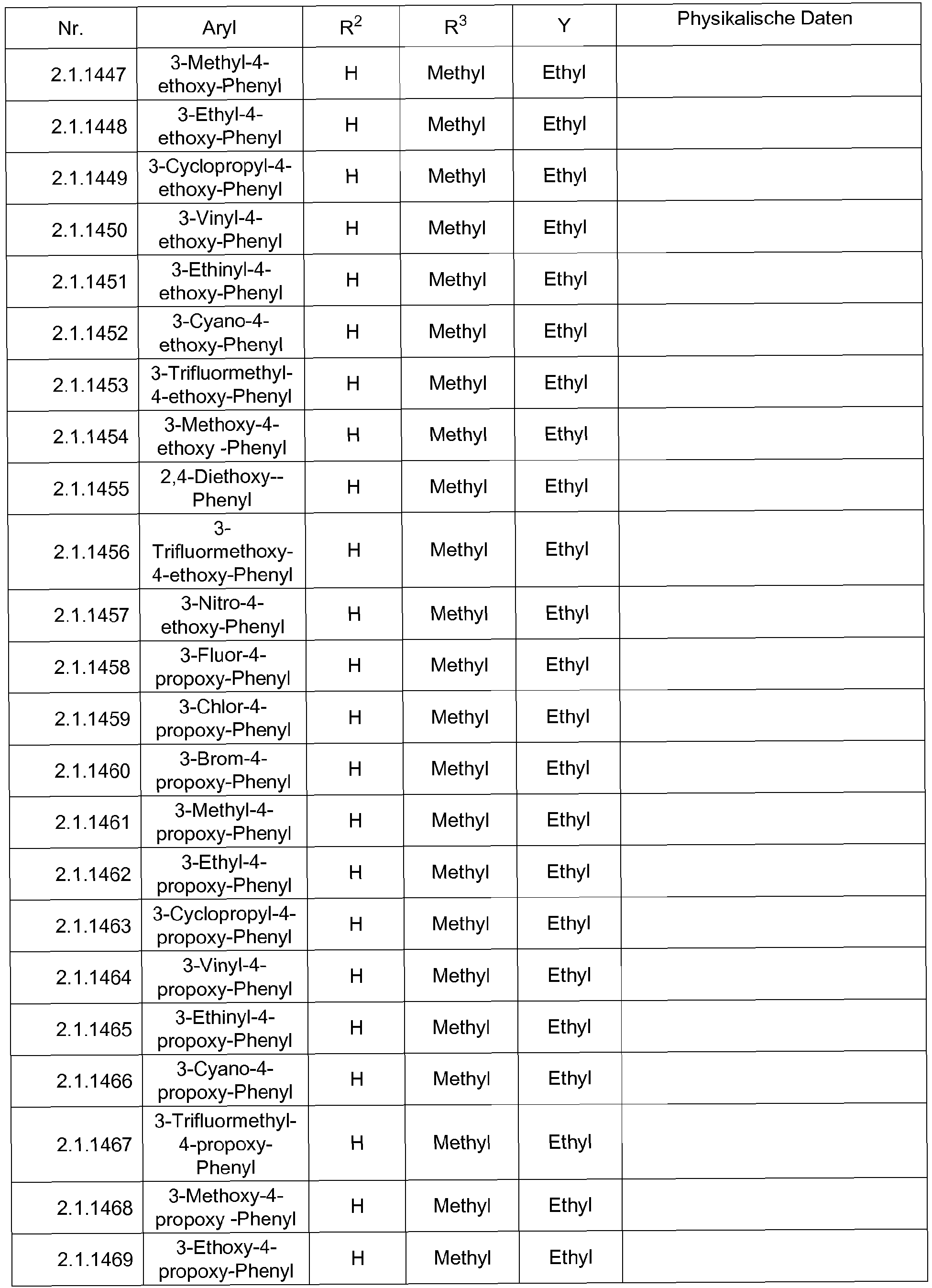

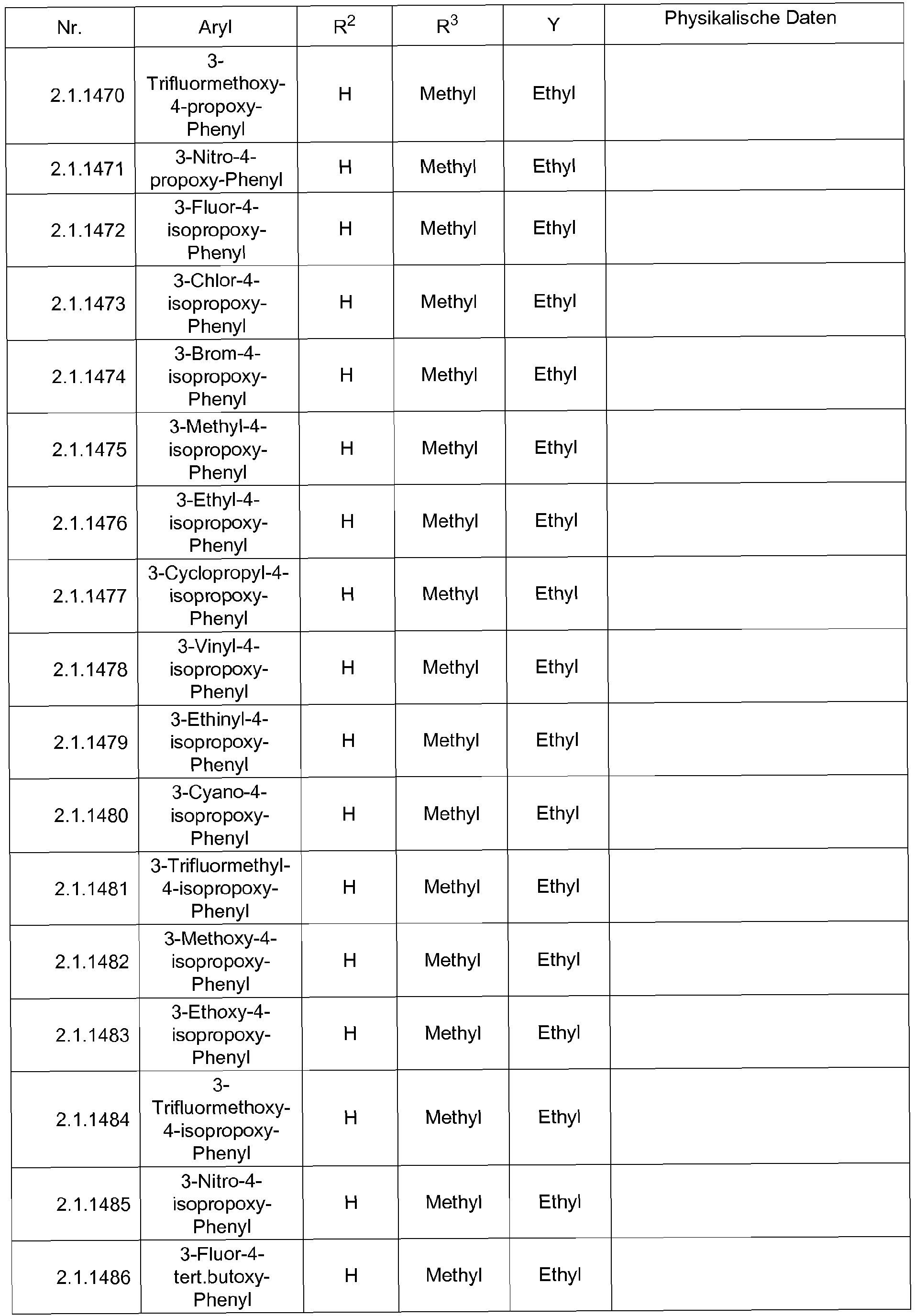

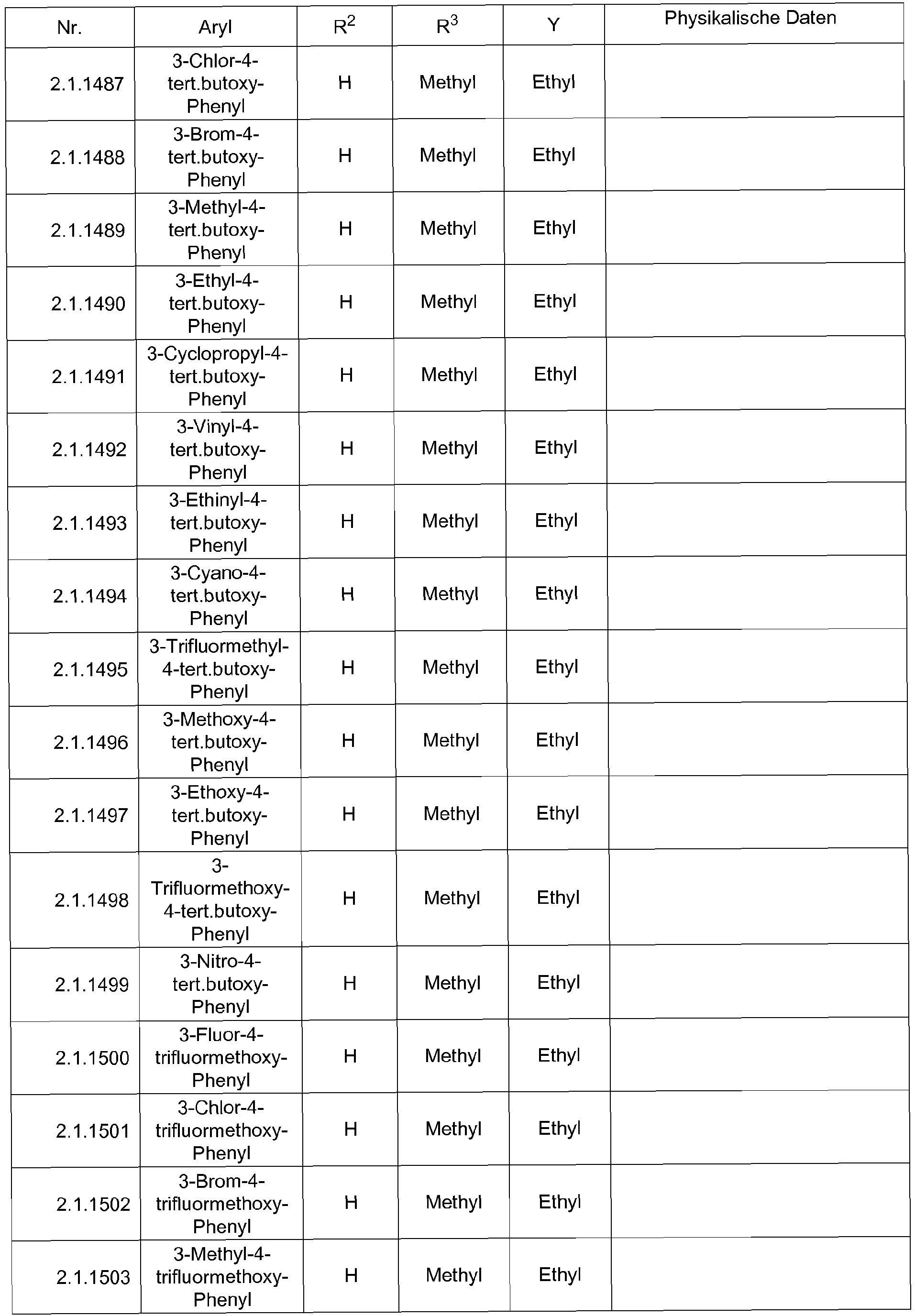

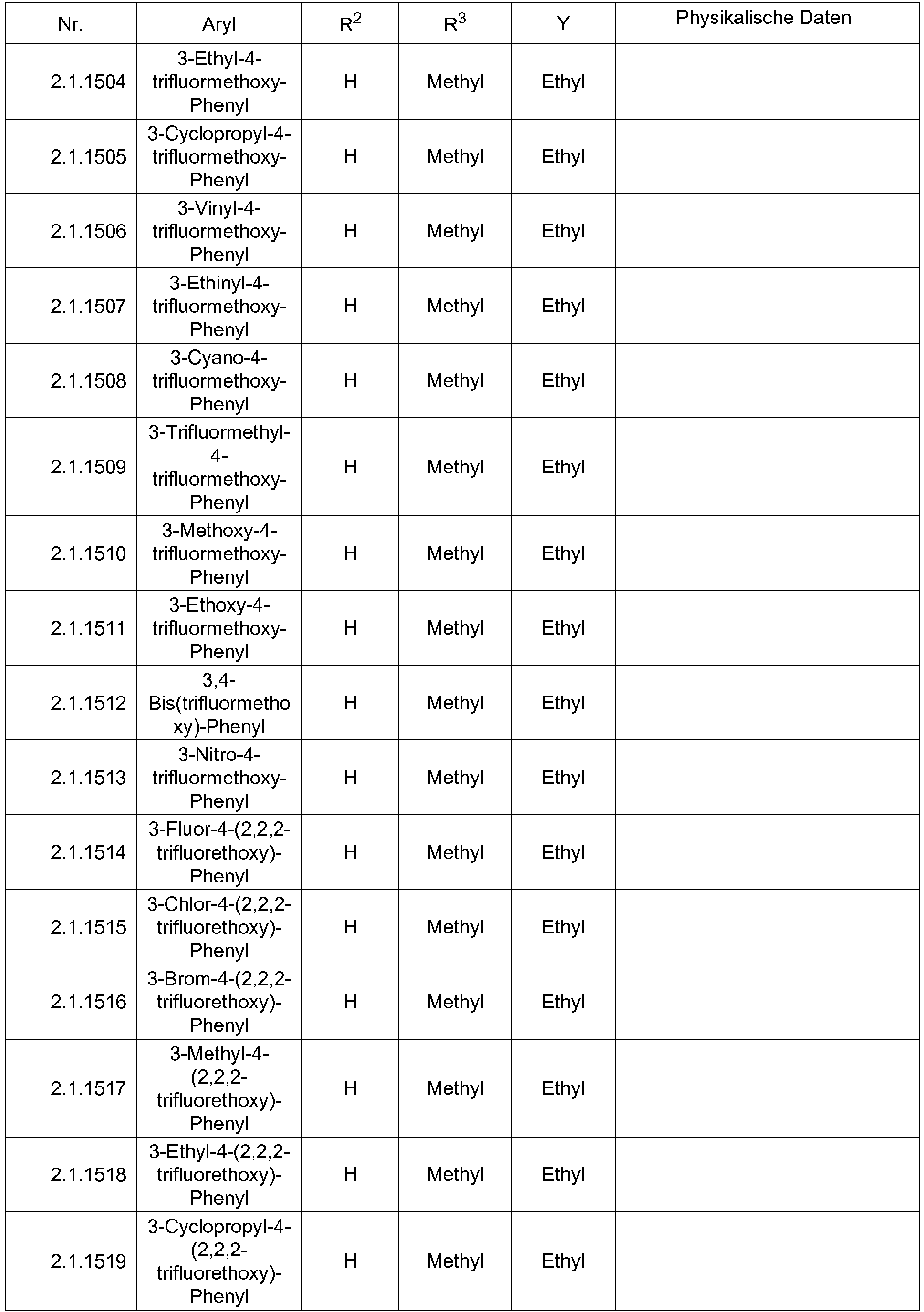

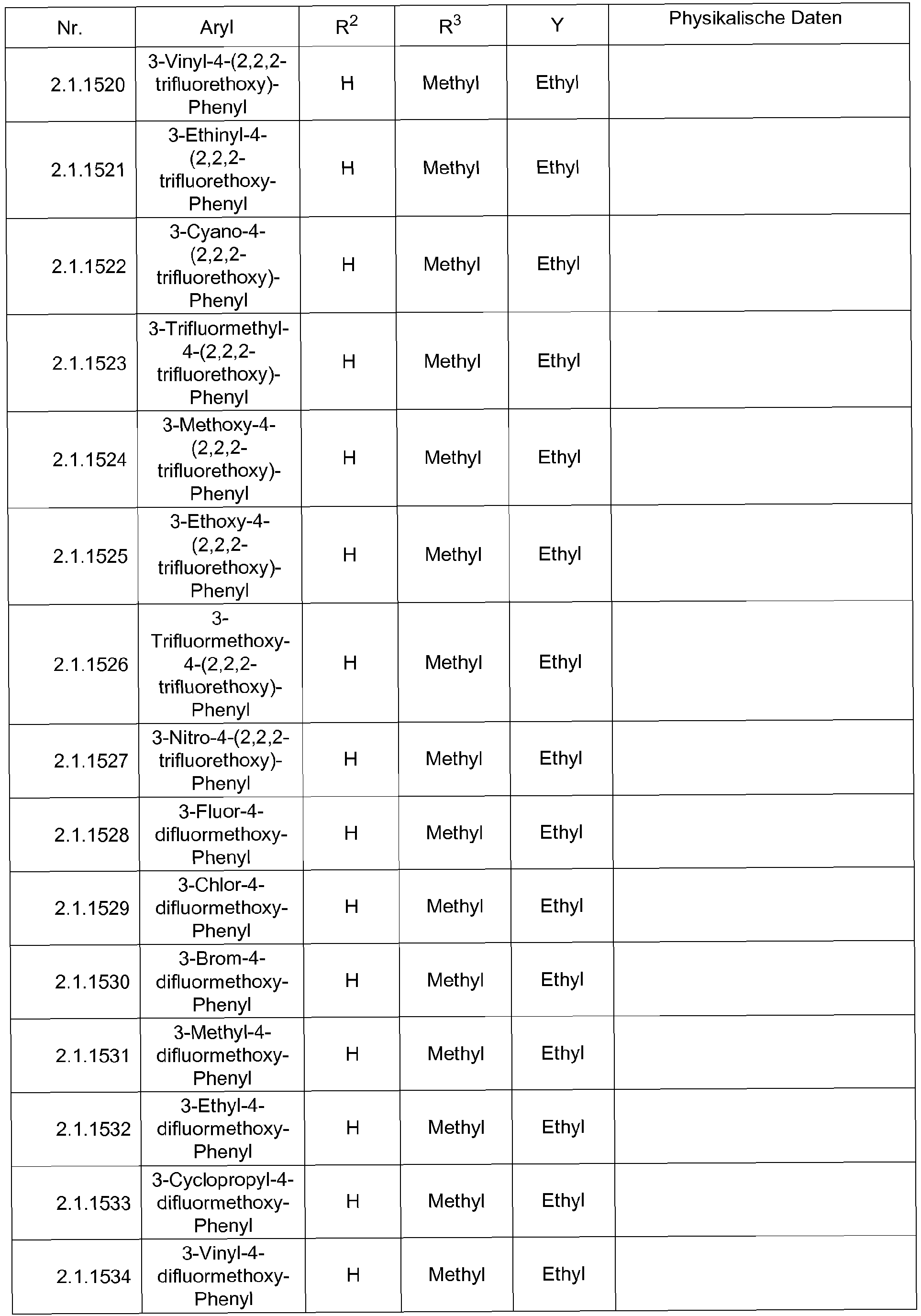

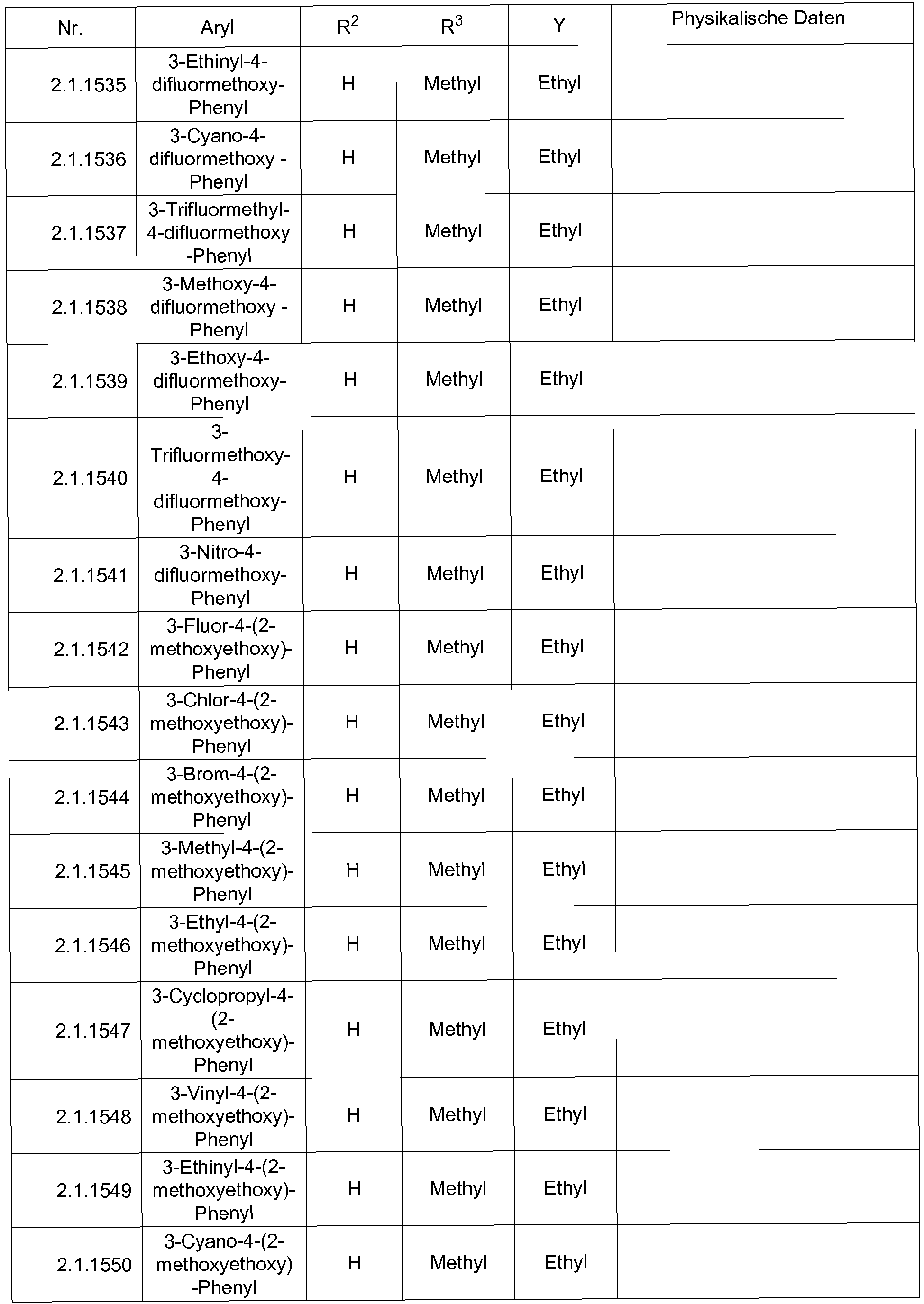

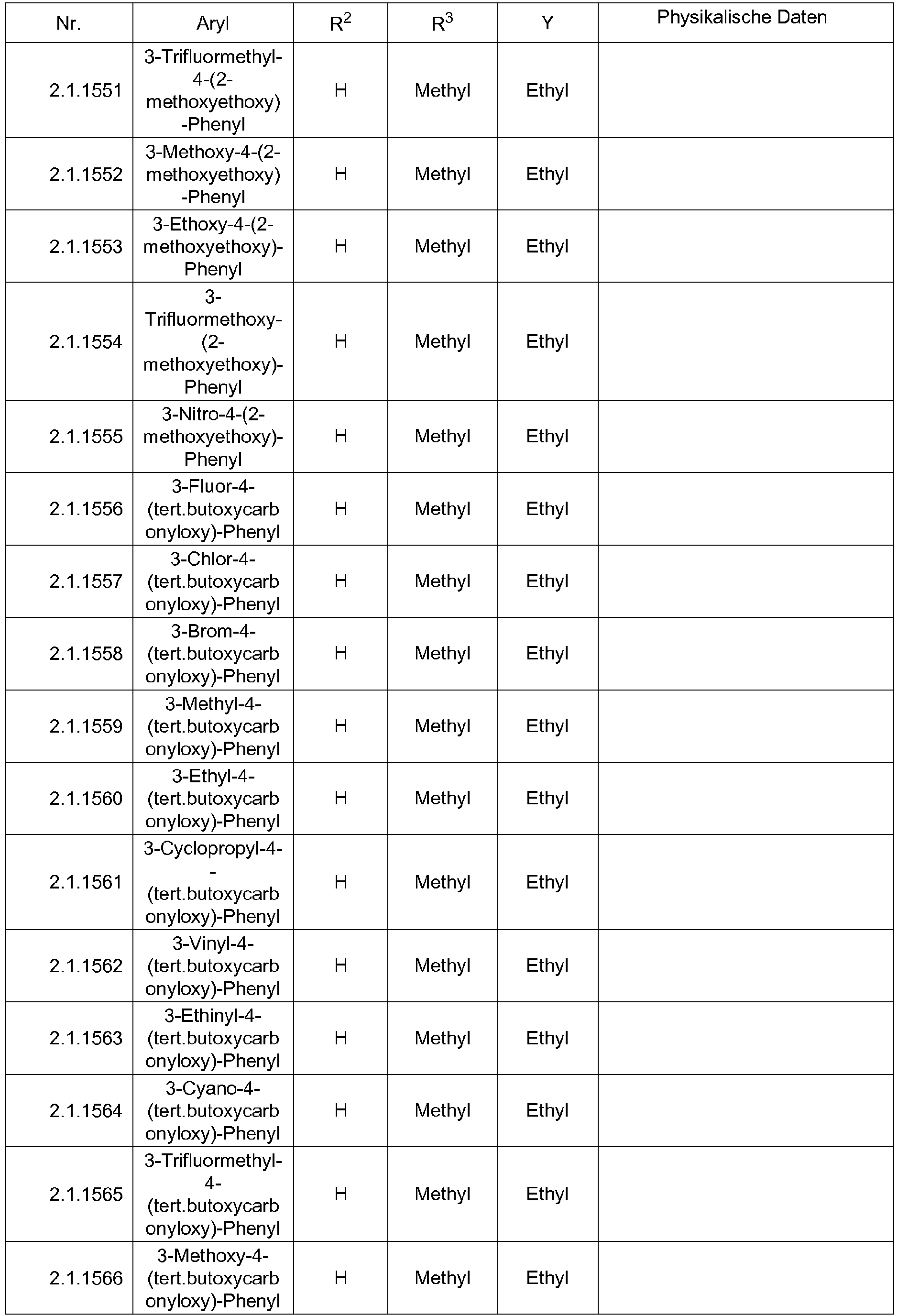

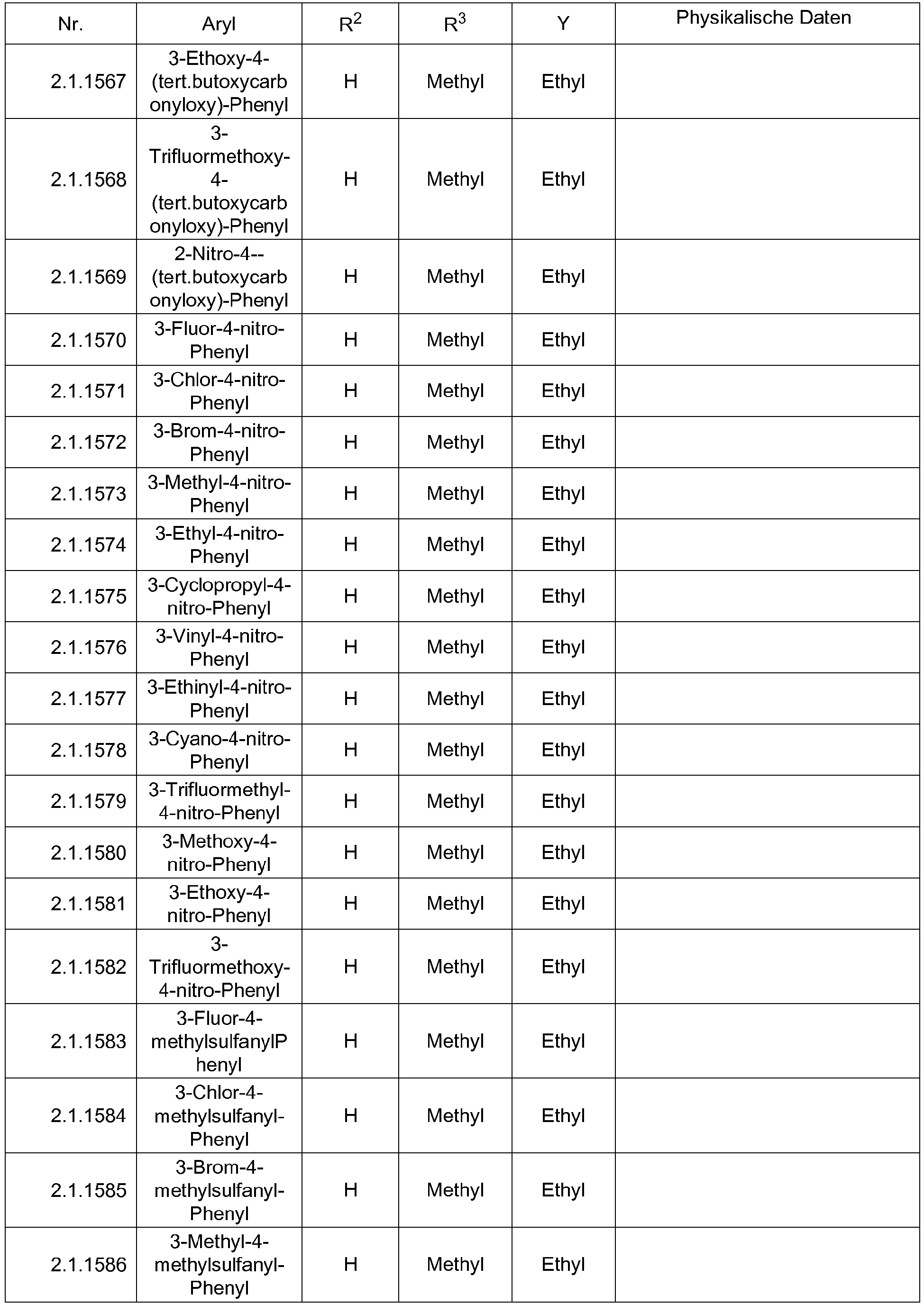

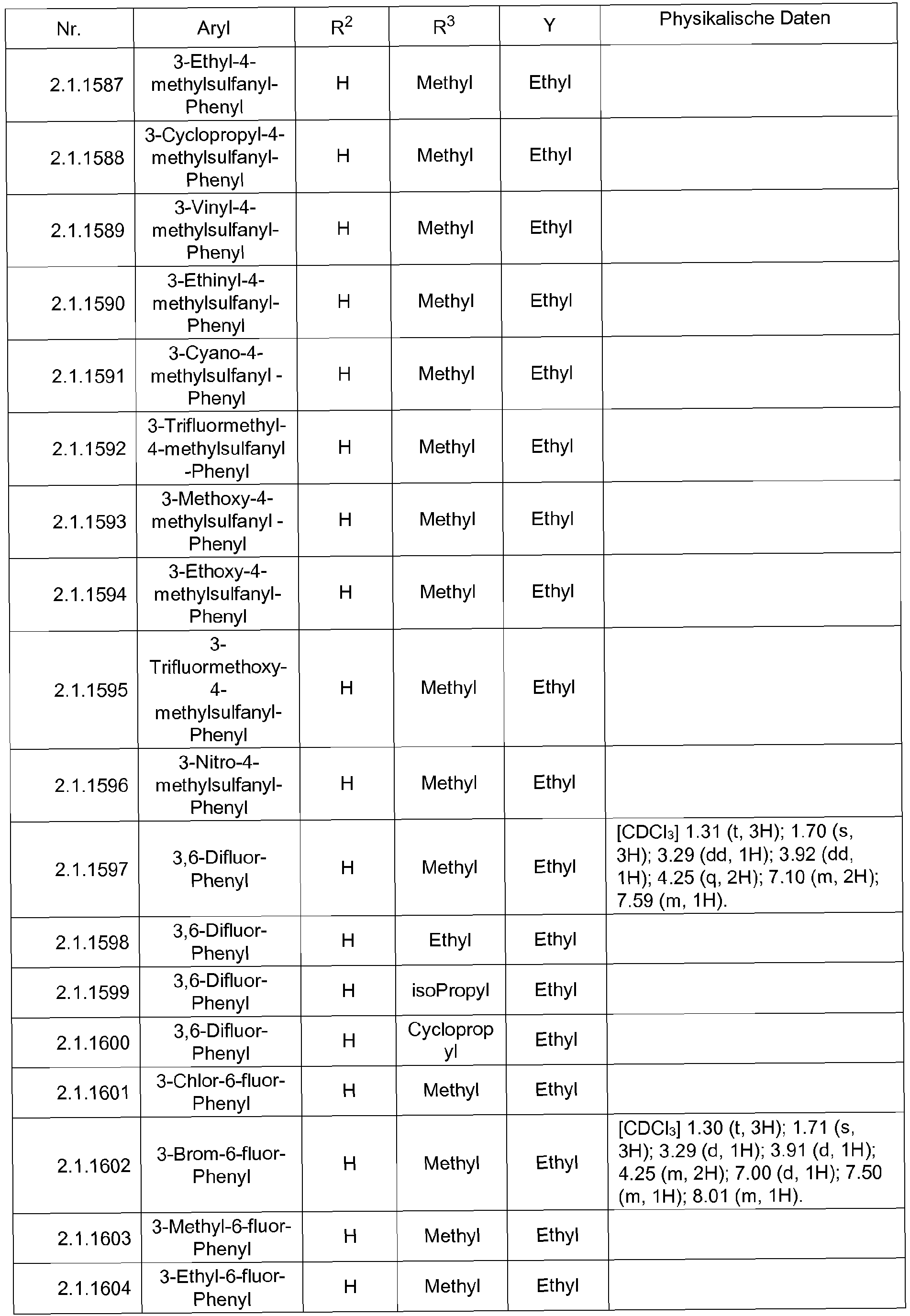

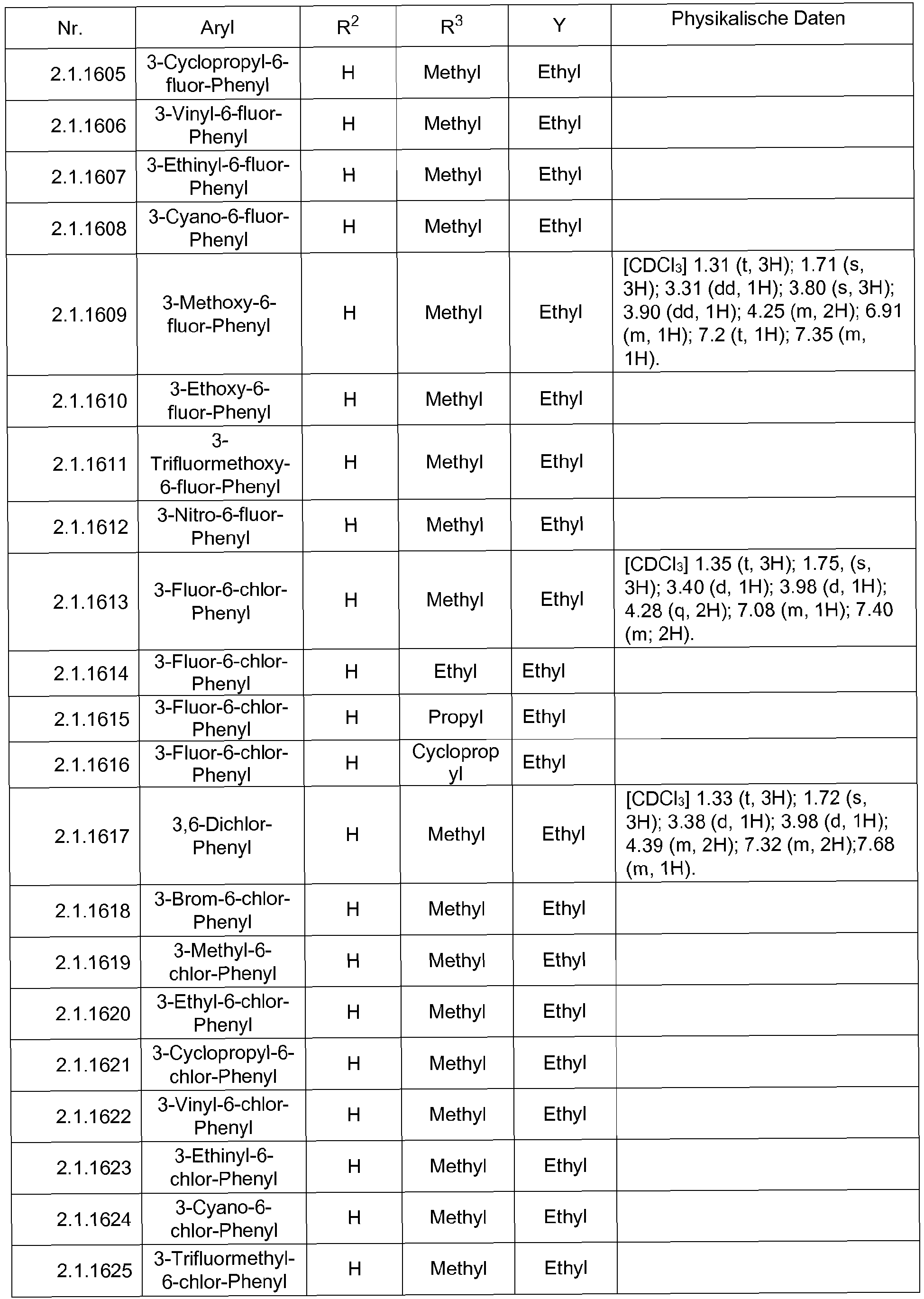

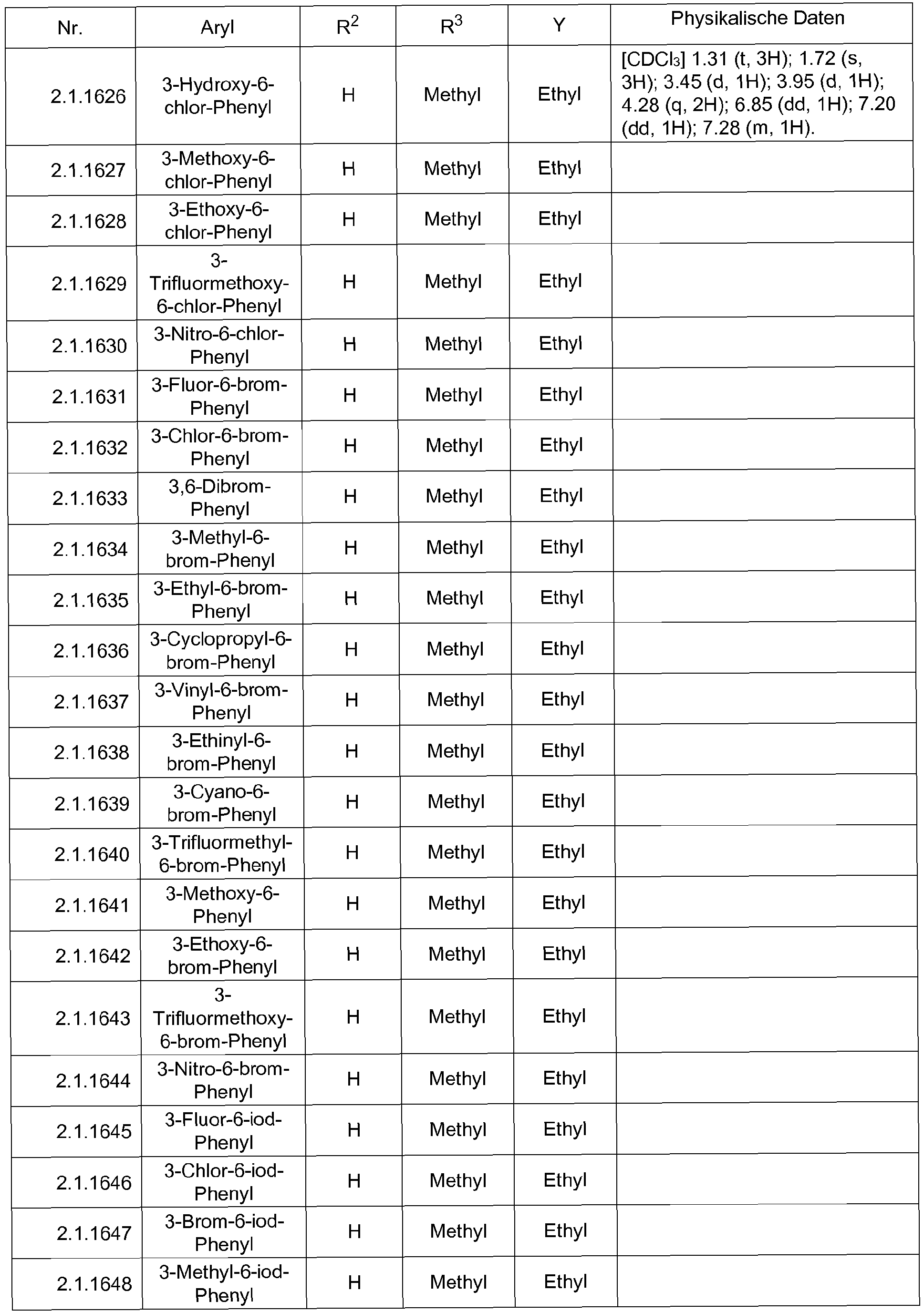

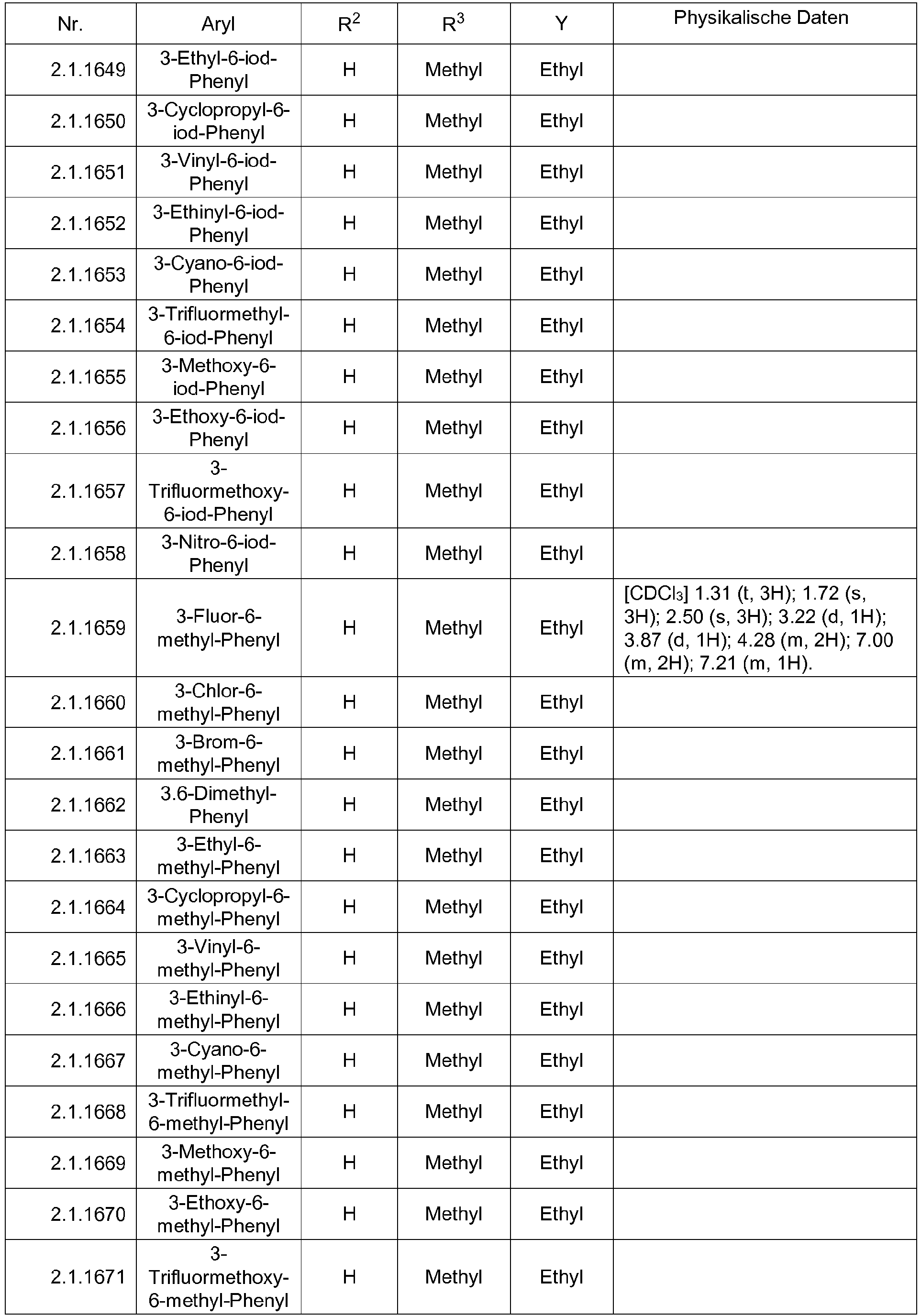

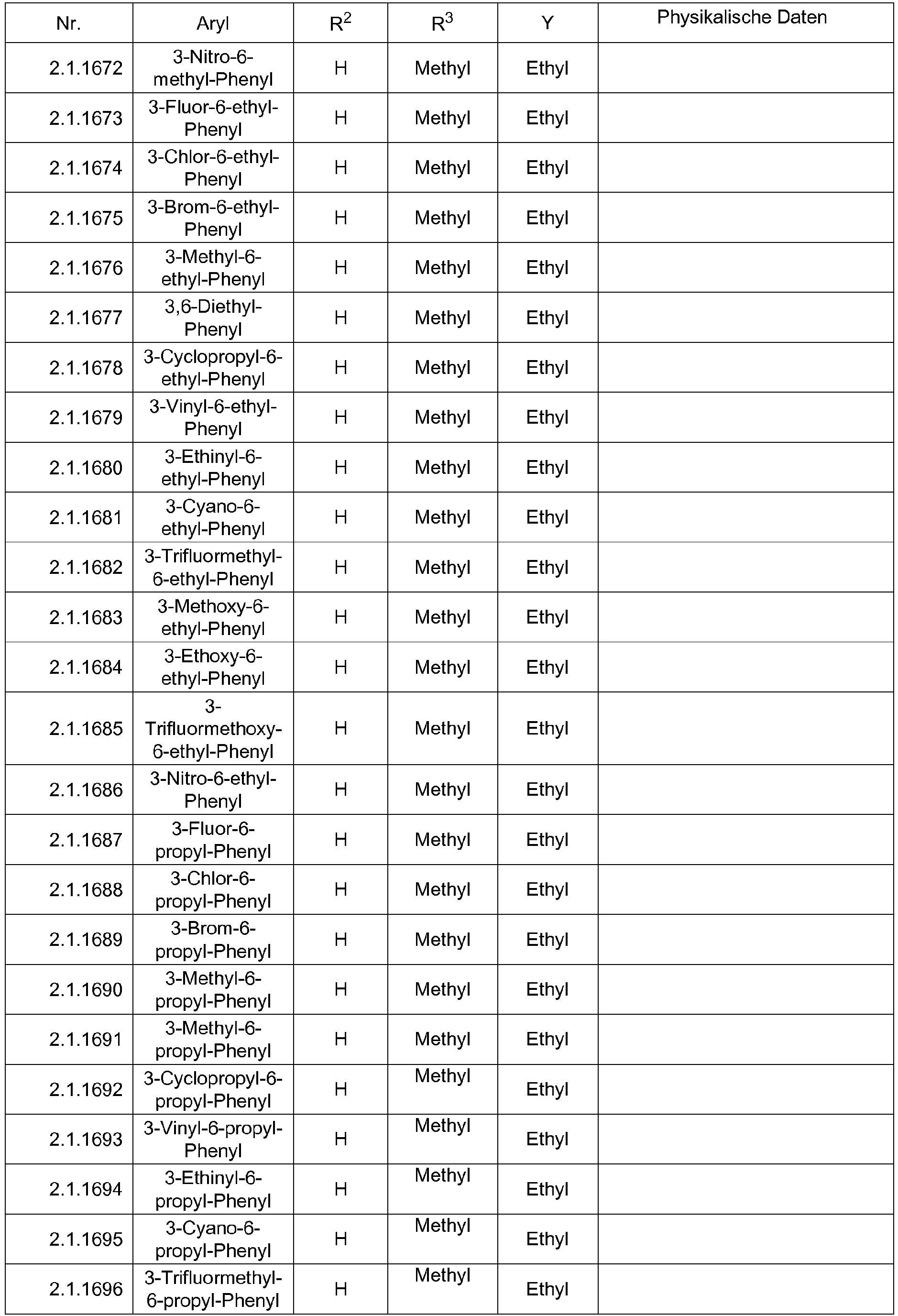

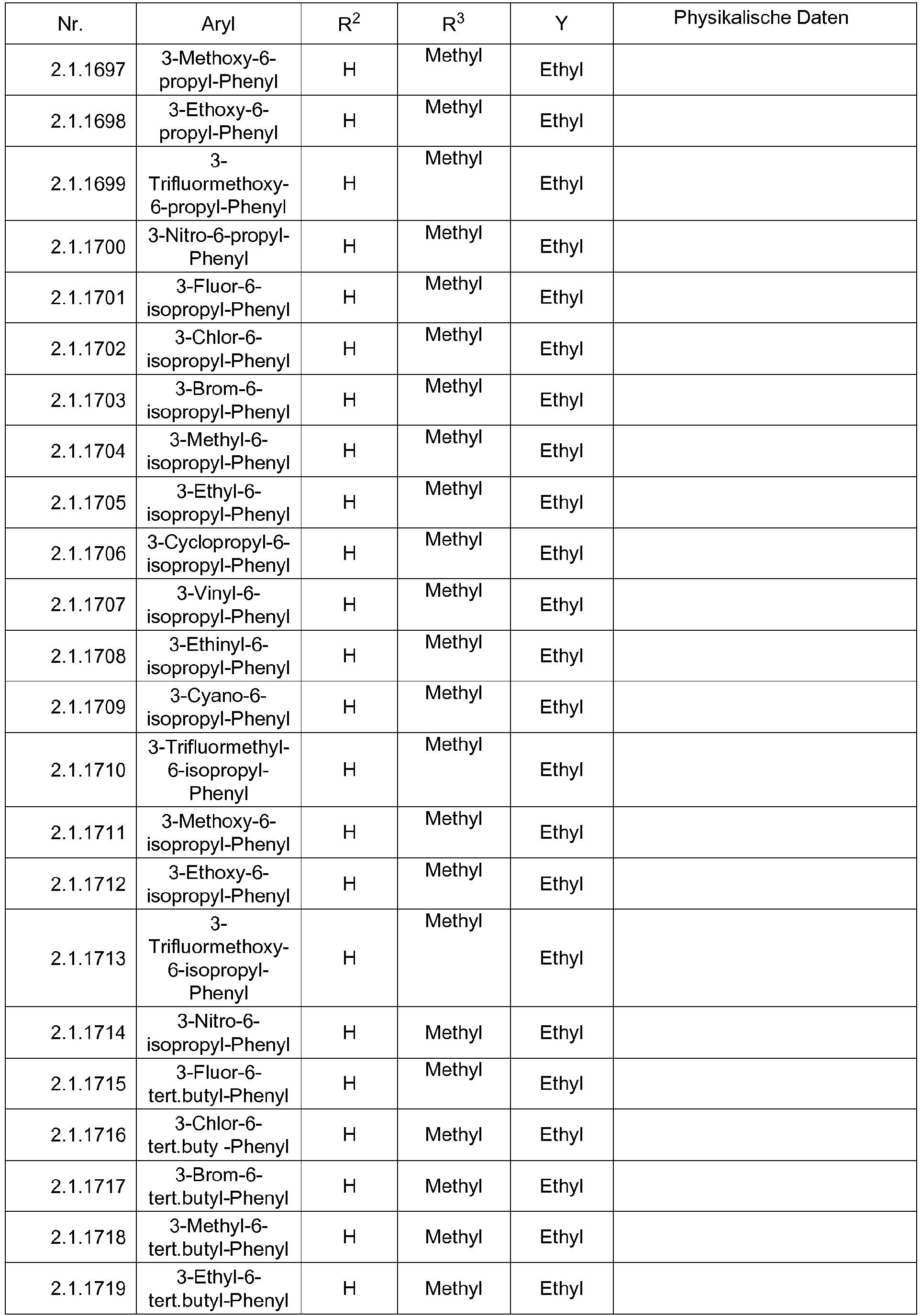

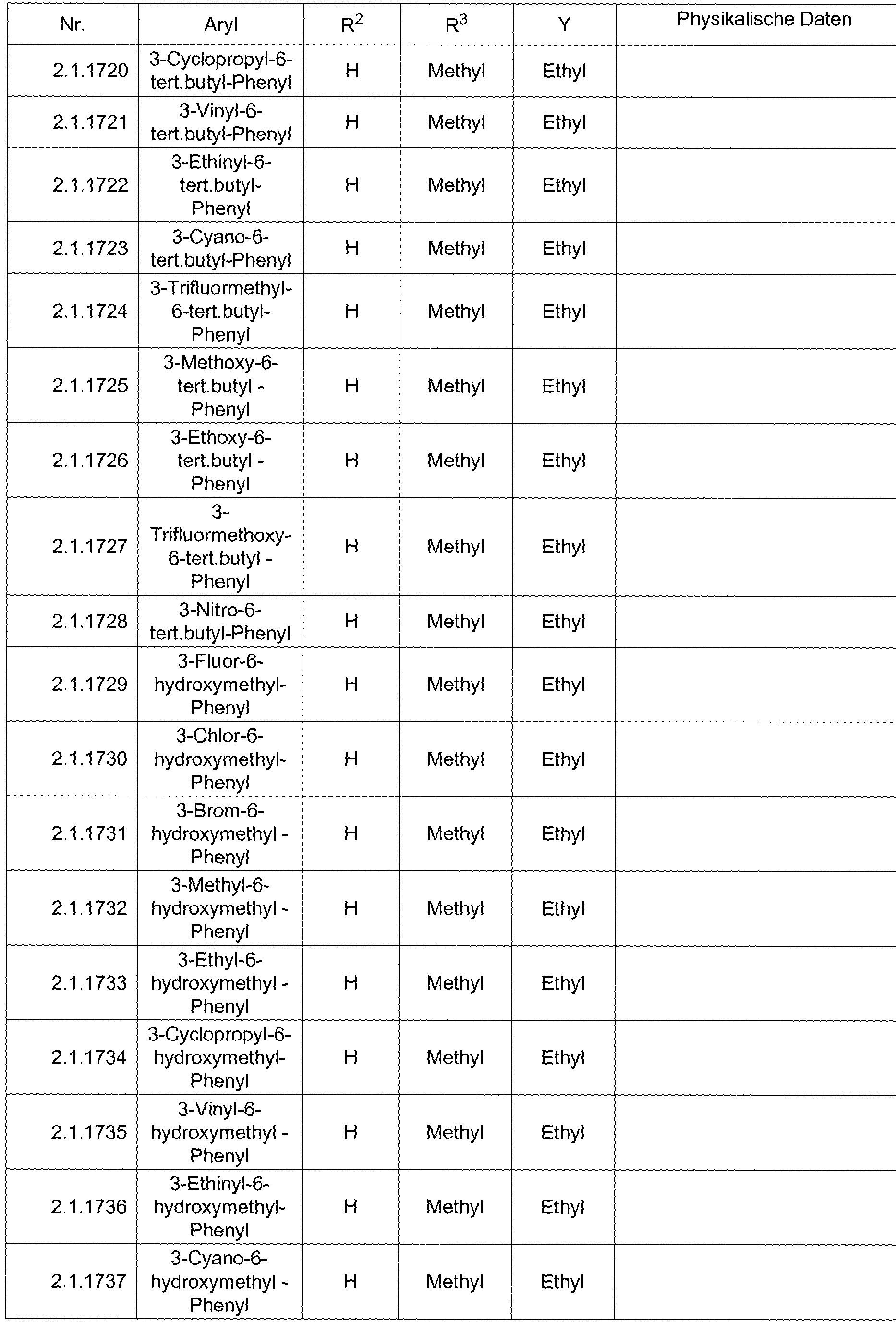

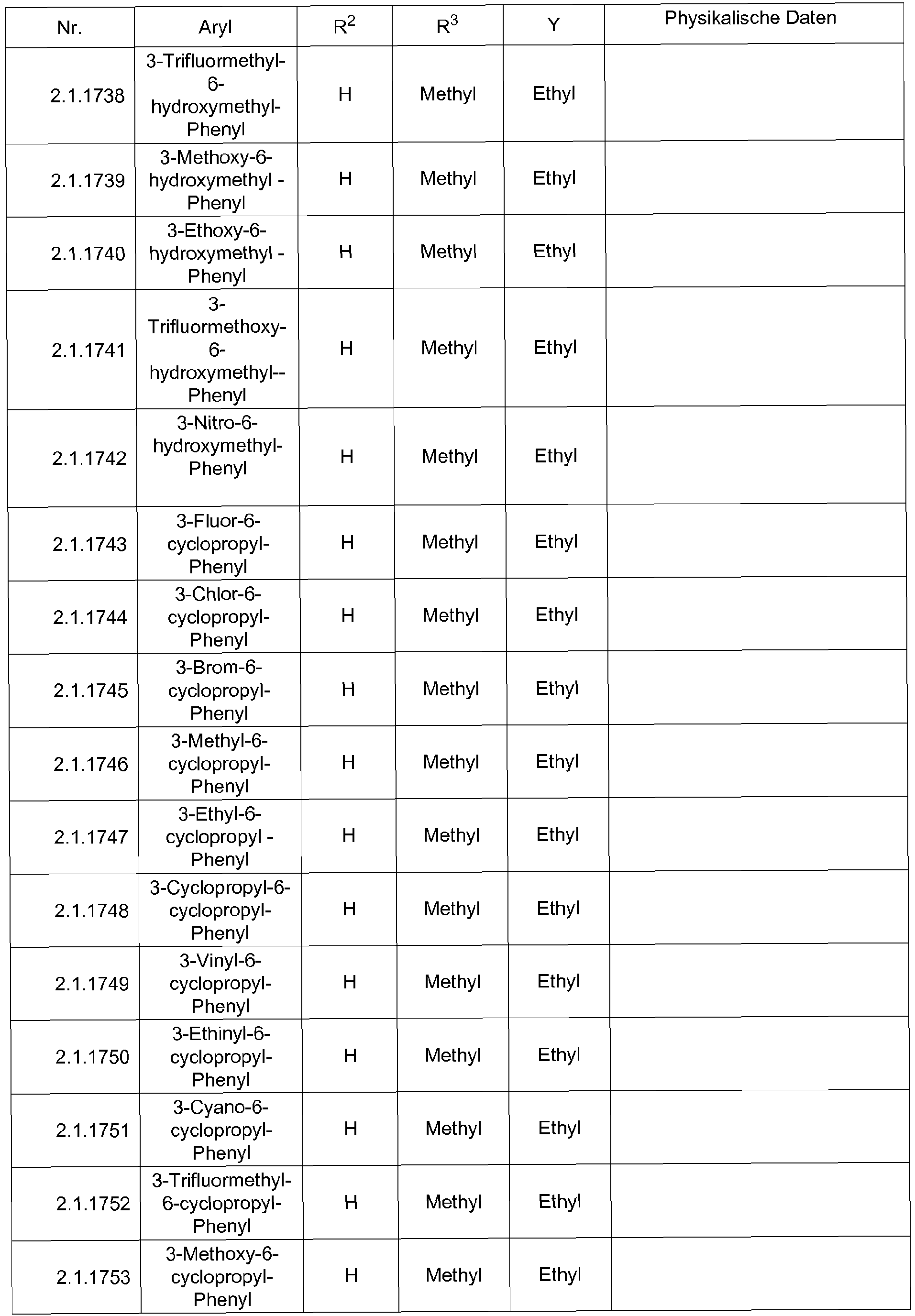

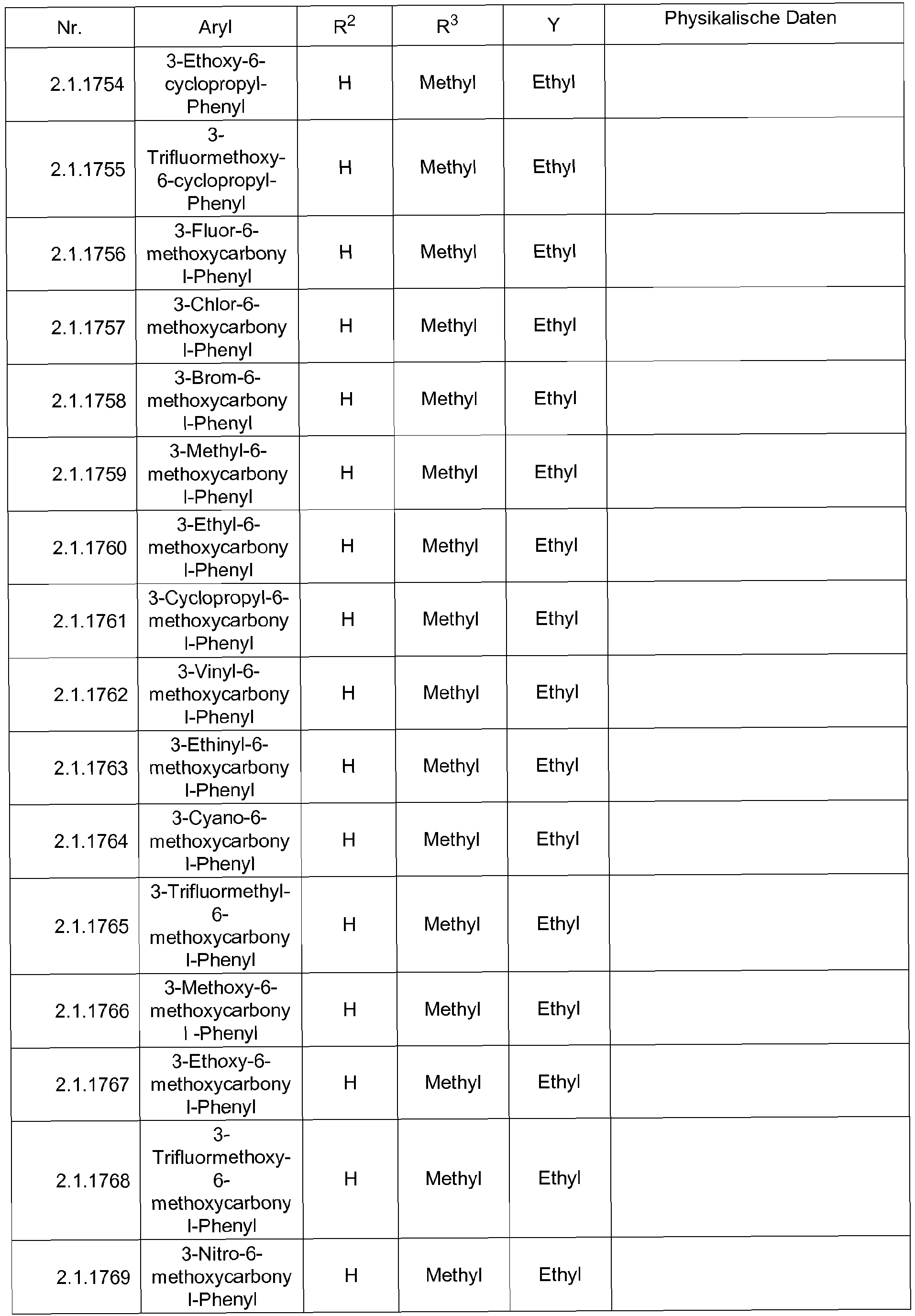

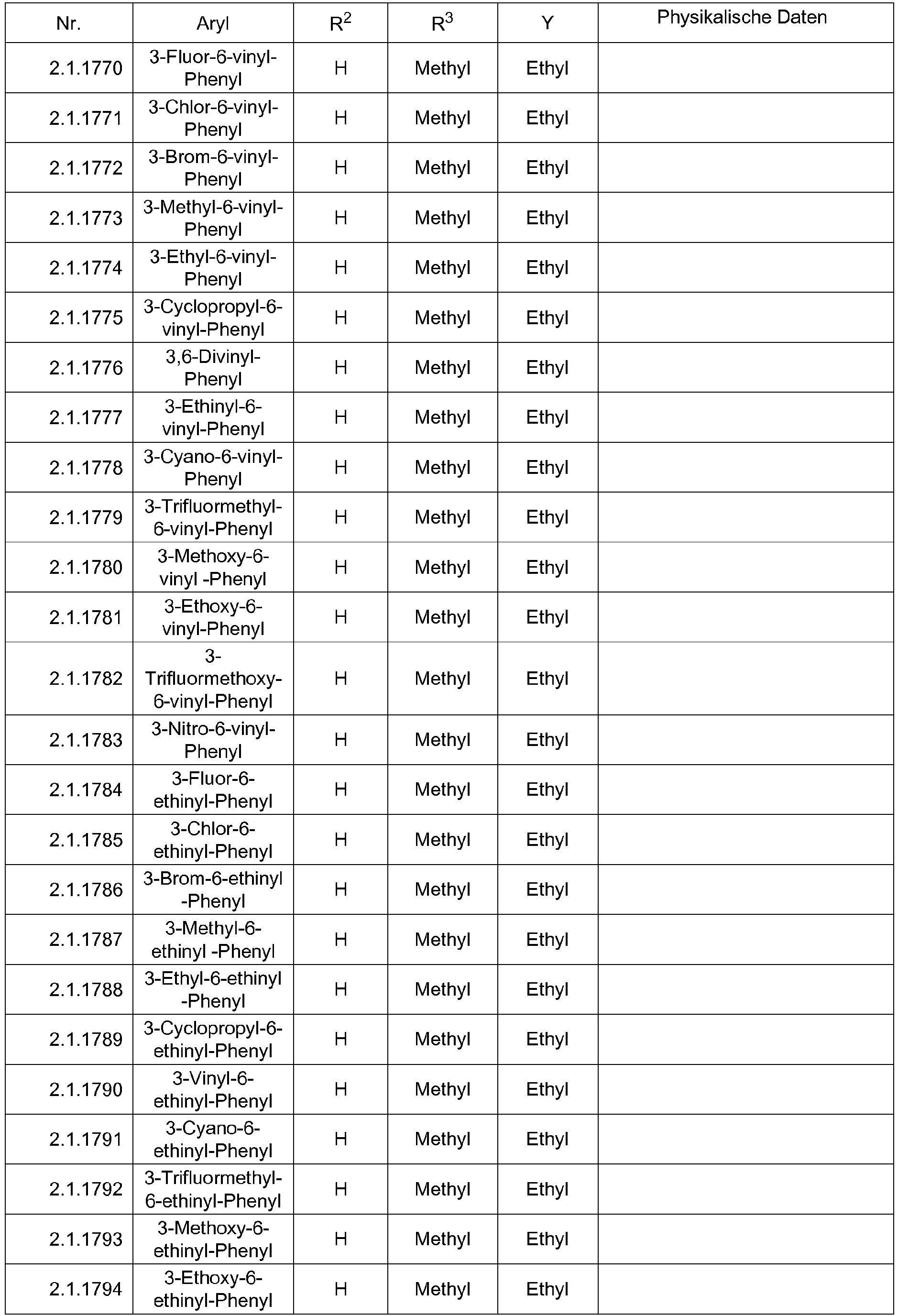

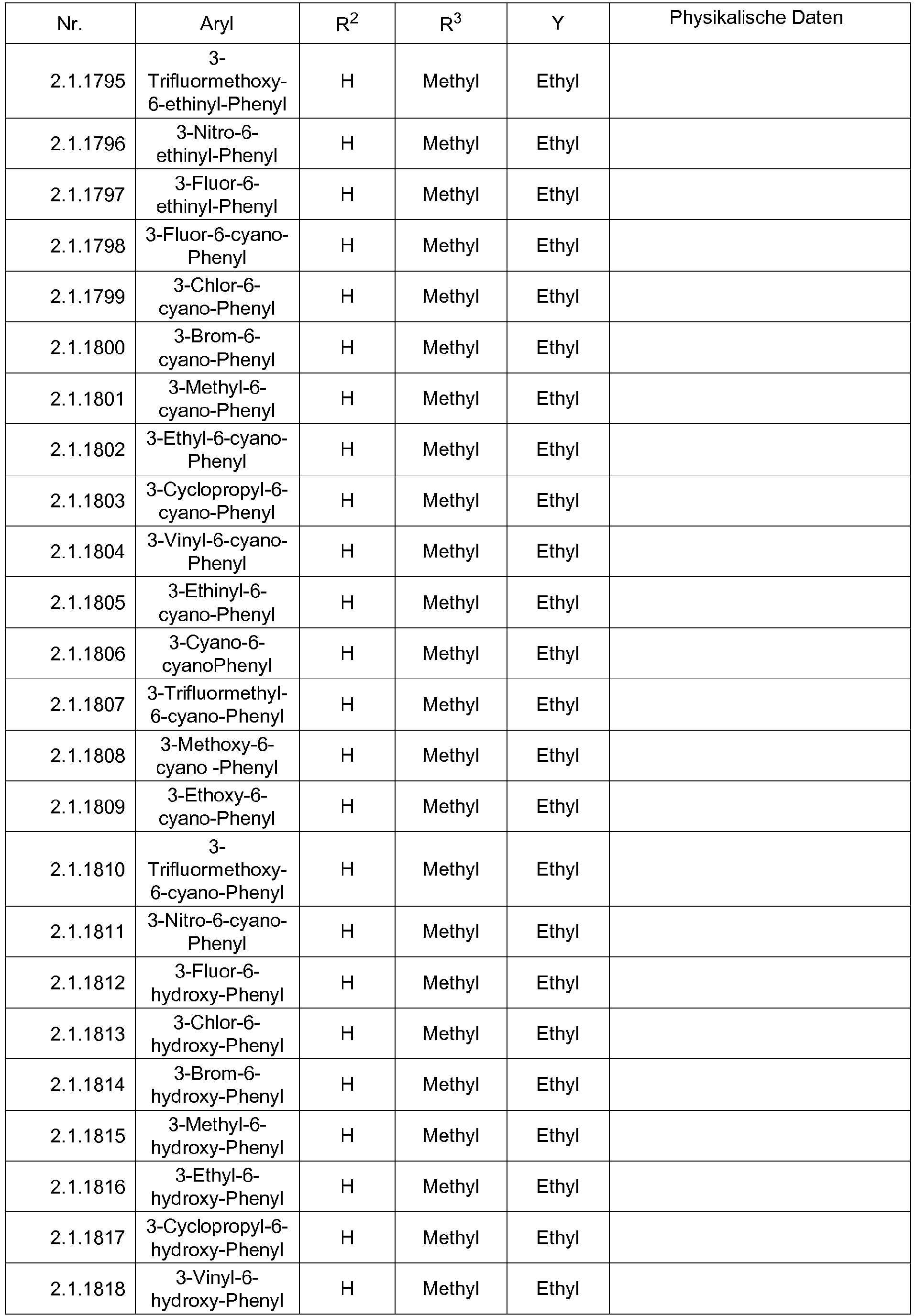

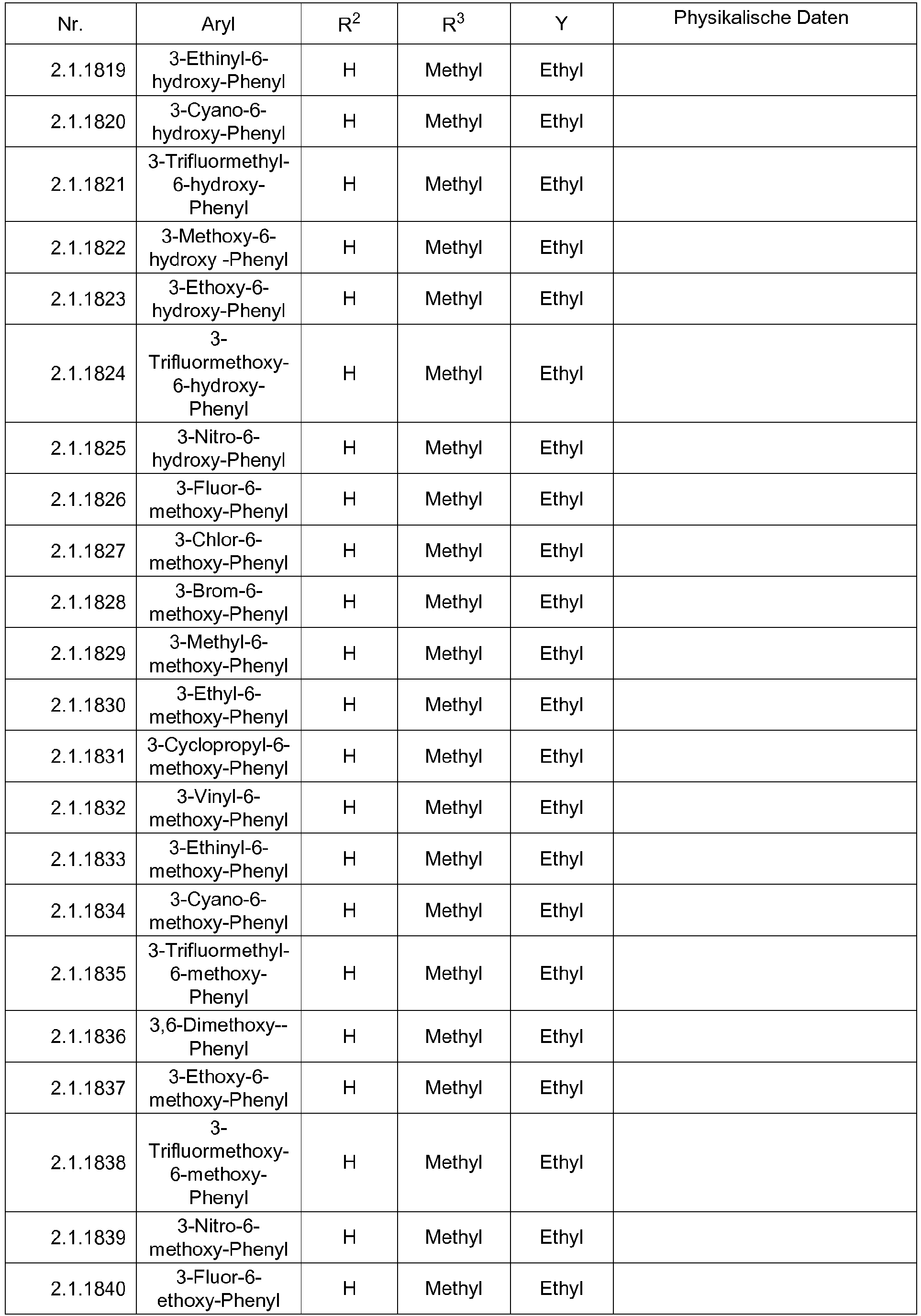

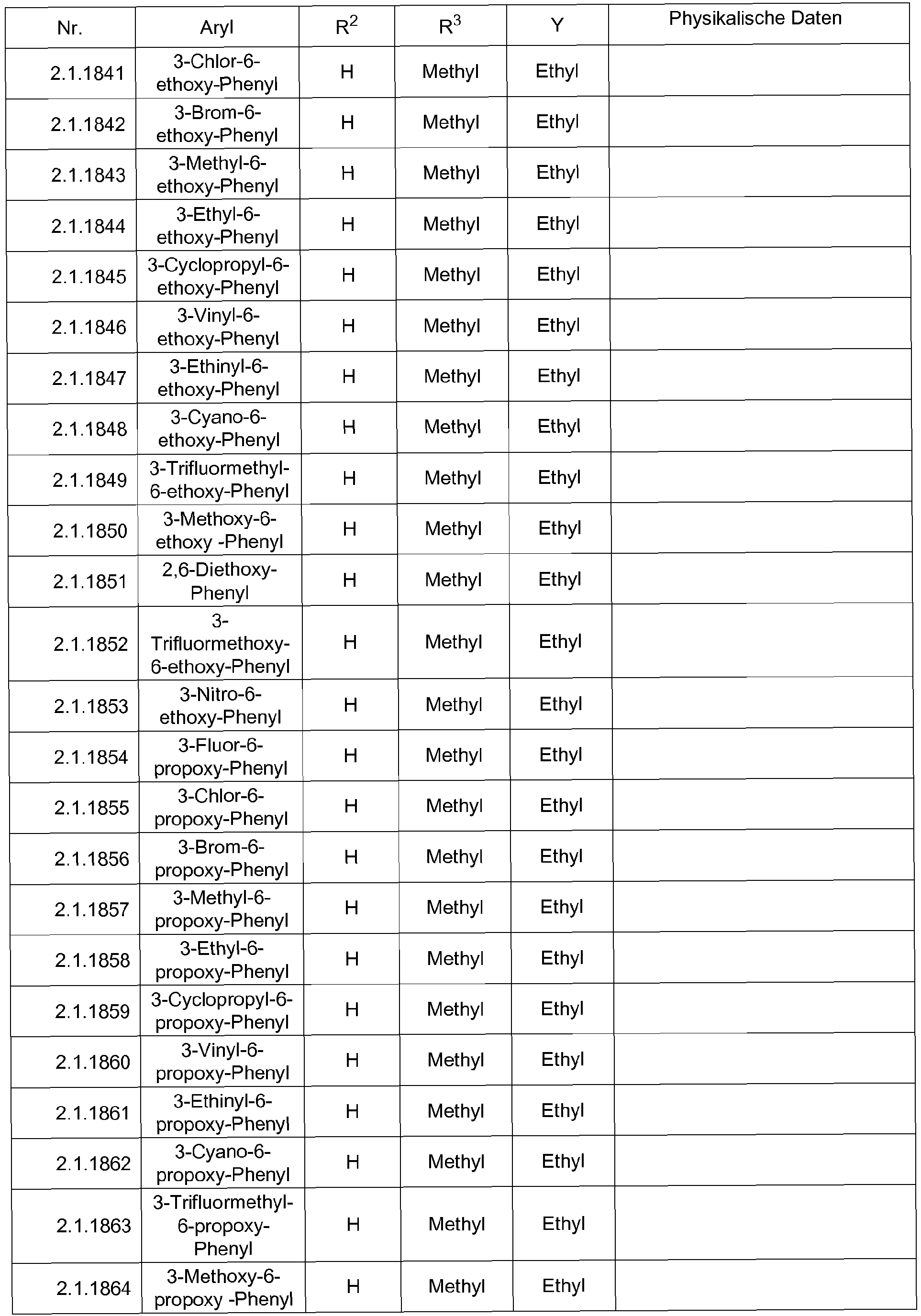

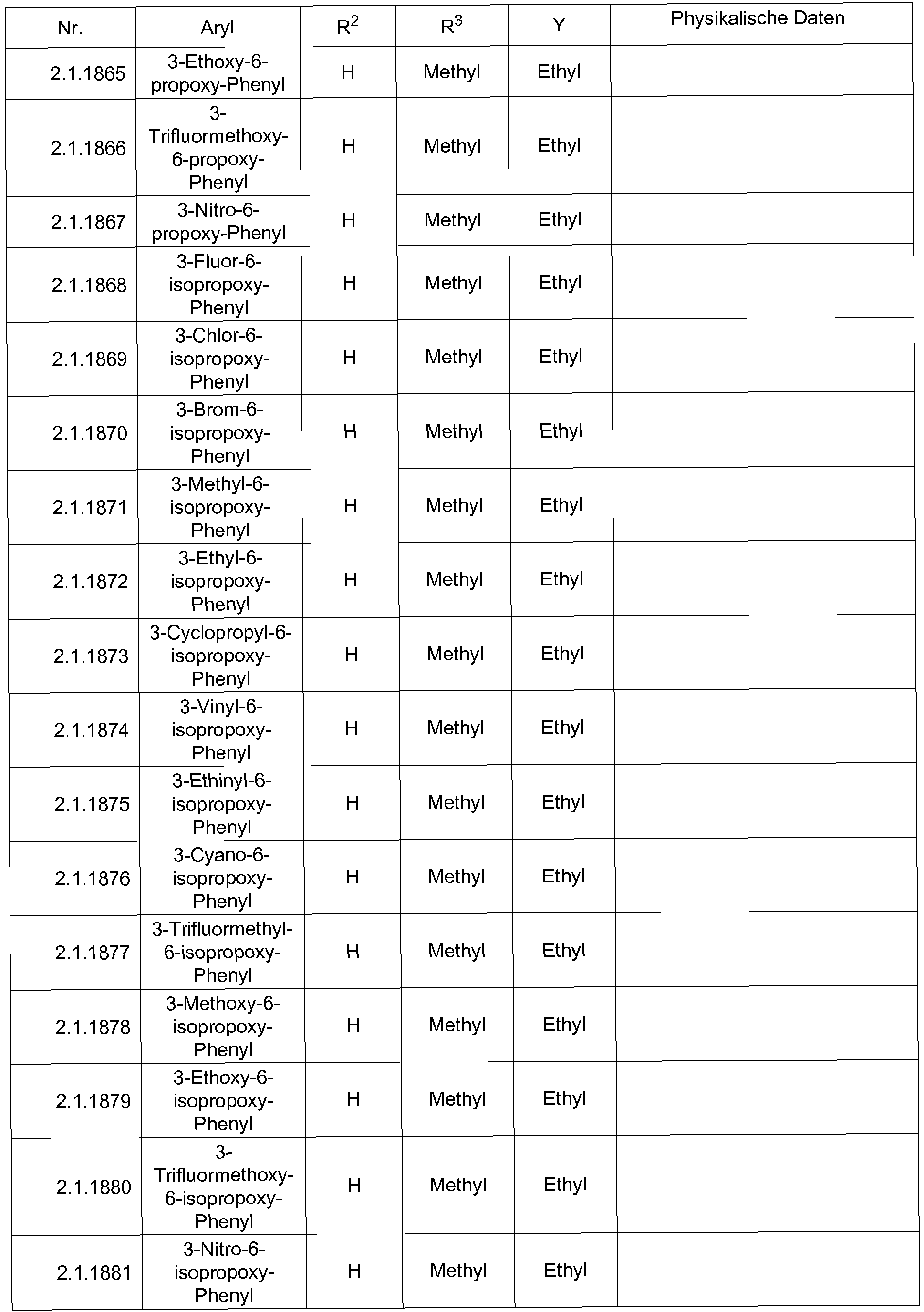

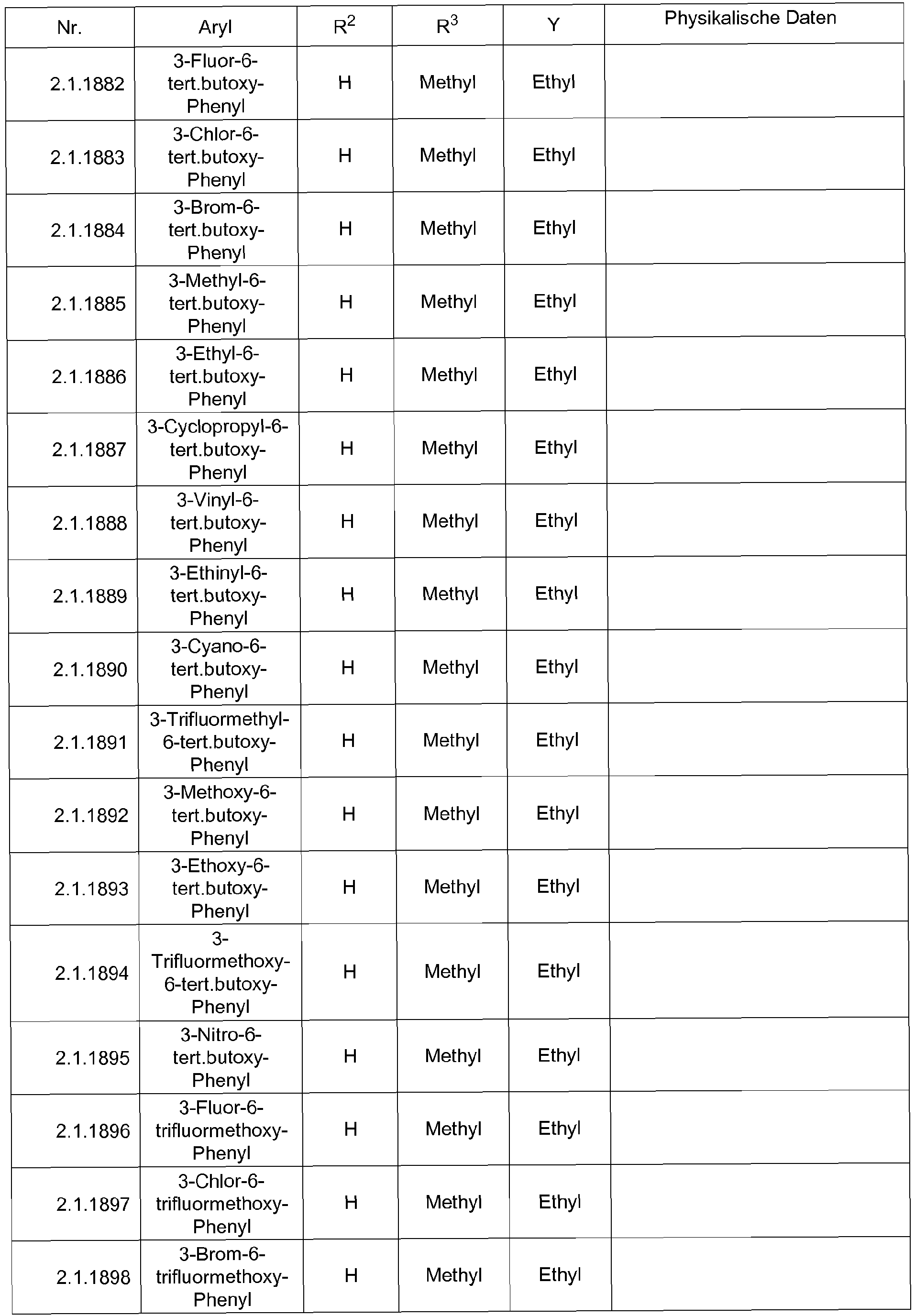

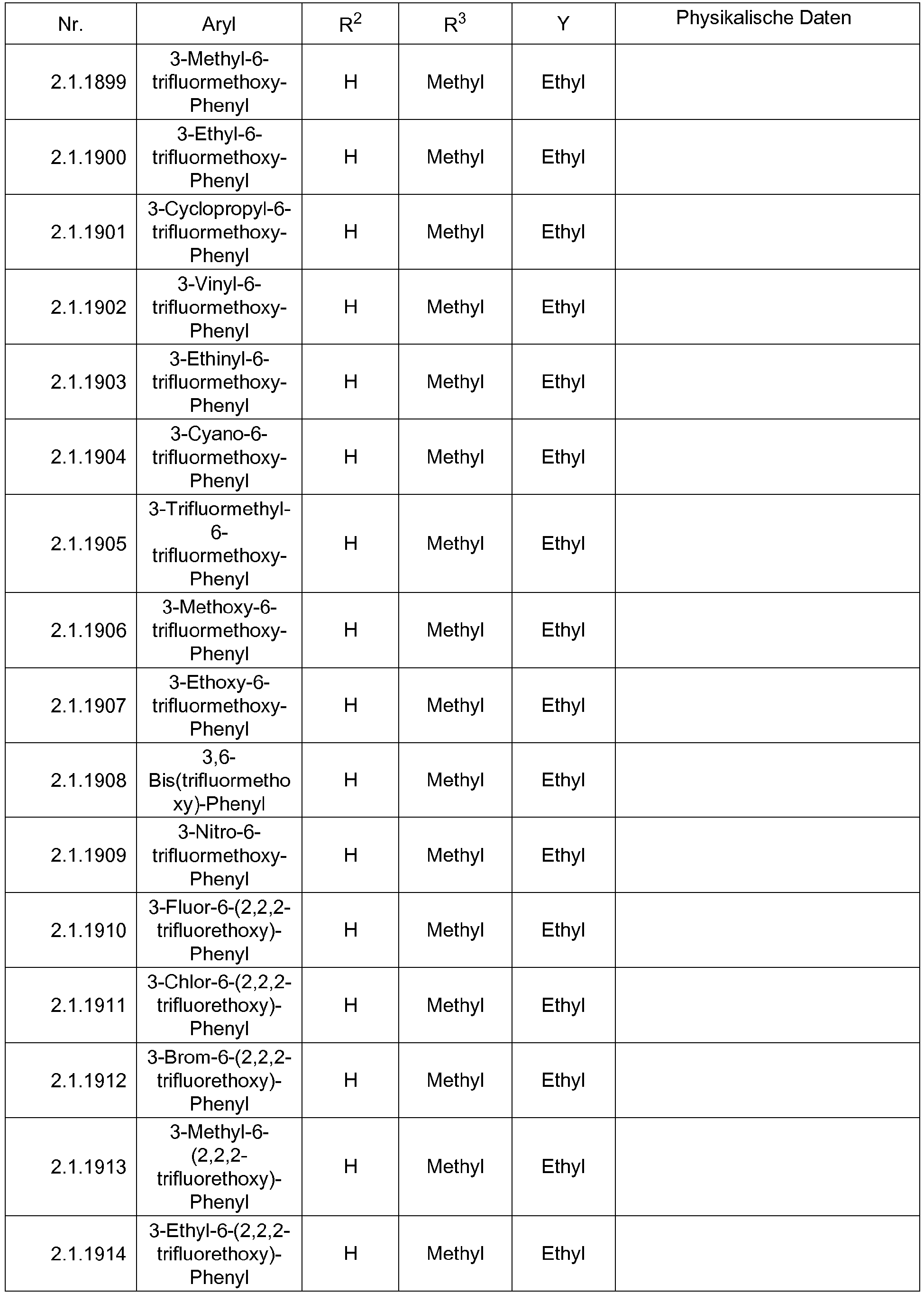

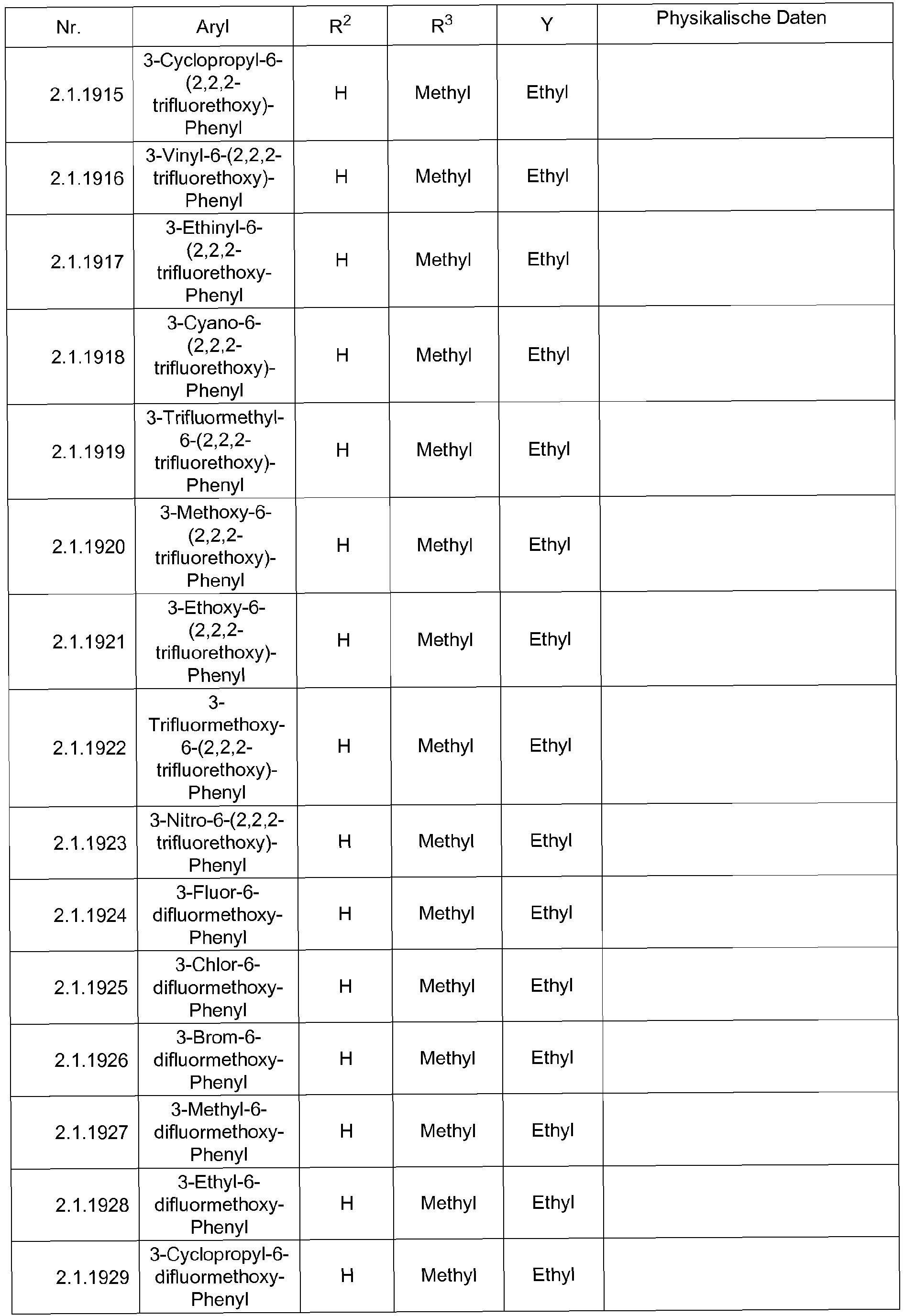

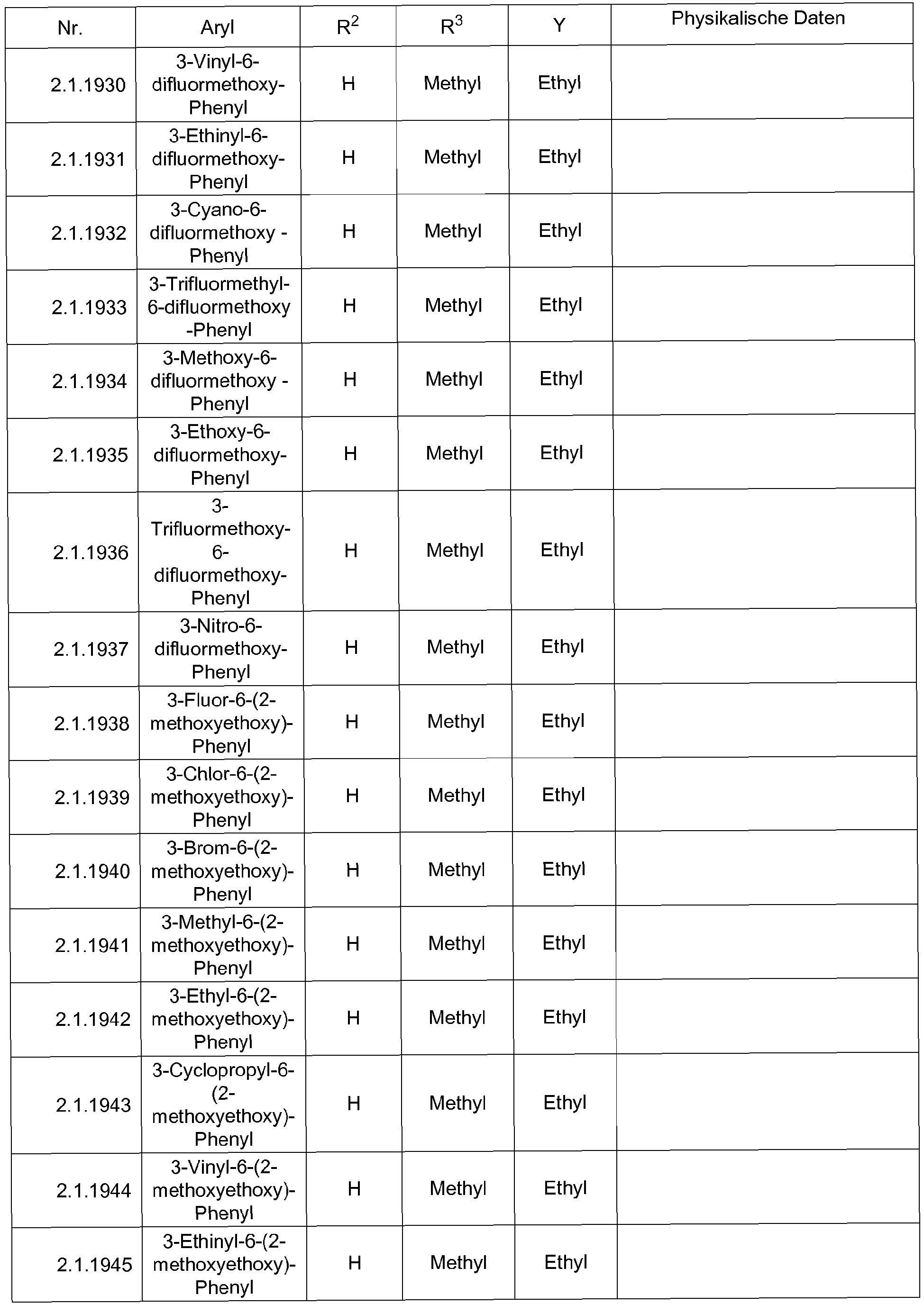

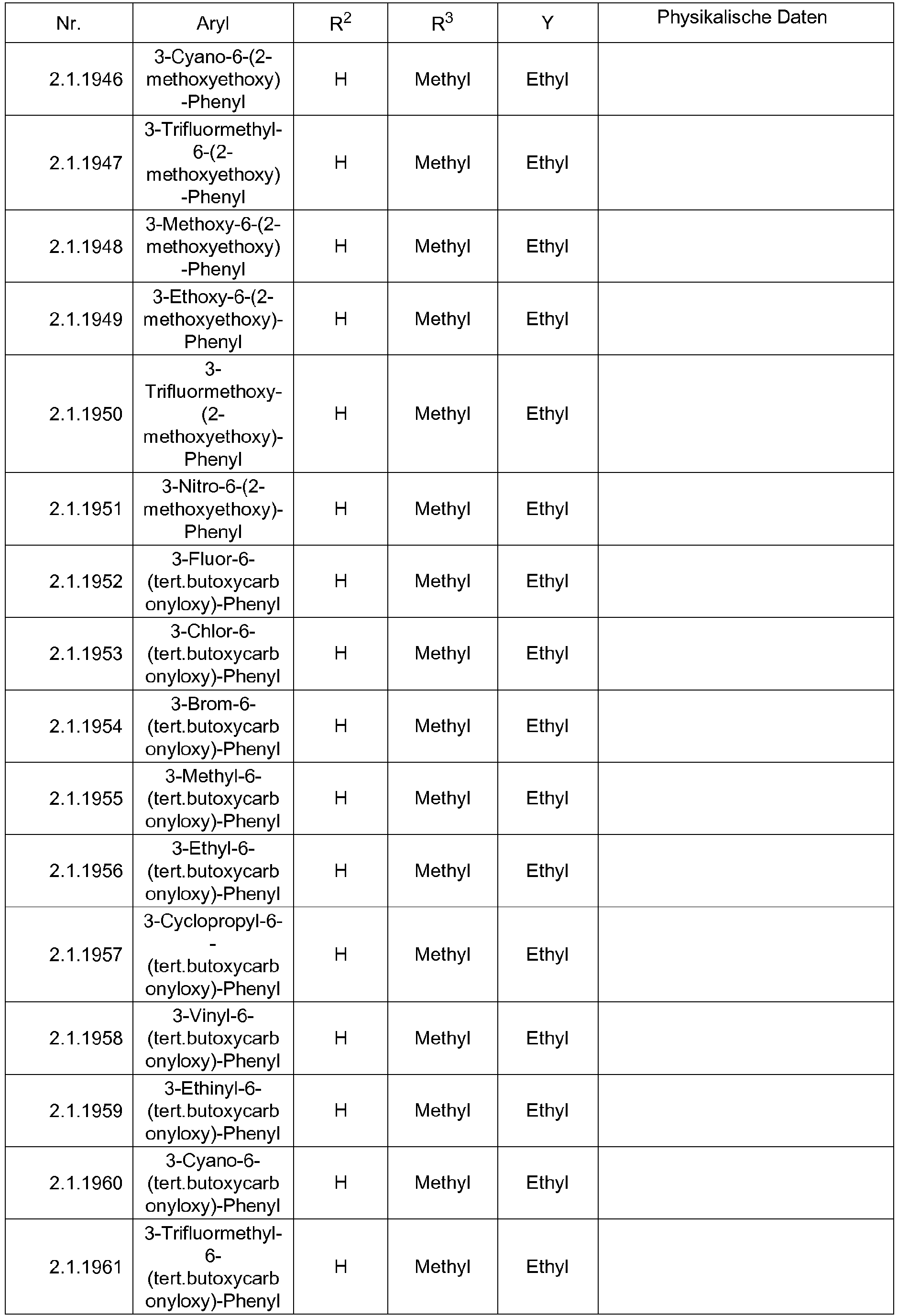

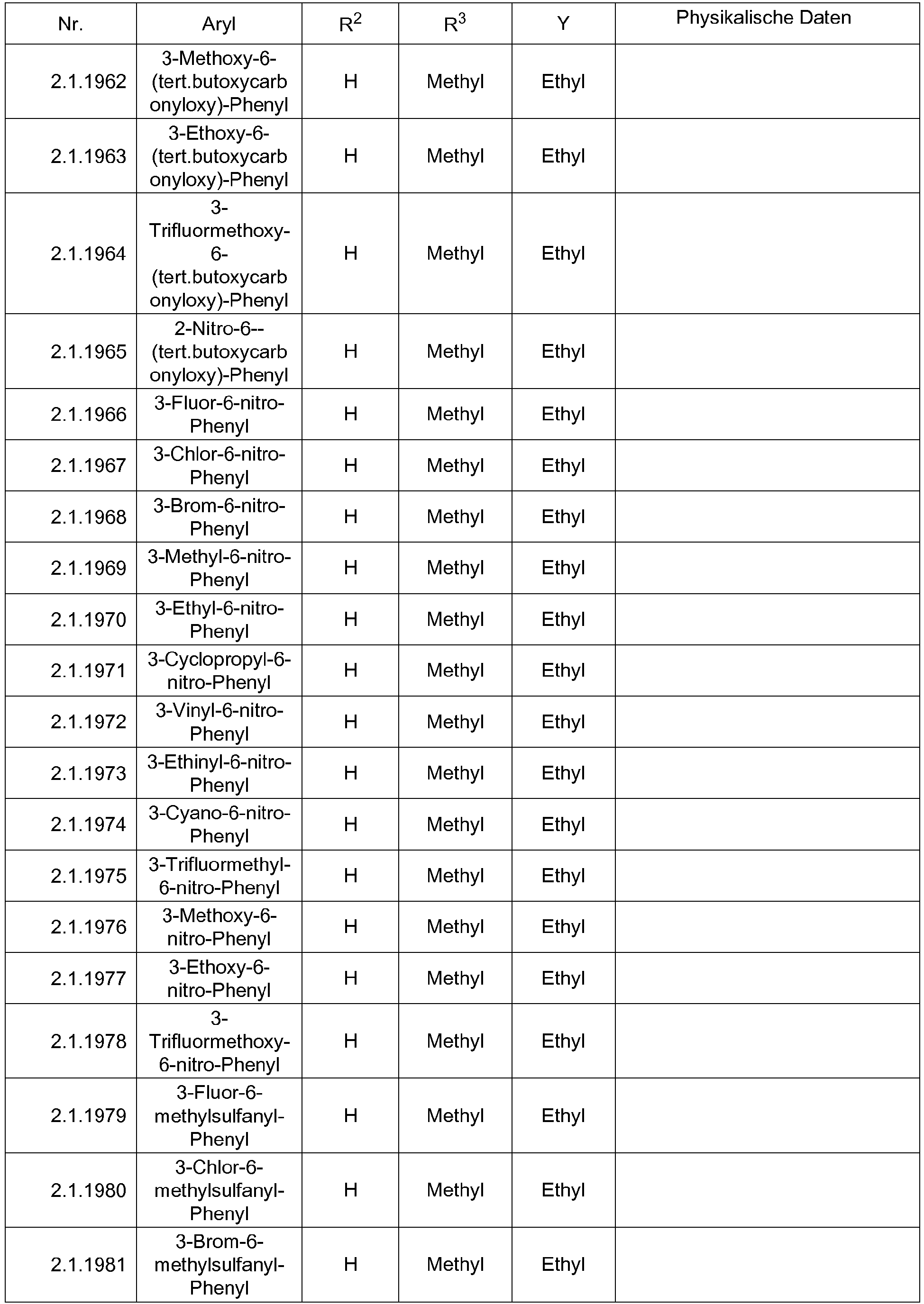

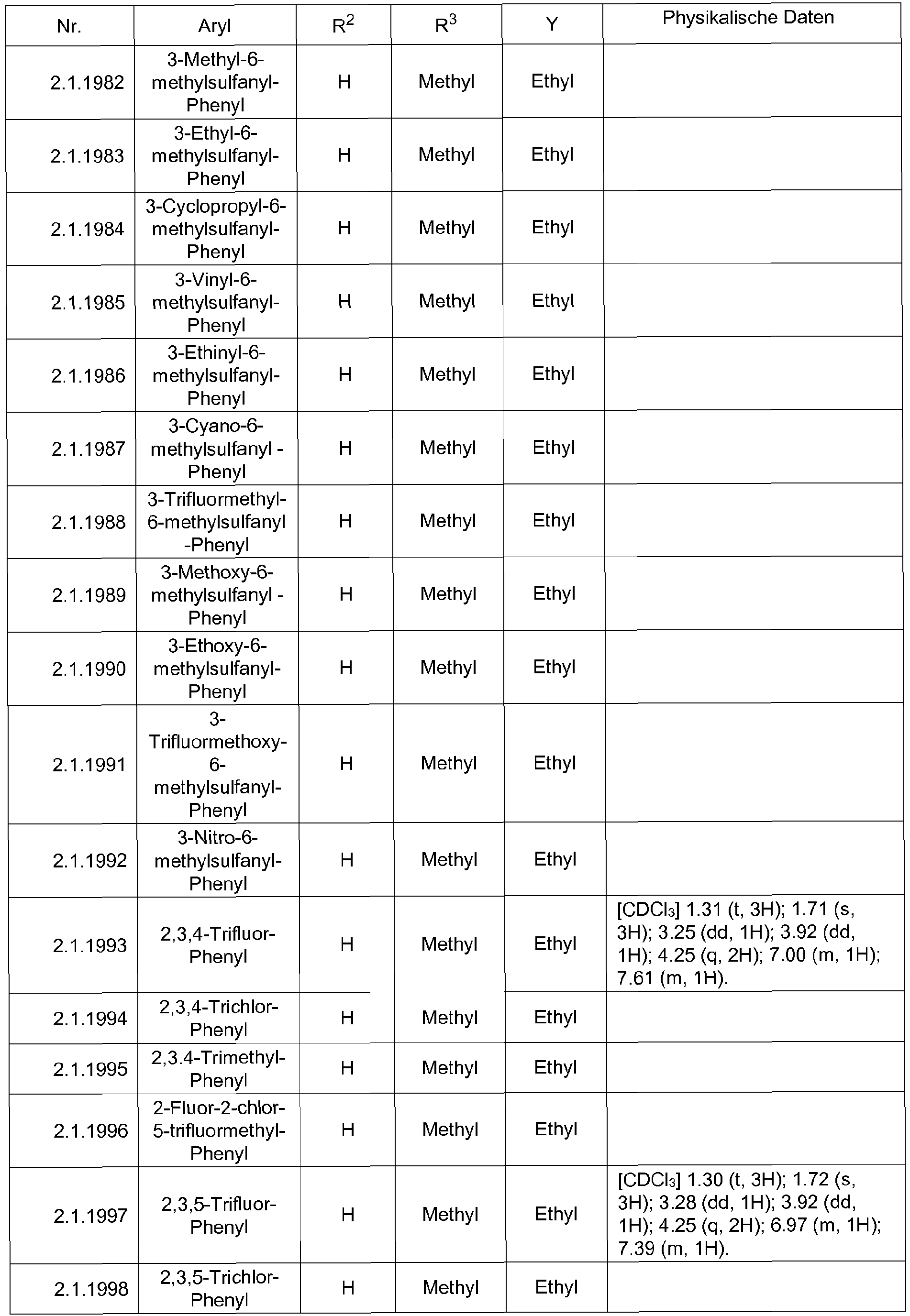

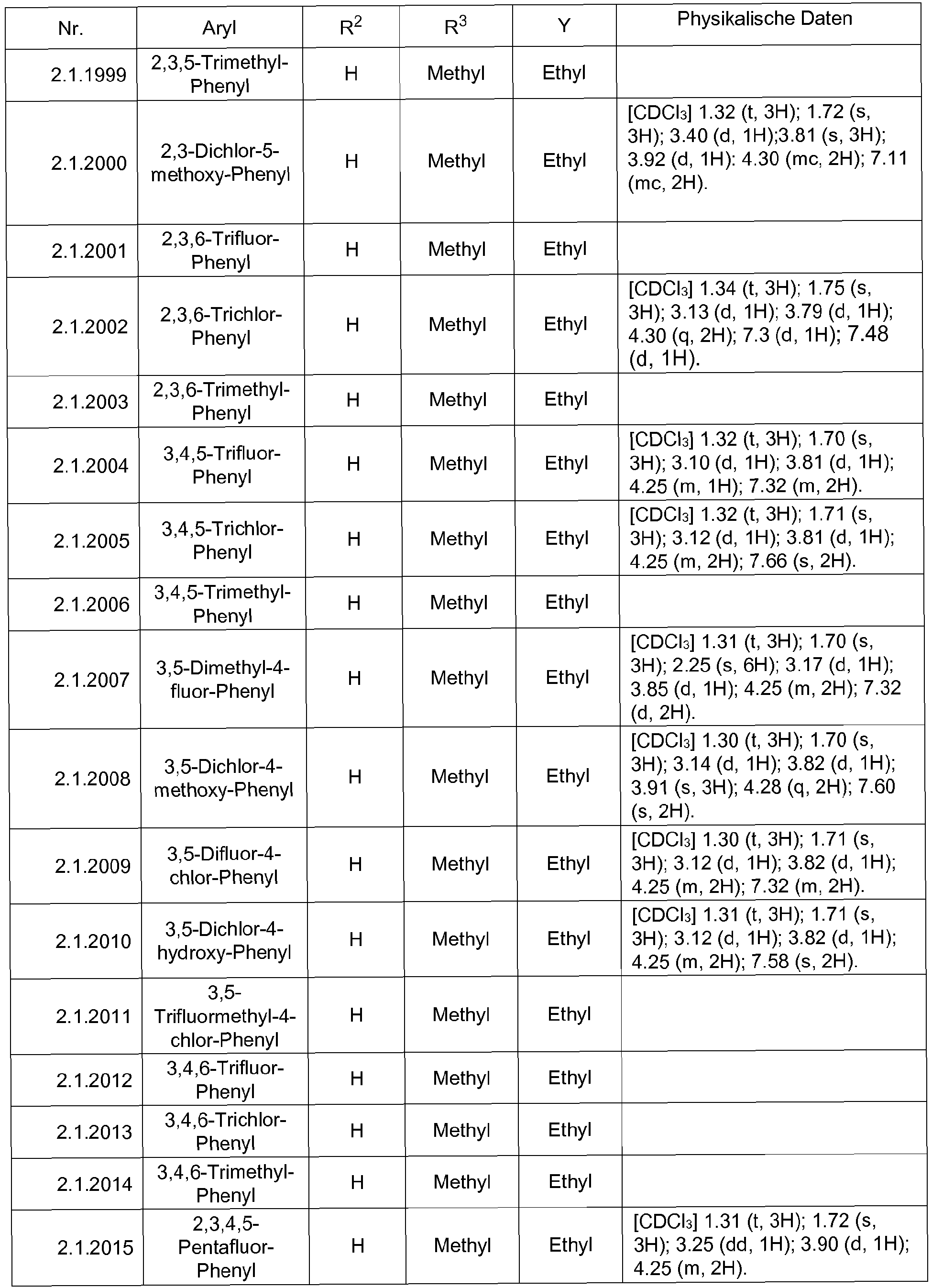

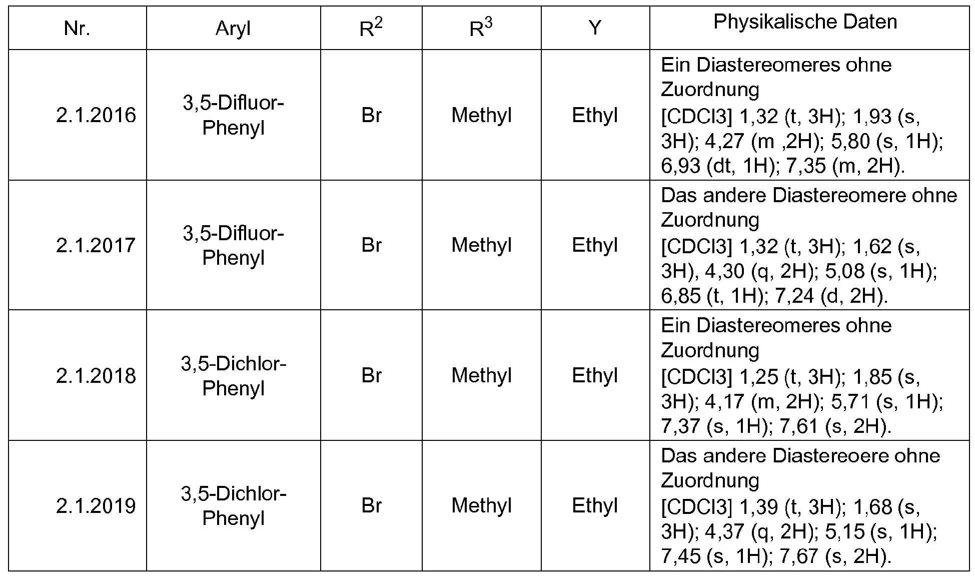

**Tabelle 2.2: Erfindungsgemäße Verbindungen der allgemeinen Formel (I), worin W* für COOY, R¹ und R² für Wasserstoff stehen, und Aryl den Rest**

| | | | | |
|---|---|---|---|---|
| | 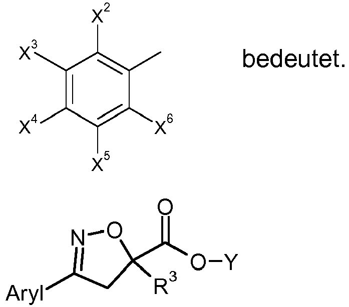 | | | |

| Nr. | Aryl | R³ | Y | Physikalische Daten |
|---|---|---|---|---|
| 2.2.001 | 3-Fluor-Phenyl | Fluormethyl | Ethyl | [CDCl₃] 1.33 (t,3H); 3.53 (d,1H); 3.84 (d,1H); 4.31 (q,2H); 4.68 (d,1H); 4.78 (d,1H); 7.14 (t,1H); 7.41 (m,3H). |
| 2.2.002 | 3-Fluor-Phenyl | Chlormethyl | Ethyl | [CDCl₃] 1.35 (t,3H); 3.55 (d,1H); 3.79 (d,1H); 4.01 (dd,2H); 4.32 (m,2H); 7.15 (t,1H); 7.41 (m,3H). |
| 2.2.003 | 3-Fluor-Phenyl | Brommethyl | Ethyl | [CDCl₃] 1.35 (t,3H); 3.50 (d,1H); 3.62 (d,1H); 3.90 (d,1H); 4.05 (d,1H); 4.33 (m,2H); 7.15 (t,1H); 7.43 (m,3H). |
| 2.2.004 | 3-Fluor-Phenyl | Difluormethyl | Ethyl | |
| 2.2.005 | 3-Fluor-Phenyl | 1,1-Dichlorethyl | Methyl | [CDCl₃] 2.40 (s,3H);3.88 (s,3H); 4.07 (AB,2H); 7.16 (t,1H); 7.39-7.46 (m,3H). |
| 2.2.006 | 3-Fluor-Phenyl | Cyano | Ethyl | |
| 2.2.007 | 3-Chlor-Phenyl | Fluormethyl | Ethyl | |
| 2.2.008 | 3-Chlor-Phenyl | Chlormethyl | Ethyl | |
| 2.2.009 | 3-Chlor-Phenyl | Brommethyl | Ethyl | |
| 2.2.010 | 3-Chlor-Phenyl | Difluormethyl | Ethyl | |
| 2.2.011 | 3-Chlor-Phenyl | Trifluormethyl | Ethyl | |
| 2.2.012 | 3-Chlor-Phenyl | Cyano | Ethyl | |
| 2.2.013 | 3-Brom-Phenyl | Fluormethyl | Ethyl | |
| 2.2.014 | 3-Brom-Phenyl | Chlormethyl | Ethyl | |
| 2.2.015 | 3-Iod-Phenyl | Fluormethyl | Ethyl | |
| 2.2.016 | 3-Iod-Phenyl | Chlormethyl | Ethyl | |
| 2.2.017 | 3-Methyl-Phenyl | Fluormethyl | Ethyl | |
| 2.2.018 | 3-Methyl-Phenyl | Chlormethyl | Ethyl | |
| 2.2.019 | 3-Ethyl-Phenyl | Fluormethyl | Ethyl | |
| 2.2.020 | 3-Propyl-Phenyl | Fluormethyl | Ethyl | |
| 2.2.021 | 3-isoPropyl-Phenyl | Fluormethyl | Ethyl | |
| 2.2.022 | 3-nButyl-Phenyl | Fluormethyl | Ethyl | |
| 2.2.023 | 3-iButyl-Phenyl | Fluormethyl | Ethyl | |
| 2.2.024 | 3-tert.Butyl-Phenyl | Fluormethyl | Ethyl | |
| 2.2.025 | 3-Cyclopropyl-Phenyl | Fluormethyl | Ethyl | |
| 2.2.026 | 3-Cyclobutyl-Phenyl | Fluormethyl | Ethyl | |
| 2.2.027 | 3-Cyclopentyl-Phenyl | Fluormethyl | Ethyl | |
| 2.2.028 | 3-Vinyl-Phenyl | Fluormethyl | Ethyl | |
| 2.2.029 | 3-Ethinyl-Phenyl | Fluormethyl | Ethyl | |
| 2.2.030 | 3-Cyano-Phenyl | Fluormethyl | Ethyl | |
| 2.2.031 | 3-Trifluormethyl-Phenyl | Fluormethyl | Ethyl | |
| 2.2.032 | 3-Difluormethyl-Phenyl | Fluormethyl | Ethyl | |
| 2.2.033 | 3-(Hydroxycarbonyl)-Phenyl | Fluormethyl | Ethyl | |
| 2.2.034 | 3-(Methoxycarbony)l-Phenyl | Fluormethyl | Ethyl | |
| 2.2.035 | 3-(Ethoxycarbonyl)-Phenyl | Fluormethyl | Ethyl | |
| 2.2.036 | 3-Hydroxymethyl-Phenyl | Fluormethyl | Ethyl | |
| 2.2.037 | 3-Carbamoyl-Phenyl | Fluormethyl | Ethyl | |
| 2.2.038 | 3-Hydroxy-Phenyl- | Fluormethyl | Ethyl | |
| 2.2.039 | 3-Methoxy-Phenyl | Fluormethyl | Ethyl | |
| 2.2.040 | 3-Ethoxy-Phenyl | Fluormethyl | Ethyl | |
| 2.2.041 | 3-Propyloxy-Phenyl | Fluormethyl | Ethyl | |
| 2.2.042 | 3-isoPropyloxy-Phenyl | Fluormethyl | Ethyl | |
| 2.2.043 | 3-nButyloxy-Phenyl | Fluormethyl | Ethyl | |
| 2.2.044 | 3-iButyloxy-Phenyl | Fluormethyl | Ethyl | |
| 2.2.045 | 3-tButyloxy-Phenyl | Fluormethyl | Ethyl | |
| 2.2.046 | 3-Difluormethoxy-Phenyl | Fluormethyl | Ethyl | |
| 2.2.047 | 3-Trifluormethoxy-Phenyl | Fluormethyl | Ethyl | |
| 2.2.048 | 3-(2,2,2-Trifluorethoxy)-Phenyl | Fluormethyl | Ethyl | |
| 2.2.049 | 3-(2-Chlorethoxy)-Phenyl | Fluormethyl | Ethyl | |
| 2.2.050 | 3-(2-Hydroxyethoxy)-Phenyl- | Fluormethyl | Ethyl | |
| 2.2.051 | 3-(2-Methoxyethoxy)-Phenyl- | Fluormethyl | Ethyl | |
| 2.2.052 | 3-[(tert-Butoxycarbonyl)oxy]-Phenyl | Fluormethyl | Ethyl | |
| 2.2.053 | 3-Nitro-Phenyl | Fluormethyl | Ethyl | |
| 2.2.054 | 3-Acetoxy-Phenyl | Fluormethyl | Ethyl | |
| 2.2.055 | {3-[(tert-Butoxycarbonyl)amino]-Phenyl}- | Fluormethyl | Ethyl | |
| 2.2.056 | 3-Methylsulfanyl-Phenyl | Fluormethyl | Ethyl | |
| 2.2.057 | 3-Ethylsulfanyl-Phenyl | Fluormethyl | Ethyl | |
| 2.2.058 | 3-(Pentafluor-lambda⁶-sulfanyl)-Phenyl | Fluormethyl | Ethyl | |
| 2.2.059 | 2,3-Difluor-Phenyl | Fluormethyl | Ethyl | |
| 2.2.060 | 2,3-Difluor-Phenyl | Chlormethyl | Ethyl | |
| 2.2.061 | 2,3-Difluor-Phenyl | Brommethyl | Ethyl | |
| 2.2.062 | 2,3-Difluor-Phenyl | Difluormethyl | Ethyl | |
| 2.2.063 | 2,3-Difluor-Phenyl | Trifluormethyl | Ethyl | |
| 2.2.064 | 2,3-Difluor-Phenyl | Cyano | Ethyl | |
| 2.2.065 | 2-Chlor-3-fluor-Phenyl | Fluormethyl | Ethyl | |
| 2.2.066 | 2-Brom-3-fluor-Phenyl | Fluormethyl | Ethyl | |
| 2.2.067 | 2-Methyl-3-fluor-Phenyl | Fluormethyl | Ethyl | |
| 2.2.068 | 2-Ethyl-3-fluor-Phenyl | Fluormethyl | Ethyl | |
| 2.2.069 | 2-Cyclopropyl-3-fluor-Phenyl | Fluormethyl | Ethyl | |
| 2.2.070 | 2-Vinyl-3-fluor-Phenyl | Fluormethyl | Ethyl | |
| 2.2.071 | 2-Ethinyl-3-fluor-Phenyl | Fluormethyl | Ethyl | |
| 2.2.072 | 2-Cyano-3-fluor-Phenyl | Fluormethyl | Ethyl | |
| 2.2.073 | 2-Methoxy-3-fluor-Phenyl | Fluormethyl | Ethyl | |
| 2.2.074 | 2-Ethoxy-3-fluor-Phenyl | Fluormethyl | Ethyl | |
| 2.2.075 | 2-Trifluormethoxy-3-fluor-Phenyl | Fluormethyl | Ethyl | |
| 2.2.076 | 2-Nitro-3-fluor-Phenyl | Fluormethyl | Ethyl | |
| 2.2.077 | 2-Fluor-3-chlor-Phenyl | Fluormethyl | Ethyl | |
| 2.2.078 | 2,3-Dichlor-Phenyl | Fluormethyl | Ethyl | |
| 2.2.079 | 2,3-Dichlor-Phenyl | Chlormethyl | Ethyl | |
| 2.2.080 | 2,3-Dichlor-Phenyl | Brommethyl | Ethyl | |
| 2.2.081 | 2,3-Dichlor-Phenyl | Difluormethyl | Ethyl | |
| 2.2.082 | 2-Brom-3-chlor-Phenyl | Fluormethyl | Ethyl | |
| 2.2.083 | 2-Methyl-3-chlor-Phenyl | Fluormethyl | Ethyl | |
| 2.2.084 | 2-Ethyl-3-chlor-Phenyl | Fluormethyl | Ethyl | |
| 2.2.085 | 2-Cyclopropyl-3-chlor-Phenyl | Fluormethyl | Ethyl | |
| 2.2.086 | 2-Vinyl-3-chlor-Phenyl | Fluormethyl | Ethyl | |
| 2.2.087 | 2-Ethinyl-3-chlor-Phenyl | Fluormethyl | Ethyl | |
| 2.2.088 | 2-Cyano-3-chlor-Phenyl | Fluormethyl | Ethyl | |
| 2.2.089 | 2-Trifluormethyl-2-chlor-Phenyl | Fluormethyl | Ethyl | |
| 2.2.090 | 2-Methoxy-3-chlor-Phenyl | Fluormethyl | Ethyl | |
| 2.2.091 | 2-Ethoxy-3-chlor-Phenyl | Fluormethyl | Ethyl | |
| 2.2.092 | 2-Trifluormethoxy-3-chlor-Phenyl | Fluormethyl | Ethyl | |
| 2.2.093 | 2-Nitro-3-chlor-Phenyl | Fluormethyl | Ethyl | |
| 2.2.094 | 2-Fluor-3-brom-Phenyl | Fluormethyl | Ethyl | |
| 2.2.095 | 2-Chlor-3-brom-Phenyl | Fluormethyl | Ethyl | |
| 2.2.096 | 2,3-Dibrom-Phenyl | Fluormethyl | Ethyl | |
| 2.2.097 | 2-Methyl-3-brom-Phenyl | Fluormethyl | Ethyl | |
| 2.2.098 | 2-Ethyl-3-brom-Phenyl | Fluormethyl | Ethyl | |
| 2.2.099 | 2-Cyclopropyl-3-brom-Phenyl | Fluormethyl | Ethyl | |
| 2.2.100 | 2-Vinyl-3-brom-Phenyl | Fluormethyl | Ethyl | |
| 2.2.101 | 2-Ethinyl-3-brom-Phenyl | Fluormethyl | Ethyl | |
| 2.2.102 | 2-Cyano-3-brom-Phenyl | Fluormethyl | Ethyl | |
| 2.2.103 | 2-Trifluormethyl-3-brom-Phenyl | Fluormethyl | Ethyl | |
| 2.2.104 | 2-Methoxy-3-Phenyl | Fluormethyl | Ethyl | |
| 2.2.105 | 2-Ethoxy-3-brom-Phenyl | Fluormethyl | Ethyl | |
| 2.2.106 | 2-Trifluormethoxy-3-brom-Phenyl | Fluormethyl | Ethyl | |
| 2.2.107 | 2-Nitro-3-brom-Phenyl | Fluormethyl | Ethyl | |
| 2.2.108 | 2-Fluor-3-iod-Phenyl | Fluormethyl | Ethyl | |
| 2.2.109 | 2-Chlor-3-iod-Phenyl | Fluormethyl | Ethyl | |
| 2.2.110 | 2-Brom-3-iod-Phenyl | Fluormethyl | Ethyl | |
| 2.2.111 | 2-Methyl-3-iod-Phenyl | Fluormethyl | Ethyl | |
| 2.2.112 | 2-Ethyl-3-iod-Phenyl | Fluormethyl | Ethyl | |
| 2.2.113 | 2-Cyclopropyl-3-iod-Phenyl | Fluormethyl | Ethyl | |
| 2.2.114 | 2-Vinyl-3-iod-Phenyl | Fluormethyl | Ethyl | |
| 2.2.115 | 2-Ethinyl-3-iod-Phenyl | Fluormethyl | Ethyl | |
| 2.2.116 | 2-Cyano-3-iod-Phenyl | Fluormethyl | Ethyl | |
| 2.2.117 | 2-Trifluormethyl-3-iod-Phenyl | Fluormethyl | Ethyl | |
| 2.2.118 | 2-Methoxy-3-iod-Phenyl | Fluormethyl | Ethyl | |
| 2.2.119 | 2-Ethoxy-3-iod-Phenyl | Fluormethyl | Ethyl | |
| 2.2.120 | 2-Trifluormethoxy-3-iod-Phenyl | Fluormethyl | Ethyl | |
| 2.2.121 | 2-Nitro-3-iod-Phenyl | Fluormethyl | Ethyl | |
| 2.2.122 | 2-Fluor-3-methyl-Phenyl | Fluormethyl | Ethyl | |
| 2.2.123 | 2-Fluor-3-methyl-Phenyl | Chlormethyl | Ethyl | |
| 2.2.124 | 2-Fluor-3-methyl-Phenyl | Brommethyl | Ethyl | |
| 2.2.125 | 2-Fluor-3-methyl-Phenyl | Difluormethyl | Ethyl | |
| 2.2.126 | 2-Chlor-3-methyl-Phenyl | Fluormethyl | Ethyl | |
| 2.2.127 | 2-Chlor-3-methyl-Phenyl | Chlormethyl | Ethyl | |
| 2.2.128 | 2-Chlor-3-methyl-Phenyl | Brommethyl | Ethyl | |
| 2.2.129 | 2-Chlor-3-methyl-Phenyl | Difluormethyl | Ethyl | |
| 2.2.130 | 2-Brom-3-methyl-Phenyl | Fluormethyl | Ethyl | |
| 2.2.131 | 2,3-Dimethyl-Phenyl | Fluormethyl | Ethyl | |
| 2.2.132 | 2,3-Dimethyl-Phenyl | Chlormethyl | Ethyl | |
| 2.2.133 | 2,3-Dimethyl-Phenyl | Brommethyl | Ethyl | |
| 2.2.134 | 2,3-Dimethyl-Phenyl | Difluormethyl | Ethyl | |
| 2.2.135 | 2-Ethyl-3-methyl-Phenyl | Fluormethyl | Ethyl | |
| 2.2.136 | 2-Cyclopropyl-3-methyl-Phenyl | Fluormethyl | Ethyl | |
| 2.2.137 | 2-Vinyl-3-methyl-Phenyl | Fluormethyl | Ethyl | |
| 2.2.138 | 2-Ethinyl-3-methyl-Phenyl | Fluormethyl | Ethyl | |
| 2.2.139 | 2-Cyano-3-methyl-Phenyl | Fluormethyl | Ethyl | |
| 2.2.140 | 2-Trifluormethyl-3-methyl-Phenyl | Fluormethyl | Ethyl | |
| 2.2.141 | 2-Methoxy-3-methyl-Phenyl | Fluormethyl | Ethyl | |
| 2.2.142 | 2-Ethoxy-3-methyl-Phenyl | Fluormethyl | Ethyl | |
| 2.2.143 | 2-Trifluormethoxy-3-methyl-Phenyl | Fluormethyl | Ethyl | |
| 2.2.144 | 2-Nitro-3-methyl-Phenyl | Fluormethyl | Ethyl | |
| 2.2.145 | 2-Fluor-3-ethyl-Phenyl | Fluormethyl | Ethyl | |
| 2.2.146 | 2-Chlor-3-ethyl-Phenyl | Fluormethyl | Ethyl | |
| 2.2.147 | 2-Brom-3-ethyl-Phenyl | Fluormethyl | Ethyl | |
| 2.2.148 | 2-Methyl-3-ethyl-Phenyl | Fluormethyl | Ethyl | |
| 2.2.149 | 2,3-Diethyl-Phenyl | Fluormethyl | Ethyl | |
| 2.2.150 | 2-Cyclopropyl-3-ethyl-Phenyl | Fluormethyl | Ethyl | |
| 2.2.151 | 2-Vinyl-3-ethyl-Phenyl | Fluormethyl | Ethyl | |
| 2.2.152 | 2-Ethinyl-3-ethyl-Phenyl | Fluormethyl | Ethyl | |
| 2.2.153 | 2-Cyano-3-ethyl-Phenyl | Fluormethyl | Ethyl | |
| 2.2.154 | 2-Trifluormethyl-3-ethyl-Phenyl | Fluormethyl | Ethyl | |
| 2.2.155 | 2-Methoxy-3-ethyl-Phenyl | Fluormethyl | Ethyl | |
| 2.2.156 | 2-Ethoxy-3-ethyl-Phenyl | Fluormethyl | Ethyl | |
| 2.2.157 | 2-Trifluormethoxy-3-ethyl-Phenyl | Fluormethyl | Ethyl | |
| 2.2.158 | 2-Nitro-3-ethyl-Phenyl | Fluormethyl | Ethyl | |
| 2.2.159 | 2-Fluor-3-propyl-Phenyl | Fluormethyl | Ethyl | |
| 2.2.160 | 2-Chlor-3-propyl-Phenyl | Fluormethyl | Ethyl | |
| 2.2.161 | 2-Brom-3-propyl-Phenyl | Fluormethyl | Ethyl | |
| 2.2.162 | 2-Methyl-3-propyl-Phenyl | Fluormethyl | Ethyl | |
| 2.2.163 | 2-Methyl-3-propyl-Phenyl | Fluormethyl | Ethyl | |
| 2.2.164 | 2-Cyclopropyl-3-propyl-Phenyl | Fluormethyl | Ethyl | |
| 2.2.165 | 2-Vinyl-3-propyl-Phenyl | Fluormethyl | Ethyl | |
| 2.2.166 | 2-Ethinyl-3propyl-Phenyl | Fluormethyl | Ethyl | |
| 2.2.167 | 2-Cyano-3-propyl-Phenyl | Fluormethyl | Ethyl | |
| 2.2.168 | 2-Trifluormethyl-3-propyl-Phenyl | Fluormethyl | Ethyl | |
| 2.2.169 | 2-Methoxy-3-propyl-Phenyl | Fluormethyl | Ethyl | |
| 2.2.170 | 2-Ethoxy-3-propyl-Phenyl | Fluormethyl | Ethyl | |
| 2.2.171 | 2-Trifluormethoxy-3-propyl-Phenyl | Fluormethyl | Ethyl | |
| 2.2.172 | 2-Nitro-3-propyl-Phenyl | Fluormethyl | Ethyl | |
| 2.2.173 | 2-Fluor-3-isopropyl-Phenyl | Fluormethyl | Ethyl | |
| 2.2.174 | 2-Chlor-3-isopropyl-Phenyl | Fluormethyl | Ethyl | |
| 2.2.175 | 2-Brom-3-isopropyl-Phenyl | Fluormethyl | Ethyl | |
| 2.2.176 | 2-Methyl-3-isopropyl-Phenyl | Fluormethyl | Ethyl | |
| 2.2.177 | 2-Ethyl-3-isopropyl-Phenyl | Fluormethyl | Ethyl | |
| 2.2.178 | 2-Cyclopropyl-3-isopropyl-Phenyl | Fluormethyl | Ethyl | |
| 2.2.179 | 2-Vinyl-3-isopropyl-Phenyl | Fluormethyl | Ethyl | |
| 2.2.180 | 2-Ethinyl-3-isopropyl-Phenyl | Fluormethyl | Ethyl | |
| 2.2.181 | 2-Cyano-3-isopropyl-Phenyl | Fluormethyl | Ethyl | |
| 2.2.182 | 2-Trifluormethyl-3-isopropyl-Phenyl | Fluormethyl | Ethyl | |
| 2.2.183 | 2-Methoxy-3-isopropyl-Phenyl | Fluormethyl | Ethyl | |
| 2.2.184 | 2-Ethoxy-3-isopropyl-Phenyl | Fluormethyl | Ethyl | |
| 2.2.185 | 2-Trifluormethoxy-3-isopropyl-Phenyl | Fluormethyl | Ethyl | |
| 2.2.186 | 2-Nitro-3-isopropyl-Phenyl | Fluormethyl | Ethyl | |
| 2.2.187 | 2-Fluor-3-tert.butyl-Phenyl | Fluormethyl | Ethyl | |
| 2.2.188 | 2-Chlor-3-tert.buty-Phenyl | Fluormethyl | Ethyl | |
| 2.2.189 | 2-Brom-3-tert.butyl-Phenyl | Fluormethyl | Ethyl | |
| 2.2.190 | 2-Methyl-3-tert.butyl-Phenyl | Fluormethyl | Ethyl | |
| 2.2.191 | 2-Ethyl-3-tert.butyl-Phenyl | Fluormethyl | Ethyl | |
| 2.2.192 | 2-Cyclopropyl-3-tert.butyl-Phenyl | Fluormethyl | Ethyl | |
| 2.2.193 | 2-Vinyl-3-tert.butyl-Phenyl | Fluormethyl | Ethyl | |
| 2.2.194 | 2-Ethinyl-3-tert.butyl-Phenyl | Fluormethyl | Ethyl | |
| 2.2.195 | 2-Cyano-3-tert.butyl-Phenyl | Fluormethyl | Ethyl | |
| 2.2.196 | 2-Trifluormethyl-3-tert.butyl-Phenyl | Fluormethyl | Ethyl | |
| 2.2.197 | 2-Methoxy-3-tert.butyl-Phenyl | Fluormethyl | Ethyl | |
| 2.2.198 | 2-Ethoxy-3-tert.butyl-Phenyl | Fluormethyl | Ethyl | |
| 2.2.199 | 2-Trifluormethoxy-3-tert.butyl-Phenyl | Fluormethyl | Ethyl | |
| 2.2.200 | 2-Nitro-3-tert.butyl-Phenyl | Fluormethyl | Ethyl | |
| 2.2.201 | 2-Fluor-3-hydroxymethyl-Phenyl | Fluormethyl | Ethyl | |
| 2.2.202 | 2-Chlor-3-hydroxymethyl-Phenyl | Fluormethyl | Ethyl | |
| 2.2.203 | 2-Brom-3-hydroxymethyl-Phenyl | Fluormethyl | Ethyl | |
| 2.2.204 | 2-Methyl-3-hydroxymethyl -Phenyl | Fluormethyl | Ethyl | |
| 2.2.205 | 2-Ethyl-3-hydroxymethyl-Phenyl | Fluormethyl | Ethyl | |
| 2.2.206 | 2-Cyclopropyl-3-hydroxymethyl-Phenyl | Fluormethyl | Ethyl | |
| 2.2.207 | 2-Vinyl-3-hydroxymethyl-Phenyl | Fluormethyl | Ethyl | |
| 2.2.208 | 2-Ethinyl-3-hydroxymethyl-Phenyl | Fluormethyl | Ethyl | |
| 2.2.209 | 2-Cyano-3-hydroxymethyl -Phenyl | Fluormethyl | Ethyl | |
| 2.2.210 | 2-Trifluormethyl-3-hydroxymethyl-Phenyl | Fluormethyl | Ethyl | |
| 2.2.211 | 2-Methoxy-3-hydroxymethyl-Phenyl | Fluormethyl | Ethyl | |
| 2.2.212 | 2-Ethoxy-3-hydroxymethyl -Phenyl | Fluormethyl | Ethyl | |
| 2.2.213 | 2-Trifluormethoxy-3-hydroxymethyl--Phenyl | Fluormethyl | Ethyl | |
| 2.2.214 | 2-Nitro-3-hydroxymethyl-Phenyl | Fluormethyl | Ethyl | |
| 2.2.215 | 2-Fluor-3-cyclopropyl-Phenyl | Fluormethyl | Ethyl | |
| 2.2.216 | 2-Chlor-3-cyclopropyl-Phenyl | Fluormethyl | Ethyl | |
| 2.2.217 | 2-Brom-3-cyclopropyl-Phenyl | Fluormethyl | Ethyl | |
| 2.2.218 | 2-Methyl-3-cyclopropyl-Phenyl | Fluormethyl | Ethyl | |
| 2.2.219 | 2-Ethyl-3-cyclopropyl-Phenyl | Fluormethyl | Ethyl | |
| 2.2.220 | 2-Cyclopropyl-3-cyclopropyl-Phenyl | Fluormethyl | Ethyl | |
| 2.2.221 | 2-Vinyl-3-cyclopropyl-Phenyl | Fluormethyl | Ethyl | |
| 2.2.222 | 2-Ethinyl-3-cyclopropyl-Phenyl | Fluormethyl | Ethyl | |
| 2.2.223 | 2-Cyano-3-cyclopropyl-Phenyl | Fluormethyl | Ethyl | |
| 2.2.224 | 2-Trifluormethyl-3-cyclopropyl-Phenyl | Fluormethyl | Ethyl | |
| 2.2.225 | 2-Methoxy-3-cyclopropyl-Phenyl | Fluormethyl | Ethyl | |
| 2.2.226 | 2-Ethoxy-3-cyclopropyl-Phenyl | Fluormethyl | Ethyl | |
| 2.2.227 | 2-Trifluormethoxy-3-cyclopropyl-Phenyl | Fluormethyl | Ethyl | |
| 2.2.228 | 2-Fluor-3-methoxycarbonyl-Phenyl | Fluormethyl | Ethyl | |
| 2.2.229 | 2-Chlor-3-methoxycarbonyl-Phenyl | Fluormethyl | Ethyl | |
| 2.2.230 | 2-Brom-3-methoxycarbonyl-Phenyl | Fluormethyl | Ethyl | |
| 2.2.231 | 2-Methyl-3-methoxycarbonyl-Phenyl | Fluormethyl | Ethyl | |
| 2.2.232 | 2-Ethyl-3-methoxycarbonyl-Phenyl | Fluormethyl | Ethyl | |
| 2.2.233 | 2-Cyclopropyl-3-methoxycarbonyl-Phenyl | Fluormethyl | Ethyl | |
| 2.2.234 | 2-Vinyl-3-methoxycarbonyl-Phenyl | Fluormethyl | Ethyl | |
| 2.2.235 | 2-Ethinyl-3-methoxycarbonyl-Phenyl | Fluormethyl | Ethyl | |
| 2.2.236 | 2-Cyano-3-methoxycarbonyl-Phenyl | Fluormethyl | Ethyl | |
| 2.2.237 | 2-Trifluormethyl-3-methoxycarbonyl-Phenyl | Fluormethyl | Ethyl | |
| 2.2.238 | 2-Methoxy-3-methoxycarbonyl -Phenyl | Fluormethyl | Ethyl | |
| 2.2.239 | 2-Ethoxy-3-methoxycarbonyl-Phenyl | Fluormethyl | Ethyl | |
| 2.2.240 | 2-Trifluormethoxy-3-m ethoxycarbonyl-Phenyl | Fluormethyl | Ethyl | |
| 2.2.241 | 2-Nitro-3-methoxycarbonyl-Phenyl | Fluormethyl | Ethyl | |
| 2.2.242 | 2-Fluor-3-vinyl-Phenyl | Fluormethyl | Ethyl | |
| 2.2.243 | 2-Chlor-3-vinyl-Phenyl | Fluormethyl | Ethyl | |
| 2.2.244 | 2-Brom-3-vinyl-Phenyl | Fluormethyl | Ethyl | |
| 2.2.245 | 2-Methyl-3-vinyl-Phenyl | Fluormethyl | Ethyl | |
| 2.2.246 | 2-Ethyl-3-vinyl-Phenyl | Fluormethyl | Ethyl | |
| 2.2.247 | 2-Cyclopropyl-3-vinyl-Phenyl | Fluormethyl | Ethyl | |
| 2.2.248 | 2-Vinyl-3-vinyl-Phenyl | Fluormethyl | Ethyl | |
| 2.2.249 | 2-Ethinyl-3-vinyl-Phenyl | Fluormethyl | Ethyl | |
| 2.2.250 | 2-Cyano-3-vinyl-Phenyl | Fluormethyl | Ethyl | |
| 2.2.251 | 2-Trifluormethyl-3-vinyl-Phenyl | Fluormethyl | Ethyl | |
| 2.2.252 | 2-Methoxy-3-vinyl-Phenyl | Fluormethyl | Ethyl | |
| 2.2.253 | 2-Ethoxy-3-vinyl-Phenyl | Fluormethyl | Ethyl | |
| 2.2.254 | 2-Trifluormethoxy-3-vinyl-Phenyl | Fluormethyl | Ethyl | |
| 2.2.255 | 2-Nitro-3-vinyl-Phenyl | Fluormethyl | Ethyl | |
| 2.2.256 | 2-Fluor-3-ethinyl-Phenyl | Fluormethyl | Ethyl | |
| 2.2.257 | 2-Chlor-3-ethinyl-Phenyl | Fluormethyl | Ethyl | |
| 2.2.258 | 2-Brom-3-ethinyl -Phenyl | Fluormethyl | Ethyl | |
| 2.2.259 | 2-Methyl-3-ethinyl -Phenyl | Fluormethyl | Ethyl | |
| 2.2.260 | 2-Ethyl-3-ethinyl-Phenyl | Fluormethyl | Ethyl | |
| 2.2.261 | 2-Cyclopropyl-3-ethinyl-Phenyl | Fluormethyl | Ethyl | |
| 2.2.262 | 2-Vinyl-3-ethinyl-Phenyl | Fluormethyl | Ethyl | |
| 2.2.263 | 2-Cyano-3-ethinyl-Phenyl | Fluormethyl | Ethyl | |
| 2.2.264 | 2-Trifluormethyl-3-ethinyl-Phenyl | Fluormethyl | Ethyl | |
| 2.2.265 | 2-Methoxy-3-ethinyl-Phenyl | Fluormethyl | Ethyl | |
| 2.2.266 | 2-Ethoxy-3-ethinyl-Phenyl | Fluormethyl | Ethyl | |
| 2.2.267 | 2-Trifluormethoxy-3-ethinyl--Phenyl | Fluormethyl | Ethyl | |
| 2.2.268 | 2-Nitro-3-ethinyl-Phenyl | Fluormethyl | Ethyl | |
| 2.2.269 | 2-Fluor-3-ethinyl-Phenyl | Fluormethyl | Ethyl | |
| 2.2.270 | 2-Fluor-3-cyano-Phenyl | Fluormethyl | Ethyl | |
| 2.2.271 | 2-Chlor-3-cyano-Phenyl | Fluormethyl | Ethyl | |
| 2.2.272 | 2-Brom-3-cyano-Phenyl | Fluormethyl | Ethyl | |
| 2.2.273 | 2-Methyl-3-cyano-Phenyl | Fluormethyl | Ethyl | |
| 2.2.274 | 2-Ethyl-3-cyano-Phenyl | Fluormethyl | Ethyl | |
| 2.2.275 | 2-Ethyl-3-cyano-Phenyl | Chlormethyl | Ethyl | |
| 2.2.276 | 2-Ethyl-3-cyano-Phenyl | Brommethyl | Ethyl | |
| 2.2.277 | 2-Ethyl-3-cyano-Phenyl | Difluormethyl | Ethyl | |
| 2.2.278 | 2-Cyclopropyl-3-cyano-Phenyl | Fluormethyl | Ethyl | |
| 2.2.279 | 2-Vinyl-3-cyano-Phenyl | Fluormethyl | Ethyl | |
| 2.2.280 | 2-Ethinyl-3-cyano-Phenyl | Fluormethyl | Ethyl | |
| 2.2.281 | 2-Cyano-3-cyanoPhenyl | Fluormethyl | Ethyl | |
| 2.2.282 | 2-Trifluormethyl-3-cyano-Phenyl | Fluormethyl | Ethyl | |
| 2.2.283 | 2-Methoxy-3-cyano-Phenyl | Fluormethyl | Ethyl | |
| 2.2.284 | 2-Ethoxy-3-cyano-Phenyl | Fluormethyl | Ethyl | |
| 2.2.285 | 2-Trifluormethoxy-3-cyano-Phenyl | Fluormethyl | Ethyl | |
| 2.2.286 | 2-Nitro-3-cyano-Phenyl | Fluormethyl | Ethyl | |
| 2.2.287 | 2-Fluor-3-hydroxy-Phenyl | Fluormethyl | Ethyl | |
| 2.2.288 | 2-Chlor-3-hydroxy-Phenyl | Fluormethyl | Ethyl | |
| 2.2.289 | 2-Brom-3-hydroxy-Phenyl | Fluormethyl | Ethyl | |
| 2.2.290 | 2-Methyl-3-hydroxy-Phenyl | Fluormethyl | Ethyl | |
| 2.2.291 | 2-Ethyl-3-hydroxy-Phenyl | Fluormethyl | Ethyl | |
| 2.2.292 | 2-Cyclopropyl-3-hydroxy-Phenyl | Fluormethyl | Ethyl | |
| 2.2.293 | 2-Vinyl-3-hydroxy-Phenyl | Fluormethyl | Ethyl | |
| 2.2.294 | 2-Ethinyl-3-hydroxy-Phenyl | Fluormethyl | Ethyl | |
| 2.2.295 | 2-Cyano-3-hydroxy-Phenyl | Fluormethyl | Ethyl | |
| 2.2.296 | 2-Trifluormethyl-3-hydroxy-Phenyl | Fluormethyl | Ethyl | |
| 2.2.297 | 2-Methoxy-3-hydroxy-Phenyl | Fluormethyl | Ethyl | |
| 2.2.298 | 2-Ethoxy-3-hydroxy-Phenyl | Fluormethyl | Ethyl | |
| 2.2.299 | 2-Trifluormethoxy-3-hydroxy-Phenyl | Fluormethyl | Ethyl | |
| 2.2.300 | 2-Nitro-3-hydroxy-Phenyl | Fluormethyl | Ethyl | |
| 2.2.301 | 2-Fluor-3-methoxy-Phenyl | Fluormethyl | Ethyl | |
| 2.2.302 | 2-Chlor-3-methoxy-Phenyl | Fluormethyl | Ethyl | |
| 2.2.303 | 2-Brom-3-methoxy-Phenyl | Fluormethyl | Ethyl | |
| 2.2.304 | 2-Methyl-3-methoxy-Phenyl | Fluormethyl | Ethyl | |
| 2.2.305 | 2-Ethyl-3-methoxy-Phenyl | Fluormethyl | Ethyl | |
| 2.2.306 | 2-Cyclopropyl-3-methoxy-Phenyl | Fluormethyl | Ethyl | |
| 2.2.307 | 2-Vinyl-3-methoxy-Phenyl | Fluormethyl | Ethyl | |
| 2.2.308 | 2-Ethinyl-3-methoxy-Phenyl | Fluormethyl | Ethyl | |
| 2.2.309 | 2-Cyano-3-methoxy-Phenyl | Fluormethyl | Ethyl | |
| 2.2.310 | 2-Trifluormethyl-3-methoxy-Phenyl | Fluormethyl | Ethyl | |
| 2.2.311 | 2,3-Dimethoxy--Phenyl | Fluormethyl | Ethyl | |
| 2.2.312 | 2-Ethoxy-3-methoxy-Phenyl | Fluormethyl | Ethyl | |
| 2.2.313 | 2-Trifluormethoxy-3-methoxy-Phenyl | Fluormethyl | Ethyl | |
| 2.2.314 | 2-Nitro-3-methoxy-Phenyl | Fluormethyl | Ethyl | |
| 2.2.315 | 2-Fluor-3-ethoxy-Phenyl | Fluormethyl | Ethyl | |
| 2.2.316 | 2-Chlor-3-ethoxy-Phenyl | Fluormethyl | Ethyl | |
| 2.2.317 | 2-Brom-3-ethoxy-Phenyl | Fluormethyl | Ethyl | |
| 2.2.318 | 2-Methyl-3-ethoxy-Phenyl | Fluormethyl | Ethyl | |
| 2.2.319 | 2-Ethyl-3-ethoxy-Phenyl | Fluormethyl | Ethyl | |
| 2.2.320 | 2-Cyclopropyl-3-ethoxy-Phenyl | Fluormethyl | Ethyl | |
| 2.2.321 | 2-Vinyl-3-ethoxy-Phenyl | Fluormethyl | Ethyl | |
| 2.2.322 | 2-Ethinyl-3-ethoxy-Phenyl | Fluormethyl | Ethyl | |
| 2.2.323 | 2-Cyano-3-ethoxy-Phenyl | Fluormethyl | Ethyl | |
| 2.2.324 | 2-Trifluormethyl-3-ethoxy-Phenyl | Fluormethyl | Ethyl | |
| 2.2.325 | 2-Methoxy-3-ethoxy-Phenyl | Fluormethyl | Ethyl | |
| 2.2.326 | 2,3-Diethoxy--Phenyl | Fluormethyl | Ethyl | |
| 2.2.327 | 2-Trifluormethoxy-3-ethoxy-Phenyl | Fluormethyl | Ethyl | |
| 2.2.328 | 2-Nitro-3-ethoxy-Phenyl | Fluormethyl | Ethyl | |
| 2.2.329 | 2-Fluor-3-propoxy-Phenyl | Fluormethyl | Ethyl | |
| 2.2.330 | 2-Chlor-3-propoxy-Phenyl | Fluormethyl | Ethyl | |
| 2.2.331 | 2-Brom-3-propoxy-Phenyl | Fluormethyl | Ethyl | |
| 2.2.332 | 2-Methyl-3-propoxy-Phenyl | Fluormethyl | Ethyl | |
| 2.2.333 | 2-Ethyl-3-propoxy-Phenyl | Fluormethyl | Ethyl | |
| 2.2.334 | 2-Cyclopropyl-3-propoxy-Phenyl | Fluormethyl | Ethyl | |
| 2.2.335 | 2-Vinyl-3-propoxy-Phenyl | Fluormethyl | Ethyl | |
| 2.2.336 | 2-Ethinyl-3-propoxy-Phenyl | Fluormethyl | Ethyl | |
| 2.2.337 | 2-Cyano-3-propoxy-Phenyl | Fluormethyl | Ethyl | |
| 2.2.338 | 2-Trifluormethyl-3-propoxy-Phenyl | Fluormethyl | Ethyl | |
| 2.2.339 | 2-Methoxy-3-propoxy-Phenyl | Fluormethyl | Ethyl | |
| 2.2.340 | 2-Ethoxy-3-propoxy-Phenyl | Fluormethyl | Ethyl | |
| 2.2.341 | 2-Trifluormethoxy-3-propoxy-Phenyl | Fluormethyl | Ethyl | |
| 2.2.342 | 2-Nitro-3-propoxy-Phenyl | Fluormethyl | Ethyl | |
| 2.2.343 | 2-Fluor-3-isopropoxy-Phenyl | Fluormethyl | Ethyl | |
| 2.2.344 | 2-Chlor-3-isopropoxy-Phenyl | Fluormethyl | Ethyl | |
| 2.2.345 | 2-Brom-3-isopropoxy-Phenyl | Fluormethyl | Ethyl | |
| 2.2.346 | 2-Methyl-3-isopropoxy-Phenyl | Fluormethyl | Ethyl | |
| 2.2.347 | 2-Ethyl-3-isopropoxy-Phenyl | Fluormethyl | Ethyl | |
| 2.2.348 | 2-Cyclopropyl-3-isopropoxy-Phenyl | Fluormethyl | Ethyl | |
| 2.2.349 | 2-Vinyl-3-isopropoxy-Phenyl | Fluormethyl | Ethyl | |
| 2.2.350 | 2-Ethinyl-3-isopropoxy-Phenyl | Fluormethyl | Ethyl | |
| 2.2.351 | 2-Cyano-3-isopropoxy-Phenyl | Fluormethyl | Ethyl | |
| 2.2.352 | 2-Trifluormethyl-3-isopropoxy-Phenyl | Fluormethyl | Ethyl | |
| 2.2.353 | 2-Methoxy-3-isopropoxy-Phenyl | Fluormethyl | Ethyl | |
| 2.2.354 | 2-Ethoxy-3-isopropoxy-Phenyl | Fluormethyl | Ethyl | |
| 2.2.355 | 2-Trifluormethoxy-3-isopropoxy-Phenyl | Fluormethyl | Ethyl | |
| 2.2.356 | 2-Nitro-3-isopropoxy-Phenyl | Fluormethyl | Ethyl | |
| 2.2.357 | 2-Fluor-3-tert.butoxy-Phenyl | Fluormethyl | Ethyl | |
| 2.2.358 | 2-Chlor-3-tert.butoxy-Phenyl | Fluormethyl | Ethyl | |
| 2.2.359 | 2-Brom-3-tert.butoxy-Phenyl | Fluormethyl | Ethyl | |
| 2.2.360 | 2-Methyl-3-tert.butoxy-Phenyl | Fluormethyl | Ethyl | |
| 2.2.361 | 2-Ethyl-3-tert.butoxy-Phenyl | Fluormethyl | Ethyl | |
| 2.2.362 | 2-Cyclopropyl-3-tert.butoxy-Phenyl | Fluormethyl | Ethyl | |
| 2.2.363 | 2-Vinyl-3-tert.butoxy-Phenyl | Fluormethyl | Ethyl | |
| 2.2.364 | 2-Ethinyl-3-tert.butoxy-Phenyl | Fluormethyl | Ethyl | |
| 2.2.365 | 2-Cyano-3-tertbutoxy-Phenyl | Fluormethyl | Ethyl | |
| 2.2.366 | 2-Trifluormethyl-3-tert.butoxy-Phenyl | Fluormethyl | Ethyl | |
| 2.2.367 | 2-Methoxy-3-tert.butoxy-Phenyl | Fluormethyl | Ethyl | |
| 2.2.368 | 2-Ethoxy-3-tert.butoxy-Phenyl | Fluormethyl | Ethyl | |
| 2.2.369 | 2-Trifluormethoxy-3-tert.butoxy-Phenyl | Fluormethyl | Ethyl | |
| 2.2.370 | 2-Nitro-3-tert.butoxy-Phenyl | Fluormethyl | Ethyl | |
| 2.2.371 | 2-Fluor-3-trifluormethoxy-Phenyl | Fluormethyl | Ethyl | |
| 2.2.372 | 2-Chlor-3-trifluormethoxy-Phenyl | Fluormethyl | Ethyl | |
| 2.2.373 | 2-Brom-3-trifluormethoxy-Phenyl | Fluormethyl | Ethyl | |
| 2.2.374 | 2-Methyl-3-trifluormethoxy-Phenyl | Fluormethyl | Ethyl | |
| 2.2.375 | 2-Ethyl-3-trifluormethoxy-Phenyl | Fluormethyl | Ethyl | |
| 2.2.376 | 2-Cyclopropyl-3-trifluormethoxy-Phenyl | Fluormethyl | Ethyl | |
| 2.2.377 | 2-Vinyl-3-trifluormethoxy-Phenyl | Fluormethyl | Ethyl | |
| 2.2.378 | 2-Ethinyl-3-trifluormethoxy-Phenyl | Fluormethyl | Ethyl | |
| 2.2.379 | 2-Cyano-3-trifluormethoxy-Phenyl | Fluormethyl | Ethyl | |
| 2.2.380 | 2-Trifluormethyl-3-trifluormethoxy-Phenyl | Fluormethyl | Ethyl | |
| 2.2.381 | 2-Methoxy-3-trifluormethoxy-Phenyl | Fluormethyl | Ethyl | |
| 2.2.382 | 2-Ethoxy-3-trifluormethoxy-Phenyl | Fluormethyl | Ethyl | |
| 2.2.383 | 2,3-Bis(trifluormethoxy)-Phenyl | Fluormethyl | Ethyl | |
| 2.2.384 | 2-Nitro-3-trifluormethoxy-Phenyl | Fluormethyl | Ethyl | |
| 2.2.385 | 2-Fluor-3-(2,2,2-trifluorethoxy)-Phenyl | Fluormethyl | Ethyl | |
| 2.2.386 | 2-Chlor-3-(2,2,2-trifluorethoxy)-Phenyl | Fluormethyl | Ethyl | |
| 2.2.387 | 2-Brom-3-(2,2,2-trifluorethoxy)-Phenyl | Fluormethyl | Ethyl | |
| 2.2.388 | 2-Methyl-3-(2,2,2-trifluorethoxy)-Phenyl | Fluormethyl | Ethyl | |
| 2.2.389 | 2-Ethyl-3-(2,2,2-trifluorethoxy)-Phenyl | Fluormethyl | Ethyl | |
| 2.2.390 | 2-Cyclopropyl-3-(2,2,2-trifluorethoxy)-Phenyl | Fluormethyl | Ethyl | |
| 2.2.391 | 2-Vinyl-3-(2,2,2-trifluorethoxy)-Phenyl | Fluormethyl | Ethyl | |
| 2.2.392 | 2-Ethinyl-3-(2,2,2-trifluorethoxy-Phenyl | Fluormethyl | Ethyl | |
| 2.2.393 | 2-Cyano-3-(2,2,2-trifluorethoxy)-Phenyl | Fluormethyl | Ethyl | |
| 2.2.394 | 2-Trifluormethyl-3-(2,2,2-trifluorethoxy)-Phenyl | Fluormethyl | Ethyl | |
| 2.2.395 | 2-Methoxy-3-(2,2,2-trifluorethoxy)-Phenyl | Fluormethyl | Ethyl | |
| 2.2.396 | 2-Ethoxy-3-(2,2,2-trifluorethoxy)-Phenyl | Fluormethyl | Ethyl | |
| 2.2.397 | 2-Trifluormethoxy-3-(2,2,2-trifluorethoxy)-Phenyl | Fluormethyl | Ethyl | |
| 2.2.398 | 2-Nitro-3-(2,2,2-trifluorethoxy)-Phenyl | Fluormethyl | Ethyl | |
| 2.2.399 | 2-Fluor-3-difluormethoxy-Phenyl | Fluormethyl | Ethyl | |
| 2.2.400 | 2-Chlor-3-difluormethoxy-Phenyl | Fluormethyl | Ethyl | |
| 2.2.401 | 2-Brom-3-difluormethoxy-Phenyl | Fluormethyl | Ethyl | |
| 2.2.402 | 2-Methyl-3-difluormethoxy-Phenyl | Fluormethyl | Ethyl | |
| 2.2.403 | 2-Ethyl-3-difluormethoxy-Phenyl | Fluormethyl | Ethyl | |
| 2.2.404 | 2-Cyclopropyl-3-difluormethoxy-Phenyl | Fluormethyl | Ethyl | |
| 2.2.405 | 2-Vinyl-3-difluormethoxy-Phenyl | Fluormethyl | Ethyl | |
| 2.2.406 | 2-Ethinyl-3-difluormethoxy-Phenyl | Fluormethyl | Ethyl | |
| 2.2.407 | 2-Cyano-3-difluormethoxy -Phenyl | Fluormethyl | Ethyl | |
| 2.2.408 | 2-Trifluormethyl-3-difluormethoxy -Phenyl | Fluormethyl | Ethyl | |
| 2.2.409 | 2-Methoxy-3-difluormethoxy -Phenyl | Fluormethyl | Ethyl | |
| 2.2.410 | 2-Ethoxy-3-difluormethoxy-Phenyl | Fluormethyl | Ethyl | |
| 2.2.411 | 2-Trifluormethoxy-3-difluormethoxy-Phenyl | Fluormethyl | Ethyl | |
| 2.2.412 | 2-Nitro-3-difluormethoxy-Phenyl | Fluormethyl | Ethyl | |
| 2.2.413 | 2-Fluor-3-(2-methoxyethoxy)-Phenyl | Fluormethyl | Ethyl | |
| 2.2.414 | 2-Chlor-3-(2-methoxyethoxy)-Phenyl | Fluormethyl | Ethyl | |
| 2.2.415 | 2-Brom-3-(2-methoxyethoxy)-Phenyl | Fluormethyl | Ethyl | |
| 2.2.416 | 2-Methyl-3-(2-methoxyethoxy)-Phenyl | Fluormethyl | Ethyl | |
| 2.2.417 | 2-Ethyl-3-(2-methoxyethoxy)-Phenyl | Fluormethyl | Ethyl | |
| 2.2.418 | 2-Cyclopropyl-3-(2-methoxyethoxy)-Phenyl | Fluormethyl | Ethyl | |
| 2.2.419 | 2-Vinyl-3-(2-methoxyethoxy)-Phenyl | Fluormethyl | Ethyl | |
| 2.2.420 | 2-Ethinyl-3-(2-methoxyethoxy)-Phenyl | Fluormethyl | Ethyl | |
| 2.2.421 | 2-Cyano-3-(2-methoxyethoxy)-Phenyl | Fluormethyl | Ethyl | |
| 2.2.422 | 2-Trifluormethyl-3-(2-methoxyethoxy)-Phenyl | Fluormethyl | Ethyl | |
| 2.2.423 | 2-Methoxy-3-(2-methoxyethoxy)-Phenyl | Fluormethyl | Ethyl | |
| 2.2.424 | 2-Ethoxy-3-(2-methoxyethoxy)-Phenyl | Fluormethyl | Ethyl | |
| 2.2.425 | 2-Trifluormethoxy-(2-methoxyethoxy)-Phenyl | Fluormethyl | Ethyl | |
| 2.2.426 | 2-Nitro-3-(2-methoxyethoxy)-Phenyl | Fluormethyl | Ethyl | |
| 2.2.427 | 2-Fluor-3-(tert.butoxycarbonyloxy)-Phenyl | Fluormethyl | Ethyl | |
| 2.2.428 | 2-Chlor-3-(tert.butoxycarbonyloxy)-Phenyl | Fluormethyl | Ethyl | |
| 2.2.429 | 2-Brom-3-(tert.butoxycarbonyloxy)-Phenyl | Fluormethyl | Ethyl | |
| 2.2.430 | 2-Methyl-3-(tert.butoxycarbonyloxy)-Phenyl | Fluormethyl | Ethyl | |
| 2.2.431 | 2-Ethyl-3-(tert.butoxycarbonyloxy)-Phenyl | Fluormethyl | Ethyl | |
| 2.2.432 | 2-Cyclopropyl-3-(tert.butoxycarbonyloxy)-Phenyl | Fluormethyl | Ethyl | |
| 2.2.433 | 2-Vinyl-3-(tert.butoxycarbonyloxy)-Phenyl | Fluormethyl | Ethyl | |
| 2.2.434 | 2-Ethinyl-3-(tert.butoxycarbonyloxy)-Phenyl | Fluormethyl | Ethyl | |
| 2.2.435 | 2-Cyano-3-(tert.butoxycarbonyloxy)-Phenyl | Fluormethyl | Ethyl | |
| 2.2.436 | 2-Trifluormethyl-3-(tert.butoxycarbonyloxy)-Phenyl | Fluormethyl | Ethyl | |
| 2.2.437 | 2-Methoxy-3-(tert.butoxycarbonyloxy)-Phenyl | Fluormethyl | Ethyl | |
| 2.2.438 | 2-Ethoxy-3-(tert.butoxycarbonyloxy)-Phenyl | Fluormethyl | Ethyl | |
| 2.2.439 | 2-Trifluormethoxy-3-(tert.butoxycarbonyloxy)-Phenyl | Fluormethyl | Ethyl | |
| 2.2.440 | 2-Nitro-3-(tert.butoxycarbonyloxy)-Phenyl | Fluormethyl | Ethyl | |
| 2.2.441 | 2-Fluor-3-nitro-Phenyl | Fluormethyl | Ethyl | |
| 2.2.442 | 2-Chlor-3-nitro-Phenyl | Fluormethyl | Ethyl | |
| 2.2.443 | 2-Brom-3-nitro-Phenyl | Fluormethyl | Ethyl | |
| 2.2.444 | 2-Methyl-3-nitro-Phenyl | Fluormethyl | Ethyl | |
| 2.2.445 | 2-Ethyl-3-nitro-Phenyl | Fluormethyl | Ethyl | |
| 2.2.446 | 2-Cyclopropyl-3-nitro-Phenyl | Fluormethyl | Ethyl | |
| 2.2.447 | 2-Vinyl-3-nitro-Phenyl | Fluormethyl | Ethyl | |
| 2.2.448 | 2-Ethinyl-3-nitro-Phenyl | Fluormethyl | Ethyl | |
| 2.2.449 | 2-Cyano-3-nitro-Phenyl | Fluormethyl | Ethyl | |
| 2.2.450 | 2-Trifluormethyl-3-nitro-Phenyl | Fluormethyl | Ethyl | |
| 2.2.451 | 2-Methoxy-3-nitro-Phenyl | Fluormethyl | Ethyl | |
| 2.2.452 | 2-Ethoxy-3-nitro-Phenyl | Fluormethyl | Ethyl | |
| 2.2.453 | 2-Trifluormethoxy-3-nitro-Phenyl | Fluormethyl | Ethyl | |
| 2.2.454 | 2-Fluor-3-methylsulfanylPhenyl | Fluormethyl | Ethyl | |
| 2.2.455 | 2-Chlor-3-methylsulfanyl-Phenyl | Fluormethyl | Ethyl | |
| 2.2.456 | 2-Brom-3-methylsulfanyl-Phenyl | Fluormethyl | Ethyl | |
| 2.2.457 | 2-Methyl-3-methylsulfanyl-Phenyl | Fluormethyl | Ethyl | |
| 2.2.458 | 2-Ethyl-3-methylsulfanyl-Phenyl | Fluormethyl | Ethyl | |
| 2.2.459 | 2-Cyclopropyl-3-methylsulfanyl-Phenyl | Fluormethyl | Ethyl | |
| 2.2.460 | 2-Vinyl-3-methylsulfanyl-Phenyl | Fluormethyl | Ethyl | |
| 2.2.461 | 2-Ethinyl-3-methylsulfanyl-Phenyl | Fluormethyl | Ethyl | |
| 2.2.462 | 2-Cyano-3-methylsulfanyl -Phenyl | Fluormethyl | Ethyl | |
| 2.2.463 | 2-Trifluormethyl-3-methylsulfanyl-Phenyl | Fluormethyl | Ethyl | |
| 2.2.464 | 2-Methoxy-3-methylsulfanyl -Phenyl | Fluormethyl | Ethyl | |
| 2.2.465 | 2-Ethoxy-3-methylsulfanyl-Phenyl | Fluormethyl | Ethyl | |
| 2.2.466 | 2-Trifluormethoxy-3-methylsulfanyl-Phenyl | Fluormethyl | Ethyl | |
| 2.2.467 | 2-Nitro-3-methylsulfanyl-Phenyl | Fluormethyl | Ethyl | |
| 2.2.468 | 3,5-Difluor-Phenyl | F | Methyl | [CDCl3] 3.65 (dd,1H); 3.96 (s,3H); 4.12 (dd,1H); 6.96 (t,1H); 7.22 (d,2H). |
| 2.2.469 | 3,5-Difluor-Phenyl | Fluormethyl | Ethyl | [CDCl₃] 1.33 (t,3H); 3.50 (d,1H); 3.80 (d,1H); 4.30 (q,2H); 4.67 (s,1H); 4.79 (s,1H); 6.89 (t,1H); 7.20 (d,2H). |
| 2.2.470 | 3,5-Difluor-Phenyl | Chlormethyl | Ethyl | [CDCl₃] 1.34 (t,3H); 3.50 (d,1H); 3.80 (d,1H); 4.00 (dd,2H); 4.32 (m,2H); 6.89 (t,1H); 7.20 (d,2H). |
| 2.2.471 | 3,5-Difluor-Phenyl | Brommethyl | Ethyl | [CDCl₃] 1.36 (t, 3H); 3.48 (1H) und 3.55 (d,1H); 3.89 (d, 1H) und 4.01 (d, 1H); 4.32 (m, 2H); 6.89 (m, 1H); 7.23 (m, 2H). |
| 2.2.472 | 3,5-Difluor-Phenyl | Difluormethyl | Ethyl | [CDCl₃] 1.36 (t,3H); 3.63 (d,1H); 3.91 (d,1H); 4.35 (q,2H); 6.25 (tt,1H); 6.91 (t,1H), 7.21 (d,2H). |
| 2.2.473 | 3,5-Difluor-Phenyl | Trifluormethyl | Ethyl | |
| 2.2.474 | 3,5-Difluor-Phenyl | 1,1-Dichlorethyl | Methyl | [CDCl₃] 2.40 (s,3H); 3.88 (s,3H); 3.98 (d,1H) 4.10 (d,1H); 6.91 (t,1H); 7.21 (d,2H). |
| 2.2.475 | 3,5-Difluor-Phenyl | CN | Ethyl | [DMSO] 1.30 (t, 3H); 4.11 (d, 1H); 4.32 (q, 2H); 4.48 (d, 1H); 7.48 (m, 3H). |
| 2.2.476 | 3,5-Difluor-Phenyl | CN | isoPropyl | [CDCl₃] 1.40 (m,6H); 3.91 (d,1H); 4.12 (d,1H); 5.20 (m,1H); 6.96 (m,1H); 7.19 (m,2H). |
| 2.2.477 | 3-Chlor-5-fluor-Phenyl | Fluormethyl | Ethyl | |
| 2.2.478 | 3-Chlor-5-fluor-Phenyl | Chlormethyl | Ethyl | |
| 2.2.479 | 3-Chlor-5-fluor-Phenyl | Brommethyl | Ethyl | |
| 2.2.480 | 3-Chlor-5-fluor-Phenyl | Difluormethyl | Ethyl | |
| 2.2.481 | 3-Chlor-5-fluor-Phenyl | Cyano | Ethyl | [CDCl₃] 1.45 (t, 3H); 3.91 (d, 1H); 4.15 (d, 1H); 4.42 (q, 2H); 7.25 (m, 1 H); 7.32 (d, 1H); 7.40 (s, 1H). |
| 2.2.482 | 3-Brom-5-fluor-Phenyl | Fluormethyl | Ethyl | |
| 2.2.483 | 3-Brom-5-fluor-Phenyl | Chlormethyl | Ethyl | |
| 2.2.484 | 3-Brom-5-fluor-Phenyl | Brommethyl | Ethyl | |
| 2.2.485 | 3-Brom-5-fluor-Phenyl | Difluormethyll | Ethyl | |
| 2.2.486 | 3-Iod-5-fluor-Phenyl | Fluormethyl | Ethyl | |
| 2.2.487 | 3-Methyl-5-fluor-Phenyl | Fluormethyl | Ethyl | |
| 2.2.488 | 3-Methyl-5-fluor-Phenyl | Chlormethyl | Ethyl | |
| 2.2.489 | 3-Methyl-5-fluor-Phenyl | Brommethyl | Ethyl | |
| 2.2.490 | 3-Methyl-5-fluor-Phenyl | Difluormethyl | Ethyl | |
| 2.2.491 | 3-Ethyl-5-fluor-Phenyl | Fluormethyl | Ethyl | |
| 2.2.492 | 3-Propyl-5-fluor-Phenyl | Fluormethyl | Ethyl | |
| 2.2.493 | 3-iPropyl-5-fluor-Phenyl | Fluormethyl | Ethyl | |
| 2.2.494 | 3-nButyl-5-fluor-Phenyl | Fluormethyl | Ethyl | |
| 2.2.495 | 3-isoButyl-5-fluor-Phenyl | Fluormethyl | Ethyl | |
| 2.2.496 | 3-tert.Butyl-5-fluor-Phenyl | Fluormethyl | Ethyl | |
| 2.2.497 | 3-Cyclopropyl-5-fluor-Phenyl | Fluormethyl | Ethyl | |
| 2.2.498 | 3-Vinyl-5-fluor-Phenyl | Fluormethyl | Ethyl | |
| 2.2.499 | 3-Ethinyl-5-fluor-Phenyl | Fluormethyl | Ethyl | |
| 2.2.500 | 3-Cyano-5-fluor-Phenyl | Fluormethyl | Ethyl | |
| 2.2.501 | 3-Trifluormethyl-5-fluor-Phenyl | Fluormethyl | Ethyl | |
| 2.2.502 | 3-Trifluormethyl-5-fluor-Phenyl | Chlormethyl | Ethyl | |
| 2.2.503 | 3-Trifluormethyl-5-fluor-Phenyl | Brommethyl | Ethyl | |
| 2.2.504 | 3-Trifluormethyl-5-fluor-Phenyl | Difluormethyl | Ethyl | |
| 2.2.505 | 3-(Methoxycarbony)l-5-fluor-Phenyl | Fluormethyl | Ethyl | |
| 2.2.506 | 3-Hydroxymethyl-5-fluor-Phenyl | Fluormethyl | Ethyl | |
| 2.2.507 | 3-Carbamoyl-5-fluor-Phenyl | Fluormethyl | Ethyl | |
| 2.2.508 | 3-Hydroxy-5-fluor-Phenyl- | Fluormethyl | Ethyl | |
| 2.2.509 | 3-Methoxy-5-fluor-Phenyl | Fluormethyl | Ethyl | |
| 2.2.510 | 3-Ethoxy-5-fluor-Phenyl | Fluormethyl | Ethyl | |
| 2.2.511 | 3-nPropyoxy-5-fluor-Phenyl | Fluormethyl | Ethyl | |
| 2.2.512 | 3-isoPropoxy-5-fluor-Phenyl | Fluormethyl | Ethyl | |
| 2.2.513 | 3-nButoxy-5-fluor-Phenyl | Fluormethyl | Ethyl | |
| 2.2.514 | 3-isoButoxy-5-fluor-Phenyl | Fluormethyl | Ethyl | |
| 2.2.515 | 3-tert. Butoxy-5-fluor-Phenyl | Fluormethyl | Ethyl | |
| 2.2.516 | 3-Difluormethoxy-5-fluor-Phenyl | Fluormethyl | Ethyl | |
| 2.2.517 | 3-Trifluormethoxy-5-fluor-Phenyl | Fluormethyl | Ethyl | |
| 2.2.518 | 3-(2,2,2-Trifluorethoxy)-5-fluor-Phenyl | Fluormethyl | Ethyl | |
| 2.2.519 | 3-(2-Chlorethoxy)-5-fluor-Phenyl | Fluormethyl | Ethyl | |
| 2.2.520 | 3-(2-Hyd roxyethoxy)-5-fluor-Phenyl- | Fluormethyl | Ethyl | |
| 2.2.521 | 3-[(tert-Butoxycarbonyl)oxy]-5-fluor-Phenyl | Fluormethyl | Ethyl | |
| 2.2.522 | 3-Nitro-5-fluor-Phenyl | Fluormethyl | Ethyl | |
| 2.2.523 | 3-Acetoxy-5-fluor-Phenyl | Fluormethyl | Ethyl | |
| 2.2.524 | {3-[(tert-Butoxycarbonyl)amino] -5-fluor-Phenyl} | Fluormethyl | Ethyl | |
| 2.2.525 | 3-Methylsulfanyl-5-fluor-Phenyl | Fluormethyl | Ethyl | |
| 2.2.526 | 3,5-Dichlor-Phenyl | Fluormethyl | Ethyl | |
| 2.2.527 | 3,5-Dichlor-Phenyl | Chlormethyl | Ethyl | [CDCl₃] 1.35 (t, 3H); 3.51 (d, 1H); 3.80 (d, 1H); 3.96 (d, 1H); 4.03 (d, 1 H); 4.33 (m, 2H); 7.43 (s, 1H); 7.58 (s, 2H). |
| 2.2.528 | 3,5-Dichlor-Phenyl | Brommethyl | Ethyl | [CDCl₃] 1.35 (t, 3H); 3.47 (d, 1H); 3.62 (d, 1H); 3.90 (d, 1H); 4.32 (m, 2H); 7.40 (s, 1H); 7.55 (s, 2H). |
| 2.2.529 | 3,5-Dichlor-Phenyl | Difluormethyl | Ethyl | |
| 2.2.530 | 3,5-Dichlor-Phenyl | Trifluormethyl | Methyl | [CDCl₃] 3.75 (d, 1H); 3.92 (s, 3H); 4.00 (d, 1H); 7.45 (s, 1H); 7.58 (s, 2H). |
| 2.2.531 | 3,5-Dichlor-Phenyl | Cyano | Ethyl | [CDCl₃] 1.41 (t, 3H); 3.90 (d, 1H); 4.15 (d, 1H); 4.42 (q, 2H); 7.49 (d, 1 H); 7.55 (d, 2H). |
| 2.2.532 | 3-Brom-5-chlor-Phenyl | Fluormethyl | Ethyl | |
| 2.2.533 | 3-Iod-5-chlor-Phenyl | Fluormethyl | Ethyl | |
| 2.2.534 | 3-Methyl-5-chlor-Phenyl | Fluormethyl | Ethyl | |
| 2.2.535 | 3-Ethyl-5-chlor-Phenyl | Fluormethyl | Ethyl | |
| 2.2.536 | 3-Propyl-5-chlor-Phenyl | Fluormethyl | Ethyl | |
| 2.2.537 | 3-isoPropyl-5-chlor-Phenyl | Fluormethyl | Ethyl | |
| 2.2.538 | 3-nButyl-5-chlor-Phenyl | Fluormethyl | Ethyl | |
| 2.2.539 | 3-isoButyl-5-chlor-Phenyl | Fluormethyl | Ethyl | |
| 2.2.540 | 3-tert.Butyl-5-chlor-Phenyl | Fluormethyl | Ethyl | |
| 2.2.541 | 3-Cyclopropyl-5-chlor-Phenyl | Fluormethyl | Ethyl | |
| 2.2.542 | 3-Vinyl-5-chlor-Phenyl | Fluormethyl | Ethyl | |
| 2.2.543 | 3-Ethinyl-5-chlor-Phenyl | Fluormethyl | Ethyl | |
| 2.2.544 | 3-Cyano-5-chlor-Phenyl | Fluormethyl | Ethyl | |
| 2.2.545 | 3-Trifluormethyl-5-chlor-Phenyl | Fluormethyl | Ethyl | |
| 2.2.546 | 3-(Hydroxycarbonyl)-5-chlor-Phenyl | Fluormethyl | Ethyl | |
| 2.2.547 | 3-(Methoxycarbony)l-5-chlor-Phenyl | Fluormethyl | Ethyl | |
| 2.2.548 | 3-Hydroxymethyl-5-chlor-Phenyl | Fluormethyl | Ethyl | |
| 2.2.549 | 3-Carbamoyl-5-chlor-Phenyl | Fluormethyl | Ethyl | |
| 2.2.550 | 3-Hydroxy-5-chlor-Phenyl- | Fluormethyl | Ethyl | |
| 2.2.551 | 3-Methoxy-5-chlor-Phenyl | Fluormethyl | Ethyl | |
| 2.2.552 | 3-Ethoxy-5-chlor-Phenyl | Fluormethyl | Ethyl | |
| 2.2.553 | 3-nPropyoxy-5-chlor-Phenyl | Fluormethyl | Ethyl | |
| 2.2.554 | 3-isoPropoxy-5-chlor-Phenyl | Fluormethyl | Ethyl | |
| 2.2.555 | 3-nButoxy-5-chlor-Phenyl | Fluormethyl | Ethyl | |
| 2.2.556 | 3-isoButoxy-5-chlor-Phenyl | Fluormethyl | Ethyl | |
| 2.2.557 | 3-tert. Butoxy-5-chlor-Phenyl | Fluormethyl | Ethyl | |
| 2.2.558 | 3-Difluormethoxy-5-chlor-Phenyl | Fluormethyl | Ethyl | |
| 2.2.559 | 3-Trifluormethoxy-5-chlor-Phenyl | Fluormethyl | Ethyl | |
| 2.2.560 | 3-(2,2,2-Trifluorethoxy)-5-chlor-Phenyl | Fluormethyl | Ethyl | |
| 2.2.561 | 3-(2-Chlorethoxy)-5-chlor-Phenyl | Fluormethyl | Ethyl | |
| 2.2.562 | 3-(2-Hydroxyethoxy)-5-chlor-Phenyl- | Fluormethyl | Ethyl | |
| 2.2.563 | 3-[(tert-Butoxycarbonyl)oxy]-5-chlor-Phenyl | Fluormethyl | Ethyl | |
| 2.2.564 | 3-Nitro-5-chlor-Phenyl | Fluormethyl | Ethyl | |
| 2.2.565 | 3-Acetoxy-5-chlor-Phenyl | Fluormethyl | Ethyl | |
| 2.2.566 | {3-[(tert-Butoxycarbonyl)amino] -5-chlor-Phenyl} | Fluormethyl | Ethyl | |
| 2.2.567 | 3-Methylsulfanyl-5-chlor-Phenyl | Fluormethyl | Ethyl | |
| 2.2.568 | 3,5-Dibrom-Phenyl | Fluormethyl | Ethyl | |
| 2.2.569 | 3,5-Dibrom-Phenyl | Chlormethyl | Ethyl | |
| 2.2.570 | 3-Iod-5-brom-Phenyl | Fluormethyl | Ethyl | |
| 2.2.571 | 3-Methyl-5-brom-Phenyl | Fluormethyl | Ethyl | |
| 2.2.572 | 3-Methyl-5-brom-Phenyl | Chlormethyl | Ethyl | |
| 2.2.573 | 3-Methyl-5-brom-Phenyl | Brommethyl | Ethyl | |
| 2.2.574 | 3-Methyl-5-brom-Phenyl | Difluormethyl | Ethyl | |
| 2.2.575 | 3-Methyl-5-brom-Phenyl | Trifluormethyl | Ethyl | |
| 2.2.576 | 3-Methyl-5-brom-Phenyl | Cyano | Ethyl | |
| 2.2.577 | 3-Ethyl-5-brom-Phenyl | Fluormethyl | Ethyl | |
| 2.2.578 | 3-Propyl-5-brom-Phenyl | Fluormethyl | Ethyl | |
| 2.2.579 | 3-isoPropyl-5-brom-Phenyl | Fluormethyl | Ethyl | |
| 2.2.580 | 3-nButyl-5-brom-Phenyl | Fluormethyl | Ethyl | |
| 2.2.581 | 3-isoButyl-5-brom-Phenyl | Fluormethyl | Ethyl | |
| 2.2.582 | 3-tert. Butyl-5-brom-Phenyl | Fluormethyl | Ethyl | |
| 2.2.583 | 3-Cyclopropyl-5-brom-Phenyl | Fluormethyl | Ethyl | |
| 2.2.584 | 3-Vinyl-5-brom-Phenyl | Fluormethyl | Ethyl | |
| 2.2.585 | 3-Ethinyl-5-brom-Phenyl | Fluormethyl | Ethyl | |
| 2.2.586 | 3-Cyano-5-brom-Phenyl | Fluormethyl | Ethyl | |
| 2.2.587 | 3-Trifluormethyl-5-brom-Phenyl | Fluormethyl | Ethyl | |
| 2.2.588 | 3-(Hydroxycarbonyl)-5-brom-Phenyl | Fluormethyl | Ethyl | |
| 2.2.589 | 3-(Methoxycarbony)l-5-brom-Phenyl | Fluormethyl | Ethyl | |
| 2.2.590 | 3-Hydroxymethyl-5-brom-Phenyl | Fluormethyl | Ethyl | |
| 2.2.591 | 3-Carbamoyl-5-brom-Phenyl | Fluormethyl | Ethyl | |
| 2.2.592 | 3-Hydroxy-5-brom-Phenyl- | Fluormethyl | Ethyl | |
| 2.2.593 | 3-Methoxy-5-brom-Phenyl | Fluormethyl | Ethyl | |
| 2.2.594 | 3-Ethoxy-5-brom-Phenyl | Fluormethyl | Ethyl | |
| 2.2.595 | 3-nPropyoxy-5-brom-Phenyl | Fluormethyl | Ethyl | |
| 2.2.596 | 3-isoPropoxy-5-brom-Phenyl | Fluormethyl | Ethyl | |
| 2.2.597 | 3-nButoxy-5-brom-Phenyl | Fluormethyl | Ethyl | |
| 2.2.598 | 3-isoButoxy-5-brom-Phenyl | Fluormethyl | Ethyl | |
| 2.2.599 | 3-tert. Butoxy-5-brom-Phenyl | Fluormethyl | Ethyl | |
| 2.2.600 | 3-Difluormethoxy-5-brom-Phenyl | Fluormethyl | Ethyl | |
| 2.2.601 | 3-Trifluormethoxy-5-brom-Phenyl | Fluormethyl | Ethyl | |
| 2.2.602 | 3-(2,2,2-Trifluorethoxy)-5-brom-Phenyl | Fluormethyl | Ethyl | |
| 2.2.603 | 3-(2-Chlorethoxy)-5-brom-Phenyl | Fluormethyl | Ethyl | |
| 2.2.604 | 3-(2-Hydroxyethoxy)-5-brom-Phenyl- | Fluormethyl | Ethyl | |
| 2.2.605 | 3-[(tert-Butoxycarbonyl)oxy]-5-brom-Phenyl | Fluormethyl | Ethyl | |
| 2.2.606 | 3-Nitro-5-brom-Phenyl | Fluormethyl | Ethyl | |
| 2.2.607 | 3-Acetoxy-5-brom-Phenyl | Fluormethyl | Ethyl | |
| 2.2.608 | {3-[(tert-Butoxycarbonyl)amino] -5-brom-Phenyl} | Fluormethyl | Ethyl | |
| 2.2.609 | 3-Methylsulfanyl-5-brom-Phenyl | Fluormethyl | Ethyl | |
| 2.2.610 | 3,5-Diiod-Phenyl | Fluormethyl | Ethyl | |
| 2.2.611 | 3-Methyl-5-iod-Phenyl | Fluormethyl | Ethyl | |
| 2.2.612 | 3-Ethyl-5-iod-Phenyl | Fluormethyl | Ethyl | |
| 2.2.613 | 3-Propyl-5-iod-Phenyl | Fluormethyl | Ethyl | |
| 2.2.614 | 3-isoPropyl-5-iod-Phenyl | Fluormethyl | Ethyl | |
| 2.2.615 | 3-nButyl-5-iod-Phenyl | Fluormethyl | Ethyl | |
| 2.2.616 | 3-isoButyl-5-iod-Phenyl | Fluormethyl | Ethyl | |
| 2.2.617 | 3-tert.Butyl-5-iod-Phenyl | Fluormethyl | Ethyl | |
| 2.2.618 | 3-Cyclopropyl-5-iod-Phenyl | Fluormethyl | Ethyl | |
| 2.2.619 | 3-Vinyl-5-iod-Phenyl | Fluormethyl | Ethyl | |
| 2.2.620 | 3-Ethinyl-5-iod-Phenyl | Fluormethyl | Ethyl | |
| 2.2.621 | 3-Cyano-5-iod-Phenyl | Fluormethyl | Ethyl | |
| 2.2.622 | 3-Trifluormethyl-5-iod-Phenyl | Fluormethyl | Ethyl | |
| 2.2.623 | 3-(Hydroxycarbonyl)-5-iod-Phenyl | Fluormethyl | Ethyl | |
| 2.2.624 | 3-(Methoxycarbonyl)-5-iod-Phenyl | Fluormethyl | Ethyl | |
| 2.2.625 | 3-Hydroxymethyl-5-iod-Phenyl | Fluormethyl | Ethyl | |
| 2.2.626 | 3-Carbamoyl-5-iod-Phenyl | Fluormethyl | Ethyl | |
| 2.2.627 | 3-Hydroxy-5-iod-Phenyl- | Fluormethyl | Ethyl | |
| 2.2.628 | 3-Methoxy-5-iod-Phenyl | Fluormethyl | Ethyl | |
| 2.2.629 | 3-Ethoxy-5-iod-Phenyl | Fluormethyl | Ethyl | |
| 2.2.630 | 3-nPropyoxy-5-iod-Phenyl | Fluormethyl | Ethyl | |
| 2.2.631 | 3-isoPropoxy-5-iod-Phenyl | Fluormethyl | Ethyl | |
| 2.2.632 | 3-nButoxy-5-iod-Phenyl | Fluormethyl | Ethyl | |
| 2.2.633 | 3-isoButoxy-5-iod-Phenyl | Fluormethyl | Ethyl | |
| 2.2.634 | 3-tert.Butoxy-5-iod-Phenyl | Fluormethyl | Ethyl | |
| 2.2.635 | 3-Difluormethoxy-5-iod-Phenyl | Fluormethyl | Ethyl | |
| 2.2.636 | 3-Trifluormethoxy-5-iod-Phenyl | Fluormethyl | Ethyl | |
| 2.2.637 | 3-(2,2,2-Trifluorethoxy)-5-iod-Phenyl | Fluormethyl | Ethyl | |
| 2.2.638 | 3-(2-Chlorethoxy)-5-iod-Phenyl | Fluormethyl | Ethyl | |
| 2.2.639 | 3-(2-Hydroxyethoxy)-5-iod-Phenyl- | Fluormethyl | Ethyl | |
| 2.2.640 | 3-[(tert-Butoxycarbonyl)oxy]-5-iod-Phenyl | Fluormethyl | Ethyl | |
| 2.2.641 | 3-Nitro-5-iod-Phenyl | Fluormethyl | Ethyl | |
| 2.2.642 | 3-Acetoxy-5-iod-Phenyl | Fluormethyl | Ethyl | |
| 2.2.643 | {3-[(tert-Butoxycarbonyl)amino] -5-iod-Phenyl} | Fluormethyl | Ethyl | |
| 2.2.644 | 3-Methylsulfanyl-5-iod-Phenyl | Fluormethyl | Ethyl | |
| 2.2.645 | 3,5-Dimethyl-Phenyl | Fluormethyl | Ethyl | |
| 2.2.646 | 3-Ethyl-5-methyl-Phenyl | Fluormethyl | Ethyl | |
| 2.2.647 | 3-Propyl-5-m ethyl-Phenyl | Fluormethyl | Ethyl | |
| 2.2.648 | 3-isoPropyl-5-methyl-Phenyl | Fluormethyl | Ethyl | |
| 2.2.649 | 3-nButyl-5-methyl-Phenyl | Fluormethyl | Ethyl | |
| 2.2.650 | 3-isoButyl-5-methyl-Phenyl | Fluormethyl | Ethyl | |
| 2.2.651 | 3-tert. Butyl-5-methyl-Phenyl | Fluormethyl | Ethyl | |
| 2.2.652 | 3-Cyclopropyl-5-methyl-Phenyl | Fluormethyl | Ethyl | |
| 2.2.653 | 3-Vinyl-5-methyl-Phenyl | Fluormethyl | Ethyl | |
| 2.2.654 | 3-Ethinyl-5-methyl-Phenyl | Fluormethyl | Ethyl | |
| 2.2.655 | 3-Cyano-5-methyl-Phenyl | Fluormethyl | Ethyl | |
| 2.2.656 | 3-Trifluormethyl-5-methyl-Phenyl | Fluormethyl | Ethyl | |
| 2.2.657 | 3-(Hydroxycarbonyl)-5-methyl-Phenyl | Fluormethyl | Ethyl | |
| 2.2.658 | 3-(Methoxycarbony)l-5-methyl-Phenyl | Fluormethyl | Ethyl | |
| 2.2.659 | 3-Hydroxymethyl-5-methyl-Phenyl | Fluormethyl | Ethyl | |
| 2.2.660 | 3-Carbamoyl-5-methyl-Phenyl | Fluormethyl | Ethyl | |
| 2.2.661 | 3-Hydroxy-5-methyl-Phenyl- | Fluormethyl | Ethyl | |
| 2.2.662 | 3-Methoxy-5-methyl-Phenyl | Fluormethyl | Ethyl | |
| 2.2.663 | 3-Ethoxy-5-methyl-Phenyl | Fluormethyl | Ethyl | |
| 2.2.664 | 3-nPropyoxy-5-methyl-Phenyl | Fluormethyl | Ethyl | |
| 2.2.665 | 3-nButoxy-5-methyl-Phenyl | Fluormethyl | Ethyl | |
| 2.2.666 | 3-isoButoxy-5-methyl-Phenyl | Fluormethyl | Ethyl | |
| 2.2.667 | 3-tert. Butoxy-5-methyl-Phenyl | Fluormethyl | Ethyl | |
| 2.2.668 | 3-Difluormethoxy-5-methyl-Phenyl | Fluormethyl | Ethyl | |
| 2.2.669 | 3-Trifluormethoxy-5-methyl-Phenyl | Fluormethyl | Ethyl | |
| 2.2.670 | 3-(2,2,2-Trifluorethoxy)-5-methyl-Phenyl | Fluormethyl | Ethyl | |
| 2.2.671 | 3-(2-Chlorethoxy)-5-methyl-Phenyl | Fluormethyl | Ethyl | |
| 2.2.672 | 3-(2-Hydroxyethoxy)-5-methyl-Phenyl- | Fluormethyl | Ethyl | |
| 2.2.673 | 3-[(tert-Butoxycarbonyl)oxy]-5-methyl-Phenyl | Fluormethyl | Ethyl | |
| 2.2.674 | 3-Nitro-5-methyl-Phenyl | Fluormethyl | Ethyl | |
| 2.2.675 | 3-Acetoxy-5-methyl-Phenyl | Fluormethyl | Ethyl | |
| 2.2.676 | {3-[(tert-Butoxycarbonyl)amino] -5-methyl-Phenyl} | Fluormethyl | Ethyl | |
| 2.2.677 | 3-Methylsulfanyl-5-methyl-Phenyl | Fluormethyl | Ethyl | |
| 2.2.678 | 3,5-Diethyl-Phenyl | Fluormethyl | Ethyl | |
| 2.2.679 | 3-Propyl-5-ethyl-Phenyl | Fluormethyl | Ethyl | |
| 2.2.680 | 3-isoPropyl-5-ethyl-Phenyl | Fluormethyl | Ethyl | |
| 2.2.681 | 3-nButyl-5-ethyl-Phenyl | Fluormethyl | Ethyl | |
| 2.2.682 | 3-isoButyl-5-ethyl-Phenyl | Fluormethyl | Ethyl | |
| 2.2.683 | 3-tert.Butyl-5-ethyl-Phenyl | Fluormethyl | Ethyl | |
| 2.2.684 | 3-Cyclopropyl-5-ethyl-Phenyl | Fluormethyl | Ethyl | |
| 2.2.685 | 3-Vinyl-5-ethyl-Phenyl | Fluormethyl | Ethyl | |
| 2.2.686 | 3-Ethinyl-5-ethyl-Phenyl | Fluormethyl | Ethyl | |
| 2.2.687 | 3-Cyano-5-ethyl-Phenyl | Fluormethyl | Ethyl | |
| 2.2.688 | 3-Trifluormethyl-5-ethyl-Phenyl | Fluormethyl | Ethyl | |
| 2.2.689 | 3-(Hydroxycarbonyl)-5-ethyl-Phenyl | Fluormethyl | Ethyl | |
| 2.2.690 | 3-(Methoxycarbony)l-5-ethyl-Phenyl | Fluormethyl | Ethyl | |
| 2.2.691 | 3-Hydroxymethyl-5-ethyl-Phenyl | Fluormethyl | Ethyl | |
| 2.2.692 | 3-Carbamoyl-5-ethyl-Phenyl | Fluormethyl | Ethyl | |
| 2.2.693 | 3-Hydroxy-5-ethyl-Phenyl- | Fluormethyl | Ethyl | |
| 2.2.694 | 3-Methoxy-5-ethyl-Phenyl | Fluormethyl | Ethyl | |
| 2.2.695 | 3-Ethoxy-5-ethyl-Phenyl | Fluormethyl | Ethyl | |
| 2.2.696 | 3-nPropyoxy-5-ethyl-Phenyl | Fluormethyl | Ethyl | |
| 2.2.697 | 3-nButoxy-5-ethyl-Phenyl | Fluormethyl | Ethyl | |
| 2.2.698 | 3-isoButoxy-5-ethyl-Phenyl | Fluormethyl | Ethyl | |
| 2.2.699 | 3-tert.Butoxy-5-ethyl-Phenyl | Fluormethyl | Ethyl | |
| 2.2.700 | 3-Difluormethoxy-5-ethyl-Phenyl | Fluormethyl | Ethyl | |
| 2.2.701 | 3-Trifluormethoxy-5-ethyl-Phenyl | Fluormethyl | Ethyl | |
| 2.2.702 | 3-(2,2,2-Trifluorethoxy)-5-ethyl-Phenyl | Fluormethyl | Ethyl | |
| 2.2.703 | 3-(2-Chlorethoxy)-5-ethyl-Phenyl | Fluormethyl | Ethyl | |
| 2.2.704 | 3-(2-Hydroxyethoxy)-5-ethyl-Phenyl- | Fluormethyl | Ethyl | |
| 2.2.705 | 3-[(tert-Butoxycarbonyl)oxy]-5-ethyl-Phenyl | Fluormethyl | Ethyl | |
| 2.2.706 | 3-Nitro-5-ethyl-Phenyl | Fluormethyl | Ethyl | |
| 2.2.707 | 3-Acetoxy-5-ethyl-Phenyl | Fluormethyl | Ethyl | |
| 2.2.708 | {3-[(tert-Butoxycarbonyl)amino] -5-ethyl-Phenyl} | Fluormethyl | Ethyl | |
| 2.2.709 | 3-Methylsulfanyl-5-ethyl-Phenyl | Fluormethyl | Ethyl | |
| 2.2.710 | 3,5-Dipropyl-Phenyl | Fluormethyl | Ethyl | |
| 2.2.711 | 3-isoPropyl-5-propyl-Phenyl | Fluormethyl | Ethyl | |
| 2.2.712 | 3-nButyl-5-propyl-Phenyl | Fluormethyl | Ethyl | |
| 2.2.713 | 3-isoButyl-5-propyl-Phenyl | Fluormethyl | Ethyl | |
| 2.2.714 | 3-tert. Butyl-5-propyl-Phenyl | Fluormethyl | Ethyl | |
| 2.2.715 | 3-Cyclopropyl-5-propyl-Phenyl | Fluormethyl | Ethyl | |
| 2.2.716 | 3-Vinyl-5-propyl-Phenyl | Fluormethyl | Ethyl | |
| 2.2.717 | 3-Ethinyl-5-propyl-Phenyl | Fluormethyl | Ethyl | |
| 2.2.718 | 3-Cyano-5-propyl-Phenyl | Fluormethyl | Ethyl | |
| 2.2.719 | 3-Trifluormethyl-5-propyl-Phenyl | Fluormethyl | Ethyl | |
| 2.2.720 | 3-(Hydroxycarbonyl)-5-propyl-Phenyl | Fluormethyl | Ethyl | |
| 2.2.721 | 3-(Methoxycarbonyl)-5-propyl-Phenyl | Fluormethyl | Ethyl | |
| 2.2.722 | 3-Hydroxymethyl-5-propyl-Phenyl | Fluormethyl | Ethyl | |
| 2.2.723 | 3-Carbamoyl-5-propyl-Phenyl | Fluormethyl | Ethyl | |
| 2.2.724 | 3-Hydroxy-5-propyl-Phenyl- | Fluormethyl | Ethyl | |
| 2.2.725 | 3-Methoxy-5-propyl-Phenyl | Fluormethyl | Ethyl | |
| 2.2.726 | 3-Ethoxy-5-propyl-Phenyl | Fluormethyl | Ethyl | |
| 2.2.727 | 3-nPropyoxy-5-propyl-Phenyl | Fluormethyl | Ethyl | |
| 2.2.728 | 3-nButoxy-5-propyl-Phenyl | Fluormethyl | Ethyl | |
| 2.2.729 | 3-isoButoxy-5-propyl-Phenyl | Fluormethyl | Ethyl | |
| 2.2.730 | 3-tert. Butoxy-5-propyl-Phenyl | Fluormethyl | Ethyl | |
| 2.2.731 | 3-Difluormethoxy-5-propyl-Phenyl | Fluormethyl | Ethyl | |
| 2.2.732 | 3-Trifluormethoxy-5-ethyl-Phenyl | Fluormethyl | Ethyl | |
| 2.2.733 | 3-(2,2,2-Trifluorethoxy)-5-propyl-Phenyl | Fluormethyl | Ethyl | |
| 2.2.734 | 3-(2-Chlorethoxy)-5-propyl-Phenyl | Fluormethyl | Ethyl | |
| 2.2.735 | 3-(2-Hydroxyethoxy)-5-propyl-Phenyl- | Fluormethyl | Ethyl | |
| 2.2.736 | 3-[(tert-Butoxycarbonyl)oxy]-5-propyl-Phenyl | Fluormethyl | Ethyl | |
| 2.2.737 | 3-Nitro-5-propyl-Phenyl | Fluormethyl | Ethyl | |
| 2.2.738 | 3-Acetoxy-5-propyl-Phenyl | Fluormethyl | Ethyl | |
| 2.2.739 | {3-[(tert-Butoxycarbonyl)amino] -5-propyl-Phenyl} | Fluormethyl | Ethyl | |
| 2.2.740 | 3-Methylsulfanyl-5-propyl-Phenyl | Fluormethyl | Ethyl | |
| 2.2.741 | 3,5-Diisopropyl-Phenyl | Fluormethyl | Ethyl | |
| 2.2.742 | 3-nButyl-5-isopropyl-Phenyl | Fluormethyl | Ethyl | |
| 2.2.743 | 3-isoButyl-5-isopropyl-Phenyl | Fluormethyl | Ethyl | |
| 2.2.744 | 3-tert. Butyl-5-isopropyl-Phenyl | Fluormethyl | Ethyl | |
| 2.2.745 | 3-Cyclopropyl-5-isopropyl-Phenyl | Fluormethyl | Ethyl | |
| 2.2.746 | 3-Vinyl-5-isopropyl-Phenyl | Fluormethyl | Ethyl | |
| 2.2.747 | 3-Ethinyl-5-isopropyl-Phenyl | Fluormethyl | Ethyl | |
| 2.2.748 | 3-Cyano-5-isopropyl-Phenyl | Fluormethyl | Ethyl | |
| 2.2.749 | 3-Trifluormethyl-5-isopropyl-Phenyl | Fluormethyl | Ethyl | |
| 2.2.750 | 3-(Hydroxycarbonyl)-5-isopropyl-Phenyl | Fluormethyl | Ethyl | |
| 2.2.751 | 3-(Methoxycarbony)l-5-isopropyl-Phenyl | Fluormethyl | Ethyl | |
| 2.2.752 | 3-Hydroxymethyl-5-isopropyl-Phenyl | Fluormethyl | Ethyl | |
| 2.2.753 | 3-Carbamoyl-5-isopropyl-Phenyl | Fluormethyl | Ethyl | |
| 2.2.754 | 3-Hydroxy-5-isopropyl-Phenyl- | Fluormethyl | Ethyl | |
| 2.2.755 | 3-Methoxy-5-isopropyl-Phenyl | Fluormethyl | Ethyl | |
| 2.2.756 | 3-Ethoxy-5-isopropyl-Phenyl | Fluormethyl | Ethyl | |
| 2.2.757 | 3-nPropyoxy-5-isopropyl-Phenyl | Fluormethyl | Ethyl | |
| 2.2.758 | 3-nButoxy-5-isopropyl-Phenyl | Fluormethyl | Ethyl | |
| 2.2.759 | 3-isoButoxy-5-isopropyl-Phenyl | Fluormethyl | Ethyl | |
| 2.2.760 | 3-tert. Butoxy-5-isopropyl-Phenyl | Fluormethyl | Ethyl | |
| 2.2.761 | 3-Difluormethoxy-5-isopropyl-Phenyl | Fluormethyl | Ethyl | |
| 2.2.762 | 3-Trifluormethoxy-5-isopropyl-Phenyl | Fluormethyl | Ethyl | |
| 2.2.763 | 3-(2,2,2-Trifluorethoxy)-5-isopropyl-Phenyl | Fluormethyl | Ethyl | |
| 2.2.764 | 3-(2-Chlorethoxy)-5-isopropyl-Phenyl | Fluormethyl | Ethyl | |
| 2.2.765 | 3-(2-Hydroxyethoxy)-5-propyl-Phenyl- | Fluormethyl | Ethyl | |
| 2.2.766 | 3-[(tert-Butoxycarbonyl)oxy]-5-isopropyl-Phenyl | Fluormethyl | Ethyl | |
| 2.2.767 | 3-Nitro-5-isopropyl-Phenyl | Fluormethyl | Ethyl | |
| 2.2.768 | 3-Acetoxy-5-isopropyl-Phenyl | Fluormethyl | Ethyl | |
| 2.2.769 | {3-[(tert-Butoxycarbonyl)amino] -5-isopropyl-Phenyl} | Fluormethyl | Ethyl | |
| 2.2.770 | 3-Methylsulfanyl-5-isopropyl-Phenyl | Fluormethyl | Ethyl | |
| 2.2.771 | 3,5-Dibutyl-Phenyl | Fluormethyl | Ethyl | |
| 2.2.772 | 3-isoButyl-5-butyl-Phenyl | Fluormethyl | Ethyl | |
| 2.2.773 | 3-tert.Butyl-5-butyl-Phenyl | Fluormethyl | Ethyl | |
| 2.2.774 | 3-Cyclopropyl-5-butyl-Phenyl | Fluormethyl | Ethyl | |
| 2.2.775 | 3-Vinyl-5-butyl-Phenyl | Fluormethyl | Ethyl | |
| 2.2.776 | 3-Ethinyl-5-butyl-Phenyl | Fluormethyl | Ethyl | |
| 2.2.777 | 3-Cyano-5-butyl-Phenyl | Fluormethyl | Ethyl | |
| 2.2.778 | 3-Trifluormethyl-5-butyl-Phenyl | Fluormethyl | Ethyl | |
| 2.2.779 | 3-(Hydroxycarbonyl)-5-butyl-Phenyl | Fluormethyl | Ethyl | |
| 2.2.780 | 3-(Methoxycarbony)l-5-butyl-Phenyl | Fluormethyl | Ethyl | |
| 2.2.781 | 3-Hydroxymethyl-5-butyl-Phenyl | Fluormethyl | Ethyl | |
| 2.2.782 | 3-Carbamoyl-5-butyl-Phenyl | Fluormethyl | Ethyl | |
| 2.2.783 | 3-Hydroxy-5-butyl-Phenyl- | Fluormethyl | Ethyl | |
| 2.2.784 | 3-Methoxy-5-butyl-Phenyl | Fluormethyl | Ethyl | |
| 2.2.785 | 3-Ethoxy-5-butyl-Phenyl | Fluormethyl | Ethyl | |
| 2.2.786 | 3-nPropyoxy-5-butyl-Phenyl | Fluormethyl | Ethyl | |
| 2.2.787 | 3-nButoxy-5-butyl-Phenyl | Fluormethyl | Ethyl | |
| 2.2.788 | 3-isoButoxy-5-butyl-Phenyl | Fluormethyl | Ethyl | |
| 2.2.789 | 3-tert. Butoxy-5-butyl-Phenyl | Fluormethyl | Ethyl | |
| 2.2.790 | 3-Difluormethoxy-5-butyl-Phenyl | Fluormethyl | Ethyl | |
| 2.2.791 | 3-Trifluormethoxy-5-butyl-Phenyl | Fluormethyl | Ethyl | |
| 2.2.792 | 3-(2,2,2-Trifluorethoxy)-5-butyl-Phenyl | Fluormethyl | Ethyl | |
| 2.2.793 | 3-(2-Chlorethoxy)-5-butyl-Phenyl | Fluormethyl | Ethyl | |
| 2.2.794 | 3-(2-Hydroxyethoxy)-5-butyl-Phenyl- | Fluormethyl | Ethyl | |
| 2.2.795 | 3-[(tert-Butoxycarbonyl)oxy]-5-butyl-Phenyl | Fluormethyl | Ethyl | |
| 2.2.796 | 3-Nitro-5-butyl-Phenyl | Fluormethyl | Ethyl | |
| 2.2.797 | 3-Acetoxy-5-butyl-Phenyl | Fluormethyl | Ethyl | |
| 2.2.798 | {3-[(tert-Butoxycarbonyl)amino] -5-butyl-Phenyl} | Fluormethyl | Ethyl | |
| 2.2.799 | 3-Methylsulfanyl-5-butyl-Phenyl | Fluormethyl | Ethyl | |
| 2.2.800 | 3,5-Diisobutyl-Phenyl | Fluormethyl | Ethyl | |
| 2.2.801 | 3-tert. Butyl-5-isobutyl-Phenyl | Fluormethyl | Ethyl | |
| 2.2.802 | 3-Cyclopropyl-5-isobutyl-Phenyl | Fluormethyl | Ethyl | |
| 2.2.803 | 3-Vinyl-5-isobutyl-Phenyl | Fluormethyl | Ethyl | |
| 2.2.804 | 3-Ethinyl-5-isobutyl-Phenyl | Fluormethyl | Ethyl | |
| 2.2.805 | 3-Cyano-5-isobutyl-Phenyl | Fluormethyl | Ethyl | |
| 2.2.806 | 3-Trifluormethyl-5-isobutyl-Phenyl | Fluormethyl | Ethyl | |
| 2.2.807 | 3-(Hydroxycarbonyl)-5-isobutyl-Phenyl | Fluormethyl | Ethyl | |
| 2.2.808 | 3-(Methoxycarbonyl)-5-isobutyl-Phenyl | Fluormethyl | Ethyl | |
| 2.2.809 | 3-Hydroxymethyl-5-isobutyl-Phenyl | Fluormethyl | Ethyl | |
| 2.2.810 | 3-Carbamoyl-5-isobutyl-Phenyl | Fluormethyl | Ethyl | |
| 2.2.811 | 3-Hydroxy-5-isobutylPhenyl- | Fluormethyl | Ethyl | |
| 2.2.812 | 3-Methoxy-5-isobutylPhenyl | Fluormethyl | Ethyl | |
| 2.2.813 | 3-Ethoxy-5-isobutylPhenyl | Fluormethyl | Ethyl | |
| 2.2.814 | 3-nPropyoxy-5-isobutylPhenyl | Fluormethyl | Ethyl | |
| 2.2.815 | 3-nButoxy-5-isobutylPhenyl | Fluormethyl | Ethyl | |
| 2.2.816 | 3-isoButoxy-5-isobutylPhenyl | Fluormethyl | Ethyl | |
| 2.2.817 | 3-tert. Butoxy-5-isobutylPhenyl | Fluormethyl | Ethyl | |
| 2.2.818 | 3-Difluormethoxy-5-isobutyl-Phenyl | Fluormethyl | Ethyl | |
| 2.2.819 | 3-Trifluormethoxy-5-isobutyl-Phenyl | Fluormethyl | Ethyl | |
| 2.2.820 | 3-(2,2,2-Trifluorethoxy)-5-isobutyl-Phenyl | Fluormethyl | Ethyl | |
| 2.2.821 | 3-(2-Chlorethoxy)-5-isobutyl-Phenyl | Fluormethyl | Ethyl | |
| 2.2.822 | 3-(2-Hydroxyethoxy)-5-isobutyl-Phenyl- | Fluormethyl | Ethyl | |
| 2.2.823 | 3-[(tert-Butoxycarbonyl)oxy]-5-isobutylPhenyl | Fluormethyl | Ethyl | |
| 2.2.824 | 3-Nitro-5-isobutyl-Phenyl | Fluormethyl | Ethyl | |
| 2.2.825 | 3-Acetoxy-5-isobutylPhenyl | Fluormethyl | Ethyl | |
| 2.2.826 | {3-[(tert-Butoxycarbonyl)amino] -5-isobutyl-Phenyl} | Fluormethyl | Ethyl | |
| 2.2.827 | 3-Methylsulfanyl-5-isobutyl-Phenyl | Fluormethyl | Ethyl | |
| 2.2.828 | 3,5-D(itert.butyl)-Phenyl | Fluormethyl | Ethyl | |
| 2.2.829 | 3-Cyclopropyl-5-tert.butyl-Phenyl | Fluormethyl | Ethyl | |
| 2.2.830 | 3-Vinyl-5-tert.butyl-Phenyl | Fluormethyl | Ethyl | |
| 2.2.831 | 3-Ethinyl-5-tert.butyl-Phenyl | Fluormethyl | Ethyl | |
| 2.2.832 | 3-Cyano-5-tert.butyl-Phenyl | Fluormethyl | Ethyl | |
| 2.2.833 | 3-Trifluormethyl-5-tert.butyl-Phenyl | Fluormethyl | Ethyl | |
| 2.2.834 | 3-(Hydroxycarbonyl)-5-tert.butyl-Phenyl | Fluormethyl | Ethyl | |
| 2.2.835 | 3-(Methoxycarbonyl)-5-tert.butyl-Phenyl | Fluormethyl | Ethyl | |
| 2.2.836 | 3-Hydroxymethyl-5-tert.butyl-Phenyl | Fluormethyl | Ethyl | |
| 2.2.837 | 3-Carbamoyl-5-tert.butyl-Phenyl | Fluormethyl | Ethyl | |
| 2.2.838 | 3-Hydroxy-5-tert.butyl-Phenyl- | Fluormethyl | Ethyl | |
| 2.2.839 | 3-Methoxy-5-tert.butyl-Phenyl | Fluormethyl | Ethyl | |
| 2.2.840 | 3-Ethoxy-5-tert.butyl-Phenyl | Fluormethyl | Ethyl | |
| 2.2.841 | 3-nPropyoxy-5-tert.butyl-Phenyl | Fluormethyl | Ethyl | |
| 2.2.842 | 3-nButoxy-5-tert.butyl-Phenyl | Fluormethyl | Ethyl | |
| 2.2.843 | 3-isoButoxy-5-tert.butyl-Phenyl | Fluormethyl | Ethyl | |
| 2.2.844 | 3-tert.Butoxy-5-tert.butyl-Phenyl | Fluormethyl | Ethyl | |
| 2.2.845 | 3-Difluormethoxy-5-tert.butyl-Phenyl | Fluormethyl | Ethyl | |
| 2.2.846 | 3-Trifluormethoxy-5-tert.butyl-Phenyl | Fluormethyl | Ethyl | |
| 2.2.847 | 3-(2,2,2-Trifluorethoxy)-5-tert.butyl-Phenyl | Fluormethyl | Ethyl | |
| 2.2.848 | 3-(2-Chlorethoxy)-5-tert.butyl-Phenyl | Fluormethyl | Ethyl | |
| 2.2.849 | 3-(2-Hydroxyethoxy)-5-tert.butyl-Phenyl- | Fluormethyl | Ethyl | |
| 2.2.850 | 3-[(tert-Butoxycarbonyl)oxy]-5-tert.butyl-Phenyl | Fluormethyl | Ethyl | |
| 2.2.851 | 3-Nitro-5-tert.butyl-Phenyl | Fluormethyl | Ethyl | |
| 2.2.852 | 3-Acetoxy-5-tert.butyl-Phenyl | Fluormethyl | Ethyl | |
| 2.2.853 | {3-[(tert-Butoxycarbonyl)amino] -5-tert.butyl-Phenyl} | Fluormethyl | Ethyl | |
| 2.2.854 | 3-Methylsulfanyl-5-tert.butyl-Phenyl | Fluormethyl | Ethyl | |
| 2.2.855 | 3-tert. Butyl-5-cyclopropyl-Phenyl | Fluormethyl | Ethyl | |
| 2.2.856 | 3,5-Dicyclopropyl-Phenyl | Fluormethyl | Ethyl | |
| 2.2.857 | 3-Vinyl-5-cyclopropyl-Phenyl | Fluormethyl | Ethyl | |
| 2.2.858 | 3-Ethinyl-5-cyclopropyl-Phenyl | Fluormethyl | Ethyl | |
| 2.2.859 | 3-Cyano-5-cyclopropyl-Phenyl | Fluormethyl | Ethyl | |
| 2.2.860 | 3-Trifluormethyl-5-cyclopropyl-Phenyl | Fluormethyl | Ethyl | |
| 2.2.861 | 3-(Hydroxycarbonyl)-5-cyclopropyl-Phenyl | Fluormethyl | Ethyl | |
| 2.2.862 | 3-(Methoxycarbonyl)-5-cyclopropyl-Phenyl | Fluormethyl | Ethyl | |
| 2.2.863 | 3-Hydroxymethyl-5-cyclopropyl-Phenyl | Fluormethyl | Ethyl | |
| 2.2.864 | 3-Carbamoyl-5-cyclopropyl-Phenyl | Fluormethyl | Ethyl | |
| 2.2.865 | 3-Hydroxy-5-cyclopropyl-Phenyl- | Fluormethyl | Ethyl | |
| 2.2.866 | 3-Methoxy-5-cyclopropyl-Phenyl | Fluormethyl | Ethyl | |
| 2.2.867 | 3-Ethoxy-5-cyclopropyl-Phenyl | Fluormethyl | Ethyl | |
| 2.2.868 | 3-nPropyoxy-5-cyclopropyl-Phenyl | Fluormethyl | Ethyl | |
| 2.2.869 | 3-nButoxy-5-cyclopropyl-Phenyl | Fluormethyl | Ethyl | |
| 2.2.870 | 3-isoButoxy-5-cyclopropyl-Phenyl | Fluormethyl | Ethyl | |
| 2.2.871 | 3-tert.Butoxy-5-cyclopropyl-Phenyl | Fluormethyl | Ethyl | |
| 2.2.872 | 3-Difluormethoxy-5-cyclopropyl-Phenyl | Fluormethyl | Ethyl | |
| 2.2.873 | 3-Trifluormethoxy-5-cyclopropyl-Phenyl | Fluormethyl | Ethyl | |
| 2.2.874 | 3-(2,2,2-Trifluorethoxy)-5-cyclopropyl-Phenyl | Fluormethyl | Ethyl | |
| 2.2.875 | 3-(2-Chlorethoxy)-5-cyclopropyl-Phenyl | Fluormethyl | Ethyl | |
| 2.2.876 | 3-(2-Hydroxyethoxy)-5-cyclopropyl-Phenyl- | Fluormethyl | Ethyl | |
| 2.2.877 | 3-[(tert-Butoxycarbonyl)oxy]-5-cyclopropyl-Phenyl | Fluormethyl | Ethyl | |
| 2.2.878 | 3-Nitro-5-cyclopropyl-Phenyl | Fluormethyl | Ethyl | |
| 2.2.879 | 3-Acetoxy-5-cyclopropyl-Phenyl | Fluormethyl | Ethyl | |
| 2.2.880 | {3-[(tert-Butoxycarbonyl)amino] -5-cyclopropyl-Phenyl} | Fluormethyl | Ethyl | |
| 2.2.881 | 3-Methylsulfanyl-5-cyclopropyl-Phenyl | Fluormethyl | Ethyl | |
| 2.2.882 | 3,5-Divinyl-Phenyl | Fluormethyl | Ethyl | |
| 2.2.883 | 3-Ethinyl-5-vinyl-Phenyl | Fluormethyl | Ethyl | |
| 2.2.884 | 3-Cyano-5-vinyl-Phenyl | Fluormethyl | Ethyl | |
| 2.2.885 | 3-Trifluormethyl-5-vinyl-Phenyl | Fluormethyl | Ethyl | |
| 2.2.886 | 3-(Hydroxycarbonyl)-5-vinyl-Phenyl | Fluormethyl | Ethyl | |
| 2.2.887 | 3-(Methoxycarbonyl)-5-vinyl-Phenyl | Fluormethyl | Ethyl | |
| 2.2.888 | 3-Hydroxymethyl-5-vinyl-Phenyl | Fluormethyl | Ethyl | |
| 2.2.889 | 3-Carbamoyl-5-vinyl-Phenyl | Fluormethyl | Ethyl | |
| 2.2.890 | 3-Hydroxy-5-vinyl-Phenyl- | Fluormethyl | Ethyl | |
| 2.2.891 | 3-Methoxy-5-vinyl-Phenyl | Fluormethyl | Ethyl | |
| 2.2.892 | 3-Ethoxy-5-vinyl-Phenyl | Fluormethyl | Ethyl | |
| 2.2.893 | 3-nPropyoxy-5-vinyl-Phenyl | Fluormethyl | Ethyl | |
| 2.2.894 | 3-nButoxy-5-vinyl-Phenyl | Fluormethyl | Ethyl | |
| 2.2.895 | 3-isoButoxy-5-vinyl-Phenyl | Fluormethyl | Ethyl | |
| 2.2.896 | 3-tert. Butoxy-5-vinyl-Phenyl | Fluormethyl | Ethyl | |
| 2.2.897 | 3-Difluormethoxy-5-vinyl-Phenyl | Fluormethyl | Ethyl | |
| 2.2.898 | 3-Trifluormethoxy-5-vinyl-Phenyl | Fluormethyl | Ethyl | |
| 2.2.899 | 3-(2,2,2-Trifluorethoxy)-5-vinyl-Phenyl | Fluormethyl | Ethyl | |
| 2.2.900 | 3-(2-Chlorethoxy)-5-vinyl-Phenyl | Fluormethyl | Ethyl | |
| 2.2.901 | 3-(2-Hydroxyethoxy)-5-vinyl-Phenyl- | Fluormethyl | Ethyl | |
| 2.2.902 | 3-[(tert-Butoxycarbonyl)oxy]-5-vinyl-Phenyl | Fluormethyl | Ethyl | |
| 2.2.903 | 3-Nitro-5-vinyl-Phenyl | Fluormethyl | Ethyl | |
| 2.2.904 | 3-Acetoxy-5-vinyl-Phenyl | Fluormethyl | Ethyl | |
| 2.2.905 | {3-[(tert-Butoxycarbonyl)amino] -5-vinyl-Phenyl} | Fluormethyl | Ethyl | |
| 2.2.906 | 3-Methylsulfanyl-5-vinyl-Phenyl | Fluormethyl | Ethyl | |
| 2.2.907 | 3,5-Diethinyl-Phenyl | Fluormethyl | Ethyl | |
| 2.2.908 | 3-Cyano-5-ethinyl-Phenyl | Fluormethyl | Ethyl | |
| 2.2.909 | 3-Trifluormethyl-5-ethinyl-Phenyl | Fluormethyl | Ethyl | |
| 2.2.910 | 3-(Hydroxycarbonyl)-5-ethinyl-Phenyl | Fluormethyl | Ethyl | |
| 2.2.911 | 3-(Methoxycarbonyl)-5-ethinyl-Phenyl | Fluormethyl | Ethyl | |
| 2.2.912 | 3-Hydroxymethyl-5-ethinyl-Phenyl | Fluormethyl | Ethyl | |
| 2.2.913 | 3-Carbamoyl-5-ethinyl-Phenyl | Fluormethyl | Ethyl | |
| 2.2.914 | 3-Hydroxy-5-ethinyl-Phenyl- | Fluormethyl | Ethyl | |
| 2.2.915 | 3-Methoxy-5-ethinyl-Phenyl | Fluormethyl | Ethyl | |
| 2.2.916 | 3-Ethoxy-5-ethinyl-Phenyl | Fluormethyl | Ethyl | |
| 2.2.917 | 3-nPropyoxy-5-ethinyl-Phenyl | Fluormethyl | Ethyl | |
| 2.2.918 | 3-nButoxy-5-ethinyl-Phenyl | Fluormethyl | Ethyl | |
| 2.2.919 | 3-isoButoxy-5-ethinyl-Phenyl | Fluormethyl | Ethyl | |
| 2.2.920 | 3-tert. Butoxy-5-ethinyl-Phenyl | Fluormethyl | Ethyl | |
| 2.2.921 | 3-Difluormethoxy-5-ethinyl-Phenyl | Fluormethyl | Ethyl | |
| 2.2.922 | 3-Trifluormethoxy-5-ethinyl-Phenyl | Fluormethyl | Ethyl | |
| 2.2.923 | 3-(2,2,2-Trifluorethoxy)-5-ethinyl-Phenyl | Fluormethyl | Ethyl | |
| 2.2.924 | 3-(2-Chlorethoxy)-5-ethinyl-Phenyl | Fluormethyl | Ethyl | |
| 2.2.925 | 3-(2-Hydroxyethoxy)-5-ethinyl-Phenyl- | Fluormethyl | Ethyl | |
| 2.2.926 | 3-[(tert-Butoxycarbonyl)oxy]-5-ethinyl-Phenyl | Fluormethyl | Ethyl | |
| 2.2.927 | 3-Nitro-5-ethinyl-Phenyl | Fluormethyl | Ethyl | |
| 2.2.928 | 3-Acetoxy-5-ethinyl-Phenyl | Fluormethyl | Ethyl | |
| 2.2.929 | {3-[(tert-Butoxycarbonyl)amino]-5-ethinyl-Phenyl} | Fluormethyl | Ethyl | |
| 2.2.930 | 3-Methylsulfanyl-5-ethinyl-Phenyl | Fluormethyl | Ethyl | |
| 2.2.931 | 3,5-Dicyano-Phenyl | Fluormethyl | Ethyl | |
| 2.2.932 | 3-Trifluormethyl-5-cyano-Phenyl | Fluormethyl | Ethyl | |
| 2.2.933 | 3-(Hydroxycarbonyl)-5-cyano-Phenyl | Fluormethyl | Ethyl | |
| 2.2.934 | 3-(Methoxycarbonyl)-5-cyano-Phenyl | Fluormethyl | Ethyl | |
| 2.2.935 | 3-Hydroxymethyl-5-cyano-Phenyl | Fluormethyl | Ethyl | |
| 2.2.936 | 3-Carbamoyl-5-cyano-Phenyl | Fluormethyl | Ethyl | |
| 2.2.937 | 3-Hydroxy-5-cyano-Phenyl- | Fluormethyl | Ethyl | |
| 2.2.938 | 3-Methoxy-5-cyano-Phenyl | Fluormethyl | Ethyl | |
| 2.2.939 | 3-Ethoxy-5-cyano-Phenyl | Fluormethyl | Ethyl | |
| 2.2.940 | 3-nPropyoxy-5-cyano-Phenyl | Fluormethyl | Ethyl | |
| 2.2.941 | 3-nButoxy-5-cyano-Phenyl | Fluormethyl | Ethyl | |
| 2.2.942 | 3-isoButoxy-5-cyano-Phenyl | Fluormethyl | Ethyl | |
| 2.2.943 | 3-tert. Butoxy-5-cyano-Phenyl | Fluormethyl | Ethyl | |
| 2.2.944 | 3-Difluormethoxy-5-cyano-Phenyl | Fluormethyl | Ethyl | |
| 2.2.945 | 3-Trifluormethoxy-5-cyano-Phenyl | Fluormethyl | Ethyl | |
| 2.2.946 | 3-(2,2,2-Trifluorethoxy)-5-cyano-Phenyl | Fluormethyl | Ethyl | |
| 2.2.947 | 3-(2-Chlorethoxy)-5-cyano-Phenyl | Fluormethyl | Ethyl | |
| 2.2.948 | 3-(2-Hydroxyethoxy)-5-cyano-Phenyl- | Fluormethyl | Ethyl | |
| 2.2.949 | 3-[(tert-Butoxycarbonyl)oxy]-5-cyano-Phenyl | Fluormethyl | Ethyl | |
| 2.2.950 | 3-Nitro-5-cyano-Phenyl | Fluormethyl | Ethyl | |
| 2.2.951 | 3-Acetoxy-5-cyano-Phenyl | Fluormethyl | Ethyl | |
| 2.2.952 | {3-[(tert-Butoxycarbonyl)amino]-5-cyano-Phenyl} | Fluormethyl | Ethyl | |
| 2.2.953 | 3-Methylsulfanyl-5-cyano-Phenyl | Fluormethyl | Ethyl | |
| 2.2.954 | 3,5-Di(trifluormethyl)-Phenyl | Fluormethyl | Ethyl | |
| 2.2.955 | 3-(Hydroxycarbonyl)-5-trifluormethyl-Phenyl | Fluormethyl | Ethyl | |
| 2.2.956 | 3-(Methoxycarbonyl)-5-trifluormethyl-Phenyl | Fluormethyl | Ethyl | |
| 2.2.957 | 3-Hydroxymethyl-5-trifluormethyl-Phenyl | Fluormethyl | Ethyl | |
| 2.2.958 | 3-Carbamoyl-5-trifluormethyl-Phenyl | Fluormethyl | Ethyl | |
| 2.2.959 | 3-Hydroxy-5-trifluormethyl-Phenyl- | Fluormethyl | Ethyl | |
| 2.2.960 | 3-Methoxy-5-trifluormethyl-Phenyl | Fluormethyl | Ethyl | |
| 2.2.961 | 3-Ethoxy-5trifluormethyl-Phenyl | Fluormethyl | Ethyl | |
| 2.2.962 | 3-nPropyoxy-5-trifluormethyl-Phenyl | Fluormethyl | Ethyl | |
| 2.2.963 | 3-nButoxy-5-trifluormethyl-Phenyl | Fluormethyl | Ethyl | |
| 2.2.964 | 3-isoButoxy-5-trifluormethyl-Phenyl | Fluormethyl | Ethyl | |
| 2.2.965 | 3-tert.Butoxy-5-trifluormethyl-Phenyl | Fluormethyl | Ethyl | |
| 2.2.966 | 3-Difluormethoxy-5-trifluormethyl-Phenyl | Fluormethyl | Ethyl | |
| 2.2.967 | 3-Trifluormethoxy-5-trifluormethyl-Phenyl | Fluormethyl | Ethyl | |
| 2.2.968 | 3-(2,2,2-Trifluorethoxy)-5-trifluormethyl-Phenyl | Fluormethyl | Ethyl | |
| 2.2.969 | 3-(2-Chlorethoxy)-5-trifluormethyl-Phenyl | Fluormethyl | Ethyl | |
| 2.2.970 | 3-(2-Hydroxyethoxy)-5-trifluormethyl-Phenyl- | Fluormethyl | Ethyl | |
| 2.2.971 | 3-[(tert-Butoxycarbonyl)oxy]-5-trifluormethyl-Phenyl | Fluormethyl | Ethyl | |
| 2.2.972 | 3-Nitro-5-trifluormethyl-Phenyl | Fluormethyl | Ethyl | |
| 2.2.973 | 3-Acetoxy-5-trifluormethyl-Phenyl | Fluormethyl | Ethyl | |
| 2.2.974 | {3-[(tert-Butoxycarbonyl)amino] -5-trifluormethyl-Phenyl} | Fluormethyl | Ethyl | |
| 2.2.975 | 3-Methylsulfanyl-5-trifluormethyl-Phenyl | Fluormethyl | Ethyl | |
| 2.2.976 | 3,5-Bis(hydroxycarbonyl)-Phenyl | Fluormethyl | Ethyl | |
| 2.2.977 | 3-(Methoxycarbonyl)-5-(hydroxycarbonyl)-Phenyl | Fluormethyl | Ethyl | |
| 2.2.978 | 3-Hydroxymethyl-5-(hydroxycarbonyl)-Phenyl | Fluormethyl | Ethyl | |
| 2.2.979 | 3-Carbamoyl-5-(hydroxycarbonyl)-Phenyl | Fluormethyl | Ethyl | |
| 2.2.980 | 3-Hydroxy-5-(hydroxycarbonyl)-Phenyl- | Fluormethyl | Ethyl | |
| 2.2.981 | 3-Methoxy-5-(hydroxycarbonyl)-Phenyl | Fluormethyl | Ethyl | |
| 2.2.982 | 3-Ethoxy-5-(hydroxycarbonyl)-Phenyl | Fluormethyl | Ethyl | |
| 2.2.983 | 3-nPropyoxy-5-(hydroxycarbonyl)-Phenyl | Fluormethyl | Ethyl | |
| 2.2.984 | 3-nButoxy-5-(hydroxycarbonyl)-Phenyl | Fluormethyl | Ethyl | |
| 2.2.985 | 3-isoButoxy-5-(hydroxycarbonyl)-Phenyl | Fluormethyl | Ethyl | |
| 2.2.986 | 3-tert.Butoxy-5-(hydroxycarbonyl)-Phenyl | Fluormethyl | Ethyl | |
| 2.2.987 | 3-Difluormethoxy-5-(hydroxycarbonyl)-Phenyl | Fluormethyl | Ethyl | |
| 2.2.988 | 3-Trifluormethoxy-5-(hydroxycarbonyl)-Phenyl | Fluormethyl | Ethyl | |
| 2.2.989 | 3-(2,2,2-Trifluorethoxy)-5-(hydroxycarbonyl)-Phenyl | Fluormethyl | Ethyl | |
| 2.2.990 | 3-(2-Chlorethoxy)-5-(hydroxycarbonyl)-Phenyl | Fluormethyl | Ethyl | |
| 2.2.991 | 3-(2-Hydroxyethoxy)-5-(hydroxycarbonyl)-Phenyl- | Fluormethyl | Ethyl | |
| 2.2.992 | 3-[(tert-Butoxycarbonyl)oxy]-5-(hydroxycarbonyl)-Phenyl | Fluormethyl | Ethyl | |
| 2.2.993 | 3-Nitro-5-(hydroxycarbonyl)-Phenyl | Fluormethyl | Ethyl | |
| 2.2.994 | 3-Acetoxy-5-(hydroxycarbonyl)-Phenyl | Fluormethyl | Ethyl | |
| 2.2.995 | {3-[(tert-Butoxycarbonyl)amino]-5-(hydroxycarbonyl)-Phenyl} | Fluormethyl | Ethyl | |
| 2.2.996 | 3-Methylsulfanyl-5-(hydroxycarbonyl)-Phenyl | Fluormethyl | Ethyl | |
| 2.2.997 | 3,5-Di(methoxycarbonyl)-Phenyl | Fluormethyl | Ethyl | |
| 2.2.998 | 3-Hydroxymethyl-5-(methoxycarbonyl)-Phenyl | Fluormethyl | Ethyl | |
| 2.2.999 | 3-Carbamoyl-5-(methoxycarbonyl)-Phenyl | Fluormethyl | Ethyl | |
| 2.2.1000 | 3-Hydroxy-5-(methoxycarbonyl)-Phenyl- | Fluormethyl | Ethyl | |
| 2.2.1001 | 3-Methoxy-5-(methoxycarbonyl)-Phenyl | Fluormethyl | Ethyl | |
| 2.2.1002 | 3-Ethoxy-5-(methoxycarbonyl)-Phenyl | Fluormethyl | Ethyl | |
| 2.2.1003 | 3-nPropyoxy-5-(methoxycarbonyl)-Phenyl | Fluormethyl | Ethyl | |
| 2.2.1004 | 3-nButoxy-5-(methoxycarbonyl)-Phenyl | Fluormethyl | Ethyl | |
| 2.2.1005 | 3-isoButoxy-5-(methoxycarbonyl)-Phenyl | Fluormethyl | Ethyl | |
| 2.2.1006 | 3-tert.Butoxy-5-(methoxycarbonyl)-Phenyl | Fluormethyl | Ethyl | |
| 2.2.1007 | 3-Difluormethoxy-5-(methoxycarbonyl)-Phenyl | Fluormethyl | Ethyl | |
| 2.2.1008 | 3-Trifluormethoxy-5-(methoxycarbonyl)-Phenyl | Fluormethyl | Ethyl | |
| 2.2.1009 | 3-(2,2,2-Trifluorethoxy)-5-(methoxycarbonyl)-Phenyl | Fluormethyl | Ethyl | |
| 2.2.1010 | 3-(2-Chlorethoxy)-5-(methoxycarbonyl)-Phenyl | Fluormethyl | Ethyl | |
| 2.2.1011 | 3-(2-Hydroxyethoxy)-5-(methoxycarbonyl)-Phenyl- | Fluormethyl | Ethyl | |
| 2.2.1012 | 3-[(tert-Butoxycarbonyl)oxy]-5-(methoxycarbonyl)-Phenyl | Fluormethyl | Ethyl | |
| 2.2.1013 | 3-Nitro-5-(methoxycarbonyl)-Phenyl | Fluormethyl | Ethyl | |
| 2.2.1014 | 3-Acetoxy-5-(methoxycarbonyl)-Phenyl | Fluormethyl | Ethyl | |
| 2.2.1015 | {3-[(tert-Butoxycarbonyl)amino]-5-(methoxycarbonyl)-Phenyl} | Fluormethyl | Ethyl | |
| 2.2.1016 | 3-Methylsulfanyl-5-(methoxycarbonyl)-Phenyl | Fluormethyl | Ethyl | |
| 2.2.1017 | 3,5-Di(hydroxymethy)l-Phenyl | Fluormethyl | Ethyl | |
| 2.2.1018 | 3-Carbamoyl-5-hydroxymethyl-Phenyl | Fluormethyl | Ethyl | |
| 2.2.1019 | 3-Hyd roxy-5-hydroxymethyl-Phenyl- | Fluormethyl | Ethyl | |
| 2.2.1020 | 3-Methoxy-5-hydroxymethyl-Phenyl | Fluormethyl | Ethyl | |
| 2.2.1021 | 3-Ethoxy-5-hydroxymethyl-Phenyl | Fluormethyl | Ethyl | |
| 2.2.1022 | 3-nPropyoxy-5-hydroxymethyl-Phenyl | Fluormethyl | Ethyl | |
| 2.2.1023 | 3-nButoxy-5-hydroxymethyl-Phenyl | Fluormethyl | Ethyl | |
| 2.2.1024 | 3-isoButoxy-5-hydroxymethyl-Phenyl | Fluormethyl | Ethyl | |
| 2.2.1025 | 3-tert. Butoxy-5-hyd roxymethyl-Phenyl | Fluormethyl | Ethyl | |
| 2.2.1026 | 3-Difluormethoxy-5-hydroxymethyl-Phenyl | Fluormethyl | Ethyl | |
| 2.2.1027 | 3-Trifluormethoxy-5-hydroxymethyl-Phenyl | Fluormethyl | Ethyl | |
| 2.2.1028 | 3-(2,2,2-Trifluorethoxy)-5-hydroxymethyl-Phenyl | Fluormethyl | Ethyl | |
| 2.2.1029 | 3-(2-Chlorethoxy)-5-hydroxymethyl-Phenyl | Fluormethyl | Ethyl | |
| 2.2.1030 | 3-(2-Hydroxyethoxy)-5-hydroxymethyl-Phenyl | Fluormethyl | Ethyl | |
| 2.2.1031 | 3-[(tert-Butoxycarbonyl)oxy]-5-hydroxymethyl-Phenyl | Fluormethyl | Ethyl | |
| 2.2.1032 | 3-Nitro-5-hydroxymethyl-Phenyl | Fluormethyl | Ethyl | |
| 2.2.1033 | 3-Acetoxy-5-hydroxymethyl-Phenyl | Fluormethyl | Ethyl | |
| 2.2.1034 | {3-[(tert-Butoxycarbonyl)amino] -5-hydroxymethyl-Phenyl} | Fluormethyl | Ethyl | |
| 2.2.1035 | 3-Methylsulfanyl-5-hydroxymethyl-Phenyl | Fluormethyl | Ethyl | |
| 2.2.1036 | 3,5-Dicarbamoyl-5-carbamoyl-Phenyl | Fluormethyl | Ethyl | |
| 2.2.1037 | 3-Hydroxy-5-carbamoyl-Phenyl- | Fluormethyl | Ethyl | |
| 2.2.1038 | 3-Methoxy-5-carbamoyl-Phenyl | Fluormethyl | Ethyl | |
| 2.2.1039 | 3-Ethoxy-5-carbamoyl-Phenyl | Fluormethyl | Ethyl | |
| 2.2.1040 | 3-nPropyoxy-5-carbamoyl-Phenyl | Fluormethyl | Ethyl | |
| 2.2.1041 | 3-nButoxy-5-carbamoyl-Phenyl | Fluormethyl | Ethyl | |
| 2.2.1042 | 3-isoButoxy-5-carbamoyl-Phenyl | Fluormethyl | Ethyl | |
| 2.2.1043 | 3-tert.Butoxy-5-carbamoyl-Phenyl | Fluormethyl | Ethyl | |
| 2.2.1044 | 3-Difluormethoxy-5-carbamoyl-Phenyl | Fluormethyl | Ethyl | |
| 2.2.1045 | 3-Trifluormethoxy-5-carbamoyl-Phenyl | Fluormethyl | Ethyl | |
| 2.2.1046 | 3-(2,2,2-Trifluorethoxy)-5-carbamoyl-Phenyl | Fluormethyl | Ethyl | |
| 2.2.1047 | 3-(2-Chlorethoxy)-5-carbamoyl-Phenyl | Fluormethyl | Ethyl | |
| 2.2.1048 | 3-(2-Hydroxyethoxy)-5-carbamoyl-Phenyl- | Fluormethyl | Ethyl | |
| 2.2.1049 | 3-[(tert-Butoxycarbonyl)oxy]-5-carbamoyl-Phenyl | Fluormethyl | Ethyl | |
| 2.2.1050 | 3-Nitro-5-carbamoyl-Phenyl | Fluormethyl | Ethyl | |
| 2.2.1051 | 3-Acetoxy-5- carbamoyl-Phenyl | Fluormethyl | Ethyl | |
| 2.2.1052 | {3-[(tert-Butoxycarbonyl)amino] -5-carbamoyl-Phenyl} | Fluormethyl | Ethyl | |
| 2.2.1053 | 3-Methylsulfanyl-5-carbamoyl-Phenyl | Fluormethyl | Ethyl | |
| 2.2.1054 | 3,5-Dihydroxy-Phenyl | Fluormethyl | Ethyl | |
| 2.2.1055 | 3-Methoxy-5-hydroxyPhenyl | Fluormethyl | Ethyl | |
| 2.2.1056 | 3-Ethoxy-5-hydroxyPhenyl | Fluormethyl | Ethyl | |
| 2.2.1057 | 3-nPropyoxy-5-hydroxyPhenyl | Fluormethyl | Ethyl | |
| 2.2.1058 | 3-nButoxy-5-hydroxyPhenyl | Fluormethyl | Ethyl | |
| 2.2.1059 | 3-isoButoxy-5-hydroxyPhenyl | Fluormethyl | Ethyl | |
| 2.2.1060 | 3-tert. Butoxy-5-hydroxy-Phenyl | Fluormethyl | Ethyl | |
| 2.2.1061 | 3-Difluormethoxy-5-hydroxy-Phenyl | Fluormethyl | Ethyl | |
| 2.2.1062 | 3-Trifluormethoxy-5-hydroxy-Phenyl | Fluormethyl | Ethyl | |
| 2.2.1063 | 3-(2,2,2-Trifluorethoxy)-5-hydroxy-Phenyl | Fluormethyl | Ethyl | |
| 2.2.1064 | 3-(2-Chlorethoxy)-5-hydroxy-Phenyl | Fluormethyl | Ethyl | |
| 2.2.1065 | 3-(2-Hydroxyethoxy)-5-hydroxy-Phenyl- | Fluormethyl | Ethyl | |
| 2.2.1066 | 3-[(tert-Butoxycarbonyl)oxy]-5-hydroxyPhenyl | Fluormethyl | Ethyl | |
| 2.2.1067 | 3-Nitro-5-hydroxy-Phenyl | Fluormethyl | Ethyl | |
| 2.2.1068 | 3-Acetoxy-5-hydroxyPhenyl | Fluormethyl | Ethyl | |
| 2.2.1069 | {3-[(tert-Butoxycarbonyl)amino]-5-hydroxy-Phenyl} | Fluormethyl | Ethyl | |
| 2.2.1070 | 3-Methylsulfanyl-5-hydroxy-Phenyl | Fluormethyl | Ethyl | |
| 2.2.1071 | 3,5-Dimethoxy-Phenyl | Fluormethyl | Ethyl | |
| 2.2.1072 | 3-Ethoxy-5-methoxy-Phenyl | Fluormethyl | Ethyl | |
| 2.2.1073 | 3-nPropyoxy-5-methoxy-Phenyl | Fluormethyl | Ethyl | |
| 2.2.1074 | 3-nButoxy-5-methoxy-Phenyl | Fluormethyl | Ethyl | |
| 2.2.1075 | 3-isoButoxy-5-methoxy-Phenyl | Fluormethyl | Ethyl | |
| 2.2.1076 | 3-tert. Butoxy-5-methoxy-Phenyl | Fluormethyl | Ethyl | |
| 2.2.1077 | 3-Difluormethoxy-5-methoxy-Phenyl | Fluormethyl | Ethyl | |
| 2.2.1078 | 3-Trifluormethoxy-5-methoxy-Phenyl | Fluormethyl | Ethyl | |
| 2.2.1079 | 3-(2,2,2-Trifluorethoxy)-5-methoxy-Phenyl | Fluormethyl | Ethyl | |
| 2.2.1080 | 3-(2-Chlorethoxy)-5-methoxy-Phenyl | Fluormethyl | Ethyl | |
| 2.2.1081 | 3-(2-Hydroxyethoxy)-5-methoxy-Phenyl- | Fluormethyl | Ethyl | |
| 2.2.1082 | 3-[(tert-Butoxycarbonyl)oxy]-5-methoxy-Phenyl | Fluormethyl | Ethyl | |
| 2.2.1083 | 3-Nitro-5-methoxy-Phenyl | Fluormethyl | Ethyl | |
| 2.2.1084 | 3-Acetoxy-5-methoxy-Phenyl | Fluormethyl | Ethyl | |
| 2.2.1085 | {3-[(tert-Butoxycarbonyl)amino]-5-methoxy-Phenyl} | Fluormethyl | Ethyl | |
| 2.2.1086 | 3-Methylsulfanyl-5-methoxy-Phenyl | Fluormethyl | Ethyl | |
| 2.2.1087 | 3,5-Diethoxy-Phenyl | Fluormethyl | Ethyl | |
| 2.2.1088 | 3-nPropyoxy-5-ethoxy-Phenyl | Fluormethyl | Ethyl | |
| 2.2.1089 | 3-nButoxy-5-ethoxy-Phenyl | Fluormethyl | Ethyl | |
| 2.2.1090 | 3-isoButoxy-5-ethoxy-Phenyl | Fluormethyl | Ethyl | |
| 2.2.1091 | 3-tert. Butoxy-5-ethoxy-Phenyl | Fluormethyl | Ethyl | |
| 2.2.1092 | 3-Difluormethoxy-5-ethoxy-Phenyl | Fluormethyl | Ethyl | |
| 2.2.1093 | 3-Trifluormethoxy-5-ethoxy-Phenyl | Fluormethyl | Ethyl | |
| 2.2.1094 | 3-(2,2,2-Trifluorethoxy)-5-ethoxy-Phenyl | Fluormethyl | Ethyl | |
| 2.2.1095 | 3-(2-Chlorethoxy)-5-ethoxy-Phenyl | Fluormethyl | Ethyl | |
| 2.2.1096 | 3-(2-Hydroxyethoxy)-5-ethoxy-Phenyl- | Fluormethyl | Ethyl | |
| 2.2.1097 | 3-[(tert-Butoxycarbonyl)oxy]-5-ethoxy-Phenyl | Fluormethyl | Ethyl | |
| 2.2.1098 | 3-Nitro-5-ethoxy-Phenyl | Fluormethyl | Ethyl | |
| 2.2.1099 | 3-Acetoxy-5-ethoxy-Phenyl | Fluormethyl | Ethyl | |
| 2.2.1100 | {3-[(tert-Butoxycarbonyl)amino]-5-ethoxy-Phenyl} | Fluormethyl | Ethyl | |
| 2.2.1101 | 3-Methylsulfanyl-5-ethoxy-Phenyl | Fluormethyl | Ethyl | |
| 2.2.1102 | 3,5-Dipropyoxy-Phenyl | Fluormethyl | Ethyl | |
| 2.2.1103 | 3-nButoxy-5-propoxy-Phenyl | Fluormethyl | Ethyl | |
| 2.2.1104 | 3-isoButoxy-5-propoxy-Phenyl | Fluormethyl | Ethyl | |
| 2.2.1105 | 3-tert. Butoxy-5-propoxy-Phenyl | Fluormethyl | Ethyl | |
| 2.2.1106 | 3-Difluormethoxy-5-propoxy-Phenyl | Fluormethyl | Ethyl | |
| 2.2.1107 | 3-Trifluormethoxy-5-propoxy-Phenyl | Fluormethyl | Ethyl | |
| 2.2.1108 | 3-(2,2,2-Trifluorethoxy)-5-propoxy-Phenyl | Fluormethyl | Ethyl | |
| 2.2.1109 | 3-(2-Chlorethoxy)-5-propoxy-Phenyl | Fluormethyl | Ethyl | |
| 2.2.1110 | 3-(2-Hydroxyethoxy)-5-propoxy-Phenyl- | Fluormethyl | Ethyl | |
| 2.2.1111 | 3-[(tert-Butoxycarbonyl)oxy]-5-propoxy-Phenyl | Fluormethyl | Ethyl | |
| 2.2.1112 | 3-Nitro-5-propoxy-Phenyl | Fluormethyl | Ethyl | |
| 2.2.1113 | 3-Acetoxy-5-propoxy-Phenyl | Fluormethyl | Ethyl | |
| 2.2.1114 | {3-[(tert-Butoxycarbonyl)amino]-5-propoxy-Phenyl} | Fluormethyl | Ethyl | |
| 2.2.1115 | 3-Methylsulfanyl-5-propoxy-Phenyl | Fluormethyl | Ethyl | |
| 2.2.1116 | 3,5-Di(isopropyoxy)-Phenyl | Fluormethyl | Ethyl | |
| 2.2.1117 | 3-nButoxy-5-isopropoxy-Phenyl | Fluormethyl | Ethyl | |
| 2.2.1118 | 3-isoButoxy-5-isopropoxy-Phenyl | Fluormethyl | Ethyl | |
| 2.2.1119 | 3-tert.Butoxy-5-isopropoxy-Phenyl | Fluormethyl | Ethyl | |
| 2.2.1120 | 3-Difluormethoxy-5-isopropoxy-Phenyl | Fluormethyl | Ethyl | |
| 2.2.1121 | 3-Trifluormethoxy-5-isopropoxy-Phenyl | Fluormethyl | Ethyl | |
| 2.2.1122 | 3-(2,2,2-Trifluorethoxy)-5-isopropoxy-Phenyl | Fluormethyl | Ethyl | |
| 2.2.1123 | 3-(2-Chlorethoxy)-5-isopropoxy-Phenyl | Fluormethyl | Ethyl | |
| 2.2.1124 | 3-(2-Hydroxyethoxy)-5-isopropoxy-Phenyl- | Fluormethyl | Ethyl | |
| 2.2.1125 | 3-[(tert-Butoxycarbonyl)oxy]-5-isopropoxy-Phenyl | Fluormethyl | Ethyl | |
| 2.2.1126 | 3-Nitro-5-isopropoxy-Phenyl | Fluormethyl | Ethyl | |
| 2.2.1127 | 3-Acetoxy-5-isopropoxy-Phenyl | Fluormethyl | Ethyl | |
| 2.2.1128 | {3-[(tert-Butoxycarbonyl)amino]-5-isopropoxy-Phenyl} | Fluormethyl | Ethyl | |
| 2.2.1129 | 3-Methylsulfanyl-5-isopropoxy-Phenyl | Fluormethyl | Ethyl | |
| 2.2.1130 | 3,5-Di(tert.butoxy)-Phenyl | Fluormethyl | Ethyl | |
| 2.2.1131 | 3-Difluormethoxy-5-tert.butoxy-Phenyl | Fluormethyl | Ethyl | |
| 2.2.1132 | 3-Trifluormethoxy-5-tert.butoxy-Phenyl | Fluormethyl | Ethyl | |
| 2.2.1133 | 3-(2,2,2-Trifluorethoxy)-5-tert.butoxy-Phenyl | Fluormethyl | Ethyl | |
| 2.2.1134 | 3-(2-Chlorethoxy)-5-tert.butoxyl-Phenyl | Fluormethyl | Ethyl | |
| 2.2.1135 | 3-(2-Hydroxyethoxy)-5-tert.butoxy-Phenyl- | Fluormethyl | Ethyl | |
| 2.2.1136 | 3-[(tert-Butoxycarbonyl)oxy]-5-tert.butoxy-Phenyl | Fluormethyl | Ethyl | |
| 2.2.1137 | 3-Nitro-5-tert.butoxy-Phenyl | Fluormethyl | Ethyl | |
| 2.2.1138 | 3-Acetoxy-5-tert.butoxy-Phenyl | Fluormethyl | Ethyl | |
| 2.2.1139 | {3-[(tert-Butoxycarbonyl)amino] -5-tert.butoxy-Phenyl} | Fluormethyl | Ethyl | |
| 2.2.1140 | 3-Methylsulfanyl-5-tert.butoxy-Phenyl | Fluormethyl | Ethyl | |
| 2.2.1141 | 3,5-Di(trifluormethoxy)-Phenyl | Fluormethyl | Ethyl | |
| 2.2.1142 | 3-(2,2,2-Trifluorethoxy)-5-trifluormethoxy-Phenyl | Fluormethyl | Ethyl | |
| 2.2.1143 | 3-(2-Chlorethoxy)-5-trifluormethoxy-Phenyl | Fluormethyl | Ethyl | |
| 2.2.1144 | 3-(2-Hydroxyethoxy)-5-trifluormethoxy-Phenyl- | Fluormethyl | Ethyl | |
| 2.2.1145 | 3-[(tert-Butoxycarbonyl)oxy]-5-trifluormethoxy-Phenyl | Fluormethyl | Ethyl | |
| 2.2.1146 | 3-Nitro-5-trifluormethoxy-Phenyl | Fluormethyl | Ethyl | |
| 2.2.1147 | 3-Acetoxy-5-tert.butoxy-Phenyl | Fluormethyl | Ethyl | |
| 2.2.1148 | {3-[(tert-Butoxycarbonyl)amino]-5-trifluormethoxy-Phenyl} | Fluormethyl | Ethyl | |
| 2.2.1149 | 3-Methylsulfanyl-5-trifluormethoxy-Phenyl | Fluormethyl | Ethyl | |
| 2.2.1150 | 3,5-Bis(difluormethoxy)-Phenyl | Fluormethyl | Ethyl | |
| 2.2.1151 | 3,5-Bis(difluormethoxy)-Phenyl | Chlormethyl | Ethyl | |
| 2.2.1152 | 3,5-Bis(difluormethoxy)-Phenyl | Brommethyl | Ethyl | |
| 2.2.1153 | 3,5-Bis(difluormethoxy)-Phenyl | Fluormethyl | Ethyl | |
| 2.2.1154 | 3-Trifluormethoxy-5-difluormethoxy-Phenyl | Fluormethyl | Ethyl | |
| 2.2.1155 | 3-(2,2,2-Trifluorethoxy)-5-difluormethoxy-Phenyl | Fluormethyl | Ethyl | |
| 2.2.1156 | 3-(2-Chlorethoxy)-5-difluormethoxy-Phenyl | Fluormethyl | Ethyl | |
| 2.2.1157 | 3-(2-Hydroxyethoxy)-5-difluormethoxy-Phenyl- | Fluormethyl | Ethyl | |
| 2.2.1158 | 3-[(tert-Butoxycarbonyl)oxy]-5-difluormethoxy-Phenyl | Fluormethyl | Ethyl | |
| 2.2.1159 | 3-Nitro-5-difluormethoxy-Phenyl | Fluormethyl | Ethyl | |
| 2.2.1160 | 3-Acetoxy-5-difluormethoxy-Phenyl | Fluormethyl | Ethyl | |
| 2.2.1161 | {3-[(tert-Butoxycarbonyl)amino]-5-difluormethoxy-Phenyl} | Fluormethyl | Ethyl | |
| 2.2.1162 | 3-Methylsulfanyl-5-difluormethoxy-Phenyl | Fluormethyl | Ethyl | |
| 2.2.1163 | 3,5-Bis(2,2,2-Trifluorethoxy)-Phenyl | Fluormethyl | Ethyl | |
| 2.2.1164 | 3-(2-Chlorethoxy)-5-(2,2,2-trifluorethoxy)-Phenyl | Fluormethyl | Ethyl | |
| 2.2.1165 | 3-(2-Hydroxyethoxy)-5-(2,2,2-trifluorethoxy)-Phenyl- | Fluormethyl | Ethyl | |
| 2.2.1166 | 3-[(tert-Butoxycarbonyl)oxy]-5-(2,2,2-trifluorethoxy)-Phenyl | Fluormethyl | Ethyl | |
| 2.2.1167 | 3-Nitro-5-(2,2,2-trifluorethoxy)-Phenyl | Fluormethyl | Ethyl | |
| 2.2.1168 | 3-Acetoxy-5-(2,2,2-trifluorethoxy)-Phenyl | Fluormethyl | Ethyl | |
| 2.2.1169 | {3-[(tert-Butoxycarbonyl)amino]-5-(2,2,2-trifluorethoxy)-Phenyl} | Fluormethyl | Ethyl | |
| 2.2.1170 | 3-Methylsulfanyl-5-(2,2,2-trifluorethoxy)-Phenyl | Fluormethyl | Ethyl | |
| 2.2.1171 | 3,5-Bis(2-Chlorethoxy)-Phenyl | Fluormethyl | Ethyl | |
| 2.2.1172 | 3-(2-Hydroxyethoxy)-5-(2-chlorethoxy)-Phenyl- | Fluormethyl | Ethyl | |
| 2.2.1173 | 3-[(tert-Butoxycarbonyl)oxy]-5-(2-chlorethoxy)-Phenyl | Fluormethyl | Ethyl | |
| 2.2.1174 | 3-Nitro-5-(2-chlorethoxy)-Phenyl | Fluormethyl | Ethyl | |
| 2.2.1175 | 3-Acetoxy-5-(2-chlorethoxy)-Phenyl | Fluormethyl | Ethyl | |
| 2.2.1176 | {3-[(tert-Butoxycarbonyl)amino] -5-(2-chlorethoxy)-Phenyl} | Fluormethyl | Ethyl | |
| 2.2.1177 | 3-Methylsulfanyl-5-(2-chlorethoxy)-Phenyl | Fluormethyl | Ethyl | |
| 2.2.1178 | 3,5-Bis(2-hydroxyethoxy)-Phenyl | Fluormethyl | Ethyl | |
| 2.2.1179 | 3-[(tert-Butoxycarbonyl)oxy]-5-(2-hydroxyethoxy)-Phenyl | Fluormethyl | Ethyl | |
| 2.2.1180 | 3-Nitro-5-(2-hydroxyethoxy)-Phenyl | Fluormethyl | Ethyl | |
| 2.2.1181 | 3-Acetoxy-5-(2-hydroxyethoxy)-Phenyl | Fluormethyl | Ethyl | |
| 2.2.1182 | 3-[(tert-Butoxycarbonyl)amino] -5-(2-hydroxyethoxy)-Phenyl | Fluormethyl | Ethyl | |
| 2.2.1183 | 3-Methylsulfanyl-5-(2-hydroxyethoxy)-Phenyl | Fluormethyl | Ethyl | |
| 2.2.1184 | 3,5-Bis[(tert-Butoxycarbonyl)oxy]-Phenyl | Fluormethyl | Ethyl | |
| 2.2.1185 | 3-Nitro-5-[(tert-Butoxy-carbonyl)oxy]--Phenyl | Fluormethyl | Ethyl | |
| 2.2.1186 | 3-Acetoxy-5-[(tert-Butoxy-carbonyl)oxy]-Phenyl | Fluormethyl | Ethyl | |
| 2.2.1187 | {3-[(tert-Butoxycarbonyl)amino] - 5[(tert-Butoxycarbonyl)oxy]-Phenyl} | Fluormethyl | Ethyl | |
| 2.2.1188 | 3,5-Bis(acetoxy)-Phenyl | Fluormethyl | Ethyl | |
| 2.2.1189 | {3-[(tert-Butoxycarbonyl)amino]-5-acetoxy-Phenyl} | Fluormethyl | Ethyl | |
| 2.2.1190 | 3-Methylsulfanyl-5-acetoxy-Phenyl | Fluormethyl | Ethyl | |
| 2.2.1191 | 3,5-Dinitro -Phenyl | Fluormethyl | Ethyl | |
| 2.2.1192 | 3-Acetoxy-5-nitro-Phenyl | Fluormethyl | Ethyl | |
| 2.2.1193 | {3-[(tert-Butoxycarbonyl)amino] -5-nitro-Phenyl} | Fluormethyl | Ethyl | |
| 2.2.1194 | 3-Methylsulfanyl-5-nitroPhenyl | Fluormethyl | Ethyl | |
| 2.2.1195 | 3,5-Bis[(tert-Butoxycarbonyl)amino] - Phenyl | Fluormethyl | Ethyl | |
| 2.2.1196 | 3-Methylsulfanyl-5-[(tert.butoxycarbonyl)amino]-Phenyl | Fluormethyl | Ethyl | |
| 2.2.1197 | 3,5-Di(methylsulfanyl)-Phenyl | Fluormethyl | Ethyl | |
| 2.2.1198 | 3,4-Difluor-Phenyl | Fluormethyl | Ethyl | |
| 2.2.1199 | 3,4-Difluor-Phenyl | Chlormethyl | Ethyl | |
| 2.2.1200 | 3,4-Difluor-Phenyl | Brommethyl | Ethyl | |
| 2.2.1201 | 3,4-Difluor-Phenyl | Difluormethyll | Ethyl | |
| 2.2.1202 | 3,4-Difluor-Phenyl | Trifluormethyl | Ethyl | |
| 2.2.1203 | 3,4-Difluor-Phenyl | Cyano | Ethyl | |
| 2.2.1204 | 3-Chlor-4-fluor-Phenyl | Fluormethyl | Ethyl | |
| 2.2.1205 | 3-Chlor-4-fluor-Phenyl | Chlormethyl | Ethyl | |
| 2.2.1206 | 3-Chlor-4-fluor-Phenyl | Brommethyl | Ethyl | |
| 2.2.1207 | 3-Chlor-4-fluor-Phenyl | Difluormethyl | Ethyl | |
| 2.2.1208 | 3-Chlor-4-fluor-Phenyl | Trifluormethyl | Methyl | [CDCl₃] 1.52 (s, 3H); 3.74 (d, 1H); 3.94 (s, 3H); 4.03 (d, 1H); 7.22 (m, 1H); 7.58 (m, 1H); 7.72 (m, 1H). |
| 2.2.1209 | 3-Chlor-4-fluor-Phenyl | Cyano | Ethyl | |
| 2.2.1210 | 3-Brom-4-fluor-Phenyl | Fluormethyl | Ethyl | |
| 2.2.1211 | 3-Methyl-4-fluor-Phenyl | Fluormethyl | Ethyl | |
| 2.2.1212 | 3-Methyl-4-fluor-Phenyl | Chlormethyl | Ethyl | |
| 2.2.1213 | 3-Cyclopropyl-4-fluor- Phenyl | Fluormethyl | Ethyl | |
| 2.2.1214 | 3-Cyano-4-fluor-Phenyl | Fluormethyl | Ethyl | |
| 2.2.1215 | 3-Methoxy-4-fluor-Phenyl | Fluormethyl | Ethyl | |
| 2.2.1216 | 3-Ethoxy-4-fluor-Phenyl | Fluormethyl | Ethyl | |
| 2.2.1217 | 3-Trifluormethoxy-4-fluorPhenyl | Fluormethyl | Ethyl | |
| 2.2.1218 | 3-Nitro-4-fluor-Phenyl | Fluormethyl | Ethyl | |
| 2.2.1219 | 3-Fluor-4-chlor-Phenyl | Fluormethyl | Ethyl | |
| 2.2.1220 | 3,4-Dichlor-Phenyl | Fluormethyl | Ethyl | |
| 2.2.1221 | 3-Brom-4-chlor-Phenyl | Fluormethyl | Ethyl | |
| 2.2.1222 | 3-Methyl-4-chlor-Phenyl | Fluormethyl | Ethyl | |
| 2.2.1223 | 3-Cyclopropyl-4-chlorPhenyl | Fluormethyl | Ethyl | |
| 2.2.1224 | 3-Cyano-4-chlor-Phenyl | Fluormethyl | Ethyl | |
| 2.2.1225 | 3-Trifluormethyl-4-chlorPhenyl | Fluormethyl | Ethyl | |
| 2.2.1226 | 3-Methoxy-4-chlor-Phenyl | Fluormethyl | Ethyl | |
| 2.2.1227 | 3-Ethoxy-4-chlor-Phenyl | Fluormethyl | Ethyl | |
| 2.2.1228 | 3-Trifluormethoxy-4-chlorPhenyl | Fluormethyl | Ethyl | |
| 2.2.1229 | 3-Nitro-4-chlor-Phenyl | Fluormethyl | Ethyl | |
| 2.2.1230 | 3-Fluor-4-brom-Phenyl | Fluormethyl | Ethyl | |
| 2.2.1231 | 3-Chlor-4-brom-Phenyl | Fluormethyl | Ethyl | |
| 2.2.1232 | 3,4-Dibrom-Phenyl | Fluormethyl | Ethyl | |
| 2.2.1233 | 3-Methyl-4-brom-Phenyl | Fluormethyl | Ethyl | |
| 2.2.1234 | 3-Ethyl-4-brom-Phenyl | Fluormethyl | Ethyl | |
| 2.2.1235 | 3-Cyclopropyl-4-bromPhenyl | Fluormethyl | Ethyl | |
| 2.2.1236 | 3-Cyano-4-brom-Phenyl | Fluormethyl | Ethyl | |
| 2.2.1237 | 3-Trifluormethyl-4-bromPhenyl | Fluormethyl | Ethyl | |
| 2.2.1238 | 3-Methoxy-4-Phenyl | Fluormethyl | Ethyl | |
| 2.2.1239 | 3-Ethoxy-4-brom-Phenyl | Fluormethyl | Ethyl | |
| 2.2.1240 | 3- Trifluormethoxy-4-bromPhenyl | Fluormethyl | Ethyl | |
| 2.2.1241 | 3-Nitro-4-brom-Phenyl | Fluormethyl | Ethyl | |
| 2.2.1242 | 3-Fluor-4-iod-Phenyl | Fluormethyl | Ethyl | |
| 2.2.1243 | 3-Chlor-4-iod-Phenyl | Fluormethyl | Ethyl | |
| 2.2.1244 | 3-Brom-4-iod-Phenyl | Fluormethyl | Ethyl | |
| 2.2.1245 | 3-Methyl-4-iod-Phenyl | Fluormethyl | Ethyl | |
| 2.2.1246 | 3-Cyclopropyl-4-iod-Phenyl | Fluormethyl | Ethyl | |
| 2.2.1247 | 3-Cyano-4-iod-Phenyl | Fluormethyl | Ethyl | |
| 2.2.1248 | 3- Trifluormethyl-4-iod-Phenyl | Fluormethyl | Ethyl | |
| 2.2.1249 | 3-Methoxy-4-iod-Phenyl | Fluormethyl | Ethyl | |
| 2.2.1250 | 3-Ethoxy-4-iod-Phenyl | Fluormethyl | Ethyl | |
| 2.2.1251 | 3-Trifluormethoxy-4-iod-Phenyl | Fluormethyl | Ethyl | |
| 2.2.1252 | 3-Nitro-4-iod-Phenyl | Fluormethyl | Ethyl | |
| 2.2.1253 | 3-Fluor-4-methyl-Phenyl | Fluormethyl | Ethyl | |
| 2.2.1254 | 3-Chlor-4-methyl-Phenyl | Fluormethyl | Ethyl | |
| 2.2.1255 | 3-Brom-4-methyl-Phenyl | Fluormethyl | Ethyl | |
| 2.2.1256 | 3.4-Dimethyl-Phenyl | Fluormethyl | Ethyl | |
| 2.2.1257 | 3.4-Dimethyl-Phenyl | Chlormethyl | Ethyl | |
| 2.2.1258 | 3.4-Dimethyl-Phenyl | Brommethyl | Ethyl | |
| 2.2.1259 | 3.4-Dimethyl-Phenyl | Difluormethyl | Ethyl | |
| 2.2.1260 | 3.4-Dimethyl-Phenyl | Trifluormethyl | Ethyl | |
| 2.2.1261 | 3.4-Dimethyl-Phenyl | Cyano | Ethyl | |
| 2.2.1262 | 3-Ethyl-4-methyl-Phenyl | Fluormethyl | Ethyl | |
| 2.2.1263 | 3-Cyclopropyl-4-methylPhenyl | Fluormethyl | Ethyl | |
| 2.2.1264 | 3-Cyano-4-methyl-Phenyl | Fluormethyl | Ethyl | |
| 2.2.1265 | 3-Trifluormethyl-4-methylPhenyl | Fluormethyl | Ethyl | |
| 2.2.1266 | 3-Methoxy-4-methylPhenyl | Fluormethyl | Ethyl | |
| 2.2.1267 | 3-Ethoxy-4-methyl-Phenyl | Fluormethyl | Ethyl | |
| 2.2.1268 | 3-Trifluormethoxy-4-methyl-Phenyl | Fluormethyl | Ethyl | |
| 2.2.1269 | 3-Nitro-4-methyl-Phenyl | Fluormethyl | Ethyl | |
| 2.2.1270 | 3-Fluor-4-ethyl-Phenyl | Fluormethyl | Ethyl | |
| 2.2.1271 | 3-Chlor-4-ethyl-Phenyl | Fluormethyl | Ethyl | |
| 2.2.1272 | 3-Brom-4-ethyl-Phenyl | Fluormethyl | Ethyl | |
| 2.2.1273 | 3-Methyl-4-ethyl-Phenyl | Fluormethyl | Ethyl | |
| 2.2.1274 | 3,4-Diethyl-Phenyl | Fluormethyl | Ethyl | |
| 2.2.1275 | 3-Cyclopropyl-4-ethylPhenyl | Fluormethyl | Ethyl | |
| 2.2.1276 | 3-Cyano-4-ethyl-Phenyl | Fluormethyl | Ethyl | |
| 2.2.1277 | 3-Trifluormethyl-4-ethylPhenyl | Fluormethyl | Ethyl | |
| 2.2.1278 | 3-Methoxy-4-ethyl-Phenyl | Fluormethyl | Ethyl | |
| 2.2.1279 | 3-Ethoxy-4-ethyl-Phenyl | Fluormethyl | Ethyl | |
| 2.2.1280 | 3-Trifluormethoxy-4-ethylPhenyl | Fluormethyl | Ethyl | |
| 2.2.1281 | 3-Nitro-4-ethyl-Phenyl | Fluormethyl | Ethyl | |
| 2.2.1282 | 3-Fluor-4-propyl-Phenyl | Fluormethyl | Ethyl | |
| 2.2.1283 | 3-Chlor-4-propyl-Phenyl | Fluormethyl | Ethyl | |
| 2.2.1284 | 3-Brom-4-propyl-Phenyl | Fluormethyl | Ethyl | |
| 2.2.1285 | 3-Methyl-4-propyl-Phenyl | Fluormethyl | Ethyl | |
| 2.2.1286 | 3-Cyclop ropyl-4-propylPhenyl | Fluormethyl | Ethyl | |
| 2.2.1287 | 3-Cyano-4-propyl-Phenyl | Fluormethyl | Ethyl | |
| 2.2.1288 | 3-Trifluormethyl-4-propylPhenyl | Fluormethyl | Ethyl | |
| 2.2.1289 | 3-Methoxy-4-propylPhenyl | Fluormethyl | Ethyl | |
| 2.2.1290 | 3-Ethoxy-4-propyl-Phenyl | Fluormethyl | Ethyl | |
| 2.2.1291 | 3-Trifluormethoxy-4-propyl-Phenyl | Fluormethyl | Ethyl | |
| 2.2.1292 | 3-Nitro-4-propyl-Phenyl | Fluormethyl | Ethyl | |
| 2.2.1293 | 3-Fluor-4-isopropylPhenyl | Fluormethyl | Ethyl | |
| 2.2.1294 | 3-Chlor-4-isopropylPhenyl | Fluormethyl | Ethyl | |
| 2.2.1295 | 3-Brom-4-isopropylPhenyl | Fluormethyl | Ethyl | |
| 2.2.1296 | 3-Methyl-4-isopropylPhenyl | Fluormethyl | Ethyl | |
| 2.2.1297 | 3-Cyclop ropyl-4-isopropylPhenyl | Fluormethyl | Ethyl | |
| 2.2.1298 | 3-Cyano-4-isopropylPhenyl | Fluormethyl | Ethyl | |
| 2.2.1299 | 3-Trifluormethyl-4-isopropyl-Phenyl | Fluormethyl | Ethyl | |
| 2.2.1300 | 3-Methoxy-4-isopropylPhenyl | Fluormethyl | Ethyl | |
| 2.2.1301 | 3-Ethoxy-4-isopropylPhenyl | Fluormethyl | Ethyl | |
| 2.2.1302 | 3-Trifluormethoxy-4-isopropyl-Phenyl | Fluormethyl | Ethyl | |
| 2.2.1303 | 3-Nitro-4-isopropyl-Phenyl | Fluormethyl | Ethyl | |
| 2.2.1304 | 3-Fluor-4-tert.butyl-Phenyl | Fluormethyl | Ethyl | |
| 2.2.1305 | 3-Chlor-4-tert.buty-Phenyl | Fluormethyl | Ethyl | |
| 2.2.1306 | 3-Brom-4-tert.butyl-Phenyl | Fluormethyl | Ethyl | |
| 2.2.1307 | 3-Methyl-4-tert.butyl-Phenyl | Fluormethyl | Ethyl | |
| 2.2.1308 | 3-Ethyl-4-tert.butyl-Phenyl | Fluormethyl | Ethyl | |
| 2.2.1309 | 3-Cyclopropyl-4-tert.butyl-Phenyl | Fluormethyl | Ethyl | |
| 2.2.1310 | 3-Cyano-4-tert.butyl-Phenyl | Fluormethyl | Ethyl | |
| 2.2.1311 | 3-Trifluormethyl-4-tert.butyl-Phenyl | Fluormethyl | Ethyl | |
| 2.2.1312 | 3-Trifluormethyl-4-tert.butyl-Phenyl | Chlormethyl | Ethyl | |
| 2.2.1313 | 3-Trifluormethyl-4-tert.butyl-Phenyl | Brommethyl | Ethyl | |
| 2.2.1314 | 3-Trifluormethyl-4-tert.butyl-Phenyl | Difluormethyl | Ethyl | |
| 2.2.1315 | 3-Methoxy-4-tert.butyl- Phenyl | Fluormethyl | Ethyl | |
| 2.2.1316 | 3-Ethoxy-4-tert.butyl- Phenyl | Fluormethyl | Ethyl | |
| 2.2.1317 | 3-Trifluormethoxy-4-tert.butyl-Phenyl | Fluormethyl | Ethyl | |
| 2.2.1318 | 3-Nitro-4-tert.butyl-Phenyl | Fluormethyl | Ethyl | |
| 2.2.1319 | 3-Fluor-4-cyclopropyl-Phenyl | Fluormethyl | Ethyl | |
| 2.2.1320 | 3-Chlor-4-cyclopropyl-Phenyl | Fluormethyl | Ethyl | |
| 2.2.1321 | 3-Brom-4-cyclopropyl-Phenyl | Fluormethyl | Ethyl | |
| 2.2.1322 | 3-Methyl-4-cyclopropyl-Phenyl | Fluormethyl | Ethyl | |
| 2.2.1323 | 3-Cyclopropyl-4-cyclopropyl-Phenyl | Fluormethyl | Ethyl | |
| 2.2.1324 | 3-Cyano-4-cyclopropyl-Phenyl | Fluormethyl | Ethyl | |
| 2.2.1325 | 3-Trifluormethyl-4-cyclopropyl-Phenyl | Fluormethyl | Ethyl | |
| 2.2.1326 | 3-Methoxy-4-cyclopropyl-Phenyl | Fluormethyl | Ethyl | |
| 2.2.1327 | 3-Ethoxy-4-cyclopropyl-Phenyl | Fluormethyl | Ethyl | |
| 2.2.1328 | 3-Trifluormethoxy-4-cyclopropyl-Phenyl | Fluormethyl | Ethyl | |
| 2.2.1329 | 3-Fluor-4-methoxycarbonyl-Phenyl | Fluormethyl | Ethyl | |
| 2.2.1330 | 3-Chlor-4-methoxycarbonyl-Phenyl | Fluormethyl | Ethyl | |
| 2.2.1331 | 3-Brom-4-methoxycarbonyl-Phenyl | Fluormethyl | Ethyl | |
| 2.2.1332 | 3-Methyl-4-methoxycarbonyl-Phenyl | Fluormethyl | Ethyl | |
| 2.2.1333 | 3-Ethyl-4-methoxycarbonyl-Phenyl | Fluormethyl | Ethyl | |
| 2.2.1334 | 3-Cyclopropyl-4-methoxycarbonyl-Phenyl | Fluormethyl | Ethyl | |
| 2.2.1335 | 3-Cyano-4-methoxycarbonyl-Phenyl | Fluormethyl | Ethyl | |
| 2.2.1336 | 3-Trifluormethyl-4-methoxycarbonyl-Phenyl | Fluormethyl | Ethyl | |
| 2.2.1337 | 3-Methoxy-4-methoxycarbonyl -Phenyl | Fluormethyl | Ethyl | |
| 2.2.1338 | 3-Ethoxy-4-methoxycarbonyl-Phenyl | Fluormethyl | Ethyl | |
| 2.2.1339 | 3-Trifluormethoxy-4-methoxycarbonyl-Phenyl | Fluormethyl | Ethyl | |
| 2.2.1340 | 3-Nitro-4-methoxycarbonyl-Phenyl | Fluormethyl | Ethyl | |
| 2.2.1341 | 3-Fluor-4-cyano-Phenyl | Fluormethyl | Ethyl | |
| 2.2.1342 | 3-Chlor-4-cyano-Phenyl | Fluormethyl | Ethyl | |
| 2.2.1343 | 3-Brom-4-cyano-Phenyl | Fluormethyl | Ethyl | |
| 2.2.1344 | 3-Methyl-4-cyano-Phenyl | Fluormethyl | Ethyl | |
| 2.2.1345 | 3-Cyclop ropyl-4-cyano-Phenyl | Fluormethyl | Ethyl | |
| 2.2.1346 | 3,4-Dicyano-Phenyl | Fluormethyl | Ethyl | |
| 2.2.1347 | 3-Trifluormethyl-4-cyano-Phenyl | Fluormethyl | Ethyl | |
| 2.2.1348 | 3-Trifluormethyl-4-cyano-Phenyl | Chlormethyl | Ethyl | |
| 2.2.1349 | 3-Trifluormethyl-4-cyano-Phenyl | Brommethyl | Ethyl | |
| 2.2.1350 | 3-Trifluormethyl-4-cyano-Phenyl | Difluormethyl | Ethyl | |
| 2.2.1351 | 3-Methoxy-4-cyano - Phenyl | Fluormethyl | Ethyl | |
| 2.2.1352 | 3-Ethoxy-4-cyano-Phenyl | Fluormethyl | Ethyl | |
| 2.2.1353 | 3-Trifluormethoxy-4-cyano-Phenyl | Fluormethyl | Ethyl | |
| 2.2.1354 | 3-Nitro-4-cyano-Phenyl | Fluormethyl | Ethyl | |
| 2.2.1355 | 3-Fluor-4-methoxy-Phenyl | Fluormethyl | Ethyl | |
| 2.2.1356 | 3-Chlor-4-methoxy-Phenyl | Fluormethyl | Ethyl | |
| 2.2.1357 | 3-Brom-4-methoxy-Phenyl | Fluormethyl | Ethyl | |
| 2.2.1358 | 3-Methyl-4-methoxyPhenyl | Fluormethyl | Ethyl | |
| 2.2.1359 | 3-Cyclopropyl-4-methoxyPhenyl | Fluormethyl | Ethyl | |
| 2.2.1360 | 3-Cyano-4-methoxyPhenyl | Fluormethyl | Ethyl | |
| 2.2.1361 | 3-Trifluormethyl-4-methoxy-Phenyl | Fluormethyl | Ethyl | |
| 2.2.1362 | 3,4-Dimethoxy-Phenyl | Fluormethyl | Ethyl | |
| 2.2.1363 | 3-Ethoxy-4-methoxyPhenyl | Fluormethyl | Ethyl | |
| 2.2.1364 | 3-Trifluormethoxy-4-methoxy-Phenyl | Fluormethyl | Ethyl | |
| 2.2.1365 | 3-Nitro-4-methoxy-Phenyl | Fluormethyl | Ethyl | |
| 2.2.1366 | 3-Fluor-4-ethoxy-Phenyl | Fluormethyl | Ethyl | |
| 2.2.1367 | 3-Chlor-4-ethoxy-Phenyl | Fluormethyl | Ethyl | |
| 2.2.1368 | 3-Chlor-4-ethoxy-Phenyl | Chlormethyl | Ethyl | |
| 2.2.1369 | 3-Chlor-4-ethoxy-Phenyl | Brommethyl | Ethyl | |
| 2.2.1370 | 3-Chlor-4-ethoxy-Phenyl | Difluormethyl | Ethyl | |
| 2.2.1371 | 3-Brom-4-ethoxy-Phenyl | Fluormethyl | Ethyl | |
| 2.2.1372 | 3-Methyl-4-ethoxy-Phenyl | Fluormethyl | Ethyl | |
| 2.2.1373 | 3-Cyclopropyl-4-ethoxyPhenyl | Fluormethyl | Ethyl | |
| 2.2.1374 | 3-Cyano-4-ethoxy-Phenyl | Fluormethyl | Ethyl | |
| 2.2.1375 | 3-Trifluormethyl-4-ethoxyPhenyl | Fluormethyl | Ethyl | |
| 2.2.1376 | 3-Methoxy-4-ethoxy- Phenyl | Fluormethyl | Ethyl | |
| 2.2.1377 | 2,4-Diethoxy-Phenyl | Fluormethyl | Ethyl | |
| 2.2.1378 | 3-Trifluormethoxy-4-ethoxy-Phenyl | Fluormethyl | Ethyl | |
| 2.2.1379 | 3-Nitro-4-ethoxy-Phenyl | Fluormethyl | Ethyl | |
| 2.2.1380 | 3-Fluor-4-propoxy-Phenyl | Fluormethyl | Ethyl | |
| 2.2.1381 | 3-Chlor-4-propoxy-Phenyl | Fluormethyl | Ethyl | |
| 2.2.1382 | 3-Brom-4-propoxy-Phenyl | Fluormethyl | Ethyl | |
| 2.2.1383 | 3-Methyl-4-propoxy-Phenyl | Fluormethyl | Ethyl | |
| 2.2.1384 | 3-Cyclopropyl-4-propoxy-Phenyl | Fluormethyl | Ethyl | |
| 2.2.1385 | 3-Cyano-4-propoxy-Phenyl | Fluormethyl | Ethyl | |
| 2.2.1386 | 3-Trifluormethyl-4-propoxy-Phenyl | Fluormethyl | Ethyl | |
| 2.2.1387 | 3-Methoxy-4-propoxy-Phenyl | Fluormethyl | Ethyl | |
| 2.2.1388 | 3-Ethoxy-4-propoxy-Phenyl | Fluormethyl | Ethyl | |
| 2.2.1389 | 3-Trifluormethoxy-4-propoxy-Phenyl | Fluormethyl | Ethyl | |
| 2.2.1390 | 3-Nitro-4-propoxy-Phenyl | Fluormethyl | Ethyl | |
| 2.2.1391 | 3-Fluor-4-isopropoxy-Phenyl | Fluormethyl | Ethyl | |
| 2.2.1392 | 3-Chlor-4-isopropoxy-Phenyl | Fluormethyl | Ethyl | |
| 2.2.1393 | 3-Brom-4-isopropoxy-Phenyl | Fluormethyl | Ethyl | |
| 2.2.1394 | 3-Methyl-4-isopropoxy-Phenyl | Fluormethyl | Ethyl | |
| 2.2.1395 | 3-Cyclopropyl-4-isopropoxy-Phenyl | Fluormethyl | Ethyl | |
| 2.2.1396 | 3-Cyano-4-isopropoxy-Phenyl | Fluormethyl | Ethyl | |
| 2.2.1397 | 3-Trifluormethyl-4-isopropoxy-Phenyl | Fluormethyl | Ethyl | |
| 2.2.1398 | 3-Methoxy-4-isopropoxy-Phenyl | Fluormethyl | Ethyl | |
| 2.2.1399 | 3-Ethoxy-4-isopropoxy-Phenyl | Fluormethyl | Ethyl | |
| 2.2.1400 | 3-Trifluormethoxy-4-isopropoxy-Phenyl | Fluormethyl | Ethyl | |
| 2.2.1401 | 3-Nitro-4-isopropoxy-Phenyl | Fluormethyl | Ethyl | |
| 2.2.1402 | 3-Fluor-4-trifluormethoxy-Phenyl | Fluormethyl | Ethyl | |
| 2.2.1403 | 3-Chlor-4-trifluormethoxy-Phenyl | Fluormethyl | Ethyl | |
| 2.2.1404 | 3-Brom-4-trifluormethoxy-Phenyl | Fluormethyl | Ethyl | |
| 2.2.1405 | 3-Methyl-4-trifluormethoxy-Phenyl | Fluormethyl | Ethyl | |
| 2.2.1406 | 3-Cyclopropyl-4-trifluormethoxy-Phenyl | Fluormethyl | Ethyl | |
| 2.2.1407 | 3-Cyano-4-trifluormethoxy-Phenyl | Fluormethyl | Ethyl | |
| 2.2.1408 | 3-Trifluormethyl-4-trifluormethoxy-Phenyl | Fluormethyl | Ethyl | |
| 2.2.1409 | 3-Methoxy-4-trifluormethoxy-Phenyl | Fluormethyl | Ethyl | |
| 2.2.1410 | 3-Ethoxy-4-trifluormethoxy-Phenyl | Fluormethyl | Ethyl | |
| 2.2.1411 | 3,4-Bis(trifluormethoxy)-Phenyl | Fluormethyl | Ethyl | |
| 2.2.1412 | 3-Nitro-4-trifluormethoxyPhenyl | Fluormethyl | Ethyl | |
| 2.2.1413 | 3-Fluor-4-difluormethoxy-Phenyl | Fluormethyl | Ethyl | |
| 2.2.1414 | 3-Chlor-4-difluormethoxy-Phenyl | Fluormethyl | Ethyl | |
| 2.2.1415 | 3-Brom-4-difluormethoxy-Phenyl | Fluormethyl | Ethyl | |
| 2.2.1416 | 3-Methyl-4-difluormethoxy-Phenyl | Fluormethyl | Ethyl | |
| 2.2.1417 | 3-Cyclopropyl-4-difluormethoxy-Phenyl | Fluormethyl | Ethyl | |
| 2.2.1418 | 3-Cyano-4-difluormethoxy-Phenyl | Fluormethyl | Ethyl | |
| 2.2.1419 | 3-Trifluormethyl-4-difluormethoxy-Phenyl | Fluormethyl | Ethyl | |
| 2.2.1420 | 3-Methoxy-4-difluormethoxy-Phenyl | Fluormethyl | Ethyl | |
| 2.2.1421 | 3-Ethoxy-4-difluormethoxy-Phenyl | Fluormethyl | Ethyl | |
| 2.2.1422 | 3-Trifluormethoxy-4-difluormethoxy-Phenyl | Fluormethyl | Ethyl | |
| 2.2.1423 | 3-Nitro-4-difluormethoxyPhenyl | Fluormethyl | Ethyl | |
| 2.2.1424 | 3-Fluor-4-nitro-Phenyl | Fluormethyl | Ethyl | |
| 2.2.1425 | 3-Chlor-4-nitro-Phenyl | Fluormethyl | Ethyl | |
| 2.2.1426 | 3-Brom-4-nitro-Phenyl | Fluormethyl | Ethyl | |
| 2.2.1427 | 3-Methyl-4-nitro-Phenyl | Fluormethyl | Ethyl | |
| 2.2.1428 | 3-Cyclopropyl-4-nitroPhenyl | Fluormethyl | Ethyl | |
| 2.2.1429 | 3-Cyano-4-nitro-Phenyl | Fluormethyl | Ethyl | |
| 2.2.1430 | 3-Trifluormethyl-4-nitroPhenyl | Fluormethyl | Ethyl | |
| 2.2.1431 | 3-Methoxy-4-nitro-Phenyl | Fluormethyl | Ethyl | |
| 2.2.1432 | 3-Ethoxy-4-nitro-Phenyl | Fluormethyl | Ethyl | |
| 2.2.1433 | 3-Trifluormethoxy-4-nitroPhenyl | Fluormethyl | Ethyl | |
| 2.2.1434 | 3-Fluor-4-methylsulfanylPhenyl | Fluormethyl | Ethyl | |
| 2.2.1435 | 3-Chlor-4-methylsulfanyl-Phenyl | Fluormethyl | Ethyl | |
| 2.2.1436 | 3-Brom-4-methylsulfanyl-Phenyl | Fluormethyl | Ethyl | |
| 2.2.1437 | 3-Methyl-4-methylsulfanyl-Phenyl | Fluormethyl | Ethyl | |
| 2.2.1438 | 3-Cyclopropyl-4-methylsulfanyl-Phenyl | Fluormethyl | Ethyl | |
| 2.2.1439 | 3-Cyano-4-methylsulfanyl-Phenyl | Fluormethyl | Ethyl | |
| 2.2.1440 | 3-Trifluormethyl-4-methylsulfanyl-Phenyl | Fluormethyl | Ethyl | |
| 2.2.1441 | 3-Methoxy-4-methylsulfanyl-Phenyl | Fluormethyl | Ethyl | |
| 2.2.1442 | 3-Ethoxy-4-methylsulfanyl-Phenyl | Fluormethyl | Ethyl | |
| 2.2.1443 | 3-Trifluormethoxy-4-methylsulfanyl-Phenyl | Fluormethyl | Ethyl | |
| 2.2.1444 | 3-Nitro-4-methylsulfanyl-Phenyl | Fluormethyl | Ethyl | |
| 2.2.1445 | 3,6-Difluor-Phenyl | Fluormethyl | Ethyl | |
| 2.2.1446 | 3,6-Difluor-Phenyl | Chlormethyl | Ethyl | |
| 2.2.1447 | 3,6-Difluor-Phenyl | Brommethyl | Ethyl | |
| 2.2.1448 | 3,6-Difluor-Phenyl | Difluormethyl | Ethyl | |
| 2.2.1449 | 3,6-Difluor-Phenyl | Trifluormethyl | Ethyl | |
| 2.2.1450 | 3,6-Difluor-Phenyl | Cyano | Ethyl | |
| 2.2.1451 | 3-Chlor-6-fluor-Phenyl | Fluormethyl | Ethyl | |
| 2.2.1452 | 3-Brom-6-fluor-Phenyl | Fluormethyl | Ethyl | |
| 2.2.1453 | 3-Methyl-6-fluor-Phenyl | Fluormethyl | Ethyl | |
| 2.2.1454 | 3-Ethyl-6-fluor-Phenyl | Fluormethyl | Ethyl | |
| 2.2.1455 | 3-Cyclopropyl-6-fluorPhenyl | Fluormethyl | Ethyl | |
| 2.2.1456 | 3-Cyano-6-fluor-Phenyl | Fluormethyl | Ethyl | |
| 2.2.1457 | 3-Methoxy-6-fluor-Phenyl | Fluormethyl | Ethyl | |
| 2.2.1458 | 3-Ethoxy-6-fluor-Phenyl | Fluormethyl | Ethyl | |
| 2.2.1459 | 3-Trifluormethoxy-6-fluorPhenyl | Fluormethyl | Ethyl | |
| 2.2.1460 | 3-Nitro-6-fluor-Phenyl | Fluormethyl | Ethyl | |
| 2.2.1461 | 3-Fluor-6-chlor-Phenyl | Fluormethyl | Ethyl | |
| 2.2.1462 | 3-Fluor-6-chlor-Phenyl | Chlormethyl | Ethyl | |
| 2.2.1463 | 3-Fluor-6-chlor-Phenyl | Brommethyl | Ethyl | |
| 2.2.1464 | 3-Fluor-6-chlor-Phenyl | Difluormethyl | Ethyl | |
| 2.2.1465 | 3,6-Dichlor-Phenyl | Fluormethyl | Ethyl | |
| 2.2.1466 | 3,6-Dichlor-Phenyl | Chlormethyl | Ethyl | |
| 2.2.1467 | 3,6-Dichlor-Phenyl | Brommethyl | Ethyl | |
| 2.2.1468 | 3,6-Dichlor-Phenyl | Difluormethyl | Ethyl | |
| 2.2.1469 | 3,6-Dichlor-Phenyl | Trifluor-methyl | Ethyl | |
| 2.2.1470 | 3,6-Dichlor-Phenyl | Cyano | Ethyl | |
| 2.2.1471 | 3-Brom-6-chlor-Phenyl | Fluormethyl | Ethyl | |
| 2.2.1472 | 3-Methyl-6-chlor-Phenyl | Fluormethyl | Ethyl | |
| 2.2.1473 | 3-Cyclopropyl-6-chlorPhenyl | Fluormethyl | Ethyl | |
| 2.2.1474 | 3-Cyano-6-chlor-Phenyl | Fluormethyl | Ethyl | |
| 2.2.1475 | 3-Trifluormethyl-6-chlorPhenyl | Fluormethyl | Ethyl | |
| 2.2.1476 | 3-Methoxy-6-chlor-Phenyl | Fluormethyl | Ethyl | |
| 2.2.1477 | 3-Ethoxy-6-chlor-Phenyl | Fluormethyl | Ethyl | |
| 2.2.1478 | 3-Trifluormethoxy-6-chlorPhenyl | Fluormethyl | Ethyl | |
| 2.2.1479 | 3-Nitro-6-chlor-Phenyl | Fluormethyl | Ethyl | |
| 2.2.1480 | 3-Fluor-6-brom-Phenyl | Fluormethyl | Ethyl | |
| 2.2.1481 | 3-Chlor-6-brom-Phenyl | Fluormethyl | Ethyl | |
| 2.2.1482 | 3,6-Dibrom-Phenyl | Fluormethyl | Ethyl | |
| 2.2.1483 | 3-Methyl-6-brom-Phenyl | Fluormethyl | Ethyl | |
| 2.2.1484 | 3-Cyclopropyl-6-bromPhenyl | Fluormethyl | Ethyl | |
| 2.2.1485 | 3-Cyano-6-brom-Phenyl | Fluormethyl | Ethyl | |
| 2.2.1486 | 3-Trifluormethyl-6-bromPhenyl | Fluormethyl | Ethyl | |
| 2.2.1487 | 3-Methoxy-6-Phenyl | Fluormethyl | Ethyl | |
| 2.2.1488 | 3-Ethoxy-6-brom-Phenyl | Fluormethyl | Ethyl | |
| 2.2.1489 | 3-Trifluormethoxy-6-bromPhenyl | Fluormethyl | Ethyl | |
| 2.2.1490 | 3-Nitro-6-brom-Phenyl | Fluormethyl | Ethyl | |
| 2.2.1491 | 3-Fluor-6-iod-Phenyl | Fluormethyl | Ethyl | |
| 2.2.1492 | 3-Chlor-6-iod-Phenyl | Fluormethyl | Ethyl | |
| 2.2.1493 | 3-Brom-6-iod-Phenyl | Fluormethyl | Ethyl | |
| 2.2.1494 | 3-Methyl-6-iod-Phenyl | Fluormethyl | Ethyl | |
| 2.2.1495 | 3-Cyclopropyl-6-iod-Phenyl | Fluormethyl | Ethyl | |
| 2.2.1496 | 3-Cyano-6-iod-Phenyl | Fluormethyl | Ethyl | |
| 2.2.1497 | 3-Trifluormethyl-6-iod-Phenyl | Fluormethyl | Ethyl | |
| 2.2.1498 | 3-Methoxy-6-iod-Phenyl | Fluormethyl | Ethyl | |
| 2.2.1499 | 3-Ethoxy-6-iod-Phenyl | Fluormethyl | Ethyl | |
| 2.2.1500 | 3-Trifluormethoxy-6-iod-Phenyl | Fluormethyl | Ethyl | |
| 2.2.1501 | 3-Nitro-6-iod-Phenyl | Fluormethyl | Ethyl | |
| 2.2.1502 | 3-Fluor-6-methyl-Phenyl | Fluormethyl | Ethyl | |
| 2.2.1503 | 3-Chlor-6-methyl-Phenyl | Fluormethyl | Ethyl | |
| 2.2.1504 | 3-Brom-6-methyl-Phenyl | Fluormethyl | Ethyl | |
| 2.2.1505 | 3.6-Dimethyl-Phenyl | Fluormethyl | Ethyl | |
| 2.2.1506 | 3-Ethyl-6-methyl-Phenyl | Fluormethyl | Ethyl | |
| 2.2.1507 | 3-Cyclopropyl-6-methylPhenyl | Fluormethyl | Ethyl | |
| 2.2.1508 | 3-Cyano-6-methyl-Phenyl | Fluormethyl | Ethyl | |
| 2.2.1509 | 3-Trifluormethyl-6-methyl-Phenyl | Fluormethyl | Ethyl | |
| 2.2.1510 | 3-Methoxy-6-methylPhenyl | Fluormethyl | Ethyl | |
| 2.2.1511 | 3-Ethoxy-6-methyl-Phenyl | Fluormethyl | Ethyl | |
| 2.2.1512 | 3-Trifluormethoxy-6-methyl-Phenyl | Fluormethyl | Ethyl | |
| 2.2.1513 | 3-Nitro-6-methyl-Phenyl | Fluormethyl | Ethyl | |
| 2.2.1514 | 3-Fluor-6-ethyl-Phenyl | Fluormethyl | Ethyl | |
| 2.2.1515 | 3-Chlor-6-ethyl-Phenyl | Fluormethyl | Ethyl | |
| 2.2.1516 | 3-Brom-6-ethyl-Phenyl | Fluormethyl | Ethyl | |
| 2.2.1517 | 3-Methyl-6-ethyl-Phenyl | Fluormethyl | Ethyl | |
| 2.2.1518 | 3,6-Diethyl-Phenyl | Fluormethyl | Ethyl | |
| 2.2.1519 | 3-Cyclopropyl-6-ethylPhenyl | Fluormethyl | Ethyl | |
| 2.2.1520 | 3-Cyano-6-ethyl-Phenyl | Fluormethyl | Ethyl | |
| 2.2.1521 | 3-Trifluormethyl-6-ethylPhenyl | Fluormethyl | Ethyl | |
| 2.2.1522 | 3-Methoxy-6-ethyl-Phenyl | Fluormethyl | Ethyl | |
| 2.2.1523 | 3-Ethoxy-6-ethyl-Phenyl | Fluormethyl | Ethyl | |
| 2.2.1524 | 3-Trifluormethoxy-6-ethylPhenyl | Fluormethyl | Ethyl | |
| 2.2.1525 | 3-Nitro-6-ethyl-Phenyl | Fluormethyl | Ethyl | |
| 2.2.1526 | 3-Fluor-6-propyl-Phenyl | Fluormethyl | Ethyl | |
| 2.2.1527 | 3-Chlor-6-propyl-Phenyl | Fluormethyl | Ethyl | |
| 2.2.1528 | 3-Brom-6-propyl-Phenyl | Fluormethyl | Ethyl | |
| 2.2.1529 | 3-Methyl-6-propyl-Phenyl | Fluormethyl | Ethyl | |
| 2.2.1530 | 3-Cyclopropyl-6-propylPhenyl | Fluormethyl | Ethyl | |
| 2.2.1531 | 3-Cyano-6-propyl-Phenyl | Fluormethyl | Ethyl | |
| 2.2.1532 | 3-Trifluormethyl-6-propylPhenyl | Fluormethyl | Ethyl | |
| 2.2.1533 | 3-Methoxy-6-propyl-Phenyl | Fluormethyl | Ethyl | |
| 2.2.1534 | 3-Ethoxy-6-propyl-Phenyl | Fluormethyl | Ethyl | |
| 2.2.1535 | 3-Trifluormethoxy-6-propyl-Phenyl | Fluormethyl | Ethyl | |
| 2.2.1536 | 3-Nitro-6-propyl-Phenyl | Fluormethyl | Ethyl | |
| 2.2.1537 | 3-Fluor-6-isopropyl-Phenyl | Fluormethyl | Ethyl | |
| 2.2.1538 | 3-Chlor-6-isopropyl-Phenyl | Fluormethyl | Ethyl | |
| 2.2.1539 | 3-Brom-6-isopropyl-Phenyl | Fluormethyl | Ethyl | |
| 2.2.1540 | 3-Methyl-6-isopropyl-Phenyl | Fluormethyl | Ethyl | |
| 2.2.1541 | 3-Cyclopropyl-6-isopropyl-Phenyl | Fluormethyl | Ethyl | |
| 2.2.1542 | 3-Cyano-6-isopropyl-Phenyl | Fluormethyl | Ethyl | |
| 2.2.1543 | 3-Trifluormethyl-6-isopropyl-Phenyl | Fluormethyl | Ethyl | |
| 2.2.1544 | 3-Methoxy-6-isopropyl-Phenyl | Fluormethyl | Ethyl | |
| 2.2.1545 | 3-Ethoxy-6-isopropyl-Phenyl | Fluormethyl | Ethyl | |
| 2.2.1546 | 3-Trifluormethoxy-6-isopropyl-Phenyl | Fluormethyl | Ethyl | |
| 2.2.1547 | 3-Nitro-6-isopropyl-Phenyl | Fluormethyl | Ethyl | |
| 2.2.1548 | 3-Fluor-6-tert.butyl-Phenyl | Fluormethyl | Ethyl | |
| 2.2.1549 | 3-Chlor-6-tert.buty- Phenyl | Fluormethyl | Ethyl | |
| 2.2.1550 | 3-Brom-6-tert.butyl-Phenyl | Fluormethyl | Ethyl | |
| 2.2.1551 | 3-Methyl-6-tert.butyl-Phenyl | Fluormethyl | Ethyl | |
| 2.2.1552 | 3-Cyclopropyl-6-tert.butyl-Phenyl | Fluormethyl | Ethyl | |
| 2.2.1553 | 3-Cyano-6-tert.butyl-Phenyl | Fluormethyl | Ethyl | |
| 2.2.1554 | 3-Trifluormethyl-6-tert.butyl-Phenyl | Fluormethyl | Ethyl | |
| 2.2.1555 | 3-Methoxy-6-tert.butyl-Phenyl | Fluormethyl | Ethyl | |
| 2.2.1556 | 3-Ethoxy-6-tert.butyl-Phenyl | Fluormethyl | Ethyl | |
| 2.2.1557 | 3-Trifluormethoxy-6-tert.butyl-Phenyl | Fluormethyl | Ethyl | |
| 2.2.1558 | 3-Nitro-6-tert.butyl-Phenyl | Fluormethyl | Ethyl | |
| 2.2.1559 | 3-Fluor-6-cyclopropyl-Phenyl | Fluormethyl | Ethyl | |
| 2.2.1560 | 3-Chlor-6-cyclopropyl-Phenyl | Fluormethyl | Ethyl | |
| 2.2.1561 | 3-Brom-6-cyclopropyl-Phenyl | Fluormethyl | Ethyl | |
| 2.2.1562 | 3-Methyl-6-cyclopropyl-Phenyl | Fluormethyl | Ethyl | |
| 2.2.1563 | 3-Cyclopropyl-6-cyclopropyl-Phenyl | Fluormethyl | Ethyl | |
| 2.2.1564 | 3-Cyano-6-cyclopropyl-Phenyl | Fluormethyl | Ethyl | |
| 2.2.1565 | 3-Trifluormethyl-6-cyclopropyl-Phenyl | Fluormethyl | Ethyl | |
| 2.2.1566 | 3-Methoxy-6-cyclopropyl-Phenyl | Fluormethyl | Ethyl | |
| 2.2.1567 | 3-Ethoxy-6-cyclopropyl-Phenyl | Fluormethyl | Ethyl | |
| 2.2.1568 | 3-Trifluormethoxy-6-cyclopropyl-Phenyl | Fluormethyl | Ethyl | |
| 2.2.1569 | 3-Fluor-6-methoxycarbonyl-Phenyl | Fluormethyl | Ethyl | |
| 2.2.1570 | 3-Chlor-6-methoxycarbonyl-Phenyl | Fluormethyl | Ethyl | |
| 2.2.1571 | 3-Brom-6-methoxycarbonyl-Phenyl | Fluormethyl | Ethyl | |
| 2.2.1572 | 3-Methyl-6-methoxycarbonyl-Phenyl | Fluormethyl | Ethyl | |
| 2.2.1573 | 3-Cyclopropyl-6-methoxycarbonyl-Phenyl | Fluormethyl | Ethyl | |
| 2.2.1574 | 3-Cyano-6-methoxycarbonyl-Phenyl | Fluormethyl | Ethyl | |
| 2.2.1575 | 3-Trifluormethyl-6-methoxycarbonyl-Phenyl | Fluormethyl | Ethyl | |
| 2.2.1576 | 3-Methoxy-6-methoxycarbonyl-Phenyl | Fluormethyl | Ethyl | |
| 2.2.1577 | 3-Ethoxy-6-methoxycarbonyl-Phenyl | Fluormethyl | Ethyl | |
| 2.2.1578 | 3-Trifluormethoxy-6-methoxycarbonyl-Phenyl | Fluormethyl | Ethyl | |
| 2.2.1579 | 3-Nitro-6-methoxycarbonyl-Phenyl | Fluormethyl | Ethyl | |
| 2.2.1580 | 3-Fluor-6-cyano-Phenyl | Fluormethyl | Ethyl | |
| 2.2.1581 | 3-Chlor-6-cyano-Phenyl | Fluormethyl | Ethyl | |
| 2.2.1582 | 3-Brom-6-cyano-Phenyl | Fluormethyl | Ethyl | |
| 2.2.1583 | 3-Methyl-6-cyano-Phenyl | Fluormethyl | Ethyl | |
| 2.2.1584 | 3-Cyclopropyl-6-cyano-Phenyl | Fluormethyl | Ethyl | |
| 2.2.1585 | 3-Cyano-6-cyano-Phenyl | Fluormethyl | Ethyl | |
| 2.2.1586 | 3-Trifluormethyl-6-cyano-Phenyl | Fluormethyl | Ethyl | |
| 2.2.1587 | 3-Methoxy-6-cyano-Phenyl | Fluormethyl | Ethyl | |
| 2.2.1588 | 3-Ethoxy-6-cyano-Phenyl | Fluormethyl | Ethyl | |
| 2.2.1589 | 3-Trifluormethoxy-6-cyano-Phenyl | Fluormethyl | Ethyl | |
| 2.2.1590 | 3-Nitro-6-cyano-Phenyl | Fluormethyl | Ethyl | |
| 2.2.1591 | 3-Fluor-6-methoxy-Phenyl | Fluormethyl | Ethyl | |
| 2.2.1592 | 3-Chlor-6-methoxy-Phenyl | Fluormethyl | Ethyl | |
| 2.2.1593 | 3-Brom-6-methoxy-Phenyl | Fluormethyl | Ethyl | |
| 2.2.1594 | 3-Methyl-6-methoxy-Phenyl | Fluormethyl | Ethyl | |
| 2.2.1595 | 3-Cyclopropyl-6-methoxy-Phenyl | Fluormethyl | Ethyl | |
| 2.2.1596 | 3-Cyano-6-methoxy-Phenyl | Fluormethyl | Ethyl | |
| 2.2.1597 | 3-Trifluormethyl-6-methoxy-Phenyl | Fluormethyl | Ethyl | |
| 2.2.1598 | 3,6-Dimethoxy-Phenyl | Fluormethyl | Ethyl | |
| 2.2.1599 | 3-Ethoxy-6-methoxy-Phenyl | Fluormethyl | Ethyl | |
| 2.2.1600 | 3-Trifluormethoxy-6-methoxy-Phenyl | Fluormethyl | Ethyl | |
| 2.2.1601 | 3-Nitro-6-methoxy-Phenyl | Fluormethyl | Ethyl | |
| 2.2.1602 | 3-Fluor-6-ethoxy-Phenyl | Fluormethyl | Ethyl | |
| 2.2.1603 | 3-Chlor-6-ethoxy-Phenyl | Fluormethyl | Ethyl | |
| 2.2.1604 | 3-Brom-6-ethoxy-Phenyl | Fluormethyl | Ethyl | |
| 2.2.1605 | 3-Methyl-6-ethoxy-Phenyl | Fluormethyl | Ethyl | |
| 2.2.1606 | 3-Cyclopropyl-6-ethoxy-Phenyl | Fluormethyl | Ethyl | |
| 2.2.1607 | 3-Cyano-6-ethoxy-Phenyl | Fluormethyl | Ethyl | |
| 2.2.1608 | 3-Trifluormethyl-6-ethoxy-Phenyl | Fluormethyl | Ethyl | |
| 2.2.1609 | 3-Methoxy-6-ethoxy-Phenyl | Fluormethyl | Ethyl | |
| 2.2.1610 | 2,6-Diethoxy-Phenyl | Fluormethyl | Ethyl | |
| 2.2.1611 | 3-Trifluormethoxy-6-ethoxy-Phenyl | Fluormethyl | Ethyl | |
| 2.2.1612 | 3-Nitro-6-ethoxy-Phenyl | Fluormethyl | Ethyl | |
| 2.2.1613 | 3-Fluor-6-propoxy-Phenyl | Fluormethyl | Ethyl | |
| 2.2.1614 | 3-Chlor-6-propoxy-Phenyl | Fluormethyl | Ethyl | |
| 2.2.1615 | 3-Brom-6-propoxy-Phenyl | Fluormethyl | Ethyl | |
| 2.2.1616 | 3-Methyl-6-propoxy-Phenyl | Fluormethyl | Ethyl | |
| 2.2.1617 | 3-Cyclopropyl-6-propoxy-Phenyl | Fluormethyl | Ethyl | |
| 2.2.1618 | 3-Cyano-6-propoxy-Phenyl | Fluormethyl | Ethyl | |
| 2.2.1619 | 3-Trifluormethyl-6-propoxy-Phenyl | Fluormethyl | Ethyl | |
| 2.2.1620 | 3-Methoxy-6-propoxy-Phenyl | Fluormethyl | Ethyl | |
| 2.2.1621 | 3-Ethoxy-6-propoxy-Phenyl | Fluormethyl | Ethyl | |
| 2.2.1622 | 3-Trifluormethoxy-6-propoxy-Phenyl | Fluormethyl | Ethyl | |
| 2.2.1623 | 3-Nitro-6-propoxy-Phenyl | Fluormethyl | Ethyl | |
| 2.2.1624 | 3-Fluor-6-isopropoxy-Phenyl | Fluormethyl | Ethyl | |
| 2.2.1625 | 3-Chlor-6-isopropoxy-Phenyl | Fluormethyl | Ethyl | |
| 2.2.1626 | 3-Brom-6-isopropoxy-Phenyl | Fluormethyl | Ethyl | |
| 2.2.1627 | 3-Methyl-6-isopropoxy-Phenyl | Fluormethyl | Ethyl | |
| 2.2.1628 | 3-Cyclopropyl-6-isopropoxy-Phenyl | Fluormethyl | Ethyl | |
| 2.2.1629 | 3-Cyano-6-isopropoxy-Phenyl | Fluormethyl | Ethyl | |
| 2.2.1630 | 3-Trifluormethyl-6-isopropoxy-Phenyl | Fluormethyl | Ethyl | |
| 2.2.1631 | 3-Methoxy-6-isopropoxy-Phenyl | Fluormethyl | Ethyl | |
| 2.2.1632 | 3-Ethoxy-6-isopropoxy-Phenyl | Fluormethyl | Ethyl | |
| 2.2.1633 | 3-Trifluormethoxy-6-isopropoxy-Phenyl | Fluormethyl | Ethyl | |
| 2.2.1634 | 3-Nitro-6-isopropoxy-Phenyl | Fluormethyl | Ethyl | |
| 2.2.1635 | 3-Fluor-6-trifluormethoxy-Phenyl | Fluormethyl | Ethyl | |
| 2.2.1636 | 3-Chlor-6-trifluormethoxy-Phenyl | Fluormethyl | Ethyl | |
| 2.2.1637 | 3-Brom-6-trifluormethoxy-Phenyl | Fluormethyl | Ethyl | |
| 2.2.1638 | 3-Methyl-6-trifluormethoxy-Phenyl | Fluormethyl | Ethyl | |
| 2.2.1639 | 3-Cyclopropyl-6-trifluormethoxy-Phenyl | Fluormethyl | Ethyl | |
| 2.2.1640 | 3-Cyano-6-trifluormethoxy-Phenyl | Fluormethyl | Ethyl | |
| 2.2.1641 | 3-Trifluormethyl-6-trifluormethoxy-Phenyl | Fluormethyl | Ethyl | |
| 2.2.1642 | 3-Methoxy-6-trifluormethoxy-Phenyl | Fluormethyl | Ethyl | |
| 2.2.1643 | 3-Ethoxy-6-trifluormethoxy-Phenyl | Fluormethyl | Ethyl | |
| 2.2.1644 | 3,6-Bis(trifluormethoxy)-Phenyl | Fluormethyl | Ethyl | |
| 2.2.1645 | 3-Nitro-6-trifluormethoxy-Phenyl | Fluormethyl | Ethyl | |
| 2.2.1646 | 3-Fluor-6-difluormethoxy-Phenyl | Fluormethyl | Ethyl | |
| 2.2.1647 | 3-Chlor-6-difluormethoxy-Phenyl | Fluormethyl | Ethyl | |
| 2.2.1648 | 3-Brom-6-difluormethoxy-Phenyl | Fluormethyl | Ethyl | |
| 2.2.1649 | 3-Methyl-6-difluormethoxy-Phenyl | Fluormethyl | Ethyl | |
| 2.2.1650 | 3-Cyclopropyl-6-difluormethoxy-Phenyl | Fluormethyl | Ethyl | |
| 2.2.1651 | 3-Cyano-6-difluormethoxy-Phenyl | Fluormethyl | Ethyl | |
| 2.2.1652 | 3-Trifluormethyl-6-difluormethoxy-Phenyl | Fluormethyl | Ethyl | |
| 2.2.1653 | 3-Methoxy-6-difluormethoxy-Phenyl | Fluormethyl | Ethyl | |
| 2.2.1654 | 3-Ethoxy-6-difluormethoxy-Phenyl | Fluormethyl | Ethyl | |
| 2.2.1655 | 3-Trifluormethoxy-6-difluor methoxy-Phenyl | Fluormethyl | Ethyl | |
| 2.2.1656 | 3-Nitro-6-difluormethoxy-Phenyl | Fluormethyl | Ethyl | |
| 2.2.1657 | 3-Fluor-6-nitro-Phenyl | Fluormethyl | Ethyl | |
| 2.2.1658 | 3-Chlor-6-nitro-Phenyl | Fluormethyl | Ethyl | |
| 2.2.1659 | 3-Brom-6-nitro-Phenyl | Fluormethyl | Ethyl | |
| 2.2.1660 | 3-Methyl-6-nitro-Phenyl | Fluormethyl | Ethyl | |
| 2.2.1661 | 3-Cyclopropyl-6-nitro-Phenyl | Fluormethyl | Ethyl | |
| 2.2.1662 | 3-Cyano-6-nitro-Phenyl | Fluormethyl | Ethyl | |
| 2.2.1663 | 3-Trifluormethyl-6-nitro-Phenyl | Fluormethyl | Ethyl | |
| 2.2.1664 | 3-Methoxy-6-nitro-Phenyl | Fluormethyl | Ethyl | |
| 2.2.1665 | 3-Ethoxy-6-nitro-Phenyl | Fluormethyl | Ethyl | |
| 2.2.1666 | 3-Trifluormethoxy-6-nitro-Phenyl | Fluormethyl | Ethyl | |
| 2.2.1667 | 3-Fluor-6-methylsulfanylPhenyl | Fluormethyl | Ethyl | |
| 2.2.1668 | 3-Chlor-6-methylsulfanyl-Phenyl | Fluormethyl | Ethyl | |
| 2.2.1669 | 3-Brom-6-methylsulfanyl-Phenyl | Fluormethyl | Ethyl | |
| 2.2.1670 | 3-Methyl-6-methylsulfanyl-Phenyl | Fluormethyl | Ethyl | |
| 2.2.1671 | 3-Cyclopropyl-6-methylsulfanyl-Phenyl | Fluormethyl | Ethyl | |
| 2.2.1672 | 3-Cyano-6-methylsulfanyl-Phenyl | Fluormethyl | Ethyl | |
| 2.2.1673 | 3-Trifluormethyl-6-methylsulfanyl -Phenyl | Fluormethyl | Ethyl | |
| 2.2.1674 | 3-Methoxy-6-methylsulfanyl-Phenyl | Fluormethyl | Ethyl | |
| 2.2.1675 | 3-Ethoxy-6-methylsulfanyl-Phenyl | Fluormethyl | Ethyl | |
| 2.2.1676 | 3-Trifluormethoxy-6-methylsulfanyl-Phenyl | Fluormethyl | Ethyl | |
| 2.2.1677 | 3-Nitro-6-methylsulfanyl-Phenyl | Fluormethyl | Ethyl | |
| 2.2.1678 | 2,3,4-Trifluor-Phenyl | Fluormethyl | Ethyl | |
| 2.2.1679 | 2,3,4-Trichlor-Phenyl | Fluormethyl | Ethyl | |
| 2.2.1680 | 2,3.4-Trimethyl-Phenyl | Fluormethyl | Ethyl | |
| 2.2.1681 | 2-Fluor-2-chlor-5-trifluormethyl-Phenyl | Fluormethyl | Ethyl | |
| 2.2.1682 | 2,3,5-Trifluor-Phenyl | Fluormethyl | Ethyl | |
| 2.2.1683 | 2,3,5-Trichlor-Phenyl | Fluormethyl | Ethyl | |
| 2.2.1684 | 2,3,5-Trimethyl-Phenyl | Fluormethyl | Ethyl | |
| 2.2.1685 | 2,3-Dichlor-5-methoxy-Phenyl | Fluormethyl | Ethyl | |
| 2.2.1686 | 2,3,6-Trifluor-Phenyl | Fluormethyl | Ethyl | |
| 2.2.1687 | 2,3,6-Trichlor-Phenyl | Fluormethyl | Ethyl | |
| 2.2.1688 | 2,3,6-Trimethyl-Phenyl | Fluormethyl | Ethyl | |
| 2.2.1689 | 3,4,5-Trifluor-Phenyl | Fluormethyl | Ethyl | |
| 2.2.1690 | 3,4,5-Trichlor-Phenyl | Fluormethyl | Ethyl | |
| 2.2.1691 | 3,4,5-Trimethyl-Phenyl | Fluormethyl | Ethyl | |
| 2.2.1692 | 3,5-Dimethyl-4-fluor-Phenyl | Fluormethyl | Ethyl | |
| 2.2.1693 | 3,5-Dichlor-4-methoxy-Phenyl | Fluormethyl | Ethyl | |
| 2.2.1694 | 3,5-Difluor-4-chlor-Phenyl | Fluormethyl | Ethyl | |
| 2.2.1695 | 3,5-Dichlor-4-hydroxyPhenyl | Fluormethyl | Ethyl | |
| 2.2.1696 | 3,5-Trifluormethyl-4-chlor-Phenyl | Fluormethyl | Ethyl | |
| 2.2.1697 | 3,4,6-Trifluor-Phenyl | Fluormethyl | Ethyl | |
| 2.2.1698 | 3,4,6-Trichlor-Phenyl | Fluormethyl | Ethyl | |
| 2.2.1699 | 3,4,6-Trimethyl-Phenyl | Fluormethyl | Ethyl | |
| 2.2.1700 | Pentafluorphenyl | Fluormethyl | Ethyl | |

**Tabelle 2.3: Erfindungsgemäße Verbindungen der allgemeinen Formel (I), worin W* für COOY, R¹ und R² für Wasserstoff stehen, und Aryl den Rest**

| | | | | |
|---|---|---|---|---|
| 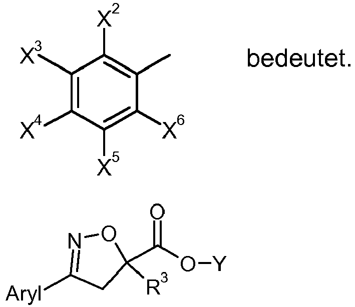 | | | | |

| Nr. | Aryl | R³ | Y | Physikalische Daten |
|---|---|---|---|---|
| 2.3.001 | 3-Fluor-Phenyl | Vinyl | Methyl | [CDCl₃] 3.37 (d, 1H); 3.84 (s,3H); 3.96 (d,1H); 5.37 (d,1H); 5.56 (d,1H); 6.15 (dd,1H); 7.13 (t,1H); 7.37-7.42 (m,3H). |
| 2.3.002 | 3-Fluor-Phenyl | 1-Methyl vinyl | Ethyl | |
| 2.3.003 | 3-Fluor-Phenyl | Allyl | Ethyl | |
| 2.3.004 | 3-Fluor-Phenyl | 1-Chlor vinyl | Methyl | [CDCl3] 3.57 (d, 1H); 3.88 (s,3H); 4.25 (d, 1H); 5.55 (s, 1H); 5.94 (s,1H); 7.15 (t, 1H); 7.39-7.43 (m,3H). |
| 2.3.005 | 3-Fluor-Phenyl | Ethinyl | Ethyl | |
| 2.3.006 | 3-Chlor-Phenyl | Vinyl | Ethyl | |
| 2.3.007 | 3-Chlor-Phenyl | 1-Methyl vinyl | Ethyl | |
| 2.3.008 | 3-Chlor-Phenyl | Allyl | Ethyl | |
| 2.3.009 | 3-Chlor-Phenyl | 1-Chlor vinyl | Ethyl | |
| 2.3.010 | 3-Chlor-Phenyl | Ethinyl | Ethyl | |
| 2.3.011 | 3-Brom-Phenyl | Vinyl | Ethyl | |
| 2.3.012 | 3-Brom-Phenyl | 1-Methyl vinyl | Ethyl | |
| 2.3.013 | 3-Iod-Phenyl | Vinyl | Ethyl | |
| 2.3.014 | 3-Iod-Phenyl | 1-Methyl vinyl | Ethyl | |
| 2.3.015 | 3-Methyl-Phenyl | Vinyl | Ethyl | |
| 2.3.016 | 3-Methyl-Phenyl | 1-Methyl vinyl | Ethyl | |
| 2.3.017 | 3-Ethyl-Phenyl | Vinyl | Ethyl | |
| 2.3.018 | 3-Propyl-Phenyl | Vinyl | Ethyl | |
| 2.3.019 | 3-isoPropyl-Phenyl | Vinyl | Ethyl | |
| 2.3.020 | 3-nButyl-Phenyl | Vinyl | Ethyl | |
| 2.3.021 | 3-iButyl-Phenyl | Vinyl | Ethyl | |
| 2.3.022 | 3-tert.Butyl-Phenyl | Vinyl | Ethyl | |
| 2.3.023 | 3-Cyclopropyl-Phenyl | Vinyl | Ethyl | |
| 2.3.024 | 3-Cyclobutyl-Phenyl | Vinyl | Ethyl | |
| 2.3.025 | 3-Cyclopentyl-Phenyl | Vinyl | Ethyl | |
| 2.3.026 | 3-Vinyl-Phenyl | Vinyl | Ethyl | |
| 2.3.027 | 3-Ethinyl-Phenyl | Vinyl | Ethyl | |
| 2.3.028 | 3-Cyano-Phenyl | Vinyl | Ethyl | |
| 2.3.029 | 3-Trifluormethyl-Phenyl | Vinyl | Ethyl | |
| 2.3.030 | 3-Difluormethyl-Phenyl | Vinyl | Ethyl | |
| 2.3.031 | 3-(Hydroxycarbonyl)-Phenyl | Vinyl | Ethyl | |
| 2.3.032 | 3-(Methoxycarbony)l-Phenyl | Vinyl | Ethyl | |
| 2.3.033 | 3-(Ethoxycarbonyl)-Phenyl | Vinyl | Ethyl | |
| 2.3.034 | 3-Hydroxymethyl-Phenyl | Vinyl | Ethyl | |
| 2.3.035 | 3-Carbamoyl-Phenyl | Vinyl | Ethyl | |
| 2.3.036 | 3-Hydroxy-Phenyl- | Vinyl | Ethyl | |
| 2.3.037 | 3-Methoxy-Phenyl | Vinyl | Ethyl | |
| 2.3.038 | 3-Ethoxy-Phenyl | Vinyl | Ethyl | |
| 2.3.039 | 3-Propyloxy-Phenyl | Vinyl | Ethyl | |
| 2.3.040 | 3-isoPropyloxy-Phenyl | Vinyl | Ethyl | |
| 2.3.041 | 3-nButyloxy-Phenyl | Vinyl | Ethyl | |
| 2.3.042 | 3-iButyloxy-Phenyl | Vinyl | Ethyl | |
| 2.3.043 | 3-tButyloxy-Phenyl | Vinyl | Ethyl | |
| 2.3.044 | 3-Difluormethoxy-Phenyl | Vinyl | Ethyl | |
| 2.3.045 | 3-Trifluormethoxy-Phenyl | Vinyl | Ethyl | |
| 2.3.046 | 3-(2,2,2-Trifluorethoxy)-Phenyl | Vinyl | Ethyl | |
| 2.3.047 | 3-(2-Chlorethoxy)-Phenyl | Vinyl | Ethyl | |
| 2.3.048 | 3-(2-Hydroxyethoxy)-Phenyl- | Vinyl | Ethyl | |
| 2.3.049 | 3-(2-Methoxyethoxy)-Phenyl- | Vinyl | Ethyl | |
| 2.3.050 | 3-[(tert-Butoxycarbonyl)oxy]-Phenyl | Vinyl | Ethyl | |
| 2.3.051 | 3-Nitro-Phenyl | Vinyl | Ethyl | |
| 2.3.052 | 3-Acetoxy-Phenyl | Vinyl | Ethyl | |
| 2.3.053 | {3-[(tert-Butoxycarbonyl)amino]-Phenyl}- | Vinyl | Ethyl | |
| 2.3.054 | 3-Methylsulfanyl-Phenyl | Vinyl | Ethyl | |
| 2.3.055 | 3-Ethylsulfanyl-Phenyl | Vinyl | Ethyl | |
| 2.3.056 | 3-(Pentafluor-lambda⁶-sulfanyl)-Phenyl | Vinyl | Ethyl | |
| 2.3.057 | 2,3-Difluor-Phenyl | Vinyl | Ethyl | |
| 2.3.058 | 2,3-Difluor-Phenyl | 1-Methyl vinyl | Ethyl | |
| 2.3.059 | 2,3-Difluor-Phenyl | Allyl | Ethyl | |
| 2.3.060 | 2,3-Difluor-Phenyl | 1-Chlor vinyl | Ethyl | |
| 2.3.061 | 2,3-Difluor-Phenyl | Ethinyl | Ethyl | |
| 2.3.062 | 2-Chlor-3-fluor-Phenyl | Vinyl | Ethyl | |
| 2.3.063 | 2-Brom-3-fluor-Phenyl | Vinyl | Ethyl | |
| 2.3.064 | 2-Methyl-3-fluor-Phenyl | Vinyl | Ethyl | |
| 2.3.065 | 2-Ethyl-3-fluor-Phenyl | Vinyl | Ethyl | |
| 2.3.066 | 2-Cyclopropyl-3-fluor-Phenyl | Vinyl | Ethyl | |
| 2.3.067 | 2-Vinyl-3-fluor-Phenyl | Vinyl | Ethyl | |
| 2.3.068 | 2-Ethinyl-3-fluor-Phenyl | Vinyl | Ethyl | |
| 2.3.069 | 2-Cyano-3-fluor-Phenyl | Vinyl | Ethyl | |
| 2.3.070 | 2-Methoxy-3-fluor-Phenyl | Vinyl | Ethyl | |
| 2.3.071 | 2-Ethoxy-3-fluor-Phenyl | Vinyl | Ethyl | |
| 2.3.072 | 2-Trifluormethoxy-3-fluor-Phenyl | Vinyl | Ethyl | |
| 2.3.073 | 2-Nitro-3-fluor-Phenyl | Vinyl | Ethyl | |
| 2.3.074 | 2-Fluor-3-chlor-Phenyl | Vinyl | Ethyl | |
| 2.3.075 | 2,3-Dichlor-Phenyl | Vinyl | Ethyl | |
| 2.3.076 | 2,3-Dichlor-Phenyl | 1-Methyl vinyl | Ethyl | |
| 2.3.077 | 2,3-Dichlor-Phenyl | Allyl | Ethyl | |
| 2.3.078 | 2,3-Dichlor-Phenyl | 1-Chlor vinyl | Ethyl | |
| 2.3.079 | 2,3-Dichlor-Phenyl | Ethinyl | Ethyl | |
| 2.3.080 | 2-Brom-3-chlor-Phenyl | Vinyl | Ethyl | |
| 2.3.081 | 2-Methyl-3-chlor-Phenyl | Vinyl | Ethyl | |
| 2.3.082 | 2-Ethyl-3-chlor-Phenyl | Vinyl | Ethyl | |
| 2.3.083 | 2-Cyclopropyl-3-chlor-Phenyl | Vinyl | Ethyl | |
| 2.3.084 | 2-Vinyl-3-chlor-Phenyl | Vinyl | Ethyl | |
| 2.3.085 | 2-Ethinyl-3-chlor-Phenyl | Vinyl | Ethyl | |
| 2.3.086 | 2-Cyano-3-chlor-Phenyl | Vinyl | Ethyl | |
| 2.3.087 | 2-Trifluormethyl-2-chlor-Phenyl | Vinyl | Ethyl | |
| 2.3.088 | 2-Methoxy-3-chlor-Phenyl | Vinyl | Ethyl | |
| 2.3.089 | 2-Ethoxy-3-chlor-Phenyl | Vinyl | Ethyl | |
| 2.3.090 | 2-Trifluormethoxy-3-chlor-Phenyl | Vinyl | Ethyl | |
| 2.3.091 | 2-Nitro-3-chlor-Phenyl | Vinyl | Ethyl | |
| 2.3.092 | 2-Fluor-3-brom-Phenyl | Vinyl | Ethyl | |
| 2.3.093 | 2-Chlor-3-brom-Phenyl | Vinyl | Ethyl | |
| 2.3.094 | 2,3-Dibrom-Phenyl | Vinyl | Ethyl | |
| 2.3.095 | 2-Methyl-3-brom-Phenyl | Vinyl | Ethyl | |
| 2.3.096 | 2-Ethyl-3-brom-Phenyl | Vinyl | Ethyl | |
| 2.3.097 | 2-Cyclopropyl-3-brom-Phenyl | Vinyl | Ethyl | |
| 2.3.098 | 2-Vinyl-3-brom-Phenyl | Vinyl | Ethyl | |
| 2.3.099 | 2-Ethinyl-3-brom-Phenyl | Vinyl | Ethyl | |
| 2.3.100 | 2-Cyano-3-brom-Phenyl | Vinyl | Ethyl | |
| 2.3.101 | 2-Trifluormethyl-3-brom-Phenyl | Vinyl | Ethyl | |
| 2.3.102 | 2-Methoxy-3-Phenyl | Vinyl | Ethyl | |
| 2.3.103 | 2-Ethoxy-3-brom-Phenyl | Vinyl | Ethyl | |
| 2.3.104 | 2-Trifluormethoxy-3-brom-Phenyl | Vinyl | Ethyl | |
| 2.3.105 | 2-Nitro-3-brom-Phenyl | Vinyl | Ethyl | |
| 2.3.106 | 2-Fluor-3-iod-Phenyl | Vinyl | Ethyl | |
| 2.3.107 | 2-Chlor-3-iod-Phenyl | Vinyl | Ethyl | |
| 2.3.108 | 2-Brom-3-iod-Phenyl | Vinyl | Ethyl | |
| 2.3.109 | 2-Methyl-3-iod-Phenyl | Vinyl | Ethyl | |
| 2.3.110 | 2-Ethyl-3-iod-Phenyl | Vinyl | Ethyl | |
| 2.3.111 | 2-Cyclopropyl-3-iod-Phenyl | Vinyl | Ethyl | |
| 2.3.112 | 2-Vinyl-3-iod-Phenyl | Vinyl | Ethyl | |
| 2.3.113 | 2-Ethinyl-3-iod-Phenyl | Vinyl | Ethyl | |
| 2.3.114 | 2-Cyano-3-iod-Phenyl | Vinyl | Ethyl | |
| 2.3.115 | 2-Trifluormethyl-3-iod-Phenyl | Vinyl | Ethyl | |
| 2.3.116 | 2-Methoxy-3-iod-Phenyl | Vinyl | Ethyl | |
| 2.3.117 | 2-Ethoxy-3-iod-Phenyl | Vinyl | Ethyl | |
| 2.3.118 | 2-Trifluormethoxy-3-iod-Phenyl | Vinyl | Ethyl | |
| 2.3.119 | 2-Nitro-3-iod-Phenyl | Vinyl | Ethyl | |
| 2.3.120 | 2-Fluor-3-methyl-Phenyl | Vinyl | Ethyl | |
| 2.3.121 | 2-Fluor-3-methyl-Phenyl | 1-Methyl vinyl | Ethyl | |
| 2.3.122 | 2-Fluor-3-methyl-Phenyl | Allyl | Ethyl | |
| 2.3.123 | 2-Fluor-3-methyl-Phenyl | 1-Chlor vinyl | Ethyl | |
| 2.3.124 | 2-Fluor-3-methyl-Phenyl | Ethinyl | Ethyl | |
| 2.3.125 | 2-Chlor-3-methyl-Phenyl | Vinyl | Ethyl | |
| 2.3.126 | 2-Chlor-3-methyl-Phenyl | 1-Methyl vinyl | Ethyl | |
| 2.3.127 | 2-Chlor-3-methyl-Phenyl | Allyl | Ethyl | |
| 2.3.128 | 2-Chlor-3-methyl-Phenyl | 1-Chlor vinyl | Ethyl | |
| 2.3.129 | 2-Chlor-3-methyl-Phenyl | Ethinyl | Ethyl | |
| 2.3.130 | 2-Brom-3-methyl-Phenyl | Vinyl | Ethyl | |
| 2.3.131 | 2,3-Dimethyl-Phenyl | Vinyl | Ethyl | |
| 2.3.132 | 2,3-Dimethyl-Phenyl | 1-Methyl vinyl | Ethyl | |
| 2.3.133 | 2,3-Dimethyl-Phenyl | Allyl | Ethyl | |
| 2.3.134 | 2,3-Dimethyl-Phenyl | 1-Chlor vinyl | Ethyl | |
| 2.3.135 | 2,3-Dimethyl-Phenyl | Ethinyl | Ethyl | |
| 2.3.136 | 2-Ethyl-3-methyl-Phenyl | Vinyl | Ethyl | |
| 2.3.137 | 2-Cyclopropyl-3-methyl-Phenyl | Vinyl | Ethyl | |
| 2.3.138 | 2-Vinyl-3-methyl-Phenyl | Vinyl | Ethyl | |
| 2.3.139 | 2-Ethinyl-3-methyl-Phenyl | Vinyl | Ethyl | |
| 2.3.140 | 2-Cyano-3-methyl-Phenyl | Vinyl | Ethyl | |
| 2.3.141 | 2-Trifluormethyl-3-methyl-Phenyl | Vinyl | Ethyl | |
| 2.3.142 | 2-Methoxy-3-methyl-Phenyl | Vinyl | Ethyl | |
| 2.3.143 | 2-Ethoxy-3-methyl-Phenyl | Vinyl | Ethyl | |
| 2.3.144 | 2-Trifluormethoxy-3-methyl-Phenyl | Vinyl | Ethyl | |
| 2.3.145 | 2-Nitro-3-methyl-Phenyl | Vinyl | Ethyl | |
| 2.3.146 | 2-Fluor-3-ethyl-Phenyl | Vinyl | Ethyl | |
| 2.3.147 | 2-Chlor-3-ethyl-Phenyl | Vinyl | Ethyl | |
| 2.3.148 | 2-Brom-3-ethyl-Phenyl | Vinyl | Ethyl | |
| 2.3.149 | 2-Methyl-3-ethyl-Phenyl | Vinyl | Ethyl | |
| 2.3.150 | 2,3-Diethyl-Phenyl | Vinyl | Ethyl | |
| 2.3.151 | 2-Cyclopropyl-3-ethyl-Phenyl | Vinyl | Ethyl | |
| 2.3.152 | 2-Vinyl-3-ethyl-Phenyl | Vinyl | Ethyl | |
| 2.3.153 | 2-Ethinyl-3-ethyl-Phenyl | Vinyl | Ethyl | |
| 2.3.154 | 2-Cyano-3-ethyl-Phenyl | Vinyl | Ethyl | |
| 2.3.155 | 2-Trifluormethyl-3-ethyl-Phenyl | Vinyl | Ethyl | |
| 2.3.156 | 2-Methoxy-3-ethyl-Phenyl | Vinyl | Ethyl | |
| 2.3.157 | 2-Ethoxy-3-ethyl-Phenyl | Vinyl | Ethyl | |
| 2.3.158 | 2-Trifluormethoxy-3-ethyl-Phenyl | Vinyl | Ethyl | |
| 2.3.159 | 2-Nitro-3-ethyl-Phenyl | Vinyl | Ethyl | |
| 2.3.160 | 2-Fluor-3-propyl-Phenyl | Vinyl | Ethyl | |
| 2.3.161 | 2-Chlor-3-propyl-Phenyl | Vinyl | Ethyl | |
| 2.3.162 | 2-Brom-3-propyl-Phenyl | Vinyl | Ethyl | |
| 2.3.163 | 2-Methyl-3-propyl-Phenyl | Vinyl | Ethyl | |
| 2.3.164 | 2-Methyl-3-propyl-Phenyl | Vinyl | Ethyl | |
| 2.3.165 | 2-Cyclopropyl-3-propyl-Phenyl | Vinyl | Ethyl | |
| 2.3.166 | 2-Vinyl-3-propyl-Phenyl | Vinyl | Ethyl | |
| 2.3.167 | 2-Ethinyl-3propyl-Phenyl | Vinyl | Ethyl | |
| 2.3.168 | 2-Cyano-3-propyl-Phenyl | Vinyl | Ethyl | |
| 2.3.169 | 2-Trifluormethyl-3-propyl-Phenyl | Vinyl | Ethyl | |
| 2.3.170 | 2-Methoxy-3-propyl-Phenyl | Vinyl | Ethyl | |
| 2.3.171 | 2-Ethoxy-3-propyl-Phenyl | Vinyl | Ethyl | |
| 2.3.172 | 2-Trifluormethoxy-3-propyl-Phenyl | Vinyl | Ethyl | |
| 2.3.173 | 2-Nitro-3-propyl-Phenyl | Vinyl | Ethyl | |
| 2.3.174 | 2-Fluor-3-isopropyl-Phenyl | Vinyl | Ethyl | |
| 2.3.175 | 2-Chlor-3-isopropyl-Phenyl | Vinyl | Ethyl | |
| 2.3.176 | 2-Brom-3-isopropyl-Phenyl | Vinyl | Ethyl | |
| 2.3.177 | 2-Methyl-3-isopropyl-Phenyl | Vinyl | Ethyl | |
| 2.3.178 | 2-Ethyl-3-isopropyl-Phenyl | Vinyl | Ethyl | |
| 2.3.179 | 2-Cyclopropyl-3-isopropyl-Phenyl | Vinyl | Ethyl | |
| 2.3.180 | 2-Vinyl-3-isopropyl-Phenyl | Vinyl | Ethyl | |
| 2.3.181 | 2-Ethinyl-3-isopropyl-Phenyl | Vinyl | Ethyl | |
| 2.3.182 | 2-Cyano-3-isopropyl-Phenyl | Vinyl | Ethyl | |
| 2.3.183 | 2-Trifluormethyl-3-isopropyl-Phenyl | Vinyl | Ethyl | |
| 2.3.184 | 2-Methoxy-3-isopropyl-Phenyl | Vinyl | Ethyl | |
| 2.3.185 | 2-Ethoxy-3-isopropyl-Phenyl | Vinyl | Ethyl | |
| 2.3.186 | 2-Trifluormethoxy-3-isopropyl-Phenyl | Vinyl | Ethyl | |
| 2.3.187 | 2-Nitro-3-isopropyl-Phenyl | Vinyl | Ethyl | |
| 2.3.188 | 2-Fluor-3-tert.butyl-Phenyl | Vinyl | Ethyl | |
| 2.3.189 | 2-Chlor-3-tert.buty-Phenyl | Vinyl | Ethyl | |
| 2.3.190 | 2-Brom-3-tert.butyl-Phenyl | Vinyl | Ethyl | |
| 2.3.191 | 2-Methyl-3-tert.butyl-Phenyl | Vinyl | Ethyl | |
| 2.3.192 | 2-Ethyl-3-tert.butyl-Phenyl | Vinyl | Ethyl | |
| 2.3.193 | 2-Cyclopropyl-3-tert.butyl-Phenyl | Vinyl | Ethyl | |
| 2.3.194 | 2-Vinyl-3-tert.butyl-Phenyl | Vinyl | Ethyl | |
| 2.3.195 | 2-Ethinyl-3-tert.butyl-Phenyl | Vinyl | Ethyl | |
| 2.3.196 | 2-Cyano-3-tert.butyl-Phenyl | Vinyl | Ethyl | |
| 2.3.197 | 2-Trifluormethyl-3-tert.butyl-Phenyl | Vinyl | Ethyl | |
| 2.3.198 | 2-Methoxy-3-tert.butyl-Phenyl | Vinyl | Ethyl | |
| 2.3.199 | 2-Ethoxy-3-tert.butyl-Phenyl | Vinyl | Ethyl | |
| 2.3.200 | 2-Trifluormethoxy-3-tert.butyl-Phenyl | Vinyl | Ethyl | |
| 2.3.201 | 2-Nitro-3-tert.butyl-Phenyl | Vinyl | Ethyl | |
| 2.3.202 | 2-Fluor-3-hydroxymethyl-Phenyl | Vinyl | Ethyl | |
| 2.3.203 | 2-Chlor-3-hydroxymethyl-Phenyl | Vinyl | Ethyl | |
| 2.3.204 | 2-Brom-3-hydroxymethyl - Phenyl | Vinyl | Ethyl | |
| 2.3.205 | 2-Methyl-3-hydroxymethyl-Phenyl | Vinyl | Ethyl | |
| 2.3.206 | 2-Ethyl-3-hydroxymethyl-Phenyl | Vinyl | Ethyl | |
| 2.3.207 | 2-Cyclopropyl-3-hydroxymethyl-Phenyl | Vinyl | Ethyl | |
| 2.3.208 | 2-Vinyl-3-hydroxymethyl-Phenyl | Vinyl | Ethyl | |
| 2.3.209 | 2-Ethinyl-3-hydroxymethyl-Phenyl | Vinyl | Ethyl | |
| 2.3.210 | 2-Cyano-3-hydroxymethyl - Phenyl | Vinyl | Ethyl | |
| 2.3.211 | 2-Trifluormethyl-3-hydroxymethyl-Phenyl | Vinyl | Ethyl | |
| 2.3.212 | 2-Methoxy-3-hydroxymethyl - Phenyl | Vinyl | Ethyl | |
| 2.3.213 | 2-Ethoxy-3-hydroxymethyl-Phenyl | Vinyl | Ethyl | |
| 2.3.214 | 2-Trifluormethoxy-3-hydroxymethyl--Phenyl | Vinyl | Ethyl | |
| 2.3.215 | 2-Nitro-3-hydroxymethyl-Phenyl | Vinyl | Ethyl | |
| 2.3.216 | 2-Fluor-3-cyclopropyl-Phenyl | Vinyl | Ethyl | |
| 2.3.217 | 2-Chlor-3-cyclopropyl-Phenyl | Vinyl | Ethyl | |
| 2.3.218 | 2-Brom-3-cyclopropyl-Phenyl | Vinyl | Ethyl | |
| 2.3.219 | 2-Methyl-3-cyclopropyl-Phenyl | Vinyl | Ethyl | |
| 2.3.220 | 2-Ethyl-3-cyclopropyl-Phenyl | Vinyl | Ethyl | |
| 2.3.221 | 2-Cyclopropyl-3-cyclopropyl-Phenyl | Vinyl | Ethyl | |
| 2.3.222 | 2-Vinyl-3-cyclopropyl-Phenyl | Vinyl | Ethyl | |
| 2.3.223 | 2-Ethinyl-3-cyclopropyl-Phenyl | Vinyl | Ethyl | |
| 2.3.224 | 2-Cyano-3-cyclopropyl-Phenyl | Vinyl | Ethyl | |
| 2.3.225 | 2-Trifluormethyl-3-cyclopropyl-Phenyl | Vinyl | Ethyl | |
| 2.3.226 | 2-Methoxy-3-cyclopropyl-Phenyl | Vinyl | Ethyl | |
| 2.3.227 | 2-Ethoxy-3-cyclopropyl-Phenyl | Vinyl | Ethyl | |
| 2.3.228 | 2-Trifluormethoxy-3-cyclopropyl-Phenyl | Vinyl | Ethyl | |
| 2.3.229 | 2-Fluor-3-methoxycarbonyl-Phenyl | Vinyl | Ethyl | |
| 2.3.230 | 2-Chlor-3-methoxycarbonyl-Phenyl | Vinyl | Ethyl | |
| 2.3.231 | 2-Brom-3-methoxycarbonyl-Phenyl | Vinyl | Ethyl | |
| 2.3.232 | 2-Methyl-3-methoxycarbonyl-Phenyl | Vinyl | Ethyl | |
| 2.3.233 | 2-Ethyl-3-methoxycarbonyl-Phenyl | Vinyl | Ethyl | |
| 2.3.234 | 2-Cyclopropyl-3-methoxycarbonyl-Phenyl | Vinyl | Ethyl | |
| 2.3.235 | 2-Vinyl-3-methoxycarbonyl-Phenyl | Vinyl | Ethyl | |
| 2.3.236 | 2-Ethinyl-3-methoxycarbonyl-Phenyl | Vinyl | Ethyl | |
| 2.3.237 | 2-Cyano-3-methoxycarbonyl-Phenyl | Vinyl | Ethyl | |
| 2.3.238 | 2-Trifluormethyl-3-methoxycarbonyl-Phenyl | Vinyl | Ethyl | |
| 2.3.239 | 2-Methoxy-3-methoxycarbonyl-Phenyl | Vinyl | Ethyl | |
| 2.3.240 | 2-Ethoxy-3-methoxycarbonyl-Phenyl | Vinyl | Ethyl | |
| 2.3.241 | 2-Trifluormethoxy-3-methoxycarbonyl-Phenyl | Vinyl | Ethyl | |
| 2.3.242 | 2-Nitro-3-methoxycarbonyl-Phenyl | Vinyl | Ethyl | |
| 2.3.243 | 2-Fluor-3-vinyl-Phenyl | Vinyl | Ethyl | |
| 2.3.244 | 2-Chlor-3-vinyl-Phenyl | Vinyl | Ethyl | |
| 2.3.245 | 2-Brom-3-vinyl-Phenyl | Vinyl | Ethyl | |
| 2.3.246 | 2-Methyl-3-vinyl-Phenyl | Vinyl | Ethyl | |
| 2.3.247 | 2-Ethyl-3-vinyl-Phenyl | Vinyl | Ethyl | |
| 2.3.248 | 2-Cyclopropyl-3-vinyl-Phenyl | Vinyl | Ethyl | |
| 2.3.249 | 2-Vinyl-3-vinyl-Phenyl | Vinyl | Ethyl | |
| 2.3.250 | 2-Ethinyl-3-vinyl-Phenyl | Vinyl | Ethyl | |
| 2.3.251 | 2-Cyano-3-vinyl-Phenyl | Vinyl | Ethyl | |
| 2.3.252 | 2-Trifluormethyl-3-vinyl-Phenyl | Vinyl | Ethyl | |
| 2.3.253 | 2-Methoxy-3-vinyl-Phenyl | Vinyl | Ethyl | |
| 2.3.254 | 2-Ethoxy-3-vinyl-Phenyl | Vinyl | Ethyl | |
| 2.3.255 | 2-Trifluormethoxy-3-vinyl-Phenyl | Vinyl | Ethyl | |
| 2.3.256 | 2-Nitro-3-vinyl-Phenyl | Vinyl | Ethyl | |
| 2.3.257 | 2-Fluor-3-ethinyl-Phenyl | Vinyl | Ethyl | |
| 2.3.258 | 2-Chlor-3-ethinyl-Phenyl | Vinyl | Ethyl | |
| 2.3.259 | 2-Brom-3-ethinyl-Phenyl | Vinyl | Ethyl | |
| 2.3.260 | 2-Methyl-3-ethinyl-Phenyl | Vinyl | Ethyl | |
| 2.3.261 | 2-Ethyl-3-ethinyl -Phenyl | Vinyl | Ethyl | |
| 2.3.262 | 2-Cyclopropyl-3-ethinyl-Phenyl | Vinyl | Ethyl | |
| 2.3.263 | 2-Vinyl-3-ethinyl-Phenyl | Vinyl | Ethyl | |
| 2.3.264 | 2-Cyano-3-ethinyl-Phenyl | Vinyl | Ethyl | |
| 2.3.265 | 2-Trifluormethyl-3-ethinyl-Phenyl | Vinyl | Ethyl | |
| 2.3.266 | 2-Methoxy-3-ethinyl-Phenyl | Vinyl | Ethyl | |
| 2.3.267 | 2-Ethoxy-3-ethinyl-Phenyl | Vinyl | Ethyl | |
| 2.3.268 | 2-Trifluormethoxy-3-ethinyl-Phenyl | Vinyl | Ethyl | |
| 2.3.269 | 2-Nitro-3-ethinyl-Phenyl | Vinyl | Ethyl | |
| 2.3.270 | 2-Fluor-3-ethinyl-Phenyl | Vinyl | Ethyl | |
| 2.3.271 | 2-Fluor-3-cyano-Phenyl | Vinyl | Ethyl | |
| 2.3.272 | 2-Chlor-3-cyano-Phenyl | Vinyl | Ethyl | |
| 2.3.273 | 2-Brom-3-cyano-Phenyl | Vinyl | Ethyl | |
| 2.3.274 | 2-Methyl-3-cyano-Phenyl | Vinyl | Ethyl | |
| 2.3.275 | 2-Ethyl-3-cyano-Phenyl | Vinyl | Ethyl | |
| 2.3.276 | 2-Ethyl-3-cyano-Phenyl | 1-Methylvinyl | Ethyl | |
| 2.3.277 | 2-Ethyl-3-cyano-Phenyl | Allyl | Ethyl | |
| 2.3.278 | 2-Ethyl-3-cyano-Phenyl | 1-Chlorvinyl | Ethyl | |
| 2.3.279 | 2-Ethyl-3-cyano-Phenyl | Ethinyl | Ethyl | |
| 2.3.280 | 2-Cyclopropyl-3-cyano-Phenyl | Vinyl | Ethyl | |
| 2.3.281 | 2-Vinyl-3-cyano-Phenyl | Vinyl | Ethyl | |
| 2.3.282 | 2-Ethinyl-3-cyano-Phenyl | Vinyl | Ethyl | |
| 2.3.283 | 2-Cyano-3-cyanoPhenyl | Vinyl | Ethyl | |
| 2.3.284 | 2-Trifluormethyl-3-cyano-Phenyl | Vinyl | Ethyl | |
| 2.3.285 | 2-Methoxy-3-cyano-Phenyl | Vinyl | Ethyl | |
| 2.3.286 | 2-Ethoxy-3-cyano-Phenyl | Vinyl | Ethyl | |
| 2.3.287 | 2-Trifluormethoxy-3-cyano-Phenyl | Vinyl | Ethyl | |
| 2.3.288 | 2-Nitro-3-cyano-Phenyl | Vinyl | Ethyl | |
| 2.3.289 | 2-Fluor-3-hydroxy-Phenyl | Vinyl | Ethyl | |
| 2.3.290 | 2-Chlor-3-hydroxy-Phenyl | Vinyl | Ethyl | |
| 2.3.291 | 2-Brom-3-hydroxy-Phenyl | Vinyl | Ethyl | |
| 2.3.292 | 2-Methyl-3-hydroxy-Phenyl | Vinyl | Ethyl | |
| 2.3.293 | 2-Ethyl-3-hydroxy-Phenyl | Vinyl | Ethyl | |
| 2.3.294 | 2-Cyclopropyl-3-hydroxy-Phenyl | Vinyl | Ethyl | |
| 2.3.295 | 2-Vinyl-3-hydroxy-Phenyl | Vinyl | Ethyl | |
| 2.3.296 | 2-Ethinyl-3-hydroxy-Phenyl | Vinyl | Ethyl | |
| 2.3.297 | 2-Cyano-3-hyd roxy-Phenyl | Vinyl | Ethyl | |
| 2.3.298 | 2-Trifluormethyl-3-hydroxy-Phenyl | Vinyl | Ethyl | |
| 2.3.299 | 2-Methoxy-3-hydroxy-Phenyl | Vinyl | Ethyl | |
| 2.3.300 | 2-Ethoxy-3-hydroxy-Phenyl | Vinyl | Ethyl | |
| 2.3.301 | 2-Trifluormethoxy-3-hydroxy-Phenyl | Vinyl | Ethyl | |
| 2.3.302 | 2-Nitro-3-hydroxy-Phenyl | Vinyl | Ethyl | |
| 2.3.303 | 2-Fluor-3-methoxy-Phenyl | Vinyl | Ethyl | |
| 2.3.304 | 2-Chlor-3-methoxy-Phenyl | Vinyl | Ethyl | |
| 2.3.305 | 2-Brom-3-methoxy-Phenyl | Vinyl | Ethyl | |
| 2.3.306 | 2-Methyl-3-methoxy-Phenyl | Vinyl | Ethyl | |
| 2.3.307 | 2-Ethyl-3-methoxy-Phenyl | Vinyl | Ethyl | |
| 2.3.308 | 2-Cyclopropyl-3-methoxy-Phenyl | Vinyl | Ethyl | |
| 2.3.309 | 2-Vinyl-3-methoxy-Phenyl | Vinyl | Ethyl | |
| 2.3.310 | 2-Ethinyl-3-methoxy-Phenyl | Vinyl | Ethyl | |
| 2.3.311 | 2-Cyano-3-methoxy-Phenyl | Vinyl | Ethyl | |
| 2.3.312 | 2-Trifluormethyl-3-methoxy-Phenyl | Vinyl | Ethyl | |
| 2.3.313 | 2,3-Dimethoxy-Phenyl | Vinyl | Ethyl | |
| 2.3.314 | 2-Ethoxy-3-methoxy-Phenyl | Vinyl | Ethyl | |
| 2.3.315 | 2-Trifluormethoxy-3-methoxy-Phenyl | Vinyl | Ethyl | |
| 2.3.316 | 2-Nitro-3-methoxy-Phenyl | Vinyl | Ethyl | |
| 2.3.317 | 2-Fluor-3-ethoxy-Phenyl | Vinyl | Ethyl | |
| 2.3.318 | 2-Chlor-3-ethoxy-Phenyl | Vinyl | Ethyl | |
| 2.3.319 | 2-Brom-3-ethoxy-Phenyl | Vinyl | Ethyl | |
| 2.3.320 | 2-Methyl-3-ethoxy-Phenyl | Vinyl | Ethyl | |
| 2.3.321 | 2-Ethyl-3-ethoxy-Phenyl | Vinyl | Ethyl | |
| 2.3.322 | 2-Cyclopropyl-3-ethoxy-Phenyl | Vinyl | Ethyl | |
| 2.3.323 | 2-Vinyl-3-ethoxy-Phenyl | Vinyl | Ethyl | |
| 2.3.324 | 2-Ethinyl-3-ethoxy-Phenyl | Vinyl | Ethyl | |
| 2.3.325 | 2-Cyano-3-ethoxy-Phenyl | Vinyl | Ethyl | |
| 2.3.326 | 2-Trifluormethyl-3-ethoxy-Phenyl | Vinyl | Ethyl | |
| 2.3.327 | 2-Methoxy-3-ethoxy-Phenyl | Vinyl | Ethyl | |
| 2.3.328 | 2,3-Diethoxy-Phenyl | Vinyl | Ethyl | |
| 2.3.329 | 2-Trifluormethoxy-3-ethoxy-Phenyl | Vinyl | Ethyl | |
| 2.3.330 | 2-Nitro-3-ethoxy-Phenyl | Vinyl | Ethyl | |
| 2.3.331 | 2-Fluor-3-propoxy-Phenyl | Vinyl | Ethyl | |
| 2.3.332 | 2-Chlor-3-propoxy-Phenyl | Vinyl | Ethyl | |
| 2.3.333 | 2-Brom-3-propoxy-Phenyl | Vinyl | Ethyl | |
| 2.3.334 | 2-Methyl-3-propoxy-Phenyl | Vinyl | Ethyl | |
| 2.3.335 | 2-Ethyl-3-propoxy-Phenyl | Vinyl | Ethyl | |
| 2.3.336 | 2-Cyclopropyl-3-propoxy-Phenyl | Vinyl | Ethyl | |
| 2.3.337 | 2-Vinyl-3-propoxy-Phenyl | Vinyl | Ethyl | |
| 2.3.338 | 2-Ethinyl-3-propoxy-Phenyl | Vinyl | Ethyl | |
| 2.3.339 | 2-Cyano-3-propoxy-Phenyl | Vinyl | Ethyl | |
| 2.3.340 | 2-Trifluormethyl-3-propoxy-Phenyl | Vinyl | Ethyl | |
| 2.3.341 | 2-Methoxy-3-propoxy -Phenyl | Vinyl | Ethyl | |
| 2.3.342 | 2-Ethoxy-3-propoxy-Phenyl | Vinyl | Ethyl | |
| 2.3.343 | 2-Trifluormethoxy-3-propoxy-Phenyl | Vinyl | Ethyl | |
| 2.3.344 | 2-Nitro-3-propoxy-Phenyl | Vinyl | Ethyl | |
| 2.3.345 | 2-Fluor-3-isopropoxy-Phenyl | Vinyl | Ethyl | |
| 2.3.346 | 2-Chlor-3-isopropoxy-Phenyl | Vinyl | Ethyl | |
| 2.3.347 | 2-Brom-3-isopropoxy-Phenyl | Vinyl | Ethyl | |
| 2.3.348 | 2-Methyl-3-isopropoxy-Phenyl | Vinyl | Ethyl | |
| 2.3.349 | 2-Ethyl-3-isopropoxy-Phenyl | Vinyl | Ethyl | |
| 2.3.350 | 2-Cyclopropyl-3-isopropoxy-Phenyl | Vinyl | Ethyl | |
| 2.3.351 | 2-Vinyl-3-isopropoxy-Phenyl | Vinyl | Ethyl | |
| 2.3.352 | 2-Ethinyl-3-isopropoxy-Phenyl | Vinyl | Ethyl | |
| 2.3.353 | 2-Cyano-3-isopropoxy-Phenyl | Vinyl | Ethyl | |
| 2.3.354 | 2-Trifluormethyl-3-isopropoxy-Phenyl | Vinyl | Ethyl | |
| 2.3.355 | 2-Methoxy-3-isopropoxy-Phenyl | Vinyl | Ethyl | |
| 2.3.356 | 2-Ethoxy-3-isopropoxy-Phenyl | Vinyl | Ethyl | |
| 2.3.357 | 2-Trifluormethoxy-3-isopropoxy-Phenyl | Vinyl | Ethyl | |
| 2.3.358 | 2-Nitro-3-isopropoxy-Phenyl | Vinyl | Ethyl | |
| 2.3.359 | 2-Fluor-3-tert.butoxy-Phenyl | Vinyl | Ethyl | |
| 2.3.360 | 2-Chlor-3-tert.butoxy-Phenyl | Vinyl | Ethyl | |
| 2.3.361 | 2-Brom-3-tert.butoxy-Phenyl | Vinyl | Ethyl | |
| 2.3.362 | 2-Methyl-3-tert.butoxy-Phenyl | Vinyl | Ethyl | |
| 2.3.363 | 2-Ethyl-3-tert.butoxy-Phenyl | Vinyl | Ethyl | |
| 2.3.364 | 2-Cyclopropyl-3-tert.butoxy-Phenyl | Vinyl | Ethyl | |
| 2.3.365 | 2-Vinyl-3-tert.butoxy-Phenyl | Vinyl | Ethyl | |
| 2.3.366 | 2-Ethinyl-3-tert.butoxy-Phenyl | Vinyl | Ethyl | |
| 2.3.367 | 2-Cyano-3-tert.butoxy-Phenyl | Vinyl | Ethyl | |
| 2.3.368 | 2-Trifluormethyl-3-tert.butoxy-Phenyl | Vinyl | Ethyl | |
| 2.3.369 | 2-Methoxy-3-tert.butoxy-Phenyl | Vinyl | Ethyl | |
| 2.3.370 | 2-Ethoxy-3-tert.butoxy-Phenyl | Vinyl | Ethyl | |
| 2.3.371 | 2-Trifluormethoxy-3-tert.butoxy-Phenyl | Vinyl | Ethyl | |
| 2.3.372 | 2-Nitro-3-tert.butoxy-Phenyl | Vinyl | Ethyl | |
| 2.3.373 | 2-Fluor-3-trifluormethoxy-Phenyl | Vinyl | Ethyl | |
| 2.3.374 | 2-Chlor-3-trifluormethoxy-Phenyl | Vinyl | Ethyl | |
| 2.3.375 | 2-Brom-3-trifluormethoxy-Phenyl | Vinyl | Ethyl | |
| 2.3.376 | 2-Methyl-3-trifluormethoxy-Phenyl | Vinyl | Ethyl | |
| 2.3.377 | 2-Ethyl-3-trifluormethoxy-Phenyl | Vinyl | Ethyl | |
| 2.3.378 | 2-Cyclopropyl-3-trifluormethoxy-Phenyl | Vinyl | Ethyl | |
| 2.3.379 | 2-Vinyl-3-trifluormethoxy-Phenyl | Vinyl | Ethyl | |
| 2.3.380 | 2-Ethinyl-3-trifluormethoxy-Phenyl | Vinyl | Ethyl | |
| 2.3.381 | 2-Cyano-3-trifluormethoxy-Phenyl | Vinyl | Ethyl | |
| 2.3.382 | 2-Trifluormethyl-3-trifluormethoxy-Phenyl | Vinyl | Ethyl | |
| 2.3.383 | 2-Methoxy-3-trifluormethoxy-Phenyl | Vinyl | Ethyl | |
| 2.3.384 | 2-Ethoxy-3-trifluormethoxy-Phenyl | Vinyl | Ethyl | |
| 2.3.385 | 2,3-Bis(trifluormethoxy)-Phenyl | Vinyl | Ethyl | |
| 2.3.386 | 2-Nitro-3-trifluormethoxy-Phenyl | Vinyl | Ethyl | |
| 2.3.387 | 2-Fluor-3-(2,2,2-trifluorethoxy)-Phenyl | Vinyl | Ethyl | |
| 2.3.388 | 2-Chlor-3-(2,2,2-trifluorethoxy)-Phenyl | Vinyl | Ethyl | |
| 2.3.389 | 2-Brom-3-(2,2,2-trifluorethoxy)-Phenyl | Vinyl | Ethyl | |
| 2.3.390 | 2-Methyl-3-(2,2,2-trifluorethoxy)-Phenyl | Vinyl | Ethyl | |
| 2.3.391 | 2-Ethyl-3-(2,2,2-trifluorethoxy)-Phenyl | Vinyl | Ethyl | |
| 2.3.392 | 2-Cyclopropyl-3-(2,2,2-trifluorethoxy)-Phenyl | Vinyl | Ethyl | |
| 2.3.393 | 2-Vinyl-3-(2,2,2-trifluorethoxy)-Phenyl | Vinyl | Ethyl | |
| 2.3.394 | 2-Ethinyl-3-(2,2,2-trifluorethoxy-Phenyl | Vinyl | Ethyl | |
| 2.3.395 | 2-Cyano-3-(2,2,2-trifluorethoxy)-Phenyl | Vinyl | Ethyl | |
| 2.3.396 | 2-Trifluormethyl-3-(2,2,2-trifluorethoxy)-Phenyl | Vinyl | Ethyl | |
| 2.3.397 | 2-Methoxy-3-(2,2,2-trifluorethoxy)-Phenyl | Vinyl | Ethyl | |
| 2.3.398 | 2-Ethoxy-3-(2,2,2-trifluorethoxy)-Phenyl | Vinyl | Ethyl | |
| 2.3.399 | 2-Trifluormethoxy-3-(2,2,2-trifluorethoxy)-Phenyl | Vinyl | Ethyl | |
| 2.3.400 | 2-Nitro-3-(2,2,2-trifluorethoxy)-Phenyl | Vinyl | Ethyl | |
| 2.3.401 | 2-Fluor-3-difluormethoxy-Phenyl | Vinyl | Ethyl | |
| 2.3.402 | 2-Chlor-3-difluormethoxy-Phenyl | Vinyl | Ethyl | |
| 2.3.403 | 2-Brom-3-difluormethoxy-Phenyl | Vinyl | Ethyl | |
| 2.3.404 | 2-Methyl-3-difluormethoxy-Phenyl | Vinyl | Ethyl | |
| 2.3.405 | 2-Ethyl-3-difluormethoxy-Phenyl | Vinyl | Ethyl | |
| 2.3.406 | 2-Cyclopropyl-3-difluormethoxy-Phenyl | Vinyl | Ethyl | |
| 2.3.407 | 2-Vinyl-3-difluormethoxy-Phenyl | Vinyl | Ethyl | |
| 2.3.408 | 2-Ethinyl-3-difluormethoxy-Phenyl | Vinyl | Ethyl | |
| 2.3.409 | 2-Cyano-3-difluormethoxy-Phenyl | Vinyl | Ethyl | |
| 2.3.410 | 2-Trifluormethyl-3-difluormethoxy-Phenyl | Vinyl | Ethyl | |
| 2.3.411 | 2-Methoxy-3-difluormethoxy-Phenyl | Vinyl | Ethyl | |
| 2.3.412 | 2-Ethoxy-3-difluormethoxy-Phenyl | Vinyl | Ethyl | |
| 2.3.413 | 2-Trifluormethoxy-3-difluormethoxy-Phenyl | Vinyl | Ethyl | |
| 2.3.414 | 2-Nitro-3-difluormethoxy-Phenyl | Vinyl | Ethyl | |
| 2.3.415 | 2-Fluor-3-(2-methoxyethoxy)-Phenyl | Vinyl | Ethyl | |
| 2.3.416 | 2-Chlor-3-(2-methoxyethoxy)-Phenyl | Vinyl | Ethyl | |
| 2.3.417 | 2-Brom-3-(2-methoxyethoxy)-Phenyl | Vinyl | Ethyl | |
| 2.3.418 | 2-Methyl-3-(2-methoxyethoxy)-Phenyl | Vinyl | Ethyl | |
| 2.3.419 | 2-Ethyl-3-(2-methoxyethoxy)-Phenyl | Vinyl | Ethyl | |
| 2.3.420 | 2-Cyclopropyl-3-(2-methoxyethoxy)-Phenyl | Vinyl | Ethyl | |
| 2.3.421 | 2-Vinyl-3-(2-methoxyethoxy)-Phenyl | Vinyl | Ethyl | |
| 2.3.422 | 2-Ethinyl-3-(2-methoxyethoxy)-Phenyl | Vinyl | Ethyl | |
| 2.3.423 | 2-Cyano-3-(2-methoxyethoxy)-Phenyl | Vinyl | Ethyl | |
| 2.3.424 | 2-Trifluormethyl-3-(2-methoxyethoxy)-Phenyl | Vinyl | Ethyl | |
| 2.3.425 | 2-Methoxy-3-(2-methoxyethoxy) -Phenyl | Vinyl | Ethyl | |
| 2.3.426 | 2-Ethoxy-3-(2-methoxyethoxy)-Phenyl | Vinyl | Ethyl | |
| 2.3.427 | 2-Trifluormethoxy-(2-methoxyethoxy)-Phenyl | Vinyl | Ethyl | |
| 2.3.428 | 2-Nitro-3-(2-methoxyethoxy)-Phenyl | Vinyl | Ethyl | |
| 2.3.429 | 2-Fluor-3-(tert.butoxycarbonyloxy)-Phenyl | Vinyl | Ethyl | |
| 2.3.430 | 2-Chlor-3-(tert.butoxycarbonyloxy)-Phenyl | Vinyl | Ethyl | |
| 2.3.431 | 2-Brom-3-(tert.butoxycarbonyloxy)-Phenyl | Vinyl | Ethyl | |
| 2.3.432 | 2-Methyl-3-(tert.butoxycarbonyloxy)-Phenyl | Vinyl | Ethyl | |
| 2.3.433 | 2-Ethyl-3-(tert.butoxycarbonyloxy)-Phenyl | Vinyl | Ethyl | |
| 2.3.434 | 2-Cyclopropyl-3-(tert.butoxycarbonyloxy)-Phenyl | Vinyl | Ethyl | |
| 2.3.435 | 2-Vinyl-3-(tert.butoxycarbonyloxy)-Phenyl | Vinyl | Ethyl | |
| 2.3.436 | 2-Ethinyl-3-(tert.butoxycarbonyloxy)-Phenyl | Vinyl | Ethyl | |
| 2.3.437 | 2-Cyano-3-(tert.butoxycarbonyloxy)-Phenyl | Vinyl | Ethyl | |
| 2.3.438 | 2-Trifluormethyl-3-(tert.butoxycarbonyloxy)-Phenyl | Vinyl | Ethyl | |
| 2.3.439 | 2-Methoxy-3-(tert.butoxycarbonyloxy)-Phenyl | Vinyl | Ethyl | |
| 2.3.440 | 2-Ethoxy-3-(tert.butoxycarbonyloxy)-Phenyl | Vinyl | Ethyl | |
| 2.3.441 | 2-Trifluormethoxy-3-(tert.butoxycarbonyloxy)-Phenyl | Vinyl | Ethyl | |
| 2.3.442 | 2-Nitro-3-(tert.butoxycarbonyloxy)-Phenyl | Vinyl | Ethyl | |
| 2.3.443 | 2-Fluor-3-nitro-Phenyl | Vinyl | Ethyl | |
| 2.3.444 | 2-Chlor-3-nitro-Phenyl | Vinyl | Ethyl | |
| 2.3.445 | 2-Brom-3-nitro-Phenyl | Vinyl | Ethyl | |
| 2.3.446 | 2-Methyl-3-nitro-Phenyl | Vinyl | Ethyl | |
| 2.3.447 | 2-Ethyl-3-nitro-Phenyl | Vinyl | Ethyl | |
| 2.3.448 | 2-Cyclopropyl-3-nitro-Phenyl | Vinyl | Ethyl | |
| 2.3.449 | 2-Vinyl-3-nitro-Phenyl | Vinyl | Ethyl | |
| 2.3.450 | 2-Ethinyl-3-nitro-Phenyl | Vinyl | Ethyl | |
| 2.3.451 | 2-Cyano-3-nitro-Phenyl | Vinyl | Ethyl | |
| 2.3.452 | 2-Trifluormethyl-3-nitro-Phenyl | Vinyl | Ethyl | |
| 2.3.453 | 2-Methoxy-3-nitro-Phenyl | Vinyl | Ethyl | |
| 2.3.454 | 2-Ethoxy-3-nitro-Phenyl | Vinyl | Ethyl | |
| 2.3.455 | 2-Trifluormethoxy-3-nitro-Phenyl | Vinyl | Ethyl | |
| 2.3.456 | 2-Fluor-3-methylsulfanylPhenyl | Vinyl | Ethyl | |
| 2.3.457 | 2-Chlor-3-methylsulfanyl-Phenyl | Vinyl | Ethyl | |
| 2.3.458 | 2-Brom-3-methylsulfanyl-Phenyl | Vinyl | Ethyl | |
| 2.3.459 | 2-Methyl-3-methylsulfanyl-Phenyl | Vinyl | Ethyl | |
| 2.3.460 | 2-Ethyl-3-methylsulfanyl-Phenyl | Vinyl | Ethyl | |
| 2.3.461 | 2-Cyclopropyl-3-methylsulfanyl-Phenyl | Vinyl | Ethyl | |
| 2.3.462 | 2-Vinyl-3-methylsulfanyl-Phenyl | Vinyl | Ethyl | |
| 2.3.463 | 2-Ethinyl-3-methylsulfanyl-Phenyl | Vinyl | Ethyl | |
| 2.3.464 | 2-Cyano-3-methylsulfanyl-Phenyl | Vinyl | Ethyl | |
| 2.3.465 | 2-Trifluormethyl-3-methylsulfanyl -Phenyl | Vinyl | Ethyl | |
| 2.3.466 | 2-Methoxy-3-methylsulfanyl-Phenyl | Vinyl | Ethyl | |
| 2.3.467 | 3,5-Difluor-Phenyl | Vinyl | Methyl | Ein Enantiomere ohne Zuordnung |
| | | | | [CDCl₃] 3.34 (d,1H); 3.84 (s,3H); 3.94 (d,1H); 5.38 (d,1H); 5.55 (d,1H); 6.14 (dd,1H); 8.88 t,1H); 7.18 (d,2H). |
| 2.3.468 | 3,5-Difluor-Phenyl | Vinyl | Methyl | Das andere Enantiomere ohne Zuordnung [CDCl₃] 3.34 (d,1H); 3.84 (s,3H); 3.94 (d,1H); 5.38 (d,1H); 5.55 (d,1H); 6.14 (dd,1H); 8.88 t,1H); 7.18 (d,2H). |
| 2.3.469 | 3,5-Difluor-Phenyl | 2,2-Difluor-Vinyl | Ethyl | [CDCl₃] 1.33 (t,3H); 3.46 (d,1H); 4.02 (d,1H); 4.23-4.37 (m,2H); 4.91 (dd,1H); 6.91 (t,1H); 7.19 (d,2H). |
| 2.3.470 | 3,5-Difluor-Phenyl | Vinyl | Ethyl | [CDCl₃] 1.33 (t,3H); 3.32 (d,1H); 3.92 (d,1H); 4.29 (m,2H); 5.38 (d,1H); 5.57 (d,1H); 6.13 (dd,1H); 6.89 (m,1H); 7.19 (m,2H). |
| 2.3.471 | 3,5-Difluor-Phenyl | 1-Methyl vinyl | Methyl | [CDCl₃] 1.85 (s,3H); 3.30 (d,1H); 3.83 (s,3H); 4.06 (d,1H); 5.10 (s,1H); 5.28 (s,1H); 6.87 (m,1H); 7.19 (d,2H). |
| 2.3.472 | 3,5-Difluor-Phenyl | Allyl | Ethyl | |
| 2.3.473 | 3,5-Difluor-Phenyl | 1-Chlor vinyl | Methyl | [CDCl₃] 3.53 (d,1H); 3.88 (s,3H); 4.22 (d,1H); 5.55 (s,1H); 5.93 (s,1H); 6.90 (m,1H); 7.20 (d,2H). |
| 2.3.474 | 3,5-Difluor-Phenyl | Ethinyl | Ethyl | |
| 2.3.475 | 3-Chlor-5-fluor-Phenyl | Vinyl | Methyl | [CDCl₃] 3.34 (d,1H); 3.84 (s,3H); 3.93 (d,1H); 5.39 (d,1H); 5.55 (d,1H), 6.14 (dd,1H); 7.14 (d,1H); 7.30 (d,1H); 7.41 (s,1 H). |
| 2.3.476 | 3-Chlor-5-fluor-Phenyl | 1-Methyl vinyl | Methyl | [CDCl₃] 1.84 (s,3H); 3.30 (d,1H); 3.83 (s,3H); 4.07 (d,1H); 5.10 (s,1H); 5.28 (s,1H); 7.14 (d,1H); 7.32 (d,1H); 7.43 (s,1 H). |
| 2.3.477 | 3-Chlor-5-fluor-Phenyl | Allyl | Ethyl | |
| 2.3.478 | 3-Chlor-5-fluor-Phenyl | 1-Chlor vinyl | Ethyl | |
| 2.3.479 | 3-Chlor-5-fluor-Phenyl | Ethinyl | Ethyl | |
| 2.3.480 | 3-Brom-5-fluor-Phenyl | Vinyl | Ethyl | |
| 2.3.481 | 3-Brom-5-fluor-Phenyl | 1-Methyl vinyl | Ethyl | |
| 2.3.482 | 3-Brom-5-fluor-Phenyl | Allyl | Ethyl | |
| 2.3.483 | 3-Brom-5-fluor-Phenyl | 1-Chlor vinyl | Ethyl | |
| 2.3.484 | 3-Brom-5-fluor-Phenyl | Ethinyl | Ethyl | |
| 2.3.485 | 3-Iod-5-fluor-Phenyl | Vinyl | Ethyl | |
| 2.3.486 | 3-Methyl-5-fluor-Phenyl | Vinyl | Methyl | [CDCl₃] 2.37 (s,3H); 3.36 (d,1H); 3.83 (s,3H); 3.94 (d,1H); 5.37 (d,1H); 5.55 (d,1H); 6.13 (dd,1H); 6.94 (d,1H); 7.18 (d,1H); 7.24 (s,1H). |
| 2.3.487 | 3-Methyl-5-fluor-Phenyl | 1-Methyl vinyl | Ethyl | |
| 2.3.488 | 3-Methyl-5-fluor-Phenyl | Allyl | Ethyl | |
| 2.3.489 | 3-Methyl-5-fluor-Phenyl | 1-Chlor vinyl | Ethyl | |
| 2.3.490 | 3-Methyl-5-fluor-Phenyl | Ethinyl | Ethyl | |
| 2.3.491 | 3-Ethyl-5-fluor-Phenyl | Vinyl | Ethyl | |
| 2.3.492 | 3-Propyl-5-fluor-Phenyl | Vinyl | Ethyl | |
| 2.3.493 | 3-iPropyl-5-fluor-Phenyl | Vinyl | Ethyl | |
| 2.3.494 | 3-nButyl-5-fluor-Phenyl | Vinyl | Ethyl | |
| 2.3.495 | 3-isoButyl-5-fluor-Phenyl | Vinyl | Ethyl | |
| 2.3.496 | 3-tert.Butyl-5-fluor-Phenyl | Vinyl | Ethyl | |
| 2.3.497 | 3-Cyclopropyl-5-fluor-Phenyl | Vinyl | Ethyl | |
| 2.3.498 | 3-Vinyl-5-fluor-Phenyl | Vinyl | Ethyl | |
| 2.3.499 | 3-Ethinyl-5-fluor-Phenyl | Vinyl | Ethyl | |
| 2.3.500 | 3-Cyano-5-fluor-Phenyl | Vinyl | Ethyl | |
| 2.3.501 | 3-Trifluormethyl-5-fluor-Phenyl | Vinyl | Ethyl | |
| 2.3.502 | 3-Trifluormethyl-5-fluor-Phenyl | 1-Methyl vinyl | Ethyl | |
| 2.3.503 | 3-Trifluormethyl-5-fluor-Phenyl | Allyl | Ethyl | |
| 2.3.504 | 3-Trifluormethyl-5-fluor-Phenyl | 1-Chlor vinyl | Ethyl | |
| 2.3.505 | 3-Trifluormethyl-5-fluor-Phenyl | Ethinyl | Ethyl | |
| 2.3.506 | 3-(Methoxycarbony)l-5-fluor-Phenyl | Vinyl | Ethyl | |
| 2.3.507 | 3-Hydroxymethyl-5-fluor-Phenyl | Vinyl | Ethyl | |
| 2.3.508 | 3-Carbamoyl-5-fluor-Phenyl | Vinyl | Ethyl | |
| 2.3.509 | 3-Hydroxy-5-fluor-Phenyl- | Vinyl | Ethyl | |
| 2.3.510 | 3-Methoxy-5-fluor-Phenyl | Vinyl | Ethyl | |
| 2.3.511 | 3-Ethoxy-5-fluor-Phenyl | Vinyl | Ethyl | |
| 2.3.512 | 3-nPropyoxy-5-fluor-Phenyl | Vinyl | Ethyl | |
| 2.3.513 | 3-isoPropoxy-5-fluor-Phenyl | Vinyl | Ethyl | |
| 2.3.514 | 3-nButoxy-5-fluor-Phenyl | Vinyl | Ethyl | |
| 2.3.515 | 3-isoButoxy-5-fluor-Phenyl | Vinyl | Ethyl | |
| 2.3.516 | 3-tert.Butoxy-5-fluor-Phenyl | Vinyl | Ethyl | |
| 2.3.517 | 3-Difluormethoxy-5-fluor-Phenyl | Vinyl | Ethyl | |
| 2.3.518 | 3-Trifluormethoxy-5-fluor-Phenyl | Vinyl | Ethyl | |
| 2.3.519 | 3-(2,2,2-Trifluorethoxy)-5-fluor-Phenyl | Vinyl | Ethyl | |
| 2.3.520 | 3-(2-Chlorethoxy)-5-fluor-Phenyl | Vinyl | Ethyl | |
| 2.3.521 | 3-(2-Hydroxyethoxy)-5-fluor-Phenyl- | Vinyl | Ethyl | |
| 2.3.522 | 3-[(tert-Butoxycarbonyl)oxy]-5-fluor-Phenyl | Vinyl | Ethyl | |
| 2.3.523 | 3-Nitro-5-fluor-Phenyl | Vinyl | Ethyl | |
| 2.3.524 | 3-Acetoxy-5-fluor-Phenyl | Vinyl | Ethyl | |
| 2.3.525 | {3-[(tert-Butoxycarbonyl)amino]-5-fluor-Phenyl} | Vinyl | Ethyl | |
| 2.3.526 | 3-Methylsulfanyl-5-fluor-Phenyl | Vinyl | Ethyl | |
| 2.3.527 | 3,5-Dichlor-Phenyl | Vinyl | Methyl | [CDCl₃] 3.34 (d,1H); 3.84 (s,3H); 3.93 (d,1H), 5.39 (d,1H); 5.55 (d,1H); 6.14 (dd,1H); 7.40 (s,1H); 7.54 (s,2H). |
| 2.3.528 | 3,5-Dichlor-Phenyl | 1-Methyl vinyl | Methyl | [CDCl₃] 1.84 (s,3H); 3.30 (d,1H); 3.83 (s,3H); 4.06 (d,1H); 5.10 (s,1H); 5.28 (s,1H); 7.41 (s,1H); 7.55 (s,2H). |
| 2.3.529 | 3,5-Dichlor-Phenyl | Allyl | Ethyl | |
| 2.3.530 | 3,5-Dichlor-Phenyl | 1-Chlor vinyl | Ethyl | |
| 2.3.531 | 3,5-Dichlor-Phenyl | Ethinyl | Ethyl | |
| 2.3.532 | 3-Brom-5-chlor-Phenyl | Vinyl | Ethyl | |
| 2.3.533 | 3-Iod-5-chlor-Phenyl | Vinyl | Ethyl | |
| 2.3.534 | 3-Methyl-5-chlor-Phenyl | Vinyl | Methyl | [CDCl₃] 2.36 (s,3H), 3.35 (d,1H); 3.83 (s,3H); 3.94 (d,1H); 5.37 (d,1H); 5.55 (d,1H); 6.13 (dd,1H); 7.22 (s,1H); 7.38 (s,1H); 7.43 (s,1H). |
| 2.3.535 | 3-Methyl-5-chlor-Phenyl | 1-Methyl vinyl | Methyl | [CDCl₃] 1.85 (s,3H); 2.36 (s,3H); 3.32 (d,1H); 3.82 (s,3H); 4.07 (d,1H); 5.08 (s,1H); 5.28 (s,1H); 7.21 (s,1H); 7.38 (s,1H); 7.45 (s,1H). |
| 2.3.536 | 3-Ethyl-5-chlor-Phenyl | Vinyl | Methyl | [CDCl₃] 1.24 (t,1H); 2.65 (q,2H); 3.37 (d,1H); 3.83 (s,3H); 3.95 (d,2H); 5.37 (d,1H); 5.55 (d,1H); 6.14 (dd,1H); 7.24 (s,1H); 7.40 (s,1H); 7.43 (s,1H). |
| 2.3.537 | 3-Propyl-5-chlor-Phenyl | Vinyl | Ethyl | |
| 2.3.538 | 3-isoPropyl-5-chlor-Phenyl | Vinyl | Ethyl | |
| 2.3.539 | 3-nButyl-5-chlor-Phenyl | Vinyl | Ethyl | |
| 2.3.540 | 3-isoButyl-5-chlor-Phenyl | Vinyl | Ethyl | |
| 2.3.541 | 3-tert.Butyl-5-chlor-Phenyl | Vinyl | Ethyl | |
| 2.3.542 | 3-Cyclopropyl-5-chlor-Phenyl | Vinyl | Ethyl | |
| 2.3.543 | 3-Vinyl-5-chlor-Phenyl | Vinyl | Ethyl | |
| 2.3.544 | 3-Ethinyl-5-chlor-Phenyl | Vinyl | Ethyl | |
| 2.3.545 | 3-Cyano-5-chlor-Phenyl | Vinyl | Ethyl | |
| 2.3.546 | 3-Trifluormethyl-5-chlor-Phenyl | Vinyl | Ethyl | |
| 2.3.547 | 3-(Hydroxycarbonyl)-5-chlor-Phenyl | Vinyl | Ethyl | |
| 2.3.548 | 3-(Methoxycarbony)l-5-chlor-Phenyl | Vinyl | Ethyl | |
| 2.3.549 | 3-Hydroxymethyl-5-chlor-Phenyl | Vinyl | Ethyl | |
| 2.3.550 | 3-Carbamoyl-5-chlor-Phenyl | Vinyl | Ethyl | |
| 2.3.551 | 3-Hydroxy-5-chlor-Phenyl- | Vinyl | Ethyl | |
| 2.3.552 | 3-Methoxy-5-chlor-Phenyl | Vinyl | Ethyl | |
| 2.3.553 | 3-Ethoxy-5-chlor-Phenyl | Vinyl | Ethyl | |
| 2.3.554 | 3-nPropyoxy-5-chlor-Phenyl | Vinyl | Ethyl | |
| 2.3.555 | 3-isoPropoxy-5-chlor-Phenyl | Vinyl | Ethyl | |
| 2.3.556 | 3-nButoxy-5-chlor-Phenyl | Vinyl | Ethyl | |
| 2.3.557 | 3-isoButoxy-5-chlor-Phenyl | Vinyl | Ethyl | |
| 2.3.558 | 3-tert.Butoxy-5-chlor-Phenyl | Vinyl | Ethyl | |
| 2.3.559 | 3-Difluormethoxy-5-chlor-Phenyl | Vinyl | Ethyl | |
| 2.3.560 | 3-Trifluormethoxy-5-chlor-Phenyl | Vinyl | Ethyl | |
| 2.3.561 | 3-(2,2,2-Trifluorethoxy)-5-chlor-Phenyl | Vinyl | Ethyl | |
| 2.3.562 | 3-(2-Chlorethoxy)-5-chlor-Phenyl | Vinyl | Ethyl | |
| 2.3.563 | 3-(2-Hydroxyethoxy)-5-chlor-Phenyl- | Vinyl | Ethyl | |
| 2.3.564 | 3-[(tert-Butoxycarbonyl)oxy]-5-chlor-Phenyl | Vinyl | Ethyl | |
| 2.3.565 | 3-Nitro-5-chlor-Phenyl | Vinyl | Ethyl | |
| 2.3.566 | 3-Acetoxy-5-chlor-Phenyl | Vinyl | Ethyl | |
| 2.3.567 | {3-[(tert-Butoxycarbonyl)amino]-5-chlor-Phenyl} | Vinyl | Ethyl | |
| 2.3.568 | 3-Methylsulfanyl-5-chlor-Phenyl | Vinyl | Ethyl | |
| 2.3.569 | 3,5-Dibrom-Phenyl | Vinyl | Ethyl | |
| 2.3.570 | 3,5-Dibrom-Phenyl | 1-Methyl vinyl | Ethyl | |
| 2.3.571 | 3-Iod-5-brom-Phenyl | Vinyl | Ethyl | |
| 2.3.572 | 3-Methyl-5-brom-Phenyl | Vinyl | Methyl | [CDCl₃] 2.35 (s,3H), 3.35 (d,1H); 3.83 (s,3H); 3.93 (d,1H); 5.35 (d,1H), 5.55 (d,1H); 6.13 (dd,1H); 7.38 (s,1H), 7.42 (s,1H); 7.58 (s,1H). |
| 2.3.573 | 3-Methyl-5-brom-Phenyl | 1-Methyl vinyl | Methyl | [CDCl₃] 1.84 (s,3H); 2.35 (s,3H); 3.31 (d,1H); 3.82 (s,3H); 4.07 (d,1H); 5.08 (s,1H); 5.28 (s,1H); 7.38 (s,1H), 7.43 (s,1H); 7.59 (s,1H). |
| 2.3.574 | 3-Methyl-5-brom-Phenyl | Allyl | Ethyl | |
| 2.3.575 | 3-Methyl-5-brom-Phenyl | 1-Chlorvinyl | Ethyl | |
| 2.3.576 | 3-Methyl-5-brom-Phenyl | Ethinyl | Ethyl | |
| 2.3.577 | 3-Ethyl-5-brom-Phenyl | Vinyl | Ethyl | |
| 2.3.578 | 3-Propyl-5-brom-Phenyl | Vinyl | Ethyl | |
| 2.3.579 | 3-isoPropyl-5-brom-Phenyl | Vinyl | Ethyl | |
| 2.3.580 | 3-nButyl-5-brom-Phenyl | Vinyl | Ethyl | |
| 2.3.581 | 3-isoButyl-5-brom-Phenyl | Vinyl | Ethyl | |
| 2.3.582 | 3-tert.Butyl-5-brom-Phenyl | Vinyl | Ethyl | |
| 2.3.583 | 3-Cyclopropyl-5-brom-Phenyl | Vinyl | Ethyl | |
| 2.3.584 | 3-Vinyl-5-brom-Phenyl | Vinyl | Ethyl | |
| 2.3.585 | 3-Ethinyl-5-brom-Phenyl | Vinyl | Ethyl | |
| 2.3.586 | 3-Cyano-5-brom-Phenyl | Vinyl | Ethyl | |
| 2.3.587 | 3-Trifluormethyl-5-brom-Phenyl | Vinyl | Ethyl | |
| 2.3.588 | 3-(Hydroxycarbonyl)-5-brom-Phenyl | Vinyl | Ethyl | |
| 2.3.589 | 3-(Methoxycarbony)l-5-brom-Phenyl | Vinyl | Ethyl | |
| 2.3.590 | 3-Hydroxymethyl-5-brom-Phenyl | Vinyl | Ethyl | |
| 2.3.591 | 3-Carbamoyl-5-brom-Phenyl | Vinyl | Ethyl | |
| 2.3.592 | 3-Hydroxy-5-brom-Phenyl- | Vinyl | Ethyl | |
| 2.3.593 | 3-Methoxy-5-brom-Phenyl | Vinyl | Ethyl | |
| 2.3.594 | 3-Ethoxy-5-brom-Phenyl | Vinyl | Ethyl | |
| 2.3.595 | 3-nPropyoxy-5-brom-Phenyl | Vinyl | Ethyl | |
| 2.3.596 | 3-isoPropoxy-5-brom-Phenyl | Vinyl | Ethyl | |
| 2.3.597 | 3-nButoxy-5-brom-Phenyl | Vinyl | Ethyl | |
| 2.3.598 | 3-isoButoxy-5-brom-Phenyl | Vinyl | Ethyl | |
| 2.3.599 | 3-tert.Butoxy-5-brom-Phenyl | Vinyl | Ethyl | |
| 2.3.600 | 3-Difluormethoxy-5-brom-Phenyl | Vinyl | Ethyl | |
| 2.3.601 | 3-Trifluormethoxy-5-brom-Phenyl | Vinyl | Ethyl | |
| 2.3.602 | 3-(2,2,2-Trifluorethoxy)-5-brom-Phenyl | Vinyl | Ethyl | |
| 2.3.603 | 3-(2-Chlorethoxy)-5-brom-Phenyl | Vinyl | Ethyl | |
| 2.3.604 | 3-(2-Hydroxyethoxy)-5-brom-Phenyl- | Vinyl | Ethyl | |
| 2.3.605 | 3-[(tert-Butoxycarbonyl)oxy]-5-brom-Phenyl | Vinyl | Ethyl | |
| 2.3.606 | 3-Nitro-5-brom-Phenyl | Vinyl | Ethyl | |
| 2.3.607 | 3-Acetoxy-5-brom-Phenyl | Vinyl | Ethyl | |
| 2.3.608 | {3-[(tert-Butoxycarbonyl)amino]-5-brom-Phenyl} | Vinyl | Ethyl | |
| 2.3.609 | 3-Methylsulfanyl-5-brom-Phenyl | Vinyl | Ethyl | |
| 2.3.610 | 3,5-Diiod-Phenyl | Vinyl | Ethyl | |
| 2.3.611 | 3-Methyl-5-iod-Phenyl | Vinyl | Ethyl | |
| 2.3.612 | 3-Ethyl-5-iod-Phenyl | Vinyl | Ethyl | |
| 2.3.613 | 3-Propyl-5-iod-Phenyl | Vinyl | Ethyl | |
| 2.3.614 | 3-isoPropyl-5-iod-Phenyl | Vinyl | Ethyl | |
| 2.3.615 | 3-nButyl-5-iod-Phenyl | Vinyl | Ethyl | |
| 2.3.616 | 3-isoButyl-5-iod-Phenyl | Vinyl | Ethyl | |
| 2.3.617 | 3-tert.Butyl-5-iod-Phenyl | Vinyl | Ethyl | |
| 2.3.618 | 3-Cyclopropyl-5-iod-Phenyl | Vinyl | Ethyl | |
| 2.3.619 | 3-Vinyl-5-iod-Phenyl | Vinyl | Ethyl | |
| 2.3.620 | 3-Ethinyl-5-iod-Phenyl | Vinyl | Ethyl | |
| 2.3.621 | 3-Cyano-5-iod-Phenyl | Vinyl | Ethyl | |
| 2.3.622 | 3-Trifluormethyl-5-iod-Phenyl | Vinyl | Ethyl | |
| 2.3.623 | 3-(Hydroxycarbonyl)-5-iod-Phenyl | Vinyl | Ethyl | |
| 2.3.624 | 3-(Methoxycarbonyl)-5-iod-Phenyl | Vinyl | Ethyl | |
| 2.3.625 | 3-Hydroxymethyl-5-iod-Phenyl | Vinyl | Ethyl | |
| 2.3.626 | 3-Carbamoyl-5-iod-Phenyl | Vinyl | Ethyl | |
| 2.3.627 | 3-Hydroxy-5-iod-Phenyl- | Vinyl | Ethyl | |
| 2.3.628 | 3-Methoxy-5-iod-Phenyl | Vinyl | Ethyl | |
| 2.3.629 | 3-Ethoxy-5-iod-Phenyl | Vinyl | Ethyl | |
| 2.3.630 | 3-nPropyoxy-5-iod-Phenyl | Vinyl | Ethyl | |
| 2.3.631 | 3-isoPropoxy-5-iod-Phenyl | Vinyl | Ethyl | |
| 2.3.632 | 3-nButoxy-5-iod-Phenyl | Vinyl | Ethyl | |
| 2.3.633 | 3-isoButoxy-5-iod-Phenyl | Vinyl | Ethyl | |
| 2.3.634 | 3-tert.Butoxy-5-iod-Phenyl | Vinyl | Ethyl | |
| 2.3.635 | 3-Difluormethoxy-5-iod-Phenyl | Vinyl | Ethyl | |
| 2.3.636 | 3-Trifluormethoxy-5-iod-Phenyl | Vinyl | Ethyl | |
| 2.3.637 | 3-(2,2,2-Trifluorethoxy)-5-iod-Phenyl | Vinyl | Ethyl | |
| 2.3.638 | 3-(2-Chlorethoxy)-5-iod-Phenyl | Vinyl | Ethyl | |
| 2.3.639 | 3-(2-Hydroxyethoxy)-5-iod-Phenyl- | Vinyl | Ethyl | |
| 2.3.640 | 3-[(tert-Butoxycarbonyl)oxy]-5-iod-Phenyl | Vinyl | Ethyl | |
| 2.3.641 | 3-Nitro-5-iod-Phenyl | Vinyl | Ethyl | |
| 2.3.642 | 3-Acetoxy-5-iod-Phenyl | Vinyl | Ethyl | |
| 2.3.643 | {3-[(tert-Butoxycarbonyl)amino]-5-iod-Phenyl} | Vinyl | Ethyl | |
| 2.3.644 | 3-Methylsulfanyl-5-iod-Phenyl | Vinyl | Ethyl | |
| 2.3.645 | 3,5-Dimethyl-Phenyl | Vinyl | Methyl | [CDCl₃] 2.33 (s,6H); 3.38 (d,1H); 3.83 (s,3H); 3.96 (d,1H); 5.35 (d,1H); 5.55 (d,1H); 6.15 (dd,1H); 7.06 (s,1H); 7.28 (s,2H). |
| 2.3.646 | 3-Ethyl-5-methyl-Phenyl | Vinyl | Ethyl | |
| 2.3.647 | 3-Propyl-5-methyl-Phenyl | Vinyl | Ethyl | |
| 2.3.648 | 3-isoPropyl-5-methyl-Phenyl | Vinyl | Ethyl | |
| 2.3.649 | 3-nButyl-5-methyl-Phenyl | Vinyl | Ethyl | |
| 2.3.650 | 3-isoButyl-5-methyl-Phenyl | Vinyl | Ethyl | |
| 2.3.651 | 3-tert.Butyl-5-methyl-Phenyl | Vinyl | Ethyl | |
| 2.3.652 | 3-Cyclopropyl-5-methyl-Phenyl | Vinyl | Ethyl | |
| 2.3.653 | 3-Cyano-5-methyl-Phenyl | Vinyl | Ethyl | |
| 2.3.654 | 3-Trifluormethyl-5-methyl-Phenyl | Vinyl | Ethyl | |
| 2.3.655 | 3-(Methoxycarbony)l-5-methyl-Phenyl | Vinyl | Ethyl | |
| 2.3.656 | 3-Methoxy-5-methyl-Phenyl | Vinyl | Ethyl | |
| 2.3.657 | 3-Ethoxy-5-methyl-Phenyl | Vinyl | Ethyl | |
| 2.3.658 | 3-nPropyoxy-5-methyl-Phenyl | Vinyl | Ethyl | |
| 2.3.659 | 3-nButoxy-5-methyl-Phenyl | Vinyl | Ethyl | |
| 2.3.660 | 3-isoButoxy-5-methyl-Phenyl | Vinyl | Ethyl | |
| 2.3.661 | 3-Difluormethoxy-5-methyl-Phenyl | Vinyl | Ethyl | |
| 2.3.662 | 3-Trifluormethoxy-5-methyl-Phenyl | Vinyl | Ethyl | |
| 2.3.663 | 3-Nitro-5-methyl-Phenyl | Vinyl | Ethyl | |
| 2.3.664 | 3-Acetoxy-5-methyl-Phenyl | Vinyl | Ethyl | |
| 2.3.665 | 3-Methylsulfanyl-5-methyl-Phenyl | Vinyl | Ethyl | |
| 2.3.666 | 3,5-Diethyl-Phenyl | Vinyl | Ethyl | |
| 2.3.667 | 3-Propyl-5-ethyl-Phenyl | Vinyl | Ethyl | |
| 2.3.668 | 3-isoPropyl-5-ethyl-Phenyl | Vinyl | Ethyl | |
| 2.3.669 | 3-nButyl-5-ethyl-Phenyl | Vinyl | Ethyl | |
| 2.3.670 | 3-isoButyl-5-ethyl-Phenyl | Vinyl | Ethyl | |
| 2.3.671 | 3-tert.Butyl-5-ethyl-Phenyl | Vinyl | Ethyl | |
| 2.3.672 | 3-Cyclopropyl-5-ethyl-Phenyl | Vinyl | Ethyl | |
| 2.3.673 | 3-Cyano-5-ethyl-Phenyl | Vinyl | Ethyl | |
| 2.3.674 | 3-Trifluormethyl-5-ethyl-Phenyl | Vinyl | Ethyl | |
| 2.3.675 | 3-(Methoxycarbony)l-5-ethyl-Phenyl | Vinyl | Ethyl | |
| 2.3.676 | 3-Methoxy-5-ethyl-Phenyl | Vinyl | Ethyl | |
| 2.3.677 | 3-Ethoxy-5-ethyl-Phenyl | Vinyl | Ethyl | |
| 2.3.678 | 3-nPropyoxy-5-ethyl-Phenyl | Vinyl | Ethyl | |
| 2.3.679 | 3-nButoxy-5-ethyl-Phenyl | Vinyl | Ethyl | |
| 2.3.680 | 3-isoButoxy-5-ethyl-Phenyl | Vinyl | Ethyl | |
| 2.3.681 | 3-Difluormethoxy-5-ethyl-Phenyl | Vinyl | Ethyl | |
| 2.3.682 | 3-Trifluormethoxy-5-ethyl-Phenyl | Vinyl | Ethyl | |
| 2.3.683 | 3-Nitro-5-ethyl-Phenyl | Vinyl | Ethyl | |
| 2.3.684 | 3-Acetoxy-5-ethyl-Phenyl | Vinyl | Ethyl | |
| 2.3.685 | 3-Methylsulfanyl-5-ethyl-Phenyl | Vinyl | Ethyl | |
| 2.3.686 | 3,5-Dipropyl-Phenyl | Vinyl | Ethyl | |
| 2.3.687 | 3-isoPropyl-5-propyl-Phenyl | Vinyl | Ethyl | |
| 2.3.688 | 3-nButyl-5-propyl-Phenyl | Vinyl | Ethyl | |
| 2.3.689 | 3-isoButyl-5-propyl-Phenyl | Vinyl | Ethyl | |
| 2.3.690 | 3-tert.Butyl-5-propyl-Phenyl | Vinyl | Ethyl | |
| 2.3.691 | 3-Cyclopropyl-5-propyl-Phenyl | Vinyl | Ethyl | |
| 2.3.692 | 3-Cyano-5-propyl-Phenyl | Vinyl | Ethyl | |
| 2.3.693 | 3-Trifluormethyl-5-propyl-Phenyl | Vinyl | Ethyl | |
| 2.3.694 | 3-(Methoxycarbonyl)-5-propyl-Phenyl | Vinyl | Ethyl | |
| 2.3.695 | 3-Methoxy-5-propyl-Phenyl | Vinyl | Ethyl | |
| 2.3.696 | 3-Ethoxy-5-propyl-Phenyl | Vinyl | Ethyl | |
| 2.3.697 | 3-nPropyoxy-5-propyl-Phenyl | Vinyl | Ethyl | |
| 2.3.698 | 3-nButoxy-5-propyl-Phenyl | Vinyl | Ethyl | |
| 2.3.699 | 3-isoButoxy-5-propyl-Phenyl | Vinyl | Ethyl | |
| 2.3.700 | 3-Difluormethoxy-5-propyl-Phenyl | Vinyl | Ethyl | |
| 2.3.701 | 3-Trifluormethoxy-5-ethyl-Phenyl | Vinyl | Ethyl | |
| 2.3.702 | 3-Nitro-5-propyl-Phenyl | Vinyl | Ethyl | |
| 2.3.703 | 3-Acetoxy-5-propyl-Phenyl | Vinyl | Ethyl | |
| 2.3.704 | 3-Methylsulfanyl-5-propyl-Phenyl | Vinyl | Ethyl | |
| 2.3.705 | 3,5-Diisopropyl-Phenyl | Vinyl | Ethyl | |
| 2.3.706 | 3-nButyl-5-isopropyl-Phenyl | Vinyl | Ethyl | |
| 2.3.707 | 3-isoButyl-5-isopropyl-Phenyl | Vinyl | Ethyl | |
| 2.3.708 | 3-tert.Butyl-5-isopropyl-Phenyl | Vinyl | Ethyl | |
| 2.3.709 | 3-Cyclopropyl-5-isopropyl-Phenyl | Vinyl | Ethyl | |
| 2.3.710 | 3-Cyano-5-isopropyl-Phenyl | Vinyl | Ethyl | |
| 2.3.711 | 3-Trifluormethyl-5-isopropyl-Phenyl | Vinyl | Ethyl | |
| 2.3.712 | 3-(Methoxycarbony)l-5-isopropyl-Phenyl | Vinyl | Ethyl | |
| 2.3.713 | 3-Methoxy-5-isopropyl-Phenyl | Vinyl | Ethyl | |
| 2.3.714 | 3-Ethoxy-5-isopropyl-Phenyl | Vinyl | Ethyl | |
| 2.3.715 | 3-nPropyoxy-5-isopropyl-Phenyl | Vinyl | Ethyl | |
| 2.3.716 | 3-nButoxy-5-isopropyl-Phenyl | Vinyl | Ethyl | |
| 2.3.717 | 3-isoButoxy-5-isopropyl-Phenyl | Vinyl | Ethyl | |
| 2.3.718 | 3-Difluormethoxy-5-isopropyl-Phenyl | Vinyl | Ethyl | |
| 2.3.719 | 3-Trifluormethoxy-5-isopropyl-Phenyl | Vinyl | Ethyl | |
| 2.3.720 | 3-Nitro-5-isopropyl-Phenyl | Vinyl | Ethyl | |
| 2.3.721 | 3-Acetoxy-5-isopropyl-Phenyl | Vinyl | Ethyl | |
| 2.3.722 | 3-Methylsulfanyl-5-isopropyl-Phenyl | Vinyl | Ethyl | |
| 2.3.723 | 3,5-Dibutyl-Phenyl | Vinyl | Ethyl | |
| 2.3.724 | 3-isoButyl-5-butyl-Phenyl | Vinyl | Ethyl | |
| 2.3.725 | 3-tert.Butyl-5-butyl-Phenyl | Vinyl | Ethyl | |
| 2.3.726 | 3-Cyclopropyl-5-butyl-Phenyl | Vinyl | Ethyl | |
| 2.3.727 | 3-Cyano-5-butyl-Phenyl | Vinyl | Ethyl | |
| 2.3.728 | 3-Trifluormethyl-5-butyl-Phenyl | Vinyl | Ethyl | |
| 2.3.729 | 3-(Methoxycarbony)l-5-butyl-Phenyl | Vinyl | Ethyl | |
| 2.3.730 | 3-Methoxy-5-butyl-Phenyl | Vinyl | Ethyl | |
| 2.3.731 | 3-Ethoxy-5-butyl-Phenyl | Vinyl | Ethyl | |
| 2.3.732 | 3-nPropyoxy-5-butyl-Phenyl | Vinyl | Ethyl | |
| 2.3.733 | 3-nButoxy-5-butyl-Phenyl | Vinyl | Ethyl | |
| 2.3.734 | 3-isoButoxy-5-butyl-Phenyl | Vinyl | Ethyl | |
| 2.3.735 | 3-Difluormethoxy-5-butyl-Phenyl | Vinyl | Ethyl | |
| 2.3.736 | 3-Trifluormethoxy-5-butyl-Phenyl | Vinyl | Ethyl | |
| 2.3.737 | 3-Nitro-5-butyl-Phenyl | Vinyl | Ethyl | |
| 2.3.738 | 3-Acetoxy-5-butyl-Phenyl | Vinyl | Ethyl | |
| 2.3.739 | 3-Methylsulfanyl-5-butyl-Phenyl | Vinyl | Ethyl | |
| 2.3.740 | 3,5-Diisobutyl-Phenyl | Vinyl | Ethyl | |
| 2.3.741 | 3-tert.Butyl-5-isobutyl-Phenyl | Vinyl | Ethyl | |
| 2.3.742 | 3-Cyclopropyl-5-isobutyl-Phenyl | Vinyl | Ethyl | |
| 2.3.743 | 3-Cyano-5-isobutyl-Phenyl | Vinyl | Ethyl | |
| 2.3.744 | 3-Trifluormethyl-5-isobutyl-Phenyl | Vinyl | Ethyl | |
| 2.3.745 | 3-(Methoxycarbonyl)-5-isobutyl-Phenyl | Vinyl | Ethyl | |
| 2.3.746 | 3-Methoxy-5-isobutyl-Phenyl | Vinyl | Ethyl | |
| 2.3.747 | 3-Ethoxy-5-isobutyl-Phenyl | Vinyl | Ethyl | |
| 2.3.748 | 3-nPropyoxy-5-isobutyl-Phenyl | Vinyl | Ethyl | |
| 2.3.749 | 3-nButoxy-5-isobutyl-Phenyl | Vinyl | Ethyl | |
| 2.3.750 | 3-isoButoxy-5-isobutyl-Phenyl | Vinyl | Ethyl | |
| 2.3.751 | 3-Difluormethoxy-5-isobutyl-Phenyl | Vinyl | Ethyl | |
| 2.3.752 | 3-Trifluormethoxy-5-isobutyl-Phenyl | Vinyl | Ethyl | |
| 2.3.753 | 3-Nitro-5-isobutyl-Phenyl | Vinyl | Ethyl | |
| 2.3.754 | 3-Acetoxy-5-isobutyl-Phenyl | Vinyl | Ethyl | |
| 2.3.755 | 3-Methylsulfanyl-5-isobutyl-Phenyl | Vinyl | Ethyl | |
| 2.3.756 | 3,5-D(itert.butyl)-Phenyl | Vinyl | Ethyl | |
| 2.3.757 | 3-Cyclopropyl-5-tert.butyl-Phenyl | Vinyl | Ethyl | |
| 2.3.758 | 3-Cyano-5-tert.butyl-Phenyl | Vinyl | Ethyl | |
| 2.3.759 | 3-Trifluormethyl-5-tert.butyl-Phenyl | Vinyl | Ethyl | |
| 2.3.760 | 3-(Methoxycarbonyl)-5-tert.butyl-Phenyl | Vinyl | Ethyl | |
| 2.3.761 | 3-Methoxy-5-tert.butyl-Phenyl | Vinyl | Ethyl | |
| 2.3.762 | 3-Ethoxy-5-tert.butyl-Phenyl | Vinyl | Ethyl | |
| 2.3.763 | 3-nPropyoxy-5-tert.butyl-Phenyl | Vinyl | Ethyl | |
| 2.3.764 | 3-nButoxy-5-tert.butyl-Phenyl | Vinyl | Ethyl | |
| 2.3.765 | 3-isoButoxy-5-tert.butyl-Phenyl | Vinyl | Ethyl | |
| 2.3.766 | 3-Difluormethoxy-5-tert.butyl-Phenyl | Vinyl | Ethyl | |
| 2.3.767 | 3-Trifluormethoxy-5-tert.butyl-Phenyl | Vinyl | Ethyl | |
| 2.3.768 | 3-Nitro-5-tert.butyl-Phenyl | Vinyl | Ethyl | |
| 2.3.769 | 3-Acetoxy-5-tert.butyl-Phenyl | Vinyl | Ethyl | |
| 2.3.770 | 3-Methylsulfanyl-5-tert.butyl-Phenyl | Vinyl | Ethyl | |
| 2.3.771 | 3-tert.Butyl-5-cyclopropyl-Phenyl | Vinyl | Ethyl | |
| 2.3.772 | 3,5-Dicyclopropyl-Phenyl | Vinyl | Ethyl | |
| 2.3.773 | 3-Cyano-5-cyclopropyl-Phenyl | Vinyl | Ethyl | |
| 2.3.774 | 3-Trifluormethyl-5-cyclopropyl-Phenyl | Vinyl | Ethyl | |
| 2.3.775 | 3-(Methoxycarbonyl)-5-cyclopropyl-Phenyl | Vinyl | Ethyl | |
| 2.3.776 | 3-Methoxy-5-cyclopropyl-Phenyl | Vinyl | Ethyl | |
| 2.3.777 | 3-Ethoxy-5-cyclopropyl-Phenyl | Vinyl | Ethyl | |
| 2.3.778 | 3-nPropyoxy-5-cyclopropyl-Phenyl | Vinyl | Ethyl | |
| 2.3.779 | 3-nButoxy-5-cyclopropyl-Phenyl | Vinyl | Ethyl | |
| 2.3.780 | 3-isoButoxy-5-cyclopropyl-Phenyl | Vinyl | Ethyl | |
| 2.3.781 | 3-Difluormethoxy-5-cyclopropyl-Phenyl | Vinyl | Ethyl | |
| 2.3.782 | 3-Trifluormethoxy-5-cyclopropyl-Phenyl | Vinyl | Ethyl | |
| 2.3.783 | 3-Nitro-5-cyclopropyl-Phenyl | Vinyl | Ethyl | |
| 2.3.784 | 3-Acetoxy-5-cyclopropyl-Phenyl | Vinyl | Ethyl | |
| 2.3.785 | 3-Methylsulfanyl-5-cyclopropyl-Phenyl | Vinyl | Ethyl | |
| 2.3.786 | 3,5-Dicyano-Phenyl | Vinyl | Ethyl | |
| 2.3.787 | 3-Trifluormethyl-5-cyano-Phenyl | Vinyl | Ethyl | |
| 2.3.788 | 3-(Methoxycarbonyl)-5-cyano-Phenyl | Vinyl | Ethyl | |
| 2.3.789 | 3-Methoxy-5-cyano-Phenyl | Vinyl | Ethyl | |
| 2.3.790 | 3-Ethoxy-5-cyano-Phenyl | Vinyl | Ethyl | |
| 2.3.791 | 3-nPropyoxy-5-cyano-Phenyl | Vinyl | Ethyl | |
| 2.3.792 | 3-nButoxy-5-cyano-Phenyl | Vinyl | Ethyl | |
| 2.3.793 | 3-isoButoxy-5-cyano-Phenyl | Vinyl | Ethyl | |
| 2.3.794 | 3-Difluormethoxy-5-cyano-Phenyl | Vinyl | Ethyl | |
| 2.3.795 | 3-Trifluormethoxy-5-cyano-Phenyl | Vinyl | Ethyl | |
| 2.3.796 | 3-Nitro-5-cyano-Phenyl | Vinyl | Ethyl | |
| 2.3.797 | 3-Acetoxy-5-cyano-Phenyl | Vinyl | Ethyl | |
| 2.3.798 | 3-Methylsulfanyl-5-cyano-Phenyl | Vinyl | Ethyl | |
| 2.3.799 | 3,5-Di(trifluormethyl)-Phenyl | Vinyl | Ethyl | |
| 2.3.800 | 3-(Methoxycarbonyl)-5-trifluormethyl-Phenyl | Vinyl | Ethyl | |
| 2.3.801 | 3-Methoxy-5-trifluormethyl-Phenyl | Vinyl | Ethyl | |
| 2.3.802 | 3-Ethoxy-5trifluormethyl-Phenyl | Vinyl | Ethyl | |
| 2.3.803 | 3-nPropyoxy-5-trifluormethyl-Phenyl | Vinyl | Ethyl | |
| 2.3.804 | 3-isoButoxy-5-trifluormethyl-Phenyl | Vinyl | Ethyl | |
| 2.3.805 | 3-Difluormethoxy-5-trifluormethyl-Phenyl | Vinyl | Ethyl | |
| 2.3.806 | 3-Trifluormethoxy-5-trifluormethyl-Phenyl | Vinyl | Ethyl | |
| 2.3.807 | 3-Nitro-5-trifluormethyl-Phenyl | Vinyl | Ethyl | |
| 2.3.808 | 3-Acetoxy-5-trifluormethyl-Phenyl | Vinyl | Ethyl | |
| 2.3.809 | 3-Methylsulfanyl-5-trifluormethyl-Phenyl | Vinyl | Ethyl | |
| 2.3.810 | 3,5-Di(methoxycarbonyl)-Phenyl | Vinyl | Ethyl | |
| 2.3.811 | 3-Methoxy-5-(methoxycarbonyl)-Phenyl | Vinyl | Ethyl | |
| 2.3.812 | 3-Ethoxy-5-(methoxycarbonyl)-Phenyl | Vinyl | Ethyl | |
| 2.3.813 | 3-nPropyoxy-5-(methoxycarbonyl)-Phenyl | Vinyl | Ethyl | |
| 2.3.814 | 3-nButoxy-5-(methoxycarbonyl)-Phenyl | Vinyl | Ethyl | |
| 2.3.815 | 3-isoButoxy-5-(methoxycarbonyl)-Phenyl | Vinyl | Ethyl | |
| 2.3.816 | 3-Difluormethoxy-5-(methoxycarbonyl)-Phenyl | Vinyl | Ethyl | |
| 2.3.817 | 3-Trifluormethoxy-5-(methoxycarbonyl)-Phenyl | Vinyl | Ethyl | |
| 2.3.818 | 3-Nitro-5-(methoxycarbonyl)-Phenyl | Vinyl | Ethyl | |
| 2.3.819 | 3-Acetoxy-5-(methoxycarbonyl)-Phenyl | Vinyl | Ethyl | |
| 2.3.820 | 3-Methylsulfanyl-5-(methoxycarbonyl)-Phenyl | Vinyl | Ethyl | |
| 2.3.821 | 3,5-Dimethoxy-Phenyl | Vinyl | Ethyl | |
| 2.3.822 | 3-Ethoxy-5-methoxy-Phenyl | Vinyl | Ethyl | |
| 2.3.823 | 3-nPropyoxy-5-methoxy-Phenyl | Vinyl | Ethyl | |
| 2.3.824 | 3-nButoxy-5-methoxy-Phenyl | Vinyl | Ethyl | |
| 2.3.825 | 3-isoButoxy-5-methoxy-Phenyl | Vinyl | Ethyl | |
| 2.3.826 | 3-Difluormethoxy-5-methoxy-Phenyl | Vinyl | Ethyl | |
| 2.3.827 | 3-Trifluormethoxy-5-methoxy-Phenyl | Vinyl | Ethyl | |
| 2.3.828 | 3-Nitro-5-methoxy-Phenyl | Vinyl | Ethyl | |
| 2.3.829 | 3-Acetoxy-5-methoxy-Phenyl | Vinyl | Ethyl | |
| 2.3.830 | 3-Methylsulfanyl-5-methoxy-Phenyl | Vinyl | Ethyl | |
| 2.3.831 | 3,5-Diethoxy-Phenyl | Vinyl | Ethyl | |
| 2.3.832 | 3-nPropyoxy-5-ethoxy-Phenyl | Vinyl | Ethyl | |
| 2.3.833 | 3-nButoxy-5-ethoxy-Phenyl | Vinyl | Ethyl | |
| 2.3.834 | 3-isoButoxy-5-ethoxy-Phenyl | Vinyl | Ethyl | |
| 2.3.835 | 3-Difluormethoxy-5-ethoxy-Phenyl | Vinyl | Ethyl | |
| 2.3.836 | 3-Trifluormethoxy-5-ethoxy-Phenyl | Vinyl | Ethyl | |
| 2.3.837 | 3-Nitro-5-ethoxy-Phenyl | Vinyl | Ethyl | |
| 2.3.838 | 3-Acetoxy-5-ethoxy-Phenyl | Vinyl | Ethyl | |
| 2.3.839 | 3-Methylsulfanyl-5-ethoxy-Phenyl | Vinyl | Ethyl | |
| 2.3.840 | 3,5-Di(isopropyoxy)-Phenyl | Vinyl | Ethyl | |
| 2.3.841 | 3-nButoxy-5-isopropoxy-Phenyl | Vinyl | Ethyl | |
| 2.3.842 | 3-isoButoxy-5-isopropoxy-Phenyl | Vinyl | Ethyl | |
| 2.3.843 | 3-Difluormethoxy-5-isopropoxy-Phenyl | Vinyl | Ethyl | |
| 2.3.844 | 3-Trifluormethoxy-5-isopropoxy-Phenyl | Vinyl | Ethyl | |
| 2.3.845 | 3-Nitro-5-isopropoxy-Phenyl | Vinyl | Ethyl | |
| 2.3.846 | 3-Acetoxy-5-isopropoxy-Phenyl | Vinyl | Ethyl | |
| 2.3.847 | 3-Methylsulfanyl-5-isopropoxy-Phenyl | Vinyl | Ethyl | |
| 2.3.848 | 3,5-Di(trifluormethoxy)-Phenyl | Vinyl | Ethyl | |
| 2.3.849 | 3-Nitro-5-trifluormethoxy-Phenyl | Vinyl | Ethyl | |
| 2.3.850 | 3-Methylsulfanyl-5-trifluormethoxy-Phenyl | Vinyl | Ethyl | |
| 2.3.851 | 3,5-Bis(difluormethoxy)-Phenyl | Vinyl | Ethyl | |
| 2.3.852 | 3,5-Bis(difluormethoxy)-Phenyl | 1-Methyl vinyl | Ethyl | |
| 2.3.853 | 3,5-Bis(difluormethoxy)-Phenyl | Allyl | Ethyl | |
| 2.3.854 | 3,5-Bis(difluormethoxy)-Phenyl | 1-Chlor vinyl | Ethyl | |
| 2.3.855 | 3-Trifluormethoxy-5-difluormethoxy-Phenyl | Vinyl | Ethyl | |
| 2.3.856 | 3-Nitro-5-difluormethoxy-Phenyl | Vinyl | Ethyl | |
| 2.3.857 | 3-Acetoxy-5-difluormethoxy-Phenyl | Vinyl | Ethyl | |
| 2.3.858 | 3-Methylsulfanyl-5-difluormethoxy-Phenyl | Vinyl | Ethyl | |
| 2.3.859 | 3,5-Bis(acetoxy)-Phenyl | Vinyl | Ethyl | |
| 2.3.860 | 3-Methylsulfanyl-5-acetoxy-Phenyl | Vinyl | Ethyl | |
| 2.3.861 | 3,5-Dinitro-Phenyl | Vinyl | Ethyl | |
| 2.3.862 | 3-Acetoxy-5-nitro-Phenyl | Vinyl | Ethyl | |
| 2.3.863 | 3-Methylsulfanyl-5-nitro-Phenyl | Vinyl | Ethyl | |
| 2.3.864 | 3,5-Di(methylsulfanyl)-Phenyl | Vinyl | Ethyl | |
| 2.3.865 | 3,4-Difluor-Phenyl | Vinyl | Ethyl | |
| 2.3.866 | 3,4-Difluor-Phenyl | 1-Methyl vinyl | Ethyl | |
| 2.3.867 | 3,4-Difluor-Phenyl | Allyl | Ethyl | |
| 2.3.868 | 3,4-Difluor-Phenyl | 1-Chlorvinyl | Ethyl | |
| 2.3.869 | 3,4-Difluor-Phenyl | Ethinyl | Ethyl | |
| 2.3.870 | 3-Chlor-4-fluor-Phenyl | Vinyl | Ethyl | |
| 2.3.871 | 3-Chlor-4-fluor-Phenyl | 1-Methylvinyl | Ethyl | |
| 2.3.872 | 3-Chlor-4-fluor-Phenyl | Allyl | Ethyl | |
| 2.3.873 | 3-Chlor-4-fluor-Phenyl | 1-Chlorvinyl | Ethyl | |
| 2.3.874 | 3-Chlor-4-fluor-Phenyl | Ethinyl | Ethyl | |
| 2.3.875 | 3-Brom-4-fluor-Phenyl | Vinyl | Ethyl | |
| 2.3.876 | 3-Methyl-4-fluor-Phenyl | Vinyl | Ethyl | |
| 2.3.877 | 3-Methyl-4-fluor-Phenyl | 1-Methyl-vinyl | Ethyl | |
| 2.3.878 | 3-Ethyl-4-fluor-Phenyl | Vinyl | Ethyl | |
| 2.3.879 | 3-Cyclopropyl-4-fluor-Phenyl | Vinyl | Ethyl | |
| 2.3.880 | 3-Cyano-4-fluor-Phenyl | Vinyl | Ethyl | |
| 2.3.881 | 3-Methoxy-4-fluor-Phenyl | Vinyl | Ethyl | |
| 2.3.882 | 3-Ethoxy-4-fluor-Phenyl | Vinyl | Ethyl | |
| 2.3.883 | 3-Trifluormethoxy-4-fluor-Phenyl | Vinyl | Ethyl | |
| 2.3.884 | 3-Nitro-4-fluor-Phenyl | Vinyl | Ethyl | |
| 2.3.885 | 3-Fluor-4-chlor-Phenyl | Vinyl | Ethyl | |
| 2.3.886 | 3,4-Dichlor-Phenyl | Vinyl | Ethyl | |
| 2.3.887 | 3-Brom-4-chlor-Phenyl | Vinyl | Ethyl | |
| 2.3.888 | 3-Methyl-4-chlor-Phenyl | Vinyl | Ethyl | |
| 2.3.889 | 3-Cyclopropyl-4-chlor-Phenyl | Vinyl | Ethyl | |
| 2.3.890 | 3-Cyano-4-chlor-Phenyl | Vinyl | Ethyl | |
| 2.3.891 | 3-Trifluormethyl-4-chlor-Phenyl | Vinyl | Ethyl | |
| 2.3.892 | 3-Methoxy-4-chlor-Phenyl | Vinyl | Ethyl | |
| 2.3.893 | 3-Ethoxy-4-chlor-Phenyl | Vinyl | Ethyl | |
| 2.3.894 | 3-Trifluormethoxy-4-chlor-Phenyl | Vinyl | Ethyl | |
| 2.3.895 | 3-Nitro-4-chlor-Phenyl | Vinyl | Ethyl | |
| 2.3.896 | 3-Fluor-4-brom-Phenyl | Vinyl | Ethyl | |
| 2.3.897 | 3-Chlor-4-brom-Phenyl | Vinyl | Ethyl | |
| 2.3.898 | 3,4-Dibrom-Phenyl | Vinyl | Ethyl | |
| 2.3.899 | 3-Methyl-4-brom-Phenyl | Vinyl | Ethyl | |
| 2.3.900 | 3-Ethyl-4-brom-Phenyl | Vinyl | Ethyl | |
| 2.3.901 | 3-Cyclopropyl-4-brom-Phenyl | Vinyl | Ethyl | |
| 2.3.902 | 3-Cyano-4-brom-Phenyl | Vinyl | Ethyl | |
| 2.3.903 | 3-Trifluormethyl-4-brom-Phenyl | Vinyl | Ethyl | |
| 2.3.904 | 3-Methoxy-4-Phenyl | Vinyl | Ethyl | |
| 2.3.905 | 3-Ethoxy-4-brom-Phenyl | Vinyl | Ethyl | |
| 2.3.906 | 3-Trifluormethoxy-4-brom-Phenyl | Vinyl | Ethyl | |
| 2.3.907 | 3-Nitro-4-brom-Phenyl | Vinyl | Ethyl | |
| 2.3.908 | 3-Fluor-4-iod-Phenyl | Vinyl | Ethyl | |
| 2.3.909 | 3-Chlor-4-iod-Phenyl | Vinyl | Ethyl | |
| 2.3.910 | 3-Brom-4-iod-Phenyl | Vinyl | Ethyl | |
| 2.3.911 | 3-Methyl-4-iod-Phenyl | Vinyl | Ethyl | |
| 2.3.912 | 3-Cyclopropyl-4-iod-Phenyl | Vinyl | Ethyl | |
| 2.3.913 | 3-Cyano-4-iod-Phenyl | Vinyl | Ethyl | |
| 2.3.914 | 3-Trifluormethyl-4-iod-Phenyl | Vinyl | Ethyl | |
| 2.3.915 | 3-Methoxy-4-iod-Phenyl | Vinyl | Ethyl | |
| 2.3.916 | 3-Ethoxy-4-iod-Phenyl | Vinyl | Ethyl | |
| 2.3.917 | 3-Trifluormethoxy-4-iod-Phenyl | Vinyl | Ethyl | |
| 2.3.918 | 3-Nitro-4-iod-Phenyl | Vinyl | Ethyl | |
| 2.3.919 | 3-Fluor-4-methyl-Phenyl | Vinyl | Ethyl | |
| 2.3.920 | 3-Chlor-4-methyl-Phenyl | Vinyl | Ethyl | |
| 2.3.921 | 3-Brom-4-methyl-Phenyl | Vinyl | Ethyl | |
| 2.3.922 | 3.4-Dimethyl-Phenyl | Vinyl | Ethyl | |
| 2.3.923 | 3.4-Dimethyl-Phenyl | 1-Methylvinyl | Ethyl | |
| 2.3.924 | 3.4-Dimethyl-Phenyl | Allyl | Ethyl | |
| 2.3.925 | 3.4-Dimethyl-Phenyl | 1-Chlorvinyl | Ethyl | |
| 2.3.926 | 3.4-Dimethyl-Phenyl | Ethinyl | Ethyl | |
| 2.3.927 | 3-Ethyl-4-methyl-Phenyl | Vinyl | Ethyl | |
| 2.3.928 | 3-Cyclopropyl-4-methyl-Phenyl | Vinyl | Ethyl | |
| 2.3.929 | 3-Cyano-4-methyl-Phenyl | Vinyl | Ethyl | |
| 2.3.930 | 3-Trifluormethyl-4-methyl-Phenyl | Vinyl | Ethyl | |
| 2.3.931 | 3-Methoxy-4-methyl-Phenyl | Vinyl | Ethyl | |
| 2.3.932 | 3-Ethoxy-4-methyl-Phenyl | Vinyl | Ethyl | |
| 2.3.933 | 3-Trifluormethoxy-4-methyl-Phenyl | Vinyl | Ethyl | |
| 2.3.934 | 3-Nitro-4-methyl-Phenyl | Vinyl | Ethyl | |
| 2.3.935 | 3-Fluor-4-ethyl-Phenyl | Vinyl | Ethyl | |
| 2.3.936 | 3-Chlor-4-ethyl-Phenyl | Vinyl | Ethyl | |
| 2.3.937 | 3-Brom-4-ethyl-Phenyl | Vinyl | Ethyl | |
| 2.3.938 | 3-Methyl-4-ethyl-Phenyl | Vinyl | Ethyl | |
| 2.3.939 | 3,4-Diethyl-Phenyl | Vinyl | Ethyl | |
| 2.3.940 | 3-Cyclopropyl-4-ethyl-Phenyl | Vinyl | Ethyl | |
| 2.3.941 | 3-Cyano-4-ethyl-Phenyl | Vinyl | Ethyl | |
| 2.3.942 | 3-Trifluormethyl-4-ethyl-Phenyl | Vinyl | Ethyl | |
| 2.3.943 | 3-Methoxy-4-ethyl-Phenyl | Vinyl | Ethyl | |
| 2.3.944 | 3-Ethoxy-4-ethyl-Phenyl | Vinyl | Ethyl | |
| 2.3.945 | 3-Trifluormethoxy-4-ethyl-Phenyl | Vinyl | Ethyl | |
| 2.3.946 | 3-Nitro-4-ethyl-Phenyl | Vinyl | Ethyl | |
| 2.3.947 | 3-Fluor-4-propyl-Phenyl | Vinyl | Ethyl | |
| 2.3.948 | 3-Chlor-4-propyl-Phenyl | Vinyl | Ethyl | |
| 2.3.949 | 3-Brom-4-propyl-Phenyl | Vinyl | Ethyl | |
| 2.3.950 | 3-Methyl-4-propyl-Phenyl | Vinyl | Ethyl | |
| 2.3.951 | 3-Cyclopropyl-4-propyl-Phenyl | Vinyl | Ethyl | |
| 2.3.952 | 3-Cyano-4-propyl-Phenyl | Vinyl | Ethyl | |
| 2.3.953 | 3-Trifluormethyl-4-propyl-Phenyl | Vinyl | Ethyl | |
| 2.3.954 | 3-Methoxy-4-propyl-Phenyl | Vinyl | Ethyl | |
| 2.3.955 | 3-Ethoxy-4-propyl-Phenyl | Vinyl | Ethyl | |
| 2.3.956 | 3-Trifluormethoxy-4-propyl-Phenyl | Vinyl | Ethyl | |
| 2.3.957 | 3-Nitro-4-propyl-Phenyl | Vinyl | Ethyl | |
| 2.3.958 | 3-Fluor-4-isopropyl-Phenyl | Vinyl | Ethyl | |
| 2.3.959 | 3-Chlor-4-isopropyl-Phenyl | Vinyl | Ethyl | |
| 2.3.960 | 3-Brom-4-isopropyl-Phenyl | Vinyl | Ethyl | |
| 2.3.961 | 3-Methyl-4-isopropyl-Phenyl | Vinyl | Ethyl | |
| 2.3.962 | 3-Cyclopropyl-4-isopropyl-Phenyl | Vinyl | Ethyl | |
| 2.3.963 | 3-Cyano-4-isopropyl-Phenyl | Vinyl | Ethyl | |
| 2.3.964 | 3-Trifluormethyl-4-isopropyl-Phenyl | Vinyl | Ethyl | |
| 2.3.965 | 3-Methoxy-4-isopropyl-Phenyl | Vinyl | Ethyl | |
| 2.3.966 | 3-Ethoxy-4-isopropyl-Phenyl | Vinyl | Ethyl | |
| 2.3.967 | 3-Trifluormethoxy-4-isopropyl-Phenyl | Vinyl | Ethyl | |
| 2.3.968 | 3-Nitro-4-isopropyl-Phenyl | Vinyl | Ethyl | |
| 2.3.969 | 3-Fluor-4-tert.butyl-Phenyl | Vinyl | Ethyl | |
| 2.3.970 | 3-Chlor-4-tert.buty-Phenyl | Vinyl | Ethyl | |
| 2.3.971 | 3-Brom-4-tert.butyl-Phenyl | Vinyl | Ethyl | |
| 2.3.972 | 3-Methyl-4-tert.butyl-Phenyl | Vinyl | Ethyl | |
| 2.3.973 | 3-Cyclopropyl-4-tert.butyl-Phenyl | Vinyl | Ethyl | |
| 2.3.974 | 3-Cyano-4-tert.butyl-Phenyl | Vinyl | Ethyl | |
| 2.3.975 | 3-Trifluormethyl-4-tert.butyl-Phenyl | Vinyl | Ethyl | |
| 2.3.976 | 3-Trifluormethyl-4-tert.butyl-Phenyl | 1-Methylvinyl | Ethyl | |
| 2.3.977 | 3-Trifluormethyl-4-tert.butyl-Phenyl | Allyl | Ethyl | |
| 2.3.978 | 3-Trifluormethyl-4-tert.butyl-Phenyl | 1-Chlorvinyl | Ethyl | |
| 2.3.979 | 3-Trifluormethyl-4-tert.butyl-Phenyl | Ethinyl | Ethyl | |
| 2.3.980 | 3-Methoxy-4-tert.butyl-Phenyl | Vinyl | Ethyl | |
| 2.3.981 | 3-Ethoxy-4-tert.butyl-Phenyl | Vinyl | Ethyl | |
| 2.3.982 | 3-Trifluormethoxy-4-tert.butyl-Phenyl | Vinyl | Ethyl | |
| 2.3.983 | 3-Nitro-4-tert.butyl-Phenyl | Vinyl | Ethyl | |
| 2.3.984 | 3-Fluor-4-cyclopropyl-Phenyl | Vinyl | Ethyl | |
| 2.3.985 | 3-Chlor-4-cyclopropyl-Phenyl | Vinyl | Ethyl | |
| 2.3.986 | 3-Brom-4-cyclopropyl-Phenyl | Vinyl | Ethyl | |
| 2.3.987 | 3-Methyl-4-cyclopropyl-Phenyl | Vinyl | Ethyl | |
| 2.3.988 | 3-Cyclopropyl-4-cyclopropyl-Phenyl | Vinyl | Ethyl | |
| 2.3.989 | 3-Cyano-4-cyclopropyl-Phenyl | Vinyl | Ethyl | |
| 2.3.990 | 3-Trifluormethyl-4-cyclopropyl-Phenyl | Vinyl | Ethyl | |
| 2.3.991 | 3-Methoxy-4-cyclopropyl-Phenyl | Vinyl | Ethyl | |
| 2.3.992 | 3-Ethoxy-4-cyclopropyl-Phenyl | Vinyl | Ethyl | |
| 2.3.993 | 3-Trifluormethoxy-4-cyclopropyl-Phenyl | Vinyl | Ethyl | |
| 2.3.994 | 3-Fluor-4-methoxycarbonyl-Phenyl | Vinyl | Ethyl | |
| 2.3.995 | 3-Chlor-4-methoxycarbonyl-Phenyl | Vinyl | Ethyl | |
| 2.3.996 | 3-Brom-4-methoxycarbonyl-Phenyl | Vinyl | Ethyl | |
| 2.3.997 | 3-Methyl-4-methoxycarbonyl-Phenyl | Vinyl | Ethyl | |
| 2.3.998 | 3-Cyclopropyl-4-methoxycarbonyl-Phenyl | Vinyl | Ethyl | |
| 2.3.999 | 3-Cyano-4-methoxycarbonyl-Phenyl | Vinyl | Ethyl | |
| 2.3.1000 | 3-Trifluormethyl-4-methoxycarbonyl-Phenyl | Vinyl | Ethyl | |
| 2.3.1001 | 3-Methoxy-4-methoxycarbonyl-Phenyl | Vinyl | Ethyl | |
| 2.3.1002 | 3-Ethoxy-4-methoxycarbonyl-Phenyl | Vinyl | Ethyl | |
| 2.3.1003 | 3-Trifluormethoxy-4-methoxycarbonyl-Phenyl | Vinyl | Ethyl | |
| 2.3.1004 | 3-Nitro-4-methoxycarbonyl-Phenyl | Vinyl | Ethyl | |
| 2.3.1005 | 3-Fluor-4-cyano-Phenyl | Vinyl | Ethyl | |
| 2.3.1006 | 3-Chlor-4-cyano-Phenyl | Vinyl | Ethyl | |
| 2.3.1007 | 3-Brom-4-cyano-Phenyl | Vinyl | Ethyl | |
| 2.3.1008 | 3-Methyl-4-cyano-Phenyl | Vinyl | Ethyl | |
| 2.3.1009 | 3-Cyclopropyl-4-cyano-Phenyl | Vinyl | Ethyl | |
| 2.3.1010 | 3,4-Dicyano-Phenyl | Vinyl | Ethyl | |
| 2.3.1011 | 3-Trifluormethyl-4-cyano-Phenyl | Vinyl | Ethyl | |
| 2.3.1012 | 3-Trifluormethyl-4-cyano-Phenyl | 1-Methylvinyl | Ethyl | |
| 2.3.1013 | 3-Trifluormethyl-4-cyano-Phenyl | Allyl | Ethyl | |
| 2.3.1014 | 3-Trifluormethyl-4-cyano-Phenyl | 1-Chlorvinyl | Ethyl | |
| 2.3.1015 | 3-Trifluormethyl-4-cyano-Phenyl | Ethinyl | Ethyl | |
| 2.3.1016 | 3-Methoxy-4-cyano-Phenyl | Vinyl | Ethyl | |
| 2.3.1017 | 3-Ethoxy-4-cyano-Phenyl | Vinyl | Ethyl | |
| 2.3.1018 | 3-Trifluormethoxy-4-cyano-Phenyl | Vinyl | Ethyl | |
| 2.3.1019 | 3-Nitro-4-cyano-Phenyl | Vinyl | Ethyl | |
| 2.3.1020 | 3-Fluor-4-methoxy-Phenyl | Vinyl | Ethyl | |
| 2.3.1021 | 3-Chlor-4-methoxy-Phenyl | Vinyl | Ethyl | |
| 2.3.1022 | 3-Brom-4-methoxy-Phenyl | Vinyl | Ethyl | |
| 2.3.1023 | 3-Methyl-4-methoxy-Phenyl | Vinyl | Ethyl | |
| 2.3.1024 | 3-Cyclopropyl-4-methoxy-Phenyl | Vinyl | Ethyl | |
| 2.3.1025 | 3-Cyano-4-methoxy-Phenyl | Vinyl | Ethyl | |
| 2.3.1026 | 3-Trifluormethyl-4-methoxy-Phenyl | Vinyl | Ethyl | |
| 2.3.1027 | 3,4-Dimethoxy-Phenyl | Vinyl | Ethyl | |
| 2.3.1028 | 3-Ethoxy-4-methoxy-Phenyl | Vinyl | Ethyl | |
| 2.3.1029 | 3-Trifluormethoxy-4-methoxy-Phenyl | Vinyl | Ethyl | |
| 2.3.1030 | 3-Nitro-4-methoxy-Phenyl | Vinyl | Ethyl | |
| 2.3.1031 | 3-Fluor-4-ethoxy-Phenyl | Vinyl | Ethyl | |
| 2.3.1032 | 3-Chlor-4-ethoxy-Phenyl | Vinyl | Ethyl | |
| 2.3.1033 | 3-Chlor-4-ethoxy-Phenyl | 1-Methylvinyl | Ethyl | |
| 2.3.1034 | 3-Chlor-4-ethoxy-Phenyl | Allyl | Ethyl | |
| 2.3.1035 | 3-Chlor-4-ethoxy-Phenyl | 1-Chlorvinyl | Ethyl | |
| 2.3.1036 | 3-Chlor-4-ethoxy-Phenyl | Ethinyl | Ethyl | |
| 2.3.1037 | 3-Brom-4-ethoxy-Phenyl | Vinyl | Ethyl | |
| 2.3.1038 | 3-Methyl-4-ethoxy-Phenyl | Vinyl | Ethyl | |
| 2.3.1039 | 3-Cyclopropyl-4-ethoxy-Phenyl | Vinyl | Ethyl | |
| 2.3.1040 | 3-Cyano-4-ethoxy-Phenyl | Vinyl | Ethyl | |
| 2.3.1041 | 3-Trifluormethyl-4-ethoxy-Phenyl | Vinyl | Ethyl | |
| 2.3.1042 | 3-Methoxy-4-ethoxy-Phenyl | Vinyl | Ethyl | |
| 2.3.1043 | 2,4-Diethoxy-Phenyl | Vinyl | Ethyl | |
| 2.3.1044 | 3-Trifluormethoxy-4-ethoxy-Phenyl | Vinyl | Ethyl | |
| 2.3.1045 | 3-Nitro-4-ethoxy-Phenyl | Vinyl | Ethyl | |
| 2.3.1046 | 3-Fluor-4-isopropoxy-Phenyl | Vinyl | Ethyl | |
| 2.3.1047 | 3-Chlor-4-isopropoxy-Phenyl | Vinyl | Ethyl | |
| 2.3.1048 | 3-Brom-4-isopropoxy-Phenyl | Vinyl | Ethyl | |
| 2.3.1049 | 3-Methyl-4-isopropoxy-Phenyl | Vinyl | Ethyl | |
| 2.3.1050 | 3-Cyclopropyl-4-isopropoxy-Phenyl | Vinyl | Ethyl | |
| 2.3.1051 | 3-Cyano-4-isopropoxy-Phenyl | Vinyl | Ethyl | |
| 2.3.1052 | 3-Trifluormethyl-4-isopropoxy-Phenyl | Vinyl | Ethyl | |
| 2.3.1053 | 3-Methoxy-4-isopropoxy-Phenyl | Vinyl | Ethyl | |
| 2.3.1054 | 3-Ethoxy-4-isopropoxy-Phenyl | Vinyl | Ethyl | |
| 2.3.1055 | 3-Trifluormethoxy-4-isopropoxy-Phenyl | Vinyl | Ethyl | |
| 2.3.1056 | 3-Nitro-4-isopropoxy-Phenyl | Vinyl | Ethyl | |
| 2.3.1057 | 3-Fluor-4-trifluormethoxy-Phenyl | Vinyl | Ethyl | |
| 2.3.1058 | 3-Chlor-4-trifluormethoxy-Phenyl | Vinyl | Ethyl | |
| 2.3.1059 | 3-Brom-4-trifluormethoxy-Phenyl | Vinyl | Ethyl | |
| 2.3.1060 | 3-Methyl-4-trifluormethoxy-Phenyl | Vinyl | Ethyl | |
| 2.3.1061 | 3-Cyclopropyl-4-trifluormethoxy-Phenyl | Vinyl | Ethyl | |
| 2.3.1062 | 3-Cyano-4-trifluormethoxy-Phenyl | Vinyl | Ethyl | |
| 2.3.1063 | 3-Trifluormethyl-4-trifluormethoxy-Phenyl | Vinyl | Ethyl | |
| 2.3.1064 | 3-Methoxy-4-trifluormethoxy-Phenyl | Vinyl | Ethyl | |
| 2.3.1065 | 3-Ethoxy-4-trifluormethoxy-Phenyl | Vinyl | Ethyl | |
| 2.3.1066 | 3,4-Bis(trifluormethoxy)-Phenyl | Vinyl | Ethyl | |
| 2.3.1067 | 3-Nitro-4-trifluormethoxy-Phenyl | Vinyl | Ethyl | |
| 2.3.1068 | 3-Fluor-4-difluormethoxy-Phenyl | Vinyl | Ethyl | |
| 2.3.1069 | 3-Chlor-4-difluormethoxy-Phenyl | Vinyl | Ethyl | |
| 2.3.1070 | 3-Brom-4-difluormethoxy-Phenyl | Vinyl | Ethyl | |
| 2.3.1071 | 3-Methyl-4-difluormethoxy-Phenyl | Vinyl | Ethyl | |
| 2.3.1072 | 3-Cyclopropyl-4-difluormethoxy-Phenyl | Vinyl | Ethyl | |
| 2.3.1073 | 3-Cyano-4-difluormethoxy-Phenyl | Vinyl | Ethyl | |
| 2.3.1074 | 3-Trifluormethyl-4-difluormethoxy-Phenyl | Vinyl | Ethyl | |
| 2.3.1075 | 3-Methoxy-4-difluormethoxy-Phenyl | Vinyl | Ethyl | |
| 2.3.1076 | 3-Ethoxy-4-difluormethoxy-Phenyl | Vinyl | Ethyl | |
| 2.3.1077 | 3-Trifluormethoxy-4-difluormethoxy-Phenyl | Vinyl | Ethyl | |
| 2.3.1078 | 3-Nitro-4-difluormethoxy-Phenyl | Vinyl | Ethyl | |
| 2.3.1079 | 3-Fluor-4-nitro-Phenyl | Vinyl | Ethyl | |
| 2.3.1080 | 3-Chlor-4-nitro-Phenyl | Vinyl | Ethyl | |
| 2.3.1081 | 3-Brom-4-nitro-Phenyl | Vinyl | Ethyl | |
| 2.3.1082 | 3-Methyl-4-nitro-Phenyl | Vinyl | Ethyl | |
| 2.3.1083 | 3-Cyclopropyl-4-nitro-Phenyl | Vinyl | Ethyl | |
| 2.3.1084 | 3-Cyano-4-nitro-Phenyl | Vinyl | Ethyl | |
| 2.3.1085 | 3-Trifluormethyl-4-nitro-Phenyl | Vinyl | Ethyl | |
| 2.3.1086 | 3-Methoxy-4-nitro-Phenyl | Vinyl | Ethyl | |
| 2.3.1087 | 3-Ethoxy-4-nitro-Phenyl | Vinyl | Ethyl | |
| 2.3.1088 | 3-Trifluormethoxy-4-nitro-Phenyl | Vinyl | Ethyl | |
| 2.3.1089 | 3-Fluor-4-methylsulfanylPhenyl | Vinyl | Ethyl | |
| 2.3.1090 | 3-Chlor-4-methylsulfanyl-Phenyl | Vinyl | Ethyl | |
| 2.3.1091 | 3-Brom-4-methylsulfanyl-Phenyl | Vinyl | Ethyl | |
| 2.3.1092 | 3-Methyl-4-methylsulfanyl-Phenyl | Vinyl | Ethyl | |
| 2.3.1093 | 3-Cyclopropyl-4-methylsulfanyl-Phenyl | Vinyl | Ethyl | |
| 2.3.1094 | 3-Cyano-4-methylsulfanyl-Phenyl | Vinyl | Ethyl | |
| 2.3.1095 | 3-Trifluormethyl-4-methylsulfanyl-Phenyl | Vinyl | Ethyl | |
| 2.3.1096 | 3-Methoxy-4-methylsulfanyl-Phenyl | Vinyl | Ethyl | |
| 2.3.1097 | 3-Ethoxy-4-methylsulfanyl-Phenyl | Vinyl | Ethyl | |
| 2.3.1098 | 3-Trifluormethoxy-4-methylsulfanyl-Phenyl | Vinyl | Ethyl | |
| 2.3.1099 | 3-Nitro-4-methylsulfanyl-Phenyl | Vinyl | Ethyl | |
| 2.3.1100 | 3,6-Difluor-Phenyl | Vinyl | Ethyl | |
| 2.3.1101 | 3,6-Difluor-Phenyl | 1-Methylvinyl | Ethyl | |
| 2.3.1102 | 3,6-Difluor-Phenyl | Allyl | Ethyl | |
| 2.3.1103 | 3,6-Difluor-Phenyl | 1-Chlorvinyl | Ethyl | |
| 2.3.1104 | 3,6-Difluor-Phenyl | Ethinyl | Ethyl | |
| 2.3.1105 | 3-Chlor-6-fluor-Phenyl | Vinyl | Ethyl | |
| 2.3.1106 | 3-Brom-6-fluor-Phenyl | Vinyl | Ethyl | |
| 2.3.1107 | 3-Methyl-6-fluor-Phenyl | Vinyl | Ethyl | |
| 2.3.1108 | 3-Cyclopropyl-6-fluor-Phenyl | Vinyl | Ethyl | |
| 2.3.1109 | 3-Cyano-6-fluor-Phenyl | Vinyl | Ethyl | |
| 2.3.1110 | 3-Methoxy-6-fluor-Phenyl | Vinyl | Ethyl | |
| 2.3.1111 | 3-Ethoxy-6-fluor-Phenyl | Vinyl | Ethyl | |
| 2.3.1112 | 3-Trifluormethoxy-6-fluor-Phenyl | Vinyl | Ethyl | |
| 2.3.1113 | 3-Nitro-6-fluor-Phenyl | Vinyl | Ethyl | |
| 2.3.1114 | 3-Fluor-6-chlor-Phenyl | Vinyl | Ethyl | |
| 2.3.1115 | 3-Fluor-6-chlor-Phenyl | 1-Methylvinyl | Ethyl | |
| 2.3.1116 | 3-Fluor-6-chlor-Phenyl | Allyl | Ethyl | |
| 2.3.1117 | 3-Fluor-6-chlor-Phenyl | 1-Chlorvinyl | Ethyl | |
| 2.3.1118 | 3-Fluor-6-chlor-Phenyl | Ethinyl | Ethyl | |
| 2.3.1119 | 3,6-Dichlor-Phenyl | Vinyl | Ethyl | |
| 2.3.1120 | 3,6-Dichlor-Phenyl | 1-Methylvinyl | Ethyl | |
| 2.3.1121 | 3,6-Dichlor-Phenyl | Allyl | Ethyl | |
| 2.3.1122 | 3,6-Dichlor-Phenyl | 1-Chlorvinyl | Ethyl | |
| 2.3.1123 | 3,6-Dichlor-Phenyl | Ethinyl | Ethyl | |
| 2.3.1124 | 3-Brom-6-chlor-Phenyl | Vinyl | Ethyl | |
| 2.3.1125 | 3-Methyl-6-chlor-Phenyl | Vinyl | Ethyl | |
| 2.3.1126 | 3-Cyclopropyl-6-chlor-Phenyl | Vinyl | Ethyl | |
| 2.3.1127 | 3-Cyano-6-chlor-Phenyl | Vinyl | Ethyl | |
| 2.3.1128 | 3-Trifluormethyl-6-chlor-Phenyl | Vinyl | Ethyl | |
| 2.3.1129 | 3-Methoxy-6-chlor-Phenyl | Vinyl | Ethyl | |
| 2.3.1130 | 3-Ethoxy-6-chlor-Phenyl | Vinyl | Ethyl | |
| 2.3.1131 | 3-Trifluormethoxy-6-chlor-Phenyl | Vinyl | Ethyl | |
| 2.3.1132 | 3-Nitro-6-chlor-Phenyl | Vinyl | Ethyl | |
| 2.3.1133 | 3-Fluor-6-brom-Phenyl | Vinyl | Ethyl | |
| 2.3.1134 | 3-Chlor-6-brom-Phenyl | Vinyl | Ethyl | |
| 2.3.1135 | 3,6-Dibrom-Phenyl | Vinyl | Ethyl | |
| 2.3.1136 | 3-Methyl-6-brom-Phenyl | Vinyl | Ethyl | |
| 2.3.1137 | 3-Cyclopropyl-6-brom-Phenyl | Vinyl | Ethyl | |
| 2.3.1138 | 3-Cyano-6-brom-Phenyl | Vinyl | Ethyl | |
| 2.3.1139 | 3-Trifluormethyl-6-brom-Phenyl | Vinyl | Ethyl | |
| 2.3.1140 | 3-Methoxy-6-Phenyl | Vinyl | Ethyl | |
| 2.3.1141 | 3-Ethoxy-6-brom-Phenyl | Vinyl | Ethyl | |
| 2.3.1142 | 3-Trifluormethoxy-6-brom-Phenyl | Vinyl | Ethyl | |
| 2.3.1143 | 3-Nitro-6-brom-Phenyl | Vinyl | Ethyl | |
| 2.3.1144 | 3-Fluor-6-iod-Phenyl | Vinyl | Ethyl | |
| 2.3.1145 | 3-Chlor-6-iod-Phenyl | Vinyl | Ethyl | |
| 2.3.1146 | 3-Brom-6-iod-Phenyl | Vinyl | Ethyl | |
| 2.3.1147 | 3-Methyl-6-iod-Phenyl | Vinyl | Ethyl | |
| 2.3.1148 | 3-Cyclopropyl-6-iod-Phenyl | Vinyl | Ethyl | |
| 2.3.1149 | 3-Cyano-6-iod-Phenyl | Vinyl | Ethyl | |
| 2.3.1150 | 3-Trifluormethyl-6-iod-Phenyl | Vinyl | Ethyl | |
| 2.3.1151 | 3-Methoxy-6-iod-Phenyl | Vinyl | Ethyl | |
| 2.3.1152 | 3-Ethoxy-6-iod-Phenyl | Vinyl | Ethyl | |
| 2.3.1153 | 3-Trifluormethoxy-6-iod-Phenyl | Vinyl | Ethyl | |
| 2.3.1154 | 3-Nitro-6-iod-Phenyl | Vinyl | Ethyl | |
| 2.3.1155 | 3-Fluor-6-methyl-Phenyl | Vinyl | Ethyl | |
| 2.3.1156 | 3-Chlor-6-methyl-Phenyl | Vinyl | Ethyl | |
| 2.3.1157 | 3-Brom-6-methyl-Phenyl | Vinyl | Ethyl | |
| 2.3.1158 | 3.6-Dimethyl-Phenyl | Vinyl | Ethyl | |
| 2.3.1159 | 3-Ethyl-6-methyl-Phenyl | Vinyl | Ethyl | |
| 2.3.1160 | 3-Cyclopropyl-6-methyl-Phenyl | Vinyl | Ethyl | |
| 2.3.1161 | 3-Cyano-6-methyl-Phenyl | Vinyl | Ethyl | |
| 2.3.1162 | 3-Trifluormethyl-6-methyl-Phenyl | Vinyl | Ethyl | |
| 2.3.1163 | 3-Methoxy-6-methyl-Phenyl | Vinyl | Ethyl | |
| 2.3.1164 | 3-Ethoxy-6-methyl-Phenyl | Vinyl | Ethyl | |
| 2.3.1165 | 3-Trifluormethoxy-6-methyl-Phenyl | Vinyl | Ethyl | |
| 2.3.1166 | 3-Nitro-6-methyl-Phenyl | Vinyl | Ethyl | |
| 2.3.1167 | 3-Fluor-6-ethyl-Phenyl | Vinyl | Ethyl | |
| 2.3.1168 | 3-Chlor-6-ethyl-Phenyl | Vinyl | Ethyl | |
| 2.3.1169 | 3-Brom-6-ethyl-Phenyl | Vinyl | Ethyl | |
| 2.3.1170 | 3-Methyl-6-ethyl-Phenyl | Vinyl | Ethyl | |
| 2.3.1171 | 3,6-Diethyl-Phenyl | Vinyl | Ethyl | |
| 2.3.1172 | 3-Cyclopropyl-6-ethyl-Phenyl | Vinyl | Ethyl | |
| 2.3.1173 | 3-Cyano-6-ethyl-Phenyl | Vinyl | Ethyl | |
| 2.3.1174 | 3-Trifluormethyl-6-ethyl-Phenyl | Vinyl | Ethyl | |
| 2.3.1175 | 3-Methoxy-6-ethyl-Phenyl | Vinyl | Ethyl | |
| 2.3.1176 | 3-Ethoxy-6-ethyl-Phenyl | Vinyl | Ethyl | |
| 2.3.1177 | 3-Trifluormethoxy-6-ethyl-Phenyl | Vinyl | Ethyl | |
| 2.3.1178 | 3-Nitro-6-ethyl-Phenyl | Vinyl | Ethyl | |
| 2.3.1179 | 3-Fluor-6-propyl-Phenyl | Vinyl | Ethyl | |
| 2.3.1180 | 3-Chlor-6-propyl-Phenyl | Vinyl | Ethyl | |
| 2.3.1181 | 3-Brom-6-propyl-Phenyl | Vinyl | Ethyl | |
| 2.3.1182 | 3-Methyl-6-propyl-Phenyl | Vinyl | Ethyl | |
| 2.3.1183 | 3-Cyclopropyl-6-propyl-Phenyl | Vinyl | Ethyl | |
| 2.3.1184 | 3-Cyano-6-propyl-Phenyl | Vinyl | Ethyl | |
| 2.3.1185 | 3-Trifluormethyl-6-propyl-Phenyl | Vinyl | Ethyl | |
| 2.3.1186 | 3-Methoxy-6-propyl-Phenyl | Vinyl | Ethyl | |
| 2.3.1187 | 3-Ethoxy-6-propyl-Phenyl | Vinyl | Ethyl | |
| 2.3.1188 | 3-Trifluormethoxy-6-propyl-Phenyl | Vinyl | Ethyl | |
| 2.3.1189 | 3-Nitro-6-propyl-Phenyl | Vinyl | Ethyl | |
| 2.3.1190 | 3-Fluor-6-isopropyl-Phenyl | Vinyl | Ethyl | |
| 2.3.1191 | 3-Chlor-6-isopropyl-Phenyl | Vinyl | Ethyl | |
| 2.3.1192 | 3-Brom-6-isopropyl-Phenyl | Vinyl | Ethyl | |
| 2.3.1193 | 3-Methyl-6-isopropyl-Phenyl | Vinyl | Ethyl | |
| 2.3.1194 | 3-Cyclopropyl-6-isopropyl-Phenyl | Vinyl | Ethyl | |
| 2.3.1195 | 3-Cyano-6-isopropyl-Phenyl | Vinyl | Ethyl | |
| 2.3.1196 | 3-Trifluormethyl-6-isopropyl-Phenyl | Vinyl | Ethyl | |
| 2.3.1197 | 3-Methoxy-6-isopropyl-Phenyl | Vinyl | Ethyl | |
| 2.3.1198 | 3-Ethoxy-6-isopropyl-Phenyl | Vinyl | Ethyl | |
| 2.3.1199 | 3-Trifluormethoxy-6-isopropyl-Phenyl | Vinyl | Ethyl | |
| 2.3.1200 | 3-Nitro-6-isopropyl-Phenyl | Vinyl | Ethyl | |
| 2.3.1201 | 3-Fluor-6-tert.butyl-Phenyl | Vinyl | Ethyl | |
| 2.3.1202 | 3-Chlor-6-tert.buty -Phenyl | Vinyl | Ethyl | |
| 2.3.1203 | 3-Brom-6-tert.butyl-Phenyl | Vinyl | Ethyl | |
| 2.3.1204 | 3-Methyl-6-tert.butyl-Phenyl | Vinyl | Ethyl | |
| 2.3.1205 | 3-Ethyl-6-tert.butyl-Phenyl | Vinyl | Ethyl | |
| 2.3.1206 | 3-Cyclopropyl-6-tert.butyl-Phenyl | Vinyl | Ethyl | |
| 2.3.1207 | 3-Cyano-6-tert.butyl-Phenyl | Vinyl | Ethyl | |
| 2.3.1208 | 3-Trifluormethyl-6-tert.butyl-Phenyl | Vinyl | Ethyl | |
| 2.3.1209 | 3-Methoxy-6-tert.butyl-Phenyl | Vinyl | Ethyl | |
| 2.3.1210 | 3-Ethoxy-6-tert.butyl-Phenyl | Vinyl | Ethyl | |
| 2.3.1211 | 3-Trifluormethoxy-6-tert.butyl-Phenyl | Vinyl | Ethyl | |
| 2.3.1212 | 3-Nitro-6-tert.butyl-Phenyl | Vinyl | Ethyl | |
| 2.3.1213 | 3-Fluor-6-cyclopropyl-Phenyl | Vinyl | Ethyl | |
| 2.3.1214 | 3-Chlor-6-cyclopropyl-Phenyl | Vinyl | Ethyl | |
| 2.3.1215 | 3-Brom-6-cyclopropyl-Phenyl | Vinyl | Ethyl | |
| 2.3.1216 | 3-Methyl-6-cyclopropyl-Phenyl | Vinyl | Ethyl | |
| 2.3.1217 | 3-Cyclop ropyl-6-cyclopropyl-Phenyl | Vinyl | Ethyl | |
| 2.3.1218 | 3-Cyano-6-cyclopropyl-Phenyl | Vinyl | Ethyl | |
| 2.3.1219 | 3-Trifluormethyl-6-cyclopropyl-Phenyl | Vinyl | Ethyl | |
| 2.3.1220 | 3-Methoxy-6-cyclopropyl-Phenyl | Vinyl | Ethyl | |
| 2.3.1221 | 3-Ethoxy-6-cyclopropyl-Phenyl | Vinyl | Ethyl | |
| 2.3.1222 | 3-Trifluormethoxy-6-cyclopropyl-Phenyl | Vinyl | Ethyl | |
| 2.3.1223 | 3-Fluor-6-methoxycarbonyl-Phenyl | Vinyl | Ethyl | |
| 2.3.1224 | 3-Chlor-6-methoxycarbonyl-Phenyl | Vinyl | Ethyl | |
| 2.3.1225 | 3-Brom-6-methoxycarbonyl-Phenyl | Vinyl | Ethyl | |
| 2.3.1226 | 3-Methyl-6-methoxycarbonyl-Phenyl | Vinyl | Ethyl | |
| 2.3.1227 | 3-Cyclopropyl-6-methoxycarbonyl-Phenyl | Vinyl | Ethyl | |
| 2.3.1228 | 3-Cyano-6-methoxycarbonyl-Phenyl | Vinyl | Ethyl | |
| 2.3.1229 | 3-Trifluormethyl-6-methoxycarbonyl-Phenyl | Vinyl | Ethyl | |
| 2.3.1230 | 3-Methoxy-6-methoxycarbonyl-Phenyl | Vinyl | Ethyl | |
| 2.3.1231 | 3-Ethoxy-6-methoxycarbonyl-Phenyl | Vinyl | Ethyl | |
| 2.3.1232 | 3-Trifluormethoxy-6-methoxycarbonyl-Phenyl | Vinyl | Ethyl | |
| 2.3.1233 | 3-Nitro-6-methoxycarbonyl-Phenyl | Vinyl | Ethyl | |
| 2.3.1234 | 3-Fluor-6-cyano-Phenyl | Vinyl | Ethyl | |
| 2.3.1235 | 3-Chlor-6-cyano-Phenyl | Vinyl | Ethyl | |
| 2.3.1236 | 3-Brom-6-cyano-Phenyl | Vinyl | Ethyl | |
| 2.3.1237 | 3-Methyl-6-cyano-Phenyl | Vinyl | Ethyl | |
| 2.3.1238 | 3-Cyclopropyl-6-cyano-Phenyl | Vinyl | Ethyl | |
| 2.3.1239 | 3-Cyano-6-cyano-Phenyl | Vinyl | Ethyl | |
| 2.3.1240 | 3-Trifluormethyl-6-cyano-Phenyl | Vinyl | Ethyl | |
| 2.3.1241 | 3-Methoxy-6-cyano-Phenyl | Vinyl | Ethyl | |
| 2.3.1242 | 3-Ethoxy-6-cyano-Phenyl | Vinyl | Ethyl | |
| 2.3.1243 | 3-Trifluormethoxy-6-cyano-Phenyl | Vinyl | Ethyl | |
| 2.3.1244 | 3-Nitro-6-cyano-Phenyl | Vinyl | Ethyl | |
| 2.3.1245 | 3-Fluor-6-methoxy-Phenyl | Vinyl | Ethyl | |
| 2.3.1246 | 3-Chlor-6-methoxy-Phenyl | Vinyl | Ethyl | |
| 2.3.1247 | 3-Brom-6-methoxy-Phenyl | Vinyl | Ethyl | |
| 2.3.1248 | 3-Methyl-6-methoxy-Phenyl | Vinyl | Ethyl | |
| 2.3.1249 | 3-Cyclopropyl-6-methoxy-Phenyl | Vinyl | Ethyl | |
| 2.3.1250 | 3-Cyano-6-methoxy-Phenyl | Vinyl | Ethyl | |
| 2.3.1251 | 3-Trifluormethyl-6-methoxy-Phenyl | Vinyl | Ethyl | |
| 2.3.1252 | 3,6-Dimethoxy-Phenyl | Vinyl | Ethyl | |
| 2.3.1253 | 3-Ethoxy-6-methoxy-Phenyl | Vinyl | Ethyl | |
| 2.3.1254 | 3-Trifluormethoxy-6-methoxy-Phenyl | Vinyl | Ethyl | |
| 2.3.1255 | 3-Nitro-6-methoxy-Phenyl | Vinyl | Ethyl | |
| 2.3.1256 | 3-Fluor-6-ethoxy-Phenyl | Vinyl | Ethyl | |
| 2.3.1257 | 3-Chlor-6-ethoxy-Phenyl | Vinyl | Ethyl | |
| 2.3.1258 | 3-Brom-6-ethoxy-Phenyl | Vinyl | Ethyl | |
| 2.3.1259 | 3-Methyl-6-ethoxy-Phenyl | Vinyl | Ethyl | |
| 2.3.1260 | 3-Cyclopropyl-6-ethoxy-Phenyl | Vinyl | Ethyl | |
| 2.3.1261 | 3-Cyano-6-ethoxy-Phenyl | Vinyl | Ethyl | |
| 2.3.1262 | 3-Trifluormethyl-6-ethoxy-Phenyl | Vinyl | Ethyl | |
| 2.3.1263 | 3-Methoxy-6-ethoxy-Phenyl | Vinyl | Ethyl | |
| 2.3.1264 | 2,6-Diethoxy-Phenyl | Vinyl | Ethyl | |
| 2.3.1265 | 3-Trifluormethoxy-6-ethoxy-Phenyl | Vinyl | Ethyl | |
| 2.3.1266 | 3-Nitro-6-ethoxy-Phenyl | Vinyl | Ethyl | |
| 2.3.1267 | 3-Fluor-6-isopropoxy-Phenyl | Vinyl | Ethyl | |
| 2.3.1268 | 3-Chlor-6-isopropoxy-Phenyl | Vinyl | Ethyl | |
| 2.3.1269 | 3-Brom-6-isopropoxy-Phenyl | Vinyl | Ethyl | |
| 2.3.1270 | 3-Methyl-6-isopropoxy-Phenyl | Vinyl | Ethyl | |
| 2.3.1271 | 3-Cyclopropyl-6-isopropoxy-Phenyl | Vinyl | Ethyl | |
| 2.3.1272 | 3-Cyano-6-isopropoxy-Phenyl | Vinyl | Ethyl | |
| 2.3.1273 | 3-Trifluormethyl-6-isopropoxy-Phenyl | Vinyl | Ethyl | |
| 2.3.1274 | 3-Methoxy-6-isopropoxy-Phenyl | Vinyl | Ethyl | |
| 2.3.1275 | 3-Ethoxy-6-isopropoxy-Phenyl | Vinyl | Ethyl | |
| 2.3.1276 | 3-Trifluormethoxy-6-isopropoxy-Phenyl | Vinyl | Ethyl | |
| 2.3.1277 | 3-Nitro-6-isopropoxy-Phenyl | Vinyl | Ethyl | |
| 2.3.1278 | 3-Fluor-6-trifluormethoxy-Phenyl | Vinyl | Ethyl | |
| 2.3.1279 | 3-Chlor-6-trifluormethoxy-Phenyl | Vinyl | Ethyl | |
| 2.3.1280 | 3-Brom-6-trifluormethoxy-Phenyl | Vinyl | Ethyl | |
| 2.3.1281 | 3-Methyl-6-trifluormethoxy-Phenyl | Vinyl | Ethyl | |
| 2.3.1282 | 3-Cyclopropyl-6-trifluormethoxy-Phenyl | Vinyl | Ethyl | |
| 2.3.1283 | 3-Cyano-6-trifluormethoxy-Phenyl | Vinyl | Ethyl | |
| 2.3.1284 | 3-Trifluormethyl-6-trifluormethoxy-Phenyl | Vinyl | Ethyl | |
| 2.3.1285 | 3-Methoxy-6-trifluormethoxy-Phenyl | Vinyl | Ethyl | |
| 2.3.1286 | 3-Ethoxy-6-trifluormethoxy-Phenyl | Vinyl | Ethyl | |
| 2.3.1287 | 3,6-Bis(trifluormethoxy)-Phenyl | Vinyl | Ethyl | |
| 2.3.1288 | 3-Nitro-6-trifluormethoxy-Phenyl | Vinyl | Ethyl | |
| 2.3.1289 | 3-Fluor-6-difluormethoxy-Phenyl | Vinyl | Ethyl | |
| 2.3.1290 | 3-Chlor-6-difluormethoxy-Phenyl | Vinyl | Ethyl | |
| 2.3.1291 | 3-Brom-6-difluormethoxy-Phenyl | Vinyl | Ethyl | |
| 2.3.1292 | 3-Methyl-6-difluormethoxy-Phenyl | Vinyl | Ethyl | |
| 2.3.1293 | 3-Cyclopropyl-6-difluormethoxy-Phenyl | Vinyl | Ethyl | |
| 2.3.1294 | 3-Cyano-6-difluormethoxy-Phenyl | Vinyl | Ethyl | |
| 2.3.1295 | 3-Trifluormethyl-6-difluormethoxy-Phenyl | Vinyl | Ethyl | |
| 2.3.1296 | 3-Methoxy-6-difluormethoxy-Phenyl | Vinyl | Ethyl | |
| 2.3.1297 | 3-Ethoxy-6-difluormethoxy-Phenyl | Vinyl | Ethyl | |
| 2.3.1298 | 3-Trifluormethoxy-6-difluormethoxy-Phenyl | Vinyl | Ethyl | |
| 2.3.1299 | 3-Nitro-6-difluormethoxy-Phenyl | Vinyl | Ethyl | |
| 2.3.1300 | 3-Fluor-6-nitro-Phenyl | Vinyl | Ethyl | |
| 2.3.1301 | 3-Chlor-6-nitro-Phenyl | Vinyl | Ethyl | |
| 2.3.1302 | 3-Brom-6-nitro-Phenyl | Vinyl | Ethyl | |
| 2.3.1303 | 3-Methyl-6-nitro-Phenyl | Vinyl | Ethyl | |
| 2.3.1304 | 3-Cyclopropyl-6-nitro-Phenyl | Vinyl | Ethyl | |
| 2.3.1305 | 3-Cyano-6-nitro-Phenyl | Vinyl | Ethyl | |
| 2.3.1306 | 3-Trifluormethyl-6-nitro-Phenyl | Vinyl | Ethyl | |
| 2.3.1307 | 3-Methoxy-6-nitro-Phenyl | Vinyl | Ethyl | |
| 2.3.1308 | 3-Ethoxy-6-nitro-Phenyl | Vinyl | Ethyl | |
| 2.3.1309 | 3-Trifluormethoxy-6-nitro-Phenyl | Vinyl | Ethyl | |
| 2.3.1310 | 3-Fluor-6-methylsulfanylPhenyl | Vinyl | Ethyl | |
| 2.3.1311 | 3-Chlor-6-methylsulfanyl-Phenyl | Vinyl | Ethyl | |
| 2.3.1312 | 3-Brom-6-methylsulfanyl-Phenyl | Vinyl | Ethyl | |
| 2.3.1313 | 3-Methyl-6-methylsulfanyl-Phenyl | Vinyl | Ethyl | |
| 2.3.1314 | 3-Cyclopropyl-6-methylsulfanyl-Phenyl | Vinyl | Ethyl | |
| 2.3.1315 | 3-Cyano-6-methylsulfanyl-Phenyl | Vinyl | Ethyl | |
| 2.3.1316 | 3-Trifluormethyl-6-methylsulfanyl-Phenyl | Vinyl | Ethyl | |
| 2.3.1317 | 3-Methoxy-6-methylsulfanyl-Phenyl | Vinyl | Ethyl | |
| 2.3.1318 | 3-Ethoxy-6-methylsulfanyl-Phenyl | Vinyl | Ethyl | |
| 2.3.1319 | 3-Trifluormethoxy-6-methylsulfanyl-Phenyl | Vinyl | Ethyl | |
| 2.3.1320 | 3-Nitro-6-methylsulfanyl-Phenyl | Vinyl | Ethyl | |
| 2.3.1321 | 2,3,4-Trifluor-Phenyl | Vinyl | Ethyl | |
| 2.3.1322 | 2,3,4-Trichlor-Phenyl | Vinyl | Ethyl | |
| 2.3.1323 | 2,3.4-Trimethyl-Phenyl | Vinyl | Ethyl | |
| 2.3.1324 | 2-Fluor-2-chlor-5-trifluormethyl-Phenyl | Vinyl | Ethyl | |
| 2.3.1325 | 2,3,5-Trifluor-Phenyl | Vinyl | Ethyl | |
| 2.3.1326 | 2,3,5-Trichlor-Phenyl | Vinyl | Ethyl | |
| 2.3.1327 | 2,3,5-Trimethyl-Phenyl | Vinyl | Ethyl | |
| 2.3.1328 | 2,3-Dichlor-5-methoxy-Phenyl | Vinyl | Ethyl | |
| 2.3.1329 | 2,3,6-Trifluor-Phenyl | Vinyl | Ethyl | |
| 2.3.1330 | 2,3,6-Trichlor-Phenyl | Vinyl | Ethyl | |
| 2.3.1331 | 2,3,6-Trimethyl-Phenyl | Vinyl | Ethyl | |
| 2.3.1332 | 3,4,5-Trifluor-Phenyl | Vinyl | Ethyl | |
| 2.3.1333 | 3,4,5-Trichlor-Phenyl | Vinyl | Ethyl | |
| 2.3.1334 | 3,4,5-Trimethyl-Phenyl | Vinyl | Ethyl | |
| 2.3.1335 | 3,5-Dimethyl-4-fluor-Phenyl | Vinyl | Ethyl | |
| 2.3.1336 | 3,5-Dichlor-4-methoxy-Phenyl | Vinyl | Ethyl | |
| 2.3.1337 | 3,5-Difluor-4-chlor-Phenyl | Vinyl | Ethyl | |
| 2.3.1338 | 3,5-Dichlor-4-hydroxy-Phenyl | Vinyl | Ethyl | |
| 2.3.1339 | 3,5-Trifluormethyl-4-chlor-Phenyl | Vinyl | Ethyl | |
| 2.3.1340 | 3,4,6-Trifluor-Phenyl | Vinyl | Ethyl | |
| 2.3.1341 | 3,4,6-Trichlor-Phenyl | Vinyl | Ethyl | |
| 2.3.1342 | 3,4,6-Trimethyl-Phenyl | Vinyl | Ethyl | |
| 2.3.1343 | Pentafluorphenyl | Vinyl | Ethyl | |

**Tabelle 2.4: Erfindungsgemäße Verbindungen der allgemeinen Formel (I), worin W* für COOY, R¹ und R² für Wasserstoff stehen, und Aryl den Rest**

| | | | | |
|---|---|---|---|---|
| | bedeutet. 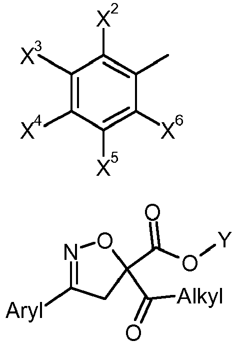 | | | |

| Nr. | Aryl | Alkyl | Y | Physikalische Daten |
|---|---|---|---|---|
| 2.4.001 | 3-Fluor-Phenyl | Methyl | Methyl | [CDCl3] 2.44 (s,3H); 3.87 (s,3H); 3.87 (AB,2H); 7.12-7.20 (m,1H); 7.38-7.43 (m,3H). |
| 2.4.002 | 3-Fluor-Phenyl | Ethyl | Ethyl | |
| 2.4.003 | 3-Fluor-Phenyl | Propyl | Ethyl | |
| 2.4.004 | 3-Fluor-Phenyl | Butyl | Ethyl | |
| 2.4.005 | 3-Chlor-Phenyl | Methyl | Ethyl | |
| 2.4.006 | 3-Chlor-Phenyl | Ethyl | Ethyl | |
| 2.4.007 | 3-Chlor-Phenyl | Propyl | Ethyl | |
| 2.4.008 | 3-Chlor-Phenyl | Butyl | Ethyl | |
| 2.4.009 | 3-Brom-Phenyl | Methyl | Ethyl | |
| 2.4.010 | 3-Brom-Phenyl | Ethyl | Ethyl | |
| 2.4.011 | 3-Iod-Phenyl | Methyl | Ethyl | |
| 2.4.012 | 3-Iod-Phenyl | Ethyl | Ethyl | |
| 2.4.013 | 3-Methyl-Phenyl | Methyl | Ethyl | |
| 2.4.014 | 3-Methyl-Phenyl | Ethyl | Ethyl | |
| 2.4.015 | 3-Ethyl-Phenyl | Methyl | Ethyl | |
| 2.4.016 | 3-Propyl-Phenyl | Methyl | Ethyl | |
| 2.4.017 | 3-isoPropyl-Phenyl | Methyl | Ethyl | |
| 2.4.018 | 3-nButyl-Phenyl | Methyl | Ethyl | |
| 2.4.019 | 3-iButyl-Phenyl | Methyl | Ethyl | |
| 2.4.020 | 3-tert.Butyl-Phenyl | Methyl | Ethyl | |
| 2.4.021 | 3-Cyclopropyl-Phenyl | Methyl | Ethyl | |
| 2.4.022 | 3-Cyclobutyl-Phenyl | Methyl | Ethyl | |
| 2.4.023 | 3-Cyclopentyl-Phenyl | Methyl | Ethyl | |
| 2.4.024 | 3-Vinyl-Phenyl | Methyl | Ethyl | |
| 2.4.025 | 3-Ethinyl-Phenyl | Methyl | Ethyl | |
| 2.4.026 | 3-Cyano-Phenyl | Methyl | Ethyl | |
| 2.4.027 | 3-Trifluormethyl-Phenyl | Methyl | Ethyl | |
| 2.4.028 | 3-Difluormethyl-Phenyl | Methyl | Ethyl | |
| 2.4.029 | 3-(Hydroxycarbonyl)-Phenyl | Methyl | Ethyl | |
| 2.4.030 | 3-(Methoxycarbony)l-Phenyl | Methyl | Ethyl | |
| 2.4.031 | 3-(Ethoxycarbonyl)-Phenyl | Methyl | Ethyl | |
| 2.4.032 | 3-Hydroxymethyl-Phenyl | Methyl | Ethyl | |
| 2.4.033 | 3-Carbamoyl-Phenyl | Methyl | Ethyl | |
| 2.4.034 | 3-Hydroxy-Phenyl- | Methyl | Ethyl | |
| 2.4.035 | 3-Methoxy-Phenyl | Methyl | Ethyl | |
| 2.4.036 | 3-Ethoxy-Phenyl | Methyl | Ethyl | |
| 2.4.037 | 3-Propyloxy-Phenyl | Methyl | Ethyl | |
| 2.4.038 | 3-isoPropyloxy-Phenyl | Methyl | Ethyl | |
| 2.4.039 | 3-nButyloxy-Phenyl | Methyl | Ethyl | |
| 2.4.040 | 3-iButyloxy-Phenyl | Methyl | Ethyl | |
| 2.4.041 | 3-tButyloxy-Phenyl | Methyl | Ethyl | |
| 2.4.042 | 3-Difluormethoxy-Phenyl | Methyl | Ethyl | |
| 2.4.043 | 3-Trifluormethoxy-Phenyl | Methyl | Ethyl | |
| 2.4.044 | 3-(2,2,2-Trifluorethoxy)-Phenyl | Methyl | Ethyl | |
| 2.4.045 | 3-(2-Chlorethoxy)-Phenyl | Methyl | Ethyl | |
| 2.4.046 | 3-(2-Hydroxyethoxy)-Phenyl- | Methyl | Ethyl | |
| 2.4.047 | 3-(2-Methoxyethoxy)-Phenyl- | Methyl | Ethyl | |
| 2.4.048 | 3-[(tert-Butoxycarbonyl)oxy]-Phenyl | Methyl | Ethyl | |
| 2.4.049 | 3-Nitro-Phenyl | Methyl | Ethyl | |
| 2.4.050 | 3-Acetoxy-Phenyl | Methyl | Ethyl | |
| 2.4.051 | {3-[(tert-Butoxycarbonyl)amino]-Phenyl}- | Methyl | Ethyl | |
| 2.4.052 | 3-Methylsulfanyl-Phenyl | Methyl | Ethyl | |
| 2.4.053 | 3-Ethylsulfanyl-Phenyl | Methyl | Ethyl | |
| 2.4.054 | 3-(Pentafluor-lambda⁶-sulfanyl)-Phenyl | Methyl | Ethyl | |
| 2.4.055 | 2,3-Difluor-Phenyl | Methyl | Ethyl | |
| 2.4.056 | 2,3-Difluor-Phenyl | Ethyl | Ethyl | |
| 2.4.057 | 2,3-Difluor-Phenyl | Propyl | Ethyl | |
| 2.4.058 | 2,3-Difluor-Phenyl | Butyl | Ethyl | |
| 2.4.059 | 2-Chlor-3-fluor-Phenyl | Methyl | Ethyl | |
| 2.4.060 | 2-Brom-3-fluor-Phenyl | Methyl | Ethyl | |
| 2.4.061 | 2-Methyl-3-fluor-Phenyl | Methyl | Ethyl | |
| 2.4.062 | 2-Ethyl-3-fluor-Phenyl | Methyl | Ethyl | |
| 2.4.063 | 2-Cyclopropyl-3-fluor-Phenyl | Methyl | Ethyl | |
| 2.4.064 | 2-Vinyl-3-fluor-Phenyl | Methyl | Ethyl | |
| 2.4.065 | 2-Ethinyl-3-fluor-Phenyl | Methyl | Ethyl | |
| 2.4.066 | 2-Cyano-3-fluor-Phenyl | Methyl | Ethyl | |
| 2.4.067 | 2-Methoxy-3-fluor-Phenyl | Methyl | Ethyl | |
| 2.4.068 | 2-Ethoxy-3-fluor-Phenyl | Methyl | Ethyl | |
| 2.4.069 | 2-Trifluormethoxy-3-fluor-Phenyl | Methyl | Ethyl | |
| 2.4.070 | 2-Nitro-3-fluor-Phenyl | Methyl | Ethyl | |
| 2.4.071 | 2-Fluor-3-chlor-Phenyl | Methyl | Ethyl | |
| 2.4.072 | 2,3-Dichlor-Phenyl | Methyl | Ethyl | |
| 2.4.073 | 2,3-Dichlor-Phenyl | Ethyl | Ethyl | |
| 2.4.074 | 2,3-Dichlor-Phenyl | Propyl | Ethyl | |
| 2.4.075 | 2,3-Dichlor-Phenyl | Butyl | Ethyl | |
| 2.4.076 | 2-Brom-3-chlor-Phenyl | Methyl | Ethyl | |
| 2.4.077 | 2-Methyl-3-chlor-Phenyl | Methyl | Ethyl | |
| 2.4.078 | 2-Ethyl-3-chlor-Phenyl | Methyl | Ethyl | |
| 2.4.079 | 2-Cyclopropyl-3-chlor-Phenyl | Methyl | Ethyl | |
| 2.4.080 | 2-Vinyl-3-chlor-Phenyl | Methyl | Ethyl | |
| 2.4.081 | 2-Ethinyl-3-chlor-Phenyl | Methyl | Ethyl | |
| 2.4.082 | 2-Cyano-3-chlor-Phenyl | Methyl | Ethyl | |
| 2.4.083 | 2-Trifluormethyl-2-chlor-Phenyl | Methyl | Ethyl | |
| 2.4.084 | 2-Methoxy-3-chlor-Phenyl | Methyl | Ethyl | |
| 2.4.085 | 2-Ethoxy-3-chlor-Phenyl | Methyl | Ethyl | |
| 2.4.086 | 2-Trifluormethoxy-3-chlor-Phenyl | Methyl | Ethyl | |
| 2.4.087 | 2-Nitro-3-chlor-Phenyl | Methyl | Ethyl | |
| 2.4.088 | 2-Fluor-3-brom-Phenyl | Methyl | Ethyl | |
| 2.4.089 | 2-Chlor-3-brom-Phenyl | Methyl | Ethyl | |
| 2.4.090 | 2,3-Dibrom-Phenyl | Methyl | Ethyl | |
| 2.4.091 | 2-Methyl-3-brom-Phenyl | Methyl | Ethyl | |
| 2.4.092 | 2-Ethyl-3-brom-Phenyl | Methyl | Ethyl | |
| 2.4.093 | 2-Cyclopropyl-3-brom-Phenyl | Methyl | Ethyl | |
| 2.4.094 | 2-Vinyl-3-brom-Phenyl | Methyl | Ethyl | |
| 2.4.095 | 2-Ethinyl-3-brom-Phenyl | Methyl | Ethyl | |
| 2.4.096 | 2-Cyano-3-brom-Phenyl | Methyl | Ethyl | |
| 2.4.097 | 2-Trifluormethyl-3-brom-Phenyl | Methyl | Ethyl | |
| 2.4.098 | 2-Methoxy-3-Phenyl | Methyl | Ethyl | |
| 2.4.099 | 2-Ethoxy-3-brom-Phenyl | Methyl | Ethyl | |
| 2.4.100 | 2-Trifluormethoxy-3-brom-Phenyl | Methyl | Ethyl | |
| 2.4.101 | 2-Nitro-3-brom-Phenyl | Methyl | Ethyl | |
| 2.4.102 | 2-Fluor-3-iod-Phenyl | Methyl | Ethyl | |
| 2.4.103 | 2-Chlor-3-iod-Phenyl | Methyl | Ethyl | |
| 2.4.104 | 2-Brom-3-iod-Phenyl | Methyl | Ethyl | |
| 2.4.105 | 2-Methyl-3-iod-Phenyl | Methyl | Ethyl | |
| 2.4.106 | 2-Ethyl-3-iod-Phenyl | Methyl | Ethyl | |
| 2.4.107 | 2-Cyclopropyl-3-iod-Phenyl | Methyl | Ethyl | |
| 2.4.108 | 2-Vinyl-3-iod-Phenyl | Methyl | Ethyl | |
| 2.4.109 | 2-Ethinyl-3-iod-Phenyl | Methyl | Ethyl | |
| 2.4.110 | 2-Cyano-3-iod-Phenyl | Methyl | Ethyl | |
| 2.4.111 | 2-Trifluormethyl-3-iod-Phenyl | Methyl | Ethyl | |
| 2.4.112 | 2-Methoxy-3-iod-Phenyl | Methyl | Ethyl | |
| 2.4.113 | 2-Ethoxy-3-iod-Phenyl | Methyl | Ethyl | |
| 2.4.114 | 2-Trifluormethoxy-3-iod-Phenyl | Methyl | Ethyl | |
| 2.4.115 | 2-Nitro-3-iod-Phenyl | Methyl | Ethyl | |
| 2.4.116 | 2-Fluor-3-methyl-Phenyl | Methyl | Ethyl | |
| 2.4.117 | 2-Fluor-3-methyl-Phenyl | Ethyl | Ethyl | |
| 2.4.118 | 2-Fluor-3-methyl-Phenyl | Propyl | Ethyl | |
| 2.4.119 | 2-Fluor-3-methyl-Phenyl | Butyl | Ethyl | |
| 2.4.120 | 2-Chlor-3-methyl-Phenyl | Methyl | Ethyl | |
| 2.4.121 | 2-Chlor-3-methyl-Phenyl | Ethyl | Ethyl | |
| 2.4.122 | 2-Chlor-3-methyl-Phenyl | Propyl | Ethyl | |
| 2.4.123 | 2-Chlor-3-methyl-Phenyl | Butyl | Ethyl | |
| 2.4.124 | 2-Brom-3-methyl-Phenyl | Methyl | Ethyl | |
| 2.4.125 | 2,3-Dimethyl-Phenyl | Methyl | Ethyl | |
| 2.4.126 | 2,3-Dimethyl-Phenyl | Ethyl | Ethyl | |
| 2.4.127 | 2,3-Dimethyl-Phenyl | Propyl | Ethyl | |
| 2.4.128 | 2,3-Dimethyl-Phenyl | Butyl | Ethyl | |
| 2.4.129 | 2-Ethyl-3-methyl-Phenyl | Methyl | Ethyl | |
| 2.4.130 | 2-Cyclopropyl-3-methyl-Phenyl | Methyl | Ethyl | |
| 2.4.131 | 2-Vinyl-3-methyl-Phenyl | Methyl | Ethyl | |
| 2.4.132 | 2-Ethinyl-3-methyl-Phenyl | Methyl | Ethyl | |
| 2.4.133 | 2-Cyano-3-methyl-Phenyl | Methyl | Ethyl | |
| 2.4.134 | 2-Trifluormethyl-3-methyl-Phenyl | Methyl | Ethyl | |
| 2.4.135 | 2-Methoxy-3-methyl-Phenyl | Methyl | Ethyl | |
| 2.4.136 | 2-Ethoxy-3-methyl-Phenyl | Methyl | Ethyl | |
| 2.4.137 | 2-Trifluormethoxy-3-methyl-Phenyl | Methyl | Ethyl | |
| 2.4.138 | 2-Nitro-3-methyl-Phenyl | Methyl | Ethyl | |
| 2.4.139 | 2-Fluor-3-ethyl-Phenyl | Methyl | Ethyl | |
| 2.4.140 | 2-Chlor-3-ethyl-Phenyl | Methyl | Ethyl | |
| 2.4.141 | 2-Brom-3-ethyl-Phenyl | Methyl | Ethyl | |
| 2.4.142 | 2-Methyl-3-ethyl-Phenyl | Methyl | Ethyl | |
| 2.4.143 | 2,3-Diethyl-Phenyl | Methyl | Ethyl | |
| 2.4.144 | 2-Cyclopropyl-3-ethyl-Phenyl | Methyl | Ethyl | |
| 2.4.145 | 2-Vinyl-3-ethyl-Phenyl | Methyl | Ethyl | |
| 2.4.146 | 2-Ethinyl-3-ethyl-Phenyl | Methyl | Ethyl | |
| 2.4.147 | 2-Cyano-3-ethyl-Phenyl | Methyl | Ethyl | |
| 2.4.148 | 2-Trifluormethyl-3-ethyl-Phenyl | Methyl | Ethyl | |
| 2.4.149 | 2-Methoxy-3-ethyl-Phenyl | Methyl | Ethyl | |
| 2.4.150 | 2-Ethoxy-3-ethyl-Phenyl | Methyl | Ethyl | |
| 2.4.151 | 2-Trifluormethoxy-3-ethyl-Phenyl | Methyl | Ethyl | |
| 2.4.152 | 2-Nitro-3-ethyl-Phenyl | Methyl | Ethyl | |
| 2.4.153 | 2-Fluor-3-propyl-Phenyl | Methyl | Ethyl | |
| 2.4.154 | 2-Chlor-3-propyl-Phenyl | Methyl | Ethyl | |
| 2.4.155 | 2-Brom-3-propyl-Phenyl | Methyl | Ethyl | |
| 2.4.156 | 2-Methyl-3-propyl-Phenyl | Methyl | Ethyl | |
| 2.4.157 | 2-Methyl-3-propyl-Phenyl | Methyl | Ethyl | |
| 2.4.158 | 2-Cyclopropyl-3-propyl-Phenyl | Methyl | Ethyl | |
| 2.4.159 | 2-Vinyl-3-propyl-Phenyl | Methyl | Ethyl | |
| 2.4.160 | 2-Ethinyl-3propyl-Phenyl | Methyl | Ethyl | |
| 2.4.161 | 2-Cyano-3-propyl-Phenyl | Methyl | Ethyl | |
| 2.4.162 | 2-Trifluormethyl-3-propyl-Phenyl | Methyl | Ethyl | |
| 2.4.163 | 2-Methoxy-3-propyl-Phenyl | Methyl | Ethyl | |
| 2.4.164 | 2-Ethoxy-3-propyl-Phenyl | Methyl | Ethyl | |
| 2.4.165 | 2-Trifluormethoxy-3-propyl-Phenyl | Methyl | Ethyl | |
| 2.4.166 | 2-Nitro-3-propyl-Phenyl | Methyl | Ethyl | |
| 2.4.167 | 2-Fluor-3-isopropyl-Phenyl | Methyl | Ethyl | |
| 2.4.168 | 2-Chlor-3-isopropyl-Phenyl | Methyl | Ethyl | |
| 2.4.169 | 2-Brom-3-isopropyl-Phenyl | Methyl | Ethyl | |
| 2.4.170 | 2-Methyl-3-isopropyl-Phenyl | Methyl | Ethyl | |
| 2.4.171 | 2-Ethyl-3-isopropyl-Phenyl | Methyl | Ethyl | |
| 2.4.172 | 2-Cyclopropyl-3-isopropyl-Phenyl | Methyl | Ethyl | |
| 2.4.173 | 2-Vinyl-3-isopropyl-Phenyl | Methyl | Ethyl | |
| 2.4.174 | 2-Ethinyl-3-isopropyl-Phenyl | Methyl | Ethyl | |
| 2.4.175 | 2-Cyano-3-isopropyl-Phenyl | Methyl | Ethyl | |
| 2.4.176 | 2-Trifluormethyl-3-isopropyl-Phenyl | Methyl | Ethyl | |
| 2.4.177 | 2-Methoxy-3-isopropyl-Phenyl | Methyl | Ethyl | |
| 2.4.178 | 2-Ethoxy-3-isopropyl-Phenyl | Methyl | Ethyl | |
| 2.4.179 | 2-Trifluormethoxy-3-isopropyl-Phenyl | Methyl | Ethyl | |
| 2.4.180 | 2-Nitro-3-isopropyl-Phenyl | Methyl | Ethyl | |
| 2.4.181 | 2-Fluor-3-tert.butyl-Phenyl | Methyl | Ethyl | |
| 2.4.182 | 2-Chlor-3-tert.buty -Phenyl | Methyl | Ethyl | |
| 2.4.183 | 2-Brom-3-tert.butyl-Phenyl | Methyl | Ethyl | |
| 2.4.184 | 2-Methyl-3-tert.butyl-Phenyl | Methyl | Ethyl | |
| 2.4.185 | 2-Ethyl-3-tert.butyl-Phenyl | Methyl | Ethyl | |
| 2.4.186 | 2-Cyclopropyl-3-tert.butyl-Phenyl | Methyl | Ethyl | |
| 2.4.187 | 2-Vinyl-3-tert.butyl-Phenyl | Methyl | Ethyl | |
| 2.4.188 | 2-Ethinyl-3-tert.butyl-Phenyl | Methyl | Ethyl | |
| 2.4.189 | 2-Cyano-3-tert.butyl-Phenyl | Methyl | Ethyl | |
| 2.4.190 | 2-Trifluormethyl-3-tert.butyl-Phenyl | Methyl | Ethyl | |
| 2.4.191 | 2-Methoxy-3-tert.butyl-Phenyl | Methyl | Ethyl | |
| 2.4.192 | 2-Ethoxy-3-tert.butyl-Phenyl | Methyl | Ethyl | |
| 2.4.193 | 2-Trifluormethoxy-3-tert.butyl-Phenyl | Methyl | Ethyl | |
| 2.4.194 | 2-Nitro-3-tert.butyl-Phenyl | Methyl | Ethyl | |
| 2.4.195 | 2-Fluor-3-hydroxymethyl-Phenyl | Methyl | Ethyl | |
| 2.4.196 | 2-Chlor-3-hydroxymethyl-Phenyl | Methyl | Ethyl | |
| 2.4.197 | 2-Brom-3-hydroxymethyl-Phenyl | Methyl | Ethyl | |
| 2.4.198 | 2-Methyl-3-hydroxymethyl-Phenyl | Methyl | Ethyl | |
| 2.4.199 | 2-Ethyl-3-hydroxymethyl-Phenyl | Methyl | Ethyl | |
| 2.4.200 | 2-Cyclopropyl-3-hydroxymethyl-Phenyl | Methyl | Ethyl | |
| 2.4.201 | 2-Vinyl-3-hydroxymethyl-Phenyl | Methyl | Ethyl | |
| 2.4.202 | 2-Ethinyl-3-hydroxymethyl-Phenyl | Methyl | Ethyl | |
| 2.4.203 | 2-Cyano-3-hydroxymethyl-Phenyl | Methyl | Ethyl | |
| 2.4.204 | 2-Trifluormethyl-3-hydroxymethyl-Phenyl | Methyl | Ethyl | |
| 2.4.205 | 2-Methoxy-3-hydroxymethyl-Phenyl | Methyl | Ethyl | |
| 2.4.206 | 2-Ethoxy-3-hydroxymethyl-Phenyl | Methyl | Ethyl | |
| 2.4.207 | 2-Trifluormethoxy-3-hydroxymethyl--Phenyl | Methyl | Ethyl | |
| 2.4.208 | 2-Nitro-3-hydroxymethyl-Phenyl | Methyl | Ethyl | |
| 2.4.209 | 2-Fluor-3-cyclopropyl-Phenyl | Methyl | Ethyl | |
| 2.4.210 | 2-Chlor-3-cyclopropyl-Phenyl | Methyl | Ethyl | |
| 2.4.211 | 2-Brom-3-cyclopropyl-Phenyl | Methyl | Ethyl | |
| 2.4.212 | 2-Methyl-3-cyclopropyl-Phenyl | Methyl | Ethyl | |
| 2.4.213 | 2-Ethyl-3-cyclopropyl -Phenyl | Methyl | Ethyl | |
| 2.4.214 | 2-Cyclopropyl-3-cyclopropyl-Phenyl | Methyl | Ethyl | |
| 2.4.215 | 2-Vinyl-3-cyclopropyl-Phenyl | Methyl | Ethyl | |
| 2.4.216 | 2-Ethinyl-3-cyclopropyl-Phenyl | Methyl | Ethyl | |
| 2.4.217 | 2-Cyano-3-cyclopropyl-Phenyl | Methyl | Ethyl | |
| 2.4.218 | 2-Trifluormethyl-3-cyclopropyl-Phenyl | Methyl | Ethyl | |
| 2.4.219 | 2-Methoxy-3-cyclopropyl-Phenyl | Methyl | Ethyl | |
| 2.4.220 | 2-Ethoxy-3-cyclopropyl-Phenyl | Methyl | Ethyl | |
| 2.4.221 | 2-Trifluormethoxy-3-cyclopropyl-Phenyl | Methyl | Ethyl | |
| 2.4.222 | 2-Fluor-3-methoxycarbonyl-Phenyl | Methyl | Ethyl | |
| 2.4.223 | 2-Chlor-3-methoxycarbonyl-Phenyl | Methyl | Ethyl | |
| 2.4.224 | 2-Brom-3-methoxycarbonyl-Phenyl | Methyl | Ethyl | |
| 2.4.225 | 2-Methyl-3-methoxycarbonyl-Phenyl | Methyl | Ethyl | |
| 2.4.226 | 2-Ethyl-3-methoxycarbonyl-Phenyl | Methyl | Ethyl | |
| 2.4.227 | 2-Cyclopropyl-3-methoxycarbonyl-Phenyl | Methyl | Ethyl | |
| 2.4.228 | 2-Vinyl-3-methoxycarbonyl-Phenyl | Methyl | Ethyl | |
| 2.4.229 | 2-Ethinyl-3-methoxycarbonyl-Phenyl | Methyl | Ethyl | |
| 2.4.230 | 2-Cyano-3-methoxycarbonyl-Phenyl | Methyl | Ethyl | |
| 2.4.231 | 2-Trifluormethyl-3-methoxycarbonyl-Phenyl | Methyl | Ethyl | |
| 2.4.232 | 2-Methoxy-3-methoxycarbonyl-Phenyl | Methyl | Ethyl | |
| 2.4.233 | 2-Ethoxy-3-methoxycarbonyl-Phenyl | Methyl | Ethyl | |
| 2.4.234 | 2-Trifluormethoxy-3-methoxycarbonyl-Phenyl | Methyl | Ethyl | |
| 2.4.235 | 2-Nitro-3-methoxycarbonyl-Phenyl | Methyl | Ethyl | |
| 2.4.236 | 2-Fluor-3-vinyl-Phenyl | Methyl | Ethyl | |
| 2.4.237 | 2-Chlor-3-vinyl-Phenyl | Methyl | Ethyl | |
| 2.4.238 | 2-Brom-3-vinyl-Phenyl | Methyl | Ethyl | |
| 2.4.239 | 2-Methyl-3-vinyl-Phenyl | Methyl | Ethyl | |
| 2.4.240 | 2-Ethyl-3-vinyl-Phenyl | Methyl | Ethyl | |
| 2.4.241 | 2-Cyclopropyl-3-vinyl-Phenyl | Methyl | Ethyl | |
| 2.4.242 | 2-Vinyl-3-vinyl-Phenyl | Methyl | Ethyl | |
| 2.4.243 | 2-Ethinyl-3-vinyl-Phenyl | Methyl | Ethyl | |
| 2.4.244 | 2-Cyano-3-vinyl-Phenyl | Methyl | Ethyl | |
| 2.4.245 | 2-Trifluormethyl-3-vinyl-Phenyl | Methyl | Ethyl | |
| 2.4.246 | 2-Methoxy-3-vinyl-Phenyl | Methyl | Ethyl | |
| 2.4.247 | 2-Ethoxy-3-vinyl-Phenyl | Methyl | Ethyl | |
| 2.4.248 | 2-Trifluormethoxy-3-vinyl-Phenyl | Methyl | Ethyl | |
| 2.4.249 | 2-Nitro-3-vinyl-Phenyl | Methyl | Ethyl | |
| 2.4.250 | 2-Fluor-3-ethinyl-Phenyl | Methyl | Ethyl | |
| 2.4.251 | 2-Chlor-3-ethinyl-Phenyl | Methyl | Ethyl | |
| 2.4.252 | 2-Brom-3-ethinyl-Phenyl | Methyl | Ethyl | |
| 2.4.253 | 2-Methyl-3-ethinyl-Phenyl | Methyl | Ethyl | |
| 2.4.254 | 2-Ethyl-3-ethinyl-Phenyl | Methyl | Ethyl | |
| 2.4.255 | 2-Cyclopropyl-3-ethinyl-Phenyl | Methyl | Ethyl | |
| 2.4.256 | 2-Vinyl-3-ethinyl-Phenyl | Methyl | Ethyl | |
| 2.4.257 | 2-Cyano-3-ethinyl-Phenyl | Methyl | Ethyl | |
| 2.4.258 | 2-Trifluormethyl-3-ethinyl-Phenyl | Methyl | Ethyl | |
| 2.4.259 | 2-Methoxy-3-ethinyl-Phenyl | Methyl | Ethyl | |
| 2.4.260 | 2-Ethoxy-3-ethinyl-Phenyl | Methyl | Ethyl | |
| 2.4.261 | 2-Trifluormethoxy-3-ethinyl--Phenyl | Methyl | Ethyl | |
| 2.4.262 | 2-Nitro-3-ethinyl-Phenyl | Methyl | Ethyl | |
| 2.4.263 | 2-Fluor-3-ethinyl-Phenyl | Methyl | Ethyl | |
| 2.4.264 | 2-Fluor-3-cyano-Phenyl | Methyl | Ethyl | |
| 2.4.265 | 2-Chlor-3-cyano-Phenyl | Methyl | Ethyl | |
| 2.4.266 | 2-Brom-3-cyano-Phenyl | Methyl | Ethyl | |
| 2.4.267 | 2-Methyl-3-cyano-Phenyl | Methyl | Ethyl | |
| 2.4.268 | 2-Ethyl-3-cyano-Phenyl | Methyl | Ethyl | |
| 2.4.269 | 2-Ethyl-3-cyano-Phenyl | Ethyl | Ethyl | |
| 2.4.270 | 2-Ethyl-3-cyano-Phenyl | Propyl | Ethyl | |
| 2.4.271 | 2-Ethyl-3-cyano-Phenyl | Butyl | Ethyl | |
| 2.4.272 | 2-Cyclopropyl-3-cyano-Phenyl | Methyl | Ethyl | |
| 2.4.273 | 2-Vinyl-3-cyano-Phenyl | Methyl | Ethyl | |
| 2.4.274 | 2-Ethinyl-3-cyano-Phenyl | Methyl | Ethyl | |
| 2.4.275 | 2-Cyano-3-cyanoPhenyl | Methyl | Ethyl | |
| 2.4.276 | 2-Trifluormethyl-3-cyano-Phenyl | Methyl | Ethyl | |
| 2.4.277 | 2-Methoxy-3-cyano-Phenyl | Methyl | Ethyl | |
| 2.4.278 | 2-Ethoxy-3-cyano-Phenyl | Methyl | Ethyl | |
| 2.4.279 | 2-Trifluormethoxy-3-cyano-Phenyl | Methyl | Ethyl | |
| 2.4.280 | 2-Nitro-3-cyano-Phenyl | Methyl | Ethyl | |
| 2.4.281 | 2-Fluor-3-hydroxy-Phenyl | Methyl | Ethyl | |
| 2.4.282 | 2-Chlor-3-hydroxy-Phenyl | Methyl | Ethyl | |
| 2.4.283 | 2-Brom-3-hydroxy-Phenyl | Methyl | Ethyl | |
| 2.4.284 | 2-Methyl-3-hydroxy-Phenyl | Methyl | Ethyl | |
| 2.4.285 | 2-Ethyl-3-hydroxy-Phenyl | Methyl | Ethyl | |
| 2.4.286 | 2-Cyclopropyl-3-hydroxy-Phenyl | Methyl | Ethyl | |
| 2.4.287 | 2-Vinyl-3-hydroxy-Phenyl | Methyl | Ethyl | |
| 2.4.288 | 2-Ethinyl-3-hydroxy-Phenyl | Methyl | Ethyl | |
| 2.4.289 | 2-Cyano-3-hydroxy-Phenyl | Methyl | Ethyl | |
| 2.4.290 | 2-Trifluormethyl-3-hydroxy-Phenyl | Methyl | Ethyl | |
| 2.4.291 | 2-Methoxy-3-hydroxy-Phenyl | Methyl | Ethyl | |
| 2.4.292 | 2-Ethoxy-3-hydroxy-Phenyl | Methyl | Ethyl | |
| 2.4.293 | 2-Trifluormethoxy-3-hydroxy-Phenyl | Methyl | Ethyl | |
| 2.4.294 | 2-Nitro-3-hydroxy-Phenyl | Methyl | Ethyl | |
| 2.4.295 | 2-Fluor-3-methoxy-Phenyl | Methyl | Ethyl | |
| 2.4.296 | 2-Chlor-3-methoxy-Phenyl | Methyl | Ethyl | |
| 2.4.297 | 2-Brom-3-methoxy-Phenyl | Methyl | Ethyl | |
| 2.4.298 | 2-Methyl-3-methoxy-Phenyl | Methyl | Ethyl | |
| 2.4.299 | 2-Ethyl-3-methoxy-Phenyl | Methyl | Ethyl | |
| 2.4.300 | 2-Cyclopropyl-3-methoxy-Phenyl | Methyl | Ethyl | |
| 2.4.301 | 2-Vinyl-3-methoxy-Phenyl | Methyl | Ethyl | |
| 2.4.302 | 2-Ethinyl-3-methoxy-Phenyl | Methyl | Ethyl | |
| 2.4.303 | 2-Cyano-3-methoxy-Phenyl | Methyl | Ethyl | |
| 2.4.304 | 2-Trifluormethyl-3-methoxy-Phenyl | Methyl | Ethyl | |
| 2.4.305 | 2,3-Dimethoxy-Phenyl | Methyl | Ethyl | |
| 2.4.306 | 2-Ethoxy-3-methoxy-Phenyl | Methyl | Ethyl | |
| 2.4.307 | 2-Trifluormethoxy-3-methoxy-Phenyl | Methyl | Ethyl | |
| 2.4.308 | 2-Nitro-3-methoxy-Phenyl | Methyl | Ethyl | |
| 2.4.309 | 2-Fluor-3-ethoxy-Phenyl | Methyl | Ethyl | |
| 2.4.310 | 2-Chlor-3-ethoxy-Phenyl | Methyl | Ethyl | |
| 2.4.311 | 2-Brom-3-ethoxy-Phenyl | Methyl | Ethyl | |
| 2.4.312 | 2-Methyl-3-ethoxy-Phenyl | Methyl | Ethyl | |
| 2.4.313 | 2-Ethyl-3-ethoxy-Phenyl | Methyl | Ethyl | |
| 2.4.314 | 2-Cyclopropyl-3-ethoxy-Phenyl | Methyl | Ethyl | |
| 2.4.315 | 2-Vinyl-3-ethoxy-Phenyl | Methyl | Ethyl | |
| 2.4.316 | 2-Ethinyl-3-ethoxy-Phenyl | Methyl | Ethyl | |
| 2.4.317 | 2-Cyano-3-ethoxy-Phenyl | Methyl | Ethyl | |
| 2.4.318 | 2-Trifluormethyl-3-ethoxy-Phenyl | Methyl | Ethyl | |
| 2.4.319 | 2-Methoxy-3-ethoxy -Phenyl | Methyl | Ethyl | |
| 2.4.320 | 2,3-Diethoxy--Phenyl | Methyl | Ethyl | |
| 2.4.321 | 2-Trifluormethoxy-3-ethoxy-Phenyl | Methyl | Ethyl | |
| 2.4.322 | 2-Nitro-3-ethoxy-Phenyl | Methyl | Ethyl | |
| 2.4.323 | 2-Fluor-3-propoxy-Phenyl | Methyl | Ethyl | |
| 2.4.324 | 2-Chlor-3-propoxy-Phenyl | Methyl | Ethyl | |
| 2.4.325 | 2-Brom-3-propoxy-Phenyl | Methyl | Ethyl | |
| 2.4.326 | 2-Methyl-3-propoxy-Phenyl | Methyl | Ethyl | |
| 2.4.327 | 2-Ethyl-3-propoxy-Phenyl | Methyl | Ethyl | |
| 2.4.328 | 2-Cyclopropyl-3-propoxy-Phenyl | Methyl | Ethyl | |
| 2.4.329 | 2-Vinyl-3-propoxy-Phenyl | Methyl | Ethyl | |
| 2.4.330 | 2-Ethinyl-3-propoxy-Phenyl | Methyl | Ethyl | |
| 2.4.331 | 2-Cyano-3-propoxy-Phenyl | Methyl | Ethyl | |
| 2.4.332 | 2-Trifluormethyl-3-propoxy-Phenyl | Methyl | Ethyl | |
| 2.4.333 | 2-Methoxy-3-propoxy-Phenyl | Methyl | Ethyl | |
| 2.4.334 | 2-Ethoxy-3-propoxy-Phenyl | Methyl | Ethyl | |
| 2.4.335 | 2-Trifluormethoxy-3-propoxy-Phenyl | Methyl | Ethyl | |
| 2.4.336 | 2-Nitro-3-propoxy-Phenyl | Methyl | Ethyl | |
| 2.4.337 | 2-Fluor-3-isopropoxy-Phenyl | Methyl | Ethyl | |
| 2.4.338 | 2-Chlor-3-isopropoxy-Phenyl | Methyl | Ethyl | |
| 2.4.339 | 2-Brom-3-isopropoxy-Phenyl | Methyl | Ethyl | |
| 2.4.340 | 2-Methyl-3-isopropoxy-Phenyl | Methyl | Ethyl | |
| 2.4.341 | 2-Ethyl-3-isopropoxy-Phenyl | Methyl | Ethyl | |
| 2.4.342 | 2-Cyclopropyl-3-isopropoxy-Phenyl | Methyl | Ethyl | |
| 2.4.343 | 2-Vinyl-3-isopropoxy-Phenyl | Methyl | Ethyl | |
| 2.4.344 | 2-Ethinyl-3-isopropoxy-Phenyl | Methyl | Ethyl | |
| 2.4.345 | 2-Cyano-3-isopropoxy-Phenyl | Methyl | Ethyl | |
| 2.4.346 | 2-Trifluormethyl-3-isopropoxy-Phenyl | Methyl | Ethyl | |
| 2.4.347 | 2-Methoxy-3-isopropoxy-Phenyl | Methyl | Ethyl | |
| 2.4.348 | 2-Ethoxy-3-isopropoxy-Phenyl | Methyl | Ethyl | |
| 2.4.349 | 2-Trifluormethoxy-3-isopropoxy-Phenyl | Methyl | Ethyl | |
| 2.4.350 | 2-Nitro-3-isopropoxy-Phenyl | Methyl | Ethyl | |
| 2.4.351 | 2-Fluor-3-tert.butoxy-Phenyl | Methyl | Ethyl | |
| 2.4.352 | 2-Chlor-3-tert.butoxy-Phenyl | Methyl | Ethyl | |
| 2.4.353 | 2-Brom-3-tert.butoxy-Phenyl | Methyl | Ethyl | |
| 2.4.354 | 2-Methyl-3-tert.butoxy-Phenyl | Methyl | Ethyl | |
| 2.4.355 | 2-Ethyl-3-tert.butoxy-Phenyl | Methyl | Ethyl | |
| 2.4.356 | 2-Cyclopropyl-3-tert.butoxy-Phenyl | Methyl | Ethyl | |
| 2.4.357 | 2-Vinyl-3-tert.butoxy-Phenyl | Methyl | Ethyl | |
| 2.4.358 | 2-Ethinyl-3-tert.butoxy-Phenyl | Methyl | Ethyl | |
| 2.4.359 | 2-Cyano-3-tert.butoxy-Phenyl | Methyl | Ethyl | |
| 2.4.360 | 2-Trifluormethyl-3-tert.butoxy-Phenyl | Methyl | Ethyl | |
| 2.4.361 | 2-Methoxy-3-tert.butoxy-Phenyl | Methyl | Ethyl | |
| 2.4.362 | 2-Ethoxy-3-tert.butoxy-Phenyl | Methyl | Ethyl | |
| 2.4.363 | 2-Trifluormethoxy-3-tert.butoxy-Phenyl | Methyl | Ethyl | |
| 2.4.364 | 2-Nitro-3-tert.butoxy-Phenyl | Methyl | Ethyl | |
| 2.4.365 | 2-Fluor-3-trifluormethoxy-Phenyl | Methyl | Ethyl | |
| 2.4.366 | 2-Chlor-3-trifluormethoxy-Phenyl | Methyl | Ethyl | |
| 2.4.367 | 2-Brom-3-trifluormethoxy-Phenyl | Methyl | Ethyl | |
| 2.4.368 | 2-Methyl-3-trifluormethoxy-Phenyl | Methyl | Ethyl | |
| 2.4.369 | 2-Ethyl-3-trifluormethoxy-Phenyl | Methyl | Ethyl | |
| 2.4.370 | 2-Cyclopropyl-3-trifluormethoxy-Phenyl | Methyl | Ethyl | |
| 2.4.371 | 2-Vinyl-3-trifluormethoxy-Phenyl | Methyl | Ethyl | |
| 2.4.372 | 2-Ethinyl-3-trifluormethoxy-Phenyl | Methyl | Ethyl | |
| 2.4.373 | 2-Cyano-3-trifluormethoxy-Phenyl | Methyl | Ethyl | |
| 2.4.374 | 2-Trifluormethyl-3-trifluormethoxy-Phenyl | Methyl | Ethyl | |
| 2.4.375 | 2-Methoxy-3-trifluormethoxy-Phenyl | Methyl | Ethyl | |
| 2.4.376 | 2-Ethoxy-3-trifluormethoxy-Phenyl | Methyl | Ethyl | |
| 2.4.377 | 2,3-Bis(trifluormethoxy)-Phenyl | Methyl | Ethyl | |
| 2.4.378 | 2-Nitro-3-trifluormethoxy-Phenyl | Methyl | Ethyl | |
| 2.4.379 | 2-Fluor-3-(2,2,2-trifluorethoxy)-Phenyl | Methyl | Ethyl | |
| 2.4.380 | 2-Chlor-3-(2,2,2-trifluorethoxy)-Phenyl | Methyl | Ethyl | |
| 2.4.381 | 2-Brom-3-(2,2,2-trifluorethoxy)-Phenyl | Methyl | Ethyl | |
| 2.4.382 | 2-Methyl-3-(2,2,2-trifluorethoxy)-Phenyl | Methyl | Ethyl | |
| 2.4.383 | 2-Ethyl-3-(2,2,2-trifluorethoxy)-Phenyl | Methyl | Ethyl | |
| 2.4.384 | 2-Cyclopropyl-3-(2,2,2-trifluorethoxy)-Phenyl | Methyl | Ethyl | |
| 2.4.385 | 2-Vinyl-3-(2,2,2-trifluorethoxy)-Phenyl | Methyl | Ethyl | |
| 2.4.386 | 2-Ethinyl-3-(2,2,2-trifluorethoxy-Phenyl | Methyl | Ethyl | |
| 2.4.387 | 2-Cyano-3-(2,2,2-trifluorethoxy)-Phenyl | Methyl | Ethyl | |
| 2.4.388 | 2-Trifluormethyl-3-(2,2,2-trifluorethoxy)-Phenyl | Methyl | Ethyl | |
| 2.4.389 | 2-Methoxy-3-(2,2,2-trifluorethoxy)-Phenyl | Methyl | Ethyl | |
| 2.4.390 | 2-Ethoxy-3-(2,2,2-trifluorethoxy)-Phenyl | Methyl | Ethyl | |
| 2.4.391 | 2-Trifluormethoxy-3-(2,2,2-trifluorethoxy)-Phenyl | Methyl | Ethyl | |
| 2.4.392 | 2-Nitro-3-(2,2,2-trifluorethoxy)-Phenyl | Methyl | Ethyl | |
| 2.4.393 | 2-Fluor-3-difluormethoxy-Phenyl | Methyl | Ethyl | |
| 2.4.394 | 2-Chlor-3-difluormethoxy-Phenyl | Methyl | Ethyl | |
| 2.4.395 | 2-Brom-3-difluormethoxy-Phenyl | Methyl | Ethyl | |
| 2.4.396 | 2-Methyl-3-difluormethoxy-Phenyl | Methyl | Ethyl | |
| 2.4.397 | 2-Ethyl-3-difluormethoxy-Phenyl | Methyl | Ethyl | |
| 2.4.398 | 2-Cyclopropyl-3-difluormethoxy-Phenyl | Methyl | Ethyl | |
| 2.4.399 | 2-Vinyl-3-difluormethoxy-Phenyl | Methyl | Ethyl | |
| 2.4.400 | 2-Ethinyl-3-difluormethoxy-Phenyl | Methyl | Ethyl | |
| 2.4.401 | 2-Cyano-3-difluormethoxy -Phenyl | Methyl | Ethyl | |
| 2.4.402 | 2-Trifluormethyl-3-difluormethoxy - Phenyl | Methyl | Ethyl | |
| 2.4.403 | 2-Methoxy-3-difluormethoxy - Phenyl | Methyl | Ethyl | |
| 2.4.404 | 2-Ethoxy-3-difluormethoxy-Phenyl | Methyl | Ethyl | |
| 2.4.405 | 2-Trifluormethoxy-3-difluormethoxy-Phenyl | Methyl | Ethyl | |
| 2.4.406 | 2-Nitro-3-difluormethoxy-Phenyl | Methyl | Ethyl | |
| 2.4.407 | 2-Fluor-3-(2-methoxyethoxy)-Phenyl | Methyl | Ethyl | |
| 2.4.408 | 2-Chlor-3-(2-methoxyethoxy)-Phenyl | Methyl | Ethyl | |
| 2.4.409 | 2-Brom-3-(2-methoxyethoxy)-Phenyl | Methyl | Ethyl | |
| 2.4.410 | 2-Methyl-3-(2-methoxyethoxy)-Phenyl | Methyl | Ethyl | |
| 2.4.411 | 2-Ethyl-3-(2-methoxyethoxy)-Phenyl | Methyl | Ethyl | |
| 2.4.412 | 2-Cyclopropyl-3-(2-methoxyethoxy)-Phenyl | Methyl | Ethyl | |
| 2.4.413 | 2-Vinyl-3-(2-methoxyethoxy)-Phenyl | Methyl | Ethyl | |
| 2.4.414 | 2-Ethinyl-3-(2-methoxyethoxy)-Phenyl | Methyl | Ethyl | |
| 2.4.415 | 2-Cyano-3-(2-methoxyethoxy) - Phenyl | Methyl | Ethyl | |
| 2.4.416 | 2-Trifluormethyl-3-(2-methoxyethoxy) -Phenyl | Methyl | Ethyl | |
| 2.4.417 | 2-Methoxy-3-(2-methoxyethoxy) - Phenyl | Methyl | Ethyl | |
| 2.4.418 | 2-Ethoxy-3-(2-methoxyethoxy)-Phenyl | Methyl | Ethyl | |
| 2.4.419 | 2-Trifluormethoxy-(2-methoxyethoxy)-Phenyl | Methyl | Ethyl | |
| 2.4.420 | 2-Nitro-3-(2-methoxyethoxy)-Phenyl | Methyl | Ethyl | |
| 2.4.421 | 2-Fluor-3-(tert.butoxycarbonyloxy)-Phenyl | Methyl | Ethyl | |
| 2.4.422 | 2-Chlor-3-(tert.butoxycarbonyloxy)-Phenyl | Methyl | Ethyl | |
| 2.4.423 | 2-Brom-3-(tert.butoxycarbonyloxy)-Phenyl | Methyl | Ethyl | |
| 2.4.424 | 2-Methyl-3-(tert.butoxycarbonyloxy)-Phenyl | Methyl | Ethyl | |
| 2.4.425 | 2-Ethyl-3-(tert.butoxycarbonyloxy)-Phenyl | Methyl | Ethyl | |
| 2.4.426 | 2-Cyclopropyl-3-(tert.butoxycarbonyloxy)-Phenyl | Methyl | Ethyl | |
| 2.4.427 | 2-Vinyl-3-(tert.butoxycarbonyloxy)-Phenyl | Methyl | Ethyl | |
| 2.4.428 | 2-Ethinyl-3-(tert.butoxycarbonyloxy)-Phenyl | Methyl | Ethyl | |
| 2.4.429 | 2-Cyano-3-(tert.butoxycarbonyloxy)-Phenyl | Methyl | Ethyl | |
| 2.4.430 | 2-Trifluormethyl-3-(tert.butoxycarbonyloxy)-Phenyl | Methyl | Ethyl | |
| 2.4.431 | 2-Methoxy-3-(tert.butoxycarbonyloxy)-Phenyl | Methyl | Ethyl | |
| 2.4.432 | 2-Ethoxy-3-(tert.butoxycarbonyloxy)-Phenyl | Methyl | Ethyl | |
| 2.4.433 | 2-Trifluormethoxy-3-(tert.butoxycarbonyloxy)-Phenyl | Methyl | Ethyl | |
| 2.4.434 | 2-Nitro-3--(tert.butoxycarbonyloxy)-Phenyl | Methyl | Ethyl | |
| 2.4.435 | 2-Fluor-3-nitro-Phenyl | Methyl | Ethyl | |
| 2.4.436 | 2-Chlor-3-nitro-Phenyl | Methyl | Ethyl | |
| 2.4.437 | 2-Brom-3-nitro-Phenyl | Methyl | Ethyl | |
| 2.4.438 | 2-Methyl-3-nitro-Phenyl | Methyl | Ethyl | |
| 2.4.439 | 2-Ethyl-3-nitro-Phenyl | Methyl | Ethyl | |
| 2.4.440 | 2-Cyclopropyl-3-nitro-Phenyl | Methyl | Ethyl | |
| 2.4.441 | 2-Vinyl-3-nitro-Phenyl | Methyl | Ethyl | |
| 2.4.442 | 2-Ethinyl-3-nitro-Phenyl | Methyl | Ethyl | |
| 2.4.443 | 2-Cyano-3-nitro-Phenyl | Methyl | Ethyl | |
| 2.4.444 | 2-Trifluormethyl-3-nitro-Phenyl | Methyl | Ethyl | |
| 2.4.445 | 2-Methoxy-3-nitro-Phenyl | Methyl | Ethyl | |
| 2.4.446 | 2-Ethoxy-3-nitro-Phenyl | Methyl | Ethyl | |
| 2.4.447 | 2-Trifluormethoxy-3-nitro-Phenyl | Methyl | Ethyl | |
| 2.4.448 | 2-Fluor-3-methylsulfanylPhenyl | Methyl | Ethyl | |
| 2.4.449 | 2-Chlor-3-methylsulfanyl-Phenyl | Methyl | Ethyl | |
| 2.4.450 | 2-Brom-3-methylsulfanyl-Phenyl | Methyl | Ethyl | |
| 2.4.451 | 2-Methyl-3-methylsulfanyl-Phenyl | Methyl | Ethyl | |
| 2.4.452 | 2-Ethyl-3-methylsulfanyl-Phenyl | Methyl | Ethyl | |
| 2.4.453 | 2-Cyclopropyl-3-methylsulfanyl-Phenyl | Methyl | Ethyl | |
| 2.4.454 | 2-Vinyl-3-methylsulfanyl-Phenyl | Methyl | Ethyl | |
| 2.4.455 | 2-Ethinyl-3-methylsulfanyl-Phenyl | Methyl | Ethyl | |
| 2.4.456 | 2-Cyano-3-methylsulfanyl -Phenyl | Methyl | Ethyl | |
| 2.4.457 | 2-Trifluormethyl-3-methylsulfanyl - Phenyl | Methyl | Ethyl | |
| 2.4.458 | 2-Methoxy-3-methylsulfanyl -Phenyl | Methyl | Ethyl | |
| 2.4.459 | 2-Ethoxy-3-methylsulfanyl-Phenyl | Methyl | Ethyl | |
| 2.4.460 | 2-Trifluormethoxy-3-methylsulfanyl-Phenyl | Methyl | Ethyl | |
| 2.4.461 | 2-Nitro-3-methylsulfanyl-Phenyl | Methyl | Ethyl | |
| 2.4.462 | 3,5-Difluor-Phenyl | Methyl | Methyl | [CDCl3] 2.44 (s,3H); 3.80 (d,1H); 3.86 (s,3H); 3.89 (d,1H); 6.90 (m,1H); 7.19 (d,2H). |
| 2.4.463 | 3,5-Difluor-Phenyl | Ethyl | Methyl | [CDCl3] 1.12 (t,3H); 2.83 (q,2H); 3.81 (d,1H); 3.86 (s,4H); 6.90 (m,1H); 7.20 (d,2H). |
| 2.4.464 | 3,5-Difluor-Phenyl | Propyl | Ethyl | |
| 2.4.465 | 3,5-Difluor-Phenyl | Butyl | Ethyl | |
| 2.4.466 | 3-Chlor-5-fluor-Phenyl | Methyl | Methyl | [CDCl3] 2.43 (s,3H); 3.80 (d,1H); 3.87 (s,3H); 3.90 (d,1H); 7.18 (d,1H), 7.30 (d,1H), 7.43 (s,1H). |
| 2.4.467 | 3-Chlor-5-fluor-Phenyl | Ethyl | Ethyl | |
| 2.4.468 | 3-Chlor-5-fluor-Phenyl | Propyl | Ethyl | |
| 2.4.469 | 3-Chlor-5-fluor-Phenyl | Butyl | Ethyl | |
| 2.4.470 | 3-Brom-5-fluor-Phenyl | Methyl | Ethyl | |
| 2.4.471 | 3-Brom-5-fluor-Phenyl | Ethyl | Ethyl | |
| 2.4.472 | 3-Brom-5-fluor-Phenyl | Propyl | Ethyl | |
| 2.4.473 | 3-Brom-5-fluor-Phenyl | Butyl | Ethyl | |
| 2.4.474 | 3-Iod-5-fluor-Phenyl | Methyl | Ethyl | |
| 2.4.475 | 3-Methyl-5-fluor-Phenyl | Methyl | Ethyl | |
| 2.4.476 | 3-Methyl-5-fluor-Phenyl | Ethyl | Ethyl | |
| 2.4.477 | 3-Methyl-5-fluor-Phenyl | Propyl | Ethyl | |
| 2.4.478 | 3-Methyl-5-fluor-Phenyl | Butyl | Ethyl | |
| 2.4.479 | 3-Ethyl-5-fluor-Phenyl | Methyl | Ethyl | |
| 2.4.480 | 3-Propyl-5-fluor-Phenyl | Methyl | Ethyl | |
| 2.4.481 | 3-iPropyl-5-fluor-Phenyl | Methyl | Ethyl | |
| 2.4.482 | 3-nButyl-5-fluor-Phenyl | Methyl | Ethyl | |
| 2.4.483 | 3-isoButyl-5-fluor-Phenyl | Methyl | Ethyl | |
| 2.4.484 | 3-tert.Butyl-5-fluor-Phenyl | Methyl | Ethyl | |
| 2.4.485 | 3-Cyclopropyl-5-fluor-Phenyl | Methyl | Ethyl | |
| 2.4.486 | 3-Vinyl-5-fluor-Phenyl | Methyl | Ethyl | |
| 2.4.487 | 3-Ethinyl-5-fluor-Phenyl | Methyl | Ethyl | |
| 2.4.488 | 3-Cyano-5-fluor-Phenyl | Methyl | Ethyl | |
| 2.4.489 | 3-Trifluormethyl-5-fluor-Phenyl | Methyl | Ethyl | |
| 2.4.490 | 3-Trifluormethyl-5-fluor-Phenyl | Ethyl | Ethyl | |
| 2.4.491 | 3-Trifluormethyl-5-fluor-Phenyl | Propyl | Ethyl | |
| 2.4.492 | 3-Trifluormethyl-5-fluor-Phenyl | Butyl | Ethyl | |
| 2.4.493 | 3-(Methoxycarbony)l-5-fluor-Phenyl | Methyl | Ethyl | |
| 2.4.494 | 3-Hydroxymethyl-5-fluor-Phenyl | Methyl | Ethyl | |
| 2.4.495 | 3-Carbamoyl-5-fluor-Phenyl | Methyl | Ethyl | |
| 2.4.496 | 3-Hydroxy-5-fluor-Phenyl- | Methyl | Ethyl | |
| 2.4.497 | 3-Methoxy-5-fluor-Phenyl | Methyl | Ethyl | |
| 2.4.498 | 3-Ethoxy-5-fluor-Phenyl | Methyl | Ethyl | |
| 2.4.499 | 3-nPropyoxy-5-fluor-Phenyl | Methyl | Ethyl | |
| 2.4.500 | 3-isoPropoxy-5-fluor-Phenyl | Methyl | Ethyl | |
| 2.4.501 | 3-nButoxy-5-fluor-Phenyl | Methyl | Ethyl | |
| 2.4.502 | 3-isoButoxy-5-fluor-Phenyl | Methyl | Ethyl | |
| 2.4.503 | 3-tert.Butoxy-5-fluor-Phenyl | Methyl | Ethyl | |
| 2.4.504 | 3-Difluormethoxy-5-fluor-Phenyl | Methyl | Ethyl | |
| 2.4.505 | 3-Trifluormethoxy-5-fluor-Phenyl | Methyl | Ethyl | |
| 2.4.506 | 3-(2,2,2-Trifluorethoxy)-5-fluorPhenyl | Methyl | Ethyl | |
| 2.4.507 | 3-(2-Chlorethoxy)-5-fluor-Phenyl | Methyl | Ethyl | |
| 2.4.508 | 3-(2-Hydroxyethoxy)-5-fluorPhenyl- | Methyl | Ethyl | |
| 2.4.509 | 3-[(tert-Butoxycarbonyl)oxy]-5-fluorPhenyl | Methyl | Ethyl | |
| 2.4.510 | 3-Nitro-5-fluor-Phenyl | Methyl | Ethyl | |
| 2.4.511 | 3-Acetoxy-5-fluor-Phenyl | Methyl | Ethyl | |
| 2.4.512 | {3-[(tert-Butoxycarbonyl)amino] -5-fluor-Phenyl} | Methyl | Ethyl | |
| 2.4.513 | 3-Methylsulfanyl-5-fluor-Phenyl | Methyl | Ethyl | |
| 2.4.514 | 3,5-Dichlor-Phenyl | Methyl | Methyl | [CDCl3] 2.43 (s,3H); 3.79 (d,1H); 3.86 (s,3H), 3.90 (d,1H), 7.43 (s,1H); 7.55 (s,2H). |
| 2.4.515 | 3,5-Dichlor-Phenyl | Ethyl | Methyl | [CDCl3] 1.12 (t,3H); 2.82 (q,2H); 3.81 (d,1H); 3.85 (s,4H); 7.43 (s,1H); 7.54 (s,2H). |
| 2.4.516 | 3,5-Dichlor-Phenyl | Propyl | Ethyl | |
| 2.4.517 | 3,5-Dichlor-Phenyl | Butyl | Ethyl | |
| 2.4.518 | 3-Brom-5-chlor-Phenyl | Methyl | Ethyl | |
| 2.4.519 | 3-Iod-5-chlor-Phenyl | Methyl | Ethyl | |
| 2.4.520 | 3-Methyl-5-chlor-Phenyl | Methyl | Methyl | [CDCl3] 2.36 (s,3H), 2.43 (s,3H); 3.82 (d,1H); 3.86 (s,4H); 7.24 (s,1H); 7.36 (s,1H); 7.46 (s,1H). |
| 2.4.521 | 3-Ethyl-5-chlor-Phenyl | Methyl | Ethyl | |
| 2.4.522 | 3-Propyl-5-chlor-Phenyl | Methyl | Ethyl | |
| 2.4.523 | 3-isoPropyl-5-chlor-Phenyl | Methyl | Ethyl | |
| 2.4.524 | 3-nButyl-5-chlor-Phenyl | Methyl | Ethyl | |
| 2.4.525 | 3-isoButyl-5-chlor-Phenyl | Methyl | Ethyl | |
| 2.4.526 | 3-tert.Butyl-5-chlor-Phenyl | Methyl | Ethyl | |
| 2.4.527 | 3-Cyclopropyl-5-chlor-Phenyl | Methyl | Ethyl | |
| 2.4.528 | 3-Vinyl-5-chlor-Phenyl | Methyl | Ethyl | |
| 2.4.529 | 3-Ethinyl-5-chlor-Phenyl | Methyl | Ethyl | |
| 2.4.530 | 3-Cyano-5-chlor-Phenyl | Methyl | Ethyl | |
| 2.4.531 | 3-Trifluormethyl-5-chlor-Phenyl | Methyl | Ethyl | |
| 2.4.532 | 3-(Hydroxycarbonyl)-5-chlor-Phenyl | Methyl | Ethyl | |
| 2.4.533 | 3-(Methoxycarbony)l-5-chlor-Phenyl | Methyl | Ethyl | |
| 2.4.534 | 3-Hydroxymethyl-5-chlor-Phenyl | Methyl | Ethyl | |
| 2.4.535 | 3-Carbamoyl-5-chlor-Phenyl | Methyl | Ethyl | |
| 2.4.536 | 3-Hydroxy-5-chlor-Phenyl- | Methyl | Ethyl | |
| 2.4.537 | 3-Methoxy-5-chlor-Phenyl | Methyl | Ethyl | |
| 2.4.538 | 3-Ethoxy-5-chlor-Phenyl | Methyl | Ethyl | |
| 2.4.539 | 3-nPropyoxy-5-chlor-Phenyl | Methyl | Ethyl | |
| 2.4.540 | 3-isoPropoxy-5-chlor-Phenyl | Methyl | Ethyl | |
| 2.4.541 | 3-nButoxy-5-chlor-Phenyl | Methyl | Ethyl | |
| 2.4.542 | 3-isoButoxy-5-chlor-Phenyl | Methyl | Ethyl | |
| 2.4.543 | 3-tert.Butoxy-5-chlor-Phenyl | Methyl | Ethyl | |
| 2.4.544 | 3-Difluormethoxy-5-chlor-Phenyl | Methyl | Ethyl | |
| 2.4.545 | 3-Trifluormethoxy-5-chlor-Phenyl | Methyl | Ethyl | |
| 2.4.546 | 3-(2,2,2-Trifluorethoxy)-5-chlorPhenyl | Methyl | Ethyl | |
| 2.4.547 | 3-(2-Chlorethoxy)-5-chlor-Phenyl | Methyl | Ethyl | |
| 2.4.548 | 3-(2-Hydroxyethoxy)-5-chlorPhenyl- | Methyl | Ethyl | |
| 2.4.549 | 3-[(tert-Butoxycarbonyl)oxy]-5-chlor-Phenyl | Methyl | Ethyl | |
| 2.4.550 | 3-Nitro-5-chlor-Phenyl | Methyl | Ethyl | |
| 2.4.551 | 3-Acetoxy-5-chlor-Phenyl | Methyl | Ethyl | |
| 2.4.552 | {3-[(tert-Butoxycarbonyl)amino] -5-chlor-Phenyl} | Methyl | Ethyl | |
| 2.4.553 | 3-Methylsulfanyl-5-chlor-Phenyl | Methyl | Ethyl | |
| 2.4.554 | 3,5-Dibrom-Phenyl | Methyl | Ethyl | |
| 2.4.555 | 3,5-Dibrom-Phenyl | Ethyl | Ethyl | |
| 2.4.556 | 3-Iod-5-brom-Phenyl | Methyl | Ethyl | |
| 2.4.557 | 3-Methyl-5-brom-Phenyl | Methyl | Methyl | [CDCl₃] 2.36 (s, 3H); 2.43 (s, 3H); 3.81 (d, 1H), 3.86 (s, 3H); 3.89 (d, 1H) 7.41 (s,2H); 7.61 (s,1H). |
| 2.4.558 | 3-Methyl-5-brom-Phenyl | Ethyl | Ethyl | |
| 2.4.559 | 3-Methyl-5-brom-Phenyl | Propyl | Ethyl | |
| 2.4.560 | 3-Methyl-5-brom-Phenyl | Butyl | Ethyl | |
| 2.4.561 | 3-Ethyl-5-brom-Phenyl | Methyl | Ethyl | |
| 2.4.562 | 3-Propyl-5-brom-Phenyl | Methyl | Ethyl | |
| 2.4.563 | 3-isoPropyl-5-brom-Phenyl | Methyl | Ethyl | |
| 2.4.564 | 3-nButyl-5-brom-Phenyl | Methyl | Ethyl | |
| 2.4.565 | 3-isoButyl-5-brom-Phenyl | Methyl | Ethyl | |
| 2.4.566 | 3-tert.Butyl-5-brom-Phenyl | Methyl | Ethyl | |
| 2.4.567 | 3-Cyclopropyl-5-brom-Phenyl | Methyl | Ethyl | |
| 2.4.568 | 3-Cyano-5-brom-Phenyl | Methyl | Ethyl | |
| 2.4.569 | 3-Trifluormethyl-5-brom-Phenyl | Methyl | Ethyl | |
| 2.4.570 | 3-(Methoxycarbony)l-5-bromPhenyl | Methyl | Ethyl | |
| 2.4.571 | 3-Methoxy-5-brom-Phenyl | Methyl | Ethyl | |
| 2.4.572 | 3-Ethoxy-5-brom-Phenyl | Methyl | Ethyl | |
| 2.4.573 | 3-nPropyoxy-5-brom-Phenyl | Methyl | Ethyl | |
| 2.4.574 | 3-isoPropoxy-5-brom-Phenyl | Methyl | Ethyl | |
| 2.4.575 | 3-nButoxy-5-brom-Phenyl | Methyl | Ethyl | |
| 2.4.576 | 3-isoButoxy-5-brom-Phenyl | Methyl | Ethyl | |
| 2.4.577 | 3-Difluormethoxy-5-brom-Phenyl | Methyl | Ethyl | |
| 2.4.578 | 3-Trifluormethoxy-5-brom-Phenyl | Methyl | Ethyl | |
| 2.4.579 | 3-Nitro-5-brom-Phenyl | Methyl | Ethyl | |
| 2.4.580 | 3-Acetoxy-5-brom-Phenyl | Methyl | Ethyl | |
| 2.4.581 | 3-Methylsulfanyl-5-brom-Phenyl | Methyl | Ethyl | |
| 2.4.582 | 3,5-Diiod-Phenyl | Methyl | Ethyl | |
| 2.4.583 | 3-Methyl-5-iod-Phenyl | Methyl | Ethyl | |
| 2.4.584 | 3-Ethyl-5-iod-Phenyl | Methyl | Ethyl | |
| 2.4.585 | 3-Propyl-5-iod-Phenyl | Methyl | Ethyl | |
| 2.4.586 | 3-isoPropyl-5-iod-Phenyl | Methyl | Ethyl | |
| 2.4.587 | 3-nButyl-5-iod-Phenyl | Methyl | Ethyl | |
| 2.4.588 | 3-isoButyl-5-iod-Phenyl | Methyl | Ethyl | |
| 2.4.589 | 3-tert.Butyl-5-iod-Phenyl | Methyl | Ethyl | |
| 2.4.590 | 3-Cyclopropyl-5-iod-Phenyl | Methyl | Ethyl | |
| 2.4.591 | 3-Cyano-5-iod-Phenyl | Methyl | Ethyl | |
| 2.4.592 | 3-Trifluormethyl-5-iod-Phenyl | Methyl | Ethyl | |
| 2.4.593 | 3-(Methoxycarbonyl)-5-iod-Phenyl | Methyl | Ethyl | |
| 2.4.594 | 3-Methoxy-5-iod-Phenyl | Methyl | Ethyl | |
| 2.4.595 | 3-Ethoxy-5-iod-Phenyl | Methyl | Ethyl | |
| 2.4.596 | 3-nPropyoxy-5-iod-Phenyl | Methyl | Ethyl | |
| 2.4.597 | 3-isoPropoxy-5-iod-Phenyl | Methyl | Ethyl | |
| 2.4.598 | 3-nButoxy-5-iod-Phenyl | Methyl | Ethyl | |
| 2.4.599 | 3-isoButoxy-5-iod-Phenyl | Methyl | Ethyl | |
| 2.4.600 | 3-Difluormethoxy-5-iod-Phenyl | Methyl | Ethyl | |
| 2.4.601 | 3-Trifluormethoxy-5-iod-Phenyl | Methyl | Ethyl | |
| 2.4.602 | 3-Nitro-5-iod-Phenyl | Methyl | Ethyl | |
| 2.4.603 | 3-Acetoxy-5-iod-Phenyl | Methyl | Ethyl | |
| 2.4.604 | 3-Methylsulfanyl-5-iod-Phenyl | Methyl | Ethyl | |
| 2.4.605 | 3,5-Dimethyl-Phenyl | Methyl | Ethyl | |
| 2.4.606 | 3-Ethyl-5-methyl-Phenyl | Methyl | Ethyl | |
| 2.4.607 | 3-Propyl-5-methyl-Phenyl | Methyl | Ethyl | |
| 2.4.608 | 3-isoPropyl-5-methyl-Phenyl | Methyl | Ethyl | |
| 2.4.609 | 3-nButyl-5-methyl-Phenyl | Methyl | Ethyl | |
| 2.4.610 | 3-isoButyl-5-methyl-Phenyl | Methyl | Ethyl | |
| 2.4.611 | 3-tert.Butyl-5-methyl-Phenyl | Methyl | Ethyl | |
| 2.4.612 | 3-Cyclopropyl-5-methyl-Phenyl | Methyl | Ethyl | |
| 2.4.613 | 3-Cyano-5-methyl-Phenyl | Methyl | Ethyl | |
| 2.4.614 | 3-Trifluormethyl-5-methyl-Phenyl | Methyl | Ethyl | |
| 2.4.615 | 3-(Methoxycarbony)l-5-methylPhenyl | Methyl | Ethyl | |
| 2.4.616 | 3-Methoxy-5-methyl-Phenyl | Methyl | Ethyl | |
| 2.4.617 | 3-Ethoxy-5-methyl-Phenyl | Methyl | Ethyl | |
| 2.4.618 | 3-nPropyoxy-5-methyl-Phenyl | Methyl | Ethyl | |
| 2.4.619 | 3-nButoxy-5-methyl-Phenyl | Methyl | Ethyl | |
| 2.4.620 | 3-isoButoxy-5-methyl-Phenyl | Methyl | Ethyl | |
| 2.4.621 | 3-Difluormethoxy-5-methyl-Phenyl | Methyl | Ethyl | |
| 2.4.622 | 3-Trifluormethoxy-5-methyl-Phenyl | Methyl | Ethyl | |
| 2.4.623 | 3-Nitro-5-methyl-Phenyl | Methyl | Ethyl | |
| 2.4.624 | 3-Acetoxy-5-methyl-Phenyl | Methyl | Ethyl | |
| 2.4.625 | 3-Methylsulfanyl-5-methyl-Phenyl | Methyl | Ethyl | |
| 2.4.626 | 3,5-Diethyl-Phenyl | Methyl | Ethyl | |
| 2.4.627 | 3-Propyl-5-ethyl-Phenyl | Methyl | Ethyl | |
| 2.4.628 | 3-isoPropyl-5-ethyl-Phenyl | Methyl | Ethyl | |
| 2.4.629 | 3-nButyl-5-ethyl-Phenyl | Methyl | Ethyl | |
| 2.4.630 | 3-isoButyl-5-ethyl-Phenyl | Methyl | Ethyl | |
| 2.4.631 | 3-tert.Butyl-5-ethyl-Phenyl | Methyl | Ethyl | |
| 2.4.632 | 3-Cyclopropyl-5-ethyl-Phenyl | Methyl | Ethyl | |
| 2.4.633 | 3-Cyano-5-ethyl-Phenyl | Methyl | Ethyl | |
| 2.4.634 | 3-Trifluormethyl-5-ethyl-Phenyl | Methyl | Ethyl | |
| 2.4.635 | 3-(Methoxycarbony)l-5-ethyl-Phenyl | Methyl | Ethyl | |
| 2.4.636 | 3-Methoxy-5-ethyl-Phenyl | Methyl | Ethyl | |
| 2.4.637 | 3-Ethoxy-5-ethyl-Phenyl | Methyl | Ethyl | |
| 2.4.638 | 3-nPropyoxy-5-ethyl-Phenyl | Methyl | Ethyl | |
| 2.4.639 | 3-nButoxy-5-ethyl-Phenyl | Methyl | Ethyl | |
| 2.4.640 | 3-isoButoxy-5-ethyl-Phenyl | Methyl | Ethyl | |
| 2.4.641 | 3-Difluormethoxy-5-ethyl-Phenyl | Methyl | Ethyl | |
| 2.4.642 | 3-Trifluormethoxy-5-ethyl-Phenyl | Methyl | Ethyl | |
| 2.4.643 | 3-Nitro-5-ethyl-Phenyl | Methyl | Ethyl | |
| 2.4.644 | 3-Acetoxy-5-ethyl-Phenyl | Methyl | Ethyl | |
| 2.4.645 | 3-Methylsulfanyl-5-ethyl-Phenyl | Methyl | Ethyl | |
| 2.4.646 | 3,5-Dipropyl-Phenyl | Methyl | Ethyl | |
| 2.4.647 | 3-isoPropyl-5-propyl-Phenyl | Methyl | Ethyl | |
| 2.4.648 | 3-nButyl-5-propyl-Phenyl | Methyl | Ethyl | |
| 2.4.649 | 3-isoButyl-5-propyl-Phenyl | Methyl | Ethyl | |
| 2.4.650 | 3-tert.Butyl-5-propyl-Phenyl | Methyl | Ethyl | |
| 2.4.651 | 3-Cyclopropyl-5-propyl-Phenyl | Methyl | Ethyl | |
| 2.4.652 | 3-Cyano-5-propyl-Phenyl | Methyl | Ethyl | |
| 2.4.653 | 3-Trifluormethyl-5-propyl-Phenyl | Methyl | Ethyl | |
| 2.4.654 | 3-(Hydroxycarbonyl)-5-propylPhenyl | Methyl | Ethyl | |
| 2.4.655 | 3-(Methoxycarbonyl)-5-propylPhenyl | Methyl | Ethyl | |
| 2.4.656 | 3-Methoxy-5-propyl-Phenyl | Methyl | Ethyl | |
| 2.4.657 | 3-Ethoxy-5-propyl-Phenyl | Methyl | Ethyl | |
| 2.4.658 | 3-nPropyoxy-5-propyl-Phenyl | Methyl | Ethyl | |
| 2.4.659 | 3-nButoxy-5-propyl-Phenyl | Methyl | Ethyl | |
| 2.4.660 | 3-isoButoxy-5-propyl-Phenyl | Methyl | Ethyl | |
| 2.4.661 | 3-Difluormethoxy-5-propyl-Phenyl | Methyl | Ethyl | |
| 2.4.662 | 3-Trifluormethoxy-5-ethyl-Phenyl | Methyl | Ethyl | |
| 2.4.663 | 3-Nitro-5-propyl-Phenyl | Methyl | Ethyl | |
| 2.4.664 | 3-Acetoxy-5-propyl-Phenyl | Methyl | Ethyl | |
| 2.4.665 | 3-Methylsulfanyl-5-propyl-Phenyl | Methyl | Ethyl | |
| 2.4.666 | 3,5-Diisopropyl-Phenyl | Methyl | Ethyl | |
| 2.4.667 | 3-nButyl-5-isopropyl-Phenyl | Methyl | Ethyl | |
| 2.4.668 | 3-isoButyl-5-isopropyl-Phenyl | Methyl | Ethyl | |
| 2.4.669 | 3-tert.Butyl-5-isopropyl-Phenyl | Methyl | Ethyl | |
| 2.4.670 | 3-Cyclopropyl-5-isopropyl-Phenyl | Methyl | Ethyl | |
| 2.4.671 | 3-Cyano-5-isopropyl-Phenyl | Methyl | Ethyl | |
| 2.4.672 | 3-Trifluormethyl-5-isopropyl-Phenyl | Methyl | Ethyl | |
| 2.4.673 | 3-(Methoxycarbony)l-5-isopropylPhenyl | Methyl | Ethyl | |
| 2.4.674 | 3-Methoxy-5-isopropyl-Phenyl | Methyl | Ethyl | |
| 2.4.675 | 3-Ethoxy-5-isopropyl-Phenyl | Methyl | Ethyl | |
| 2.4.676 | 3-nPropyoxy-5-isopropyl-Phenyl | Methyl | Ethyl | |
| 2.4.677 | 3-nButoxy-5-isopropyl-Phenyl | Methyl | Ethyl | |
| 2.4.678 | 3-isoButoxy-5-isopropyl-Phenyl | Methyl | Ethyl | |
| 2.4.679 | 3-Difluormethoxy-5-isopropylPhenyl | Methyl | Ethyl | |
| 2.4.680 | 3-Trifluormethoxy-5-isopropylPhenyl | Methyl | Ethyl | |
| 2.4.681 | 3-Nitro-5-isopropyl-Phenyl | Methyl | Ethyl | |
| 2.4.682 | 3-Acetoxy-5-isopropyl-Phenyl | Methyl | Ethyl | |
| 2.4.683 | 3-Methylsulfanyl-5-isopropyl-Phenyl | Methyl | Ethyl | |
| 2.4.684 | 3,5-Dibutyl-Phenyl | Methyl | Ethyl | |
| 2.4.685 | 3-isoButyl-5-butyl-Phenyl | Methyl | Ethyl | |
| 2.4.686 | 3-tert.Butyl-5-butyl-Phenyl | Methyl | Ethyl | |
| 2.4.687 | 3-Cyclopropyl-5-butyl-Phenyl | Methyl | Ethyl | |
| 2.4.688 | 3-Cyano-5-butyl-Phenyl | Methyl | Ethyl | |
| 2.4.689 | 3-Trifluormethyl-5-butyl-Phenyl | Methyl | Ethyl | |
| 2.4.690 | 3-(Methoxycarbony)l-5-butyl-Phenyl | Methyl | Ethyl | |
| 2.4.691 | 3-Methoxy-5-butyl-Phenyl | Methyl | Ethyl | |
| 2.4.692 | 3-Ethoxy-5-butyl-Phenyl | Methyl | Ethyl | |
| 2.4.693 | 3-nPropyoxy-5-butyl-Phenyl | Methyl | Ethyl | |
| 2.4.694 | 3-nButoxy-5-butyl-Phenyl | Methyl | Ethyl | |
| 2.4.695 | 3-isoButoxy-5-butyl-Phenyl | Methyl | Ethyl | |
| 2.4.696 | 3-Difluormethoxy-5-butyl-Phenyl | Methyl | Ethyl | |
| 2.4.697 | 3-Trifluormethoxy-5-butyl-Phenyl | Methyl | Ethyl | |
| 2.4.698 | 3-Nitro-5-butyl-Phenyl | Methyl | Ethyl | |
| 2.4.699 | 3-Acetoxy-5-butyl-Phenyl | Methyl | Ethyl | |
| 2.4.700 | 3-Methylsulfanyl-5-butyl-Phenyl | Methyl | Ethyl | |
| 2.4.701 | 3,5-Diisobutyl-Phenyl | Methyl | Ethyl | |
| 2.4.702 | 3-tert.Butyl-5-isobutyl-Phenyl | Methyl | Ethyl | |
| 2.4.703 | 3-Cyclopropyl-5-isobutyl-Phenyl | Methyl | Ethyl | |
| 2.4.704 | 3-Cyano-5-isobutyl-Phenyl | Methyl | Ethyl | |
| 2.4.705 | 3-Trifluormethyl-5-isobutyl-Phenyl | Methyl | Ethyl | |
| 2.4.706 | 3-(Methoxycarbonyl)-5-isobutylPhenyl | Methyl | Ethyl | |
| 2.4.707 | 3-Methoxy-5-isobutyl-Phenyl | Methyl | Ethyl | |
| 2.4.708 | 3-Ethoxy-5-isobutyl-Phenyl | Methyl | Ethyl | |
| 2.4.709 | 3-nPropyoxy-5-isobutyl-Phenyl | Methyl | Ethyl | |
| 2.4.710 | 3-nButoxy-5-isobutyl-Phenyl | Methyl | Ethyl | |
| 2.4.711 | 3-isoButoxy-5-isobutyl-Phenyl | Methyl | Ethyl | |
| 2.4.712 | 3-Difluormethoxy-5-isobutyl-Phenyl | Methyl | Ethyl | |
| 2.4.713 | 3-Trifluormethoxy-5-isobutyl-Phenyl | Methyl | Ethyl | |
| 2.4.714 | 3-Nitro-5-isobutyl-Phenyl | Methyl | Ethyl | |
| 2.4.715 | 3-Acetoxy-5-isobutyl-Phenyl | Methyl | Ethyl | |
| 2.4.716 | 3-Methylsulfanyl-5-isobutyl-Phenyl | Methyl | Ethyl | |
| 2.4.717 | 3,5-D(itert.butyl)-Phenyl | Methyl | Ethyl | |
| 2.4.718 | 3-Cyclopropyl-5-tert.butyl-Phenyl | Methyl | Ethyl | |
| 2.4.719 | 3-Cyano-5-tert.butyl-Phenyl | Methyl | Ethyl | |
| 2.4.720 | 3-Trifluormethyl-5-tert.butyl-Phenyl | Methyl | Ethyl | |
| 2.4.721 | 3-(Methoxycarbonyl)-5-ter.butylPhenyl | Methyl | Ethyl | |
| 2.4.722 | 3-Methoxy-5-tert.butyl-Phenyl | Methyl | Ethyl | |
| 2.4.723 | 3-Ethoxy-5-tert.butyl-Phenyl | Methyl | Ethyl | |
| 2.4.724 | 3-nPropyoxy-5-tert.butyl-Phenyl | Methyl | Ethyl | |
| 2.4.725 | 3-nButoxy-5-tert.butyl-Phenyl | Methyl | Ethyl | |
| 2.4.726 | 3-isoButoxy-5-tert.butyl-Phenyl | Methyl | Ethyl | |
| 2.4.727 | 3-Difluormethoxy-5-tert.butylPhenyl | Methyl | Ethyl | |
| 2.4.728 | 3-Trifluormethoxy-5-tert.butylPhenyl | Methyl | Ethyl | |
| 2.4.729 | 3-Nitro-5-tert.butyl-Phenyl | Methyl | Ethyl | |
| 2.4.730 | 3-Acetoxy-5-tert.butyl-Phenyl | Methyl | Ethyl | |
| 2.4.731 | 3-Methylsulfanyl-5-tert.butyl-Phenyl | Methyl | Ethyl | |
| 2.4.732 | 3-tert.Butyl-5-cyclopropyl-Phenyl | Methyl | Ethyl | |
| 2.4.733 | 3,5-Dicyclopropyl-Phenyl | Methyl | Ethyl | |
| 2.4.734 | 3-Cyano-5-cyclopropyl-Phenyl | Methyl | Ethyl | |
| 2.4.735 | 3-Trifluormethyl-5-cyclopropyl-Phenyl | Methyl | Ethyl | |
| 2.4.736 | 3-(Methoxycarbonyl)-5-cyclopropyl-Phenyl | Methyl | Ethyl | |
| 2.4.737 | 3-Methoxy-5-cyclopropyl-Phenyl | Methyl | Ethyl | |
| 2.4.738 | 3-Ethoxy-5-cyclopropyl-Phenyl | Methyl | Ethyl | |
| 2.4.739 | 3-nPropyoxy-5-cyclopropyl-Phenyl | Methyl | Ethyl | |
| 2.4.740 | 3-nButoxy-5-cyclopropyl-Phenyl | Methyl | Ethyl | |
| 2.4.741 | 3-isoButoxy-5-cyclopropyl-Phenyl | Methyl | Ethyl | |
| 2.4.742 | 3-Difluormethoxy-5-cyclopropyl-Phenyl | Methyl | Ethyl | |
| 2.4.743 | 3-Trifluormethoxy-5-cyclopropyl-Phenyl | Methyl | Ethyl | |
| 2.4.744 | 3-Nitro-5-cyclopropyl-Phenyl | Methyl | Ethyl | |
| 2.4.745 | 3-Acetoxy-5-cyclopropyl-Phenyl | Methyl | Ethyl | |
| 2.4.746 | 3-Methylsulfanyl-5-cyclopropyl-Phenyl | Methyl | Ethyl | |
| 2.4.747 | 3,5-Dicyano-Phenyl | Methyl | Ethyl | |
| 2.4.748 | 3-Trifluormethyl-5-cyano-Phenyl | Methyl | Ethyl | |
| 2.4.749 | 3-(Methoxycarbonyl)-5-cyano-Phenyl | Methyl | Ethyl | |
| 2.4.750 | 3-Methoxy-5-cyano-Phenyl | Methyl | Ethyl | |
| 2.4.751 | 3-Ethoxy-5-cyano-Phenyl | Methyl | Ethyl | |
| 2.4.752 | 3-nPropyoxy-5-cyano-Phenyl | Methyl | Ethyl | |
| 2.4.753 | 3-nButoxy-5-cyano-Phenyl | Methyl | Ethyl | |
| 2.4.754 | 3-isoButoxy-5-cyano-Phenyl | Methyl | Ethyl | |
| 2.4.755 | 3-Difluormethoxy-5-cyano-Phenyl | Methyl | Ethyl | |
| 2.4.756 | 3-Trifluormethoxy-5-cyano-Phenyl | Methyl | Ethyl | |
| 2.4.757 | 3-Nitro-5-cyano-Phenyl | Methyl | Ethyl | |
| 2.4.758 | 3-Acetoxy-5-cyano-Phenyl | Methyl | Ethyl | |
| 2.4.759 | 3-Methylsulfanyl-5-cyano-Phenyl | Methyl | Ethyl | |
| 2.4.760 | 3,5-Di(trifluormethyl)-Phenyl | Methyl | Ethyl | |
| 2.4.761 | 3-(Methoxycarbonyl)-5-trifluormethyl-Phenyl | Methyl | Ethyl | |
| 2.4.762 | 3-Methoxy-5-trifluormethyl-Phenyl | Methyl | Ethyl | |
| 2.4.763 | 3-Ethoxy-5trifluormethyl-Phenyl | Methyl | Ethyl | |
| 2.4.764 | 3-nPropyoxy-5-trifluormethylPhenyl | Methyl | Ethyl | |
| 2.4.765 | 3-nButoxy-5-trifluormethyl-Phenyl | Methyl | Ethyl | |
| 2.4.766 | 3-isoButoxy-5-trifluormethyl-Phenyl | Methyl | Ethyl | |
| 2.4.767 | 3-Difluormethoxy-5-trifluormethylPhenyl | Methyl | Ethyl | |
| 2.4.768 | 3-Trifluormethoxy-5-trifluormethylPhenyl | Methyl | Ethyl | |
| 2.4.769 | 3-Nitro-5-trifluormethyl-Phenyl | Methyl | Ethyl | |
| 2.4.770 | 3-Acetoxy-5-trifluormethyl-Phenyl | Methyl | Ethyl | |
| 2.4.771 | 3-Methylsulfanyl-5-trifluormethylPhenyl | Methyl | Ethyl | |
| 2.4.772 | 3,5-Di(methoxycarbonyl)-Phenyl | Methyl | Ethyl | |
| 2.4.773 | 3-Methoxy-5-(methoxycarbonyl)-Phenyl | Methyl | Ethyl | |
| 2.4.774 | 3-Ethoxy-5-(methoxycarbonyl)-Phenyl | Methyl | Ethyl | |
| 2.4.775 | 3-nPropyoxy-5-(methoxycarbonyl)-Phenyl | Methyl | Ethyl | |
| 2.4.776 | 3-isoButoxy-5-(methoxycarbonyl)-Phenyl | Methyl | Ethyl | |
| 2.4.777 | 3-Difluormethoxy-5-(methoxycarbonyl)-Phenyl | Methyl | Ethyl | |
| 2.4.778 | 3-Trifluormethoxy-5-(methoxycarbonyl)-Phenyl | Methyl | Ethyl | |
| 2.4.779 | 3-Nitro-5-(methoxycarbonyl)-Phenyl | Methyl | Ethyl | |
| 2.4.780 | 3-Acetoxy-5-(methoxycarbonyl)-Phenyl | Methyl | Ethyl | |
| 2.4.781 | 3-Methylsulfanyl-5-(methoxycarbonyl)-Phenyl | Methyl | Ethyl | |
| 2.4.782 | 3,5-Dimethoxy-Phenyl | Methyl | Ethyl | |
| 2.4.783 | 3-Ethoxy-5-methoxy-Phenyl | Methyl | Ethyl | |
| 2.4.784 | 3-nPropyoxy-5-methoxy-Phenyl | Methyl | Ethyl | |
| 2.4.785 | 3-nButoxy-5-methoxy-Phenyl | Methyl | Ethyl | |
| 2.4.786 | 3-isoButoxy-5-methoxy-Phenyl | Methyl | Ethyl | |
| 2.4.787 | 3-Difluormethoxy-5-methoxyPhenyl | Methyl | Ethyl | |
| 2.4.788 | 3-Trifluormethoxy-5-methoxyPhenyl | Methyl | Ethyl | |
| 2.4.789 | 3-Nitro-5-methoxy-Phenyl | Methyl | Ethyl | |
| 2.4.790 | 3-Acetoxy-5-methoxy-Phenyl | Methyl | Ethyl | |
| 2.4.791 | 3-Methylsulfanyl-5-methoxy-Phenyl | Methyl | Ethyl | |
| 2.4.792 | 3,5-Diethoxy-Phenyl | Methyl | Ethyl | |
| 2.4.793 | 3-nPropyoxy-5-ethoxy-Phenyl | Methyl | Ethyl | |
| 2.4.794 | 3-nButoxy-5-ethoxy-Phenyl | Methyl | Ethyl | |
| 2.4.795 | 3-isoButoxy-5-ethoxy-Phenyl | Methyl | Ethyl | |
| 2.4.796 | 3-Difluormethoxy-5-ethoxy-Phenyl | Methyl | Ethyl | |
| 2.4.797 | 3-Trifluormethoxy-5-ethoxy-Phenyl | Methyl | Ethyl | |
| 2.4.798 | 3-Nitro-5-ethoxy-Phenyl | Methyl | Ethyl | |
| 2.4.799 | 3-Acetoxy-5-ethoxy-Phenyl | Methyl | Ethyl | |
| 2.4.800 | 3-Methylsulfanyl-5-ethoxy-Phenyl | Methyl | Ethyl | |
| 2.4.801 | 3,5-Dipropyoxy-Phenyl | Methyl | Ethyl | |
| 2.4.802 | 3-nButoxy-5-propoxy-Phenyl | Methyl | Ethyl | |
| 2.4.803 | 3-isoButoxy-5-propoxy-Phenyl | Methyl | Ethyl | |
| 2.4.804 | 3-Difluormethoxy-5-propoxy-Phenyl | Methyl | Ethyl | |
| 2.4.805 | 3-Trifluormethoxy-5-propoxy-Phenyl | Methyl | Ethyl | |
| 2.4.806 | 3-Nitro-5-propoxy-Phenyl | Methyl | Ethyl | |
| 2.4.807 | 3-Acetoxy-5-propoxy-Phenyl | Methyl | Ethyl | |
| 2.4.808 | 3-Methylsulfanyl-5-propoxy-Phenyl | Methyl | Ethyl | |
| 2.4.809 | 3,5-Di(isopropyoxy)-Phenyl | Methyl | Ethyl | |
| 2.4.810 | 3-nButoxy-5-isopropoxy-Phenyl | Methyl | Ethyl | |
| 2.4.811 | 3-isoButoxy-5-isopropoxy-Phenyl | Methyl | Ethyl | |
| 2.4.812 | 3-Difluormethoxy-5-isopropoxy-Phenyl | Methyl | Ethyl | |
| 2.4.813 | 3-Trifluormethoxy-5-isopropoxy-Phenyl | Methyl | Ethyl | |
| 2.4.814 | 3-Nitro-5-isopropoxy-Phenyl | Methyl | Ethyl | |
| 2.4.815 | 3-Acetoxy-5-isopropoxy-Phenyl | Methyl | Ethyl | |
| 2.4.816 | 3-Methylsulfanyl-5-isopropoxy-Phenyl | Methyl | Ethyl | |
| 2.4.817 | 3,5-Di(trifluormethoxy)-Phenyl | Methyl | Ethyl | |
| 2.4.818 | 3-Nitro-5-trifluormethoxy-Phenyl | Methyl | Ethyl | |
| 2.4.819 | 3-Acetoxy-5-tert.butoxy-Phenyl | Methyl | Ethyl | |
| 2.4.820 | 3-Methylsulfanyl-5-trifluormethoxy-Phenyl | Methyl | Ethyl | |
| 2.4.821 | 3,5-Bis(difluormethoxy)-Phenyl | Methyl | Ethyl | |
| 2.4.822 | 3,5-Bis(difluormethoxy)-Phenyl | Ethyl | Ethyl | |
| 2.4.823 | 3,5-Bis(difluormethoxy)-Phenyl | Propyl | Ethyl | |
| 2.4.824 | 3,5-Bis(difluormethoxy)-Phenyl | Butyl | Ethyl | |
| 2.4.825 | 3-Trifluormethoxy-5-difluormethoxy-Phenyl | Methyl | Ethyl | |
| 2.4.826 | 3-Nitro-5-difluormethoxy-Phenyl | Methyl | Ethyl | |
| 2.4.827 | 3-Acetoxy-5-difluormethoxy-Phenyl | Methyl | Ethyl | |
| 2.4.828 | 3-Methylsulfanyl-5-difluormethoxy-Phenyl | Methyl | Ethyl | |
| 2.4.829 | 3,5-Bis(acetoxy)-Phenyl | Methyl | Ethyl | |
| 2.4.830 | 3-Methylsulfanyl-5-acetoxy-Phenyl | Methyl | Ethyl | |
| 2.4.831 | 3-Acetoxy-5-nitro-Phenyl | Methyl | Ethyl | |
| 2.4.832 | 3-Methylsulfanyl-5-nitro-Phenyl | Methyl | Ethyl | |
| 2.4.833 | 3,5-Di(methylsulfanyl)-Phenyl | Methyl | Ethyl | |
| 2.4.834 | 3,4-Difluor-Phenyl | Methyl | Ethyl | |
| 2.4.835 | 3,4-Difluor-Phenyl | Ethyl | Ethyl | |
| 2.4.836 | 3,4-Difluor-Phenyl | Propyl | Ethyl | |
| 2.4.837 | 3,4-Difluor-Phenyl | Butyl | Ethyl | |
| 2.4.838 | 3-Chlor-4-fluor-Phenyl | Methyl | Ethyl | |
| 2.4.839 | 3-Chlor-4-fluor-Phenyl | Ethyl | Ethyl | |
| 2.4.840 | 3-Chlor-4-fluor-Phenyl | Propyl | Ethyl | |
| 2.4.841 | 3-Chlor-4-fluor-Phenyl | Butyl | Ethyl | |
| 2.4.842 | 3-Brom-4-fluor-Phenyl | Methyl | Ethyl | |
| 2.4.843 | 3-Methyl-4-fluor-Phenyl | Methyl | Ethyl | |
| 2.4.844 | 3-Methyl-4-fluor-Phenyl | Ethyl | Ethyl | |
| 2.4.845 | 3-Ethyl-4-fluor-Phenyl | Methyl | Ethyl | |
| 2.4.846 | 3-Cyclopropyl-4-fluor-Phenyl | Methyl | Ethyl | |
| 2.4.847 | 3-Cyano-4-fluor-Phenyl | Methyl | Ethyl | |
| 2.4.848 | 3-Methoxy-4-fluor-Phenyl | Methyl | Ethyl | |
| 2.4.849 | 3-Ethoxy-4-fluor-Phenyl | Methyl | Ethyl | |
| 2.4.850 | 3-Trifluormethoxy-4-fluor-Phenyl | Methyl | Ethyl | |
| 2.4.851 | 3-Nitro-4-fluor-Phenyl | Methyl | Ethyl | |
| 2.4.852 | 3-Fluor-4-chlor-Phenyl | Methyl | Ethyl | |
| 2.4.853 | 3,4-Dichlor-Phenyl | Methyl | Ethyl | |
| 2.4.854 | 3-Brom-4-chlor-Phenyl | Methyl | Ethyl | |
| 2.4.855 | 3-Methyl-4-chlor-Phenyl | Methyl | Ethyl | |
| 2.4.856 | 3-Cyclopropyl-4-chlor-Phenyl | Methyl | Ethyl | |
| 2.4.857 | 3-Cyano-4-chlor-Phenyl | Methyl | Ethyl | |
| 2.4.858 | 3-Trifluormethyl-4-chlor-Phenyl | Methyl | Ethyl | |
| 2.4.859 | 3-Methoxy-4-chlor-Phenyl | Methyl | Ethyl | |
| 2.4.860 | 3-Ethoxy-4-chlor-Phenyl | Methyl | Ethyl | |
| 2.4.861 | 3-Trifluormethoxy-4-chlor-Phenyl | Methyl | Ethyl | |
| 2.4.862 | 3-Nitro-4-chlor-Phenyl | Methyl | Ethyl | |
| 2.4.863 | 3-Fluor-4-brom-Phenyl | Methyl | Ethyl | |
| 2.4.864 | 3-Chlor-4-brom-Phenyl | Methyl | Ethyl | |
| 2.4.865 | 3,4-Dibrom-Phenyl | Methyl | Ethyl | |
| 2.4.866 | 3-Methyl-4-brom-Phenyl | Methyl | Ethyl | |
| 2.4.867 | 3-Cyclopropyl-4-brom-Phenyl | Methyl | Ethyl | |
| 2.4.868 | 3-Cyano-4-brom-Phenyl | Methyl | Ethyl | |
| 2.4.869 | 3-Trifluormethyl-4-brom-Phenyl | Methyl | Ethyl | |
| 2.4.870 | 3-Methoxy-4-Phenyl | Methyl | Ethyl | |
| 2.4.871 | 3-Ethoxy-4-brom-Phenyl | Methyl | Ethyl | |
| 2.4.872 | 3-Trifluormethoxy-4-brom-Phenyl | Methyl | Ethyl | |
| 2.4.873 | 3-Nitro-4-brom-Phenyl | Methyl | Ethyl | |
| 2.4.874 | 3-Fluor-4-iod-Phenyl | Methyl | Ethyl | |
| 2.4.875 | 3-Chlor-4-iod-Phenyl | Methyl | Ethyl | |
| 2.4.876 | 3-Brom-4-iod-Phenyl | Methyl | Ethyl | |
| 2.4.877 | 3-Methyl-4-iod-Phenyl | Methyl | Ethyl | |
| 2.4.878 | 3-Cyclopropyl-4-iod-Phenyl | Methyl | Ethyl | |
| 2.4.879 | 3-Cyano-4-iod-Phenyl | Methyl | Ethyl | |
| 2.4.880 | 3-Trifluormethyl-4-iod-Phenyl | Methyl | Ethyl | |
| 2.4.881 | 3-Methoxy-4-iod-Phenyl | Methyl | Ethyl | |
| 2.4.882 | 3-Ethoxy-4-iod-Phenyl | Methyl | Ethyl | |
| 2.4.883 | 3-Trifluormethoxy-4-iod-Phenyl | Methyl | Ethyl | |
| 2.4.884 | 3-Nitro-4-iod-Phenyl | Methyl | Ethyl | |
| 2.4.885 | 3-Fluor-4-methyl-Phenyl | Methyl | Ethyl | |
| 2.4.886 | 3-Chlor-4-methyl-Phenyl | Methyl | Ethyl | |
| 2.4.887 | 3-Brom-4-methyl-Phenyl | Methyl | Ethyl | |
| 2.4.888 | 3.4-Dimethyl-Phenyl | Methyl | Ethyl | |
| 2.4.889 | 3.4-Dimethyl-Phenyl | Ethyl | Ethyl | |
| 2.4.890 | 3.4-Dimethyl-Phenyl | Propyl | Ethyl | |
| 2.4.891 | 3.4-Dimethyl-Phenyl | Butyl | Ethyl | |
| 2.4.892 | 3-Cyclopropyl-4-methyl-Phenyl | Methyl | Ethyl | |
| 2.4.893 | 3-Cyano-4-methyl-Phenyl | Methyl | Ethyl | |
| 2.4.894 | 3-Trifluormethyl-4-methyl-Phenyl | Methyl | Ethyl | |
| 2.4.895 | 3-Methoxy-4-methyl-Phenyl | Methyl | Ethyl | |
| 2.4.896 | 3-Ethoxy-4-methyl-Phenyl | Methyl | Ethyl | |
| 2.4.897 | 3-Trifluormethoxy-4-methyl-Phenyl | Methyl | Ethyl | |
| 2.4.898 | 3-Nitro-4-methyl-Phenyl | Methyl | Ethyl | |
| 2.4.899 | 3-Fluor-4-ethyl-Phenyl | Methyl | Ethyl | |
| 2.4.900 | 3-Chlor-4-ethyl-Phenyl | Methyl | Ethyl | |
| 2.4.901 | 3-Brom-4-ethyl-Phenyl | Methyl | Ethyl | |
| 2.4.902 | 3-Methyl-4-ethyl-Phenyl | Methyl | Ethyl | |
| 2.4.903 | 3,4-Diethyl-Phenyl | Methyl | Ethyl | |
| 2.4.904 | 3-Cyclopropyl-4-ethyl-Phenyl | Methyl | Ethyl | |
| 2.4.905 | 3-Cyano-4-ethyl-Phenyl | Methyl | Ethyl | |
| 2.4.906 | 3-Trifluormethyl-4-ethyl-Phenyl | Methyl | Ethyl | |
| 2.4.907 | 3-Methoxy-4-ethyl-Phenyl | Methyl | Ethyl | |
| 2.4.908 | 3-Ethoxy-4-ethyl-Phenyl | Methyl | Ethyl | |
| 2.4.909 | 3-Trifluormethoxy-4-ethyl-Phenyl | Methyl | Ethyl | |
| 2.4.910 | 3-Nitro-4-ethyl-Phenyl | Methyl | Ethyl | |
| 2.4.911 | 3-Fluor-4-isopropyl-Phenyl | Methyl | Ethyl | |
| 2.4.912 | 3-Chlor-4-isopropyl-Phenyl | Methyl | Ethyl | |
| 2.4.913 | 3-Brom-4-isopropyl-Phenyl | Methyl | Ethyl | |
| 2.4.914 | 3-Methyl-4-isopropyl-Phenyl | Methyl | Ethyl | |
| 2.4.915 | 3-Cyclopropyl-4-isopropyl-Phenyl | Methyl | Ethyl | |
| 2.4.916 | 3-Cyano-4-isopropyl-Phenyl | Methyl | Ethyl | |
| 2.4.917 | 3-Trifluormethyl-4-isopropyl-Phenyl | Methyl | Ethyl | |
| 2.4.918 | 3-Methoxy-4-isopropyl-Phenyl | Methyl | Ethyl | |
| 2.4.919 | 3-Ethoxy-4-isopropyl-Phenyl | Methyl | Ethyl | |
| 2.4.920 | 3-Trifluormethoxy-4-isopropylPhenyl | Methyl | Ethyl | |
| 2.4.921 | 3-Nitro-4-isopropyl-Phenyl | Methyl | Ethyl | |
| 2.4.922 | 3-Fluor-4-tert.butyl-Phenyl | Methyl | Ethyl | |
| 2.4.923 | 3-Chlor-4-tert.butyl-Phenyl | Methyl | Ethyl | |
| 2.4.924 | 3-Brom-4-tert.butyl-Phenyl | Methyl | Ethyl | |
| 2.4.925 | 3-Methyl-4-tert.butyl-Phenyl | Methyl | Ethyl | |
| 2.4.926 | 3-Ethyl-4-tert.butyl-Phenyl | Methyl | Ethyl | |
| 2.4.927 | 3-Cyclopropyl-4-tert.butyl-Phenyl | Methyl | Ethyl | |
| 2.4.928 | 3-Cyano-4-tert.butyl-Phenyl | Methyl | Ethyl | |
| 2.4.929 | 3-Trifluormethyl-4-tert.butyl-Phenyl | Methyl | Ethyl | |
| 2.4.930 | 3-Trifluormethyl-4-tert.butyl-Phenyl | Ethyl | Ethyl | |
| 2.4.931 | 3-Trifluormethyl-4-tert.butyl-Phenyl | Propyl | Ethyl | |
| 2.4.932 | 3-Trifluormethyl-4-tert.butyl-Phenyl | Butyl | Ethyl | |
| 2.4.933 | 3-Methoxy-4-tert.butyl-Phenyl | Methyl | Ethyl | |
| 2.4.934 | 3-Ethoxy-4-tert.butyl-Phenyl | Methyl | Ethyl | |
| 2.4.935 | 3-Trifluormethoxy-4-tert.butylPhenyl | Methyl | Ethyl | |
| 2.4.936 | 3-Nitro-4-tert.butyl-Phenyl | Methyl | Ethyl | |
| 2.4.937 | 3-Fluor-4-cyclopropyl-Phenyl | Methyl | Ethyl | |
| 2.4.938 | 3-Chlor-4-cyclopropyl-Phenyl | Methyl | Ethyl | |
| 2.4.939 | 3-Brom-4-cyclopropyl-Phenyl | Methyl | Ethyl | |
| 2.4.940 | 3-Methyl-4-cyclopropyl-Phenyl | Methyl | Ethyl | |
| 2.4.941 | 3-Cyclopropyl-4-cyclopropyl-Phenyl | Methyl | Ethyl | |
| 2.4.942 | 3-Cyano-4-cyclopropyl-Phenyl | Methyl | Ethyl | |
| 2.4.943 | 3-Trifluormethyl-4-cyclopropyl-Phenyl | Methyl | Ethyl | |
| 2.4.944 | 3-Methoxy-4-cyclopropyl-Phenyl | Methyl | Ethyl | |
| 2.4.945 | 3-Ethoxy-4-cyclopropyl-Phenyl | Methyl | Ethyl | |
| 2.4.946 | 3-Trifluormethoxy-4-cyclopropyl-Phenyl | Methyl | Ethyl | |
| 2.4.947 | 3-Fluor-4-methoxycarbonyl-Phenyl | Methyl | Ethyl | |
| 2.4.948 | 3-Chlor-4-methoxycarbonyl-Phenyl | Methyl | Ethyl | |
| 2.4.949 | 3-Brom-4-methoxycarbonyl-Phenyl | Methyl | Ethyl | |
| 2.4.950 | 3-Methyl-4-methoxycarbonyl-Phenyl | Methyl | Ethyl | |
| 2.4.951 | 3-Cyclopropyl-4-methoxycarbonyl-Phenyl | Methyl | Ethyl | |
| 2.4.952 | 3-Cyano-4-methoxycarbonyl-Phenyl | Methyl | Ethyl | |
| 2.4.953 | 3-Trifluormethyl-4-methoxycarbonyl-Phenyl | Methyl | Ethyl | |
| 2.4.954 | 3-Methoxy-4-methoxycarbonyl - Phenyl | Methyl | Ethyl | |
| 2.4.955 | 3-Ethoxy-4-methoxycarbonyl-Phenyl | Methyl | Ethyl | |
| 2.4.956 | 3-Trifluormethoxy-4-methoxycarbonyl-Phenyl | Methyl | Ethyl | |
| 2.4.957 | 3-Nitro-4-methoxycarbonyl-Phenyl | Methyl | Ethyl | |
| 2.4.958 | 3-Fluor-4-cyano-Phenyl | Methyl | Ethyl | |
| 2.4.959 | 3-Chlor-4-cyano-Phenyl | Methyl | Ethyl | |
| 2.4.960 | 3-Brom-4-cyano-Phenyl | Methyl | Ethyl | |
| 2.4.961 | 3-Methyl-4-cyano-Phenyl | Methyl | Ethyl | |
| 2.4.962 | 3-Cyclopropyl-4-cyano-Phenyl | Methyl | Ethyl | |
| 2.4.963 | 3,4-Dicyano-Phenyl | Methyl | Ethyl | |
| 2.4.964 | 3-Trifluormethyl-4-cyano-Phenyl | Methyl | Ethyl | |
| 2.4.965 | 3-Trifluormethyl-4-cyano-Phenyl | Ethyl | Ethyl | |
| 2.4.966 | 3-Trifluormethyl-4-cyano-Phenyl | Propyl | Ethyl | |
| 2.4.967 | 3-Trifluormethyl-4-cyano-Phenyl | Butyl | Ethyl | |
| 2.4.968 | 3-Methoxy-4-cyano-Phenyl | Methyl | Ethyl | |
| 2.4.969 | 3-Ethoxy-4-cyano-Phenyl | Methyl | Ethyl | |
| 2.4.970 | 3-Trifluormethoxy-4-cyano-Phenyl | Methyl | Ethyl | |
| 2.4.971 | 3-Nitro-4-cyano-Phenyl | Methyl | Ethyl | |
| 2.4.972 | 3-Fluor-4-methoxy-Phenyl | Methyl | Ethyl | |
| 2.4.973 | 3-Chlor-4-methoxy-Phenyl | Methyl | Ethyl | |
| 2.4.974 | 3-Brom-4-methoxy-Phenyl | Methyl | Ethyl | |
| 2.4.975 | 3-Methyl-4-methoxy-Phenyl | Methyl | Ethyl | |
| 2.4.976 | 3-Ethyl-4-methoxy-Phenyl | Methyl | Ethyl | |
| 2.4.977 | 3-Cyclopropyl-4-methoxy-Phenyl | Methyl | Ethyl | |
| 2.4.978 | 3-Cyano-4-methoxy-Phenyl | Methyl | Ethyl | |
| 2.4.979 | 3-Trifluormethyl-4-methoxy-Phenyl | Methyl | Ethyl | |
| 2.4.980 | 3,4-Dimethoxy-Phenyl | Methyl | Ethyl | |
| 2.4.981 | 3-Ethoxy-4-methoxy-Phenyl | Methyl | Ethyl | |
| 2.4.982 | 3-Trifluormethoxy-4-methoxyPhenyl | Methyl | Ethyl | |
| 2.4.983 | 3-Nitro-4-methoxy-Phenyl | Methyl | Ethyl | |
| 2.4.984 | 3-Fluor-4-ethoxy-Phenyl | Methyl | Ethyl | |
| 2.4.985 | 3-Chlor-4-ethoxy-Phenyl | Methyl | Ethyl | |
| 2.4.986 | 3-Chlor-4-ethoxy-Phenyl | Ethyl | Ethyl | |
| 2.4.987 | 3-Chlor-4-ethoxy-Phenyl | Propyl | Ethyl | |
| 2.4.988 | 3-Chlor-4-ethoxy-Phenyl | Butyl | Ethyl | |
| 2.4.989 | 3-Brom-4-ethoxy-Phenyl | Methyl | Ethyl | |
| 2.4.990 | 3-Methyl-4-ethoxy-Phenyl | Methyl | Ethyl | |
| 2.4.991 | 3-Cyclopropyl-4-ethoxy-Phenyl | Methyl | Ethyl | |
| 2.4.992 | 3-Cyano-4-ethoxy-Phenyl | Methyl | Ethyl | |
| 2.4.993 | 3-Trifluormethyl-4-ethoxy-Phenyl | Methyl | Ethyl | |
| 2.4.994 | 3-Methoxy-4-ethoxy-Phenyl | Methyl | Ethyl | |
| 2.4.995 | 2,4-Diethoxy-Phenyl | Methyl | Ethyl | |
| 2.4.996 | 3-Trifluormethoxy-4-ethoxy-Phenyl | Methyl | Ethyl | |
| 2.4.997 | 3-Nitro-4-ethoxy-Phenyl | Methyl | Ethyl | |
| 2.4.998 | 3-Fluor-4-propoxy-Phenyl | Methyl | Ethyl | |
| 2.4.999 | 3-Chlor-4-propoxy-Phenyl | Methyl | Ethyl | |
| 2.4.1000 | 3-Brom-4-propoxy-Phenyl | Methyl | Ethyl | |
| 2.4.1001 | 3-Methyl-4-propoxy-Phenyl | Methyl | Ethyl | |
| 2.4.1002 | 3-Cyclopropyl-4-propoxy-Phenyl | Methyl | Ethyl | |
| 2.4.1003 | 3-Cyano-4-propoxy-Phenyl | Methyl | Ethyl | |
| 2.4.1004 | 3-Trifluormethyl-4-propoxy-Phenyl | Methyl | Ethyl | |
| 2.4.1005 | 3-Methoxy-4-propoxy-Phenyl | Methyl | Ethyl | |
| 2.4.1006 | 3-Ethoxy-4-propoxy-Phenyl | Methyl | Ethyl | |
| 2.4.1007 | 3-Trifluormethoxy-4-propoxy-Phenyl | Methyl | Ethyl | |
| 2.4.1008 | 3-Nitro-4-propoxy-Phenyl | Methyl | Ethyl | |
| 2.4.1009 | 3-Fluor-4-isopropoxy-Phenyl | Methyl | Ethyl | |
| 2.4.1010 | 3-Chlor-4-isopropoxy-Phenyl | Methyl | Ethyl | |
| 2.4.1011 | 3-Brom-4-isopropoxy-Phenyl | Methyl | Ethyl | |
| 2.4.1012 | 3-Methyl-4-isopropoxy-Phenyl | Methyl | Ethyl | |
| 2.4.1013 | 3-Cyclopropyl-4-isopropoxy-Phenyl | Methyl | Ethyl | |
| 2.4.1014 | 3-Cyano-4-isopropoxy-Phenyl | Methyl | Ethyl | |
| 2.4.1015 | 3-Trifluormethyl-4-isopropoxy-Phenyl | Methyl | Ethyl | |
| 2.4.1016 | 3-Methoxy-4-isopropoxy-Phenyl | Methyl | Ethyl | |
| 2.4.1017 | 3-Ethoxy-4-isopropoxy-Phenyl | Methyl | Ethyl | |
| 2.4.1018 | 3-Trifluormethoxy-4-isopropoxy-Phenyl | Methyl | Ethyl | |
| 2.4.1019 | 3-Nitro-4-isopropoxy-Phenyl | Methyl | Ethyl | |
| 2.4.1020 | 3-Fluor-4-trifluormethoxy-Phenyl | Methyl | Ethyl | |
| 2.4.1021 | 3-Chlor-4-trifluormethoxy-Phenyl | Methyl | Ethyl | |
| 2.4.1022 | 3-Brom-4-trifluormethoxy-Phenyl | Methyl | Ethyl | |
| 2.4.1023 | 3-Methyl-4-trifluormethoxy-Phenyl | Methyl | Ethyl | |
| 2.4.1024 | 3-Cyclopropyl-4-trifluormethoxy-Phenyl | Methyl | Ethyl | |
| 2.4.1025 | 3-Cyano-4-trifluormethoxy-Phenyl | Methyl | Ethyl | |
| 2.4.1026 | 3-Trifluormethyl-4-trifluormethoxy-Phenyl | Methyl | Ethyl | |
| 2.4.1027 | 3-Methoxy-4-trifluormethoxy-Phenyl | Methyl | Ethyl | |
| 2.4.1028 | 3-Ethoxy-4-trifluormethoxy-Phenyl | Methyl | Ethyl | |
| 2.4.1029 | 3,4-Bis(trifluormethoxy)-Phenyl | Methyl | Ethyl | |
| 2.4.1030 | 3-Nitro-4-trifluormethoxy-Phenyl | Methyl | Ethyl | |
| 2.4.1031 | 3-Fluor-4-difluormethoxy-Phenyl | Methyl | Ethyl | |
| 2.4.1032 | 3-Chlor-4-difluormethoxy-Phenyl | Methyl | Ethyl | |
| 2.4.1033 | 3-Brom-4-difluormethoxy-Phenyl | Methyl | Ethyl | |
| 2.4.1034 | 3-Methyl-4-difluormethoxy-Phenyl | Methyl | Ethyl | |
| 2.4.1035 | 3-Cyclopropyl-4-difluormethoxy-Phenyl | Methyl | Ethyl | |
| 2.4.1036 | 3-Cyano-4-difluormethoxy-Phenyl | Methyl | Ethyl | |
| 2.4.1037 | 3-Trifluormethyl-4-difluormethoxy-Phenyl | Methyl | Ethyl | |
| 2.4.1038 | 3-Methoxy-4-difluormethoxy-Phenyl | Methyl | Ethyl | |
| 2.4.1039 | 3-Ethoxy-4-difluormethoxy-Phenyl | Methyl | Ethyl | |
| 2.4.1040 | 3-Trifluormethoxy-4-difluormethoxy-Phenyl | Methyl | Ethyl | |
| 2.4.1041 | 3-Nitro-4-difluormethoxy-Phenyl | Methyl | Ethyl | |
| 2.4.1042 | 3-Fluor-4-nitro-Phenyl | Methyl | Ethyl | |
| 2.4.1043 | 3-Chlor-4-nitro-Phenyl | Methyl | Ethyl | |
| 2.4.1044 | 3-Brom-4-nitro-Phenyl | Methyl | Ethyl | |
| 2.4.1045 | 3-Methyl-4-nitro-Phenyl | Methyl | Ethyl | |
| 2.4.1046 | 3-Cyclopropyl-4-nitro-Phenyl | Methyl | Ethyl | |
| 2.4.1047 | 3-Cyano-4-nitro-Phenyl | Methyl | Ethyl | |
| 2.4.1048 | 3-Trifluormethyl-4-nitro-Phenyl | Methyl | Ethyl | |
| 2.4.1049 | 3-Methoxy-4-nitro-Phenyl | Methyl | Ethyl | |
| 2.4.1050 | 3-Ethoxy-4-nitro-Phenyl | Methyl | Ethyl | |
| 2.4.1051 | 3-Trifluormethoxy-4-nitro-Phenyl | Methyl | Ethyl | |
| 2.4.1052 | 3-Fluor-4-methylsulfanylPhenyl | Methyl | Ethyl | |
| 2.4.1053 | 3-Chlor-4-methylsulfanyl-Phenyl | Methyl | Ethyl | |
| 2.4.1054 | 3-Brom-4-methylsulfanyl-Phenyl | Methyl | Ethyl | |
| 2.4.1055 | 3-Methyl-4-methylsulfanyl-Phenyl | Methyl | Ethyl | |
| 2.4.1056 | 3-Cyclopropyl-4-methylsulfanyl-Phenyl | Methyl | Ethyl | |
| 2.4.1057 | 3-Cyano-4-methylsulfanyl-Phenyl | Methyl | Ethyl | |
| 2.4.1058 | 3-Trifluormethyl-4-methylsulfanyl-Phenyl | Methyl | Ethyl | |
| 2.4.1059 | 3-Methoxy-4-methylsulfanyl-Phenyl | Methyl | Ethyl | |
| 2.4.1060 | 3-Ethoxy-4-methylsulfanyl-Phenyl | Methyl | Ethyl | |
| 2.4.1061 | 3-Trifluormethoxy-4-methylsulfanyl-Phenyl | Methyl | Ethyl | |
| 2.4.1062 | 3-Nitro-4-methylsulfanyl-Phenyl | Methyl | Ethyl | |
| 2.4.1063 | 3,6-Difluor-Phenyl | Methyl | Ethyl | |
| 2.4.1064 | 3,6-Difluor-Phenyl | Ethyl | Ethyl | |
| 2.4.1065 | 3,6-Difluor-Phenyl | Propyl | Ethyl | |
| 2.4.1066 | 3,6-Difluor-Phenyl | Butyl | Ethyl | |
| 2.4.1067 | 3-Chlor-6-fluor-Phenyl | Methyl | Ethyl | |
| 2.4.1068 | 3-Brom-6-fluor-Phenyl | Methyl | Ethyl | |
| 2.4.1069 | 3-Methyl-6-fluor-Phenyl | Methyl | Ethyl | |
| 2.4.1070 | 3-Ethyl-6-fluor-Phenyl | Methyl | Ethyl | |
| 2.4.1071 | 3-Cyclopropyl-6-fluor-Phenyl | Methyl | Ethyl | |
| 2.4.1072 | 3-Cyano-6-fluor-Phenyl | Methyl | Ethyl | |
| 2.4.1073 | 3-Methoxy-6-fluor-Phenyl | Methyl | Ethyl | |
| 2.4.1074 | 3-Ethoxy-6-fluor-Phenyl | Methyl | Ethyl | |
| 2.4.1075 | 3-Trifluormethoxy-6-fluor-Phenyl | Methyl | Ethyl | |
| 2.4.1076 | 3-Nitro-6-fluor-Phenyl | Methyl | Ethyl | |
| 2.4.1077 | 3-Fluor-6-chlor-Phenyl | Methyl | Ethyl | |
| 2.4.1078 | 3-Fluor-6-chlor-Phenyl | Ethyl | Ethyl | |
| 2.4.1079 | 3-Fluor-6-chlor-Phenyl | Propyl | Ethyl | |
| 2.4.1080 | 3-Fluor-6-chlor-Phenyl | Butyl | Ethyl | |
| 2.4.1081 | 3,6-Dichlor-Phenyl | Methyl | Ethyl | |
| 2.4.1082 | 3,6-Dichlor-Phenyl | Ethyl | Ethyl | |
| 2.4.1083 | 3,6-Dichlor-Phenyl | Propyl | Ethyl | |
| 2.4.1084 | 3,6-Dichlor-Phenyl | Butyl | Ethyl | |
| 2.4.1085 | 3-Brom-6-chlor-Phenyl | Methyl | Ethyl | |
| 2.4.1086 | 3-Methyl-6-chlor-Phenyl | Methyl | Ethyl | |
| 2.4.1087 | 3-Ethyl-6-chlor-Phenyl | Methyl | Ethyl | |
| 2.4.1088 | 3-Cyclopropyl-6-chlor-Phenyl | Methyl | Ethyl | |
| 2.4.1089 | 3-Cyano-6-chlor-Phenyl | Methyl | Ethyl | |
| 2.4.1090 | 3-Trifluormethyl-6-chlor-Phenyl | Methyl | Ethyl | |
| 2.4.1091 | 3-Methoxy-6-chlor-Phenyl | Methyl | Ethyl | |
| 2.4.1092 | 3-Ethoxy-6-chlor-Phenyl | Methyl | Ethyl | |
| 2.4.1093 | 3-Trifluormethoxy-6-chlor-Phenyl | Methyl | Ethyl | |
| 2.4.1094 | 3-Nitro-6-chlor-Phenyl | Methyl | Ethyl | |
| 2.4.1095 | 3-Fluor-6-brom-Phenyl | Methyl | Ethyl | |
| 2.4.1096 | 3-Chlor-6-brom-Phenyl | Methyl | Ethyl | |
| 2.4.1097 | 3,6-Dibrom-Phenyl | Methyl | Ethyl | |
| 2.4.1098 | 3-Methyl-6-brom-Phenyl | Methyl | Ethyl | |
| 2.4.1099 | 3-Ethyl-6-brom-Phenyl | Methyl | Ethyl | |
| 2.4.1100 | 3-Cyclopropyl-6-brom-Phenyl | Methyl | Ethyl | |
| 2.4.1101 | 3-Cyano-6-brom-Phenyl | Methyl | Ethyl | |
| 2.4.1102 | 3-Trifluormethyl-6-brom-Phenyl | Methyl | Ethyl | |
| 2.4.1103 | 3-Methoxy-6-Phenyl | Methyl | Ethyl | |
| 2.4.1104 | 3-Ethoxy-6-brom-Phenyl | Methyl | Ethyl | |
| 2.4.1105 | 3-Trifluormethoxy-6-brom-Phenyl | Methyl | Ethyl | |
| 2.4.1106 | 3-Nitro-6-brom-Phenyl | Methyl | Ethyl | |
| 2.4.1107 | 3-Fluor-6-iod-Phenyl | Methyl | Ethyl | |
| 2.4.1108 | 3-Chlor-6-iod-Phenyl | Methyl | Ethyl | |
| 2.4.1109 | 3-Brom-6-iod-Phenyl | Methyl | Ethyl | |
| 2.4.1110 | 3-Methyl-6-iod-Phenyl | Methyl | Ethyl | |
| 2.4.1111 | 3-Ethyl-6-iod-Phenyl | Methyl | Ethyl | |
| 2.4.1112 | 3-Cyclopropyl-6-iod-Phenyl | Methyl | Ethyl | |
| 2.4.1113 | 3-Cyano-6-iod-Phenyl | Methyl | Ethyl | |
| 2.4.1114 | 3-Trifluormethyl-6-iod-Phenyl | Methyl | Ethyl | |
| 2.4.1115 | 3-Methoxy-6-iod-Phenyl | Methyl | Ethyl | |
| 2.4.1116 | 3-Ethoxy-6-iod-Phenyl | Methyl | Ethyl | |
| 2.4.1117 | 3-Trifluormethoxy-6-iod-Phenyl | Methyl | Ethyl | |
| 2.4.1118 | 3-Nitro-6-iod-Phenyl | Methyl | Ethyl | |
| 2.4.1119 | 3-Fluor-6-methyl-Phenyl | Methyl | Ethyl | |
| 2.4.1120 | 3-Chlor-6-methyl-Phenyl | Methyl | Ethyl | |
| 2.4.1121 | 3-Brom-6-methyl-Phenyl | Methyl | Ethyl | |
| 2.4.1122 | 3.6-Dimethyl-Phenyl | Methyl | Ethyl | |
| 2.4.1123 | 3-Ethyl-6-methyl-Phenyl | Methyl | Ethyl | |
| 2.4.1124 | 3-Cyclopropyl-6-methyl-Phenyl | Methyl | Ethyl | |
| 2.4.1125 | 3-Cyano-6-methyl-Phenyl | Methyl | Ethyl | |
| 2.4.1126 | 3-Trifluormethyl-6-methyl-Phenyl | Methyl | Ethyl | |
| 2.4.1127 | 3-Methoxy-6-methyl-Phenyl | Methyl | Ethyl | |
| 2.4.1128 | 3-Ethoxy-6-methyl-Phenyl | Methyl | Ethyl | |
| 2.4.1129 | 3-Trifluormethoxy-6-methyl-Phenyl | Methyl | Ethyl | |
| 2.4.1130 | 3-Nitro-6-methyl-Phenyl | Methyl | Ethyl | |
| 2.4.1131 | 3-Fluor-6-ethyl-Phenyl | Methyl | Ethyl | |
| 2.4.1132 | 3-Chlor-6-ethyl-Phenyl | Methyl | Ethyl | |
| 2.4.1133 | 3-Brom-6-ethyl-Phenyl | Methyl | Ethyl | |
| 2.4.1134 | 3-Methyl-6-ethyl-Phenyl | Methyl | Ethyl | |
| 2.4.1135 | 3,6-Diethyl-Phenyl | Methyl | Ethyl | |
| 2.4.1136 | 3-Cyclopropyl-6-ethyl-Phenyl | Methyl | Ethyl | |
| 2.4.1137 | 3-Cyano-6-ethyl-Phenyl | Methyl | Ethyl | |
| 2.4.1138 | 3-Trifluormethyl-6-ethyl-Phenyl | Methyl | Ethyl | |
| 2.4.1139 | 3-Methoxy-6-ethyl-Phenyl | Methyl | Ethyl | |
| 2.4.1140 | 3-Ethoxy-6-ethyl-Phenyl | Methyl | Ethyl | |
| 2.4.1141 | 3-Trifluormethoxy-6-ethyl-Phenyl | Methyl | Ethyl | |
| 2.4.1142 | 3-Nitro-6-ethyl-Phenyl | Methyl | Ethyl | |
| 2.4.1143 | 3-Fluor-6-propyl-Phenyl | Methyl | Ethyl | |
| 2.4.1144 | 3-Chlor-6-propyl-Phenyl | Methyl | Ethyl | |
| 2.4.1145 | 3-Brom-6-propyl-Phenyl | Methyl | Ethyl | |
| 2.4.1146 | 3-Methyl-6-propyl-Phenyl | Methyl | Ethyl | |
| 2.4.1147 | 3-Methyl-6-propyl-Phenyl | Methyl | Ethyl | |
| 2.4.1148 | 3-Cyclopropyl-6-propyl-Phenyl | Methyl | Ethyl | |
| 2.4.1149 | 3-Cyano-6-propyl-Phenyl | Methyl | Ethyl | |
| 2.4.1150 | 3-Trifluormethyl-6-propyl-Phenyl | Methyl | Ethyl | |
| 2.4.1151 | 3-Methoxy-6-propyl-Phenyl | Methyl | Ethyl | |
| 2.4.1152 | 3-Ethoxy-6-propyl-Phenyl | Methyl | Ethyl | |
| 2.4.1153 | 3-Trifluormethoxy-6-propyl-Phenyl | Methyl | Ethyl | |
| 2.4.1154 | 3-Nitro-6-propyl-Phenyl | Methyl | Ethyl | |
| 2.4.1155 | 3-Fluor-6-isopropyl-Phenyl | Methyl | Ethyl | |
| 2.4.1156 | 3-Chlor-6-isopropyl-Phenyl | Methyl | Ethyl | |
| 2.4.1157 | 3-Brom-6-isopropyl-Phenyl | Methyl | Ethyl | |
| 2.4.1158 | 3-Methyl-6-isopropyl-Phenyl | Methyl | Ethyl | |
| 2.4.1159 | 3-Cyclopropyl-6-isopropyl-Phenyl | Methyl | Ethyl | |
| 2.4.1160 | 3-Cyano-6-isopropyl-Phenyl | Methyl | Ethyl | |
| 2.4.1161 | 3-Trifluormethyl-6-isopropyl-Phenyl | Methyl | Ethyl | |
| 2.4.1162 | 3-Methoxy-6-isopropyl-Phenyl | Methyl | Ethyl | |
| 2.4.1163 | 3-Ethoxy-6-isopropyl-Phenyl | Methyl | Ethyl | |
| 2.4.1164 | 3-Trifluormethoxy-6-isopropylPhenyl | Methyl | Ethyl | |
| 2.4.1165 | 3-Nitro-6-isopropyl-Phenyl | Methyl | Ethyl | |
| 2.4.1166 | 3-Fluor-6-tert.butyl-Phenyl | Methyl | Ethyl | |
| 2.4.1167 | 3-Chlor-6-tert.buty-Phenyl | Methyl | Ethyl | |
| 2.4.1168 | 3-Brom-6-tert.butyl-Phenyl | Methyl | Ethyl | |
| 2.4.1169 | 3-Methyl-6-tert.butyl-Phenyl | Methyl | Ethyl | |
| 2.4.1170 | 3-Cyclopropyl-6-tert.butyl-Phenyl | Methyl | Ethyl | |
| 2.4.1171 | 3-Cyano-6-tert.butyl-Phenyl | Methyl | Ethyl | |
| 2.4.1172 | 3-Trifluormethyl-6-tert.butyl-Phenyl | Methyl | Ethyl | |
| 2.4.1173 | 3-Methoxy-6-tert.butyl -Phenyl | Methyl | Ethyl | |
| 2.4.1174 | 3-Ethoxy-6-tert.butyl -Phenyl | Methyl | Ethyl | |
| 2.4.1175 | 3-Trifluormethoxy-6-tert.butyl - Phenyl | Methyl | Ethyl | |
| 2.4.1176 | 3-Nitro-6-tert.butyl-Phenyl | Methyl | Ethyl | |
| 2.4.1177 | 3-Fluor-6-cyclopropyl-Phenyl | Methyl | Ethyl | |
| 2.4.1178 | 3-Chlor-6-cyclopropyl-Phenyl | Methyl | Ethyl | |
| 2.4.1179 | 3-Brom-6-cyclopropyl-Phenyl | Methyl | Ethyl | |
| 2.4.1180 | 3-Methyl-6-cyclopropyl-Phenyl | Methyl | Ethyl | |
| 2.4.1181 | 3-Cyclopropyl-6-cyclopropyl-Phenyl | Methyl | Ethyl | |
| 2.4.1182 | 3-Cyano-6-cyclopropyl-Phenyl | Methyl | Ethyl | |
| 2.4.1183 | 3-Trifluormethyl-6-cyclopropyl-Phenyl | Methyl | Ethyl | |
| 2.4.1184 | 3-Methoxy-6-cyclopropyl-Phenyl | Methyl | Ethyl | |
| 2.4.1185 | 3-Ethoxy-6-cyclopropyl-Phenyl | Methyl | Ethyl | |
| 2.4.1186 | 3-Trifluormethoxy-6-cyclopropyl-Phenyl | Methyl | Ethyl | |
| 2.4.1187 | 3-Fluor-6-methoxycarbonyl-Phenyl | Methyl | Ethyl | |
| 2.4.1188 | 3-Chlor-6-methoxycarbonyl-Phenyl | Methyl | Ethyl | |
| 2.4.1189 | 3-Brom-6-methoxycarbonyl-Phenyl | Methyl | Ethyl | |
| 2.4.1190 | 3-Methyl-6-methoxycarbonyl-Phenyl | Methyl | Ethyl | |
| 2.4.1191 | 3-Cyclopropyl-6-methoxycarbonyl-Phenyl | Methyl | Ethyl | |
| 2.4.1192 | 3-Cyano-6-methoxycarbonyl-Phenyl | Methyl | Ethyl | |
| 2.4.1193 | 3-Trifluormethyl-6-methoxycarbonyl-Phenyl | Methyl | Ethyl | |
| 2.4.1194 | 3-Methoxy-6-methoxycarbonyl - Phenyl | Methyl | Ethyl | |
| 2.4.1195 | 3-Ethoxy-6-methoxycarbonyl-Phenyl | Methyl | Ethyl | |
| 2.4.1196 | 3-Trifluormethoxy-6-methoxycarbonyl-Phenyl | Methyl | Ethyl | |
| 2.4.1197 | 3-Nitro-6-methoxycarbonyl-Phenyl | Methyl | Ethyl | |
| 2.4.1198 | 3-Fluor-6-cyano-Phenyl | Methyl | Ethyl | |
| 2.4.1199 | 3-Chlor-6-cyano-Phenyl | Methyl | Ethyl | |
| 2.4.1200 | 3-Brom-6-cyano-Phenyl | Methyl | Ethyl | |
| 2.4.1201 | 3-Methyl-6-cyano-Phenyl | Methyl | Ethyl | |
| 2.4.1202 | 3-Cyclopropyl-6-cyano-Phenyl | Methyl | Ethyl | |
| 2.4.1203 | 3-Cyano-6-cyano-Phenyl | Methyl | Ethyl | |
| 2.4.1204 | 3-Trifluormethyl-6-cyano-Phenyl | Methyl | Ethyl | |
| 2.4.1205 | 3-Methoxy-6-cyano-Phenyl | Methyl | Ethyl | |
| 2.4.1206 | 3-Ethoxy-6-cyano-Phenyl | Methyl | Ethyl | |
| 2.4.1207 | 3-Trifluormethoxy-6-cyano-Phenyl | Methyl | Ethyl | |
| 2.4.1208 | 3-Nitro-6-cyano-Phenyl | Methyl | Ethyl | |
| 2.4.1209 | 3-Fluor-6-methoxy-Phenyl | Methyl | Ethyl | |
| 2.4.1210 | 3-Chlor-6-methoxy-Phenyl | Methyl | Ethyl | |
| 2.4.1211 | 3-Brom-6-methoxy-Phenyl | Methyl | Ethyl | |
| 2.4.1212 | 3-Methyl-6-methoxy-Phenyl | Methyl | Ethyl | |
| 2.4.1213 | 3-Cyclopropyl-6-methoxy-Phenyl | Methyl | Ethyl | |
| 2.4.1214 | 3-Cyano-6-methoxy-Phenyl | Methyl | Ethyl | |
| 2.4.1215 | 3-Trifluormethyl-6-methoxy-Phenyl | Methyl | Ethyl | |
| 2.4.1216 | 3,6-Dimethoxy-Phenyl | Methyl | Ethyl | |
| 2.4.1217 | 3-Ethoxy-6-methoxy-Phenyl | Methyl | Ethyl | |
| 2.4.1218 | 3-Trifluormethoxy-6-methoxyPhenyl | Methyl | Ethyl | |
| 2.4.1219 | 3-Nitro-6-methoxy-Phenyl | Methyl | Ethyl | |
| 2.4.1220 | 3-Fluor-6-ethoxy-Phenyl | Methyl | Ethyl | |
| 2.4.1221 | 3-Chlor-6-ethoxy-Phenyl | Methyl | Ethyl | |
| 2.4.1222 | 3-Brom-6-ethoxy-Phenyl | Methyl | Ethyl | |
| 2.4.1223 | 3-Methyl-6-ethoxy-Phenyl | Methyl | Ethyl | |
| 2.4.1224 | 3-Cyclopropyl-6-ethoxy-Phenyl | Methyl | Ethyl | |
| 2.4.1225 | 3-Cyano-6-ethoxy-Phenyl | Methyl | Ethyl | |
| 2.4.1226 | 3-Trifluormethyl-6-ethoxy-Phenyl | Methyl | Ethyl | |
| 2.4.1227 | 3-Methoxy-6-ethoxy-Phenyl | Methyl | Ethyl | |
| 2.4.1228 | 2,6-Diethoxy-Phenyl | Methyl | Ethyl | |
| 2.4.1229 | 3-Trifluormethoxy-6-ethoxy-Phenyl | Methyl | Ethyl | |
| 2.4.1230 | 3-Nitro-6-ethoxy-Phenyl | Methyl | Ethyl | |
| 2.4.1231 | 3-Fluor-6-propoxy-Phenyl | Methyl | Ethyl | |
| 2.4.1232 | 3-Chlor-6-propoxy-Phenyl | Methyl | Ethyl | |
| 2.4.1233 | 3-Brom-6-propoxy-Phenyl | Methyl | Ethyl | |
| 2.4.1234 | 3-Methyl-6-propoxy-Phenyl | Methyl | Ethyl | |
| 2.4.1235 | 3-Cyclopropyl-6-propoxy-Phenyl | Methyl | Ethyl | |
| 2.4.1236 | 3-Cyano-6-propoxy-Phenyl | Methyl | Ethyl | |
| 2.4.1237 | 3-Trifluormethyl-6-propoxy-Phenyl | Methyl | Ethyl | |
| 2.4.1238 | 3-Methoxy-6-propoxy-Phenyl | Methyl | Ethyl | |
| 2.4.1239 | 3-Ethoxy-6-propoxy-Phenyl | Methyl | Ethyl | |
| 2.4.1240 | 3-Trifluormethoxy-6-propoxy-Phenyl | Methyl | Ethyl | |
| 2.4.1241 | 3-Nitro-6-propoxy-Phenyl | Methyl | Ethyl | |
| 2.4.1242 | 3-Fluor-6-isopropoxy-Phenyl | Methyl | Ethyl | |
| 2.4.1243 | 3-Chlor-6-isopropoxy-Phenyl | Methyl | Ethyl | |
| 2.4.1244 | 3-Brom-6-isopropoxy-Phenyl | Methyl | Ethyl | |
| 2.4.1245 | 3-Methyl-6-isopropoxy-Phenyl | Methyl | Ethyl | |
| 2.4.1246 | 3-Cyclopropyl-6-isopropoxy-Phenyl | Methyl | Ethyl | |
| 2.4.1247 | 3-Cyano-6-isopropoxy-Phenyl | Methyl | Ethyl | |
| 2.4.1248 | 3-Trifluormethyl-6-isopropoxy-Phenyl | Methyl | Ethyl | |
| 2.4.1249 | 3-Methoxy-6-isopropoxy-Phenyl | Methyl | Ethyl | |
| 2.4.1250 | 3-Ethoxy-6-isopropoxy-Phenyl | Methyl | Ethyl | |
| 2.4.1251 | 3-Trifluormethoxy-6-isopropoxy-Phenyl | Methyl | Ethyl | |
| 2.4.1252 | 3-Nitro-6-isopropoxy-Phenyl | Methyl | Ethyl | |
| 2.4.1253 | 3-Fluor-6-trifluormethoxy-Phenyl | Methyl | Ethyl | |
| 2.4.1254 | 3-Chlor-6-trifluormethoxy-Phenyl | Methyl | Ethyl | |
| 2.4.1255 | 3-Brom-6-trifluormethoxy-Phenyl | Methyl | Ethyl | |
| 2.4.1256 | 3-Methyl-6-trifluormethoxy-Phenyl | Methyl | Ethyl | |
| 2.4.1257 | 3-Cyclopropyl-6-trifluormethoxy-Phenyl | Methyl | Ethyl | |
| 2.4.1258 | 3-Cyano-6-trifluormethoxy-Phenyl | Methyl | Ethyl | |
| 2.4.1259 | 3-Trifluormethyl-6-trifluormethoxy-Phenyl | Methyl | Ethyl | |
| 2.4.1260 | 3-Methoxy-6-trifluormethoxy-Phenyl | Methyl | Ethyl | |
| 2.4.1261 | 3-Ethoxy-6-trifluormethoxy-Phenyl | Methyl | Ethyl | |
| 2.4.1262 | 3,6-Bis(trifluormethoxy)-Phenyl | Methyl | Ethyl | |
| 2.4.1263 | 3-Nitro-6-trifluormethoxy-Phenyl | Methyl | Ethyl | |
| 2.4.1264 | 3-Fluor-6-difluormethoxy-Phenyl | Methyl | Ethyl | |
| 2.4.1265 | 3-Chlor-6-difluormethoxy-Phenyl | Methyl | Ethyl | |
| 2.4.1266 | 3-Brom-6-difluormethoxy-Phenyl | Methyl | Ethyl | |
| 2.4.1267 | 3-Methyl-6-difluormethoxy-Phenyl | Methyl | Ethyl | |
| 2.4.1268 | 3-Cyclopropyl-6-difluormethoxy-Phenyl | Methyl | Ethyl | |
| 2.4.1269 | 3-Cyano-6-difluormethoxy-Phenyl | Methyl | Ethyl | |
| 2.4.1270 | 3-Trifluormethyl-6-difluormethoxy - Phenyl | Methyl | Ethyl | |
| 2.4.1271 | 3-Methoxy-6-difluormethoxy-Phenyl | Methyl | Ethyl | |
| 2.4.1272 | 3-Ethoxy-6-difluormethoxy-Phenyl | Methyl | Ethyl | |
| 2.4.1273 | 3-Trifluormethoxy-6-difluormethoxy-Phenyl | Methyl | Ethyl | |
| 2.4.1274 | 3-Nitro-6-difluormethoxy-Phenyl | Methyl | Ethyl | |
| 2.4.1275 | 3-Fluor-6-nitro-Phenyl | Methyl | Ethyl | |
| 2.4.1276 | 3-Chlor-6-nitro-Phenyl | Methyl | Ethyl | |
| 2.4.1277 | 3-Brom-6-nitro-Phenyl | Methyl | Ethyl | |
| 2.4.1278 | 3-Methyl-6-nitro-Phenyl | Methyl | Ethyl | |
| 2.4.1279 | 3-Cyclopropyl-6-nitro-Phenyl | Methyl | Ethyl | |
| 2.4.1280 | 3-Cyano-6-nitro-Phenyl | Methyl | Ethyl | |
| 2.4.1281 | 3-Trifluormethyl-6-nitro-Phenyl | Methyl | Ethyl | |
| 2.4.1282 | 3-Methoxy-6-nitro-Phenyl | Methyl | Ethyl | |
| 2.4.1283 | 3-Ethoxy-6-nitro-Phenyl | Methyl | Ethyl | |
| 2.4.1284 | 3-Trifluormethoxy-6-nitro-Phenyl | Methyl | Ethyl | |
| 2.4.1285 | 3-Fluor-6-methylsulfanylPhenyl | Methyl | Ethyl | |
| 2.4.1286 | 3-Chlor-6-methylsulfanyl-Phenyl | Methyl | Ethyl | |
| 2.4.1287 | 3-Brom-6-methylsulfanyl-Phenyl | Methyl | Ethyl | |
| 2.4.1288 | 3-Methyl-6-methylsulfanyl-Phenyl | Methyl | Ethyl | |
| 2.4.1289 | 3-Cyclopropyl-6-methylsulfanyl-Phenyl | Methyl | Ethyl | |
| 2.4.1290 | 3-Cyano-6-methylsulfanyl-Phenyl | Methyl | Ethyl | |
| 2.4.1291 | 3-Trifluormethyl-6-methylsulfanyl-Phenyl | Methyl | Ethyl | |
| 2.4.1292 | 3-Methoxy-6-methylsulfanyl-Phenyl | Methyl | Ethyl | |
| 2.4.1293 | 3-Ethoxy-6-methylsulfanyl-Phenyl | Methyl | Ethyl | |
| 2.4.1294 | 3-Trifluormethoxy-6-methylsulfanyl-Phenyl | Methyl | Ethyl | |
| 2.4.1295 | 3-Nitro-6-methylsulfanyl-Phenyl | Methyl | Ethyl | |
| 2.4.1296 | 2,3,4-Trifluor-Phenyl | Methyl | Ethyl | |
| 2.4.1297 | 2,3,4-Trichlor-Phenyl | Methyl | Ethyl | |
| 2.4.1298 | 2,3.4-Trimethyl-Phenyl | Methyl | Ethyl | |
| 2.4.1299 | 2-Fluor-2-chlor-5-trifluormethylPhenyl | Methyl | Ethyl | |
| 2.4.1300 | 2,3,5-Trifluor-Phenyl | Methyl | Ethyl | |
| 2.4.1301 | 2,3,5-Trichlor-Phenyl | Methyl | Ethyl | |
| 2.4.1302 | 2,3,5-Trimethyl-Phenyl | Methyl | Ethyl | |
| 2.4.1303 | 2,3-Dichlor-5-methoxy-Phenyl | Methyl | Ethyl | |
| 2.4.1304 | 2,3,6-Trifluor-Phenyl | Methyl | Ethyl | |
| 2.4.1305 | 2,3,6-Trichlor-Phenyl | Methyl | Ethyl | |
| 2.4.1306 | 2,3,6-Trimethyl-Phenyl | Methyl | Ethyl | |
| 2.4.1307 | 3,4,5-Trifluor-Phenyl | Methyl | Ethyl | |
| 2.4.1308 | 3,4,5-Trichlor-Phenyl | Methyl | Ethyl | |
| 2.4.1309 | 3,4,5-Trimethyl-Phenyl | Methyl | Ethyl | |
| 2.4.1310 | 3,5-Dimethyl-4-fluor-Phenyl | Methyl | Ethyl | |
| 2.4.1311 | 3,5-Dichlor-4-methoxy-Phenyl | Methyl | Ethyl | |
| 2.4.1312 | 3,5-Difluor-4-chlor-Phenyl | Methyl | Ethyl | |
| 2.4.1313 | 3,5-Dichlor-4-hydroxy-Phenyl | Methyl | Ethyl | |
| 2.4.1314 | 3,5-Trifluormethyl-4-chlor-Phenyl | Methyl | Ethyl | |
| 2.4.1315 | 3,4,6-Trifluor-Phenyl | Methyl | Ethyl | |
| 2.4.1316 | 3,4,6-Trichlor-Phenyl | Methyl | Ethyl | |
| 2.4.1317 | 3,4,6-Trimethyl-Phenyl | Methyl | Ethyl | |
| 2.4.1318 | Pentafluorphenyl | Methyl | Ethyl | |

**Tabelle 2.5: Erfindungsgemäße Verbindungen der allgemeinen Formel (I), worin W* für COOY, R¹ und R² für Wasserstoff stehen, und Aryl den Rest**

| | | | | |
|---|---|---|---|---|
| 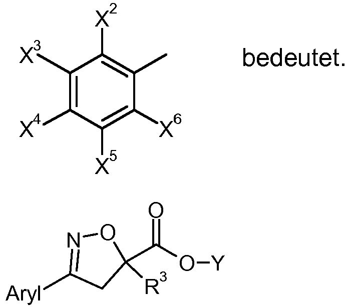 | | | | |

| Nr. | Aryl | R³ | Y | Physikalische Daten |
|---|---|---|---|---|
| 2.5.001 | 3-Fluor-Phenyl | Hydroxymethyl | Ethyl | |
| 2.5.002 | 3-Fluor-Phenyl | 1-Hydroxyethyl | Methyl | [CDCl] D1 1.20 (d,3H); 2.39 (d br,1H); 3.60 (d,1H); 3.71 (d,1H); 3.83 (s,3H); 4.30-4.37 (m,1H); 7.13 (t,1H); 7.34-7.45 (m,2H). D2 1.28 (d,3H); 2.17 (d br,1H); 3.56 (d,1H); 3.76 (d,1H); 3.84 (s,3H); 4.20-4.28 (m,1H); 7.13 (t,1H); 7.34-7.45 (m,3H). |
| 2.5.003 | 3-Fluor-Phenyl | 1-Hydroxypropyl | Ethyl | |
| 2.5.004 | 3-Fluor-Phenyl | (1-Hydroxy-2-methylpropyl) | Ethyl | |
| 2.5.005 | 3-Fluor-Phenyl | Methoxymethyl | Ethyl | |
| 2.5.006 | 3-Fluor-Phenyl | 2-Methoxyethyl | Ethyl | |
| 2.5.007 | 3-Chlor-Phenyl | Hydroxymethyl | Ethyl | |
| 2.5.008 | 3-Chlor-Phenyl | 1-Hydroxyethyl | Ethyl | |
| 2.5.009 | 3-Chlor-Phenyl | 1-Hydroxypropyl | Ethyl | |
| 2.5.010 | 3-Chlor-Phenyl | (1-Hydroxy-2-methylpropyl) | Ethyl | |
| 2.5.011 | 3-Chlor-Phenyl | Methoxymethyl | Ethyl | |
| 2.5.012 | 3-Chlor-Phenyl | 2-Methoxyethyl | Ethyl | |
| 2.5.013 | 3-Brom-Phenyl | Hydroxymethyl | Ethyl | |
| 2.5.014 | 3-Brom-Phenyl | 1-Hydroxyethyl | Ethyl | |
| 2.5.015 | 3-Iod-Phenyl | Hydroxymethyl | Ethyl | |
| 2.5.016 | 3-Iod-Phenyl | 1-Hydroxyethyl | Ethyl | |
| 2.5.017 | 3-Methyl-Phenyl | Hydroxymethyl | Ethyl | |
| 2.5.018 | 3-Methyl-Phenyl | 1-Hydroxyethyl | Ethyl | |
| 2.5.019 | 3-Ethyl-Phenyl | Hydroxymethyl | Ethyl | |
| 2.5.020 | 3-Propyl-Phenyl | Hydroxymethyl | Ethyl | |
| 2.5.021 | 3-isoPropyl-Phenyl | Hydroxymethyl | Ethyl | |
| 2.5.022 | 3-nButyl-Phenyl | Hydroxymethyl | Ethyl | |
| 2.5.023 | 3-iButyl-Phenyl | Hydroxymethyl | Ethyl | |
| 2.5.024 | 3-tert.Butyl-Phenyl | Hydroxymethyl | Ethyl | |
| 2.5.025 | 3-Cyclopropyl-Phenyl | Hydroxymethyl | Ethyl | |
| 2.5.026 | 3-Cyclobutyl-Phenyl | Hydroxymethyl | Ethyl | |
| 2.5.027 | 3-Cyclopentyl-Phenyl | Hydroxymethyl | Ethyl | |
| 2.5.028 | 3-Vinyl-Phenyl | Hydroxymethyl | Ethyl | |
| 2.5.029 | 3-Ethinyl-Phenyl | Hydroxymethyl | Ethyl | |
| 2.5.030 | 3-Cyano-Phenyl | Hydroxymethyl | Ethyl | |
| 2.5.031 | 3-Trifluormethyl-Phenyl | Hydroxymethyl | Ethyl | |
| 2.5.032 | 3-Difluormethyl-Phenyl | Hydroxymethyl | Ethyl | |
| 2.5.033 | 3-(Hydroxycarbonyl)-Phenyl | Hydroxymethyl | Ethyl | |
| 2.5.034 | 3-(Methoxycarbony)l-Phenyl | Hydroxymethyl | Ethyl | |
| 2.5.035 | 3-(Ethoxycarbonyl)-Phenyl | Hydroxymethyl | Ethyl | |
| 2.5.036 | 3-Hydroxymethyl-Phenyl | Hydroxymethyl | Ethyl | |
| 2.5.037 | 3-Carbamoyl-Phenyl | Hydroxymethyl | Ethyl | |
| 2.5.038 | 3-Hydroxy-Phenyl | Hydroxymethyl | Ethyl | |
| 2.5.039 | 3-Methoxy-Phenyl | Hydroxymethyl | Ethyl | |
| 2.5.040 | 3-Ethoxy-Phenyl | Hydroxymethyl | Ethyl | |
| 2.5.041 | 3-Propyloxy-Phenyl | Hydroxymethyl | Ethyl | |
| 2.5.042 | 3-isoPropyloxy-Phenyl | Hydroxymethyl | Ethyl | |
| 2.5.043 | 3-nButyloxy-Phenyl | Hydroxymethyl | Ethyl | |
| 2.5.044 | 3-iButyloxy-Phenyl | Hydroxymethyl | Ethyl | |
| 2.5.045 | 3-tButyloxy-Phenyl | Hydroxymethyl | Ethyl | |
| 2.5.046 | 3-Difluormethoxy-Phenyl | Hydroxymethyl | Ethyl | |
| 2.5.047 | 3-Trifluormethoxy-Phenyl | Hydroxymethyl | Ethyl | |
| 2.5.048 | 3-(2,2,2-Trifluorethoxy)-Phenyl | Hydroxymethyl | Ethyl | |
| 2.5.049 | 3-(2-Chlorethoxy)-Phenyl | Hydroxymethyl | Ethyl | |
| 2.5.050 | 3-(2-Hydroxyethoxy)-Phenyl- | Hydroxymethyl | Ethyl | |
| 2.5.051 | 3-(2-Methoxyethoxy)-Phenyl- | Hydroxymethyl | Ethyl | |
| 2.5.052 | 3-[(tert-Butoxycarbonyl)oxy]-Phenyl | Hydroxymethyl | Ethyl | |
| 2.5.053 | 3-Nitro-Phenyl | Hydroxymethyl | Ethyl | |
| 2.5.054 | 3-Acetoxy-Phenyl | Hydroxymethyl | Ethyl | |
| 2.5.055 | {3-[(tert-Butoxycarbonyl)amino]-Phenyl}- | Hydroxymethyl | Ethyl | |
| 2.5.056 | 3-Methylsulfanyl-Phenyl | Hydroxymethyl | Ethyl | |
| 2.5.057 | 3-Ethylsulfanyl-Phenyl | Hydroxymethyl | Ethyl | |
| 2.5.058 | 3-(Pentafluor-lambda⁶-sulfanyl)-Phenyl | Hydroxymethyl | Ethyl | |
| 2.5.059 | 2,3-Difluor-Phenyl | Hydroxymethyl | Ethyl | |
| 2.5.060 | 2,3-Difluor-Phenyl | 1-Hydroxyethyl | Ethyl | |
| 2.5.061 | 2,3-Difluor-Phenyl | 1-Hydroxypropyl | Ethyl | |
| 2.5.062 | 2,3-Difluor-Phenyl | (1-Hydroxy-2-methylpropyl) | Ethyl | |
| 2.5.063 | 2,3-Difluor-Phenyl | Methoxymethyl | Ethyl | |
| 2.5.064 | 2,3-Difluor-Phenyl | 2-Methoxyethyl | Ethyl | |
| 2.5.065 | 2-Chlor-3-fluor-Phenyl | Hydroxymethyl | Ethyl | |
| 2.5.066 | 2-Brom-3-fluor-Phenyl | Hydroxymethyl | Ethyl | |
| 2.5.067 | 2-Methyl-3-fluor-Phenyl | Hydroxymethyl | Ethyl | |
| 2.5.068 | 2-Ethyl-3-fluor-Phenyl | Hydroxymethyl | Ethyl | |
| 2.5.069 | 2-Cyclopropyl-3-fluor-Phenyl | Hydroxymethyl | Ethyl | |
| 2.5.070 | 2-Vinyl-3-fluor-Phenyl | Hydroxymethyl | Ethyl | |
| 2.5.071 | 2-Ethinyl-3-fluor-Phenyl | Hydroxymethyl | Ethyl | |
| 2.5.072 | 2-Cyano-3-fluor-Phenyl | Hydroxymethyl | Ethyl | |
| 2.5.073 | 2-Methoxy-3-fluor-Phenyl | Hydroxymethyl | Ethyl | |
| 2.5.074 | 2-Ethoxy-3-fluor-Phenyl | Hydroxymethyl | Ethyl | |
| 2.5.075 | 2-Trifluormethoxy-3-fluor-Phenyl | Hydroxymethyl | Ethyl | |
| 2.5.076 | 2-Nitro-3-fluor-Phenyl | Hydroxymethyl | Ethyl | |
| 2.5.077 | 2-Fluor-3-chlor-Phenyl | Hydroxymethyl | Ethyl | |
| 2.5.078 | 2,3-Dichlor-Phenyl | Hydroxymethyl | Ethyl | |
| 2.5.079 | 2,3-Dichlor-Phenyl | 1-Hydroxyethyl | Ethyl | |
| 2.5.080 | 2,3-Dichlor-Phenyl | 1-Hydroxypropyl | Ethyl | |
| 2.5.081 | 2,3-Dichlor-Phenyl | (1-Hydroxy-2-methylpropyl) | Ethyl | |
| 2.5.082 | 2,3-Dichlor-Phenyl | Methoxymethyl | Ethyl | |
| 2.5.083 | 2,3-Dichlor-Phenyl | 2-Methoxyethyl | Ethyl | |
| 2.5.084 | 2-Brom-3-chlor-Phenyl | Hydroxymethyl | Ethyl | |
| 2.5.085 | 2-Methyl-3-chlor-Phenyl | Hydroxymethyl | Ethyl | |
| 2.5.086 | 2-Ethyl-3-chlor-Phenyl | Hydroxymethyl | Ethyl | |
| 2.5.087 | 2-Cyclopropyl-3-chlor-Phenyl | Hydroxymethyl | Ethyl | |
| 2.5.088 | 2-Vinyl-3-chlor-Phenyl | Hydroxymethyl | Ethyl | |
| 2.5.089 | 2-Ethinyl-3-chlor-Phenyl | Hydroxymethyl | Ethyl | |
| 2.5.090 | 2-Cyano-3-chlor-Phenyl | Hydroxymethyl | Ethyl | |
| 2.5.091 | 2-Trifluormethyl-2-chlor-Phenyl | Hydroxymethyl | Ethyl | |
| 2.5.092 | 2-Methoxy-3-chlor-Phenyl | Hydroxymethyl | Ethyl | |
| 2.5.093 | 2-Ethoxy-3-chlor-Phenyl | Hydroxymethyl | Ethyl | |
| 2.5.094 | 2-Trifluormethoxy-3-chlor-Phenyl | Hydroxymethyl | Ethyl | |
| 2.5.095 | 2-Nitro-3-chlor-Phenyl | Hydroxymethyl | Ethyl | |
| 2.5.096 | 2-Fluor-3-brom-Phenyl | Hydroxymethyl | Ethyl | |
| 2.5.097 | 2-Chlor-3-brom-Phenyl | Hydroxymethyl | Ethyl | |
| 2.5.098 | 2,3-Dibrom-Phenyl | Hydroxymethyl | Ethyl | |
| 2.5.099 | 2-Methyl-3-brom-Phenyl | Hydroxymethyl | Ethyl | |
| 2.5.100 | 2-Ethyl-3-brom-Phenyl | Hydroxymethyl | Ethyl | |
| 2.5.101 | 2-Cyclopropyl-3-brom-Phenyl | Hydroxymethyl | Ethyl | |
| 2.5.102 | 2-Vinyl-3-brom-Phenyl | Hydroxymethyl | Ethyl | |
| 2.5.103 | 2-Ethinyl-3-brom-Phenyl | Hydroxymethyl | Ethyl | |
| 2.5.104 | 2-Cyano-3-brom-Phenyl | Hydroxymethyl | Ethyl | |
| 2.5.105 | 2-Trifluormethyl-3-brom-Phenyl | Hydroxymethyl | Ethyl | |
| 2.5.106 | 2-Methoxy-3-Phenyl | Hydroxymethyl | Ethyl | |
| 2.5.107 | 2-Ethoxy-3-brom-Phenyl | Hydroxymethyl | Ethyl | |
| 2.5.108 | 2-Trifluormethoxy-3-brom-Phenyl | Hydroxymethyl | Ethyl | |
| 2.5.109 | 2-Nitro-3-brom-Phenyl | Hydroxymethyl | Ethyl | |
| 2.5.110 | 2-Fluor-3-iod-Phenyl | Hydroxymethyl | Ethyl | |
| 2.5.111 | 2-Chlor-3-iod-Phenyl | Hydroxymethyl | Ethyl | |
| 2.5.112 | 2-Brom-3-iod-Phenyl | Hydroxymethyl | Ethyl | |
| 2.5.113 | 2-Methyl-3-iod-Phenyl | Hydroxymethyl | Ethyl | |
| 2.5.114 | 2-Ethyl-3-iod-Phenyl | Hydroxymethyl | Ethyl | |
| 2.5.115 | 2-Cyclopropyl-3-iod-Phenyl | Hydroxymethyl | Ethyl | |
| 2.5.116 | 2-Vinyl-3-iod-Phenyl | Hydroxymethyl | Ethyl | |
| 2.5.117 | 2-Ethinyl-3-iod-Phenyl | Hydroxymethyl | Ethyl | |
| 2.5.118 | 2-Cyano-3-iod-Phenyl | Hydroxymethyl | Ethyl | |
| 2.5.119 | 2-Trifluormethyl-3-iod-Phenyl | Hydroxymethyl | Ethyl | |
| 2.5.120 | 2-Methoxy-3-iod-Phenyl | Hydroxymethyl | Ethyl | |
| 2.5.121 | 2-Ethoxy-3-iod-Phenyl | Hydroxymethyl | Ethyl | |
| 2.5.122 | 2-Trifluormethoxy-3-iod-Phenyl | Hydroxymethyl | Ethyl | |
| 2.5.123 | 2-Nitro-3-iod-Phenyl | Hydroxymethyl | Ethyl | |
| 2.5.124 | 2-Fluor-3-methyl-Phenyl | Hydroxymethyl | Ethyl | |
| 2.5.125 | 2-Fluor-3-methyl-Phenyl | 1-Hydroxyethyl | Ethyl | |
| 2.5.126 | 2-Fluor-3-methyl-Phenyl | 1-Hydroxypropyl | Ethyl | |
| 2.5.127 | 2-Fluor-3-methyl-Phenyl | (1-Hydroxy-2-methylpropyl) | Ethyl | |
| 2.5.128 | 2-Fluor-3-methyl-Phenyl | Methoxymethyl | Ethyl | |
| 2.5.129 | 2-Fluor-3-methyl-Phenyl | 2-Methoxyethyl | Ethyl | |
| 2.5.130 | 2-Chlor-3-methyl-Phenyl | Hydroxymethyl | Ethyl | |
| 2.5.131 | 2-Chlor-3-methyl-Phenyl | 1-Hydroxyethyl | Ethyl | |
| 2.5.132 | 2-Chlor-3-methyl-Phenyl | 1-Hydroxypropyl | Ethyl | |
| 2.5.133 | 2-Chlor-3-methyl-Phenyl | (1-Hydroxy-2-methylpropyl) | Ethyl | |
| 2.5.134 | 2-Chlor-3-methyl-Phenyl | Methoxymethyl | Ethyl | |
| 2.5.135 | 2-Chlor-3-methyl-Phenyl | 2-Methoxyethyl | Ethyl | |
| 2.5.136 | 2-Brom-3-methyl-Phenyl | Hydroxymethyl | Ethyl | |
| 2.5.137 | 2,3-Dimethyl-Phenyl | Hydroxymethyl | Ethyl | |
| 2.5.138 | 2,3-Dimethyl-Phenyl | 1-Hydroxyethyl | Ethyl | |
| 2.5.139 | 2,3-Dimethyl-Phenyl | 1-Hydroxypropyl | Ethyl | |
| 2.5.140 | 2,3-Dimethyl-Phenyl | (1-Hydroxy-2-methylpropyl) | Ethyl | |
| 2.5.141 | 2,3-Dimethyl-Phenyl | Methoxymethyl | Ethyl | |
| 2.5.142 | 2,3-Dimethyl-Phenyl | 2-Methoxyethyl | Ethyl | |
| 2.5.143 | 2-Ethyl-3-methyl-Phenyl | Hydroxymethyl | Ethyl | |
| 2.5.144 | 2-Cyclopropyl-3-methyl-Phenyl | Hydroxymethyl | Ethyl | |
| 2.5.145 | 2-Vinyl-3-methyl-Phenyl | Hydroxymethyl | Ethyl | |
| 2.5.146 | 2-Ethinyl-3-methyl-Phenyl | Hydroxymethyl | Ethyl | |
| 2.5.147 | 2-Cyano-3-methyl-Phenyl | Hydroxymethyl | Ethyl | |
| 2.5.148 | 2-Trifluormethyl-3-methyl-Phenyl | Hydroxymethyl | Ethyl | |
| 2.5.149 | 2-Methoxy-3-methyl-Phenyl | Hydroxymethyl | Ethyl | |
| 2.5.150 | 2-Ethoxy-3-methyl-Phenyl | Hydroxymethyl | Ethyl | |
| 2.5.151 | 2-Trifluormethoxy-3-methyl-Phenyl | Hydroxymethyl | Ethyl | |
| 2.5.152 | 2-Nitro-3-methyl-Phenyl | Hydroxymethyl | Ethyl | |
| 2.5.153 | 2-Fluor-3-ethyl-Phenyl | Hydroxymethyl | Ethyl | |
| 2.5.154 | 2-Chlor-3-ethyl-Phenyl | Hydroxymethyl | Ethyl | |
| 2.5.155 | 2-Brom-3-ethyl-Phenyl | Hydroxymethyl | Ethyl | |
| 2.5.156 | 2-Methyl-3-ethyl-Phenyl | Hydroxymethyl | Ethyl | |
| 2.5.157 | 2,3-Diethyl-Phenyl | Hydroxymethyl | Ethyl | |
| 2.5.158 | 2-Cyclopropyl-3-ethyl-Phenyl | Hydroxymethyl | Ethyl | |
| 2.5.159 | 2-Vinyl-3-ethyl-Phenyl | Hydroxymethyl | Ethyl | |
| 2.5.160 | 2-Ethinyl-3-ethyl-Phenyl | Hydroxymethyl | Ethyl | |
| 2.5.161 | 2-Cyano-3-ethyl-Phenyl | Hydroxymethyl | Ethyl | |
| 2.5.162 | 2-Trifluormethyl-3-ethyl-Phenyl | Hydroxymethyl | Ethyl | |
| 2.5.163 | 2-Methoxy-3-ethyl-Phenyl | Hydroxymethyl | Ethyl | |
| 2.5.164 | 2-Ethoxy-3-ethyl-Phenyl | Hydroxymethyl | Ethyl | |
| 2.5.165 | 2-Trifluormethoxy-3-ethyl-Phenyl | Hydroxymethyl | Ethyl | |
| 2.5.166 | 2-Nitro-3-ethyl-Phenyl | Hydroxymethyl | Ethyl | |
| 2.5.167 | 2-Fluor-3-propyl-Phenyl | Hydroxymethyl | Ethyl | |
| 2.5.168 | 2-Chlor-3-propyl-Phenyl | Hydroxymethyl | Ethyl | |
| 2.5.169 | 2-Brom-3-propyl-Phenyl | Hydroxymethyl | Ethyl | |
| 2.5.170 | 2-Methyl-3-propyl-Phenyl | Hydroxymethyl | Ethyl | |
| 2.5.171 | 2-Methyl-3-propyl-Phenyl | Hydroxymethyl | Ethyl | |
| 2.5.172 | 2-Cyclopropyl-3-propyl-Phenyl | Hydroxymethyl | Ethyl | |
| 2.5.173 | 2-Vinyl-3-propyl-Phenyl | Hydroxymethyl | Ethyl | |
| 2.5.174 | 2-Ethinyl-3propyl-Phenyl | Hydroxymethyl | Ethyl | |
| 2.5.175 | 2-Cyano-3-propyl-Phenyl | Hydroxymethyl | Ethyl | |
| 2.5.176 | 2-Trifluormethyl-3-propyl-Phenyl | Hydroxymethyl | Ethyl | |
| 2.5.177 | 2-Methoxy-3-propyl-Phenyl | Hydroxymethyl | Ethyl | |
| 2.5.178 | 2-Ethoxy-3-propyl-Phenyl | Hydroxymethyl | Ethyl | |
| 2.5.179 | 2-Trifluormethoxy-3-propyl-Phenyl | Hydroxymethyl | Ethyl | |
| 2.5.180 | 2-Nitro-3-propyl-Phenyl | Hydroxymethyl | Ethyl | |
| 2.5.181 | 2-Fluor-3-isopropyl-Phenyl | Hydroxymethyl | Ethyl | |
| 2.5.182 | 2-Chlor-3-isopropyl-Phenyl | Hydroxymethyl | Ethyl | |
| 2.5.183 | 2-Brom-3-isopropyl-Phenyl | Hydroxymethyl | Ethyl | |
| 2.5.184 | 2-Methyl-3-isopropyl-Phenyl | Hydroxymethyl | Ethyl | |
| 2.5.185 | 2-Ethyl-3-isopropyl-Phenyl | Hydroxymethyl | Ethyl | |
| 2.5.186 | 2-Cyclopropyl-3-isopropyl-Phenyl | Hydroxymethyl | Ethyl | |
| 2.5.187 | 2-Vinyl-3-isopropyl-Phenyl | Hydroxymethyl | Ethyl | |
| 2.5.188 | 2-Ethinyl-3-isopropyl-Phenyl | Hydroxymethyl | Ethyl | |
| 2.5.189 | 2-Cyano-3-isopropyl-Phenyl | Hydroxymethyl | Ethyl | |
| 2.5.190 | 2-Trifluormethyl-3-isopropyl-Phenyl | Hydroxymethyl | Ethyl | |
| 2.5.191 | 2-Methoxy-3-isopropyl-Phenyl | Hydroxymethyl | Ethyl | |
| 2.5.192 | 2-Ethoxy-3-isopropyl-Phenyl | Hydroxymethyl | Ethyl | |
| 2.5.193 | 2-Trifluormethoxy-3-isopropyl-Phenyl | Hydroxymethyl | Ethyl | |
| 2.5.194 | 2-Nitro-3-isopropyl-Phenyl | Hydroxymethyl | Ethyl | |
| 2.5.195 | 2-Fluor-3-tert.butyl-Phenyl | Hydroxymethyl | Ethyl | |
| 2.5.196 | 2-Chlor-3-tert.buty-Phenyl | Hydroxymethyl | Ethyl | |
| 2.5.197 | 2-Brom-3-tert.butyl-Phenyl | Hydroxymethyl | Ethyl | |
| 2.5.198 | 2-Methyl-3-tert.butyl-Phenyl | Hydroxymethyl | Ethyl | |
| 2.5.199 | 2-Ethyl-3-tert.butyl-Phenyl | Hydroxymethyl | Ethyl | |
| 2.5.200 | 2-Cyclopropyl-3-tert.butylPhenyl | Hydroxymethyl | Ethyl | |
| 2.5.201 | 2-Vinyl-3-tert.butyl-Phenyl | Hydroxymethyl | Ethyl | |
| 2.5.202 | 2-Ethinyl-3-tert.butylPhenyl | Hydroxymethyl | Ethyl | |
| 2.5.203 | 2-Cyano-3-tert.butyl-Phenyl | Hydroxymethyl | Ethyl | |
| 2.5.204 | 2-Trifluormethyl-3-tert.butylPhenyl | Hydroxymethyl | Ethyl | |
| 2.5.205 | 2-Methoxy-3-tert.butyl-Phenyl | Hydroxymethyl | Ethyl | |
| 2.5.206 | 2-Ethoxy-3-tert.butyl-Phenyl | Hydroxymethyl | Ethyl | |
| 2.5.207 | 2-Trifluormethoxy-3-tert.butyl- Phenyl | Hydroxymethyl | Ethyl | |
| 2.5.208 | 2-Nitro-3-tert.butyl-Phenyl | Hydroxymethyl | Ethyl | |
| 2.5.209 | 2-Fluor-3-hydroxymethyl-Phenyl | Hydroxymethyl | Ethyl | |
| 2.5.210 | 2-Chlor-3-hydroxymethyl-Phenyl | Hydroxymethyl | Ethyl | |
| 2.5.211 | 2-Brom-3-hydroxymethyl- Phenyl | Hydroxymethyl | Ethyl | |
| 2.5.212 | 2-Methyl-3-hydroxymethyl- Phenyl | Hydroxymethyl | Ethyl | |
| 2.5.213 | 2-Ethyl-3-hydroxymethyl-Phenyl | Hydroxymethyl | Ethyl | |
| 2.5.214 | 2-Cyclopropyl-3-hydroxymethyl-Phenyl | Hydroxymethyl | Ethyl | |
| 2.5.215 | 2-Vinyl-3-hydroxymethyl-Phenyl | Hydroxymethyl | Ethyl | |
| 2.5.216 | 2-Ethinyl-3-hydroxymethyl-Phenyl | Hydroxymethyl | Ethyl | |
| 2.5.217 | 2-Cyano-3-hydroxymethyl- Phenyl | Hydroxymethyl | Ethyl | |
| 2.5.218 | 2-Trifluormethyl-3-hydroxymethyl-Phenyl | Hydroxymethyl | Ethyl | |
| 2.5.219 | 2-Methoxy-3-hydroxymethyl- Phenyl | Hydroxymethyl | Ethyl | |
| 2.5.220 | 2-Ethoxy-3-hydroxymethyl- Phenyl | Hydroxymethyl | Ethyl | |
| 2.5.221 | 2-Trifluormethoxy-3-hydroxymethyl--Phenyl | Hydroxymethyl | Ethyl | |
| 2.5.222 | 2-Nitro-3-hydroxymethyl-Phenyl | Hydroxymethyl | Ethyl | |
| 2.5.223 | 2-Fluor-3-cyclopropyl-Phenyl | Hydroxymethyl | Ethyl | |
| 2.5.224 | 2-Chlor-3-cyclopropyl-Phenyl | Hydroxymethyl | Ethyl | |
| 2.5.225 | 2-Brom-3-cyclopropyl-Phenyl | Hydroxymethyl | Ethyl | |
| 2.5.226 | 2-Methyl-3-cyclopropyl-Phenyl | Hydroxymethyl | Ethyl | |
| 2.5.227 | 2-Ethyl-3-cyclopropyl-Phenyl | Hydroxymethyl | Ethyl | |
| 2.5.228 | 2-Cyclopropyl-3-cyclopropyl-Phenyl | Hydroxymethyl | Ethyl | |
| 2.5.229 | 2-Vinyl-3-cyclopropyl-Phenyl | Hydroxymethyl | Ethyl | |
| 2.5.230 | 2-Ethinyl-3-cyclopropyl-Phenyl | Hydroxymethyl | Ethyl | |
| 2.5.231 | 2-Cyano-3-cyclopropyl-Phenyl | Hydroxymethyl | Ethyl | |
| 2.5.232 | 2-Trifluormethyl-3-cyclopropyl-Phenyl | Hydroxymethyl | Ethyl | |
| 2.5.233 | 2-Methoxy-3-cyclopropyl-Phenyl | Hydroxymethyl | Ethyl | |
| 2.5.234 | 2-Ethoxy-3-cyclopropyl-Phenyl | Hydroxymethyl | Ethyl | |
| 2.5.235 | 2-Trifluormethoxy-3-cyclopropyl-Phenyl | Hydroxymethyl | Ethyl | |
| 2.5.236 | 2-Fluor-3-methoxycarbonyl-Phenyl | Hydroxymethyl | Ethyl | |
| 2.5.237 | 2-Chlor-3-methoxycarbonyl-Phenyl | Hydroxymethyl | Ethyl | |
| 2.5.238 | 2-Brom-3-methoxycarbonyl-Phenyl | Hydroxymethyl | Ethyl | |
| 2.5.239 | 2-Methyl-3-methoxycarbonyl-Phenyl | Hydroxymethyl | Ethyl | |
| 2.5.240 | 2-Ethyl-3-methoxycarbonyl-Phenyl | Hydroxymethyl | Ethyl | |
| 2.5.241 | 2-Cyclopropyl-3-methoxycarbonyl-Phenyl | Hydroxymethyl | Ethyl | |
| 2.5.242 | 2-Vinyl-3-methoxycarbonyl-Phenyl | Hydroxymethyl | Ethyl | |
| 2.5.243 | 2-Ethinyl-3-methoxycarbonyl-Phenyl | Hydroxymethyl | Ethyl | |
| 2.5.244 | 2-Cyano-3-methoxycarbonyl-Phenyl | Hydroxymethyl | Ethyl | |
| 2.5.245 | 2-Trifluormethyl-3-methoxycarbonyl-Phenyl | Hydroxymethyl | Ethyl | |
| 2.5.246 | 2-Methoxy-3-methoxycarbonyl- Phenyl | Hydroxymethyl | Ethyl | |
| 2.5.247 | 2-Ethoxy-3-methoxycarbonyl-Phenyl | Hydroxymethyl | Ethyl | |
| 2.5.248 | 2-Trifluormethoxy-3-methoxycarbonyl-Phenyl | Hydroxymethyl | Ethyl | |
| 2.5.249 | 2-Nitro-3-methoxycarbonyl-Phenyl | Hydroxymethyl | Ethyl | |
| 2.5.250 | 2-Fluor-3-vinyl-Phenyl | Hydroxymethyl | Ethyl | |
| 2.5.251 | 2-Chlor-3-vinyl-Phenyl | Hydroxymethyl | Ethyl | |
| 2.5.252 | 2-Brom-3-vinyl-Phenyl | Hydroxymethyl | Ethyl | |
| 2.5.253 | 2-Methyl-3-vinyl-Phenyl | Hydroxymethyl | Ethyl | |
| 2.5.254 | 2-Ethyl-3-vinyl-Phenyl | Hydroxymethyl | Ethyl | |
| 2.5.255 | 2-Cyclopropyl-3-vinyl-Phenyl | Hydroxymethyl | Ethyl | |
| 2.5.256 | 2-Vinyl-3-vinyl-Phenyl | Hydroxymethyl | Ethyl | |
| 2.5.257 | 2-Ethinyl-3-vinyl-Phenyl | Hydroxymethyl | Ethyl | |
| 2.5.258 | 2-Cyano-3-vinyl-Phenyl | Hydroxymethyl | Ethyl | |
| 2.5.259 | 2-Trifluormethyl-3-vinyl-Phenyl | Hydroxymethyl | Ethyl | |
| 2.5.260 | 2-Methoxy-3-vinyl-Phenyl | Hydroxymethyl | Ethyl | |
| 2.5.261 | 2-Ethoxy-3-vinyl-Phenyl | Hydroxymethyl | Ethyl | |
| 2.5.262 | 2-Trifluormethoxy-3-vinyl-Phenyl | Hydroxymethyl | Ethyl | |
| 2.5.263 | 2-Nitro-3-vinyl-Phenyl | Hydroxymethyl | Ethyl | |
| 2.5.264 | 2-Fluor-3-ethinyl-Phenyl | Hydroxymethyl | Ethyl | |
| 2.5.265 | 2-Chlor-3-ethinyl-Phenyl | Hydroxymethyl | Ethyl | |
| 2.5.266 | 2-Brom-3-ethinyl-Phenyl | Hydroxymethyl | Ethyl | |
| 2.5.267 | 2-Methyl-3-ethinyl-Phenyl | Hydroxymethyl | Ethyl | |
| 2.5.268 | 2-Ethyl-3-ethinyl-Phenyl | Hydroxymethyl | Ethyl | |
| 2.5.269 | 2-Cyclopropyl-3-ethinyl-Phenyl | Hydroxymethyl | Ethyl | |
| 2.5.270 | 2-Vinyl-3-ethinyl-Phenyl | Hydroxymethyl | Ethyl | |
| 2.5.271 | 2-Cyano-3-ethinyl-Phenyl | Hydroxymethyl | Ethyl | |
| 2.5.272 | 2-Trifluormethyl-3-ethinyl-Phenyl | Hydroxymethyl | Ethyl | |
| 2.5.273 | 2-Methoxy-3-ethinyl-Phenyl | Hydroxymethyl | Ethyl | |
| 2.5.274 | 2-Ethoxy-3-ethinyl-Phenyl | Hydroxymethyl | Ethyl | |
| 2.5.275 | 2-Trifluormethoxy-3-ethinyl-Phenyl | Hydroxymethyl | Ethyl | |
| 2.5.276 | 2-Nitro-3-ethinyl-Phenyl | Hydroxymethyl | Ethyl | |
| 2.5.277 | 2-Fluor-3-ethinyl-Phenyl | Hydroxymethyl | Ethyl | |
| 2.5.278 | 2-Fluor-3-cyano-Phenyl | Hydroxymethyl | Ethyl | |
| 2.5.279 | 2-Chlor-3-cyano-Phenyl | Hydroxymethyl | Ethyl | |
| 2.5.280 | 2-Brom-3-cyano-Phenyl | Hydroxymethyl | Ethyl | |
| 2.5.281 | 2-Methyl-3-cyano-Phenyl | Hydroxymethyl | Ethyl | |
| 2.5.282 | 2-Ethyl-3-cyano-Phenyl | Hydroxymethyl | Ethyl | |
| 2.5.283 | 2-Ethyl-3-cyano-Phenyl | 1-Hydroxyethyl | Ethyl | |
| 2.5.284 | 2-Ethyl-3-cyano-Phenyl | 1-Hydroxypropyl | Ethyl | |
| 2.5.285 | 2-Ethyl-3-cyano-Phenyl | (1-Hydroxy-2-methylpropyl) | Ethyl | |
| 2.5.286 | 2-Ethyl-3-cyano-Phenyl | Methoxymethyl | Ethyl | |
| 2.5.287 | 2-Ethyl-3-cyano-Phenyl | 2-Methoxyethyl | Ethyl | |
| 2.5.288 | 2-Cyclopropyl-3-cyano-Phenyl | Hydroxymethyl | Ethyl | |
| 2.5.289 | 2-Vinyl-3-cyano-Phenyl | Hydroxymethyl | Ethyl | |
| 2.5.290 | 2-Ethinyl-3-cyano-Phenyl | Hydroxymethyl | Ethyl | |
| 2.5.291 | 2-Cyano-3-cyanoPhenyl | Hydroxymethyl | Ethyl | |
| 2.5.292 | 2-Trifluormethyl-3-cyano-Phenyl | Hydroxymethyl | Ethyl | |
| 2.5.293 | 2-Methoxy-3-cyano-Phenyl | Hydroxymethyl | Ethyl | |
| 2.5.294 | 2-Ethoxy-3-cyano-Phenyl | Hydroxymethyl | Ethyl | |
| 2.5.295 | 2-Trifluormethoxy-3-cyano-Phenyl | Hydroxymethyl | Ethyl | |
| 2.5.296 | 2-Nitro-3-cyano-Phenyl | Hydroxymethyl | Ethyl | |
| 2.5.297 | 2-Fluor-3-hydroxy-Phenyl | Hydroxymethyl | Ethyl | |
| 2.5.298 | 2-Chlor-3-hydroxy-Phenyl | Hydroxymethyl | Ethyl | |
| 2.5.299 | 2-Brom-3-hydroxy-Phenyl | Hydroxymethyl | Ethyl | |
| 2.5.300 | 2-Methyl-3-hydroxy-Phenyl | Hydroxymethyl | Ethyl | |
| 2.5.301 | 2-Ethyl-3-hydroxy-Phenyl | Hydroxymethyl | Ethyl | |
| 2.5.302 | 2-Cyclopropyl-3-hydroxy-Phenyl | Hydroxymethyl | Ethyl | |
| 2.5.303 | 2-Vinyl-3-hydroxy-Phenyl | Hydroxymethyl | Ethyl | |
| 2.5.304 | 2-Ethinyl-3-hydroxy-Phenyl | Hydroxymethyl | Ethyl | |
| 2.5.305 | 2-Cyano-3-hydroxy-Phenyl | Hydroxymethyl | Ethyl | |
| 2.5.306 | 2-Trifluormethyl-3-hydroxy-Phenyl | Hydroxymethyl | Ethyl | |
| 2.5.307 | 2-Methoxy-3-hydroxy-Phenyl | Hydroxymethyl | Ethyl | |
| 2.5.308 | 2-Ethoxy-3-hydroxy-Phenyl | Hydroxymethyl | Ethyl | |
| 2.5.309 | 2-Trifluormethoxy-3-hydroxy-Phenyl | Hydroxymethyl | Ethyl | |
| 2.5.310 | 2-Nitro-3-hydroxy-Phenyl | Hydroxymethyl | Ethyl | |
| 2.5.311 | 2-Fluor-3-methoxy-Phenyl | Hydroxymethyl | Ethyl | |
| 2.5.312 | 2-Chlor-3-methoxy-Phenyl | Hydroxymethyl | Ethyl | |
| 2.5.313 | 2-Brom-3-methoxy-Phenyl | Hydroxymethyl | Ethyl | |
| 2.5.314 | 2-Methyl-3-methoxy-Phenyl | Hydroxymethyl | Ethyl | |
| 2.5.315 | 2-Ethyl-3-methoxy-Phenyl | Hydroxymethyl | Ethyl | |
| 2.5.316 | 2-Cyclopropyl-3-methoxy-Phenyl | Hydroxymethyl | Ethyl | |
| 2.5.317 | 2-Vinyl-3-methoxy-Phenyl | Hydroxymethyl | Ethyl | |
| 2.5.318 | 2-Ethinyl-3-methoxy-Phenyl | Hydroxymethyl | Ethyl | |
| 2.5.319 | 2-Cyano-3-methoxy-Phenyl | Hydroxymethyl | Ethyl | |
| 2.5.320 | 2-Trifluormethyl-3-methoxy-Phenyl | Hydroxymethyl | Ethyl | |
| 2.5.321 | 2,3-Dimethoxy-Phenyl | Hydroxymethyl | Ethyl | |
| 2.5.322 | 2-Ethoxy-3-methoxy-Phenyl | Hydroxymethyl | Ethyl | |
| 2.5.323 | 2-Trifluormethoxy-3-methoxy-Phenyl | Hydroxymethyl | Ethyl | |
| 2.5.324 | 2-Nitro-3-methoxy-Phenyl | Hydroxymethyl | Ethyl | |
| 2.5.325 | 2-Fluor-3-ethoxy-Phenyl | Hydroxymethyl | Ethyl | |
| 2.5.326 | 2-Chlor-3-ethoxy-Phenyl | Hydroxymethyl | Ethyl | |
| 2.5.327 | 2-Brom-3-ethoxy-Phenyl | Hydroxymethyl | Ethyl | |
| 2.5.328 | 2-Methyl-3-ethoxy-Phenyl | Hydroxymethyl | Ethyl | |
| 2.5.329 | 2-Ethyl-3-ethoxy-Phenyl | Hydroxymethyl | Ethyl | |
| 2.5.330 | 2-Cyclopropyl-3-ethoxy-Phenyl | Hydroxymethyl | Ethyl | |
| 2.5.331 | 2-Vinyl-3-ethoxy-Phenyl | Hydroxymethyl | Ethyl | |
| 2.5.332 | 2-Ethinyl-3-ethoxy-Phenyl | Hydroxymethyl | Ethyl | |
| 2.5.333 | 2-Cyano-3-ethoxy-Phenyl | Hydroxymethyl | Ethyl | |
| 2.5.334 | 2-Trifluormethyl-3-ethoxy-Phenyl | Hydroxymethyl | Ethyl | |
| 2.5.335 | 2-Methoxy-3-ethoxy-Phenyl | Hydroxymethyl | Ethyl | |
| 2.5.336 | 2,3-Diethoxy--Phenyl | Hydroxymethyl | Ethyl | |
| 2.5.337 | 2-Trifluormethoxy-3-ethoxy-Phenyl | Hydroxymethyl | Ethyl | |
| 2.5.338 | 2-Nitro-3-ethoxy-Phenyl | Hydroxymethyl | Ethyl | |
| 2.5.339 | 2-Fluor-3-propoxy-Phenyl | Hydroxymethyl | Ethyl | |
| 2.5.340 | 2-Chlor-3-propoxy-Phenyl | Hydroxymethyl | Ethyl | |
| 2.5.341 | 2-Brom-3-propoxy-Phenyl | Hydroxymethyl | Ethyl | |
| 2.5.342 | 2-Methyl-3-propoxy-Phenyl | Hydroxymethyl | Ethyl | |
| 2.5.343 | 2-Ethyl-3-propoxy-Phenyl | Hydroxymethyl | Ethyl | |
| 2.5.344 | 2-Cyclopropyl-3-propoxy-Phenyl | Hydroxymethyl | Ethyl | |
| 2.5.345 | 2-Vinyl-3-propoxy-Phenyl | Hydroxymethyl | Ethyl | |
| 2.5.346 | 2-Ethinyl-3-propoxy-Phenyl | Hydroxymethyl | Ethyl | |
| 2.5.347 | 2-Cyano-3-propoxy-Phenyl | Hydroxymethyl | Ethyl | |
| 2.5.348 | 2-Trifluormethyl-3-propoxy-Phenyl | Hydroxymethyl | Ethyl | |
| 2.5.349 | 2-Methoxy-3-propoxy-Phenyl | Hydroxymethyl | Ethyl | |
| 2.5.350 | 2-Ethoxy-3-propoxy-Phenyl | Hydroxymethyl | Ethyl | |
| 2.5.351 | 2-Trifluormethoxy-3-propoxy-Phenyl | Hydroxymethyl | Ethyl | |
| 2.5.352 | 2-Nitro-3-propoxy-Phenyl | Hydroxymethyl | Ethyl | |
| 2.5.353 | 2-Fluor-3-isopropoxy-Phenyl | Hydroxymethyl | Ethyl | |
| 2.5.354 | 2-Chlor-3-isopropoxy-Phenyl | Hydroxymethyl | Ethyl | |
| 2.5.355 | 2-Brom-3-isopropoxy-Phenyl | Hydroxymethyl | Ethyl | |
| 2.5.356 | 2-Methyl-3-isopropoxy-Phenyl | Hydroxymethyl | Ethyl | |
| 2.5.357 | 2-Ethyl-3-isopropoxy-Phenyl | Hydroxymethyl | Ethyl | |
| 2.5.358 | 2-Cyclopropyl-3-isopropoxy-Phenyl | Hydroxymethyl | Ethyl | |
| 2.5.359 | 2-Vinyl-3-isopropoxy-Phenyl | Hydroxymethyl | Ethyl | |
| 2.5.360 | 2-Ethinyl-3-isopropoxy-Phenyl | Hydroxymethyl | Ethyl | |
| 2.5.361 | 2-Cyano-3-isopropoxy-Phenyl | Hydroxymethyl | Ethyl | |
| 2.5.362 | 2-Trifluormethyl-3-isopropoxy-Phenyl | Hydroxymethyl | Ethyl | |
| 2.5.363 | 2-Methoxy-3-isopropoxy-Phenyl | Hydroxymethyl | Ethyl | |
| 2.5.364 | 2-Ethoxy-3-isopropoxy-Phenyl | Hydroxymethyl | Ethyl | |
| 2.5.365 | 2-Trifluormethoxy-3-isopropoxy-Phenyl | Hydroxymethyl | Ethyl | |
| 2.5.366 | 2-Nitro-3-isopropoxy-Phenyl | Hydroxymethyl | Ethyl | |
| 2.5.367 | 2-Fluor-3-tert.butoxy-Phenyl | Hydroxymethyl | Ethyl | |
| 2.5.368 | 2-Chlor-3-tert.butoxy-Phenyl | Hydroxymethyl | Ethyl | |
| 2.5.369 | 2-Brom-3-tert.butoxy-Phenyl | Hydroxymethyl | Ethyl | |
| 2.5.370 | 2-Methyl-3-tert.butoxy-Phenyl | Hydroxymethyl | Ethyl | |
| 2.5.371 | 2-Ethyl-3-tert.butoxy-Phenyl | Hydroxymethyl | Ethyl | |
| 2.5.372 | 2-Cyclopropyl-3-tert.butoxy-Phenyl | Hydroxymethyl | Ethyl | |
| 2.5.373 | 2-Vinyl-3-tert.butoxy-Phenyl | Hydroxymethyl | Ethyl | |
| 2.5.374 | 2-Ethinyl-3-tert.butoxy-Phenyl | Hydroxymethyl | Ethyl | |
| 2.5.375 | 2-Cyano-3-tert.butoxy-Phenyl | Hydroxymethyl | Ethyl | |
| 2.5.376 | 2-Trifluormethyl-3-tert.butoxy-Phenyl | Hydroxymethyl | Ethyl | |
| 2.5.377 | 2-Methoxy-3-tert.butoxy-Phenyl | Hydroxymethyl | Ethyl | |
| 2.5.378 | 2-Ethoxy-3-tert.butoxy-Phenyl | Hydroxymethyl | Ethyl | |
| 2.5.379 | 2-Trifluormethoxy-3-tert.butoxy-Phenyl | Hydroxymethyl | Ethyl | |
| 2.5.380 | 2-Nitro-3-tert.butoxy-Phenyl | Hydroxymethyl | Ethyl | |
| 2.5.381 | 2-Fluor-3-trifluormethoxy-Phenyl | Hydroxymethyl | Ethyl | |
| 2.5.382 | 2-Chlor-3-trifluormethoxy-Phenyl | Hydroxymethyl | Ethyl | |
| 2.5.383 | 2-Brom-3-trifluormethoxy-Phenyl | Hydroxymethyl | Ethyl | |
| 2.5.384 | 2-Methyl-3-trifluormethoxy-Phenyl | Hydroxymethyl | Ethyl | |
| 2.5.385 | 2-Ethyl-3-trifluormethoxy-Phenyl | Hydroxymethyl | Ethyl | |
| 2.5.386 | 2-Cyclopropyl-3-trifluormethoxy-Phenyl | Hydroxymethyl | Ethyl | |
| 2.5.387 | 2-Vinyl-3-trifluormethoxy-Phenyl | Hydroxymethyl | Ethyl | |
| 2.5.388 | 2-Ethinyl-3-trifluormethoxy-Phenyl | Hydroxymethyl | Ethyl | |
| 2.5.389 | 2-Cyano-3-trifluormethoxy-Phenyl | Hydroxymethyl | Ethyl | |
| 2.5.390 | 2-Trifluormethyl-3-trifluormethoxy-Phenyl | Hydroxymethyl | Ethyl | |
| 2.5.391 | 2-Methoxy-3-trifluormethoxy-Phenyl | Hydroxymethyl | Ethyl | |
| 2.5.392 | 2-Ethoxy-3-trifluormethoxy-Phenyl | Hydroxymethyl | Ethyl | |
| 2.5.393 | 2,3-Bis(trifluormethoxy)-Phenyl | Hydroxymethyl | Ethyl | |
| 2.5.394 | 2-Nitro-3-trifluormethoxy-Phenyl | Hydroxymethyl | Ethyl | |
| 2.5.395 | 2-Fluor-3-(2,2,2-trifluorethoxy)-Phenyl | Hydroxymethyl | Ethyl | |
| 2.5.396 | 2-Chlor-3-(2,2,2-trifluorethoxy)-Phenyl | Hydroxymethyl | Ethyl | |
| 2.5.397 | 2-Brom-3-(2,2,2-trifluorethoxy)-Phenyl | Hydroxymethyl | Ethyl | |
| 2.5.398 | 2-Methyl-3-(2,2,2-trifluorethoxy)-Phenyl | Hydroxymethyl | Ethyl | |
| 2.5.399 | 2-Ethyl-3-(2,2,2-trifluorethoxy)-Phenyl | Hydroxymethyl | Ethyl | |
| 2.5.400 | 2-Cyclopropyl-3-(2,2,2-trifluorethoxy)-Phenyl | Hydroxymethyl | Ethyl | |
| 2.5.401 | 2-Vinyl-3-(2,2,2-trifluorethoxy)-Phenyl | Hydroxymethyl | Ethyl | |
| 2.5.402 | 2-Ethinyl-3-(2,2,2-trifluorethoxy-Phenyl | Hydroxymethyl | Ethyl | |
| 2.5.403 | 2-Cyano-3-(2,2,2-trifluorethoxy)-Phenyl | Hydroxymethyl | Ethyl | |
| 2.5.404 | 2-Trifluormethyl-3-(2,2,2-trifluorethoxy)-Phenyl | Hydroxymethyl | Ethyl | |
| 2.5.405 | 2-Methoxy-3-(2,2,2-trifluorethoxy)-Phenyl | Hydroxymethyl | Ethyl | |
| 2.5.406 | 2-Ethoxy-3-(2,2,2-trifluorethoxy)-Phenyl | Hydroxymethyl | Ethyl | |
| 2.5.407 | 2-Trifluormethoxy-3-(2,2,2-trifluorethoxy)-Phenyl | Hydroxymethyl | Ethyl | |
| 2.5.408 | 2-Nitro-3-(2,2,2-trifluorethoxy)-Phenyl | Hydroxymethyl | Ethyl | |
| 2.5.409 | 2-Fluor-3-difluormethoxy-Phenyl | Hydroxymethyl | Ethyl | |
| 2.5.410 | 2-Chlor-3-difluormethoxy-Phenyl | Hydroxymethyl | Ethyl | |
| 2.5.411 | 2-Brom-3-difluormethoxy-Phenyl | Hydroxymethyl | Ethyl | |
| 2.5.412 | 2-Methyl-3-difluormethoxy-Phenyl | Hydroxymethyl | Ethyl | |
| 2.5.413 | 2-Ethyl-3-difluormethoxy-Phenyl | Hydroxymethyl | Ethyl | |
| 2.5.414 | 2-Cyclopropyl-3-difluormethoxy-Phenyl | Hydroxymethyl | Ethyl | |
| 2.5.415 | 2-Vinyl-3-difluormethoxy-Phenyl | Hydroxymethyl | Ethyl | |
| 2.5.416 | 2-Ethinyl-3-difluormethoxy-Phenyl | Hydroxymethyl | Ethyl | |
| 2.5.417 | 2-Cyano-3-difluormethoxy- Phenyl | Hydroxymethyl | Ethyl | |
| 2.5.418 | 2-Trifluormethyl-3-difluormethoxy -Phenyl | Hydroxymethyl | Ethyl | |
| 2.5.419 | 2-Methoxy-3-difluormethoxy- Phenyl | Hydroxymethyl | Ethyl | |
| 2.5.420 | 2-Ethoxy-3-difluormethoxy-Phenyl | Hydroxymethyl | Ethyl | |
| 2.5.421 | 2-Trifluormethoxy-3-difluormethoxy-Phenyl | Hydroxymethyl | Ethyl | |
| 2.5.422 | 2-Nitro-3-difluormethoxy-Phenyl | Hydroxymethyl | Ethyl | |
| 2.5.423 | 2-Fluor-3-(2-methoxyethoxy)-Phenyl | Hydroxymethyl | Ethyl | |
| 2.5.424 | 2-Chlor-3-(2-methoxyethoxy)-Phenyl | Hydroxymethyl | Ethyl | |
| 2.5.425 | 2-Brom-3-(2-methoxyethoxy)-Phenyl | Hydroxymethyl | Ethyl | |
| 2.5.426 | 2-Methyl-3-(2-methoxyethoxy)-Phenyl | Hydroxymethyl | Ethyl | |
| 2.5.427 | 2-Ethyl-3-(2-methoxyethoxy)-Phenyl | Hydroxymethyl | Ethyl | |
| 2.5.428 | 2-Cyclopropyl-3-(2-methoxyethoxy)-Phenyl | Hydroxymethyl | Ethyl | |
| 2.5.429 | 2-Vinyl-3-(2-methoxyethoxy)-Phenyl | Hydroxymethyl | Ethyl | |
| 2.5.430 | 2-Ethinyl-3-(2-methoxyethoxy)-Phenyl | Hydroxymethyl | Ethyl | |
| 2.5.431 | 2-Cyano-3-(2-methoxyethoxy)- Phenyl | Hydroxymethyl | Ethyl | |
| 2.5.432 | 2-Trifluormethyl-3-(2-methoxyethoxy)-Phenyl | Hydroxymethyl | Ethyl | |
| 2.5.433 | 2-Methoxy-3-(2-methoxyethoxy) -Phenyl | Hydroxymethyl | Ethyl | |
| 2.5.434 | 2-Ethoxy-3-(2-methoxyethoxy)-Phenyl | Hydroxymethyl | Ethyl | |
| 2.5.435 | 2-Trifluormethoxy-(2-methoxyethoxy)-Phenyl | Hydroxymethyl | Ethyl | |
| 2.5.436 | 2-Nitro-3-(2-methoxyethoxy)-Phenyl | Hydroxymethyl | Ethyl | |
| 2.5.437 | 2-Fluor-3-(tert.butoxycarbonyloxy)-Phenyl | Hydroxymethyl | Ethyl | |
| 2.5.438 | 2-Chlor-3-(tert.butoxycarbonyloxy)-Phenyl | Hydroxymethyl | Ethyl | |
| 2.5.439 | 2-Brom-3-(tert.butoxycarbonyloxy)-Phenyl | Hydroxymethyl | Ethyl | |
| 2.5.440 | 2-Methyl-3-(tert.butoxycarbonyloxy)-Phenyl | Hydroxymethyl | Ethyl | |
| 2.5.441 | 2-Ethyl-3-(tert.butoxycarbonyloxy)-Phenyl | Hydroxymethyl | Ethyl | |
| 2.5.442 | 2-Cyclopropyl-3-(tert.butoxycarbonyloxy)-Phenyl | Hydroxymethyl | Ethyl | |
| 2.5.443 | 2-Vinyl-3-(tert.butoxycarbonyloxy)-Phenyl | Hydroxymethyl | Ethyl | |
| 2.5.444 | 2-Ethinyl-3-(tert.butoxycarbonyloxy)-Phenyl | Hydroxymethyl | Ethyl | |
| 2.5.445 | 2-Cyano-3-(tert.butoxycarbonyloxy)-Phenyl | Hydroxymethyl | Ethyl | |
| 2.5.446 | 2-Trifluormethyl-3-(tert.butoxycarbonyloxy)-Phenyl | Hydroxymethyl | Ethyl | |
| 2.5.447 | 2-Methoxy-3-(tert.butoxycarbonyloxy)-Phenyl | Hydroxymethyl | Ethyl | |
| 2.5.448 | 2-Ethoxy-3-(tert.butoxycarbonyloxy)-Phenyl | Hydroxymethyl | Ethyl | |
| 2.5.449 | 2-Trifluormethoxy-3-(tert.butoxycarbonyloxy)-Phenyl | Hydroxymethyl | Ethyl | |
| 2.5.450 | 2-Nitro-3-(tert.butoxycarbonyloxy)-Phenyl | Hydroxymethyl | Ethyl | |
| 2.5.451 | 2-Fluor-3-nitro-Phenyl | Hydroxymethyl | Ethyl | |
| 2.5.452 | 2-Chlor-3-nitro-Phenyl | Hydroxymethyl | Ethyl | |
| 2.5.453 | 2-Brom-3-nitro-Phenyl | Hydroxymethyl | Ethyl | |
| 2.5.454 | 2-Methyl-3-nitro-Phenyl | Hydroxymethyl | Ethyl | |
| 2.5.455 | 2-Ethyl-3-nitro-Phenyl | Hydroxymethyl | Ethyl | |
| 2.5.456 | 2-Cyclopropyl-3-nitro-Phenyl | Hydroxymethyl | Ethyl | |
| 2.5.457 | 2-Vinyl-3-nitro-Phenyl | Hydroxymethyl | Ethyl | |
| 2.5.458 | 2-Ethinyl-3-nitro-Phenyl | Hydroxymethyl | Ethyl | |
| 2.5.459 | 2-Cyano-3-nitro-Phenyl | Hydroxymethyl | Ethyl | |
| 2.5.460 | 2-Trifluormethyl-3-nitro-Phenyl | Hydroxymethyl | Ethyl | |
| 2.5.461 | 2-Methoxy-3-nitro-Phenyl | Hydroxymethyl | Ethyl | |
| 2.5.462 | 2-Ethoxy-3-nitro-Phenyl | Hydroxymethyl | Ethyl | |
| 2.5.463 | 2-Trifluormethoxy-3-nitro-Phenyl | Hydroxymethyl | Ethyl | |
| 2.5.464 | 2-Fluor-3-methylsulfanylPhenyl | Hydroxymethyl | Ethyl | |
| 2.5.465 | 2-Chlor-3-methylsulfanyl-Phenyl | Hydroxymethyl | Ethyl | |
| 2.5.466 | 2-Brom-3-methylsulfanyl-Phenyl | Hydroxymethyl | Ethyl | |
| 2.5.467 | 2-Methyl-3-methylsulfanyl-Phenyl | Hydroxymethyl | Ethyl | |
| 2.5.468 | 2-Ethyl-3-methylsulfanyl-Phenyl | Hydroxymethyl | Ethyl | |
| 2.5.469 | 2-Cyclopropyl-3-methylsulfanyl-Phenyl | Hydroxymethyl | Ethyl | |
| 2.5.470 | 2-Vinyl-3-methylsulfanyl-Phenyl | Hydroxymethyl | Ethyl | |
| 2.5.471 | 2-Ethinyl-3-methylsulfanyl-Phenyl | Hydroxymethyl | Ethyl | |
| 2.5.472 | 2-Cyano-3-methylsulfanyl - Phenyl | Hydroxymethyl | Ethyl | |
| 2.5.473 | 2-Trifluormethyl-3-methylsulfanyl -Phenyl | Hydroxymethyl | Ethyl | |
| 2.5.474 | 2-Methoxy-3-methylsulfanyl - Phenyl | Hydroxymethyl | Ethyl | |
| 2.5.475 | 2-Ethoxy-3-methylsulfanyl-Phenyl | Hydroxymethyl | Ethyl | |
| 2.5.476 | 2-Trifluormethoxy-3-methylsulfanyl-Phenyl | Hydroxymethyl | Ethyl | |
| 2.5.477 | 2-Nitro-3-methylsulfanyl-Phenyl | Hydroxymethyl | Ethyl | |
| 2.5.478 | 3,5-Difluor-Phenyl | Hydroxymethyl | Ethyl | [CDCl3] 1.32 (t,3H); 2.23 (m,1H); 3.56-3.69 (m,2H); 3.90 (m,1H); 4.02 (m,1H); 4.29 (m,2H); 6.88 (m,1H); 7.19 (m,2H). |
| 2.5.4 79 | 3,5-Difluor-Phenyl | 1-Hydroxyethyl | Methyl | [CDCl3] D1 1.06 (t,3H); 1.30-1.49 (m,2H); 2.27 (d,1H); 3.52 (d,1H); 3.73 (d,1H); 3.83 (s,3H); 4.05 (m,1H); 6.88 (t,1H); 7.20 (d,2H). [CDCl3] D2 1.06 (t,3H); 1.55 (m,2H); 1.97 (d,1H); 3.58 (d,1H); 3.70 (d,1H); 3.83 (s,3H); 3.94 (t,1H); 6.88 (t,1H); 7.18 (d,2H). |
| 2.5.480 | 3,5-Difluor-Phenyl | 1-Hydroxypropyl | Ethyl | |
| 2.5.481 | 3,5-Difluor-Phenyl | (1-Hydroxy-2-methylpropyl) | Ethyl | |
| 2.5.482 | 3,5-Difluor-Phenyl | Methoxymethyl | Ethyl | [CDCl3] 1.33 (t,3H); 3.44 (s,3H); 3.47-3.51 (d,1H); 3.72-3.83 (m,3H); 4.29 (m,2H): 6.88 (m,1H); 7.20 (m,2H). |
| 2.5.483 | 2-Ethyl-3-cyano-Phenyl | 2-Methoxyethyl | Ethyl | |
| 2.5.484 | 3-Chlor-5-fluor-Phenyl | Hydroxymethyl | Ethyl | |
| 2.5.485 | 3-Chlor-5-fluor-Phenyl | 1-Hydroxyethyl | Methyl | [CDCl3]D1 1.19 (d,3H); 2.42 (s br,1H); 3.53 (d,1H); 3.68 (d,1H); 3.83 (s,3H); 4.34 (m,1H); 7.14 (d,1H); 7.31 (d,1H); 7.44 (s,1H). D2 1.29 (d,3H); 2.18 (s br,1H); 3.58 (d,1H); 3.73 (d,1H); 3.73 (s,3H); 4.22 (m,1H); 7.14 (d,1H); 7.31 (d,1H); 7.42 (s,1H). |
| 2.5.486 | 3-Chlor-5-fluor-Phenyl | 1-Hydroxypropyl | Ethyl | |
| 2.5.487 | 3-Chlor-5-fluor-Phenyl | (1-Hydroxy-2-methylpropyl) | Ethyl | |
| 2.5.488 | 3-Chlor-5-fluor-Phenyl | Methoxymethyl | Ethyl | |
| 2.5.489 | 3-Chlor-5-fluor-Phenyl | 2-Methoxyethyl | Ethyl | |
| 2.5.490 | 3-Brom-5-fluor-Phenyl | Hydroxymethyl | Ethyl | |
| 2.5.491 | 3-Brom-5-fluor-Phenyl | 1-Hydroxyethyl | Ethyl | |
| 2.5.492 | 3-Brom-5-fluor-Phenyl | 1-Hydroxypropyl | Ethyl | |
| 2.5.493 | 3-Brom-5-fluor-Phenyl | (1-Hydroxy-2-methylpropyl) | Ethyl | |
| 2.5.494 | 3-Brom-5-fluor-Phenyl | Methoxymethyl | Ethyl | |
| 2.5.495 | 3-Brom-5-fluor-Phenyl | 2-Methoxyethyl | Ethyl | |
| 2.5.496 | 3-lod-5-fluor-Phenyl | Hydroxymethyl | Ethyl | |
| 2.5.497 | 3-Methyl-5-fluor-Phenyl | Hydroxymethyl | Ethyl | |
| 2.5.498 | 3-Methyl-5-fluor-Phenyl | 1-Hydroxyethyl | Methyl | [CDCl3] D1 1.20 (d,3H); 2.37 (d,1H); 2.38 (s,3H); 3.54 (d,1H), 3.69 (d,1H); 3.83 (s,3H); 4.33 (m,1H); 6.96 (s,1H); 7.23 (s,1H); 7.25 (s,1H). D2 1.26 (d,3H); 2.18 (d,1H); 2.38 (s,3H); 3.60 (d,1H); 3.72 (d,1H); 3.83 (s,3H); 4.22 (m,1H); 6.93 (s,1H); 7.21 (s,1H); 7.25 (s,1H) |
| 2.5.499 | 3-Methyl-5-fluor-Phenyl | 1-Hydroxypropyl | Ethyl | |
| 2.5.500 | 3-Methyl-5-fluor-Phenyl | (1-Hydroxy-2-methylpropyl) | Ethyl | |
| 2.5.501 | 3-Methyl-5-fluor-Phenyl | Methoxymethyl | Ethyl | |
| 2.5.502 | 3-Methyl-5-fluor-Phenyl | 2-Methoxyethyl | Ethyl | |
| 2.5.503 | 3-Ethyl-5-fluor-Phenyl | Hydroxymethyl | Ethyl | |
| 2.5.504 | 3-Propyl-5-fluor-Phenyl | Hydroxymethyl | Ethyl | |
| 2.5.505 | 3-iPropyl-5-fluor-Phenyl | Hydroxymethyl | Ethyl | |
| 2.5.506 | 3-nButyl-5-fluor-Phenyl | Hydroxymethyl | Ethyl | |
| 2.5.507 | 3-isoButyl-5-fluor-Phenyl | Hydroxymethyl | Ethyl | |
| 2.5.508 | 3-tert.Butyl-5-fluor-Phenyl | Hydroxymethyl | Ethyl | |
| 2.5.509 | 3-Cyclopropyl-5-fluor-Phenyl | Hydroxymethyl | Ethyl | |
| 2.5.510 | 3-Vinyl-5-fluor-Phenyl | Hydroxymethyl | Ethyl | |
| 2.5.511 | 3-Ethinyl-5-fluor-Phenyl | Hydroxymethyl | Ethyl | |
| 2.5.512 | 3-Cyano-5-fluor-Phenyl | Hydroxymethyl | Ethyl | |
| 2.5.513 | 3-Trifluormethyl-5-fluor-Phenyl | Hydroxymethyl | Ethyl | |
| 2.5.514 | 3-Trifluormethyl-5-fluor-Phenyl | 1-Hydroxyethyl | Ethyl | |
| 2.5.515 | 3-Trifluormethyl-5-fluor-Phenyl | 1-Hydroxypropyl | Ethyl | |
| 2.5.516 | 3-Trifluormethyl-5-fluor-Phenyl | (1-Hydroxy-2-methylpropyl) | Ethyl | |
| 2.5.517 | 3-Trifluormethyl-5-fluor-Phenyl | Methoxymethyl | Ethyl | |
| 2.5.518 | 3-Trifluormethyl-5-fluor-Phenyl | 2-Methoxyethyl | Ethyl | |
| 2.5.519 | 3-(Methoxycarbony)l-5-fluor-Phenyl | Hydroxymethyl | Ethyl | |
| 2.5.520 | 3-Hydroxymethyl-5-fluor-Phenyl | Hydroxymethyl | Ethyl | |
| 2.5.521 | 3-Carbamoyl-5-fluor-Phenyl | Hydroxymethyl | Ethyl | |
| 2.5.522 | 3-Hydroxy-5-fluor-Phenyl- | Hydroxymethyl | Ethyl | |
| 2.5.523 | 3-Methoxy-5-fluor-Phenyl | Hydroxymethyl | Ethyl | |
| 2.5.524 | 3-Ethoxy-5-fluor-Phenyl | Hydroxymethyl | Ethyl | |
| 2.5.525 | 3-nPropyoxy-5-fluor-Phenyl | Hydroxymethyl | Ethyl | |
| 2.5.526 | 3-isoPropoxy-5-fluor-Phenyl | Hydroxymethyl | Ethyl | |
| 2.5.527 | 3-nButoxy-5-fluor-Phenyl | Hydroxymethyl | Ethyl | |
| 2.5.528 | 3-isoButoxy-5-fluor-Phenyl | Hydroxymethyl | Ethyl | |
| 2.5.529 | 3-tert.Butoxy-5-fluor-Phenyl | Hydroxymethyl | Ethyl | |
| 2.5.530 | 3-Difluormethoxy-5-fluor-Phenyl | Hydroxymethyl | Ethyl | |
| 2.5.531 | 3-Trifluormethoxy-5-fluor-Phenyl | Hydroxymethyl | Ethyl | |
| 2.5.532 | 3-(2,2,2-Trifluorethoxy)-5-fluor-Phenyl | Hydroxymethyl | Ethyl | |
| 2.5.533 | 3-(2-Chlorethoxy)-5-fluor-Phenyl | Hydroxymethyl | Ethyl | |
| 2.5.534 | 3-(2-Hydroxyethoxy)-5-fluor-Phenyl- | Hydroxymethyl | Ethyl | |
| 2.5.535 | 3-[(tert-Butoxycarbonyl)oxy]-5-fluor-Phenyl | Hydroxymethyl | Ethyl | |
| 2.5.536 | 3-Nitro-5-fluor-Phenyl | Hydroxymethyl | Ethyl | |
| 2.5.537 | 3-Acetoxy-5-fluor-Phenyl | Hydroxymethyl | Ethyl | |
| 2.5.538 | {3-[(tert-Butoxycarbonyl)amino]-5-fluor-Phenyl} | Hydroxymethyl | Ethyl | |
| 2.5.539 | 3-Methylsulfanyl-5-fluor-Phenyl | Hydroxymethyl | Ethyl | |
| 2.5.540 | 3,5-Dichlor-Phenyl | Hydroxymethyl | Ethyl | [CDCl₃] 1.31 (t, 3H); 2.05 (m, 1H, OH) 3.65 (q, 2H); 3.90 (m, 1H); 4.01 (m, 1H): 4.29 (q, 2H); 7.40 (s, 1H): 7.55 (s, 2H). |
| 2.5.541 | 3,5-Dichlor-Phenyl | 1-Hydroxyethyl | Methyl | [CDCl] D1 1.08 (t,3H); 1.36-1.51 (m,2H); 2.27 (s,1H); 3.53 (d,1H); 3.73 (d,1H); 3.84 (s,3H); 4.06 (m,1H); 7.42 (s,1H) 7.55 (s,2H). D2 1.08 (t,3H); 1.36-1.51 (m,2H); 1.96 (s,1H); 3.53 (d,1H); 3.73 (d,1H); 3.84 (s,3H); 3.95 (m,1H); 7.42 (s,1H) 7.55 (s,2H). |
| 2.5.542 | 3,5-Dichlor-Phenyl | 1-Hydroxypropyl | Ethyl | |
| 2.5.543 | 3,5-Dichlor-Phenyl | (1-Hydroxy-2-methylpropyl) | Ethyl | |
| 2.5.544 | 3,5-Dichlor-Phenyl | Methoxymethyl | Methyl | [CDCl₃] 3.43 (s, 3H); 3.50 (d, 1H); 3.72 (d, 1H); 3.80 (mc, 2H); 3.82 (s, 3H);7.41 (m, 1H); 7.55 (s, 2H). |
| 2.5.545 | 3,5-Dichlor-Phenyl | 2-Methoxyethyl | Methyl | [CDCl₃] 1.55 (s, 3H); 2.30 (m, 2H), 3.22 (s, 3H); 3.42 (d, 1 H); 3.55 (m,2H); 3.75 (d, 1H); 3.82 (s, 3H), 7.40 (s, 1H); 7.55 (s, 2H). |
| 2.5.546 | 3-Brom-5-chlor-Phenyl | 1-Hydroxyethyl | Methyl | [CDCl₃] D1 1.19 (d,3H); 2.35 (d br,1H); 3.53 (d,1H); 3,73 (d,1H); 3.83 (s,3H); 4.30-4.37 (m,1H); 7.56-7.61 (m,2H); 7.68-7.72 (m,1H). D2 1.28 (d,3H); 2.10 (d br,1H); 3.58 (d,1H); 3,68 (d,1H); 3.83 (s,3H); |
| 2.5.547 | 3-Iod-5-chlor-Phenyl | Hydroxymethyl | Ethyl | |
| 2.5.548 | 3-Methyl-5-chlor-Phenyl | Hydroxymethyl | Ethyl | |
| 2.5.549 | 3-Methyl-5-chlor-Phenyl | 1-Hydroxyethyl | Methyl | [CDCl3] D1 1.20 (d,3H); 2.33 (d,1H); 2.36 (s,3H); 3.54 (d,1H); 3.69 (d,1H); 3.83 (s,3H); 4.33 (m,1H); 7.23 (s,1H); 7.38 (s,1H); 7.46 (s, 1H). D2 1.29 (d,3H); 2.14 (d,1H); 2.36 (s,3H); 3.59 (d,1H); 3.74 (d,1H); 3.83 (s,3H); 4.23 (m,1H); 7.23 (s,1H); 7.37 (s,1H); 7.44 (s,1H). |
| 2.5.550 | 3-Ethyl-5-chlor-Phenyl | 1-Hydroxyethyl | Methyl | Ein Diastereomeres ohne Zuordnung [CDCl₃] 1.24 (t,3H); 1.28 (d,3H); 2.14 (d,1H); 2.65 (q,2H); 3.64 (AB,2H); 4.20-4.28 (m,1H); 7.24 (s,1H); 7.40(s,1H); 7.45 (1H). |
| 2.5.551 | 3-Ethyl-5-chlor-Phenyl | 1-Hydroxyethyl | Methyl | Das andere Diastereomere ohne Zuordnung [CDCl₃] 1.20 (d,3H); 1.25 (t,3H); 2.33 (d,1H); 2.65 (q,2H); 3.55 (d,1H); 3.74 (d,1H); 3.83 (s,3H); 4.30-4.36 (m,1H); 7.24 (s,1H); 7.41 (s,1H); 7.47 (s,1H) |
| 2.5.552 | 3-Ethyl-5-chlor-Phenyl | 1-Hydroxyethyl | Ethyl | [CDCl₃] D1 1.20 (d,3H); 1.25 (t,3H); 2.33 (d,1H); 2.65 (q,2H); 3.55 (d,1H); 3.74 (d,1H); 3.83 (s,3H); 4.30-4.36 (m,1H); 7.24 (s,1H); 7.41 (s,1H); 7.47 (s,1H). D2 1.24 (t,3H); 1.28 (d,3H); 2.14 (d,1H); 2.65 (q,2H); 3.64 (AB,2H); 4.20-4.28 (m,1H); 7.24 (s,1H); 7.40 s,1H); 7.45 (1H). |
| 2.5.553 | 3-nButyl-5-chlor-Phenyl | Hydroxymethyl | Ethyl | |
| 2.5.554 | 3-isoButyl-5-chlor-Phenyl | Hydroxymethyl | Ethyl | |
| 2.5.555 | 3-tert.Butyl-5-chlor-Phenyl | Hydroxymethyl | Ethyl | |
| 2.5.556 | 3-Cyclopropyl-5-chlor-Phenyl | Hydroxymethyl | Ethyl | |
| 2.5.557 | 3-Vinyl-5-chlor-Phenyl | Hydroxymethyl | Ethyl | |
| 2.5.558 | 3-Ethinyl-5-chlor-Phenyl | Hydroxymethyl | Ethyl | |
| 2.5.559 | 3-Cyano-5-chlor-Phenyl | Hydroxymethyl | Ethyl | |
| 2.5.560 | 3-Trifluormethyl-5-chlor-Phenyl | Hydroxymethyl | Ethyl | |
| 2.5.561 | 3-(Hydroxycarbonyl)-5-chlor-Phenyl | Hydroxymethyl | Ethyl | |
| 2.5.562 | 3-(Methoxycarbony)l-5-chlor-Phenyl | Hydroxymethyl | Ethyl | |
| 2.5.563 | 3-Hydroxymethyl-5-chlor-Phenyl | Hydroxymethyl | Ethyl | |
| 2.5.564 | 3-Carbamoyl-5-chlor-Phenyl | Hydroxymethyl | Ethyl | |
| 2.5.565 | 3-Hydroxy-5-chlor-Phenyl- | Hydroxymethyl | Ethyl | |
| 2.5.566 | 3-Methoxy-5-chlor-Phenyl | Hydroxymethyl | Ethyl | |
| 2.5.567 | 3-Ethoxy-5-chlor-Phenyl | Hydroxymethyl | Ethyl | |
| 2.5.568 | 3-nPropyoxy-5-chlor-Phenyl | Hydroxymethyl | Ethyl | |
| 2.5.569 | 3-isoPropoxy-5-chlor-Phenyl | Hydroxymethyl | Ethyl | |
| 2.5.570 | 3-nButoxy-5-chlor-Phenyl | Hydroxymethyl | Ethyl | |
| 2.5.571 | 3-isoButoxy-5-chlor-Phenyl | Hydroxymethyl | Ethyl | |
| 2.5.572 | 3-tert.Butoxy-5-chlor-Phenyl | Hydroxymethyl | Ethyl | |
| 2.5.573 | 3-Difluormethoxy-5-chlor-Phenyl | Hydroxymethyl | Ethyl | |
| 2.5.574 | 3-Trifluormethoxy-5-chlor-Phenyl | Hydroxymethyl | Ethyl | |
| 2.5.575 | 3-(2,2,2-Trifluorethoxy)-5-chlor-Phenyl | Hydroxymethyl | Ethyl | |
| 2.5.576 | 3-(2-Chlorethoxy)-5-chlor-Phenyl | Hydroxymethyl | Ethyl | |
| 2.5.577 | 3-(2-Hydroxyethoxy)-5-chlor-Phenyl- | Hydroxymethyl | Ethyl | |
| 2.5.578 | 3-[(tert-Butoxycarbonyl)oxy]-5-chlor-Phenyl | Hydroxymethyl | Ethyl | |
| 2.5.579 | 3-Nitro-5-chlor-Phenyl | Hydroxymethyl | Ethyl | |
| 2.5.580 | 3-Acetoxy-5-chlor-Phenyl | Hydroxymethyl | Ethyl | |
| 2.5.581 | {3-[(tert-Butoxycarbonyl)amino]-5-chlor-Phenyl} | Hydroxymethyl | Ethyl | |
| 2.5.582 | 3-Methylsulfanyl-5-chlor-Phenyl | Hydroxymethyl | Ethyl | |
| 2.5.583 | 3,5-Dibrom-Phenyl | Hydroxymethyl | Ethyl | |
| 2.5.584 | 3,5-Dibrom-Phenyl | 1-Hydroxyethyl | Ethyl | |
| 2.5.585 | 3-lod-5-brom-Phenyl | Hydroxymethyl | Ethyl | |
| 2.5.586 | 3-Methyl-5-brom-Phenyl | Hydroxymethyl | Ethyl | |
| 2.5.587 | 3-Methyl-5-brom-Phenyl | 1-Hydroxyethyl | Methyl | [CDCl3] D1 1.20 (d,3H); 1.55 (s br,1H); 2.36 (s,3H); 3.53 (d,1H); 3.68 (d,1H); 3.83 (s,3H); 4.32 (m,1H); 7.38 (s,1H); 7.43 (s,1H); 7.62 (s,1H). D2 1.28 (d,3H); 2.14 (d,1H); 2.36 (s,3H); 3.58 (d,1H); 3.73 (d,1H); 3.83 (s,3H); 4.25 (m,1H); 7.39 (s,1H); 7.41 (s,1H); 7.60 (s,1H). |
| 2.5.588 | 3-Methyl-5-brom-Phenyl | 1-Hydroxypropyl | Ethyl | |
| 2.5.589 | 3-Methyl-5-brom-Phenyl | (1-Hydroxy-2-methylpropyl) | Ethyl | |
| 2.5.590 | 3-Methyl-5-brom-Phenyl | Methoxymethyl | Ethyl | |
| 2.5.591 | 3-Methyl-5-brom-Phenyl | 2-Methoxyethyl | Ethyl | |
| 2.5.592 | 3-Ethyl-5-brom-Phenyl | Hydroxymethyl | Ethyl | |
| 2.5.593 | 3-Propyl-5-brom-Phenyl | Hydroxymethyl | Ethyl | |
| 2.5.594 | 3-isoPropyl-5-brom-Phenyl | Hydroxymethyl | Ethyl | |
| 2.5.595 | 3-nButyl-5-brom-Phenyl | Hydroxymethyl | Ethyl | |
| 2.5.596 | 3-isoButyl-5-brom-Phenyl | Hydroxymethyl | Ethyl | |
| 2.5.597 | 3-tert.Butyl-5-brom-Phenyl | Hydroxymethyl | Ethyl | |
| 2.5.598 | 3-Cyclopropyl-5-brom-Phenyl | Hydroxymethyl | Ethyl | |
| 2.5.599 | 3-Vinyl-5-brom-Phenyl | Hydroxymethyl | Ethyl | |
| 2.5.600 | 3-Ethinyl-5-brom-Phenyl | Hydroxymethyl | Ethyl | |
| 2.5.601 | 3-Cyano-5-brom-Phenyl | Hydroxymethyl | Ethyl | |
| 2.5.602 | 3-Trifluormethyl-5-brom-Phenyl | Hydroxymethyl | Ethyl | |
| 2.5.603 | 3-(Hydroxycarbonyl)-5-brom-Phenyl | Hydroxymethyl | Ethyl | |
| 2.5.604 | 3-(Methoxycarbony)l-5-brom-Phenyl | Hydroxymethyl | Ethyl | |
| 2.5.605 | 3-Hydroxymethyl-5-brom-Phenyl | Hydroxymethyl | Ethyl | |
| 2.5.606 | 3-Carbamoyl-5-brom-Phenyl | Hydroxymethyl | Ethyl | |
| 2.5.607 | 3-Hydroxy-5-brom-Phenyl- | Hydroxymethyl | Ethyl | |
| 2.5.608 | 3-Methoxy-5-brom-Phenyl | Hydroxymethyl | Ethyl | |
| 2.5.609 | 3-Ethoxy-5-brom-Phenyl | Hydroxymethyl | Ethyl | |
| 2.5.610 | 3-nPropyoxy-5-brom-Phenyl | Hydroxymethyl | Ethyl | |
| 2.5.611 | 3-isoPropoxy-5-brom-Phenyl | Hydroxymethyl | Ethyl | |
| 2.5.612 | 3-nButoxy-5-brom-Phenyl | Hydroxymethyl | Ethyl | |
| 2.5.613 | 3-isoButoxy-5-brom-Phenyl | Hydroxymethyl | Ethyl | |
| 2.5.614 | 3-tert.Butoxy-5-brom-Phenyl | Hydroxymethyl | Ethyl | |
| 2.5.615 | 3-Difluormethoxy-5-brom-Phenyl | Hydroxymethyl | Ethyl | |
| 2.5.616 | 3-Trifluormethoxy-5-brom-Phenyl | Hydroxymethyl | Ethyl | |
| 2.5.617 | 3-(2,2,2-Trifluorethoxy)-5-brom-Phenyl | Hydroxymethyl | Ethyl | |
| 2.5.618 | 3-(2-Chlorethoxy)-5-brom-Phenyl | Hydroxymethyl | Ethyl | |
| 2.5.619 | 3-(2-Hydroxyethoxy)-5-brom-Phenyl- | Hydroxymethyl | Ethyl | |
| 2.5.620 | 3-[(tert-Butoxycarbonyl)oxy]-5-brom-Phenyl | Hydroxymethyl | Ethyl | |
| 2.5.621 | 3-Nitro-5-brom-Phenyl | Hydroxymethyl | Ethyl | |
| 2.5.622 | 3-Acetoxy-5-brom-Phenyl | Hydroxymethyl | Ethyl | |
| 2.5.623 | {3-[(tert-Butoxycarbonyl)amino]-5-brom-Phenyl} | Hydroxymethyl | Ethyl | |
| 2.5.624 | 3-Methylsulfanyl-5-brom-Phenyl | Hydroxymethyl | Ethyl | |
| 2.5.625 | 3,5-Diiod-Phenyl | Hydroxymethyl | Ethyl | |
| 2.5.626 | 3-Methyl-5-iod-Phenyl | Hydroxymethyl | Ethyl | |
| 2.5.627 | 3-Ethyl-5-iod-Phenyl | Hydroxymethyl | Ethyl | |
| 2.5.628 | 3-Propyl-5-iod-Phenyl | Hydroxymethyl | Ethyl | |
| 2.5.629 | 3-isoPropyl-5-iod-Phenyl | Hydroxymethyl | Ethyl | |
| 2.5.630 | 3-nButyl-5-iod-Phenyl | Hydroxymethyl | Ethyl | |
| 2.5.631 | 3-isoButyl-5-iod-Phenyl | Hydroxymethyl | Ethyl | |
| 2.5.632 | 3-tert.Butyl-5-iod-Phenyl | Hydroxymethyl | Ethyl | |
| 2.5.633 | 3-Cyclopropyl-5-iod-Phenyl | Hydroxymethyl | Ethyl | |
| 2.5.634 | 3-Vinyl-5-iod-Phenyl | Hydroxymethyl | Ethyl | |
| 2.5.635 | 3-Ethinyl-5-iod-Phenyl | Hydroxymethyl | Ethyl | |
| 2.5.636 | 3-Cyano-5-iod-Phenyl | Hydroxymethyl | Ethyl | |
| 2.5.637 | 3-Trifluormethyl-5-iod-Phenyl | Hydroxymethyl | Ethyl | |
| 2.5.638 | 3-(Hydroxycarbonyl)-5-iod-Phenyl | Hydroxymethyl | Ethyl | |
| 2.5.639 | 3-(Methoxycarbonyl)-5-iod-Phenyl | Hydroxymethyl | Ethyl | |
| 2.5.640 | 3-Hydroxymethyl-5-iod-Phenyl | Hydroxymethyl | Ethyl | |
| 2.5.641 | 3-Carbamoyl-5-iod-Phenyl | Hydroxymethyl | Ethyl | |
| 2.5.642 | 3-Hydroxy-5-iod-Phenyl- | Hydroxymethyl | Ethyl | |
| 2.5.643 | 3-Methoxy-5-iod-Phenyl | Hydroxymethyl | Ethyl | |
| 2.5.644 | 3-Ethoxy-5-iod-Phenyl | Hydroxymethyl | Ethyl | |
| 2.5.645 | 3-nPropyoxy-5-iod-Phenyl | Hydroxymethyl | Ethyl | |
| 2.5.646 | 3-isoPropoxy-5-iod-Phenyl | Hydroxymethyl | Ethyl | |
| 2.5.647 | 3-nButoxy-5-iod-Phenyl | Hydroxymethyl | Ethyl | |
| 2.5.648 | 3-isoButoxy-5-iod-Phenyl | Hydroxymethyl | Ethyl | |
| 2.5.649 | 3-tert.Butoxy-5-iod-Phenyl | Hydroxymethyl | Ethyl | |
| 2.5.650 | 3-Difluormethoxy-5-iod-Phenyl | Hydroxymethyl | Ethyl | |
| 2.5.651 | 3-Trifluormethoxy-5-iod-Phenyl | Hydroxymethyl | Ethyl | |
| 2.5.652 | 3-(2,2,2-Trifluorethoxy)-5-iod-Phenyl | Hydroxymethyl | Ethyl | |
| 2.5.653 | 3-(2-Chlorethoxy)-5-iod-Phenyl | Hydroxymethyl | Ethyl | |
| 2.5.654 | 3-(2-Hydroxyethoxy)-5-iod-Phenyl- | Hydroxymethyl | Ethyl | |
| 2.5.655 | 3-[(tert-Butoxycarbonyl)oxy]-5-iod-Phenyl | Hydroxymethyl | Ethyl | |
| 2.5.656 | 3-Nitro-5-iod-Phenyl | Hydroxymethyl | Ethyl | |
| 2.5.657 | 3-Acetoxy-5-iod-Phenyl | Hydroxymethyl | Ethyl | |
| 2.5.658 | {3-[(tert-Butoxycarbonyl)amino]-5-iod-Phenyl} | Hydroxymethyl | Ethyl | |
| 2.5.659 | 3-Methylsulfanyl-5-iod-Phenyl | Hydroxymethyl | Ethyl | |
| 2.5.660 | 3,5-Dimethyl-Phenyl | Hydroxymethyl | Ethyl | |
| 2.5.661 | 3,5-Dimethyl-Phenyl | 1-Hydroxyethyl | Methyl | [CDCl3] D1 1.29 (d,3H), 2.19 (d,1H); 2.35 (s,6H); 3.56 (d,1H); 3.75 (d,1H); 3.82 (s,3H); 4.31 (m,1H); 7.07 (s,1H); 7.29 (s,2H). D2 1.20 (d,3H); 1.40 (d,1H); 2.35 (s,6H); 3.62 (d,1H); 3.78 (d,1H); 3.83 (s,3H); 4.22 (m,1H); 7.07 (s,1H); 7.29 (s,2H). |
| 2.5.662 | 3-Ethyl-5-methyl-Phenyl | Hydroxymethyl | Ethyl | |
| 2.5.663 | 3-Propyl-5-methyl-Phenyl | Hydroxymethyl | Ethyl | |
| 2.5.664 | 3-isoPropyl-5-methyl-Phenyl | Hydroxymethyl | Ethyl | |
| 2.5.665 | 3-nButyl-5-methyl-Phenyl | Hydroxymethyl | Ethyl | |
| 2.5.666 | 3-isoButyl-5-methyl-Phenyl | Hydroxymethyl | Ethyl | |
| 2.5.667 | 3-tert.Butyl-5-methyl-Phenyl | Hydroxymethyl | Ethyl | |
| 2.5.668 | 3-Cyclopropyl-5-methyl-Phenyl | Hydroxymethyl | Ethyl | |
| 2.5.669 | 3-Cyano-5-methyl-Phenyl | Hydroxymethyl | Ethyl | |
| 2.5.670 | 3-Trifluormethyl-5-methyl-Phenyl | Hydroxymethyl | Ethyl | |
| 2.5.671 | 3-(Methoxycarbony)l-5-methyl-Phenyl | Hydroxymethyl | Ethyl | |
| 2.5.672 | 3-Methoxy-5-methyl-Phenyl | Hydroxymethyl | Ethyl | |
| 2.5.673 | 3-Ethoxy-5-methyl-Phenyl | Hydroxymethyl | Ethyl | |
| 2.5.674 | 3-nPropyoxy-5-methyl-Phenyl | Hydroxymethyl | Ethyl | |
| 2.5.675 | 3-isoButoxy-5-methyl-Phenyl | Hydroxymethyl | Ethyl | |
| 2.5.676 | 3-Difluormethoxy-5-methyl-Phenyl | Hydroxymethyl | Ethyl | |
| 2.5.677 | 3-Trifluormethoxy-5-methyl-Phenyl | Hydroxymethyl | Ethyl | |
| 2.5.678 | 3-Nitro-5-methyl-Phenyl | Hydroxymethyl | Ethyl | |
| 2.5.679 | 3-Acetoxy-5-methyl-Phenyl | Hydroxymethyl | Ethyl | |
| 2.5.680 | 3-Methylsulfanyl-5-methyl-Phenyl | Hydroxymethyl | Ethyl | |
| 2.5.681 | 3,5-Diethyl-Phenyl | Hydroxymethyl | Ethyl | |
| 2.5.682 | 3-Propyl-5-ethyl-Phenyl | Hydroxymethyl | Ethyl | |
| 2.5.683 | 3-isoPropyl-5-ethyl-Phenyl | Hydroxymethyl | Ethyl | |
| 2.5.684 | 3-nButyl-5-ethyl-Phenyl | Hydroxymethyl | Ethyl | |
| 2.5.685 | 3-isoButyl-5-ethyl-Phenyl | Hydroxymethyl | Ethyl | |
| 2.5.686 | 3-tert.Butyl-5-ethyl-Phenyl | Hydroxymethyl | Ethyl | |
| 2.5.687 | 3-Cyclopropyl-5-ethyl-Phenyl | Hydroxymethyl | Ethyl | |
| 2.5.688 | 3-Cyano-5-ethyl-Phenyl | Hydroxymethyl | Ethyl | |
| 2.5.689 | 3-Trifluormethyl-5-ethyl-Phenyl | Hydroxymethyl | Ethyl | |
| 2.5.690 | 3-(Methoxycarbony)l-5-ethyl-Phenyl | Hydroxymethyl | Ethyl | |
| 2.5.691 | 3-Methoxy-5-ethyl-Phenyl | Hydroxymethyl | Ethyl | |
| 2.5.692 | 3-Ethoxy-5-ethyl-Phenyl | Hydroxymethyl | Ethyl | |
| 2.5.693 | 3-nPropyoxy-5-ethyl-Phenyl | Hydroxymethyl | Ethyl | |
| 2.5.694 | 3-isoButoxy-5-ethyl-Phenyl | Hydroxymethyl | Ethyl | |
| 2.5.695 | 3-Difluormethoxy-5-ethyl-Phenyl | Hydroxymethyl | Ethyl | |
| 2.5.696 | 3-Trifluormethoxy-5-ethyl-Phenyl | Hydroxymethyl | Ethyl | |
| 2.5.697 | 3-Nitro-5-ethyl-Phenyl | Hydroxymethyl | Ethyl | |
| 2.5.698 | 3-Acetoxy-5-ethyl-Phenyl | Hydroxymethyl | Ethyl | |
| 2.5.699 | 3-Methylsulfanyl-5-ethyl-Phenyl | Hydroxymethyl | Ethyl | |
| 2.5.700 | 3,5-Dipropyl-Phenyl | Hydroxymethyl | Ethyl | |
| 2.5.701 | 3-isoPropyl-5-propyl-Phenyl | Hydroxymethyl | Ethyl | |
| 2.5.702 | 3-nButyl-5-propyl-Phenyl | Hydroxymethyl | Ethyl | |
| 2.5.703 | 3-isoButyl-5-propyl-Phenyl | Hydroxymethyl | Ethyl | |
| 2.5.704 | 3-tert.Butyl-5-propyl-Phenyl | Hydroxymethyl | Ethyl | |
| 2.5.705 | 3-Cyclopropyl-5-propyl-Phenyl | Hydroxymethyl | Ethyl | |
| 2.5.706 | 3-Cyano-5-propyl-Phenyl | Hydroxymethyl | Ethyl | |
| 2.5.707 | 3-Trifluormethyl-5-propyl-Phenyl | Hydroxymethyl | Ethyl | |
| 2.5.708 | 3-(Methoxycarbonyl)-5-propyl-Phenyl | Hydroxymethyl | Ethyl | |
| 2.5.709 | 3-Methoxy-5-propyl-Phenyl | Hydroxymethyl | Ethyl | |
| 2.5.710 | 3-Ethoxy-5-propyl-Phenyl | Hydroxymethyl | Ethyl | |
| 2.5.711 | 3-nPropyoxy-5-propyl-Phenyl | Hydroxymethyl | Ethyl | |
| 2.5.712 | 3-isoButoxy-5-propyl-Phenyl | Hydroxymethyl | Ethyl | |
| 2.5.713 | 3-Difluormethoxy-5-propyl-Phenyl | Hydroxymethyl | Ethyl | |
| 2.5.714 | 3-Trifluormethoxy-5-ethyl-Phenyl | Hydroxymethyl | Ethyl | |
| 2.5.715 | 3-Nitro-5-propyl-Phenyl | Hydroxymethyl | Ethyl | |
| 2.5.716 | 3-Acetoxy-5-propyl-Phenyl | Hydroxymethyl | Ethyl | |
| 2.5.717 | 3-Methylsulfanyl-5-propyl-Phenyl | Hydroxymethyl | Ethyl | |
| 2.5.718 | 3,5-Diisopropyl-Phenyl | Hydroxymethyl | Ethyl | |
| 2.5.719 | 3-nButyl-5-isopropyl-Phenyl | Hydroxymethyl | Ethyl | |
| 2.5.720 | 3-isoButyl-5-isopropyl-Phenyl | Hydroxymethyl | Ethyl | |
| 2.5.721 | 3-tert.Butyl-5-isopropyl-Phenyl | Hydroxymethyl | Ethyl | |
| 2.5.722 | 3-Cyclopropyl-5-isopropyl-Phenyl | Hydroxymethyl | Ethyl | |
| 2.5.723 | 3-Cyano-5-isopropyl-Phenyl | Hydroxymethyl | Ethyl | |
| 2.5.724 | 3-Trifluormethyl-5-isopropyl-Phenyl | Hydroxymethyl | Ethyl | |
| 2.5.725 | 3-(Methoxycarbony)l-5-isopropyl-Phenyl | Hydroxymethyl | Ethyl | |
| 2.5.726 | 3-Methoxy-5-isopropyl-Phenyl | Hydroxymethyl | Ethyl | |
| 2.5.727 | 3-Ethoxy-5-isopropyl-Phenyl | Hydroxymethyl | Ethyl | |
| 2.5.728 | 3-nPropyoxy-5-isopropyl-Phenyl | Hydroxymethyl | Ethyl | |
| 2.5.729 | 3-isoButoxy-5-isopropyl-Phenyl | Hydroxymethyl | Ethyl | |
| 2.5.730 | 3-Difluormethoxy-5-isopropyl-Phenyl | Hydroxymethyl | Ethyl | |
| 2.5.731 | 3-Trifluormethoxy-5-isopropyl-Phenyl | Hydroxymethyl | Ethyl | |
| 2.5.732 | 3-Nitro-5-isopropyl-Phenyl | Hydroxymethyl | Ethyl | |
| 2.5.733 | 3-Acetoxy-5-isopropyl-Phenyl | Hydroxymethyl | Ethyl | |
| 2.5.734 | 3-Methylsulfanyl-5-isopropyl-Phenyl | Hydroxymethyl | Ethyl | |
| 2.5.735 | 3,5-Dibutyl-Phenyl | Hydroxymethyl | Ethyl | |
| 2.5.736 | 3-isoButyl-5-butyl-Phenyl | Hydroxymethyl | Ethyl | |
| 2.5.737 | 3-tert.Butyl-5-butyl-Phenyl | Hydroxymethyl | Ethyl | |
| 2.5.738 | 3-Cyclopropyl-5-butyl-Phenyl | Hydroxymethyl | Ethyl | |
| 2.5.739 | 3-Cyano-5-butyl-Phenyl | Hydroxymethyl | Ethyl | |
| 2.5.740 | 3-Trifluormethyl-5-butyl-Phenyl | Hydroxymethyl | Ethyl | |
| 2.5.741 | 3-(Methoxycarbony)l-5-butyl-Phenyl | Hydroxymethyl | Ethyl | |
| 2.5.742 | 3-Methoxy-5-butyl-Phenyl | Hydroxymethyl | Ethyl | |
| 2.5.743 | 3-Ethoxy-5-butyl-Phenyl | Hydroxymethyl | Ethyl | |
| 2.5.744 | 3-nPropyoxy-5-butyl-Phenyl | Hydroxymethyl | Ethyl | |
| 2.5.745 | 3-isoButoxy-5-butyl-Phenyl | Hydroxymethyl | Ethyl | |
| 2.5.746 | 3-Difluormethoxy-5-butyl-Phenyl | Hydroxymethyl | Ethyl | |
| 2.5.747 | 3-Trifluormethoxy-5-butyl-Phenyl | Hydroxymethyl | Ethyl | |
| 2.5.748 | 3-Nitro-5-butyl-Phenyl | Hydroxymethyl | Ethyl | |
| 2.5.749 | 3-Acetoxy-5-butyl-Phenyl | Hydroxymethyl | Ethyl | |
| 2.5.750 | 3-Methylsulfanyl-5-butyl-Phenyl | Hydroxymethyl | Ethyl | |
| 2.5.751 | 3,5-Diisobutyl-Phenyl | Hydroxymethyl | Ethyl | |
| 2.5.752 | 3-tert.Butyl-5-isobutyl-Phenyl | Hydroxymethyl | Ethyl | |
| 2.5.753 | 3-Cyclopropyl-5-isobutyl-Phenyl | Hydroxymethyl | Ethyl | |
| 2.5.754 | 3-Cyano-5-isobutyl-Phenyl | Hydroxymethyl | Ethyl | |
| 2.5.755 | 3-Trifluormethyl-5-isobutyl-Phenyl | Hydroxymethyl | Ethyl | |
| 2.5.756 | 3-(Methoxycartonyl)-5-isobutyl-Phenyl | Hydroxymethyl | Ethyl | |
| 2.5.757 | 3-Methoxy-5-isobutyl-Phenyl | Hydroxymethyl | Ethyl | |
| 2.5.758 | 3-Ethoxy-5-isobutyl-Phenyl | Hydroxymethyl | Ethyl | |
| 2.5.759 | 3-nPropyoxy-5-isobutyl-Phenyl | Hydroxymethyl | Ethyl | |
| 2.5.760 | 3-isoButoxy-5-isobutyl-Phenyl | Hydroxymethyl | Ethyl | |
| 2.5.761 | 3-Difluormethoxy-5-isobutyl-Phenyl | Hydroxymethyl | Ethyl | |
| 2.5.762 | 3-Trifluormethoxy-5-isobutyl-Phenyl | Hydroxymethyl | Ethyl | |
| 2.5.763 | 3-Nitro-5-isobutyl-Phenyl | Hydroxymethyl | Ethyl | |
| 2.5.764 | 3-Acetoxy-5-isobutyl-Phenyl | Hydroxymethyl | Ethyl | |
| 2.5.765 | 3-Methylsulfanyl-5-isobutyl-Phenyl | Hydroxymethyl | Ethyl | |
| 2.5.766 | 3,5-(Ditert.butyl)-Phenyl | Hydroxymethyl | Ethyl | |
| 2.5.767 | 3-Cyclopropyl-5-tert.butyl-Phenyl | Hydroxymethyl | Ethyl | |
| 2.5.768 | 3-Cyano-5-tert.butyl-Phenyl | Hydroxymethyl | Ethyl | |
| 2.5.769 | 3-Trifluormethyl-5-tert.butyl-Phenyl | Hydroxymethyl | Ethyl | |
| 2.5.770 | 3-(Methoxycarbonyl)-5-tert.butyl-Phenyl | Hydroxymethyl | Ethyl | |
| 2.5.771 | 3-Methoxy-5-tert.butyl-Phenyl | Hydroxymethyl | Ethyl | |
| 2.5.772 | 3-Ethoxy-5-tert.butyl-Phenyl | Hydroxymethyl | Ethyl | |
| 2.5.773 | 3-nPropyoxy-5-tert.butyl-Phenyl | Hydroxymethyl | Ethyl | |
| 2.5.774 | 3-isoButoxy-5-tert.butyl-Phenyl | Hydroxymethyl | Ethyl | |
| 2.5.775 | 3-Difluormethoxy-5-tert.butyl-Phenyl | Hydroxymethyl | Ethyl | |
| 2.5.776 | 3-Trifluormethoxy-5-tert.butyl-Phenyl | Hydroxymethyl | Ethyl | |
| 2.5.777 | 3-Nitro-5-tert.butyl-Phenyl | Hydroxymethyl | Ethyl | |
| 2.5.778 | 3-Acetoxy-5-tert.butyl-Phenyl | Hydroxymethyl | Ethyl | |
| 2.5.779 | 3-Methylsulfanyl-5-tert.butyl-Phenyl | Hydroxymethyl | Ethyl | |
| 2.5.780 | 3-tert.Butyl-5-cyclopropyl-Phenyl | Hydroxymethyl | Ethyl | |
| 2.5.781 | 3,5-Dicyclopropyl-Phenyl | Hydroxymethyl | Ethyl | |
| 2.5.782 | 3-Cyano-5-cyclopropyl-Phenyl | Hydroxymethyl | Ethyl | |
| 2.5.783 | 3-Trifluormethyl-5-cyclopropyl-Phenyl | Hydroxymethyl | Ethyl | |
| 2.5.784 | 3-(Methoxycarbonyl)-5-cyclopropyl-Phenyl | Hydroxymethyl | Ethyl | |
| 2.5.785 | 3-Methoxy-5-cyclopropyl-Phenyl | Hydroxymethyl | Ethyl | |
| 2.5.786 | 3-Ethoxy-5-cyclopropyl-Phenyl | Hydroxymethyl | Ethyl | |
| 2.5.787 | 3-nPropyoxy-5-cyclopropyl-Phenyl | Hydroxymethyl | Ethyl | |
| 2.5.788 | 3-isoButoxy-5-cyclopropyl-Phenyl | Hydroxymethyl | Ethyl | |
| 2.5.789 | 3-Difluormethoxy-5-cyclopropyl-Phenyl | Hydroxymethyl | Ethyl | |
| 2.5.790 | 3-Trifluormethoxy-5-cyclopropyl-Phenyl | Hydroxymethyl | Ethyl | |
| 2.5.791 | 3-Nitro-5-cyclopropyl-Phenyl | Hydroxymethyl | Ethyl | |
| 2.5.792 | 3-Acetoxy-5-cyclopropyl-Phenyl | Hydroxymethyl | Ethyl | |
| 2.5.793 | 3-Methylsulfanyl-5-cyclopropyl-Phenyl | Hydroxymethyl | Ethyl | |
| 2.5.794 | 3,5-Dicyano-Phenyl | Hydroxymethyl | Ethyl | |
| 2.5.795 | 3-Trifluormethyl-5-cyano-Phenyl | Hydroxymethyl | Ethyl | |
| 2.5.796 | 3-(Methoxycarbonyl)-5-cyano-Phenyl | Hydroxymethyl | Ethyl | |
| 2.5.797 | 3-Methoxy-5-cyano-Phenyl | Hydroxymethyl | Ethyl | |
| 2.5.798 | 3-Ethoxy-5-cyano-Phenyl | Hydroxymethyl | Ethyl | |
| 2.5.799 | 3-nPropyoxy-5-cyano-Phenyl | Hydroxymethyl | Ethyl | |
| 2.5.800 | 3-isoButoxy-5-cyano-Phenyl | Hydroxymethyl | Ethyl | |
| 2.5.801 | 3-Difluormethoxy-5-cyano-Phenyl | Hydroxymethyl | Ethyl | |
| 2.5.802 | 3-Trifluormethoxy-5-cyano-Phenyl | Hydroxymethyl | Ethyl | |
| 2.5.803 | 3-Nitro-5-cyano-Phenyl | Hydroxymethyl | Ethyl | |
| 2.5.804 | 3-Acetoxy-5-cyano-Phenyl | Hydroxymethyl | Ethyl | |
| 2.5.805 | 3-Methylsulfanyl-5-cyano-Phenyl | Hydroxymethyl | Ethyl | |
| 2.5.806 | 3,5-Di(trifluormethyl)-Phenyl | Hydroxymethyl | Ethyl | |
| 2.5.807 | 3-(Methoxycarbonyl)-5-trifluormethyl-Phenyl | Hydroxymethyl | Ethyl | |
| 2.5.808 | 3-Methoxy-5-trifluormethyl-Phenyl | Hydroxymethyl | Ethyl | |
| 2.5.809 | 3-Ethoxy-5-trifluormethyl-Phenyl | Hydroxymethyl | Ethyl | |
| 2.5.810 | 3-nPropyoxy-5-trifluormethyl-Phenyl | Hydroxymethyl | Ethyl | |
| 2.5.811 | 3-isoButoxy-5-trifluormethyl-Phenyl | Hydroxymethyl | Ethyl | |
| 2.5.812 | 3-Difluormethoxy-5-trifluormethyl-Phenyl | Hydroxymethyl | Ethyl | |
| 2.5.813 | 3-Trifluormethoxy-5-trifluormethyl-Phenyl | Hydroxymethyl | Ethyl | |
| 2.5.814 | 3-Nitro-5-trifluormethyl-Phenyl | Hydroxymethyl | Ethyl | |
| 2.5.815 | 3-Acetoxy-5-trifluormethyl-Phenyl | Hydroxymethyl | Ethyl | |
| 2.5.816 | 3-Methylsulfanyl-5-trifluormethyl-Phenyl | Hydroxymethyl | Ethyl | |
| 2.5.817 | 3,5-Di(methoxycarbonyl)-Phenyl | Hydroxymethyl | Ethyl | |
| 2.5.818 | 3-Methoxy-5-(methoxycarbonyl)-Phenyl | Hydroxymethyl | Ethyl | |
| 2.5.819 | 3-Ethoxy-5-(methoxycarbonyl)-Phenyl | Hydroxymethyl | Ethyl | |
| 2.5.820 | 3-nPropyoxy-5-(methoxycarbonyl)-Phenyl | Hydroxymethyl | Ethyl | |
| 2.5.821 | 3-isoButoxy-5-(methoxycarbonyl)-Phenyl | Hydroxymethyl | Ethyl | |
| 2.5.822 | 3-Difluormethoxy-5-(methoxycarbonyl)-Phenyl | Hydroxymethyl | Ethyl | |
| 2.5.823 | 3-Trifluormethoxy-5-(methoxycarbonyl)-Phenyl | Hydroxymethyl | Ethyl | |
| 2.5.824 | 3-Nitro-5-(methoxycarbonyl)-Phenyl | Hydroxymethyl | Ethyl | |
| 2.5.825 | 3-Acetoxy-5-(methoxycarbonyl)-Phenyl | Hydroxymethyl | Ethyl | |
| 2.5.826 | 3-Methylsulfanyl-5-(methoxycarbonyl)-Phenyl | Hydroxymethyl | Ethyl | |
| 2.5.827 | 3,5-Dimethoxy-Phenyl | Hydroxymethyl | Ethyl | |
| 2.5.828 | 3-Ethoxy-5-methoxy-Phenyl | Hydroxymethyl | Ethyl | |
| 2.5.829 | 3-nPropyoxy-5-methoxy-Phenyl | Hydroxymethyl | Ethyl | |
| 2.5.830 | 3-isoButoxy-5-methoxy-Phenyl | Hydroxymethyl | Ethyl | |
| 2.5.831 | 3-Difluormethoxy-5-methoxy-Phenyl | Hydroxymethyl | Ethyl | |
| 2.5.832 | 3-Trifluormethoxy-5-methoxy-Phenyl | Hydroxymethyl | Ethyl | |
| 2.5.833 | 3-Nitro-5-methoxy-Phenyl | Hydroxymethyl | Ethyl | |
| 2.5.834 | 3-Acetoxy-5-methoxy-Phenyl | Hydroxymethyl | Ethyl | |
| 2.5.835 | 3-Methylsulfanyl-5-methoxy-Phenyl | Hydroxymethyl | Ethyl | |
| 2.5.836 | 3,5-Diethoxy-Phenyl | Hydroxymethyl | Ethyl | |
| 2.5.837 | 3-nPropyoxy-5-ethoxy-Phenyl | Hydroxymethyl | Ethyl | |
| 2.5.838 | 3-isoButoxy-5-ethoxy-Phenyl | Hydroxymethyl | Ethyl | |
| 2.5.839 | 3-Difluormethoxy-5-ethoxy-Phenyl | Hydroxymethyl | Ethyl | |
| 2.5.840 | 3-Trifluormethoxy-5-ethoxy-Phenyl | Hydroxymethyl | Ethyl | |
| 2.5.841 | 3-Nitro-5-ethoxy-Phenyl | Hydroxymethyl | Ethyl | |
| 2.5.842 | 3-Acetoxy-5-ethoxy-Phenyl | Hydroxymethyl | Ethyl | |
| 2.5.843 | 3-Methylsulfanyl-5-ethoxy-Phenyl | Hydroxymethyl | Ethyl | |
| 2.5.844 | 3,5-Di(isopropyoxy)-Phenyl | Hydroxymethyl | Ethyl | |
| 2.5.845 | 3-isoButoxy-5-isopropoxy-Phenyl | Hydroxymethyl | Ethyl | |
| 2.5.846 | 3-Difluormethoxy-5-isopropoxy-Phenyl | Hydroxymethyl | Ethyl | |
| 2.5.847 | 3-Trifluormethoxy-5-isopropoxy-Phenyl | Hydroxymethyl | Ethyl | |
| 2.5.848 | 3-Nitro-5-isopropoxy-Phenyl | Hydroxymethyl | Ethyl | |
| 2.5.849 | 3-Acetoxy-5-isopropoxy-Phenyl | Hydroxymethyl | Ethyl | |
| 2.5.850 | 3-Methylsulfanyl-5-isopropoxy-Phenyl | Hydroxymethyl | Ethyl | |
| 2.5.851 | 3,5-Di(trifluormethoxy)-Phenyl | Hydroxymethyl | Ethyl | |
| 2.5.852 | 3-Nitro-5-trifluormethoxy-Phenyl | Hydroxymethyl | Ethyl | |
| 2.5.853 | 3-Methylsulfanyl-5-trifluormethoxy-Phenyl | Hydroxymethyl | Ethyl | |
| 2.5.854 | 3,5-Bis(difluormethoxy)-Phenyl | Hydroxymethyl | Ethyl | |
| 2.5.855 | 3,5-Bis(difluormethoxy)-Phenyl | 1-Hydroxyethyl | Ethyl | |
| 2.5.856 | 3,5-Bis(difluormethoxy)-Phenyl | 1-Hydroxypropyl | Ethyl | |
| 2.5.857 | 3,5-Bis(difluormethoxy)-Phenyl | (1-Hydroxy-2-methylpropyl) | Ethyl | |
| 2.5.858 | 3-Trifluormethoxy-5-difluormethoxy-Phenyl | Hydroxymethyl | Ethyl | |
| 2.5.859 | 3-Nitro-5-difluormethoxy-Phenyl | Hydroxymethyl | Ethyl | |
| 2.5.860 | 3-Acetoxy-5-difluormethoxy-Phenyl | Hydroxymethyl | Ethyl | |
| 2.5.861 | 3-Methylsulfanyl-5-difluormethoxy-Phenyl | Hydroxymethyl | Ethyl | |
| 2.5.862 | 3,5-Bis(acetoxy)-Phenyl | Hydroxymethyl | Ethyl | |
| 2.5.863 | 3-Methylsulfanyl-5-acetoxy-Phenyl | Hydroxymethyl | Ethyl | |
| 2.5.864 | 3-Acetoxy-5-nitro-Phenyl | Hydroxymethyl | Ethyl | |
| 2.5.865 | 3-Methylsulfanyl-5-nitro-Phenyl | Hydroxymethyl | Ethyl | |
| 2.5.866 | 3,4-Difluor-Phenyl | Hydroxymethyl | Ethyl | |
| 2.5.867 | 3,4-Difluor-Phenyl | 1-Hydroxyethyl | Ethyl | |
| 2.5.868 | 3,4-Difluor-Phenyl | 1-Hydroxypropyl | Ethyl | |
| 2.5.869 | 3,4-Difluor-Phenyl | (1-Hydroxy-2-methylpropyl) | Ethyl | |
| 2.5.870 | 3,4-Difluor-Phenyl | Methoxymethyl | Ethyl | |
| 2.5.871 | 3,4-Difluor-Phenyl | 2-Methoxyethyl | Ethyl | |
| 2.5.872 | 3-Chlor-4-fluor-Phenyl | Hydroxymethyl | Ethyl | |
| 2.5.873 | 3-Chlor-4-fluor-Phenyl | 1-Hydroxyethyl | Ethyl | |
| 2.5.874 | 3-Chlor-4-fluor-Phenyl | 1-Hydroxypropyl | Ethyl | |
| 2.5.875 | 3-Chlor-4-fluor-Phenyl | (1-Hydroxy-2-methylpropyl) | Ethyl | |
| 2.5.876 | 3-Chlor-4-fluor-Phenyl | Methoxymethyl | Ethyl | |
| 2.5.877 | 3-Chlor-4-fluor-Phenyl | 2-Methoxyethyl | Ethyl | |
| 2.5.878 | 3-Brom-4-fluor-Phenyl | Hydroxymethyl | Ethyl | |
| 2.5.879 | 3-Methyl-4-fluor-Phenyl | Hydroxymethyl | Ethyl | |
| 2.5.880 | 3-Methyl-4-fluor-Phenyl | 1-Hydroxyethyl | Ethyl | |
| 2.5.881 | 3-Cyclopropyl-4-fluor-Phenyl | Hydroxymethyl | Ethyl | |
| 2.5.882 | 3-Cyano-4-fluor-Phenyl | Hydroxymethyl | Ethyl | |
| 2.5.883 | 3-Methoxy-4-fluor-Phenyl | Hydroxymethyl | Ethyl | |
| 2.5.884 | 3-Ethoxy-4-fluor-Phenyl | Hydroxymethyl | Ethyl | |
| 2.5.885 | 3-Trifluormethoxy-4-fluor-Phenyl | Hydroxymethyl | Ethyl | |
| 2.5.886 | 3-Nitro-4-fluor-Phenyl | Hydroxymethyl | Ethyl | |
| 2.5.887 | 3-Fluor-4-chlor-Phenyl | Hydroxymethyl | Ethyl | |
| 2.5.888 | 3,4-Dichlor-Phenyl | Hydroxymethyl | Ethyl | |
| 2.5.889 | 3-Brom-4-chlor-Phenyl | Hydroxymethyl | Ethyl | |
| 2.5.890 | 3-Methyl-4-chlor-Phenyl | Hydroxymethyl | Ethyl | |
| 2.5.891 | 3-Cyclopropyl-4-chlor-Phenyl | Hydroxymethyl | Ethyl | |
| 2.5.892 | 3-Cyano-4-chlor-Phenyl | Hydroxymethyl | Ethyl | |
| 2.5.893 | 3-Trifluormethyl-4-chlor-Phenyl | Hydroxymethyl | Ethyl | |
| 2.5.894 | 3-Methoxy-4-chlor-Phenyl | Hydroxymethyl | Ethyl | |
| 2.5.895 | 3-Ethoxy-4-chlor-Phenyl | Hydroxymethyl | Ethyl | |
| 2.5.896 | 3-Trifluormethoxy-4-chlor-Phenyl | Hydroxymethyl | Ethyl | |
| 2.5.897 | 3-Nitro-4-chlor-Phenyl | Hydroxymethyl | Ethyl | |
| 2.5.898 | 3-Fluor-4-brom-Phenyl | Hydroxymethyl | Ethyl | |
| 2.5.899 | 3-Chlor-4-brom-Phenyl | Hydroxymethyl | Ethyl | |
| 2.5.900 | 3,4-Dibrom-Phenyl | Hydroxymethyl | Ethyl | |
| 2.5.901 | 3-Methyl-4-brom-Phenyl | Hydroxymethyl | Ethyl | |
| 2.5.902 | 3-Cyclopropyl-4-brom-Phenyl | Hydroxymethyl | Ethyl | |
| 2.5.903 | 3-Cyano-4-brom-Phenyl | Hydroxymethyl | Ethyl | |
| 2.5.904 | 3-Trifluormethyl-4-brom-Phenyl | Hydroxymethyl | Ethyl | |
| 2.5.905 | 3-Methoxy-4-Phenyl | Hydroxymethyl | Ethyl | |
| 2.5.906 | 3-Ethoxy-4-brom-Phenyl | Hydroxymethyl | Ethyl | |
| 2.5.907 | 3-Trifluormethoxy-4-brom-Phenyl | Hydroxymethyl | Ethyl | |
| 2.5.908 | 3-Nitro-4-brom-Phenyl | Hydroxymethyl | Ethyl | |
| 2.5.909 | 3-Fluor-4-iod-Phenyl | Hydroxymethyl | Ethyl | |
| 2.5.910 | 3-Chlor-4-iod-Phenyl | Hydroxymethyl | Ethyl | |
| 2.5.911 | 3-Brom-4-iod-Phenyl | Hydroxymethyl | Ethyl | |
| 2.5.912 | 3-Methyl-4-iod-Phenyl | Hydroxymethyl | Ethyl | |
| 2.5.913 | 3-Cyclopropyl-4-iod-Phenyl | Hydroxymethyl | Ethyl | |
| 2.5.914 | 3-Cyano-4-iod-Phenyl | Hydroxymethyl | Ethyl | |
| 2.5.915 | 3-Trifluormethyl-4-iod-Phenyl | Hydroxymethyl | Ethyl | |
| 2.5.916 | 3-Methoxy-4-iod-Phenyl | Hydroxymethyl | Ethyl | |
| 2.5.917 | 3-Ethoxy-4-iod-Phenyl | Hydroxymethyl | Ethyl | |
| 2.5.918 | 3-Trifluormethoxy-4-iod-Phenyl | Hydroxymethyl | Ethyl | |
| 2.5.919 | 3-Nitro-4-iod-Phenyl | Hydroxymethyl | Ethyl | |
| 2.5.920 | 3-Fluor-4-methyl-Phenyl | Hydroxymethyl | Ethyl | |
| 2.5.921 | 3-Chlor-4-methyl-Phenyl | Hydroxymethyl | Ethyl | |
| 2.5.922 | 3-Brom-4-methyl-Phenyl | Hydroxymethyl | Ethyl | |
| 2.5.923 | 3.4-Dimethyl-Phenyl | Hydroxymethyl | Ethyl | |
| 2.5.924 | 3.4-Dimethyl-Phenyl | 1-Hydroxyethyl | Ethyl | |
| 2.5.925 | 3.4-Dimethyl-Phenyl | 1-Hydroxypropyl | Ethyl | |
| 2.5.926 | 3.4-Dimethyl-Phenyl | (1-Hydroxy-2-methylpropyl) | Ethyl | |
| 2.5.927 | 3.4-Dimethyl-Phenyl | Methoxymethyl | Ethyl | |
| 2.5.928 | 3.4-Dimethyl-Phenyl | 2-Methoxyethyl | Ethyl | |
| 2.5.929 | 3-Cyclopropyl-4-methyl-Phenyl | Hydroxymethyl | Ethyl | |
| 2.5.930 | 3-Cyano-4-methyl-Phenyl | Hydroxymethyl | Ethyl | |
| 2.5.931 | 3-Trifluormethyl-4-methyl-Phenyl | Hydroxymethyl | Ethyl | |
| 2.5.932 | 3-Methoxy-4-methyl-Phenyl | Hydroxymethyl | Ethyl | |
| 2.5.933 | 3-Ethoxy-4-methyl-Phenyl | Hydroxymethyl | Ethyl | |
| 2.5.934 | 3-Trifluormethoxy-4-methyl-Phenyl | Hydroxymethyl | Ethyl | |
| 2.5.935 | 3-Nitro-4-methyl-Phenyl | Hydroxymethyl | Ethyl | |
| 2.5.936 | 3-Fluor-4-ethyl-Phenyl | Hydroxymethyl | Ethyl | |
| 2.5.937 | 3-Chlor-4-ethyl-Phenyl | Hydroxymethyl | Ethyl | |
| 2.5.938 | 3-Brom-4-ethyl-Phenyl | Hydroxymethyl | Ethyl | |
| 2.5.939 | 3-Methyl-4-ethyl-Phenyl | Hydroxymethyl | Ethyl | |
| 2.5.940 | 3,4-Diethyl-Phenyl | Hydroxymethyl | Ethyl | |
| 2.5.941 | 3-Cyclopropyl-4-ethyl-Phenyl | Hydroxymethyl | Ethyl | |
| 2.5.942 | 3-Cyano-4-ethyl-Phenyl | Hydroxymethyl | Ethyl | |
| 2.5.943 | 3-Trifluormethyl-4-ethyl-Phenyl | Hydroxymethyl | Ethyl | |
| 2.5.944 | 3-Methoxy-4-ethyl-Phenyl | Hydroxymethyl | Ethyl | |
| 2.5.945 | 3-Ethoxy-4-ethyl-Phenyl | Hydroxymethyl | Ethyl | |
| 2.5.946 | 3-Trifluormethoxy-4-ethyl-Phenyl | Hydroxymethyl | Ethyl | |
| 2.5.947 | 3-Nitro-4-ethyl-Phenyl | Hydroxymethyl | Ethyl | |
| 2.5.948 | 3-Fluor-4-propyl-Phenyl | Hydroxymethyl | Ethyl | |
| 2.5.949 | 3-Chlor-4-propyl-Phenyl | Hydroxymethyl | Ethyl | |
| 2.5.950 | 3-Brom-4-propyl-Phenyl | Hydroxymethyl | Ethyl | |
| 2.5.951 | 3-Methyl-4-propyl-Phenyl | Hydroxymethyl | Ethyl | |
| 2.5.952 | 3-Cyclopropyl-4-propyl-Phenyl | Hydroxymethyl | Ethyl | |
| 2.5.953 | 3-Cyano-4-propyl-Phenyl | Hydroxymethyl | Ethyl | |
| 2.5.954 | 3-Trifluormethyl-4-propyl-Phenyl | Hydroxymethyl | Ethyl | |
| 2.5.955 | 3-Methoxy-4-propyl-Phenyl | Hydroxymethyl | Ethyl | |
| 2.5.956 | 3-Ethoxy-4-propyl-Phenyl | Hydroxymethyl | Ethyl | |
| 2.5.957 | 3-Trifluormethoxy-4-propyl-Phenyl | Hydroxymethyl | Ethyl | |
| 2.5.958 | 3-Nitro-4-propyl-Phenyl | Hydroxymethyl | Ethyl | |
| 2.5.959 | 3-Fluor-4-isopropyl-Phenyl | Hydroxymethyl | Ethyl | |
| 2.5.960 | 3-Chlor-4-isopropyl-Phenyl | Hydroxymethyl | Ethyl | |
| 2.5.961 | 3-Brom-4-isopropyl-Phenyl | Hydroxymethyl | Ethyl | |
| 2.5.962 | 3-Methyl-4-isopropyl-Phenyl | Hydroxymethyl | Ethyl | |
| 2.5.963 | 3-Cyclopropyl-4-isopropyl-Phenyl | Hydroxymethyl | Ethyl | |
| 2.5.964 | 3-Cyano-4-isopropyl-Phenyl | Hydroxymethyl | Ethyl | |
| 2.5.965 | 3-Trifluormethyl-4-isopropyl-Phenyl | Hydroxymethyl | Ethyl | |
| 2.5.966 | 3-Methoxy-4-isopropyl-Phenyl | Hydroxymethyl | Ethyl | |
| 2.5.967 | 3-Ethoxy-4-isopropyl-Phenyl | Hydroxymethyl | Ethyl | |
| 2.5.968 | 3-Trifluormethoxy-4-isopropyl-Phenyl | Hydroxymethyl | Ethyl | |
| 2.5.969 | 3-Nitro-4-isopropyl-Phenyl | Hydroxymethyl | Ethyl | |
| 2.5.970 | 3-Fluor-4-tert.butyl-Phenyl | Hydroxymethyl | Ethyl | |
| 2.5.971 | 3-Chlor-4-tert.buty-Phenyl | Hydroxymethyl | Ethyl | |
| 2.5.972 | 3-Brom-4-tert.butyl-Phenyl | Hydroxymethyl | Ethyl | |
| 2.5.973 | 3-Methyl-4-tert.butyl-Phenyl | Hydroxymethyl | Ethyl | |
| 2.5.974 | 3-Cyclopropyl-4-tert.butyl-Phenyl | Hydroxymethyl | Ethyl | |
| 2.5.975 | 3-Cyano-4-tert.butyl-Phenyl | Hydroxymethyl | Ethyl | |
| 2.5.976 | 3-Trifluormethyl-4-tert.butyl-Phenyl | Hydroxymethyl | Ethyl | |
| 2.5.977 | 3-Trifluormethyl-4-tert.butyl-Phenyl | 1-Hydroxyethyl | Ethyl | |
| 2.5.978 | 3-Trifluormethyl-4-tert.butyl-Phenyl | 1-Hydroxypropyl | Ethyl | |
| 2.5.979 | 3-Trifluormethyl-4-tert.butyl-Phenyl | (1-Hydroxy-2-methylpropyl) | Ethyl | |
| 2.5.980 | 3-Trifluormethyl-4-tert.butyl-Phenyl | Methoxymethyl | Ethyl | |
| 2.5.981 | 3-Trifluormethyl-4-tert.butyl-Phenyl | 2-Methoxyethyl | Ethyl | |
| 2.5.982 | 3-Methoxy-4-tert.butyl-Phenyl | Hydroxymethyl | Ethyl | |
| 2.5.983 | 3-Ethoxy-4-tert.butyl-Phenyl | Hydroxymethyl | Ethyl | |
| 2.5.984 | 3-Trifluormethoxy-4-tert.butyl-Phenyl | Hydroxymethyl | Ethyl | |
| 2.5.985 | 3-Nitro-4-tert.butyl-Phenyl | Hydroxymethyl | Ethyl | |
| 2.5.986 | 3-Fluor-4-cyclopropyl-Phenyl | Hydroxymethyl | Ethyl | |
| 2.5.987 | 3-Chlor-4-cyclopropyl-Phenyl | Hydroxymethyl | Ethyl | |
| 2.5.988 | 3-Brom-4-cyclopropyl-Phenyl | Hydroxymethyl | Ethyl | |
| 2.5.989 | 3-Methyl-4-cyclopropyl-Phenyl | Hydroxymethyl | Ethyl | |
| 2.5.990 | 3-Cyano-4-cyclopropyl-Phenyl | Hydroxymethyl | Ethyl | |
| 2.5.991 | 3-Trifluormethyl-4-cyclopropyl-Phenyl | Hydroxymethyl | Ethyl | |
| 2.5.992 | 3-Methoxy-4-cyclopropyl-Phenyl | Hydroxymethyl | Ethyl | |
| 2.5.993 | 3-Ethoxy-4-cyclopropyl-Phenyl | Hydroxymethyl | Ethyl | |
| 2.5.994 | 3-Trifluormethoxy-4-cyclopropyl-Phenyl | Hydroxymethyl | Ethyl | |
| 2.5.995 | 3-Fluor-4-methoxycarbonyl-Phenyl | Hydroxymethyl | Ethyl | |
| 2.5.996 | 3-Chlor-4-methoxycarbonyl-Phenyl | Hydroxymethyl | Ethyl | |
| 2.5.997 | 3-Brom-4-methoxycarbonyl-Phenyl | Hydroxymethyl | Ethyl | |
| 2.5.998 | 3-Methyl-4-methoxycarbonyl-Phenyl | Hydroxymethyl | Ethyl | |
| 2.5.999 | 3-Cyclopropyl-4-methoxycarbonyl-Phenyl | Hydroxymethyl | Ethyl | |
| 2.5.1000 | 3-Cyano-4-methoxycarbonyl-Phenyl | Hydroxymethyl | Ethyl | |
| 2.5.1001 | 3-Trifluormethyl-4-methoxycarbonyl-Phenyl | Hydroxymethyl | Ethyl | |
| 2.5.1002 | 3-Methoxy-4-methoxycarbonyl-Phenyl | Hydroxymethyl | Ethyl | |
| 2.5.1003 | 3-Ethoxy-4-methoxycarbonyl-Phenyl | Hydroxymethyl | Ethyl | |
| 2.5.1004 | 3-Trifluormethoxy-4-methoxycarbonyl-Phenyl | Hydroxymethyl | Ethyl | |
| 2.5.1005 | 3-Nitro-4-methoxycarbonyl-Phenyl | Hydroxymethyl | Ethyl | |
| 2.5.1006 | 3-Fluor-4-cyano-Phenyl | Hydroxymethyl | Ethyl | |
| 2.5.1007 | 3-Chlor-4-cyano-Phenyl | Hydroxymethyl | Ethyl | |
| 2.5.1008 | 3-Brom-4-cyano-Phenyl | Hydroxymethyl | Ethyl | |
| 2.5.1009 | 3-Methyl-4-cyano-Phenyl | Hydroxymethyl | Ethyl | |
| 2.5.1010 | 3-Cyclopropyl-4-cyano-Phenyl | Hydroxymethyl | Ethyl | |
| 2.5.1011 | 3,4-Dicyano-Phenyl | Hydroxymethyl | Ethyl | |
| 2.5.1012 | 3-Trifluormethyl-4-cyano-Phenyl | Hydroxymethyl | Ethyl | |
| 2.5.1013 | 3-Trifluormethyl-4-cyano-Phenyl | 1-Hydroxyethyl | Ethyl | |
| 2.5.1014 | 3-Trifluormethyl-4-cyano-Phenyl | 1-Hydroxypropyl | Ethyl | |
| 2.5.1015 | 3-Trifluormethyl-4-cyano-Phenyl | (1-Hydroxy-2-methylpropyl) | Ethyl | |
| 2.5.1016 | 3-Trifluormethyl-4-cyano-Phenyl | Methoxymethyl | Ethyl | |
| 2.5.1017 | 3-Trifluormethyl-4-cyano-Phenyl | 2-Methoxyethyl | Ethyl | |
| 2.5.1018 | 3-Methoxy-4-cyano-Phenyl | Hydroxymethyl | Ethyl | |
| 2.5.1019 | 3-Ethoxy-4-cyano-Phenyl | Hydroxymethyl | Ethyl | |
| 2.5.1020 | 3-Trifluormethoxy-4-cyano-Phenyl | Hydroxymethyl | Ethyl | |
| 2.5.1021 | 3-Nitro-4-cyano-Phenyl | Hydroxymethyl | Ethyl | |
| 2.5.1022 | 3-Fluor-4-methoxy-Phenyl | Hydroxymethyl | Ethyl | |
| 2.5.1023 | 3-Chlor-4-methoxy-Phenyl | Hydroxymethyl | Ethyl | |
| 2.5.1024 | 3-Brom-4-methoxy-Phenyl | Hydroxymethyl | Ethyl | |
| 2.5.1025 | 3-Methyl-4-methoxy-Phenyl | Hydroxymethyl | Ethyl | |
| 2.5.1026 | 3-Cyclopropyl-4-methoxy-Phenyl | Hydroxymethyl | Ethyl | |
| 2.5.1027 | 3-Cyano-4-methoxy-Phenyl | Hydroxymethyl | Ethyl | |
| 2.5.1028 | 3-Trifluormethyl-4-methoxy-Phenyl | Hydroxymethyl | Ethyl | |
| 2.5.1029 | 3,4-Dimethoxy-Phenyl | Hydroxymethyl | Ethyl | |
| 2.5.1030 | 3-Ethoxy-4-methoxy-Phenyl | Hydroxymethyl | Ethyl | |
| 2.5.1031 | 3-Trifluormethoxy-4-methoxy-Phenyl | Hydroxymethyl | Ethyl | |
| 2.5.1032 | 3-Nitro-4-methoxy-Phenyl | Hydroxymethyl | Ethyl | |
| 2.5.1033 | 3-Fluor-4-ethoxy-Phenyl | Hydroxymethyl | Ethyl | |
| 2.5.1034 | 3-Chlor-4-ethoxy-Phenyl | Hydroxymethyl | Ethyl | |
| 2.5.1035 | 3-Chlor-4-ethoxy-Phenyl | 1-Hydroxyethyl | Ethyl | |
| 2.5.1036 | 3-Chlor-4-ethoxy-Phenyl | 1-Hydroxypropyl | Ethyl | |
| 2.5.1037 | 3-Chlor-4-ethoxy-Phenyl | (1-Hydroxy-2-methylpropyl) | Ethyl | |
| 2.5.1038 | 3-Chlor-4-ethoxy-Phenyl | Methoxymethyl | Ethyl | |
| 2.5.1039 | 3-Chlor-4-ethoxy-Phenyl | 2-Methoxyethyl | Ethyl | |
| 2.5.1040 | 3-Brom-4-ethoxy-Phenyl | Hydroxymethyl | Ethyl | |
| 2.5.1041 | 3-Methyl-4-ethoxy-Phenyl | Hydroxymethyl | Ethyl | |
| 2.5.1042 | 3-Cyclopropyl-4-ethoxy-Phenyl | Hydroxymethyl | Ethyl | |
| 2.5.1043 | 3-Cyano-4-ethoxy-Phenyl | Hydroxymethyl | Ethyl | |
| 2.5.1044 | 3-Trifluormethyl-4-ethoxy-Phenyl | Hydroxymethyl | Ethyl | |
| 2.5.1045 | 3-Methoxy-4-ethoxy-Phenyl | Hydroxymethyl | Ethyl | |
| 2.5.1046 | 2,4-Diethoxy-Phenyl | Hydroxymethyl | Ethyl | |
| 2.5.1047 | 3-Trifluormethoxy-4-ethoxy-Phenyl | Hydroxymethyl | Ethyl | |
| 2.5.1048 | 3-Nitro-4-ethoxy-Phenyl | Hydroxymethyl | Ethyl | |
| 2.5.1049 | 3-Fluor-4-isopropoxy-Phenyl | Hydroxymethyl | Ethyl | |
| 2.5.1050 | 3-Chlor-4-isopropoxy-Phenyl | Hydroxymethyl | Ethyl | |
| 2.5.1051 | 3-Brom-4-isopropoxy-Phenyl | Hydroxymethyl | Ethyl | |
| 2.5.1052 | 3-Methyl-4-isopropoxy-Phenyl | Hydroxymethyl | Ethyl | |
| 2.5.1053 | 3-Cyclopropyl-4-isopropoxy-Phenyl | Hydroxymethyl | Ethyl | |
| 2.5.1054 | 3-Cyano-4-isopropoxy-Phenyl | Hydroxymethyl | Ethyl | |
| 2.5.1055 | 3-Trifluormethyl-4-isopropoxy-Phenyl | Hydroxymethyl | Ethyl | |
| 2.5.1056 | 3-Methoxy-4-isopropoxy-Phenyl | Hydroxymethyl | Ethyl | |
| 2.5.1057 | 3-Ethoxy-4-isopropoxy-Phenyl | Hydroxymethyl | Ethyl | |
| 2.5.1058 | 3-Trifluormethoxy-4-isopropoxy-Phenyl | Hydroxymethyl | Ethyl | |
| 2.5.1059 | 3-Nitro-4-isopropoxy-Phenyl | Hydroxymethyl | Ethyl | |
| 2.5.1060 | 3-Fluor-4-trifluormethoxy-Phenyl | Hydroxymethyl | Ethyl | |
| 2.5.1061 | 3-Chlor-4-trifluormethoxy-Phenyl | Hydroxymethyl | Ethyl | |
| 2.5.1062 | 3-Brom-4-trifluormethoxy-Phenyl | Hydroxymethyl | Ethyl | |
| 2.5.1063 | 3-Methyl-4-trifluormethoxy-Phenyl | Hydroxymethyl | Ethyl | |
| 2.5.1064 | 3-Cyclopropyl-4-trifluormethoxy-Phenyl | Hydroxymethyl | Ethyl | |
| 2.5.1065 | 3-Cyano-4-trifluormethoxy-Phenyl | Hydroxymethyl | Ethyl | |
| 2.5.1066 | 3-Trifluormethyl-4-trifluormethoxy-Phenyl | Hydroxymethyl | Ethyl | |
| 2.5.1067 | 3-Methoxy-4-trifluormethoxy-Phenyl | Hydroxymethyl | Ethyl | |
| 2.5.1068 | 3-Ethoxy-4-trifluormethoxy-Phenyl | Hydroxymethyl | Ethyl | |
| 2.5.1069 | 3,4-Bis(trifluormethoxy)-Phenyl | Hydroxymethyl | Ethyl | |
| 2.5.1070 | 3-Nitro-4-trifluormethoxy-Phenyl | Hydroxymethyl | Ethyl | |
| 2.5.1071 | 3-Fluor-4-difluormethoxy-Phenyl | Hydroxymethyl | Ethyl | |
| 2.5.1072 | 3-Chlor-4-difluormethoxy-Phenyl | Hydroxymethyl | Ethyl | |
| 2.5.1073 | 3-Brom-4-difluormethoxy-Phenyl | Hydroxymethyl | Ethyl | |
| 2.5.1074 | 3-Methyl-4-difluormethoxy-Phenyl | Hydroxymethyl | Ethyl | |
| 2.5.1075 | 3-Cyclopropyl-4-difluormethoxy-Phenyl | Hydroxymethyl | Ethyl | |
| 2.5.1076 | 3-Cyano-4-difluormethoxy-Phenyl | Hydroxymethyl | Ethyl | |
| 2.5.1077 | 3-Trifluormethyl-4-difluormethoxy-Phenyl | Hydroxymethyl | Ethyl | |
| 2.5.1078 | 3-Methoxy-4-difluormethoxy-Phenyl | Hydroxymethyl | Ethyl | |
| 2.5.1079 | 3-Ethoxy-4-difluormethoxy-Phenyl | Hydroxymethyl | Ethyl | |
| 2.5.1080 | 3-Trifluormethoxy-4-difluormethoxy-Phenyl | Hydroxymethyl | Ethyl | |
| 2.5.1081 | 3-Nitro-4-difluormethoxy-Phenyl | Hydroxymethyl | Ethyl | |
| 2.5.1082 | 3-Fluor-4-nitro-Phenyl | Hydroxymethyl | Ethyl | |
| 2.5.1083 | 3-Chlor-4-nitro-Phenyl | Hydroxymethyl | Ethyl | |
| 2.5.1084 | 3-Brom-4-nitro-Phenyl | Hydroxymethyl | Ethyl | |
| 2.5.1085 | 3-Methyl-4-nitro-Phenyl | Hydroxymethyl | Ethyl | |
| 2.5.1086 | 3-Cyclopropyl-4-nitro-Phenyl | Hydroxymethyl | Ethyl | |
| 2.5.1087 | 3-Cyano-4-nitro-Phenyl | Hydroxymethyl | Ethyl | |
| 2.5.1088 | 3-Trifluormethyl-4-nitro-Phenyl | Hydroxymethyl | Ethyl | |
| 2.5.1089 | 3-Methoxy-4-nitro-Phenyl | Hydroxymethyl | Ethyl | |
| 2.5.1090 | 3-Ethoxy-4-nitro-Phenyl | Hydroxymethyl | Ethyl | |
| 2.5.1091 | 3-Trifluormethoxy-4-nitro-Phenyl | Hydroxymethyl | Ethyl | |
| 2.5.1092 | 3-Fluor-4-methylsulfanylPhenyl | Hydroxymethyl | Ethyl | |
| 2.5.1093 | 3-Chlor-4-methylsulfanyl-Phenyl | Hydroxymethyl | Ethyl | |
| 2.5.1094 | 3-Brom-4-methylsulfanyl-Phenyl | Hydroxymethyl | Ethyl | |
| 2.5.1095 | 3-Methyl-4-methylsulfanyl-Phenyl | Hydroxymethyl | Ethyl | |
| 2.5.1096 | 3-Cyclopropyl-4-methylsulfanyl-Phenyl | Hydroxymethyl | Ethyl | |
| 2.5.1097 | 3-Cyano-4-methylsulfanyl-Phenyl | Hydroxymethyl | Ethyl | |
| 2.5.1098 | 3-Trifluormethyl-4-methylsulfanyl -Phenyl | Hydroxymethyl | Ethyl | |
| 2.5.1099 | 3-Methoxy-4-methylsulfanyl-Phenyl | Hydroxymethyl | Ethyl | |
| 2.5.1100 | 3-Ethoxy-4-methylsulfanyl-Phenyl | Hydroxymethyl | Ethyl | |
| 2.5.1101 | 3-Trifluormethoxy-4-methylsulfanyl-Phenyl | Hydroxymethyl | Ethyl | |
| 2.5.1102 | 3-Nitro-4-methylsulfanyl-Phenyl | Hydroxymethyl | Ethyl | |
| 2.5.1103 | 3,6-Difluor-Phenyl | Hydroxymethyl | Ethyl | |
| 2.5.1104 | 3,6-Difluor-Phenyl | 1-Hydroxyethyl | Ethyl | |
| 2.5.1105 | 3,6-Difluor-Phenyl | 1-Hydroxypropyl | Ethyl | |
| 2.5.1106 | 3,6-Difluor-Phenyl | (1-Hydroxy-2-methylpropyl) | Ethyl | |
| 2.5.1107 | 3,6-Difluor-Phenyl | Methoxymethyl | Ethyl | |
| 2.5.1108 | 3,6-Difluor-Phenyl | 2-Methoxyethyl | Ethyl | |
| 2.5.1109 | 3-Chlor-6-fluor-Phenyl | Hydroxymethyl | Ethyl | |
| 2.5.1110 | 3-Brom-6-fluor-Phenyl | Hydroxymethyl | Ethyl | |
| 2.5.1111 | 3-Methyl-6-fluor-Phenyl | Hydroxymethyl | Ethyl | |
| 2.5.1112 | 3-Cyclopropyl-6-fluor-Phenyl | Hydroxymethyl | Ethyl | |
| 2.5.1113 | 3-Cyano-6-fluor-Phenyl | Hydroxymethyl | Ethyl | |
| 2.5.1114 | 3-Methoxy-6-fluor-Phenyl | Hydroxymethyl | Ethyl | |
| 2.5.1115 | 3-Ethoxy-6-fluor-Phenyl | Hydroxymethyl | Ethyl | |
| 2.5.1116 | 3-Trifluormethoxy-6-fluor-Phenyl | Hydroxymethyl | Ethyl | |
| 2.5.1117 | 3-Nitro-6-fluor-Phenyl | Hydroxymethyl | Ethyl | |
| 2.5.1118 | 3-Fluor-6-chlor-Phenyl | Hydroxymethyl | Ethyl | |
| 2.5.1119 | 3-Fluor-6-chlor-Phenyl | 1-Hydroxyethyl | Ethyl | |
| 2.5.1120 | 3-Fluor-6-chlor-Phenyl | 1-Hydroxypropyl | Ethyl | |
| 2.5.1121 | 3-Fluor-6-chlor-Phenyl | (1-Hydroxy-2-methylpropyl) | Ethyl | |
| 2.5.1122 | 3-Fluor-6-chlor-Phenyl | Methoxymethyl | Ethyl | |
| 2.5.1123 | 3-Fluor-6-chlor-Phenyl | 2-Methoxyethyl | Ethyl | |
| 2.5.1124 | 3,6-Dichlor-Phenyl | Hydroxymethyl | Ethyl | |
| 2.5.1125 | 3,6-Dichlor-Phenyl | 1-Hydroxyethyl | Ethyl | |
| 2.5.1126 | 3,6-Dichlor-Phenyl | 1-Hydroxypropyl | Ethyl | |
| 2.5.1127 | 3,6-Dichlor-Phenyl | (1-Hydroxy-2-methylpropyl) | Ethyl | |
| 2.5.1128 | 3,6-Dichlor-Phenyl | Methoxymethyl | Ethyl | |
| 2.5.1129 | 3,6-Dichlor-Phenyl | 2-Methoxyethyl | Ethyl | |
| 2.5.1130 | 3-Brom-6-chlor-Phenyl | Hydroxymethyl | Ethyl | |
| 2.5.1131 | 3-Methyl-6-chlor-Phenyl | Hydroxymethyl | Ethyl | |
| 2.5.1132 | 3-Ethyl-6-chlor-Phenyl | Hydroxymethyl | Ethyl | |
| 2.5.1133 | 3-Cyclopropyl-6-chlor-Phenyl | Hydroxymethyl | Ethyl | |
| 2.5.1134 | 3-Cyano-6-chlor-Phenyl | Hydroxymethyl | Ethyl | |
| 2.5.1135 | 3-Trifluormethyl-6-chlor-Phenyl | Hydroxymethyl | Ethyl | |
| 2.5.1136 | 3-Methoxy-6-chlor-Phenyl | Hydroxymethyl | Ethyl | |
| 2.5.1137 | 3-Ethoxy-6-chlor-Phenyl | Hydroxymethyl | Ethyl | |
| 2.5.1138 | 3-Trifluormethoxy-6-chlor-Phenyl | Hydroxymethyl | Ethyl | |
| 2.5.1139 | 3-Nitro-6-chlor-Phenyl | Hydroxymethyl | Ethyl | |
| 2.5.1140 | 3-Fluor-6-brom-Phenyl | Hydroxymethyl | Ethyl | |
| 2.5.1141 | 3-Chlor-6-brom-Phenyl | Hydroxymethyl | Ethyl | |
| 2.5.1142 | 3,6-Dibrom-Phenyl | Hydroxymethyl | Ethyl | |
| 2.5.1143 | 3-Methyl-6-brom-Phenyl | Hydroxymethyl | Ethyl | |
| 2.5.1144 | 3-Cyclopropyl-6-brom-Phenyl | Hydroxymethyl | Ethyl | |
| 2.5.1145 | 3-Cyano-6-brom-Phenyl | Hydroxymethyl | Ethyl | |
| 2.5.1146 | 3-Trifluormethyl-6-brom-Phenyl | Hydroxymethyl | Ethyl | |
| 2.5.1147 | 3-Methoxy-6-Phenyl | Hydroxymethyl | Ethyl | |
| 2.5.1148 | 3-Ethoxy-6-brom-Phenyl | Hydroxymethyl | Ethyl | |
| 2.5.1149 | 3-Trifluormethoxy-6-brom-Phenyl | Hydroxymethyl | Ethyl | |
| 2.5.1150 | 3-Nitro-6-brom-Phenyl | Hydroxymethyl | Ethyl | |
| 2.5.1151 | 3-Fluor-6-iod-Phenyl | Hydroxymethyl | Ethyl | |
| 2.5.1152 | 3-Chlor-6-iod-Phenyl | Hydroxymethyl | Ethyl | |
| 2.5.1153 | 3-Brom-6-iod-Phenyl | Hydroxymethyl | Ethyl | |
| 2.5.1154 | 3-Methyl-6-iod-Phenyl | Hydroxymethyl | Ethyl | |
| 2.5.1155 | 3-Cyclopropyl-6-iod-Phenyl | Hydroxymethyl | Ethyl | |
| 2.5.1156 | 3-Cyano-6-iod-Phenyl | Hydroxymethyl | Ethyl | |
| 2.5.1157 | 3-Trifluormethyl-6-iod-Phenyl | Hydroxymethyl | Ethyl | |
| 2.5.1158 | 3-Methoxy-6-iod-Phenyl | Hydroxymethyl | Ethyl | |
| 2.5.1159 | 3-Ethoxy-6-iod-Phenyl | Hydroxymethyl | Ethyl | |
| 2.5.1160 | 3-Trifluormethoxy-6-iod-Phenyl | Hydroxymethyl | Ethyl | |
| 2.5.1161 | 3-Nitro-6-iod-Phenyl | Hydroxymethyl | Ethyl | |
| 2.5.1162 | 3-Fluor-6-methyl-Phenyl | Hydroxymethyl | Ethyl | |
| 2.5.1163 | 3-Chlor-6-methyl-Phenyl | Hydroxymethyl | Ethyl | |
| 2.5.1164 | 3-Brom-6-methyl-Phenyl | Hydroxymethyl | Ethyl | |
| 2.5.1165 | 3.6-Dimethyl-Phenyl | Hydroxymethyl | Ethyl | |
| 2.5.1166 | 3-Cyclopropyl-6-methyl-Phenyl | Hydroxymethyl | Ethyl | |
| 2.5.1167 | 3-Cyano-6-methyl-Phenyl | Hydroxymethyl | Ethyl | |
| 2.5.1168 | 3-Trifluormethyl-6-methyl-Phenyl | Hydroxymethyl | Ethyl | |
| 2.5.1169 | 3-Methoxy-6-methyl-Phenyl | Hydroxymethyl | Ethyl | |
| 2.5.1170 | 3-Ethoxy-6-methyl-Phenyl | Hydroxymethyl | Ethyl | |
| 2.5.1171 | 3-Trifluormethoxy-6-methyl-Phenyl | Hydroxymethyl | Ethyl | |
| 2.5.1172 | 3-Nitro-6-methyl-Phenyl | Hydroxymethyl | Ethyl | |
| 2.5.1173 | 3-Fluor-6-ethyl-Phenyl | Hydroxymethyl | Ethyl | |
| 2.5.1174 | 3-Chlor-6-ethyl-Phenyl | Hydroxymethyl | Ethyl | |
| 2.5.1175 | 3-Brom-6-ethyl-Phenyl | Hydroxymethyl | Ethyl | |
| 2.5.1176 | 3-Methyl-6-ethyl-Phenyl | Hydroxymethyl | Ethyl | |
| 2.5.1177 | 3,6-Diethyl-Phenyl | Hydroxymethyl | Ethyl | |
| 2.5.1178 | 3-Cyclopropyl-6-ethyl-Phenyl | Hydroxymethyl | Ethyl | |
| 2.5.1179 | 3-Cyano-6-ethyl-Phenyl | Hydroxymethyl | Ethyl | |
| 2.5.1180 | 3-Trifluormethyl-6-ethyl-Phenyl | Hydroxymethyl | Ethyl | |
| 2.5.1181 | 3-Methoxy-6-ethyl-Phenyl | Hydroxymethyl | Ethyl | |
| 2.5.1182 | 3-Ethoxy-6-ethyl-Phenyl | Hydroxymethyl | Ethyl | |
| 2.5.1183 | 3-Trifluormethoxy-6-ethyl-Phenyl | Hydroxymethyl | Ethyl | |
| 2.5.1184 | 3-Nitro-6-ethyl-Phenyl | Hydroxymethyl | Ethyl | |
| 2.5.1185 | 3-Fluor-6-isopropyl-Phenyl | Hydroxymethyl | Ethyl | |
| 2.5.1186 | 3-Chlor-6-isopropyl-Phenyl | Hydroxymethyl | Ethyl | |
| 2.5.1187 | 3-Brom-6-isopropyl-Phenyl | Hydroxymethyl | Ethyl | |
| 2.5.1188 | 3-Methyl-6-isopropyl-Phenyl | Hydroxymethyl | Ethyl | |
| 2.5.1189 | 3-Ethyl-6-isopropyl-Phenyl | Hydroxymethyl | Ethyl | |
| 2.5.1190 | 3-Cyclopropyl-6-isopropyl-Phenyl | Hydroxymethyl | Ethyl | |
| 2.5.1191 | 3-Cyano-6-isopropyl-Phenyl | Hydroxymethyl | Ethyl | |
| 2.5.1192 | 3-Trifluormethyl-6-isopropyl-Phenyl | Hydroxymethyl | Ethyl | |
| 2.5.1193 | 3-Methoxy-6-isopropyl-Phenyl | Hydroxymethyl | Ethyl | |
| 2.5.1194 | 3-Ethoxy-6-isopropyl-Phenyl | Hydroxymethyl | Ethyl | |
| 2.5.1195 | 3-Trifluormethoxy-6-isopropyl-Phenyl | Hydroxymethyl | Ethyl | |
| 2.5.1196 | 3-Nitro-6-isopropyl-Phenyl | Hydroxymethyl | Ethyl | |
| 2.5.1197 | 3-Fluor-6-tert.butyl-Phenyl | Hydroxymethyl | Ethyl | |
| 2.5.1198 | 3-Chlor-6-tert.buty-Phenyl | Hydroxymethyl | Ethyl | |
| 2.5.1199 | 3-Brom-6-tert.butyl-Phenyl | Hydroxymethyl | Ethyl | |
| 2.5.1200 | 3-Methyl-6-tert.butyl-Phenyl | Hydroxymethyl | Ethyl | |
| 2.5.1201 | 3-Cyclopropyl-6-tert.butyl-Phenyl | Hydroxymethyl | Ethyl | |
| 2.5.1202 | 3-Cyano-6-tert.butyl-Phenyl | Hydroxymethyl | Ethyl | |
| 2.5.1203 | 3-Trifluormethyl-6-tert.butyl-Phenyl | Hydroxymethyl | Ethyl | |
| 2.5.1204 | 3-Methoxy-6-tert.butyl-Phenyl | Hydroxymethyl | Ethyl | |
| 2.5.1205 | 3-Ethoxy-6-tert.butyl-Phenyl | Hydroxymethyl | Ethyl | |
| 2.5.1206 | 3-Trifluormethoxy-6-tert.butyl-Phenyl | Hydroxymethyl | Ethyl | |
| 2.5.1207 | 3-Nitro-6-tert.butyl-Phenyl | Hydroxymethyl | Ethyl | |
| 2.5.1208 | 3-Fluor-6-cyclopropyl-Phenyl | Hydroxymethyl | Ethyl | |
| 2.5.1209 | 3-Chlor-6-cyclopropyl-Phenyl | Hydroxymethyl | Ethyl | |
| 2.5.1210 | 3-Brom-6-cyclopropyl-Phenyl | Hydroxymethyl | Ethyl | |
| 2.5.1211 | 3-Methyl-6-cyclopropyl-Phenyl | Hydroxymethyl | Ethyl | |
| 2.5.1212 | 3,6-Dicyclopropyl-Phenyl | Hydroxymethyl | Ethyl | |
| 2.5.1213 | 3-Cyano-6-cyclopropyl-Phenyl | Hydroxymethyl | Ethyl | |
| 2.5.1214 | 3-Trifluormethyl-6-cyclopropyl-Phenyl | Hydroxymethyl | Ethyl | |
| 2.5.1215 | 3-Methoxy-6-cyclopropyl-Phenyl | Hydroxymethyl | Ethyl | |
| 2.5.1216 | 3-Ethoxy-6-cyclopropyl-Phenyl | Hydroxymethyl | Ethyl | |
| 2.5.1217 | 3-Trifluormethoxy-6-cyclopropyl-Phenyl | Hydroxymethyl | Ethyl | |
| 2.5.1218 | 3-Fluor-6-methoxycarbonyl-Phenyl | Hydroxymethyl | Ethyl | |
| 2.5.1219 | 3-Chlor-6-methoxycarbonyl-Phenyl | Hydroxymethyl | Ethyl | |
| 2.5.1220 | 3-Brom-6-methoxycarbonyl-Phenyl | Hydroxymethyl | Ethyl | |
| 2.5.1221 | 3-Methyl-6-methoxycarbonyl-Phenyl | Hydroxymethyl | Ethyl | |
| 2.5.1222 | 3-Cyclopropyl-6-methoxycarbonyl-Phenyl | Hydroxymethyl | Ethyl | |
| 2.5.1223 | 3-Cyano-6-methoxycarbonyl-Phenyl | Hydroxymethyl | Ethyl | |
| 2.5.1224 | 3-Trifluormethyl-6-methoxycarbonyl-Phenyl | Hydroxymethyl | Ethyl | |
| 2.5.1225 | 3-Methoxy-6-methoxycarbonyl-Phenyl | Hydroxymethyl | Ethyl | |
| 2.5.1226 | 3-Ethoxy-6-methoxycarbonyl-Phenyl | Hydroxymethyl | Ethyl | |
| 2.5.1227 | 3-Trifluormethoxy-6-methoxycarbonyl-Phenyl | Hydroxymethyl | Ethyl | |
| 2.5.1228 | 3-Nitro-6-methoxycarbonyl-Phenyl | Hydroxymethyl | Ethyl | |
| 2.5.1229 | 3-Fluor-6-cyano-Phenyl | Hydroxymethyl | Ethyl | |
| 2.5.1230 | 3-Chlor-6-cyano-Phenyl | Hydroxymethyl | Ethyl | |
| 2.5.1231 | 3-Brom-6-cyano-Phenyl | Hydroxymethyl | Ethyl | |
| 2.5.1232 | 3-Methyl-6-cyano-Phenyl | Hydroxymethyl | Ethyl | |
| 2.5.1233 | 3-Cyclopropyl-6-cyano-Phenyl | Hydroxymethyl | Ethyl | |
| 2.5.1234 | 3,6-Dicyano-Phenyl | Hydroxymethyl | Ethyl | |
| 2.5.1235 | 3-Trifluormethyl-6-cyano-Phenyl | Hydroxymethyl | Ethyl | |
| 2.5.1236 | 3-Methoxy-6-cyano -Phenyl | Hydroxymethyl | Ethyl | |
| 2.5.1237 | 3-Ethoxy-6-cyano-Phenyl | Hydroxymethyl | Ethyl | |
| 2.5.1238 | 3-Trifluormethoxy-6-cyano-Phenyl | Hydroxymethyl | Ethyl | |
| 2.5.1239 | 3-Nitro-6-cyano-Phenyl | Hydroxymethyl | Ethyl | |
| 2.5.1240 | 3-Fluor-6-methoxy-Phenyl | Hydroxymethyl | Ethyl | |
| 2.5.1241 | 3-Chlor-6-methoxy-Phenyl | Hydroxymethyl | Ethyl | |
| 2.5.1242 | 3-Brom-6-methoxy-Phenyl | Hydroxymethyl | Ethyl | |
| 2.5.1243 | 3-Methyl-6-methoxy-Phenyl | Hydroxymethyl | Ethyl | |
| 2.5.1244 | 3-Cyclopropyl-6-methoxy-Phenyl | Hydroxymethyl | Ethyl | |
| 2.5.1245 | 3-Cyano-6-methoxy-Phenyl | Hydroxymethyl | Ethyl | |
| 2.5.1246 | 3-Trifluormethyl-6-methoxy-Phenyl | Hydroxymethyl | Ethyl | |
| 2.5.1247 | 3,6-Dimethoxy-Phenyl | Hydroxymethyl | Ethyl | |
| 2.5.1248 | 3-Ethoxy-6-methoxy-Phenyl | Hydroxymethyl | Ethyl | |
| 2.5.1249 | 3-Trifluormethoxy-6-methoxy-Phenyl | Hydroxymethyl | Ethyl | |
| 2.5.1250 | 3-Nitro-6-methoxy-Phenyl | Hydroxymethyl | Ethyl | |
| 2.5.1251 | 3-Fluor-6-ethoxy-Phenyl | Hydroxymethyl | Ethyl | |
| 2.5.1252 | 3-Chlor-6-ethoxy-Phenyl | Hydroxymethyl | Ethyl | |
| 2.5.1253 | 3-Brom-6-ethoxy-Phenyl | Hydroxymethyl | Ethyl | |
| 2.5.1254 | 3-Methyl-6-ethoxy-Phenyl | Hydroxymethyl | Ethyl | |
| 2.5.1255 | 3-Cyclopropyl-6-ethoxy-Phenyl | Hydroxymethyl | Ethyl | |
| 2.5.1256 | 3-Cyano-6-ethoxy-Phenyl | Hydroxymethyl | Ethyl | |
| 2.5.1257 | 3-Trifluormethyl-6-ethoxy-Phenyl | Hydroxymethyl | Ethyl | |
| 2.5.1258 | 3-Methoxy-6-ethoxy-Phenyl | Hydroxymethyl | Ethyl | |
| 2.5.1259 | 2,6-Diethoxy-Phenyl | Hydroxymethyl | Ethyl | |
| 2.5.1260 | 3-Trifluormethoxy-6-ethoxy-Phenyl | Hydroxymethyl | Ethyl | |
| 2.5.1261 | 3-Nitro-6-ethoxy-Phenyl | Hydroxymethyl | Ethyl | |
| 2.5.1262 | 3-Fluor-6-isopropoxy-Phenyl | Hydroxymethyl | Ethyl | |
| 2.5.1263 | 3-Chlor-6-isopropoxy-Phenyl | Hydroxymethyl | Ethyl | |
| 2.5.1264 | 3-Brom-6-isopropoxy-Phenyl | Hydroxymethyl | Ethyl | |
| 2.5.1265 | 3-Methyl-6-isopropoxy-Phenyl | Hydroxymethyl | Ethyl | |
| 2.5.1266 | 3-Cyclopropyl-6-isopropoxy-Phenyl | Hydroxymethyl | Ethyl | |
| 2.5.1267 | 3-Cyano-6-isopropoxy-Phenyl | Hydroxymethyl | Ethyl | |
| 2.5.1268 | 3-Trifluormethyl-6-isopropoxy-Phenyl | Hydroxymethyl | Ethyl | |
| 2.5.1269 | 3-Methoxy-6-isopropoxy-Phenyl | Hydroxymethyl | Ethyl | |
| 2.5.1270 | 3-Trifluormethoxy-6-isopropoxy-Phenyl | Hydroxymethyl | Ethyl | |
| 2.5.1271 | 3-Nitro-6-isopropoxy-Phenyl | Hydroxymethyl | Ethyl | |
| 2.5.1272 | 3-Fluor-6-trifluormethoxy-Phenyl | Hydroxymethyl | Ethyl | |
| 2.5.1273 | 3-Chlor-6-trifluormethoxy-Phenyl | Hydroxymethyl | Ethyl | |
| 2.5.1274 | 3-Brom-6-trifluormethoxy-Phenyl | Hydroxymethyl | Ethyl | |
| 2.5.1275 | 3-Methyl-6-trifluormethoxy-Phenyl | Hydroxymethyl | Ethyl | |
| 2.5.1276 | 3-Cyclopropyl-6-trifluormethoxy-Phenyl | Hydroxymethyl | Ethyl | |
| 2.5.1277 | 3-Cyano-6-trifluormethoxy-Phenyl | Hydroxymethyl | Ethyl | |
| 2.5.1278 | 3-Trifluormethyl-6-trifluormethoxy-Phenyl | Hydroxymethyl | Ethyl | |
| 2.5.1279 | 3-Methoxy-6-trifluormethoxy-Phenyl | Hydroxymethyl | Ethyl | |
| 2.5.1280 | 3-Ethoxy-6-trifluormethoxy-Phenyl | Hydroxymethyl | Ethyl | |
| 2.5.1281 | 3,6-Bis(trifluormethoxy)-Phenyl | Hydroxymethyl | Ethyl | |
| 2.5.1282 | 3-Nitro-6-trifluormethoxy-Phenyl | Hydroxymethyl | Ethyl | |
| 2.5.1283 | 3-Fluor-6-difluormethoxy-Phenyl | Hydroxymethyl | Ethyl | |
| 2.5.1284 | 3-Chlor-6-difluormethoxy-Phenyl | Hydroxymethyl | Ethyl | |
| 2.5.1285 | 3-Brom-6-difluormethoxy-Phenyl | Hydroxymethyl | Ethyl | |
| 2.5.1286 | 3-Methyl-6-difluormethoxy-Phenyl | Hydroxymethyl | Ethyl | |
| 2.5.1287 | 3-Ethyl-6-difluormethoxy-Phenyl | Hydroxymethyl | Ethyl | |
| 2.5.1288 | 3-Cyclopropyl-6-difluormethoxy-Phenyl | Hydroxymethyl | Ethyl | |
| 2.5.1289 | 3-Cyano-6-difluormethoxy-Phenyl | Hydroxymethyl | Ethyl | |
| 2.5.1290 | 3-Trifluormethyl-6-difluormethoxy-Phenyl | Hydroxymethyl | Ethyl | |
| 2.5.1291 | 3-Methoxy-6-difluormethoxy-Phenyl | Hydroxymethyl | Ethyl | |
| 2.5.1292 | 3-Nitro-6-difluormethoxy-Phenyl | Hydroxymethyl | Ethyl | |
| 2.5.1293 | 3-Fluor-6-nitro-Phenyl | Hydroxymethyl | Ethyl | |
| 2.5.1294 | 3-Chlor-6-nitro-Phenyl | Hydroxymethyl | Ethyl | |
| 2.5.1295 | 3-Brom-6-nitro-Phenyl | Hydroxymethyl | Ethyl | |
| 2.5.1296 | 3-Methyl-6-nitro-Phenyl | Hydroxymethyl | Ethyl | |
| 2.5.1297 | 3-Cyclopropyl-6-nitro-Phenyl | Hydroxymethyl | Ethyl | |
| 2.5.1298 | 3-Cyano-6-nitro-Phenyl | Hydroxymethyl | Ethyl | |
| 2.5.1299 | 3-Trifluormethyl-6-nitro-Phenyl | Hydroxymethyl | Ethyl | |
| 2.5.1300 | 3-Methoxy-6-nitro-Phenyl | Hydroxymethyl | Ethyl | |
| 2.5.1301 | 3-Trifluormethoxy-6-nitro-Phenyl | Hydroxymethyl | Ethyl | |
| 2.5.1302 | 3-Fluor-6-methylsulfanylPhenyl | Hydroxymethyl | Ethyl | |
| 2.5.1303 | 3-Chlor-6-methylsulfanyl-Phenyl | Hydroxymethyl | Ethyl | |
| 2.5.1304 | 3-Brom-6-methylsulfanyl-Phenyl | Hydroxymethyl | Ethyl | |
| 2.5.1305 | 3-Methyl-6-methylsulfanyl-Phenyl | Hydroxymethyl | Ethyl | |
| 2.5.1306 | 3-Cyclopropyl-6-methylsulfanyl-Phenyl | Hydroxymethyl | Ethyl | |
| 2.5.1307 | 3-Cyano-6-methylsulfanyl-Phenyl | Hydroxymethyl | Ethyl | |
| 2.5.1308 | 3-Trifluormethyl-6-methylsulfanyl -Phenyl | Hydroxymethyl | Ethyl | |
| 2.5.1309 | 3-Methoxy-6-methylsulfanyl-Phenyl | Hydroxymethyl | Ethyl | |
| 2.5.1310 | 3-Trifluormethoxy-6-methylsulfanyl-Phenyl | Hydroxymethyl | Ethyl | |
| 2.5.1311 | 3-Nitro-6-methylsulfanyl-Phenyl | Hydroxymethyl | Ethyl | |
| 2.5.1312 | 2,3,4-Trifluor-Phenyl | Hydroxymethyl | Ethyl | |
| 2.5.1313 | 2,3,4-Trichlor-Phenyl | Hydroxymethyl | Ethyl | |
| 2.5.1314 | 2,3.4-Trimethyl-Phenyl | Hydroxymethyl | Ethyl | |
| 2.5.1315 | 2-Fluor-2-chlor-5-trifluormethyl-Phenyl | Hydroxymethyl | Ethyl | |
| 2.5.1316 | 2,3,5-Trifluor-Phenyl | Hydroxymethyl | Ethyl | |
| 2.5.1317 | 2,3,5-Trichlor-Phenyl | Hydroxymethyl | Ethyl | |
| 2.5.1318 | 2,3,5-Trimethyl-Phenyl | Hydroxymethyl | Ethyl | |
| 2.5.1319 | 2,3-Dichlor-5-methoxy-Phenyl | Hydroxymethyl | Ethyl | |
| 2.5.1320 | 2,3,6-Trifluor-Phenyl | Hydroxymethyl | Ethyl | |
| 2.5.1321 | 2,3,6-Trichlor-Phenyl | Hydroxymethyl | Ethyl | |
| 2.5.1322 | 2,3,6-Trimethyl-Phenyl | Hydroxymethyl | Ethyl | |
| 2.5.1323 | 3,4,5-Trifluor-Phenyl | Hydroxymethyl | Ethyl | |
| 2.5.1324 | 3,4,5-Trichlor-Phenyl | Hydroxymethyl | Ethyl | |
| 2.5.1325 | 3,4,5-Trimethyl-Phenyl | Hydroxymethyl | Ethyl | |
| 2.5.1326 | 3,5-Dimethyl-4-fluor-Phenyl | Hydroxymethyl | Ethyl | |
| 2.5.1327 | 3,5-Dichlor-4-methoxy-Phenyl | Hydroxymethyl | Ethyl | |
| 2.5.1328 | 3,5-Difluor-4-chlor-Phenyl | Hydroxymethyl | Ethyl | |
| 2.5.1329 | 3,5-Dichlor-4-hydroxy-Phenyl | Hydroxymethyl | Ethyl | |
| 2.5.1330 | 3,5-Trifluormethyl-4-chlor-Phenyl | Hydroxymethyl | Ethyl | |
| 2.5.1331 | 3,4,6-Trifluor-Phenyl | Hydroxymethyl | Ethyl | |
| 2.5.1332 | 3,4,6-Trichlor-Phenyl | Hydroxymethyl | Ethyl | |
| 2.5.1333 | 3,4,6-Trimethyl-Phenyl | Hydroxymethyl | Ethyl | |
| 2.5.1334 | Pentafluorphenyl | Hydroxymethyl | Ethyl | |

**Tabelle 2.6: Erfindungsgemäße Verbindungen der allgemeinen Formel (I), worin W* für COOY, R¹ und R² für Wasserstoff stehen, und Aryl den Rest**

| | | | | |
|---|---|---|---|---|
| 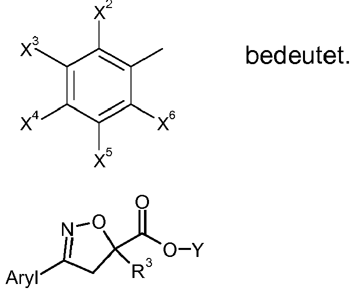 | | | | |

| Nr. | Aryl | R³ | Y | Physikalische Daten |
|---|---|---|---|---|
| 2.6.001 | 3-Fluor-Phenyl | Methyl | (Cyclohex-2-en-1-on)-3-yl | |
| 2.6.002 | 3-Fluor-Phenyl | Methyl | (Propan-1-ol)-3-yl | |
| 2.6.003 | 3-Fluor-Phenyl | Methyl | (2,2-Dimethylpropan-1-ol)-3yl | |
| 2.6.004 | 3-Fluor-Phenyl | Methyl | (Methyl-2,2-dimethylpropanoat)-3-yl | |
| 2.6.005 | 3-Fluor-Phenyl | Methyl | (Methylpropanoat)-3-yl | |
| 2.6.006 | 3-Fluor-Phenyl | Methyl | (Ethylpropanoat)-3-yl | |
| 2.6.007 | 3-Fluor-Phenyl | Methyl | 4-Ethoxy-4-oxobutan-2-yl | |
| 2.6.008 | 3-Fluor-Phenyl | Methyl | (Ethyl-(3R)-4,4,4-trifluorbutanoat)-3-yl | |
| 2.6.009 | 3-Fluor-Phenyl | Methyl | (Butan-1-ol)-4-yl | [CDCl₃] 1.55 (bs, 1H); 1.65 (mc, 2H); 1.75 (s, 3H); 1.80 (mc, 2H); 3.18 (d, 1H); 3.68 (t, 2H); 3.85 (d, 1H); 4.25 (t, 2H); 7.11 (m, 1H); 7.40 (m, 3H). |
| 2.6.010 | 3-Fluor-Phenyl | Methyl | (3-Methylbutan-2-on)-4-yl | |
| 2.6.011 | 3-Fluor-Phenyl | Methyl | (Pent-3-en-2-on)-4-yl | |
| 2.6.012 | 3-Fluor-Phenyl | Methyl | ((2S)-Dimethyl-butandioat)-2-yl | |
| 2.6.013 | 3-Fluor-Phenyl | Methyl | (Dimethylpentandioat)-3-yl | |
| 2.6.014 | 3-Fluor-Phenyl | Ethyl | (Dimethylpentandioat)-3-yl | [CDCl₃] D1 0.98 (t,3H); 1.24 (t,3H); 1.35 (d,3H); 1.95-2.12 (m,2H); 2.50-2.75 (m,2H); 3.18 (d,1H); 3.81 (d,1H); 3.98-4.09 (m,2H); 5.33-5.42 (m,1H); 7.12 (t,1H); 7.33-7.43 (m 3H). D2 1.02 (t,3H); 1.19 (t,3H); 1.36 (d,3H); 1.95-2.12 (m,2H); 2.50-2.75 (m,2H); 3.20 (d,1H); 3.76 (d,1H); 4.09-4.17 (m,2H); 5.33-5.42 (m,1H); 7.12 (t,1H); 7.33-7.43 (m 3H). |
| 2.6.015 | 3-Fluor-Phenyl | Methyl | (Methyl-(2R)-2-methylpropanoat)-3-yl | |
| 2.6.016 | 3-Fluor-Phenyl | Methyl | 4-Methoxycarbonylbenzyl | |
| 2.6.017 | 3-Fluor-Phenyl | Methyl | 3,5-Difluorbenzyl | |
| 2.6.018 | 3-Fluor-Phenyl | Methyl | 3,4-Difluorbenzyl | |
| 2.6.019 | 3-Fluor-Phenyl | Methyl | 2,6-Difluorbenzyl | |
| 2.6.020 | 3-Fluor-Phenyl | Methyl | (5-Methylpyridin-3-yl)methyl | [CDCl₃] 1.73 (s,3H); 2.36 (s,3H); 3.20 (d,1H); 3.85 (d,1H); 5.22 (s,2H); 7.13 (m,1H); 7.39 (m,3H); 7.51 (s,1H); 8.42 (d,2H). |
| 2.6.021 | 3-Fluor-Phenyl | Methyl | (Tetrahydrofuran)-3-yl | |
| 2.6.022 | 3-Fluor-Phenyl | Ethyl | 4-Ethoxy-4-oxobutan-2-yl | |
| 2.6.023 | 3-Chlor-Phenyl | Methyl | (Ethylbutanoat)-3-yl | |
| 2.6.024 | 3-lod-Phenyl | Methyl | (Ethylbutanoat)-3-yl | |
| 2.6.025 | 3-lod-Phenyl | Ethyl | (Ethylbutanoat)-3-yl | |
| 2.6.026 | 3-Methyl-Phenyl | Methyl | (Ethylbutanoat)-3-yl | |
| 2.6.027 | 3-Methyl-Phenyl | Ethyl | (Ethylbutanoat)-3-yl | |
| 2.6.028 | 3-Ethyl-Phenyl | Methyl | (Ethylbutanoat)-3-yl | |
| 2.6.029 | 3-Propyl-Phenyl | Methyl | (Ethylbutanoat)-3-yl | |
| 2.6.030 | 3-isoPropyl-Phenyl | Methyl | (Ethylbutanoat)-3-yl | |
| 2.6.031 | 3-nButyl-Phenyl | Methyl | (Ethylbutanoat)-3-yl | |
| 2.6.032 | 3-iButyl-Phenyl | Methyl | (Ethylbutanoat)-3-yl | |
| 2.6.033 | 3-tert. Butyl-Phenyl | Methyl | (Ethylbutanoat)-3-yl | |
| 2.6.034 | 3-Cyclopropyl-Phenyl | Methyl | (Ethylbutanoat)-3-yl | |
| 2.6.035 | 3-Cyclobutyl-Phenyl | Methyl | (Ethylbutanoat)-3-yl | |
| 2.6.036 | 3-Cyclopentyl-Phenyl | Methyl | (Ethylbutanoat)-3-yl | |
| 2.6.037 | 3-Vinyl-Phenyl | Methyl | (Ethylbutanoat)-3-yl | |
| 2.6.038 | 3-Ethinyl-Phenyl | Methyl | (Ethylbutanoat)-3-yl | |
| 2.6.039 | 3-Cyano-Phenyl | Methyl | (Ethylbutanoat)-3-yl | |
| 2.6.040 | 3-Trifluormethyl-Phenyl | Methyl | (Ethylbutanoat)-3-yl | |
| 2.6.041 | 3-Difluormethyl-Phenyl | Methyl | (Ethylbutanoat)-3-yl | |
| 2.6.042 | 3-(Hydroxycarbonyl) -Phenyl | Methyl | (Ethylbutanoat)-3-yl | |
| 2.6.043 | 3-(Methoxycarbony) l-Phenyl | Methyl | (Ethylbutanoat)-3-yl | |
| 2.6.044 | 3-(Ethoxycarbonyl)-Phenyl | Methyl | (Ethylbutanoat)-3-yl | |
| 2.6.045 | 3-Hydroxymethyl-Phenyl | Methyl | (Ethylbutanoat)-3-yl | |
| 2.6.046 | 3-Acetyl-Phenyl | Methyl | (Ethylbutanoat)-3-yl | |
| 2.6.047 | 3-Carbamoyl-Phenyl | Methyl | (Ethylbutanoat)-3-yl | |
| 2.6.048 | 3-Hydroxy-Phenyl- | Methyl | (Ethylbutanoat)-3-yl | |
| 2.6.049 | 3-Methoxy-Phenyl | Methyl | (Ethylbutanoat)-3-yl | |
| 2.6.050 | 3-Ethoxy-Phenyl | Methyl | (Ethylbutanoat)-3-yl | |
| 2.6.051 | 3-Propyloxy-Phenyl | Methyl | (Ethylbutanoat)-3-yl | |
| 2.6.052 | 3-isoPropyloxy-Phenyl | Methyl | (Ethylbutanoat)-3-yl | |
| 2.6.053 | 3-nButyloxy-Phenyl | Methyl | (Ethylbutanoat)-3-yl | |
| 2.6.054 | 3-iButyloxy-Phenyl | Methyl | (Ethylbutanoat)-3-yl | |
| 2.6.055 | 3-tButyloxy-Phenyl | Methyl | (Ethylbutanoat)-3-yl | |
| 2.6.056 | 3-Difluormethoxy-Phenyl | Methyl | (Ethylbutanoat)-3-yl | |
| 2.6.057 | 3-Trifluormethoxy-Phenyl | Methyl | (Ethylbutanoat)-3-yl | |
| 2.6.058 | 3-(2,2,2-Trifluorethoxy)-Phenyl | Methyl | (Ethylbutanoat)-3-yl | |
| 2.6.059 | 3-(2-Chlorethoxy)-Phenyl | Methyl | (Ethylbutanoat)-3-yl | |
| 2.6.060 | 3-(2-Hydroxyethoxy)-Phenyl- | Methyl | (Ethylbutanoat)-3-yl | |
| 2.6.061 | 3-(2-Methoxyethoxy)-Phenyl- | Methyl | (Ethylbutanoat)-3-yl | |
| 2.6.062 | 3-[(tert-Butoxy-carbonyl)oxy]-Phenyl | Methyl | (Ethylbutanoat)-3-yl | |
| 2.6.063 | 3-Nitro-Phenyl | Methyl | (Ethylbutanoat)-3-yl | |
| 2.6.064 | 3-Acetoxy-Phenyl | Methyl | (Ethylbutanoat)-3-yl | |
| 2.6.065 | {3-[(tert-Butoxycarbonyl)a mino]-Phenyl}- | Methyl | (Ethylbutanoat)-3-yl | |
| 2.6.066 | 3-Methylsulfanyl-Phenyl | Methyl | (Ethylbutanoat)-3-yl | |
| 2.6.067 | 3-Ethylsulfanyl-Phenyl | Methyl | (Ethylbutanoat)-3-yl | |
| 2.6.068 | 3-(Pentafluor-lambda⁶-sulfanyl)-Phenyl | Methyl | (Ethylbutanoat)-3-yl | |
| 2.6.069 | 2,3-Difluor-Phenyl | Methyl | (Ethylbutanoat)-3-yl | |
| 2.6.070 | 2-Chlor-3-fluor-Phenyl | Methyl | (Ethylbutanoat)-3-yl | |
| 2.6.071 | 2-Brom-3-fluor-Phenyl | Methyl | (Ethylbutanoat)-3-yl | |
| 2.6.072 | 2-Methyl-3-fluor-Phenyl | Methyl | (Ethylbutanoat)-3-yl | |
| 2.6.073 | 2-Ethyl-3-fluor-Phenyl | Methyl | (Ethylbutanoat)-3-yl | |
| 2.6.074 | 2-Cyclopropyl-3-fluor-Phenyl | Methyl | (Ethylbutanoat)-3-yl | |
| 2.6.075 | 2-Vinyl-3-fluor-Phenyl | Methyl | (Ethylbutanoat)-3-yl | |
| 2.6.076 | 2-Ethinyl-3-fluor-Phenyl | Methyl | (Ethylbutanoat)-3-yl | |
| 2.6.077 | 2-Cyano-3-fluor-Phenyl | Methyl | (Ethylbutanoat)-3-yl | |
| 2.6.078 | 2-Methoxy-3-fluor-Phenyl | Methyl | (Ethylbutanoat)-3-yl | |
| 2.6.079 | 2-Ethoxy-3-fluor-Phenyl | Methyl | (Ethylbutanoat)-3-yl | |
| 2.6.080 | 2-Trifluormethoxy-3-fluor-Phenyl | Methyl | (Ethylbutanoat)-3-yl | |
| 2.6.081 | 2-Nitro-3-fluor-Phenyl | Methyl | (Ethylbutanoat)-3-yl | |
| 2.6.082 | 2-Fluor-3-chlor-Phenyl | Methyl | (Ethylbutanoat)-3-yl | |
| 2.6.083 | 2,3-Dichlor-Phenyl | Methyl | (Ethylbutanoat)-3-yl | |
| 2.6.084 | 2-Brom-3-chlor-Phenyl | Methyl | (Ethylbutanoat)-3-yl | |
| 2.6.085 | 2-Methyl-3-chlor-Phenyl | Methyl | (Ethylbutanoat)-3-yl | |
| 2.6.086 | 2-Ethyl-3-chlor-Phenyl | Methyl | (Ethylbutanoat)-3-yl | |
| 2.6.087 | 2-Cyclopropyl-3-chlor-Phenyl | Methyl | (Ethylbutanoat)-3-yl | |
| 2.6.088 | 2-Vinyl-3-chlor-Phenyl | Methyl | (Ethylbutanoat)-3-yl | |
| 2.6.089 | 2-Ethinyl-3-chlor-Phenyl | Methyl | (Ethylbutanoat)-3-yl | |
| 2.6.090 | 2-Cyano-3-chlor-Phenyl | Methyl | (Ethylbutanoat)-3-yl | |
| 2.6.091 | 2-Trifluormethyl-2-chlor-Phenyl | Methyl | (Ethylbutanoat)-3-yl | |
| 2.6.092 | 2-Methoxy-3-chlor-Phenyl | Methyl | (Ethylbutanoat)-3-yl | |
| 2.6.093 | 2-Ethoxy-3-chlor-Phenyl | Methyl | (Ethylbutanoat)-3-yl | |
| 2.6.094 | 2-Trifluormethoxy-3-chlor-Phenyl | Methyl | (Ethylbutanoat)-3-yl | |
| 2.6.095 | 2-Nitro-3-chlor-Phenyl | Methyl | (Ethylbutanoat)-3-yl | |
| 2.6.096 | 2-Fluor-3-brom-Phenyl | Methyl | (Ethylbutanoat)-3-yl | |
| 2.6.097 | 2-Chlor-3-brom-Phenyl | Methyl | (Ethylbutanoat)-3-yl | |
| 2.6.098 | 2,3-Dibrom-Phenyl | Methyl | (Ethylbutanoat)-3-yl | |
| 2.6.099 | 2-Methyl-3-brom-Phenyl | Methyl | (Ethylbutanoat)-3-yl | |
| 2.6.100 | 2-Ethyl-3-brom-Phenyl | Methyl | (Ethylbutanoat)-3-yl | |
| 2.6.101 | 2-Cyclopropyl-3-brom-Phenyl | Methyl | (Ethylbutanoat)-3-yl | |
| 2.6.102 | 2-Vinyl-3-brom-Phenyl | Methyl | (Ethylbutanoat)-3-yl | |
| 2.6.103 | 2-Ethinyl-3-brom-Phenyl | Methyl | (Ethylbutanoat)-3-yl | |
| 2.6.104 | 2-Cyano-3-brom-Phenyl | Methyl | (Ethylbutanoat)-3-yl | |
| 2.6.105 | 2-Trifluormethyl-3-brom-Phenyl | Methyl | (Ethylbutanoat)-3-yl | |
| 2.6.106 | 2-Methoxy-3-Phenyl | Methyl | (Ethylbutanoat)-3-yl | |
| 2.6.107 | 2-Ethoxy-3-brom-Phenyl | Methyl | (Ethylbutanoat)-3-yl | |
| 2.6.108 | 2-Trifluormethoxy-3-brom-Phenyl | Methyl | (Ethylbutanoat)-3-yl | |
| 2.6.109 | 2-Nitro-3-brom-Phenyl | Methyl | (Ethylbutanoat)-3-yl | |
| 2.6.110 | 2-Fluor-3-iod-Phenyl | Methyl | (Ethylbutanoat)-3-yl | |
| 2.6.111 | 2-Chlor-3-iod-Phenyl | Methyl | (Ethylbutanoat)-3-yl | |
| 2.6.112 | 2-Brom-3-iod-Phenyl | Methyl | (Ethylbutanoat)-3-yl | |
| 2.6.113 | 2-Methyl-3-iod-Phenyl | Methyl | (Ethylbutanoat)-3-yl | |
| 2.6.114 | 2-Ethyl-3-iod-Phenyl | Methyl | (Ethylbutanoat)-3-yl | |
| 2.6.115 | 2-Cyclopropyl-3-iod-Phenyl | Methyl | (Ethylbutanoat)-3-yl | |
| 2.6.116 | 2-Vinyl-3-iod-Phenyl | Methyl | (Ethylbutanoat)-3-yl | |
| 2.6.117 | 2-Ethinyl-3-iod-Phenyl | Methyl | (Ethylbutanoat)-3-yl | |
| 2.6.118 | 2-Cyano-3-iod-Phenyl | Methyl | (Ethylbutanoat)-3-yl | |
| 2.6.119 | 2-Trifluormethyl-3-iod-Phenyl | Methyl | (Ethylbutanoat)-3-yl | |
| 2.6.120 | 2-Methoxy-3-iod-Phenyl | Methyl | (Ethylbutanoat)-3-yl | |
| 2.6.121 | 2-Ethoxy-3-iod-Phenyl | Methyl | (Ethylbutanoat)-3-yl | |
| 2.6.122 | 2-Trifluormethoxy-3-iod-Phenyl | Methyl | (Ethylbutanoat)-3-yl | |
| 2.6.123 | 2-Nitro-3-iod-Phenyl | Methyl | (Ethylbutanoat)-3-yl | |
| 2.6.124 | 2-Fluor-3-methyl-Phenyl | Methyl | (Ethylbutanoat)-3-yl | |
| 2.6.125 | 2-Chlor-3-methyl-Phenyl | Methyl | (Ethylbutanoat)-3-yl | |
| 2.6.126 | 2-Brom-3-methyl-Phenyl | Methyl | (Ethylbutanoat)-3-yl | |
| 2.6.127 | 2,3-Dimethyl-Phenyl | Methyl | (Ethylbutanoat)-3-yl | |
| 2.6.128 | 2-Ethyl-3-methyl-Phenyl | Methyl | (Ethylbutanoat)-3-yl | |
| 2.6.129 | 2-Cyclopropyl-3-methyl-Phenyl | Methyl | (Ethylbutanoat)-3-yl | |
| 2.6.130 | 2-Vinyl-3-methyl-Phenyl | Methyl | (Ethylbutanoat)-3-yl | |
| 2.6.131 | 2-Ethinyl-3-methyl-Phenyl | Methyl | (Ethylbutanoat)-3-yl | |
| 2.6.132 | 2-Cyano-3-methyl-Phenyl | Methyl | (Ethylbutanoat)-3-yl | |
| 2.6.133 | 2-Trifluormethyl-3-methyl-Phenyl | Methyl | (Ethylbutanoat)-3-yl | |
| 2.6.134 | 2-Methoxy-3-methyl-Phenyl | Methyl | (Ethylbutanoat)-3-yl | |
| 2.6.135 | 2-Ethoxy-3-methyl-Phenyl | Methyl | (Ethylbutanoat)-3-yl | |
| 2.6.136 | 2-Trifluormethoxy-3-methyl-Phenyl | Methyl | (Ethylbutanoat)-3-yl | |
| 2.6.137 | 2-Nitro-3-methyl-Phenyl | Methyl | (Ethylbutanoat)-3-yl | |
| 2.6.138 | 2-Fluor-3-ethyl-Phenyl | Methyl | (Ethylbutanoat)-3-yl | |
| 2.6.139 | 2-Chlor-3-ethyl-Phenyl | Methyl | (Ethylbutanoat)-3-yl | |
| 2.6.140 | 2-Brom-3-ethyl-Phenyl | Methyl | (Ethylbutanoat)-3-yl | |
| 2.6.141 | 2-Methyl-3-ethyl-Phenyl | Methyl | (Ethylbutanoat)-3-yl | |
| 2.6.142 | 2,3-Diethyl-Phenyl | Methyl | (Ethylbutanoat)-3-yl | |
| 2.6.143 | 2-Cyclopropyl-3-ethyl-Phenyl | Methyl | (Ethylbutanoat)-3-yl | |
| 2.6.144 | 2-Vinyl-3-ethyl-Phenyl | Methyl | (Ethylbutanoat)-3-yl | |
| 2.6.145 | 2-Ethinyl-3-ethyl-Phenyl | Methyl | (Ethylbutanoat)-3-yl | |
| 2.6.146 | 2-Cyano-3-ethyl-Phenyl | Methyl | (Ethylbutanoat)-3-yl | |
| 2.6.147 | 2-Trifluormethyl-3-ethyl-Phenyl | Methyl | (Ethylbutanoat)-3-yl | |
| 2.6.148 | 2-Methoxy-3-ethyl-Phenyl | Methyl | (Ethylbutanoat)-3-yl | |
| 2.6.149 | 2-Ethoxy-3-ethyl-Phenyl | Methyl | (Ethylbutanoat)-3-yl | |
| 2.6.150 | 2-Trifluormethoxy-3-ethyl-Phenyl | Methyl | (Ethylbutanoat)-3-yl | |
| 2.6.151 | 2-Nitro-3-ethyl-Phenyl | Methyl | (Ethylbutanoat)-3-yl | |
| 2.6.152 | 2-Fluor-3-propyl-Phenyl | Methyl | (Ethylbutanoat)-3-yl | |
| 2.6.153 | 2-Chlor-3-propyl-Phenyl | Methyl | (Ethylbutanoat)-3-yl | |
| 2.6.154 | 2-Brom-3-propyl-Phenyl | Methyl | (Ethylbutanoat)-3-yl | |
| 2.6.155 | 2-Methyl-3-propyl-Phenyl | Methyl | (Ethylbutanoat)-3-yl | |
| 2.6.156 | 2-Methyl-3-propyl-Phenyl | Methyl | (Ethylbutanoat)-3-yl | |
| 2.6.157 | 2-Cyclopropyl-3-propyl-Phenyl | Methyl | (Ethylbutanoat)-3-yl | |
| 2.6.158 | 2-Vinyl-3-propyl-Phenyl | Methyl | (Ethylbutanoat)-3-yl | |
| 2.6.159 | 2-Ethinyl-3propyl-Phenyl | Methyl | (Ethylbutanoat)-3-yl | |
| 2.6.160 | 2-Cyano-3-propyl-Phenyl | Methyl | (Ethylbutanoat)-3-yl | |
| 2.6.161 | 2-Trifluormethyl-3-propyl-Phenyl | Methyl | (Ethylbutanoat)-3-yl | |
| 2.6.162 | 2-Methoxy-3-propyl-Phenyl | Methyl | (Ethylbutanoat)-3-yl | |
| 2.6.163 | 2-Ethoxy-3-propyl-Phenyl | Methyl | (Ethylbutanoat)-3-yl | |
| 2.6.164 | 2-Trifluormethoxy-3-propyl-Phenyl | Methyl | (Ethylbutanoat)-3-yl | |
| 2.6.165 | 2-Nitro-3-propyl-Phenyl | Methyl | (Ethylbutanoat)-3-yl | |
| 2.6.166 | 2-Fluor-3-isopropyl-Phenyl | Methyl | (Ethylbutanoat)-3-yl | |
| 2.6.167 | 2-Chlor-3-isopropyl-Phenyl | Methyl | (Ethylbutanoat)-3-yl | |
| 2.6.168 | 2-Brom-3-isopropyl-Phenyl | Methyl | (Ethylbutanoat)-3-yl | |
| 2.6.169 | 2-Methyl-3-isopropyl-Phenyl | Methyl | (Ethylbutanoat)-3-yl | |
| 2.6.170 | 2-Ethyl-3-isopropyl-Phenyl | Methyl | (Ethylbutanoat)-3-yl | |
| 2.6.171 | 2-Cyclopropyl-3-isopropyl-Phenyl | Methyl | (Ethylbutanoat)-3-yl | |
| 2.6.172 | 2-Vinyl-3-isopropyl-Phenyl | Methyl | (Ethylbutanoat)-3-yl | |
| 2.6.173 | 2-Ethinyl-3-isopropyl-Phenyl | Methyl | (Ethylbutanoat)-3-yl | |
| 2.6.174 | 2-Cyano-3-isopropyl-Phenyl | Methyl | (Ethylbutanoat)-3-yl | |
| 2.6.175 | 2-Trifluormethyl-3-isopropyl-Phenyl | Methyl | (Ethylbutanoat)-3-yl | |
| 2.6.176 | 2-Methoxy-3-isopropyl-Phenyl | Methyl | (Ethylbutanoat)-3-yl | |
| 2.6.177 | 2-Ethoxy-3-isopropyl-Phenyl | Methyl | (Ethylbutanoat)-3-yl | |
| 2.6.178 | 2-Trifluormethoxy-3-isopropyl-Phenyl | Methyl | (Ethylbutanoat)-3-yl | |
| 2.6.179 | 2-Nitro-3-isopropyl-Phenyl | Methyl | (Ethylbutanoat)-3-yl | |
| 2.6.180 | 2-Fluor-3-tert.butyl-Phenyl | Methyl | (Ethylbutanoat)-3-yl | |
| 2.6.181 | 2-Chlor-3-tert.butyl-Phenyl | Methyl | (Ethylbutanoat)-3-yl | |
| 2.6.182 | 2-Brom-3-tert.butyl-Phenyl | Methyl | (Ethylbutanoat)-3-yl | |
| 2.6.183 | 2-Methyl-3-tert.butyl-Phenyl | Methyl | (Ethylbutanoat)-3-yl | |
| 2.6.184 | 2-Ethyl-3-tert.butyl-Phenyl | Methyl | (Ethylbutanoat)-3-yl | |
| 2.6.185 | 2-Cyclopropyl-3-tert.butyl-Phenyl | Methyl | (Ethylbutanoat)-3-yl | |
| 2.6.186 | 2-Vinyl-3-tert.butyl-Phenyl | Methyl | (Ethylbutanoat)-3-yl | |
| 2.6.187 | 2-Ethinyl-3-tert.butyl-Phenyl | Methyl | (Ethylbutanoat)-3-yl | |
| 2.6.188 | 2-Cyano-3-tert.butyl-Phenyl | Methyl | (Ethylbutanoat)-3-yl | |
| 2.6.189 | 2-Trifluormethyl-3-tert.butyl-Phenyl | Methyl | (Ethylbutanoat)-3-yl | |
| 2.6.190 | 2-Methoxy-3-tert.butyl-Phenyl | Methyl | (Ethylbutanoat)-3-yl | |
| 2.6.191 | 2-Ethoxy-3-tert.butyl-Phenyl | Methyl | (Ethylbutanoat)-3-yl | |
| 2.6.192 | 2-Trifluormethoxy-3-tert.butyl-Phenyl | Methyl | (Ethylbutanoat)-3-yl | |
| 2.6.193 | 2-Nitro-3-tert.butyl-Phenyl | Methyl | (Ethylbutanoat)-3-yl | |
| 2.6.194 | 2-Fluor-3-hydroxymethyl-Phenyl | Methyl | (Ethylbutanoat)-3-yl | |
| 2.6.195 | 2-Chlor-3-hydroxymethyl-Phenyl | Methyl | (Ethylbutanoat)-3-yl | |
| 2.6.196 | 2-Brom-3-hydroxymethyl -Phenyl | Methyl | (Ethylbutanoat)-3-yl | |
| 2.6.197 | 2-Methyl-3-hydroxymethyl -Phenyl | Methyl | (Ethylbutanoat)-3-yl | |
| 2.6.198 | 2-Ethyl-3-hydroxymethyl -Phenyl | Methyl | (Ethylbutanoat)-3-yl | |
| 2.6.199 | 2-Cyclopropyl-3-hydroxymethyl-Phenyl | Methyl | (Ethylbutanoat)-3-yl | |
| 2.6.200 | 2-Vinyl-3-hydroxymethyl -Phenyl | Methyl | (Ethylbutanoat)-3-yl | |
| 2.6.201 | 2-Ethinyl-3-hydroxymethyl-Phenyl | Methyl | (Ethylbutanoat)-3-yl | |
| 2.6.202 | 2-Cyano-3-hydroxymethyl -Phenyl | Methyl | (Ethylbutanoat)-3-yl | |
| 2.6.203 | 2-Trifluormethyl-3-hydroxymethyl-Phenyl | Methyl | (Ethylbutanoat)-3-yl | |
| 2.6.204 | 2-Methoxy-3-hydroxymethyl -Phenyl | Methyl | (Ethylbutanoat)-3-yl | |
| 2.6.205 | 2-Ethoxy-3-hydroxymethyl -Phenyl | Methyl | (Ethylbutanoat)-3-yl | |
| 2.6.206 | 2-Trifluormethoxy-3-hydroxymethyl-Phenyl | Methyl | (Ethylbutanoat)-3-yl | |
| 2.6.207 | 2-Nitro-3-hydroxymethyl-Phenyl | Methyl | (Ethylbutanoat)-3-yl | |
| 2.6.208 | 2-Fluor-3-cyclopropyl-Phenyl | Methyl | (Ethylbutanoat)-3-yl | |
| 2.6.209 | 2-Chlor-3-cyclopropyl-Phenyl | Methyl | (Ethylbutanoat)-3-yl | |
| 2.6.210 | 2-Brom-3-cyclopropyl-Phenyl | Methyl | (Ethylbutanoat)-3-yl | |
| 2.6.211 | 2-Methyl-3-cyclopropyl-Phenyl | Methyl | (Ethylbutanoat)-3-yl | |
| 2.6.212 | 2-Ethyl-3-cyclopropyl-Phenyl | Methyl | (Ethylbutanoat)-3-yl | |
| 2.6.213 | 2-Cyclopropyl-3-cyclopropyl-Phenyl | Methyl | (Ethylbutanoat)-3-yl | |
| 2.6.214 | 2-Vinyl-3-cyclopropyl-Phenyl | Methyl | (Ethylbutanoat)-3-yl | |
| 2.6.215 | 2-Ethinyl-3-cyclopropyl-Phenyl | Methyl | (Ethylbutanoat)-3-yl | |
| 2.6.216 | 2-Cyano-3-cyclopropyl-Phenyl | Methyl | (Ethylbutanoat)-3-yl | |
| 2.6.217 | 2-Trifluormethyl-3-cyclopropyl-Phenyl | Methyl | (Ethylbutanoat)-3-yl | |
| 2.6.218 | 2-Methoxy-3-cyclopropyl-Phenyl | Methyl | (Ethylbutanoat)-3-yl | |
| 2.6.219 | 2-Ethoxy-3-cyclopropyl-Phenyl | Methyl | (Ethylbutanoat)-3-yl | |
| 2.6.220 | 2-Trifluormethoxy-3-cyclopropyl-Phenyl | Methyl | (Ethylbutanoat)-3-yl | |
| 2.6.221 | 2-Fluor-3-methoxycarbonyl-Phenyl | Methyl | (Ethylbutanoat)-3-yl | |
| 2.6.222 | 2-Chlor-3-methoxycarbonyl-Phenyl | Methyl | (Ethylbutanoat)-3-yl | |
| 2.6.223 | 2-Brom-3-methoxycarbonyl-Phenyl | Methyl | (Ethylbutanoat)-3-yl | |
| 2.6.224 | 2-Methyl-3-methoxycarbonyl-Phenyl | Methyl | (Ethylbutanoat)-3-yl | |
| 2.6.225 | 2-Ethyl-3-methoxycarbonyl-Phenyl | Methyl | (Ethylbutanoat)-3-yl | |
| 2.6.226 | 2-Cyclopropyl-3-methoxycarbonyl-Phenyl | Methyl | (Ethylbutanoat)-3-yl | |
| 2.6.227 | 2-Vinyl-3-methoxycarbonyl-Phenyl | Methyl | (Ethylbutanoat)-3-yl | |
| 2.6.228 | 2-Ethinyl-3-methoxycarbonyl-Phenyl | Methyl | (Ethylbutanoat)-3-yl | |
| 2.6.229 | 2-Cyano-3-methoxycarbonyl-Phenyl | Methyl | (Ethylbutanoat)-3-yl | |
| 2.6.230 | 2-Trifluormethylmethoxycarbonyl-Phenyl | Methyl | (Ethylbutanoat)-3-yl | |
| 2.6.231 | 2-Methoxy-3-methoxycarbonyl -Phenyl | Methyl | (Ethylbutanoat)-3-yl | |
| 2.6.232 | 2-Ethoxy-3-methoxycarbonyl-Phenyl | Methyl | (Ethylbutanoat)-3-yl | |
| 2.6.233 | 2-Trifluormethoxymethoxycarbonyl-Phenyl | Methyl | (Ethylbutanoat)-3-yl | |
| 2.6.234 | 2-Nitro-3-methoxycarbonyl-Phenyl | Methyl | (Ethylbutanoat)-3-yl | |
| 2.6.235 | 2-Fluor-3-vinyl-Phenyl | Methyl | (Ethylbutanoat)-3-yl | |
| 2.6.236 | 2-Chlor-3-vinyl-Phenyl | Methyl | (Ethylbutanoat)-3-yl | |
| 2.6.237 | 2-Brom-3-vinyl-Phenyl | Methyl | (Ethylbutanoat)-3-yl | |
| 2.6.238 | 2-Methyl-3-vinyl-Phenyl | Methyl | (Ethylbutanoat)-3-yl | |
| 2.6.239 | 2-Ethyl-3-vinyl-Phenyl | Methyl | (Ethylbutanoat)-3-yl | |
| 2.6.240 | 2-Cyclopropyl-3-vinyl-Phenyl | Methyl | (Ethylbutanoat)-3-yl | |
| 2.6.241 | 2-Vinyl-3-vinyl-Phenyl | Methyl | (Ethylbutanoat)-3-yl | |
| 2.6.242 | 2-Ethinyl-3-vinyl-Phenyl | Methyl | (Ethylbutanoat)-3-yl | |
| 2.6.243 | 2-Cyano-3-vinyl-Phenyl | Methyl | (Ethylbutanoat)-3-yl | |
| 2.6.244 | 2-Trifluormethyl-3-vinyl-Phenyl | Methyl | (Ethylbutanoat)-3-yl | |
| 2.6.245 | 2-Methoxy-3-vinyl -Phenyl | Methyl | (Ethylbutanoat)-3-yl | |
| 2.6.246 | 2-Ethoxy-3-vinyl-Phenyl | Methyl | (Ethylbutanoat)-3-yl | |
| 2.6.247 | 2-Trifluormethoxy-3-vinyl-Phenyl | Methyl | (Ethylbutanoat)-3-yl | |
| 2.6.248 | 2-Nitro-3-vinyl-Phenyl | Methyl | (Ethylbutanoat)-3-yl | |
| 2.6.249 | 2-Fluor-3-ethinyl-Phenyl | Methyl | (Ethylbutanoat)-3-yl | |
| 2.6.250 | 2-Chlor-3-ethinyl-Phenyl | Methyl | (Ethylbutanoat)-3-yl | |
| 2.6.251 | 2-Brom-3-ethinyl - Phenyl | Methyl | (Ethylbutanoat)-3-yl | |
| 2.6.252 | 2-Methyl-3-ethinyl -Phenyl | Methyl | (Ethylbutanoat)-3-yl | |
| 2.6.253 | 2-Ethyl-3-ethinyl-Phenyl | Methyl | (Ethylbutanoat)-3-yl | |
| 2.6.254 | 2-Cyclopropyl-3-ethinyl-Phenyl | Methyl | (Ethylbutanoat)-3-yl | |
| 2.6.255 | 2-Vinyl-3-ethinyl-Phenyl | Methyl | (Ethylbutanoat)-3-yl | |
| 2.6.256 | 2-Cyano-3-ethinyl-Phenyl | Methyl | (Ethylbutanoat)-3-yl | |
| 2.6.257 | 2-Trifluormethyl-3-ethinyl-Phenyl | Methyl | (Ethylbutanoat)-3-yl | |
| 2.6.258 | 2-Methoxy-3-ethinyl-Phenyl | Methyl | (Ethylbutanoat)-3-yl | |
| 2.6.259 | 2-Ethoxy-3-ethinyl-Phenyl | Methyl | (Ethylbutanoat)-3-yl | |
| 2.6.260 | 2-Trifluormethoxy-3-ethinyl--Phenyl | Methyl | (Ethylbutanoat)-3-yl | |
| 2.6.261 | 2-Nitro-3-ethinyl-Phenyl | Methyl | (Ethylbutanoat)-3-yl | |
| 2.6.262 | 2-Fluor-3-ethinyl-Phenyl | Methyl | (Ethylbutanoat)-3-yl | |
| 2.6.263 | 2-Fluor-3-cyano-Phenyl | Methyl | (Ethylbutanoat)-3-yl | |
| 2.6.264 | 2-Chlor-3-cyano-Phenyl | Methyl | (Ethylbutanoat)-3-yl | |
| 2.6.265 | 2-Brom-3-cyano-Phenyl | Methyl | (Ethylbutanoat)-3-yl | |
| 2.6.266 | 2-Methyl-3-cyano-Phenyl | Methyl | (Ethylbutanoat)-3-yl | |
| 2.6.267 | 2-Ethyl-3-cyano-Phenyl | Methyl | (Ethylbutanoat)-3-yl | |
| 2.6.268 | 2-Ethyl-3-cyano-Phenyl | Ethyl | (Ethylbutanoat)-3-yl | |
| 2.6.269 | 2-Ethyl-3-cyano-Phenyl | Propyl | (Ethylbutanoat)-3-yl | |
| 2.6.270 | 2-Ethyl-3-cyano-Phenyl | Cyclopropy I | (Ethylbutanoat)-3-yl | |
| 2.6.271 | 2-Cyclopropyl-3-cyano-Phenyl | Methyl | (Ethylbutanoat)-3-yl | |
| 2.6.272 | 2-Vinyl-3-cyano-Phenyl | Methyl | (Ethylbutanoat)-3-yl | |
| 2.6.273 | 2-Ethinyl-3-cyano-Phenyl | Methyl | (Ethylbutanoat)-3-yl | |
| 2.6.274 | 2-Cyano-3-cyanoPhenyl | Methyl | (Ethylbutanoat)-3-yl | |
| 2.6.275 | 2-Trifluormethyl-3-cyano-Phenyl | Methyl | (Ethylbutanoat)-3-yl | |
| 2.6.276 | 2-Methoxy-3-cyano -Phenyl | Methyl | (Ethylbutanoat)-3-yl | |
| 2.6.277 | 2-Ethoxy-3-cyano-Phenyl | Methyl | (Ethylbutanoat)-3-yl | |
| 2.6.278 | 2-Trifluormethoxy-3-cyano-Phenyl | Methyl | (Ethylbutanoat)-3-yl | |
| 2.6.279 | 2-Nitro-3-cyano-Phenyl | Methyl | (Ethylbutanoat)-3-yl | |
| 2.6.280 | 2-Fluor-3-hyd roxy-Phenyl | Methyl | (Ethylbutanoat)-3-yl | |
| 2.6.281 | 2-Chlor-3-hyd roxy-Phenyl | Methyl | (Ethylbutanoat)-3-yl | |
| 2.6.282 | 2-Brom-3-hyd roxy-Phenyl | Methyl | (Ethylbutanoat)-3-yl | |
| 2.6.283 | 2-Methyl-3-hyd roxy-Phenyl | Methyl | (Ethylbutanoat)-3-yl | |
| 2.6.284 | 2-Ethyl-3-hyd roxy-Phenyl | Methyl | (Ethylbutanoat)-3-yl | |
| 2.6.285 | 2-Cyclopropyl-3-hyd roxy-Phenyl | Methyl | (Ethylbutanoat)-3-yl | |
| 2.6.286 | 2-Vinyl-3-hydroxy-Phenyl | Methyl | (Ethylbutanoat)-3-yl | |
| 2.6.287 | 2-Ethinyl-3-hyd roxy-Phenyl | Methyl | (Ethylbutanoat)-3-yl | |
| 2.6.288 | 2-Cyano-3-hyd roxy-Phenyl | Methyl | (Ethylbutanoat)-3-yl | |
| 2.6.289 | 2-Trifluormethyl-3-hydroxy-Phenyl | Methyl | (Ethylbutanoat)-3-yl | |
| 2.6.290 | 2-Methoxy-3-hydroxy -Phenyl | Methyl | (Ethylbutanoat)-3-yl | |
| 2.6.291 | 2-Ethoxy-3-hydroxy-Phenyl | Methyl | (Ethylbutanoat)-3-yl | |
| 2.6.292 | 2-Trifluormethoxy-3-hydroxy-Phenyl | Methyl | (Ethylbutanoat)-3-yl | |
| 2.6.293 | 2-Nitro-3-hydroxy-Phenyl | Methyl | (Ethylbutanoat)-3-yl | |
| 2.6.294 | 2-Fluor-3-methoxy-Phenyl | Methyl | (Ethylbutanoat)-3-yl | |
| 2.6.295 | 2-Chlor-3-methoxy-Phenyl | Methyl | (Ethylbutanoat)-3-yl | |
| 2.6.296 | 2-Brom-3-methoxy-Phenyl | Methyl | (Ethylbutanoat)-3-yl | |
| 2.6.297 | 2-Methyl-3-methoxy-Phenyl | Methyl | (Ethylbutanoat)-3-yl | |
| 2.6.298 | 2-Ethyl-3-methoxy-Phenyl | Methyl | (Ethylbutanoat)-3-yl | |
| 2.6.299 | 2-Cyclopropyl-3-methoxy-Phenyl | Methyl | (Ethylbutanoat)-3-yl | |
| 2.6.300 | 2-Vinyl-3-methoxy-Phenyl | Methyl | (Ethylbutanoat)-3-yl | |
| 2.6.301 | 2-Ethinyl-3-methoxy-Phenyl | Methyl | (Ethylbutanoat)-3-yl | |
| 2.6.302 | 2-Cyano-3-methoxy-Phenyl | Methyl | (Ethylbutanoat)-3-yl | |
| 2.6.303 | 2-Trifluormethyl-3-methoxy-Phenyl | Methyl | (Ethylbutanoat)-3-yl | |
| 2.6.304 | 2,3-DimethoxyPhenyl | Methyl | (Ethylbutanoat)-3-yl | |
| 2.6.305 | 2-Ethoxy-3-methoxy-Phenyl | Methyl | (Ethylbutanoat)-3-yl | |
| 2.6.306 | 2-Trifluormethoxy-3-methoxy-Phenyl | Methyl | (Ethylbutanoat)-3-yl | |
| 2.6.307 | 2-Nitro-3-methoxy-Phenyl | Methyl | (Ethylbutanoat)-3-yl | |
| 2.6.308 | 2-Fluor-3-ethoxyPhenyl | Methyl | (Ethylbutanoat)-3-yl | |
| 2.6.309 | 2-Chlor-3-ethoxyPhenyl | Methyl | (Ethylbutanoat)-3-yl | |
| 2.6.310 | 2-Brom-3-ethoxyPhenyl | Methyl | (Ethylbutanoat)-3-yl | |
| 2.6.311 | 2-Methyl-3-ethoxy-Phenyl | Methyl | (Ethylbutanoat)-3-yl | |
| 2.6.312 | 2-Ethyl-3-ethoxyPhenyl | Methyl | (Ethylbutanoat)-3-yl | |
| 2.6.313 | 2-Cyclopropyl-3-ethoxy-Phenyl | Methyl | (Ethylbutanoat)-3-yl | |
| 2.6.314 | 2-Vinyl-3-ethoxyPhenyl | Methyl | (Ethylbutanoat)-3-yl | |
| 2.6.315 | 2-Ethinyl-3-ethoxy-Phenyl | Methyl | (Ethylbutanoat)-3-yl | |
| 2.6.316 | 2-Cyano-3-ethoxy-Phenyl | Methyl | (Ethylbutanoat)-3-yl | |
| 2.6.317 | 2-Trifluormethyl-3-ethoxy-Phenyl | Methyl | (Ethylbutanoat)-3-yl | |
| 2.6.318 | 2-Methoxy-3-ethoxy-Phenyl | Methyl | (Ethylbutanoat)-3-yl | |
| 2.6.319 | 2,3-Diethoxy-Phenyl | Methyl | (Ethylbutanoat)-3-yl | |
| 2.6.320 | 2-Trifluormethoxy-3-ethoxy-Phenyl | Methyl | (Ethylbutanoat)-3-yl | |
| 2.6.321 | 2-Nitro-3-ethoxyPhenyl | Methyl | (Ethylbutanoat)-3-yl | |
| 2.6.322 | 2-Fluor-3-propoxy-Phenyl | Methyl | (Ethylbutanoat)-3-yl | |
| 2.6.323 | 2-Chlor-3-propoxy-Phenyl | Methyl | (Ethylbutanoat)-3-yl | |
| 2.6.324 | 2-Brom-3-propoxy-Phenyl | Methyl | (Ethylbutanoat)-3-yl | |
| 2.6.325 | 2-Methyl-3-propoxy-Phenyl | Methyl | (Ethylbutanoat)-3-yl | |
| 2.6.326 | 2-Ethyl-3-propoxy-Phenyl | Methyl | (Ethylbutanoat)-3-yl | |
| 2.6.327 | 2-Cyclopropyl-3-propoxy-Phenyl | Methyl | (Ethylbutanoat)-3-yl | |
| 2.6.328 | 2-Vinyl-3-propoxy-Phenyl | Methyl | (Ethylbutanoat)-3-yl | |
| 2.6.329 | 2-Ethinyl-3-propoxy-Phenyl | Methyl | (Ethylbutanoat)-3-yl | |
| 2.6.330 | 2-Cyano-3-propoxy-Phenyl | Methyl | (Ethylbutanoat)-3-yl | |
| 2.6.331 | 2-Trifluormethyl-3-propoxy-Phenyl | Methyl | (Ethylbutanoat)-3-yl | |
| 2.6.332 | 2-Methoxy-3-propoxy-Phenyl | Methyl | (Ethylbutanoat)-3-yl | |
| 2.6.333 | 2-Ethoxy-3-propoxy-Phenyl | Methyl | (Ethylbutanoat)-3-yl | |
| 2.6.334 | 2-Trifluormethoxy-3-propoxy-Phenyl | Methyl | (Ethylbutanoat)-3-yl | |
| 2.6.335 | 2-Nitro-3-propoxy-Phenyl | Methyl | (Ethylbutanoat)-3-yl | |
| 2.6.336 | 2-Fluor-3-isopropoxy-Phenyl | Methyl | (Ethylbutanoat)-3-yl | |
| 2.6.337 | 2-Chlor-3-isopropoxy-Phenyl | Methyl | (Ethylbutanoat)-3-yl | |
| 2.6.338 | 2-Brom-3-isopropoxy-Phenyl | Methyl | (Ethylbutanoat)-3-yl | |
| 2.6.339 | 2-Methyl-3-isopropoxy-Phenyl | Methyl | (Ethylbutanoat)-3-yl | |
| 2.6.340 | 2-Ethyl-3-isopropoxy-Phenyl | Methyl | (Ethylbutanoat)-3-yl | |
| 2.6.341 | 2-Cyclopropyl-3-isopropoxy-Phenyl | Methyl | (Ethylbutanoat)-3-yl | |
| 2.6.342 | 2-Vinyl-3-isopropoxy-Phenyl | Methyl | (Ethylbutanoat)-3-yl | |
| 2.6.343 | 2-Ethinyl-3-isopropoxy-Phenyl | Methyl | (Ethylbutanoat)-3-yl | |
| 2.6.344 | 2-Cyano-3-isopropoxy-Phenyl | Methyl | (Ethylbutanoat)-3-yl | |
| 2.6.345 | 2-Trifluormethyl-3-isopropoxy-Phenyl | Methyl | (Ethylbutanoat)-3-yl | |
| 2.6.346 | 2-Methoxy-3-isopropoxy-Phenyl | Methyl | (Ethylbutanoat)-3-yl | |
| 2.6.347 | 2-Ethoxy-3-isopropoxy-Phenyl | Methyl | (Ethylbutanoat)-3-yl | |
| 2.6.348 | 2-Trifluormethoxy-3-isopropoxy-Phenyl | Methyl | (Ethylbutanoat)-3-yl | |
| 2.6.349 | 2-Nitro-3-isopropoxy-Phenyl | Methyl | (Ethylbutanoat)-3-yl | |
| 2.6.350 | 2-Fluor-3-tert.butoxy-Phenyl | Methyl | (Ethylbutanoat)-3-yl | |
| 2.6.351 | 2-Chlor-3-tert.butoxy-Phenyl | Methyl | (Ethylbutanoat)-3-yl | |
| 2.6.352 | 2-Brom-3-tert.butoxy-Phenyl | Methyl | (Ethylbutanoat)-3-yl | |
| 2.6.353 | 2-Methyl-3-tert.butoxy-Phenyl | Methyl | (Ethylbutanoat)-3-yl | |
| 2.6.354 | 2-Ethyl-3-tert.butoxy-Phenyl | Methyl | (Ethylbutanoat)-3-yl | |
| 2.6.355 | 2-Cyclopropyl-3-tert.butoxy-Phenyl | Methyl | (Ethylbutanoat)-3-yl | |
| 2.6.356 | 2-Vinyl-3-tert.butoxy-Phenyl | Methyl | (Ethylbutanoat)-3-yl | |
| 2.6.357 | 2-Ethinyl-3-tert.butoxy-Phenyl | Methyl | (Ethylbutanoat)-3-yl | |
| 2.6.358 | 2-Cyano-3-tert.butoxy-Phenyl | Methyl | (Ethylbutanoat)-3-yl | |
| 2.6.359 | 2-Trifluormethyl-3-tert.butoxyPhenyl | Methyl | (Ethylbutanoat)-3-yl | |
| 2.6.360 | 2-Methoxy-3-tert.butoxy-Phenyl | Methyl | (Ethylbutanoat)-3-yl | |
| 2.6.361 | 2-Ethoxy-3-tert.butoxy-Phenyl | Methyl | (Ethylbutanoat)-3-yl | |
| 2.6.362 | 2-Trifluormethoxy-3-tert.butoxyPhenyl | Methyl | (Ethylbutanoat)-3-yl | |
| 2.6.363 | 2-Nitro-3-tert.butoxy-Phenyl | Methyl | (Ethylbutanoat)-3-yl | |
| 2.6.364 | 2-Fluor-3-trifluormethoxy-Phenyl | Methyl | (Ethylbutanoat)-3-yl | |
| 2.6.365 | 2-Chlor-3-trifluormethoxy-Phenyl | Methyl | (Ethylbutanoat)-3-yl | |
| 2.6.366 | 2-Brom-3-trifluormethoxy-Phenyl | Methyl | (Ethylbutanoat)-3-yl | |
| 2.6.367 | 2-Methyl-3-trifluormethoxy-Phenyl | Methyl | (Ethylbutanoat)-3-yl | |
| 2.6.368 | 2-Ethyl-3-trifluormethoxy-Phenyl | Methyl | (Ethylbutanoat)-3-yl | |
| 2.6.369 | 2-Cyclopropyl-3-trifluormethoxy-Phenyl | Methyl | (Ethylbutanoat)-3-yl | |
| 2.6.370 | 2-Vinyl-3-trifluormethoxy-Phenyl | Methyl | (Ethylbutanoat)-3-yl | |
| 2.6.371 | 2-Ethinyl-3-trifluormethoxy-Phenyl | Methyl | (Ethylbutanoat)-3-yl | |
| 2.6.372 | 2-Cyano-3-trifluormethoxy-Phenyl | Methyl | (Ethylbutanoat)-3-yl | |
| 2.6.373 | 2-Trifluormethyl-3-trifluormethoxy-Phenyl | Methyl | (Ethylbutanoat)-3-yl | |
| 2.6.374 | 2-Methoxy-3-trifluormethoxy-Phenyl | Methyl | (Ethylbutanoat)-3-yl | |
| 2.6.375 | 2-Ethoxy-3-trifluormethoxy-Phenyl | Methyl | (Ethylbutanoat)-3-yl | |
| 2.6.376 | 2,3-Bis(trifluormethox y)-Phenyl | Methyl | (Ethylbutanoat)-3-yl | |
| 2.6.377 | 2-Nitro-3-trifluormethoxy-Phenyl | Methyl | (Ethylbutanoat)-3-yl | |
| 2.6.378 | 2-Fluor-3-(2,2,2-trifluorethoxy)-Phenyl | Methyl | (Ethylbutanoat)-3-yl | |
| 2.6.379 | 2-Chlor-3-(2,2,2-trifluorethoxy)-Phenyl | Methyl | (Ethylbutanoat)-3-yl | |
| 2.6.380 | 2-Brom-3-(2,2,2-trifluorethoxy)-Phenyl | Methyl | (Ethylbutanoat)-3-yl | |
| 2.6.381 | 2-Methyl-3-(2,2,2-trifluorethoxy)-Phenyl | Methyl | (Ethylbutanoat)-3-yl | |
| 2.6.382 | 2-Ethyl-3-(2,2,2-trifluorethoxy)-Phenyl | Methyl | (Ethylbutanoat)-3-yl | |
| 2.6.383 | 2-Cyclopropyl-3-trifluorethoxy)-Phenyl | Methyl | (Ethylbutanoat)-3-yl | |
| 2.6.384 | 2-Vinyl-3-(2,2,2-trifluorethoxy)-Phenyl | Methyl | (Ethylbutanoat)-3-yl | |
| 2.6.385 | 2-Ethinyl-3-(2,2,2-trifluorethoxy-Phenyl | Methyl | (Ethylbutanoat)-3-yl | |
| 2.6.386 | 2-Cyano-3-(2,2,2-trifluorethoxy)-Phenyl | Methyl | (Ethylbutanoat)-3-yl | |
| 2.6.387 | 2-Trifluormethyl-3-(2,2,2-trifluorethoxy)-Phenyl | Methyl | (Ethylbutanoat)-3-yl | |
| 2.6.388 | 2-Methoxy-3-(2,2,2-trifluorethoxy)-Phenyl | Methyl | (Ethylbutanoat)-3-yl | |
| 2.6.389 | 2-Ethoxy-3-(2,2,2-trifluorethoxy)-Phenyl | Methyl | (Ethylbutanoat)-3-yl | |
| 2.6.390 | 2-Trifluormethoxy-3-(2,2,2-trifluorethoxy)-Phenyl | Methyl | (Ethylbutanoat)-3-yl | |
| 2.6.391 | 2-Nitro-3-(2,2,2-trifluorethoxy)-Phenyl | Methyl | (Ethylbutanoat)-3-yl | |
| 2.6.392 | 2-Fluor-3-difluormethoxy-Phenyl | Methyl | (Ethylbutanoat)-3-yl | |
| 2.6.393 | 2-Chlor-3-difluormethoxy-Phenyl | Methyl | (Ethylbutanoat)-3-yl | |
| 2.6.394 | 2-Brom-3-difluormethoxy-Phenyl | Methyl | (Ethylbutanoat)-3-yl | |
| 2.6.395 | 2-Methyl-3-difluormethoxy-Phenyl | Methyl | (Ethylbutanoat)-3-yl | |
| 2.6.396 | 2-Ethyl-3-difluormethoxy-Phenyl | Methyl | (Ethylbutanoat)-3-yl | |
| 2.6.397 | 2-Cyclopropyl-3-difluormethoxy-Phenyl | Methyl | (Ethylbutanoat)-3-yl | |
| 2.6.398 | 2-Vinyl-3-difluormethoxy-Phenyl | Methyl | (Ethylbutanoat)-3-yl | |
| 2.6.399 | 2-Ethinyl-3-difluormethoxy-Phenyl | Methyl | (Ethylbutanoat)-3-yl | |
| 2.6.400 | 2-Cyano-3-difluormethoxy - Phenyl | Methyl | (Ethylbutanoat)-3-yl | |
| 2.6.401 | 2-Trifluormethyl-3-difluormethoxy-Phenyl | Methyl | (Ethylbutanoat)-3-yl | |
| 2.6.402 | 2-Methoxy-3-difluormethoxy - Phenyl | Methyl | (Ethylbutanoat)-3-yl | |
| 2.6.403 | 2-Ethoxy-3-difluormethoxy-Phenyl | Methyl | (Ethylbutanoat)-3-yl | |
| 2.6.404 | 2-Trifluormethoxy-3-difluormethoxy-Phenyl | Methyl | (Ethylbutanoat)-3-yl | |
| 2.6.405 | 2-Nitro-3-difluormethoxy-Phenyl | Methyl | (Ethylbutanoat)-3-yl | |
| 2.6.406 | 2-Fluor-3-(2-methoxyethoxy)-Phenyl | Methyl | (Ethylbutanoat)-3-yl | |
| 2.6.407 | 2-Chlor-3-(2-methoxyethoxy)-Phenyl | Methyl | (Ethylbutanoat)-3-yl | |
| 2.6.408 | 2-Brom-3-(2-methoxyethoxy)-Phenyl | Methyl | (Ethylbutanoat)-3-yl | |
| 2.6.409 | 2-Methyl-3-(2-methoxyethoxy)-Phenyl | Methyl | (Ethylbutanoat)-3-yl | |
| 2.6.410 | 2-Ethyl-3-(2-methoxyethoxy)-Phenyl | Methyl | (Ethylbutanoat)-3-yl | |
| 2.6.411 | 2-Cyclopropyl-3-(2-methoxyethoxy)-Phenyl | Methyl | (Ethylbutanoat)-3-yl | |
| 2.6.412 | 2-Vinyl-3-(2-methoxyethoxy)-Phenyl | Methyl | (Ethylbutanoat)-3-yl | |
| 2.6.413 | 2-Ethinyl-3-(2-methoxyethoxy)-Phenyl | Methyl | (Ethylbutanoat)-3-yl | |
| 2.6.414 | 2-Cyano-3-(2-methoxyethoxy)-Phenyl | Methyl | (Ethylbutanoat)-3-yl | |
| 2.6.415 | 2-Trifluormethyl-3-(2-methoxyethoxy)-Phenyl | Methyl | (Ethylbutanoat)-3-yl | |
| 2.6.416 | 2-Methoxy-3-(2-methoxyethoxy)-Phenyl | Methyl | (Ethylbutanoat)-3-yl | |
| 2.6.417 | 2-Ethoxy-3-(2-methoxyethoxy)-Phenyl | Methyl | (Ethylbutanoat)-3-yl | |
| 2.6.418 | 2-Trifluormethoxy-(2-methoxyethoxy)-Phenyl | Methyl | (Ethylbutanoat)-3-yl | |
| 2.6.419 | 2-Nitro-3-(2-methoxyethoxy)-Phenyl | Methyl | (Ethylbutanoat)-3-yl | |
| 2.6.420 | 2-Fluor-3-(tert.butoxycarbon yloxy)-Phenyl | Methyl | (Ethylbutanoat)-3-yl | |
| 2.6.421 | 2-Chlor-3-(tert.butoxycarbon yloxy)-Phenyl | Methyl | (Ethylbutanoat)-3-yl | |
| 2.6.422 | 2-Brom-3-(tert.butoxycarbon yloxy)-Phenyl | Methyl | (Ethylbutanoat)-3-yl | |
| 2.6.423 | 2-Methyl-3-(tert.butoxycarbon yloxy)-Phenyl | Methyl | (Ethylbutanoat)-3-yl | |
| 2.6.424 | 2-Ethyl-3-(tert.butoxycarbon yloxy)-Phenyl | Methyl | (Ethylbutanoat)-3-yl | |
| 2.6.425 | 2-Cyclopropyl-3-(tert. butoxycarbon yloxy)-Phenyl | Methyl | (Ethylbutanoat)-3-yl | |
| 2.6.426 | 2-Vinyl-3-(tert.butoxycarbon yloxy)-Phenyl | Methyl | (Ethylbutanoat)-3-yl | |
| 2.6.427 | 2-Ethinyl-3-(tert. butoxycarbon yloxy)-Phenyl | Methyl | (Ethylbutanoat)-3-yl | |
| 2.6.428 | 2-Cyano-3-(tert. butoxycarbon yloxy)-Phenyl | Methyl | (Ethylbutanoat)-3-yl | |
| 2.6.429 | 2-Trifluormethyl-(tert.butoxycarbon yloxy)-Phenyl | Methyl | (Ethylbutanoat)-3-yl | |
| 2.6.430 | 2-Methoxy-3-(tert.butoxycarbon yloxy)-Phenyl | Methyl | (Ethylbutanoat)-3-yl | |
| 2.6.431 | 2-Ethoxy-3-(tert.butoxycarbon yloxy)-Phenyl | Methyl | (Ethylbutanoat)-3-yl | |
| 2.6.432 | 2-Trifluormethoxy-(tert.butoxycarbon yloxy)-Phenyl | Methyl | (Ethylbutanoat)-3-yl | |
| 2.6.433 | 2-Nitro-3-(tert. butoxycarbon yloxy)-Phenyl | Methyl | (Ethylbutanoat)-3-yl | |
| 2.6.434 | 2-Fluor-3-nitroPhenyl | Methyl | (Ethylbutanoat)-3-yl | |
| 2.6.435 | 2-Chlor-3-nitroPhenyl | Methyl | (Ethylbutanoat)-3-yl | |
| 2.6.436 | 2-Brom-3-nitroPhenyl | Methyl | (Ethylbutanoat)-3-yl | |
| 2.6.437 | 2-Methyl-3-nitroPhenyl | Methyl | (Ethylbutanoat)-3-yl | |
| 2.6.438 | 2-Ethyl-3-nitroPhenyl | Methyl | (Ethylbutanoat)-3-yl | |
| 2.6.439 | 2-Cyclopropyl-3-nitro-Phenyl | Methyl | (Ethylbutanoat)-3-yl | |
| 2.6.440 | 2-Vinyl-3-nitroPhenyl | Methyl | (Ethylbutanoat)-3-yl | |
| 2.6.441 | 2-Ethinyl-3-nitroPhenyl | Methyl | (Ethylbutanoat)-3-yl | |
| 2.6.442 | 2-Cyano-3-nitroPhenyl | Methyl | (Ethylbutanoat)-3-yl | |
| 2.6.443 | 2-Trifluormethyl-3-nitro-Phenyl | Methyl | (Ethylbutanoat)-3-yl | |
| 2.6.444 | 2-Methoxy-3-nitro-Phenyl | Methyl | (Ethylbutanoat)-3-yl | |
| 2.6.445 | 2-Ethoxy-3-nitroPhenyl | Methyl | (Ethylbutanoat)-3-yl | |
| 2.6.446 | 2-Trifluormethoxy-3-nitro-Phenyl | Methyl | (Ethylbutanoat)-3-yl | |
| 2.6.447 | 2-Fluor-3-methylsulfanyl-Phenyl | Methyl | (Ethylbutanoat)-3-yl | |
| 2.6.448 | 2-Chlor-3-methylsulfanyl-Phenyl | Methyl | (Ethylbutanoat)-3-yl | |
| 2.6.449 | 2-Brom-3-methylsulfanyl-Phenyl | Methyl | (Ethylbutanoat)-3-yl | |
| 2.6.450 | 2-Methyl-3-methylsulfanyl-Phenyl | Methyl | (Ethylbutanoat)-3-yl | |
| 2.6.451 | 2-Ethyl-3-methylsulfanyl-Phenyl | Methyl | (Ethylbutanoat)-3-yl | |
| 2.6.452 | 2-Cyclopropyl-3-methylssulfanyl-Phenyl | Methyl | (Ethylbutanoat)-3-yl | |
| 2.6.453 | 2-Vinyl-3-methylsulfanyl-Phenyl | Methyl | (Ethylbutanoat)-3-yl | |
| 2.6.454 | 2-Ethinyl-3-methylsulfanyl-Phenyl | Methyl | (Ethylbutanoat)-3-yl | |
| 2.6.455 | 2-Cyano-3-methylsulfanyl- Phenyl | Methyl | (Ethylbutanoat)-3-yl | |
| 2.6.456 | 2-Trifluormethyl-3-methylsulfanyl- Phenyl | Methyl | (Ethylbutanoat)-3-yl | |
| 2.6.457 | 2-Methoxy-3-methylsulfanyl- Phenyl | Methyl | (Ethylbutanoat)-3-yl | |
| 2.6.458 | 2-Ethoxy-3-methylsulfanyl-Phenyl | Methyl | (Ethylbutanoat)-3-yl | |
| 2.6.459 | 2-Trifluormethoxy-3-methylsulfanyl-Phenyl | Methyl | (Ethylbutanoat)-3-yl | |
| 2.6.460 | 2-Nitro-3-methylsulfanyl-Phenyl | Methyl | (Ethylbutanoat)-3-yl | |
| 2.6.461 | 3,5-Difluor-Phenyl | Methyl | (Cyclohex-2-en-1-on)-3-yl | |
| 2.6.462 | 3,5-Difluor-Phenyl | Methyl | (Propan-1-ol)-3-yl | [CDCl₃] 1.73 (s,3H); 1.94 (quin,2H); 3.15 (d,1H); 3.73 (t,2H); 3.85 (d,1H); 4.38 (t,2H); 6.88 (t,1H); 7.17 (d,2H). |
| 2.6.463 | 3,5-Difluor-Phenyl | Methyl | (2,2-Dimethylpropan-1-ol)-3yl | [CDCl₃] 0.89 (d,6H), 1.74 (s,3H), 3.16 (d,1H); 3.33 (s,2H); 3.84 (d,1H); 4.07 (s,2H), 6.88 (t,1H); 7.18 (d,2H). |
| 2.6.464 | 3,5-Difluor-Phenyl | Methyl | (Methyl-2,2-dimethylpropanoat)-3-yl | [CDCl₃] 1.24 (d,6H); 1.71 (s,3H); 3.14 (d,1H); 3.65 (s,3H); 3.81 (d,1H); 4.22 (s,2H); 6.87 (t,1H); 7.18 (d,2H). |
| 2.6.465 | 3,5-Difluor-Phenyl | Methyl | (Methylpropanoat)-3-yl | [CDCl₃] 1.71 (s,3H), 2.72 (t,2H); 3.14 (d,1H); 3.68 (s,3H); 3.82 (d,1H); 4.47 (m,2H); 6.86 (t,1H); 7.18 (d,2H). |
| 2.6.466 | 3,5-Difluor-Phenyl | Methyl | (Ethylpropanoat)-3-yl | |
| 2.6.467 | 3,5-Difluor-Phenyl | Methyl | (Ethylbutanoat)-3-yl | [CDCl₃] 1.17-1.26 (m,3H); 1.36 (d,3H); 1.69 (d,3H); 2.55 (m,1H); 2.69 (m,1H); 3.11 (dd,1H); 3.81 (dd,1H); 4.03-4.15 (m,2H); 5.35 (m,1H); 6.87 (t,1H); 7.17 (m,2H). |
| 2.6.468 | 3,5-Difluor-Phenyl | Methyl | ( Ethyl-(3R)-4,4,4-trifluorbutanoat)-3-yl ein Diastereomeres | [CDCl₃] 1.20 (t,3H); 1.75 (s,3H); 2.85 (t,2H); 3.19 (d,1H); 3.85 (d,1H); 4.09 (m,2H); 5.82 (m,1H); 6.88 (t,1H); 7.17 (d,2H). |
| 2.6.469 | 3,5-Difluor-Phenyl | Methyl | ( Ethyl-(3R)-4,4,4-trifluorbutanoat)-3-yl das andere Diastereomere | [CDCl₃] 1.25 (t,3H); 1.74 (s,3H); 2.86 (d,2H); 3.20 (d,1H); 3.80 (d,1H); 4.15 (q,2H); 5.80 (m,1H); 6.88 (t,1H); 7.18 (d,2H). |
| 2.6.470 | 3,5-Difluor-Phenyl | Methyl | (Butan-2-on)-4-yl | [CDCl₃]1.70 (s,3H); 2.18 (s,3H); 2.83 (q,2H); 3.15 (d,1H), 3.83 (d,1H), 4.44 (m,2H); 6.87 (t,1H); 7.18 (d,2H). |
| 2.6.471 | 3,5-Difluor-Phenyl | Methyl | (3-Methylbutan-2-on)-4-yl | [CDCl₃] 1.16 (t,3H), 1.70 (d,3H), 2.19 (d,3H); 2.95 (quin,1H), 3.11 (dd,1H); 3.81 (d,1H); 4.21 (dd,1 H); 4.37 (dd,1H); 6.87 (t,1H); 7.18 (m,2H). |
| 2.6.472 | 3,5-Difluor-Phenyl | Methyl | (Pent-3-en-2-on)-4-yl | |
| 2.6.473 | 3,5-Difluor-Phenyl | Methyl | ((2S)-Dimethyl-butandioat)-2yl | [CDCl₃] 1.75 (d,3H); 2.95 (m,2H); 3.18 (dd,1H); 3.68 (d,3H); 3.76 (d,3H); 3.88 (dd,1H); 5.55 (t,1H); 6.87 (t,1H); 7.18 (d,2H). |
| 2.6.474 | 3,5-Difluor-Phenyl | Methyl | (Dimethylpentandioat)-3yl | [CDCl₃] 1.69 (s,3H); 2.75 (t,4H); 3.13 (d,1H); 3.62 (s,3H); 3.69 (s,3H); 3.83 (d,1H); 5.58 (quin,1H); 6.87 (t,1H); 7.18 (d,2H). |
| 2.6.475 | 3,5-Difluor-Phenyl | Methyl | (Methyl-(2R)-2-methylpropanoat)-3-yl | [CDCl₃] D1: 1.22 (t,3H), 1.71 (s,3H); 2.88 (m,1H), 3.15 (d,1H), 3.66 (s,3H); 3.81 (d,1H), 4.29 (m,1H); 4.37 (m,1H), 6.87 (t,1H); 7.17 (d,2H). D2: 1.22 (t,3H), 1.71 (s,3H); 2.88 (m,1H), 3.15 (d,1H), 3.67 (s,3H); 3.83 (d,1H), 4.29 (m,1H); 4.37 (m,1H), 6.87 (t,1H); 7.17 (d,2H). |
| 2.6.476 | 3,5-Difluor-Phenyl | Methyl | 4-Methoxycarbonylbenzyl | [CDCl₃] 1.75 (s,3H); 3.17 (d,1H), 3.84 (d,1H); 3.92 (s,3H); 5.28 (d,2H); 6.88 (t,1H); 7.17 (d,2H); 7.42 (d,2H); 8.04 (d,2H). |
| 2.6.477 | 3,5-Difluor-Phenyl | Methyl | 3,5-Difluorbenzyl | [CDCl₃] 1.76 (s,3H); 3.19 (d,1H); 3.84 (d,1H); 5.19 (d,2H); 6.76 (t,1H); 6.88 (m,3H); 7.17 (d,2H). |
| 2.6.478 | 3,5-Difluor-Phenyl | Methyl | 3,4-Difluorbenzyl | [CDCl₃] 1.73 (s,3H), 3.17 (d,1H), 3.82 (d,1H); 5.18 (d,2H); 6.87 (t,1H); 7.10 (m,1H); 7.15 (m,4H). |
| 2.6.479 | 3,5-Difluor-Phenyl | Methyl | 2,6-Difluorbenzyl | [CDCl₃] 1.72 (s,3H); 3.15 (d,1H); 3.82 (d,1H); 3.33 (d,2H); 6.86 (t, 1H); 6.94 (t,2H); 7.16 (d,2H); 7.35 (m,1H). |
| 2.6.480 | 3,5-Difluor-Phenyl | Methyl | (5-Methylpyridin-3-yl)methyl | [CDCl₃] 1.73 (s,3H), 2.34 (s,3H), 3.17 (d,1H); 3.82 (d,1H); 5.22 (d,2H); 6.88 (t,1H); 7.17 (d,2H), 7.51 (s,1H); 8.43 (s,2H). |
| 2.6.481 | 3,5-Difluor-Phenyl | Methyl | (Tetrahydrofuran)-3yl | [CDCl₃] 1.72 (s,3H); 2.06 (m,1H), 2.22 (m,1H); 3.17 (dd,1H); 3.81-3.99 (m,5H); 5.38 (m,1H); 6.89 (t,1H); 7.17 (d,2H). |
| 2.6.482 | 3,5-Difluor-Phenyl | Ethyl | (Cyclohex-2-en-1-on)-3-yl | |
| 2.6.483 | 3,5-Difluor-Phenyl | Ethyl | (Propan-1-ol)-3-yl | |
| 2.6.484 | 3,5-Difluor-Phenyl | Ethyl | (2,2-Dimethylpropan-1-ol)-3yl | |
| 2.6.485 | 3,5-Difluor-Phenyl | Ethyl | (Methyl-2,2-dimethylpropanoat)-3-yl | |
| 2.6.486 | 3,5-Difluor-Phenyl | Ethyl | (Methylpropanoat)-3-yl | [CDCl₃] 0.99 (t,3H); 2.01-2.07 (m,2H); 2.72 (t,2H), 3.17 (d,1H); 3.68 (s,3H); 3.75 (d,1H); 4.41-4.53 (m,2H); 6.86 (t,1H); 7.18 (d,2H). |
| 2.6.487 | 3,5-Difluor-Phenyl | Ethyl | (Ethylpropanoat)-3-yl | |
| 2.6.488 | 3,5-Difluor-Phenyl | Ethyl | (Ethylbutanoat)-3-yl | [CDCl₃] D1 0.98 (t,3H); 1.27 (t,3H); 1.36 (d,3H); 1.95-2.12 (m,2H); 2.51-2.73 (m,2H); 3.14 (d,1H); 3.78 (d,1H); 4.07-4.18(m,2H); 5.32-5.43 (m,1H); 6.86 (t,1H); 7.13-7.22 (m,2H). D2 1.00 (t,3H); 1.22 (t,3H); 1.35 (d,3H); 1.95-2.12 (m,2H); 2.51-2.73 (m,2H); 3.17 (d,1H); 3.72 (d,1H); 4.00-4.07(m,2H); 5.32-5.43 (m,1H); 6.86 (t,1H); 7.13-7.22 (m,2H). |
| 2.6.489 | 3,5-Difluor-Phenyl | Ethyl | ( Ethyl-(3R)-4,4,4-trifluorbutanoat)-3-yl ein Diastereomeres | [CDCl₃] D1 1.01 (t,3H); 1.19 (t,3H); 2.05-2.11 (m,2H); 2.81-2.91 (m,2H); 3.21 (d,1H); 3.77 (d,1H); 4.04-4.11 (m,2H); 5.84 (m,1H); 6.87 (t,1H); 7.17 (d,2H). |
| 2.6.490 | 3,5-Difluor-Phenyl | Ethyl | ( Ethyl-(3R)-4,4,4-trifluorbutanoat)-3-yl das andere Diastereomere | [CDCl₃] 1.01 (t,3H); 1.24 (t,3H); 2.01-2.13 (m,2H); 2.85 (d,2H); 3.23 (d,1H); 3.72 (d,1H); 4.13 (q,2H); 5.83 (m,1H); 6.87 (t,1H); 7.16 (d,2H). |
| 2.6.491 | 3,5-Difluor-Phenyl | Ethyl | (Butan-2-on)-4-yl | |
| 2.6.492 | 3,5-Difluor-Phenyl | Ethyl | (3-Methylbutan-2-on)-4-yl | |
| 2.6.493 | 3,5-Difluor-Phenyl | Ethyl | (Pent-3-en-2-on)-4-yl | |
| 2.6.494 | 3,5-Difluor-Phenyl | Ethyl | ((2S)-Dimethyl-butandioat)-2-yl | [CDCl₃] D1 1.02-1.07 (m,3H), 2.02-2.15 (m,2H); 2.95 (m,2H); 3.21 (d,1H); 3.69 (s,3H), 3.75 (s,3H), 3.79 (d,1H), 5.58 (t,1H); 6.87 (t,1H); 7.19 (d,2H). D2 1.02-1.07 (m,3H); 2.02-2.15 (m,2H); 2.95 (m,2H); 3.22 (d,1H); 3.68 (s,3H); 3.77 (s,3H), 3.81 (d,1H); 5.58 (t,1H); 6.87 (t,1H); 7.19 (d,2H). |
| 2.6.495 | 3,5-Difluor-Phenyl | Ethyl | (Dimethylpentandioat)-3-yl | [CDCl₃] 0.99 (t,3H); 1.97-2.06 (m,2H); 2.75 (m,4H); 3.16 (d,1H); 3.62 (s,3H); 3.67 (s,3H); 3.75 (d,1H); 5.60 (quin,1H); 6.86 (t,1H); 7.19 (d,2H). |
| 2.6.496 | 3,5-Difluor-Phenyl | Ethyl | (Methyl-(2R)-2-methylpropanoat)-3-yl | |
| 2.6.497 | 3,5-Difluor-Phenyl | Ethyl | 4-Methoxycarbonylbenzyl | |
| 2.6.498 | 3,5-Difluor-Phenyl | Ethyl | 3,5-Difluorbenzyl | |
| 2.6.499 | 3,5-Difluor-Phenyl | Ethyl | 3,4-Difluorbenzyl | |
| 2.6.500 | 3,5-Difluor-Phenyl | Ethyl | 2,6-Difluorbenzyl | |
| 2.6.501 | 3,5-Difluor-Phenyl | Ethyl | (5-Methylpyridin-3-yl)-methyl | |
| 2.6.502 | 3,5-Difluor-Phenyl | Ethyl | (Tetrahydrofuran)-3yl | |
| 2.6.503 | (S)-3,5-Difluor-Phenyl | Methyl | (Ethylbutanoat)-3-yl | |
| 2.6.504 | (R)-3,5-Difluor-Phenyl | Methyl | (Ethylbutanoat)-3-yl | |
| 2.6.505 | 3-Chlor-5-fluor-Phenyl | Methyl | (Ethylbutanoat)-3-yl | |
| 2.6.506 | 3-Brom-5-fluor-Phenyl | Methyl | (Ethylbutanoat)-3-yl | |
| 2.6.507 | 3-Iod-5-fluor-Phenyl | Methyl | (Ethylbutanoat)-3-yl | |
| 2.6.508 | 3-Methyl-5-fluor-Phenyl | Methyl | (Ethylbutanoat)-3-yl | |
| 2.6.509 | 3-Ethyl-5-fluor-Phenyl | Methyl | (Ethylbutanoat)-3-yl | |
| 2.6.510 | 3-Propyl-5-fluor-Phenyl | Methyl | (Ethylbutanoat)-3-yl | |
| 2.6.511 | 3-iPropyl-5-fluor-Phenyl | Methyl | (Ethylbutanoat)-3-yl | |
| 2.6.512 | 3-nButyl-5-fluor-Phenyl | Methyl | (Ethylbutanoat)-3-yl | |
| 2.6.513 | 3-isoButyl-5-fluor-Phenyl | Methyl | (Ethylbutanoat)-3-yl | |
| 2.6.514 | 3-tert.Butyl-5-fluor-Phenyl | Methyl | (Ethylbutanoat)-3-yl | |
| 2.6.515 | 3-Cyclopropyl-5-fluor-Phenyl | Methyl | (Ethylbutanoat)-3-yl | |
| 2.6.516 | 3-Vinyl-5-fluor-Phenyl | Methyl | (Ethylbutanoat)-3-yl | |
| 2.6.517 | 3-Ethinyl-5-fluor-Phenyl | Methyl | (Ethylbutanoat)-3-yl | |
| 2.6.518 | 3-Cyano-5-fluor-Phenyl | Methyl | (Ethylbutanoat)-3-yl | |
| 2.6.519 | 3-Trifluormethyl-5-fluor-Phenyl | Methyl | (Ethylbutanoat)-3-yl | |
| 2.6.520 | 3-(Methoxycarbony) l-5-fluor-Phenyl | Methyl | (Ethylbutanoat)-3-yl | |
| 2.6.521 | 3-Hydroxymethyl-5-fluor-Phenyl | Methyl | (Ethylbutanoat)-3-yl | |
| 2.6.522 | 3-Carbamoyl-5-fluor-Phenyl | Methyl | (Ethylbutanoat)-3-yl | |
| 2.6.523 | 3-Hydroxy-5-fluor-Phenyl- | Methyl | (Ethylbutanoat)-3-yl | |
| 2.6.524 | 3-Methoxy-5-fluor-Phenyl | Methyl | (Ethylbutanoat)-3-yl | |
| 2.6.525 | 3-Ethoxy-5-fluor-Phenyl | Methyl | (Ethylbutanoat)-3-yl | |
| 2.6.526 | 3-nPropyoxy-5-fluor-Phenyl | Methyl | (Ethylbutanoat)-3-yl | |
| 2.6.527 | 3-isoPropoxy-5-fluor-Phenyl | Methyl | (Ethylbutanoat)-3-yl | |
| 2.6.528 | 3-nButoxy-5-fluor-Phenyl | Methyl | (Ethylbutanoat)-3-yl | |
| 2.6.529 | 3-isoButoxy-5-fluor-Phenyl | Methyl | (Ethylbutanoat)-3-yl | |
| 2.6.530 | 3-tert. Butoxy-5-fluor-Phenyl | Methyl | (Ethylbutanoat)-3-yl | |
| 2.6.531 | 3-Difluormethoxy-5-fluor-Phenyl | Methyl | (Ethylbutanoat)-3-yl | |
| 2.6.532 | 3-Trifluormethoxy-5-fluor-Phenyl | Methyl | (Ethylbutanoat)-3-yl | |
| 2.6.533 | 3-(2,2,2-Trifluorethoxy)-5-fluor-Phenyl | Methyl | (Ethylbutanoat)-3-yl | |
| 2.6.534 | 3-(2-Chlorethoxy)-5-fluor-Phenyl | Methyl | (Ethylbutanoat)-3-yl | |
| 2.6.535 | 3-(2-Hydroxyethoxy)-5-fluor-Phenyl- | Methyl | (Ethylbutanoat)-3-yl | |
| 2.6.536 | 3-[(tert-Butoxycarbonyl)oxy]-5-fluor-Phenyl | Methyl | (Ethylbutanoat)-3-yl | |
| 2.6.537 | 3-Nitro-5-fluor-Phenyl | Methyl | (Ethylbutanoat)-3-yl | |
| 2.6.538 | 3-Acetoxy-5-fluor-Phenyl | Methyl | (Ethylbutanoat)-3-yl | |
| 2.6.539 | {3-[(tert-Butoxycarbonyl)a mino]-5-fluor-Phenyl} | Methyl | (Ethylbutanoat)-3-yl | |
| 2.6.540 | 3-Methylsulfanyl-5-fluor-Phenyl | Methyl | (Ethylbutanoat)-3-yl | |
| 2.6.541 | 3,5-Dichlor-Phenyl | Methyl | (Cyclohex-2-en-1-on)-3-yl | [CDCl₃] 1.79 (s,3H); 2.08 (quin,2H); 2.43 (t,2H), 2.58 (t,2H); 3.22 (d,1H); 3.88 (d,1H); 5.97 (s,1H); 7.42 (s,1H); 7.53 (s,2H). |
| 2.6.542 | 3,5-Dichlor-Phenyl | Methyl | (Propan-1-ol)-3-yl | |
| 2.6.543 | 3,5-Dichlor-Phenyl | Methyl | (2,2-Dimethylpropan-1-ol)-3-yl | |
| 2.6.544 | 3,5-Dichlor-Phenyl | Methyl | (Methyl-2,2-dimethylpropanoat)-3-yl | |
| 2.6.545 | 3,5-Dichlor-Phenyl | Methyl | (Methylpropanoat)-3-yl | [CDCl₃] 1.71 (s,3H); 2.72 (t,2H); 3.15 (d,1H); 3.68 (s,3H); 3.83 (d,1H); 4.46 (t,2H); 7.40 (s,1H); 7.53 (s,2H). |
| 2.6.546 | 3,5-Dichlor-Phenyl | Methyl | (Ethylpropanoat)-3-yl | [CDCl₃] 1.23 (t,3H), 1.70 (s,3H); 2.70 (t,2H); 3.15 (d,1H); 3.82 (d,1H); 4.12 (q,2H), 4.47 (t,2H); 7.40 (s,1H); 7.52 (s,2H). |
| 2.6.547 | 3,5-Dichlor-Phenyl | Methyl | (Ethylbutanoat)-3-yl | [CDCl₃] 1.18-1.27 (m,3H); 1.36 (d,3H); 1.68 (d,3H); 2.55 (m,1H); 2.67 (m,1H); 3.12 (dd,1H); 3.81 (dd,1H), 4.03-4.16 (m,2H); 5.36 (m,1H); 7.39 (s,1H); 7.53 (s,2H). |
| 2.6.548 | 3,5-Dichlor-Phenyl | Methyl | ( Ethyl-(3R)-4,4,4-trifluorbutanoat)-3-yl(1 Diastereomeres) | [CDCl₃] (t,3H); 1.75 (s,3H); 2.85 (m,2H); 3.19 (d,1H); 3.84 (d,1H); 4.09 (m,2H); 5.81 (m,1H); 7.41 (s,1H); 7.52 (s,2H). 6-1-1 |
| 2.6.549 | 3,5-Dichlor-Phenyl | Methyl | ( Ethyl-(3R)-4,4,4-trifluorbutanoat)-3-yl (das andere Diastereomere) | [CDCl₃] 1.25 (t,3H); 1.74 (s,3H); 2.86 (d,2H); 3.20 (d,1H); 3.80 (d,1H); 4.16 (q,2H); 5.81 (m,1H); 7.41 (s,1H); 7.53 (s,2H). |
| 2.6.550 | 3,5-Dichlor-Phenyl | Methyl | (Butan-1-ol)-4-yl | [CDCl₃] 1.58 (bs, 1H); 1.65 (m, 2H); 1.72 (s, 3H); 1.80 (mc, 2H); 3.13 (d, 1H); 3.68 (t, 2H); 3.82 (d, 1H); 4.25 (t, 2H); 7.40 (d, 1H); 7.52 (s, 2d). |
| 2.6.551 | 3,5-Dichlor-Phenyl | Methyl | (3-Methylbutan-2-on)-4-yl | |
| 2.6.552 | 3,5-Dichlor-Phenyl | Methyl | (Pent-3-en-2-on)-4-yl (E oder Z) | [CDCl₃] 1.79 (s,3H); 2.21 (s,3H); 2.33 (s,3H); 3.21 (d,1H); 3.89 (d,1H); 6.11 (s,1H); 7.41 (s,1H); 7.55 (s,2H). |
| 2.6.553 | 3,5-Dichlor-Phenyl | Methyl | (Pent-3-en-2-on)-4-yl (Z oder E) | [CDCl₃] 1.80 (s,3H); 2.10 (s,6H); 3.22 (d,1H); 4.20 (d,1H); 5.96 (s,1H); 7.40 (s,1H); 7.58 (s,2H). |
| 2.6.554 | 3,5-Dichlor-Phenyl | Methyl | ((2S)-Dimethyl-butandioat)-2-yl | [CDCl₃] 1.75 (d,3H); 2.96 (m,2H); 3.20 (dd,1H); 3.68 (d,3H); 3.77 (d,3H); 3.90 (dd,1H); 5.57 (t,1H); 7.41 (s,1H); 7.53 (s,2H). |
| 2.6.555 | 3,5-Dichlor-Phenyl | Methyl | (Dimethylpentandioat)-3-yl | [CDCl₃] 1.68 (s,3H); 2.75 (t,4H); 3.11 (d,1H); 3.62 (s,3H); 3.69 (s,3H); 3.81 (d,1H); 5.58 (quin,1H); 7.41 (s,1H), 7.53 (s,2H). |
| 2.6.556 | 3,5-Dichlor-Phenyl | Methyl | (Methyl-(2R)-2-methylpropanoat)-3-yl | |
| 2.6.557 | 3,5-Dichlor-Phenyl | Methyl | 4-Methoxycarbonylbenzyl | |
| 2.6.558 | 3,5-Dichlor-Phenyl | Methyl | 3,5-Difluorbenzyl | |
| 2.6.559 | 3,5-Dichlor-Phenyl | Methyl | 3,4-Difluorbenzyl | |
| 2.6.560 | 3,5-Dichlor-Phenyl | Methyl | 2,6-Difluorbenzyl | |
| 2.6.561 | 3,5-Dichlor-Phenyl | Methyl | (5-Methylpyridin-3-yl)methyl | [CDCl₃] 1.72 (s,3H); 2.34 (s,3H); 3.17 (d,1H); 3.82 (d,1H); 5.22 (d,2H); 7.41 (s,1H); 7.51 (m,3H); 8.43 (s,2H). |
| 2.6.562 | 3,5-Dichlor-Phenyl | Methyl | (Tetrahydrofuran)-3yl | |
| 2.6.563 | 3,5-Dichlor-Phenyl | Ethyl | (Cyclohex-2-en-1-on)-3-yl | |
| 2.6.564 | 3,5-Dichlor-Phenyl | Ethyl | (Propan-1-ol)-3-yl | |
| 2.6.565 | 3,5-Dichlor-Phenyl | Ethyl | (2,2-Dimethylpropan-1-ol)-3-yl | |
| 2.6.566 | 3,5-Dichlor-Phenyl | Ethyl | (Methyl-2,2-dimethylpropanoat)-3-yl | |
| 2.6.567 | 3,5-Dichlor-Phenyl | Ethyl | (Methylpropanoat)-3-yl | |
| 2.6.568 | 3,5-Dichlor-Phenyl | Ethyl | (Ethylpropanoat)-3-yl | |
| 2.6.569 | 3,5-Dichlor-Phenyl | Ethyl | (Ethylbutanoat)-3-yl | |
| 2.6.570 | 3,5-Dichlor-Phenyl | Ethyl | (Ethyl-(3R)-4,4,4-trifluorbutanoat)-3-yl | |
| 2.6.571 | 3,5-Dichlor-Phenyl | Ethyl | (Butan-2-on)-4-yl | |
| 2.6.572 | 3,5-Dichlor-Phenyl | Ethyl | (3-Methylbutan-2-on)-4-yl | |
| 2.6.573 | 3,5-Dichlor-Phenyl | Ethyl | (Pent-3-en-2-on)-4-yl | |
| 2.6.574 | 3,5-Dichlor-Phenyl | Ethyl | ((2S)-Dimethyl-butandioat)-2-yl | |
| 2.6.575 | 3,5-Dichlor-Phenyl | Ethyl | (Dimethylpentandioat)-3-yl | |
| 2.6.576 | 3,5-Dichlor-Phenyl | Ethyl | (Methyl-(2R)-2-methylpropanoat)-3-yl | |
| 2.6.577 | 3,5-Dichlor-Phenyl | Ethyl | 4-Methoxycarbonylbenzyl | |
| 2.6.578 | 3,5-Dichlor-Phenyl | Ethyl | 3,5-Difluorbenzyl | |
| 2.6.579 | 3,5-Dichlor-Phenyl | Ethyl | 3,4-Difluorbenzyl | |
| 2.6.580 | 3,5-Dichlor-Phenyl | Ethyl | 2,6-Difluorbenzyl | |
| 2.6.581 | 3,5-Dichlor-Phenyl | Ethyl | (5-Methylpyridin-3-yl)-methyl | |
| 2.6.582 | 3,5-Dichlor-Phenyl | Ethyl | (Tetrahydrofuran)-3-yl | |
| 2.6.583 | (S)-3,5-Dichlor-Phenyl | Methyl | (Ethylbutanoat)-3-yl | |
| 2.6.584 | (R)-3,5-Dichlor-Phenyl | Methyl | (Ethylbutanoat)-3-yl | |
| 2.6.585 | 3-Brom-5-chlor-Phenyl | Methyl | (Ethylbutanoat)-3-yl | |
| 2.6.586 | 3-Iod-5-chlor-Phenyl | Methyl | (Ethylbutanoat)-3-yl | |
| 2.6.587 | 3-Methyl-5-chlor-Phenyl | Methyl | (Ethylbutanoat)-3-yl | |
| 2.6.588 | 3-Ethyl-5-chlor-Phenyl | Methyl | (Ethylbutanoat)-3-yl | |
| 2.6.589 | 3-Propyl-5-chlor-Phenyl | Methyl | (Ethylbutanoat)-3-yl | |
| 2.6.590 | 3-isoPropyl-5-chlor-Phenyl | Methyl | (Ethylbutanoat)-3-yl | |
| 2.6.591 | 3-nButyl-5-chlor-Phenyl | Methyl | (Ethylbutanoat)-3-yl | |
| 2.6.592 | 3-isoButyl-5-chlor-Phenyl | Methyl | (Ethylbutanoat)-3-yl | |
| 2.6.593 | 3-tert.Butyl-5-chlor-Phenyl | Methyl | (Ethylbutanoat)-3-yl | |
| 2.6.594 | 3-Cyclopropyl-5-chlor-Phenyl | Methyl | (Ethylbutanoat)-3-yl | |
| 2.6.595 | 3-Cyano-5-chlor-Phenyl | Methyl | (Ethylbutanoat)-3-yl | |
| 2.6.596 | 3-Trifluormethyl-5-chlor-Phenyl | Methyl | (Ethylbutanoat)-3-yl | |
| 2.6.597 | 3-Trifluormethyl-5-chlor-Phenyl | Methyl | (Ethylbutanoat)-3-yl | |
| 2.6.598 | 3-(Hydroxycarbonyl) -5-chlor-Phenyl | Methyl | (Ethylbutanoat)-3-yl | |
| 2.6.599 | 3-(Methoxycarbonyl )-5-chlor-Phenyl | Methyl | (Ethylbutanoat)-3-yl | |
| 2.6.600 | 3-Methoxy-5-chlor-Phenyl | Methyl | (Ethylbutanoat)-3-yl | |
| 2.6.601 | 3-Ethoxy-5-chlor-Phenyl | Methyl | (Ethylbutanoat)-3-yl | |
| 2.6.602 | 3-nPropyoxy-5-chlor-Phenyl | Methyl | (Ethylbutanoat)-3-yl | |
| 2.6.603 | 3-isoPropoxy-5-chlor-Phenyl | Methyl | (Ethylbutanoat)-3-yl | |
| 2.6.604 | 3-nButoxy-5-chlor-Phenyl | Methyl | (Ethylbutanoat)-3-yl | |
| 2.6.605 | 3-isoButoxy-5-chlor-Phenyl | Methyl | (Ethylbutanoat)-3-yl | |
| 2.6.606 | 3-Difluormethoxy-5-chlor-Phenyl | Methyl | (Ethylbutanoat)-3-yl | |
| 2.6.607 | 3-Trifluormethoxy-5-chlor-Phenyl | Methyl | (Ethylbutanoat)-3-yl | |
| 2.6.608 | 3-Nitro-5-chlor-Phenyl | Methyl | (Ethylbutanoat)-3-yl | |
| 2.6.609 | 3-Acetoxy-5-chlor-Phenyl | Methyl | (Ethylbutanoat)-3-yl | |
| 2.6.610 | 3-Methylsulfanyl-5-chlor-Phenyl | Methyl | (Ethylbutanoat)-3-yl | |
| 2.6.611 | 3,5-Dibrom-Phenyl | Methyl | (Ethylbutanoat)-3-yl | |
| 2.6.612 | 3-Iod-5-brom-Phenyl | Methyl | (Ethylbutanoat)-3-yl | |
| 2.6.613 | 3-Methyl-5-brom-Phenyl | Methyl | (Ethylbutanoat)-3-yl | |
| 2.6.614 | 3-Ethyl-5-brom-Phenyl | Methyl | (Ethylbutanoat)-3-yl | |
| 2.6.615 | 3-Propyl-5-brom-Phenyl | Methyl | (Ethylbutanoat)-3-yl | |
| 2.6.616 | 3-isoPropyl-5-brom-Phenyl | Methyl | (Ethylbutanoat)-3-yl | |
| 2.6.617 | 3-nButyl-5-brom-Phenyl | Methyl | (Ethylbutanoat)-3-yl | |
| 2.6.618 | 3-isoButyl-5-brom-Phenyl | Methyl | (Ethylbutanoat)-3-yl | |
| 2.6.619 | 3-tert. Butyl-5-brom-Phenyl | Methyl | (Ethylbutanoat)-3-yl | |
| 2.6.620 | 3-Cyclopropyl-5-brom-Phenyl | Methyl | (Ethylbutanoat)-3-yl | |
| 2.6.621 | 3-Cyano-5-brom-Phenyl | Methyl | (Ethylbutanoat)-3-yl | |
| 2.6.622 | 3-Trifluormethyl-5-brom-Phenyl | Methyl | (Ethylbutanoat)-3-yl | |
| 2.6.623 | 3-(Hydroxycarbonyl) -5-brom-Phenyl | Methyl | (Ethylbutanoat)-3-yl | |
| 2.6.624 | 3-(Methoxycarbony) l-5-brom-Phenyl | Methyl | (Ethylbutanoat)-3-yl | |
| 2.6.625 | 3-Methoxy-5-brom-Phenyl | Methyl | (Ethylbutanoat)-3-yl | |
| 2.6.626 | 3-Ethoxy-5-brom-Phenyl | Methyl | (Ethylbutanoat)-3-yl | |
| 2.6.627 | 3-nPropyoxy-5-brom-Phenyl | Methyl | (Ethylbutanoat)-3-yl | |
| 2.6.628 | 3-isoPropoxy-5-brom-Phenyl | Methyl | (Ethylbutanoat)-3-yl | |
| 2.6.629 | 3-nButoxy-5-brom-Phenyl | Methyl | (Ethylbutanoat)-3-yl | |
| 2.6.630 | 3-isoButoxy-5-brom-Phenyl | Methyl | (Ethylbutanoat)-3-yl | |
| 2.6.631 | 3-Difluormethoxy-5-brom-Phenyl | Methyl | (Ethylbutanoat)-3-yl | |
| 2.6.632 | 3-Trifluormethoxy-5-brom-Phenyl | Methyl | (Ethylbutanoat)-3-yl | |
| 2.6.633 | 3-Nitro-5-brom-Phenyl | Methyl | (Ethylbutanoat)-3-yl | |
| 2.6.634 | 3-Acetoxy-5-brom-Phenyl | Methyl | (Ethylbutanoat)-3-yl | |
| 2.6.635 | 3-Methylsulfanyl-5-brom-Phenyl | Methyl | (Ethylbutanoat)-3-yl | |
| 2.6.636 | 3,5-Diiod-Phenyl | Methyl | (Ethylbutanoat)-3-yl | |
| 2.6.637 | 3-Methyl-5-iod-Phenyl | Methyl | (Ethylbutanoat)-3-yl | |
| 2.6.638 | 3-Ethyl-5-iod-Phenyl | Methyl | (Ethylbutanoat)-3-yl | |
| 2.6.639 | 3-Propyl-5-iod-Phenyl | Methyl | (Ethylbutanoat)-3-yl | |
| 2.6.640 | 3-isoPropyl-5-iod-Phenyl | Methyl | (Ethylbutanoat)-3-yl | |
| 2.6.641 | 3-nButyl-5-iod-Phenyl | Methyl | (Ethylbutanoat)-3-yl | |
| 2.6.642 | 3-isoButyl-5-iod-Phenyl | Methyl | (Ethylbutanoat)-3-yl | |
| 2.6.643 | 3-tert. Butyl-5-iod-Phenyl | Methyl | (Ethylbutanoat)-3-yl | |
| 2.6.644 | 3-Cyclopropyl-5-iod-Phenyl | Methyl | (Ethylbutanoat)-3-yl | |
| 2.6.645 | 3-Cyano-5-iod-Phenyl | Methyl | (Ethylbutanoat)-3-yl | |
| 2.6.646 | 3-Trifluormethyl-5-iod-Phenyl | Methyl | (Ethylbutanoat)-3-yl | |
| 2.6.647 | 3-(Hydroxycarbonyl) -5-iod-Phenyl | Methyl | (Ethylbutanoat)-3-yl | |
| 2.6.648 | 3-(Methoxycarbonyl )-5-iod-Phenyl | Methyl | (Ethylbutanoat)-3-yl | |
| 2.6.649 | 3-Methoxy-5-iod-Phenyl | Methyl | (Ethylbutanoat)-3-yl | |
| 2.6.650 | 3-Ethoxy-5-iod-Phenyl | Methyl | (Ethylbutanoat)-3-yl | |
| 2.6.651 | 3-nPropyoxy-5-iod-Phenyl | Methyl | (Ethylbutanoat)-3-yl | |
| 2.6.652 | 3-isoPropoxy-5-iod-Phenyl | Methyl | (Ethylbutanoat)-3-yl | |
| 2.6.653 | 3-nButoxy-5-iod-Phenyl | Methyl | (Ethylbutanoat)-3-yl | |
| 2.6.654 | 3-isoButoxy-5-iod-Phenyl | Methyl | (Ethylbutanoat)-3-yl | |
| 2.6.655 | 3-Difluormethoxy-5-iod-Phenyl | Methyl | (Ethylbutanoat)-3-yl | |
| 2.6.656 | 3-Trifluormethoxy-5-iod-Phenyl | Methyl | (Ethylbutanoat)-3-yl | |
| 2.6.657 | 3-Nitro-5-iod-Phenyl | Methyl | (Ethylbutanoat)-3-yl | |
| 2.6.658 | 3-Acetoxy-5-iod-Phenyl | Methyl | (Ethylbutanoat)-3-yl | |
| 2.6.659 | 3-Methylsulfanyl-5-iod-Phenyl | Methyl | (Ethylbutanoat)-3-yl | |
| 2.6.660 | 3,5-Dimethyl-Phenyl | Methyl | (Ethylbutanoat)-3-yl | |
| 2.6.661 | 3-Ethyl-5-methyl-Phenyl | Methyl | (Ethylbutanoat)-3-yl | |
| 2.6.662 | 3-Propyl-5-methyl-Phenyl | Methyl | (Ethylbutanoat)-3-yl | |
| 2.6.663 | 3-isoPropyl-5-methyl-Phenyl | Methyl | (Ethylbutanoat)-3-yl | |
| 2.6.664 | 3-nButyl-5-methyl-Phenyl | Methyl | (Ethylbutanoat)-3-yl | |
| 2.6.665 | 3-isoButyl-5-methyl-Phenyl | Methyl | (Ethylbutanoat)-3-yl | |
| 2.6.666 | 3-tert.Butyl-5-methyl-Phenyl | Methyl | (Ethylbutanoat)-3-yl | |
| 2.6.667 | 3-Cyclopropyl-5-methyl-Phenyl | Methyl | (Ethylbutanoat)-3-yl | |
| 2.6.668 | 3-Cyano-5-methyl-Phenyl | Methyl | (Ethylbutanoat)-3-yl | |
| 2.6.669 | 3-Trifluormethyl-5-methyl-Phenyl | Methyl | (Ethylbutanoat)-3-yl | |
| 2.6.670 | 3-(Hydroxycarbonyl) -5-methyl-Phenyl | Methyl | (Ethylbutanoat)-3-yl | |
| 2.6.671 | 3-(Methoxycarbony) l-5-methyl-Phenyl | Methyl | (Ethylbutanoat)-3-yl | |
| 2.6.672 | 3-Methoxy-5-methyl-Phenyl | Methyl | (Ethylbutanoat)-3-yl | |
| 2.6.673 | 3-Ethoxy-5-methyl-Phenyl | Methyl | (Ethylbutanoat)-3-yl | |
| 2.6.674 | 3-Propyoxy-5-methyl-Phenyl | Methyl | (Ethylbutanoat)-3-yl | |
| 2.6.675 | 3-Butoxy-5-methyl-Phenyl | Methyl | (Ethylbutanoat)-3-yl | |
| 2.6.676 | 3-isoButoxy-5-methyl-Phenyl | Methyl | (Ethylbutanoat)-3-yl | |
| 2.6.677 | 3-Difluormethoxy-5-methyl-Phenyl | Methyl | (Ethylbutanoat)-3-yl | |
| 2.6.678 | 3-Trifluormethoxy-5-methyl-Phenyl | Methyl | (Ethylbutanoat)-3-yl | |
| 2.6.679 | 3-Nitro-5-methyl-Phenyl | Methyl | (Ethylbutanoat)-3-yl | |
| 2.6.680 | 3-Acetoxy-5-methyl-Phenyl | Methyl | (Ethylbutanoat)-3-yl | |
| 2.6.681 | 3-Methylsulfanyl-5-methyl-Phenyl | Methyl | (Ethylbutanoat)-3-yl | |
| 2.6.682 | 3,5-Diethyl-Phenyl | Methyl | (Ethylbutanoat)-3-yl | |
| 2.6.683 | 3-Propyl-5-ethyl-Phenyl | Methyl | (Ethylbutanoat)-3-yl | |
| 2.6.684 | 3-isoPropyl-5-ethyl-Phenyl | Methyl | (Ethylbutanoat)-3-yl | |
| 2.6.685 | 3-nButyl-5-ethyl-Phenyl | Methyl | (Ethylbutanoat)-3-yl | |
| 2.6.686 | 3-isoButyl-5-ethyl-Phenyl | Methyl | (Ethylbutanoat)-3-yl | |
| 2.6.687 | 3-tert.Butyl-5-ethyl-Phenyl | Methyl | (Ethylbutanoat)-3-yl | |
| 2.6.688 | 3-Cyclopropyl-5-ethyl-Phenyl | Methyl | (Ethylbutanoat)-3-yl | |
| 2.6.689 | 3-Cyano-5-ethyl-Phenyl | Methyl | (Ethylbutanoat)-3-yl | |
| 2.6.690 | 3-Trifluormethyl-5-ethyl-Phenyl | Methyl | (Ethylbutanoat)-3-yl | |
| 2.6.691 | 3-(Hydroxycarbonyl) -5-ethyl-Phenyl | Methyl | (Ethylbutanoat)-3-yl | |
| 2.6.692 | 3-(Methoxycarbony) l-5-ethyl-Phenyl | Methyl | (Ethylbutanoat)-3-yl | |
| 2.6.693 | 3-Methoxy-5-ethyl-Phenyl | Methyl | (Ethylbutanoat)-3-yl | |
| 2.6.694 | 3-Ethoxy-5-ethyl-Phenyl | Methyl | (Ethylbutanoat)-3-yl | |
| 2.6.695 | 3-nPropyoxy-5-ethyl-Phenyl | Methyl | (Ethylbutanoat)-3-yl | |
| 2.6.696 | 3-nButoxy-5-ethyl-Phenyl | Methyl | (Ethylbutanoat)-3-yl | |
| 2.6.697 | 3-isoButoxy-5-ethyl-Phenyl | Methyl | (Ethylbutanoat)-3-yl | |
| 2.6.698 | 3-Difluormethoxy-5-ethyl-Phenyl | Methyl | (Ethylbutanoat)-3-yl | |
| 2.6.699 | 3-Trifluormethoxy-5-ethyl-Phenyl | Methyl | (Ethylbutanoat)-3-yl | |
| 2.6.700 | 3-Nitro-5-ethyl-Phenyl | Methyl | (Ethylbutanoat)-3-yl | |
| 2.6.701 | 3-Acetoxy-5-ethyl-Phenyl | Methyl | (Ethylbutanoat)-3-yl | |
| 2.6.702 | 3-Methylsulfanyl-5-ethyl-Phenyl | Methyl | (Ethylbutanoat)-3-yl | |
| 2.6.703 | 3,5-Dipropyl-Phenyl | Methyl | (Ethylbutanoat)-3-yl | |
| 2.6.704 | 3-isoPropyl-5-propyl-Phenyl | Methyl | (Ethylbutanoat)-3-yl | |
| 2.6.705 | 3-nButyl-5-propyl-Phenyl | Methyl | (Ethylbutanoat)-3-yl | |
| 2.6.706 | 3-isoButyl-5-propyl-Phenyl | Methyl | (Ethylbutanoat)-3-yl | |
| 2.6.707 | 3-tert.Butyl-5-propyl-Phenyl | Methyl | (Ethylbutanoat)-3-yl | |
| 2.6.708 | 3-Cyclopropyl-5-propyl-Phenyl | Methyl | (Ethylbutanoat)-3-yl | |
| 2.6.709 | 3-Cyano-5-propyl-Phenyl | Methyl | (Ethylbutanoat)-3-yl | |
| 2.6.710 | 3-Trifluormethyl-5-propyl-Phenyl | Methyl | (Ethylbutanoat)-3-yl | |
| 2.6.711 | 3-(Hydroxycarbonyl) -5-propyl-Phenyl | Methyl | (Ethylbutanoat)-3-yl | |
| 2.6.712 | 3-(Methoxycarbonyl )-5-propyl-Phenyl | Methyl | (Ethylbutanoat)-3-yl | |
| 2.6.713 | 3-Methoxy-5-propyl-Phenyl | Methyl | (Ethylbutanoat)-3-yl | |
| 2.6.714 | 3-Ethoxy-5-propyl-Phenyl | Methyl | (Ethylbutanoat)-3-yl | |
| 2.6.715 | 3-nPropyoxy-5-propyl-Phenyl | Methyl | (Ethylbutanoat)-3-yl | |
| 2.6.716 | 3-isoButoxy-5-propyl-Phenyl | Methyl | (Ethylbutanoat)-3-yl | |
| 2.6.717 | 3-Difluormethoxy-5-propyl-Phenyl | Methyl | (Ethylbutanoat)-3-yl | |
| 2.6.718 | 3-Trifluormethoxy-5-ethyl-Phenyl | Methyl | (Ethylbutanoat)-3-yl | |
| 2.6.719 | 3-Nitro-5-propyl-Phenyl | Methyl | (Ethylbutanoat)-3-yl | |
| 2.6.720 | 3-Acetoxy-5-propyl-Phenyl | Methyl | (Ethylbutanoat)-3-yl | |
| 2.6.721 | 3-Methylsulfanyl-5-propyl-Phenyl | Methyl | (Ethylbutanoat)-3-yl | |
| 2.6.722 | 3,5-Diisopropyl-Phenyl | Methyl | (Ethylbutanoat)-3-yl | |
| 2.6.723 | 3-nButyl-5-isopropyl-Phenyl | Methyl | (Ethylbutanoat)-3-yl | |
| 2.6.724 | 3-isoButyl-5-isopropyl-Phenyl | Methyl | (Ethylbutanoat)-3-yl | |
| 2.6.725 | 3-tert.Butyl-5-isopropyl-Phenyl | Methyl | (Ethylbutanoat)-3-yl | |
| 2.6.726 | 3-Cyclopropyl-5-isopropyl-Phenyl | Methyl | (Ethylbutanoat)-3-yl | |
| 2.6.727 | 3-Cyano-5-isopropyl-Phenyl | Methyl | (Ethylbutanoat)-3-yl | |
| 2.6.728 | 3-Trifluormethyl-5-isopropyl-Phenyl | Methyl | (Ethylbutanoat)-3-yl | |
| 2.6.729 | 3-(Hydroxycarbonyl) -5-isopropyl-Phenyl | Methyl | (Ethylbutanoat)-3-yl | |
| 2.6.730 | 3-(Methoxycarbony) l-5-isopropyl-Phenyl | Methyl | (Ethylbutanoat)-3-yl | |
| 2.6.731 | 3-Methoxy-5-isopropyl-Phenyl | Methyl | (Ethylbutanoat)-3-yl | |
| 2.6.732 | 3-Ethoxy-5-isopropyl-Phenyl | Methyl | (Ethylbutanoat)-3-yl | |
| 2.6.733 | 3-nPropyoxy-5-isopropyl-Phenyl | Methyl | (Ethylbutanoat)-3-yl | |
| 2.6.734 | 3-isoButoxy-5-isopropyl-Phenyl | Methyl | (Ethylbutanoat)-3-yl | |
| 2.6.735 | 3-Difluormethoxy-5-isopropyl-Phenyl | Methyl | (Ethylbutanoat)-3-yl | |
| 2.6.736 | 3-Trifluormethoxy-5-isopropyl-Phenyl | Methyl | (Ethylbutanoat)-3-yl | |
| 2.6.737 | 3-Nitro-5-isopropyl-Phenyl | Methyl | (Ethylbutanoat)-3-yl | |
| 2.6.738 | 3-Acetoxy-5-isopropyl-Phenyl | Methyl | (Ethylbutanoat)-3-yl | |
| 2.6.739 | 3-Methylsulfanyl-5-isopropyl-Phenyl | Methyl | (Ethylbutanoat)-3-yl | |
| 2.6.740 | 3,5-Diisobutyl-Phenyl | Methyl | (Ethylbutanoat)-3-yl | |
| 2.6.741 | 3-tertButyl-5-isobutyl-Phenyl | Methyl | (Ethylbutanoat)-3-yl | |
| 2.6.742 | 3-Cyclopropyl-5-isobutyl-Phenyl | Methyl | (Ethylbutanoat)-3-yl | |
| 2.6.743 | 3-Cyano-5-isobutyl-Phenyl | Methyl | (Ethylbutanoat)-3-yl | |
| 2.6.744 | 3-Trifluormethyl-5-isobutyl-Phenyl | Methyl | (Ethylbutanoat)-3-yl | |
| 2.6.745 | 3-(Methoxycarbonyl )-5-isobutyl-Phenyl | Methyl | (Ethylbutanoat)-3-yl | |
| 2.6.746 | 3-Methoxy-5-isobutyl-Phenyl | Methyl | (Ethylbutanoat)-3-yl | |
| 2.6.747 | 3-Ethoxy-5-isobutyl-Phenyl | Methyl | (Ethylbutanoat)-3-yl | |
| 2.6.748 | 3-nPropyoxy-5-isobutyl-Phenyl | Methyl | (Ethylbutanoat)-3-yl | |
| 2.6.749 | 3-isoButoxy-5-isobutyl-Phenyl | Methyl | (Ethylbutanoat)-3-yl | |
| 2.6.750 | 3-Difluormethoxy-5-isobutyl-Phenyl | Methyl | (Ethylbutanoat)-3-yl | |
| 2.6.751 | 3-Trifluormethoxy-5-isobutyl-Phenyl | Methyl | (Ethylbutanoat)-3-yl | |
| 2.6.752 | 3-Nitro-5-isobutyl-Phenyl | Methyl | (Ethylbutanoat)-3-yl | |
| 2.6.753 | 3-Acetoxy-5-isobutyl-Phenyl | Methyl | (Ethylbutanoat)-3-yl | |
| 2.6.754 | 3-Methylsulfanyl-5-isobutyl-Phenyl | Methyl | (Ethylbutanoat)-3-yl | |
| 2.6.755 | 3,5-Ditert.butyl-Phenyl | Methyl | (Ethylbutanoat)-3-yl | |
| 2.6.756 | 3-Cyclopropyl-5-tert.butyl-Phenyl | Methyl | (Ethylbutanoat)-3-yl | |
| 2.6.757 | 3-Cyano-5-tert. butyl-Phenyl | Methyl | (Ethylbutanoat)-3-yl | |
| 2.6.758 | 3-Trifluormethyl-5-tert.butyl-Phenyl | Methyl | (Ethylbutanoat)-3-yl | |
| 2.6.759 | 3-(Hydroxycarbonyl) -5-tert.butyl-Phenyl | Methyl | (Ethylbutanoat)-3-yl | |
| 2.6.760 | 3-(Methoxycarbonyl )-5tert.butyl-Phenyl | Methyl | (Ethylbutanoat)-3-yl | |
| 2.6.761 | 3-Methoxy-5-tert.butyl-Phenyl | Methyl | (Ethylbutanoat)-3-yl | |
| 2.6.762 | 3-Ethoxy-5-tert.butyl-Phenyl | Methyl | (Ethylbutanoat)-3-yl | |
| 2.6.763 | 3-nPropyoxy-5-tert.butyl-Phenyl | Methyl | (Ethylbutanoat)-3-yl | |
| 2.6.764 | 3-isoButoxy-5-tert.butyl-Phenyl | Methyl | (Ethylbutanoat)-3-yl | |
| 2.6.765 | 3-Difluormethoxy-5-tert.butyl-Phenyl | Methyl | (Ethylbutanoat)-3-yl | |
| 2.6.766 | 3-Trifluormethoxy-5-tert.butyl-Phenyl | Methyl | (Ethylbutanoat)-3-yl | |
| 2.6.767 | 3-Nitro-5-tert.butyl-Phenyl | Methyl | (Ethylbutanoat)-3-yl | |
| 2.6.768 | 3-Acetoxy-5-tert.butyl-Phenyl | Methyl | (Ethylbutanoat)-3-yl | |
| 2.6.769 | 3-Methylsulfanyl-5-tert.butyl-Phenyl | Methyl | (Ethylbutanoat)-3-yl | |
| 2.6.770 | 3-tert.Butyl-5-cyclopropyl-Phenyl | Methyl | (Ethylbutanoat)-3-yl | |
| 2.6.771 | 3,5-Dicyclopropyl-Phenyl | Methyl | (Ethylbutanoat)-3-yl | |
| 2.6.772 | 3-Cyano-5-cyclopropyl-Phenyl | Methyl | (Ethylbutanoat)-3-yl | |
| 2.6.773 | 3-Trifluormethyl-5-cyclopropyl-Phenyl | Methyl | (Ethylbutanoat)-3-yl | |
| 2.6.774 | 3-(Hydroxycarbonyl) -5-cyclopropyl-Phenyl | Methyl | (Ethylbutanoat)-3-yl | |
| 2.6.775 | 3-(Methoxycarbonyl )-5-cyclopropyl-Phenyl | Methyl | (Ethylbutanoat)-3-yl | |
| 2.6.776 | 3-Methoxy-5-cyclopropyl-Phenyl | Methyl | (Ethylbutanoat)-3-yl | |
| 2.6.777 | 3-Ethoxy-5-cyclopropyl-Phenyl | Methyl | (Ethylbutanoat)-3-yl | |
| 2.6.778 | 3-nPropyoxy-5-cyclopropyl-Phenyl | Methyl | (Ethylbutanoat)-3-yl | |
| 2.6.779 | 3-isoButoxy-5-cyclopropyl-Phenyl | Methyl | (Ethylbutanoat)-3-yl | |
| 2.6.780 | 3-Difluormethoxy-5-cyclopropyl-Phenyl | Methyl | (Ethylbutanoat)-3-yl | |
| 2.6.781 | 3-Trifluormethoxy-5-cyclopropyl-Phenyl | Methyl | (Ethylbutanoat)-3-yl | |
| 2.6.782 | 3-Nitro-5-cyclopropyl-Phenyl | Methyl | (Ethylbutanoat)-3-yl | |
| 2.6.783 | 3-Acetoxy-5-cyclopropyl-Phenyl | Methyl | (Ethylbutanoat)-3-yl | |
| 2.6.784 | 3-Methylsulfanyl-5-cyclopropyl-Phenyl | Methyl | (Ethylbutanoat)-3-yl | |
| 2.6.785 | 3,5-Dicyano-Phenyl | Methyl | (Ethylbutanoat)-3-yl | |
| 2.6.786 | 3-Trifluormethyl-5-cyano-Phenyl | Methyl | (Ethylbutanoat)-3-yl | |
| 2.6.787 | 3-(Methoxycarbonyl )-5-cyano-Phenyl | Methyl | (Ethylbutanoat)-3-yl | |
| 2.6.788 | 3-Methoxy-5-cyano-Phenyl | Methyl | (Ethylbutanoat)-3-yl | |
| 2.6.789 | 3-Ethoxy-5-cyano-Phenyl | Methyl | (Ethylbutanoat)-3-yl | |
| 2.6.790 | 3-nPropyoxy-5-cyano-Phenyl | Methyl | (Ethylbutanoat)-3-yl | |
| 2.6.791 | 3-nButoxy-5-cyano-Phenyl | Methyl | (Ethylbutanoat)-3-yl | |
| 2.6.792 | 3-isoButoxy-5-cyano-Phenyl | Methyl | (Ethylbutanoat)-3-yl | |
| 2.6.793 | 3-Difluormethoxy-5-cyano-Phenyl | Methyl | (Ethylbutanoat)-3-yl | |
| 2.6.794 | 3-Trifluormethoxy-5-cyano-Phenyl | Methyl | (Ethylbutanoat)-3-yl | |
| 2.6.795 | 3-Nitro-5-cyano-Phenyl | Methyl | (Ethylbutanoat)-3-yl | |
| 2.6.796 | 3-Acetoxy-5-cyano-Phenyl | Methyl | (Ethylbutanoat)-3-yl | |
| 2.6.797 | 3-Methylsulfanyl-5-cyano-Phenyl | Methyl | (Ethylbutanoat)-3-yl | |
| 2.6.798 | 3,5-Di(trifluormethyl)-Phenyl | Methyl | (Ethylbutanoat)-3-yl | |
| 2.6.799 | 3-(Hydroxycarbonyl) -5-trifluormethyl-Phenyl | Methyl | (Ethylbutanoat)-3-yl | |
| 2.6.800 | 3-(Methoxycarbonyl )-5-trifluormethyl-Phenyl | Methyl | (Ethylbutanoat)-3-yl | |
| 2.6.801 | 3-Methoxy-5-trifluormethyl-Phenyl | Methyl | (Ethylbutanoat)-3-yl | |
| 2.6.802 | 3-Ethoxy-5trifluormethyl-Phenyl | Methyl | (Ethylbutanoat)-3-yl | |
| 2.6.803 | 3-nPropyoxy-5-trifluormethyl-Phenyl | Methyl | (Ethylbutanoat)-3-yl | |
| 2.6.804 | 3-isoButoxy-5-trifluormethyl-Phenyl | Methyl | (Ethylbutanoat)-3-yl | |
| 2.6.805 | 3-Difluormethoxy-5-trifluormethyl-Phenyl | Methyl | (Ethylbutanoat)-3-yl | |
| 2.6.806 | 3-Trifluormethoxy-5-trifluormethyl-Phenyl | Methyl | (Ethylbutanoat)-3-yl | |
| 2.6.807 | 3-Nitro-5-trifluormethyl-Phenyl | Methyl | (Ethylbutanoat)-3-yl | |
| 2.6.808 | 3-Acetoxy-5-trifluormethyl-Phenyl | Methyl | (Ethylbutanoat)-3-yl | |
| 2.6.809 | 3-Methylsulfanyl-5-trifluormethyl-Phenyl | Methyl | (Ethylbutanoat)-3-yl | |
| 2.6.810 | 3,5-Di(methoxycarbon yl)-Phenyl | Methyl | (Ethylbutanoat)-3-yl | |
| 2.6.811 | 3-Methoxy-5-(methoxycarbonyl )-Phenyl | Methyl | (Ethylbutanoat)-3-yl | |
| 2.6.812 | 3-Ethoxy-5-(methoxycarbonyl )-Phenyl | Methyl | (Ethylbutanoat)-3-yl | |
| 2.6.813 | 3-nPropyoxy-5-(methoxycarbonyl )-Phenyl | Methyl | (Ethylbutanoat)-3-yl | |
| 2.6.814 | 3-isoButoxy-5-(methoxycarbonyl )-Phenyl | Methyl | (Ethylbutanoat)-3-yl | |
| 2.6.815 | 3-Difluormethoxy-(methoxycarbonyl )-Phenyl | Methyl | (Ethylbutanoat)-3-yl | |
| 2.6.816 | 3-Trifluormethoxy-(methoxycarbonyl )-Phenyl | Methyl | (Ethylbutanoat)-3-yl | |
| 2.6.817 | 3-Nitro-5-(methoxycarbonyl )-Phenyl | Methyl | (Ethylbutanoat)-3-yl | |
| 2.6.818 | 3-Acetoxy-5-(methoxycarbonyl )-Phenyl | Methyl | (Ethylbutanoat)-3-yl | |
| 2.6.819 | 3-Methylsulfanyl-(methoxycarbonyl )-Phenyl | Methyl | (Ethylbutanoat)-3-yl | |
| 2.6.820 | 3,5-Dimethoxy-Phenyl | Methyl | (Ethylbutanoat)-3-yl | |
| 2.6.821 | 3-Ethoxy-5-methoxy-Phenyl | Methyl | (Ethylbutanoat)-3-yl | |
| 2.6.822 | 3-nPropyoxy-5-methoxy-Phenyl | Methyl | (Ethylbutanoat)-3-yl | |
| 2.6.823 | 3-isoButoxy-5-methoxy-Phenyl | Methyl | (Ethylbutanoat)-3-yl | |
| 2.6.824 | 3-Difluormethoxy-5-methoxy-Phenyl | Methyl | (Ethylbutanoat)-3-yl | |
| 2.6.825 | 3-Trifluormethoxy-5-methoxy-Phenyl | Methyl | (Ethylbutanoat)-3-yl | |
| 2.6.826 | 3-Nitro-5-methoxy-Phenyl | Methyl | (Ethylbutanoat)-3-yl | |
| 2.6.827 | 3-Acetoxy-5-methoxy-Phenyl | Methyl | (Ethylbutanoat)-3-yl | |
| 2.6.828 | 3-Methylsulfanyl-5-methoxy-Phenyl | Methyl | (Ethylbutanoat)-3-yl | |
| 2.6.829 | 3,5-Diethoxy-Phenyl | Methyl | (Ethylbutanoat)-3-yl | |
| 2.6.830 | 3-nPropyoxy-5-ethoxy-Phenyl | Methyl | (Ethylbutanoat)-3-yl | |
| 2.6.831 | 3-isoButoxy-5-ethoxy-Phenyl | Methyl | (Ethylbutanoat)-3-yl | |
| 2.6.832 | 3-Difluormethoxy-5-ethoxy-Phenyl | Methyl | (Ethylbutanoat)-3-yl | |
| 2.6.833 | 3-Trifluormethoxy-5-ethoxy-Phenyl | Methyl | (Ethylbutanoat)-3-yl | |
| 2.6.834 | 3-Nitro-5-ethoxy-Phenyl | Methyl | (Ethylbutanoat)-3-yl | |
| 2.6.835 | 3-Acetoxy-5-ethoxy-Phenyl | Methyl | (Ethylbutanoat)-3-yl | |
| 2.6.836 | 3-Methylsulfanyl-5-ethoxy-Phenyl | Methyl | (Ethylbutanoat)-3-yl | |
| 2.6.837 | 3,5-Di(isopropyoxy)-Phenyl | Methyl | (Ethylbutanoat)-3-yl | |
| 2.6.838 | 3-nButoxy-5-isopropoxy-Phenyl | Methyl | (Ethylbutanoat)-3-yl | |
| 2.6.839 | 3-isoButoxy-5-isopropoxy-Phenyl | Methyl | (Ethylbutanoat)-3-yl | |
| 2.6.840 | 3-Difluormethoxy-5-isopropoxy-Phenyl | Methyl | (Ethylbutanoat)-3-yl | |
| 2.6.841 | 3-Trifluormethoxy-5-isopropoxy-Phenyl | Methyl | (Ethylbutanoat)-3-yl | |
| 2.6.842 | 3-Nitro-5-isopropoxy-Phenyl | Methyl | (Ethylbutanoat)-3-yl | |
| 2.6.843 | 3-Acetoxy-5-isopropoxy-Phenyl | Methyl | (Ethylbutanoat)-3-yl | |
| 2.6.844 | 3-Methylsulfanyl-5-isopropoxy-Phenyl | Methyl | (Ethylbutanoat)-3-yl | |
| 2.6.845 | 3,5-Di(trifluormethoxy )-Phenyl | Methyl | (Ethylbutanoat)-3-yl | |
| 2.6.846 | 3-Nitro-5-trifluormethoxy-Phenyl | Methyl | (Ethylbutanoat)-3-yl | |
| 2.6.847 | 3-Methylsulfanyl-5-trifluormethoxy-Phenyl | Methyl | (Ethylbutanoat)-3-yl | |
| 2.6.848 | 3,5-Bis(Difluormethox y)-Phenyl | Methyl | (Ethylbutanoat)-3-yl | |
| 2.6.849 | 3-Trifluormethoxy-5-difluormethoxy-Phenyl | Methyl | (Ethylbutanoat)-3-yl | |
| 2.6.850 | 3-Nitro-5-difluormethoxy-Phenyl | Methyl | (Ethylbutanoat)-3-yl | |
| 2.6.851 | 3-Acetoxy-5-difluormethoxy-Phenyl | Methyl | (Ethylbutanoat)-3-yl | |
| 2.6.852 | 3-Methylsulfanyl-5-difluormethoxy-Phenyl | Methyl | (Ethylbutanoat)-3-yl | |
| 2.6.853 | 3,5-Bis(acetoxy)-Phenyl | Methyl | (Ethylbutanoat)-3-yl | |
| 2.6.854 | 3-Methylsulfanyl-5-acetoxy-Phenyl | Methyl | (Ethylbutanoat)-3-yl | |
| 2.6.855 | 3,5-Dinitro-Phenyl | Methyl | (Ethylbutanoat)-3-yl | |
| 2.6.856 | 3-Acetoxy-5-nitro-Phenyl | Methyl | (Ethylbutanoat)-3-yl | |
| 2.6.857 | 3-Methylsulfanyl-5-nitro-Phenyl | Methyl | (Ethylbutanoat)-3-yl | |
| 2.6.858 | 3,5-Di(methylsulfanyl) -Phenyl | Methyl | (Ethylbutanoat)-3-yl | |
| 2.6.859 | 3,4-Difluor-Phenyl | Methyl | (Ethylbutanoat)-3-yl | |
| 2.6.860 | 3-Chlor-4-fluor-Phenyl | Methyl | (Ethylbutanoat)-3-yl | |
| 2.6.861 | 3-Brom-4-fluor-Phenyl | Methyl | (Ethylbutanoat)-3-yl | |
| 2.6.862 | 3-Methyl-4-fluor-Phenyl | Methyl | (Ethylbutanoat)-3-yl | |
| 2.6.863 | 3-Ethyl-4-fluor-Phenyl | Methyl | (Ethylbutanoat)-3-yl | |
| 2.6.864 | 3-Cyclopropyl-4-fluor-Phenyl | Methyl | (Ethylbutanoat)-3-yl | |
| 2.6.865 | 3-Cyano-4-fluor-Phenyl | Methyl | (Ethylbutanoat)-3-yl | |
| 2.6.866 | 3-Methoxy-4-fluor-Phenyl | Methyl | (Ethylbutanoat)-3-yl | |
| 2.6.867 | 3-Ethoxy-4-fluor-Phenyl | Methyl | (Ethylbutanoat)-3-yl | |
| 2.6.868 | 3-Trifluormethoxy-4-fluor-Phenyl | Methyl | (Ethylbutanoat)-3-yl | |
| 2.6.869 | 3-Nitro-4-fluor-Phenyl | Methyl | (Ethylbutanoat)-3-yl | |
| 2.6.870 | 3-Fluor-4-chlor-Phenyl | Methyl | (Ethylbutanoat)-3-yl | |
| 2.6.871 | 3,4-Dichlor-Phenyl | Methyl | (Ethylbutanoat)-3-yl | |
| 2.6.872 | 3-Brom-4-chlor-Phenyl | Methyl | (Ethylbutanoat)-3-yl | |
| 2.6.873 | 3-Methyl-4-chlor-Phenyl | Methyl | (Ethylbutanoat)-3-yl | |
| 2.6.874 | 3-Cyclopropyl-4-chlor-Phenyl | Methyl | (Ethylbutanoat)-3-yl | |
| 2.6.875 | 3-Cyano-4-chlor-Phenyl | Methyl | (Ethylbutanoat)-3-yl | |
| 2.6.876 | 3-Trifluormethyl-4-chlor-Phenyl | Methyl | (Ethylbutanoat)-3-yl | |
| 2.6.877 | 3-Methoxy-4-chlor-Phenyl | Methyl | (Ethylbutanoat)-3-yl | |
| 2.6.878 | 3-Ethoxy-4-chlor-Phenyl | Methyl | (Ethylbutanoat)-3-yl | |
| 2.6.879 | 3-Trifluormethoxy-4-chlor-Phenyl | Methyl | (Ethylbutanoat)-3-yl | |
| 2.6.880 | 3-Nitro-4-chlor-Phenyl | Methyl | (Ethylbutanoat)-3-yl | |
| 2.6.881 | 3-Fluor-4-brom-Phenyl | Methyl | (Ethylbutanoat)-3-yl | |
| 2.6.882 | 3-Chlor-4-brom-Phenyl | Methyl | (Ethylbutanoat)-3-yl | |
| 2.6.883 | 3,4-Dibrom-Phenyl | Methyl | (Ethylbutanoat)-3-yl | |
| 2.6.884 | 3-Methyl-4-brom-Phenyl | Methyl | (Ethylbutanoat)-3-yl | |
| 2.6.885 | 3-Ethyl-4-brom-Phenyl | Methyl | (Ethylbutanoat)-3-yl | |
| 2.6.886 | 3-Cyclopropyl-4-brom-Phenyl | Methyl | (Ethylbutanoat)-3-yl | |
| 2.6.887 | 3-Cyano-4-brom-Phenyl | Methyl | (Ethylbutanoat)-3-yl | |
| 2.6.888 | 3-Trifluormethyl-4-brom-Phenyl | Methyl | (Ethylbutanoat)-3-yl | |
| 2.6.889 | 3-Methoxy-4-Phenyl | Methyl | (Ethylbutanoat)-3-yl | |
| 2.6.890 | 3-Ethoxy-4-brom-Phenyl | Methyl | (Ethylbutanoat)-3-yl | |
| 2.6.891 | 3-Trifluormethoxy-4-brom-Phenyl | Methyl | (Ethylbutanoat)-3-yl | |
| 2.6.892 | 3-Nitro-4-brom-Phenyl | Methyl | (Ethylbutanoat)-3-yl | |
| 2.6.893 | 3-Fluor-4-iod-Phenyl | Methyl | (Ethylbutanoat)-3-yl | |
| 2.6.894 | 3-Chlor-4-iod-Phenyl | Methyl | (Ethylbutanoat)-3-yl | |
| 2.6.895 | 3-Brom-4-iod-Phenyl | Methyl | (Ethylbutanoat)-3-yl | |
| 2.6.896 | 3-Methyl-4-iod-Phenyl | Methyl | (Ethylbutanoat)-3-yl | |
| 2.6.897 | 3-Cyclopropyl-4-iod-Phenyl | Methyl | (Ethylbutanoat)-3-yl | |
| 2.6.898 | 3-Cyano-4-iod-Phenyl | Methyl | (Ethylbutanoat)-3-yl | |
| 2.6.899 | 3-Trifluormethyl-4-iod-Phenyl | Methyl | (Ethylbutanoat)-3-yl | |
| 2.6.900 | 3-Methoxy-4-iod-Phenyl | Methyl | (Ethylbutanoat)-3-yl | |
| 2.6.901 | 3-Ethoxy-4-iod-Phenyl | Methyl | (Ethylbutanoat)-3-yl | |
| 2.6.902 | 3-Trifluormethoxy-4-iod-Phenyl | Methyl | (Ethylbutanoat)-3-yl | |
| 2.6.903 | 3-Nitro-4-iod-Phenyl | Methyl | (Ethylbutanoat)-3-yl | |
| 2.6.904 | 3-Fluor-4-methyl-Phenyl | Methyl | (Ethylbutanoat)-3-yl | |
| 2.6.905 | 3-Chlor-4-methyl-Phenyl | Methyl | (Ethylbutanoat)-3-yl | |
| 2.6.906 | 3-Brom-4-methyl-Phenyl | Methyl | (Ethylbutanoat)-3-yl | |
| 2.6.907 | 3.4-Dimethyl-Phenyl | Methyl | (Ethylbutanoat)-3-yl | |
| 2.6.908 | 3.4-Dimethyl-Phenyl | Ethyl | (Ethylbutanoat)-3-yl | |
| 2.6.909 | 3.4-Dimethyl-Phenyl | isoPropyl | (Ethylbutanoat)-3-yl | |
| 2.6.910 | 3.4-Dimethyl-Phenyl | Cyclopropyl | (Ethylbutanoat)-3-yl | |
| 2.6.911 | 3-Ethyl-4-methyl-Phenyl | Methyl | (Ethylbutanoat)-3-yl | |
| 2.6.912 | 3-Cyclopropyl-4-methyl-Phenyl | Methyl | (Ethylbutanoat)-3-yl | |
| 2.6.913 | 3-Cyano-4-methyl-Phenyl | Methyl | (Ethylbutanoat)-3-yl | |
| 2.6.914 | 3-Trifluormethyl-4-methyl-Phenyl | Methyl | (Ethylbutanoat)-3-yl | |
| 2.6.915 | 3-Methoxy-4-methyl-Phenyl | Methyl | (Ethylbutanoat)-3-yl | |
| 2.6.916 | 3-Ethoxy-4-methyl-Phenyl | Methyl | (Ethylbutanoat)-3-yl | |
| 2.6.917 | 3-Trifluormethoxy-4-methyl-Phenyl | Methyl | (Ethylbutanoat)-3-yl | |
| 2.6.918 | 3-Nitro-4-methyl-Phenyl | Methyl | (Ethylbutanoat)-3-yl | |
| 2.6.919 | 3-Fluor-4-ethyl-Phenyl | Methyl | (Ethylbutanoat)-3-yl | |
| 2.6.920 | 3-Chlor-4-ethyl-Phenyl | Methyl | (Ethylbutanoat)-3-yl | |
| 2.6.921 | 3-Brom-4-ethyl-Phenyl | Methyl | (Ethylbutanoat)-3-yl | |
| 2.6.922 | 3-Methyl-4-ethyl-Phenyl | Methyl | (Ethylbutanoat)-3-yl | |
| 2.6.923 | 3,4-Diethyl-Phenyl | Methyl | (Ethylbutanoat)-3-yl | |
| 2.6.924 | 3-Cyclopropyl-4-ethyl-Phenyl | Methyl | (Ethylbutanoat)-3-yl | |
| 2.6.925 | 3-Cyano-4-ethyl-Phenyl | Methyl | (Ethylbutanoat)-3-yl | |
| 2.6.926 | 3-Trifluormethyl-4-ethyl-Phenyl | Methyl | (Ethylbutanoat)-3-yl | |
| 2.6.927 | 3-Methoxy-4-ethyl-Phenyl | Methyl | (Ethylbutanoat)-3-yl | |
| 2.6.928 | 3-Ethoxy-4-ethyl-Phenyl | Methyl | (Ethylbutanoat)-3-yl | |
| 2.6.929 | 3-Trifluormethoxy-4-ethyl-Phenyl | Methyl | (Ethylbutanoat)-3-yl | |
| 2.6.930 | 3-Nitro-4-ethyl-Phenyl | Methyl | (Ethylbutanoat)-3-yl | |
| 2.6.931 | 3-Fluor-4-propyl-Phenyl | Methyl | (Ethylbutanoat)-3-yl | |
| 2.6.932 | 3-Chlor-4-propyl-Phenyl | Methyl | (Ethylbutanoat)-3-yl | |
| 2.6.933 | 3-Brom-4-propyl-Phenyl | Methyl | (Ethylbutanoat)-3-yl | |
| 2.6.934 | 3-Methyl-4-propyl-Phenyl | Methyl | (Ethylbutanoat)-3-yl | |
| 2.6.935 | 3-Methyl-4-propyl-Phenyl | Methyl | (Ethylbutanoat)-3-yl | |
| 2.6.936 | 3-Cyclopropyl-4-propyl-Phenyl | Methyl | (Ethylbutanoat)-3-yl | |
| 2.6.937 | 3-Cyano-4-propyl-Phenyl | Methyl | (Ethylbutanoat)-3-yl | |
| 2.6.938 | 3-Trifluormethyl-4-propyl-Phenyl | Methyl | (Ethylbutanoat)-3-yl | |
| 2.6.939 | 3-Methoxy-4-propyl-Phenyl | Methyl | (Ethylbutanoat)-3-yl | |
| 2.6.940 | 3-Ethoxy-4-propyl-Phenyl | Methyl | (Ethylbutanoat)-3-yl | |
| 2.6.941 | 3-Trifluormethoxy-4-propyl-Phenyl | Methyl | (Ethylbutanoat)-3-yl | |
| 2.6.942 | 3-Nitro-4-propyl-Phenyl | Methyl | (Ethylbutanoat)-3-yl | |
| 2.6.943 | 3-Fluor-4-isopropyl-Phenyl | Methyl | (Ethylbutanoat)-3-yl | |
| 2.6.944 | 3-Chlor-4-isopropyl-Phenyl | Methyl | (Ethylbutanoat)-3-yl | |
| 2.6.945 | 3-Brom-4-isopropyl-Phenyl | Methyl | (Ethylbutanoat)-3-yl | |
| 2.6.946 | 3-Methyl-4-isopropyl-Phenyl | Methyl | (Ethylbutanoat)-3-yl | |
| 2.6.947 | 3-Cyclopropyl-4-isopropyl-Phenyl | Methyl | (Ethylbutanoat)-3-yl | |
| 2.6.948 | 3-Cyano-4-isopropyl-Phenyl | Methyl | (Ethylbutanoat)-3-yl | |
| 2.6.949 | 3-Trifluormethyl-4-isopropyl-Phenyl | Methyl | (Ethylbutanoat)-3-yl | |
| 2.6.950 | 3-Methoxy-4-isopropyl-Phenyl | Methyl | (Ethylbutanoat)-3-yl | |
| 2.6.951 | 3-Ethoxy-4-isopropyl-Phenyl | Methyl | (Ethylbutanoat)-3-yl | |
| 2.6.952 | 3-Trifluormethoxy-4-isopropyl-Phenyl | Methyl | (Ethylbutanoat)-3-yl | |
| 2.6.953 | 3-Nitro-4-isopropyl-Phenyl | Methyl | (Ethylbutanoat)-3-yl | |
| 2.6.954 | 3-Fluor-4-tert.butyl-Phenyl | Methyl | (Ethylbutanoat)-3-yl | |
| 2.6.955 | 3-Chlor-4-tert.butyl-Phenyl | Methyl | (Ethylbutanoat)-3-yl | |
| 2.6.956 | 3-Brom-4-tert.butyl-Phenyl | Methyl | (Ethylbutanoat)-3-yl | |
| 2.6.957 | 3-Methyl-4-tert.butyl-Phenyl | Methyl | (Ethylbutanoat)-3-yl | |
| 2.6.958 | 3-Cyclopropyl-4-tert.butyl-Phenyl | Methyl | (Ethylbutanoat)-3-yl | |
| 2.6.959 | 3-Cyano-4-tert.butyl-Phenyl | Methyl | (Ethylbutanoat)-3-yl | |
| 2.6.960 | 3-Trifluormethyl-4-tert.butyl-Phenyl | Methyl | (Ethylbutanoat)-3-yl | |
| 2.6.961 | 3-Methoxy-4-tert.butyl-Phenyl | Methyl | (Ethylbutanoat)-3-yl | |
| 2.6.962 | 3-Ethoxy-4-tert.butyl-Phenyl | Methyl | (Ethylbutanoat)-3-yl | |
| 2.6.963 | 3-Trifluormethoxy-4-tert.butyl-Phenyl | Methyl | (Ethylbutanoat)-3-yl | |
| 2.6.964 | 3-Nitro-4-tert.butyl-Phenyl | Methyl | (Ethylbutanoat)-3-yl | |
| 2.6.965 | 3-Fluor-4-cyclopropyl-Phenyl | Methyl | (Ethylbutanoat)-3-yl | |
| 2.6.966 | 3-Chlor-4-cyclopropyl-Phenyl | Methyl | (Ethylbutanoat)-3-yl | |
| 2.6.967 | 3-Brom-4-cyclopropyl-Phenyl | Methyl | (Ethylbutanoat)-3-yl | |
| 2.6.968 | 3-Methyl-4-cyclopropyl-Phenyl | Methyl | (Ethylbutanoat)-3-yl | |
| 2.6.969 | 3,4-Dicyclopropyl-Phenyl | Methyl | (Ethylbutanoat)-3-yl | |
| 2.6.970 | 3-Cyano-4-cyclopropyl-Phenyl | Methyl | (Ethylbutanoat)-3-yl | |
| 2.6.971 | 3-Trifluormethyl-4-cyclopropyl-Phenyl | Methyl | (Ethylbutanoat)-3-yl | |
| 2.6.972 | 3-Methoxy-4-cyclopropyl-Phenyl | Methyl | (Ethylbutanoat)-3-yl | |
| 2.6.973 | 3-Ethoxy-4-cyclopropyl-Phenyl | Methyl | (Ethylbutanoat)-3-yl | |
| 2.6.974 | 3-Trifluormethoxy-4-cyclopropyl-Phenyl | Methyl | (Ethylbutanoat)-3-yl | |
| 2.6.975 | 3-Fluor-4-methoxycarbonyl-Phenyl | Methyl | (Ethylbutanoat)-3-yl | |
| 2.6.976 | 3-Chlor-4-methoxycarbonyl-Phenyl | Methyl | (Ethylbutanoat)-3-yl | |
| 2.6.977 | 3-Brom-4-methoxycarbonyl-Phenyl | Methyl | (Ethylbutanoat)-3-yl | |
| 2.6.978 | 3-Methyl-4-methoxycarbonyl-Phenyl | Methyl | (Ethylbutanoat)-3-yl | |
| 2.6.979 | 3-Cyclopropyl-4-methoxycarbonyl-Phenyl | Methyl | (Ethylbutanoat)-3-yl | |
| 2.6.980 | 3-Cyano-4-methoxycarbonyl-Phenyl | Methyl | (Ethylbutanoat)-3-yl | |
| 2.6.981 | 3-Trifluormethylmethoxycarbonyl-Phenyl | Methyl | (Ethylbutanoat)-3-yl | |
| 2.6.982 | 3-Methoxy-4-methoxycarbonyl-Phenyl | Methyl | (Ethylbutanoat)-3-yl | |
| 2.6.983 | 3-Ethoxy-4-methoxycarbonyl-Phenyl | Methyl | (Ethylbutanoat)-3-yl | |
| 2.6.984 | 3-Trifluormethoxymethoxycarbonyl-Phenyl | Methyl | (Ethylbutanoat)-3-yl | |
| 2.6.985 | 3-Nitro-4-methoxycarbonyl-Phenyl | Methyl | (Ethylbutanoat)-3-yl | |
| 2.6.986 | 3-Fluor-4-cyano-Phenyl | Methyl | (Ethylbutanoat)-3-yl | |
| 2.6.987 | 3-Chlor-4-cyano-Phenyl | Methyl | (Ethylbutanoat)-3-yl | |
| 2.6.988 | 3-Brom-4-cyano-Phenyl | Methyl | (Ethylbutanoat)-3-yl | |
| 2.6.989 | 3-Methyl-4-cyano-Phenyl | Methyl | (Ethylbutanoat)-3-yl | |
| 2.6.990 | 3-Cyclopropyl-4-cyano-Phenyl | Methyl | (Ethylbutanoat)-3-yl | |
| 2.6.991 | 3-Cyano-4-cyano-Phenyl | Methyl | (Ethylbutanoat)-3-yl | |
| 2.6.992 | 3-Trifluormethyl-4-cyano-Phenyl | Methyl | (Ethylbutanoat)-3-yl | |
| 2.6.993 | 3-Methoxy-4-cyano-Phenyl | Methyl | (Ethylbutanoat)-3-yl | |
| 2.6.994 | 3-Ethoxy-4-cyano-Phenyl | Methyl | (Ethylbutanoat)-3-yl | |
| 2.6.995 | 3-Trifluormethoxy-4-cyano-Phenyl | Methyl | (Ethylbutanoat)-3-yl | |
| 2.6.996 | 3-Nitro-4-cyano-Phenyl | Methyl | (Ethylbutanoat)-3-yl | |
| 2.6.997 | 3-Fluor-4-methoxy-Phenyl | Methyl | (Ethylbutanoat)-3-yl | |
| 2.6.998 | 3-Chlor-4-methoxy-Phenyl | Methyl | (Ethylbutanoat)-3-yl | |
| 2.6.999 | 3-Brom-4-methoxy-Phenyl | Methyl | (Ethylbutanoat)-3-yl | |
| 2.6.1000 | 3-Methyl-4-methoxy-Phenyl | Methyl | (Ethylbutanoat)-3-yl | |
| 2.6.1001 | 3-Cyclopropyl-4-methoxy-Phenyl | Methyl | (Ethylbutanoat)-3-yl | |
| 2.6.1002 | 3-Cyano-4-methoxy-Phenyl | Methyl | (Ethylbutanoat)-3-yl | |
| 2.6.1003 | 3-Trifluormethyl-4-methoxy-Phenyl | Methyl | (Ethylbutanoat)-3-yl | |
| 2.6.1004 | 3,4-Dimethoxy-Phenyl | Methyl | (Ethylbutanoat)-3-yl | |
| 2.6.1005 | 3-Ethoxy-4-methoxy-Phenyl | Methyl | (Ethylbutanoat)-3-yl | |
| 2.6.1006 | 3-Trifluormethoxy-4-methoxy-Phenyl | Methyl | (Ethylbutanoat)-3-yl | |
| 2.6.1007 | 3-Nitro-4-methoxy-Phenyl | Methyl | (Ethylbutanoat)-3-yl | |
| 2.6.1008 | 3-Fluor-4-ethoxy-Phenyl | Methyl | (Ethylbutanoat)-3-yl | |
| 2.6.1009 | 3-Chlor-4-ethoxy-Phenyl | Methyl | (Ethylbutanoat)-3-yl | |
| 2.6.1010 | 3-Brom-4-ethoxy-Phenyl | Methyl | (Ethylbutanoat)-3-yl | |
| 2.6.1011 | 3-Methyl-4-ethoxy-Phenyl | Methyl | (Ethylbutanoat)-3-yl | |
| 2.6.1012 | 3-Cyclopropyl-4-ethoxy-Phenyl | Methyl | (Ethylbutanoat)-3-yl | |
| 2.6.1013 | 3-Cyano-4-ethoxy-Phenyl | Methyl | (Ethylbutanoat)-3-yl | |
| 2.6.1014 | 3-Trifluormethyl-4-ethoxy-Phenyl | Methyl | (Ethylbutanoat)-3-yl | |
| 2.6.1015 | 3-Methoxy-4-ethoxy-Phenyl | Methyl | (Ethylbutanoat)-3-yl | |
| 2.6.1016 | 2,4-Diethoxy-Phenyl | Methyl | (Ethylbutanoat)-3-yl | |
| 2.6.1017 | 3-Trifluormethoxy-4-ethoxy-Phenyl | Methyl | (Ethylbutanoat)-3-yl | |
| 2.6.1018 | 3-Nitro-4-ethoxy-Phenyl | Methyl | (Ethylbutanoat)-3-yl | |
| 2.6.1019 | 3-Fluor-4-isopropoxy-Phenyl | Methyl | (Ethylbutanoat)-3-yl | |
| 2.6.1020 | 3-Chlor-4-isopropoxy-Phenyl | Methyl | (Ethylbutanoat)-3-yl | |
| 2.6.1021 | 3-Brom-4-isopropoxy-Phenyl | Methyl | (Ethylbutanoat)-3-yl | |
| 2.6.1022 | 3-Methyl-4-isopropoxy-Phenyl | Methyl | (Ethylbutanoat)-3-yl | |
| 2.6.1023 | 3-Cyclopropyl-4-isopropoxy-Phenyl | Methyl | (Ethylbutanoat)-3-yl | |
| 2.6.1024 | 3-Cyano-4-isopropoxy-Phenyl | Methyl | (Ethylbutanoat)-3-yl | |
| 2.6.1025 | 3-Trifluormethyl-4-isopropoxy-Phenyl | Methyl | (Ethylbutanoat)-3-yl | |
| 2.6.1026 | 3-Methoxy-4-isopropoxy-Phenyl | Methyl | (Ethylbutanoat)-3-yl | |
| 2.6.1027 | 3-Ethoxy-4-isopropoxy-Phenyl | Methyl | (Ethylbutanoat)-3-yl | |
| 2.6.1028 | 3-Trifluormethoxy-4-isopropoxy-Phenyl | Methyl | (Ethylbutanoat)-3-yl | |
| 2.6.1029 | 3-Nitro-4-isopropoxy-Phenyl | Methyl | (Ethylbutanoat)-3-yl | |
| 2.6.1030 | 3-Fluor-4-trifluormethoxy-Phenyl | Methyl | (Ethylbutanoat)-3-yl | |
| 2.6.1031 | 3-Chlor-4-trifluormethoxy-Phenyl | Methyl | (Ethylbutanoat)-3-yl | |
| 2.6.1032 | 3-Brom-4-trifluormethoxy-Phenyl | Methyl | (Ethylbutanoat)-3-yl | |
| 2.6.1033 | 3-Methyl-4-trifluormethoxy-Phenyl | Methyl | (Ethylbutanoat)-3-yl | |
| 2.6.1034 | 3-Cyclopropyl-4-trifluormethoxy-Phenyl | Methyl | (Ethylbutanoat)-3-yl | |
| 2.6.1035 | 3-Cyano-4-trifluormethoxy-Phenyl | Methyl | (Ethylbutanoat)-3-yl | |
| 2.6.1036 | 3-Trifluormethyl-4-trifluormethoxy-Phenyl | Methyl | (Ethylbutanoat)-3-yl | |
| 2.6.1037 | 3-Methoxy-4-trifluormethoxy-Phenyl | Methyl | (Ethylbutanoat)-3-yl | |
| 2.6.1038 | 3-Ethoxy-4-trifluormethoxy-Phenyl | Methyl | (Ethylbutanoat)-3-yl | |
| 2.6.1039 | 3,4-Bis(trifluormethox y)-Phenyl | Methyl | (Ethylbutanoat)-3-yl | |
| 2.6.1040 | 3-Nitro-4-trifluormethoxy-Phenyl | Methyl | (Ethylbutanoat)-3-yl | |
| 2.6.1041 | 3-Fluor-4-difluormethoxy-Phenyl | Methyl | (Ethylbutanoat)-3-yl | |
| 2.6.1042 | 3-Chlor-4-difluormethoxy-Phenyl | Methyl | (Ethylbutanoat)-3-yl | |
| 2.6.1043 | 3-Brom-4-difluormethoxy-Phenyl | Methyl | (Ethylbutanoat)-3-yl | |
| 2.6.1044 | 3-Methyl-4-difluormethoxy-Phenyl | Methyl | (Ethylbutanoat)-3-yl | |
| 2.6.1045 | 3-Cyclopropyl-4-difluormethoxy-Phenyl | Methyl | (Ethylbutanoat)-3-yl | |
| 2.6.1046 | 3-Cyano-4-difluormethoxy-Phenyl | Methyl | (Ethylbutanoat)-3-yl | |
| 2.6.1047 | 3-Trifluormethyl-4-difluormethoxy-Phenyl | Methyl | (Ethylbutanoat)-3-yl | |
| 2.6.1048 | 3-Methoxy-4-difluormethoxy-Phenyl | Methyl | (Ethylbutanoat)-3-yl | |
| 2.6.1049 | 3-Ethoxy-4-difluormethoxy-Phenyl | Methyl | (Ethylbutanoat)-3-yl | |
| 2.6.1050 | 3-Trifluormethoxy-4-difluormethoxy-Phenyl | Methyl | (Ethylbutanoat)-3-yl | |
| 2.6.1051 | 3-Nitro-4-difluormethoxy-Phenyl | Methyl | (Ethylbutanoat)-3-yl | |
| 2.6.1052 | 3-Fluor-4-nitro-Phenyl | Methyl | (Ethylbutanoat)-3-yl | |
| 2.6.1053 | 3-Chlor-4-nitro-Phenyl | Methyl | (Ethylbutanoat)-3-yl | |
| 2.6.1054 | 3-Brom-4-nitro-Phenyl | Methyl | (Ethylbutanoat)-3-yl | |
| 2.6.1055 | 3-Methyl-4-nitro-Phenyl | Methyl | (Ethylbutanoat)-3-yl | |
| 2.6.1056 | 3-Ethyl-4-nitro-Phenyl | Methyl | (Ethylbutanoat)-3-yl | |
| 2.6.1057 | 3-Cyclopropyl-4-nitro-Phenyl | Methyl | (Ethylbutanoat)-3-yl | |
| 2.6.1058 | 3-Cyano-4-nitro-Phenyl | Methyl | (Ethylbutanoat)-3-yl | |
| 2.6.1059 | 3-Trifluormethyl-4-nitro-Phenyl | Methyl | (Ethylbutanoat)-3-yl | |
| 2.6.1060 | 3-Methoxy-4-nitro-Phenyl | Methyl | (Ethylbutanoat)-3-yl | |
| 2.6.1061 | 3-Ethoxy-4-nitro-Phenyl | Methyl | (Ethylbutanoat)-3-yl | |
| 2.6.1062 | 3-Trifluormethoxy-4-nitro-Phenyl | Methyl | (Ethylbutanoat)-3-yl | |
| 2.6.1063 | 3-Fluor-4-methylsulfanylPhe nyl | Methyl | (Ethylbutanoat)-3-yl | |
| 2.6.1064 | 3-Chlor-4-methylsulfanyl-Phenyl | Methyl | (Ethylbutanoat)-3-yl | |
| 2.6.1065 | 3-Brom-4-methylsulfanyl-Phenyl | Methyl | (Ethylbutanoat)-3-yl | |
| 2.6.1066 | 3-Methyl-4-methylsulfanyl-Phenyl | Methyl | (Ethylbutanoat)-3-yl | |
| 2.6.1067 | 3-Cyclopropyl-4-methylsulfanyl-Phenyl | Methyl | (Ethylbutanoat)-3-yl | |
| 2.6.1068 | 3-Cyano-4-methylsulfanyl-Phenyl | Methyl | (Ethylbutanoat)-3-yl | |
| 2.6.1069 | 3-Trifluormethyl-4-methylsulfanyl-Phenyl | Methyl | (Ethylbutanoat)-3-yl | |
| 2.6.1070 | 3-Methoxy-4-methylsulfanyl-Phenyl | Methyl | (Ethylbutanoat)-3-yl | |
| 2.6.1071 | 3-Ethoxy-4-methylsulfanyl-Phenyl | Methyl | (Ethylbutanoat)-3-yl | |
| 2.6.1072 | 3-Trifluormethoxy-4-methylsulfanyl-Phenyl | Methyl | (Ethylbutanoat)-3-yl | |
| 2.6.1073 | 3-Nitro-4-methylsulfanyl-Phenyl | Methyl | (Ethylbutanoat)-3-yl | |
| 2.6.1074 | 3,6-Difluor-Phenyl | Methyl | (Ethylbutanoat)-3-yl | |
| 2.6.1075 | 3-Chlor-6-fluor-Phenyl | Methyl | (Ethylbutanoat)-3-yl | |
| 2.6.1076 | 3-Brom-6-fluor-Phenyl | Methyl | (Ethylbutanoat)-3-yl | |
| 2.6.1077 | 3-Methyl-6-fluor-Phenyl | Methyl | (Ethylbutanoat)-3-yl | |
| 2.6.1078 | 3-Ethyl-6-fluor-Phenyl | Methyl | (Ethylbutanoat)-3-yl | |
| 2.6.1079 | 3-Cyclopropyl-6-fluor-Phenyl | Methyl | (Ethylbutanoat)-3-yl | |
| 2.6.1080 | 3-Cyano-6-fluor-Phenyl | Methyl | (Ethylbutanoat)-3-yl | |
| 2.6.1081 | 3-Methoxy-6-fluor-Phenyl | Methyl | (Ethylbutanoat)-3-yl | |
| 2.6.1082 | 3-Ethoxy-6-fluor-Phenyl | Methyl | (Ethylbutanoat)-3-yl | |
| 2.6.1083 | 3-Trifluormethoxy-6-fluor-Phenyl | Methyl | (Ethylbutanoat)-3-yl | |
| 2.6.1084 | 3-Nitro-6-fluor-Phenyl | Methyl | (Ethylbutanoat)-3-yl | |
| 2.6.1085 | 3-Fluor-6-chlor-Phenyl | Methyl | (Ethylbutanoat)-3-yl | |
| 2.6.1086 | 3,6-Dichlor-Phenyl | Methyl | (Ethylbutanoat)-3-yl | |
| 2.6.1087 | 3-Brom-6-chlor-Phenyl | Methyl | (Ethylbutanoat)-3-yl | |
| 2.6.1088 | 3-Methyl-6-chlor-Phenyl | Methyl | (Ethylbutanoat)-3-yl | |
| 2.6.1089 | 3-Cyclopropyl-6-chlor-Phenyl | Methyl | (Ethylbutanoat)-3-yl | |
| 2.6.1090 | 3-Cyano-6-chlor-Phenyl | Methyl | (Ethylbutanoat)-3-yl | |
| 2.6.1091 | 3-Trifluormethyl-6-chlor-Phenyl | Methyl | (Ethylbutanoat)-3-yl | |
| 2.6.1092 | 3-Methoxy-6-chlor-Phenyl | Methyl | (Ethylbutanoat)-3-yl | |
| 2.6.1093 | 3-Ethoxy-6-chlor-Phenyl | Methyl | (Ethylbutanoat)-3-yl | |
| 2.6.1094 | 3-Trifluormethoxy-6-chlor-Phenyl | Methyl | (Ethylbutanoat)-3-yl | |
| 2.6.1095 | 3-Nitro-6-chlor-Phenyl | Methyl | (Ethylbutanoat)-3-yl | |
| 2.6.1096 | 3-Fluor-6-brom-Phenyl | Methyl | (Ethylbutanoat)-3-yl | |
| 2.6.1097 | 3-Chlor-6-brom-Phenyl | Methyl | (Ethylbutanoat)-3-yl | |
| 2.6.1098 | 3,6-Dibrom-Phenyl | Methyl | (Ethylbutanoat)-3-yl | |
| 2.6.1099 | 3-Methyl-6-brom-Phenyl | Methyl | (Ethylbutanoat)-3-yl | |
| 2.6.1100 | 3-Ethyl-6-brom-Phenyl | Methyl | (Ethylbutanoat)-3-yl | |
| 2.6.1101 | 3-Cyclopropyl-6-brom-Phenyl | Methyl | (Ethylbutanoat)-3-yl | |
| 2.6.1102 | 3-Cyano-6-brom-Phenyl | Methyl | (Ethylbutanoat)-3-yl | |
| 2.6.1103 | 3-Trifluormethyl-6-brom-Phenyl | Methyl | (Ethylbutanoat)-3-yl | |
| 2.6.1104 | 3-Methoxy-6-Phenyl | Methyl | (Ethylbutanoat)-3-yl | |
| 2.6.1105 | 3-Ethoxy-6-brom-Phenyl | Methyl | (Ethylbutanoat)-3-yl | |
| 2.6.1106 | 3-Trifluormethoxy-6-brom-Phenyl | Methyl | (Ethylbutanoat)-3-yl | |
| 2.6.1107 | 3-Nitro-6-brom-Phenyl | Methyl | (Ethylbutanoat)-3-yl | |
| 2.6.1108 | 3-Fluor-6-iod-Phenyl | Methyl | (Ethylbutanoat)-3-yl | |
| 2.6.1109 | 3-Chlor-6-iod-Phenyl | Methyl | (Ethylbutanoat)-3-yl | |
| 2.6.1110 | 3-Brom-6-iod-Phenyl | Methyl | (Ethylbutanoat)-3-yl | |
| 2.6.1111 | 3-Methyl-6-iod-Phenyl | Methyl | (Ethylbutanoat)-3-yl | |
| 2.6.1112 | 3-Cyclopropyl-6-iod-Phenyl | Methyl | (Ethylbutanoat)-3-yl | |
| 2.6.1113 | 3-Cyano-6-iod-Phenyl | Methyl | (Ethylbutanoat)-3-yl | |
| 2.6.1114 | 3-Trifluormethyl-6-iod-Phenyl | Methyl | (Ethylbutanoat)-3-yl | |
| 2.6.1115 | 3-Methoxy-6-iod-Phenyl | Methyl | (Ethylbutanoat)-3-yl | |
| 2.6.1116 | 3-Ethoxy-6-iod-Phenyl | Methyl | (Ethylbutanoat)-3-yl | |
| 2.6.1117 | 3-Trifluormethoxy-6-iod-Phenyl | Methyl | (Ethylbutanoat)-3-yl | |
| 2.6.1118 | 3-Nitro-6-iod-Phenyl | Methyl | (Ethylbutanoat)-3-yl | |
| 2.6.1119 | 3-Fluor-6-methyl-Phenyl | Methyl | (Ethylbutanoat)-3-yl | |
| 2.6.1120 | 3-Chlor-6-methyl-Phenyl | Methyl | (Ethylbutanoat)-3-yl | |
| 2.6.1121 | 3-Brom-6-methyl-Phenyl | Methyl | (Ethylbutanoat)-3-yl | |
| 2.6.1122 | 3.6-Dimethyl-Phenyl | Methyl | (Ethylbutanoat)-3-yl | |
| 2.6.1123 | 3-Cyclopropyl-6-methyl-Phenyl | Methyl | (Ethylbutanoat)-3-yl | |
| 2.6.1124 | 3-Cyano-6-methyl-Phenyl | Methyl | (Ethylbutanoat)-3-yl | |
| 2.6.1125 | 3-Trifluormethyl-6-methyl-Phenyl | Methyl | (Ethylbutanoat)-3-yl | |
| 2.6.1126 | 3-Methoxy-6-methyl-Phenyl | Methyl | (Ethylbutanoat)-3-yl | |
| 2.6.1127 | 3-Ethoxy-6-methyl-Phenyl | Methyl | (Ethylbutanoat)-3-yl | |
| 2.6.1128 | 3-Trifluormethoxy-6-methyl-Phenyl | Methyl | (Ethylbutanoat)-3-yl | |
| 2.6.1129 | 3-Nitro-6-methyl-Phenyl | Methyl | (Ethylbutanoat)-3-yl | |
| 2.6.1130 | 3-Fluor-6-ethyl-Phenyl | Methyl | (Ethylbutanoat)-3-yl | |
| 2.6.1131 | 3-Chlor-6-ethyl-Phenyl | Methyl | (Ethylbutanoat)-3-yl | |
| 2.6.1132 | 3-Brom-6-ethyl-Phenyl | Methyl | (Ethylbutanoat)-3-yl | |
| 2.6.1133 | 3-Methyl-6-ethyl-Phenyl | Methyl | (Ethylbutanoat)-3-yl | |
| 2.6.1134 | 3,6-Diethyl-Phenyl | Methyl | (Ethylbutanoat)-3-yl | |
| 2.6.1135 | 3-Cyclopropyl-6-ethyl-Phenyl | Methyl | (Ethylbutanoat)-3-yl | |
| 2.6.1136 | 3-Cyano-6-ethyl-Phenyl | Methyl | (Ethylbutanoat)-3-yl | |
| 2.6.1137 | 3-Trifluormethyl-6-ethyl-Phenyl | Methyl | (Ethylbutanoat)-3-yl | |
| 2.6.1138 | 3-Methoxy-6-ethyl-Phenyl | Methyl | (Ethylbutanoat)-3-yl | |
| 2.6.1139 | 3-Ethoxy-6-ethyl-Phenyl | Methyl | (Ethylbutanoat)-3-yl | |
| 2.6.1140 | 3-Trifluormethoxy-6-ethyl-Phenyl | Methyl | (Ethylbutanoat)-3-yl | |
| 2.6.1141 | 3-Nitro-6-ethyl-Phenyl | Methyl | (Ethylbutanoat)-3-yl | |
| 2.6.1142 | 3-Fluor-6-isopropyl-Phenyl | Methyl | (Ethylbutanoat)-3-yl | |
| 2.6.1143 | 3-Chlor-6-isopropyl-Phenyl | Methyl | (Ethylbutanoat)-3-yl | |
| 2.6.1144 | 3-Brom-6-isopropyl-Phenyl | Methyl | (Ethylbutanoat)-3-yl | |
| 2.6.1145 | 3-Methyl-6-isopropyl-Phenyl | Methyl | (Ethylbutanoat)-3-yl | |
| 2.6.1146 | 3-Ethyl-6-isopropyl-Phenyl | Methyl | (Ethylbutanoat)-3-yl | |
| 2.6.1147 | 3-Cyclopropyl-6-isopropyl-Phenyl | Methyl | (Ethylbutanoat)-3-yl | |
| 2.6.1148 | 3-Cyano-6-isopropyl-Phenyl | Methyl | (Ethylbutanoat)-3-yl | |
| 2.6.1149 | 3-Trifluormethyl-6-isopropyl-Phenyl | Methyl | (Ethylbutanoat)-3-yl | |
| 2.6.1150 | 3-Methoxy-6-isopropyl-Phenyl | Methyl | (Ethylbutanoat)-3-yl | |
| 2.6.1151 | 3-Ethoxy-6-isopropyl-Phenyl | Methyl | (Ethylbutanoat)-3-yl | |
| 2.6.1152 | 3-Trifluormethoxy-6-isopropyl-Phenyl | Methyl | (Ethylbutanoat)-3-yl | |
| 2.6.1153 | 3-Nitro-6-isopropyl-Phenyl | Methyl | (Ethylbutanoat)-3-yl | |
| 2.6.1154 | 3-Fluor-6-tert. butyl-Phenyl | Methyl | (Ethylbutanoat)-3-yl | |
| 2.6.1155 | 3-Chlor-6-tert. butyl-Phenyl | Methyl | (Ethylbutanoat)-3-yl | |
| 2.6.1156 | 3-Brom-6-tert. butyl-Phenyl | Methyl | (Ethylbutanoat)-3-yl | |
| 2.6.1157 | 3-Methyl-6-tert. butyl-Phenyl | Methyl | (Ethylbutanoat)-3-yl | |
| 2.6.1158 | 3-Cyclopropyl-6-tert. butyl-Phenyl | Methyl | (Ethylbutanoat)-3-yl | |
| 2.6.1159 | 3-Cyano-6-tert. butyl-Phenyl | Methyl | (Ethylbutanoat)-3-yl | |
| 2.6.1160 | 3-Trifluormethyl-6-tert.butyl-Phenyl | Methyl | (Ethylbutanoat)-3-yl | |
| 2.6.1161 | 3-Methoxy-6-tert.butyl-Phenyl | Methyl | (Ethylbutanoat)-3-yl | |
| 2.6.1162 | 3-Ethoxy-6-tert.butyl-Phenyl | Methyl | (Ethylbutanoat)-3-yl | |
| 2.6.1163 | 3-Trifluormethoxy-6-tert.butyl-Phenyl | Methyl | (Ethylbutanoat)-3-yl | |
| 2.6.1164 | 3-Nitro-6-tert. butyl-Phenyl | Methyl | (Ethylbutanoat)-3-yl | |
| 2.6.1165 | 3-Fluor-6-cyclopropyl-Phenyl | Methyl | (Ethylbutanoat)-3-yl | |
| 2.6.1166 | 3-Chlor-6-cyclopropyl-Phenyl | Methyl | (Ethylbutanoat)-3-yl | |
| 2.6.1167 | 3-Brom-6-cyclopropyl-Phenyl | Methyl | (Ethylbutanoat)-3-yl | |
| 2.6.1168 | 3-Methyl-6-cyclopropyl-Phenyl | Methyl | (Ethylbutanoat)-3-yl | |
| 2.6.1169 | 3-Cyano-6-cyclopropyl-Phenyl | Methyl | (Ethylbutanoat)-3-yl | |
| 2.6.1170 | 3-Trifluormethyl-6-cyclopropyl-Phenyl | Methyl | (Ethylbutanoat)-3-yl | |
| 2.6.1171 | 3-Methoxy-6-cyclopropyl-Phenyl | Methyl | (Ethylbutanoat)-3-yl | |
| 2.6.1172 | 3-Ethoxy-6-cyclopropyl-Phenyl | Methyl | (Ethylbutanoat)-3-yl | |
| 2.6.1173 | 3-Trifluormethoxy-6-cyclopropyl-Phenyl | Methyl | (Ethylbutanoat)-3-yl | |
| 2.6.1174 | 3-Fluor-6-methoxycarbonyl-Phenyl | Methyl | (Ethylbutanoat)-3-yl | |
| 2.6.1175 | 3-Chlor-6-methoxycarbonyl-Phenyl | Methyl | (Ethylbutanoat)-3-yl | |
| 2.6.1176 | 3-Brom-6-methoxycarbonyl-Phenyl | Methyl | (Ethylbutanoat)-3-yl | |
| 2.6.1177 | 3-Methyl-6-methoxycarbonyl-Phenyl | Methyl | (Ethylbutanoat)-3-yl | |
| 2.6.1178 | 3-Cyclopropyl-6-methoxycarbonyl-Phenyl | Methyl | (Ethylbutanoat)-3-yl | |
| 2.6.1179 | 3-Cyano-6-methoxycarbonyl-Phenyl | Methyl | (Ethylbutanoat)-3-yl | |
| 2.6.1180 | 3-Trifluormethylmethoxycarbonyl-Phenyl | Methyl | (Ethylbutanoat)-3-yl | |
| 2.6.1181 | 3-Methoxy-6-methoxycarbonyl -Phenyl | Methyl | (Ethylbutanoat)-3-yl | |
| 2.6.1182 | 3-Ethoxy-6-methoxycarbonyl-Phenyl | Methyl | (Ethylbutanoat)-3-yl | |
| 2.6.1183 | 3-Trifluormethoxymethoxycarbonyl-Phenyl | Methyl | (Ethylbutanoat)-3-yl | |
| 2.6.1184 | 3-Nitro-6-methoxycarbonyl-Phenyl | Methyl | (Ethylbutanoat)-3-yl | |
| 2.6.1185 | 3-Fluor-6-cyano-Phenyl | Methyl | (Ethylbutanoat)-3-yl | |
| 2.6.1186 | 3-Chlor-6-cyano-Phenyl | Methyl | (Ethylbutanoat)-3-yl | |
| 2.6.1187 | 3-Brom-6-cyano-Phenyl | Methyl | (Ethylbutanoat)-3-yl | |
| 2.6.1188 | 3-Methyl-6-cyano-Phenyl | Methyl | (Ethylbutanoat)-3-yl | |
| 2.6.1189 | 3-Cyclopropyl-6-cyano-Phenyl | Methyl | (Ethylbutanoat)-3-yl | |
| 2.6.1190 | 3-Cyano-6-cyanoPhenyl | Methyl | (Ethylbutanoat)-3-yl | |
| 2.6.1191 | 3-Trifluormethyl-6-cyano-Phenyl | Methyl | (Ethylbutanoat)-3-yl | |
| 2.6.1192 | 3-Methoxy-6-cyano -Phenyl | Methyl | (Ethylbutanoat)-3-yl | |
| 2.6.1193 | 3-Ethoxy-6-cyano-Phenyl | Methyl | (Ethylbutanoat)-3-yl | |
| 2.6.1194 | 3-Trifluormethoxy-6-cyano-Phenyl | Methyl | (Ethylbutanoat)-3-yl | |
| 2.6.1195 | 3-Nitro-6-cyano-Phenyl | Methyl | (Ethylbutanoat)-3-yl | |
| 2.6.1196 | 3-Fluor-6-methoxy-Phenyl | Methyl | (Ethylbutanoat)-3-yl | |
| 2.6.1197 | 3-Chlor-6-methoxy-Phenyl | Methyl | (Ethylbutanoat)-3-yl | |
| 2.6.1198 | 3-Brom-6-methoxy-Phenyl | Methyl | (Ethylbutanoat)-3-yl | |
| 2.6.1199 | 3-Methyl-6-methoxy-Phenyl | Methyl | (Ethylbutanoat)-3-yl | |
| 2.6.1200 | 3-Cyclopropyl-6-methoxy-Phenyl | Methyl | (Ethylbutanoat)-3-yl | |
| 2.6.1201 | 3-Cyano-6-methoxy-Phenyl | Methyl | (Ethylbutanoat)-3-yl | |
| 2.6.1202 | 3-Trifluormethyl-6-methoxy-Phenyl | Methyl | (Ethylbutanoat)-3-yl | |
| 2.6.1203 | 3,6-Dimethoxy-Phenyl | Methyl | (Ethylbutanoat)-3-yl | |
| 2.6.1204 | 3-Ethoxy-6-methoxy-Phenyl | Methyl | (Ethylbutanoat)-3-yl | |
| 2.6.1205 | 3-Trifluormethoxy-6-methoxy-Phenyl | Methyl | (Ethylbutanoat)-3-yl | |
| 2.6.1206 | 3-Nitro-6-methoxy-Phenyl | Methyl | (Ethylbutanoat)-3-yl | |
| 2.6.1207 | 3-Fluor-6-ethoxy-Phenyl | Methyl | (Ethylbutanoat)-3-yl | |
| 2.6.1208 | 3-Chlor-6-ethoxy-Phenyl | Methyl | (Ethylbutanoat)-3-yl | |
| 2.6.1209 | 3-Brom-6-ethoxy-Phenyl | Methyl | (Ethylbutanoat)-3-yl | |
| 2.6.1210 | 3-Methyl-6-ethoxy-Phenyl | Methyl | (Ethylbutanoat)-3-yl | |
| 2.6.1211 | 3-Cyclopropyl-6-ethoxy-Phenyl | Methyl | (Ethylbutanoat)-3-yl | |
| 2.6.1212 | 3-Cyano-6-ethoxy-Phenyl | Methyl | (Ethylbutanoat)-3-yl | |
| 2.6.1213 | 3-Trifluormethyl-6-ethoxy-Phenyl | Methyl | (Ethylbutanoat)-3-yl | |
| 2.6.1214 | 3-Methoxy-6-ethoxy -Phenyl | Methyl | (Ethylbutanoat)-3-yl | |
| 2.6.1215 | 2,6-Diethoxy-Phenyl | Methyl | (Ethylbutanoat)-3-yl | |
| 2.6.1216 | 3-Trifluormethoxy-6-ethoxy-Phenyl | Methyl | (Ethylbutanoat)-3-yl | |
| 2.6.1217 | 3-Nitro-6-ethoxy-Phenyl | Methyl | (Ethylbutanoat)-3-yl | |
| 2.6.1218 | 3-Fluor-6-propoxy-Phenyl | Methyl | (Ethylbutanoat)-3-yl | |
| 2.6.1219 | 3-Chlor-6-propoxy-Phenyl | Methyl | (Ethylbutanoat)-3-yl | |
| 2.6.1220 | 3-Brom-6-propoxy-Phenyl | Methyl | (Ethylbutanoat)-3-yl | |
| 2.6.1221 | 3-Methyl-6-propoxy-Phenyl | Methyl | (Ethylbutanoat)-3-yl | |
| 2.6.1222 | 3-Cyclopropyl-6-propoxy-Phenyl | Methyl | (Ethylbutanoat)-3-yl | |
| 2.6.1223 | 3-Cyano-6-propoxy-Phenyl | Methyl | (Ethylbutanoat)-3-yl | |
| 2.6.1224 | 3-Trifluormethyl-6-propoxy-Phenyl | Methyl | (Ethylbutanoat)-3-yl | |
| 2.6.1225 | 3-Methoxy-6-propoxy -Phenyl | Methyl | (Ethylbutanoat)-3-yl | |
| 2.6.1226 | 3-Ethoxy-6-propoxy-Phenyl | Methyl | (Ethylbutanoat)-3-yl | |
| 2.6.1227 | 3-Trifluormethoxy-6-propoxy-Phenyl | Methyl | (Ethylbutanoat)-3-yl | |
| 2.6.1228 | 3-Nitro-6-propoxy-Phenyl | Methyl | (Ethylbutanoat)-3-yl | |
| 2.6.1229 | 3-Fluor-6-isopropoxy-Phenyl | Methyl | (Ethylbutanoat)-3-yl | |
| 2.6.1230 | 3-Chlor-6-isopropoxy-Phenyl | Methyl | (Ethylbutanoat)-3-yl | |
| 2.6.1231 | 3-Brom-6-isopropoxy-Phenyl | Methyl | (Ethylbutanoat)-3-yl | |
| 2.6.1232 | 3-Methyl-6-isopropoxy-Phenyl | Methyl | (Ethylbutanoat)-3-yl | |
| 2.6.1233 | 3-Cyclopropyl-6-isopropoxy-Phenyl | Methyl | (Ethylbutanoat)-3-yl | |
| 2.6.1234 | 3-Cyano-6-isopropoxy-Phenyl | Methyl | (Ethylbutanoat)-3-yl | |
| 2.6.1235 | 3-Trifluormethyl-6-isopropoxy-Phenyl | Methyl | (Ethylbutanoat)-3-yl | |
| 2.6.1236 | 3-Methoxy-6-isopropoxy-Phenyl | Methyl | (Ethylbutanoat)-3-yl | |
| 2.6.1237 | 3-Ethoxy-6-isopropoxy-Phenyl | Methyl | (Ethylbutanoat)-3-yl | |
| 2.6.1238 | 3-Trifluormethoxy-6-isopropoxy-Phenyl | Methyl | (Ethylbutanoat)-3-yl | |
| 2.6.1239 | 3-Nitro-6-isopropoxy-Phenyl | Methyl | (Ethylbutanoat)-3-yl | |
| 2.6.1240 | 3-Fluor-6-trifluormethoxy-Phenyl | Methyl | (Ethylbutanoat)-3-yl | |
| 2.6.1241 | 3-Chlor-6-trifluormethoxy-Phenyl | Methyl | (Ethylbutanoat)-3-yl | |
| 2.6.1242 | 3-Brom-6-trifluormethoxy-Phenyl | Methyl | (Ethylbutanoat)-3-yl | |
| 2.6.1243 | 3-Methyl-6-trifluormethoxy-Phenyl | Methyl | (Ethylbutanoat)-3-yl | |
| 2.6.1244 | 3-Cyclopropyl-6-trifluormethoxy-Phenyl | Methyl | (Ethylbutanoat)-3-yl | |
| 2.6.1245 | 3-Cyano-6-trifluormethoxy-Phenyl | Methyl | (Ethylbutanoat)-3-yl | |
| 2.6.1246 | 3-Trifluormethyl-6-trifluormethoxy-Phenyl | Methyl | (Ethylbutanoat)-3-yl | |
| 2.6.1247 | 3-Methoxy-6-trifluormethoxy-Phenyl | Methyl | (Ethylbutanoat)-3-yl | |
| 2.6.1248 | 3-Ethoxy-6-trifluormethoxy-Phenyl | Methyl | (Ethylbutanoat)-3-yl | |
| 2.6.1249 | 3,6-Bis(trifluormethox y)-Phenyl | Methyl | (Ethylbutanoat)-3-yl | |
| 2.6.1250 | 3-Nitro-6-trifluormethoxy-Phenyl | Methyl | (Ethylbutanoat)-3-yl | |
| 2.6.1251 | 3-Fluor-6-difluormethoxy-Phenyl | Methyl | (Ethylbutanoat)-3-yl | |
| 2.6.1252 | 3-Chlor-6-difluormethoxy-Phenyl | Methyl | (Ethylbutanoat)-3-yl | |
| 2.6.1253 | 3-Brom-6-difluormethoxy-Phenyl | Methyl | (Ethylbutanoat)-3-yl | |
| 2.6.1254 | 3-Methyl-6-difluormethoxy-Phenyl | Methyl | (Ethylbutanoat)-3-yl | |
| 2.6.1255 | 3-Cyclopropyl-6-difluormethoxy-Phenyl | Methyl | (Ethylbutanoat)-3-yl | |
| 2.6.1256 | 3-Cyano-6-difluormethoxy-Phenyl | Methyl | (Ethylbutanoat)-3-yl | |
| 2.6.1257 | 3-Trifluormethyl-6-difluormethoxy-Phenyl | Methyl | (Ethylbutanoat)-3-yl | |
| 2.6.1258 | 3-Methoxy-6-difluormethoxy-Phenyl | Methyl | (Ethylbutanoat)-3-yl | |
| 2.6.1259 | 3-Ethoxy-6-difluormethoxy-Phenyl | Methyl | (Ethylbutanoat)-3-yl | |
| 2.6.1260 | 3-Trifluormethoxy-6-difluormethoxy-Phenyl | Methyl | (Ethylbutanoat)-3-yl | |
| 2.6.1261 | 3-Nitro-6-difluormethoxy-Phenyl | Methyl | (Ethylbutanoat)-3-yl | |
| 2.6.1262 | 3-Fluor-6-nitro-Phenyl | Methyl | (Ethylbutanoat)-3-yl | |
| 2.6.1263 | 3-Chlor-6-nitro-Phenyl | Methyl | (Ethylbutanoat)-3-yl | |
| 2.6.1264 | 3-Brom-6-nitro-Phenyl | Methyl | (Ethylbutanoat)-3-yl | |
| 2.6.1265 | 3-Methyl-6-nitro-Phenyl | Methyl | (Ethylbutanoat)-3-yl | |
| 2.6.1266 | 3-Cyclopropyl-6-nitro-Phenyl | Methyl | (Ethylbutanoat)-3-yl | |
| 2.6.1267 | 3-Cyano-6-n itro-Phenyl | Methyl | (Ethylbutanoat)-3-yl | |
| 2.6.1268 | 3-Trifluormethyl-6-nitro-Phenyl | Methyl | (Ethylbutanoat)-3-yl | |
| 2.6.1269 | 3-Methoxy-6-nitro-Phenyl | Methyl | (Ethylbutanoat)-3-yl | |
| 2.6.1270 | 3-Ethoxy-6-nitro-Phenyl | Methyl | (Ethylbutanoat)-3-yl | |
| 2.6.1271 | 3-Trifluormethoxy-6-nitro-Phenyl | Methyl | (Ethylbutanoat)-3-yl | |
| 2.6.1272 | 3-Fluor-6-methylsulfanylPhe nyl | Methyl | (Ethylbutanoat)-3-yl | |
| 2.6.1273 | 3-Chlor-6-methylsulfanyl-Phenyl | Methyl | (Ethylbutanoat)-3-yl | |
| 2.6.1274 | 3-Brom-6-methylsulfanyl-Phenyl | Methyl | (Ethylbutanoat)-3-yl | |
| 2.6.1275 | 3-Methyl-6-methylsulfanyl-Phenyl | Methyl | (Ethylbutanoat)-3-yl | |
| 2.6.1276 | 3-Cyclopropyl-6-methylsulfanyl-Phenyl | Methyl | (Ethylbutanoat)-3-yl | |
| 2.6.1277 | 3-Cyano-6-methylsulfanyl - Phenyl | Methyl | (Ethylbutanoat)-3-yl | |
| 2.6.1278 | 3-Trifluormethyl-6-methylsulfanyl-Phenyl | Methyl | (Ethylbutanoat)-3-yl | |
| 2.6.1279 | 3-Methoxy-6-methylsulfanyl - Phenyl | Methyl | (Ethylbutanoat)-3-yl | |
| 2.6.1280 | 3-Ethoxy-6-methylsulfanyl-Phenyl | Methyl | (Ethylbutanoat)-3-yl | |
| 2.6.1281 | 3-Trifluormethoxy-6-methylsulfanyl-Phenyl | Methyl | (Ethylbutanoat)-3-yl | |
| 2.6.1282 | 3-Nitro-6-methylsulfanyl-Phenyl | Methyl | (Ethylbutanoat)-3-yl | |
| 2.6.1283 | 2,3,4-Trifluor-Phenyl | Methyl | (Ethylbutanoat)-3-yl | |
| 2.6.1284 | 2,3,4-Trichlor-Phenyl | Methyl | (Ethylbutanoat)-3-yl | |
| 2.6.1285 | 2,3.4-Trimethyl-Phenyl | Methyl | (Ethylbutanoat)-3-yl | |
| 2.6.1286 | 2-Fluor-2-chlor-5-trifluormethyl-Phenyl | Methyl | (Ethylbutanoat)-3-yl | |
| 2.6.1287 | 2,3,5-Trifluor-Phenyl | Methyl | (Ethylbutanoat)-3-yl | |
| 2.6.1288 | 2,3,5-Trichlor-Phenyl | Methyl | (Ethylbutanoat)-3-yl | |
| 2.6.1289 | 2,3,5-Trimethyl-Phenyl | Methyl | (Ethylbutanoat)-3-yl | |
| 2.6.1290 | 2,3-Dichlor-5-methoxy-Phenyl | Methyl | (Ethylbutanoat)-3-yl | |
| 2.6.1291 | 2,3,6-Trifluor-Phenyl | Methyl | (Ethylbutanoat)-3-yl | |
| 2.6.1292 | 2,3,6-Trichlor-Phenyl | Methyl | (Ethylbutanoat)-3-yl | |
| 2.6.1293 | 2,3,6-Trimethyl-Phenyl | Methyl | (Ethylbutanoat)-3-yl | |
| 2.6.1294 | 3,4,5-Trifluor-Phenyl | Methyl | (Ethylbutanoat)-3-yl | |
| 2.6.1295 | 3,4,5-Trichlor-Phenyl | Methyl | (Ethylbutanoat)-3-yl | |
| 2.6.1296 | 3,4,5-Trimethyl-Phenyl | Methyl | (Ethylbutanoat)-3-yl | |
| 2.6.1297 | 3,5-Dimethyl-4-fluor-Phenyl | Methyl | (Ethylbutanoat)-3-yl | |
| 2.6.1298 | 3,5-Dichlor-4-methoxy-Phenyl | Methyl | (Ethylbutanoat)-3-yl | |
| 2.6.1299 | 3,5-Difluor-4-chlor-Phenyl | Methyl | (Ethylbutanoat)-3-yl | |
| 2.6.1300 | 3,5-Dichlor-4-hyd roxy-Phenyl | Methyl | (Ethylbutanoat)-3-yl | |
| 2.6.1301 | 3,5-Trifluormethyl-4-chlor-Phenyl | Methyl | (Ethylbutanoat)-3-yl | |
| 2.6.1302 | 3,4,6-Trifluor-Phenyl | Methyl | (Ethylbutanoat)-3-yl | |
| 2.6.1303 | 3,4,6-Trichlor-Phenyl | Methyl | (Ethylbutanoat)-3-yl | |
| 2.6.1304 | 3,4,6-Trimethyl-Phenyl | Methyl | (Ethylbutanoat)-3-yl | |
| 2.6.1305 | 2,3,4,5-Pentafluor-Phenyl | Methyl | (Ethylbutanoat)-3-yl | |

**Tabelle 3: Erfindungsgemäße Verbindungen der allgemeinen Formel (I), worin W* für CN, R¹ und R² für Wasserstoff stehen, und Aryl den Rest**

| | | | |
|---|---|---|---|
| 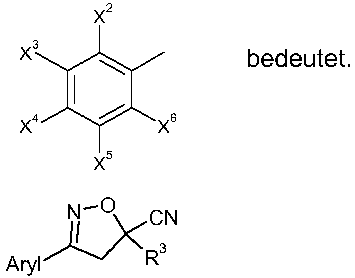 | | | |

| Nr. | Aryl | R³ | Physikalische Daten |
|---|---|---|---|
| 3.001 | 3-Fluor-Phenyl | Methyl | |
| 3.002 | 3-Fluor-Phenyl | Ethyl | |
| 3.003 | 3-Fluor-Phenyl | CN | |
| 3.004 | 3-Fluor-Phenyl | Fluormethyl | |
| 3.005 | 3-Fluor-Phenyl | Hydroxymethyl | |
| 3.006 | 3-Fluor-Phenyl | Vinyl | |
| 3.007 | 3-Fluor-Phenyl | 1-Chlorvinyl | |
| 3.008 | 3-Fluor-Phenyl | Ethinyl | |
| 3.009 | 3-Chlor-Phenyl | Methyl | |
| 3.010 | 3-Chlor-Phenyl | Ethyl | |
| 3.011 | 3-Chlor-Phenyl | CN | |
| 3.012 | 3-Chlor-Phenyl | Fluormethyl | |
| 3.013 | 3-Chlor-Phenyl | Hydroxymethyl | |
| 3.014 | 3-Chlor-Phenyl | Fluormethyl | |
| 3.015 | 3-Chlor-Phenyl | Ethinyl | |
| 3.016 | 3-Brom-Phenyl | Methyl | |
| 3.017 | 3-Brom-Phenyl | Ethyl | |
| 3.018 | 3-Brom-Phenyl | Vinyl | |
| 3.019 | 3-Iod-Phenyl | Methyl | |
| 3.020 | 3-Methyl-Phenyl | Methyl | |
| 3.021 | 3-Ethyl-Phenyl | Methyl | |
| 3.022 | 3-Propyl-Phenyl | Methyl | |
| 3.023 | 3-isoPropyl-Phenyl | Methyl | |
| 3.024 | 3-nButyl-Phenyl | Methyl | |
| 3.025 | 3-iButyl-Phenyl | Methyl | |
| 3.026 | 3-tert.Butyl-Phenyl | Methyl | |
| 3.027 | 3-Cyclopropyl-Phenyl | Methyl | |
| 3.028 | 3-Cyclobutyl-Phenyl | Methyl | |
| 3.029 | 3-Cyclopentyl-Phenyl | Methyl | |
| 3.030 | 3-Vinyl-Phenyl | Methyl | |
| 3.031 | 3-Ethinyl-Phenyl | Methyl | |
| 3.032 | 3-Cyano-Phenyl | Methyl | |
| 3.033 | 3-Trifluormethyl-Phenyl | Methyl | |
| 3.034 | 3-Difluormethyl-Phenyl | Methyl | |
| 3.035 | 3-(Hydroxycarbonyl)-Phenyl | Methyl | |
| 3.036 | 3-(Methoxycarbony)l-Phenyl | Methyl | |
| 3.037 | 3-(Ethoxycarbonyl)-Phenyl | Methyl | |
| 3.038 | 3-Hydroxymethyl-Phenyl | Methyl | |
| 3.039 | 3-Carbamoyl-Phenyl | Methyl | |
| 3.040 | 3-Hydroxy-Phenyl- | Methyl | |
| 3.041 | 3-Methoxy-Phenyl | Methyl | |
| 3.042 | 3-Methoxy-Phenyl | Ethyl | |
| 3.043 | 3-Methoxy-Phenyl | CN | |
| 3.044 | 3-Methoxy-Phenyl | Fluormethyl | |
| 3.045 | 3-Methoxy-Phenyl | Hydroxymethyl | |
| 3.046 | 3-Methoxy-Phenyl | Ethinyl | |
| 3.047 | 3-Ethoxy-Phenyl | Methyl | |
| 3.048 | 3-Propyloxy-Phenyl | Methyl | |
| 3.049 | 3-isoPropyloxy-Phenyl | Methyl | |
| 3.050 | 3-nButyloxy-Phenyl | Methyl | |
| 3.051 | 3-iButyloxy-Phenyl | Methyl | |
| 3.052 | 3-tButyloxy-Phenyl | Methyl | |
| 3.053 | 3-Difluormethoxy-Phenyl | Methyl | |
| 3.054 | 3-Trifluormethoxy-Phenyl | Methyl | |
| 3.055 | 3-Trifluormethoxy-Phenyl | Ethyl | |
| 3.056 | 3-Trifluormethoxy-Phenyl | CN | |
| 3.057 | 3-Trifluormethoxy-Phenyl | Fluormethyl | |
| 3.058 | 3-Trifluormethoxy- | Hydroxymethyl | |
| 3.059 | 3-Trifluormethoxy-Phenyl | Ethinyl | |
| 3.060 | 3-(2,2,2-Trifluorethoxy)-Phenyl | Methyl | |
| 3.061 | 3-Nitro-Phenyl | Methyl | |
| 3.062 | 3-Acetoxy-Phenyl | Methyl | |
| 3.063 | {3-[(tert-Butoxycarbonyl)amino]-Phenyl} | Methyl | |
| 3.064 | 3-Methylsulfanyl-Phenyl | Methyl | |
| 3.065 | 3-Ethylsulfanyl-Phenyl | Methyl | |
| 3.066 | 3-(Pentafluor-lambda⁶-sulfanyl)-Phenyl | Methyl | |
| 3.067 | 2,3-Difluor-Phenyl | Methyl | |
| 3.068 | 2,3-Difluor-Phenyl | Ethyl | |
| 3.069 | 2,3-Difluor-Phenyl | CN | |
| 3.070 | 2,3-Difluor-Phenyl | Fluormethyl | |
| 3.071 | 2,3-Difluor-Phenyl | Hydroxymethyl | |
| 3.072 | 2,3-Difluor-Phenyl | Ethinyl | |
| 3.073 | 2-Chlor-3-fluor-Phenyl | Methyl | |
| 3.074 | 2-Brom-3-fluor-Phenyl | Methyl | |
| 3.075 | 2-Methyl-3-fluor-Phenyl | Methyl | |
| 3.076 | 2-Ethyl-3-fluor-Phenyl | Methyl | |
| 3.077 | 2-Cyclopropyl-3-fluor-Phenyl | Methyl | |
| 3.078 | 2-Cyano-3-fluor-Phenyl | Methyl | |
| 3.079 | 2-Methoxy-3-fluor-Phenyl | Methyl | |
| 3.080 | 2-Ethoxy-3-fluor-Phenyl | Methyl | |
| 3.081 | 2-Trifluormethoxy-3-fluor-Phenyl | Methyl | |
| 3.082 | 2-Nitro-3-fluor-Phenyl | Methyl | |
| 3.083 | 2-Fluor-3-chlor-Phenyl | Methyl | |
| 3.084 | 2,3-Dichlor-Phenyl | Methyl | |
| 3.085 | 2,3-Dichlor-Phenyl | Ethyl | |
| 3.086 | 2,3-Dichlor-Phenyl | CN | |
| 3.087 | 2,3-Dichlor-Phenyl | Fluormethyl | |
| 3.088 | 2,3-Dichlor-Phenyl | Hydroxymethyl | |
| 3.089 | 2,3-Dichlor-Phenyl | Ethinyl | |
| 3.090 | 2-Brom-3-chlor-Phenyl | Methyl | |
| 3.091 | 2-Methyl-3-chlor-Phenyl | Methyl | |
| 3.092 | 2-Methyl-3-chlor-Phenyl | Ethyl | |
| 3.093 | 2-Methyl-3-chlor-Phenyl | CN | |
| 3.094 | 2-Methyl-3-chlor-Phenyl | Fluormethyl | |
| 3.095 | 2-Methyl-3-chlor-Phenyl | Hydroxymethyl | |
| 3.096 | 2-Methyl-3-chlor-Phenyl | Ethinyl | |
| 3.097 | 2-Ethyl-3-chlor-Phenyl | Methyl | |
| 3.098 | 2-Cyclopropyl-3-chlor-Phenyl | Methyl | |
| 3.099 | 2-Cyano-3-chlor-Phenyl | Methyl | |
| 3.100 | 2-Trifluormethyl-2-chlor-Phenyl | Methyl | |
| 3.101 | 2-Methoxy-3-chlor-Phenyl | Methyl | |
| 3.102 | 2-Ethoxy-3-chlor-Phenyl | Methyl | |
| 3.103 | 2-Trifluormethoxy-3-chlor-Phenyl | Methyl | |
| 3.104 | 2-Nitro-3-chlor-Phenyl | Methyl | |
| 3.105 | 2-Fluor-3-brom-Phenyl | Methyl | |
| 3.106 | 2-Chlor-3-brom-Phenyl | Methyl | |
| 3.107 | 2,3-Dibrom-Phenyl | Methyl | |
| 3.108 | 2-Methyl-3-brom-Phenyl | Methyl | |
| 3.109 | 2-Ethyl-3-brom-Phenyl | Methyl | |
| 3.110 | 2-Cyclopropyl-3-brom-Phenyl | Methyl | |
| 3.111 | 2-Cyano-3-brom-Phenyl | Methyl | |
| 3.112 | 2-Trifluormethyl-3-brom-Phenyl | Methyl | |
| 3.113 | 2-Methoxy-3-Phenyl | Methyl | |
| 3.114 | 2-Ethoxy-3-brom-Phenyl | Methyl | |
| 3.115 | 2-Trifluormethoxy-3-brom-Phenyl | Methyl | |
| 3.116 | 2-Nitro-3-brom-Phenyl | Methyl | |
| 3.117 | 2-Fluor-3-iod-Phenyl | Methyl | |
| 3.118 | 2-Chlor-3-iod-Phenyl | Methyl | |
| 3.119 | 2-Brom-3-iod-Phenyl | Methyl | |
| 3.120 | 2-Methyl-3-iod-Phenyl | Methyl | |
| 3.121 | 2-Ethyl-3-iod-Phenyl | Methyl | |
| 3.122 | 2-Cyclopropyl-3-iod-Phenyl | Methyl | |
| 3.123 | 2-Cyano-3-iod-Phenyl | Methyl | |
| 3.124 | 2-Trifluormethyl-3-iod-Phenyl | Methyl | |
| 3.125 | 2-Methoxy-3-iod-Phenyl | Methyl | |
| 3.126 | 2-Ethoxy-3-iod-Phenyl | Methyl | |
| 3.127 | 2-Trifluormethoxy-3-iod-Phenyl | Methyl | |
| 3.128 | 2-Nitro-3-iod-Phenyl | Methyl | |
| 3.129 | 2-Fluor-3-methyl-Phenyl | Methyl | |
| 3.130 | 2-Fluor-3-methyl-Phenyl | Ethyl | |
| 3.131 | 2-Fluor-3-methyl-Phenyl | CN | |
| 3.132 | 2-Fluor-3-methyl-Phenyl | Fluormethyl | |
| 3.133 | 2-Fluor-3-methyl-Phenyl | Hydroxymethyl | |
| 3.134 | 2-Fluor-3-methyl-Phenyl | Ethinyl | |
| 3.135 | 2-Chlor-3-methyl-Phenyl | Methyl | |
| 3.136 | 2-Chlor-3-methyl-Phenyl | Ethyl | |
| 3.137 | 2-Chlor-3-methyl-Phenyl | CN | |
| 3.138 | 2-Chlor-3-methyl-Phenyl | Fluormethyl | |
| 3.139 | 2-Chlor-3-methyl-Phenyl | Hydroxymethyl | |
| 3.140 | 2-Chlor-3-methyl-Phenyl | Ethinyl | |
| 3.141 | 2-Brom-3-methyl-Phenyl | Methyl | |
| 3.142 | 2,3-Dimethyl-Phenyl | Methyl | |
| 3.143 | 2-Ethyl-3-methyl-Phenyl | Methyl | |
| 3.144 | 2-Cyclopropyl-3-methyl-Phenyl | Methyl | |
| 3.145 | 2-Cyano-3-methyl-Phenyl | Methyl | |
| 3.146 | 2-Trifluormethyl-3-methyl-Phenyl | Methyl | |
| 3.147 | 2-Methoxy-3-methyl-Phenyl | Methyl | |
| 3.148 | 2-Ethoxy-3-methyl-Phenyl | Methyl | |
| 3.149 | 2-Trifluormethoxy-3-methyl-Phenyl | Methyl | |
| 3.150 | 2-Nitro-3-methyl-Phenyl | Methyl | |
| 3.151 | 2-Fluor-3-ethyl-Phenyl | Methyl | |
| 3.152 | 2-Chlor-3-ethyl-Phenyl | Methyl | |
| 3.153 | 2-Brom-3-ethyl-Phenyl | Methyl | |
| 3.154 | 2-Methyl-3-ethyl-Phenyl | Methyl | |
| 3.155 | 2,3-Diethyl-Phenyl | Methyl | |
| 3.156 | 2-Cyclopropyl-3-ethyl-Phenyl | Methyl | |
| 3.157 | 2-Cyano-3-ethyl-Phenyl | Methyl | |
| 3.158 | 2-Trifluormethyl-3-ethyl-Phenyl | Methyl | |
| 3.159 | 2-Methoxy-3-ethyl-Phenyl | Methyl | |
| 3.160 | 2-Ethoxy-3-ethyl-Phenyl | Methyl | |
| 3.161 | 2-Trifluormethoxy-3-ethyl-Phenyl | Methyl | |
| 3.162 | 2-Nitro-3-ethyl-Phenyl | Methyl | |
| 3.163 | 2-Fluor-3-propyl-Phenyl | Methyl | |
| 3.164 | 2-Chlor-3-propyl-Phenyl | Methyl | |
| 3.165 | 2-Brom-3-propyl-Phenyl | Methyl | |
| 3.166 | 2-Methyl-3-propyl-Phenyl | Methyl | |
| 3.167 | 2-Methyl-3-propyl-Phenyl | Methyl | |
| 3.168 | 2-Cyclopropyl-3-propyl-Phenyl | Methyl | |
| 3.169 | 2-Cyano-3-propyl-Phenyl | Methyl | |
| 3.170 | 2-Trifluormethyl-3-propyl-Phenyl | Methyl | |
| 3.171 | 2-Methoxy-3-propyl-Phenyl | Methyl | |
| 3.172 | 2-Ethoxy-3-propyl-Phenyl | Methyl | |
| 3.173 | 2-Trifluormethoxy-3-propyl-Phenyl | Methyl | |
| 3.174 | 2-Nitro-3-propyl-Phenyl | Methyl | |
| 3.175 | 2-Fluor-3-isopropyl-Phenyl | Methyl | |
| 3.176 | 2-Chlor-3-isopropyl-Phenyl | Methyl | |
| 3.177 | 2-Brom-3-isopropyl-Phenyl | Methyl | |
| 3.178 | 2-Methyl-3-isopropyl-Phenyl | Methyl | |
| 3.179 | 2-Ethyl-3-isopropyl-Phenyl | Methyl | |
| 3.180 | 2-Cyclopropyl-3-isopropyl-Phenyl | Methyl | |
| 3.181 | 2-Cyano-3-isopropyl-Phenyl | Methyl | |
| 3.182 | 2-Trifluormethyl-3-isopropyl-Phenyl | Methyl | |
| 3.183 | 2-Methoxy-3-isopropyl-Phenyl | Methyl | |
| 3.184 | 2-Ethoxy-3-isopropyl-Phenyl | Methyl | |
| 3.185 | 2-Trifluormethoxy-3-isopropyl-Phenyl | Methyl | |
| 3.186 | 2-Nitro-3-isopropyl-Phenyl | Methyl | |
| 3.187 | 2-Fluor-3-tert.butyl-Phenyl | Methyl | |
| 3.188 | 2-Fluor-3-tert.butyl-Phenyl | Ethyl | |
| 3.189 | 2-Fluor-3-tert.butyl-Phenyl | CN | |
| 3.190 | 2-Fluor-3-tert.butyl-Phenyl | Fluormethyl | |
| 3.191 | 2-Fluor-3-tert.butyl-Phenyl | Hydroxymethyl | |
| 3.192 | 2-Fluor-3-tert.butyl-Phenyl | Ethinyl | |
| 3.193 | 2-Chlor-3-tert.butyl-Phenyl | Methyl | |
| 3.194 | 2-Brom-3-tert.butyl-Phenyl | Methyl | |
| 3.195 | 2-Methyl-3-tert.butyl-Phenyl | Methyl | |
| 3.196 | 2-Ethyl-3-tert.butyl-Phenyl | Methyl | |
| 3.197 | 2-Cyclopropyl-3-tert.butyl-Phenyl | Methyl | |
| 3.198 | 2-Cyano-3-tert.butyl-Phenyl | Methyl | |
| 3.199 | 2-Trifluormethyl-3-tert.butyl-Phenyl | Methyl | |
| 3.200 | 2-Methoxy-3-tert.butyl-Phenyl | Methyl | |
| 3.201 | 2-Ethoxy-3-tert.butyl-Phenyl | Methyl | |
| 3.202 | 2-Trifluormethoxy-3-tert.butyl-Phenyl | Methyl | |
| 3.203 | 2-Nitro-3-tert.butyl-Phenyl | Methyl | |
| 3.204 | 2-Fluor-3-cyclopropyl-Phenyl | Methyl | |
| 3.205 | 2-Chlor-3-cyclopropyl-Phenyl | Methyl | |
| 3.206 | 2-Brom-3-cyclopropyl-Phenyl | Methyl | |
| 3.207 | 2-Methyl-3-cyclopropyl-Phenyl | Methyl | |
| 3.208 | 2-Ethyl-3-cyclopropyl-Phenyl | Methyl | |
| 3.209 | 2-Cyclopropyl-3-cyclopropyl-Phenyl | Methyl | |
| 3.210 | 2-Cyano-3-cyclopropyl-Phenyl | Methyl | |
| 3.211 | 2-Trifluormethyl-3-cyclopropyl-Phenyl | Methyl | |
| 3.212 | 2-Methoxy-3-cyclopropyl-Phenyl | Methyl | |
| 3.213 | 2-Ethoxy-3-cyclopropyl-Phenyl | Methyl | |
| 3.214 | 2-Trifluormethoxy-3-cyclopropyl-Phenyl | Methyl | |
| 3.215 | 2-Fluor-3-methoxycarbonyl-Phenyl | Methyl | |
| 3.216 | 2-Chlor-3-methoxycarbonyl-Phenyl | Methyl | |
| 3.217 | 2-Brom-3-methoxycarbonyl-Phenyl | Methyl | |
| 3.218 | 2-Methyl-3-methoxycarbonyl-Phenyl | Methyl | |
| 3.219 | 2-Methyl-3-methoxycarbonyl-Phenyl | Ethyl | |
| 3.220 | 2-Methyl-3-methoxycarbonyl-Phenyl | CN | |
| 3.221 | 2-Methyl-3-methoxycarbonyl-Phenyl | Vinyl | |
| 3.222 | 2-Methyl-3-methoxycarbonyl-Phenyl | Fluormethyl | |
| 3.223 | 2-Methyl-3-methoxycarbonyl-Phenyl | Hydroxymethyl | |
| 3.224 | 2-Methyl-3-methoxycarbonyl-Phenyl | Ethinyl | |
| 3.225 | 2-Ethyl-3-methoxycarbonyl-Phenyl | Methyl | |
| 3.226 | 2-Cyclopropyl-3-methoxycarbonyl-Phenyl | Methyl | |
| 3.227 | 2-Cyano-3-methoxycarbonyl-Phenyl | Methyl | |
| 3.228 | 2-Trifluormethyl-3-methoxycarbonyl-Phenyl | Methyl | |
| 3.229 | 2-Methoxy-3-methoxycarbonyl -Phenyl | Methyl | |
| 3.230 | 2-Ethoxy-3-methoxycarbonyl-Phenyl | Methyl | |
| 3.231 | 2-Trifluormethoxy-3-methoxycarbonyl-Phenyl | Methyl | |
| 3.232 | 2-Nitro-3-methoxycarbonyl-Phenyl | Methyl | |
| 3.233 | 2-Fluor-3-cyano-Phenyl | Methyl | |
| 3.234 | 2-Fluor-3-cyano-Phenyl | Ethyl | |
| 3.235 | 2-Fluor-3-cyano-Phenyl | CN | |
| 3.236 | 2-Fluor-3-cyano-Phenyl | Vinyl | |
| 3.237 | 2-Fluor-3-cyano-Phenyl | Fluormethyl | |
| 3.238 | 2-Fluor-3-cyano-Phenyl | Hydroxymethyl | |
| 3.239 | 2-Fluor-3-cyano-Phenyl | Ethinyl | |
| 3.240 | 2-Chlor-3-cyano-Phenyl | Methyl | |
| 3.241 | 2-Chlor-3-cyano-Phenyl | Ethyl | |
| 3.242 | 2-Chlor-3-cyano-Phenyl | CN | |
| 3.243 | 2-Chlor-3-cyano-Phenyl | Vinyl | |
| 3.244 | 2-Chlor-3-cyano-Phenyl | Fluormethyl | |
| 3.245 | 2-Chlor-3-cyano-Phenyl | Hydroxymethyl | |
| 3.246 | 2-Chlor-3-cyano-Phenyl | Ethinyl | |
| 3.247 | 2-Brom-3-cyano-Phenyl | Methyl | |
| 3.248 | 2-Methyl-3-cyano-Phenyl | Methyl | |
| 3.249 | 2-Ethyl-3-cyano-Phenyl | Methyl | |
| 3.250 | 2-Cyclopropyl-3-cyano-Phenyl | Methyl | |
| 3.251 | 2-Cyano-3-cyano-Phenyl | Methyl | |
| 3.252 | 2-Trifluormethyl-3-cyano-Phenyl | Methyl | |
| 3.253 | 2-Methoxy-3-cyano-Phenyl | Methyl | |
| 3.254 | 2-Ethoxy-3-cyano-Phenyl | Methyl | |
| 3.255 | 2-Trifluormethoxy-3-cyano-Phenyl | Methyl | |
| 3.256 | 2-Nitro-3-cyano-Phenyl | Methyl | |
| 3.257 | 2-Chlor-3-methoxy-Phenyl | Methyl | |
| 3.258 | 2-Brom-3-methoxy-Phenyl | Methyl | |
| 3.259 | 2-Methyl-3-methoxy-Phenyl | Methyl | |
| 3.260 | 2-Ethyl-3-methoxy-Phenyl | Methyl | |
| 3.261 | 2-Cyclopropyl-3-methoxy-Phenyl | Methyl | |
| 3.262 | 2-Cyano-3-methoxy-Phenyl | Methyl | |
| 3.263 | 2-Trifluormethyl-3-methoxy-Phenyl | Methyl | |
| 3.264 | 2,3-Dimethoxy-Phenyl | Methyl | |
| 3.265 | 2-Ethoxy-3-methoxy-Phenyl | Methyl | |
| 3.266 | 2-Trifluormethoxy-3-methoxy-Phenyl | Methyl | |
| 3.267 | 2-Nitro-3-methoxy-Phenyl | Methyl | |
| 3.268 | 2-Fluor-3-ethoxy-Phenyl | Methyl | |
| 3.269 | 2-Chlor-3-ethoxy-Phenyl | Methyl | |
| 3.270 | 2-Brom-3-ethoxy-Phenyl | Methyl | |
| 3.271 | 2-Methyl-3-ethoxy-Phenyl | Methyl | |
| 3.272 | 2-Ethyl-3-ethoxy-Phenyl | Methyl | |
| 3.273 | 2-Cyclopropyl-3-ethoxy-Phenyl | Methyl | |
| 3.274 | 2-Cyano-3-ethoxy-Phenyl | Methyl | |
| 3.275 | 2-Trifluormethyl-3-ethoxy-Phenyl | Methyl | |
| 3.276 | 2-Methoxy-3-ethoxy-Phenyl | Methyl | |
| 3.277 | 2,3-Diethoxy-Phenyl | Methyl | |
| 3.278 | 2-Trifluormethoxy-3-ethoxy-Phenyl | Methyl | |
| 3.279 | 2-Nitro-3-ethoxy-Phenyl | Methyl | |
| 3.280 | 2-Fluor-3-propoxy-Phenyl | Methyl | |
| 3.281 | 2-Chlor-3-propoxy-Phenyl | Methyl | |
| 3.282 | 2-Brom-3-propoxy-Phenyl | Methyl | |
| 3.283 | 2-Methyl-3-propoxy-Phenyl | Methyl | |
| 3.284 | 2-Ethyl-3-propoxy-Phenyl | Methyl | |
| 3.285 | 2-Cyclopropyl-3-propoxy-Phenyl | Methyl | |
| 3.286 | 2-Vinyl-3-propoxy-Phenyl | Methyl | |
| 3.287 | 2-Ethinyl-3-propoxy-Phenyl | Methyl | |
| 3.288 | 2-Cyano-3-propoxy-Phenyl | Methyl | |
| 3.289 | 2-Trifluormethyl-3-propoxy-Phenyl | Methyl | |
| 3.290 | 2-Methoxy-3-propoxy-Phenyl | Methyl | |
| 3.291 | 2-Ethoxy-3-propoxy-Phenyl | Methyl | |
| 3.292 | 2-Trifluormethoxy-3-propoxy-Phenyl | Methyl | |
| 3.293 | 2-Nitro-3-propoxy-Phenyl | Methyl | |
| 3.294 | 2-Fluor-3-isopropoxy-Phenyl | Methyl | |
| 3.295 | 2-Chlor-3-isopropoxy-Phenyl | Methyl | |
| 3.296 | 2-Brom-3-isopropoxy-Phenyl | Methyl | |
| 3.297 | 2-Methyl-3-isopropoxy-Phenyl | Methyl | |
| 3.298 | 2-Ethyl-3-isopropoxy-Phenyl | Methyl | |
| 3.299 | 2-Cyclopropyl-3-isopropoxy-Phenyl | Methyl | |
| 3.300 | 2-Vinyl-3-isopropoxy-Phenyl | Methyl | |
| 3.301 | 2-Ethinyl-3-isopropoxy-Phenyl | Methyl | |
| 3.302 | 2-Cyano-3-isopropoxy-Phenyl | Methyl | |
| 3.303 | 2-Trifluormethyl-3-isopropoxy-Phenyl | Methyl | |
| 3.304 | 2-Methoxy-3-isopropoxy-Phenyl | Methyl | |
| 3.305 | 2-Ethoxy-3-isopropoxy-Phenyl | Methyl | |
| 3.306 | 2-Trifluormethoxy-3-isopropoxy-Phenyl | Methyl | |
| 3.307 | 2-Nitro-3-isopropoxy-Phenyl | Methyl | |
| 3.308 | 2-Fluor-3-trifluormethoxy-Phenyl | Methyl | |
| 3.309 | 2-Chlor-3-trifluormethoxy-Phenyl | Methyl | |
| 3.310 | 2-Brom-3-trifluormethoxy-Phenyl | Methyl | |
| 3.311 | 2-Methyl-3-trifluormethoxy-Phenyl | Methyl | |
| 3.312 | 2-Ethyl-3-trifluormethoxy-Phenyl | Methyl | |
| 3.313 | 2-Cyclopropyl-3-trifluormethoxy-Phenyl | Methyl | |
| 3.314 | 2-Cyano-3-trifluormethoxy-Phenyl | Methyl | |
| 3.315 | 2-Trifluormethyl-3-trifluormethoxy-Phenyl | Methyl | |
| 3.316 | 2-Methoxy-3-trifluormethoxy-Phenyl | Methyl | |
| 3.317 | 2-Ethoxy-3-trifluormethoxy-Phenyl | Methyl | |
| 3.318 | 2,3-Bis(trifluormethoxy)-Phenyl | Methyl | |
| 3.319 | 2-Nitro-3-trifluormethoxy-Phenyl | Methyl | |
| 3.320 | 2-Fluor-3-difluormethoxy-Phenyl | Methyl | |
| 3.321 | 2-Chlor-3-difluormethoxy-Phenyl | Methyl | |
| 3.322 | 2-Brom-3-difluormethoxy-Phenyl | Methyl | |
| 3.323 | 2-Methyl-3-difluormethoxy-Phenyl | Methyl | |
| 3.324 | 2-Ethyl-3-difluormethoxy-Phenyl | Methyl | |
| 3.325 | 2-Cyclopropyl-3-difluormethoxy-Phenyl | Methyl | |
| 3.326 | 2-Cyano-3-difluormethoxy-Phenyl | Methyl | |
| 3.327 | 2-Trifluormethyl-3-difluormethoxy-Phenyl | Methyl | |
| 3.328 | 2-Methoxy-3-difluormethoxy-Phenyl | Methyl | |
| 3.329 | 2-Ethoxy-3-difluormethoxy-Phenyl | Methyl | |
| 3.330 | 2-Trifluormethoxy-3-difluormethoxy-Phenyl | Methyl | |
| 3.331 | 2-Nitro-3-difluormethoxy-Phenyl | Methyl | |
| 3.332 | 2-Fluor-3-nitro-Phenyl | Methyl | |
| 3.333 | 2-Chlor-3-nitro-Phenyl | Methyl | |
| 3.334 | 2-Brom-3-nitro-Phenyl | Methyl | |
| 3.335 | 2-Methyl-3-nitro-Phenyl | Methyl | |
| 3.336 | 2-Ethyl-3-nitro-Phenyl | Methyl | |
| 3.337 | 2-Cyclopropyl-3-nitro-Phenyl | Methyl | |
| 3.338 | 2-Cyano-3-nitro-Phenyl | Methyl | |
| 3.339 | 2-Trifluormethyl-3-nitro-Phenyl | Methyl | |
| 3.340 | 2-Methoxy-3-nitro-Phenyl | Methyl | |
| 3.341 | 2-Ethoxy-3-nitro-Phenyl | Methyl | |
| 3.342 | 2-Trifluormethoxy-3-nitro-Phenyl | Methyl | |
| 3.343 | 2-Fluor-3-methylsulfanylPhenyl | Methyl | |
| 3.344 | 2-Chlor-3-methylsulfanyl-Phenyl | Methyl | |
| 3.345 | 2-Brom-3-methylsulfanyl-Phenyl | Methyl | |
| 3.346 | 2-Methyl-3-methylsulfanyl-Phenyl | Methyl | |
| 3.347 | 2-Ethyl-3-methylsulfanyl-Phenyl | Methyl | |
| 3.348 | 2-Cyclopropyl-3-methylsulfanyl-Phenyl | Methyl | |
| 3.349 | 2-Cyano-3-methylsulfanyl-Phenyl | Methyl | |
| 3.350 | 2-Trifluormethyl-3-methylsulfanyl-Phenyl | Methyl | |
| 3.351 | 2-Methoxy-3-methylsulfanyl -Phenyl | Methyl | |
| 3.352 | 2-Ethoxy-3-methylsulfanyl-Phenyl | Methyl | |
| 3.353 | 2-Trifluormethoxy-3-methylsulfanyl-Phenyl | Methyl | |
| 3.354 | 2-Nitro-3-methylsulfanyl-Phenyl | Methyl | |
| 3.355 | 3,5-Difluor-Phenyl | Methyl | [CDCl₃] 1.91 (s, 3H), 3.38 (d, 1H), 3.81 (d, 1H), 6.93 (m, 1H), 7.18 (m, 2H) |
| 3.356 | 3,5-Difluor-Phenyl | Ethyl | |
| 3.357 | 3,5-Difluor-Phenyl | CN | |
| 3.358 | 3,5-Difluor-Phenyl | Vinyl | |
| 3.359 | 3,5-Difluor-Phenyl | Fluormethyl | |
| 3.360 | 3,5-Difluor-Phenyl | Hydroxymethyl | |
| 3.361 | 3,5-Difluor-Phenyl | Ethinyl | |
| 3.362 | 3-Chlor-5-fluor-Phenyl | Methyl | |
| 3.363 | 3-Chlor-5-fluor-Phenyl | Ethyl | |
| 3.364 | 3-Chlor-5-fluor-Phenyl | CN | |
| 3.365 | 3-Chlor-5-fluor-Phenyl | Vinyl | |
| 3.366 | 3-Chlor-5-fluor-Phenyl | Fluormethyl | |
| 3.367 | 3-Chlor-5-fluor-Phenyl | Hydroxymethyl | |
| 3.368 | 3-Chlor-5-fluor-Phenyl | Ethinyl | |
| 3.369 | 3-Brom-5-fluor-Phenyl | Methyl | |
| 3.370 | 3-Iod-5-fluor-Phenyl | Methyl | |
| 3.371 | 3-Methyl-5-fluor-Phenyl | Methyl | |
| 3.372 | 3-Methyl-5-fluor-Phenyl | Ethyl | |
| 3.373 | 3-Methyl-5-fluor-Phenyl | CN | |
| 3.374 | 3-Methyl-5-fluor-Phenyl | Vinyl | |
| 3.375 | 3-Methyl-5-fluor-Phenyl | Fluormethyl | |
| 3.376 | 3-Methyl-5-fluor-Phenyl | Hydroxymethyl | |
| 3.377 | 3-Methyl-5-fluor-Phenyl | Ethinyl | |
| 3.378 | 3-Ethyl-5-fluor-Phenyl | Methyl | |
| 3.379 | 3-Propyl-5-fluor-Phenyl | Methyl | |
| 3.380 | 3-iPropyl-5-fluor-Phenyl | Methyl | |
| 3.381 | 3-nButyl-5-fluor-Phenyl | Methyl | |
| 3.382 | 3-isoButyl-5-fluor-Phenyl | Methyl | |
| 3.383 | 3-tert.Butyl-5-fluor-Phenyl | Methyl | |
| 3.384 | 3-tert.Butyl-5-fluor-Phenyl | Ethyl | |
| 3.385 | 3-tert.Butyl-5-fluor-Phenyl | CN | |
| 3.386 | 3-tert.Butyl-5-fluor-Phenyl | Vinyl | |
| 3.387 | 3-tert.Butyl-5-fluor-Phenyl | Fluormethyl | |
| 3.388 | 3-tert.Butyl-5-fluor-Phenyl | Hyd roxymethyl | |
| 3.389 | 3-tert.Butyl-5-fluor-Phenyl | Ethinyl | |
| 3.390 | 3-Cyclopropyl-5-fluor-Phenyl | Methyl | |
| 3.391 | 3-Cyano-5-fluor-Phenyl | Methyl | |
| 3.392 | 3-Cyano-5-fluor-Phenyl | Ethyl | |
| 3.393 | 3-Cyano-5-fluor-Phenyl | CN | |
| 3.394 | 3-Cyano-5-fluor-Phenyl | Vinyl | |
| 3.395 | 3-Cyano-5-fluor-Phenyl | Fluormethyl | |
| 3.396 | 3-Cyano-5-fluor-Phenyl | Hydroxymethyl | |
| 3.397 | 3-Cyano-5-fluor-Phenyl | Ethinyl | |
| 3.398 | 3-Trifluormethyl-5-fluor-Phenyl | Methyl | |
| 3.399 | 3-(Methoxycarbony)l-5-fluor-Phenyl | Methyl | |
| 3.400 | 3-Methoxy-5-fluor-Phenyl | Methyl | |
| 3.401 | 3-Methoxy-5-fluor-Phenyl | Ethyl | |
| 3.402 | 3-Methoxy-5-fluor-Phenyl | CN | |
| 3.403 | 3-Methoxy-5-fluor-Phenyl | Vinyl | |
| 3.404 | 3-Methoxy-5-fluor-Phenyl | Fluormethyl | |
| 3.405 | 3-Methoxy-5-fluor-Phenyl | Hydroxymethyl | |
| 3.406 | 3-Methoxy-5-fluor-Phenyl | Ethinyl | |
| 3.407 | 3-Ethoxy-5-fluor-Phenyl | Methyl | |
| 3.408 | 3-nPropyoxy-5-fluor-Phenyl | Methyl | |
| 3.409 | 3-isoPropoxy-5-fluor-Phenyl | Methyl | |
| 3.410 | 3-nButoxy-5-fluor-Phenyl | Methyl | |
| 3.411 | 3-isoButoxy-5-fluor-Phenyl | Methyl | |
| 3.412 | 3-Difluormethoxy-5-fluor-Phenyl | Methyl | |
| 3.413 | 3-Trifluormethoxy-5-fluor-Phenyl | Methyl | |
| 3.414 | 3-Nitro-5-fluor-Phenyl | Methyl | |
| 3.415 | 3-Acetoxy-5-fluor-Phenyl | Methyl | |
| 3.416 | 3-Methylsulfanyl-5-fluor-Phenyl | Methyl | |
| 3.417 | 3,5-Dichlor-Phenyl | Methyl | [CDCl₃] 1.91 (s, 3H); 3.38 (d, 1H); 3.80 (d, 1H); 7.45 (s, 1H); 7.52 (s, 1H). |
| 3.418 | 3,5-Dichlor-Phenyl | Ethyl | |
| 3.419 | 3,5-Dichlor-Phenyl | Isopropyl | [DMSO] 1,06 (d, 3H); 1,10 (d, 3H); 2,25 (m, 1H); 3,85 (d, 1H); 4,10 (d, 1H); 7,74 (d, 1H); 7,79 (d, 1H). |
| 3.420 | 3,5-Dichlor-Phenyl | Vinyl | |
| 3.421 | 3,5-Dichlor-Phenyl | Fluormethyl | |
| 3.422 | 3,5-Dichlor-Phenyl | Hydroxymethyl | |
| 3.423 | 3,5-Dichlor-Phenyl | Ethinyl | |
| 3.424 | 3-Brom-5-chlor-Phenyl | Methyl | |
| 3.425 | 3-Iod-5-chlor-Phenyl | Methyl | |
| 3.426 | 3-Methyl-5-chlor-Phenyl | Methyl | |
| 3.427 | 3-Methyl-5-chlor-Phenyl | Ethyl | |
| 3.428 | 3-Methyl-5-chlor-Phenyl | CN | |
| 3.429 | 3-Methyl-5-chlor-Phenyl | Vinyl | |
| 3.430 | 3-Methyl-5-chlor-Phenyl | Fluormethyl | |
| 3.431 | 3-Methyl-5-chlor-Phenyl | Hydroxymethyl | |
| 3.432 | 3-Methyl-5-chlor-Phenyl | Ethinyl | |
| 3.433 | 3-Ethyl-5-chlor-Phenyl | Methyl | |
| 3.434 | 3-Propyl-5-chlor-Phenyl | Methyl | |
| 3.435 | 3-isoPropyl-5-chlor-Phenyl | Methyl | |
| 3.436 | 3-nButyl-5-chlor-Phenyl | Methyl | |
| 3.437 | 3-isoButyl-5-chlor-Phenyl | Methyl | |
| 3.438 | 3-tert.Butyl-5-chlor-Phenyl | Methyl | |
| 3.439 | 3-Cyclopropyl-5-chlor-Phenyl | Methyl | |
| 3.440 | 3-Cyano-5-chlor-Phenyl | Methyl | |
| 3.441 | 3-Cyano-5-chlor-Phenyl | Ethyl | |
| 3.442 | 3-Cyano-5-chlor-Phenyl | CN | |
| 3.443 | 3-Cyano-5-chlor-Phenyl | Vinyl | |
| 3.444 | 3-Cyano-5-chlor-Phenyl | Fluormethyl | |
| 3.445 | 3-Cyano-5-chlor-Phenyl | Hydroxymethyl | |
| 3.446 | 3-Cyano-5-chlor-Phenyl | Ethinyl | |
| 3.447 | 3-Trifluormethyl-5-chlor-Phenyl | Methyl | |
| 3.448 | 3-Trifluormethyl-5-chlor-Phenyl | Ethyl | |
| 3.449 | 3-Trifluormethyl-5-chlor-Phenyl | CN | |
| 3.450 | 3-Trifluormethyl-5-chlor-Phenyl | Vinyl | |
| 3.451 | 3-Trifluormethyl-5-chlor-Phenyl | Fluormethyl | |
| 3.452 | 3-Trifluormethyl-5-chlor-Phenyl | Hydroxymethyl | |
| 3.453 | 3-Trifluormethyl-5-chlor-Phenyl | Ethinyl | |
| 3.454 | 3-(Methoxycarbony)l-5-chlor-Phenyl | Methyl | |
| 3.455 | 3-Methoxy-5-chlor-Phenyl | Methyl | |
| 3.456 | 3-Methoxy-5-chlor-Phenyl | Ethyl | |
| 3.457 | 3-Methoxy-5-chlor-Phenyl | CN | |
| 3.458 | 3-Methoxy-5-chlor-Phenyl | Vinyl | |
| 3.459 | 3-Methoxy-5-chlor-Phenyl | Fluormethyl | |
| 3.460 | 3-Methoxy-5-chlor-Phenyl | Hydroxymethyl | |
| 3.461 | 3-Methoxy-5-chlor-Phenyl | Ethinyl | |
| 3.462 | 3-Ethoxy-5-chlor-Phenyl | Methyl | |
| 3.463 | 3-nPropyoxy-5-chlor-Phenyl | Methyl | |
| 3.464 | 3-isoPropoxy-5-chlor-Phenyl | Methyl | |
| 3.465 | 3-isoButoxy-5-chlor-Phenyl | Methyl | |
| 3.466 | 3-Difluormethoxy-5-chlor-Phenyl | Methyl | |
| 3.467 | 3-Trifluormethoxy-5-chlor-Phenyl | Methyl | |
| 3.468 | 3-Trifluormethoxy-5-chlor-Phenyl | Ethyl | |
| 3.469 | 3-Trifluormethoxy-5-chlor-Phenyl | CN | |
| 3.470 | 3-Trifluormethoxy-5-chlor-Phenyl | Vinyl | |
| 3.471 | 3-Trifluormethoxy-5-chlor-Phenyl | Fluormethyl | |
| 3.472 | 3-Trifluormethoxy-5-chlor-Phenyl | Hydroxymethyl | |
| 3.473 | 3-Trifluormethoxy-5-chlor-Phenyl | Ethinyl | |
| 3.474 | 3-Nitro-5-chlor-Phenyl | Methyl | |
| 3.475 | 3-Acetoxy-5-chlor-Phenyl | Methyl | |
| 3.476 | 3-Methylsulfanyl-5-chlor-Phenyl | Methyl | |
| 3.477 | 3,5-Dibrom-Phenyl-Phenyl | Methyl | |
| 3.478 | 3-Iod-5-brom-Phenyl | Methyl | |
| 3.479 | 3-Methyl-5-brom-Phenyl | Methyl | |
| 3.480 | 3-Ethyl-5-brom-Phenyl | Methyl | |
| 3.481 | 3-Propyl-5-brom-Phenyl | Methyl | |
| 3.482 | 3-isoPropyl-5-brom-Phenyl | Methyl | |
| 3.483 | 3-nButyl-5-brom-Phenyl | Methyl | |
| 3.484 | 3-isoButyl-5-brom-Phenyl | Methyl | |
| 3.485 | 3-tert.Butyl-5-brom-Phenyl | Methyl | |
| 3.486 | 3-Cyclopropyl-5-brom-Phenyl | Methyl | |
| 3.487 | 3-Cyano-5-brom-Phenyl | Methyl | |
| 3.488 | 3-Trifluormethyl-5-brom-Phenyl | Methyl | |
| 3.489 | 3-(Methoxycarbonyl)-5-brom-Phenyl | Methyl | |
| 3.490 | 3-Methoxy-5-brom-Phenyl | Methyl | |
| 3.491 | 3-Ethoxy-5-brom-Phenyl | Methyl | |
| 3.492 | 3-nPropyoxy-5-brom-Phenyl | Methyl | |
| 3.493 | 3-isoPropoxy-5-brom-Phenyl | Methyl | |
| 3.494 | 3-nButoxy-5-brom-Phenyl | Methyl | |
| 3.495 | 3-isoButoxy-5-brom-Phenyl | Methyl | |
| 3.496 | 3-Difluormethoxy-5-brom-Phenyl | Methyl | |
| 3.497 | 3-Trifluormethoxy-5-brom-Phenyl | Methyl | |
| 3.498 | 3-Nitro-5-brom-Phenyl | Methyl | |
| 3.499 | 3-Acetoxy-5-brom-Phenyl | Methyl | |
| 3.500 | 3-Methylsulfanyl-5-brom-Phenyl | Methyl | |
| 3.501 | 3,5-Diiod-Phenyl | Methyl | |
| 3.502 | 3-Methyl-5-iod-Phenyl | Methyl | |
| 3.503 | 3-Ethyl-5-iod-Phenyl | Methyl | |
| 3.504 | 3-Propyl-5-iod-Phenyl | Methyl | |
| 3.505 | 3-isoPropyl-5-iod-Phenyl | Methyl | |
| 3.506 | 3-nButyl-5-iod-Phenyl | Methyl | |
| 3.507 | 3-isoButyl-5-iod-Phenyl | Methyl | |
| 3.508 | 3-tert.Butyl-5-iod-Phenyl | Methyl | |
| 3.509 | 3-Cyclopropyl-5-iod-Phenyl | Methyl | |
| 3.510 | 3-Cyano-5-iod-Phenyl | Methyl | |
| 3.511 | 3-Trifluormethyl-5-iod-Phenyl | Methyl | |
| 3.512 | 3-(Methoxycarbonyl)-5-iod-Phenyl | Methyl | |
| 3.513 | 3-Methoxy-5-iod-Phenyl | Methyl | |
| 3.514 | 3-Ethoxy-5-iod-Phenyl | Methyl | |
| 3.515 | 3-nPropyoxy-5-iod-Phenyl | Methyl | |
| 3.516 | 3-isoPropoxy-5-iod-Phenyl | Methyl | |
| 3.517 | 3-nButoxy-5-iod-Phenyl | Methyl | |
| 3.518 | 3-isoButoxy-5-iod-Phenyl | Methyl | |
| 3.519 | 3-Difluormethoxy-5-iod-Phenyl | Methyl | |
| 3.520 | 3-Trifluormethoxy-5-iod-Phenyl | Methyl | |
| 3.521 | 3-Nitro-5-iod-Phenyl | Methyl | |
| 3.522 | 3-Acetoxy-5-iod-Phenyl | Methyl | |
| 3.523 | 3-Methylsulfanyl-5-iod-Phenyl | Methyl | |
| 3.524 | 3,5-Dimethyl-Phenyl | Methyl | |
| 3.525 | 3-Ethyl-5-methyl-Phenyl | Methyl | |
| 3.526 | 3-Propyl-5-methyl-Phenyl | Methyl | |
| 3.527 | 3-isoPropyl-5-methyl-Phenyl | Methyl | |
| 3.528 | 3-nButyl-5-methyl-Phenyl | Methyl | |
| 3.529 | 3-isoButyl-5-methyl-Phenyl | Methyl | |
| 3.530 | 3-tert.Butyl-5-methyl-Phenyl | Methyl | |
| 3.531 | 3-Cyclopropyl-5-methyl-Phenyl | Methyl | |
| 3.532 | 3-Cyano-5-methyl-Phenyl | Methyl | |
| 3.533 | 3-Trifluormethyl-5-methyl-Phenyl | Methyl | |
| 3.534 | 3-(Methoxycarbony)l-5-methyl-Phenyl | Methyl | |
| 3.535 | 3-Methoxy-5-methyl-Phenyl | Methyl | |
| 3.536 | 3-Ethoxy-5-methyl-Phenyl | Methyl | |
| 3.537 | 3-nPropyoxy-5-methyl-Phenyl | Methyl | |
| 3.538 | 3-isoButoxy-5-methyl-Phenyl | Methyl | |
| 3.539 | 3-Difluormethoxy-5-methyl-Phenyl | Methyl | |
| 3.540 | 3-Trifluormethoxy-5-methyl-Phenyl | Methyl | |
| 3.541 | 3-Nitro-5-methyl-Phenyl | Methyl | |
| 3.542 | 3-Acetoxy-5-methyl-Phenyl | Methyl | |
| 3.543 | 3-Methylsulfanyl-5-methyl-Phenyl | Methyl | |
| 3.544 | 3,5-Diethyl-Phenyl | Methyl | |
| 3.545 | 3-Propyl-5-ethyl-Phenyl | Methyl | |
| 3.546 | 3-isoPropyl-5-ethyl-Phenyl | Methyl | |
| 3.547 | 3-nButyl-5-ethyl-Phenyl | Methyl | |
| 3.548 | 3-isoButyl-5-ethyl-Phenyl | Methyl | |
| 3.549 | 3-Cyclopropyl-5-ethyl-Phenyl | Methyl | |
| 3.550 | 3-Cyano-5-ethyl-Phenyl | Methyl | |
| 3.551 | 3-Trifluormethyl-5-ethyl-Phenyl | Methyl | |
| 3.552 | 3-(Methoxycarbonyl)-5-ethyl-Phenyl | Methyl | |
| 3.553 | 3-Methoxy-5-ethyl-Phenyl | Methyl | |
| 3.554 | 3-Ethoxy-5-ethyl-Phenyl | Methyl | |
| 3.555 | 3-nPropyoxy-5-ethyl-Phenyl | Methyl | |
| 3.556 | 3-nButoxy-5-ethyl-Phenyl | Methyl | |
| 3.557 | 3-isoButoxy-5-ethyl-Phenyl | Methyl | |
| 3.558 | 3-Difluormethoxy-5-ethyl-Phenyl | Methyl | |
| 3.559 | 3-Trifluormethoxy-5-ethyl-Phenyl | Methyl | |
| 3.560 | 3-Nitro-5-ethyl-Phenyl | Methyl | |
| 3.561 | 3-Acetoxy-5-ethyl-Phenyl | Methyl | |
| 3.562 | 3-Methylsulfanyl-5-ethyl-Phenyl | Methyl | |
| 3.563 | 3,5-Dipropyl-Phenyl | Methyl | |
| 3.564 | 3-isoPropyl-5-propyl-Phenyl | Methyl | |
| 3.565 | 3-nButyl-5-propyl-Phenyl | Methyl | |
| 3.566 | 3-isoButyl-5-propyl-Phenyl | Methyl | |
| 3.567 | 3-tert.Butyl-5-propyl-Phenyl | Methyl | |
| 3.568 | 3-Cyclopropyl-5-propyl-Phenyl | Methyl | |
| 3.569 | 3-Cyano-5-propyl-Phenyl | Methyl | |
| 3.570 | 3-Trifluormethyl-5-propyl-Phenyl | Methyl | |
| 3.571 | 3-(Methoxycarbonyl)-5-propyl-Phenyl | Methyl | |
| 3.572 | 3-Methoxy-5-propyl-Phenyl | Methyl | |
| 3.573 | 3-Ethoxy-5-propyl-Phenyl | Methyl | |
| 3.574 | 3-nPropyoxy-5-propyl-Phenyl | Methyl | |
| 3.575 | 3-isoButoxy-5-propyl-Phenyl | Methyl | |
| 3.576 | 3-Difluormethoxy-5-propyl-Phenyl | Methyl | |
| 3.577 | 3-Trifluormethoxy-5-ethyl-Phenyl | Methyl | |
| 3.578 | 3-Nitro-5-propyl-Phenyl | Methyl | |
| 3.579 | 3-Acetoxy-5-propyl-Phenyl | Methyl | |
| 3.580 | 3-Methylsulfanyl-5-propyl-Phenyl | Methyl | |
| 3.581 | 3,5-Diisopropyl-Phenyl | Methyl | |
| 3.582 | 3-nButyl-5-isopropyl-Phenyl | Methyl | |
| 3.583 | 3-isoButyl-5-isopropyl-Phenyl | Methyl | |
| 3.584 | 3-tert.Butyl-5-isopropyl-Phenyl | Methyl | |
| 3.585 | 3-Cyclopropyl-5-isopropyl-Phenyl | Methyl | |
| 3.586 | 3-Cyano-5-isopropyl-Phenyl | Methyl | |
| 3.587 | 3-Trifluormethyl-5-isopropyl-Phenyl | Methyl | |
| 3.588 | 3-(Methoxycarbony)l-5-isopropyl-Phenyl | Methyl | |
| 3.589 | 3-Methoxy-5-isopropyl-Phenyl | Methyl | |
| 3.590 | 3-Ethoxy-5-isopropyl-Phenyl | Methyl | |
| 3.591 | 3-nPropyoxy-5-isopropyl-Phenyl | Methyl | |
| 3.592 | 3-isoButoxy-5-isopropyl-Phenyl | Methyl | |
| 3.593 | 3-Difluormethoxy-5-isopropyl-Phenyl | Methyl | |
| 3.594 | 3-Trifluormethoxy-5-isopropyl-Phenyl | Methyl | |
| 3.595 | 3-Nitro-5-isopropyl-Phenyl | Methyl | |
| 3.596 | 3-Acetoxy-5-isopropyl-Phenyl | Methyl | |
| 3.597 | 3-Methylsulfanyl-5-isopropyl-Phenyl | Methyl | |
| 3.598 | 3,5-Diisobutyl-Phenyl | Methyl | |
| 3.599 | 3-tert.Butyl-5-isobutyl-Phenyl | Methyl | |
| 3.600 | 3-Cyclopropyl-5-isobutyl-Phenyl | Methyl | |
| 3.601 | 3-Cyano-5-isobutyl-Phenyl | Methyl | |
| 3.602 | 3-Trifluormethyl-5-isobutyl-Phenyl | Methyl | |
| 3.603 | 3-(Methoxycarbonyl)-5-isobutyl-Phenyl | Methyl | |
| 3.604 | 3-Ethoxy-5-isobutyl-Phenyl | Methyl | |
| 3.605 | 3-nPropyoxy-5-isobutyl-Phenyl | Methyl | |
| 3.606 | 3-isoButoxy-5-isobutyl-Phenyl | Methyl | |
| 3.607 | 3-Difluormethoxy-5-isobutyl-Phenyl | Methyl | |
| 3.608 | 3-Trifluormethoxy-5-isobutyl-Phenyl | Methyl | |
| 3.609 | 3-Nitro-5-isobutyl-Phenyl | Methyl | |
| 3.610 | 3-Acetoxy-5-isobutyl-Phenyl | Methyl | |
| 3.611 | 3-Methylsulfanyl-5-isobutyl-Phenyl | Methyl | |
| 3.612 | 3,5-Ditert.butyl-Phenyl | Methyl | |
| 3.613 | 3-Cyclopropyl-5-tert.butyl-Phenyl | Methyl | |
| 3.614 | 3-Cyano-5-tert.butyl-Phenyl | Methyl | |
| 3.615 | 3-Trifluormethyl-5-tert.butyl-Phenyl | Methyl | |
| 3.616 | 3-(Methoxycarbonyl)-5tert.butyl-Phenyl | Methyl | |
| 3.617 | 3-Methoxy-5-tert.butyl-Phenyl | Methyl | |
| 3.618 | 3-Ethoxy-5-tert.butyl-Phenyl | Methyl | |
| 3.619 | 3-nPropyoxy-5-tert.butyl-Phenyl | Methyl | |
| 3.620 | 3-isoButoxy-5-tert.butyl-Phenyl | Methyl | |
| 3.621 | 3-Difluormethoxy-5-tert.butyl-Phenyl | Methyl | |
| 3.622 | 3-Trifluormethoxy-5-tert.butyl-Phenyl | Methyl | |
| 3.623 | 3-Nitro-5-tert.butyl-Phenyl | Methyl | |
| 3.624 | 3-Acetoxy-5-tert.butyl-Phenyl | Methyl | |
| 3.625 | 3-Methylsulfanyl-5-tert.butyl-Phenyl | Methyl | |
| 3.626 | 3-tert.Butyl-5-cyclopropyl-Phenyl | Methyl | |
| 3.627 | 3,5-Dicyclopropyl-Phenyl | Methyl | |
| 3.628 | 3-Cyano-5-cyclopropyl-Phenyl | Methyl | |
| 3.629 | 3-Cyano-5-cyclopropyl-Phenyl | Ethyl | |
| 3.630 | 3-Cyano-5-cyclopropyl-Phenyl | CN | |
| 3.631 | 3-Cyano-5-cyclopropyl-Phenyl | Vinyl | |
| 3.632 | 3-Cyano-5-cyclopropyl-Phenyl | Fluormethyl | |
| 3.633 | 3-Cyano-5-cyclopropyl-Phenyl | Hydroxymethyl | |
| 3.634 | 3-Cyano-5-cyclopropyl-Phenyl | Ethinyl | |
| 3.635 | 3-Trifluormethyl-5-cyclopropyl-Phenyl | Methyl | |
| 3.636 | 3-(Methoxycarbonyl)-5-cyclopropyl-Phenyl | Methyl | |
| 3.637 | 3-Methoxy-5-cyclopropyl-Phenyl | Methyl | |
| 3.638 | 3-Ethoxy-5-cyclopropyl-Phenyl | Methyl | |
| 3.639 | 3-nPropyoxy-5-cyclopropyl-Phenyl | Methyl | |
| 3.640 | 3-isoButoxy-5-cyclopropyl-Phenyl | Methyl | |
| 3.641 | 3-Difluormethoxy-5-cyclopropyl-Phenyl | Methyl | |
| 3.642 | 3-Trifluormethoxy-5-cyclopropyl-Phenyl | Methyl | |
| 3.643 | 3-Nitro-5-cyclopropyl-Phenyl | Methyl | |
| 3.644 | 3-Acetoxy-5-cyclopropyl-Phenyl | Methyl | |
| 3.645 | 3-Methylsulfanyl-5-cyclopropyl-Phenyl | Methyl | |
| 3.646 | 3,5-Dicyano-Phenyl | Methyl | |
| 3.647 | 3-Trifluormethyl-5-cyano-Phenyl | Methyl | |
| 3.648 | 3-(Methoxycarbonyl)-5-cyano-Phenyl | Methyl | |
| 3.649 | 3-Methoxy-5-cyano-Phenyl | Methyl | |
| 3.650 | 3-Ethoxy-5-cyano-Phenyl | Methyl | |
| 3.651 | 3-nPropyoxy-5-cyano-Phenyl | Methyl | |
| 3.652 | 3-isoButoxy-5-cyano-Phenyl | Methyl | |
| 3.653 | 3-Difluormethoxy-5-cyano-Phenyl | Methyl | |
| 3.654 | 3-Trifluormethoxy-5-cyano-Phenyl | Methyl | |
| 3.655 | 3-Nitro-5-cyano-Phenyl | Methyl | |
| 3.656 | 3-Acetoxy-5-cyano-Phenyl | Methyl | |
| 3.657 | 3-Methylsulfanyl-5-cyano-Phenyl | Methyl | |
| 3.658 | 3,5-Di(trifluormethyl)-Phenyl | Methyl | |
| 3.659 | 3,5-Di(trifluormethyl)-Phenyl | Ethyl | |
| 3.660 | 3,5-Di(trifluormethyl)-Phenyl | CN | |
| 3.661 | 3,5-Di(trifluormethyl)-Phenyl | Vinyl | |
| 3.662 | 3,5-Di(trifluormethyl)-Phenyl | Fluormethyl | |
| 3.663 | 3,5-Di(trifluormethyl)-Phenyl | Hydroxymethyl | |
| 3.664 | 3,5-Di(trifluormethyl)-Phenyl | Ethinyl | |
| 3.665 | 3-(Methoxycarbonyl)-5-trifluormethyl-Phenyl | Methyl | |
| 3.666 | 3-Methoxy-5-trifluormethyl-Phenyl | Methyl | |
| 3.667 | 3-Ethoxy-5trifluormethyl-Phenyl | Methyl | |
| 3.668 | 3-nPropyoxy-5-trifluormethyl-Phenyl | Methyl | |
| 3.669 | 3-nButoxy-5-trifluormethyl-Phenyl | Methyl | |
| 3.670 | 3-isoButoxy-5-trifluormethyl-Phenyl | Methyl | |
| 3.671 | 3-Difluormethoxy-5-trifluormethyl-Phenyl | Methyl | |
| 3.672 | 3-Trifluormethoxy-5-trifluormethyl-Phenyl | Methyl | |
| 3.673 | 3-Nitro-5-trifluormethyl-Phenyl | Methyl | |
| 3.674 | 3-Acetoxy-5-trifluormethyl-Phenyl | Methyl | |
| 3.675 | 3-Methylsulfanyl-5-trifluormethyl-Phenyl | Methyl | |
| 3.676 | 3,5-Di(methoxycarbonyl)-Phenyl | Methyl | |
| 3.677 | 3-Methoxy-5-(methoxycarbonyl)-Phenyl | Methyl | |
| 3.678 | 3-Ethoxy-5-(methoxycarbonyl)-Phenyl | Methyl | |
| 3.679 | 3-nPropyoxy-5-(methoxycarbonyl)-Phenyl | Methyl | |
| 3.680 | 3-isoButoxy-5-(methoxycarbonyl)-Phenyl | Methyl | |
| 3.681 | 3-Difluormethoxy-5-(methoxycarbonyl)-Phenyl | Methyl | |
| 3.682 | 3-Trifluormethoxy-5-(methoxycarbonyl)-Phenyl | Methyl | |
| 3.683 | 3-Nitro-5-(methoxycarbonyl)-Phenyl | Methyl | |
| 3.684 | 3-Acetoxy-5-(methoxycarbonyl)-Phenyl | Methyl | |
| 3.685 | 3-Methylsulfanyl-5-(methoxycarbonyl)-Phenyl | Methyl | |
| 3.686 | 3,5-Dimethoxy-Phenyl | Methyl | |
| 3.687 | 3,5-Dimethoxy-Phenyl | Ethyl | |
| 3.688 | 3,5-Dimethoxy-Phenyl | CN | |
| 3.689 | 3,5-Dimethoxy-Phenyl | Vinyl | |
| 3.690 | 3,5-Dimethoxy-Phenyl | Fluormethyl | |
| 3.691 | 3,5-Dimethoxy-Phenyl | Hydroxymethyl | |
| 3.692 | 3,5-Dimethoxy-Phenyl | Ethinyl | |
| 3.693 | 3-Ethoxy-5-methoxy-Phenyl | Methyl | |
| 3.694 | 3-nPropyoxy-5-methoxy-Phenyl | Methyl | |
| 3.695 | 3-isoButoxy-5-methoxy-Phenyl | Methyl | |
| 3.696 | 3-Difluormethoxy-5-methoxy-Phenyl | Methyl | |
| 3.697 | 3-Trifluormethoxy-5-methoxy-Phenyl | Methyl | |
| 3.698 | 3-Nitro-5-methoxy-Phenyl | Methyl | |
| 3.699 | 3-Acetoxy-5-methoxy-Phenyl | Methyl | |
| 3.700 | 3-Methylsulfanyl-5-methoxy-Phenyl | Methyl | |
| 3.701 | 3,5-Diethoxy-Phenyl | Methyl | |
| 3.702 | 3-nPropyoxy-5-ethoxy-Phenyl | Methyl | |
| 3.703 | 3-nButoxy-5-ethoxy-Phenyl | Methyl | |
| 3.704 | 3-isoButoxy-5-ethoxy-Phenyl | Methyl | |
| 3.705 | 3-Difluormethoxy-5-ethoxy-Phenyl | Methyl | |
| 3.706 | 3-Trifluormethoxy-5-ethoxy-Phenyl | Methyl | |
| 3.707 | 3-Nitro-5-ethoxy-Phenyl | Methyl | |
| 3.708 | 3-Acetoxy-5-ethoxy-Phenyl | Methyl | |
| 3.709 | 3-Methylsulfanyl-5-ethoxy-Phenyl | Methyl | |
| 3.710 | 3,5-Di(isopropyoxy)-Phenyl | Methyl | |
| 3.711 | 3-isoButoxy-5-isopropoxy-Phenyl | Methyl | |
| 3.712 | 3-Difluormethoxy-5-isopropoxy-Phenyl | Methyl | |
| 3.713 | 3-Trifluormethoxy-5-isopropoxy-Phenyl | Methyl | |
| 3.714 | 3-Nitro-5-isopropoxy-Phenyl | Methyl | |
| 3.715 | 3-Acetoxy-5-isopropoxy-Phenyl | Methyl | |
| 3.716 | 3-Methylsulfanyl-5-isopropoxy-Phenyl | Methyl | |
| 3.717 | 3,5-Di(trifluormethoxy)-Phenyl | Methyl | |
| 3.718 | 3-Nitro-5-trifluormethoxy-Phenyl | Methyl | |
| 3.719 | 3-Methylsulfanyl-5-trifluormethoxy-Phenyl | Methyl | |
| 3.720 | 3,5-Bis(difluormethoxy)-Phenyl | Methyl | |
| 3.721 | 3-Nitro-5-difluormethoxy-Phenyl | Methyl | |
| 3.722 | 3-Acetoxy-5-difluormethoxy-Phenyl | Methyl | |
| 3.723 | 3-Methylsulfanyl-5-acetoxy-Phenyl | Methyl | |
| 3.724 | 3-Acetoxy-5-nitro-Phenyl | Methyl | |
| 3.725 | 3-Methylsulfanyl-5-nitro-Phenyl | Methyl | |
| 3.726 | 3,5-Di(methylsulfanyl)-Phenyl | Methyl | |
| 3.727 | 3,4-Difluor-Phenyl | Methyl | |
| 3.728 | 3,4-Difluor-Phenyl | Ethyl | |
| 3.729 | 3,4-Difluor-Phenyl | CN | |
| 3.730 | 3,4-Difluor-Phenyl | Vinyl | |
| 3.731 | 3,4-Difluor-Phenyl | Fluormethyl | |
| 3.732 | 3,4-Difluor-Phenyl | Hydroxymethyl | |
| 3.733 | 3,4-Difluor-Phenyl | Ethinyl | |
| 3.734 | 3-Chlor-4-fluor-Phenyl | Methyl | |
| 3.735 | 3-Brom-4-fluor-Phenyl | Methyl | |
| 3.736 | 3-Methyl-4-fluor-Phenyl | Methyl | |
| 3.737 | 3-Methyl-4-fluor-Phenyl | Ethyl | |
| 3.738 | 3-Methyl-4-fluor-Phenyl | CN | |
| 3.739 | 3-Methyl-4-fluor-Phenyl | Vinyl | |
| 3.740 | 3-Methyl-4-fluor-Phenyl | Fluormethyl | |
| 3.741 | 3-Methyl-4-fluor-Phenyl | Hydroxymethyl | |
| 3.742 | 3-Methyl-4-fluor-Phenyl | Ethinyl | |
| 3.743 | 3-Ethyl-4-fluor-Phenyl | Methyl | |
| 3.744 | 3-Cyclopropyl-4-fluor-Phenyl | Methyl | |
| 3.745 | 3-Cyano-4-fluor-Phenyl | Methyl | |
| 3.746 | 3-Methoxy-4-fluor-Phenyl | Methyl | |
| 3.747 | 3-Ethoxy-4-fluor-Phenyl | Methyl | |
| 3.748 | 3-Trifluormethoxy-4-fluor-Phenyl | Methyl | |
| 3.749 | 3-Nitro-4-fluor-Phenyl | Methyl | |
| 3.750 | 3-Fluor-4-chlor-Phenyl | Methyl | |
| 3.751 | 3,4-Dichlor-Phenyl | Methyl | [CDCl₃] 1.92 (s, 3H), 3.38 (d, 1H), 3.82 (d, 1H), 7.5 (m, 2H), 7.7 (d, 1H). |
| 3.752 | 3,4-Dichlor-Phenyl | tert. Butyl | [DMSO] 1,10 (s, 9H); 4,2 (dd, 2H); 7,75 (m, 2H); 7,95 (d, 1H). |
| 3.753 | 3-,4-Dichlor-Phenyl | isoPropyl | [DMSO] 1,09 (2d; 6H); 2,28 (m, 1H); 3,85 (d, 1H); 4,10 (d, 1H); 7,72 (dd, 1H); 7,78 (d, 1H); 7,93 (d, 1H). |
| 3.754 | 3-Cyclopropyl-4-chlor-Phenyl | Methyl | |
| 3.755 | 3-Cyano-4-chlor-Phenyl | Methyl | |
| 3.756 | 3-Trifluormethyl-4-chlor-Phenyl | Methyl | |
| 3.757 | 3-Methoxy-4-chlor-Phenyl | Methyl | |
| 3.758 | 3-Ethoxy-4-chlor-Phenyl | Methyl | |
| 3.759 | 3-Trifluormethoxy-4-chlor-Phenyl | Methyl | |
| 3.760 | 3-Nitro-4-chlor-Phenyl | Methyl | |
| 3.761 | 3-Fluor-4-brom-Phenyl | Methyl | |
| 3.762 | 3-Chlor-4-brom-Phenyl | Methyl | |
| 3.763 | 3,4-Dibrom-Phenyl | Methyl | |
| 3.764 | 3-Methyl-4-brom-Phenyl | Methyl | |
| 3.765 | 3-Cyclopropyl-4-brom-Phenyl | Methyl | |
| 3.766 | 3-Cyano-4-brom-Phenyl | Methyl | |
| 3.767 | 3-Trifluormethyl-4-brom-Phenyl | Methyl | |
| 3.768 | 3-Methoxy-4-Phenyl | Methyl | |
| 3.769 | 3-Ethoxy-4-brom-Phenyl | Methyl | |
| 3.770 | 3-Trifluormethoxy-4-brom-Phenyl | Methyl | |
| 3.771 | 3-Nitro-4-brom-Phenyl | Methyl | |
| 3.772 | 3-Fluor-4-iod-Phenyl | Methyl | |
| 3.773 | 3-Chlor-4-iod-Phenyl | Methyl | |
| 3.774 | 3-Brom-4-iod-Phenyl | Methyl | |
| 3.775 | 3-Methyl-4-iod-Phenyl | Methyl | |
| 3.776 | 3-Cyclopropyl-4-iod-Phenyl | Methyl | |
| 3.777 | 3-Cyano-4-iod-Phenyl | Methyl | |
| 3.778 | 3-Trifluormethyl-4-iod-Phenyl | Methyl | |
| 3.779 | 3-Methoxy-4-iod-Phenyl | Methyl | |
| 3.780 | 3-Ethoxy-4-iod-Phenyl | Methyl | |
| 3.781 | 3-Trifluormethoxy-4-iod-Phenyl | Methyl | |
| 3.782 | 3-Nitro-4-iod-Phenyl | Methyl | |
| 3.783 | 3-Fluor-4-methyl-Phenyl | Methyl | |
| 3.784 | 3-Chlor-4-methyl-Phenyl | Methyl | |
| 3.785 | 3-Chlor-4-methyl-Phenyl | Ethyl | |
| 3.786 | 3-Chlor-4-methyl-Phenyl | CN | |
| 3.787 | 3-Chlor-4-methyl-Phenyl | Vinyl | |
| 3.788 | 3-Chlor-4-methyl-Phenyl | Fluormethyl | |
| 3.789 | 3-Chlor-4-methyl-Phenyl | Hydroxymethyl | |
| 3.790 | 3-Chlor-4-methyl-Phenyl | Ethinyl | |
| 3.791 | 3-Brom-4-methyl-Phenyl | Methyl | |
| 3.792 | 3.4-Dimethyl-Phenyl | Methyl | |
| 3.793 | 3-Cyclopropyl-4-methyl-Phenyl | Methyl | |
| 3.794 | 3-Cyano-4-methyl-Phenyl | Methyl | |
| 3.795 | 3-Trifluormethyl-4-methyl-Phenyl | Methyl | |
| 3.796 | 3-Methoxy-4-methyl-Phenyl | Methyl | |
| 3.797 | 3-Ethoxy-4-methyl-Phenyl | Methyl | |
| 3.798 | 3-Trifluormethoxy-4-methyl-Phenyl | Methyl | |
| 3.799 | 3-Nitro-4-methyl-Phenyl | Methyl | |
| 3.800 | 3-Fluor-4-ethyl-Phenyl | Methyl | |
| 3.801 | 3-Chlor-4-ethyl-Phenyl | Methyl | |
| 3.802 | 3-Brom-4-ethyl-Phenyl | Methyl | |
| 3.803 | 3-Methyl-4-ethyl-Phenyl | Methyl | |
| 3.804 | 3,4-Diethyl-Phenyl | Methyl | |
| 3.805 | 3-Cyclopropyl-4-ethyl-Phenyl | Methyl | |
| 3.806 | 3-Cyano-4-ethyl-Phenyl | Methyl | |
| 3.807 | 3-Trifluormethyl-4-ethyl-Phenyl | Methyl | |
| 3.808 | 3-Methoxy-4-ethyl-Phenyl | Methyl | |
| 3.809 | 3-Ethoxy-4-ethyl-Phenyl | Methyl | |
| 3.810 | 3-Trifluormethoxy-4-ethyl-Phenyl | Methyl | |
| 3.811 | 3-Nitro-4-ethyl-Phenyl | Methyl | |
| 3.812 | 3-Fluor-4-propyl-Phenyl | Methyl | |
| 3.813 | 3-Chlor-4-propyl-Phenyl | Methyl | |
| 3.814 | 3-Brom-4-propyl-Phenyl | Methyl | |
| 3.815 | 3-Methyl-4-propyl-Phenyl | Methyl | |
| 3.816 | 3-Cyclopropyl-4-propyl-Phenyl | Methyl | |
| 3.817 | 3-Cyano-4-propyl-Phenyl | Methyl | |
| 3.818 | 3-Trifluormethyl-4-propyl-Phenyl | Methyl | |
| 3.819 | 3-Methoxy-4-propyl-Phenyl | Methyl | |
| 3.820 | 3-Ethoxy-4-propyl-Phenyl | Methyl | |
| 3.821 | 3-Trifluormethoxy-4-propyl-Phenyl | Methyl | |
| 3.822 | 3-Nitro-4-propyl-Phenyl | Methyl | |
| 3.823 | 3-Fluor-4-isopropyl-Phenyl | Methyl | |
| 3.824 | 3-Chlor-4-isopropyl-Phenyl | Methyl | |
| 3.825 | 3-Brom-4-isopropyl-Phenyl | Methyl | |
| 3.826 | 3-Methyl-4-isopropyl-Phenyl | Methyl | |
| 3.827 | 3-Cyclopropyl-4-isopropyl-Phenyl | Methyl | |
| 3.828 | 3-Cyano-4-isopropyl-Phenyl | Methyl | |
| 3.829 | 3-Trifluormethyl-4-isopropyl-Phenyl | Methyl | |
| 3.830 | 3-Methoxy-4-isopropyl-Phenyl | Methyl | |
| 3.831 | 3-Ethoxy-4-isopropyl-Phenyl | Methyl | |
| 3.832 | 3-Trifluormethoxy-4-isopropyl-Phenyl | Methyl | |
| 3.833 | 3-Nitro-4-isopropyl-Phenyl | Methyl | |
| 3.834 | 3-Fluor-4-tert.butyl-Phenyl | Methyl | |
| 3.835 | 3-Fluor-4-tert.butyl-Phenyl | Ethyl | |
| 3.836 | 3-Fluor-4-tert.butyl-Phenyl | CN | |
| 3.837 | 3-Fluor-4-tert.butyl-Phenyl | Vinyl | |
| 3.838 | 3-Fluor-4-tert.butyl-Phenyl | Fluormethyl | |
| 3.839 | 3-Fluor-4-tert.butyl-Phenyl | Hydroxymethyl | |
| 3.840 | 3-Fluor-4-tert.butyl-Phenyl | Ethinyl | |
| 3.841 | 3-Chlor-4-tert.buty-Phenyl | Methyl | |
| 3.842 | 3-Brom-4-tert.butyl-Phenyl | Methyl | |
| 3.843 | 3-Methyl-4-tert.butyl-Phenyl | Methyl | |
| 3.844 | 3-Ethyl-4-tert.butyl-Phenyl | Methyl | |
| 3.845 | 3-Cyclopropyl-4-tert.butyl-Phenyl | Methyl | |
| 3.846 | 3-Cyano-4-tert.butyl-Phenyl | Methyl | |
| 3.847 | 3-Trifluormethyl-4-tert.butyl-Phenyl | Methyl | |
| 3.848 | 3-Methoxy-4-tert.butyl-Phenyl | Methyl | |
| 3.849 | 3-Ethoxy-4-tert.butyl-Phenyl | Methyl | |
| 3.850 | 3-Trifluormethoxy-4-tert.butyl-Phenyl | Methyl | |
| 3.851 | 3-Nitro-4-tert.butyl-Phenyl | Methyl | |
| 3.852 | 3-Fluor-4-cyclopropyl-Phenyl | Methyl | |
| 3.853 | 3-Chlor-4-cyclopropyl-Phenyl | Methyl | |
| 3.854 | 3-Brom-4-cyclopropyl-Phenyl | Methyl | |
| 3.855 | 3-Methyl-4-cyclopropyl-Phenyl | Methyl | |
| 3.856 | 3-Cyclopropyl-4-cyclopropyl-Phenyl | Methyl | |
| 3.857 | 3-Cyano-4-cyclopropyl-Phenyl | Methyl | |
| 3.858 | 3-Trifluormethyl-4-cyclopropyl-Phenyl | Methyl | |
| 3.859 | 3-Methoxy-4-cyclopropyl-Phenyl | Methyl | |
| 3.860 | 3-Ethoxy-4-cyclopropyl-Phenyl | Methyl | |
| 3.861 | 3-Trifluormethoxy-4-cyclopropyl-Phenyl | Methyl | |
| 3.862 | 3-Fluor-4-methoxycarbonyl-Phenyl | Methyl | |
| 3.863 | 3-Chlor-4-methoxycarbonyl-Phenyl | Methyl | |
| 3.864 | 3-Brom-4-methoxycarbonyl-Phenyl | Methyl | |
| 3.865 | 3-Methyl-4-methoxycarbonyl-Phenyl | Methyl | |
| 3.866 | 3-Cyclopropyl-4-methoxycarbonyl-Phenyl | Methyl | |
| 3.867 | 3-Cyano-4-methoxycarbonyl-Phenyl | Methyl | |
| 3.868 | 3-Trifluormethyl-4-methoxycarbonyl-Phenyl | Methyl | |
| 3.869 | 3-Methoxy-4-methoxycarbonyl-Phenyl | Methyl | |
| 3.870 | 3-Ethoxy-4-methoxycarbonyl-Phenyl | Methyl | |
| 3.871 | 3-Nitro-4-methoxycarbonyl-Phenyl | Methyl | |
| 3.872 | 3-Fluor-4-cyano-Phenyl | Methyl | |
| 3.873 | 3-Chlor-4-cyano-Phenyl | Methyl | |
| 3.874 | 3-Brom-4-cyano-Phenyl | Methyl | |
| 3.875 | 3-Methyl-4-cyano-Phenyl | Methyl | |
| 3.876 | 3-Cyclopropyl-4-cyano-Phenyl | Methyl | |
| 3.877 | 3-Cyano-4-cyanoPhenyl | Methyl | |
| 3.878 | 3-Trifluormethyl-4-cyano-Phenyl | Methyl | |
| 3.879 | 3-Methoxy-4-cyano-Phenyl | Methyl | |
| 3.880 | 3-Ethoxy-4-cyano-Phenyl | Methyl | |
| 3.881 | 3-Trifluormethoxy-4-cyano-Phenyl | Methyl | |
| 3.882 | 3-Nitro-4-cyano-Phenyl | Methyl | |
| 3.883 | 3-Fluor-4-methoxy-Phenyl | Methyl | |
| 3.884 | 3-Chlor-4-methoxy-Phenyl | Methyl | |
| 3.885 | 3-Brom-4-methoxy-Phenyl | Methyl | |
| 3.886 | 3-Methyl-4-methoxy-Phenyl | Methyl | |
| 3.887 | 3-Cyclopropyl-4-methoxy-Phenyl | Methyl | |
| 3.888 | 3-Cyano-4-methoxy-Phenyl | Methyl | |
| 3.889 | 3-Trifluormethyl-4-methoxy-Phenyl | Methyl | |
| 3.890 | 3,4-Dimethoxy-Phenyl | Methyl | |
| 3.891 | 3-Ethoxy-4-methoxy-Phenyl | Methyl | |
| 3.892 | 3-Trifluormethoxy-4-methoxy-Phenyl | Methyl | |
| 3.893 | 3-Nitro-4-methoxy-Phenyl | Methyl | |
| 3.894 | 3-Fluor-4-ethoxy-Phenyl | Methyl | |
| 3.895 | 3-Chlor-4-ethoxy-Phenyl | Methyl | |
| 3.896 | 3-Brom-4-ethoxy-Phenyl | Methyl | |
| 3.897 | 3-Methyl-4-ethoxy-Phenyl | Methyl | |
| 3.898 | 3-Cyclopropyl-4-ethoxy-Phenyl | Methyl | |
| 3.899 | 3-Cyano-4-ethoxy-Phenyl | Methyl | |
| 3.900 | 3-Trifluormethyl-4-ethoxy-Phenyl | Methyl | |
| 3.901 | 3-Methoxy-4-ethoxy-Phenyl | Methyl | |
| 3.902 | 2,4-Diethoxy-Phenyl | Methyl | |
| 3.903 | 3-Trifluormethoxy-4-ethoxy-Phenyl | Methyl | |
| 3.904 | 3-Nitro-4-ethoxy-Phenyl | Methyl | |
| 3.905 | 3-Fluor-4-isopropoxy-Phenyl | Methyl | |
| 3.906 | 3-Chlor-4-isopropoxy-Phenyl | Methyl | |
| 3.907 | 3-Brom-4-isopropoxy-Phenyl | Methyl | |
| 3.908 | 3-Methyl-4-isopropoxy-Phenyl | Methyl | |
| 3.909 | 3-Cyclopropyl-4-isopropoxy-Phenyl | Methyl | |
| 3.910 | 3-Cyano-4-isopropoxy-Phenyl | Methyl | |
| 3.911 | 3-Trifluormethyl-4-isopropoxy-Phenyl | Methyl | |
| 3.912 | 3-Methoxy-4-isopropoxy-Phenyl | Methyl | |
| 3.913 | 3-Ethoxy-4-isopropoxy-Phenyl | Methyl | |
| 3.914 | 3-Trifluormethoxy-4-isopropoxy-Phenyl | Methyl | |
| 3.915 | 3-Nitro-4-isopropoxy-Phenyl | Methyl | |
| 3.916 | 3-Fluor-4-trifluormethoxy-Phenyl | Methyl | |
| 3.917 | 3-Chlor-4-trifluormethoxy-Phenyl | Methyl | |
| 3.918 | 3-Brom-4-trifluormethoxy-Phenyl | Methyl | |
| 3.919 | 3-Methyl-4-trifluormethoxy-Phenyl | Methyl | |
| 3.920 | 3-Ethyl-4-trifluormethoxy-Phenyl | Methyl | |
| 3.921 | 3-Cyclopropyl-4-trifluormethoxy-Phenyl | Methyl | |
| 3.922 | 3-Cyano-4-trifluormethoxy-Phenyl | Methyl | |
| 3.923 | 3-Trifluormethyl-4-trifluormethoxy-Phenyl | Methyl | |
| 3.924 | 3-Methoxy-4-trifluormethoxy-Phenyl | Methyl | |
| 3.925 | 3-Ethoxy-4-trifluormethoxy-Phenyl | Methyl | |
| 3.926 | 3,4-Bis(trifluormethoxy)-Phenyl | Methyl | |
| 3.927 | 3-Nitro-4-trifluormethoxy-Phenyl | Methyl | |
| 3.928 | 3-Fluor-4-difluormethoxy-Phenyl | Methyl | |
| 3.929 | 3-Chlor-4-difluormethoxy-Phenyl | Methyl | |
| 3.930 | 3-Brom-4-difluormethoxy-Phenyl | Methyl | |
| 3.931 | 3-Methyl-4-difluormethoxy-Phenyl | Methyl | |
| 3.932 | 3-Cyclopropyl-4-difluormethoxy-Phenyl | Methyl | |
| 3.933 | 3-Cyano-4-difluormethoxy-Phenyl | Methyl | |
| 3.934 | 3-Trifluormethyl-4-difluormethoxy-Phenyl | Methyl | |
| 3.935 | 3-Methoxy-4-difluormethoxy-Phenyl | Methyl | |
| 3.936 | 3-Ethoxy-4-difluormethoxy-Phenyl | Methyl | |
| 3.937 | 3-Nitro-4-difluormethoxy-Phenyl | Methyl | |
| 3.938 | 3-Fluor-4-nitro-Phenyl | Methyl | |
| 3.939 | 3-Chlor-4-nitro-Phenyl | Methyl | |
| 3.940 | 3-Brom-4-nitro-Phenyl | Methyl | |
| 3.941 | 3-Methyl-4-nitro-Phenyl | Methyl | |
| 3.942 | 3-Cyclopropyl-4-nitro-Phenyl | Methyl | |
| 3.943 | 3-Cyano-4-nitro-Phenyl | Methyl | |
| 3.944 | 3-Trifluormethyl-4-nitro-Phenyl | Methyl | |
| 3.945 | 3-Methoxy-4-nitro-Phenyl | Methyl | |
| 3.946 | 3-Ethoxy-4-nitro-Phenyl | Methyl | |
| 3.947 | 3-Trifluormethoxy-4-nitro-Phenyl | Methyl | |
| 3.948 | 3-Fluor-4-methylsulfanylPhenyl | Methyl | |
| 3.949 | 3-Chlor-4-methylsulfanyl-Phenyl | Methyl | |
| 3.950 | 3-Brom-4-methylsulfanyl-Phenyl | Methyl | |
| 3.951 | 3-Methyl-4-methylsulfanyl-Phenyl | Methyl | |
| 3.952 | 3-Cyclopropyl-4-methylsulfanyl-Phenyl | Methyl | |
| 3.953 | 3-Cyano-4-methylsulfanyl-Phenyl | Methyl | |
| 3.954 | 3-Trifluormethyl-4-methylsulfanyl-Phenyl | Methyl | |
| 3.955 | 3-Methoxy-4-methylsulfanyl-Phenyl | Methyl | |
| 3.956 | 3-Ethoxy-4-methylsulfanyl-Phenyl | Methyl | |
| 3.957 | 3-Trifluormethoxy-4-methylsulfanyl-Phenyl | Methyl | |
| 3.958 | 3-Nitro-4-methylsulfanyl-Phenyl | Methyl | |
| 3.959 | 3,6-Difluor-Phenyl | Methyl | |
| 3.960 | 3,6-Difluor-Phenyl | Ethyl | |
| 3.961 | 3,6-Difluor-Phenyl | CN | |
| 3.962 | 3,6-Difluor-Phenyl | Vinyl | |
| 3.963 | 3,6-Difluor-Phenyl | Fluormethyl | |
| 3.964 | 3,6-Difluor-Phenyl | Hydroxymethyl | |
| 3.965 | 3,6-Difluor-Phenyl | Ethinyl | |
| 3.966 | 3-Chlor-6-fluor-Phenyl | Methyl | |
| 3.967 | 3-Brom-6-fluor-Phenyl | Methyl | |
| 3.968 | 3-Methyl-6-fluor-Phenyl | Methyl | |
| 3.969 | 3-Cyclopropyl-6-fluor-Phenyl | Methyl | |
| 3.970 | 3-Cyano-6-fluor-Phenyl | Methyl | |
| 3.971 | 3-Methoxy-6-fluor-Phenyl | Methyl | |
| 3.972 | 3-Ethoxy-6-fluor-Phenyl | Methyl | |
| 3.973 | 3-Trifluormethoxy-6-fluor-Phenyl | Methyl | |
| 3.974 | 3-Nitro-6-fluor-Phenyl | Methyl | |
| 3.975 | 3-Fluor-6-chlor-Phenyl | Methyl | |
| 3.976 | 3,6-Dichlor-Phenyl | Methyl | |
| 3.977 | 3-Brom-6-chlor-Phenyl | Methyl | |
| 3.978 | 3-Methyl-6-chlor-Phenyl | Methyl | |
| 3.979 | 3-Cyclopropyl-6-chlor-Phenyl | Methyl | |
| 3.980 | 3-Cyano-6-chlor-Phenyl | Methyl | |
| 3.981 | 3-Trifluormethyl-6-chlor-Phenyl | Methyl | |
| 3.982 | 3-Methoxy-6-chlor-Phenyl | Methyl | |
| 3.983 | 3-Ethoxy-6-chlor-Phenyl | Methyl | |
| 3.984 | 3-Trifluormethoxy-6-chlor-Phenyl | Methyl | |
| 3.985 | 3-Nitro-6-chlor-Phenyl | Methyl | |
| 3.986 | 3-Fluor-6-brom-Phenyl | Methyl | |
| 3.987 | 3-Chlor-6-brom-Phenyl | Methyl | |
| 3.988 | 3,6-Dibrom-Phenyl | Methyl | |
| 3.989 | 3-Methyl-6-brom-Phenyl | Methyl | |
| 3.990 | 3-Ethyl-6-brom-Phenyl | Methyl | |
| 3.991 | 3-Cyclopropyl-6-brom-Phenyl | Methyl | |
| 3.992 | 3-Cyano-6-brom-Phenyl | Methyl | |
| 3.993 | 3-Trifluormethyl-6-brom-Phenyl | Methyl | |
| 3.994 | 3-Methoxy-6-Phenyl | Methyl | |
| 3.995 | 3-Ethoxy-6-brom-Phenyl | Methyl | |
| 3.996 | 3-Trifluormethoxy-6-brom-Phenyl | Methyl | |
| 3.997 | 3-Nitro-6-brom-Phenyl | Methyl | |
| 3.998 | 3-Fluor-6-iod-Phenyl | Methyl | |
| 3.999 | 3-Chlor-6-iod-Phenyl | Methyl | |
| 3.1000 | 3-Brom-6-iod-Phenyl | Methyl | |
| 3.1001 | 3-Methyl-6-iod-Phenyl | Methyl | |
| 3.1002 | 3-Cyclopropyl-6-iod-Phenyl | Methyl | |
| 3.1003 | 3-Cyano-6-iod-Phenyl | Methyl | |
| 3.1004 | 3-Trifluormethyl-6-iod-Phenyl | Methyl | |
| 3.1005 | 3-Methoxy-6-iod-Phenyl | Methyl | |
| 3.1006 | 3-Ethoxy-6-iod-Phenyl | Methyl | |
| 3.1007 | 3-Trifluormethoxy-6-iod-Phenyl | Methyl | |
| 3.1008 | 3-Nitro-6-iod-Phenyl | Methyl | |
| 3.1009 | 3-Fluor-6-methyl-Phenyl | Methyl | |
| 3.1010 | 3-Chlor-6-methyl-Phenyl | Methyl | |
| 3.1011 | 3-Brom-6-methyl-Phenyl | Methyl | |
| 3.1012 | 3.6-Dimethyl-Phenyl | Methyl | |
| 3.1013 | 3.6-Dimethyl-Phenyl | Ethyl | |
| 3.1014 | 3.6-Dimethyl-Phenyl | CN | |
| 3.1015 | 3.6-Dimethyl-Phenyl | Vinyl | |
| 3.1016 | 3.6-Dimethyl-Phenyl | Fluormethyl | |
| 3.1017 | 3.6-Dimethyl-Phenyl | Hydroxymethyl | |
| 3.1018 | 3.6-Dimethyl-Phenyl | Ethinyl | |
| 3.1019 | 3-Cyclopropyl-6-methyl-Phenyl | Methyl | |
| 3.1020 | 3-Cyano-6-methyl-Phenyl | Methyl | |
| 3.1021 | 3-Trifluormethyl-6-methyl-Phenyl | Methyl | |
| 3.1022 | 3-Methoxy-6-methyl-Phenyl | Methyl | |
| 3.1023 | 3-Ethoxy-6-methyl-Phenyl | Methyl | |
| 3.1024 | 3-Trifluormethoxy-6-methyl-Phenyl | Methyl | |
| 3.1025 | 3-Nitro-6-methyl-Phenyl | Methyl | |
| 3.1026 | 3-Fluor-6-ethyl-Phenyl | Methyl | |
| 3.1027 | 3-Chlor-6-ethyl-Phenyl | Methyl | |
| 3.1028 | 3-Brom-6-ethyl-Phenyl | Methyl | |
| 3.1029 | 3-Methyl-6-ethyl-Phenyl | Methyl | |
| 3.1030 | 3,6-Diethyl-Phenyl | Methyl | |
| 3.1031 | 3-Cyclopropyl-6-ethyl-Phenyl | Methyl | |
| 3.1032 | 3-Cyano-6-ethyl-Phenyl | Methyl | |
| 3.1033 | 3-Trifluormethyl-6-ethyl-Phenyl | Methyl | |
| 3.1034 | 3-Methoxy-6-ethyl-Phenyl | Methyl | |
| 3.1035 | 3-Ethoxy-6-ethyl-Phenyl | Methyl | |
| 3.1036 | 3-Trifluormethoxy-6-ethyl-Phenyl | Methyl | |
| 3.1037 | 3-Nitro-6-ethyl-Phenyl | Methyl | |
| 3.1038 | 3-Fluor-6-isopropyl-Phenyl | Methyl | |
| 3.1039 | 3-Chlor-6-isopropyl-Phenyl | Methyl | |
| 3.1040 | 3-Brom-6-isopropyl-Phenyl | Methyl | |
| 3.1041 | 3-Methyl-6-isopropyl-Phenyl | Methyl | |
| 3.1042 | 3-Cyclopropyl-6-isopropyl-Phenyl | Methyl | |
| 3.1043 | 3-Cyano-6-isopropyl-Phenyl | Methyl | |
| 3.1044 | 3-Trifluormethyl-6-isopropyl-Phenyl | Methyl | |
| 3.1045 | 3-Methoxy-6-isopropyl-Phenyl | Methyl | |
| 3.1046 | 3-Ethoxy-6-isopropyl-Phenyl | Methyl | |
| 3.1047 | 3-Trifluormethoxy-6-isopropyl-Phenyl | Methyl | |
| 3.1048 | 3-Nitro-6-isopropyl-Phenyl | Methyl | |
| 3.1049 | 3-Fluor-6-tert.butyl-Phenyl | Methyl | |
| 3.1050 | 3-Chlor-6-tert.butyl-Phenyl | Methyl | |
| 3.1051 | 3-Brom-6-tert.butyl-Phenyl | Methyl | |
| 3.1052 | 3-Methyl-6-tert.butyl-Phenyl | Methyl | |
| 3.1053 | 3-Cyclopropyl-6-tert.butyl-Phenyl | Methyl | |
| 3.1054 | 3-Cyano-6-tert.butyl-Phenyl | Methyl | |
| 3.1055 | 3-Trifluormethyl-6-tert.butyl-Phenyl | Methyl | |
| 3.1056 | 3-Methoxy-6-tert.butyl-Phenyl | Methyl | |
| 3.1057 | 3-Ethoxy-6-tert.butyl-Phenyl | Methyl | |
| 3.1058 | 3-Trifluormethoxy-6-tert.butyl-Phenyl | Methyl | |
| 3.1059 | 3-Nitro-6-tert.butyl-Phenyl | Methyl | |
| 3.1060 | 3-Fluor-6-cyclopropyl-Phenyl | Methyl | |
| 3.1061 | 3-Chlor-6-cyclopropyl-Phenyl | Methyl | |
| 3.1062 | 3-Brom-6-cyclopropyl-Phenyl | Methyl | |
| 3.1063 | 3-Methyl-6-cyclopropyl-Phenyl | Methyl | |
| 3.1064 | 3-Ethyl-6-cyclopropyl-Phenyl | Methyl | |
| 3.1065 | 3-Cyclopropyl-6-cyclopropyl-Phenyl | Methyl | |
| 3.1066 | 3-Cyano-6-cyclopropyl-Phenyl | Methyl | |
| 3.1067 | 3-Trifluormethyl-6-cyclopropyl-Phenyl | Methyl | |
| 3.1068 | 3-Methoxy-6-cyclopropyl-Phenyl | Methyl | |
| 3.1069 | 3-Ethoxy-6-cyclopropyl-Phenyl | Methyl | |
| 3.1070 | 3-Trifluormethoxy-6-cyclopropyl-Phenyl | Methyl | |
| 3.1071 | 3-Fluor-6-methoxycarbonyl-Phenyl | Methyl | |
| 3.1072 | 3-Chlor-6-methoxycarbonyl-Phenyl | Methyl | |
| 3.1073 | 3-Brom-6-methoxycarbonyl-Phenyl | Methyl | |
| 3.1074 | 3-Methyl-6-methoxycarbonyl-Phenyl | Methyl | |
| 3.1075 | 3-Cyclopropyl-6-methoxycarbonyl-Phenyl | Methyl | |
| 3.1076 | 3-Cyano-6-methoxycarbonyl-Phenyl | Methyl | |
| 3.1077 | 3-Trifluormethyl-6-methoxycarbonyl-Phenyl | Methyl | |
| 3.1078 | 3-Methoxy-6-methoxycarbonyl-Phenyl | Methyl | |
| 3.1079 | 3-Ethoxy-6-methoxycarbonyl-Phenyl | Methyl | |
| 3.1080 | 3-Trifluormethoxy-6-methoxycarbonyl-Phenyl | Methyl | |
| 3.1081 | 3-Nitro-6-methoxycarbonyl-Phenyl | Methyl | |
| 3.1082 | 3-Fluor-6-cyano-Phenyl | Methyl | |
| 3.1083 | 3-Chlor-6-cyano-Phenyl | Methyl | |
| 3.1084 | 3-Brom-6-cyano-Phenyl | Methyl | |
| 3.1085 | 3-Methyl-6-cyano-Phenyl | Methyl | |
| 3.1086 | 3-Cyclopropyl-6-cyano-Phenyl | Methyl | |
| 3.1087 | 3-Cyano-6-cyano-Phenyl | Methyl | |
| 3.1088 | 3-Trifluormethyl-6-cyano-Phenyl | Methyl | |
| 3.1089 | 3-Methoxy-6-cyano-Phenyl | Methyl | |
| 3.1090 | 3-Ethoxy-6-cyano-Phenyl | Methyl | |
| 3.1091 | 3-Trifluormethoxy-6-cyano-Phenyl | Methyl | |
| 3.1092 | 3-Nitro-6-cyano-Phenyl | Methyl | |
| 3.1093 | 3-Fluor-6-methoxy-Phenyl | Methyl | |
| 3.1094 | 3-Chlor-6-methoxy-Phenyl | Methyl | |
| 3.1095 | 3-Brom-6-methoxy-Phenyl | Methyl | |
| 3.1096 | 3-Methyl-6-methoxy-Phenyl | Methyl | |
| 3.1097 | 3-Cyclopropyl-6-methoxy-Phenyl | Methyl | |
| 3.1098 | 3-Cyano-6-methoxy-Phenyl | Methyl | |
| 3.1099 | 3-Trifluormethyl-6-methoxy-Phenyl | Methyl | |
| 3.1100 | 3,6-Dimethoxy--Phenyl | Methyl | |
| 3.1101 | 3-Ethoxy-6-methoxy-Phenyl | Methyl | |
| 3.1102 | 3-Trifluormethoxy-6-methoxy-Phenyl | Methyl | |
| 3.1103 | 3-Nitro-6-methoxy-Phenyl | Methyl | |
| 3.1104 | 3-Fluor-6-ethoxy-Phenyl | Methyl | |
| 3.1105 | 3-Chlor-6-ethoxy-Phenyl | Methyl | |
| 3.1106 | 3-Brom-6-ethoxy-Phenyl | Methyl | |
| 3.1107 | 3-Methyl-6-ethoxy-Phenyl | Methyl | |
| 3.1108 | 3-Cyclopropyl-6-ethoxy-Phenyl | Methyl | |
| 3.1109 | 3-Cyano-6-ethoxy-Phenyl | Methyl | |
| 3.1110 | 3-Trifluormethyl-6-ethoxy-Phenyl | Methyl | |
| 3.1111 | 3-Methoxy-6-ethoxy-Phenyl | Methyl | |
| 3.1112 | 2,6-Diethoxy-Phenyl | Methyl | |
| 3.1113 | 3-Trifluormethoxy-6-ethoxy-Phenyl | Methyl | |
| 3.1114 | 3-Nitro-6-ethoxy-Phenyl | Methyl | |
| 3.1115 | 3-Fluor-6-isopropoxy-Phenyl | Methyl | |
| 3.1116 | 3-Chlor-6-isopropoxy-Phenyl | Methyl | |
| 3.1117 | 3-Brom-6-isopropoxy-Phenyl | Methyl | |
| 3.1118 | 3-Methyl-6-isopropoxy-Phenyl | Methyl | |
| 3.1119 | 3-Cyclopropyl-6-isopropoxy-Phenyl | Methyl | |
| 3.1120 | 3-Cyano-6-isopropoxy-Phenyl | Methyl | |
| 3.1121 | 3-Trifluormethyl-6-isopropoxy-Phenyl | Methyl | |
| 3.1122 | 3-Methoxy-6-isopropoxy-Phenyl | Methyl | |
| 3.1123 | 3-Ethoxy-6-isopropoxy-Phenyl | Methyl | |
| 3.1124 | 3-Trifluormethoxy-6-isopropoxy-Phenyl | Methyl | |
| 3.1125 | 3-Nitro-6-isopropoxy-Phenyl | Methyl | |
| 3.1126 | 3-Fluor-6-trifluormethoxy-Phenyl | Methyl | |
| 3.1127 | 3-Chlor-6-trifluormethoxy-Phenyl | Methyl | |
| 3.1128 | 3-Brom-6-trifluormethoxy-Phenyl | Methyl | |
| 3.1129 | 3-Methyl-6-trifluormethoxy-Phenyl | Methyl | |
| 3.1130 | 3-Ethyl-6-trifluormethoxy-Phenyl | Methyl | |
| 3.1131 | 3-Cyclopropyl-6-trifluormethoxy-Phenyl | Methyl | |
| 3.1132 | 3-Cyano-6-trifluormethoxy-Phenyl | Methyl | |
| 3.1133 | 3-Trifluormethyl-6-trifluormethoxy-Phenyl | Methyl | |
| 3.1134 | 3-Methoxy-6-trifluormethoxy-Phenyl | Methyl | |
| 3.1135 | 3-Ethoxy-6-trifluormethoxy-Phenyl | Methyl | |
| 3.1136 | 3,6-Bis(trifluormethoxy)-Phenyl | Methyl | |
| 3.1137 | 3-Nitro-6-trifluormethoxy-Phenyl | Methyl | |
| 3.1138 | 3-Fluor-6-difluormethoxy-Phenyl | Methyl | |
| 3.1139 | 3-Chlor-6-difluormethoxy-Phenyl | Methyl | |
| 3.1140 | 3-Brom-6-difluormethoxy-Phenyl | Methyl | |
| 3.1141 | 3-Methyl-6-difluormethoxy-Phenyl | Methyl | |
| 3.1142 | 3-Cyclopropyl-6-difluormethoxy -Phenyl | Methyl | |
| 3.1143 | 3-Cyano-6-difluormethoxy-Phenyl | Methyl | |
| 3.1144 | 3-Trifluormethyl-6-difluormethoxy -Phenyl | Methyl | |
| 3.1145 | 3-Methoxy-6-difluormethoxy -Phenyl | Methyl | |
| 3.1146 | 3-Ethoxy-6-difluormethoxy-Phenyl | Methyl | |
| 3.1147 | 3-Nitro-6-difluormethoxy-Phenyl | Methyl | |
| 3.1148 | 3-Fluor-6-nitro-Phenyl | Methyl | |
| 3.1149 | 3-Chlor-6-nitro-Phenyl | Methyl | |
| 3.1150 | 3-Brom-6-nitro-Phenyl | Methyl | |
| 3.1151 | 3-Methyl-6-nitro-Phenyl | Methyl | |
| 3.1152 | 3-Ethyl-6-nitro-Phenyl | Methyl | |
| 3.1153 | 3-Cyclopropyl-6-nitro-Phenyl | Methyl | |
| 3.1154 | 3-Cyano-6-nitro-Phenyl | Methyl | |
| 3.1155 | 3-Trifluormethyl-6-nitro-Phenyl | Methyl | |
| 3.1156 | 3-Methoxy-6-nitro-Phenyl | Methyl | |
| 3.1157 | 3-Ethoxy-6-nitro-Phenyl | Methyl | |
| 3.1158 | 3-Trifluormethoxy-6-nitro-Phenyl | Methyl | |
| 3.1159 | 3-Fluor-6-methylsulfanylPhenyl | Methyl | |
| 3.1160 | 3-Chlor-6-methylsulfanyl-Phenyl | Methyl | |
| 3.1161 | 3-Brom-6-methylsulfanyl-Phenyl | Methyl | |
| 3.1162 | 3-Methyl-6-methylsulfanyl-Phenyl | Methyl | |
| 3.1163 | 3-Cyclopropyl-6-methylsulfanyl-Phenyl | Methyl | |
| 3.1164 | 3-Cyano-6-methylsulfanyl-Phenyl | Methyl | |
| 3.1165 | 3-Trifluormethyl-6-methylsulfanyl-Phenyl | Methyl | |
| 3.1166 | 3-Methoxy-6-methylsulfanyl-Phenyl | Methyl | |
| 3.1167 | 3-Ethoxy-6-methylsulfanyl-Phenyl | Methyl | |
| 3.1168 | 3-Trifluormethoxy-6-nnethylsulfanyl-Phenyl | Methyl | |
| 3.1169 | 3-Nitro-6-methylsulfanyl-Phenyl | Methyl | |
| 3.1170 | 2,3,4-Trifluor-Phenyl | Methyl | |
| 3.1171 | 2,3,4-Trichlor-Phenyl | Methyl | |
| 3.1172 | 2,3.4-Trimethyl-Phenyl | Methyl | |
| 3.1173 | 2-Fluor-2-chlor-5-trifluormethyl-Phenyl | Methyl | |
| 3.1174 | 2,3,5-Trifluor-Phenyl | Methyl | |
| 3.1175 | 2,3,5-Trichlor-Phenyl | Methyl | [CDCl3] 1,92 (s, 3H); 3,40 (d, 1H); 3,95 (d, 1H); 7,58 (s, 1H); 7,85 (s, 1H) |
| 3.1176 | 2,3,5-Trichlor-Phenyl | IsoPropyl | [DMSO] 1,10 (2d, 6H); 2,30 (m, 1H); 3.89 (d, 1H); 4,08 (d, 1H). |
| 3.1177 | 2,3,5-Trichlor-Phenyl | tert. Butyl | [CDCl3] 1,17 (s, 9H); 3,73 (dd, 2H); 7,57 (s, 1H); 7,80 (s, 1H) |
| 3.1178 | 2,3,6-Trifluor-Phenyl | Methyl | |
| 3.1179 | 2,3,6-Trichlor-Phenyl | Methyl | |
| 3.1180 | 2,3,6-Trimethyl-Phenyl | Methyl | |
| 3.1181 | 3,4,5-Trifluor-Phenyl | Methyl | |
| 3.1182 | 3,4,6-Trifluor-Phenyl | Methyl | |
| 3.1183 | 3,4,6-Trichlor-Phenyl | Methyl | [CDCl₃] 1.94 (s, 3H), 3.51 (d, 1H), 3.95 (d, 1H), 7.58 (s, 1H), 7.83 (s, 1H). |
| 3.1184 | 3,4,6-Trichlor-Phenyl | Ethyl | |
| 3.1185 | 3,4,6-Trichlor-Phenyl | CN | |
| 3.1186 | 3,4,6-Trichlor-Phenyl | Vinyl | |
| 3.1187 | 3,4,6-Trichlor-Phenyl | Fluormethyl | |
| 3.1188 | 3,4,6-Trichlor-Phenyl | Hydroxymethyl | |
| 3.1189 | 3,4,6-Trichlor-Phenyl | Ethinyl | |
| 3.1190 | 3,4,5-Trimethyl-Phenyl | Methyl | |
| 3.1191 | 3,5-Dimethyl-4-fluor-Phenyl | Methyl | |
| 3.1192 | 3,5-Dichlor-4-methoxy-Phenyl | Methyl | |
| 3.1193 | 3,5-Difluor-4-chlor-Phenyl | Methyl | |
| 3.1194 | 3,5-Dichlor-4-hydroxy-Phenyl | Methyl | |
| 3.1195 | 3,5-Trifluormethyl-4-chlor-Phenyl | Methyl | |
| 3.1196 | 3,4,6-Trifluor-Phenyl | Methyl | |
| 3.1197 | 3,4,6-Trichlor-Phenyl | Methyl | |
| 3.1198 | 3,4,6-Trimethyl-Phenyl | Methyl | |
| 3.1199 | 2,3,4,5-Pentafluor-Phenyl | Methyl | |

**Tabelle 4: Erfindungsgemäße Verbindungen der allgemeinen Formel (I), worin W* für CHO, R¹ und R² für Wasserstoff stehen, und Aryl den Rest**

| | | | |
|---|---|---|---|
| | 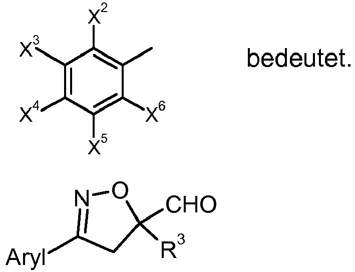 | | |

| Nr. | Aryl | R³ | Physikalische Daten |
|---|---|---|---|
| 4.001 | 3-Fluor-Phenyl | Methyl | |
| 4.002 | 3-Fluor-Phenyl | Ethyl | |
| 4.003 | 3-Fluor-Phenyl | CN | |
| 4.004 | 3-Fluor-Phenyl | Fluormethyl | |
| 4.005 | 3-Fluor-Phenyl | Methylcarbonyl | |
| 4.006 | 3-Fluor-Phenyl | Hydroxymethyl | |
| 4.007 | 3-Fluor-Phenyl | Vinyl | |
| 4.008 | 3-Fluor-Phenyl | 1-Chlorvinyl | |
| 4.009 | 3-Fluor-Phenyl | Ethinyl | |
| 4.010 | 3-Chlor-Phenyl | Methyl | |
| 4.011 | 3-Chlor-Phenyl | Ethyl | |
| 4.012 | 3-Chlor-Phenyl | CN | |
| 4.013 | 3-Chlor-Phenyl | Fluormethyl | |
| 4.014 | 3-Chlor-Phenyl | Hydroxymethyl | |
| 4.015 | 3-Chlor-Phenyl | Methylcarbonyl | |
| 4.016 | 3-Chlor-Phenyl | Fluormethyl | |
| 4.017 | 3-Chlor-Phenyl | Ethinyl | |
| 4.018 | 3-Brom-Phenyl | Methyl | |
| 4.019 | 3-Brom-Phenyl | Ethyl | |
| 4.020 | 3-Brom-Phenyl | Vinyl | |
| 4.021 | 3-Iod-Phenyl | Methyl | |
| 4.022 | 3-Methyl-Phenyl | Methyl | |
| 4.023 | 3-Ethyl-Phenyl | Methyl | |
| 4.024 | 3-Propyl-Phenyl | Methyl | |
| 4.025 | 3-isoPropyl-Phenyl | Methyl | |
| 4.026 | 3-nButyl-Phenyl | Methyl | |
| 4.027 | 3-iButyl-Phenyl | Methyl | |
| 4.028 | 3-tert.Butyl-Phenyl | Methyl | |
| 4.029 | 3-Cyclopropyl-Phenyl | Methyl | |
| 4.030 | 3-Cyclobutyl-Phenyl | Methyl | |
| 4.031 | 3-Cyclopentyl-Phenyl | Methyl | |
| 4.032 | 3-Vinyl-Phenyl | Methyl | |
| 4.033 | 3-Ethinyl-Phenyl | Methyl | |
| 4.034 | 3-Cyano-Phenyl | Methyl | |
| 4.035 | 3-Trifluormethyl-Phenyl | Methyl | |
| 4.036 | 3-Difluormethyl-Phenyl | Methyl | |
| 4.037 | 3-(Methoxycarbony)l-Phenyl | Methyl | |
| 4.038 | 3-(Ethoxycarbonyl)-Phenyl | Methyl | |
| 4.039 | 3-Hydroxymethyl-Phenyl | Methyl | |
| 4.040 | 3-Methoxy-Phenyl | Methyl | |
| 4.041 | 3-Methoxy-Phenyl | Ethyl | |
| 4.042 | 3-Methoxy-Phenyl | CN | |
| 4.043 | 3-Methoxy-Phenyl | Fluormethyl | |
| 4.044 | 3-Methoxy-Phenyl | Methylcarbonyl | |
| 4.045 | 3-Methoxy-Phenyl | Hydroxymethyl | |
| 4.046 | 3-Methoxy-Phenyl | Ethinyl | |
| 4.047 | 3-Ethoxy-Phenyl | Methyl | |
| 4.048 | 3-Propyloxy-Phenyl | Methyl | |
| 4.049 | 3-isoPropyloxy-Phenyl | Methyl | |
| 4.050 | 3-iButyloxy-Phenyl | Methyl | |
| 4.051 | 3-tButyloxy-Phenyl | Methyl | |
| 4.052 | 3-Difluormethoxy-Phenyl | Methyl | |
| 4.053 | 3-Trifluormethoxy-Phenyl | Methyl | |
| 4.054 | 3-Trifluormethoxy-Phenyl | Ethyl | |
| 4.055 | 3-Trifluormethoxy-Phenyl | CN | |
| 4.056 | 3-Trifluormethoxy-Phenyl | Fluormethyl | |
| 4.057 | 3-Trifluormethoxy-Phenyl | Methylcarbonyl | |
| 4.058 | 3-Trifluormethoxy- | Hydroxymethyl | |
| 4.059 | 3-Trifluormethoxy-Phenyl | Ethinyl | |
| 4.060 | 3-Nitro-Phenyl | Methyl | |
| 4.061 | 3-Acetoxy-Phenyl | Methyl | |
| 4.062 | 3-Methylsulfanyl-Phenyl | Methyl | |
| 4.063 | 3-Ethylsulfanyl-Phenyl | Methyl | |
| 4.064 | 3-(Pentafluor-lambda⁶-sulfanyl)-Phenyl | Methyl | |
| 4.065 | 2,3-Difluor-Phenyl | Methyl | |
| 4.066 | 2,3-Difluor-Phenyl | Ethyl | |
| 4.067 | 2,3-Difluor-Phenyl | CN | |
| 4.068 | 2,3-Difluor-Phenyl | Fluormethyl | |
| 4.069 | 2,3-Difluor-Phenyl | Methylcarbonyl | |
| 4.070 | 2,3-Difluor-Phenyl | Hydroxymethyl | |
| 4.071 | 2,3-Difluor-Phenyl | Ethinyl | |
| 4.072 | 2-Chlor-3-fluor-Phenyl | Methyl | |
| 4.073 | 2-Brom-3-fluor-Phenyl | Methyl | |
| 4.074 | 2-Methyl-3-fluor-Phenyl | Methyl | |
| 4.075 | 2-Ethyl-3-fluor-Phenyl | Methyl | |
| 4.076 | 2-Cyclopropyl-3-fluor-Phenyl | Methyl | |
| 4.077 | 2-Cyano-3-fluor-Phenyl | Methyl | |
| 4.078 | 2-Methoxy-3-fluor-Phenyl | Methyl | |
| 4.079 | 2-Trifluormethoxy-3-fluor-Phenyl | Methyl | |
| 4.080 | 2-Nitro-3-fluor-Phenyl | Methyl | |
| 4.081 | 2-Fluor-3-chlor-Phenyl | Methyl | |
| 4.082 | 2,3-Dichlor-Phenyl | Methyl | |
| 4.083 | 2,3-Dichlor-Phenyl | Ethyl | |
| 4.084 | 2,3-Dichlor-Phenyl | CN | |
| 4.085 | 2,3-Dichlor-Phenyl | Fluormethyl | |
| 4.086 | 2,3-Dichlor-Phenyl | Methylcarbonyl | |
| 4.087 | 2,3-Dichlor-Phenyl | Hydroxymethyl | |
| 4.088 | 2,3-Dichlor-Phenyl | Ethinyl | |
| 4.089 | 2-Brom-3-chlor-Phenyl | Methyl | |
| 4.090 | 2-Methyl-3-chlor-Phenyl | Methyl | |
| 4.091 | 2-Methyl-3-chlor-Phenyl | Ethyl | |
| 4.092 | 2-Methyl-3-chlor-Phenyl | CN | |
| 4.093 | 2-Methyl-3-chlor-Phenyl | Fluormethyl | |
| 4.094 | 2-Methyl-3-chlor-Phenyl | Methylcarbonyl | |
| 4.095 | 2-Methyl-3-chlor-Phenyl | Hydroxymethyl | |
| 4.096 | 2-Methyl-3-chlor-Phenyl | Ethinyl | |
| 4.097 | 2-Ethyl-3-chlor-Phenyl | Methyl | |
| 4.098 | 2-Cyclopropyl-3-chlor-Phenyl | Methyl | |
| 4.099 | 2-Cyano-3-chlor-Phenyl | Methyl | |
| 4.100 | 2-Trifluormethyl-2-chlor-Phenyl | Methyl | |
| 4.101 | 2-Methoxy-3-chlor-Phenyl | Methyl | |
| 4.102 | 2-Ethoxy-3-chlor-Phenyl | Methyl | |
| 4.103 | 2-Trifluormethoxy-3-chlor-Phenyl | Methyl | |
| 4.104 | 2-Nitro-3-chlor-Phenyl | Methyl | |
| 4.105 | 2-Fluor-3-brom-Phenyl | Methyl | |
| 4.106 | 2-Chlor-3-brom-Phenyl | Methyl | |
| 4.107 | 2,3-Dibrom-Phenyl | Methyl | |
| 4.108 | 2-Methyl-3-brom-Phenyl | Methyl | |
| 4.109 | 2-Ethyl-3-brom-Phenyl | Methyl | |
| 4.110 | 2-Cyclopropyl-3-brom-Phenyl | Methyl | |
| 4.111 | 2-Cyano-3-brom-Phenyl | Methyl | |
| 4.112 | 2-Trifluormethyl-3-brom-Phenyl | Methyl | |
| 4.113 | 2-Methoxy-3-Phenyl | Methyl | |
| 4.114 | 2-Trifluormethoxy-3-brom-Phenyl | Methyl | |
| 4.115 | 2-Nitro-3-brom-Phenyl | Methyl | |
| 4.116 | 2-Fluor-3-iod-Phenyl | Methyl | |
| 4.117 | 2-Chlor-3-iod-Phenyl | Methyl | |
| 4.118 | 2-Brom-3-iod-Phenyl | Methyl | |
| 4.119 | 2-Methyl-3-iod-Phenyl | Methyl | |
| 4.120 | 2-Ethyl-3-iod-Phenyl | Methyl | |
| 4.121 | 2-Cyclopropyl-3-iod-Phenyl | Methyl | |
| 4.122 | 2-Cyano-3-iod-Phenyl | Methyl | |
| 4.123 | 2-Trifluormethyl-3-iod-Phenyl | Methyl | |
| 4.124 | 2-Methoxy-3-iod-Phenyl | Methyl | |
| 4.125 | 2-Trifluormethoxy-3-iod-Phenyl | Methyl | |
| 4.126 | 2-Nitro-3-iod-Phenyl | Methyl | |
| 4.127 | 2-Fluor-3-methyl-Phenyl | Methyl | |
| 4.128 | 2-Fluor-3-methyl-Phenyl | Ethyl | |
| 4.129 | 2-Fluor-3-methyl-Phenyl | CN | |
| 4.130 | 2-Fluor-3-methyl-Phenyl | Fluormethyl | |
| 4.131 | 2-Fluor-3-methyl-Phenyl | Hydroxymethyl | |
| 4.132 | 2-Fluor-3-methyl-Phenyl | Ethinyl | |
| 4.133 | 2-Chlor-3-methyl-Phenyl | Methyl | |
| 4.134 | 2-Chlor-3-methyl-Phenyl | Ethyl | |
| 4.135 | 2-Chlor-3-methyl-Phenyl | CN | |
| 4.136 | 2-Chlor-3-methyl-Phenyl | Fluormethyl | |
| 4.137 | 2-Chlor-3-methyl-Phenyl | Methylcarbonyl | |
| 4.138 | 2-Chlor-3-methyl-Phenyl | Hydroxymethyl | |
| 4.139 | 2-Chlor-3-methyl-Phenyl | Ethinyl | |
| 4.140 | 2-Brom-3-methyl-Phenyl | Methyl | |
| 4.141 | 2,3-Dimethyl-Phenyl | Methyl | |
| 4.142 | 2-Ethyl-3-methyl-Phenyl | Methyl | |
| 4.143 | 2-Cyclopropyl-3-methyl-Phenyl | Methyl | |
| 4.144 | 2-Cyano-3-methyl-Phenyl | Methyl | |
| 4.145 | 2-Trifluormethyl-3-methyl-Phenyl | Methyl | |
| 4.146 | 2-Methoxy-3-methyl-Phenyl | Methyl | |
| 4.147 | 2-Trifluormethoxy-3-methyl-Phenyl | Methyl | |
| 4.148 | 2-Nitro-3-methyl-Phenyl | Methyl | |
| 4.149 | 2-Fluor-3-ethyl-Phenyl | Methyl | |
| 4.150 | 2-Chlor-3-ethyl-Phenyl | Methyl | |
| 4.151 | 2-Brom-3-ethyl-Phenyl | Methyl | |
| 4.152 | 2-Methyl-3-ethyl-Phenyl | Methyl | |
| 4.153 | 2,3-Diethyl-Phenyl | Methyl | |
| 4.154 | 2-Cyclopropyl-3-ethyl-Phenyl | Methyl | |
| 4.155 | 2-Cyano-3-ethyl-Phenyl | Methyl | |
| 4.156 | 2-Trifluormethyl-3-ethyl-Phenyl | Methyl | |
| 4.157 | 2-Methoxy-3-ethyl-Phenyl | Methyl | |
| 4.158 | 2-Trifluormethoxy-3-ethyl-Phenyl | Methyl | |
| 4.159 | 2-Nitro-3-ethyl-Phenyl | Methyl | |
| 4.160 | 2-Fluor-3-propyl-Phenyl | Methyl | |
| 4.161 | 2-Chlor-3-propyl-Phenyl | Methyl | |
| 4.162 | 2-Brom-3-propyl-Phenyl | Methyl | |
| 4.163 | 2-Methyl-3-propyl-Phenyl | Methyl | |
| 4.164 | 2-Methyl-3-propyl-Phenyl | Methyl | |
| 4.165 | 2-Cyclopropyl-3-propyl-Phenyl | Methyl | |
| 4.166 | 2-Cyano-3-propyl-Phenyl | Methyl | |
| 4.167 | 2-Trifluormethyl-3-propyl-Phenyl | Methyl | |
| 4.168 | 2-Methoxy-3-propyl-Phenyl | Methyl | |
| 4.169 | 2-Trifluormethoxy-3-propyl-Phenyl | Methyl | |
| 4.170 | 2-Nitro-3-propyl-Phenyl | Methyl | |
| 4.171 | 2-Fluor-3-isopropyl-Phenyl | Methyl | |
| 4.172 | 2-Chlor-3-isopropyl-Phenyl | Methyl | |
| 4.173 | 2-Brom-3-isopropyl-Phenyl | Methyl | |
| 4.174 | 2-Methyl-3-isopropyl-Phenyl | Methyl | |
| 4.175 | 2-Cyclopropyl-3-isopropyl-Phenyl | Methyl | |
| 4.176 | 2-Cyano-3-isopropyl-Phenyl | Methyl | |
| 4.177 | 2-Trifluormethyl-3-isopropyl-Phenyl | Methyl | |
| 4.178 | 2-Methoxy-3-isopropyl-Phenyl | Methyl | |
| 4.179 | 2-Trifluormethoxy-3-isopropyl-Phenyl | Methyl | |
| 4.180 | 2-Nitro-3-isopropyl-Phenyl | Methyl | |
| 4.181 | 2-Fluor-3-tert.butyl-Phenyl | Methyl | |
| 4.182 | 2-Fluor-3-tert.butyl-Phenyl | Ethyl | |
| 4.183 | 2-Fluor-3-tert.butyl-Phenyl | CN | |
| 4.184 | 2-Fluor-3-tert.butyl-Phenyl | Fluormethyl | |
| 4.185 | 2-Fluor-3-tert.butyl-Phenyl | Methylcarbonyl | |
| 4.186 | 2-Fluor-3-tert.butyl-Phenyl | Hydroxymethyl | |
| 4.187 | 2-Fluor-3-tert.butyl-Phenyl | Ethinyl | |
| 4.188 | 2-Chlor-3-tert.butyl-Phenyl | Methyl | |
| 4.189 | 2-Brom-3-tert.butyl-Phenyl | Methyl | |
| 4.190 | 2-Methyl-3-tert.butyl-Phenyl | Methyl | |
| 4.191 | 2-Cyclopropyl-3-tert.butyl-Phenyl | Methyl | |
| 4.192 | 2-Cyano-3-tert.butyl-Phenyl | Methyl | |
| 4.193 | 2-Trifluormethyl-3-tert.butyl-Phenyl | Methyl | |
| 4.194 | 2-Methoxy-3-tert.butyl-Phenyl | Methyl | |
| 4.195 | 2-Trifluormethoxy-3-tert.butyl-Phenyl | Methyl | |
| 4.196 | 2-Nitro-3-tert.butyl-Phenyl | Methyl | |
| 4.197 | 2-Fluor-3-cyclopropyl-Phenyl | Methyl | |
| 4.198 | 2-Chlor-3-cyclopropyl-Phenyl | Methyl | |
| 4.199 | 2-Brom-3-cyclopropyl-Phenyl | Methyl | |
| 4.200 | 2-Methyl-3-cyclopropyl-Phenyl | Methyl | |
| 4.201 | 2-Cyclopropyl-3-cyclopropyl-Phenyl | Methyl | |
| 4.202 | 2-Cyano-3-cyclopropyl-Phenyl | Methyl | |
| 4.203 | 2-Trifluormethyl-3-cyclopropyl-Phenyl | Methyl | |
| 4.204 | 2-Methoxy-3-cyclopropyl-Phenyl | Methyl | |
| 4.205 | 2-Ethoxy-3-cyclopropyl-Phenyl | Methyl | |
| 4.206 | 2-Trifluormethoxy-3-cyclopropyl-Phenyl | Methyl | |
| 4.207 | 2-Fluor-3-methoxycarbonyl-Phenyl | Methyl | |
| 4.208 | 2-Chlor-3-methoxycarbonyl-Phenyl | Methyl | |
| 4.209 | 2-Brom-3-methoxycarbonyl-Phenyl | Methyl | |
| 4.210 | 2-Methyl-3-methoxycarbonyl-Phenyl | Methyl | |
| 4.211 | 2-Methyl-3-methoxycarbonyl-Phenyl | Ethyl | |
| 4.212 | 2-Methyl-3-methoxycarbonyl-Phenyl | CN | |
| 4.213 | 2-Methyl-3-methoxycarbonyl-Phenyl | Vinyl | |
| 4.214 | 2-Methyl-3-methoxycarbonyl-Phenyl | Fluormethyl | |
| 4.215 | 2-Methyl-3-methoxycarbonyl-Phenyl | Methylcarbonyl | |
| 4.216 | 2-Methyl-3-methoxycarbonyl-Phenyl | Hydroxymethyl | |
| 4.217 | 2-Methyl-3-methoxycarbonyl-Phenyl | Ethinyl | |
| 4.218 | 2-Cyclopropyl-3-methoxycarbonyl-Phenyl | Methyl | |
| 4.219 | 2-Cyano-3-methoxycarbonyl-Phenyl | Methyl | |
| 4.220 | 2-Trifluormethyl-3-methoxycarbonyl-Phenyl | Methyl | |
| 4.221 | 2-Methoxy-3-methoxycarbonyl -Phenyl | Methyl | |
| 4.222 | 2-Ethoxy-3-methoxycarbonyl-Phenyl | Methyl | |
| 4.223 | 2-Trifluormethoxy-3-methoxycarbonyl-Phenyl | Methyl | |
| 4.224 | 2-Nitro-3-methoxycarbonyl-Phenyl | Methyl | |
| 4.225 | 2-Fluor-3-cyano-Phenyl | Methyl | |
| 4.226 | 2-Fluor-3-cyano-Phenyl | Ethyl | |
| 4.227 | 2-Fluor-3-cyano-Phenyl | CN | |
| 4.228 | 2-Fluor-3-cyano-Phenyl | Vinyl | |
| 4.229 | 2-Fluor-3-cyano-Phenyl | Fluormethyl | |
| 4.230 | 2-Fluor-3-cyano-Phenyl | Methylcarbonyl | |
| 4.231 | 2-Fluor-3-cyano-Phenyl | Hydroxymethyl | |
| 4.232 | 2-Fluor-3-cyano-Phenyl | Ethinyl | |
| 4.233 | 2-Chlor-3-cyano-Phenyl | Methyl | |
| 4.234 | 2-Chlor-3-cyano-Phenyl | Ethyl | |
| 4.235 | 2-Chlor-3-cyano-Phenyl | CN | |
| 4.236 | 2-Chlor-3-cyano-Phenyl | Vinyl | |
| 4.237 | 2-Chlor-3-cyano-Phenyl | Fluormethyl | |
| 4.238 | 2-Chlor-3-cyano-Phenyl | Methylcarbonyl | |
| 4.239 | 2-Chlor-3-cyano-Phenyl | Hydroxymethyl | |
| 4.240 | 2-Chlor-3-cyano-Phenyl | Ethinyl | |
| 4.241 | 2-Brom-3-cyano-Phenyl | Methyl | |
| 4.242 | 2-Methyl-3-cyano-Phenyl | Methyl | |
| 4.243 | 2-Ethyl-3-cyano-Phenyl | Methyl | |
| 4.244 | 2-Cyclopropyl-3-cyano-Phenyl | Methyl | |
| 4.245 | 2-Cyano-3-cyano-Phenyl | Methyl | |
| 4.246 | 2-Trifluormethyl-3-cyano-Phenyl | Methyl | |
| 4.247 | 2-Methoxy-3-cyano-Phenyl | Methyl | |
| 4.248 | 2-Trifluormethoxy-3-cyano-Phenyl | Methyl | |
| 4.249 | 2-Nitro-3-cyano-Phenyl | Methyl | |
| 4.250 | 2-Fluor-3-methoxy-Phenyl | Methyl | |
| 4.251 | 2-Chlor-3-methoxy-Phenyl | Methyl | |
| 4.252 | 2-Brom-3-methoxy-Phenyl | Methyl | |
| 4.253 | 2-Methyl-3-methoxy-Phenyl | Methyl | |
| 4.254 | 2-Cyclopropyl-3-methoxy-Phenyl | Methyl | |
| 4.255 | 2-Cyano-3-methoxy-Phenyl | Methyl | |
| 4.256 | 2-Trifluormethyl-3-methoxy-Phenyl | Methyl | |
| 4.257 | 2,3-Dimethoxy-Phenyl | Methyl | |
| 4.258 | 2-Ethoxy-3-methoxy-Phenyl | Methyl | |
| 4.259 | 2-Trifluormethoxy-3-methoxy-Phenyl | Methyl | |
| 4.260 | 2-Nitro-3-methoxy-Phenyl | Methyl | |
| 4.261 | 2-Fluor-3-ethoxy-Phenyl | Methyl | |
| 4.262 | 2-Chlor-3-ethoxy-Phenyl | Methyl | |
| 4.263 | 2-Brom-3-ethoxy-Phenyl | Methyl | |
| 4.264 | 2-Methyl-3-ethoxy-Phenyl | Methyl | |
| 4.265 | 2-Cyclopropyl-3-ethoxy-Phenyl | Methyl | |
| 4.266 | 2-Cyano-3-ethoxy-Phenyl | Methyl | |
| 4.267 | 2-Trifluormethyl-3-ethoxy-Phenyl | Methyl | |
| 4.268 | 2-Methoxy-3-ethoxy-Phenyl | Methyl | |
| 4.269 | 2,3-Diethoxy-Phenyl | Methyl | |
| 4.270 | 2-Trifluormethoxy-3-ethoxy-Phenyl | Methyl | |
| 4.271 | 2-Nitro-3-ethoxy-Phenyl | Methyl | |
| 4.272 | 2-Fluor-3-isopropoxy-Phenyl | Methyl | |
| 4.273 | 2-Chlor-3-isopropoxy-Phenyl | Methyl | |
| 4.274 | 2-Brom-3-isopropoxy-Phenyl | Methyl | |
| 4.275 | 2-Methyl-3-isopropoxy-Phenyl | Methyl | |
| 4.276 | 2-Cyclopropyl-3-isopropoxy-Phenyl | Methyl | |
| 4.277 | 2-Cyano-3-isopropoxy-Phenyl | Methyl | |
| 4.278 | 2-Trifluormethyl-3-isopropoxy-Phenyl | Methyl | |
| 4.279 | 2-Methoxy-3-isopropoxy-Phenyl | Methyl | |
| 4.280 | 2-Trifluormethoxy-3-isopropoxy-Phenyl | Methyl | |
| 4.281 | 2-Nitro-3-isopropoxy-Phenyl | Methyl | |
| 4.282 | 2-Fluor-3-trifluormethoxy-Phenyl | Methyl | |
| 4.283 | 2-Chlor-3-trifluormethoxy-Phenyl | Methyl | |
| 4.284 | 2-Brom-3-trifluormethoxy-Phenyl | Methyl | |
| 4.285 | 2-Methyl-3-trifluormethoxy-Phenyl | Methyl | |
| 4.286 | 2-Cyclopropyl-3-trifluormethoxy-Phenyl | Methyl | |
| 4.287 | 2-Cyano-3-trifluormethoxy-Phenyl | Methyl | |
| 4.288 | 2-Trifluormethyl-3-trifluormethoxy-Phenyl | Methyl | |
| 4.289 | 2-Methoxy-3-trifluormethoxy-Phenyl | Methyl | |
| 4.290 | 2,3-Bis(trifluormethoxy)-Phenyl | Methyl | |
| 4.291 | 2-Nitro-3-trifluormethoxy-Phenyl | Methyl | |
| 4.292 | 2-Fluor-3-difluormethoxy-Phenyl | Methyl | |
| 4.293 | 2-Chlor-3-difluormethoxy-Phenyl | Methyl | |
| 4.294 | 2-Brom-3-difluormethoxy-Phenyl | Methyl | |
| 4.295 | 2-Methyl-3-difluormethoxy-Phenyl | Methyl | |
| 4.296 | 2-Cyclopropyl-3-difluormethoxy-Phenyl | Methyl | |
| 4.297 | 2-Cyano-3-difluormethoxy-Phenyl | Methyl | |
| 4.298 | 2-Trifluormethyl-3-difluormethoxy-Phenyl | Methyl | |
| 4.299 | 2-Methoxy-3-difluormethoxy-Phenyl | Methyl | |
| 4.300 | 2-Ethoxy-3-difluormethoxy-Phenyl | Methyl | |
| 4.301 | 2-Nitro-3-difluormethoxy-Phenyl | Methyl | |
| 4.302 | 2-Fluor-3-nitro-Phenyl | Methyl | |
| 4.303 | 2-Chlor-3-nitro-Phenyl | Methyl | |
| 4.304 | 2-Brom-3-nitro-Phenyl | Methyl | |
| 4.305 | 2-Methyl-3-nitro-Phenyl | Methyl | |
| 4.306 | 2-Cyclopropyl-3-nitro-Phenyl | Methyl | |
| 4.307 | 2-Cyano-3-nitro-Phenyl | Methyl | |
| 4.308 | 2-Trifluormethyl-3-nitro-Phenyl | Methyl | |
| 4.309 | 2-Methoxy-3-nitro-Phenyl | Methyl | |
| 4.310 | 2-Ethoxy-3-nitro-Phenyl | Methyl | |
| 4.311 | 2-Trifluormethoxy-3-nitro-Phenyl | Methyl | |
| 4.312 | 2-Fluor-3-methylsulfanylPhenyl | Methyl | |
| 4.313 | 2-Chlor-3-methylsulfanyl-Phenyl | Methyl | |
| 4.314 | 2-Brom-3-methylsulfanyl-Phenyl | Methyl | |
| 4.315 | 2-Methyl-3-methylsulfanyl-Phenyl | Methyl | |
| 4.316 | 2-Ethyl-3-methylsulfanyl-Phenyl | Methyl | |
| 4.317 | 2-Cyclopropyl-3-methylsulfanyl-Phenyl | Methyl | |
| 4.318 | 2-Cyano-3-methylsulfanyl-Phenyl | Methyl | |
| 4.319 | 2-Trifluormethyl-3-methylsulfanyl-Phenyl | Methyl | |
| 4.320 | 2-Methoxy-3-methylsulfanyl-Phenyl | Methyl | |
| 4.321 | 2-Ethoxy-3-methylsulfanyl-Phenyl | Methyl | |
| 4.322 | 2-Trifluormethoxy-3-methylsulfanyl-Phenyl | Methyl | |
| 4.323 | 2-Nitro-3-methylsulfanyl-Phenyl | Methyl | |
| 4.324 | 3,5-Difluor-Phenyl | Methyl | [CDCl₃] 1.61 (s, 3H); 3.05 (d, 1H); 3.68 (d, 1H); 6.88 (m, 1H); 7.15 (m, 2H); 9.65 (s, 1H). |
| 4.325 | 3,5-Difluor-Phenyl | Ethyl | |
| 4.326 | 3,5-Difluor-Phenyl | CN | |
| 4.327 | 3,5-Difluor-Phenyl | Vinyl | |
| 4.328 | 3,5-Difluor-Phenyl | Fluormethyl | |
| 4.329 | 3,5-Difluor-Phenyl | Methylcarbonyl | |
| 4.330 | 3,5-Difluor-Phenyl | Hydroxymethyl | |
| 4.331 | 3,5-Difluor-Phenyl | Ethinyl | |
| 4.332 | 3-Chlor-5-fluor-Phenyl | Methyl | |
| 4.333 | 3-Chlor-5-fluor-Phenyl | Ethyl | |
| 4.334 | 3-Chlor-5-fluor-Phenyl | CN | |
| 4.335 | 3-Chlor-5-fluor-Phenyl | Vinyl | |
| 4.336 | 3-Chlor-5-fluor-Phenyl | Fluormethyl | |
| 4.337 | 3-Chlor-5-fluor-Phenyl | Methylcarbonyl | |
| 4.338 | 3-Chlor-5-fluor-Phenyl | Hydroxymethyl | |
| 4.339 | 3-Chlor-5-fluor-Phenyl | Ethinyl | |
| 4.340 | 3-Brom-5-fluor-Phenyl | Methyl | |
| 4.341 | 3-Iod-5-fluor-Phenyl | Methyl | |
| 4.342 | 3-Methyl-5-fluor-Phenyl | Methyl | |
| 4.343 | 3-Methyl-5-fluor-Phenyl | Ethyl | |
| 4.344 | 3-Methyl-5-fluor-Phenyl | CN | |
| 4.345 | 3-Methyl-5-fluor-Phenyl | Vinyl | |
| 4.346 | 3-Methyl-5-fluor-Phenyl | Fluormethyl | |
| 4.347 | 3-Methyl-5-fluor-Phenyl | Methylcarbonyl | |
| 4.348 | 3-Methyl-5-fluor-Phenyl | Hydroxymethyl | |
| 4.349 | 3-Methyl-5-fluor-Phenyl | Ethinyl | |
| 4.350 | 3-Ehylt-5-fluor-Phenyl | Methyl | |
| 4.351 | 3-Propyl-5-fluor-Phenyl | Methyl | |
| 4.352 | 3-iPropyl-5-fluor-Phenyl | Methyl | |
| 4.353 | 3-nButyl-5-fluor-Phenyl | Methyl | |
| 4.354 | 3-isoButyl-5-fluor-Phenyl | Methyl | |
| 4.355 | 3-tert.Butyl-5-fluor-Phenyl | Methyl | |
| 4.356 | 3-tert.Butyl-5-fluor-Phenyl | Ethyl | |
| 4.357 | 3-tert.Butyl-5-fluor-Phenyl | CN | |
| 4.358 | 3-tert.Butyl-5-fluor-Phenyl | Vinyl | |
| 4.359 | 3-tert.Butyl-5-fluor-Phenyl | Fluormethyl | |
| 4.360 | 3-tert.Butyl-5-fluor-Phenyl | Methylcarbonyl | |
| 4.361 | 3-tert.Butyl-5-fluor-Phenyl | Hyd roxymethyl | |
| 4.362 | 3-tert.Butyl-5-fluor-Phenyl | Ethinyl | |
| 4.363 | 3-Cyclopropyl-5-fluor-Phenyl | Methyl | |
| 4.364 | 3-Cyano-5-fluor-Phenyl | Methyl | |
| 4.365 | 3-Cyano-5-fluor-Phenyl | Ethyl | |
| 4.366 | 3-Cyano-5-fluor-Phenyl | CN | |
| 4.367 | 3-Cyano-5-fluor-Phenyl | Vinyl | |
| 4.368 | 3-Cyano-5-fluor-Phenyl | Fluormethyl | |
| 4.369 | 3-Cyano-5-fluor-Phenyl | Methylcarbonyl | |
| 4.370 | 3-Cyano-5-fluor-Phenyl | Hydroxymethyl | |
| 4.371 | 3-Cyano-5-fluor-Phenyl | Ethinyl | |
| 4.372 | 3-Trifluormethyl-5-fluor-Phenyl | Methyl | |
| 4.373 | 3-(Methoxycarbony)l-5-fluor-Phenyl | Methyl | |
| 4.374 | 3-Methoxy-5-fluor-Phenyl | Methyl | |
| 4.375 | 3-Methoxy-5-fluor-Phenyl | Ethyl | |
| 4.376 | 3-Methoxy-5-fluor-Phenyl | CN | |
| 4.377 | 3-Methoxy-5-fluor-Phenyl | Vinyl | |
| 4.378 | 3-Methoxy-5-fluor-Phenyl | Fluormethyl | |
| 4.379 | 3-Methoxy-5-fluor-Phenyl | Methylcarbonyl | |
| 4.380 | 3-Methoxy-5-fluor-Phenyl | Hydroxymethyl | |
| 4.381 | 3-Methoxy-5-fluor-Phenyl | Ethinyl | |
| 4.382 | 3-Ethoxy-5-fluor-Phenyl | Methyl | |
| 4.383 | 3-nPropyoxy-5-fluor-Phenyl | Methyl | |
| 4.384 | 3-isoPropoxy-5-fluor-Phenyl | Methyl | |
| 4.385 | 3-nButoxy-5-fluor-Phenyl | Methyl | |
| 4.386 | 3-isoButoxy-5-fluor-Phenyl | Methyl | |
| 4.387 | 3-Difluormethoxy-5-fluor-Phenyl | Methyl | |
| 4.388 | 3-Trifluormethoxy-5-fluor-Phenyl | Methyl | |
| 4.389 | 3-Nitro-5-fluor-Phenyl | Methyl | |
| 4.390 | 3-Acetoxy-5-fluor-Phenyl | Methyl | |
| 4.391 | 3-Methylsulfanyl-5-fluor-Phenyl | Methyl | |
| 4.392 | 3,5-Dichlor-Phenyl | Methyl | |
| 4.393 | 3,5-Dichlor-Phenyl | Ethyl | |
| 4.394 | 3,5-Dichlor-Phenyl | CN | |
| 4.395 | 3,5-Dichlor-Phenyl | Vinyl | |
| 4.396 | 3,5-Dichlor-Phenyl | Fluormethyl | |
| 4.397 | 3,5-Dichlor-Phenyl | Methylcarbonyl | |
| 4.398 | 3,5-Dichlor-Phenyl | Hydroxymethyl | |
| 4.399 | 3,5-Dichlor-Phenyl | Ethinyl | |
| 4.400 | 3-Brom-5-chlor-Phenyl | Methyl | |
| 4.401 | 3-lod-5-chlor-Phenyl | Methyl | |
| 4.402 | 3-Methyl-5-chlor-Phenyl | Methyl | |
| 4.403 | 3-Methyl-5-chlor-Phenyl | Ethyl | |
| 4.404 | 3-Methyl-5-chlor-Phenyl | CN | |
| 4.405 | 3-Methyl-5-chlor-Phenyl | Vinyl | |
| 4.406 | 3-Methyl-5-chlor-Phenyl | Fluormethyl | |
| 4.407 | -Methyl-5-chlor-Phenyl | Methylcarbonyl | |
| 4.408 | 3-Methyl-5-chlor-Phenyl | Hydroxymethyl | |
| 4.409 | 3-Methyl-5-chlor-Phenyl | Ethinyl | |
| 4.410 | 3-Ethyl-5-chlor-Phenyl | Methyl | |
| 4.411 | 3-Propyl-5-chlor-Phenyl | Methyl | |
| 4.412 | 3-isoPropyl-5-chlor-Phenyl | Methyl | |
| 4.413 | 3-isoButyl-5-chlor-Phenyl | Methyl | |
| 4.414 | 3-tert.Butyl-5-chlor-Phenyl | Methyl | |
| 4.415 | 3-Cyclopropyl-5-chlor-Phenyl | Methyl | |
| 4.416 | 3-Cyano-5-chlor-Phenyl | Methyl | |
| 4.417 | 3-Cyano-5-chlor-Phenyl | Ethyl | |
| 4.418 | 3-Cyano-5-chlor-Phenyl | CN | |
| 4.419 | 3-Cyano-5-chlor-Phenyl | Vinyl | |
| 4.420 | 3-Cyano-5-chlor-Phenyl | Fluormethyl | |
| 4.421 | 3-Cyano-5-chlor-Phenyl | Methylcarbonyl | |
| 4.422 | 3-Cyano-5-chlor-Phenyl | Hydroxymethyl | |
| 4.423 | 3-Cyano-5-chlor-Phenyl | Ethinyl | |
| 4.424 | 3-Trifluormethyl-5-chlor-Phenyl | Methyl | |
| 4.425 | 3-Trifluormethyl-5-chlor-Phenyl | Ethyl | |
| 4.426 | 3-Trifluormethyl-5-chlor-Phenyl | CN | |
| 4.427 | 3-Trifluormethyl-5-chlor-Phenyl | Vinyl | |
| 4.428 | 3-Trifluormethyl-5-chlor-Phenyl | Fluormethyl | |
| 4.429 | 3-Trifluormethyl-5-chlor-Phenyl | Methylcarbonyl | |
| 4.430 | 3-Trifluormethyl-5-chlor-Phenyl | Hydroxymethyl | |
| 4.431 | 3-Trifluormethyl-5-chlor-Phenyl | Ethinyl | |
| 4.432 | 3-(Methoxycarbony)l-5-chlor-Phenyl | Methyl | |
| 4.433 | 3-Methoxy-5-chlor-Phenyl | Methyl | |
| 4.434 | 3-Methoxy-5-chlor-Phenyl | Ethyl | |
| 4.435 | 3-Methoxy-5-chlor-Phenyl | CN | |
| 4.436 | 3-Methoxy-5-chlor-Phenyl | Vinyl | |
| 4.437 | 3-Methoxy-5-chlor-Phenyl | Fluormethyl | |
| 4.438 | 3-Methoxy-5-chlor-Phenyl | Methylcarbonyl | |
| 4.439 | 3-Methoxy-5-chlor-Phenyl | Hydroxymethyl | |
| 4.440 | 3-Methoxy-5-chlor-Phenyl | Ethinyl | |
| 4.441 | 3-Ethoxy-5-chlor-Phenyl | Methyl | |
| 4.442 | 3-nPropyoxy-5-chlor-Phenyl | Methyl | |
| 4.443 | 3-isoPropoxy-5-chlor-Phenyl | Methyl | |
| 4.444 | 3-nButoxy-5-chlor-Phenyl | Methyl | |
| 4.445 | 3-isoButoxy-5-chlor-Phenyl | Methyl | |
| 4.446 | 3-Difluormethoxy-5-chlor-Phenyl | Methyl | |
| 4.447 | 3-Trifluormethoxy-5-chlor-Phenyl | Methyl | |
| 4.448 | 3-Trifluormethoxy-5-chlor-Phenyl | Ethyl | |
| 4.449 | 3-Trifluormethoxy-5-chlor-Phenyl | CN | |
| 4.450 | 3-Trifluormethoxy-5-chlor-Phenyl | Vinyl | |
| 4.451 | 3-Trifluormethoxy-5-chlor-Phenyl | Fluormethyl | |
| 4.452 | 3Trifluormethoxy-5-chlor-Phenyl | Methylcarbonyl | |
| 4.453 | 3-Trifluormethoxy-5-chlor-Phenyl | Hydroxymethyl | |
| 4.454 | 3-Trifluormethoxy-5-chlor-Phenyl | Ethinyl | |
| 4.455 | 3-Nitro-5-chlor-Phenyl | Methyl | |
| 4.456 | 3-Acetoxy-5-chlor-Phenyl | Methyl | |
| 4.457 | 3-Methylsulfanyl-5-chlor-Phenyl | Methyl | |
| 4.458 | 3,5-Dibrom-Phenyl-Phenyl | Methyl | |
| 4.459 | 3-Iod-5-brom-Phenyl | Methyl | |
| 4.460 | 3-Methyl-5-brom-Phenyl | Methyl | |
| 4.461 | 3-Ethyl-5-brom-Phenyl | Methyl | |
| 4.462 | 3-Propyl-5-brom-Phenyl | Methyl | |
| 4.463 | 3-isoPropyl-5-brom-Phenyl | Methyl | |
| 4.464 | 3-isoButyl-5-brom-Phenyl | Methyl | |
| 4.465 | 3-tert.Butyl-5-brom-Phenyl | Methyl | |
| 4.466 | 3-Cyclopropyl-5-brom-Phenyl | Methyl | |
| 4.467 | 3-Cyano-5-brom-Phenyl | Methyl | |
| 4.468 | 3-Trifluormethyl-5-brom-Phenyl | Methyl | |
| 4.469 | 3-(Methoxycarbony)l-5-brom-Phenyl | Methyl | |
| 4.470 | 3-Methoxy-5-brom-Phenyl | Methyl | |
| 4.471 | 3-Ethoxy-5-brom-Phenyl | Methyl | |
| 4.472 | 3-nPropyoxy-5-brom-Phenyl | Methyl | |
| 4.473 | 3-isoPropoxy-5-brom-Phenyl | Methyl | |
| 4.474 | 3-nButoxy-5-brom-Phenyl | Methyl | |
| 4.475 | 3-isoButoxy-5-brom-Phenyl | Methyl | |
| 4.476 | 3-tert.Butoxy-5-brom-Phenyl | Methyl | |
| 4.477 | 3-Difluormethoxy-5-brom-Phenyl | Methyl | |
| 4.478 | 3-Trifluormethoxy-5-brom-Phenyl | Methyl | |
| 4.479 | 3-Nitro-5-brom-Phenyl | Methyl | |
| 4.480 | 3-Acetoxy-5-brom-Phenyl | Methyl | |
| 4.481 | 3-Methylsulfanyl-5-brom-Phenyl | Methyl | |
| 4.482 | 3,5-Diiod-Phenyl | Methyl | |
| 4.483 | 3-Methyl-5-iod-Phenyl | Methyl | |
| 4.484 | 3-Ethyl-5-iod-Phenyl | Methyl | |
| 4.485 | 3-Propyl-5-iod-Phenyl | Methyl | |
| 4.486 | 3-isoPropyl-5-iod-Phenyl | Methyl | |
| 4.487 | 3-nButyl-5-iod-Phenyl | Methyl | |
| 4.488 | 3-isoButyl-5-iod-Phenyl | Methyl | |
| 4.489 | 3-tert.Butyl-5-iod-Phenyl | Methyl | |
| 4.490 | 3-Cyclopropyl-5-iod-Phenyl | Methyl | |
| 4.491 | 3-Cyano-5-iod-Phenyl | Methyl | |
| 4.492 | 3-Trifluormethyl-5-iod-Phenyl | Methyl | |
| 4.493 | 3-(Methoxycarbonyl)-5-iod-Phenyl | Methyl | |
| 4.494 | 3-Methoxy-5-iod-Phenyl | Methyl | |
| 4.495 | 3-Ethoxy-5-iod-Phenyl | Methyl | |
| 4.496 | 3-nPropyoxy-5-iod-Phenyl | Methyl | |
| 4.497 | 3-isoPropoxy-5-iod-Phenyl | Methyl | |
| 4.498 | 3-nButoxy-5-iod-Phenyl | Methyl | |
| 4.499 | 3-isoButoxy-5-iod-Phenyl | Methyl | |
| 4.500 | 3-Difluormethoxy-5-iod-Phenyl | Methyl | |
| 4.501 | 3-Trifluormethoxy-5-iod-Phenyl | Methyl | |
| 4.502 | 3-Nitro-5-iod-Phenyl | Methyl | |
| 4.503 | 3-Acetoxy-5-iod-Phenyl | Methyl | |
| 4.504 | 3-Methylsulfanyl-5-iod-Phenyl | Methyl | |
| 4.505 | 3,5-Dimethyl-Phenyl | Methyl | |
| 4.506 | 3-Ethyl-5-methyl-Phenyl | Methyl | |
| 4.507 | 3-Propyl-5-methyl-Phenyl | Methyl | |
| 4.508 | 3-isoPropyl-5-methyl-Phenyl | Methyl | |
| 4.509 | 3-nButyl-5-methyl-Phenyl | Methyl | |
| 4.510 | 3-isoButyl-5-methyl-Phenyl | Methyl | |
| 4.511 | 3-tert.Butyl-5-methyl-Phenyl | Methyl | |
| 4.512 | 3-Cyclopropyl-5-methyl-Phenyl | Methyl | |
| 4.513 | 3-Cyano-5-methyl-Phenyl | Methyl | |
| 4.514 | 3-Trifluormethyl-5-methyl-Phenyl | Methyl | |
| 4.515 | 3-(Methoxycarbony)l-5-methyl-Phenyl | Methyl | |
| 4.516 | 3-Methoxy-5-methyl-Phenyl | Methyl | |
| 4.517 | 3-Ethoxy-5-methyl-Phenyl | Methyl | |
| 4.518 | 3-nPropyoxy-5-methyl-Phenyl | Methyl | |
| 4.519 | 3-nButoxy-5-methyl-Phenyl | Methyl | |
| 4.520 | 3-isoButoxy-5-methyl-Phenyl | Methyl | |
| 4.521 | 3-Difluormethoxy-5-methyl-Phenyl | Methyl | |
| 4.522 | 3-Trifluormethoxy-5-methyl-Phenyl | Methyl | |
| 4.523 | 3-Nitro-5-methyl-Phenyl | Methyl | |
| 4.524 | 3-Acetoxy-5-methyl-Phenyl | Methyl | |
| 4.525 | 3-Methylsulfanyl-5-methyl-Phenyl | Methyl | |
| 4.526 | 3,5-Diethyl-Phenyl | Methyl | |
| 4.527 | 3-Propyl-5-ethyl-Phenyl | Methyl | |
| 4.528 | 3-isoPropyl-5-ethyl-Phenyl | Methyl | |
| 4.529 | 3-nButyl-5-ethyl-Phenyl | Methyl | |
| 4.530 | 3-isoButyl-5-ethyl-Phenyl | Methyl | |
| 4.531 | 3-tert.Butyl-5-ethyl-Phenyl | Methyl | |
| 4.532 | 3-Cyclopropyl-5-ethyl-Phenyl | Methyl | |
| 4.533 | 3-Cyano-5-ethyl-Phenyl | Methyl | |
| 4.534 | 3-Trifluormethyl-5-ethyl-Phenyl | Methyl | |
| 4.535 | 3-(Methoxycarbony)l-5-ethyl-Phenyl | Methyl | |
| 4.536 | 3-Methoxy-5-ethyl-Phenyl | Methyl | |
| 4.537 | 3-Ethoxy-5-ethyl-Phenyl | Methyl | |
| 4.538 | 3-nPropyoxy-5-ethyl-Phenyl | Methyl | |
| 4.539 | 3-nButoxy-5-ethyl-Phenyl | Methyl | |
| 4.540 | 3-isoButoxy-5-ethyl-Phenyl | Methyl | |
| 4.541 | 3-Difluormethoxy-5-ethyl-Phenyl | Methyl | |
| 4.542 | 3-Trifluormethoxy-5-ethyl-Phenyl | Methyl | |
| 4.543 | 3-Nitro-5-ethyl-Phenyl | Methyl | |
| 4.544 | 3-Acetoxy-5-ethyl-Phenyl | Methyl | |
| 4.545 | 3-Methylsulfanyl-5-ethyl-Phenyl | Methyl | |
| 4.546 | 3,5-Dipropyl-Phenyl | Methyl | |
| 4.547 | 3-isoPropyl-5-propyl-Phenyl | Methyl | |
| 4.548 | 3-nButyl-5-propyl-Phenyl | Methyl | |
| 4.549 | 3-isoButyl-5-propyl-Phenyl | Methyl | |
| 4.550 | 3-tert.Butyl-5-propyl-Phenyl | Methyl | |
| 4.551 | 3-Cyclopropyl-5-propyl-Phenyl | Methyl | |
| 4.552 | 3-Cyano-5-propyl-Phenyl | Methyl | |
| 4.553 | 3-Trifluormethyl-5-propyl-Phenyl | Methyl | |
| 4.554 | 3-(Methoxycarbonyl)-5-propyl-Phenyl | Methyl | |
| 4.555 | 3-Methoxy-5-propyl-Phenyl | Methyl | |
| 4.556 | 3-Ethoxy-5-propyl-Phenyl | Methyl | |
| 4.557 | 3-nPropyoxy-5-propyl-Phenyl | Methyl | |
| 4.558 | 3-nButoxy-5-propyl-Phenyl | Methyl | |
| 4.559 | 3-isoButoxy-5-propyl-Phenyl | Methyl | |
| 4.560 | 3-tert.Butoxy-5-propyl-Phenyl | Methyl | |
| 4.561 | 3-Difluormethoxy-5-propyl-Phenyl | Methyl | |
| 4.562 | 3-Trifluormethoxy-5-ethyl-Phenyl | Methyl | |
| 4.563 | 3-Nitro-5-propyl-Phenyl | Methyl | |
| 4.564 | 3-Acetoxy-5-propyl-Phenyl | Methyl | |
| 4.565 | 3-Methylsulfanyl-5-propyl-Phenyl | Methyl | |
| 4.566 | 3,5-Diisopropyl-Phenyl | Methyl | |
| 4.567 | 3-nButyl-5-isopropyl-Phenyl | Methyl | |
| 4.568 | 3-isoButyl-5-isopropyl-Phenyl | Methyl | |
| 4.569 | 3-tert.Butyl-5-isopropyl-Phenyl | Methyl | |
| 4.570 | 3-Cyclopropyl-5-isopropyl-Phenyl | Methyl | |
| 4.571 | 3-Cyano-5-isopropyl-Phenyl | Methyl | |
| 4.572 | 3-Trifluormethyl-5-isopropyl-Phenyl | Methyl | |
| 4.573 | 3-(Methoxycarbony)l-5-isopropyl-Phenyl | Methyl | |
| 4.574 | 3-Methoxy-5-isopropyl-Phenyl | Methyl | |
| 4.575 | 3-Ethoxy-5-isopropyl-Phenyl | Methyl | |
| 4.576 | 3-nPropyoxy-5-isopropyl-Phenyl | Methyl | |
| 4.577 | 3-nButoxy-5-isopropyl-Phenyl | Methyl | |
| 4.578 | 3-isoButoxy-5-isopropyl-Phenyl | Methyl | |
| 4.579 | 3-tert.Butoxy-5-isopropyl-Phenyl | Methyl | |
| 4.580 | 3-Difluormethoxy-5-isopropyl-Phenyl | Methyl | |
| 4.581 | 3-Trifluormethoxy-5-isopropyl-Phenyl | Methyl | |
| 4.582 | 3-Nitro-5-isopropyl-Phenyl | Methyl | |
| 4.583 | 3-Acetoxy-5-isopropyl-Phenyl | Methyl | |
| 4.584 | 3-Methylsulfanyl-5-isopropyl-Phenyl | Methyl | |
| 4.585 | 3,5-Diisobutyl-Phenyl | Methyl | |
| 4.586 | 3-tert.Butyl-5-isobutyl-Phenyl | Methyl | |
| 4.587 | 3-Cyclopropyl-5-isobutyl-Phenyl | Methyl | |
| 4.588 | 3-Cyano-5-isobutyl-Phenyl | Methyl | |
| 4.589 | 3-Trifluormethyl-5-isobutyl-Phenyl | Methyl | |
| 4.590 | 3-(Methoxycarbonyl)-5-isobutyl-Phenyl | Methyl | |
| 4.591 | 3-Methoxy-5-isobutyl-Phenyl | Methyl | |
| 4.592 | 3-Ethoxy-5-isobutyl-Phenyl | Methyl | |
| 4.593 | 3-nPropyoxy-5-isobutyl-Phenyl | Methyl | |
| 4.594 | 3-nButoxy-5-isobutyl-Phenyl | Methyl | |
| 4.595 | 3-isoButoxy-5-isobutyl-Phenyl | Methyl | |
| 4.596 | 3-tert.Butoxy-5-isobutyl-Phenyl | Methyl | |
| 4.597 | 3-Difluormethoxy-5-isobutyl-Phenyl | Methyl | |
| 4.598 | 3-Trifluormethoxy-5-isobutyl-Phenyl | Methyl | |
| 4.599 | 3-Nitro-5-isobutyl-Phenyl | Methyl | |
| 4.600 | 3-Acetoxy-5-isobutyl-Phenyl | Methyl | |
| 4.601 | 3-Methylsulfanyl-5-isobutyl-Phenyl | Methyl | |
| 4.602 | 3-tert.Butyl-5-cyclopropyl-Phenyl | Methyl | |
| 4.603 | 3,5-Dicyclopropyl-Phenyl | Methyl | |
| 4.604 | 3-Cyano-5-cyclopropyl-Phenyl | Methyl | |
| 4.605 | 3-Cyano-5-cyclopropyl-Phenyl | Ethyl | |
| 4.606 | 3-Cyano-5-cyclopropyl-Phenyl | CN | |
| 4.607 | 3-Cyano-5-cyclopropyl-Phenyl | Vinyl | |
| 4.608 | 3-Cyano-5-cyclopropyl-Phenyl | Fluormethyl | |
| 4.609 | 3-Cyano-5-cyclopropyl-Phenyl | Methylcarbonyl | |
| 4.610 | 3-Cyano-5-cyclopropyl-Phenyl | Hydroxymethyl | |
| 4.611 | 3-Cyano-5-cyclopropyl-Phenyl | Ethinyl | |
| 4.612 | 3-Trifluormethyl-5-cyclopropyl-Phenyl | Methyl | |
| 4.613 | 3-(Methoxycarbonyl)-5-cyclopropyl-Phenyl | Methyl | |
| 4.614 | 3-Methoxy-5-cyclopropyl-Phenyl | Methyl | |
| 4.615 | 3-Ethoxy-5-cyclopropyl-Phenyl | Methyl | |
| 4.616 | 3-nPropyoxy-5-cyclopropyl-Phenyl | Methyl | |
| 4.617 | 3-nButoxy-5-cyclopropyl-Phenyl | Methyl | |
| 4.618 | 3-isoButoxy-5-cyclopropyl-Phenyl | Methyl | |
| 4.619 | 3-Difluormethoxy-5-cyclopropyl-Phenyl | Methyl | |
| 4.620 | 3-Trifluormethoxy-5-cyclopropyl-Phenyl | Methyl | |
| 4.621 | 3-Nitro-5-cyclopropyl-Phenyl | Methyl | |
| 4.622 | 3-Acetoxy-5-cyclopropyl-Phenyl | Methyl | |
| 4.623 | 3-Methylsulfanyl-5-cyclopropyl-Phenyl | Methyl | |
| 4.624 | 3,5-Dicyano-Phenyl | Methyl | |
| 4.625 | 3-Trifluormethyl-5-cyano-Phenyl | Methyl | |
| 4.626 | 3-(Methoxycarbonyl)-5-cyano-Phenyl | Methyl | |
| 4.627 | 3-Methoxy-5-cyano-Phenyl | Methyl | |
| 4.628 | 3-Ethoxy-5-cyano-Phenyl | Methyl | |
| 4.629 | 3-nPropyoxy-5-cyano-Phenyl | Methyl | |
| 4.630 | 3-nButoxy-5-cyano-Phenyl | Methyl | |
| 4.631 | 3-isoButoxy-5-cyano-Phenyl | Methyl | |
| 4.632 | 3-Difluormethoxy-5-cyano-Phenyl | Methyl | |
| 4.633 | 3-Trifluormethoxy-5-cyano-Phenyl | Methyl | |
| 4.634 | 3-Nitro-5-cyano-Phenyl | Methyl | |
| 4.635 | 3-Acetoxy-5-cyano-Phenyl | Methyl | |
| 4.636 | 3-Methylsulfanyl-5-cyano-Phenyl | Methyl | |
| 4.637 | 3,5-Di(trifluormethyl)-Phenyl | Methyl | |
| 4.638 | 3,5-Di(trifluormethyl)-Phenyl | Ethyl | |
| 4.639 | 3,5-Di(trifluormethyl)-Phenyl | CN | |
| 4.640 | 3,5-Di(trifluormethyl)-Phenyl | Vinyl | |
| 4.641 | 3,5-Di(trifluormethyl)-Phenyl | Fluormethyl | |
| 4.642 | 3,5-Di(trifluormethyl)-Phenyl | Methylcarbonyl | |
| 4.643 | 3,5-Di(trifluormethyl)-Phenyl | Hydroxymethyl | |
| 4.644 | 3,5-Di(trifluormethyl)-Phenyl | Ethinyl | |
| 4.645 | 3-(Methoxycarbonyl)-5-trifluormethylPhenyl | Methyl | |
| 4.646 | 3-Methoxy-5-trifluormethyl-Phenyl | Methyl | |
| 4.647 | 3-Ethoxy-5trifluormethyl-Phenyl | Methyl | |
| 4.648 | 3-nPropyoxy-5-trifluormethyl-Phenyl | Methyl | |
| 4.649 | 3-nButoxy-5-trifluormethyl-Phenyl | Methyl | |
| 4.650 | 3-isoButoxy-5-trifluormethyl-Phenyl | Methyl | |
| 4.651 | 3-Difluormethoxy-5-trifluormethyl-Phenyl | Methyl | |
| 4.652 | 3-Trifluormethoxy-5-trifluormethyl-Phenyl | Methyl | |
| 4.653 | 3-Nitro-5-trifluormethyl-Phenyl | Methyl | |
| 4.654 | 3-Acetoxy-5-trifluormethyl-Phenyl | Methyl | |
| 4.655 | 3-Methylsulfanyl-5-trifluormethyl-Phenyl | Methyl | |
| 4.656 | 3,5-Di(methoxycarbonyl)-Phenyl | Methyl | |
| 4.657 | 3-Methoxy-5-(methoxycarbonyl)-Phenyl | Methyl | |
| 4.658 | 3-Ethoxy-5-(methoxycarbonyl)-Phenyl | Methyl | |
| 4.659 | 3-nPropyoxy-5-(methoxycarbonyl)-Phenyl | Methyl | |
| 4.660 | 3-nButoxy-5-(methoxycarbonyl)-Phenyl | Methyl | |
| 4.661 | 3-isoButoxy-5-(methoxycarbonyl)-Phenyl | Methyl | |
| 4.662 | 3-Difluormethoxy-5-(methoxycarbonyl)-Phenyl | Methyl | |
| 4.663 | 3-Trifluormethoxy-5-(methoxycarbonyl)-Phenyl | Methyl | |
| 4.664 | 3-Nitro-5-(methoxycarbonyl)-Phenyl | Methyl | |
| 4.665 | 3-Acetoxy-5-(methoxycarbonyl)-Phenyl | Methyl | |
| 4.666 | 3-Methylsulfanyl-5-(methoxycarbonyl)-Phenyl | Methyl | |
| 4.667 | 3,5-Dimethoxy-Phenyl | Methyl | |
| 4.668 | 3,5-Dimethoxy-Phenyl | Ethyl | |
| 4.669 | 3,5-Dimethoxy-Phenyl | CN | |
| 4.670 | 3,5-Dimethoxy-Phenyl | Vinyl | |
| 4.671 | 3,5-Dimethoxy-Phenyl | Fluormethyl | |
| 4.672 | 3,5-Dimethoxy-Phenyl | Methylcarbonyl | |
| 4.673 | 3,5-Dimethoxy-Phenyl | Hydroxymethyl | |
| 4.674 | 3,5-Dimethoxy-Phenyl | Ethinyl | |
| 4.675 | 3-Ethoxy-5-methoxy-Phenyl | Methyl | |
| 4.676 | 3-nPropyoxy-5-methoxy-Phenyl | Methyl | |
| 4.677 | 3-nButoxy-5-methoxy-Phenyl | Methyl | |
| 4.678 | 3-isoButoxy-5-methoxy-Phenyl | Methyl | |
| 4.679 | 3-Difluormethoxy-5-methoxy-Phenyl | Methyl | |
| 4.680 | 3-Trifluormethoxy-5-methoxy-Phenyl | Methyl | |
| 4.681 | 3-Nitro-5-methoxy-Phenyl | Methyl | |
| 4.682 | 3-Acetoxy-5-methoxy-Phenyl | Methyl | |
| 4.683 | 3-Methylsulfanyl-5-methoxy-Phenyl | Methyl | |
| 4.684 | 3,5-Diethoxy-Phenyl | Methyl | |
| 4.685 | 3-nPropyoxy-5-ethoxy-Phenyl | Methyl | |
| 4.686 | 3-nButoxy-5-ethoxy-Phenyl | Methyl | |
| 4.687 | 3-isoButoxy-5-ethoxy-Phenyl | Methyl | |
| 4.688 | 3-tert.Butoxy-5-ethoxy-Phenyl | Methyl | |
| 4.689 | 3-Difluormethoxy-5-ethoxy-Phenyl | Methyl | |
| 4.690 | 3-Trifluormethoxy-5-ethoxy-Phenyl | Methyl | |
| 4.691 | 3-Nitro-5-ethoxy-Phenyl | Methyl | |
| 4.692 | 3-Acetoxy-5-ethoxy-Phenyl | Methyl | |
| 4.693 | 3-Methylsulfanyl-5-ethoxy-Phenyl | Methyl | |
| 4.694 | 3,5-Di(trifluormethoxy)-Phenyl | Methyl | |
| 4.695 | 3-Nitro-5-trifluormethoxy-Phenyl | Methyl | |
| 4.696 | 3-Methylsulfanyl-5-trifluormethoxy-Phenyl | Methyl | |
| 4.697 | 3,5-Bis(Difluormethoxy)-Phenyl | Methyl | |
| 4.698 | 3-Trifluormethoxy-5-difluormethoxy-Phenyl | Methyl | |
| 4.699 | 3-Nitro-5-difluormethoxy-Phenyl | Methyl | |
| 4.700 | 3-Acetoxy-5-difluormethoxy-Phenyl | Methyl | |
| 4.701 | 3-Methylsulfanyl-5-difluormethoxy-Phenyl | Methyl | |
| 4.702 | 3-Methylsulfanyl-5-acetoxy-Phenyl | Methyl | |
| 4.703 | 3-Acetoxy-5-nitro-Phenyl | Methyl | |
| 4.704 | 3-Methylsulfanyl-5-nitro-Phenyl | Methyl | |
| 4.705 | 3,5-Di(methylsulfanyl)-Phenyl | Methyl | |
| 4.706 | 3,4-Difluor-Phenyl | Methyl | |
| 4.707 | 3,4-Difluor-Phenyl | Ethyl | |
| 4.708 | 3,4-Difluor-Phenyl | CN | |
| 4.709 | 3,4-Difluor-Phenyl | Vinyl | |
| 4.710 | 3,4-Difluor-Phenyl | Fluormethyl | |
| 4.711 | 3,4-Difluor-Phenyl | Methylcarbonyl | |
| 4.712 | 3,4-Difluor-Phenyl | Hydroxymethyl | |
| 4.713 | 3,4-Difluor-Phenyl | Ethinyl | |
| 4.714 | 3-Chlor-4-fluor-Phenyl | Methyl | |
| 4.715 | 3-Brom-4-fluor-Phenyl | Methyl | |
| 4.716 | 3-Methyl-4-fluor-Phenyl | Methyl | |
| 4.717 | 3-Methyl-4-fluor-Phenyl | Ethyl | |
| 4.718 | 3-Methyl-4-fluor-Phenyl | CN | |
| 4.719 | 3-Methyl-4-fluor-Phenyl | Vinyl | |
| 4.720 | 3-Methyl-4-fluor-Phenyl | Fluormethyl | |
| 4.721 | 3-Methyl-4-fluor-Phenyl | Methylcarbonyl | |
| 4.722 | 3-Methyl-4-fluor-Phenyl | Hydroxymethyl | |
| 4.723 | 3-Methyl-4-fluor-Phenyl | Ethinyl | |
| 4.724 | 3-Ethyl-4-fluor-Phenyl | Methyl | |
| 4.725 | 3-Cyclopropyl-4-fluor-Phenyl | Methyl | |
| 4.726 | 3-Cyano-4-fluor-Phenyl | Methyl | |
| 4.727 | 3-Methoxy-4-fluor-Phenyl | Methyl | |
| 4.728 | 3-Ethoxy-4-fluor-Phenyl | Methyl | |
| 4.729 | 3-Trifluormethoxy-4-fluor-Phenyl | Methyl | |
| 4.730 | 3-Nitro-4-fluor-Phenyl | Methyl | |
| 4.731 | 3-Fluor-4-chlor-Phenyl | Methyl | |
| 4.732 | 3,4-Dichlor-Phenyl | Methyl | |
| 4.733 | 3-Brom-4-chlor-Phenyl | Methyl | |
| 4.734 | 3-Methyl-4-chlor-Phenyl | Methyl | |
| 4.735 | 3-Cyclopropyl-4-chlor-Phenyl | Methyl | |
| 4.736 | 3-Cyano-4-chlor-Phenyl | Methyl | |
| 4.737 | 3-Trifluormethyl-4-chlor-Phenyl | Methyl | |
| 4.738 | 3-Methoxy-4-chlor-Phenyl | Methyl | |
| 4.739 | 3-Ethoxy-4-chlor-Phenyl | Methyl | |
| 4.740 | 3-Trifluormethoxy-4-chlor-Phenyl | Methyl | |
| 4.741 | 3-Nitro-4-chlor-Phenyl | Methyl | |
| 4.742 | 3-Fluor-4-brom-Phenyl | Methyl | |
| 4.743 | 3-Chlor-4-brom-Phenyl | Methyl | |
| 4.744 | 3,4-Dibrom-Phenyl | Methyl | |
| 4.745 | 3-Methyl-4-brom-Phenyl | Methyl | |
| 4.746 | 3-Cyclopropyl-4-brom-Phenyl | Methyl | |
| 4.747 | 3-Cyano-4-brom-Phenyl | Methyl | |
| 4.748 | 3-Trifluormethyl-4-brom-Phenyl | Methyl | |
| 4.749 | 3-Methoxy-4-Phenyl | Methyl | |
| 4.750 | 3-Ethoxy-4-brom-Phenyl | Methyl | |
| 4.751 | 3-Trifluormethoxy-4-brom-Phenyl | Methyl | |
| 4.752 | 3-Nitro-4-brom-Phenyl | Methyl | |
| 4.753 | 3-Fluor-4-iod-Phenyl | Methyl | |
| 4.754 | 3-Chlor-4-iod-Phenyl | Methyl | |
| 4.755 | 3-Brom-4-iod-Phenyl | Methyl | |
| 4.756 | 3-Methyl-4-iod-Phenyl | Methyl | |
| 4.757 | 3-Cyclopropyl-4-iod-Phenyl | Methyl | |
| 4.758 | 3-Cyano-4-iod-Phenyl | Methyl | |
| 4.759 | 3-Trifluormethyl-4-iod-Phenyl | Methyl | |
| 4.760 | 3-Methoxy-4-iod-Phenyl | Methyl | |
| 4.761 | 3-Ethoxy-4-iod-Phenyl | Methyl | |
| 4.762 | 3-Trifluormethoxy-4-iod-Phenyl | Methyl | |
| 4.763 | 3-Nitro-4-iod-Phenyl | Methyl | |
| 4.764 | 3-Fluor-4-methyl-Phenyl | Methyl | |
| 4.765 | 3-Chlor-4-methyl-Phenyl | Methyl | |
| 4.766 | 3-Chlor-4-methyl-Phenyl | Ethyl | |
| 4.767 | 3-Chlor-4-methyl-Phenyl | CN | |
| 4.768 | 3-Chlor-4-methyl-Phenyl | Vinyl | |
| 4.769 | 3-Chlor-4-methyl-Phenyl | Fluormethyl | |
| 4.770 | 3-Chlor-4-methyl-Phenyl | Methylcarbonyl | |
| 4.771 | 3-Chlor-4-methyl-Phenyl | Hydroxymethyl | |
| 4.772 | 3-Chlor-4-methyl-Phenyl | Ethinyl | |
| 4.773 | 3-Brom-4-methyl-Phenyl | Methyl | |
| 4.774 | 3.4-Dimethyl-Phenyl | Methyl | |
| 4.775 | 3-Ethyl-4-methyl-Phenyl | Methyl | |
| 4.776 | 3-Cyclopropyl-4-methyl-Phenyl | Methyl | |
| 4.777 | 3-Cyano-4-methyl-Phenyl | Methyl | |
| 4.778 | 3-Trifluormethyl-4-methyl-Phenyl | Methyl | |
| 4.779 | 3-Methoxy-4-methyl-Phenyl | Methyl | |
| 4.780 | 3-Ethoxy-4-methyl-Phenyl | Methyl | |
| 4.781 | 3-Trifluormethoxy-4-methyl-Phenyl | Methyl | |
| 4.782 | 3-Nitro-4-methyl-Phenyl | Methyl | |
| 4.783 | 3-Fluor-4-ethyl-Phenyl | Methyl | |
| 4.784 | 3-Chlor-4-ethyl-Phenyl | Methyl | |
| 4.785 | 3-Brom-4-ethyl-Phenyl | Methyl | |
| 4.786 | 3-Methyl-4-ethyl-Phenyl | Methyl | |
| 4.787 | 3,4-Diethyl-Phenyl | Methyl | |
| 4.788 | 3-Cyclopropyl-4-ethyl-Phenyl | Methyl | |
| 4.789 | 3-Cyano-4-ethyl-Phenyl | Methyl | |
| 4.790 | 3-Trifluormethyl-4-ethyl-Phenyl | Methyl | |
| 4.791 | 3-Methoxy-4-ethyl-Phenyl | Methyl | |
| 4.792 | 3-Ethoxy-4-ethyl-Phenyl | Methyl | |
| 4.793 | 3-Trifluormethoxy-4-ethyl-Phenyl | Methyl | |
| 4.794 | 3-Nitro-4-ethyl-Phenyl | Methyl | |
| 4.795 | 3-Fluor-4-propyl-Phenyl | Methyl | |
| 4.796 | 3-Chlor-4-propyl-Phenyl | Methyl | |
| 4.797 | 3-Brom-4-propyl-Phenyl | Methyl | |
| 4.798 | 3-Methyl-4-propyl-Phenyl | Methyl | |
| 4.799 | 3-Cyclopropyl-4-propyl-Phenyl | Methyl | |
| 4.800 | 3-Cyano-4-propyl-Phenyl | Methyl | |
| 4.801 | 3-Trifluormethyl-4-propyl-Phenyl | Methyl | |
| 4.802 | 3-Methoxy-4-propyl-Phenyl | Methyl | |
| 4.803 | 3-Ethoxy-4-propyl-Phenyl | Methyl | |
| 4.804 | 3-Trifluormethoxy-4-propyl-Phenyl | Methyl | |
| 4.805 | 3-Nitro-4-propyl-Phenyl | Methyl | |
| 4.806 | 3-Fluor-4-isopropyl-Phenyl | Methyl | |
| 4.807 | 3-Chlor-4-isopropyl-Phenyl | Methyl | |
| 4.808 | 3-Brom-4-isopropyl-Phenyl | Methyl | |
| 4.809 | 3-Methyl-4-isopropyl-Phenyl | Methyl | |
| 4.810 | 3-Cyclopropyl-4-isopropyl-Phenyl | Methyl | |
| 4.811 | 3-Cyano-4-isopropyl-Phenyl | Methyl | |
| 4.812 | 3-Trifluormethyl-4-isopropyl-Phenyl | Methyl | |
| 4.813 | 3-Methoxy-4-isopropyl-Phenyl | Methyl | |
| 4.814 | 3-Ethoxy-4-isopropyl-Phenyl | Methyl | |
| 4.815 | 3-Trifluormethoxy-4-isopropyl-Phenyl | Methyl | |
| 4.816 | 3-Nitro-4-isopropyl-Phenyl | Methyl | |
| 4.817 | 3-Fluor-4-tert.butyl-Phenyl | Methyl | |
| 4.818 | 3-Fluor-4-tert.butyl-Phenyl | Ethyl | |
| 4.819 | 3-Fluor-4-tert.butyl-Phenyl | CN | |
| 4.820 | 3-Fluor-4-tert.butyl-Phenyl | Vinyl | |
| 4.821 | 3-Fluor-4-tert.butyl-Phenyl | Fluormethyl | |
| 4.822 | 3-Fluor-4-tert.butyl-Phenyl | Methylcarbonyl | |
| 4.823 | 3-Fluor-4-tert.butyl-Phenyl | Hydroxymethyl | |
| 4.824 | 3-Fluor-4-tert.butyl-Phenyl | Ethinyl | |
| 4.825 | 3-Chlor-4-tert.butyl-Phenyl | Methyl | |
| 4.826 | 3-Brom-4-tert.butyl-Phenyl | Methyl | |
| 4.827 | 3-Methyl-4-tert.butyl-Phenyl | Methyl | |
| 4.828 | 3-Cyclopropyl-4-tert.butyl-Phenyl | Methyl | |
| 4.829 | 3-Cyano-4-tert.butyl-Phenyl | Methyl | |
| 4.830 | 3-Trifluormethyl-4-tert.butyl-Phenyl | Methyl | |
| 4.831 | 3-Methoxy-4-tert.butyl-Phenyl | Methyl | |
| 4.832 | 3-Ethoxy-4-tert.butyl-Phenyl | Methyl | |
| 4.833 | 3-Trifluormethoxy-4-tert.butyl-Phenyl | Methyl | |
| 4.834 | 3-Nitro-4-tert.butyl-Phenyl | Methyl | |
| 4.835 | 3-Fluor-4-cyclopropyl-Phenyl | Methyl | |
| 4.836 | 3-Chlor-4-cyclopropyl-Phenyl | Methyl | |
| 4.837 | 3-Brom-4-cyclopropyl-Phenyl | Methyl | |
| 4.838 | 3-Methyl-4-cyclopropyl-Phenyl | Methyl | |
| 4.839 | 3-Cyclopropyl-4-cyclopropyl-Phenyl | Methyl | |
| 4.840 | 3-Cyano-4-cyclopropyl-Phenyl | Methyl | |
| 4.841 | 3-Trifluormethyl-4-cyclopropyl-Phenyl | Methyl | |
| 4.842 | 3-Methoxy-4-cyclopropyl-Phenyl | Methyl | |
| 4.843 | 3-Ethoxy-4-cyclopropyl-Phenyl | Methyl | |
| 4.844 | 3-Trifluormethoxy-4-cyclopropyl-Phenyl | Methyl | |
| 4.845 | 3-Fluor-4-methoxycarbonyl-Phenyl | Methyl | |
| 4.846 | 3-Chlor-4-methoxycarbonyl-Phenyl | Methyl | |
| 4.847 | 3-Brom-4-methoxycarbonyl-Phenyl | Methyl | |
| 4.848 | 3-Methyl-4-methoxycarbonyl-Phenyl | Methyl | |
| 4.849 | 3-Cyclopropyl-4-methoxycarbonyl-Phenyl | Methyl | |
| 4.850 | 3-Cyano-4-methoxycarbonyl-Phenyl | Methyl | |
| 4.851 | 3-Trifluormethyl-4-nriethoxycarbonyl-Phenyl | Methyl | |
| 4.852 | 3-Methoxy-4-methoxycarbonyl-Phenyl | Methyl | |
| 4.853 | 3-Ethoxy-4-methoxycarbonyl-Phenyl | Methyl | |
| 4.854 | 3-Trifluormethoxy-4-nriethoxycarbonyl-Phenyl | Methyl | |
| 4.855 | 3-Nitro-4-methoxycarbonyl-Phenyl | Methyl | |
| 4.856 | 3-Fluor-4-cyano-Phenyl | Methyl | |
| 4.857 | 3-Chlor-4-cyano-Phenyl | Methyl | |
| 4.858 | 3-Brom-4-cyano-Phenyl | Methyl | |
| 4.859 | 3-Methyl-4-cyano-Phenyl | Methyl | |
| 4.860 | 3-Cyclopropyl-4-cyano-Phenyl | Methyl | |
| 4.861 | 3-Cyano-4-cyanoPhenyl | Methyl | |
| 4.862 | 3-Trifluormethyl-4-cyano-Phenyl | Methyl | |
| 4.863 | 3-Methoxy-4-cyano-Phenyl | Methyl | |
| 4.864 | 3-Ethoxy-4-cyano-Phenyl | Methyl | |
| 4.865 | 3-Trifluormethoxy-4-cyano-Phenyl | Methyl | |
| 4.866 | 3-Nitro-4-cyano-Phenyl | Methyl | |
| 4.867 | 3-Fluor-4-methoxy-Phenyl | Methyl | |
| 4.868 | 3-Chlor-4-methoxy-Phenyl | Methyl | |
| 4.869 | 3-Brom-4-methoxy-Phenyl | Methyl | |
| 4.870 | 3-Methyl-4-methoxy-Phenyl | Methyl | |
| 4.871 | 3-Cyclopropyl-4-methoxy-Phenyl | Methyl | |
| 4.872 | 3-Cyano-4-methoxy-Phenyl | Methyl | |
| 4.873 | 3-Trifluormethyl-4-methoxy-Phenyl | Methyl | |
| 4.874 | 3,4-Dimethoxy-Phenyl | Methyl | |
| 4.875 | 3-Ethoxy-4-methoxy-Phenyl | Methyl | |
| 4.876 | 3-Trifluormethoxy-4-methoxy-Phenyl | Methyl | |
| 4.877 | 3-Nitro-4-methoxy-Phenyl | Methyl | |
| 4.878 | 3-Fluor-4-ethoxy-Phenyl | Methyl | |
| 4.879 | 3-Chlor-4-ethoxy-Phenyl | Methyl | |
| 4.880 | 3-Brom-4-ethoxy-Phenyl | Methyl | |
| 4.881 | 3-Methyl-4-ethoxy-Phenyl | Methyl | |
| 4.882 | 3-Cyclopropyl-4-ethoxy-Phenyl | Methyl | |
| 4.883 | 3-Cyano-4-ethoxy-Phenyl | Methyl | |
| 4.884 | 3-Trifluormethyl-4-ethoxy-Phenyl | Methyl | |
| 4.885 | 3-Methoxy-4-ethoxy-Phenyl | Methyl | |
| 4.886 | 2,4-Diethoxy-Phenyl | Methyl | |
| 4.887 | 3-Trifluormethoxy-4-ethoxy-Phenyl | Methyl | |
| 4.888 | 3-Nitro-4-ethoxy-Phenyl | Methyl | |
| 4.889 | 3-Fluor-4-isopropoxy-Phenyl | Methyl | |
| 4.890 | 3-Chlor-4-isopropoxy-Phenyl | Methyl | |
| 4.891 | 3-Brom-4-isopropoxy-Phenyl | Methyl | |
| 4.892 | 3-Methyl-4-isopropoxy-Phenyl | Methyl | |
| 4.893 | 3-Cyclopropyl-4-isopropoxy-Phenyl | Methyl | |
| 4.894 | 3-Cyano-4-isopropoxy-Phenyl | Methyl | |
| 4.895 | 3-Trifluormethyl-4-isopropoxy-Phenyl | Methyl | |
| 4.896 | 3-Methoxy-4-isopropoxy-Phenyl | Methyl | |
| 4.897 | 3-Ethoxy-4-isopropoxy-Phenyl | Methyl | |
| 4.898 | 3-Trifluormethoxy-4-isopropoxy-Phenyl | Methyl | |
| 4.899 | 3-Nitro-4-isopropoxy-Phenyl | Methyl | |
| 4.900 | 3-Fluor-4-trifluormethoxy-Phenyl | Methyl | |
| 4.901 | 3-Chlor-4-trifluormethoxy-Phenyl | Methyl | |
| 4.902 | 3-Brom-4-trifluormethoxy-Phenyl | Methyl | |
| 4.903 | 3-Methyl-4-trifluormethoxy-Phenyl | Methyl | |
| 4.904 | 3-Cyclopropyl-4-trifluormethoxy-Phenyl | Methyl | |
| 4.905 | 3-Cyano-4-trifluormethoxy-Phenyl | Methyl | |
| 4.906 | 3-Trifluormethyl-4-trifluormethoxy-Phenyl | Methyl | |
| 4.907 | 3-Methoxy-4-trifluormethoxy-Phenyl | Methyl | |
| 4.908 | 3-Ethoxy-4-trifluormethoxy-Phenyl | Methyl | |
| 4.909 | 3,4-Bis(trifluormethoxy)-Phenyl | Methyl | |
| 4.910 | 3-Nitro-4-trifluormethoxy-Phenyl | Methyl | |
| 4.911 | 3-Fluor-4-difluormethoxy-Phenyl | Methyl | |
| 4.912 | 3-Chlor-4-difluormethoxy-Phenyl | Methyl | |
| 4.913 | 3-Brom-4-difluormethoxy-Phenyl | Methyl | |
| 4.914 | 3-Methyl-4-difluormethoxy-Phenyl | Methyl | |
| 4.915 | 3-Cyclopropyl-4-difluormethoxy-Phenyl | Methyl | |
| 4.916 | 3-Cyano-4-difluormethoxy-Phenyl | Methyl | |
| 4.917 | 3-Trifluormethyl-4-difluormethoxy-Phenyl | Methyl | |
| 4.918 | 3-Methoxy-4-difluormethoxy-Phenyl | Methyl | |
| 4.919 | 3-Ethoxy-4-difluormethoxy-Phenyl | Methyl | |
| 4.920 | 3-Trifluormethoxy-4-difluormethoxy-Phenyl | Methyl | |
| 4.921 | 3-Nitro-4-difluormethoxy-Phenyl | Methyl | |
| 4.922 | 3-Fluor-4-nitro-Phenyl | Methyl | |
| 4.923 | 3-Chlor-4-nitro-Phenyl | Methyl | |
| 4.924 | 3-Brom-4-nitro-Phenyl | Methyl | |
| 4.925 | 3-Methyl-4-nitro-Phenyl | Methyl | |
| 4.926 | 3-Cyclopropyl-4-nitro-Phenyl | Methyl | |
| 4.927 | 3-Cyano-4-nitro-Phenyl | Methyl | |
| 4.928 | 3-Trifluormethyl-4-nitro-Phenyl | Methyl | |
| 4.929 | 3-Methoxy-4-nitro-Phenyl | Methyl | |
| 4.930 | 3-Ethoxy-4-nitro-Phenyl | Methyl | |
| 4.931 | 3-Trifluormethoxy-4-nitro-Phenyl | Methyl | |
| 4.932 | 3-Fluor-4-methylsulfanylPhenyl | Methyl | |
| 4.933 | 3-Chlor-4-methylsulfanyl-Phenyl | Methyl | |
| 4.934 | 3-Brom-4-methylsulfanyl-Phenyl | Methyl | |
| 4.935 | 3-Methyl-4-methylsulfanyl-Phenyl | Methyl | |
| 4.936 | 3-Cyclopropyl-4-methylsulfanyl-Phenyl | Methyl | |
| 4.937 | 3-Cyano-4-methylsulfanyl-Phenyl | Methyl | |
| 4.938 | 3-Trifluormethyl-4-nriethylsulfanyl-Phenyl | Methyl | |
| 4.939 | 3-Methoxy-4-methylsulfanyl-Phenyl | Methyl | |
| 4.940 | 3-Ethoxy-4-methylsulfanyl-Phenyl | Methyl | |
| 4.941 | 3-Trifluormethoxy-4-methylsulfanyl-Phenyl | Methyl | |
| 4.942 | 3-Nitro-4-methylsulfanyl-Phenyl | Methyl | |
| 4.943 | 3,6-Difluor-Phenyl | Methyl | |
| 4.944 | 3,6-Difluor-Phenyl | Ethyl | |
| 4.945 | 3,6-Difluor-Phenyl | CN | |
| 4.946 | 3,6-Difluor-Phenyl | Vinyl | |
| 4.947 | 3,6-Difluor-Phenyl | Fluormethyl | |
| 4.948 | ,6-Difluor-Phenyl | Methylcarbonyl | |
| 4.949 | 3,6-Difluor-Phenyl | Hydroxymethyl | |
| 4.950 | 3,6-Difluor-Phenyl | Ethinyl | |
| 4.951 | 3-Chlor-6-fluor-Phenyl | Methyl | |
| 4.952 | 3-Brom-6-fluor-Phenyl | Methyl | |
| 4.953 | 3-Methyl-6-fluor-Phenyl | Methyl | |
| 4.954 | 3-Cyclopropyl-6-fluor-Phenyl | Methyl | |
| 4.955 | 3-Cyano-6-fluor-Phenyl | Methyl | |
| 4.956 | 3-Methoxy-6-fluor-Phenyl | Methyl | |
| 4.957 | 3-Ethoxy-6-fluor-Phenyl | Methyl | |
| 4.958 | 3-Trifluormethoxy-6-fluor-Phenyl | Methyl | |
| 4.959 | 3-Nitro-6-fluor-Phenyl | Methyl | |
| 4.960 | 3-Fluor-6-chlor-Phenyl | Methyl | |
| 4.961 | 3,6-Dichlor-Phenyl | Methyl | |
| 4.962 | 3-Brom-6-chlor-Phenyl | Methyl | |
| 4.963 | 3-Methyl-6-chlor-Phenyl | Methyl | |
| 4.964 | 3-Cyclopropyl-6-chlor-Phenyl | Methyl | |
| 4.965 | 3-Cyano-6-chlor-Phenyl | Methyl | |
| 4.966 | 3-Trifluormethyl-6-chlor-Phenyl | Methyl | |
| 4.967 | 3-Methoxy-6-chlor-Phenyl | Methyl | |
| 4.968 | 3-Ethoxy-6-chlor-Phenyl | Methyl | |
| 4.969 | 3-Trifluormethoxy-6-chlor-Phenyl | Methyl | |
| 4.970 | 3-Nitro-6-chlor-Phenyl | Methyl | |
| 4.971 | 3-Fluor-6-brom-Phenyl | Methyl | |
| 4.972 | 3-Chlor-6-brom-Phenyl | Methyl | |
| 4.973 | 3,6-Dibrom-Phenyl | Methyl | |
| 4.974 | 3-Methyl-6-brom-Phenyl | Methyl | |
| 4.975 | 3-Cyclopropyl-6-brom-Phenyl | Methyl | |
| 4.976 | 3-Cyano-6-brom-Phenyl | Methyl | |
| 4.977 | 3-Trifluormethyl-6-brom-Phenyl | Methyl | |
| 4.978 | 3-Methoxy-6-Phenyl | Methyl | |
| 4.979 | 3-Ethoxy-6-brom-Phenyl | Methyl | |
| 4.980 | 3-Trifluormethoxy-6-brom-Phenyl | Methyl | |
| 4.981 | 3-Nitro-6-brom-Phenyl | Methyl | |
| 4.982 | 3-Fluor-6-iod-Phenyl | Methyl | |
| 4.983 | 3-Chlor-6-iod-Phenyl | Methyl | |
| 4.984 | 3-Brom-6-iod-Phenyl | Methyl | |
| 4.985 | 3-Methyl-6-iod-Phenyl | Methyl | |
| 4.986 | 3-Ethyl-6-iod-Phenyl | Methyl | |
| 4.987 | 3-Cyclopropyl-6-iod-Phenyl | Methyl | |
| 4.988 | 3-Cyano-6-iod-Phenyl | Methyl | |
| 4.989 | 3-Trifluormethyl-6-iod-Phenyl | Methyl | |
| 4.990 | 3-Methoxy-6-iod-Phenyl | Methyl | |
| 4.991 | 3-Ethoxy-6-iod-Phenyl | Methyl | |
| 4.992 | 3-Trifluormethoxy-6-iod-Phenyl | Methyl | |
| 4.993 | 3-Nitro-6-iod-Phenyl | Methyl | |
| 4.994 | 3-Fluor-6-methyl-Phenyl | Methyl | |
| 4.995 | 3-Chlor-6-methyl-Phenyl | Methyl | |
| 4.996 | 3-Brom-6-methyl-Phenyl | Methyl | |
| 4.997 | 3.6-Dimethyl-Phenyl | Methyl | |
| 4.998 | 3.6-Dimethyl-Phenyl | Ethyl | |
| 4.999 | 3.6-Dimethyl-Phenyl | CN | |
| 4.1000 | 3.6-Dimethyl-Phenyl | Vinyl | |
| 4.1001 | 3.6-Dimethyl-Phenyl | Fluormethyl | |
| 4.1002 | 3.6-Dimethyl-Phenyl | Methylcarbonyl | |
| 4.1003 | 3.6-Dimethyl-Phenyl | Hydroxymethyl | |
| 4.1004 | 3.6-Dimethyl-Phenyl | Ethinyl | |
| 4.1005 | 3-Cyclopropyl-6-methyl-Phenyl | Methyl | |
| 4.1006 | 3-Cyano-6-methyl-Phenyl | Methyl | |
| 4.1007 | 3-Trifluormethyl-6-methyl-Phenyl | Methyl | |
| 4.1008 | 3-Methoxy-6-methyl-Phenyl | Methyl | |
| 4.1009 | 3-Ethoxy-6-methyl-Phenyl | Methyl | |
| 4.1010 | 3-Trifluormethoxy-6-methyl-Phenyl | Methyl | |
| 4.1011 | 3-Nitro-6-methyl-Phenyl | Methyl | |
| 4.1012 | 3-Fluor-6-ethyl-Phenyl | Methyl | |
| 4.1013 | 3-Chlor-6-ethyl-Phenyl | Methyl | |
| 4.1014 | 3-Brom-6-ethyl-Phenyl | Methyl | |
| 4.1015 | 3-Methyl-6-ethyl-Phenyl | Methyl | |
| 4.1016 | 3,6-Diethyl-Phenyl | Methyl | |
| 4.1017 | 3-Cyclopropyl-6-ethyl-Phenyl | Methyl | |
| 4.1018 | 3-Cyano-6-ethyl-Phenyl | Methyl | |
| 4.1019 | 3-Trifluormethyl-6-ethyl-Phenyl | Methyl | |
| 4.1020 | 3-Methoxy-6-ethyl-Phenyl | Methyl | |
| 4.1021 | 3-Ethoxy-6-ethyl-Phenyl | Methyl | |
| 4.1022 | 3-Trifluormethoxy-6-ethyl-Phenyl | Methyl | |
| 4.1023 | 3-Nitro-6-ethyl-Phenyl | Methyl | |
| 4.1024 | 3-Fluor-6-isopropyl-Phenyl | Methyl | |
| 4.1025 | 3-Chlor-6-isopropyl-Phenyl | Methyl | |
| 4.1026 | 3-Brom-6-isopropyl-Phenyl | Methyl | |
| 4.1027 | 3-Methyl-6-isopropyl-Phenyl | Methyl | |
| 4.1028 | 3-Cyclopropyl-6-isopropyl-Phenyl | Methyl | |
| 4.1029 | 3-Cyano-6-isopropyl-Phenyl | Methyl | |
| 4.1030 | 3-Trifluormethyl-6-isopropyl-Phenyl | Methyl | |
| 4.1031 | 3-Methoxy-6-isopropyl-Phenyl | Methyl | |
| 4.1032 | 3-Ethoxy-6-isopropyl-Phenyl | Methyl | |
| 4.1033 | 3-Trifluormethoxy-6-isopropyl-Phenyl | Methyl | |
| 4.1034 | 3-Nitro-6-isopropyl-Phenyl | Methyl | |
| 4.1035 | 3-Fluor-6-tert.butyl-Phenyl | Methyl | |
| 4.1036 | 3-Chlor-6-tert.buty-Phenyl | Methyl | |
| 4.1037 | 3-Brom-6-tert.butyl-Phenyl | Methyl | |
| 4.1038 | 3-Methyl-6-tert.butyl-Phenyl | Methyl | |
| 4.1039 | 3-Ethyl-6-tert.butyl-Phenyl | Methyl | |
| 4.1040 | 3-Cyclopropyl-6-tert.butyl-Phenyl | Methyl | |
| 4.1041 | 3-Cyano-6-tert.butyl-Phenyl | Methyl | |
| 4.1042 | 3-Trifluormethyl-6-tert.butyl-Phenyl | Methyl | |
| 4.1043 | 3-Methoxy-6-tert.butyl-Phenyl | Methyl | |
| 4.1044 | 3-Ethoxy-6-tert.butyl-Phenyl | Methyl | |
| 4.1045 | 3-Trifluormethoxy-6-tert.butyl-Phenyl | Methyl | |
| 4.1046 | 3-Nitro-6-tert.butyl-Phenyl | Methyl | |
| 4.1047 | 3-Fluor-6-cyclopropyl-Phenyl | Methyl | |
| 4.1048 | 3-Chlor-6-cyclopropyl-Phenyl | Methyl | |
| 4.1049 | 3-Brom-6-cyclopropyl-Phenyl | Methyl | |
| 4.1050 | 3-Methyl-6-cyclopropyl-Phenyl | Methyl | |
| 4.1051 | 3-Cyclopropyl-6-cyclopropyl-Phenyl | Methyl | |
| 4.1052 | 3-Cyano-6-cyclopropyl-Phenyl | Methyl | |
| 4.1053 | 3-Trifluormethyl-6-cyclopropyl-Phenyl | Methyl | |
| 4.1054 | 3-Methoxy-6-cyclopropyl-Phenyl | Methyl | |
| 4.1055 | 3-Ethoxy-6-cyclopropyl-Phenyl | Methyl | |
| 4.1056 | 3-Trifluormethoxy-6-cyclopropyl-Phenyl | Methyl | |
| 4.1057 | 3-Fluor-6-methoxycarbonyl-Phenyl | Methyl | |
| 4.1058 | 3-Chlor-6-methoxycarbonyl-Phenyl | Methyl | |
| 4.1059 | 3-Brom-6-methoxycarbonyl-Phenyl | Methyl | |
| 4.1060 | 3-Methyl-6-methoxycarbonyl-Phenyl | Methyl | |
| 4.1061 | 3-Cyclopropyl-6-methoxycarbonyl-Phenyl | Methyl | |
| 4.1062 | 3-Cyano-6-methoxycarbonyl-Phenyl | Methyl | |
| 4.1063 | 3-Trifluormethyl-6-methoxycarbonyl-Phenyl | Methyl | |
| 4.1064 | 3-Methoxy-6-methoxycarbonyl-Phenyl | Methyl | |
| 4.1065 | 3-Ethoxy-6-methoxycarbonyl-Phenyl | Methyl | |
| 4.1066 | 3-Trifluormethoxy-6-methoxycarbonyl-Phenyl | Methyl | |
| 4.1067 | 3-Nitro-6-methoxycarbonyl-Phenyl | Methyl | |
| 4.1068 | 3-Fluor-6-cyano-Phenyl | Methyl | |
| 4.1069 | 3-Chlor-6-cyano-Phenyl | Methyl | |
| 4.1070 | 3-Brom-6-cyano-Phenyl | Methyl | |
| 4.1071 | 3-Methyl-6-cyano-Phenyl | Methyl | |
| 4.1072 | 3-Cyclopropyl-6-cyano-Phenyl | Methyl | |
| 4.1073 | 3-Cyano-6-cyano-Phenyl | Methyl | |
| 4.1074 | 3-Trifluormethyl-6-cyano-Phenyl | Methyl | |
| 4.1075 | 3-Methoxy-6-cyano-Phenyl | Methyl | |
| 4.1076 | 3-Ethoxy-6-cyano-Phenyl | Methyl | |
| 4.1077 | 3-Trifluormethoxy-6-cyano-Phenyl | Methyl | |
| 4.1078 | 3-Nitro-6-cyano-Phenyl | Methyl | |
| 4.1079 | 3-Fluor-6-methoxy-Phenyl | Methyl | |
| 4.1080 | 3-Chlor-6-methoxy-Phenyl | Methyl | |
| 4.1081 | 3-Brom-6-methoxy-Phenyl | Methyl | |
| 4.1082 | 3-Methyl-6-methoxy-Phenyl | Methyl | |
| 4.1083 | 3-Cyclopropyl-6-methoxy-Phenyl | Methyl | |
| 4.1084 | 3-Cyano-6-methoxy-Phenyl | Methyl | |
| 4.1085 | 3-Trifluormethyl-6-methoxy-Phenyl | Methyl | |
| 4.1086 | 3,6-Dimethoxy-Phenyl | Methyl | |
| 4.1087 | 3-Ethoxy-6-methoxy-Phenyl | Methyl | |
| 4.1088 | 3-Trifluormethoxy-6-methoxy-Phenyl | Methyl | |
| 4.1089 | 3-Nitro-6-methoxy-Phenyl | Methyl | |
| 4.1090 | 3-Fluor-6-ethoxy-Phenyl | Methyl | |
| 4.1091 | 3-Chlor-6-ethoxy-Phenyl | Methyl | |
| 4.1092 | 3-Brom-6-ethoxy-Phenyl | Methyl | |
| 4.1093 | 3-Methyl-6-ethoxy-Phenyl | Methyl | |
| 4.1094 | 3-Cyclopropyl-6-ethoxy-Phenyl | Methyl | |
| 4.1095 | 3-Cyano-6-ethoxy-Phenyl | Methyl | |
| 4.1096 | 3-Trifluormethyl-6-ethoxy-Phenyl | Methyl | |
| 4.1097 | 3-Methoxy-6-ethoxy-Phenyl | Methyl | |
| 4.1098 | 2,6-Diethoxy-Phenyl | Methyl | |
| 4.1099 | 3-Trifluormethoxy-6-ethoxy-Phenyl | Methyl | |
| 4.1100 | 3-Nitro-6-ethoxy-Phenyl | Methyl | |
| 4.1101 | 3-Fluor-6-isopropoxy-Phenyl | Methyl | |
| 4.1102 | 3-Chlor-6-isopropoxy-Phenyl | Methyl | |
| 4.1103 | 3-Brom-6-isopropoxy-Phenyl | Methyl | |
| 4.1104 | 3-Methyl-6-isopropoxy-Phenyl | Methyl | |
| 4.1105 | 3-Cyclopropyl-6-isopropoxy-Phenyl | Methyl | |
| 4.1106 | 3-Cyano-6-isopropoxy-Phenyl | Methyl | |
| 4.1107 | 3-Trifluormethyl-6-isopropoxy-Phenyl | Methyl | |
| 4.1108 | 3-Methoxy-6-isopropoxy-Phenyl | Methyl | |
| 4.1109 | 3-Ethoxy-6-isopropoxy-Phenyl | Methyl | |
| 4.1110 | 3-Trifluormethoxy-6-isopropoxy-Phenyl | Methyl | |
| 4.1111 | 3-Nitro-6-isopropoxy-Phenyl | Methyl | |
| 4.1112 | 3-Fluor-6-trifluormethoxy-Phenyl | Methyl | |
| 4.1113 | 3-Chlor-6-trifluormethoxy-Phenyl | Methyl | |
| 4.1114 | 3-Brom-6-trifluormethoxy-Phenyl | Methyl | |
| 4.1115 | 3-Methyl-6-trifluormethoxy-Phenyl | Methyl | |
| 4.1116 | 3-Cyclopropyl-6-trifluormethoxy-Phenyl | Methyl | |
| 4.1117 | 3-Cyano-6-trifluormethoxy-Phenyl | Methyl | |
| 4.1118 | 3-Trifluormethyl-6-trifluormethoxy-Phenyl | Methyl | |
| 4.1119 | 3-Methoxy-6-trifluormethoxy-Phenyl | Methyl | |
| 4.1120 | 3-Ethoxy-6-trifluormethoxy-Phenyl | Methyl | |
| 4.1121 | 3,6-Bis(trifluormethoxy)-Phenyl | Methyl | |
| 4.1122 | 3-Nitro-6-trifluormethoxy-Phenyl | Methyl | |
| 4.1123 | 3-Fluor-6-difluormethoxy-Phenyl | Methyl | |
| 4.1124 | 3-Chlor-6-difluormethoxy-Phenyl | Methyl | |
| 4.1125 | 3-Brom-6-difluormethoxy-Phenyl | Methyl | |
| 4.1126 | 3-Methyl-6-difluormethoxy-Phenyl | Methyl | |
| 4.1127 | 3-Cyclopropyl-6-difluormethoxy-Phenyl | Methyl | |
| 4.1128 | 3-Cyano-6-difluormethoxy-Phenyl | Methyl | |
| 4.1129 | 3-Trifluormethyl-6-difluormethoxy-Phenyl | Methyl | |
| 4.1130 | 3-Methoxy-6-difluormethoxy-Phenyl | Methyl | |
| 4.1131 | 3-Ethoxy-6-difluormethoxy-Phenyl | Methyl | |
| 4.1132 | 3-Trifluormethoxy-6-difluormethoxy-Phenyl | Methyl | |
| 4.1133 | 3-Nitro-6-difluormethoxy-Phenyl | Methyl | |
| 4.1134 | 3-Fluor-6-nitro-Phenyl | Methyl | |
| 4.1135 | 3-Chlor-6-nitro-Phenyl | Methyl | |
| 4.1136 | 3-Brom-6-nitro-Phenyl | Methyl | |
| 4.1137 | 3-Methyl-6-nitro-Phenyl | Methyl | |
| 4.1138 | 3-Cyclopropyl-6-nitro-Phenyl | Methyl | |
| 4.1139 | 3-Cyano-6-nitro-Phenyl | Methyl | |
| 4.1140 | 3-Trifluormethyl-6-nitro-Phenyl | Methyl | |
| 4.1141 | 3-Methoxy-6-nitro-Phenyl | Methyl | |
| 4.1142 | 3-Ethoxy-6-nitro-Phenyl | Methyl | |
| 4.1143 | 3-Trifluormethoxy-6-nitro-Phenyl | Methyl | |
| 4.1144 | 3-Fluor-6-methylsulfanylPhenyl | Methyl | |
| 4.1145 | 3-Chlor-6-methylsulfanyl-Phenyl | Methyl | |
| 4.1146 | 3-Brom-6-methylsulfanyl-Phenyl | Methyl | |
| 4.1147 | 3-Methyl-6-methylsulfanyl-Phenyl | Methyl | |
| 4.1148 | 3-Ethyl-6-methylsulfanyl-Phenyl | Methyl | |
| 4.1149 | 3-Cyclopropyl-6-methylsulfanyl-Phenyl | Methyl | |
| 4.1150 | 3-Cyano-6-methylsulfanyl-Phenyl | Methyl | |
| 4.1151 | 3-Trifluormethyl-6-methylsulfanyl-Phenyl | Methyl | |
| 4.1152 | 3-Methoxy-6-methylsulfanyl-Phenyl | Methyl | |
| 4.1153 | 3-Ethoxy-6-methylsulfanyl-Phenyl | Methyl | |
| 4.1154 | 3-Trifluormethoxy-6-methylsulfanyl-Phenyl | Methyl | |
| 4.1155 | 3-Nitro-6-methylsulfanyl-Phenyl | Methyl | |
| 4.1156 | 2,3,4-Trifluor-Phenyl | Methyl | |
| 4.1157 | 2,3,4-Trichlor-Phenyl | Methyl | |
| 4.1158 | 2,3.4-Trimethyl-Phenyl | Methyl | |
| 4.1159 | 2-Fluor-2-chlor-5-trifluormethyl-Phenyl | Methyl | |
| 4.1160 | 2,3,5-Trifluor-Phenyl | Methyl | |
| 4.1161 | 2,3,5-Trichlor-Phenyl | Methyl | |
| 4.1162 | 2,3,5-Trimethyl-Phenyl | Methyl | |
| 4.1163 | 2,3-Dichlor-5-methoxy-Phenyl | Methyl | |
| 4.1164 | 2,3,6-Trifluor-Phenyl | Methyl | |
| 4.1165 | 2,3,6-Trichlor-Phenyl | Methyl | |
| 4.1166 | 2,3,6-Trimethyl-Phenyl | Methyl | |
| 4.1167 | 3,4,5-Trifluor-Phenyl | Methyl | |
| 4.1168 | 3,4,6-Trifluor-Phenyl | Methyl | |
| 4.1169 | 3,4,6-Trichlor-Phenyl | Methyl | |
| 4.1170 | 3,4,6-Trichlor-Phenyl | Ethyl | |
| 4.1171 | 3,4,6-Trichlor-Phenyl | CN | |
| 4.1172 | 3,4,6-Trichlor-Phenyl | Vinyl | |
| 4.1173 | 3,4,6-Trichlor-Phenyl | Fluormethyl | |
| 4.1174 | 3,4,6-Trichlor-Phenyl | Methylcarbonyl | |
| 4.1175 | 3,4,6-Trichlor-Phenyl | Hydroxymethyl | |
| 4.1176 | 3,4,6-Trichlor-Phenyl | Ethinyl | |
| 4.1177 | 3,4,5-Trimethyl-Phenyl | Methyl | |
| 4.1178 | 3,5-Dimethyl-4-fluor-Phenyl | Methyl | |
| 4.1179 | 3,5-Dichlor-4-methoxy-Phenyl | Methyl | |
| 4.1180 | 3,5-Difluor-4-chlor-Phenyl | Methyl | |
| 4.1181 | 3,5-Dichlor-4-hydroxy-Phenyl | Methyl | |
| 4.1182 | 3,5-Trifluormethyl-4-chlor-Phenyl | Methyl | |
| 4.1183 | 3,4,6-Trifluor-Phenyl | Methyl | |
| 4.1184 | 3,4,6-Trichlor-Phenyl | Methyl | |
| 4.1185 | 3,4,6-Trimethyl-Phenyl | Methyl | |
| 4.1186 | 2,3,4,5-Pentafluor-Phenyl | Methyl | |

### B. Formulierungsbeispiele

### 1. Stäubemittel

Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | | |
|---|---|---|
| 75 | Gew.-Teile einer Verbindung der Formel (I), | |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | | |
|---|---|---|
| 25 | Gew.-Teile einer Verbindung der Formel (I), | |
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha in unterschiedlichen Dosierungen auf die Oberfläche der Abdeckerde appliziert. Anschließend werden die Töpfe zur weiteren Kultivierung der Pflanzen im Gewächshaus unter optimalen Bedingungen gehalten. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigten beispielsweise die Verbindungen der Nr. 1.1.054, 1.1.2008, 1.1.462, 1.1.463, 1.1.494, 1.1.520, 1.1.527, 1.1.580, 1.1.603, 1.3.470, 1.2.471, 1.3.001, 1.3.473, 2.1.466, 2.1.478, 1.3.527, 2.1.522, 2.1.526, 2.1.546, 2.1.588, 2.2.472, 2.3.475, 2.3.528, 2.3.572, 2.6.462, 2.6.463, 2.6.464, 2.6.465, 2.6.467, 2.6.468, 2.6.469, 2.6.470, 2.6.471, 2.6.473, 2.6.474, 2.6.479, 2.6.480, 2.6.481, 2.6.486, 2.6.488, 2.6.489, 2.6.490, 2.6.541, 2.6.547, 2.6.553, 2.6.555 und 2.6.561 bei einer Aufwandmenge von 320 Gramm pro Hektar jeweils eine mindestens 80%-ige Wirkung gegen Avena fatua. Die Verbindungen der Nr. 1.1.471, 1.1.519, 1.1.540, 1.1.580, 1.3.476, 1.3.571, 1.3.644, 2.1.523, 2.1.524, 2.1.532, 2.1.569, 2.2.530, 2.3.527, 2.3.534, 2.6.545, 2.6.548, 2.6.549, 2.6.550 und 2.6.552 zeigten bei einer Aufwandmenge von 320 Gramm pro Hektar jeweils eine mindestens 80%-ige Wirkung gegen Stellaria media. Die Verbindungen der Nr. 1.1.566, 1.3.474, 2.1.531, 2.6.475 und 2.6.478 zeigten bei einer Aufwandmenge von 320 Gramm pro Hektar jeweils eine mindestens 80%-ige Wirkung gegen Lolium multiflorum.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigten beispielsweise die Verbindungen der Nr. 1.1.2009, 1.1.462, 1.1.463, 1.1.494, 1.1.519, 1.1.520, 1.1.529, 1.1.540, 1.1.566, 1.1.580, 1.2.470, 1.2.471, 1.2.525, 1.3.001, 1.3.004, 1.3.470, 1.3.472, 1.3.474,1.3.473, 1.3.476, 1.3.486, 1.3.527, 1.3.528, 1.3.571, 1.3.644, 2.1.466, 2.1.478, 2.1.522, 2.1.526, 2.1.527, 2.1.530, 2.1.532, 2.1.546, 2.1.570, 2.1.588, 2.2.471, 2.2.472, 2.2.527, 2.3.475, 2.3.476, 2.3.527, 2.3.528, 2.3.534, 2.3.535, 2.3.572, 2.3.645, 2.6.009, 2.6.462, 2.6.463, 2.6.464, 2.6.467, 2.6.468, 2.6.469, 2.6.470, 2.6.471, 2.6.473, 2.6.474, 2.6.475, 2.6.476, 2.6.477, 2.6.478, 2.6.479, 2.6.480, 2.6.481, 2.6.486, 2.6.488, 2.6.489, 2.6.490, 2.6.494, 2.6.495, 2.6.541, 2.6.545, 2.6.546, 2.6.547, 2.6.548, 2.6.549, 2.6.552, 2.6.553, 2.6.554, 2.6.555 und 2.6.561 bei einer Aufwandmenge von 320 Gramm pro Hektar jeweils eine mindestens 80%-ige Wirkung gegen Avena fatua. Die Verbindungen der Nr. 1.1.2008, 1.1.1211, 1.1.527, 1.1.502, 1.1.925, 1.2.002, 1.2.528, 2.1.523, 2.1.524, 2.1.531, 2.1.670, 2.2.475, 2.6.465 und 2.6.550 zeigten bei einer Aufwandmenge von 320 Gramm pro Hektar jeweils eine mindestens 80%-ige Wirkung gegen Veronica persica.

## Patentansprüche

1. 3-Phenylisoxazolinderivate oder deren Salze der Formel (I) 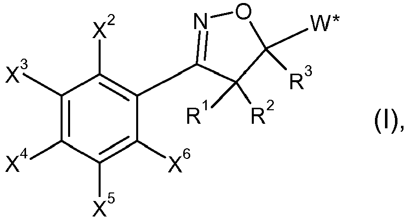 worin
R¹ und R² bedeuten unabhängig voneinander jeweils Wasserstoff, Fluor, Chlor, Brom, lod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷,
W* bedeutet COOH, COOY, CN oder CHO;
Y bedeutet durch m Reste aus der Gruppe bestehend aus Fluor, Chlor, Brom, Cyano, Hydroxy und COO-(C₁-C₈)-Alkyl substituiertes (C₁-C₈)-Alkyl, das durch n Heteroatome aus der Gruppe bestehend aus Sauerstoff, Schwefel und Stickstoff unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom, lod, Cyano, Nitro,
oder jeweils durch m Reste aus der Gruppe bestehend aus Fluor, Chlor, Brom lod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Alkylcarbonyl;
X³ bedeutet Fluor, Chlor, Brom, lod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, lod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, lod, Cyano und (C₁-C₂)-Alkoxy substituiertes (C₁-C₆)-Alkoxy, (C₂-C₆)-Alkenyloxy oder (C₂-C₆)-Alkinyloxy;
X⁵ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸,
NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, lod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, lod, Cyano und (C₁-C₂)-Alkoxy substituiertes (C₁-C₆)-Alkoxy, (C₂-C₆)-Alkenyloxy oder (C₂-C₆)-Alkinyloxy;
m bedeutet 0, 1, 2, 3, 4 oder 5;
n bedeutet 0, 1 oder 2;
p bedeutet 0 oder 1;
q bedeutet 3, 4 oder 5;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
Methyl-3-(3-cyanphenyl)-5-(isopropoxymethyl)-4,5-dihydro-1,2-oxazol-5-carboxylat,
Methyl-5-(butoxymethyl)-3-(3-cyanphenyl)-4,5-dihydro-1,2-oxazol-5-carboxylat,
Methyl-3-(3-cyanphenyl)-5-(methoxy-methyl)-4,5-dihydro-1,2-oxazol-5-carboxylat,
Methyl-3-(3-cyanphenyl)-5-(2-methoxyethyl)-4,5-dihydro-1,2-oxazol-5-carboxylat,
3-(3-Cyanphenyl)-5-(trifluormethyl)-4,5-dihydro-1,2-oxazol-5-carbonsäuremethylester,
Methyl-3-(3-cyan-phenyl)-5-methyl-4,5-dihydro-1,2-oxazol-5-carboxylat,
Ethyl-3-(3-cyanphenyl)-5-methyl-4,5-dihydro-1,2-oxazol-5-carboxylat,
Methyl-5-methyl-3-(3-nitro-phenyl)-4,5-dihydro-1,2-oxazol-5-carboxylat,
Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

2. 3-Phenylisoxazolinderivate nach Anspruch 1, worin
R¹ und R² bedeuten jeweils Wasserstoff,
R³ bedeutet (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Al kylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₂-C₆)-Alkenyloxy oder (C₂-C₆)-Alkinyloxy;
X⁵ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₂-C₆)-Alkenyloxy oder (C₂-C₆)-Alkinyloxy;
m bedeutet 0, 1, 2, 3, 4 oder 5;
n bedeutet 0, 1 oder 2;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
Methyl-3-(3-cyanphenyl)-5-(isopropoxymethyl)-4,5-dihydro-1,2-oxazol-5-carboxylat,
Methyl-5-(butoxymethyl)-3-(3-cyanphenyl)-4,5- dihydro-1,2-oxazol-5-carboxylat,
Methyl-3-(3-cyanphenyl)-5-(methoxy-methyl)-4,5-dihydro-1,2-oxazol-5-carboxylat,
Methyl-3-(3-cyanphenyl)-5-(2-methoxyethyl)-4,5-dihydro-1,2-oxazol-5-carboxylat,
3-(3-Cyanphenyl)-5-(trifluormethyl)-4,5-dihydro-1,2-oxazol-5-carbonsäuremethylester,
Methyl-3-(3-cyan-phenyl)-5-methyl-4,5-dihydro-1,2-oxazol-5-carboxylat,
Ethyl-3-(3-cyanphenyl)-5-methyl-4,5-dihydro-1,2-oxazol-5-carboxylat,
Methyl-5-methyl-3-(3-nitro-phenyl)-4,5-dihydro-1,2-oxazol-5-carboxylat,
Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

3. 3-Phenylisoxazolinderivate nach Anspruch 1 oder 2, worin
R¹ und R² bedeuten jeweils Wasserstoff;
R³ bedeutet (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet Methyl oder Ethyl;
R⁶ bedeutet Wasserstoff oder R⁵;
R⁷ bedeutet Wasserstoff oder durch jeweils m Reste aus der Gruppe bestehend aus Fluor und Chlor substituiertes (C₁-C₆)-Alkyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und C₁-C₄)-Alkoxy substituiertes (C₁-C₄)-Alkyl oder (C₁-C₄)-Alkoxy;
X³ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
X⁵ bedeutet Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
m bedeutet 0, 1, 2 oder 3;
n bedeutet 0, 1 oder 2;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
Methyl-3-(3-cyanphenyl)-5-(isopropoxymethyl)-4,5-dihydro-1,2-oxazol-5-carboxylat,
Methyl-5-(butoxymethyl)-3-(3-cyanphenyl)-4,5- dihydro-1,2-oxazol-5-carboxylat,
Methyl-3-(3-cyanphenyl)-5-(methoxy-methyl)-4,5-dihydro-1,2-oxazol-5-carboxylat,
Methyl-3-(3-cyanphenyl)-5-(2-methoxyethyl)-4,5-dihydro-1,2-oxazol-5-carboxylat,
3-(3-Cyanphenyl)-5-(trifluormethyl)-4,5-dihydro-1,2-oxazol-5-carbonsäuremethylester,
Methyl-3-(3-cyan-phenyl)-5-methyl-4,5-dihydro-1,2-oxazol-5-carboxylat,
Ethyl-3-(3-cyanphenyl)-5-methyl-4,5-dihydro-1,2-oxazol-5-carboxylat,
Methyl-5-methyl-3-(3-nitro-phenyl)-4,5-dihydro-1,2-oxazol-5-carboxylat,
Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

4. 3-Phenylisoxazolinderivate nach Anspruch 3, worin
X⁵ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy.

5. Herbizide Mittel, **gekennzeichnet durch** einen herbizid wirksamen Gehalt an mindestens einer Verbindung der Formel (I) 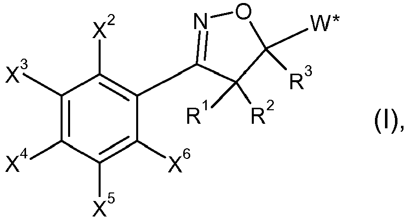 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder
fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙR⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
W* bedeutet COOH, COOY, CN oder CHO;
Y bedeutet durch m Reste aus der Gruppe bestehend aus Fluor, Chlor, Brom, Cyano, Hydroxy und COO-(C₁-C₈)-Alkyl substituiertes (C₁-C₈)-Alkyl, das durch n Heteroatome aus der Gruppe bestehend aus Sauerstoff, Schwefel und Stickstoff unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Al kylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
m bedeutet 0, 1, 2, 3, 4 oder 5;
n bedeutet 0, 1 oder 2;
p bedeutet 0 oder 1;
q bedeutet 3, 4 oder 5;
mit der Maßgabe, daß
a) X³ und X⁴ nicht gleichzeitig substituiertes oder unsubstituiertes Alkoxy bedeuten,
b) die Verbindungen, in denen R³ für Methyl steht und W* für COOH steht, X⁵ nicht Wasserstoff bedeutet, und
c) die Verbindungen
Methyl-3-(3-cyanphenyl)-5-(isopropoxymethyl)-4,5-dihydro-1,2-oxazol-5-carboxylat,
Methyl-5-(butoxymethyl)-3-(3-cyanphenyl)-4,5- dihydro-1,2-oxazol-5-carboxylat,
Methyl-3-(3-cyanphenyl)-5-(methoxy-methyl)-4,5-dihydro-1,2-oxazol-5-carboxylat,
Methyl-3-(3-cyanphenyl)-5-(2-methoxyethyl)-4,5-dihydro-1,2-oxazol-5-carboxylat,
3-(3-Cyanphenyl)-5-(trifluormethyl)-4,5-dihydro-1,2-oxazol-5-carbonsäure,
Methyl-3-(3-cyan-phenyl)-5-methyl-4,5-dihydro-1,2-oxazol-5-carboxylat,
Ethyl-3-(3-cyanphenyl)-5-methyl-4,5-dihydro-1,2-oxazol-5-carboxylat,
Methyl-5-methyl-3-(3-nitro-phenyl)-4,5-dihydro-1,2-oxazol-5-carboxylat,
Methyl-3-(3,5-di-tert-butyl-4-hydroxyphenyl)-5-methyl-4,5-dihydro-1,2-oxazol-5-carboxylat und Ethyl-3-(3-chlorphenyl)-5-methyl-4,5-dihydro-1,2-oxazol-5-carboxylat ausgenommen sind.

6. Herbizide Mittel nach Anspruch 5 in Mischung mit Formulierungshilfsmitteln.

7. Herbizide Mittel nach Anspruch 5 oder 6 enthaltend mindestens einen weiteren pestizid wirksame Stoffen aus der Gruppe Insektizide, Akarizide, Herbizide, Fungizide, Safenern und Wachstumsregulatoren.

8. Herbizide Mittel nach Anspruch 7 enthaltend einen Safener.

9. Herbizide Mittel nach Anspruch 8, worin der Safener ausgewählt ist aus der Gruppe bestehend aus Mefenpyr-diethyl, Cyprosulfamid, Isoxadifen-ethyl, Cloquintocet-mexyl, Benoxacor und Dichlormid.

10. Herbizide Mittel nach einem der Ansprüche Anspruch 7 bis 9 enthaltend ein weiteres Herbizid.

11. Verwendung von 3-Phenyl-isoxazolinderivate der Formel (la) 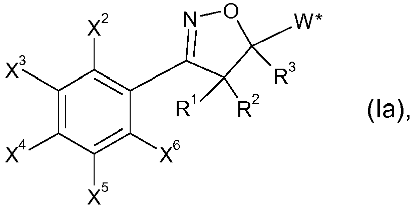 worin
R¹ und R² bedeuten unabhängig voneinander jeweils Wasserstoff, Fluor, Chlor, Brom, Iod, Cyano, oder durch jeweils m Reste aus der Gruppe bestehend aus Fluor, Chlor, Brom, Iod und Cyano substituiertes (C₁-C₄)-Alkyl oder (C₁-C₄)-Alkoxy,
oder
R¹ und R² bilden gemeinsam mit dem Kohlenstoffatom, an das sie gebunden sind, einen gesättigten, teilweise oder vollständig ungesättigten drei-, vier- oder fünfgliedrigen Ring, der aus q Kohlenstoffatomen und p Sauerstoffatomen aufgebaut ist;
R³ bedeutet Fluor, Chlor, Cyano, (C₁-C₃)-Alkylcarbonyloxy oder S(O)ₙR⁵,
oder durch jeweils m Reste aus der Gruppe bestehend aus Fluor, Chlor, Brom, Iod, Cyano, (C₁-C₄)-Alkoxy und Hydroxy substituiertes (C₁-C₆)-Alkyl, (C₃-C₆)-Cycloalkyl, (C₂-C₆)-Alkenyl oder (C₂-C₆)-Alkinyl,
oder durch jeweils m Reste aus der Gruppe bestehend aus Fluor, Chlor, Brom, Iod, Cyano und (C₁-C₆)-Alkoxy substituiertes (C₁-C₆)-Alkylcarbonyl, (C₂-C₆)-Alkenylcarbonyl oder (C₃-C₆)-Cycloalkylcarbonyl;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
W* bedeutet COOH, COOY, CN oder CHO;
Y bedeutet durch m Reste aus der Gruppe bestehend aus Fluor, Chlor, Brom, Cyano, Hydroxy und COO-(C₁-C₈)-Alkyl substituiertes (C₁-C₈)-Alkyl, das durch n Heteroatome aus der Gruppe bestehend aus Sauerstoff, Schwefel und Stickstoff unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom, Iod, Cyano, Nitro,
oder jeweils durch m Reste aus der Gruppe bestehend aus Fluor, Chlor, Brom Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Al kylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
m bedeutet 0, 1, 2, 3, 4 oder 5;
n bedeutet 0, 1 oder 2;
p bedeutet 0 oder 1;
q bedeutet 3, 4 oder 5,
zur Bekämpfung unerwünschter Pflanzen.

12. Verwendung von 3-Phenylisoxazolinderivaten nach Anspruch 11, worin
R¹ und R² bedeuten jeweils Wasserstoff,
R³ bedeutet (C₁-C₆)-Alkyl, (C₁-C₄)-Alkoxy-(C₁-C₄)-alkyl, (C₂-C₆)-Alkenyl, (C₂-C₆)-Alkinyl, Halogen-(C₁-C₆)-Alkyl oder Halogen-(C₂-C₆)-Alkenyl;
R⁵ bedeutet durch jeweils m Reste aus der Gruppe bestehend aus Fluor, Chlor, Brom, Iod, Cyano und Hydroxy substituiertes (C₁-C₆)-Alkyl oder (C₃-C₆)-Cycloalkyl;
R⁶ bedeutet Wasserstoff oder R⁵;
R⁷ bedeutet Wasserstoff oder durch jeweils m Reste aus der Gruppe bestehend aus Fluor, Chlor, Brom, Cyano und (C₁-C₂)-Alkoxy substituiertes (C₁-C₆)-Alkyl, (C₃-C₆)-Cycloalkyl, (C₂-C₄)-Alkenyl oder (C₂-C₄)-Alkinyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist, oder
einen Rest aus der Gruppe bestehend aus Cyclohex-2-en-1-on-3-yl, Propan-1-ol-3-yl, 2,2-Dimethylpropan-1-ol-3yl, Methyl-2,2-dimethylpropanoat-3-yl, Methyl-propanoat-3-yl, Ethylpropanoat-3-yl, Ethylbutanoat-3-yl, Ethyl-(3R)-4,4,4-trifluor-butanoat-3-yl, Butan-2-on-4-yl, 3-Methylbutan-2-on-4-yl, Pent-3-en-2-on-4-yl,((2S)-Dimethyl-butandioat-2yl, Dimethylpentandioat-3yl, Methyl-(2R)-2-methylpropanoat-3-yl, 4-Methoxycarbonylbenzyl, 3,5-Difluorbenzyl, 3,4-Difluorbenzyl, 2,6-Difluorbenzyl, 5-Methylpyridin-3-yl-methyl, Tetrahydrofuran-3yl und Butan-1-ol-4-yl;
X², X⁴ und X⁶ bedeuten unabhängig voneinander jeweils Wasserstoff, Fluor, Chlor, Brom Iod, Cyano, Nitro,
oder jeweils durch m Reste aus der Gruppe bestehend aus Fluor, Chlor, Brom, Iod, Cyano und (C₁-C₄)-Alkoxy substituiertes (C₁-C₄)-Alkyl, (C₃-C₅)-Cycloalkyl, (C₂-C₄)-Alkenyl, (C₂-C₄)-Alkinyl, (C₁-C₄)-Alkoxy, (C₂-C₄)-Alkenyloxy, (C₂-C₄)-Alkinyloxy oder (C₁-C₄)-Al kylcarbonyl;
X³ bedeutet Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
X⁵ bedeutet Wasserstoff, Fluor, Chlor, Brom, Iod, Hydroxy, Cyano, Nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
oder jeweils durch m Reste aus der Gruppe bestehend aus Fluor, Chlor, Brom, Iod, Hydroxy und Cyano substituiertes (C₁-C₆)-Alkyl, (C₃-C₅)-Cycloalkyl, (C₂-C₆)-Alkenyl, (C₂-C₆)-Alkinyl,
oder jeweils durch m Reste aus der Gruppe bestehend aus Fluor, Chlor, Brom, Iod, Cyano und (C₁-C₂)-Alkoxy substituiertes (C₁-C₆)-Alkoxy, (C₃-C₆)-Cycloalkoxy, (C₂-C₆)-Alkenyloxy oder (C₂-C₆)-Alkinyloxy;
m bedeutet 0, 1, 2, 3, 4 oder 5;
n bedeutet 0, 1 oder 2.

13. Verwendung von 3-Phenylisoxazolinderivaten nach Anspruch 11 oder 12, worin
R¹ und R² bedeuten jeweils Wasserstoff;
R³ bedeutet (C₁-C₄)-Alkyl, (C₁-C₄)-Alkoxy-(C₁-C₄)-alkyl, Vinyl, (C₂-C₄)-Alkinyl, Halogen-(C₁-C₄)-Alkyl oder Halogen-(C₂-C₄)-Alkenyl;
R⁵ bedeutet Methyl oder Ethyl;
R⁶ bedeutet Wasserstoff oder R⁵;
R⁷ bedeutet Wasserstoff oder durch jeweils m Reste aus der Gruppe bestehend aus Fluor und Chlor substituiertes (C₁-C₆)-Alkyl;
R⁸ bedeutet R⁷;
W* bedeutet COOH oder COOY;
Y bedeutet durch m Reste aus der Gruppe bestehend aus Fluor, Chlor, Brom, Cyano, Hydroxy und COO-(C₁-C₄)-Alkyl substituiertes (C₁-C₆)-Alkyl, das durch n Sauerstoffatome unterbrochen ist;
X², X⁴ und X⁶ bedeuten unabhängig voneinander jeweils Wasserstoff, Fluor, oder Chlor,
oder jeweils durch m Reste aus der Gruppe bestehend aus Fluor, Chlor, Cyano und (C₁-C₄)-Alkoxy substituiertes (C₁-C₄)-Alkyl oder (C₁-C₄)-Alkoxy;
X³ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
X⁵ bedeutet Wasserstoff, Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy;
m bedeutet 0, 1, 2 oder 3;
n bedeutet 0, 1 oder 2.

14. Verwendung von 3-Phenylisoxazolinderivaten nach Anspruch 13, worin
X⁵ bedeutet Fluor, Chlor, Brom, Cyano,
oder jeweils durch m Reste aus der Gruppe bestehend aus Fluor und Chlor substituiertes (C₁-C₆)-Alkyl,
oder durch m Reste aus der Gruppe bestehend aus Fluor und Chlor substituiertes (C₁-C₆)-Alkoxy.

15. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 4 oder mindestens einer Verbindung der Formel (la) gemäß einem der Ansprüche 11 bis 14 oder eines herbiziden Mittels nach einem der Ansprüche 5 bis 10 auf die Pflanzen oder auf den Ort des unerwünschten Pflanzenwachstums appliziert.

16. Verwendung von herbiziden Mitteln nach einem der Ansprüche 5 bis 10 zur Bekämpfung unerwünschter Pflanzen.

17. Verwendung nach einem der Ansprüche 11 bis 14 oder 16, **dadurch gekennzeichnet, daß** die Verbindungen der Formel (I) zur Bekämpfung unerwünschter Pflanzen in Kulturen von Nutzpflanzen eingesetzt werden.

18. Verwendung nach Anspruch 17, **dadurch gekennzeichnet, daß** die Nutzpflanzen transgene Nutzpflanzen sind.

## Claims

1. 3-Phenylisoxazoline derivatives or salts thereof, of the formula (I) 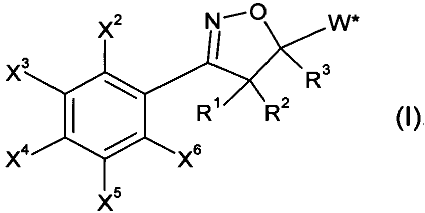 in which
R¹ and R² are each independently hydrogen, fluorine, chlorine, bromine, iodine, cyano, or (C₁-C₄)-alkyl or (C₁-C₄)-alkoxy each substituted by m radicals from the group consisting of fluorine, chlorine, bromine, iodine and cyano,
or
R¹ and R² together with the carbon atom to which they are bonded form a saturated or partly or fully unsaturated three-, four- or five-membered ring formed from q carbon atoms and p oxygen atoms;
R³ is (C₁-C₄)-alkoxy-(C₁-C₄)-alkyl, vinyl, (C₂-C₄)-alkynyl, halo-(C₁-C₄)-alkyl or halo-(C₂-C₄)-alkenyl;
R⁵ is (C₁-C₆)-alkyl or (C₃-C₆)-cycloalkyl each substituted by m radicals from the group consisting of fluorine, chlorine, bromine, iodine, cyano and hydroxyl;
R⁶ is hydrogen or R⁵;
R⁷ is hydrogen or (C₁-C₆)-alkyl, (C₃-C₆)-cycloalkyl, (C₂-C₄)-alkenyl or (C₂-C₄)-alkynyl each substituted by m radicals from the group consisting of fluorine, chlorine, bromine, cyano and (C₁-C₂)-alkoxy;
R⁸ is R⁷;
W* is COOH, COOY, CN or CHO;
Y is (C₁-C₈)-alkyl which is substituted by m radicals from the group consisting of fluorine, chlorine, bromine, cyano, hydroxyl and COO-(C₁-C₈)-alkyl and which is interrupted by n heteroatoms from the group consisting of oxygen, sulfur and nitrogen, or
a radical from the group consisting of (cyclohex-2-en-1-one)-3-yl, (propan-1-ol)-3-yl, (2,2-dimethylpropan-1-ol)-3-yl, (methyl 2,2-dimethylpropanoate)-3-yl, (methyl propanoate)-3-yl, (ethyl propanoate)-3-yl, (ethyl butanoate)-3-yl, (ethyl (3R)-4,4,4-trifluorobutanoate)-3-yl, (butan-2-one)-4-yl, (3-methylbutan-2-one)-4-yl, (pent-3-en-2-one)-4-yl,((2S)-dimethyl butanedioate)-2-yl, (dimethyl pentanedioate)-3-yl, (methyl (2R)-2-methylpropanoate)-3-yl, 4-methoxycarbonylbenzyl, 3,5-difluorobenzyl, 3,4-difluorobenzyl, 2,6-difluorobenzyl, 5-methylpyridin-3-ylmethyl, tetrahydrofuran-3-yl and (butan-1-ol)-4-yl;
X², X⁴ and X⁶ are each independently hydrogen, fluorine, chlorine, bromine, iodine, cyano, nitro,
or (C₁-C₄)-alkyl, (C₃-C₅)-cycloalkyl, (C₂-C₄)-alkenyl, (C₂-C₄)-alkynyl, (C₁-C₄)-alkoxy, (C₂-C₄)-alkenyloxy, (C₂-C₄)-alkynyloxy or (C₁-C₄)-alkylcarbonyl each substituted by m radicals from the group consisting of fluorine, chlorine, bromine, iodine, cyano and (C₁-C₄)-alkoxy;
X³ is fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
X⁵ is hydrogen, fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
m is 0, 1, 2, 3, 4 or 5;
n is 0, 1 or 2;
p is 0 or 1;
q is 3, 4 or 5;
with the proviso that
a) X³ and X⁴ are not both substituted or unsubstituted alkoxy,
b) in the compounds in which R³ is methyl and W* is COOH, X⁵ is not hydrogen, and
c) the compounds
methyl 3-(3-cyanophenyl)-5-(isopropoxymethyl)-4,5-dihydro-1,2-oxazole-5-carboxylate,
methyl 5-(butoxymethyl)-3-(3-cyanophenyl)-4,5-dihydro-1,2-oxazole-5-carboxylate, methyl 3-(3-cyanophenyl)-5-(methoxymethyl)-4,5-dihydro-1,2-oxazole-5-carboxylate,
methyl 3-(3-cyanophenyl)-5-(2-methoxyethyl)-4,5-dihydro-1,2-oxazole-5-carboxylate,
methyl 3-(3-cyanophenyl)-5-(trifluoromethyl)-4,5-dihydro-1,2-oxazole-5-carboxylate,
methyl 3-(3-cyanophenyl)-5-methyl-4,5-dihydro-1,2-oxazole-5-carboxylate,
ethyl 3-(3-cyanophenyl)-5-methyl-4,5-dihydro-1,2-oxazole-5-carboxylate,
methyl 5-methyl-3-(3-nitrophenyl)-4,5-dihydro-1,2-oxazole-5-carboxylate,
methyl 3-(3,5-di-tert-butyl-4-hydroxyphenyl)-5-methyl-4,5-dihydro-1,2-oxazole-5-carboxylate and ethyl 3-(3-chlorophenyl)-5-methyl-4,5-dihydro-1,2-oxazole-5-carboxylate
are excluded.

2. 3-Phenylisoxazoline derivatives according to Claim 1, in which
R¹ and R² are each hydrogen,
R³ is (C₁-C₄)-alkoxy-(C₁-C₄)-alkyl, vinyl, (C₂-C₄)-alkynyl, halo-(C₁-C₄)-alkyl or halo- (C₂-C₄)-alkenyl;
R⁵ is (C₁-C₆)-alkyl or (C₃-C₆)-cycloalkyl each substituted by m radicals from the group consisting of fluorine, chlorine, bromine, iodine, cyano and hydroxyl;
R⁶ is hydrogen or R⁵;
R⁷ is hydrogen or C₁-C₆)-alkyl, (C₃-C₆)-cycloalkyl, (C₂-C₄)-alkenyl or (C₂-C₄)-alkynyl each substituted by m radicals from the group consisting of fluorine, chlorine, bromine, cyano and (C₁-C₂)-alkoxy;
R⁸ is R⁷;
W* is COOH or COOY;
Y is (C₁-C₆)-alkyl which is substituted by m radicals from the group consisting of fluorine, chlorine, bromine, cyano, hydroxyl and COO-(C₁-C₄)-alkyl and which is interrupted by n oxygen atoms, or
a radical from the group consisting of (cyclohex-2-en-1-one)-3-yl, (propan-1-ol)-3-yl, (2,2-dimethylpropan-1-ol)-3-yl, (methyl 2,2-dimethylpropanoate)-3-yl, (methyl propanoate)-3-yl, (ethyl propanoate)-3-yl, (ethyl butanoate)-3-yl, (ethyl (3R)-4,4,4-trifluorobutanoate)-3-yl, (butan-2-one)-4-yl, (3-methylbutan-2-one)-4-yl, (pent-3-en-2-one)-4-yl,((2S)-dimethyl butanedioate)-2-yl, (dimethyl pentanedioate)-3-yl, (methyl (2R)-2-methylpropanoate)-3-yl, 4-methoxycarbonylbenzyl, 3,5-difluorobenzyl, 3,4-difluorobenzyl, 2,6-difluorobenzyl, 5-methylpyridin-3-ylmethyl, tetrahydrofuran-3-yl and (butan-1-ol)-4-yl;
X², X⁴ and X⁶ are each independently hydrogen, fluorine, chlorine, bromine, iodine, cyano, nitro,
or (C₁-C₄)-alkyl, (C₃-C₅)-cycloalkyl, (C₂-C₄)-alkenyl, (C₂-C₄)-alkynyl, (C₁-C₄)-alkoxy, (C₂-C₄)-alkenyloxy, (C₂-C₄)-alkynyloxy or (C₁-C₄)-alkylcarbonyl each substituted by m radicals from the group consisting of fluorine, chlorine, bromine, iodine, cyano and (C₁-C₄)-alkoxy;
X³ is fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
X⁵ is hydrogen, fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
m is 0, 1, 2, 3, 4 or 5;
n is 0, 1 or 2;
with the proviso that
a) X³ and X⁴ are not both substituted or unsubstituted alkoxy,
b) in the compounds in which R³ is methyl and W* is COOH, X⁵ is not hydrogen, and
c) the compounds
methyl 3-(3-cyanophenyl)-5-(isopropoxymethyl)-4,5-dihydro-1,2-oxazole-5-carboxylate,
methyl 5-(butoxymethyl)-3-(3-cyanophenyl)-4,5-dihydro-1,2-oxazole-5-carboxylate, methyl 3-(3-cyanophenyl)-5-(methoxymethyl)-4,5-dihydro-1,2-oxazole-5-carboxylate,
methyl 3-(3-cyanophenyl)-5-(2-methoxyethyl)-4,5-dihydro-1,2-oxazole-5-carboxylate,
methyl 3-(3-cyanophenyl)-5-(trifluoromethyl)-4,5-dihydro-1,2-oxazole-5-carboxylate,
methyl 3-(3-cyanophenyl)-5-methyl-4,5-dihydro-1,2-oxazole-5-carboxylate,
ethyl 3-(3-cyanophenyl)-5-methyl-4,5-dihydro-1,2-oxazole-5-carboxylate,
methyl 5-methyl-3-(3-nitrophenyl)-4,5-dihydro-1,2-oxazole-5-carboxylate,
methyl 3-(3,5-di-tert-butyl-4-hydroxyphenyl)-5-methyl-4,5-dihydro-1,2-oxazole-5-carboxylate and ethyl 3-(3-chlorophenyl)-5-methyl-4,5-dihydro-1,2-oxazole-5-carboxylate are excluded.

3. 3-Phenylisoxazoline derivatives according to Claim 1 or 2, in which
R¹ and R² are each hydrogen,
R³ is (C₁-C₄)-alkoxy-(C₁-C₄)-alkyl, vinyl, (C₂-C₄)-alkynyl, halo- (C₁-C₄) -alkyl or halo- (C₂-C₄) -alkenyl;
R⁵ is methyl or ethyl;
R⁶ is hydrogen or R⁵;
R⁷ is hydrogen or (C₁-C₆)-alkyl substituted by m radicals from the group consisting of fluorine and chlorine;
R⁸ is R⁷;
W* is COOH or COOY;
Y is (C₁-C₆)-alkyl which is substituted by m radicals from the group consisting of fluorine, chlorine, bromine, cyano, hydroxyl and COO-(C₁-C₄)-alkyl and which is interrupted by n oxygen atoms, or
a radical from the group consisting of (cyclohex-2-en-1-one)-3-yl, (propan-1-ol)-3-yl, (2,2-dimethylpropan-1-ol)-3-yl, (methyl 2,2-dimethylpropanoate)-3-yl, (methyl propanoate)-3-yl, (ethyl propanoate)-3-yl, (ethyl butanoate)-3-yl, (ethyl (3R)-4,4,4-trifluorobutanoate)-3-yl, (butan-2-one)-4-yl, (3-methylbutan-2-one)-4-yl, (pent-3-en-2-one)-4-yl, ((2S)-dimethyl butanedioate)-2-yl, (dimethyl pentanedioate)-3-yl, (methyl (2R)-2-methylpropanoate)-3-yl, 4-methoxycarbonylbenzyl, 3,5-difluorobenzyl, 3,4-difluorobenzyl, 2,6-difluorobenzyl, 5-methylpyridin-3-ylmethyl, tetrahydrofuran-3-yl and (butan-1-ol)-4-yl;
X², X⁴ and X⁶ are each independently hydrogen, fluorine, or chlorine,
or (C₁-C₄)-alkyl or (C₁-C₄)-alkoxy each substituted by m radicals from the group consisting of fluorine, chlorine, cyano and (C₁-C₄)-alkoxy;
X³ is fluorine, chlorine, bromine, cyano,
or (C₁-C₆)-alkyl substituted by m radicals from the group consisting of fluorine and chlorine,
or (C₁-C₆)-alkoxy substituted by m radicals from the group consisting of fluorine and chlorine;
X⁵ is hydrogen, fluorine, chlorine, bromine, cyano,
or (C₁-C₆)-alkyl substituted by m radicals from the group consisting of fluorine and chlorine,
or (C₁-C₆)-alkoxy substituted by m radicals from the group consisting of fluorine and chlorine;
m is 0, 1, 2 or 3;
n is 0, 1 or 2;
with the proviso that
a) X³ and X⁴ are not both substituted or unsubstituted alkoxy,
b) in the compounds in which R³ is methyl and W* is COOH, X⁵ is not hydrogen, and
c) the compounds
methyl 3-(3-cyanophenyl)-5-(isopropoxymethyl)-4,5-dihydro-1,2-oxazole-5-carboxylate,
methyl 5-(butoxymethyl)-3-(3-cyanophenyl)-4,5-dihydro-1,2-oxazole-5-carboxylate, methyl 3-(3-cyanophenyl)-5-(methoxymethyl)-4,5-dihydro-1,2-oxazole-5-carboxylate,
methyl 3-(3-cyanophenyl)-5-(2-methoxyethyl)-4,5-dihydro-1,2-oxazole-5-carboxylate,
methyl 3-(3-cyanophenyl)-5-(trifluoromethyl)-4,5-dihydro-1,2-oxazole-5-carboxylate,
methyl 3-(3-cyanophenyl)-5-methyl-4,5-dihydro-1,2-oxazole-5-carboxylate,
ethyl 3-(3-cyanophenyl)-5-methyl-4,5-dihydro-1,2-oxazole-5-carboxylate,
methyl 5-methyl-3-(3-nitrophenyl)-4,5-dihydro-1,2-oxazole-5-carboxylate,
methyl 3-(3,5-di-tert-butyl-4-hydroxyphenyl)-5-methyl-4,5-dihydro-1,2-oxazole-5-carboxylate and ethyl 3-(3-chlorophenyl)-5-methyl-4,5-dihydro-1,2-oxazole-5-carboxylate are excluded.

4. 3-Phenylisoxazoline derivatives according to Claim 3, in which
X⁵ is fluorine, chlorine, bromine, cyano,
or (C₁-C₆)-alkyl substituted by m radicals from the group consisting of fluorine and chlorine,
or (C₁-C₆)-alkoxy substituted by m radicals from the group consisting of fluorine and chlorine.

5. Herbicidal compositions, **characterized by** a herbicidally active content of at least one compound of the formula (I) 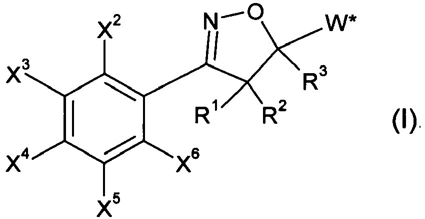 in which
R¹ and R² are each independently hydrogen, fluorine, chlorine, bromine, iodine, cyano, or (C₁-C₄)-alkyl or (C₁-C₄)-alkoxy each substituted by m radicals from the group consisting of fluorine, chlorine, bromine, iodine and cyano,
or
R¹ and R² together with the carbon atom to which they are bonded form a saturated or partly or fully unsaturated three-, four- or five-membered ring formed from q carbon atoms and p oxygen atoms;
R³ is fluorine, chlorine, cyano, (C₁-C₃)-alkylcarbonyloxy or S(O)ₙR⁵,
or (C₁-C₆)-alkyl, (C₃-C₆) -cycloalkyl, (C₂-C₆) -alkenyl or (C₂-C₆) -alkynyl each substituted by m radicals from the group consisting of fluorine, chlorine, bromine, iodine, cyano, (C₁-C₄)-alkoxy and hydroxyl,
or (C₁-C₆) -alkylcarbonyl, (C₂-C₆) -alkenylcarbonyl or (C₃-C₆)-cycloalkylcarbonyl each substituted by m radicals from the group consisting of fluorine, chlorine, bromine, iodine, cyano and (C₁-C₆)-alkoxy;
R⁵ is (C₁-C₆) -alkyl or (C₃-C₆) -cycloalkyl each substituted by m radicals from the group consisting of fluorine, chlorine, bromine, iodine, cyano and hydroxyl;
R⁶ is hydrogen or R⁵;
R⁷ is hydrogen or (C₁-C₆) -alkyl, (C₃-C₆) -cycloalkyl, (C₂-C₄)-alkenyl or (C₂-C₄)-alkynyl each substituted by m radicals from the group consisting of fluorine, chlorine, bromine, cyano and (C₁-C₂)-alkoxy;
R⁸ is R⁷;
W* is COOH, COOY, CN or CHO;
Y is (C₁-C₈)-alkyl which is substituted by m radicals from the group consisting of fluorine, chlorine, bromine, cyano, hydroxyl and COO-(C₁-C₈)-alkyl and which is interrupted by n heteroatoms from the group consisting of oxygen, sulfur and nitrogen, or
a radical from the group consisting of (cyclohex-2-en-1-one)-3-yl, (propan-1-ol)-3-yl, (2,2-dimethylpropan-1-ol)-3-yl, (methyl 2,2-dimethylpropanoate)-3-yl, (methyl propanoate)-3-yl, (ethyl propanoate)-3-yl, (ethyl butanoate)-3-yl, (ethyl (3R)-4,4,4-trifluorobutanoate)-3-yl, (butan-2-one)-4-yl, (3-methylbutan-2-one)-4-yl, (pent-3-en-2-one)-4-yl, ((2S)-dimethyl butanedioate)-2-yl, (dimethyl pentanedioate)-3-yl, (methyl (2R)-2-methylpropanoate)-3-yl, 4-methoxycarbonylbenzyl, 3,5-difluorobenzyl, 3,4-difluorobenzyl, 2,6-difluorobenzyl, 5-methylpyridin-3-ylmethyl, tetrahydrofuran-3-yl and (butan-1-ol)-4-yl;
X², X⁴ and X⁶ are each independently hydrogen, fluorine, chlorine, bromine, iodine, cyano, nitro,
or (C₁-C₄) -alkyl, (C₃-C₅) -cycloalkyl, (C₂-C₄) -alkenyl, (C₂-C₄)-alkynyl, (C₁-C₄) -alkoxy, (C₂-C₄) -alkenyloxy, (C₂-C₄)-alkynyloxy or (C₁-C₄)-alkylcarbonyl each substituted by m radicals from the group consisting of fluorine, chlorine, bromine, iodine, cyano and (C₁-C₄)-alkoxy;
X³ is fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C (R⁶)=NOR⁸, NR⁶COR⁸, NR⁶CONR⁸R⁸, NR⁶CO₂R⁸, NR⁶SO₂R⁸, NR⁶SO₂NR⁶R⁸ OCONR⁶R⁸, OSO₂R⁵, R⁵, S(O)ₙR⁵, SO²NR⁶R⁸, OSO₂NR⁶R⁸, or (C₁-C₆) -alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆) -alkoxy, (C₃-C₆) -cycloalkoxy, (C₂-C₆)-alkenyloxy or (C₂-C₆) -alkynyloxy each substituted by m radicals from the group consisting of fluorine, chlorine, bromine, iodine, cyano and (C₁-C₂)-alkoxy;
X⁵ is hydrogen, fluorine, chlorine, bromine, iodine, hydroxyl, cyano, nitro, SF₅, CONR⁸SO₂R⁵, CONR⁶R⁸, COR⁶, CO₂R⁸,CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸ OCONR⁶R⁸, OSO₂R⁵ R⁵, S(O)ₙR⁵, SO²NR⁶R⁸, OSO₂NR⁶R⁸,
or (C₁-C₆) -alkyl, (C₃-C₅) -cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆) -alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
m is 0, 1, 2, 3, 4 or 5;
n is 0, 1 or 2;
p is 0 or 1;
q is 3, 4 or 5;
with the proviso that
a) X³ and X⁴ are not both substituted or unsubstituted alkoxy,
b) in the compounds in which R³ is methyl and W* is COOH, X⁵ is not hydrogen, and
c) the compounds
methyl 3-(3-cyanophenyl)-5-(isopropoxymethyl)-4,5-dihydro-1,2-oxazole-5-carboxylate,
methyl 5-(butoxymethyl)-3-(3-cyanophenyl)-4,5-dihydro-1,2-oxazole-5-carboxylate, methyl 3-(3-cyanophenyl)-5-(methoxymethyl)-4,5-dihydro-1,2-oxazole-5-carboxylate,
methyl 3-(3-cyanophenyl)-5-(2-methoxyethyl)-4,5-dihydro-1,2-oxazole-5-carboxylate, 3-(3-cyanophenyl)-5-(trifluoromethyl)-4,5-dihydro-1,2-oxazole-5-carboxylic acid,
methyl 3-(3-cyanophenyl)-5-methyl-4,5-dihydro-1,2-oxazole-5-carboxylate,
ethyl 3-(3-cyanophenyl)-5-methyl-4,5-dihydro-1,2-oxazole-5-carboxylate,
methyl 5-methyl-3-(3-nitrophenyl)-4,5-dihydro-1,2-oxazole-5-carboxylate,
methyl 3-(3,5-di-tert-butyl-4-hydroxyphenyl)-5-methyl-4,5-dihydro-1,2-oxazole-5-carboxylate and ethyl 3-(3-chlorophenyl)-5-methyl-4,5-dihydro-1,2-oxazole-5-carboxylate
are excluded.

6. Herbicidal compositions according to Claim 5 in a mixture with formulation auxiliaries.

7. Herbicidal compositions according to Claim 5 or 6, comprising at least one further pesticidally active substance from the group of insecticides, acaricides, herbicides, fungicides, safeners and growth regulators.

8. Herbicidal compositions according to Claim 7, comprising a safener.

9. Herbicidal compositions according to Claim 8, in which the safener is selected from the group consisting of mefenpyr-diethyl, cyprosulfamide, isoxadifen-ethyl, cloquintocet-mexyl, benoxacor and dichlormid.

10. Herbicidal compositions according to any of Claims 7 to 9, comprising a further herbicide.

11. Use of 3-phenylisoxazoline derivatives of the formula (Ia) 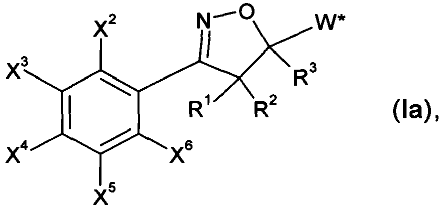 in which
R¹ and R² are each independently hydrogen, fluorine, chlorine, bromine, iodine, cyano, or (C₁-C₄) -alkyl or (C₁-C₄)-alkoxy each substituted by m radicals from the group consisting of fluorine, chlorine, bromine, iodine and cyano,
or
R¹ and R² together with the carbon atom to which they are bonded form a saturated or partly or fully unsaturated three-, four- or five-membered ring formed from q carbon atoms and p oxygen atoms;
R³ is fluorine, chlorine, cyano, (C₁-C₃)-alkylcarbonyloxy or S(O)ₙR⁵,
or (C₁-C₆) -alkyl, (C₃-C₆)-cycloalkyl, (C₂-C₆)-alkenyl or (C₂-C₆) -alkynyl each substituted by m radicals from the group consisting of fluorine, chlorine, bromine, iodine, cyano, (C₁-C₄)-alkoxy and hydroxyl,
or (C₁-C₆)-alkylcarbonyl, (C₂-C₆)-alkenylcarbonyl or (C₃-C₆) -cycloalkylcarbonyl each substituted by m radicals from the group consisting of fluorine, chlorine, bromine, iodine, cyano and (C₁-C₆)-alkoxy;
R⁵ is (C₁-C₆)-alkyl or (C₃-C₆)-cycloalkyl each substituted by m radicals from the group consisting of fluorine, chlorine, bromine, iodine, cyano and hydroxyl;
R⁶ is hydrogen or R⁵;
R⁷ is hydrogen or (C₁-C₆)-alkyl, (C₃-C₆)-cycloalkyl, (C₂-C₄)-alkenyl or (C₂-C₄)-alkynyl each substituted by m radicals from the group consisting of fluorine, chlorine, bromine, cyano and (C₁-C₂)-alkoxy;
R⁸ is R⁷;
W* is COOH, COOY, CN or CHO;
Y is (C₁-C₈)-alkyl which is substituted by m radicals from the group consisting of fluorine, chlorine, bromine, cyano, hydroxyl and COO-(C₁-C₈)-alkyl and which is interrupted by n heteroatoms from the group consisting of oxygen, sulfur and nitrogen, or a radical from the group consisting of (cyclohex-2-en-1-one)-3-yl, (propan-1-ol)-3-yl, (2,2-dimethylpropan-1-ol)-3-yl, (methyl 2,2-dimethylpropanoate)-3-yl, (methyl propanoate)-3-yl, (ethyl propanoate)-3-yl, (ethyl butanoate)-3-yl, (ethyl (3R)-4,4,4-trifluorobutanoate)-3-yl, (butan-2-one)-4-yl, (3-methylbutan-2-one)-4-yl, (pent-3-en-2-one)-4-yl, ((2S)-dimethyl butanedioate)-2-yl, (dimethyl pentanedioate)-3-yl, (methyl (2R)-2-methylpropanoate)-3-yl, 4-methoxycarbonylbenzyl, 3,5-difluorobenzyl, 3,4-difluorobenzyl, 2,6-difluorobenzyl, 5-methylpyridin-3-ylmethyl, tetrahydrofuran-3-yl and (butan-1-ol)-4-yl;
X², X⁴ and X⁶ are each independently hydrogen, fluorine, chlorine, bromine, iodine, cyano, nitro,
or (C₁-C₄) -alkyl, (C₃-C₅) -cycloalkyl, (C₂-C₄) -alkenyl, (C₂-C₄) -alkynyl, (C₁-C₄)-alkoxy, (C₂-C₄)-alkenyloxy, (C₂-C₄)-alkynyloxy or (C₁-C₄)-alkylcarbonyl each substituted by m radicals from the group consisting of fluorine, chlorine, bromine iodine, cyano and (C₁-C₄)-alkoxy;
X³ is fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸ OCONR⁶R⁸, OSO₂R⁵, R⁵, S(O)ₙR⁵, SO²NR⁶R⁸, OSO₂NR⁶R⁸,
or (C₁-C₆)-alkyl, (C₃-C₅)-cycloalkyl, (C₂-C₆)-alkenyl, (C₂-C₆)-alkynyl each substituted by m radicals from the group consisting of fluorine, chlorine, bromine, iodine, hydroxyl and cyano,
or (C₁-C₆)-alkoxy, (C₃-C₆)-cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
X⁵ is hydrogen, fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
or (C₁-C₆) -alkyl, (C₃-C₅) -cycloalkyl, (C₂-C₆) -alkenyl, (C₂-C₆)-alkynyl each substituted by m radicals from the group consisting of fluorine, chlorine, bromine, iodine, hydroxyl and cyano,
or (C₁-C₆) -alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
m is 0, 1, 2, 3, 4 or 5;
n is 0, 1 or 2;
p is 0 or 1;
q is 3, 4 or 5,
for controlling unwanted plants.

12. Use of 3-phenylisoxazoline derivatives according to Claim 11, in which
R¹ and R² are each hydrogen,
R³ is (C₁-C₆)-alkyl, (C₁-C₄)-alkoxy-(C₁-C₄)-alkyl, (C₂-C₆)-alkenyl, (C₂-C₆)-alkynyl, halo- (C₁-C₆)-alkyl or halo-(C₂-C₆)-alkenyl;
R⁵ is (C₁-C₆)-alkyl or (C₃-C₆)-cycloalkyl each substituted by m radicals from the group consisting of fluorine, chlorine, bromine, iodine, cyano and hydroxyl;
R⁶ is hydrogen or R⁵;
R⁷ is hydrogen or (C₁-C₆)-alkyl, (C₃-C₆)-cycloalkyl, (C₂-C₄)-alkenyl or (C₂-C₄) -alkynyl each substituted by m radicals from the group consisting of fluorine, chlorine, bromine, cyano and (C₁-C₂)-alkoxy;
R⁸ is R⁷;
W* is COOH or COOY;
Y is (C₁-C₆)-alkyl which is substituted by m radicals from the group consisting of fluorine, chlorine, bromine, cyano, hydroxyl and COO-(C₁-C₄)-alkyl and which is interrupted by n oxygen atoms, or
a radical from the group consisting of (cyclohex-2-en-1-one)-3-yl, (propan-1-ol)-3-yl, (2,2-dimethylpropan-1-ol)-3-yl, (methyl 2,2-dimethylpropanoate)-3-yl, (methyl propanoate)-3-yl, (ethyl propanoate)-3-yl, (ethyl butanoate)-3-yl, (ethyl (3R)-4,4,4-trifluorobutanoate)-3-yl, (butan-2-one)-4-yl, (3-methylbutan-2-one)-4-yl, (pent-3-en-2-one)-4-yl, ((2S)-dimethyl butanedioate)-2-yl, (dimethyl pentanedioate)-3-yl, (methyl (2R)-2-methylpropanoate)-3-yl, 4-methoxycarbonylbenzyl, 3,5-difluorobenzyl, 3,4-difluorobenzyl, 2,6-difluorobenzyl, 5-methylpyridin-3-ylmethyl, tetrahydrofuran-3-yl and (butan-1-ol)-4-yl;
X², X⁴ and X⁶ are each independently hydrogen, fluorine, chlorine, bromine, iodine, cyano, nitro, or (C₁-C₄)-alkyl, (C₃-C₅)-cycloalkyl, (C₂-C₄)-alkenyl, (C₂-C₄) -alkynyl, (C₁-C₄) -alkoxy, (C₂-C₄) -alkenyloxy, (C₂-C₄)-alkynyloxy or (C₁-C₄)-alkylcarbonyl each substituted by m radicals from the group consisting of fluorine, chlorine, bromine, iodine, cyano and (C₁-C₄)-alkoxy;
X³ is fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸ OCONR⁶R⁸, OSO₂R⁵, R⁵, S(O)ₙR⁵, SO₂NR⁶R⁸, OSO₂NR⁶R⁸,
or (C₁-C₆) -alkyl, (C₃-C₅) -cycloalkyl, (C₂-C₆) -alkenyl, (C₂-C₆)-alkynyl each substituted by m radicals from the group consisting of fluorine, chlorine, bromine, iodine, hydroxyl and cyano,
or (C₁-C₆) -alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
X⁵ is hydrogen, fluorine, chlorine, bromine, iodine, hydroxyl, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
or (C₁-C₆) -alkyl, (C₃-C₅) -cycloalkyl, (C₂-C₆) -alkenyl, (C₂-C₆) -alkynyl each substituted by m radicals from the group consisting of fluorine, chlorine, bromine, iodine, hydroxyl and cyano,
or (C₁-C₆) -alkoxy, (C₃-C₆) -cycloalkoxy, (C₂-C₆)-alkenyloxy or (C₂-C₆)-alkynyloxy each substituted by m radicals from the group consisting of fluorine, chlorine, bromine, iodine, cyano and (C₁-C₂)-alkoxy;
m is 0, 1, 2, 3, 4 or 5;
n is 0, 1 or 2.

13. Use of 3-phenylisoxazoline derivatives according to Claim 11 or 12, in which
R¹ and R² are each hydrogen,
R³ is (C₁-C₄) -alkyl, (C₁-C₄) -alkoxy- (C₁-C₄) -alkyl, vinyl, (C₂-C₄) -alkynyl, halo- (C₁-C₄) -alkyl or halo- (C₂-C₄)-alkenyl;
R⁵ is methyl or ethyl,
R⁶ is hydrogen or R⁵;
R⁷ is hydrogen or (C₁-C₆)-alkyl substituted by m radicals from the group consisting of fluorine and chlorine;
R⁸ is R⁷;
W* is COOH or COOY;
Y is (C₁-C₆)-alkyl which is substituted by m radicals from the group consisting of fluorine, chlorine, bromine, cyano, hydroxyl and COO-(C₁-C₄)-alkyl and which is interrupted by n oxygen atoms;
X², X⁴ and X⁶ are each independently hydrogen, fluorine, or chlorine,
or (C₁-C₄)-alkyl or (C₁-C₄)-alkoxy each substituted by m radicals from the group consisting of fluorine, chlorine, cyano and C₁-C₄)-alkoxy;
X³ is fluorine, chlorine, bromine, cyano,
or (C₁-C₆)-alkyl substituted by m radicals from the group consisting of fluorine and chlorine,
or (C₁-C₆)-alkoxy substituted by m radicals from the group consisting of fluorine and chlorine;
X⁵ is hydrogen, fluorine, chlorine, bromine, cyano,
or (C₁-C₆)-alkyl substituted by m radicals from the group consisting of fluorine and chlorine,
or (C₁-C₆)-alkoxy substituted by m radicals from the group consisting of fluorine and chlorine;
m is 0, 1, 2 or 3;
n is 0, 1 or 2.

14. Use of 3-phenylisoxazoline derivatives according to Claim 13, in which
X⁵ is fluorine, chlorine, bromine, cyano,
or (C₁-C₆)-alkyl substituted by m radicals from the group consisting of fluorine and chlorine,
or (C₁-C₆)-alkoxy substituted by m radicals from the group consisting of fluorine and chlorine.

15. Method for controlling unwanted plants, **characterized in that** an effective amount of at least one compound of the formula (I) according to any of Claims 1 to 4 or of at least one compound of the formula (Ia) according to any of Claims 11 to 14 or of a herbicidal composition according to any of Claims 5 to 10 is applied to the plants or the site of the unwanted vegetation.

16. Use of herbicidal compositions according to any of Claims 5 to 10 for controlling unwanted plants.

17. Use according to any of Claims 11 to 14 or 16, **characterized in that** the compounds of the formula (I) are used for controlling unwanted plants in crops of useful plants.

18. Use according to Claim 17, **characterized in that** the useful plants are transgenic useful plants.

## Revendications

1. Dérivés de 3-phénylisoxazoline ou leurs sels de formule (I) 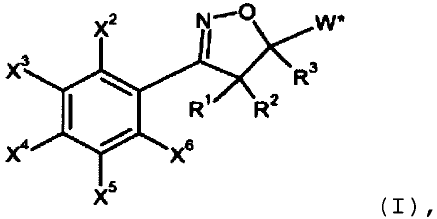
R¹ et R² signifiant indépendamment l'un de l'autre à chaque fois hydrogène, fluor, chlore, brome, iode, cyano ou (C₁₋₄)-alkyle ou (C₁₋₄)-alcoxyle substitué par à chaque fois m radicaux choisis dans le groupe constitué par fluor, chlore, brome, iode et cyano,
ou
R¹ et R² formant conjointement avec l'atome de carbone auquel ils sont liés, un cycle saturé, partiellement insaturé ou totalement insaturé à trois, quatre ou cinq chaînons, qui est constitué de q atomes de carbone et p atomes d'oxygène ;
R³ signifiant (C₁₋₄)-alcoxyl- (C₁₋₄)-alkyle, vinyle, (C₂₋₄)-alcynyle, (C₁₋₄)-halogénoalkyle, ou (C₂₋₄)-halogénoalcényle ;
R⁵ signifiant (C₁₋₆)-alkyle ou (C₃₋₆)-cycloalkyle substitué par à chaque fois m radicaux choisis dans le groupe constitué par fluor, chlore, brome, iode, cyano et hydroxyle ;
R⁶ signifiant hydrogène ou R⁵ ;
R⁷ signifiant hydrogène ou (C₁₋₆)-alkyle, (C₃₋₆)-cycloalkyle, (C₂₋₄)-alcényle ou (C₂₋₄)-alcynyle substitué par à chaque fois m radicaux choisis dans le groupe constitué par fluor, chlore, brome, cyano et (C₁₋₂)-alcoxyle ;
R⁸ signifiant R⁷ ;
W* signifiant COOH, COOY, CN ou CHO ;
Y signifiant (C₁₋₈)-alkyle substitué par m radicaux choisis dans le groupe constitué par fluor, chlore, brome, cyano, hydroxyle et COO-(C₁₋₈)-alkyle, qui est interrompu par n hétéroatomes choisis dans le groupe constitué par oxygène, soufre et azote ou un radical choisi dans le groupe constitué par cyclohex-2-én-1-on-3-yle, propan-1-ol-3-yle, 2,2-diméthylpropan-1-ol-3-yle, (2,2-diméthylpropanoate de méthyl)-3-yle, (propanoate de méthyl)-3-yle, éthylpropanoat-3-yle, (butanoate d'éthyl)-3-yle, ((3*R*)-4,4,4,-trifluorobutanoate d'éthyl)-3-yle, butan-2-on-4-yle, 3-méthylbutan-2-on-4-yle, pent-3-én-2-on-4-yle, ((2*S*)-butanedioate de diméthyl)-2-yle, (pentanedioate de diméthyl)-3-yle, ((2*R*)-2-méthylpropanoate de méthyl)-3-yle, 4-méthoxycarbonylbenzyle, 3,5-difluorobenzyle, 3,4-difluorobenzyle,
2,6-difluorobenzyle, 5-méthylpyridin-3-ylméthyle, tétrahydrofurann-3-yle et butan-1-ol-4-yle ;
X², X⁴ et X⁶ signifiant indépendamment l'un de l'autre à chaque fois hydrogène, fluor, chlore, brome, iode, cyano, nitro
ou (C₁₋₄)-alkyle, (C₃₋₅)-cycloalkyle, (C₂₋₄)-alcényle, (C₂₋₄)-alcynyle, (C₁₋₄)-alcoxyle, (C₂₋₄)-alcényloxyle, (C₂₋₄)-alcynyloxyle ou (C₁₋₄)-alkylcarbonyle à chaque fois substitué par m radicaux choisis dans le groupe constitué par fluor, chlore, brome, iode, cyano et (C₁₋₄)-alcoxyle ;
X³ signifiant fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶) =NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
X⁵ signifiant hydrogène, fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
m signifiant 0, 1, 2, 3, 4 ou 5 ;
n signifiant 0, 1 ou 2 ;
p signifiant 0 ou 1 ;
q signifiant 3, 4 ou 5 ;
étant entendu que
a) X³ et X⁴ ne signifient pas en même temps alcoxyle substitué ou non substitué,
b) dans les composés, dans lesquels R³ représente méthyle et W* représente COOH, X⁵ ne signifie pas hydrogène et
c) les composés 3-(3-cyanophényl)-5-(isopropoxyméthyl)-4,5-dihydro-1,2-oxazole-5-carboxylate de méthyle, 5-(butoxyméthyl)-3-(3-cyanophényl)-4,5-dihydro-1,2-oxazole-5-carboxylate de méthyle, 3-(3-cyanophényl)-5-(méthoxyméthyl)-4,5-dihydro-1,2-oxazole-5-carboxylate de méthyle, 3-(3-cyanophényl)-5-(2-méthoxyéthyl)-4,5-dihydro-1,2-oxazole-5-carboxylate de méthyle, ester méthylique de l'acide 3-(3-cyanophényl)-5-(trifluorométhyl)-4,5-dihydro-1,2-oxazole-5-carboxylique, 3-(3-cyanophényl)-5-méthyl-4,5-dihydro-1,2-oxazole-5-carboxylate de méthyle, 3-(3-cyanophényl)-5-méthyl-4,5-dihydro-1,2-oxazole-5-carboxylate d'éthyle, 5-méthyl-3-(3-nitrophényl)-4,5-dihydro-1,2-oxazole-5-carboxylate de méthyle, 3-(3,5-di-tert-butyl-4-hydroxyphényl)-5-méthyl-4,5-dihydro-1,2-oxazole-5-carboxylate de méthyle et 3-(3-chlorophényl)-5-méthyl-4,5-dihydro-1,2-oxazole-5-carboxylate d'éthyle sont exclus.

2. Dérivés de 3-phénylisoxazoline selon la revendication 1,
R¹ et R² signifiant à chaque fois hydrogène,
R³ signifiant (C₁₋₄)-alcoxy)-(C₁₋₄)-alkyle, vinyle, (C₂₋₄)-alcynyle, (C₁₋₄)-halogénoalkyle, ou (C₂₋₄)-halogénoalcényle ;
R⁵ signifiant (C₁₋₆)-alkyle ou (C₃₋₆)-cycloalkyle substitué par à chaque fois m radicaux choisis dans le groupe constitué par fluor, chlore, brome, iode, cyano et hydroxyle ;
R⁶ signifiant hydrogène ou R⁵ ;
R⁷ signifiant hydrogène ou (C₁₋₆)-alkyle, (C₃₋₆)-cycloalkyle, (C₂₋₄)-alcényle ou (C₂₋₄)-alcynyle substitué par à chaque fois m radicaux choisis dans le groupe constitué par fluor, chlore, brome, cyano et (C₁₋₂)-alcoxyle ;
R⁸ signifiant R⁷ ;
W* signifiant COOH ou COOY ;
Y signifiant (C₁₋₆)-alkyle substitué par m radicaux choisis dans le groupe constitué par fluor, chlore, brome, cyano, hydroxyle et COO-(C₁-₄)-alkyle, qui est interrompu par n atomes d'oxygène ou
un radical choisi dans le groupe constitué par cyclohex-2-én-1-on-3-yle, propan-1-ol-3-yle, 2,2-diméthylpropan-1-ol-3-yle, (2,2-diméthylpropanoate de méthyl)-3-yle, (propanoate de méthyl)-3-yle, éthylpropanoat-3-yle, (butanoate d'éthyl)-3-yle, ((3R)-4,4,4,-trifluorobutanoate d'éthyl)-3-yle, butan-2-on-4-yle, 3-méthylbutan-2-on-4-yle, pent-3-én-2-on-4-yle, ((2S)-butanedioate de diméthyl)-2-yle, (pentanedioate de diméthyl)-3-yle, ((2R)-2-méthylpropanoate de méthyl)-3-yle, 4-méthoxycarbonylbenzyle, 3,5-difluorobenzyle, 3,4-difluorobenzyle,
2,6-difluorobenzyle, 5-méthylpyridin-3-ylméthyle, tétrahydrofurann-3-yle et butan-1-ol-4-yle ;
X², X⁴ et X⁶ signifiant indépendamment l'un de l'autre à chaque fois hydrogène, fluor, chlore, brome, iode, cyano, nitro
ou (C₁₋₄)-alkyle, (C₃₋₅)-cycloalkyle, (C₂₋₄)-alcényle, (C₂₋₄) -alcynyle, (C₁₋₄)-alcoxyle, (C₂₋₄)-alcényloxyle, (C₂₋₄)-alcynyloxyle ou (C₁₋₄)-alkylcarbonyle à chaque fois substitué par m radicaux choisis dans le groupe constitué par fluor, chlore, brome, iode, cyano et (C₁₋₄)-alcoxyle ;
X³ signifiant fluor, chlore, brome, iode, hydroxyle, cyano, nitro, SF₅, CONR⁸SO₂R⁸, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
X⁵ signifiant hydrogène, fluor, chlore, brome, iode, hydroxyle, cyano, nitro, SF₅, CONR⁸SO₂R⁵, CONR⁶R⁸, COR⁶, CO₂R⁸,CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
m signifiant 0, 1, 2, 3, 4 ou 5 ;
n signifiant 0, 1 ou 2 ;
étant entendu que
a) X³ et X⁴ ne signifient pas en même temps alcoxyle substitué ou non substitué,
b) dans les composés, dans lesquels R³ représente méthyle et W* représente COOH, X⁵ ne signifie pas hydrogène et
c) les composés 3-(3-cyanophényl)-5-(isopropoxyméthyl)-4,5-dihydro-1,2-oxazole-5-carboxylate de méthyle, 5-(butoxyméthyl)-3-(3-cyanophényl)-4,5-dihydro-1,2-oxazole-5-carboxylate de méthyle, 3-(3-cyanophényl)-5-(méthoxyméthyl)-4,5-dihydro-1,2-oxazole-5-carboxylate de méthyle, 3-(3-cyanophényl)-5-(2-méthoxyéthyl)-4,5-dihydro-1,2-oxazole-5-carboxylate de méthyle, ester méthylique de l'acide 3-(3-cyanophényl)-5-(trifluorométhyl)-4,5-dihydro-1,2-oxazole-5-carboxylique, 3-(3-cyanophényl)-5-méthyl-4,5-dihydro-1,2-oxazole-5-carboxylate de méthyle, 3-(3-cyanophényl)-5-méthyl-4,5-dihydro-1,2-oxazole-5-carboxylate d'éthyle, 5-méthyl-3-(3-nitrophényl)-4,5-dihydro-1,2-oxazole-5-carboxylate de méthyle, 3-(3,5-di-tert-butyl-4-hydroxyphényl)-5-méthyl-4,5-dihydro-1,2-oxazole-5-carboxylate de méthyle et 3-(3-chlorophényl)-5-méthyl-4,5-dihydro-1,2-oxazole-5-carboxylate d'éthyle sont exclus.

3. Dérivés de 3-phénylisoxazoline selon la revendication 1 ou 2,
R¹ et R² signifiant à chaque fois hydrogène ;
R³ signifiant (C₁₋₄)-alcoxy-(C₁₋₄)-alkyle, vinyle, (C₂₋₄)-alcynyle, (C₁₋₄)-halogénoalkyle, ou (C₂₋₄)-halogénoalcényle ;
R⁵ signifiant méthyle ou éthyle ;
R⁶ signifiant hydrogène ou R⁵ ;
R⁷ signifiant hydrogène ou (C₁₋₆)-alkyle substitué par à chaque fois m radicaux choisis dans le groupe constitué par fluor et chlore ;
R⁸ signifiant R⁷ ;
W* signifiant COOH ou COOY ;
Y signifiant (C₁₋₆)-alkyle substitué par m radicaux choisis dans le groupe constitué par fluor, chlore, brome, cyano, hydroxyle et COO-(C₁-₄)-alkyle, qui est interrompu par n atomes d'oxygène ou
un radical choisi dans le groupe constitué par cyclohex-2-én-1-on-3-yle, propan-1-ol-3-yle, 2,2-diméthylpropan-1-ol-3-yle, (2,2-diméthylpropanoate de méthyl)-3-yle, (propanoate de méthyl)-3-yle, éthylpropanoat-3-yle, (butanoate d'éthyl)-3-yle, ((3R)-4,4,4,-trifluorobutanoate d'éthyl)-3-yle, butan-2-on-4-yle, 3-méthylbutan-2-on-4-yle, pent-3-én-2-on-4-yle, ((2S)-butanedioate de diméthyl)-2-yle, (pentanedioate de diméthyl)-3-yle, ((2*R*)-2-méthylpropanoate de méthyl)-3-yle, 4-méthoxycarbonylbenzyle, 3,5-difluorobenzyle, 3,4-difluorobenzyle,
2,6-difluorobenzyle, 5-méthylpyridin-3-ylméthyle, tétrahydrofurann-3-yle et butan-1-ol-4-yle ;
X², X⁴ et X⁶ signifiant indépendamment l'un de l'autre à chaque fois hydrogène, fluor ou chlore ou (C₁₋₄)-alkyle ou (C₁₋₄)-alcoxyle à chaque fois substitué par m radicaux choisis dans le groupe constitué par fluor, chlore, cyano et (C₁₋₄)-alcoxyle ;
X³ signifiant fluor, chlore, brome, cyano,
ou (C₁₋₆)-alkyle à chaque fois substitué par m radicaux choisis dans le groupe constitué par fluor et chlore
ou (C₁₋₆)-alcoxyle à chaque fois substitué par m radicaux choisis dans le groupe constitué par fluor et chlore ;
X⁵ signifiant hydrogène, fluor, chlore, brome, cyano
ou (C₁₋₆)-alkyle à chaque fois substitué par m radicaux choisis dans le groupe constitué par fluor et chlore
ou (C₁₋₆)-alcoxyle à chaque fois substitué par m radicaux choisis dans le groupe constitué par fluor et chlore ;
m signifiant 0, 1, 2 ou 3 ;
n signifiant 0, 1 ou 2 ;
étant entendu que
a) X³ et X⁴ ne signifient pas en même temps alcoxyle substitué ou non substitué,
b) dans les composés, dans lesquels R³ représente méthyle et W* représente COOH, X⁵ ne signifie pas hydrogène et
c) les composés 3-(3-cyanophényl)-5-(isopropoxyméthyl)-4,5-dihydro-1,2-oxazole-5-carboxylate de méthyle, 5-(butoxyméthyl)-3-(3-cyanophényl)-4,5-dihydro-1,2-oxazole-5-carboxylate de méthyle, 3-(3-cyanophényl)-5-(méthoxyméthyl)-4,5-dihydro-1,2-oxazole-5-carboxylate de méthyle, 3-(3-cyanophényl)-5-(2-méthoxyéthyl)-4,5-dihydro-1,2-oxazole-5-carboxylate de méthyle, ester méthylique de l'acide 3-(3-cyanophényl)-5-(trifluorométhyl)-4,5-dihydro-1,2-oxazole-5-carboxylique, 3-(3-cyanophényl)-5-méthyl-4,5-dihydro-1,2-oxazole-5-carboxylate de méthyle, 3-(3-cyanophényl)-5-méthyl-4,5-dihydro-1,2-oxazole-5-carboxylate d'éthyle, 5-méthyl-3-(3-nitrophényl)-4,5-dihydro-1,2-oxazole-5-carboxylate de méthyle, 3-(3,5-di-tert-butyl-4-hydroxyphényl)-5-méthyl-4,5-dihydro-1,2-oxazole-5-carboxylate de méthyle et 3-(3-chlorophényl)-5-méthyl-4,5-dihydro-1,2-oxazole-5-carboxylate d'éthyle sont exclus.

4. Dérivés de 3-phénylisoxazoline selon la revendication 3,
X⁵ signifiant fluor, chlore, brome, cyano ou (C₁₋₆)-alkyle à chaque fois substitué par m radicaux choisis dans le groupe constitué par fluor et chlore
ou (C₁₋₆)-alcoxyle substitué par m radicaux choisis dans le groupe constitué par fluor et chlore.

5. Agent herbicide, **caractérisé par** une teneur active sur le plan herbicide en au moins un composé de formule (I) 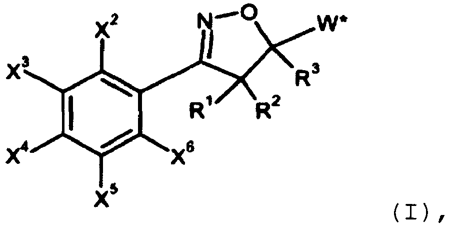
R¹ et R² signifiant indépendamment l'un de l'autre à chaque fois hydrogène, fluor, chlore, brome, iode, cyano ou (C₁₋₄)-alkyle ou (C₁₋₄)-alcoxyle substitué par à chaque fois m radicaux choisis dans le groupe constitué par fluor, chlore, brome, iode et cyano,
ou
R¹ et R² formant conjointement avec l'atome de carbone auquel ils sont liés, un cycle saturé, partiellement insaturé ou totalement insaturé à trois, quatre ou cinq chaînons, qui est constitué de q atomes de carbone et p atomes d'oxygène ;
R³ signifiant fluor, chlore, cyano, (C₁₋₃) - alkylcarbonyloxyle ou S(O)ₙR⁵,
ou (C₁₋₆)-alkyle, (C₃₋₆)-cycloalkyle, (C₂₋₆)-alcényle, (C₂₋₆)-alcynyle substitué par à chaque fois m radicaux choisis dans le groupe constitué par fluor, chlore, brome, iode, cyano, (C₁₋₄)-alcoxyle et hydroxyle
ou (C₁₋₆)-alkylcarbonyle, (C₂₋₆)-alcénylcarbonyle ou (C₃₋₆)-cycloalkylcarbonyle à chaque fois substitué par m radicaux choisis dans le groupe constitué par fluor, chlore, brome, iode, cyano et (C₁₋₆)-alcoxyle ;
R⁵ signifiant (C₁₋₆)-alkyle ou (C₃₋₆)-cycloalkyle substitué par à chaque fois m radicaux choisis dans le groupe constitué par fluor, chlore, brome, iode, cyano et hydroxyle ;
R⁶ signifiant hydrogène ou R⁵ ;
R⁷ signifiant hydrogène ou (C₁₋₆)-alkyle, (C₃₋₆)-cycloalkyle, (C₂₋₄)-alcényle ou (C₂₋₄)-alcynyle substitué par à chaque fois m radicaux choisis dans le groupe constitué par fluor, chlore, brome, cyano et (C₁₋₂)-alcoxyle ;
R⁸ signifiant R⁷ ;
W* signifiant COOH, COOY, CN ou CHO ;
Y signifiant (C₁₋₈)-alkyle substitué par m radicaux choisis dans le groupe constitué par fluor, chlore, brome, cyano, hydroxyle et COO-(C₁-₈)-alkyle, qui est interrompu par n hétéroatomes choisis dans le groupe constitué par oxygène, soufre et azote ou un radical choisi dans le groupe constitué par cyclohex-2-én-1-on-3-yle, propan-1-ol-3-yle, 2,2-diméthylpropan-1-ol-3-yle, (2,2-diméthylpropanoate de méthyl)-3-yle, (propanoate de méthyl)-3-yle, éthylpropanoat-3-yle, (butanoate d'éthyl)-3-yle, ((3R)-4,4,4,-trifluorobutanoate d'éthyl)-3-yle, butan-2-on-4-yle, 3-méthylbutan-2-on-4-yle, pent-3-én-2-on-4-yle, ((2S)-butanedioate de diméthyl)-2-yle, (pentanedioate de diméthyl)-3-yle, ((2R)-2-méthylpropanoate de méthyl)-3-yle, 4-méthoxycarbonylbenzyle, 3,5-difluorobenzyle, 3,4-difluorobenzyle,
2,6-difluorobenzyle, 5-méthylpyridin-3-ylméthyle, tétrahydrofurann-3-yle et butan-1-ol-4-yle ;
X², X⁴ et X⁶ signifiant indépendamment l'un de l'autre à chaque fois hydrogène, fluor, chlore, brome, iode, cyano, nitro
ou (C₁₋₄)-alkyle, (C₃₋₅)-cycloalkyle, (C₂₋₄)-alcényle, (C₂₋₄)-alcynyle, (C₁₋₄)-alcoxyle, (C₂₋₄)-alcényloxyle, (C₂₋₄)-alcynyloxyle ou (C₁₋₄)-alkylcarbonyle à chaque fois substitué par m radicaux choisis dans le groupe constitué par fluor, chlore, brome, iode, cyano et (C₁₋₄)-alcoxyle ;
X³ signifiant fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₃₋₆)-cycloalcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
X⁵ signifiant hydrogène, fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO²R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₃₋₆)-cycloalcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
m signifiant 0, 1, 2, 3, 4 ou 5 ;
n signifiant 0, 1 ou 2 ;
p signifiant 0 ou 1 ;
q signifiant 3, 4 ou 5 ;
étant entendu que
a) X³ et X⁴ ne signifient pas en même temps alcoxyle substitué ou non substitué,
b) dans les composés, dans lesquels R³ représente méthyle et W* représente COOH, X⁵ ne signifie pas hydrogène et
c) les composés 3-(3-cyanophényl)-5-(isopropoxyméthyl)-4,5-dihydro-1,2-oxazole-5-carboxylate de méthyle, 5-(butoxyméthyl)-3-(3-cyanophényl)-4,5-dihydro-1,2-oxazole-5-carboxylate de méthyle, 3-(3-cyanophényl)-5-(méthoxyméthyl)-4,5-dihydro-1,2-oxazole-5-carboxylate de méthyle, 3-(3-cyanophényl)-5-(2-méthoxyéthyl)-4,5-dihydro-1,2-oxazole-5-carboxylate de méthyle, acide 3-(3-cyanophényl)-5-(trifluorométhyl)-4,5-dihydro-1,2-oxazole-5-carboxylique, 3-(3-cyanophényl)-5-méthyl-4,5-dihydro-1,2-oxazole-5-carboxylate de méthyle, 3-(3-cyanophényl)-5-méthyl-4,5-dihydro-1,2-oxazole-5-carboxylate d'éthyle, 5-méthyl-3-(3-nitrophényl)-4,5-dihydro-1,2-oxazole-5-carboxylate de méthyle, 3-(3,5-di-tert-butyl-4-hydroxyphényl)-5-méthyl-4,5-dihydro-1,2-oxazole-5-carboxylate de méthyle et 3-(3-chlorophényl)-5-méthyl-4,5-dihydro-1,2-oxazole-5-carboxylate d'éthyle sont exclus.

6. Agent herbicide selon la revendication 5 en mélange avec des adjuvants de formulation.

7. Agent herbicide selon la revendication 5 ou 6 contenant au moins une autre substance à activité pesticide du groupe des insecticides, des acaricides, des herbicides, des fongicides, des phytoprotecteurs et des régulateurs de croissance.

8. Agent herbicide selon la revendication 7 contenant un phytoprotecteur.

9. Agent herbicide selon la revendication 8, le phytoprotecteur étant choisi dans le groupe constitué par le méfenpyr-diéthyle, le cyprosulfamide, l'isoxadifène-éthyle, le cloquintocet-mexyl, le bénoxacor et le dichlormide.

10. Agent herbicide selon l'une quelconque des revendications 7 à 9 contenant un autre herbicide.

11. Utilisation de dérivés de 3-phénylisoxazoline de formule (Ia) 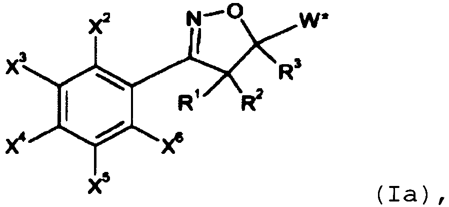
R¹ et R² signifiant indépendamment l'un de l'autre à chaque fois hydrogène, fluor, chlore, brome, iode, cyano ou (C₁₋₄)-alkyle ou (C₁₋₄)-alcoxyle substitué par à chaque fois m radicaux choisis dans le groupe constitué par fluor, chlore, brome, iode et cyano,
ou
R¹ et R² formant conjointement avec l'atome de carbone auquel ils sont liés, un cycle saturé, partiellement insaturé ou totalement insaturé à trois, quatre ou cinq chaînons, qui est constitué de q atomes de carbone et p atomes d'oxygène ;
R³ signifiant fluor, chlore, cyano, (C₁₋₃)-alkylcarbonyloxyle ou S(O)ₙR⁵,
ou (C₁₋₆)-alkyle, (C₃₋₆)-cycloalkyle, (C₂₋₆)-alcényle, (C₂₋₆)-alcynyle substitué par à chaque fois m radicaux choisis dans le groupe constitué par fluor, chlore, brome, iode, cyano, (C₁₋₄)-alcoxyle et hydroxyle
ou (C₁₋₆)-alkylcarbonyle, (C₂₋₆)-alcénylcarbonyle ou (C₃₋₆)-cycloalkylcarbonyle à chaque fois substitué par m radicaux choisis dans le groupe constitué par fluor, chlore, brome, iode, cyano et (C₁₋₆)-alcoxyle ;
R⁵ signifiant (C₁₋₆)-alkyle ou (C₃₋₆)-cycloalkyle substitué par à chaque fois m radicaux choisis dans le groupe constitué par fluor, chlore, brome, iode, cyano et hydroxyle ;
R⁶ signifiant hydrogène ou R⁵ ;
R⁷ signifiant hydrogène ou (C₁₋₆)-alkyle, (C₃₋₆)-cycloalkyle, (C₂₋₄)-alcényle ou (C₂₋₄)-alcynyle substitué par à chaque fois m radicaux choisis dans le groupe constitué par fluor, chlore, brome, cyano et (C₁₋₂)-alcoxyle ;
R⁸ signifiant R⁷ ;
W* signifiant COOH, COOY, CN ou CHO ;
Y signifiant (C₁₋₈)-alkyle substitué par m radicaux choisis dans le groupe constitué par fluor, chlore, brome, cyano, hydroxyle et COO-(C₁₋₈)-alkyle, qui est interrompu par n hétéroatomes choisis dans le groupe constitué par oxygène, soufre et azote ou
un radical choisi dans le groupe constitué par cyclohex-2-én-1-on-3-yle, propan-1-ol-3-yle, 2,2-diméthylpropan-1-ol-3-yle, (2,2-diméthylpropanoate de méthyl)-3-yle, (propanoate de méthyl)-3-yle, éthylpropanoat-3-yle, (butanoate d'éthyl)-3-yle, ((3R)-4,4,4,-trifluorobutanoate d'éthyl)-3-yle, butan-2-on-4-yle, 3-méthylbutan-2-on-4-yle, pent-3-én-2-on-4-yle, ((2*S*)-butanedioate de diméthyl)-2-yle, (pentanedioate de diméthyl)-3-yle, ((2*R*)-2-méthylpropanoate de méthyl)-3-yle, 4-méthoxycarbonylbenzyle, 3,5-difluorobenzyle, 3,4-difluorobenzyle,
2,6-difluorobenzyle, 5-méthylpyridin-3-ylméthyle, tétrahydrofurann-3-yle et butan-1-ol-4-yle ;
X², X⁴ et X⁶ signifiant indépendamment l'un de l'autre à chaque fois hydrogène, fluor, chlore, brome, iode, cyano, nitro
ou (C₁₋₄)-alkyle, (C₃₋₅)-cycloalkyle, (C₂₋₄)-alcényle, (C₂₋₄)-alcynyle, (C₁₋₄)-alcoxyle, (C₂₋₄)-alcényloxyle, (C₂₋₄)-alcynyloxyle ou (C₁₋₄)-alkylcarbonyle à chaque fois substitué par m radicaux choisis dans le groupe constitué par fluor, chlore, brome, iode, cyano et (C₁₋₄)-alcoxyle ;
X³ signifiant fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸,NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₃₋₆)-cycloalcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
X⁵ signifiant hydrogène, fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₃₋₆)-cycloalcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
m signifiant 0, 1, 2, 3, 4 ou 5 ;
n signifiant 0, 1 ou 2 ;
p signifiant 0 ou 1 ;
q signifiant 3, 4 ou 5,
pour la lutte contre des plantes indésirables.

12. Utilisation de dérivés de 3-phénylisoxazoline selon la revendication 11,
R¹ et R² signifiant à chaque fois hydrogène,
R³ signifiant (C₁₋₆)-alkyle, (C₁₋₄)-alcoxy- (C₁₋₄)-alkyle, (C₂₋₆)-alcényle, (C₂₋₆)-alcynyle, (C₁₋₆)-halogénoalkyle, ou (C₂₋₆)-halogénoalcényle ;
R⁵ signifiant (C₁₋₆)-alkyle ou (C₃₋₆)-cycloalkyle substitué par à chaque fois m radicaux choisis dans le groupe constitué par fluor, chlore, brome, iode, cyano et hydroxyle ;
R⁶ signifiant hydrogène ou R⁵ ;
R⁷ signifiant hydrogène ou (C₁₋₆)-alkyle, (C₃₋₆)-cycloalkyle, (C₂₋₄)-alcényle ou (C₂₋₄)-alcynyle substitué par à chaque fois m radicaux choisis dans le groupe constitué par fluor, chlore, brome, cyano et (C₁₋₂)-alcoxyle ;
R⁸ signifiant R⁷ ;
W* signifiant COOH ou COOY ;
Y signifiant (C₁₋₆)-alkyle substitué par m radicaux choisis dans le groupe constitué par fluor, chlore, brome, cyano, hydroxyle et COO-(C₁₋₄)-alkyle, qui est interrompu par n atomes d'oxygène ou
un radical choisi dans le groupe constitué par cyclohex-2-én-1-on-3-yle, propan-1-ol-3-yle, 2,2-diméthylpropan-1-ol-3-yle, (2,2-diméthylpropanoate de méthyl)-3-yle, (propanoate de méthyl)-3-yle, éthylpropanoat-3-yle, (butanoate d'éthyl)-3-yle, ((3R)-4,4,4,-trifluorobutanoate d'éthyl)-3-yle, butan-2-on-4-yle, 3-méthylbutan-2-on-4-yle, pent-3-én-2-on-4-yle, ((2*S*)-butanedioate de diméthyl)-2-yle, (pentanedioate de diméthyl)-3-yle, ((2R)-2-méthylpropanoate de méthyl)-3-yle, 4-méthoxycarbonylbenzyle, 3,5-difluorobenzyle, 3,4-difluorobenzyle,
2,6-difluorobenzyle, 5-méthylpyridin-3-ylméthyle, tétrahydrofurann-3-yle et butan-1-ol-4-yle ;
X², X⁴ et X⁶ signifiant indépendamment l'un de l'autre à chaque fois hydrogène, fluor, chlore, brome, iode, cyano, nitro
ou (C₁₋₄)-alkyle, (C₃₋₅)-cycloalkyle, (C₂₋₄)-alcényle, (C₂₋₄)-alcynyle, (C₁₋₄)-alcoxyle, (C₂₋₄)-alcényloxyle, (C₂₋₄)-alcynyloxyle ou (C₁₋₄)-alkylcarbonyle à chaque fois substitué par m radicaux choisis dans le groupe constitué par fluor, chlore, brome, iode, cyano et (C₁₋₄)-alcoxyle ;
X³ signifiant fluor, chlore, brome, iode, hydroxyle, cyano, nitro, SF₅, CONR⁸SO₂R⁵, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸ OCONR⁶R⁸, OSO₂R⁵, R⁵, S(O)ₙR⁵, SO²NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₃₋₆)-cycloalcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
X⁵ signifiant hydrogène, fluor, chlore, brome, iode, hydroxyle, cyano, nitro, SF₅, CONR⁸SO₂R⁶, CONR⁶R⁸, COR⁶, CO₂R⁸, CONR⁶R⁸, C(R⁶)=NOR⁸, NR⁶COR⁸, NR⁶CONR⁸R⁸, NR⁶CO₂R⁸, NR⁶SO₂R⁸, NR⁶SO₂NR⁶R⁸, OCONR⁶R⁸, OSO₂R⁵, R⁵, S(O)ₙR⁵, SO₂NR⁶R⁸, OSO₂NR⁶R⁸,
ou (C₁₋₆)-alkyle, (C₃₋₅)-cycloalkyle, (C₂₋₆)-alcényle, (C₂₋₆)-alcynyle à chaque fois substitué par m radicaux choisis dans le groupe constitué par fluor, chlore, brome, iode, hydroxyle et cyano ou (C₁₋₆)-alcoxyle, (C₃₋₆)-cycloalcoxyle, (C₂₋₆)-alcényloxyle ou (C₂₋₆)-alcynyloxyle à chaque fois substitué par m radicaux choisis dans le groupe constitué par fluor, chlore, brome, iode, cyano et (C₁₋₂)-alcoxyle ;
m signifiant 0, 1, 2, 3, 4 ou 5 ;
n signifiant 0, 1 ou 2.

13. Utilisation de dérivés de 3-phénylisoxazoline selon la revendication 11 ou 12,
R¹ et R² signifiant à chaque fois hydrogène ;
R³ signifiant (C₁₋₄)-alkyle, (C₁₋₄)-alcoxy-(C₁₋₄)-alkyle, vinyle, (C₂₋₄)-alcynyle, (C₁₋₄)-halogénoalkyle, ou (C₂₋₄)-halogénoalcényle ;
R⁵ signifiant méthyle ou éthyle ;
R⁶ signifiant hydrogène ou R⁵ ;
R⁷ signifiant hydrogène ou (C₁₋₆)-alkyle substitué par à chaque fois m radicaux choisis dans le groupe constitué par fluor et chlore ;
R⁸ signifiant R⁷ ;
W* signifiant COOH ou COOY ;
Y signifiant (C₁₋₆)-alkyle substitué par m radicaux choisis dans le groupe constitué par fluor, chlore, brome, cyano, hydroxyle et COO-(C₁₋₄)-alkyle, qui est interrompu par n atomes d'oxygène ;
X², X⁴ et X⁶ signifiant indépendamment l'un de l'autre à chaque fois hydrogène, fluor ou chlore ou (C₁₋₄)-alkyle ou (C₁₋₄)-alcoxyle à chaque fois substitué par m radicaux choisis dans le groupe constitué par fluor, chlore, cyano et (C₁₋₄)-alcoxyle ;
X³ signifiant fluor, chlore, brome, cyano,
ou (C₁₋₆)-alkyle à chaque fois substitué par m radicaux choisis dans le groupe constitué par fluor et chlore
ou (C₁₋₆)-alcoxyle substitué par m radicaux choisis dans le groupe constitué par fluor et chlore ;
X⁵ signifiant hydrogène, fluor, chlore, brome, cyano
ou (C₁₋₆)-alkyle à chaque fois substitué par m radicaux choisis dans le groupe constitué par fluor et chlore
ou (C₁₋₆)-alcoxyle substitué par m radicaux choisis dans le groupe constitué par fluor et chlore ;
m signifiant 0, 1, 2 ou 3 ;
n signifiant 0, 1 ou 2.

14. Utilisation de dérivés de 3-phénylisoxazoline selon la revendication 13,
X⁵ signifiant hydrogène, fluor, chlore, brome, cyano
ou (C₁₋₆)-alkyle à chaque fois substitué par m radicaux choisis dans le groupe constitué par fluor et chlore
ou (C₁₋₆)-alcoxyle substitué par m radicaux choisis dans le groupe constitué par fluor et chlore.

15. Procédé pour la lutte contre les plantes indésirables, **caractérisé en ce qu'**on applique une quantité efficace d'au moins un composé de formule (I) selon l'une quelconque des revendications 1 à 4 ou d'au moins un composé de formule (Ia) selon l'une quelconque des revendications 11 à 14 ou d'un agent herbicide selon l'une des revendications 5 à 10, sur les plantes ou sur le lieu de croissance des plantes indésirables.

16. Utilisation d'agents herbicides selon l'une quelconque des revendications 5 à 10 pour la lutte contre des plantes indésirables.

17. Utilisation selon l'une quelconque des revendications 11 à 14 ou 16, **caractérisée en ce que** les composés de formule (I) sont utilisés pour la lutte contre des plantes indésirables dans des cultures de plantes utiles.

18. Utilisation selon la revendication 17, **caractérisée en ce que** les plantes utiles sont des plantes utiles transgéniques.
